(19) Europäisches Patentamt
European Patent Office
Office européen des brevets

(11) EP 1 887 081 A2

(12) **EUROPEAN PATENT APPLICATION**

(43) Date of publication:
**13.02.2008 Bulletin 2008/07**

(21) Application number: **06026905.7**

(22) Date of filing: **25.02.2000**

(51) Int Cl.:
*C12N 15/29* (2006.01) *C07K 14/415* (2006.01)
*C12N 15/82* (2006.01) *A01H 5/00* (2006.01)

(84) Designated Contracting States:
**AT BE CH CY DE DK ES FI FR GB GR IE IT LI LU MC NL PT SE**

(30) Priority:
**05.03.1999 US 123180 P**
**09.03.1999 US 123548 P**
**23.03.1999 US 125788 P**
**25.03.1999 US 126264 P**
**29.03.1999 US 126785 P**
**01.04.1999 US 127462 P**
**16.04.1999 US 129845 P**
**19.04.1999 US 130077 P**
**21.04.1999 US 130449 P**
**23.04.1999 US 130891 P**
**23.04.1999 US 130510 P**
**30.04.1999 US 132407 P**
**04.05.1999 US 132484 P**
**05.05.1999 US 132485 P**
**06.05.1999 US 132486 P**
**07.05.1999 US 132863 P**
**11.05.1999 US 134256 P**
**14.05.1999 US 134218 P**
**14.05.1999 US 134219 P**
**16.06.1999 US 139452 P**
**16.06.1999 US 139453 P**
**18.06.1999 US 139461 P**
**18.06.1999 US 139463 P**
**18.06.1999 US 139457 P**
**18.06.1999 US 139459 P**
**18.06.1999 US 139462 P**
**18.06.1999 US 139455 P**
**18.06.1999 US 139458 P**
**18.06.1999 US 139454 P**
**18.06.1999 US 139456 P**
**18.06.1999 US 139460 P**
**16.07.1999 US 144085 P**
**16.07.1999 US 144086 P**
**19.07.1999 US 144333 P**
**19.07.1999 US 144335 P**
**19.07.1999 US 144334 P**
**19.07.1999 US 144332 P**
**19.07.1999 US 144331 P**
**20.07.1999 US 144884 P**
**20.07.1999 US 144352 P**
**21.07.1999 US 145086 P**
**21.07.1999 US 145088 P**
**22.07.1999 US 145085 P**
**22.07.1999 US 145089 P**
**22.07.1999 US 145087 P**
**23.07.1999 US 145145 P**
**23.07.1999 US 145224 P**
**27.07.1999 US 145919 P**
**25.02.1999 US 121825 P**
**27.07.1999 US 145918 P**
**02.08.1999 US 146388 P**
**02.08.1999 US 146389 P**
**04.08.1999 US 147302 P**
**05.08.1999 US 147192 P**
**06.08.1999 US 147303 P**
**09.08.1999 US 147935 P**
**10.08.1999 US 148171 P**
**13.08.1999 US 148684 P**
**16.08.1999 US 149368 P**
**20.08.1999 US 149929 P**
**23.08.1999 US 149930 P**
**14.10.1999 US 159329 P**

(83) **Declaration under Rule 28(4) EPC (expert solution)**

(62) Document number(s) of the earlier application(s) in accordance with Art. 76 EPC:
**00301439.6 / 1 033 405**

(71) Applicant: **Ceres Incorporated**
**Thousand Oaks, CA 91320 (US)**

(72) Inventors:
- **Alexandrov, Nickolai**
  **Thousand Oaks**
  **California 91320 (US)**
- **Chen, Xianfeng**
  **Los Angeles**
  **California 90025 (US)**
- **Subramanian, Gopalakrishnan**
  **Moorpark**
  **California 93021 (US)**
- **Brover, Vyacheslav**
  **Simi Valley**
  **California 93063 (US)**



Printed by Jouve, 75001 PARIS (FR)

**(Cont. next page)**

- **Troukhan, Maxim E.**
  **Agoura Hills**
  **Calofornia 91301 (US)**
- **Zheng, Liansheng**
  **Germantown**
  **Maryland 20874 (US)**
- **Dumas, J.**
  **Paris (FR)**

(74) Representative: **Elsy, David et al**
**Withers & Rogers LLP**
**Goldings House,**
**2 Hays Lane**
**London SE1 2HW (GB)**



Remarks:
•The complete document including Reference Tables and the Sequence Listing is available on CD-ROM from the European Patent Office, Vienna sub-office
•This application was filed on 27 - 12 - 2006 as a divisional application to the application mentioned under INID code 62.


(54) **DNA Sequences**


(57) The present invention provides DNA molecules that constitute fragments of the genome of a plant, and polypeptides encoded thereby. The DNA molecules are useful for specifying a gene product in cells, either as a promoter or as a protein coding sequence or as an UTR or as a 3' termination sequence, and are also useful in controlling the behavior of a gene in the chromosome, in controlling the expression of a gene or as tools for genetic mapping, recognizing or isolating identical or related DNA fragments, or identification of a particular individual organism, or for clustering of a group of organisms with a common trait.

## Description

## FIELD OF THE INVENTION

The present invention relates to isolated polynucleotides that represent a complete gene, or a fragment thereof, that is expressed. In addition, the present invention relates to the polypeptide or protein corresponding to the coding sequence of these polynucleotides. The present invention also relates to isolated polynucleotides that represent regulatory regions of genes. The present invention also relates to isolated polynucleotides that represent untranslated regions of genes. The present invention further relates to the use of these isolated polynucleotides and polypeptides and proteins.

## DESCRIPTION OF THE RELATED ART

Efforts to map and sequence the genome of a number of organisms are in progress; a few complete genome sequences, for example those of *E. coli* and *Saccharomyces cerevisiae are known* (Blattner et al., Science 277:1453 (1997); Goffeau et al., Science 274:546 (1996*)). The complete genome of a multicellular organism, C. elegans, has also been sequenced (See, the* C. elegans Sequencing Consortium, Science 282:2012 (1998*)). To date, no complete* genome of a plant has been sequenced, nor has a complete cDNA complement of any plant been sequenced.

## SUMMARY OF THE INVENTION

The present invention comprises polynucleotides, such as complete cDNA sequences and/or sequences of genomic DNA encompassing complete genes, fragments of genes, and/or regulatory elements of genes and/or regions with other functions and/or intergenic regions, hereinafter collectively referred to as Sequence-Determined DNA Fragments (SDFs), from different plant species, particularly corn, wheat, soybean, rice and *Arabidopsis thaliana,* and other plants and or mutants, variants, fragments or fusions of said SDFs and polypeptides or proteins derived therefrom. In some instances, the SDFs span the entirety of a protein-coding segment. In some instances, the entirety of an mRNA is represented. Other objects of the invention that are also represented by SDFs of the invention are control sequences, such as, but not limited to, promoters. Complements of any sequence of the invention are also considered part of the invention.

Other objects of the invention are polynucleotides comprising exon sequences, polynucleotides comprising intron sequences, polynucleotides comprising introns together with exons, intron/exon junction sequences, 5' untranslated sequences, and 3' untranslated sequences of the SDFs of the present invention. Polynucleotides representing the joinder of any exons described herein, in any arrangement, for example, to produce a sequence encoding any desirable amino acid sequence are within the scope of the invention.

The present invention also resides in probes useful for isolating and identifying nucleic acids that hybridize to an SDF of the invention. The probes can be of any length, but more typically are 12-2000 nucleotides in length; more typically, 15 to 200 nucleotides long; even more typically, 18 to 100 nucleotides long.

Yet another object of the invention is a method of isolating and/or identifying nucleic acids using the following steps:

(a) contacting a probe of the instant invention with a polynucleotide sample under conditions that permit hybridization and formation of a polynucleotide duplex; and
(b) detecting and/or isolating the duplex of step (a).

The conditions for hybridization can be from low to moderate to high stringency conditions. The sample can include a polynucleotide having a sequence unique in a plant genome. Probes and methods of the invention are useful, for example, without limitation, for mapping of genetic traits and/or for positional cloning of a desired fragment of genomic DNA.

Probes and methods of the invention can also be used for detecting alternatively spliced messages within a species. Probes and methods of the invention can further be used to detect or isolate related genes in other plant species using genomic DNA (gDNA) and/or cDNA libraries. In some instances, especially when longer probes and low to moderate stringency hybridization conditions are used, the probe will hybridize to a plurality of cDNA and/or gDNA sequences of a plant. This approach is useful for isolating representatives of gene families which are identifiable by possession of a common functional domain in the gene product or which have common cis-acting regulatory sequences. This approach is also useful for identifying orthologous genes from other organisms.

The present invention also resides in constructs for modulating the expression of the genes comprised of all or a fragment of an SDF. The constructs comprise all or a fragment of the expressed SDF, or of a complementary sequence. Examples of constructs include ribozymes comprising RNA encoded by an SDF or by a sequence complementary thereto, antisense constructs, constructs comprising coding regions or parts thereof, constructs comprising promoters, introns, untranslated regions, scaffold attachment regions, methylating regions, enhancing or reducing regions, DNA and chromatin conformation modifying sequences, etc. Such constructs can be constructed using viral, plasmid, bacterial artificial chromosomes (BACs), plasmid artificial chromosomes (PACs), autonomous plant plasmids, plant artificial chromosomes or other types of vectors and exist in the plant as autonomous replicating sequences or as DNA integrated into the genome. When inserted into a host cell the construct is, preferably, functionally integrated with, or operatively linked to, a heterologous polynucleotide. For instance, a coding region from an SDF might be operably linked to a promoter that is functional in a plant.

The present invention also resides in host cells, including bacterial or yeast cells or plant cells, and plants that harbor constructs such as described above. Another aspect of the invention relates to methods for modulating expression of specific genes in plants by expression of the coding sequence of the constructs, by regulation of expression of one or more endogenous genes in a plant or by suppression of expression of the polynucleotides of the invention in a plant. Methods of modulation of gene expression include without limitation (1) inserting into a host cell additional copies of a polynucleotide comprising a coding sequence; (2) modulating an endogenous promoter in a host cell; (3) inserting antisense or ribozyme constructs into a host cell and (4) inserting into a host cell a polynucleotide comprising a sequence encoding a variant, fragment, or fusion of the native polypeptides of the instant invention.

## BRIEF DESCRIPTION OF THE TABLES

The sequences of exemplary SDFs and polypeptides corresponding to the coding sequences of the instant invention are described in Reference Tables 1 and 2, "REF Tables 1 and 2"; and in Sequence Tables 1 and 2, "SEQ Tables 1 and 2." The REF Tables refer to a number of "Maximum Length Sequences" or "MLS." Each MLS corresponds to the longest cDNA obtained, either by cloning or by the prediction from genomic sequence. The sequence of the MLS is the cDNA sequence as described in the Av subsection of the REF Tables.

The REF Table includes the following information relating to each MLS:

I. cDNA Sequence

- A. 5'UTR
- B. Coding Sequence
- C. 3' UTR

II. Genomic Sequence

- A. Exons
- B. Introns
- C. Promoters

III. Link of cDNA Sequences to Clone IDs
IV. Multiple Transcription Start Sites
V. Polypeptide Sequences

- A. Signal Peptide
- B. Domains
- C. Related Polypeptides

VI. Related Polynucleotide Sequences

## <u>I. cDNA SEQUENCE</u>

The REF Tables indicate which sequence in the SEQ Tables represents the sequence of each MLS. The MLS sequence can comprise 5' and 3' UTR as well as coding sequences. In addition, specific cDNA clone numbers also are included in the REF Tables when the MLS sequence relates to a specific cDNA clone.

A. 5'UTR

The location of the 5' UTR can be determined by comparing the most 5' MLS sequence with the corresponding genomic sequence as indicated in the REF Tables. The sequence that matches, beginning at any of the transcriptional

start sites and ending at the last nucleotide before any of the translational start sites corresponds to the 5' UTR.

B. Coding Region

The coding region is the sequence in any open reading frame found in the MLS. Coding regions of interest are indicated in the Poly P SEQ subsection of the REF Tables.

C. 3' UTR

The location of the 3' UTR can be determined by comparing the most 3' MLS sequence with the corresponding genomic sequence as indicated in the REF Tables. The sequence that matches, beginning at the translational stop site and ending at the last nucleotide of the MLS corresponds to the 3' UTR.

## II. GENOMIC SEQUENCE

Further, the REF Tables indicate the specific "gi" number of the genomic sequence if the sequence resides in a public databank. For each genomic sequence, the REF Tables indicate which regions are included in the MLS. These regions can include the 5' and 3' UTRs as well as the coding sequence of the MLS. See, for example, the scheme below:

```
                Region 1              Region 2           Region 3
              _________________     __________       _________________
---------| 5' UTR    | Exon |---------|  Exon  |--------| Exon | 3' UTR |-------
    ^        -----------------   ^    ----------    ^   -----------------   ^
    |                ^           |                  |         ^             |
Promoter             |         Intron             Intron      |
                Translational                              Stop Codon
                 Start Site
```

The REF Tables report the first and last base of each region that are included in an MLS sequence. An example is shown below:

gi No. 47000:
37102 ... 37497
37593 ... 37925

The numbers indicate that the MLS contains the following sequences from two regions of gi No. 47000; a first region including bases 37102-37497, and a second region including bases 37593-37925.

### A. EXON SEQUENCES

The location of the exons can be determined by comparing the sequence of the regions from the genomic sequences with the corresponding MLS sequence as indicated by the REF Tables.

#### i. INITIAL EXON

To determine the location of the initial exon, information from the

(1) polypeptide sequence section;
(2) cDNA polynucleotide section; and
(3) the genomic sequence section

of the REF Tables are used. First, the polypeptide section will indicate where the translational start site is located in the MLS sequence. The MLS sequence can be matched to the genomic sequence that corresponds to the MLS. Based on the match between the MLS and corresponding genomic sequences , the location of the translational start site can be determined in one of the regions of the genomic sequence. The location of this translational start site is the start of the

first exon.

Generally, the last base of the exon of the corresponding genomic region, in which the translational start site was located, will represent the end of the initial exon. In some cases, the initial exon will end with a stop codon, when the initial exon is the only exon.

In the case when sequences representing the MLS are in the positive strand of the corresponding genomic sequence, the last base will be a larger number than the first base. When the sequences representing the MLS are in the negative strand of the corresponding genomic sequence, then the last base will be a smaller number than the first base.

**ii. INTERNAL EXONS**

Except for the regions that comprise the 5' and 3' UTRs, initial exon, and terminal exon, the remaining genomic regions that match the MLS sequence are the internal exons. Specifically, the bases defining the boundaries of the remaining regions also define the intron/exon junctions of the internal exons.

**iii. TERMINAL EXON**

As with the initial exon, the location of the terminal exon is determined with information from the

(1) polypeptide sequence section;
(2) cDNA polynucleotide section; and
(3) the genomic sequence section

of the REF Tables. The polypeptide section will indicate where the stop codon is located in the MLS sequence. The MLS sequence can be matched to the corresponding genomic sequence. Based on the match between MLS and corresponding genomic sequences, the location of the stop codon can be determined in one of the regions of the genomic sequence. The location of this stop codon is the end of the terminal exon. Generally, the first base of the exon of the corresponding genomic region that matches the cDNA sequence, in which the stop codon was located, will represent the beginning of the terminal exon. In some cases, the translational start site will represent the start of the terminal exon, which will be the only exon.

In the case when the MLS sequences are in the positive strand of the corresponding genomic sequence, the last base will be a larger number than the first base. When the MLS sequences are in the negative strand of the corresponding genomic sequence, then the last base will be a smaller number than the first base.

**B. INTRON SEQUENCES**

In addition, the introns corresponding to the MLS are defined by identifying the genomic sequence located between the regions where the genomic sequence comprises exons. Thus, introns are defined as starting one base downstream of a genomic region comprising an exon, and end one base upstream from a genomic region comprising an exon.

**C. PROMOTER SEQUENCES**

As indicated below, promoter sequences corresponding to the MLS are defined as sequences upstream of the first exon; more usually, as sequences upstream of the first of multiple transcription start sites; even more usually as sequences about 2,000 nucleotides upstream of the first of multiple transcription start sites.

**III. LINK of cDNA SEQUENCES to CLONE IDs**

As noted above, the REF tables identify the cDNA clone(s) that relate to each MLS. The MLS sequence can be longer than the sequences included in the cDNA clones. In such a case, the REF table indicates the region of the MLS that is included in the clone. If either the 5' or 3' termini of the cDNA clone sequence is the same as the MLS sequence, no mention will be made.

**IV. Multiple Transcription Start Sites**

Initiation of transcription can occur at a number of sites of the gene. The REF tables indicate the possible multiple transcription sites for each gene. In the REF tables, the location of the transcription start sites can be either a positive or negative number.

The positions indicated by positive numbers refer to the transcription start sites as located in the MLS sequence. The negative numbers indicate the transcription start site within the genomic sequence that corresponds to the MLS.

To determine the location of the transcription start sites with the negative numbers, the MLS sequence is aligned with the corresponding genomic sequence. In the instances when a public genomic sequence is referenced, the relevant corresponding genomic sequence can be found by direct reference to the nucleotide sequence indicated by the "gi" number shown in the public genomic DNA section of the REF tables. When the position is a negative number, the transcription start site is located in the corresponding genomic sequence upstream of the base that matches the beginning of the MLS sequence in the alignment. The negative number is relative to the first base of the MLS sequence which matches the genomic sequence corresponding to the relevant "gi" number.

In the instances when no public genomic DNA is referenced, the relevant nucleotide sequence for alignment is the nucleotide sequence associated with the amino acid sequence designated by "gi" number of the later PolyP SEQ subsection.

## V. Polypeptide Sequences

The PolyP SEQ subsection lists SEQ ID NOs and Ceres SEQ ID NO for polypeptide sequences corresponding to the coding sequence of the MLS sequence and the location of the translational start site with the coding sequence of the MLS sequence.

The MLS sequence can have multiple translational start sites and can be capable of producing more than one polypeptide sequence.

### A. Signal Peptide

The REF Tables also indicate in subsection (B) the cleavage site of the putative signal peptide of the polypeptide corresponding to the coding sequence of the MLS sequence. Typically, signal peptide coding sequences comprise a sequence encoding the first residue of the polypeptide to the cleavage site residue.

### B. Domains

Subsection (C) provides information regarding identified domains (where present) within the polypeptide and (where present) a name for the polypeptide domain.

### C. Related Polypeptides

Subsection (Dp) provides (where present) information concerning amino acid sequences that are found to be related and have some percentage of sequence identity to the polypeptide sequences of REF and SEQ TABLES 1 AND 2. These related sequences are identified by a "gi" number.

## VI. Related Polynucleotide Sequences

Subsection (Dn) provides polynucleotide sequences (where present) that are related to and have some percentage of sequence identity to the MLS or corresponding genomic sequence.

| Abbreviation | Description |
|---|---|
| Max Len. Seq. | Maximum Length Sequence |
| rel to | Related to |
| Clone Ids | Clone ID numbers |
| Pub gDNA | Public Genomic DNA |
| gi No. | gi number |
| Gen. seq. in cDNA | Genomic Sequence in cDNA (Each region for a single gene prediction is listed on a separate line. In the case of multiple gene predictions, the group of regions relating to a single prediction are separated by a blank line) |
| (Ac) cDNA SEQ | cDNA sequence |

(continued)

| Abbreviation | Description |
|---|---|
| - Pat. Appln. SEQ ID NO | Patent Application SEQ ID NO: |
| - Ceres SEQ ID NO: 1673877 | Ceres SEQ ID NO: |
| - SEQ # w. TSS | Location within the cDNA sequence, SEQ ID NO:, of Transcription Start Sites which are listed below |
| - Clone ID #: # -> # | Clone ID comprises bases # to # of the cDNA Sequence |
| PolyP SEQ | Polypeptide Sequence |
| - Pat. Appln. SEQ ID NO: | Patent Application SEQ ID NO: |
| - Ceres SEQ ID NO | Ceres SEQ ID NO: |
| - Loc. SEQ ID NO: @ nt. | Location of translational start site in cDNA of SEQ ID NO: at nucleotide number |
| (C) Pred. PP Nom. & Annot. | Nomination and Annotation of Domains within Predicted Polypeptide(s) |
| - (Title) | Name of Domain |
| - Loc. SEQ ID NO #: # -> # aa. | Location of the domain within the polypeptide of SEQ ID NO: from # to # amino acid residues. |
| (Dp) Rel. AA SEQ | Related Amino Acid Sequences |
| - Align. NO | Alignment number |
| - gi No | Gi number |
| - Desp. | Description |
| - % Idnt. | Percent identity |
| - Align. Len. | Alignment Length |
| - Loc. SEQ ID NO: # -> # aa | Location within SEQ ID NO: from # to # amino acid residue. |

**DETAILED DESCRIPTION OF THE INVENTION**

The invention relates to (I) polynucleotides and methods of use thereof, such as

IA. Probes, Primers and Substrates;
IB. Methods of Detection and Isolation;

B.1. Hybridization;
B.2. Methods of Mapping;
B.3. Southern Blotting;
B.4. Isolating cDNA from Related Organisms;
B.5. Isolating and/or Identifying Orthologous Genes

IC. Methods of Inhibiting Gene Expression

C.1. Antisense
C.2. Ribozyme Constructs;
C.3. Chimeraplasts;
C.4 Co-Suppression;
C.5. Transcriptional Silencing
C.6. Other Methods to Inhibit Gene Expression

ID. Methods of Functional Analysis;
IE. Promoter Sequences and Their Use;
IF. UTRs and/or Intron Sequences and Their Use; and

IG. Coding Sequences and Their Use.

The invention also relates to (II) polypeptides and proteins and methods of use thereof, such as I

IA. Native Polypeptides and Proteins

A.1 Antibodies
A.2 In Vitro Applications

IIB. Polypeptide Variants, Fragments and Fusions

B.1 Variants
B.2 Fragments
B.3 Fusions

The invention also includes (III) methods of modulating polypeptide production, such as

IIIA. Suppression

A.1 Antisense
A.2 Ribozymes
A.3 Co-suppression
A.4 Insertion of Sequences into the Gene to be Modulated
A.5 Promoter Modulation
A.6 Expression of Genes containing Dominant-Negative Mutations

IIIB. Enhanced Expression

B.1 Insertion of an Exogenous Gene
B.2 Promoter Modulation

The invention further concerns (IV) gene constructs and vector construction, such as

IVA. Coding Sequences
IVB. Promoters
IVC. Signal Peptides

The invention still further relates to

V Transformation Techniques

__Definitions__

Allelic variant An "allelic variant" is an alternative form of the same SDF, which resides at the same chromosomal locus in the organism. Allelic variations can occur in any portion of the gene sequence, including regulatory regions. Allelic variants can arise by normal genetic variation in a population. Allelic variants can also be produced by genetic engineering methods. An allelic variant can be one that is found in a naturally occurring plant, including a cultivar or ecotype. An allelic variant may or may not give rise to a phenotypic change, and may or may not be expressed. An allele can result in a detectable change in the phenotype of the trait represented by the locus. A phenotypically silent allele can give rise to a product.

Alternatively spliced messages Within the context of the current invention, "alternatively spliced messages" refers to mature mRNAs originating from a single gene with variations in the number and/or identity of exons, introns and/or intron-exon junctions.

Chimeric The term "chimeric" is used to describe genes, as defined supra, or contructs wherein at least two of the

elements of the gene or construct, such as the promoter and the coding sequence and/or other regulatory sequences and/or filler sequences and/or complements thereof, are heterologous to each other.

Constitutive Promoter: Promoters referred to herein as "constitutive promoters" actively promote transcription under most, but not necessarily all, environmental conditions and states of development or cell differentiation. Examples of constitutive promoters include the cauliflower mosaic virus (CaMV) 35S transcript initiation region and the 1' or 2' promoter derived from T-DNA of *Agrobacterium tumefaciens,* and other transcription initiation regions from various plant genes, such as the maize ubiquitin-1 promoter, known to those of skill.

Coordinately Expressed: The term "coordinately expressed," as used in the current invention, refers to genes that are expressed at the same or a similar time and/or stage and/or under the same or similar environmental conditions.

Domain: Domains are fingerprints or signatures that can be used to characterize protein families and/or parts of proteins. Such fingerprints or signatures can comprise conserved (1) primary sequence, (2) secondary structure, and/or (3) three-dimensional conformation. Generally, each domain has been associated with either a family of proteins or motifs. Typically, these families and/or motifs have been correlated with specific *in-vitro* and/or *in-vivo* activities. A domain can be any length, including the entirety of the sequence of a protein. Detailed descriptions of the domains, associated families and motifs, and correlated activities of the polypeptides of the instant invention are described below. Usually, the polypeptides with designated domain(s) can exhibit at least one activity that is exhibited by any polypeptide that comprises the same domain(s).

Endogenous The term "endogenous," within the context of the current invention refers to any polynucleotide, polypeptide or protein sequence which is a natural part of a cell or organisms regenerated from said cell.

Exogenous "Exogenous," as referred to within, is any polynucleotide, polypeptide or protein sequence, whether chimeric or not, that is initially or subsequently introduced into the genome of an individual host cell or the organism regenerated from said host cell by any means other than by a sexual cross. Examples of means by which this can be accomplished are described below, and include *Agrobacterium-mediated* transformation (of dicots - *e.g.* Salomon et al. EMBO J. 3:141 (1984); Herrera-Estrella et al. EMBO J. 2:987 (1983); of monocots, representative papers are those by Escudero et al., Plant J. 10:355 (1996), Ishida et al., Nature Biotechnology 14:745 (1996), May et al., Bio/Technology 13:486 (1995)), biolistic methods (Armaleo et al., Current Genetics 17:97 1990)), electroporation, *in planta* techniques, and the like. Such a plant containing the exogenous nucleic acid is referred to here as a $T_0$ for the primary transgenic plant and $T_1$ for the first generation. The term "exogenous" as used herein is also intended to encompass inserting a naturally found element into a non-naturally found location.

Filler sequence: As used herein, "filler sequence" refers to any nucleotide sequence that is inserted into DNA construct to evoke a particular spacing between particular components such as a promoter and a coding region and may provide an additional attribute such as a restriction enzyme site.

Gene: The term "gene," as used in the context of the current invention, encompasses all regulatory and coding sequence contiguously associated with a single hereditary unit with a genetic function (see SCHEMATIC 1). Genes can include non-coding sequences that modulate the genetic function that include, but are not limited to, those that specify polyadenylation, transcriptional regulation, DNA conformation, chromatin conformation, extent and position of base methylation and binding sites of proteins that control all of these. Genes comprised of "exons" (coding sequences), which may be interrupted by "introns" (non-coding sequences), encode proteins. A gene's genetic function may require only RNA expression or protein production, or may only require binding of proteins and/or nucleic acids without associated expression. In certain cases, genes adjacent to one another may share sequence in such a way that one gene will overlap the other. A gene can be found within the genome of an organism, artificial chromosome, plasmid, vector, etc., or as a separate isolated entity.

Gene Family: "Gene family" is used in the current invention to describe a group of functionally related genes, each of which encodes a separate protein.

Heterologous sequences: "Heterologous sequences" are those that are not operatively linked or are not contiguous to each other in nature. For example, a promoter from corn is considered heterologous to an *Arabidopsis* coding region sequence. Also, a promoter from a gene encoding a growth factor from corn is considered heterologous to a sequence encoding the com receptor for the growth factor. Regulatory element sequences, such as UTRs or 3' end termination sequences that do not originate in nature from the same gene as the coding sequence originates

from, are considered heterologous to said coding sequence. Elements operatively linked in nature and contiguous to each other are not heterologous to each other. On the other hand, these same elements remain operativley linked but become heterologous if other filler sequence is placed between them. Thus, the promoter and coding sequences of a corn gene expressing an amino acid transporter are not heterologous to each other, but the promoter and coding sequence of a corn gene operatively linked in a novel manner are heterologous.

Homologous gene In the current invention, "homologous gene" refers to a gene that shares sequence similarity with the gene of interest. This similarity may be in only a fragment of the sequence and often represents a functional domain such as, examples including without limitation a DNA binding domain, a domain with tyrosine kinase activity, or the like. The functional activities of homologous genes are not necessarily the same.

Inducible Promoter An "inducible promoter" in the context of the current invention refers to a promoter which is regulated under certain conditions, such as light, chemical concentration, protein concentration, conditions in an organism, cell, or organelle, etc. A typical example of an inducible promoter, which can be utilized with the polynucleotides of the present invention, is PARSK1, the promoter from the *Arabidopsis* gene encoding a serinethreonine kinase enzyme, and which promoter is induced by dehydration, abscissic acid and sodium chloride (Wang and Goodman, PlantJ. 8:37 (1995)) Examples of environmental conditions that may affect transcription by inducible promoters include anaerobic conditions, elevated temperature, or the presence of light.

Intergenic region "Intergenic region," as used in the current invention, refers to nucleotide sequence occurring in the genome that separates adjacent genes.

Mutant gene In the current invention, "mutant" refers to a heritable change in DNA sequence at a specific location. Mutants of the current invention may or may not have an associated identifiable function when the mutant gene is transcribed.

Orthologous Gene In the current invention "orthologous gene" refers to a second gene that encodes a gene product that performs a similar function as the product of a first gene. The orthologous gene may also have a degree of sequence similarity to the first gene. The orthologous gene may encode a polypeptide that exhibits a degree of sequence similarity to a polypeptide corresponding to a first gene. The sequence similarity can be found within a functional domain or along the entire length of the coding sequence of the genes and/or their corresponding polypeptides.

Percentage of sequence identity "Percentage of sequence identity," as used herein, is determined by comparing two optimally aligned sequences over a comparison window, where the fragment of the polynucleotide or amino acid sequence in the comparison window may comprise additions or deletions (e.g., gaps or overhangs) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by inspection. Given that two sequences have been identified for comparison, GAP and BESTFIT are preferably employed to determine their optimal alignment. Typically, the default values of 5.00 for gap weight and 0.30 for gap weight length are used. The term "substantial sequence identity" between polynucleotide or polypeptide sequences refers to polynucleotide or polypeptide comprising a sequence that has at least 80% sequence identity, preferably at least 85%, more preferably at least 90% and most preferably at least 95%, even more preferably, at least 96%, 97%, 98% or 99% sequence identity compared to a reference sequence using the programs.

Plant Promoter A "plant promoter" is a promoter capable of initiating transcription in plant cells and can drive or facilitate transcription of a fragment of the SDF of the instant invention or a coding sequence of the SDF of the instant invention. Such promoters need not be of plant origin. For example, promoters derived from plant viruses, such as the CaMV35S promoter or from *Agrobacterium tumefaciens* such as the T-DNA promoters, can be plant promoters. A typical example of a plant promoter of plant origin is the maize ubiquitin-1 (ubi-1)promoter known to

those of skill.

Promoter: The term "promoter," as used herein, refers to a region of sequence determinants located upstream from the start of transcription of a gene and which are involved in recognition and binding of RNA polymerase and other proteins to initiate and modulate transcription. A basal promoter is the minimal sequence necessary for assembly of a transcription complex required for transcription initiation. Basal promoters frequently include a "TATA box" element usually located between 15 and 35 nucleotides upstream from the site of initiation of transcription. Basal promoters also sometimes include a "CCAAT box" element (typically a sequence CCAAT) and/or a GGGCG sequence, usually located between 40 and 200 nucleotides, preferably 60 to 120 nucleotides, upstream from the start site of transcription.

Public sequence: The term "public sequence ," as used in the context of the instant application, refers to any sequence that has been deposited in a publicly accessible database. This term encompasses both amino acid and nucleotide sequences. Such sequences are publicly accessible, for example, on the BLAST databases on the NCBI FTP web site (accessible at ncbi.nlm.gov/blast). The database at the NCBI GTP site utilizes "gi" numbers assigned by NCBI as a unique identifier for each sequence in the databases, thereby providing a non-redundant database for sequence from various databases, including GenBank, EMBL, DBBJ, (DNA Database of Japan) and PDB (Brookhaven Protein Data Bank).

Regulatory Sequence The term "regulatory sequence," as used in the current invention, refers to any nucleotide sequence that influences transcription or translation initiation and rate, and stability and/or mobility of the transcript or polypeptide product. Regulatory sequences include, but are not limited to, promoters, promoter control elements, protein binding sequences, 5' and 3' UTRs, transcriptional start site, termination sequence, polyadenylation sequence, introns, certain sequences within a coding sequence, etc.

Related Sequences: "Related sequences" refer to either a polypeptide or a nucleotide sequence that exhibits some degree of sequence similarity with a sequence described by the REF and SEQ tables.

Scaffold Attachment Region (SAR) As used herein, "scaffold attachment region" is a DNA sequence that anchors chromatin to the nuclear matrix or scaffold to generate loop domains that can have either a transcriptionally active or inactive structure (Spiker and Thompson (1996) Plant Physiol. 110: 15-21).

Sequence-determined DNA fragments (SDFs) "Sequence-determined DNA fragments" as used in the current invention are isolated sequences of genes, fragments of genes, intergenic regions or contiguous DNA from plant genomic DNA or cDNA or RNA the sequence of which has been determined.

Signal Peptide A "signal peptide" as used in the current invention is an amino acid sequence that targets the protein for secretion, for transport to an intracellular compartment or organelle or for incorporation into a membrane. Signal peptides are indicated in the tables and a more detailed description located below.

Specific Promoter In the context of the current invention, "specific promoters" refers to a subset of inducible promoters that have a high preference for being induced in a specific tissue or cell and/or at a specific time during development of an organism. By "high preference" is meant at least 3-fold, preferably 5-fold, more preferably at least 10-fold still more preferably at least 20-fold, 50-fold or 100-fold increase in transcription in the desired tissue over the transcription in any other tissue. Typical examples of temporal and/or tissue specific promoters of plant origin that can be used with the polynucleotides of the present invention, are: PTA29, a promoter which is capable of driving gene transcription specifically in tapetum and only during anther development (Koltonow et al., Plant Cell 2:1201 (1990); RCc2 and RCc3, promoters that direct root-specific gene transcription in rice (Xu et al., Plant Mol. Biol. 27:237 (1995); TobRB27, a root-specific promoter from tobacco (Yamamoto et al., Plant Cell 3 :37 (1991)). Examples of tissue-specific promoters under developmental control include promoters that initiate transcription only in certain tissues or organs, such as root, ovule, fruit, seeds, or flowers. Other suitable promoters include those from genes encoding storage proteins or the lipid body membrane protein, oleosin. A few root-specific promoters are noted above.

Stringency "Stringency" as used herein is a function of probe length, probe composition (G + C content), and salt concentration, organic solvent concentration, and temperature of hybridization or wash conditions. Stringency is typically compared by the parameter $T_m$, which is the temperature at which 50% of the complementary molecules in the hybridization are hybridized, in terms of a temperature differential from $T_m$. High stringency conditions are those providing a condition of $T_m$ - 5°C to $T_m$ - 10°C. Medium or moderate stringency conditions are those providing

$T_m$ - 20°C to $T_m$ - 29°C. Low stringency conditions are those providing a condition of $T_m$ - 40°C to $T_m$ - 48°C. The relationship of hybridization conditions to $T_m$ (in °C) is expressed in the mathematical equation

$$T_m = 81.5 - 16.6(\log_{10}[Na^+]) + 0.41(\%G+C) - (600/N) \quad (1)$$

where N is the length of the probe. This equation works well for probes 14 to 70 nucleotides in length that are identical to the target sequence. The equation below for $T_m$ of DNA-DNA hybrids is useful for probes in the range of 50 to greater than 500 nucleotides, and for conditions that include an organic solvent (formamide).

$$T_m = 81.5+16.6 \log \{[Na^+]/(1+0.7[Na^+])\}+ 0.41(\%G+C)-500/L\ 0.63(\%formamide) \quad (2)$$

where L is the length of the probe in the hybrid. (P. Tijessen, "Hybridization with Nucleic Acid Probes" in Laboratory Techniques in Biochemistry and Molecular Biology, P.C. vand der Vliet, ed., c. 1993 by Elsevier, Amsterdam.) The $T_m$ of equation (2) is affected by the nature of the hybrid; for DNA-RNA hybrids $T_m$ is 10-15°C higher than calculated, for RNA-RNA hybrids $T_m$ is 20-25°C higher. Because the $T_m$ decreases about 1 °C for each 1 % decrease in homology when a long probe is used (Bonner et al., J. Mol. Biol. 81:123 (1973)), stringency conditions can be adjusted to favor detection of identical genes or related family members.

Equation (2) is derived assuming equilibrium and therefore, hybridizations according to the present invention are most preferably performed under conditions of probe excess and for sufficient time to achieve equilibrium. The time required to reach equilibrium can be shortened by inclusion of a hybridization accelerator such as dextran sulfate or another high volume polymer in the hybridization buffer.

Stringency can be controlled during the hybridization reaction or after hybridization has occurred by altering the salt and temperature conditions of the wash solutions used. The formulas shown above are equally valid when used to compute the stringency of a wash solution. Preferred wash solution stringencies lie within the ranges stated above; high stringency is 5-8°C below $T_m$, medium or moderate stringency is 26-29°C below $T_m$ and low stringency is 45-48°C below $T_m$.

Substantially free of A composition containing A is "substantially free of " B when at least 85% by weight of the total A+B in the composition is A. Preferably, A comprises at least about 90% by weight of the total of A+B in the composition, more preferably at least about 95% or even 99% by weight. For example, a plant gene or a DNA sequence can be considered substantially free of other plant genes or DNA sequences.

Translational start site In the context of the current invention, a "translational start site" is usually an ATG in the cDNA transcript, more usually the first ATG. A single cDNA, however, may have multiple translational start sites.

Transcription start site "Transcription start site" is used in the current invention to describe the point at which transcription is initiated. This point is typically located about 25 nucleotides downstream from a TFIID binding site, such as a TATA box. Transcription can initiate at one or more sites within the gene, and a single gene may have multiple transcriptional start sites, some of which may be specific for transcription in a particular cell-type or tissue.

Untranslated region (UTR) A "UTR" is any contiguous series of nucleotide bases that is transcribed, but is not translated. These untranslated regions may be associated with particular functions such as increasing mRNA message stability. Examples of UTRs include, but are not limited to polyadenylation signals, terminations sequences, sequences located between the transcriptional start site and the first exon (5' UTR) and sequences located between the last exon and the end of the mRNA (3' UTR).

Variant: The term "variant" is used herein to denote a polypeptide or protein or polynucleotide molecule that differs from others of its kind in some way. For example, polypeptide and protein variants can consist of changes in amino acid sequence and/or charge and/or post-translational modifications (such as glycosylation, etc).

## DETAILED DESCRIPTION OF THE INVENTION

### I. Polynucleotides

Exemplified SDFs of the invention represent fragments of the genome of corn, wheat, rice, soybean or *Arabidopsis* and/or represent mRNA expressed from that genome. The isolated nucleic acid of the invention also encompasses corresponding fragments of the genome and/or cDNA complement of other organisms as described in detail below.

Polynucleotides of the invention can be isolated from polynucleotide libraries using primers comprising sequence similar to those described by the REF and SEQ Tables. See, for example, the methods described in Sambrook et al., supra.

Alternatively, the polynucleotides of the invention can be produced by chemical synthesis. Such synthesis methods are described below.

It is contemplated that the nucleotide sequences presented herein may contain some small percentage of errors. These errors may arise in the normal course of determination of nucleotide sequences. Sequence errors can be corrected by obtaining seeds deposited under the accession numbers cited above, propagating them, isolating genomic DNA or appropriate mRNA from the resulting plants or seeds thereof, amplifying the relevant fragment of the genomic DNA or mRNA using primers having a sequence that flanks the erroneous sequence, and sequencing the amplification product.

LA. Probes, Primers and Substrates

SDFs of the invention can be applied to substrates for use in array applications such as, but not limited to, assays of global gene expression, for example under varying conditions of development, growth conditions. The arrays can also be used in diagnostic or forensic methods (WO95/35505, US 5,445,943 and US 5,410,270).

Probes and primers of the instant invention will hybridize to a polynucleotide comprising a sequence in REF and SEQ TABLES 1 AND 2. Though many different nucleotide sequences can encode an amino acid sequence, the sequences of REF and SEQ TABLES 1 AND 2 are generally preferred for encoding polypeptides of the invention. However, the sequence of the probes and/or primers of the instant invention need not be identical to those in REF and SEQ TABLES 1 AND 2 or the complements thereof. For example, some variation in probe or primer sequence and/or length can allow additional family members to be detected, as well as orthologous genes and more taxonomically distant related sequences. Similarly, probes and/or primers of the invention can include additional nucleotides that serve as a label for detecting the formed duplex or for subsequent cloning purposes.

Probe length will vary depending on the application. For use as primers, probes are 12-40 nucleotides, preferably 18-30 nucleotides long. For use in mapping, probes are preferably 50 to 500 nucleotides, preferably 100-250 nucleotides long. For Southern hybridizations, probes as long as several kilobases can be used as explained below.

The probes and/or primers can be produced by synthetic procedures such as the triester method of Matteucci et al. J. Am. Chem. Soc. 103:3185(1981); or according to Urdea et al. Proc. Natl. Acad. 80:7461 (1981) or using commercially available automated oligonucleotide synthesizers.

I.B. Methods of Detection and Isolation

The polynucleotides of the invention can be utilized in a number of methods known to those skilled in the art as probes and/or primers to isolate and detect polynucleotides, including, without limitation: Southerns, Northerns, Branched DNA hybridization assays, polymerase chain reaction, and microarray assays, and variations thereof. Specific methods given by way of examples, and discussed below include:

Hybridization
Methods of Mapping
Southern Blotting
Isolating cDNA from Related Organisms
Isolating and/or Identifying Orthologous Genes.

Also, the nucleic acid molecules of the invention can used in other methods, such as high density oligonucleotide hybridizing assays, described, for example, in U.S. Pat. Nos. 6,004,753; 5,945,306; 5,945,287; 5,945,308; 5,919,686; 5,919,661; 5,919,627; 5,874,248; 5,871,973; 5,871,971; and 5,871,930; and PCT Pub. Nos. WO 9946380; WO 9933981; WO 9933870; WO 9931252; WO 9915658; WO 9906572; WO 9858052; WO 9958672; and WO 9810858.

B.1. Hybridization

The isolated SDFs of REF and SEQ TABLES 1 AND 2 of the present invention can be used as probes and/or primers for detection and/or isolation of related polynucleotide sequences through hybridization. Hybridization of one nucleic acid to another constitutes a physical property that defines the subject SDF of the invention and the identified related sequences. Also, such hybridization imposes structural limitations on the pair. A good general discussion of the factors for determining hybridization conditions is provided by Sambrook et al. ("Molecular Cloning, a Laboratory Manual, 2nd ed., c. 1989 by Cold Spring Harbor Laboratory Press, Cold Spring Harbor, NY; *see esp.,* chapters 11 and 12). Additional considerations and details of the physical chemistry of hybridization are provided by G.H. Keller and M.M. Manak "DNA Probes", 2nd Ed. pp. 1-25, c. 1993 by Stockton Press, New York, NY.

Depending on the stringency of the conditions under which these probes and/or primers are used, polynucleotides exhibiting a wide range of similarity to those in REF and SEQ TABLES 1 AND 2 can be detected or isolated. When the practitioner wishes to examine the result of membrane hybridizations under a variety of stringencies, an efficient way to do so is to perform the hybridization under a low stringency condition, then to wash the hybridization membrane under increasingly stringent conditions.

When using SDFs to identify orthologous genes in other species, the practitioner will preferably adjust the amount of target DNA of each species so that, as nearly as is practical, the same number of genome equivalents are present for each species examined. This prevents faint signals from species having large genomes, and thus small numbers of genome equivalents per mass of DNA, from erroneously being interpreted as absence of the corresponding gene in the genome.

The probes and/or primers of the instant invention can also be used to detect or isolate nucleotides that are "identical" to the probes or primers. Two nucleic acid sequences or polypeptides are said to be "identical" if the sequence of nucleotides or amino acid residues, respectively, in the two sequences is the same when aligned for maximum correspondence as described below.

Isolated polynucleotides within the scope of the invention also include allelic variants of the specific sequences presented in REF and SEQ TABLES 1 AND 2. The probes and/or primers of the invention can also be used to detect and/or isolate polynucleotides exhibiting at least 80% sequence identity with the sequences of REF and SEQ TABLES 1 AND 2 or fragments thereof.

With respect to nucleotide sequences, degeneracy of the genetic code provides the possibility to substitute at least one base of the base sequence of a gene with a different base without causing the amino acid sequence of the polypeptide produced from the gene to be changed. Hence, the DNA of the present invention may also have any base sequence that has been changed from a sequence in REF and SEQ TABLES 1 AND 2 by substitution in accordance with degeneracy of genetic code. References describing codon usage include: Carels et al., J Mol. Evol. 46: 45 (1998) and Fennoy et al., Nucl. Acids Res. 21(23): 5294 (1993).

B.2. Mapping

The isolated SDF DNA of the invention can be used to create various types of genetic and physical maps of the genome of corn, Arabidopsis, soybean, rice, wheat, or other plants. Some SDFs may be absolutely associated with particular phenotypic traits, allowing construction of gross genetic maps. While not all SDFs will immediately be associated with a phenotype, all SDFs can be used as probes for identifying polymorphisms associated with phenotypes of interest. Briefly, one method of mapping involves total DNA isolation from individuals. It is subsequently cleaved with one or more restriction enzymes, separated according to mass, transferred to a solid support, hybridized with SDF DNA and the pattern of fragments compared. Polymorphisms associated with a particular SDF are visualized as differences in the size of fragments produced between individual DNA samples after digestion with a particular restriction enzyme and hybridization with the SDF. After identification of polymorphic SDF sequences, linkage studies can be conducted. By using the individuals showing polymorphisms as parents in crossing programs, F2 progeny recombinants or recombinant inbreds, for example, are then analyzed. The order of DNA polymorphisms along the chromosomes can be determined based on the frequency with which they are inherited together versus independently. The closer two polymorphisms are together in a chromosome the higher the probability that they are inherited together. Integration of the relative positions of all the polymorphisms and associated marker SDFs can produce a genetic map of the species, where the distances between markers reflect the recombination frequencies in that chromosome segment.

The use of recombinant inbred lines for such genetic mapping is described for *Arabidopsis* by Alonso-Blanco et al. (Methods in Molecular Biology, vol.82, "Arabidopsis Protocols", pp. 137-146, J.M. Martinez-Zapater and J. Salinas, eds., c. 1998 by Humana Press, Totowa, NJ) and for corn by Burr ("Mapping Genes with Recombinant Inbreds", pp. 249-254. In Freeling, M. and V. Walbot (Ed.), The Maize Handbook, c. 1994 by Springer-Verlag New York, Inc.: New York, NY, USA; Berlin Germany; Burr et al. Genetics (1998) 118: 519; Gardiner, J. et al., (1993) Genetics 134: 917). This procedure, however, is not limited to plants and can be used for other organisms (such as yeast) or for individual cells.

The SDFs of the present invention can also be used for simple sequence repeat (SSR) mapping. Rice SSR mapping is described by Morgante et al. (The Plant Journal (1993) 3: 165), Panaud et al. (Genome (1995) 38: 1170); Senior et al. (Crop Science (1996) 36: 1676), Taramino et al. (Genome (1996) 39: 277) and Ahn et al. (Molecular and General Genetics (1993) 241: 483-90). SSR mapping can be achieved using various methods. In one instance, polymorphisms are identified when sequence specific probes contained within an SDF flanking an SSR are made and used in polymerase chain reaction (PCR) assays with template DNA from two or more individuals of interest. Here, a change in the number of tandem repeats between the SSR-flanking sequences produces differently sized fragments (U.S. Patent 5,766,847). Alternatively, polymorphisms can be identified by using the PCR fragment produced from the SSR-flanking sequence specific primer reaction as a probe against Southern blots representing different individuals (U.H. Refseth et al., (1997) Electrophoresis 18: 1519).

Genetic and physical maps of crop species have many uses. For example, these maps can be used to devise positional cloning strategies for isolating novel genes from the mapped crop species. In addition, because the genomes of closely related species are largely syntenic (that is, they display the same ordering of genes within the genome), these maps can be used to isolate novel alleles from relatives of crop species by positional cloning strategies.

The various types of maps discussed above can be used with the SDFs of the invention to identify Quantitative Trait Loci (QTLs). Many important crop traits, such as the solids content of tomatoes, are quantitative traits and result from the combined interactions of several genes. These genes reside at different loci in the genome, oftentimes on different chromosomes, and generally exhibit multiple alleles at each locus. The SDFs of the invention can be used to identify QTLs and isolate specific alleles as described by de Vicente and Tanksley (Genetics 134:585 (1993)). In addition to isolating QTL alleles in present crop species, the SDFs of the invention can also be used to isolate alleles from the corresponding QTL of wild relatives. Transgenic plants having various combinations of QTL alleles can then be created and the effects of the combinations measured. Once a desired allele combination has been identified, crop improvement can be accomplished either through biotechnological means or by directed conventional breeding programs (for review *see* Tanksley and McCouch, Science 277:1063 (1997)).

In another embodiment, the SDFs can be used to help create physical maps of the genome of corn, *Arabidopsis* and related species. Where SDFs have been ordered on a genetic map, as described above, they can be used as probes to discover which clones in large libraries of plant DNA fragments in YACs, BACs, etc. contain the same SDF or similar sequences, thereby facilitating the assignment of the large DNA fragments to chromosomal positions. Subsequently, the large BACs, YACs, etc. can be ordered unambiguously by more detailed studies of their sequence composition (e.g. Marra et al. (1997) Genomic Research 7:1072-1084) and by using their end or other sequences to find the identical sequences in other cloned DNA fragments. The overlapping of DNA sequences in this way allows large contigs of plant sequences to be built that, when sufficiently extended, provide a complete physical map of a chromosome. Sometimes the SDFs themselves will provide the means of joining cloned sequences into a contig.

The patent publication WO95/35505 and U.S. Patents 5,445,943 and 5,410,270 describe scanning multiple alleles of a plurality of loci using hybridization to arrays of oligonucleotides. These techniques are useful for each of the types of mapping discussed above.

Following the procedures described above and using a plurality of the SDFs of the present invention, any individual can be genotyped. These individual genotypes can be used for the identification of particular cultivars, varieties, lines, ecotypes and genetically modified plants or can serve as tools for subsequent genetic studies involving multiple phenotypic traits.

B.3 Southern Blot Hybridization

The sequences from REF and SEQ TABLES 1 AND 2 can be used as probes for various hybridization techniques. These techniques are useful for detecting target polynucleotides in a sample or for determining whether transgenic plants, seeds or host cells harbor a gene or sequence of interest and thus might be expected to exhibit a particular trait or phenotype.

In addition, the SDFs from the invention can be used to isolate additional members of gene families from the same or different species and/or orthologous genes from the same or different species. This is accomplished by hybridizing an SDF to, for example, a Southern blot containing the appropriate genomic DNA or cDNA. Given the resulting hybridization data, one of ordinary skill in the art could distinguish and isolate the correct DNA fragments by size, restriction sites, sequence and stated hybridization conditions from a gel or from a library.

Identification and isolation of orthologous genes from closely related species and alleles within a species is particularly desirable because of their potential for crop improvement. Many important crop traits, such as the solid content of tomatoes, result from the combined interactions of the products of several genes residing at different loci in the genome. Generally, alleles at each of these loci can make quantitative differences to the trait. By identifying and isolating numerous alleles for each locus from within or different species, transgenic plants with various combinations of alleles can be created and the effects of the combinations measured. Once a more favorable allele combination has

been identified, crop improvement can be accomplished either through biotechnological means or by directed conventional breeding programs (Tanksley et al. Science 277:1063(1997)).

The results from hybridizations of the SDFs of the invention to, for example, Southern blots containing DNA from another species can also be used to generate restriction fragment maps for the corresponding genomic regions. These maps provide additional information about the relative positions of restriction sites within fragments, further distinguishing mapped DNA from the remainder of the genome.

Physical maps can be made by digesting genomic DNA with different combinations of restriction enzymes.

Probes for Southern blotting to distinguish individual restriction fragments can range in size from 15 to 20 nucleotides to several thousand nucleotides. More preferably, the probe is 100 to 1,000 nucleotides long for identifying members of a gene family when it is found that repetitive sequences would complicate the hybridization. For identifying an entire corresponding gene in another species, the probe is more preferably the length of the gene, typically 2,000 to 10,000 nucleotides, but probes 50-1,000 nucleotides long might be used. Some genes, however, might require probes up to 1,500 nucleotides long or overlapping probes constituting the full-length sequence to span their lengths.

Also, while it is preferred that the probe be homogeneous with respect to its sequence, it is not necessary. For example, as described below, a probe representing members of a gene family having diverse sequences can be generated using PCR to amplify genomic DNA or RNA templates using primers derived from SDFs that include sequences that define the gene family.

For identifying corresponding genes in another species, the next most preferable probe is a cDNA spanning the entire coding sequence, which allows all of the mRNA-coding fragment of the gene to be identified. Probes for Southern blotting can easily be generated from SDFs by making primers having the sequence at the ends of the SDF and using corn or *Arabidopsis* genomic DNA as a template. In instances where the SDF includes sequence conserved among species, primers including the conserved sequence can be used for PCR with genomic DNA from a species of interest to obtain a probe.

Similarly, if the SDF includes a domain of interest, that fragment of the SDF can be used to make primers and, with appropriate template DNA, used to make a probe to identify genes containing the domain. Alternatively, the PCR products can be resolved, for example by gel electrophoresis, and cloned and/or sequenced. Using Southern hybridization, the variants of the domain among members of a gene family, both within and across species, can be examined.

B.4.1 Isolating DNA from Related Organisms

The SDFs of the invention can be used to isolate the corresponding DNA from other organisms. Either cDNA or genomic DNA can be isolated. For isolating genomic DNA, a lambda, cosmid, BAC or YAC, or other large insert genomic library from the plant of interest can be constructed using standard molecular biology techniques as described in detail by Sambrook et al. 1989 (Molecular Cloning: A Laboratory Manual, 2nd ed. Cold Spring Harbor Laboratory Press, New York) and by Ausubel et al. 1992 (Current Protocols in Molecular Biology, Greene Publishing, New York).

To screen a phage library, for example, recombinant lambda clones are plated out on appropriate bacterial medium using an appropriate *E. coli* host strain. The resulting plaques are lifted from the plates using nylon or nitrocellulose filters. The plaque lifts are processed through denaturation, neutralization, and washing treatments following the standard protocols outlined by Ausubel et al. (1992). The plaque lifts are hybridized to either radioactively labeled or non-radioactively labeled SDF DNA at room temperature for about 16 hours, usually in the presence of 50% formamide and 5X SSC (sodium chloride and sodium citrate) buffer and blocking reagents. The plaque lifts are then washed at 42°C with 1% Sodium Dodecyl Sulfate (SDS) and at a particular concentration of SSC. The SSC concentration used is dependent upon the stringency at which hybridization occurred in the initial Southern blot analysis performed. For example, if a fragment hybridized under medium stringency (e.g., Tm - 20°C), then this condition is maintained or preferably adjusted to a less stringent condition (e.g., Tm-30°C) to wash the plaque lifts. Positive clones show detectable hybridization e.g., by exposure to X-ray films or chromogen formation. The positive clones are then subsequently isolated for purification using the same general protocol outlined above. Once the clone is purified, restriction analysis can be conducted to narrow the region corresponding to the gene of interest. The restriction analysis and succeeding subcloning steps can be done using procedures described by, for example Sambrook et al. (1989) cited above.

The procedures outlined for the lambda library are essentially similar to those used for YAC library screening, except that the YAC clones are harbored in bacterial colonies. The YAC clones are plated out at reasonable density on nitrocellulose or nylon filters supported by appropriate bacterial medium in petri plates. Following the growth of the bacterial clones, the filters are processed through the denaturation, neutralization, and washing steps following the procedures of Ausubel et al. 1992. The same hybridization procedures for lambda library screening are followed.

To isolate cDNA, similar procedures using appropriately modified vectors are employed. For instance, the library can be constructed in a lambda vector appropriate for cloning cDNA such as λgt11. Alternatively, the cDNA library can be made in a plasmid vector. cDNA for cloning can be prepared by any of the methods known in the art, but is preferably prepared as described above. Preferably, a cDNA library will include a high proportion of full-length clones.

B. 5. Isolating and/or Identifying Orthologous Genes

Probes and primers of the invention can be used to identify and/or isolate polynucleotides related to those in REF and SEQ TABLES 1 AND 2. Related polynucleotides are those that are native to other plant organisms and exhibit either similar sequence or encode polypeptides with similar biological activity. One specific example is an orthologous gene. Orthologous genes have the same functional activity. As such, orthologous genes may be distinguished from homologous genes. The percentage of identity is a function of evolutionary separation and, in closely related species, the percentage of identity can be 98 to 100%. The amino acid sequence of a protein encoded by an orthologous gene can be less than 75% identical, but tends to be at least75% or at least 80% identical, more preferably at least 90%, most preferably at least 95% identical to the amino acid sequence of the reference protein. To find orthologous genes, the probes are hybridized to nucleic acids from a species of interest under low stringency conditions, preferably one where sequences containing as much as 40-45% mismatches will be able to hybridize. This condition is established by $T_m$ - 40°C to Tm - 48°C *(see* below). Blots are then washed under conditions of increasing stringency. It is preferable that the wash stringency be such that sequences that are 85 to 100% identical will hybridize. More preferably, sequences 90 to 100% identical will hybridize and most preferably only sequences greater than 95% identical will hybridize. One of ordinary skill in the art will recognize that, due to degeneracy in the genetic code, amino acid sequences that are identical can be encoded by DNA sequences as little as 67% identical or less. Thus, it is preferable, for example, to make an overlapping series of shorter probes, on the order of 24 to 45 nucleotides, and individually hybridize them to the same arrayed library to avoid the problem of degeneracy introducing large numbers of mismatches.

As evolutionary divergence increases, genome sequences also tend to diverge. Thus, one of skill will recognize that searches for orthologous genes between more divergent species will require the use of lower stringency conditions compared to searches between closely related species. Also, degeneracy of the genetic code is more of a problem for searches in the genome of a species more distant evolutionarily from the species that is the source of the SDF probe sequences.

The SDFs of the invention can also be used as probes to search for genes that are related to the SDF within a species. Such related genes are typically considered to be members of a gene family. In such a case, the sequence similarity will often be concentrated into one or a few fragments of the sequence. The fragments of similar sequence that define the gene family typically encode a fragment of a protein or RNA that has an enzymatic or structural function. The percentage of identity in the amino acid sequence of the domain that defines the gene family is preferably at least 70%, more preferably 80 to 95%, most preferably 85 to 99%. To search for members of a gene family within a species, a low stringency hybridization is usually performed, but this will depend upon the size, distribution and degree of sequence divergence of domains that define the gene family. SDFs encompassing regulatory regions can be used to identify coordinately expressed genes by using the regulatory region sequence of the SDF as a probe.

In the instances where the SDFs are identified as being expressed from genes that confer a particular phenotype, then the SDFs can also be used as probes to assay plants of different species for those phenotypes.

I.C. Methods to Inhibit Gene Expression

The nucleic acid molecules of the present invention can be used to inhibit gene transcription and/or translation. Example of such methods include, without limitation:

Antisense Constructs;
Ribozyme Constructs;
Chimeraplast Constructs;
Co-Suppression;
Transcriptional Silencing; and
Other Methods of Gene Expression.

C.1 Antisense

In some instances it is desirable to suppress expression of an endogenous or exogenous gene. A well-known instance is the FLAVOR-SAVOR™ tomato, in which the gene encoding ACC synthase is inactivated by an antisense approach, thus delaying softening of the fruit after ripening. See for example, U.S. Patent No. 5,859,330; U.S. Patent No. 5,723,766; Oeller, et al, Science, 254:437-439(1991); and Hamilton et al, Nature, 346:284-287 (1990). Also, timing of flowering can be controlled by suppression of the *FLOWERING LOCUS C (FLC);* high levels of this transcript are associated with late flowering, while absence of *FLC* is associated with early flowering (S.D. Michaels et al., Plant Cell 11:949 (1999). Also, the transition of apical meristem from production of leaves with associated shoots to flowering is regulated by *TERMINAL FLOWER1, APETALA1* and *LEAFY* Thus, when it is desired to induce a transition from shoot

production to flowering, it is desirable to suppress *TFL1* expression (S.J. Liljegren, Plant Cell 11:1007 (1999)). As another instance, arrested ovule development and female sterility result from suppression of the ethylene forming enzyme but can be reversed by application of ethylene (D. De Martinis et al., Plant Cell 11:1061 (1999)). The ability to manipulate female fertility of plants is useful in increasing fruit production and creating hybrids.

In the case of polynucleotides used to inhibit expression of an endogenous gene, the introduced sequence need not be perfectly identical to a sequence of the target endogenous gene. The introduced polynucleotide sequence will typically be at least substantially identical to the target endogenous sequence.

Some polynucleotide SDFs in REF and SEQ TABLES 1 AND 2 represent sequences that are expressed in corn,wheat, rice, soybean *Arabidopsis* and/or other plants. Thus the invention includes using these sequences to generate antisense constructs to inhibit translation and/or degradation of transcripts of said SDFs, typically in a plant cell.

To accomplish this, a polynucleotide segment from the desired gene that can hybridize to the mRNA expressed from the desired gene (the "antisense segment") is operably linked to a promoter such that the antisense strand of RNA will be transcribed when the construct is present in a host cell. A regulated promoter can be used in the construct to control transcription of the antisense segment so that transcription occurs only under desired circumstances.

The antisense segment to be introduced generally will be substantially identical to at least a fragment of the endogenous gene or genes to be repressed. The sequence, however, need not be perfectly identical to inhibit expression. Further, the antisense product may hybridize to the untranslated region instead of or in addition to the coding sequence of the gene. The vectors of the present invention can be designed such that the inhibitory effect applies to other proteins within a family of genes exhibiting homology or substantial homology to the target gene.

For antisense suppression, the introduced antisense segment sequence also need not be full length relative to either the primary transcription product or the fully processed mRNA. Generally, a higher percentage of sequence identity can be used to compensate for the use of a shorter sequence. Furthermore, the introduced sequence need not have the same intron or exon pattern, and homology of non-coding segments may be equally effective. Normally, a sequence of between about 30 or 40 nucleotides and the full length of the transcript can be used, though a sequence of at least about 100 nucleotides is preferred, a sequence of at least about 200 nucleotides is more preferred, and a sequence of at least about 500 nucleotides is especially preferred.

C.2. Ribozymes

It is also contemplated that gene constructs representing ribozymes and based on the SDFs in REF AND SEQ TABLES 1 AND 2 are an object of the invention. Ribozymes can also be used to inhibit expression of genes by suppressing the translation of the mRNA into a polypeptide. It is possible to design ribozymes that specifically pair with virtually any target RNA and cleave the phosphodiester backbone at a specific location, thereby functionally inactivating the target RNA. In carrying out this cleavage, the ribozyme is not itself altered, and is thus capable of recycling and cleaving other molecules, making it a true enzyme. The inclusion of ribozyme sequences within antisense RNAs confers RNA-cleaving activity upon them, thereby increasing the activity of the constructs.

A number of classes of ribozymes have been identified. One class of ribozymes is derived from a number of small circular RNAs, which are capable of self-cleavage and replication in plants. The RNAs replicate either alone (viroid RNAs) or with a helper virus (satellite RNAs). Examples include RNAs from avocado sunblotch viroid and the satellite RNAs from tobacco ringspot virus, lucerne transient streak virus, velvet tobacco mottle virus, solanum nodiflorum mottle virus and subterranean clover mottle virus. The design and use of target RNA-specific ribozymes is described in Haseloff et al. Nature, 334:585 (1988).

Like the antisense constructs above, the ribozyme sequence fragment necessary for pairing need not be identical to the target nucleotides to be cleaved, nor identical to the sequences in REF AND SEQ TABLES 1 AND 2. Ribozymes may be constructed by combining the ribozyme sequence and some fragment of the target gene which would allow recognition of the target gene mRNA by the resulting ribozyme molecule. Generally, the sequence in the ribozyme capable of binding to the target sequence exhibits a percentage of sequence identity with at least 80%, preferably with at least 85%, more preferably with at least 90% and most preferably with at least 95%, even more preferably, with at least 96%, 97%, 98% or 99% sequence identity to some fragment of a sequence in REF AND SEQ TABLES 1 AND 2 or the complement thereof. The ribozyme can be equally effective in inhibiting mRNA translation by cleaving either in the untranslated or coding regions. Generally, a higher percentage of sequence identity can be used to compensate for the use of a shorter sequence. Furthermore, the introduced sequence need not have the same intron or exon pattern, and homology of non-coding segments may be equally effective.

C.3. Chimeraplasts

The SDFs of the invention, such as those described by the REF and SEQ Tables, can also be used to construct chimeraplasts that can be introduced into a cell to produce at least one specific nucleotide change in a sequence

corresponding to the SDF of the invention. A chimeraplast is an oligonucleotide comprising DNA and/or RNA that specifically hybridizes to a target region in a manner which creates a mismatched base-pair. This mismatched base-pair signals the cell's repair enzyme machinery which acts on the mismatched region resulting in the replacement, insertion or deletion of designated nucleotide(s). The altered sequence is then expressed by the cell's normal cellular mechanisms. Chimeraplasts can be designed to repair mutant genes, modify genes, introduce site-specific mutations, and/or act to interrupt or alter normal gene function (US Pat. Nos. 6,010,907 and 6,004,804; and PCT Pub. No. WO99/58723 and WO99/07865).

C.4. Sense Suppression

The SDFs of REF and SEQ TABLES 1 AND 2 of the present invention are also useful to modulate gene expression by sense suppression. Sense suppression represents another method of gene suppression by introducing at least one exogenous copy or fragment of the endogenous sequence to be suppressed.

Introduction of expression cassettes in which a nucleic acid is configured in the sense orientation with respect to the promoter into the chromosome of a plant or by a self-replicating virus has been shown to be an effective means by which to induce degradation of mRNAs of target genes. For an example of the use of this method to modulate expression of endogenous genes *see,* Napoli et al., The Plant Cell 2:279 (1990), and U.S. Patents Nos. 5,034,323, 5,231,020, and 5,283,184. Inhibition of expression may require some transcription of the introduced sequence.

For sense suppression, the introduced sequence generally will be substantially identical to the endogenous sequence intended to be inactivated. The minimal percentage of sequence identity will typically be greater than about 65%, but a higher percentage of sequence identity might exert a more effective reduction in the level of normal gene products. Sequence identity of more than about 80% is preferred, though about 95% to absolute identity would be most preferred. As with antisense regulation, the effect would likely apply to any other proteins within a similar family of genes exhibiting homology or substantial homology to the suppressing sequence.

C.5. Transcriptional Silencing

The nucleic acid sequences of the invention, including the SDFs of REF and SEQ TABLES 1 AND 2, and fragments thereof, contain sequences that can be inserted into the genome of an organism resulting in transcriptional silencing. Such regulatory sequences need not be operatively linked to coding sequences to modulate transcription of a gene. Specifically, a promoter sequence without any other element of a gene can be introduced into a genome to transcriptionally silence an endogenous gene (see, for example, Vaucheret, H et al. (1998) The Plant Journal 16: 651-659). As another example, triple helices can be formed using oligonucleotides based on sequences from REF AND SEQ TABLES 1 AND 2, fragments thereof, and substantially similar sequence thereto. The oligonucleotide can be delivered to the host cell and can bind to the promoter in the genome to form a triple helix and prevent transcription. An oligonucleotide of interest is one that can bind to the promoter and block binding of a transcription factor to the promoter. In such a case, the oligonucleotide can be complementary to the sequences of the promoter that interact with transcription binding factors.

C.6. Other Methods to Inhibit Gene Expression

Yet another means of suppressing gene expression is to insert a polynucleotide into the gene of interest to disrupt transcription or translation of the gene.

Low frequency homologous recombination can be used to target a polynucleotide insert to a gene by flanking the polynucleotide insert with sequences that are substantially similar to the gene to be disrupted. Sequences from REF AND SEQ TABLES 1 AND 2, fragments thereof, and substantially similar sequence thereto can be used for homologous recombination.

In addition, random insertion of polynucleotides into a host cell genome can also be used to disrupt the gene of interest. Azpiroz-Leehan et al., *Trends in Genetics 13:152* (1997). In this method, screening for clones from a library containing random insertions is preferred to identifying those that have polynucleotides inserted into the gene of interest. Such screening can be performed using probes and/or primers described above based on sequences from REF AND SEQ TABLES 1 AND 2, fragments thereof, and substantially similar sequence thereto. The screening can also be performed by selecting clones or $R_1$ plants having a desired phenotype.

I.D. Methods of Functional Analysis

The constructs described in the methods under I.C. above can be used to determine the function of the polypeptide encoded by the gene that is targeted by the constructs.

Down-regulating the transcription and translation of the targeted gene in the host cell or organisms, such as a plant, may produce phenotypic changes as compared to a wild-type cell or organism. In addition, *in vitro* assays can be used to determine if any biological activity, such as calcium flux, DNA transcription, nucleotide incorporation, etc., are being modulated by the down-regulation of the targeted gene.

Coordinated regulation of sets of genes, e.g., those contributing to a desired polygenic trait, is sometimes necessary to obtain a desired phenotype. SDFs of the invention representing transcription activation and DNA binding domains can be assembled into hybrid transcriptional activators. These hybrid transcriptional activators can be used with their corresponding DNA elements (i.e., those bound by the DNA-binding SDFs) to effect coordinated expression of desired genes (J.J. Schwarz et al., Mol. Cell. Biol. 12:266 (1992), A. Martinez et al., Mol. Gen. Genet. 261:546 (1999)).

The SDFs of the invention can also be used in the two-hybrid genetic systems to identify networks of protein-protein interactions (L. McAlister-Henn et al., Methods 19:330 (1999), J.C. Hu et al., Methods 20:80 (2000), M. Golovkin et al., J. Biol. Chem. 274:36428 (1999), K. Ichimura et al., Biochem. Biophys. Res. Comm. 253:532 (1998)). The SDFs of the invention can also be used in various expression display methods to identify important protein-DNA interactions (e.g. B. Luo et al., J. Mol. Biol. 266:479 (1997)).

I.E. <u>Promoters</u>

The SDFs of the invention are also useful as structural or regulatory sequences in a construct for modulating the expression of the corresponding gene in a plant or other organism, e.g. a symbiotic bacterium. For example, promoter sequences associated to SDFs of REF and SEQ TABLES 1 AND 2 of the present invention can be useful in directing expression of coding sequences either as constitutive promoters or to direct expression in particular cell types, tissues, or organs or in response to environmental stimuli.

With respect to the SDFs of the present invention a promoter is likely to be a relatively small portion of a genomic DNA (gDNA) sequence located in the first 2000 nucleotides upstream from an initial exon identified in a gDNA sequence or initial "ATG" or methionine codon or translational start site in a corresponding cDNA sequence. Such promoters are more likely to be found in the first 1000 nucleotides upstream of an initial ATG or methionine codon or translational start site of a cDNA sequence corresponding to a gDNA sequence. In particular, the promoter is usually located upstream of the transcription start site. The fragments of a particular gDNA sequence that function as elements of a promoter in a plant cell will preferably be found to hybridize to gDNA sequences presented and described in REF and REF AND SEQ TABLES 1 AND 2 at medium or high stringency, relevant to the length of the probe and its base composition.

Promoters are generally modular in nature. Promoters can consist of a basal promoter that functions as a site for assembly of a transcription complex comprising an RNA polymerase, for example RNA polymerase II. A typical transcription complex will include additional factors such as $TF_{II}B$, $TF_{II}D$, and $TF_{II}E$. Of these, $TF_{II}D$ appears to be the only one to bind DNA directly. The promoter might also contain one or more enhancers and/or suppressors that function as binding sites for additional transcription factors that have the function of modulating the level of transcription with respect to tissue specificity and of transcriptional responses to particular environmental or nutritional factors, and the like.

Short DNA sequences representing binding sites for proteins can be separated from each other by intervening sequences of varying length. For example, within a particular functional module, protein binding sites may be constituted by regions of 5 to 60, preferably 10 to 30, more preferably 10 to 20 nucleotides. Within such binding sites, there are typically 2 to 6 nucleotides that specifically contact amino acids of the nucleic acid binding protein. The protein binding sites are usually separated from each other by 10 to several hundred nucleotides, typically by 15 to 150 nucleotides, often by 20 to 50 nucleotides. DNA binding sites in promoter elements often display dyad symmetry in their sequence. Often elements binding several different proteins, and/or a plurality of sites that bind the same protein, will be combined in a region of 50 to 1,000 basepairs.

Elements that have transcription regulatory function can be isolated from their corresponding endogenous gene, or the desired sequence can be synthesized, and recombined in constructs to direct expression of a coding region of a gene in a desired tissue-specific, temporal-specific or other desired manner of inducibility or suppression. When hybridizations are performed to identify or isolate elements of a promoter by hybridization to the long sequences presented in REF AND SEQ TABLES 1 AND 2, conditions are adjusted to account for the above-described nature of promoters. For example short probes, constituting the element sought, are preferably used under low temperature and/or high salt conditions. When long probes, which might include several promoter elements are used, low to medium stringency conditions are preferred when hybridizing to promoters across species.

If a nucleotide sequence of an SDF, or part of the SDF, functions as a promoter or fragment of a promoter, then nucleotide substitutions, insertions or deletions that do not substantially affect the binding of relevant DNA binding proteins would be considered equivalent to the exemplified nucleotide sequence. It is envisioned that there are instances where it is desirable to decrease the binding of relevant DNA binding proteins to silence or down-regulate a promoter, or conversely to increase the binding of relevant DNA binding proteins to enhance or up-regulate a promoter and vice versa. In such instances, polynucleotides representing changes to the nucleotide sequence of the DNA-protein contact

region by insertion of additional nucleotides, changes to identity of relevant nucleotides, including use of chemically-modified bases, or deletion of one or more nucleotides are considered encompassed by the present invention. In addition, fragments of the promoter sequences described by the REF and SEQ Tables and variants thereof can be fused with other promoters or fragments to facilitate transcription and/or transcription in specific type of cells or under specific conditions.

Promoter function can be assayed by methods known in the art, preferably by measuring activity of a reporter gene operatively linked to the sequence being tested for promoter function. Examples of reporter genes include those encoding luciferase, green fluorescent protein, GUS, neo, cat and bar.

I.F. UTRs and Junctions

Polynucleotides comprising untranslated (UTR) sequences and intron/exon junctions are also within the scope of the invention. UTR sequences include introns and 5' or 3' untranslated regions (5' UTRs or 3' UTRs). Fragments of the sequences shown in REF AND SEQ TABLES 1 AND 2 can comprise UTRs and intron/exon junctions.

These fragments of SDFs, especially UTRs, can have regulatory functions related to, for example, translation rate and mRNA stability. Thus, these fragments of SDFs can be isolated for use as elements of gene constructs for regulated production of polynucleotides encoding desired polypeptides.

Introns of genomic DNA segments might also have regulatory functions. Sometimes regulatory elements, especially transcription enhancer or suppressor elements, are found within introns. Also, elements related to stability of heteronuclear RNA and efficiency of splicing and of transport to the cytoplasm for translation can be found in intron elements. Thus, these segments can also find use as elements of expression vectors intended for use to transform plants.

Just as with promoters UTR sequences and intron/exon junctions can vary from those shown in REF AND SEQ TABLES 1 AND 2. Such changes from those sequences preferably will not affect the regulatory activity of the UTRs or intron/exon junction sequences on expression, transcription, or translation unless selected to do so. However, in some instances, down- or up-regulation of such activity may be desired to modulate traits or phenotypic or *in vitro* activity.

I.G. Coding Sequences

Isolated polynucleotides of the invention can include coding sequences that encode polypeptides comprising an amino acid sequence encoded by sequences in REF AND SEQ TABLES 1 AND 2 or an amino acid sequence presented in REF AND SEQ TABLES 1 AND 2.

A nucleotide sequence encodes a polypeptide if a cell (or a cell free *in vitro* system) expressing that nucleotide sequence produces a polypeptide having the recited amino acid sequence when the nucleotide sequence is transcribed and the primary transcript is subsequently processed and translated by a host cell (or a cell free *in vitro* system) harboring the nucleic acid. Thus, an isolated nucleic acid that encodes a particular amino acid sequence can be a genomic sequence comprising exons and introns or a cDNA sequence that represents the product of splicing thereof. An isolated nucleic acid encoding an amino acid sequence also encompasses heteronuclear RNA, which contains sequences that are spliced out during expression, and mRNA, which lacks those sequences.

Coding sequences can be constructed using chemical synthesis techniques or by isolating coding sequences or by modifying such synthesized or isolated coding sequences as described above.

In addition to coding sequences encoding the polypeptide sequences of REF AND SEQ TABLES 1 AND 2, which are native to corn, *Arabidopsis,* soybean, rice, wheat, and other plants the isolated polynucleotides can be polynucleotides that encode variants, fragments, and fusions of those native proteins. Such polypeptides are described below in part II.

In variant polynucleotides generally, the number of substitutions, deletions or insertions is preferably less than 20%, more preferably less than 15%; even more preferably less than 10%, 5%, 3% or 1% of the number of nucleotides comprising a particularly exemplified sequence. It is generally expected that non-degenerate nucleotide sequence changes that result in 1 to 10, more preferably 1 to 5 and most preferably 1 to 3 amino acid insertions, deletions or substitutions will not greatly affect the function of an encoded polypeptide. The most preferred embodiments are those wherein 1 to 20, preferably 1 to 10, most preferably 1 to 5 nucleotides are added to, deleted from and/or substituted in the sequences specifically disclosed in REF AND SEQ TABLES 1 AND 2.

Insertions or deletions in polynucleotides intended to be used for encoding a polypeptide preferably preserve the reading frame. This consideration is not so important in instances when the polynucleotide is intended to be used as a hybridization probe.

## II. Polypeptides and Proteins

IIA. Native polypeptides and proteins

Polypeptides within the scope of the invention include both native proteins as well as variants, fragments, and fusions thereof. Polypeptides of the invention are those encoded by any of the six reading frames of sequences shown in REF AND SEQ TABLES 1 AND 2, preferably encoded by the three frames reading in the 5' to 3' direction of the sequences as shown.

Native polypeptides include the proteins encoded by the sequences shown in REF AND SEQ TABLES 1 AND 2. Such native polypeptides include those encoded by allelic variants.

Polypeptide and protein variants will exhibit at least 75% sequence identity to those native polypeptides of REF AND SEQ TABLES 1 AND 2. More preferably, the polypeptide variants will exhibit at least 85% sequence identity; even more preferably, at least 90% sequence identity; more preferably at least 95%, 96%, 97%, 98%, or 99% sequence identity. Fragments of polypeptide or fragments of polypeptides will exhibit similar percentages of sequence identity to the relevant fragments of the native polypeptide. Fusions will exhibit a similar percentage of sequence identity in that fragment of the fusion represented by the variant of the native peptide.

Furthermore, polypeptide variants will exhibit at least one of the functional properties of the native protein. Such properties include, without limitation, protein interaction, DNA interaction, biological activity, immunological activity, receptor binding, signal transduction, transcription activity, growth factor activity, secondary structure, three-dimensional structure, etc. As to properties related to *in vitro* or *in vivo* activities, the variants preferably exhibit at least 60% of the activity of the native protein; more preferably at least 70%, even more preferably at least 80%, 85%, 90% or 95% of at least one activity of the native protein.

One type of variant of native polypeptides comprises amino acid substitutions, deletions and/or insertions. Conservative substitutions are preferred to maintain the function or activity of the polypeptide.

Within the scope of percentage of sequence identity described above, a polypeptide of the invention may have additional individual amino acids or amino acid sequences inserted into the polypeptide in the middle thereof and/or at the N-terminal and/or C-terminal ends thereof. Likewise, some of the amino acids or amino acid sequences may be deleted from the polypeptide.

A.1 Antibodies

Isolated polypeptides can be utilized to produce antibodies. Polypeptides of the invention can generally be used, for example, as antigens for raising antibodies by known techniques. The resulting antibodies are useful as reagents for determining the distribution of the antigen protein within the tissues of a plant or within a cell of a plant. The antibodies are also useful for examining the production level of proteins in various tissues, for example in a wild-type plant or following genetic manipulation of a plant, by methods such as Western blotting.

Antibodies of the present invention, both polyclonal and monoclonal, may be prepared by conventional methods. In general, the polypeptides of the invention are first used to immunize a suitable animal, such as a mouse, rat, rabbit, or goat. Rabbits and goats are preferred for the preparation of polyclonal sera due to the volume of serum obtainable, and the availability of labeled anti-rabbit and anti-goat antibodies as detection reagents. Immunization is generally performed by mixing or emulsifying the protein in saline, preferably in an adjuvant such as Freund's complete adjuvant, and injecting the mixture or emulsion parenterally (generally subcutaneously or intramuscularly). A dose of 50-200 μg/injection is typically sufficient. Immunization is generally boosted 2-6 weeks later with one or more injections of the protein in saline, preferably using Freund's incomplete adjuvant. One may alternatively generate antibodies by *in vitro* immunization using methods known in the art, which for the purposes of this invention is considered equivalent to *in vivo* immunization.

Polyclonal antisera is obtained by bleeding the immunized animal into a glass or plastic container, incubating the blood at 25°C for one hour, followed by incubating the blood at 4°C for 2-18 hours. The serum is recovered by centrifugation (e.g., 1,000xg for 10 minutes). About 20-50 ml per bleed may be obtained from rabbits.

Monoclonal antibodies are prepared using the method of Kohler and Milstein, *Nature* 256: 495 (1975), or modification thereof. Typically, a mouse or rat is immunized as described above. However, rather than bleeding the animal to extract serum, the spleen (and optionally several large lymph nodes) is removed and dissociated into single cells. If desired, the spleen cells can be screened (after removal of nonspecifically adherent cells) by applying a cell suspension to a plate, or well, coated with the protein antigen. B-cells producing membrane-bound immunoglobulin specific for the antigen bind to the plate, and are not rinsed away with the rest of the suspension. Resulting B-cells, or all dissociated spleen cells, are then induced to fuse with myeloma cells to form hybridomas, and are cultured in a selective medium (e.g., hypoxanthine, aminopterin, thymidine medium, "HAT"). The resulting hybridomas are plated by limiting dilution, and are assayed for the production of antibodies which bind specifically to the immunizing antigen (and

which do not bind to unrelated antigens). The selected Mabsecreting hybridomas are then cultured either *in vitro (e.g.,* in tissue culture bottles or hollow fiber reactors), or *in vivo* (as ascites in mice).

Other methods for sustaining antibody-producing B-cell clones, such as by EBV transformation, are known.

If desired, the antibodies (whether polyclonal or monoclonal) may be labeled using conventional techniques. Suitable labels include fluorophores, chromophores, radioactive atoms (particularly $^{32}P$ and $^{125}I$), electron-dense reagents, enzymes, and ligands having specific binding partners. Enzymes are typically detected by their activity. For example, horseradish peroxidase is usually detected by its ability to convert 3,3',5,5'-tetramethylbenzidine (TNB) to a blue pigment, quantifiable with a spectrophotometer.

A.2 In Vitro Applications of Polypeptides

Some polypeptides of the invention will have enzymatic activities that are useful *in vitro.* For example, the soybean trypsin inhibitor (Kunitz) family is one of the numerous families of proteinase inhibitors. It comprises plant proteins which have inhibitory activity against serine proteinases from the trypsin and subtilisin families, thiol proteinases and aspartic proteinases. Thus, these peptides find *in vitro* use in protein purification protocols and perhaps in therapeutic settings requiring topical application of protease inhibitors.

Delta-aminolevulinic acid dehydratase (EC 4.2.1.24) (ALAD) catalyzes the second step in the biosynthesis of heme, the condensation of two molecules of 5-aminolevulinate to form porphobilinogen and is also involved in chlorophyll biosynthesis(Kaczor et al. (1994) Plant Physiol. 1-4: 1411-7; Smith (1988) Biochem. J. 249: 423-8; Schneider (1976) Z. naturforsch. [C] 31: 55-63). Thus, ALAD proteins can be used as catalysts in synthesis of heme derivatives. Enzymes of biosynthetic pathways generally can be used as catalysts for *in vitro* synthesis of the compounds representing products of the pathway.

Polypeptides encoded by SDFs of the invention can be engineered to provide purification reagents to identify and purify additional polypeptides that bind to them. This allows one to identify proteins that function as multimers or elucidate signal transduction or metabolic pathways. In the case of DNA binding proteins, the polypeptide can be used in a similar manner to identify the DNA determinants of specific binding (S. Pierrou et al., Anal. Biochem. 229:99 (1995), S. Chusacultanachai et al., J Biol. Chem. 274:23591 (1999), Q. Lin et al., J Biol. Chem. 272:27274 (1997)).

II.B . POLYPEPTIDE VARIANTS , FRAGMENTS, AND FUSIONS

Generally, variants , fragments, or fusions of the polypeptides encoded by the maximum length sequence(MLS) can exhibit at least one of the activities of the identified domains and/or related polypeptides described in Sections (C) and (D) of REF TABLES 1 and 2 corresponding to the MLS of interest.

II.B .(1) Variants

A type of variant of the native polypeptides comprises amino acid substitutions. Conservative substitutions, described above (see II.), are preferred to maintain the function or activity of the polypeptide. Such substitutions include conservation of charge, polarity, hydrophobicity, size, etc. For example, one or more amino acid residues within the sequence can be substituted with another amino acid of similar polarity that acts as a functional equivalent, for example providing a hydrogen bond in an enzymatic catalysis. Substitutes for an amino acid within an exemplified sequence are preferably made among the members of the class to which the amino acid belongs. For example, the nonpolar (hydrophobic) amino acids include alanine, leucine, isoleucine, valine, proline, phenylalanine, tryptophan and methionine. The polar neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid.

Within the scope of percentage of sequence identity described above, a polypeptide of the invention may have additional individual amino acids or amino acid sequences inserted into the polypeptide in the middle thereof and/or at the N-terminal and/or C-terminal ends thereof. Likewise, some of the amino acids or amino acid sequences may be deleted from the polypeptide. Amino acid substitutions may also be made in the sequences; conservative substitutions being preferred.

One preferred class of variants are those that comprise (1) the domain of an encoded polypeptide and/or (2) residues conserved between the encoded polypeptide and related polypeptides. For this class of variants, the encoded polypeptide sequence is changed by insertion, deletion, or substitution at positions flanking the domain and/or conserved residues.

Another class of variants includes those that comprise an encoded polypeptide sequence that is changed in the domain or conserved residues by a conservative substitution.

Yet another class of variants includes those that lack one of the *in vitro* activities, or structural features of the

encoded polypeptides. One example is polypeptides or proteins produced from genes comprising dominant negative mutations. Such a variant may comprise an encoded polypeptide sequence with non-conservative changes in a particular domain or group of conserved residues.

II.A.(2) FRAGMENTS

Fragments of particular interest are those that comprise a domain identified for a polypeptide encoded by an MLS of the instant invention and variants thereof. Also, fragments that comprise at least one region of residues conserved between an MLS encoded polypeptide and its related polypeptides are of great interest. Fragments are sometimes useful as polypeptides corresponding to genes comprising dominant negative mutations are.

II.A.(3)FUSIONS

Of interest are chimeras comprising (1) a fragment of the MLS encoded polypeptide or variants thereof of interest and (2) a fragment of a polypeptide comprising the same domain. For example, an AP2 helix encoded by a MLS of the invention fused to second AP2 helix from ANT protein, which comprises two AP2 helices. The present invention also encompasses fusions of MLS encoded polypeptides, variants, or fragments thereof fused with related proteins or fragments thereof.

**DEFINITION OF DOMAINS**

The polypeptides of the invention may possess identifying domains as shown in REF TABLES 1 and 2Specific domains within the MLS encoded polypeptides are indicated by the reference REF TABLES 1 and 2. In addition, the domains within the MLS encoded polypeptide can be defined by the region that exhibits at least 70% sequence identity with the consensus sequences listed in the detailed description below of each of the domains.

The majority of the protein domain descriptions given below are obtained from Prosite,
(http//www.expasy.ch/prosite/), and Pfam,
(http//pfam.wustl.edu/browse.shtml).

1. (AAA) AAA-protein family signature
A large family of ATPases has been described [1 to 5] whose key feature is that they share a conserved region of about 220 amino acids that contains anATP-binding site. This family is now called AAA, for 'A'TPases 'A'ssociated with diverse cellular 'A'ctivities. The proteins that belong to this family either contain one or two AAA domains. Proteins containing two AAA domains:

- Mammalian and drosophila NSF (N-ethylmaleimide-sensitive fusion protein) and the fungal homolog, SEC18. These proteins are involved in intracellular transport between the endoplasmic reticulum and Golgi, as well as between different Golgi cisternae.
- Mammalian transitional endoplasmic reticulum ATPase (previously known as p97 or VCP) which is involved in the transfer of membranes from the endoplasmic reticulum to the golgi apparatus. This protein forms a ring-shaped homooligomer composed of six subunits. The yeast homolog is CDC48 and it may play a role in spindle pole proliferation.
- Yeast protein PAS1, essential for peroxisome assembly and the related protein PAS1 from Pichia pastoris.
- Yeast protein AFG2.
- Sulfolobus acidocaldarius protein SAV and Halobacterium salinarium cdcH which may be part of a transduction pathway connecting light to cell division.

Proteins containing a single AAA domain:

- Escherichia coli and other bacteria ftsH (or hf1B) protein. FtsH is an ATP-dependent zinc metallopeptidase that seems to degrade the heat-shock sigma-32 factor. It is an integral membrane protein with a large cytoplasmic C-terminal domain that contain both the AAA and the protease domains.
- Yeast protein YME1, a protein important for maintaining the integrity of the mitochondrial compartment. YME1 is also a zinc-dependent protease.
- Yeast protein AFG3 (or YTA10). This protein also seems to contain a AAA domain followed by a zinc-dependent protease domain.

Subunits from the regulatory complex of the 26S proteasome [6] which is involved in the ATP-dependent degradation

of ubiquitinated proteins:

a) Mammalian subunit 4 and homologs in other higher eukaryotes, in yeast (gene YTA5) and fission yeast (gene mts2).
b) Mammalian subunit 6 (TBP7) and homologs in other higher eukaryotes and in yeast (gene YTA2).
c) Mammalian subunit 7 (MSS1) and homologs in other higher eukaryotes and in yeast (gene CIM5 or YTA3).
d) Mammalian subunit 8 (P45) and homologs in other higher eukaryotes and in yeast (SUG1 or CIM3 or TBY1) and fission yeast (gene let1).

Other probable subunits such as human TBP1 which seems to influences HIV gene expression by interacting with the virus tat transactivator protein and yeast YTA1 and YTA6.

- Yeast protein BCS1, a mitochondrial protein essential for the expression of the Rieske iron-sulfur protein.
- Yeast protein MSP1, a protein involved in intramitochondrial sorting of proteins.
- Yeast protein PAS8, and the corresponding proteins PAS5 from Pichia pastoris and PAY4 from Yarrowia lipolytica.
- Mouse protein SKD1 and its fission yeast homolog (SpAC2G11.06).
- Caenorhabditis elegans meiotic spindle formation protein mei-1.
- Yeast protein SAP1.
- Yeast protein YTA7.
- Mycobacterium leprae hypothetical protein A2126A.

It is proposed that, in general, the AAA domains in these proteins act as ATP-dependent protein clamps [5]. In addition to the ATP-binding 'A' and 13'motifs, which are located in the N-terminal half of this domain, there is a highly conserved region located in the central part of the domain which was used to develop a signature pattern.
Consensus pattern: [LIVMT]-x-[LIVMT]-[LIVMF]-x-[GATMC]-[ST]-[NS]-x(4)-[LIVM]-D-x-A-[LIFA]-x-R

[1] Froehlich K.-U., Fries H.W., Ruediger M., Erdmann R., Botstein D., Mecke D. J. Cell Biol. 114:443-453(1991).
[2] Erdmann R., Wiebel F.F., Flessau A., Rytka J., Beyer A., Froehlich K.-U., Kunau W.-H. Cell 64:499-510(1991).
[3] Peters J.-M., Walsh M.J., Franke W.W. EMBO J. 9:1757-1767(1990).
[4] Kunau W.-H., Beyer A., Goette K., Marzioch M., Saidowsky J., Skaletz-Rorowski A., Wiebel F.F. Biochimie 75:209-224(1993).
[5] Confalonieri F., Duguet M. BioEssays 17:639-650(1995).
[6] Hilt W., WolfD.H. Trends Biochem. Sci. 21:96-102(1996).

2. ABC Membrane (ABC transporter transmembrane region). This family represents a unit of six transmembrane helices. Many members of the ABC transporter family
(ABC tran)have two such regions. See also descriptions of ABC Tran, below, and ABC2 membrane, above.

3. (ABC Tran)
ABC transporters family signature
On the basis of sequence similarities a family of related ATP-bindingproteins has been characterized [1 to 5]. These proteins are associated with avariety of distinct biological processes in both prokaryotes and eukaryotes, but a majority of them are involved in active transport of small hydrophilic molecules across the cytoplasmic membrane. All these proteins share a conserved domain of some two hundred amino acid residues, which includes an ATP-binding site. These proteins are collectively known as ABC transporters. Proteins known to belong to this family are listed below (references are only provided for recently determined sequences).In prokaryotes: - Active transport systems components: alkylphosphonate uptake(phnC/phnK/ phnL); arabinose (araG); arginine (artP); dipeptide (dciAD;dppD/dppF); ferric enterobactin (fepC); ferrichrome (fhuC); galactoside (mglA); glutamine (glnQ); glycerol-3-phosphate (ugpC); glycine betaine/L-proline (proV); glutamate/aspatate (gltL); histidine (hisP); iron(III) (sfuC), iron (III) dicitrate (fecE); lactose (lacK); leucine/isoleucine/valine (braF/braG;livF/livG); maltose (malK); molybdenum (modC); nickel (nikD/ nikE); oligopeptide (amiE/amiF;oppD/oppF); peptide (sapD/sapF); phosphate (pstB); putrescine (potG); ribose (rbsA); spermidine/putrescine (potA); sulfate (cysA); vitamin B 12 (btuD). - Hemolysin/leukotoxin export proteins hlyB, cyaB and IktB. - Colicin V export protein cvaB. - Lactococcin export protein lcnC [6]. - Lantibiotic transport proteins nisT (nisin) and spaT (subtilin). - Extracellular proteases B and C export protein prtD. - Alkaline protease secretion protein aprD. - Beta-(1,2)-glucan export proteins chvA and ndvA. - Haemophilus influenzae capsule-polysaccharide export protein bexA. - Cytochrome c biogenesis proteins ccmA (also known as cycV and helA). - Polysialic acid transport protein kpsT. - Cell division associated ftsE protein (function unknown). - Copper

processing protein nosF from Pseudomonas stutzeri. - Nodulation protein nodi from Rhizobium (function unknown). - Escherichia coli proteins cydC and cydD. - Subunit A of the ABC excision nuclease (gene uvrA). - Erythromycin resistance protein from Staphylococcus epidermidis (gene msrA). - Tylosin resistance protein from Streptomyces fradiae (gene tlrC) [7]. - Heterocyst differentiation protein (gene hetA) from Anabaena PCC 7120. - Protein P29 from Mycoplasma hyorhinis, a probable component of a high affinity transport system. - yhbG, a putative protein whose gene is linked with ntrA in many bacteria such as Escherichia coli, Klebsiella pneumoniae, Pseudomonas putida, Rhizobium meliloti and Thiobacillus ferrooxidans. - Escherichia coli and related bacteria hypothetical proteins yabJ, yadG, yagC, ybbA, ycjW, yddA, yehX, yejF, yhes, yhiG, yhiH, yjcW, yjjK, yojI, yrbF and ytfR.In eukaryotes: - The multidrug transporters (Mdr) (P-glycoprotein), a family of closely related proteins which extrude a wide variety of drugs out of the cell (for a review see [8]). - Cystic fibrosis transmembrane conductance regulator (CFTR), which is most probably involved in the transport of chloride ions. - Antigen peptide transporters 1 (TAP1, PSF1, RING4, HAM-1, mtp1) and 2 (TAP2, PSF2, RING11, HAM-2, mtp2), which are involved in the transport of antigens from the cytoplasm to a membrane-bound compartment for association with MHC class I molecules. - 70 Kd peroxisomal membrane protein (PMP70). - ALDP, a peroxisomal protein involved in X-linked adrenoleukodystrophy [9]. - Sulfonylurea receptor [10], a putative subunit of the B-cell ATP-sensitive potassium channel. - Drosophila proteins white (w) and brown (bw), which are involved in the import of ommatidium screening pigments. - Fungal elongation factor 3 (EF-3). - Yeast STE6 which is responsible for the export of the a-factor pheromone. - Yeast mitochondrial transporter ATM1. - Yeast MDL1 and MDL2. - Yeast SNQ2. - Yeast sporidesmin resistance protein (gene PDR5 or STS1 or YDR1). - Fission yeast heavy metal tolerance protein hmt1 . This protein is probably involved in the transport of metal-bound phytochelatins. - Fission yeast brefeldin A resistance protein (gene bfr1 or hba2). - Fission yeast leptomycin B resistance protein (gene pmd1). - mbpX, a hypothetical chloroplast protein from Liverwort. - Prestalk-specific protein tagB from slime mold. This protein consists of two domains: a N-terminal subtilase catalytic domain and a C-terminal ABC transporter domain.As a signature pattern for this class of proteins, a conserved region which is located between the 'A' and the 'B' motifs of the ATP-binding site was used.
Consensus pattern: [LIVMFYC]-[SA]-[SAPGLVFYKQH]-G-[DENQMW]-[KRQASPCLIMFW]-[KRNQSTAVM]-[KRACLVM]-[LIVMFYPAN]-{PHY}-[LIVMFW]- [SAGCLIVP]-{FYWHP}-{KRHP}-[LIVMFYWSTA] The ATP-binding region is duplicated in araG, mdl, msrA, rbsA, tlrC, uvrA, yejF, Mdr's, CFTR, pmd1 and in EF-3. In some of those proteins, the above pattern only detect one of the two copies of the domain. The proteins belonging to this family also contain one or two copies of the ATP-binding motifs 'A'and 'B'.


[1] Higgins C.F., Hyde S.C., Mimmack M.M., Gileadi U., Gill D.R., Gallagher M.P. J. Bioenerg. Biomembr. 22: 571-592(1990).

[2] Higgins C.F., Gallagher M.P., Mimmack M.M., Pearce S.R. BioEssays 8:111-116(1988).

[3] Higgins C.F., Hiles I.D., Salmond G.P.C., Gill D.R., Downie J.A., Evans I.J., Holland I.B., Gray L., Buckels S.D., Bell A.W., Hermodson M.A. Nature 323:448-450(1986).

[4] Doolittle R.F., Johnson M.S., Husain I., van Houten B., Thomas D.C., Sancar A. Nature 323:451-453(1986).

[5] Blight M.A., Holland I.B. Mol. Microbiol. 4:873-880(1990).

[6] Stoddard G.W., Petzel J.P., van Belkum M.J., Kok J., McKay L.L. Appl. Environ. Microbiol. 58:1952-1961 (1992).

[7] Rosteck P.R. Jr., Reynolds P.A., Hershberger C.L. Gene 102:27-32(1991).

[8] Gottesman M.M., Pastan I. J. Biol. Chem. 263:12163-12166(1988).

[9] Valle D., Gaertner J. Nature 361:682-683(1993).

[10] Aguilar-Bryan L., Nichols C.G., Wechsler S.W., Clement J.P. IV, Boyd A.E. III, Gonzalez G., Herrera-Sosa H., Nguy K., Bryan J., Nelson D.A. Science 268:423-426(1995).


4. (ACBP)
Acyl-CoA-binding protein signature
Acyl-CoA-binding protein (ACBP) is a small (10 Kd) protein that binds medium- and longchain acyl-CoA esters with very high affinity and may function as an intracellular carrier of acyl-CoA esters [1]. ACBP is also known as diazepam binding inhibitor (DBI) or endozepine (EP) because of its ability to displace diazepam from the benzodiazepine (BZD) recognition site located on the GABA type A receptor. It is therefore possible that this protein also acts as a neuropeptide to modulate the action of the GABA receptor [2].ACBP is a highly conserved protein of about 90 residues that has been so far found in vertebrates, insects and yeast. ACBP is also related to the N-terminal section of a probable transmembrane protein of unknown function which has been found in mammals. As a signature pattern, the region that corresponds to residues 19 to 37 in mammalian ACBP was selected.
Consensus pattern: P-[STA]-x-[DEN]-x-[LIVMF]-x(2)-[LIVMFY]-Y-[GSTA]-x-[FY]-K-Q-[STA](2)-x-G-


[1] Rose T.M., Schultz E.R., Todaro G.J. Proc. Natl. Acad. Sci. U.S.A. 89:11287-11291(1992).

[2] Costa E., Guidotti A. Life Sci. 49:325-344(1991).

5. (AIRS)

AIR synthase related proteins

This family includes Hydrogen expression/formation protein HypE, AIR synthases, FGAM synthase and selenide, water dikinase.

6. (AMP-binding)

Putative AMP-binding domain signature

It has been shown [1 to 5] that a number of prokaryotic and eukaryotic enzymes which all probably act via an ATP-dependent covalent binding of AMP to their substrate, share a region of sequence similarity. These enzymes are: - Insects luciferase (luciferin 4-monooxygenase). Luciferase produces light by catalyzing the oxidation of luciferin in presence of ATP and molecular oxygen. - Alpha-aminoadipate reductase from yeast (gene LYS2). This enzyme catalyzes the activation of alpha-aminoadipate by ATP-dependent adenylation and the reduction of activated alpha-aminoadipate by NADPH. - Acetate--CoA ligase (acetyl-CoA synthetase), an enzyme that catalyzes the formation of acetyl-CoA from acetate and CoA. - Long-chain-fatty-acid--CoA ligase, an enzyme that activates long-chain fatty acids for both the synthesis of cellular lipids and their degradation via beta-oxidation. - 4-coumarate--CoA ligase (4CL), a plant enzyme that catalyzes the formation of 4-coumarate-CoA from 4-coumarate and coenzyme A; the branchpoint reactions between general phenylpropanoid metabolism and pathways leading to various specific end products. - O-succinylbenzoic acid--CoA ligase (OSB-CoA synthetase) (gene menE) [6], a bacterial enzyme involved in the biosynthesis of menaquinone (vitamin K2). - 4-Chlorobenzoate-CoA ligase (EC 6.2.1.-) (4-CBA--CoA ligase) [7], a Pseudomonas enzyme involved in the degradation of 4-CBA. - Indoleacetate--lysine ligase (IAA-lysine synthetase) [8], an enzyme from Pseudomonas syringae that converts indoleacetate to IAA-lysine. - Bile acid-CoA ligase (gene baiB) from Eubacterium strain VPI 12708 [4]. This enzyme catalyzes the ATP-dependent formation of a variety of C-24 bile acid-CoA. - Crotonobetaine/carnitine-CoA ligase (EC 6.3.2.-) from Escherichia coli (gene caiC). - L-(alpha-aminoadipyl)-L-cysteinyl-D-valine synthetase (ACV synthetase) from various fungi (gene acvA or pcbAB). This enzyme catalyzes the first step in the biosynthesis of penicillin and cephalosporin, the formation of ACV from the constituent amino acids. The amino acids seem to be activated by adenylation. It is a protein of around 3700 amino acids that contains three related domains of about 1000 amino acids. - Gramicidin S synthetase I (gene grsA) from Bacillus brevis. This enzyme catalyzes the first step in the biosynthesis of the cyclic antibiotic gramicidin S, the ATP-dependent racemization of phenylalanine - Tyrocidine synthetase I (gene tycA) from Bacillus brevis. The reaction carried out by tycA is identical to that catalyzed by grsA - Gramicidin S synthetase II (gene grsB) from Bacillus brevis. This enzyme is a multifunctional protein that activates and polymerizes proline, valine, ornithine and leucine. GrsB consists of four related domains. - Enterobactin synthetase components E (gene entE) and F (gene entF) from Escherichia coli. These two enzymes are involved in the ATP-dependent activation of respectively 2,3-dihydroxybenzoate and serine during enterobactin (enterochelin) biosynthesis. - Cyclic peptide antibiotic surfactin synthase subunits 1, 2 and 3 from Bacillus subtilis. Subunits 1 and 2 contains three related domains while subunit 3 only contains a single domain. - HC-toxin synthetase (gene HTS1) from Cochliobolus carbonum. This enzyme activates the four amino acids (Pro, L-Ala, D-Ala and 2-amino-9,10-epoxi-8- oxodecanoic acid) that make up HC-toxin, a cyclic tetrapeptide. HTS 1 consists of four related domains.There are also some proteins, whose exact function is not yet known, but whichare, very probably, also AMP-binding enzymes. These proteins are: - ORA (octapeptide-repeat antigen), a Plasmodium falciparum protein whose function is not known but which shows a high degree of similarity with the above proteins. - AngR, a Vibrio anguillarum protein. AngR is thought to be a transcriptional activator which modulates the anguibactin (an iron-binding siderophore) biosynthesis gene cluster operon. But it is believed [9], that angR is not a DNA-binding protein, but rather an enzyme involved in the biosynthesis of anguibactin. This conclusion is based on three facts: the presence of the AMP-binding domain; the size of angR (1048 residues), which is far bigger than any bacterial transcriptional protein; and the presence of a probable S-acyl thioesterase immediately downstream of angR. - A hypothetical protein in mmsB 3'region in Pseudomonas aeruginosa. - Escherichia coli hypothetical protein ydiD. - Yeast hypothetical protein YBR041w. - Yeast hypothetical protein YBR222c. - Yeast hypothetical protein YER147c.A11 these proteins contain a highly conserved region very rich in glycine, serine, and threonine which is followed by a conserved lysine. A parallel can be drawn between this type of domain and the G-x(4)-G-K-[ST] ATP-/GTP-binding T-loop' domain or the protein kinases G-x-G-x(2)-[SG]-x (10,20)-KATP-binding domains.

Consensus pattern: [LIVMFY]-x(2)-[STG]-[STAG]-G-[ST]-[STEI]-[SG]-x-[PASLIVM]-[KR] In a majority of cases the residue that follows the Lys at the end of the pattern is a Gly.

[1] Toh H. Protein Seq. Data Anal. 4:111-117(1991).
[2] Smith D.J., Earl A.J., Turner G. EMBO J. 9:2743-2750(1990).

[3] Schroeder J. Nucleic Acids Res. 17:460-460(1989).
[4] Mallonee D.H., Adams J.L., Hylemon P.B. J. Bacteriol. 174:2065-2071(1992).
[5] Turgay K., Krause M., Marahiel M.A. Mol. Microbiol. 6:529-546(1992).
[6] Driscoll J.R., Taber H.W. J. Bacteriol. 174:5063-5071(1992).
[7] Babbitt P.C., Kenyon G.L., Matin B.M., Charest H., Sylvestre M., Scholten J.D., Chang K.-H., Liang P.-H., Dunaway-Mariano D. Biochemistry 31:5594-5604(1992).
[8] Farrell D.H., Mikesell P., Actis L.A., Crosa J.H. Gene 86:45-51(1990).

7. AP2 domain

This 60 amino acid residue domain can bind to DNA [1]. This domain is plant specific. Members of this family are suggested to be related to pyridoxal phosphate-binding domains such as found in aminotran 2 [3]. AP2 domains are also described in Jofuku et al., copending U.S. Patent applications 08/700,152, 08/879,827, 08/912,272, 09/026,039.

[1] Ohme-takagi M, Shinshi H; Plant Cell 1995;7:173-182.
[2] Weigel D; Plant Cell 1995;7:388-389.
[3] Mushegian AR, Koonin EV; Genetics 1996;144:817-828.

8. ARID

The ARID domain is an AT-Rich Interaction domain sharing structural homology to DNA replication and repair nucleases and polymerases.

[1] Herrscher RF, Kaplan MH, Lelsz DL, Das C, Scheuermann R, Tucker PW; Genes Dev 1995;9:3067-3082.
[2] Yuan YC, Whitson RH, Liu Q, Itakura K, Chen Y; Nat Struct Biol 1998;5:959-964.

9. (ATP synt)

ATP synthase gamma subunit signature

ATP synthase (proton-translocating ATPase) (EC 3.6.1.34) [1,2] is a componentof the cytoplasmic membrane of eubacteria, the inner membrane of mitochondria, and the thylakoid membrane of chloroplasts. The ATPase complex is composed of an oligomeric transmembrane sector, called CF(0), and a catalytic core, called coupling factor CF (1). The former acts as a proton channel; the latter is composed of five subunits, alpha, beta, gamma, delta and epsilon. Subunit gamma is believed to be important in regulating ATPase activity and the flow of protons through the CF(0) complex. The best conserved region of the gamma subunit [3] is its C-terminus which seems to be essential for assembly and catalysis. As a signature pattern to detect ATPase gamma subunits, a14 residue conserved segment where the last amino acid is found one to three residues from the C-terminal extremity was used.

Consensus pattern: [IV]-T-x-E-x(2)-[DE]-x(3)-G-A-x-[SAKR]- Note: Pea chloroplast gamma and two Bacillus species gamma subunits are not detected by this motif.

[1] Futai M., Noumi T., Maeda M. Annu. Rev. Biochem. 58:111-136(1989).
[2] Senior A.E. Physiol. Rev. 68:177-231(1988).
[3] Miki J., Maeda M., Mukohata Y., Futai M. FEBS Lett. 232:221-226(1988).

10. (ATP Synt A)

Synthase a subunit signature

ATP synthase (proton-translocating ATPase) (EC 3.6.1.34) [1,2] is a component of the cytoplasmic membrane of eubacteria, the inner membrane of mitochondria,and the thylakoid membrane of chloroplasts. The ATPase complex is composed of an oligomeric transmembrane sector, called CF(0), which acts as a proton channel, and a catalytic core, termed coupling factor CF(1).The CF(0) a subunit, also called protein 6, is a key component of the proton channel; it may play a direct role in translocating protons across the membrane. It is a highly hydrophobic protein that has been predicted to contain 8 transmembrane regions [3].Sequence comparison of a subunits from all available sources reveals very few conserved regions. The best conserved region is located in what is predicted to be the fifth transmembrane domain. This region contains three perfectly conserved residues: an arginine, a leucine and an asparagine. Mutagenesis experiments of ATPase activity. This region was selected as a signature pattern.

Consensus pattern: [STAGN]-x-[STAG]-[LIVMF]-R-L-x-[SAGV]-N-[LIVMT] [R is important for proton translocation]

[1] Futai M., Noumi T., Maeda M. Annu. Rev. Biochem. 58:111-136(1989).
[2] Senior A.E. Physiol. Rev. 68:177-231(1988).
[3] Lewis M.L., Chang J.A., Simoni R.D. J. Biol. Chem. 265:10541-10550(1990).

[4] Cain B.D., Simoni R.D. J. Biol. Chem. 264:3292-3300(1989).

11. ATP synthase B

Part of the CF(0) (base unit) of the ATP synthase. The base unit is thought to translocate protons through membrane (inner membrane in mitochondria, thylakoid membrane in plants, cytoplasmic membrane in bacteria). The B subunits are thought to interact with the stalk of the CF(1) subunits.

12. (ATP synt C)

ATP synthase c subunit signature

ATP synthase (proton-translocating ATPase) [1,2] is a component of the cytoplasmic membrane of eubacteria, the inner membrane of mitochondria,and the thylakoid membrane of chloroplasts. The ATPase complex is composed of an oligomeric transmembrane sector, called CF(0), which acts as a proton channel, and a catalytic core, termed coupling factor CF(1).The CF(0) c subunit (also called protein 9, proteolipid, or subunit III) [3,4] is a highly hydrophobic protein of about 8 Kd which has been implicated in the proton-conducting activity of ATPase. Structurally subunit c consist of two long terminal hydrophobic regions, which probably span the membrane, and a central hydrophilic region. N,N'-dicyclohexylcarbodiimide (DCCD) can bind covalently to subunit c and thereby abolish the ATPase activity. DCCD binds to a specific glutamate or aspartate residue which is located in the middle ofthe second hydrophobic region near the C-terminus of the protein. A signature pattern which includes the DCCD-binding residue was derived.

Consensus pattern: [GSTA]-R-[NQ]-P-x(10)-[LIVMFYW](2)-x(3)-[LIVMFYW]-x-[DE] [D or E binds DCCD]

[1] Futai M., Noumi T., Maeda M. Annu. Rev. Biochem. 58:111-136(1989).
[2] Senior A.E. Physiol. Rev. 68:177-231(1988).
[3] Ivaschenko A.T., Karpenyuk T.A., Ponomarenko S.V. Biokhimiia 56:406-419(1991).
[4] Recipon H., Perasso R., Adoutte A., Quetier F. J. Mol. Evol. 34:292-303(1992).

13. (ATP synt DE)

ATP synthase, Delta/Epsilon chain

Part of the ATP synthase CF(1). These subunits are part of the head unit of the ATP synthase. The subunits are called delta and epsilon in human and metozoan species but in bacterial species the delta (D) subunit is theequivalent to the Oligomycin sensitive subunit (OSCP) in metozoans.

14. (ATP synt ab)

ATP synthase alpha and beta subunits signature

ATP synthase (proton-translocating ATPase) [1,2] is a component of the cytoplasmic membrane of eubacteria, the inner membrane of mitochondria,and the thylakoid membrane of chloroplasts. The ATPase complex is composed of an oligomeric transmembrane sector, called CF(0), and a catalytic core, called coupling factor CF(1). The former acts as a proton channel; the latter is composed of five subunits, alpha, beta, gamma, delta and epsilon. The sequences of subunits alpha and beta are related and both contain a nucleotide-binding site for ATP and ADP. The beta chain has catalytic activity, while the alpha chain is a regulatory subunit. Vacuolar ATPases [3] (V-ATPases) are responsible for acidifying a variety of intracellular compartments in eukaryotic cells. Like F-ATPases, they are oligomeric complexes of a transmembrane and a catalytic sector. The sequenceof the largest subunit of the catalytic sector (70 Kd) is related to that ofF-ATPase beta subunit, while a 60 Kd subunit, from the same sector, is related to the F-ATPases alpha subunit [4].Archaebacterial membrane-associated ATPases are composed of three subunits.The alpha chain is related to F-ATPases beta chain and the beta chain is related to F-ATPases alpha chain [4].A protein highly similar to F-ATPase beta subunits is found [5] in some bacterial apparatus involved in a specialized protein export pathway that proceeds without signal peptide cleavage. This protein is known as fliI in Bacillus and Salmonella, Spa47 (mxiB) in Shigella flexneri, HrpB6 in Xanthomonas campestris and yscN in Yersinia virulence plasmids.To detect these ATPase subunits, a segment of ten amino-acid residues, containing two conserved serines, as a signature pattern was selected. The first serine seems to be important for catalysis - in the ATPase alpha chain at least - as its mutagenesis causes catalytic impairment.

Consensus pattern: P-[SAP]-[LIV]-[DNH]-x(3)-S-x-S [The first S is a putative active site residue]

[1] Futai M., Noumi T., Maeda M. Annu. Rev. Biochem. 58:111-136(1989).
[2] Senior A.E. Physiol. Rev. 68:177-231(1988).
[3] Nelson N. J. Bioenerg. Biomembr. 21:553-571(1989).
[4] Gogarten J.P., Kibak H., Dittrich P., Taiz L., Bowman E.J., Bowman B.J., Manolson M.F., Poole R.J., Date T., Oshima T., Konishi J., Denda K., Yoshida M. Proc. Natl. Acad. Sci. U.S.A. 86:6661-665(1989).

[5] Dreyfus G., Williams A.W., Kawagishi I., MacNab R.M. J. Bacteriol. 175:3131-3138(1993).

15. (ATP synt ab C)
ATP synthase ab C terminal.
Number of members: 190

[1] Abrahams JP, Leslie AG, Lutter R, Walker JE; "Structure at 2.8 A resolution of F1-ATPase from bovine heart mitochondria." Nature 1994;370:621-628.

16. (A deaminase)
Adenosine and AMP deaminase signature
Adenosine deaminase catalyzes the hydrolytic deamination ofadenosine into inosine. AMP deaminase catalyzes the hydrolytic deamination of AMP into IMP. It has been shown [1] that these two types of enzymes share three regions of sequence similarities; these regions are centered on residues which are proposed to play an important role in the catalytic mechanism of these two enzymes. One of these regions, containing two conserved aspartic acid residues that are potential active site residues was selected.
Consensus pattern: [SA]-[LIVM]-[NGS]-[STA]-D-D-P [The two D's are putative active site residues]

[1] Chang Z., Nygaard P., Chinault A.C., Kellems R.E. Biochemistry 30:2273-2280(1991).

17. (Acetyltransf)
Acetyltransferase (GNAT) family.
This family contains proteins with N-acetyltransferase functions.

[1] Neuwald AF, Landsman D; Trends Biochem Sci 1997;22:154-155.

18. (Aconitase C)
Aconitase family signature
Aconitase (aconitate hydratase) (EC 4.2.1.3) [1] is the enzyme from the tricarboxylic acid cycle that catalyzes the reversible isomerization of citrate and isocitrate. Cis-aconitate is formed as an intermediary product during the course of the reaction. In eukaryotes two isozymes of aconitase are known to exist: one found in the mitochondrial matrix and the other found in the cytoplasm. Aconitase, in its active form, contains a 4Fe-4S iron-sulfur cluster; three cysteine residues have been shown to be ligands of the 4Fe-4S cluster.It has been shown that the aconitase family also contains the followingproteins: - Iron-responsive element binding protein (IRE-BP). IRE-BP is a cytosolic protein that binds to iron-responsive elements (IREs). IREs are stem-loop structures found in the 5'UTR of ferritin, and delta aminolevulinic acid synthase mRNAs, and in the 3'UTR of transferrin receptor mRNA. IRE-BP also express aconitase activity. - 3-isopropylmalate dehydratase (EC 4.2.1.33) (isopropylmalate isomerase), the enzyme that catalyzes the second step in the biosynthesis of leucine. - Homoaconitase (EC 4.2.1.36) (homoaconitate hydratase), an enzyme that participates in the alpha-aminoadipate pathway of lysine biosynthesis and that converts cis-homoaconitate into homoisocitric acid. - Esherichia coli protein ybhJ.As a signature for proteins from the aconitase family, two conserved regions that contain the three cysteine ligands of the 4Fe-4Scluster were selected.
Consensus pattern: [LIVM]-x(2)-[GSACIVM]-x-[LIV]-[GTIV]-[STP]-C-x(0,1)-T-N-[GSTANI]-x(4)-[LIVMA] [C binds the iron-sulfur center]
Consensus pattern: G-x(2)-[LIVWPQ]-x(3)-[GAC]-C-[GSTAM]-[LIMPTA]-C-[LIMV]-[GA] [The two C's bind the iron-sulfur center]

[1] Gruer M.J., Artymiuk P.J., Guest J.R. Trends Biochem. Sci. 22:3-6(1997).

19. (Acyl-CoA dh)
Acyl-CoA dehydrogenases signatures
Acyl-CoA dehydrogenases [1,2,3] are enzymes that catalyze the alpha, betadehydrogenation of acyl-CoA esters and transfer electrons to ETF, the electron transfer protein. Acyl-CoA dehydrogenases are FAD flavoproteins. This family currently includes: - Five eukaryotic isozymes that catalyze the first step of the beta-oxidation cycles for fatty acids with various chain lengths. These are short (SCAD) (EC 1.3.99.2), medium (MCAD) (EC 1.3.99.3), long (LCAD) (EC 1.3.99.13), very-long (VLCAD) and short/branched (SBCAD) chain acyl-CoA dehydrogenases. These enzymes are located in the mitochondrion. They are all homotetrameric proteins of about 400 amino acid residues except VLCAD which is a dimer and which contains, in its mature form, about 600 residues. - Glutaryl-CoA dehydrogenase (EC 1.3.99.7) (GCDH), which is involved in the catabolism of lysine, hydroxylysine and tryptophan. - Isovaleryl-CoA

dehydrogenase (EC 1.3.99.10) (IVD), involved in the catabolism of leucine. - Acyl-coA dehydrogenases acsA and mmgC from Bacillus subtilis. - Butyryl-CoA dehydrogenase (EC 1.3.99.2) from Clostridium acetobutylicum. - Escherichia coli protein caiA [4]. - Escherichia coli protein aidB. Two conserved regions were selected as signature patterns. The first is located in the center of these enzymes, the second in the C-terminal section.
Consensus pattern: [GAC]-[LIVM]-[ST]-E-x(2)-[GSAN]-G-[ST]-D-x(2)-[GSA]
Consensus pattern: [QDE]-x(2)-G-[GS]-x-G-[LIVMFY]-x(2)-[DEN]-x(4)-[KR]-x(3)-[DEN]

[1] Tanaka K., Ikeda, Matsubara Y., Hyman D.B. Enzyme 38:91-107(1987).
[2] Matsubara Y., Indo Y., Naito E., Ozasa H., Glassberg R., Vockley J., Ikeda Y., Kraus J., Tanaka K. J. Biol. Chem. 264:16321-16331(1989).
[3] Aoyama T., Ueno L, Kamijo T., Hashimoto T. J. Biol. Chem. 269:19088-19094(1994).
[4] Eichler K., Bourgis F., Buchet A., Kleber H.-P., Mandrand-Berthelot M.-A. Mol. Microbiol. 13:775-786(1994).

20. (Acyl transf)
Acyl transferase domain
Number of members: 161

[1] Serre L, Verbree EC, Dauter Z, Stuitje AR, Derewenda ZS; Medline: 95286570 "The Escherichia coli malonyl-CoA:acyl carrier protein transacylase at 1.5-A resolution. Crystal structure of a fatty acid synthase component." J Biol Chem 1995;270:12961-12964.

21. Acylphosphatase signatures
Acylphosphatase (EC 3.6.1.7) [1,2] catalyzes the hydrolysis of various acylphosphate carboxyl-phosphate bonds such as carbamyl phosphate, succinylphosphate, 1,3-diphosphoglycerate, etc. The physiological role of this enzymeis not yet clear. Acylphosphatase is a small protein of around 100 amino-acid residues. There are two known isozymes. One seems to be specific to muscular tissues, the other, called 'organcommon type', is found in many different tissues.While acylphosphatase have been so far only characterized in vertebrates,there are a number of bacterial and archebacterial hypothetical proteins that are highly similar to that enzyme and that probably possess the same activity.These proteins are: - Escherichia coli hypothetical protein yccX. - Bacillus subtilis hypothetical protein yflL. - Archaeoglobus fulgidus hypothetical protein AF0818. Two conserved regions were selected as signature patterns. The first is located in the N-terminal section, while the second is found in the central part ofthe protein sequence.
Consensus pattern: [LIV]-x-G-x-V-Q-G-V-x-[FM]-R
Consensus pattern: G-[FYW]-[AVC]-[KRQAM]-N-x(3)-G-x-V-x(5)-G

[1] Stefani M., Ramponi G. Life Chem. Rep. 12:271-301(1995).
[2] Stefani M., Taddei N., Ramponi G. Cell. Mol. Life Sci. 53:141-151(1997).

22. (Adap comp sub)
Clathrin adaptor complexes medium chain signatures.
Clathrin coated vesicles (CCV) mediate intracellular membrane traffic such asreceptor mediated endocytosis. In addition to clathrin, the CCV are composed of a number of other components including oligomeric complexes which are knownas adaptor or clathrin assembly proteins (AP) complexes [1]. The adaptor complexes are believed to interact with the cytoplasmic tails of membrane proteins, leading to their selection and concentration. In mammals two type of adaptor complexes are known: AP-1 which is associated with the Golgi complex and AP-2 which is associated with the plasma membrane. Both AP-1 and AP-2 are heterotetramers that consist of two large chains - the adaptins - (gamma and beta' in AP-1; alpha and beta in AP-2); a medium chain (AP47 in AP-1; AP50 inAP-2) and a small chain (AP19 in AP-1; AP17 in AP-2). The medium chains of AP-1 and AP-2 are evolutionary related proteins of about 50 Kd. Homologs of AP47 and AP50 have also been found in Caenorhabditis elegans (genes unc-101 and ap50) [2] and yeast (gene APM1 or YAP54) [3].Some more divergent, but clearly evolutionary related proteins have also been found in yeast: APM2 and YBR288c., Two conserved regions were selected as signature patterns, one located in the N-terminal region, the other from the central section of these proteins.
Consensus pattern: [IVT]-[GSP]-W-R-x(2,3)-[GAD]-x(2)-[HY]-x(2)-N-x- [LIVMAFY](3)-D-[LIVM]-[LIVMT]-E
Consensus pattern: [LIV]-x-F-I-P-P-x-G-x-[LIVMFY]-x-L-x(2)-Y

[1] Pearse B.M., Robinson M.S. Annu. Rev. Cell Biol. 6:151-171(1990).
[2] Lee J., Jongeward G.D., Sternberg P.W. Genes Dev. 8:60-73(1994).
[3] Nakayama Y., Goebl M., O'Brine G.B., Lemmon S., Pingchang C.E., Kirchhausen T. Eur. J. Biochem. 202:

569-574(1991).

23. (Adenylsucc synt)
Adenylosuccinate synthetase signatures
Adenylosuccinate synthetase (EC 6.3.4.4) [1] plays an important role in purinebiosynthesis, by catalyzing the GTP-dependent conversion of IMP and aspartic acid to AMP. Adenylosuccinate synthetase has been characterized from various sources ranging from Escherichia coli (gene purA) to vertebrate tissues. Invertebrates, two isozymes are present - one involved in purine biosynthesis and the other in the purine nucleotide cycle. Two conserved regions were selected as signature patterns. The first one is a perfectly conserved octapeptide located in the N-terminal section and which is involved in GTP-binding [2]. The second one includes a lysine residue known [2] to be essential for the enzyme's activity.
Consensus pattern: Q-W-G-D-E-G-K-G
Consensus pattern: G-I-[GR]-P-x-Y-x(2)-K-x(2)-R [K is the active site residue]

[1] Wiesmueller L., Wittbrodt J., Noegel A.A., Schleicher M. J. Biol. Chem. 266:2480-2485(1991).
[2] Silva M.M., Poland B.W., Hoffman C.R., Fromm H.J., Honzatko R.B. J. Mol. Biol. 254:431-446(1995).
[3] Bouyoub A., Barbier G., Forterre P., Labedan B. 2.3.CO;2-"J. Mol. Biol. 261:144-154(1996).

24. (AdoHcyase)
S-adenosyl-L-homocysteine hydrolase signatures
S-adenosyl-L-homocysteine hydrolase (EC 3.3.1.1) (AdoHcyase) is an enzyme of the activated methyl cycle, responsible for the reversible hydratation of S-adenosyl-L-homocysteine into adenosine and homocysteine. AdoHcyase is anubiquitous enzyme which binds and requires NAD+ as a cofactor. AdoHcyase is a highly conserved protein [1] of about 430 to 470 amino acids. Two highly conserved regions were selected as signature patterns. The first pattern is located in the N-terminal section; the second is derived from aglycine-rich region in the central part of AdoHcyase; a region thought to be involved in NAD-binding.
Consensus pattern: [GSA]-[CS]-N-x-[FYLM]-S-[ST]-[QA]-[DEN]-x-[AV]-[AT]-[AD]-[AC]-[LIVMCG]
Consensus pattern: [GA]-[KS]-x(3)-[LIV]-x-G-[FY]-G-x-[VC]-G-[KRL]-G-x-[ASC]

[1] Sganga M.W., Aksamit R.R., Cantoni G.L., Bauer C.E. Proc. Natl. Acad. Sci. U.S.A. 89:6328-6332(1992).

25. AhpC/TSA family
This family contains proteins related to alkyl hydroperoxide reductaseComment: (AhpC) and thiol specific antioxidant (TSA).

[1] Chae HZ, Robison K, Poole LB, Church G, Storz G, Rhee SG, Proc Natl Acad Sci U S A 1994;91:7017-7021

26. (Aldose epim)
Aldose 1-epimerase putative active site Aldose 1-epimerase (EC 5.1.3.3) (mutarotase) is the enzyme responsible for the anomeric interconversion of D-glucose and other aldoses between their alpha- and beta-forms. The sequence of mutarotase from two bacteria, Acinetobacter calcoaceticus and Streptococcus thermophilus is available [1]. It has also been shown that, on the basis of extensive sequence similarities, a mutarotase domain seem to be present in the C-terminal half of the fungal GAL10 protein which encodes, in the N-terminal part, for UDP-glucose 4-epimerase. The best conserved region in the sequence of mutarotase is centered around a conserved histidine residue which may be involved in the catalytic mechanism.
Consensus pattern: [NS]-x-T-N-H-x-Y-[FW]-N-[LI]

[1] Poolman B., Royer T.J., Mainzer S.E., Schmidt B.F. J. Bacteriol. 172:4037-4047(1990).

27. (AlkA DNA repair)
Alkylbase DNA glycosidases alkA family signature
Alkylbase DNA glycosidases [1] are DNA repair enzymes that hydrolyzes the deoxyribose N-glycosidic bond to excise various alkylated bases from a damaged DNA polymer. In Escherichia coli there are two alkylbase DNA glycosidases: one (gene tag)which is constitutively expressed and which is specific for the removal of 3-methyladenine (EC 3.2.2.20), and one (gene alkA) which is induced during adaptation to alkylation and which can remove a variety of alkylation products (EC 3.2.2.21). Tag and alkA do not share any region of sequence similarity. In yeast there is an alkylbase DNA glycosidase (gene MAGI) [2,3], which can remove 3-methyladenine or 7-methyladenine and which is structurally related to alkA. MAG and alkA are both proteins of about 300 amino acid residues. While

the C- and N-terminal ends appear to be unrelated, there is a central region of about 130 residues which is well conserved. A portion of this region has been selected as a signature pattern.
Consensus pattern: G-I-G-x-W-[ST]-[AV]-x-[LIVMFY](2)-x-[LIVM]-x(8)-[MF]-x(2)-[ED]-D

[1] Lindahl T., Sedgwick B. Annu. Rev. Biochem. 57:133-157(1988).
[2] Berdal K.G., Bjoras M., Bjelland S., Seeberg E.C. EMBO J. 9:4563-4568(1990).
[3] Chen J., Derfler B., Samson L. EMBO J. 9:4569-4575(1990).

28. Ammonium transporters signature
A number of proteins involved in the transport of ammonium ions across amembrane as well as some yet uncharacterized proteins have been shown [1,2] to be evolutionary related. These proteins are: - Yeast ammonium transporters MEP1, MEP2 and MEP3. - Arabidopsis thaliana high affinity ammonium transporter (gene AMT1). - Corynebacterium glutamicum ammonium and methylammonium transport system. - Escherichia coli putative ammonium transporter amtB. - Bacillus subtilis nrgA. - Mycobacterium tuberculosis hypothetical protein MtCY338.09c. - Synechocystis strain PCC 6803 hypothetical proteins s110108, s110537 and s111017. - Methanococcus jannaschii hypothetical proteins MJ0058 and MJ1343. - Caenorhabditis elegans hypothetical proteins C05E11.4, F49E11.3 and M195.3. As expected by their transport function, these proteins are highly hydrophobic and seem to contain from 10 to 12 transmembrane domains. The best conserved region seems to be located in the fifth (or sixth) transmembrane region and is used as a signature pattern.
Consensus pattern: D-[FYWS]-A-G-[GSC]-x(2)-[IV]-x(3)-[SAG](2)-x(2)-[SAG]-[LIVMF]-x(3)-[LIVMFYWA](2)-x-[GK]-x-R

[1] Ninnemann O., Janniaux J.-C., Frommer W.B. EMBO J. 13:3464-3471(1994).
[2] Siewe R.M., Weil B., Burkovski A., Eikmanns B.J., Eikmanns M., Kraemer R. J. Biol. Chem. 271:5398-5403 (1996).
[3] Saier M.H. Jr. Adv. Microbiol. Physiol. 40:81-136(1998).

29. (Arch_histone)
CBF/NF-Y subunits signatures
Diverse DNA binding proteins are known to bind the CCAAT box, a common cis-acting element found in the promoter and enhancer regions of a large number of genes in eukaryotes. Amongst these proteins is one known as the CCAAT-binding factor (CBF) or NF-Y [1]. CBF is a heteromeric transcription factor that consists of two different components both needed for DNA-binding. The HAP protein complex of yeast binds to the upstream activation site of cytochrome C iso-1 gene (CYC1) as well as other genes involved in mitochondrial electron transport and activates their expression. It also recognizes the sequence CCAAT and is structurally and evolutionary related to CBF. The first subunit of CBF, known as CBF-A or NF-YB in vertebrates, HAP3 in budding yeast and as php3 in fission yeast, is a protein of 116 to 210 amino-acid residues which contains a highly conserved central domain of about 90residues. This domain seems to be involved in DNA-binding; a signature pattern had been developed from its central part. The second subunit of CBF, known as CBF-B or NF-YA in vertebrates, HAP2 in budding yeast and php2 in fission yeast, is a protein of 265 to 350 amino-acid residues which contains a highly conserved region of about 60 residues. This region, called the 'essential core' [2], seems to consist of two subdomains: an N-terminal subunit-association domain and a C-terminal DNA recognition domain. A signature pattern has been developed from a section of the subunit-association domain.
Consensus pattern: C-V-S-E-x-I-S-F-[LIVM]-T-[SG]-E-A-[SC]-[DE]-[KRQ]-C-
Consensus pattern: Y-V-N-A-K-Q-Y-x-R-I-L-K-R-R-x-A-R-A-K-L-E-

[1] Li X.-Y., Mantovani R., Hooft van Huijsduijnen R., Andre I., Benoist C., Mathis D. Nucleic Acids Res. 20: 1087-1091 (1992).
[2] Olesen J.T., Fikes J.D., Guarente L. Mol. Cell. Biol. 11:611-619(1991).

30. Argininosuccinate synthase signatures
Argininosuccinate synthase (EC 6.3.4.5) (AS) is a urea cycle enzyme that catalyzes the penultimate step in arginine biosynthesis: the ATP-dependent ligation of citrulline to aspartate to form argininosuccinate, AMP andpyrophosphate [1,2].In humans, a defect in the AS gene causes citrullinemia, a genetic disease characterized by severe vomiting spells and mental retardation.AS is a homotetrameric enzyme of chains of about 400 amino-acid residues. Anarginine seems to be important for the enzyme's catalytic mechanism. The sequences of AS from various prokaryotes, archaebacteria and eukaryotes show significant similarity. Two signature patterns have been selected for AS. The first is a highly conserved stretch of nine residues located in the N-terminal extremity of these enzymes, the second

is derived from a conserved region which contains one of the conserved arginine residues.
Consensus pattern: [AS]-[FY]-S-G-G-[LV]-D-T-[ST]-
Consensus pattern: G-x-T-x-K-G-N-D-x(2)-R-F-

[1] van Vliet F., Crabeel M., Boyen A., Tricot C., Stalon V., Falmagne P., Nakamura Y., Baumberg S., Glansdorff N. Gene 95:99-104(1990).
[2] Morris C.J., Reeve J.N. J. Bacteriol. 170:3125-3130(1988).

31. Armadillo/beta-catenin-like repeats
Approx. 40 amino acid repeat. Tandem repeats form super-helix of helices that is proposed to mediate interaction of beta-catenin with its ligands. CAUTION: This family does not contain all known armadillo repeats.

[1] Huber AH, Nelson WJ, Weis WI, Cell 1997;90:871-882.
[2] Gumbiner BM, Curr Opin Cell Biol 1995;7:634-640.
[3] Cavallo R, Rubenstein D, Peifer M, Curr Opin Genet Dev 1997;7:459-466.
[4] Su LK, Vogelstein B, Kinzler KW, Science 1993;262:1734-1737.
[5] Masiarz FR, Munemitsu S, Polakis P Science 1993;262:1731-1734
[6] Peifer M, Wieschaus E, Cell 1990;63:1167-1176.

32. (Asn Synthase)
Asparagine synthase
This family is always found associated with GATase 2. Members of this family catalyse the conversion of aspartate to asparagine.

33. Asparaginase_2
Asparaginase 12 members

34. (Aspartyl tRNA N)
Aminoacyl-transfer RNA synthetases class-II signatures
Aminoacyl-tRNA synthetases (EC 6.1.1.-) [1] are a group of enzymes which activate amino acids and transfer them to specific tRNA molecules as the first step in protein biosynthesis. In prokaryotic organisms there are at least twenty different types of aminoacyl-tRNA synthetases, one for each different amino acid. In eukaryotes there are generally two aminoacyl-tRNA synthetases for each different amino acid: one cytosolic form and a mitochondrial form. While all these enzymes have a common function, they are widely diverse in terms of subunit size and of quaternary structure. The synthetases specific for alanine, asparagine, aspartic acid, glycine, histidine, lysine, phenylalanine, proline, serine, and threonine are referred to as class-II synthetases [2 to 6] and probably have a common folding pattern in their catalytic domain for the binding of ATP and amino acid which is different to the Rossmann fold observed for the class I synthetases [7]. Class-II tRNA synthetases do not share a high degree of similarity, however at least three conserved regions are present [2,5,8]. Signature patterns have been derived from two of these regions.
Consensus pattern: [FYH]-R-x-[DE]-x(4,12)-[RH]-x(3)-F-x(3)-[DE]
Consensus pattern: [GSTALVF]-{DENQHRKP}-[GSTA]-[LIVMF]-[DE]-R-[LIVMF]-x-[LIVMSTAG]-[LIVMFY]

[1] Schimmel P. Annu. Rev. Biochem. 56:125-158(1987).
[2] Delarue M., Moras D. BioEssays 15:675-687(1993).
[3] Schimmel P. Trends Biochem. Sci. 16:1-3(1991).
[4] Nagel G.M., Doolittle R.F. Proc. Natl. Acad. Sci. U.S.A. 88:8121-8125(1991).
[5] Cusack S., Haertlein M., Leberman R. Nucleic Acids Res. 19:3489-3498(1991).
[6] Cusack S. Biochimie 75:1077-1081(1993).
[7] Cusack S., Berthet-Colominas C., Haertlein M., Nassar N., Leberman R. Nature 347:249-255(1990).
[8] Leveque F., Plateau P., Dessen P., Blanquet S. Nucleic Acids Res. 18:305-312(1990).

35. (ArfGap) Putative GTP-ase activating protein for Arf. Putative zinc fingers with GTPase activating proteins (GAPs) towards the small GTPase, Arf. The GAP of ARD1 stimulates GTPase hydrolysis for ARD 1 but not ARFs. Number of members: 34

[1] Medline: 96324970. Identification and cloning of centaurin-alpha. A novel phosphatidylinositol 3,4,5-trisphosphate-binding protein from rat brain. Hammonds-Odie LP, Jackson TR, Profit AA, Blader IJ, Turck CW, Prestwich GD, Theibert AB; J Biol Chem 1996;271:18859-18868.

[2] Medline: 97296423. A target of phosphatidylinositol 3,4,5-trisphosphate with a zinc finger motif similar to that of the ADP-ribosylation -factor GTPase-activating protein and two pleckstrin homology domains. Tanaka K, Imajoh-Ohmi S, Sawada T, Shirai R, Hashimoto Y, Iwasaki S, Kaibuchi K, Kanaho Y, Shirai T, Terada Y, Kimura K, Nagata S, Fukui Y; Eur J Biochem 1997;245:512-519.
[3] 98112795. Molecular characterization of the GTPase-activating domain of ADP-ribosylation factor domain protein 1 (ARD1). Vitale N, Moss J, Vaughan M; J Biol Chem 1998;273:2553-2560.

36. Apolipoprotein. Apolipoprotein A1/A4/E family. This family includes: Swiss:P02647 Apolipoprotein A-I. Swiss: P06727 Apolipoprotein A-IV. Swiss:P02649 Apolipoprotein E. These proteins contain several 22 residue repeats which form a pair of alpha helices. Number of members: 42

[1] Medline: 91289138. Three-dimensional structure of the LDL receptor-binding domain of human apolipoprotein E. Wilson C, Wardell MR, Weisgraber KH, Mahley RW, Agard DA; Science 1991;252:1817-1822.

37. Amino acid permeases signature
Amino acid permeases are integral membrane proteins involved in the transport of amino acids into the cell. A number of such proteins have been found to be evolutionary related [1,2,3]. These proteins are: - Yeast general amino acid permeases (genes GAP1, AGP2 and AGP3). - Yeast basic amino acid permease (gene ALP1). - Yeast Leu/Val/Ile permease (gene BAP2). - Yeast arginine permease (gene CAN1). - Yeast dicarboxylic amino acid permease (gene DIP5). - Yeast asparagine/glutamine permease (gene AGP1). - Yeast glutamine permease (gene GNP1). - Yeast histidine permease (gene HIP1). - Yeast lysine permease (gene LYP1). - Yeast proline permease (gene PUT4). - Yeast valine and tyrosine permease (gene VAL1/TAT1). - Yeast tryptophan permease (gene TAT2/SCM2). - Yeast choline transport protein (gene HNM1/CTR1). - Yeast GABA permease (gene UGA4). - Yeast hypothetical protein YKL174c. - Fission yeast protein isp5. - Fission yeast hypothetical protein SpAC8A4.11 - Fission yeast hypothetical protein SpAC11D3.08c. - Emericella nidulans proline transport protein (gene prnB). - Trichoderma harzianum amino acid permease INDA1. - Salmonella typhimurium L-asparagine permease (gene ansP). - Escherichia coli aromatic amino acid transport protein (gene aroP). - Escherichia coli D-serine/D-alanine/glycine transporter (gene cycA). - Escherichia coli GABA permease (gene gabP). - Escherichia coli lysine-specific permease (gene lysP). - Escherichia coli phenylalanine-specific permease (gene pheP). - Salmonella typhimurium proline-specific permease (gene proY). - Escherichia coli and Klebsiella pneumoniae hypothetical protein yeeF. - Escherichia coli and Salmonella typhimurium hypothetical protein yifK. - Bacillus subtilis permeases rocC and rocE which probably transports arginine or ornithine. These proteins seem to contain up to 12 transmembrane segments. As a signature for this family of proteins, the best conserved region which is located in the second transmembrane segment has been selected.
Consensus pattern: [STAGC]-G-[PAG]-x(2,3)-[LIVMFYWA](2)-x-[LIVMFYW]-x-[LIVMFWSTAGC](2)-[STAGC]-x(3)-[LIVMFYWT]-x-[LIVMST]-x(3)- [LIVMCTA]-[GA]-E-x(5)-[PSAL]-

[1] Weber E., Chevalier M.R., Jund R. J. Mol. Evol. 27:341-350(1988).
[2] Vandenbol M., Jauniaux J.-C., Grenson M. Gene 83:153-159(1989).
[3] Reizer J., Finley K., Kakuda D., McLeod C.L., Reizer A., Saier M.H. Jr. Protein Sci. 2:20-30(1993).

38. aakinase (1) Glutamate 5-kinase signature
Glutamate 5-kinase (EC 2.7.2.11) (gamma-glutamyl kinase) (GK) is the enzyme that catalyzes the first step in the biosynthesis of proline from glutamate, the ATP-dependent phosphorylation of L-glutamate into L-glutamate 5-phosphate. In eubacteria (gene proB) and yeast [1] (gene PRO1), GK is a monofunctional protein, while in plants and mammals, it is a bifunctional enzyme (P5CS) [2]that consists of two domains: a N-terminal GK domain and a C-terminal gamma-glutamyl phosphate reductase domain (EC 1.2.1.41) (see <PDOC00940>).As a signature pattern, a highly conserved glycine-and alanine-rich region located in the central section of these enzymes has been selected. Yeast hypothetical protein YHR033w is highly similar to GK.
Consensus pattern: [GSTN]-x(2)-G-x-G-[GC]-[IM]-x-[STA]-K-[LIVM]-x-[SA]-[TCA]-x(2)-[GALV]-x(3)-G-

[1] Li W., Brandriss M.C. J. Bacteriol. 174:4148-4156(1992).
[2] Hu C.-A.A., Delauney A.J., Verma D.P.S. Proc. Natl. Acad. Sci. U.S.A. 89:9354-9358(1992).

aakinase (2) Aspartokinase signature
Aspartokinase (EC 2.7.2.4) (AK) [1] catalyzes the phosphorylation of aspartate. The product of this reaction can then be used in the biosynthesis of lysine or in the pathway leading to homoserine, which participates in the biosynthesis of threonine, isoleucine and methionine. In Escherichia coli, there are three different isozymes

which differ in their sensitivity to repression and inhibition by Lys, Met and Thr. AK1 (gene thrA) and AK2 (gene metL) are bifunctional enzymes which both consist of an N- terminal AK domain and a C-terminal homoserine dehydrogenase domain. AK1 is involved in threonine biosynthesis and AK2, in that of methionine. The third isozyme, AK3 (gene lysC), is monofunctional and involved in lysine synthesis. In yeast, there is a single isozyme of AK (gene HOM3).
As a signature pattern for AK, a conserved region located in the N-terminal extremity has been selected.
Consensus pattern: [LIVM]-x-K-[FY]-G-G-[ST]-[SC]-[LIVM]-

[1] Rafalski J.A., Falco S.C. J. Biol. Chem. 263:2146-2151(1988).

aakinase (3) Gamma-glutamyl phosphate reductase signature
Gamma-glutamyl phosphate reductase (EC 1.2.1.41) (GPR) is the enzyme that catalyzes the second step in the biosynthesis of proline from glutamate, the NADP-dependent reduction of L-glutamate 5-phosphate into L-glutamate 5-semialdehyde and phosphate. In eubacteria (gene proA) and yeast [1] (gene PRO2), GPR is a monofunctional protein, while in plants and mammals, it is a bifunctional enzyme (P5CS) [2]that consists of two domains: a N-terminal glutamate 5-kinase domain(EC 2.7.2.11) (see <PDOC00701>) and a C-terminal GPR domain. As a signature pattern, a conserved region that contains two histidine residues has been selected. This region is located in the last third of GPR.
Consensus pattern: V-x(5)-A-[LIV]-x-H-I-x(2)-[HY]-[GS]-[ST]-x-H-[ST]-[DE]-x- I-

[1] Pearson B.M., Hernando Y., Payne J., WolfS.S., Kalogeropoulos A., Schweizer M. Yeast 12:1021-1031 (1996).
[2] Hu C.-A.A., Delauney A.J., Verma D.P.S. Proc. Natl. Acad. Sci. U.S.A. 89:9354-9358(1992).

39. (abhydrolase) alpha/beta hydrolase fold. This catalytic domain is found in a very wide range of enzymes.

[1] Ollis DL, Cheah E, Cygler M, Dijkstra B, Frolow F, Franken SM, Harel M, Remington SJ, Silman I, Schrag J, Sussman JL, Verschueren KHG, Goldman A, Protein Eng 1992;5:197-211.

40. (Acid phosphat) Histidine acid phosphatases signatures
Acid phosphatases (EC 3.1.3.2) are a heterogeneous group of proteins that hydrolyze phosphate esters, optimally at low pH. It has been shown [1] that a number of acid phosphatases, from both prokaryotes and eukaryotes, share two regions of sequence similarity, each centered around a conserved histidine residue. These two histidines seem to be involved in the enzymes' catalytic mechanism [2,3]. The first histidine is located in the N-terminal section and forms a phosphohistidine intermediate while the second is located in the C- terminal section and possibly acts as proton donor. Enzymes belonging to this family are called 'histidine acid phosphatases' and are listed below:

- Escherichia coli pH 2.5 acid phosphatase (gene appA).
- Escherichia coli glucose-1-phosphatase (EC 3.1.3.10) (gene agp).
- Yeast constitutive and repressible acid phosphatases (genes PHO3 and PHO5)
- Fission yeast acid phosphatase (gene pho1).
- Aspergillus phytases A and B (EC 3.1.3.8) (gene phyA and phyB).
- Mammalian lysosomal acid phosphatase.
- Mammalian prostatic acid phosphatase.
- Caenorhabditis elegans hypothetical proteins B0361.7, C05C10.1, C05C10.4 and F26C11.1.

Consensus pattern[LIVM]-x(2)-[LIVMA]-x(2)-[LIVM]-x-R-H-[GN]-x-R-x-[PAS] [H is the phosphohistidine residue]
Consensus pattern[LIVMF]-x-[LIVMFAG]-x(2)-[STAGI]-H-D-[STANQ]-x-[LIVM]-x(2)-[LIVMFY]-x(2)-[STA] [H is an active site residue] Sequences known to belong to this class detected by the patternALL, except for rat prostatic acid phosphatase which seems to have Tyr instead of the active site His

[1] van Etten R.L., Davidson R., Stevis P.E., MacArthur H., Moore D.L. J. Biol. Chem. 266:2313-2319(1991).
[2] Ostanin K., Harms E.H., Stevis P.E., Kuciel R., Zhou M.-M., van Etten R.L. J. Biol. Chem. 267:22830-22836 (1992).
[3] Schneider G., Lindqvist Y., Vihko P. EMBO J. 12:2609-2615(1993).

41. Aconitase family signatures
Aconitase (aconitate hydratase) (EC 4.2.1.3) [1] is the enzyme from the tricarboxylic acid cycle that catalyzes the

reversible isomerization of citrate and isocitrate. Cis-aconitate is formed as an intermediary product during the course of the reaction. In eukaryotes two isozymes of aconitase are known to exist: one found in the mitochondrial matrix and the other found in the cytoplasm. Aconitase, in its active form, contains a 4Fe-4S iron-sulfur cluster; three cysteine residues have been shown to be ligands of the 4Fe-4S cluster. It has been shown that the aconitase family also contains the following proteins: - Iron-responsive element binding protein (IRE-BP). IRE-BP is a cytosolic protein that binds to iron-responsive elements (IREs). IREs are stem-loop structures found in the 5'UTR of ferritin, and delta aminolevulinic acid synthase mRNAs, and in the 3'UTR of transferrin receptor mRNA. IRE-BP also express aconitase activity. - 3-isopropylmalate dehydratase (EC 4.2.1.33) (isopropylmalate isomerase), the enzyme that catalyzes the second step in the biosynthesis of leucine. - Homoaconitase (EC 4.2.1.36) (homoaconitate hydratase), an enzyme that participates in the alpha-aminoadipate pathway of lysine biosynthesis and that converts cis-homoaconitate into homoisocitric acid. - Esherichia coli protein ybhJ

Consensus pattern: [LIVM]-x(2)-[GSACIVM]-x-[LIV]-[GTIV]-[STP]-C-x(0,1)-T-N-[GSTANI]-x(4)-[LIVMA] [C binds the iron-sulfur center]

Consensus pattern: G-x(2)-[LIVWPQ]-x(3)-[GAC]-C-[GSTAM]-[LIMPTA]-C-[LIMV]-[GA] [The two C's bind the iron-sulfur center]-

[1] Gruer M.J., Artymiuk P.J., Guest J.R. Trends Biochem. Sci. 22:3-6(1997).

42. Actins signatures

Actins [1 to 4] are highly conserved contractile proteins that are present in all eukaryotic cells. In vertebrates there are three groups of actin isoforms: alpha, beta and gamma. The alpha actins are found in muscle tissues and are a major constituent of the contractile apparatus. The beta and gamma actins co-exists in most cell types as components of the cytoskeleton and as mediators of internal cell motility. In plants [5] there are many isoforms which are probably involved in a variety of functions such as cytoplasmic streaming, cell shape determination, tip growth, graviperception, cell wall deposition, etc. Actin exists either in a monomeric form (G-actin) or in a polymerized form (F-actin). Each actin monomer can bind a molecule of ATP; when polymerization occurs, the ATP is hydrolyzed. Actin is a protein of from 374 to 379 amino acid residues. The structure of actin has been highly conserved in the course of evolution. Recently some divergent actin-like proteins have been identified in several species. These proteins are: - Centractin (actin-RPV) from mammals, fungi (yeast ACT5, Neurospora crassa ro-4) and Pneumocystis carinii (actin-II). Centractin seems to be a component of a multi-subunit centrosomal complex involved in microtubule based vesicle motility. This subfamily is also known as ARP1. - ARP2 subfamily which includes chicken ACTL, yeast ACT2, Drosophila 14D, C.elegans actC. - ARP3 subfamily which includes actin 2 from mammals, Drosophila 66B, yeast ACT4 and fission yeast act2. - ARP4 subfamily which includes yeast ACT3 and Drosophila 13E. Three signature patterns have been developed. The first two are specific to actins and span positions 54 to 64 and 357 to 365. The last signature picks up both actins and the actin-like proteins and corresponds to positions 106 to 118 in actins.

Consensus pattern: [FY]-[LIV]-G-[DE]-E-A-Q-x-[RKQ](2)-G-

Consensus pattern: W-[IV]-[STA]-[RK]-x-[DE]-Y-[DNE]-[DE]-

Consensus pattern: [LM]-[LIVM]-T-E-[GAPQ]-x-[LIVMFYWHQ]-N-[PSTAQ]-x(2)-N-[KR]-

[1] Sheterline P., Clayton J., Sparrow J.C. (In) Actins, 3rd Edition, Academic Press Ltd, London, (1996).
[2] Pollard T.D., Cooper J.A. Annu. Rev. Biochem. 55:987-1036(1986).
[3] Pollard T.D. Curr. Opin. Cell Biol. 1:33-40(1990).
[4] Rubenstein P.A. BioEssays 12:309-315(1990).
[5] Meagher R.B., McLean B.G. Cell Motil. Cytoskeleton 16:164-166(1990).

43. Adenylate kinase signature

Adenylate kinase (EC 2.7.4.3) (AK) [1] is a small monomeric enzyme that catalyzes the reversible transfer of MgATP to AMP (MgATP + AMP = MgADP + ADP).In mammals there are three different isozymes: - AK1 (or myokinase), which is cytosolic. - AK2, which is located in the outer compartment of mitochondria. - AK3 (or GTP:AMP phosphotransferase), which is located in the mitochondrial matrix and which uses MgGTP instead of MgATP.The sequence of AK has also been obtained from different bacterial species and from plants and fungi. Two other enzymes have been found to be evolutionary related to AK. These are: - Yeast uridylate kinase (EC 2.7.4.-) (UK) (gene URA6) [2] which catalyzes the transfer of a phosphate group from ATP to UMP to form UDP and ADP. - Slime mold UMP-CMP kinase (EC 2.7.4.14) [3] which catalyzes the transfer of a phosphate group from ATP to either CMP or UMP to form CDP or UDP and ADP. Several regions of AK family enzymes are well conserved, including the ATP-binding domains. The most conserved of all regions have been selected as a signature for this type of enzyme. This region includes an aspartic acid residue that is part of the catalytic cleft of the enzyme and that is involved in a salt bridge. It also includes an arginine residue whose modification leads to inactivation of the enzyme

Consensus pattern: [LIVMFYW](3)-D-G-[FYI]-P-R-x(3)-[NQ]-

[1] Schulz G.E. Cold Spring Harbor Symp. Quant. Biol. 52:429-439(1987).
[2] Liljelund P., Sanni A., Friesen J.D., Lacroute F. Biochem. Biophys. Res. Commun. 165:464-473(1989).
[3] Wiesmueller L., Noegel A.A., Barzu O., Gerisch G., Schleicher M. J. Biol. Chem. 265:6339-6345(1990).
[4] Kath T.H., Schmid R., Schaefer G. Arch. Biochem. Biophys. 307:405-410(1993).

44. (adh_short) Short-chain dehydrogenases/reductases family signature. The short-chain dehydrogenases/reductases family (SDR) [1] is a very large family of enzymes, most of which are known to be NAD- or NADP-dependent oxidoreductases. As the first member of this family to be characterized was Drosophila alcohol dehydrogenase, this family used to be called [2,3,4]'insect-type', or 'short-chain' alcohol dehydrogenases. Most member of this family are proteins of about 250 to 300 amino acid residues. The proteins currently known to belong to this family are listed below. - Alcohol dehydrogenase (EC 1.1.1.1) from insects such as Drosophila. - Acetoin dehydrogenase (EC 1.1.1.5) from Klebsiella terrigena (gene budC). - D-beta-hydroxybutyrate dehydrogenase (BDH) (EC 1.1.1.30) from mammals. - Acetoacetyl-CoA reductase (EC 1.1.1.36) from various bacterial species (gene phbB or phaB). - Glucose 1-dehydrogenase (EC 1.1.1.47) from Bacillus. - 3-beta-hydroxysteroid dehydrogenase (EC 1.1.1.51) from Comomonas testosteroni. - 20-beta-hydroxysteroid dehydrogenase (EC 1.1.1.53) from Streptomyces hydrogenans. - Ribitol dehydrogenase (EC 1.1.1.56) (RDH) from Klebsiella aerogenes. - Estradiol 17-beta-dehydrogenase (EC 1.1.1.62) from human. - Gluconate 5-dehydrogenase (EC 1.1.1.69) from Gluconobacter oxydans (gene gno). - 3-oxoacyl-[acyl-carrier protein] reductase (EC 1.1.1.100) from Escherichia coli (gene fabG) and from plants. - Retinol dehydrogenase (EC 1.1.1.105) from mammals. - 2-deoxy-d-gluconate 3-dehydrogenase (EC 1.1.1.125) from Escherichia coli and Erwinia chrysanthemi (gene kduD). - Sorbitol-6-phosphate 2-dehydrogenase (EC 1.1.1.140) from Escherichia coli (gene gutD) and from Klebsiella pneumoniae (gene sorD). - 15-hydroxyprostaglandin dehydrogenase (NAD+) (EC 1.1.1.141) from human. - Corticosteroid 11-beta-dehydrogenase (EC 1.1.1.146) (11-DH) from mammals. - 7-alpha-hydroxysteroid dehydrogenase (EC 1.1.1.159) from Escherichia coli (gene hdhA), Eubacterium strain VPI 12708 (gene baiA) and from Clostridium sordellii. - NADPH-dependent carbonyl reductase (EC 1.1.1.184) from mammals. - Tropinone reductase-I (EC 1.1.1.206) and -II (EC 1.1.1.236) from plants. - N-acylmannosamine 1-dehydrogenase (EC 1.1.1.233) from Flavobacterium strain 141-8. - D-arabinitol 2-dehydrogenase (ribulose forming) (EC 1.1.1.250) from fungi. - Tetrahydroxynaphthalene reductase (EC 1. 1. 1.252) from Magnaporthe grisea. - Pteridine reductase 1 (EC 1.1.1.253) (gene PTR1) from Leishmania. - 2,5-dichloro-2,5-cyclohexadiene-1,4-diol dehydrogenase (EC 1.1.-.-) from Pseudomonas paucimobilis. - Cis-1,2-dihydroxy-3,4-cyclohexadiene-1-carboxylate dehydrogenase (EC 1.3.1. -) from Acinetobacter calcoaceticus (gene benD) and Pseudomonas putida (gene xylL). - Biphenyl-2,3-dihydro-2,3-diol dehydrogenase (EC 1.3.1.-) (gene bphB) from various Pseudomonaceae. - Cis-toluene dihydrodiol dehydrogenase (EC 1.3.1.-) from Pseudomonas putida (gene todD). - Cis-benzene glycol dehydrogenase (EC 1.3.1.19) from Pseudomonas putida (gene bnzE). - 2,3-dihydro-2,3-dihydroxybenzoate dehydrogenase (EC 1.3.1.28) from Escherichia coli (gene entA) and Bacillus subtilis (gene dhbA). - Dihydropteridine reductase (EC 1.6.99.7) (HDHPR) from mammals. - Lignin degradation enzyme ligD from Pseudomonas paucimobilis. - Agropine synthesis reductase from Agrobacterium plasmids (gene mas1). - Versicolorin reductase from Aspergillus parasiticus (gene VER1). - Putative keto-acyl reductases from Streptomyces polyketide biosynthesis operons. - A trifunctional hydratase-dehydrogenase-epimerase from the peroxisomal beta-oxidation system of Candida tropicalis. This protein contains two tandemly repeated 'short-chain dehydrogenase-type' domain in its N-terminal extremity. - Nodulation protein nodG from species of Azospirillum and Rhizobium which is probably involved in the modification of the nodulation Nod factor fatty acyl chain. - Nitrogen fixation protein fixR from Bradyrhizobium japonicum. - Bacillus subtilis protein dltE which is involved in the biosynthesis of D- alanyl-lipoteichoic acid. - Human follicular variant translocation protein 1 (FVT1). - Mouse adipocyte protein p27. - Mouse protein Ke 6. - Maize sex determination protein TASSELSEED 2. - Sarcophaga peregrina 25 Kd development specific protein. - Drosophila fat body protein P6. - A Listeria monocytogenes hypothetical protein encoded in the internalins gene region. - Escherichia coli hypothetical protein yciK. - Escherichia coli hypothetical protein ydfG. - Escherichia coli hypothetical protein yjgI. - Escherichia coli hypothetical protein yjgU. - Escherichia coli hypothetical protein yohF. - Bacillus subtilis hypothetical protein yoxD. - Bacillus subtilis hypothetical protein ywfD. - Bacillus subtilis hypothetical protein ywfH. - Yeast hypothetical protein YIL124w. - Yeast hypothetical protein YIR035c. - Yeast hypothetical protein YIR036c. - Yeast hypothetical protein YKL055c. - Fission yeast hypothetical protein SpAC23D3.11. One of the best conserved regions which includes two perfectly conserved residues, a tyrosine and a lysine has been selected as a signature pattern for this family of proteins. The tyrosine residue participates in the catalytic mechanism.
Consensus pattern: [LIVSPADNK]-x(12)-Y-[PSTAGNCV]-[STAGNQCIVM]-[STAGC]-K- {PC}-[SAGFYR]-[LIVMSTAGD]-x(2)-[LIVMFYW]-x(3)- [LIVMFYWGAPTHQ]-[GSACQRHM] [Y is an active site residue] -

[1] Joernvall H., Persson B., Krook M., Atrian S., Gonzalez-Duarte R., Jeffery J., Ghosh D. Biochemistry 34:

6003-6013(1995).
[2] Villarroya A., Juan E., Egestad B., Joernvall H. Eur. J. Biochem. 180:191-197(1989).
[3] Persson B., Krook M., Joernvall H. Eur. J. Biochem. 200:537-543(1991).
[4] Neidle E.L., Hartnett C., Ornston N.L., Bairoch A., Rekik M., Harayama S. Eur. J. Biochem. 204:113-120 (1992).

45. (adh_short_C2) Short-chain dehydrogenases/reductases family signature
The short-chain dehydrogenases/reductases family (SDR) [1] is a very large family of enzymes, most of which are known to be NAD- or NADP-dependent oxidoreductases. As the first member of this family to be characterized was Drosophila alcohol dehydrogenase, this family used to be called [2,3,4]'insect-type', or 'short-chain' alcohol dehydrogenases. Most member of this family are proteins of about 250 to 300 amino acid residues. The proteins currently known to belong to this family are listed below. - Alcohol dehydrogenase (EC 1.1.1.1) from insects such as Drosophila. - Acetoin dehydrogenase (EC 1.1.1.5) from Klebsiella terrigena (gene budC). - D-beta-hydroxybutyrate dehydrogenase (BDH) (EC 1.1.1.30) from mammals. - Acetoacetyl-CoA reductase (EC 1.1.1.36) from various bacterial species (gene phbB or phaB). - Glucose 1-dehydrogenase (EC 1.1.1.47) from Bacillus. - 3-beta-hydroxysteroid dehydrogenase (EC 1.1.1.51) from Comomonas testosteroni. - 20-beta-hydroxysteroid dehydrogenase (EC 1.1.1.53) from Streptomyces hydrogenans. - Ribitol dehydrogenase (EC 1.1.1.56) (RDH) from Klebsiella aerogenes. - Estradiol 17-beta-dehydrogenase (EC 1.1.1.62) from human. - Gluconate 5-dehydrogenase (EC 1.1.1.69) from Gluconobacter oxydans (gene gno). - 3-oxoacyl-[acyl-carrier protein] reductase (EC 1.1.1.100) from Escherichia coli (gene fabG) and from plants. - Retinol dehydrogenase (EC 1.1.1.105) from mammals. - 2-deoxy-d-gluconate 3-dehydrogenase (EC 1.1.1.125) from Escherichia coli and Erwinia chrysanthemi (gene kduD). - Sorbitol-6-phosphate 2-dehydrogenase (EC 1.1.1.140) from Escherichia coli (gene gutD) and from Klebsiella pneumoniae (gene sorD). - 15-hydroxyprostaglandin dehydrogenase (NAD+) (EC 1.1.1.141) from human. - Corticosteroid 11-beta-dehydrogenase (EC 1.1.1.146) (11-DH) from mammals. - 7-alpha-hydroxysteroid dehydrogenase (EC 1.1.1.159) from Escherichia coli (gene hdhA), Eubacterium strain VPI 12708 (gene baiA) and from Clostridium sordellii. - NADPH-dependent carbonyl reductase (EC 1.1.1.184) from mammals. - Tropinone reductase-I (EC 1.1.1.206) and -II (EC 1.1.1.236) from plants. - N-acylmannosamine 1-dehydrogenase (EC 1.1.1.233) from Flavobacterium strain 141-8. - D-arabinitol 2-dehydrogenase (ribulose forming) (EC 1.1.1.250) from fungi. - Tetrahydroxynaphthalene reductase (EC 1.1.1.252) from Magnaporthe grisea. - Pteridine reductase 1 (EC 1.1.1.253) (gene PTR1) from Leishmania. - 2,5-dichloro-2,5-cyclohexadiene-1,4-diol dehydrogenase (EC 1.1.-.-) from Pseudomonas paucimobilis. - Cis-1,2-dihydroxy-3,4-cyclohexadiene-1-carboxylate dehydrogenase (EC 1.3.1. -) from Acinetobacter calcoaceticus (gene benD) and Pseudomonas putida (gene xylL). - Biphenyl-2,3-dihydro-2,3-diol dehydrogenase (EC 1.3.1.-) (gene bphB) from various Pseudomonaceae. - Cis-toluene dihydrodiol dehydrogenase (EC 1.3.1.-) from Pseudomonas putida (gene todD). - Cis-benzene glycol dehydrogenase (EC 1.3.1.19) from Pseudomonas putida (gene bnzE). - 2,3-dihydro-2,3-dihydroxybenzoate dehydrogenase (EC 1.3.1.28) from Escherichia coli (gene entA) and Bacillus subtilis (gene dhbA). - Dihydropteridine reductase (EC 1.6.99.7) (HDHPR) from mammals. - Lignin degradation enzyme ligD from Pseudomonas paucimobilis. - Agropine synthesis reductase from Agrobacterium plasmids (gene mas1). - Versicolorin reductase from Aspergillus parasiticus (gene VER1). - Putative keto-acyl reductases from Streptomyces polyketide biosynthesis operons. - A trifunctional hydratase-dehydrogenase-epimerase from the peroxisomal beta-oxidation system of Candida tropicalis. This protein contains two tandemly repeated 'short-chain dehydrogenase-type' domain in its N-terminal extremity. - Nodulation protein nodG from species of Azospirillum and Rhizobium which is probably involved in the modification of the nodulation Nod factor fatty acyl chain. - Nitrogen fixation protein fixR from Bradyrhizobium japonicum. - Bacillus subtilis protein dltE which is involved in the biosynthesis of D- alanyl-lipoteichoic acid. - Human follicular variant translocation protein 1 (FVT1). - Mouse adipocyte protein p27. - Mouse protein Ke 6. - Maize sex determination protein TASSELSEED 2. - Sarcophaga peregrina 25 Kd development specific protein. - Drosophila fat body protein P6. - A Listeria monocytogenes hypothetical protein encoded in the internalins gene region. - Escherichia coli hypothetical protein yciK. - Escherichia coli hypothetical protein ydfG. - Escherichia coli hypothetical protein yjgI. - Escherichia coli hypothetical protein yjgU. - Escherichia coli hypothetical protein yohF. - Bacillus subtilis hypothetical protein yoxD. - Bacillus subtilis hypothetical protein ywfD. - Bacillus subtilis hypothetical protein ywfH. - Yeast hypothetical protein YIL124w. - Yeast hypothetical protein YIR035c. - Yeast hypothetical protein YIR036c. - Yeast hypothetical protein YKL055c. - Fission yeast hypothetical protein SpAC23D3.11. One of the best conserved regions which includes two perfectly conserved residues, a tyrosine and a lysine has been used as a signature pattern for this family of proteins. The tyrosine residue participates in the catalytic mechanism.
Consensus pattern: [LIVSPADNK]-x(12)-Y-[PSTAGNCV]-[STAGNQCIVM]-[STAGC]-K- {PC}-[SAGFYR]-[LIVMSTAGD]-x(2)-[LIVMFYW]-x(3)- [LIVMFYWGAPTHQ]-[GSACQRHM] [Y is an active site residue]

[1] Joernvall H., Persson B., Krook M., Atrian S., Gonzalez-Duarte R., Jeffery J., Ghosh D. Biochemistry 34: 6003-6013(1995).

[2] Villarroya A., Juan E., Egestad B., Joernvall H. Eur. J. Biochem. 180:191-197(1989).
[3] Persson B., Krook M., Joernvall H. Eur. J. Biochem. 200:537-543(1991).
[4] Neidle E.L., Hartnett C., Ornston N.L., Bairoch A., Rekik M., Harayama S. Eur. J. Biochem. 204:113-120 (1992).

46. (adh_zinc) Zinc-containing alcohol dehydrogenases signatures
Alcohol dehydrogenase (EC 1.1.1.1) (ADH) catalyzes the reversible oxidation of ethanol to acetaldehyde with the concomitant reduction of NAD [1]. Currently three, structurally and catalytically, different types of alcohol dehydrogenases are known: - Zinc-containing 'long-chain' alcohol dehydrogenases. - Insect-type, or 'short-chain' alcohol dehydrogenases. - Iron-containing alcohol dehydrogenases.Zinc-containing ADH's [2,3] are dimeric or tetrameric enzymes that bind two atoms of zinc per subunit. One of the zinc atom is essential for catalytic activity while the other is not. Both zinc atoms are coordinated by either cysteine or histidine residues; the catalytic zinc is coordinated by two cysteines and one histidine. Zinc-containing ADH's are found in bacteria, mammals, plants, and in fungi. In most species there are more than one isozyme (for example, human have at least six isozymes, yeast have three, etc.). A number of other zinc-dependent dehydrogenases are closely related to zinc ADH [4], these are: - Xylitol dehydrogenase (EC 1.1.1.9) (D-xylulose reductase). - Sorbitol dehydrogenase (EC 1.1.1.14). - Aryl-alcohol dehydrogenase (EC 1.1.1.90) (benzyl alcohol dehydrogenase). - Threonine 3-dehydrogenase (EC 1.1.1.103). - Cinnamyl-alcohol dehydrogenase (EC 1.1.1.195) (CAD) [5]. CAD is a plant enzyme involved in the biosynthesis of lignin. - Galactitol-1-phosphate dehydrogenase (EC 1.1.1.251). - Pseudomonas putida 5-exo-alcohol dehydrogenase (EC 1.1.1.-) [6]. - Escherichia coli starvation sensing protein rspB. - Escherichia coli hypothetical protein yjgB. - Escherichia coli hypothetical protein yjgV. - Escherichia coli hypothetical protein yjjN. - Yeast hypothetical protein YAL060w (FUN49). - Yeast hypothetical protein YAL061w (FUN50). - Yeast hypothetical protein YCR105w. The pattern that has been developed to detect this class of enzymes is based on a conserved region that includes a histidine residue which is the second ligand of the catalytic zinc atom. This family also includes NADP-dependent quinone oxidoreductase (EC 1.6.5.5),an enzyme found in bacteria (gene qor), in yeast and in mammals where, in some species such as rodents, it has been recruited as an eye lens protein and is known as zeta-crystallin [7]. The sequence of quinone oxidoreductase is distantly related to that other zinc-containing alcohol dehydrogenases and it lacks the zinc-ligand residues. The torpedo fish and mammlian synaptic vesicle membrane protein vat-1 is related to qor. A specific pattern has been developed for this subfamily.
Consensus pattern: G-H-E-x(2)-G-x(5)-[GA]-x(2)-[IVSAC] [H is a zinc ligand]
Consensus pattern: [GSD]-[DEQH]-x(2)-L-x(3)-[SA](2)-G-G-x-G-x(4)-Q-x(2)-[KR]-

[1] Branden C.-I., Joernvall H., Eklund H., Furugren B. (In) The Enzymes (3rd edition) 11:104-190(1975).
[2] Joernvall H., Persson B., Jeffery J. Eur. J. Biochem. 167:195-201(1987).
[3] Sun H.-W., Plapp B.V. J. Mol. Evol. 34:522-535(1992).
[4] Persson B., Hallborn J., Walfridsson M., Hahn-Haegerdal B., Keraenen S., Penttilae M., Joernvall H. FEBS Lett. 324:9-14(1993).
[5] Knight M.E., Halpin C., Schuch W. Plant Mol. Biol. 19:793-801(1992).
[6] Koga H., Aramaki H., Yamaguchi E., Takeuchi K., Horiuchi T., Gunsalus I.C. J. Bacteriol. 166:1089-1095 (1986).
[7] Joernvall H., Persson B., Du Bois G., Lavers G.C., Chen J.H., Gonzalez P., Rao P.V., Zigler J.S. Jr. FEBS Lett. 322:240-244(1993).

47. (aldedh) Aldehyde dehydrogenases active sites
Aldehyde dehydrogenases (EC 1.2.1.3 and EC 1.2.1.5) are enzymes which oxidize a wide variety of aliphatic and aromatic aldehydes. In mammals at least four different forms of the enzyme are known [1]: class-1 (or Ald C) a tetrameric cytosolic enzyme, class-2 (or Ald M) a tetrameric mitochondrial enzyme, class-3 (or Ald D) a dimeric cytosolic enzyme, and class IV a microsomal enzyme. Aldehyde dehydrogenases have also been sequenced from fungal and bacterial species. A number of enzymes are known to be evolutionary related to aldehyde dehydrogenases; these enzymes are listed below. - Plants and bacterial betaine-aldehyde dehydrogenase (EC 1.2.1.8) [2], an enzyme that catalyzes the last step in the biosynthesis of betaine. - Plants and bacterial NADP-dependent glyceraldehyde-3-phosphate dehydrogenase (EC 1.2.1.9). - Escherichia coli succinate-semialdehyde dehydrogenase (NADP+) (EC 1.2.1.16) (gene gabD) [3], which reduces succinate semialdehyde into succinate. - Escherichia coli lactaldehyde dehydrogenase (EC 1.2.1.22) (gene ald) [4]. - Mammalian succinate semialdehyde dehydrogenase (NAD+) (EC 1.2.1.24). - Escherichia coli phenylacetaldehyde dehydrogenase (EC 1.2.1.39). - Escherichia coli 5-carboxymethyl-2-hydroxymuconate semialdehyde dehydrogenase (gene hpcC). - Pseudomonas putida 2-hydroxymuconic semialdehyde dehydrogenase [5] (genes dmpC and xylG), an enzyme in the meta-cleavage pathway for the degradation of phenols, cresols and catechol. - Bacterial and mammalian methylmalonate-semialdehyde dehydrogenase (MMSDH) (EC 1.2.1.27) [6], an enzyme involved in the distal pathway of valine catabolism. - Yeast delta-1-pyrroline-5-carboxylate dehydrogenase (EC 1.5.1.12) [7] (gene PUT2), which converts proline to glutamate. - Bacterial multifunctional putA protein, which contains a delta-1-pyrroline- 5-carboxylate dehydrogenase domain. - 26G, a garden pea protein of unknown function which is induced by dehydration of shoots [8]. - Mammalian formyltetrahydrofolate dehydrogenase (EC 1.5.1.6) [9]. This is a cytosolic enzyme responsible for the NADP-dependent decarboxylative reduction of 10-formyltetrahydrofolate into tetrahydrofolate. It is an protein of about 900 amino acids which consist of three domains; the C- terminal domain (480 residues) is structurally and functionally related to aldehyde dehydrogenases. - Yeast hypothetical protein YBR006w. - Yeast hypothetical protein YER073w. - Yeast hypothetical protein YHR039c. - Caenorhabditis elegans hypothetical protein F01F1.6.A glutamic acid and a cysteine residue have been implicated in the catalytic activity of mammalian aldehyde dehydrogenase. These residues are conserved in all the enzymes of this family. Two patterns have been derived for this family, one for each of the active site residues.
Consensus pattern: [LIVMFGA]-E-[LIMSTAC]-[GS]-G-[KNLM]-[SADN]-[TAPFV] [E is the active site residue]-
Consensus pattern: [FYLVA]-x(3)-G-[QE]-x-C-[LIVMGSTANC]-[AGCN]-x-[GSTADNEKR] [C is the active site residue

[1] Hempel J., Harper K., Lindahl R. Biochemistry 28:1160-1167(1989).
[2] Weretilnyk E.A., Hanson A.D. Proc. Natl. Acad. Sci. U.S.A. 87:2745-2749(1990).
[3] Niegemann E., Schulz A., Bartsch K. Arch. Microbiol. 160:454-460(1993).
[4] Hidalgo E., Chen Y.-M., Lin E.C.C., Aguilar J. J. Bacteriol. 173:6118-6123(1991).
[5] Nordlund I., Shingler V. Biochim. Biophys. Acta 1049:227-230(1990).
[6] Steele M.I., Lorenz D., Hatter K., Park A., Sokatch J.R. J. Biol. Chem. 267:13585-13592(1992).
[7] Krzywicki K.A., Brandriss M.C. Mol. Cell. Biol. 4:2837-2842(1984).
[8] Guerrero F.D., Jones J.T., Mullet J.E. Plant Mol. Biol. 15:11-26(1990).
[9] Cook R.J., Lloyd R.S., Wagner C. J. Biol. Chem. 266:4965-4973(1991).

48. Aldo/keto reductase family signatures
The aldo-keto reductase family [1,2] groups together a number of structurally and functionally related NADPH-dependent oxidoreductases as well as some other proteins. The proteins known to belong to this family are: - Aldehyde reductase (EC 1.1.1.2). - Aldose reductase (EC 1.1.1.21). - 3-alpha-hydroxysteroid dehydrogenase (EC 1.1.1.50), which terminates androgen action by converting 5-alpha-dihydrotestosterone to 3-alpha-androstanediol. - Prostaglandin F synthase (EC 1.1.1.188) which catalyzes the reduction of prostaglandins H2 and D2 to F2-alpha. - D-sorbitol-6-phosphate dehydrogenase (EC 1.1.1.200) from apple. - Morphine 6-dehydrogenase (EC 1.1.1.218) from Pseudomonas putida plasmid pMDH7.2 (gene morA). - Chlordecone reductase (EC 1.1.1.225) which reduces the pesticide chlordecone (kepone) to the corresponding alcohol. - 2,5-diketo-D-gluconic acid reductase (EC 1.1.1.-) which catalyzes the reduction of 2,5-diketogluconic acid to 2-keto-L-gulonic acid, a key intermediate in the production of ascorbic acid. - NAD(P)H-dependent xylose reductase (EC 1.1.1.-) from the yeast Pichia stipitis. This enzyme reduces xylose into xylit. - Trans-1,2-dihydrobenzene-1,2-diol dehydrogenase (EC 1.3.1.20). - 3-oxo-5-beta-steroid 4-dehydrogenase (EC 1.3.99.6) which catalyzes the reduction of delta(4)-3-oxosteroids. - A soybean reductase, which co-acts with chalcone synthase in the formation of 4,2',4'-trihydroxychalcone. - Frog eye lens rho crystallin. - Yeast GCY protein, whose function is not known. - Leishmania major P110/11E protein. P110/11E is a developmentally regulated protein whose abundance is markedly elevated in promastigotes compared with amastigotes. Its exact function is not yet known. - Escherichia coli hypothetical protein yafB. - Escherichia coli hypothetical protein yghE. - Yeast hypothetical protein YBR149w. - Yeast hypothetical protein YHR104w. - Yeast hypothetical protein YJR096w.These proteins have all about 300 amino acid residues. Three consensus patterns have been developed that are specific to this family of proteins. The first one is located in the N-terminal section of these proteins. The second pattern is located in the central section. The third pattern, located in the C-terminal, is centered on a lysine residue whose chemical modification, in aldose and aldehydereductases, affect the catalytic efficiency.
Consensus pattern: G-[FY]-R-[HSAL]-[LIVMF]-D-[STAGC]-[AS]-x(5)-E-x(2)-[LIVM]- G
Consensus pattern: [LIVMFY]-x(9)-[KREQ]-x-[LIVM]-G-[LIVM]-[SC]-N-[FY]-
Consensus pattern: [LIVM]-[PAIV]-[KR]-[ST]-x(4)-R-x(2)-[GSTAEQK]-[NSL]-x(2)-[LIVMFA] [K is a putative active site residue]-

[1] Bohren K.M., Bullock B., Wermuth B., Gabbay K.H. J. Biol. Chem. 264:9547-9551(1989).
[2] Bruce N.C., Willey D.L., Coulson A.F.W., Jeffery J. Biochem. J. 299:805-811(1994).

49. Alpha amylase. This family is classified as family 13 of the glycosyl hydrolases. The structure is an 8 stranded alpha/beta barrel, interrupted by a ~70 a.a. calcium-binding domain protruding between beta strand 3 and alpha

helix 3, and a carboxyl-terminal Greek key beta-barrel domain.

[1] Larson SB, Greenwood A, Cascio D, Day J, McPherson A, J Mol Biol 1994;235:1560-1584.

50. Aminotransferases class-I pyridoxal-phosphate attachment site

Aminotransferases share certain mechanistic features with other pyridoxal- phosphate dependent enzymes, such as the covalent binding of the pyridoxal- phosphate group to a lysine residue. On the basis of sequence similarity, these various enzymes can be grouped [1,2] into subfamilies. One of these, called class-I, currently consists of the following enzymes: - Aspartate aminotransferase (AAT) (EC 2.6.1.1). AAT catalyzes the reversible transfer of the amino group from L-aspartate to 2-oxoglutarate to form oxaloacetate and L-glutamate. In eukaryotes, there are two AAT isozymes: one is located in the mitochondrial matrix, the second is cytoplasmic. In prokaryotes, only one form of AAT is found (gene aspC). - Tyrosine aminotransferase (EC 2.6.1.5) which catalyzes the first step in tyrosine catabolism by reversibly transferring its amino group to 2- oxoglutarate to form 4-hydroxyphenylpyruvate and L-glutamate. - Aromatic aminotransferase (EC 2.6.1.57) involved in the synthesis of Phe, Tyr, Asp and Leu (gene tyrB). - 1-aminocyclopropane-1-carboxylate synthase (EC 4.4.1.14) (ACC synthase) from plants. ACC synthase catalyzes the first step in ethylene biosynthesis. - Pseudomonas denitrificans cobC, which is involved in cobalamin biosynthesis. - Yeast hypothetical protein YJL060w.The sequence around the pyridoxal-phosphate attachment site of this class of enzyme is sufficiently conserved to allow the creation of a specific pattern.

Consensus pattern: [GS]-[LIVMFYTAC]-[GSTA]-K-x(2)-[GSALVN]-[LIVMFA]-x-[GNAR]- x-R-[LIVMA]-[GA] [K is the pyridoxal-P attachment site]

[1] Bairoch A. Unpublished observations (1992).
[2] Sung M.H., Tanizawa K., Tanaka H., Kuramitsu S., Kagamiyama H., Hirotsu K., Okamoto A., Higuchi T., Soda K. J. Biol. Chem. 266:2567-2572(1991).

51. Aminotransferases class-II pyridoxal-phosphate attachment site

Aminotransferases share certain mechanistic features with other pyridoxal- phosphate dependent enzymes, such as the covalent binding of the pyridoxal- phosphate group to a lysine residue. On the basis of sequence similarity, these various enzymes can be grouped [1] into subfamilies. One of these, called class-II, currently consists of the following enzymes: - Glycine acetyltransferase (EC 2.3.1.29), which catalyzes the addition of acetyl-CoA to glycine to form 2-amino-3-oxobutanoate (gene kbl). - 5-aminolevulinic acid synthase (EC 2.3.1.37) (delta-ALA synthase), which catalyzes the first step in heme biosynthesis via the Shemin (or C4) pathway, i.e. the addition of succinyl-CoA to glycine to form 5- aminolevulinate. - 8-amino-7-oxononanoate synthase (EC 2.3.1.47) (7-KAP synthetase), a bacterial enzyme (gene bioF) which catalyzes an intermediate step in the biosynthesis of biotin: the addition of 6-carboxy-hexanoyl-CoA to alanine to form 8-amino-7-oxononanoate. - Histidinol-phosphate aminotransferase (EC 2.6.1.9), which catalyzes the eighth step in histidine biosynthetic pathway: the transfer of an amino group from 3-(imidazol-4-yl)-2-oxopropyl phosphate to glutamic acid to form histidinol phosphate and 2-oxoglutarate. - Serine palmitoyltransferase (EC 2.3.1.50) from yeast (genes LCB1 and LCB2), which catalyzes the condensation of palmitoyl-CoA and serine to form 3-ketosphinganine.The sequence around the pyridoxal-phosphate attachment site of this class of enzyme is sufficiently conserved to allow the creation of a specific pattern

Consensus pattern: T-[LIVMFYW]-[STAG]-K-[SAG]-[LIVMFYWR]-[SAG]-x(2)-[SAG] [K is the pyridoxal-P attachment site]-

[1] Bairoch A. Unpublished observations (1991).

52. Aminotransferases class-III pyridoxal-phosphate attachment site

Aminotransferases share certain mechanistic features with other pyridoxal- phosphate dependent enzymes, such as the covalent binding of the pyridoxal- phosphate group to a lysine residue. On the basis of sequence similarity, these various enzymes can be grouped [1,2] into subfamilies. One of these, called class-III, currently consists of the following enzymes: - Acetylornithine aminotransferase (EC 2.6.1.11) which catalyzes the transfer of an amino group from acetylornithine to alpha-ketoglutarate, yielding N-acetyl-glutamic-5-semi-aldehyde and glutamic acid. - Ornithine aminotransferase (EC 2.6.1.13), which catalyzes the transfer of an amino group from ornithine to alpha-ketoglutarate, yielding glutamic-5- semi-aldehyde and glutamic acid. - Omega-amino acid--pyruvate aminotransferase (EC 2.6.1.18), which catalyzes transamination between a variety of omega-amino acids, mono- and diamines, and pyruvate. It plays a pivotal role in omega amino acids metabolism. - 4-aminobutyrate aminotransferase (EC 2.6.1.19) (GABA transaminase), which catalyzes the transfer of an amino group from GABA to alpha-ketoglutarate, yielding succinate semialdehyde and glutamic acid. - DAPA aminotransferase (EC 2.6.1.62), a bacterial enzyme (gene bioA) which catalyzes an intermediate step in the biosynthesis of biotin, the transamination of 7-keto-8-aminopelargonic acid (7-KAP) to form 7,8- diaminopelargonic acid (DAPA). - 2,2-dialkylglycine decarboxylase (EC 4.1.1.64), a Pseudomonas cepacia enzyme (gene dgdA) that catalyzes the decarboxylating amino transfer of 2,2-dialkylglycine and pyruvate to dialkyl ketone, alanine and carbon dioxide. - Glutamate-1-semialdehyde aminotransferase (EC 5.4.3.8) (GSA). GSA is the enzyme involved in the second step of porphyrin biosynthesis, via the C5 pathway. It transfers the amino group on carbon 2 of glutamate-1- semialdehyde to the neighbouring carbon, to give delta-aminolevulinic acid. - Bacillus subtilis aminotransferase yhxA. - Bacillus subtilis aminotransferase yodT. - Haemophilus influenzae aminotransferase HI0949. - Caenorhabditis elegans aminotransferase TO1B11.2.The sequence around the pyridoxal-phosphate attachment site of this class ofenzyme is sufficiently conserved to allow the creation of a specific pattern.
Consensus pattern: [LIVMFYWC](2)-x-D-E-[IVA]-x(2)-G-[LIVMFAGC]-x(0,1)-[RSACLI]-x-[GSAD]-x(12,16)-D-[LIVMFC]-[LIVMFYSTA]-x(2)- [GSA]-K-x(3)-[GSTADNV]-[GSAC] [K is the pyridoxal-P attachment site]-

[1] Bairoch A. Unpublished observations (1992).
[2] Yonaha K., Nishie M., Aibara S. J. Biol. Chem. 267:12506-12510(1992).

53. Ank repeat. There's no clear separation between noise and signal on the HMM search Ankyrin repeats generally consist of a beta, alpha, alpha, beta order of secondary structures. The repeats associate to form a higher order structure.

[1] A, Holak TA, FEBS Lett 1997;401:127-132.
[2] Lux SE, John KM, Bennett V, Nature 1990;345:736-739.

54. Aminotransferases class-IV signature
Aminotransferases share certain mechanistic features with other pyridoxal-phosphate dependent enzymes, such as the covalent binding of the pyridoxal-phosphate group to a lysine residue. On the basis of sequence similarity, these various enzymes can be grouped [1,2] into subfamilies. One of these, called class-IV, currently consists of the following enzymes:

- Branched-chain amino-acid aminotransferase (EC 2.6.1.42) (transaminase B), a bacterial (gene ilvE) and eukaryotic enzyme which catalyzes the reversible transfer of an amino group from 4-methyl-2-oxopentanoate to glutamate, to form leucine and 2-oxoglutarate.
- D-alanine aminotransferase (EC 2.6.1.21). A bacterial enzyme which catalyzes the transfer of the amino group from D-alanine (and other D-amino acids) to 2-oxoglutarate, to form pyruvate and D-aspartate.
- 4-amino-4-deoxychorismate (ADC) lyase (gene pabC). A bacterial enzyme that converts ADC into 4-aminobenzoate (PABA) and pyruvate.

The above enzymes are proteins of about 270 to 415 amino-acid residues that share a few regions of sequence similarity. Surprisingly, the best-conserved region does not include the lysine residue to which the pyridoxal-phosphategroup is known to be attached, in ilvE. The region that has been selected as a signature pattern is located some 40 residues at the C-terminus side of the PIP-lysine
Consensus pattern: E-x-[STAGCI]-x(2)-N-[LIVMFAC]-[FY]-x(6,12)-[LIVMF]-x-T-x(6,8)-[LIVM]-x-[GS]-[LIVM]-x-[KR]-

[1] Green J.M., Merkel W.K., Nichols B.P. J. Bacteriol. 174:5317-5323(1992).
[2] Bairoch A. Unpublished observations (1992).

55. Aminotransferases class-V pyridoxal-phosphate attachment site
Aminotransferases share certain mechanistic features with other pyridoxal- phosphate dependent enzymes, such as the covalent binding of the pyridoxal- phosphate group to a lysine residue. On the basis of sequence similarity, these various enzymes can be grouped [1,2] into subfamilies. One of these, called class-V, currently consists of the following enzymes: - Phosphoserine aminotransferase (EC 2.6.1.52), an enzyme which catalyzes the reversible interconversion of phosphoserine and 2-oxoglutarate to 3-phosphonooxypyruvate and glutamate. It is required both in the major phosphorylated pathway of serine biosynthesis and in pyridoxine biosynthesis. The bacterial enzyme (gene serC) is highly similar to a rabbit endometrial progesterone-induced protein (EPIP), which is probably a phosphoserine aminotransferase [3]. - Serine--glyoxylate aminotransferase (EC 2.6.1.45) (SGAT) (gene sgaA) from Methylobacterium extorquens. - Serine--pyruvate aminotransferase (EC 2.6.1.51). This enzyme also acts as an alanine--glyoxylate aminotransferase (EC 2.6.1.44). In vertebrates, it is located in the peroxisomes and/or mitochondria. - Isopenicillin N epimerase (gene cefD). This enzyme is involved in the biosynthesis of cephalosporin

antibiotics and catalyzes the reversible isomerization of isopenicillin N and penicillin N. - NifS, a protein of the nitrogen fixation operon of some bacteria and cyanobacteria. The exact function of nifS is not yet known. A highly similar protein has been found in fungi (gene NFS1 or SPL1). - The small subunit of cyanobacterial soluble hydrogenase (EC 1.12.-.-). - Hypothetical protein ycbU from Bacillus subtilis. - Hypothetical protein YFL030w from yeast. The sequence around the pyridoxal-phosphate attachment site of this class of enzyme is sufficiently conserved to allow the creation of a specific pattern.
Consensus pattern: [LIVFYCHT]-[DGH]-[LIVMFYAC]-[LIVMFYA]-x(2)-[GSTAC]-[GSTA]- [HQR]-K-x(4,6)-G-x-[GSAT]-x-[LIVMFYSAC] [K is the pyridoxal-P attachment site]-

[1] Ouzounis C., Sander C. FEBS Lett. 322:159-164(1993).
[2] Bairoch A. Unpublished observations (1992).
[3] van der Zel A., Lam H.-M., Winkler M.E. Nucleic Acids Res. 17:8379-8379(1989).

56. Annexins repeated domain signature
Annexins [1 to 6] are a group of calcium-binding proteins that associate reversibly with membranes. They bind to phospholipid bilayers in the presence of micromolar free calcium concentration. The binding is specific for calcium and for acidic phospholipids. Annexins have been claimed to be involved in cytoskeletal interactions, phospholipase inhibition, intracellular signalling, anticoagulation, and membrane fusion. Each of these proteins consist of an N-terminal domain of variable length followed by four or eight copies of a conserved segment of sixty one residues. The repeat (sometimes known as an 'endonexin fold') consists of five alpha-helices that are wound into a right-handed superhelix [7].The proteins known to belong to the annexin family are listed below: - Annexin I (Lipocortin 1) (Calpactin 2) (p35) (Chromobindin 9). - Annexin II (Lipocortin 2) (Calpactin 1) (Protein I) (p36) (Chromobindin 8). - Annexin III (Lipocortin 3) (PAP-III). - Annexin IV (Lipocortin 4) (Endonexin I) (Protein II) (Chromobindin 4). - Annexin V (Lipocortin 5) (Endonexin 2) (VAC-alpha) (Anchorin CII) (PAP-I). - Annexin VI (Lipocortin 6) (Protein III) (Chromobindin 20) (p68) (p70). This is the only known annexin that contains 8 (instead of 4) repeats. - Annexin VII (Synexin). - Annexin VIII (Vascular anticoagulant-beta) (VAC-beta). - Annexin IX from Drosophila. - Annexin X from Drosophila. - Annexin XI (Calcyclin-associated annexin) (CAP-50). - Annexin XII from Hydra vulgaris. - Annexin XIII (Intestine-specific annexin) (ISA).The signature pattern for this domain spans positions 9 to 61 of the repeatand includes the only perfectly conserved residue (an arginine in position 22)-
Consensus pattern: [TG]-[STV]-x(8)-[LIVMF]-x(2)-R-x(3)-[DEQNH]-x(7)-[IFY]- x(7)-[LIVMF]-x(3)-[LIVMF]-x (11)-[LIVMFA]-x(2)-[LIVMF]-

[1] Raynal P., Pollard H.B. Biochim. Biophys. Acta 1197:63-93(1994).
[2] Barton G.J., Newman R.H., Freemont P.S., Crumpton M.J. Eur. J. Biochem. 198:749-760(1991).
[3] Burgoyne R.D., Geisow M.J. Cell Calcium 10:1-10(1989).
[4] Haigler H.T., Fitch J.M., Jones J.M., Schlaepfer D.D. Trends Biochem. Sci. 14:48-50(1989).
[5] Klee C.B. Biochemistry 27:6645-6653(1988).
[6] Smith P.D., Moss S.E. Trends Genet. 10:241-246(1994).
[7] Huber R., Roemisch J., Paques E.-P. EMBO J. 9:3867-3874(1990).
[8] Fiedler K., Simons K. Trends Biochem. Sci. 20:177-178(1995).

57. (arf_1) ADP-ribosylation factors family signature
ADP-ribosylation factors (ARF) [1,2,3,4] are 20 Kd GTP-binding proteins involved in protein trafficking. They may modulate vesicle budding and uncoating within the Golgi apparatus. ARF's also act as allosteric activators of cholera toxin ADP-ribosyltransferase activity. They are evolutionary conserved and present in all eukaryotes. At least six forms of ARF are present in mammals and three in budding yeast. The ARF family also includes proteins highly related to ARF's but which lack the cholera toxin cofactor activity, they are collectively known as ARL's (ARF-like).ARD1 is a 64 Kd mammalian protein of unknown biological function that contains an ARF domain at its C-terminal extremity. Proteins from the ARF family are generally included in the RAS 'superfamily' of small GTP-binding proteins [5], but they are only slightly related to the other RAS proteins. They also differ from RAS proteins in that they lack cysteine residues at their C-termini and are therefore not subject to prenylation. The ARFs are N-terminally myristoylated (the ARLs have not yet been shown to be modified in such a fashion). A conserved region in the C-terminal part of ARF's and ARL's has been selected as a signature pattern.
Consensus pattern: [HRQT]-x-[FYWI]-x-[LIVM]-x(4)-A-x(2)-G-x(2)-[LIVM]-x(2)-[GSA]-[LIVMF]-x-[WK]-[LIVM]-
Note: proteins belonging to this family also contain a copy of the ATP/GTP- binding motif 'A' (P-loop) (see <PDOC00017

[1] Boman A.L., Kahn R.A. Trends Biochem. Sci. 20:147-150(1995).

[2] Moss J., Vaughan M. Cell. Signal. 4.367-399(1993).
[3] Moss J., Vaughan M. Prog. Nucleic Acid Res. Mol. Biol. 45:47-65(1993).
[4] Amor J.C., Harrison D.H., Kahn R.A., Ringe D. Nature 372:704-708(1994).
[5] Valencia A., Chardin P., Wittinghofer A., Sander C. Biochemistry 30:4637-4648(1991).

(arf_2) ATP/GTP-binding site motif A (P-loop)

From sequence comparisons and crystallographic data analysis it has been shown [1,2,3,4,5,6] that an appreciable proportion of proteins that bind ATP or GTP share a number of more or less conserved sequence motifs. The best conserved of these motifs is a glycine-rich region, which typically forms a flexible loop between a beta-strand and an alpha-helix. This loop interacts with one of the phosphate groups of the nucleotide. This sequence motif is generally referred to as the 'A' consensus sequence [1] or the 'P-loop' [5].There are numerous ATP- or GTP-binding proteins in which the P-loop is found. A number of protein families for which the relevance of the presence of such motif has been noted are listed below: - ATP synthase alpha and beta subunits (see <PDOC00137>).-Myosin heavy chains. - Kinesin heavy chains and kinesin-like proteins (see <PDOC00343>). - Dynamins and dynamin-like proteins (see <PDOC00362>). - Guanylate kinase (see <PDOC00670>). - Thymidine kinase (see <PDOC00524>). - Thymidylate kinase (see <PDOC01034>). - Shikimate kinase (see <PDOC00868>). - Nitrogenase iron protein family (nifH/frxC) (see <PDOC00580>). - ATP-binding proteins involved in 'active transport' (ABC transporters) [7] (see <PDOC00185>). - DNA and RNA helicases [8,9,10]. - GTP-binding elongation factors (EF-Tu, EF-1 alpha, EF-G, EF-2, etc.). - Ras family of GTP-binding proteins (Ras, Rho, Rab, Ral, Ypt1, SEC4, etc.). - Nuclear protein ran (see <PDOC00859>). - ADP-ribosylation factors family (see <PDOC00781>). - Bacterial dnaA protein (see <PDOC00771>). - Bacterial recA protein (see <PDOC00131>). - Bacterial recF protein (see <PDOC00539>). - Guanine nucleotide-binding proteins alpha subunits (Gi, Gs, Gt, G0, etc.). - DNA mismatch repair proteins mutS family (See <PDOC00388>). - Bacterial type II secretion system protein E (see <PDOC00567>).Not all ATP- or GTP-binding proteins are picked-up by this motif. A number of proteins escape detection because the structure of their ATP-binding site is completely different from that of the P-loop. Examples of such proteins are the E1-E2 ATPases or the glycolytic kinases. In other ATP-or GTP-binding proteins the flexible loop exists in a slightly different form; this is the case for tubulins or protein kinases. A special mention must be reserved for adenylate kinase, in which there is a single deviation from the P-loop pattern: in the last position Gly is found instead of Ser or Thr.

Consensus pattern: [AG]-x(4)-G-K-[ST]-

[1] Walker J.E., Saraste M., Runswick M.J., Gay N.J. EMBO J. 1:945-951(1982).
[2] Moller W., Amons R. FEBS Lett. 186:1-7(1985).
[3] Fry D.C., Kuby S.A., Mildvan A.S. Proc. Natl. Acad. Sci. U.S.A. 83:907-911(1986).
[4] Dever T.E., Glynias M.J., Merrick W.C. Proc. Natl. Acad. Sci. U.S.A. 84:1814-1818(1987).
[5] Saraste M., Sibbald P.R., Wittinghofer A. Trends Biochem. Sci. 15:430-434(1990).
[6] Koonin E.V. J. Mol. Biol. 229:1165-1174(1993).
[7] Higgins C.F., Hyde S.C., Mimmack M.M., Gileadi U., Gill D.R., Gallagher M.P. J. Bioenerg. Biomembr. 22:571-592(1990).
[8] Hodgman T.C. Nature 333:22-23(1988) and Nature 333:578-578(1988) (Errata).
[9] Linder P., Lasko P., Ashburner M., Leroy P., Nielsen P.J., Nishi K., Schnier J., Slonimski P.P. Nature 337:121-122(1989).
[10] Gorbalenya A.E., Koonin E.V., Donchenko A.P., Blinov V.M. Nucleic Acids Res. 17:4713-4730(1989).

58. Arginase family signatures

The following enzymes have been shown [1] to be evolutionary related: - Arginase (EC 3.5.3.1), a ubiquitous enzyme which catalyzes the degradation of arginine to ornithine and urea [2]. - Agmatinase (EC 3.5.3.11) (agmatine ureohydrolase), a prokaryotic enzyme (gene speB) that catalyzes the hydrolysis of agmatine into putrescine and urea. - Formiminoglutamase (EC 3.5.3.8) (formiminoglutamate hydrolase), a prokaryotic enzyme (gene hutG) that hydrolyzes N-formimino-glutamate into glutamate and formamide. - Hypothetical proteins from methanogenic archaebacteria. These enzymes are proteins of about 300 amino-acid residues. Three conserved regions that contain charged residues which are involved in the binding of the two manganese ions [3] can be used as signature patterns.-

Consensus pattern: [LIVMF]-G-G-x-H-x-[LIVMT]-[STAV]-x-[PAG]-x(3)-[GSTA] [H binds manganese]-

Consensus pattern: [LIVM](2)-x-[LIVMFY]-D-[AS]-H-x-D [The two D's and the H bind manganese]-

Consensus pattern: [ST]-[LIVMFY]-D-[LIVM]-D-x(3)-[PAQ]-x(3)-P-[GSA]-x(7)-G [The two D's bind manganese]

[1] Ouzounis C., Kyrpides N.C. J. Mol. Evol. 39:101-104(1994).
[2] Jenkinson C.P., Grody W.W., Cederbaum S.D. Comp. Biochem. Physiol. 114B:107-132(196).
[3] Kanyo Z.F., Scolnick L.R., Ash D.E., Christianson D.W. Nature 383:554-557(1996).

59. (asp) Eukaryotic and viral aspartyl proteases active site

Aspartyl proteases, also known as acid proteases, (EC 3.4.23.-) are a widely distributed family of proteolytic enzymes [1,2,3] known to exist invertebrates, fungi, plants, retroviruses and some plant viruses. Aspartate proteases of eukaryotes are monomeric enzymes which consist of two domains. Each domain contains an active site centered on a catalytic aspartyl residue.The two domains most probably evolved from the duplication of an ancestral gene encoding a primordial domain. Currently known eukaryotic aspartyl proteases are: - Vertebrate gastric pepsins A and C (also known as gastricsin). - Vertebrate chymosin (rennin), involved in digestion and used for making cheese. - Vertebrate lysosomal cathepsins D (EC 3.4.23.5) and E (EC 3.4.23.34). - Mammalian renin (EC 3.4.23.15) whose function is to generate angiotensin I from angiotensinogen in the plasma. - Fungal proteases such as aspergillopepsin A (EC 3.4.23.18), candidapepsin (EC 3.4.23.24), mucoropepsin (EC 3.4.23.23) (mucor rennin), endothiapepsin (EC 3.4.23.22), polyporopepsin (EC 3.4.23.29), and rhizopuspepsin (EC 3.4.23.21). - Yeast saccharopepsin (EC 3.4.23.25) (proteinase A) (gene PEP4). PEP4 is implicated in posttranslational regulation of vacuolar hydrolases. - Yeast barrier pepsin (EC 3.4.23.35) (gene BAR1); a protease that cleaves alpha-factor and thus acts as an antagonist of the mating pheromone. - Fission yeast sxa1 which is involved in degrading or processing the mating pheromones. Most retroviruses and some plant viruses, such as badnaviruses, encode for anaspartyl protease which is an homodimer of a chain of about 95 to 125 amino acids. In most retroviruses, the protease is encoded as a segment of apolyprotein which is cleaved during the maturation process of the virus. It is generally part of the pol polyprotein and, more rarely, of the gagpolyprotein. Conservation of the sequence around the two aspartates of eukaryotic aspartyl proteases and around the single active site of the viral proteases allows us to develop a single signature pattern for both groups of protease.

Consensus pattern: [LIVMFGAC]-[LIVMTADN]-[LIVFSA]-D-[ST]-G-[STAV]-[STAPDENQ]- x-[LIVMFSTNC]-x-[LIVMFGTA] [D is the active site residue]

Note: these proteins belong to families A1 and A2 in the classification of peptidases [4,E1

[1] Foltmann B. Essays Biochem. 17:52-84(1981).
[2] Davies D.R. Annu. Rev. Biophys. Chem. 19:189-215(1990).
[3] Rao J.K.M., Erickson J.W., Wlodawer A. Biochemistry 30:4663-4671(1991).
[4] Rawlings N.D., Barrett A.J. Meth. Enzymol. 248:105-120(1995).

60. (BIRA) Biotin repressor

[1] Wilson KP, Shewchuk LM, Brennan RG, Otsuka AJ, Matthews BW; Proc Natl Acad Sci USA 1992;89: 9257-9261.

61. BTB/POZ domain

The BTB (for BR-C, ttk and bab) [1] or POZ (for Pox virus and Zinc finger) [2] domain is present near the N-terminus of a fraction of zinc finger
(zf-C2H2) proteins and in proteins that contain the Kelch motif
such as Kelch and a family of pox virus proteins. The BTB/POZ domain mediates homomeric dimerisation and in some instances heteromeric dimerisation [2].The structure of the dimerised PLZF BTB/POZ domain has been solved and consists of a tightly intertwined homodimer. The central scaffolding of the protein is made up of a cluster of alpha-helices flanked by short beta-sheets at both the top and bottom of the molecule [3]. POZ domains from several zinc finger proteins have been shown to mediate transcriptional repression and to interact with components of histone deacetylase co-repressor complexes including N-CoR and SMRT [4,5,6]. The POZ or BTB domain is also known as BR-C/Ttk or ZiN

[1] Zollman S, Godt D, Prive GG, Couderc JL, Laski FA; Proc Natl Acad Sci U S A 1994;91:10717-10721.
[2]Bardwell VJ, Treisman R; Genes Dev 1994;8:1664-1677.
[3] Ahmad KF, Engel CK, Prive GG; Proc Natl Acad Sci U S A 1998;95:12123-12128.
[4] Deweindt C, Albagli O, Bernardin F, Dhordain P, Quief S, Lantoine D, Kerckaert JP, Leprince D; Cell Growth Differ 1995;6:1495-1503.
[5] Huynh KD, Bardwell VJ; Oncogene 1998;17:2473-2484.
[6] Wong CW, Privalsky ML; J Biol Chem 1998;273:27695-27702.

62. (Bac GSPproteins) Bacterial type II secretion system protein D signature

A number of bacterial proteins, some of which are involved in a general secretion pathway (GSP) for the export of proteins (also called the type II pathway) [1 to 5], have been found to be evolutionary related. These proteins are listed below: - The 'D'protein from the GSP operon of: Aeromonas (gene exeD); Erwinia (gene outD); Escherichia

coli (gene yheF), Klebsiella pneumoniae (gene pulD); Pseudomonas aeruginosa (gene xcpQ); Vibrio cholerae (gene epsD) and Xanthomonas campestris (gene xpsD). - comE from Haemophilus influenzae, involved in competence (DNA uptake). - pilQ from Pseudomonas aeruginosa, which is essential for the formation of the pili. - hofQ (hopQ) from Escherichia coli. - hrpH from Pseudomonas syringae, which is involved in the secretion of a proteinaceous elicitor of the hypersensitivity response in plants. - hrpA1 from Xanthomonas campestris pv. vesicatoria, which is also involved in the hypersensitivity response. - mxiD from Shigella flexneri which is involved in the secretion of the Ipa invasins which are necessary for penetration of intestinal epithelial cells. - omc from Neisseria gonorrhoeae. - yssC from Yersinia enterocolitica virulence plasmid pYV, which seems to be required for the export of the Yop virulence proteins. - The gpIV protein from filamentous phages such as f1, ike, or m13. GpIV is said to be involved in phage assembly and morphogenesis. These proteins all seem to start with a signal sequence and are thought to be integral proteins in the outer membrane. As a signature pattern a conserved region in the C-terminal section of these proteins has been selected
Consensus pattern: [GR]-[DEQKG]-[STVM]-[LIVMA](3)-[GA]-G-[LIVMFY]-x(11)-[LIVM]-P-[LIVMFYWGS]-[LIVMF]-[GSAE]-x-[LIVM]-P- [LIVMFYW](2)-x(2)-[LV]-F

[1] Salmond G.P.C., Reeves P.J. Trends Biochem. Sci. 18:7-12(1993).
[2] Reeves P.J., Whitcombe D., Wharam S., Gibson M., Allison G., Bunce N., Barallon R., Douglas P., Mulholland V., Stevens S., Walker S., Salmond G.P.C. Mol. Microbiol. 8:443-456(1993).
[3] Martin P.R., Hobbs M., Free P.D., Jeske Y., Mattick J.S. Mol. Microbiol. 9:857-868(1993).
[4] Hobbs M., Mattick J.S. Mol. Microbiol. 10:233-243(1993).
[5] Genin S., Boucher C.A. Mol. Gen. Genet. 243:112-118(1994).

63. (Bac globin) Protozoan/cyanobacterial globins signature
Globins are heme-containing proteins involved in binding and/or transporting oxygen [1]. Almost all globins belong to a large family (see <PDOC00793>), the only exceptions are the following proteins which form a family of their own[2,3]: - Monomeric hemoglobins from the protozoan Paramecium caudatum, Tetrahymena pyriformis and Tetrahymena thermophila. - Cyanoglobin from the cyanobacteria Nostoc commune. - Globins LI637 and LI410 from the chloroplast of the alga Chlamydomonas eugametos. - Mycobacterium tuberculosis hypothetical protein MtCY48.23.These proteins contain a conserved histidine which could be involved in heme-binding. As a signature pattern, a conserved region that ends with this residue was used
Consensus pattern: F-[LF]-x(5)-G-[PA]-x(4)-G-[KRA]-x-[LIVM]-x(3)-H-

[1] Concise Encyclopedia Biochemistry, Second Edition, Walter de Gruyter, Berlin New-York (1988).
[2] Takagi T. Curr. Opin. Struct. Biol. 3:413-418(1993).
[3] Couture M., Chamberland H., St-Pierre B., Lafontaine J., Guertin M.; Mol. Gen. Genet. 243:185-197(1994).

64. Band 7 protein family signature
Mammalian band 7 protein [1] (also known as 7.2B or stomatin) is an integral membrane phosphoprotein of red blood cells thought to regulate cation conductance by interacting with other proteins of the junctional complex of the membrane skeleton. Structurally, band 7 is evolutionary related to the following proteins: - Caenorhabditis elegans protein mec-2 [2]. Mec-2 positively regulates the activity of the putative mechanosensory transduction channel. It may links the mechanosensory channel and the microtubule cytoskeleton of the touch receptor neurons. - Caenorhabditis elegans proteins sto-1 to sto-4. - Caenorhabditis elegans protein unc-1. - Escherichia coli hypothetical protein ybbK. - Mycobacterium tuberculosis hypothetical protein MtCY277.09. - Synechocystis strain PCC 6803 hypothetical protein s1r1128. - Methanococcus jannaschii hypothetical protein MJ0827. Structurally all these proteins consist of a short N-terminal domain which is followed by a transmembrane region and a variable size (from 170 to 350residues) C-terminal domain .As a signature pattern, a conserved region located about 110residues after the transmembrane domain was selected
Consensus pattern: R-x(2)-[LIV]-[SAN]-x(6)-[LIV]-D-x(2)-T-x(2)-W-G-[LIV]- [KRH]-[LIV]-x-[KR]-[LIV]-E-[LIV]-[KR]-

[1] Gallagher P.G., Forget B.G. J. Biol. Chem. 270:26358-26363(1995).
[2] Huang M., Gu G., Ferguson E.L., Chalfie M. Nature 378:292-295(1995).

65. Barwin domain signatures
Barwin [1] is a barley seed protein of 125 residues that binds weakly a chitinanalog. It contains six cysteines involved in disulfide bonds, as shown in the following schematic representation.
+---------------+ |*****| ****
xxxxxxxxxxxxxxxCxxxxxxxxxxCxxxxCxCxxxxxxxxCxxxxxxxxxxxxxxxxxxCx |||| +-----------+ +--------------------------

+'C': conserved cysteine involved in a disulfide bond.'*': position of the patterns. Barwin is closely related to the following proteins: - Hevein, a wound-induced protein found in the latex of rubber trees. - HEL, an Arabidopsis thaliana hevein-like protein [2]. - Win1 and win2, two wound-induced proteins from potato. - Pathogenesis-related protein 4 from tobacco. Hevein and the win1/2 proteins consist of an N-terminal chitin-binding domain followed by a barwin-like C-terminal domain. Barwin and its related proteins could be involved in a defense mechanism in plants. As signature patterns, two highly conserved regions that contain some of the cysteines were selected
Consensus pattern: C-G-[KR]-C-L-x-V-x-N [The two C's are involved in disulfide bonds]-
Consensus pattern: V-[DN]-Y-[EQ]-F-V-[DN]-C [C is involved in a disulfide bond]-

[1] Svensson B., Svendsen I., Hoejrup P., Roepstorff P., Ludvigsen S., Poulsen F.M. Biochemistry 31:8767-8770 (1992).
[2] Potter S., Uknes S., Lawton K., Winter A.M., Chandler D., Dimaio J., Novitzky R., Ward E., Ryals J. Mol. Plant Microbe Interact. 6:680-685(1993).

66. (Bowman-Birk leg) Bowman-Birk serine protease inhibitors family signature PROSITE cross-reference(s). The Bowman-Birk inhibitor family [1] is one of the numerous families of serine proteinase inhibitors. As it can be seen in the schematic representation, they have a duplicated structure and generally possess two distinct inhibitory sites:

```
 +----------------------------------------------+
 |    +-----+     +-------+   +-----+       |
 |    |   |    |     |  |   |     |
xxCCxxCxxCxx#xxCxxCxxxxCxxxCxxxCxxxxCxx#xxCxxCxxCxxCxx
  | |        |********|****           | |
  | |        |     |           | |
  +--|----------+     +----------------+ |
    +----------------------------------------+

 <-----------------70 residues-------------------->
```

'C': conserved cysteine involved in a disulfide bond.
'#': active site residue.
'*': position of the pattern.
These inhibitors are found in the seeds of all leguminous plants as well as in cereal grains. In cereals they exist in two forms, one of which is a duplication of the basic structure shown above [2]. The pattern that was developed to pick up sequences belonging to this family of inhibitors is in the central part of the domain and includes four cysteines. Consensus pattern C-x(5,6)-[DENQKRHSTA]-C-[PASTDH]-[PASTDK]-[ASTDV]-C-[NDKS]-[DEKRHSTA]-C [The four C's are involved in disulfide bonds] Note this pattern can be found twice in some duplicated cereal inhibitors.

[1] Laskowski M., Kato I. Annu. Rev. Biochem. 49:593-626(1980).
[2] Tashiro M., Hashino K., Shiozaki M., Ibuki F., Maki Z. J. Biochem. 102:297-306(1987).

67. Pathogenesis-related protein Bet v I family signature
A number of plant proteins, which all seem to be involved in pathogen defense response, are structurally related [1,2,3]. These proteins are:

- Bet v I, the major pollen allergen from white birch. Bet v I is the main cause of type I allergic reactions in Europe, North America and USSR.
- Aln g I, the major pollen allergen from alder.
- Api G I, the major allergen from celery.
- Car b I, the major pollen allergen from hornbeam.

- Cor a I, the major pollen allergen from hazel.
- Mal d I, the major pollen allergen from apple.
- Asparagus wound-induced protein AoPR1.
- Kidney bean pathogenesis-related proteins 1 and 2.
- Parsley pathogenesis-related proteins PR1-1 and PR1-3.
- Pea disease resistance response proteins pI49, pI176 and DRRG49-C.
- Pea abscisic acid-responsive proteins ABR17 and ABR18.
- Potato pathogenesis-related proteins STH-2 and STH-21.
- Soybean stress-induced protein SAM22.

These proteins are thought to be intracellularly located. They contain from 155 to 160 amino acid residues. As a signature pattern, a conserved region located in the third quarter of these proteins has been selected
Consensus pattern: G-x(2)-[LIVMF]-x(4)-E-x(2)-[CSTAEN]-x(8,9)-[GND]-G-[GS]- [CS]-x(2)-K-x(4)-[FY]-

[1] Breiteneder H., Pettenburger K., Bito A., Valenta R., Kraft D., Rumpold H., Scheiner O., Breitenbach M. EMBO J. 8:1935-1938(1989).
[2] Crowell D., John M.E., Russell D., Amasino R.M. Plant Mol. Biol. 18:459-466(1992).
[3] Warner S.A.J., Scott R., Draper J. Plant Mol. Biol. 19:555-561(1992).

68. bZIP transcription factors basic domain signature
The bZIP superfamily [1,2,] of eukaryotic DNA-binding transcription factors groups together proteins that contain a basic region mediating sequence-specific DNA-binding followed by a leucine zipper required for dimerization. This family is quite large, therefore only a parital list of some representative members appears here. - Transcription factor AP-1, which binds selectively to enhancer elements in the cis control regions of SV40 and metallothionein IIA. AP-1, also known as c-jun, is the cellular homolog of the avian sarcoma virus 17 (ASV17) oncogene v-jun. - Jun-B and jun-D, probable transcription factors which are highly similar to jun/AP-1. - The fos protein, a proto-oncogene that forms a non-covalent dimer with c-jun. - The fos-related proteins fra-1, and fos B. - Mammalian cAMP response element (CRE) binding proteins CREB, CREM, ATF-1, ATF-3, ATF-4, ATF-5, ATF-6 and LRF-1. - Maize Opaque 2, a trans-acting transcriptional activator involved in the regulation of the production of zein proteins during endosperm. - Arabidopsis G-box binding factors GBF1 to GBF4, Parsley CPRF-1 to CPRF-3, Tobacco TAF-1 and wheat EMBP-1. All these proteins bind the G-box promoter elements of many plant genes. - Drosophila protein Giant, which represses the expression of both the kruppel and knirps segmentation gap genes. - Drosophila Box B binding factor 2 (BBF-2), a transcriptional activator that binds to fat body-specific enhancers of alcohol dehydrogenase and yolk protein genes. - Drosophila segmentation protein cap'n'collar (gene cnc), which is involved in head morphogenesis. - Caenorhabditis elegans skn-1, a developmental protein involved in the fate of ventral blastomeres in the early embryo. - Yeast GCN4 transcription factor, a component of the general control system that regulates the expression of amino acid-synthesizing enzymes in response to amino acid starvation, and the related Neurospora crassa cpc-1 protein. - Neurospora crassa cys-3 which turns on the expression of structural genes which encode sulfur-catabolic enzymes. - Yeast MET28, a transcriptional activator of sulfur amino acids metabolism. - Yeast PDR4 (or YAP1), a transcriptional activator of the genes for some oxygen detoxification enzymes. - Epstein-Barr virus trans-activator protein BZLF1.-
Consensus pattern: [KR]-x(1,3)-[RKSAQ]-N-x(2)-[SAQ](2)-x-[RKTAENQ]-x-R-x-[RK]-

[1] Hurst H.C. Protein Prof. 2:105-168(1995).
[2] Ellenberger T. Curr. Opin. Struct. Biol. 4:12-21(1994).

69. Biotin-requiring enzymes attachment site
Biotin, which plays a catalytic role in some carboxyl transfer reactions, is covalently attached, via an amide bond, to a lysine residue in enzymes requiring this coenzyme [1,2,3,4]. Such enzymes are:

- Pyruvate carboxylase (EC 6.4.1.1).
- Acetyl-CoA carboxylase (EC 6.4.1.2).
- Propionyl-CoA carboxylase (EC 6.4.1.3).
- Methylcrotonoyl-CoA carboxylase (EC 6.4.1.4).
- Geranoyl-CoA carboxylase (EC 6.4.1.5).
- Urea carboxylase (EC 6.3.4.6).
- Oxaloacetate decarboxylase (EC 4.1.1.3).
- Methylmalonyl-CoA decarboxylase (EC 4.1.1.41).

- Glutaconyl-CoA decarboxylase (EC 4.1.1.70).
- Methylmalonyl-CoA carboxyl-transferase (EC 2.1.3.1) (transcarboxylase).

Sequence data reveal that the region around the biocytin (biotin-lysine) residue is well conserved and can be used as a signature pattern.
Consensus pattern[GN]-[DEQTR]-x-[LIVMFY]-x(2)-[LIVM]-x-[AIV]-M-K-[LMAT]-x(3)-[LIVM]-x-[SAV] [K is the biotin attachment site] Note the domain around the biotin-binding lysine residue is evolutionary related to that around the lipoyl-binding lysine residue of 2-oxo acid dehydrogenase acyltransferases

[1] Knowles J.R. Annu. Rev. Biochem. 58:195-221(1989).
[2] Samols D., Thronton C.G., Murtif V.L., Kumar G.K., Haase F.C., Wood H.G. J. Biol. Chem. 263:6461-6464 (1988).
[3] Goss N.H., Wood H.G. Meth. Enzymol. 107:261-278(1984).
[4] Shenoy B.C., Xie Y., Park V.L., Kumar G.K., Beegen H., Wood H.G., Samols D. J. Biol. Chem. 267: 18407-18412(1992).

2-oxo acid dehydrogenases acyltransferase component lipoyl binding site
The 2-oxo acid dehydrogenase multienzyme complexes [1,2] from bacterial and eukaryotic sources catalyze the oxidative decarboxylation of 2-oxo acids to the corresponding acyl-CoA. The three members of this family of multienzyme complexes are:

- Pyruvate dehydrogenase complex (PDC).
- 2-oxoglutarate dehydrogenase complex (OGDC).
- Branched-chain 2-oxo acid dehydrogenase complex (BCOADC).

These three complexes share a common architecture: they are composed of multiple copies of three component enzymes - E1, E2 and E3. E1 is a thiamine pyrophosphate-dependent 2-oxo acid dehydrogenase, E2 a dihydrolipamide acyltransferase, and E3 an FAD-containing dihydrolipamide dehydrogenase. E2 acyltransferases have an essential cofactor, lipoic acid, which is covalently bound via a amide linkage to a lysine group. The E2 components of OGCD and BCOACD bind a single lipoyl group, while those of PDC bind either one (in yeast and in Bacillus), two (in mammals), or three (in Azotobacter and in Escherichia coli) lipoyl groups [3].
In addition to the E2 components of the three enzymatic complexes described above, a lipoic acid cofactor is also found in the following proteins:

- H-protein of the glycine cleavage system (GCS) [4]. GCS is a multienzyme complex of four protein components, which catalyzes the degradation of glycine. H protein shuttles the methylamine group of glycine from the P protein to the T protein. H-protein from either prokaryotes or eukaryotes binds a single lipoic group.
- Mammalian and yeast pyruvate dehydrogenase complexes differ from that of other sources, in that they contain, in small amounts, a protein of unknown function - designated protein X or component X. Its sequence is closely related to that of E2 subunits and seems to bind a lipoic group [5].
- Fast migrating protein (FMP) (gene acoC) from Alcaligenes eutrophus [6]. This protein is most probably a dihydrolipamide acyltransferase involved in acetoin metabolism.

A signature pattern was developed which allows the detection of the lipoyl-binding site.
Consensus pattern[GN]-x(2)-[LIVF]-x(5)-[LIVFC]-x(2)-[LIVFA]-x(3)-K-[STAIV]-[STAVQDN]-x(2)-[LIVMFS]-x(5)-[GCN]-x-[LIVMFY] [K is the lipoyl-binding site] Note the domain around the lipoyl-binding lysine residue is evolutionary related to that around the biotin-binding lysine residue of biotin requiring enzymes

[1] Yeaman S.J. Biochem. J. 257:625-632(1989).
[2] Yeaman S.J. Trends Biochem. Sci. 11:293-296(1986).
[3] Russel G.C., Guest J.R. Biochim. Biophys. Acta 1076:225-232(1991).
[4] Fujiwara K., Okamura-Ikeda K., Motokawa Y. J. Biol. Chem. 261:8836-8841(1986).
[5] Behal R.H., Browning K.S., Hall T.B., Reed L.J. Proc. Natl. Acad. Sci. U.S.A. 86:8732-8736(1989).
[6] Priefert H., Hein S., Krueger N., Zeh K., Schmidt B., Steinbuechel A. J. Bacteriol. 173:4056-4071(1991).

70. C2 (C2 domain) Number of members: 295
Some isozymes of protein kinase C (PKC) [1,2] contain a domain, known as C2, of about 116 amino-acid residues which is located between the two copies of the C1 domain (that bind phorbol esters and diacylglycerol) (see

<PDOC00379>) and the protein kinase catalytic domain (see <PDOC00100>). Regions with significant homology [3,E1] to the C2-domain have been found in the following proteins:

- PKC isoforms alpha, beta and gamma and Drosophila isoforms PKC1 and PKC2.
- PKC isoforms delta, epsilon and eta, Caenorhabditis elegans kin-13 and yeast PKC1 have a C2-like domain at the N-terminal extremity [4].
- Yeast cAMP dependent protein kinase SCH9 contains a C2-like domain.
- Mammalian phosphatidylinositol-specific phospholipase C (PI-PLC) (see <PDOC50007>) isoforms beta, gamma and delta as well as several non-mammalian PI-PLCs have a C2-like domain C-terminal of the catalytic domain.
- Mammalian and plants phosphatidylinositol-3-kinase have a C2-like domain in the central region of the 110 Kd catalytic subunit.
- Yeast phosphatidylserine-decarboxylase 2 (gene PSD2) contains a C2 domain in its central region.
- Cytosolic phospholipase D from plants and cytosolic phospholipase A2 have a C2-like domain at their N-terminus.
- Synaptotagmins (p65). This is a family of related synaptic vesicle proteins that bind acidic phospholipids and that may have a regulatory role in the membrane interactions during trafficking of synaptic vesicles at the active zone of the synapse. All isoforms of synaptotagmins have two copies of the C2 domain in their C-terminal region.
- Rabphilin-3A, a synaptic protein contains two C2 domains.
- Caenorhabditis elegans protein unc-13 whose function is not known. Unc-13 has a C2 domain in its central part and a C2-like domain at the C-terminus.
- rasGAP and the breakpoint cluster protein bcr have a C2-domain C-terminal of a PH-domain.
- Yeast protein BUD2 (or CLA2) has a C2-domain in the central region.
- Yeast protein RSP5 and human protein NEDD-4, both proteins also contain WW domains (see <PDOC50020>).
- Perforin (see <PDOC00251>) has a C2 domain at the C-terminus. It is the only extracellular protein known to contain a C2 domain.
- Yeast hypothetical protein YML072C has a C2 domain.
- Yeast hypothetical protein YNL087W has three C2 domains.
- Caenorhabditis elegans hypothetical protein F37A4.7 has two C2 domains.

The C2 domain is thought to be involved in calcium-dependent phospholipid binding [5]. Since domains related to the C2 domain are also found in proteins that do not bind calcium, other putative functions for the C2 domain like e.g. binding to inositol-1,3,4,5-tetraphosphate have been suggested [6]. Recently, the 3D structure of the first C2 domain of synaptotagmin has been reported [7], the domain forms an eight-stranded beta sandwich. The signature pattern that has been developed for the C2 domain is located in a conserved part of that domain, the connecting loop between beta strands 2 and 3. A profile has been developed for the C2 domain that covers the total domain.

- Consensus pattern: [ACG]-x(2)-L-x(2,3)-D-x(1,2)-[NGSTLIF]-[GTMR]-x-[STAP]-D-[PA]-[FY]
- Note: this documentation entry is linked to both a signature pattern and a profile. As the profile is much more sensitive than the pattern, you should use it if you have access to the necessary software tools to do so.

[1]Medline: 96367095 Extending the C2 domain family: C2s in PKCs delta, epsilon, eta and theta, phospholipases, GAPs and perforin. Ponting CP, Parker PJ; Protein Sci 1996;5:162-166.
[1] Azzi A., Boscoboinik D., Hensey C. Eur. J. Biochem. 208:547-557(1992).
[2] Stabel S. Semin. Cancer Biol. 5:277-284(1994).
[3] Brose N., Hofmann K.O., Hata Y., Suedhof T.C. J. Biol. Chem. 270:25273-25280(1995).
[4] Sossin W.S., Schwartz J.H. Trends Biochem. Sci. 18:207-208(1993).
[5] Davletov B.A., Suedhof T.C. J. Biol. Chem. 268:26386-26390(1993).
[6] Fukuda M., Aruga J., Niinobe M., Aimoto S., Mikoshiba K. J. Biol. Chem. 269:29206-29211(1994).
[6] Sutton R.B., Davletov B.A., Berghuis A.M., Suedhof T.C., Sprang S.R. Cell 80:929-938(1995).

71. CAP (CAP protein) Number of members: 11

In budding and fission yeasts the CAP protein is a bifunctional protein whose N-terminal domain binds to adenylyl cyclase, thereby enabling that enzyme to be activated by upstream regulatory signals, such as Ras. The function of the C-terminal domain is less clear, but it is required for normal cellular morphology and growth control [1]. CAP is conserved in higher eukaryotic organisms where its function is not yet clear [2].

Structurally, CAP is a protein of 474 to 551 residues which consist of two domains separated by a proline-rich hinge. Two signature patterns, one corresponding to a conserved region in the N-terminal extremity and the other to a C-terminal region have been developed.

- Consensus pattern: [LIVM](2)-x-R-L-[DE]-x(4)-R-L-E
- Consensus pattern: D-[LIVMFY]-x-E-x-[PA]-x-P-E-Q-[LIVMFY]-K

[1] Kawamukai M., Gerst J., Field J., Riggs M., Rodgers L., Wigler M., Young D. Mol. Biol. Cell 3:167-180(1992).
[2] Yu G., Swiston J., Young D. J. Cell Sci. 107:1671-1678(1994).

72. CAP_GLY (CAP-Gly domain)

CAP stands for cytoskeleton-associated proteins. Swiss:P39937 may be a member but has not been included. It has a weak match to the family between residues 22-67. Number of members: 24

[1]Medline: 93242656. Sequence homologies between four cytoskeleton-associated proteins. Riehemann K, Sorg C; Trends Biochem Sci 1993;18:82-83.

It has been shown [1] that some cytoskeleton-associated proteins (CAP) share the presence of a conserved, glycine-rich domain of about 42 residues, called here CAP-Gly. Proteins known to contain this domain are listed below.

- Restin (also known as cytoplasmic linker protein-170 or CLIP-170), a 160 Kd protein associated with intermediate filaments and that links endocytic vesicles to microtubules. Restin contains two copies of the CAP-Gly domain.
- Vertebrate dynactin (150 Kd dynein-associated polypeptide; DAP) and Drosophila glued, a major component of activator I, a 20S polypeptide complex that stimulates dynein-mediated vesicle transport.
- Yeast protein BIK1 which seems to be required for the formation or stabilization of microtubules during mitosis and for spindle pole body fusion during conjugation.
- Yeast protein NIP100 (NIP80).
- Human protein CKAP1/TFCB, Schizosaccharomyces pombe protein alp11 and Caenorhabditis elegans hypothetical protein F53F4.3. These proteins contain a N-terminal ubiquitin domain (see <PDOC00271>) and a C-terminal CAP-Gly domain.
- Caenorhabditis elegans hypothetical protein M01A8.2.
- Yeast hypothetical protein YNL148c.

Structurally, these proteins are made of three distinct parts: an N-terminal section that is most probably globular and contains the CAP-Gly domain, a large central region predicted to be in an alpha-helical coiled-coil conformation and, finally, a short C-terminal globular domain. The signature for the CAP-Gly domain corresponds to the first 32 residues of the domain and includes five of the six conserved glycines.

-Consensus pattern: G-x(8,10)-[FYW]-x-G-[LIVM]-x-[LIVMFY]-x(4)-G-K-[NH]-x-G-[STAR] -x(2)-G-x(2)- [LY] -F

[1] Riehemann K., Sorg C. Trends Biochem. Sci. 18:82-83(1993).

73. (CBD 1)

Cellulose-binding domain, fungal type

The microbial degradation of cellulose and xylans requires several types of enzymes such as endoglucanases (EC 3.2.1.4), cellobiohydrolases (EC 3.2.1.91) (exoglucanases), or xylanases (EC 3.2.1.8) [1].

Structurally, cellulases and xylanases generally consist of a catalytic domain joined to a cellulose-binding domain (CBD) by a short linker sequence rich in proline and/or hydroxy-amino acids.

The CBD of a number of fungal cellulases has been shown to consist of 36 amino acid residues. Enzymes known to contain such a domain are:

- Endoglucanase I (gene eg11) from Trichoderma reesei.
- Endoglucanase II (gene eg12) from Trichoderma reesei.
- Endoglucanase V (gene eg15) from Trichoderma reesei.
- Exocellobiohydrolase I (gene CBHI) from Humicola grisea, Neurospora crassa, Phanerochaete chrysosporium, Trichoderma reesei, and Trichoderma viride.
- Exocellobiohydrolase II (gene CBHII) from Trichoderma reesei.
- Exocellobiohydrolase 3 (gene cel3) from Agaricus bisporus
- Endoglucanases B, C2, F and K from Fusarium oxysporum.

The CBD domain is found either at the N-terminal (Cbh-II or eg12) or at the C-terminal extremity (Cbh-I, egl1 or egl5) of these enzymes. As it is shown in the following schematic representation, there are four conserved cysteines in this type of CBD domain, all involved in disulfide bonds.

```
          +----------------+
          |       +-----|---------+
          |       |     |        |
xxxxxxxCxxxxxxxxxxCxxxxxCxxxxxxxxxCx
          ***************************
```

'C': conserved cysteine involved in a disulfide bond.
'*': position of the pattern.
Such a domain has also been found in a putative polysaccharide binding protein from the red alga, Porphyra purpurea [2]. Structurally, this protein consists of four tandem repeats of the CBD domain.
Consensus pattemC-G-G-x(4, 7)-G-x(3)-C-x(5)-C-x(3,5)-[NHG]-x -[FYWM]- x(2)-Q-C [The four C's are involved in disulfide bonds] Sequences known to belong to this class detected by the pattern ALL.

[1] Gilkes N.R., Henrissat B., Kilburn D.G., Miller R.C. Jr., Warren R.A.J. Microbiol. Rev. 55:303-315(1991).
[2] Liu Q., der Meer J.P., Reith M.E.

74. CBS domain. 3D Structure found as a subdomain in TIM barrel of inosine-. CBS domain web page. CBS domains are small intracellular modules mostly found in 2 or four copies within a protein. CBS domains are found in cystathionine-beta-synthase (CBS) where mutations lead to homocystinuria. Two CBS domains are found in inosine-monophosphate dehydrogenase from all species, however the CBS domains are not needed for activity. Two CBS domains are found in intracellular loops of several chloride channels. Mutations in this domain of Swiss:P35520 lead to homocystinuria.
Number of members: 414

[1]Medline: 97172695 The structure of a domain common to archaebacteria and the homocystinuria disease protein. Bateman A; Trends Biochem Sci 1997;22:12-13.
[2]Medline: 96279836 Structure and mechanism of inosine monophosphate dehydrogenase in complex with the immunosuppressant mycophenolic-acid. Sintchak MD, Fleming MA, Futer O, Raybuck SA, Chambers SP, Caron PR, Murcko MA, Wilson KP; Cell 1996;85:921-930.
Discovery of CBS domain.
[3]Medline: 97259972 CBS domains in C1C chloride channels implicated in myotonia and nephrolithiasis (kidney stones). Ponting CP; J Mol Med 1997;75:160-163.

75. CDP-OH_P_transf (CDP-alcohol phosphatidyltransferase)
All of these members have the ability to catalyze the displacement of CMP from a CDP-alcohol by a second alcohol with formation of a phosphodiester bond and concomitant breaking of a phosphoride anhydride bond. Number of members: 32
A number of phosphatidyltransferases, which are all involved in phospholipid biosynthesis and that share the property of catalyzing the displacement of CMP from a CDP-alcohol by a second alcohol with formation of a phosphodiester bond and concomitant breaking of a phosphoride anhydride bond share a conserved sequence region [1,2]. These enzymes are:

- Ethanolaminephosphotransferase (EC 2.7.8.1) from yeast (gene EPT1).
- Diacylglycerol cholinephosphotransferase (EC 2.7.8.2) from yeast (gene CPT1).
- Phosphatidylglycerophosphate synthase (EC 2.7.8.5) (CDP-diacylglycerol--glycerol-3-phosphate 3-phosphatidyltransferase) from bacteria (gene pgsA).
- Phosphatidylserine synthase (EC 2.7.8.8) (CDP-diacylglycerol--serine O-phosphatidyltransferase) from yeast (gene CHO1) and from Bacillus subtilis (gene pssA).
- Phosphatidylinositol synthase (EC 2.7.8.11) (CDP-diacylglycerol--inositol 3-phosphatidyltransferase) from yeast (gene PIS).

These enzymes are proteins of from 200 to 400 amino acid residues. The conserved region contains three aspartic acid residues and is located in the N-terminal section of the sequences.
-Consensus pattern: D-G-x(2)-A-R-x(8)-G-x(3)-D-x(3)-D

[1]Medline: 97075020 Two-dimensional 1H-NMR of transmembrane peptides from Escherichia coli phosphatidylglycerophosphate synthase in micelles. Morein S, Trouard TP, Hauksson JB, Rilfors L, Arvidson G, Lindblom G; Eur J Biochem 1996;241:489-497.
[1] Nikawa J.-I., Kodaki T., Yamashita S. J. Biol. Chem. 262:4876-4881(1987).
[2] Hjelmstad R.H., Bell R.M. J. Biol. Chem. 266:5094-5134(1991).

76. CHOD (Cholesterol oxidase) Members of the GMC oxidoreductase family. Number of members: 3

[1]Medline: 94032271. Crystal structure of cholesterol oxidase complexed with a steroid substrate: implications for flavin adenine dinucleotide dependent alcohol oxidases. Li J, Vrielink A, Brick P, Blow DM; Biochemistry 1993;32:11507-11515.

The following FAD flavoproteins oxidoreductases have been found [1,2] to be evolutionary related. These enzymes, which are called 'GMC oxidoreductases', are listed below.

- Glucose oxidase (EC 1.1.3.4) (GOX) from Aspergillus niger. Reaction catalyzed: glucose + oxygen -> delta-luconolactone + hydrogen peroxide.
- Methanol oxidase (EC 1.1.3.13) (MOX) from fungi. Reaction catalyzed: methanol + oxygen -> acetaldehyde + hydrogen peroxide.
- Choline dehydrogenase (EC 1.1.99.1) (CHD) from bacteria. Reaction catalyzed: choline + unknown acceptor -> betaine acetaldehyde + reduced acceptor.
- Glucose dehydrogenase (GLD) (EC 1.1.99.10) from Drosophila. Reaction catalyzed: glucose + unknown acceptor -> delta-gluconolactone + reduced acceptor.
- Cholesterol oxidase (CHOD) (EC 1.1.3.6) from Brevibacterium sterolicum and Streptomyces strain SA-COO. Reaction catalyzed: cholesterol + oxygen -> cholest-4-en-3-one + hydrogen peroxide.
- AlkJ [3], an alcohol dehydrogenase from Pseudomonas oleovorans, which converts aliphatic medium-chain-length alcohols into aldehydes. This family also includes a lyase:
- (R)-mandelonitrile lyase (EC 4.1.2.10) (hydroxynitrile lyase) from plants [4], an enzyme involved in cyanogenis, the release of hydrogen cyanide from injured tissues.

These enzymes are proteins of size ranging from 556 (CHD) to 664 (MOX) amino acid residues which share a number of regions of sequence similarities. One of these regions, located in the N-terminal section, corresponds to the FAD ADP- binding domain. The function of the other conserved domains is not yet known; two of these domains have been selected as signature patterns. The first one is located in the N-terminal section of these enzymes, about 50 residues after the ADP-binding domain, while the second one is located in the central section.

- Consensus pattern: [GA]-[RKN]-x-[LIV]-G(2)-[GST](2)-x-[LIVM]-N-x(3)-[FYWA]-x(2)-[PAG]-x(5)-[DNESH]
- Consensus pattern: [GS]-[PSTA]-x(2)-[ST]-P-x-[LIVM](2)-x(2)-S-G-[LIVM]-G

[1] Cavener D.R. J. Mol. Biol. 223:811-814(1992).
[2] Henikoff S., Henikoff J.G. Genomics 19:97-107(1994).
[3] van Beilen J.B., Eggink G., Enequist H., Bos R., Witholt B. Mol. Microbiol. 6:3121-3136(1992).
[4] Cheng I.P., Poulton J.E. Plant Cell Physiol. 34:1139-1143(1993).

77. CKS (Cyclin-dependent kinase regulatory subunit) Number of members: 11. Cyclin-dependent kinases (CDK) are protein kinases which associate with cyclins to regulate eukaryotic cell cycle progression. The most well known CDK is p34-cdc2 (CDC28 in yeast) which is required for entry into S-phase and mitosis. CDK's bind to a regulatory subunit which is essential for their biological function. This regulatory subunit is a small protein of 79 to 150 residues. In yeast (gene CKS1) and in fission yeast (gene suc1)a single isoform is known, while mammals have two highly related isoforms. It has been shown [1] that these CDK regulatory subunits assemble as an hexamer which then acts as a hub for the oligomerization of six CDK catalytic subunits. The sequence of CDK regulatory subunits are highly conserved therefore, the two most conserved regions have been used as signature patterns.

- Consensus pattern: Y-S-x-[KR]-Y-x-[DE](2)-x-[FY]-E-Y-R-H-V-x-[LV]-[PT]-[KRP]
- Consensus pattern: H-x-P-E-x-H-[IV]-L-L-F-[KR]

[1] Parge H.E., Arvai A.S., Murtari D.J., Reed S.I., Tainer J.A. Science 262:387-395(1993).

78. CK_II_beta (Casein kinase II regulatory subunit)
Number of members: 16. Casein kinase II (CK-2) [1] is an ubiquitous eukaryotic serine/threonine protein kinase which is found both in the cytoplasm and the nucleus and whose substrates are numerous. It generally phosphorylates Ser or Thr at the N-terminal of stretch of acidic residues (see <PDOC00006>). CK-2 exists as an heterotetramer composed of two catalytic subunits (alpha) and two regulatory subunits (beta). In most species there are two closely related isoforms of the catalytic subunit: alpha and alpha'. Some species, such as fungi and plants, express two forms of regulatory subunits: beta and beta'. The exact function of the regulatory subunit is not yet known. It is a highly conserved protein of about 25 Kd that contains, in its central section, a cysteine-rich motif that could be involved in binding a metal such as zinc [2]. This region has been used as a signature pattern.

- Consensus pattern: C-P-x-[LIVMY]-x-C-x(5)-[LI]-P-[LIVMC]-G-x(9)-V-[KR]-x(2)-C-P-x-C

[1] Allende J.E., Allende C.C. FASEB J. 9:313-323(1995).
[2] Reed J.C., Bidwai A.P., Glover C.V.C. J. Biol. Chem. 269:18192-18200(1994).

79. CLP_protease (Clp protease)
These proteins belong to family S14 in the classification of peptidases.

-!- The Clp protease has an active site catalytic triad. In E. coli Clp protease, ser-111, his-136 and asp-185 form the catalytic triad.
-!- Swiss:P48254 has lost all of these active site residues and is therefore inactive.
-!- Swiss:P42379 contains two large insertions, Swiss:P42380 contains one large insertion. Number of members: 38

The endopeptidase Clp (EC 3.4.21.92) from Escherichia coli cleaves peptides in various proteins in a process that requires ATP hydrolysis [1,2]. Clp is a dimeric protein which consists of a proteolytic subunit (gene clpP) and either of two related ATP-binding regulatory subunits (genes clpA and clpX). ClpP is a serine protease which has a chymotrypsin-like activity. Its catalytic activity seems to be provided by a charge relay system similar to that of the trypsin family of serine proteases, but which evolved by independent convergent evolution. Proteases highly similar to ClpP have been found to be encoded in the genome of the chloroplast of plants and seem to be also present in other eukaryotes. The sequences around two of the residues involved in the catalytic triad (a serine and a histidine) are highly conserved and can be used as signature patterns specific to that category of proteases.

- Consensus pattern: T-x(2)-[LIVMF]-G-x-A-[SAC]-S-[MSA]-[PAG]-[STA] [S is the active site residue]
- Consensus pattern: R-x(3)-[EAP]-x(3)-[LIVMFYT]-M-[LIVM]-H-Q-P [H is the active site residue]

[1]Medline: 98050920. The structure of ClpP at 2.3 angstroms resolution suggests a model for ATP-dependent proteolysis. Wang J, Hartling JA, Flanagan JM; Cell 1997;91:447-456.
[1] Maurizi M.R., Clark W.P., Kim S.-H., Gottesman S. J. Biol. Chem. 265:12546-12552(1990).
[2] Gottesman S., Maurizi M.R. Microbiol. Rev. 56:592-621(1992).
[3] Rawlings N.D., Barrett A.J. Meth. Enzymol. 244:19-61(1994).

80. CNG_membrane (Transmembrane region cyclic Nucleotide Gated Channel) [1]Medline: 94224763. Cyclic nucleotide-gated channels: an expanding new family of ion channels. Yau KW; Proc Natl Acad Sci USA 1994;91: 3481-3483.
This family is found to the N-terminus of the cNMP_binding. Number of members: 56. Proteins that bind cyclic nucleotides (cAMP or cGMP) share a structural domain of about 120 residues [1-3]. The best studied of these proteins is the prokaryotic catabolite gene activator (also known as the cAMP receptor protein) (gene crp) where such a domain is known to be composed of three alpha-helices and a distinctive eight-stranded, antiparallel beta-barrel structure. Such a domain is known to exist in the following proteins:

- Prokaryotic catabolite gene activator protein (CAP).
- cAMP- and cGMP-dependent protein kinases (cAPK and cGPK). Both types of kinases contains two tandem copies of the cyclic nucleotide-binding domain. The cAPK's are composed of two different subunits: a catalytic chain and a regulatory chain which contains both copies of the domain. The cGPK's are single chain enzymes that include the two copies of the domain in their N-terminal section. The nucleotide specificity of cAPK and cGPK is due to an amino acid in the conserved region of beta-barrel 7: a threonine that is invariant in cGPK is an alanine in most cAPK.

- Vertebrate cyclic nucleotide-gated ion-channels. Two such cations channels have been fully characterized. One is found in rod cells where it plays a role in visual signal transduction. It specifically binds to cGMP leading to an opening of the channel and thereby causing a depolarization of rod photoreceptors. In olfactory epithelium a similar, cAMP-binding, channel plays a role in odorant signal transduction. There are six invariant amino acids in this domain, three of which are glycine residues that are thought to be essential for maintenance of the structural integrity of the beta-barrel. Two signature patterns have been developed for this domain. The first pattern is located within beta-barrels and 3 and contains the first two conserved Gly. The second pattern is located within beta- barrels 6 and 7 and contains the third conserved Gly as well as the three other invariant residues.

- Consensus pattern: [LIVM]-[VIC]-x(2)-G-[DENQTA]-x-[GAC]-x(2)-[LIVMFY](4)-x(2)-G
- Consensus pattern: [LIVMF]-G-E-x-[GAS]-[LIVM]-x(5,11)-R-[STAQ]-A-x-[LIVMA]-x-[STACV]

[1] Weber LT., Shabb J.B., Corbin J.D. Biochemistry 28:6122-6127(1989).
[2] Kaupp U.B. Trends Neurosci. 14:150-157(1991).
[3] Shabb J.B., Corbin J.D. J. Biol. Chem. 267:5723-5726(1992).

81. COX10_ctaB_cyoE (Cytochrome c oxidase assembly factor)

[1]
Medline: 95191390 Biosynthesis and functional role of haem O and haem A Mogi T, Saiki K, Anraku Y; Mol Microbiol 1994;14:391-398.
Cytochrome c oxidase is a multi subunit enzyme. The complexity of this enzyme requires assistance in building the complex.
This is carried out by the Cytochrome c oxidase assembly factor.

Number of members: 31
Cytochrome c oxidase is an oligomeric enzymatic complex which seems to require the aid of a number of proteins that either act as chaperonins to help the subunits of the enzyme to fold correctly, or assist in the assembly of the metal centers [1]. One of these subunits is known as COX10 in yeast and as ctaB [2] in aerobic prokaryotes. It is evolutionary related to cyoE protein from the Escherichia coli cytochrome O terminal oxidase complex.
These proteins probably contain [3] seven transmembrane segments. The most conserved region is located in a loop between the second and third of these segments and has been selected as a signature pattern.

- Consensus pattern: [ED]-x-D-x(2)-M-x-R-T-x(2)-R-x(4)-G

[1] Nobrega M.P., Nobrega F.G., Tzagoloff A. J. Biol. Chem. 265:14220-14226(1990).
[2] Cao J., Hosler J., Shapleigh J., Revzin A., Ferguson-Miller S. J. Biol. Chem. 267:24273-24278(1992).
[3] Chepuri V., Gennis R.B. J. Biol. Chem. 265:12978-12986(1990).

82. COX3 (Cytochrome c oxidase subunit III)
This family corresponds to chains c and p.

[1]
Medline: 96216288 The whole structure of the 13-subunit oxidized cytochrome c oxidase at 2.8 A. Tsukihara T, Aoyama H, Yamashita E, Tomizaki T, Yamaguchi H, Shinzawa-Itoh K, Nakashima R, Yaono R, Yoshikawa S; Science 1996;272:1136-1144.
Number of members: 224

83. COX5B (Cytochrome c oxidase subunit Vb)

[1]
Medline: 96216288 The whole structure of the 13-subunit oxidized cytochrome c oxidase at 2.8 A. Tsukihara T, Aoyama H, Yamashita E, Tomizaki T, Yamaguchi H, Shinzawa-Itoh K, Nakashima R, Yaono R, Yoshikawa S; Science 1996;272:1136-1144.
This family consists of chains F and S
Number of members: 10

Cytochrome c oxidase (EC 1.9.3.1) [1] is an oligomeric enzymatic complex which is a component of the respiratory chain complex and is involved in the transfer of electrons from cytochrome c to oxygen. In eukaryotes this enzyme complex is located in the mitochondrial inner membrane; in aerobic prokaryotes it is found in the plasma membrane. In addition to the three large subunits that form the catalytic center of the enzyme complex there are, in eukaryotes, a variable number of small polypeptidic subunits. One of these subunits which is known as Vb in mammals, V in slime mold and IV in yeast, binds a zinc atom. The sequence of subunit Vb is well conserved and includes three conserved cysteines that are thought to coordinate the zinc ion [2]. Two of these cysteines are clustered in the C-terminal section of the subunit; this region has been selected as a signature pattern.

- Consensus pattern: [LIVM](2)-[FYW]-x(10)-C-x(2)-C-G-x(2)-[FY]-K-L [The two C's probably bind zinc]

[1] Capaldi R.A., Malatesta F., Darley-Usmar V.M. Biochim. Biophys. Acta 726:135-148(1983).
[2] Rizzuto R., Sandona D., Brini M., Capaldi R.A., Bisson R. Biochim. Biophys. Acta 1129:100-104(1991).

84. COesterase (Carboxylesterases)
Cholinesterase pages
The prints entry is specific to acetylcholinesterase
Number of members: 273
Higher eukaryotes have many distinct esterases. Among the different types are those which act on carboxylic esters (EC 3.1.1.-). Carboxyl-esterases have been classified into three categories (A, B and C) on the basis of differential patterns of inhibition by organophosphates. The sequence of a number of type-B carboxylesterases indicates [1,2,3] that the majority are evolutionary related. This family currently consists of the following proteins:

- Acetylcholinesterase (EC 3.1.1.7) (AChE) [E1] from vertebrates and from Drosophila.
- Mammalian cholinesterase II (butyryl cholinesterase) (EC 3.1.1.8). Acetylcholinesterase and cholinesterase II are closely related enzymes that hydrolyze choline esters [4].
- Mammalian liver microsomal carboxylesterases (EC 3.1.1.1).
- Drosophila esterase 6, produced in the anterior ejaculatory duct of the male insect reproductive system where it plays an important role in its reproductive biology.
- Drosophila esterase P.
- Culex pipiens (mosquito) esterases B1 and B2.
- Myzus persicae (peach-potato aphid) esterases E4 and FE4.
- Mammalian bile-salt-activated lipase (BAL) [5], a multifunctional lipase which catalyzes fat and vitamin absorption. It is activated by bile salts in infant intestine where it helps to digest milk fats.
- Insect juvenile hormone esterase (JH esterase) (EC 3.1.1.59).
- Lipases (EC 3.1.1.3) from the fungi Geotrichum candidum and Candida rugosa.
- Caenorhabditis gut esterase (gene ges-1).
- Duck fatty acyl-CoA hydrolase, medium chain (EC 3.1.2.14), an enzyme that may be associated with peroxisome proliferation and may play a role in the production of 3-hydroxy fatty acid diester pheromones.
- Membrane enclosed crystal proteins from slime mold. These proteins are, most probably esterases; the vesicles where they are found have therefore been termed esterosomes.

So far two bacterial proteins have been found to belong to this family:

- Phenmedipham hydrolase (phenylcarbamate hydrolase), an Arthrobacter oxidans plasmid-encoded enzyme (gene pcd) that degrades the phenylcarbamate herbicides phenmedipham and desmedipham by hydrolyzing their central carbamate linkages.
- Para-nitrobenzyl esterase from Bacillus subtilis (gene pnbA).

The following proteins, while having lost their catalytic activity, contain a domain evolutionary related to that of carboxylesterases type-B:

- Thyroglobulin (TG), a glycoprotein specific to the thyroid gland, which is the precursor of the iodinated thyroid hormones thyroxine (T4) and triiodo thyronine (T3).
- Drosophila protein neuractin (gene nrt) which may mediate or modulate cell adhesion between embryonic cells during development.
- Drosophila protein glutactin (gene glt), whose function is not known.

As is the case for lipases and serine proteases, the catalytic apparatus of esterases involves three residues (catalytic triad): a serine, a glutamate or aspartate and a histidine. The sequence around the active site serine is well conserved and can be used as a signature pattern. A conserved region located in the N-terminal section containing a cysteine involved in a disulfide bond has been selected as a second signature pattern.

- Consensus pattern: F-[GR]-G-x(4)-[LIVM]-x-[LIV]-x-G-x-S-[STAG]-G[S is the active site residue]
- Consensus pattern: [ED]-D-C-L-[YT]-[LIV]-[DNS]-[LIV]-[LIVFYW]-x-[PQR] [C is involved in a disulfide bond]

[1] Myers M., Richmond R.C., Oakeshott J.G. Mol. Biol. Evol. 5:113-119(1988).
[2] Krejci E., Duval N., Chatonnet A., Vincens P., Massoulie J. Proc. Natl. Acad. Sci. U.S.A. 88:6647-6651(1991).
[3] Cygler M., Schrag J.D., Sussman J.L., Harel M., Silman I. Gentry M.K., Doctor B.P. Protein Sci. 2:366-382 (1993).
[4] Lockridge O. BioEssays 9:125-128(1988).
[5] Wang C.-S., Hartsuck J.A. Biochim. Biophys. Acta 1166:1-19(1993).

85. CPSase_L_chain (Carbamoyl-phosphate synthase (CPSase))

[1]
Medline: 94347758 Three-dimensional structure of the biotin carboxylase subunit. of acetyl-CoA carboxylase. Waldrop GL, Rayment I, Holden HM; Biochemistry 1994;33:10249-10256.
[1]
Medline: 90285162 Mammalian carbamyl phosphate synthetase (CPS). DNA sequence and evolution of the CPS domain of the Syrian hamster multifunctional protein CAD. Simmer JP, Kelly RE, Rinker AG Jr, Scully JL, Evans DR; Biol Chem 1990;265:10395-10402.
Carbamoyl-phosphate synthase catalyzes the ATP-dependent synthesis of carbamyl-phosphate from glutamine or ammonia and bicarbonate. This important enzyme initiates both the urea cycle and the biosynthesis of arginine and/or pyrimidines [2].

The carbamoyl-phosphate synthase (CPS) enzyme in prokaryotes is a heterodimer of a small and large chain. The small chain promotes the hydrolysis of glutamine to ammonia, which is used by the large chain to synthesize carbamoyl phosphate. See CPSase_sm_chain.
The small chain has a GATase domain in the carboxyl terminus.
See GATase. Number of members: 181
Carbamoyl-phosphate synthase (CPSase) catalyzes the ATP-dependent synthesis of carbamyl-phosphate from glutamine (EC 6.3.5.5) or ammonia (EC 6.3.4.16) and bicarbonate [1]. This important enzyme initiates both the urea cycle and the biosynthesis of arginine and pyrimidines.
Glutamine-dependent CPSase (CPSase II) is involved in the biosynthesis of pyrimidines and purines. In bacteria such as Escherichia coli, a single enzyme is involved in both biosynthetic pathways while other bacteria have separate enzymes. The bacterial enzymes are formed of two subunits. A small chain (gene carA) that provides glutamine amidotransferase activity (GATase) necessary for removal of the ammonia group from glutamine, and a large chain (gene carB) that provides CPSase activity. Such a structure is also present in fungi for arginine biosynthesis (genes CPA1 and CPA2). In most eukaryotes, the first three steps of pyrimidine biosynthesis are catalyzed by a large multifunctional enzyme - called URA2 in yeast, rudimentary in Drosophila and CAD in mammals [2]. The CPSase domain is located between an N-terminal GATase domain and the C-terminal part which encompass the dihydroorotase and aspartate transcarbamylase activities.
Ammonia-dependent CPSase (CPSase I) is involved in the urea cycle in ureolytic vertebrates; it is a monofunctional protein located in the mitochondrial matrix.
The CPSase domain is typically 120 Kd in size and has arisen from the duplication of an ancestral subdomain of about 500 amino acids. Each subdomain independently binds to ATP and it is suggested that the two homologous halves act separately, one to catalyze the phosphorylation of bicarbonate to carboxy phosphate and the other that of carbamate to carbamyl phosphate.
The CPSase subdomain is also present in a single copy in the biotin-dependent enzymes acetyl-CoA carboxylase (EC 6.4.1.2) (ACC), propionyl-CoA carboxylase (EC 6.4.1.3) (PCCase), pyruvate carboxylase (EC 6.4.1.1) (PC) and urea carboxylase (EC 6.3.4.6).
Two conserved regions which are probably important for binding ATP and/or catalytic activity have been selected as signatures for the subdomain.

- Consensus pattern: [FYV]-[PS]-[LIVMC]-[LIVMA]-[LIVM]-[KR]-[PSA]-[STA]-x(3)-[SG]-G-x-[AG]

- Consensus pattern: [LIVMF]-[LIMN]-E-[LIVMCA]-N-[PATLIVM]-[KR]-[LIVMSTAC]

[1] Simmer J.P., Kelly R.E., Rinker A.G. Jr., Scully J.L., Evans D.R. J. Biol. Chem. 265:10395-10402(1990).
[2] Davidson J.N., Chen K.C., Jamison R.S., Musmanno L.A., Kern C.B. BioEssays 15:157-164(1993).

86. CPSase_sm_chain (Carbamoyl-phosphate synthase small chain, CPSase domain)

[1]
Medline: 90285162 Mammalian carbamyl phosphate synthetase (CPS). DNA sequence and evolution of the CPS domain of the Syrian hamster multifunctional protein CAD. Simmer JP, Kelly RE, Rinker AG Jr, Scully JL, Evans DR; Biol Chem 1990;265:10395-10402.
The carbamoyl-phosphate synthase domain is in the amino terminus of protein.
Carbamoyl-phosphate synthase catalyzes the ATP-dependent synthesis of carbamyl-phosphate from glutamine or ammonia and bicarbonate. This important enzyme initiates both the urea cycle and the biosynthesis of arginine and/or pyrimidines [1].

The carbamoyl-phosphate synthase (CPS) enzyme in prokaryotes is a heterodimer of a small and large chain. The small chain promotes the hydrolysis of glutamine to ammonia, which is used by the large chain to synthesize carbamoyl phosphate. See CPSase_L_chain.
The small chain has a GATase domain in the carboxyl terminus.
See GATase.
Number of members: 46
Carbamoyl-phosphate synthase (CPSase) catalyzes the ATP-dependent synthesis of carbamyl-phosphate from glutamine (EC 6.3.5.5) or ammonia (EC 6.3.4.16) and bicarbonate [1]. This important enzyme initiates both the urea cycle and the biosynthesis of arginine and pyrimidines.
Glutamine-dependent CPSase (CPSase II) is involved in the biosynthesis of pyrimidines and purines. In bacteria such as Escherichia coli, a single enzyme is involved in both biosynthetic pathways while other bacteria have separate enzymes. The bacterial enzymes are formed of two subunits. A small chain (gene carA) that provides glutamine amidotransferase activity (GATase) necessary for removal of the ammonia group from glutamine, and a large chain (gene carB) that provides CPSase activity. Such a structure is also present in fungi for arginine biosynthesis (genes CPA1 and CPA2). In most eukaryotes, the first three steps of pyrimidine biosynthesis are catalyzed by a large multifunctional enzyme - called URA2 in yeast, rudimentary in Drosophila and CAD in mammals [2]. The CPSase domain is located between an N-terminal GATase domain and the C-terminal part which encompass the dihydroorotase and aspartate transcarbamylase activities.
Ammonia-dependent CPSase (CPSase I) is involved in the urea cycle in ureolytic vertebrates; it is a monofunctional protein located in the mitochondrial matrix.
The CPSase domain is typically 120 Kd in size and has arisen from the duplication of an ancestral subdomain of about 500 amino acids. Each subdomain independently binds to ATP and it is suggested that the two homologous halves act separately, one to catalyze the phosphorylation of bicarbonate to carboxy phosphate and the other that of carbamate to carbamyl phosphate.
The CPSase subdomain is also present in a single copy in the biotin-dependent enzymes acetyl-CoA carboxylase (EC 6.4.1.2) (ACC), propionyl-CoA carboxylase (EC 6.4.1.3) (PCCase), pyruvate carboxylase (EC 6.4.1.1) (PC) and urea carboxylase (EC 6.3.4.6).
Two conserved regions which are probably important for binding ATP and/or catalytic activity have been selected as signatures for the subdomain.

- Consensus pattern: [FYV]-[PS]-[LIVMC]-[LIVMA]-[LIVM]-[KR]-[PSA]-[STA]-x(3)-[SG]-G-x-[AG]
- Consensus pattern: [LIVMF]-[LIMN]-E-[LIVMCA]-N-[PATLIVM]-[KR]-[LIVMSTAC]

[1] Simmer J.P., Kelly R.E., Rinker A.G. Jr., Scully J.L., Evans D.R. J. Biol. Chem. 265:10395-10402(1990).
[2] Davidson J.N., Chen K.C., Jamison R.S., Musmanno L.A., Kern C.B. BioEssays 15:157-164(1993).

87. CRAL_TRIO (CRAL/TRIO domain)

[1]
Medline: 98121119 Crystal structure of the Saccharomyces cerevisiae phosphatidylinositol-transfer protein. Sha B, Phillips SE, Bankaitis VA, Luo M; Nature 1998;391:506-510.
The original profile has been extended to include the carboxyl domain from the known structure of Sec14. Swiss:

P10911 has not been included in the Pfam family because it does not appear to contain a complete structural domain.
Number of members: 39

88. CSD ('Cold-shock'DNA-binding domain)

[1]
Medline: 94255482 Crystal structure of CspA, the major cold shock protein of Escherichia coli. Schindelin H, Jiang W, Inouye M, Heinemann U; Proc Natl Acad Sci U S A 1994;91:5119-5123.
Number of members: 121

A conserved domain of about 70 amino acids has been found in prokaryotic and eukaryotic DNA-binding proteins [1,2,3,E1]. This domain, which is known as the 'cold-shock domain' (CSD) is present in the proteins listed below.

- Escherichia coli protein CS7.4 (gene cspA) which is induced in response to low temperature (cold-shock protein) and which binds to and stimulates the transcription of the CCAAT-containing promoters of the HN-S protein and of gyrA.
- Mammalian Y box binding protein 1 (YB 1). A protein that binds to the CCAAT-containing Y box of mammalian HLA class II genes.
- Xenopus Y box binding proteins -1 and -2 (Y1 and Y2). Proteins that bind to the CCAAT-containing Y box of Xenopus hsp70 genes.
- Xenopus B box binding protein (YB3). YB3 binds the B box promoter element of genes transcribed by RNA polymerase III.
- Enhancer factor I subunit A (EFI-A) (dbpB). A protein that also bind to CCAAT-motif in various gene promoters.
- DbpA, a Human DNA-binding protein of unknown specificity.
- Bacillus subtilis cold-shock proteins cspB and cspC.
- Streptomyces clavuligerus protein SC 7.0.
- Escherichia coli proteins cspB, cspC, cspD, cspE and cspF.
- Unr, a mammalian gene encoded upstream of the N-ras gene. Unr contains nine repeats that are similar to the CSD domain. The function of Unr is not yet known but it could be a multivalent DNA-binding protein.

As a signature pattern for the CSD domain, its most conserved region which is located in its N-terminal section has been selected. It must be noted that the beginning of this region is highly similar [4] to the RNP-1 RNA-binding motif.

- Consensus pattern: [FY]-G-F-I-x(6,7)-[DER]-[LIVM]-F-x-H-x-[STKR]-x-[LIVMFY]

[1] Doniger J., Landsman D., Gonda M.A., Wistow G. New Biol. 4:389-395(1992).
[2] Wistow G. Nature 344:823-824(1990).
[3] Jones P.G., Inouye M. Mol. Microbiol. 11:811-818(1994).
[4] Landsman D. Nucleic Acids Res. 20:2861-2864(1992).

89. CTF_NFI (CTF/NF-I family)
Number of members: 45
Nuclear factor I (NF-I) or CCAAT box-binding transcription factor (CTF) [1,2] (also known as TGGCA-binding proteins) are a family of vertebrate nuclear proteins which recognize and bind, as dimers, the palindromic DNA sequence 5'-TGGCANNNTGCCA-3'. CTF/NF-I binding sites are present in viral and cellular promoters and in the origin of DNA replication of Adenovirus type 2.
The CTF/NF-I proteins were first identified as nuclear factor I, a collection of proteins that activate the replication of several Adenovirus serotypes (together with NF-II and NF-III) [3]. The family of proteins was also identified as the CTF transcription factors, before the NFI and CTF families were found to be identical [4]. The CTF/NF-I proteins are individually capable of activating transcription and DNA replication. The CTF/NF-I family name has also been dubbed as NFI, NF-I or NF1.
In a given species, there are a large number of different CTF/NF-I proteins. The multiplicity of CTF/NF-I is known to be generated both by alternative splicing and by the occurrence of four different genes. The known forms of NF-I genes have been classified as:

- The CTF-like factors subfamily (prototype form: CTF-1) [4]
- The NFI-X proteins.

- The NFI-A proteins.
- The NFI-B proteins.

So far, all CTF/NF-I family members appear to have similar transcription and replication activities.
CTF/NF-1 proteins contains 400 to 600 amino acids. The N-terminal 200 amino-acid sequence, almost perfectly conserved in all species and genes sequenced, mediates site-specific DNA recognition, protein dimerization and Adenovirus DNA replication. The C-terminal 100 amino acids contain the transcriptional activation domain. This activation domain is the target of gene expression regulatory pathways ellicited by growth factors and it interacts with basal transcription factors and with histone H3 [6].
A perfectly conserved, highly charged 12 residue peptide located in the N-terminal part of CTF/NF-I has been selected as a specific signature for this family of proteins.

- Consensus pattern: R-K-R-K-Y-F-K-K-H-E-K-R

[1] Mermod N., O'Neill E.A., Kelly T.J., Tjian R. Cell 58:741-753(1989).
[2] Rupp R.A.W., Kruse U., Multhaup G., Goebel U., Beyreuther K., Sippel A.E. Nucleic Acids Res. 18:2607-2616 (1990).
[3] Nagata K., Guggenheimer R.A., Enomoto T., Lichy J.H., Hurwitz J. Proc. Natl. Acad. Sci. U.S.A. 79:6438-6442 (1982).
[4] Santoro C., Mermod N., Andrews P.C., Tjian R. Nature 334:2118-2224(1988).
[5] Gil G., Smith J.R., Goldstein J.L., Slaughter C.A., Orth K., Brown M.S., Osborne T.F. Proc. Natl. Acad. Sci. U.S.A 85:8963-8967(1988).
[6] Alevizopoulos A., Dusserre Y., Tsai-Pflugfelder M., von der Weid T., Wahli W., Mermod N. Genes Dev. 9: 3051-3066(1995).

90. Calsequestrin (Calsequestrin)
Number of members: 13
Calsequestrin is a moderate-affinity, high-capacity calcium-binding protein of cardiac and skeletal muscle [1], where it is located in the lumenal space of the sarcoplasmic reticulum terminal cisternae. Calsequestrin acts as a calcium buffer and plays an important role in the muscle excitation-contraction coupling. It is a highly acidic protein of about 400 amino acid residues that binds more than 40 moles of calcium per mole of protein. There are at least two different forms of calsequestrin: one which is expressed in cardiac muscles and another in skeletal muscles. Both forms have highly similar sequences.
Two signature sequences have been developed. The first corresponds to the N-terminus of the mature protein, the second is located just in front of the C-terminus of the protein which is composed of a highly acidic tail of variable length.

- Consensus pattern: [EQ]-[DE]-G-L-[DN]-F-P-x-Y-D-G-x-D-R-V
- Consensus pattern: [DE]-L-E-D-W-[LIVM]-E-D-V-L-x-G-x-[LIVM]-N-T-E-D-D-D

[1] Treves S., Vilsen B., Chiozzi P., Andersen J.P., Zorzato F. Biochem. J. 283:767-772(1992).

91. Carboxyl_trans (Carboxyl transferase domain)

[1]
Medline: 93374821
Primary structure of the monomer of the 12S subunit of transcarboxylase as deduced from DNA and characterization of the product expressed in Escherichia coli.
Thornton CG, Kumar GK, Haase FC, Phillips NF, Woo SB, Park VM, Magner WJ, Shenoy BC, Wood HG, Samols D; J Bacteriol 1993;175:5301-5308.
[2]
Medline: 93358891 Molecular evolution of biotin-dependent carboxylases. Toh H, Kondo H, Tanabe T; Eur J Biochem 1993;215:687-696.
All of the members in this family are biotin dependent carboxylases.
The carboxyl transferase domain carries out the following reaction; transcarboxylation from biotin to an acceptor molecule. There are two recognised types of carboxyl transferase. One of them uses acyl-CoA and the other uses 2-oxo acid as the acceptor molecule of carbon dioxide. All of the members in this family utilise acyl-CoA as the acceptor molecule.
Number of members: 47

92. Chal_stil_synt (Chalcone and stilbene synthases)
Number of members: 146
Chalcone synthases (CHS) (EC 2.3.1.74) and stilbene synthases (STS) (formerly known as resveratrol synthases) are related plant enzymes [1]. CHS is an important enzyme in flavanoid biosynthesis and STS a key enzyme in stilbene-type phyloalexin biosynthesis. Both enzymes catalyze the addition of three molecules of malonyl-CoA to a starter CoA ester (a typical example is 4-coumaroyl-CoA), producing either a chalcone (with CHS) or stilbene (with STS).
These enzymes are proteins of about 390 amino-acid residues. A conserved cysteine residue, located in the central section of these proteins, has been shown [2] to be essential for the catalytic activity of both enzymes and probably represents the binding site for the 4-coumaryl-CoA group. The region around this active site residue is well conserved and can be used as a signature pattern.
In addition to the plant enzymes, this family also includes Bacillus subtilis bcsA.

- Consensus pattern: R-[LIVMFYS]-x-[LIVM]-x-[QHG]-x-G-C-[FYNA]-[GA]-G-[GA]-[STAV]-x-[LIVMF]-[RA] [C is the active site residue]

[1] Schroeder J., Schroeder G. Z. Naturforsch. 45C:1-8(1990).
[2] Lanz T., Tropf S., Marner F.-J., Schroeder J., Schroeder G. J. Biol. Chem. 266:9971-9976(1991).

93. Chorismate_synt (Chorismate synthase)
Number of members: 19
Chorismate synthase (EC 4.6.1.4) catalyzes the last of the seven steps in the shikimate pathway which is used in prokaryotes, fungi and plants for the biosynthesis of aromatic amino acids. It catalyzes the 1,4-trans elimination of the phosphate group from 5-enolpyruvylshikimate-3-phosphate (EPSP) to form chorismate which can then be used in phenylalanine, tyrosine or tryptophan biosynthesis. Chorismate synthase requires the presence of a reduced flavin mononucleotide (FMNH2 or FADH2) for its activity.
Chorismate synthase from various sources shows [1,2] a high degree of sequence conservation. It is a protein of about 360 to 400 amino-acid residues.
Three signature patterns have been developed from conserved regions rich in basic residues (mostly arginines). The first is in the N-terminal section, the second is central and the third is C-terminal.

- Consensus pattern: G-E-S-H-[GC]-x(2)-[LIVM]-[GTV]-x-[LIVM](2)-[DE]-G-x-[PV]
- Consensus pattern: [GE]-R-[SA](2)-[SAG]-R-[EV]-[ST]-x(2)-[RH]-V-x(2)-G
- Consensus pattern: R-[SH]-D-[PSV]-[CSAV]-x(4)-[GAI]-x-[IVGSP]-[LIVM]-x-E-[STAH]-[LIVM]

[1] Schaller A., Schmid J., Leibinger U., Amrhein N. J. Biol. Chem. 266:21434-21438(1991).
[2] Jones D.G.L., Reusser U., Braus G.H. Mol. Microbiol. 5:2143-2152(1991).

94. Clat_adaptor_s (Clathrin adaptor complex small chain)
Number of members: 21
Clathrin coated vesicles (CCV) mediate intracellular membrane traffic such as receptor mediated endocytosis. In addition to clathrin, the CCV are composed of a number of other components including oligomeric complexes which are known as adaptor or clathrin assembly proteins (AP) complexes [1]. The adaptor complexes are believed to interact with the cytoplasmic tails of membrane proteins, leading to their selection and concentration. In mammals two type of adaptor complexes are known: AP-1 which is associated with the Golgi complex and AP-2 which is associated with the plasma membrane. Both AP-1 and AP-2 are heterotetramers that consist of two large chains - the adaptins - (gamma and beta' in AP-1; alpha and beta in AP-2); a medium chain (AP47 in AP-1; AP50 in AP-2) and a small chain (AP19 in AP-1; AP17 in AP-2).
The small chains of AP-1 and AP-2 are evolutionary related proteins of about 18 Kd. Homologs of AP17 and AP19 have also been found in yeast (genes APS1/ YAP19 and APS2/YAP17) [2,3,4]. AP17 and AP19 are also related to the zetachain [5] of coatomer (zeta-cop), a cytosolic protein complex that reversibly associates with Golgi membranes to form vesicles that mediate biosynthetic protein transport from the endoplasmic reticulum, via the Golgi up to the trans Golgi network.
A conserved region in the central section of these proteins has been selected as a signature pattern.

- Consensus pattern: [LIVM](2)-Y-[KR]-x(4)-L-Y-F

[1] Pearse B.M., Robinson M.S. Annu. Rev. Cell Biol. 6:151-171(1990).

[2] Kirchhausen T., Davis A.C., Frucht S., O'Brine Greco B., Payne G.S., Tubb B. J. Biol. Chem. 266:11153-11157 (1991).
[3] Nakai M., Takada T., Endo T. Biochim. Biophys. Acta 1174:282-284(1993).
[4] Phan H.L., Finlay J.A., Chu D.S., Tan P.K., Kirchhausen T., Payne G.S. EMBO J. 13:1706-1717(1994).
[5] Kuge O., Hara-Kuge S., Orci L., Ravazzola M., Amherdt M., Tanigawa G., Wieland F.T., Rothman J.E. J. Cell Biol. 123:1727-1734(1993).

95. Clathrin_1g_ch (Clathrin light chain.)
Number of members: 8
Clathrin [1,2] is the major coat-forming protein that encloses vesicles such as coated pits and forms cell surface patches involved in membrane traffic within eukaryotic cells. The clathrin coats (called triskelions) are composed of three heavy chains (180 Kd) and three light chains (23 to 27 Kd).
The clathrin light chains [3], which may help to properly orient the assembly and disassembly of the clathrin coats, bind non-covalently to the heavy chain, they also bind calcium and interact with the hsc70 uncoating ATPase.

- In higher eukaryotes two genes code for distinct but related light chains: LC(a) and LC(b). Each of the two genes can yield, by tissue-specific alternative splicing, two separate forms which differ by the insertion of a sequence of respectively thirty or eighteen residues. There is, in the N-terminal part of the clathrin light chains a domain of twenty one amino acid residues which is perfectly conserved in LC(a) and LC(b).
- In yeast there is a single light chain (gene CLC1) whose sequence is only distantly related to that of higher eukaryotes.

Two signature patterns have been developed for clathrin light chains. The first pattern is a heptapeptide from the center of the conserved N-terminal region of eukaryotic light chains; the second pattern is derived from a positively charged region located in the C-terminal extremity of all known clathrin light chains.

- Consensus pattern: F-L-A-Q-Q-E-S

[1] Keen J.H. Annu. Rev. Biochem. 59:415-438(1990).
[2] Brodsky F.M. Science 242:1396-1402(1988).
[3] Brodsky F.M., Hill B.L., Acton S.L., Naethke I., Wong D.H., Ponnambalam S., Parham P. Trends Biochem. Sci. 16:208-213(1991).

96. (Clathrin repeat) 7-fold repeat in Clathrin and VPS Each repeat is about 140 amino acids long. The repeats occur in the arm region of the Clathrin heavy chain.
Number of members: 79

[1]
Medline: 92191269 Folding and trimerization of clathrin subunits at the triskelion hub. Nathke IS, Heuser J, Lupas A, Stock J, Turck CW, Brodsky FM; Cell 1992;68:899-910. [2]
Medline: 88097376 Clathrin heavy chain: molecular cloning and complete primary structure. Kirchhausen T, Harrison SC, Chow EP, Mattaliano RJ, Ramachandran KL, Smart J, Brosius J; Proc Natl Acad Sci U S A 1987; 84:8805-8809.

97. Collagen (Collagen triple helix repeat (20 copies))

[1] Medline: 94059583 New members of the collagen superfamily Mayne R, Brewton RG; Curr Opin Cell Biol 1993;5:883-890.
Scurvy is associated with collagens.
Members of this family belong to the collagen superfamily [1]. Collagens are generally extracellular structural proteins involved in formation of connective tissue structure. The alignment contains 20 copies of the G-X-Y repeat that forms a triple helix. The first position of the repeat is glycine, the second and third positions can be any residue but are frequently proline and hydroxyproline. Collagens are post translationally modified by proline hydoxylase to form the hydroxyproline residues. Defective hydroxylation is the cause of scurvy. Some members of the collagen superfamily are not involved in connective tissue structure but share the same triple helical structure.
Number of members: 2125

98. Coprogen_oxidas (Coproporphyrinogen III oxidase)
Number of members: 12
Coproporphyrinogen III oxidase (EC 1.3.3.3) (coproporphyrinogenase) [1,2] catalyzes the oxidative decarboxylation of coproporphyrinogen III into protoporphyrinogen IX, a common step in the pathway for the biosynthesis of porphyrins such as heme, chlorophyll or cobalamin.
Coproporphyrinogen III oxidase is an enzyme that requires iron for its activity. A cysteine seems to be important for the catalytic mechanism [3]. Sequences from a variety of eukaryotic and prokaryotic sources show that this enzyme has been evolutionarily conserved. A highly conserved region in the central part of the sequence has been selected as a signature pattern. This region contains the only conserved cysteine and is rich in charged amino acids.

- Consensus pattern: K-x-W-C-x(2)-[FYH](3)-[LIVM]-x-H-R-x-E-x-R-G-[LIVM]-G-G-[LIVM]-F-F-D

[1] Xu K., Elliott T. J. Bacteriol. 175:4990-4999(1993).
[2] Kohno H., Furukawa T., Yoshinaga T., Tokunaga R., Taketani S. J. Biol. Chem. 268:21359-21363(1993).
[3] Camadro J.M., Chambon H., Jolles J., Labbe P. Eur. J. Biochem. 156:579-587(1986).
[4] Xu K., Elliott T. J. Bacteriol. 176:3196-3203(1994).

99. Corona_nucleoca (Coronavirus nucleocapsid protein)

[1]
Medline: 98087828 Identification of a specific interaction between the coronavirus mouse hepatitis virus A59 nucleocapsid protein and packaging signal. Molenkamp R, Spaan WJ; Virology 1997;239:78-86.
Number of members: 44

100. Cu-oxidase (Multicopper oxidase)

[1]
Medline: 90126844 The blue oxidases, ascorbate oxidase, laccase and ceruloplasmin. Modelling and structural relationships. Messerschmidt A, Huber R; Eur J Biochem 1990;187:341-352.
Number of members: 150

Multicopper oxidases [1,2] are enzymes that possess three spectroscopically different copper centers. These centers are called: type 1 (or blue), type 2 (or normal) and type 3 (or coupled binuclear). The enzymes that belong to this family are:

- Laccase (EC 1.10.3.2) (urishiol oxidase), an enzyme found in fungi and plants, which oxidizes many different types of phenols and diamines.
- Ascorbate oxidase (EC 1.10.3.3), a higher plant enzyme.
- Ceruloplasmin (EC 1.16.3.1) (ferroxidase), a protein found in the serum of mammals and birds, which oxidizes a great variety of inorganic and organic substances. Structurally ceruloplasmin exhibits internal sequence homology, and seem to have evolved from the triplication of a copper-binding domain similar to that found in laccase and ascorbate oxidase.

In addition to the above enzymes there are a number of proteins which, on the basis of sequence similarities, can be said to belong to this family. These proteins are:

- Copper resistance protein A (copA) from a plasmid in Pseudomonas syringae. This protein seems to be involved in the resistance of the microbial host to copper.
- Blood coagulation factor V (Fa V).
- Blood coagulation factor VIII (Fa VIII) [E1].
- Yeast FET3 [3], which is required for ferrous iron uptake.
- Yeast hypothetical protein YFL041w and SpAC1F7.08, the fission yeast homolog.

Factors V and VIII act as cofactors in blood coagulation and are structurally similar [4]. Their sequence consists of a triplicated A domain, a B domain and a duplicated C domain; in the following order: A-A-B-A-C-C. The A-type domain is related to the multicopper oxidases.
Two signature patterns have been developed for these proteins. Both patterns are derived from the same region, which in ascorbate oxidase, laccase, in the third domain of ceruloplasmin, and in copA, contains five residues that

are known to be involved in the binding of copper centers. The first pattern does not make any assumption on the presence of copper-binding residues and thus can detect domains that have lost the ability to bind copper (such as those in Fa V and Fa VIII), while the second pattern is specific to copper-binding domains.

- Consensus pattern: G-x-[FYW]-x-[LIVMFYW]-x-[CST]-x(8)-G-[LM]-x(3)-[LIVMFYW]
- Consensus pattern: H-C-H-x(3)-H-x(3)-[AG]-[LM]
  [The first two H's are copper type 3 binding residues]
  [The C, the 3rd H, and L or M are copper type 1 ligands]

101. Cullin (Cullin family)
Number of members: 24
The following proteins are collectively termed cullins [1]:

- Caenorhabditis elegans cul-1 (or lin-19), a protein required for developmentally programmed transitions from the G1 phase of the cell cycle to the G0 phase or the apoptotic pathway.
- Caenorhabditis elegans cul-2, cul-3, cul-4 (F45E12.3), cul-5 (ZK856.1) and cul-6 (K08E7.7).
- Mammalian CUL1, CUL2, CUL3, CUL4A and CUL4B.
- Mammalian vasopressin-activated calcium-mobilizing receptor (VACM-1), a kidney-specific protein thought to form a cell surface receptor [2] but which does not have any structural hallmarks of a receptor.
- Drosophila 1in19.
- Yeast CDC53 [3], which acts in concert with CDC4 and UBC3 (CDC34) to control the G1-to-S phase transition.
- Yeast hypothetical protein YGR003w.
- Fission yeast hypothetical protein SpAC24H6.03.

The cullins are hydrophilic proteins of 740 to 815 amino acids. The C-terminal extremity is the most conserved part of these proteins. A signature pattern has been developed from that region.

- Consensus pattern: [LIV]-K-x(2)-[LIV]-x(2)-L-I-[DEQ]-[KRHNQ]-x-Y-[LIVM]-x-R-x(6,7)-[FY]-x-Y-x-[SA]>

[1] Kipreos E.T., Lander L.E., Wing J.P., He W.W., Hedgecock E.M. Cell 85:829-839(1996).
[2] Burnatowska-Hledin M.A., Spielman W.S., Smith W.L., Shi P., Meyer J.M., Dewitt D.L. Am. J. Physiol. 268: f1198-F1210(1995).
[3] Mathias N., Johnson S.L., Winey M., Adams A.E., Goetsch L., Pringle J.R., Byers B., Goebl M.G. Mol. Cell. Biol. 16:6634-6643(1996).

102. (Cu_amine_oxid)
Copper amine oxidase signatures
Amine oxidases (AO) [1] are enzymes that catalyze the oxidation of a wide range of biogenic amines including many neurotransmitters, histamine and xenobiotic amines. There are two classes of amine oxidases: flavin-containing (EC 1.4.3.4) and copper-containing (EC 1.4.3.6).
Copper-containing AO is found in bacteria, fungi, plants and animals, it is an homodimeric enzyme that binds one copper ion per subunit as well as a 2,4,5- trihydroxyphenylalanine quinone (or topaquinone) (TPQ) cofactor. This cofactor is derived from a tyrosine residue.
Two signature patterns were derived for copper AO, the first one contains the tyrosine which give rises to the TPQ cofactor while the second one contains one of the three histidines that bind the copper atom [2].
Consensus pattern[LIVM]-[LIVMA]-[LIVMF]-x(4)-[ST]-x(2)-N-Y-[DE]-[YN] [The first Y gives rises to TPQ] Sequences known to belong to this class detected by the patternALL.
Consensus patternT-x-[GS]-x(2)-H-[LIVMF]-x(3)-E-[DE]-x-P [H is a copper ligand] Sequences known to belong to this class detected by the pattern ALL, except for lentil AO.

[1] Knowles P.F., Dooley D.M. (In) Metal ions in biological systems; Sigel H., Sigel A., Eds., 30:361- 403, Marcel Dekker, New-York, (1993).
[2] Parsons M.R., Convery M.A., Wilmot C.M., Yadav K.D.S., Blakeley V., Corner A.S., Phillips S.E.V., McPherson M.J., Knowles P.F. Structure 3:1171-1184(1995).

103. Cys-protease (Cysteine protease)
Number of members: 358
Eukaryotic thiol proteases (EC 3.4.22.-) [1] are a family of proteolytic enzymes which contain an active site cysteine.

Catalysis proceeds through a thioester intermediate and is facilitated by a nearby histidine side chain; an asparagine completes the essential catalytic triad. The proteases which are currently known to belong to this family are listed below (references are only provided for recently determined sequences).

- Vertebrate lysosomal cathepsins B (EC 3.4.22.1), H (EC 3.4.22.16), L (EC 3.4.22.15), and S (EC 3.4.22.27) [2].
- Vertebrate lysosomal dipeptidyl peptidase I (EC 3.4.14.1) (also known as cathepsin C) [2].
- Vertebrate calpains (EC 3.4.22.17). Calpains are intracellular calcium-activated thiol protease that contain both a N-terminal catalytic domain and a C-terminal calcium-binding domain.
- Mammalian cathepsin K, which seems involved in osteoclastic bone resorption [3].
- Human cathepsin O [4].
- Bleomycin hydrolase. An enzyme that catalyzes the inactivation of the antitumor drug BLM (a glycopeptide).
- Plant enzymes: barley aleurain (EC 3.4.22.16), EP-B1/B4; kidney bean EP-C1, rice bean SH-EP; kiwi fruit actinidin (EC 3.4.22.14); papaya latex papain (EC 3.4.22.2), chymopapain (EC 3.4.22.6), caricain (EC 3.4.22.30), and proteinase IV (EC 3.4.22.25); pea turgor-responsive protein 15A; pineapple stem bromelain (EC 3.4.22.32); rape COT44; rice oryzain alpha, beta, and gamma; tomato low-temperature induced, Arabidopsis thaliana A494, RD19A and RD21A.
- House-dust mites allergens DerP1 and EurM1.
- Cathepsin B-like proteinases from the worms Caenorhabditis elegans (genes gcp-1, cpr-3, cpr-4, cpr-5 and cpr-6), Schistosoma mansoni (antigen SM31) and Japonica (antigen SJ31), Haemonchus contortus (genes AC-1 and AC-2), and Ostertagia ostertagi (CP-1 and CP-3).
- Slime mold cysteine proteinases CP1 and CP2.
- Cruzipain from Trypanosoma cruzi and brucei.
- Throphozoite cysteine proteinase (TCP) from various Plasmodium species.
- Proteases from Leishmania mexicana, Theileria annulata and Theileria parva.
- Baculoviruses cathepsin-like enzyme (v-cath).
- Drosophila small optic lobes protein (gene sol), a neuronal protein that contains a calpain-like domain.
- Yeast thiol protease BLH1/YCP1/LAP3.
- Caenorhabditis elegans hypothetical protein C06G4.2, a calpain-like protein.

Two bacterial peptidases are also part of this family:

- Aminopeptidase C from Lactococcus lactis (gene pepC) [5].
- Thiol protease tpr from Porphyromonas gingivalis.

Three other proteins are structurally related to this family, but may have lost their proteolytic activity.

- Soybean oil body protein P34. This protein has its active site cysteine replaced by a glycine.
- Rat testin, a sertoli cell secretory protein highly similar to cathepsin L but with the active site cysteine is replaced by a serine. Rat testin should not be confused with mouse testin which is a LIM-domain protein (see <PDOC00382>).
- Plasmodium falciparum serine-repeat protein (SERA), the major blood stage antigen. This protein of 111 Kd possesses a C-terminal thiol-protease-like domain [6], but the active site cysteine is replaced by a serine.

The sequences around the three active site residues are well conserved and can be used as signature patterns.

- Consensus pattern: Q-x(3)-[GE]-x-C-[YW]-x(2)-[STAGC]-[STAGCV] [C is the active site residue]
- Consensus pattern: [LIVMGSTAN]-x-H-[GSACE]-[LIVM]-x-[LIVMAT](2)-G-x-[GSADNH] [H is the active site residue]
- Consensus pattern: [FYCH]-[WI]-[LIVT]-x-[KRQAG]-N-[ST]-W-x(3)-[FYW]-G-x(2)-G-[LFYW]-[LIVMFYG]-x-[LIVMF] [N is the active site residue]

[1] Dufour E. Biochimie 70:1335-1342(1988).
[2] Kirschke H., Barrett A.J., Rawlings N.D. Protein Prof. 2:1587-1643(1995).
[3] Shi G.-P., Chapman H.A., Bhairi S.M., Deleeuw C., Reddy V.Y., Weiss S.J. FEBS Lett. 357:129-134(1995).
[4] Velasco G., Ferrando A.A., Puente X.S., Sanchez L.M., Lopez-Otin C. J. Biol. Chem. 269:27136-27142(1994).
[5] Chapot-Chartier M.P., Nardi M., Chopin M.C., Chopin A., Gripon J.C. Appl. Environ. Microbiol. 59:330-333 (1993).
[6] Higgins D.G., McConnell D.J., Sharp P.M. Nature 340:604-604(1989).

[7] Rawlings N.D., Barrett A.J. Meth. Enzymol. 244:461-486(1994).

104. Cys_Met_Meta_PP (Cys/Met metabolism PLP-dependent enzyme)

[1] Medline: 96428687 Crystal structure of the pyridoxal-5'-phosphate dependent cystathionine beta-lyase from Escherichia coli at 1.83 A. Clausen T, Huber R, Laber B, Pohlenz HD, Messerschmidt A; J Mol Biol 1996;262: 202-224.

[1] Medline: 99059720 Crystal structure of Escherichia coli cystathionine gamma-synthase at 1.5 A resolution. Clausen T, Huber R, Prade L, Wahl MC, Messerschmidt A; EMBO J 1998;17:6827-6838.
Database Reference: SCOP; 1cs1; fa; [SCOP-USA][CATH-PDBSUM]
This family includes enzymes involved in cysteine and methionine metabolism. The following are members:

Cystathionine gamma-lyase,
Cystathionine gamma-synthase,
Cystathionine beta-lyase,
Methionine gamma-lyase,
OAH/OAS sulfhydrylase,
O-succinylhomoserine sulphhydrylase
All of these members participate is slightly different reactions.
All these enzymes use PLP (pyridoxal-5'-phosphate) as a cofactor.

Number of members: 52

A number of pyridoxal-dependent enzymes involved in the metabolism of cysteine, homocysteine and methionine have been shown [1,2] to be evolutionary related. These are:

- Cystathionine gamma-lyase (EC 4.4.1.1) (gamma-cystathionase), which catalyzes the transformation of cystathionine into cysteine, oxobutanoate and ammonia. This is the final reaction in the transulfuration pathway that leads from methionine to cysteine in eukaryotes.
- Cystathionine gamma-synthase (EC 4.2.99.9), which catalyzes the conversion of cysteine and succinyl-homoserine into cystathionine and succinate: the first step in the biosynthesis of methionine from cysteine in bacteria (gene metB).
- Cystathionine beta-lyase (EC 4.4.1.8) (beta-cystathionase), which catalyzes the conversion of cystathionine into homocysteine, pyruvate and ammonia: the second step in the biosynthesis of methionine from cysteine in bacteria (gene metC).
- Methionine gamma-lyase (EC 4.4.1.11) (L-methioninase) which catalyzes the transformation of methionine into methanethiol, oxobutanoate and ammonia.
- OAH/OAS sulfhydrylase, which catalyzes the conversion of acetylhomoserine into homocysteine and that of acetylserine into cysteine (gene MET17 or MET25 in yeast).
- O-succinylhomoserine sulfhydrylase (EC 4.2.99.-).
- Yeast hypothetical protein YGL184c.
- Yeast hypothetical protein YHR112c.

These enzymes are proteins of about 400 amino-acid residues. The pyridoxal-P group is attached to a lysine residue located in the central section of these enzymes; the sequence around this residue is highly conserved and can be used as a signature pattern to detect this class of enzymes.

- Consensus pattern: [DQ]-[LIVMF]-x(3)-[STAGC]-[STAGCI]-T-K-[FYWQ]-[LIVMF]-x-G-[HQ]-[SGNH] [K is the pyridoxal-P attachment site]

[1] Ono B.I., Tanaka K., Naito K., Heike C., Shinoda S., Yamamoto S., Ohmori S., Oshima T., Toh-E A. J. Bacteriol. 174:3339-3347(1992).
[2] Barton A.B., Kaback D.B., Clark M.W., Keng T., Ouellette B.F.F., Storms R.K., Zeng B., Zhong W.W., Fortin N., Delaney S., Bussey H. Yeast 9:363-369(1993).

105. Cyt_reductase
FAD/NAD-binding Cytochrome reductase

Number of members: 60

[1] Medline: 95111952 Crystal structure of the FAD-containing fragment of corn nitrate reductase at 2.5 A resolution: relationship to other flavoprotein reductases. Lu G, Campbell WH, Schneider G, Lindqvist Y; Structure 1994;2:809-821.
[2] Medline: 92084635 The sequence of squash NADH:nitrate reductase and its relationship to the sequences of other flavoprotein oxidoreductases. A family of flavoprotein pyridine nucleotide cytochrome reductases. Hyde GE, Crawford NM, Campbell WH; J Biol Chem 1991;266:23542-23547.

106. Cytidylyltrans
Phosphatidate cytidylyltransferase
Number of members: 21
Phosphatidate cytidylyltransferase (EC 2.7.7.41) [1,2,3] (also known as CDP-diacylglycerol synthase) (CDS) is the enzyme that catalyzes the synthesis of CDP-diacylglycerol from CTP and phosphatidate (PA). CDP-diacylglycerol is an important branch point intermediate in both prokaryotic and eukaryotic organisms. CDS is a membrane-bound enzyme. A conserved region located in the C-terminal part has been selected as a signature pattern.

- Consensus pattern: S-x-[LIVMF]-K-R-x(4)-K-D-x-[GSA]-x(2)-[LI]-[PG]-x-H-G-G-[LIVM]-x-D-R-[LIVMF]-D

[1] Sparrow C.P., Raetz C.R.H. J. Biol. Chem. 260:12084-12091(1985).
[2] Shen H., Heacock P.N., Clancey C.J., Dowhan W. J. Biol. Chem. 271:789-795(1996).
[3] Saito S., Goto K., Tonosaki A., Kondo H. J. Biol. Chem. 272:9503-9509(1997).

107. (Cytidylyltransf) Cytidylyltransferase. This family includes: Cholinephosphate cytidylyltransferase. Glycerol-3-phosphate cytidylyltransferase.
Number of members: 64

[1] Medline: 10208837 CTP:Phosphocholine Cytidylyltransferase: Insights into Regulatory Mechanisms and Novel Functions. Clement JM, Kent C; Biochem Biophys Res Commun 1999;257:643-650.

108. (cNMP binding) Cyclic nucleotide-binding domain signatures and profile
Proteins that bind cyclic nucleotides (cAMP or cGMP) share a structural domainof about 120 residues [1-3]. The best studied of these proteins is theprokaryotic catabolite gene activator (also known as the cAMP receptorprotein) (gene crp) where such a domain is known to be composed of threealpha-helices and a distinctive eight-stranded, antiparallel beta-barrelstructure. Such a domain is known to exist in the following proteins: -

Prokaryotic catabolite gene activator protein (CAP). - cAMP- and cGMP-dependent protein kinases (cAPK and cGPK). Both types of kinases contains two tandem copies of the cyclic nucleotide-binding domain. The cAPK's are composed of two different subunits: a catalytic chain and a regulatory chain which contains both copies of the domain. The cGPK's are single chain enzymes that include the two copies of the domain in their N- terminal section. The nucleotide specificity of cAPK and cGPK is due to an amino acid in the conserved region of beta-barrel 7: a threonine that is invariant in cGPK is an alanine in most cAPK. - Vertebrate cyclic nucleotide-gated ion-channels. Two such cations channels have been fully characterized. One is found in rod cells where it plays a role in visual signal transduction. It specifically binds to cGMP leading to an opening of the channel and thereby causing a depolarization of rod photoreceptors. In olfactory epithelium a similar, cAMP-binding, channel plays a role in odorant signal transduction. There are six invariant amino acids in this domain, three of which are glycine residues that are thought to be essential for maintenance of the of the beta-barrel. Two signature patterns for this domain have been developed. The first pattern is located within beta-barrels 2 and 3 and contains the first two conserved Gly. The second pattern is located within beta-barrels 6 and 7 and contains the third conserved Gly as well as the three other invariant residues.-

First consensus pattern: [LIVM]-[VIC]-x(2)-G-[DENQTA]-x-[GAC]-x(2)-[LIVMFY](4)-x(2)-G
Second consensus pattern: [LIVMF]-G-E-x-[GAS]-[LIVM]-x(5,11)-R-[STAQ]-A-x-[LIVMA]-x- [STACV]-

[1] Weber I.T., Shabb J.B., Corbin J.D. Biochemistry 28:6122-6127(1989).
[2] Kaupp U.B. Trends Neurosci. 14:150-157(1991).
[3] Shabb J.B., Corbin J.D. J. Biol. Chem. 267:5723-5726(1992).

109. (cadherin)

Cadherins extracellular repeated domain signature

Cadherins [1,2] are a family of animal glycoproteins responsible for calcium-dependent cell-cell adhesion. Cadherins preferentially interact with themselves in a homophilic manner in connecting cells; thus acting as both receptor and ligand. A wide number of tissue-specific forms of cadherins are known:

- Epithelial (E-cadherin) (also known as uvomorulin or L-CAM) (CDH1).
- Neural (N-cadherin) (CDH2).
- Placental (P-cadherin) (CDH3).
- Retinal (R-cadherin) (CDH4).
- Vascular endothelial (VE-cadherin) (CDH5).
- Kidney (K-cadherin) (CDH6).
- Cadherin-8 (CDH8).
- Osteoblast (OB-cadherin) (CDH11).
- Brain (BR-cadherin) (CDH12).
- T-cadherin (truncated cadherin) (CDH13).
- Muscle (M-cadherin) (CDH14).
- Liver-intestine (LI-cadherin).
- EP-cadherin.

Structurally, cadherins are built of the following domains: a signal sequence, followed by a propeptide of about 130 residues, then an extracellular domain of around 600 residues, then a transmembrane region, and finally a C-terminal cytoplasmic domain of about 150 residues. The extracellular domain can be sub- divided into five parts: there are four repeats of about 110 residues followed by a region that contains four conserved cysteines. It is suggested that the calcium-binding region of cadherins is located in the extracellular repeats.

Cadherins are evolutionary related to the desmogleins which are component of intercellular desmosome junctions involved in the interaction of plaque proteins:

- Desmoglein 1 (desmosomal glycoprotein I).
- Desmoglein 2.
- Desmoglein 3 (Pemphigus vulgaris antigen).

The Drosophila fat protein [3] is a huge protein of over 5000 amino acids that contains 34 cadherin-like repeats in its extracellular domain.

The signature pattern that was developed for the repeated domain is located in it the C-terminal extremity which is its best conserved region. The pattern includes two conserved aspartic acid residues as well as two asparagines; these residues could be implicated in the binding of calcium.

Consensus pattern[LIV]-x-[LIV]-x-D-x-N-D-[NH]-x-P Sequences known to belong to this class detected by the pattern ALL. Note this pattern is found in the first, second, and fourth copies of the repeated domain. In the third copy there is a deletion of one residue after the second conserved Asp.

[1] Takeichi M. Annu. Rev. Biochem. 59:237-252(1990).
[2] Takeichi M. Trends Genet. 3:213-217(1987).
[3] Mahoney P.A., Weber U., Onofrechuk P., Biessmann H., Bryant P.J., Goodman C.S. Cell 67:853-868(1991).

110. Calreticulin family signatures

Calreticulin [1] (also known as calregulin, CRP55 or HACBP) is a high-capacitycalcium-binding protein which is present in most tissues and located at the periphery of the endoplasmic (ER) and the sarcoplamic reticulum (SR) membranes. It probably plays a role in the storage of calcium in the lumen ofthe ER and SR and it may well have other important functions. Structurally, calreticulin is a protein of about 400 amino acid residues consisting of three domains: a) An N-terminal, probably globular, domain of about 180 amino acid residues (N-domain); b) A central domain of about 70 residues (P-domain) which contains three repeats of an acidic 17 amino acid motif. This region binds calcium with a low-capacity, but a high-affinity; c) A C-terminal domain rich in acidic residues and in lysine (C-domain). This region binds calcium with a high-capacity but a low-affinity. Calreticulin is evolutionary related to the following proteins: - Onchocerca volvulus antigen RAL-1. RAL-1 is highly similar to calreticulin, but possesses a C-terminal domain rich in lysine and arginine and lacks acidic residues and is therefore not expected to bind calcium in that region. - Calnexin [2]. A calcium-binding protein that interacts with newly synthesized glycoproteins in the endoplasmic reticulum. It seems to play a major role in the quality control apparatus of the ER by the retention

of incorrectly folded proteins. - Calmegin [3] (or calnexin-T), a testis-specific calcium-binding protein highly similar to calnexin. Three signature patterns have been developed for this family of proteins. The first two patterns are based on conserved regions in the N-domain; the third pattern corresponds to positions 4 to 16 of the repeated motif in the P-domain.
Consensus pattern: [KRHN]-x-[DEQN]-[DEQNK]-x(3)-C-G-G-[AG]-[FY]-[LIVM]-[KN]-[LIVMFY](2)-
Consensus pattern: [LIVM](2)-F-G-P-D-x-C-[AG]-
Consensus pattern: [IV]-x-D-x-[DENST]-x(2)-K-P-[DEH]-D-W-[DEN]-

[1] Michalak M., Milner R.E., Burns K., Opas M. Biochem. J. 285:681-692(1992).
[2] Bergeron J.J.M., Brenner M.B., Thomas D.Y., Williams D.B. Trends Biochem. Sci. 19:124-128(1994).
[3] Watanabe D., Yamada K., Nishina Y., Tajima Y., Koshimizu U., Nagata A., Nishimune Y. J. Biol. Chem. 269: 7744-7749(1994).

111. Eukaryotic-type carbonic anhydrases signature (carb_anhydrase)
Carbonic anhydrases (EC 4.2.1.1) (CA) [1,2,3,4] are zinc metalloenzymes which catalyze the reversible hydration of carbon dioxide. Eight enzymatic and evolutionary related forms of carbonic anhydrase are currently known to exist in vertebrates: three cytosolic isozymes (CA-I, CA-II and CA-III); two membrane-bound forms (CA-IV and CA-VII); a mitochondrial form (CA-V); a secreted salivary form (CA-VI); and a yet uncharacterized isozyme [5].In the alga Chlamydomonas reinhardtii, two CA isozymes have been sequenced[6]. They are periplasmic glycoproteins evolutionary related to vertebrate CAs. Some bacteria, such as Neisseria gonorrhoeae [7] also have a eukaryotic-type CA.CAs contain a single zinc atom bound to three conserved histidine residues. As a signature for CAs, a pattern has been developed which includes one of these zinc-binding histidines. Protein D8 from Vaccinia and other poxviruses is related to CAs but has lost two of the zinc-binding histidines as well as many otherwise conserved residues. This is also true of the N-terminal extracellular domain of some receptor-type tyrosine-protein phosphatases (see <PDOC00323>).
Consensus pattern: S-E-[HN]-x-[LIVM]-x(4)-[FYH]-x(2)-E-[LIVMGA]-H-[LIVMFA](2) [The second H is a zinc ligand]-
Note: most prokaryotic CA's as well as plant chloroplast CA's belong to another, evolutionary distinct family of proteins (see <PDOC00586

[1] Deutsch H.F. Int. J. Biochem. 19:101-113(1987).
[2] Fernley R.T. Trends Biochem. Sci. 13:356-359(1988).
[3] Tashian R.E. BioEssays 10:186-192(1989).
[4] Edwards Y. Biochem. Soc. Trans. 18:171-175(1990).
[5] Skaggs L.A., Bergenhem N.C.H., Venta P.J., Tashian R.E. Gene 126:291-292(1993).
[6] Fujiwara S., Fukuzawa H., Tachiki A., Miyachi S. Proc. Natl. Acad. Sci. U.S.A. 87:9779-9783(1990).
[7] Huang S., Xue Y., Sauer-Eriksson E., Chirica L., Lindskog S.,Jonsson B.H. 2.3.CO;2-"J. Mol. Biol. 283: 301-310(1998).

112. Caseins alpha/beta signature
Caseins [1] are the major protein constituent of milk. Caseins can be classified into two families; the first consists of the kappa-caseins, and the second groups the alpha-s1, alpha-s2, and beta-caseins. The alpha/beta caseins are a rapidly diverging family of proteins. However two regions are conserved: a cluster of phosphorylated serine residues and the signal sequence. The signature pattern has been developed for this family of proteins based upon the last eight residues of the signal sequence.
Consensus pattern: C-L-[LV]-A-x-A-[LVF]-A -

[1] Holt C., Sawyer L. Protein Eng. 2:251-259(1988).

113. Catalase signatures
Catalase (EC 1.11.1.6) [1,2,3] is an enzyme, present in all aerobic cells,that decomposes hydrogen peroxide to molecular oxygen and water. Its main function is to protect cells from the toxic effects of hydrogen peroxide. In eukaryotic organisms and in some prokaryotes catalase is a molecule composed of four identical subunits. Each of the subunits binds one protoheme IX group. A conserved tyrosine serves as the heme proximal side ligand. The region around this residue has been used as a first signature pattern; it also includes a conserved arginine that participates in heme-binding. A conserved histidine has been shown to be important for the catalytic mechanism of the enzyme. The region around this residue has been selected as a second signature pattern.-
Consensus pattern: R-[LIVMFSTAN]-F-[GASTNP]-Y-x-D-[AST]-[QEH] [Y is the proximal heme-binding ligand]
Consensus pattern: [IF]-x-[RH]-x(4)-[EQ]-R-x(2)-H-x(2)-[GAS]-[GASTF]-[GAST] [H is an active site residue]

Note: some prokaryotic catalases belong to the peroxidase family (see <PDOC00394>).

[1] Murthy M.R.N., Reid T.J. III, Sicignano A., Tanaka N., Rossmann M.G. J. Mol. Biol. 152:465-499(1981).
[2] Melik-Adamyan W.R., Barynin V.V., Vagin A.A., Borisov V.V., Vainshtein B.K., Fita I., Murthy M.R.N., Rossmann M.G. J. Mol. Biol. 188:63-72(1986).
[3] von Ossowki I., Hausner G., Loewen P.C. J. Mol. Evol. 37:71-76(1993).

114. (chitin binding) Chitin recognition or binding domain signature
A conserved domain of 43 amino acids is found in several plant and fungal proteins that have a common binding specificity for oligosaccharides ofN-acetylglucosamine [1]. This domain may be involved in the recognition or binding of chitin subunits. It has been found in the proteins listed below. - A number of non-leguminous plant lectins. The best characterized of these lectins are the three highly homologous wheat germ agglutinins (WGA-1, 2 and 3). WGA is an N-acetylglucosamine/N-acetylneuraminic acid binding lectin which structurally consists of a fourfold repetition of the 43 amino acid domain. The same type of structure is found in a barley root-specific lectin as well as a rice lectin. - Plants endochitinases (EC 3.2.1.14) from class IA (see <PDOC00620>). Endochitinases are enzymes that catalyze the hydrolysis of the beta-1,4 linkages of N-acetyl glucosamine polymers of chitin. Plant chitinases function as a defense against chitin containing fungal pathogens. Class IA chitinases generally contain one copy of the chitin-binding domain at their N-terminal extremity. An exception is agglutinin/chitinase [2] from the stinging nettle Urtica dioica which contains two copies of the domain. - Hevein [5], a wound-induced protein found in the latex of rubber trees. - Win1 and win2, two wound-induced proteins from potato. - Kluyveromyces lactis killer toxin alpha subunit [3]. The toxin encoded by the linear plasmid pGKL1 is composed of three subunits: alpha, beta, and gamma. The gamma subunit harbors toxin activity and inhibits growth of sensitive yeast strains in the G1 phase of the cell cycle; the alpha subunit, which is proteolytically processed from a larger precursor that also contains the beta subunit, is a chitinase (see <PDOC00839>). In chitinases, as well as in the potato wound-induced proteins, the 43-residuedomain directly follows the signal sequence and is therefore at the N-terminal of the mature protein; in the killer toxin alpha subunit it is located in the central section of the protein. The domain contains eight conserved cysteine residues which have all been shown, in WGA, to be involved in disulfide bonds. The topological arrangement of the four disulfide bonds is shown in the following figure: +-------------+ +----|------+ |||| xxCgxxxxxxxCxxxxCCsxxgxCgxxxxx-CxxxCxxxxC | ******|************* |||| +----+ +--------------+'C': conserved cysteine involved in a disulfide bond.'*': position of the pattern.

- Consensus pattern: C-x(4,5)-C-C-S-x(2)-G-x-C-G-x(4)-[FYW]-C [The five C's are involved in disulfide bonds]

[1] Wright H.T., Sandrasegaram G., Wright C.S. J. Mol. Evol. 33:283-294(1991).
[2] Lerner D.R., Raikhel N.V. J. Biol. Chem. 267:11085-11091(1992).
[3] Butler A.R., O'Donnel R.W., Martin V.J., Gooday G.W., Stark M.J.R. Eur. J. Biochem. 199:483-488(1991).

115. (Chitinase 1) Chitinases family 19 signatures
Chitinases (EC 3.2.1.14) [1] are enzymes that catalyze the hydrolysis of thebeta-1,4-N-acetyl-D-glucosamine linkages in chitin polymers. From the viewpoint of sequence similarity chitinases belong to either family 18 or 19 in the classification of glycosyl hydrolases [2,E1]. Chitinases of family 19(also known as classes IA or I and IB or II) are enzymes from plants that function in the defense against fungal and insect pathogens by destroying their chitin-containing cell wall. Class IA/I and IB/II enzymes differ in the presence (IA/I) or absence (IB/II) of a N-terminal chitin-binding domain (seethe relevant entry <PDOC00025>). The catalytic domain of these enzymes consist of about 220 to 230 amino acid residues. Two highly conserved regions have been selected as signature patterns, the first one is located in the N-terminal section and contains one of the six cysteines which are conserved in most, if not all, of these chitinases and which is probably involved in a disulfide bond.
Consensus pattern: C-x(4,5)-F-Y-[ST]-x(3)-[FY]-[LIVMF]-x-A-x(3)-[YF]-x(2)-F- [GSA]
Consensus pattern: [LIVM]-[GSA]-F-x-[STAG](2)-[LIVMFY]-W-[FY]-W-[LIVM]

[1] Flach J., Pilet P.-E., Jolles P. Experientia 48:701-716(1992).
[2] Henrissat B. Biochem. J. 280:309-316(1991).

116. chloroa_b-bind
Chlorophyll A-B binding proteins. Number of members: 211

117. chromo
The 'chromo' (CHRromatin Organization MOdifier) domain [1 to 4] is a conserved region of about 60 amino acids

which was originally found in Drosophila modifiers of variegation, which are proteins that modify the structure of chromatin to the condensed morphology of heterochromatin, a cytologically visible condition where gene expression is repressed. In protein Polycomb, the chromo domain has been shown to be important for chromatin targeting. Proteins that contains a chromo domain seem to fall into three classes:

a) Proteins which have a N-terminal chromo domain followed by a region which is related to but distinct from the chromo domain and which has been termed [3] the 'chromo shadow' domain.
b) Proteins with a single chromo domain.
c) Proteins with paired tandem chromo domains.

Currently, this domain has been found in the following proteins:

Class A.

- Drosophila heterochromatin protein Su(var)205 (HP1).
- Human heterochromatin protein HP1 alpha.
- Mammalian modifier 1 and modifier 2.
- Fission yeast swi6, a protein involved in the repression of the silent mating-type loci mat2 and mat3.

Class B.

- Drosophila protein Polycomb (Pc).
- Mammalian modifier 3, a homolog of Pc.
- Drosophila protein Su(var)3-9, a suppressor of position-effect variegation.
- Human Mi-2 autoantigen, characterisitic of dermatomyosis.
- Fungal retrotranposon polyproteins: 'skippy' from Fusarium oxysporum, 'grasshopper'and 'MAGGY' from Magnaporthe grisea and CfT-1 from Cladosporium fulvum.
- Fission yeast hypothetical protein SpAC18G6.02c.
- Caenorhabditis elegans hypothetical protein C29H12.5
- Caenorhabditis elegans hypothetical protein ZK1236.2.
- Caenorhabditis elegans hypothetical protein T09A5.8.

Class C.

- Mammalian DNA-binding/helicase proteins CHD-1 to CHD-4.
- Yeast protein CHD1.

The signature pattern for this domain corresponds to its best conserved section, which is located in its central part.

- Consensus pattern: [FYL]-x-[LIVMC]-[KR]-W-x-[GDNR]-[FYWLME]-x(5,6)-[ST]-W-[ESV]-[PSTDEN]-x(2,3)-[LIVMC]

[1] Paro R. Trends Genet. 6:416-421 (1990).
[2] Singh P.B., Miller J.R., Pearce J., Kothary R., Burton R.D., Paro R., James T.C., Gaunt S.J. Nucleic Acids Res. 19:789-794(1991).
[3] Aasland R., Stewart A.F. Nucleic Acids Res. 23:3168-3173(1995).
[4] Koonin E.V., Zhou S., Lucchesis J.C. Nucleic Acids Res. 23:4229-4233(1995).

118. citrate_synt
Citrate synthase (EC 4.1.3.7) (CS) is the tricarboxylic acid cycle enzyme that catalyzes the synthesis of citrate from oxaloacetate and acetyl-CoA in an aldol condensation. CS can directly form a carbon-carbon bond in the absence of metal ion cofactors.
In prokaryotes, citrate synthase is composed of six identical subunits. In eukaryotes, there are two isozymes of citrate synthase: one is found in the mitochondrial matrix, the second is cytoplasmic. Both seem to be dimers of identical chains.
There are a number of regions of sequence similarity between prokaryotic and eukaryotic citrate synthases. One of the best conserved contains a histidine which is one of three residues shown [1] to be involved in the catalytic mechanism of the vertebrate mitochondrial enzyme. This region has been used as a signature pattern.

- Consensus pattern: G-[FYA]-[GA]-H-x-[IV]-x(1,2)-[RKT]-x(2)-D-[PS]-R [H is an active site residue]

[1] Karpusas M., Branchaud B., Remington S.J. Biochemistry 29:2213-2219(1990).

119. clpA_B
Chaperonin clpA/B
CAUTION! This family is a subfamily of the AAA superfamily. The threshold has been set very high to stop overlaps with the AAA superfamily. This entry will be subsumed by AAA in the future.
Number of members: 39
A number of ATP-binding proteins that are are thought to protect cells from extreme stress by controlling the aggregation of denaturation of vital cellular structures have been shown [1,2] to be evolutionary related. These proteins are listed below.

- Escherichia coli clpA, which acts as the regulatory subunit of the ATP-dependent protease clp.
- Rhodopseudomonas blastica clpA homolog.
- Escherichia coli heat shock protein clpB and homologs in other bacteria.
- Bacillus subtilis protein mecB.
- Yeast heat shock protein 104 (gene HSP104), which is vital for tolerance to heat, ethanol and other stresses.
- Neurospora heat shock protein hsp98.
- Yeast mitochondrial heat shock protein 78 (gene HSP78) [3].
- CD4A and CD4b, two highly related tomato proteins that seem to be located in the chloroplast.
- Trypanosoma brucei protein clp.
- Porphyra purpurea chloroplast encoded clpC.

The size of these proteins range from 84 Kd (clpA) to slightly more than 100 Kd (HSP104). They all share two conserved regions of about 200 amino acids that each contains an ATP-binding site. In addition to the ATP-binding A and B motifs there are many parts in these two domains that are also conserved. Two of these regions have been selected as signature patterns. The first signature is located in the first domain, some ten residues to the C-terminal of the ATP-binding B motif. The second pattern is located in the second domain inbetween the ATP-binding A and B motifs.

- Consensus pattern: D-[AI]-[SGA]-N-[LIVMF](2)-K-[PT]-x-L-x(2)-G
- Consensus pattern: R-[LIVMFY]-D-x-S-E-[LIVMFY]-x-E-[KRQ]-x-[STA]-x-[STA]-[KR]-[LIVM]-x-G-[STA]

[1] Gottesman S., Squires C., Pichersky E., Carrington M., Hobbs M., Mattick J.S., Dalrymple B., Kuramitsu H., Shiroza T., Foster T., Clark W.P., Ross B., Squires C.L., Maurizi M.R. Proc. Natl. Acad. Sci. U.S.A. 87:3513-3517 (1990).
[2] Parsell D.A., Sanchez Y., Stitzel J.D., Lindquist S. Nature 353:270-273(1991).
[3] Leonhardt S.A., Fearon K., Danese P.N., Mason T.L. Mol. Cell. Biol. 13:6304-6313(1993).

120. cofilin_ADF
Cofilin/tropomyosin-type actin-binding proteins

[1]
Medline: 97290449 Structure determination of yeast cofilin. Fedorov AA, Lappalainen P, Fedorov EV, Drubin DG, Almo SC; Nat Struct Biol 1997;4:366-369.
[2]
Medline: 97290450 Crystal structure of the actin-binding protein actophorin from Acanthamoeba. Leonard SA, Gittis AG, Petrella EC, Pollard TD, Lattman EE; Nat Struct Biol 1997;4:369-373.
[3]
Medline: 97420794 F-actin and G-actin binding are uncoupled by mutation of conserved tyrosine residues in maize actin depolymerizing factor. Jiang CJ, Weeds AG, Khan S, Hussey PJ; Proc Natl Acad Sci U S A 1997; 94:9973-9978.
[4]
Medline: 97357155 Cofilin promotes rapid actin filament turnover in vivo. Lappalainen P, Drubin DG; Nature 1997;388:78-82.
Severs actin filaments and binds to actin monomers.
Number of members: 44

Actin-depolymerizing proteins sever actin filaments (F-actin) and/or bind to actin monomers, or G-actin, thus preventing actin-polymerization by sequestering the monomers. The following proteins are evolutionary related and belong to a family of low molecular weight (137 to 166 residues) actin-depolymerizing proteins [1,2,3,4]:

- Cofilin from vertebrates, slime mold and yeast. Cofilin binds to F-actin and acts as a pH-dependent actin-depolymerizing protein.
- Destrin from vertebrates. Destrin binds to G-actin in a pH-independent manner and prevents polymerization.
- Caenorhabditis elegans unc-60.
- Acanthamoeba castellanii actophorin.
- Plants actin depolymerizing factor (ADF).

The most conserved region of these proteins is a twenty amino-acid segment that ends some 30 residues from their C-terminal extremity. This segment has been shown [5] to be important for actin-binding.

- Consensus pattern: P-[DE]-x-[SA]-x-[LIVMT]-[KR]-x-[KR]-M-[LIVM]-[YA]-[STA](3)-x(3)-[LIVMF]-[KR]

[1] Hawkins M., Pope B., MacIver S.K., Weeds A.G. Biochemistry 32:9985-9993(1993).
[2] Iida K., Moriyama K., Matsumoto S., Kawasaki H., Nishida E., Yahara I. Gene 124:115-120(1993).
[3] Quirk S., MacIver S.K., Ampe C., Doberstein S.K., Kaiser D.A., van Damme J., Vandekerckhove J., Pollard T.D. Biochemistry 32:8525-8533(1993).
[4] McKim K.S., Matheson C., Marra M.A., Wakarchuk M.F., Baillie D.L. Mol. Gen. Genet. 242:346-357(1994).
[5] Moriyama K., Yonezawa N., Sakai H., Yahara I., Nishida E. J. Biol. Chem. 267:7240-7244(1992).

121. (Complex 24kd) Respiratory-chain NADH dehydrogenase 24 Kd subunit signature Respiratory-chain NADH dehydrogenase (EC 1.6.5.3) [1,2] (also known as complexI or NADH-ubiquinone oxidoreductase) is an oligomeric enzymatic complex located in the inner mitochondrial membrane which also seems to exist inthe chloroplast and in cyanobacteria (as a NADH-plastoquinone oxidoreductase).Among the 25 to 30 polypeptide subunits of this bioenergetic enzyme complex there is one with a molecular weight of 24 Kd (in mammals), which is a component of the iron-sulfur (IP) fragment of the enzyme. It seems to bind a2Fe-2S iron-sulfur cluster. The 24 Kd subunit is nuclear encoded, as aprecursor form with a transit peptide in mammals, and in Neurospora crassa.The 24 Kd subunit is highly similar to [3,4]: - Subunit E of Escherichia coli NADH-ubiquinone oxidoreductase (gene nuoE). - Subunit NQO2 of Paracoccus denitrificans NADH-ubiquinone oxidoreductase. A highly conserved region, located in the central section of this subunit containing two conserved cysteines that are probably involved in the binding of the 2Fe-2S center has been selected as a signature pattern.

- Consensus pattern: D-x(2)-F-[ST]-x(5)-C-L-G-x-C-x(2) [GA]-P [The two C's are putative 2Fe-2S ligands]

[1] Ragan C.I. Curr. Top. Bioenerg. 15:1-36(1987).
[2] Weiss H., Friedrich T., Hofhaus G., Preis D. Eur. J. Biochem. 197:563-576(1991).
[3] Feamley I.M., Walker J.E. Biochim. Biophys. Acta 1140:105-134(1992).
[4] Weidner U., Geier S., Ptock A., Friedrich T., Leif H., Weiss H. J. Mol. Biol. 233:109-122(1993).

122. copper-bind
Copper binding proteins, plastocyanin/azurin family
Number of members: 70
Blue or 'type-1' copper proteins are small proteins which bind a single copper atom and which are characterized by an intense electronic absorption band near 600 nm [1,2]. The most well known members of this class of proteins are the plant chloroplastic plastocyanins, which exchange electrons with cytochrome c6, and the distantly related bacterial azurins, which exchange electrons with cytochrome c551. This family of proteins also includes all the proteins listed below (references are only provided for recently determined sequences).

- Amicyanin from bacteria such as Methylobacterium extorquens or Thiobacillus versutus that can grow on methylamine. Amicyanin appears to be an electron receptor for methylamine dehydrogenase.
- Auracyanins A and B from Chloroflexus aurantiacus [3]. These proteins can donate electrons to cytochrome c-554.
- Blue copper protein from Alcaligenes faecalis.
- Cupredoxin (CPC) from cucumber peelings [4].
- Cusacyanin (basic blue protein; plantacyanin, CBP) from cucumber.

- Halocyanin from Natrobacterium pharaonis [5], a membrane associated copper-binding protein.
- Pseudoazurin from Pseudomonas.
- Rusticyanin from Thiobacillus ferrooxidans. Rusticyanin is an electron carrier from cytochrome c-552 to the a-type oxidase [6].
- Stellacyanin from the Japanese lacquer tree.
- Umecyanin from horseradish roots.
- Allergen Ra3 from ragweed. This pollen protein is evolutionary related to the above proteins, but seems to have lost the ability to bind copper.

Although there is an appreciable amount of divergence in the sequence of all these proteins, the copper ligand sites are conserved and a pattern which includes two of the ligands (a cysteine and a histidine) has been developed.

- Consensus pattern: [GA]-x(0,2)-[YSA]-x(0,1)-[VFY]-x-C-x(1,2)-[PG]-x(0,1)-H-x(2,4)-[MQ] [C and H are copper ligands]

[1] Garret T.P.J., Clingeleffer D.J., Guss J.M., Rogers S.J., Freeman H.C. J. Biol. Chem. 259:2822-2825(1984).
[2] Ryden L.G., Hunt L.T. J. Mol. Evol. 36:41-66(1993).
[3] McManus J.D., Brune D.C., Han J., Sanders-Loehr J., Meyer T.E., Cusanovich M.A., Tollin G., Blankenship R.E. J. Biol. Chem. 267:6531-6540(1992).
[4] Mann K., Schaefer W., Thoenes U., Messerschmidt A., Mehrabian Z., Nalbandyan R. FEBS Lett. 314:220-223 (1992).
[5] Mattar S., Scharf B., Kent S.B.H., Rodewald K., Oesterhelt D., Engelhard M. J. Biol. Chem. 269:14939-14945 (1994).
[6] Yano T., Fukumori Y., Yamanaka T. FEBS Lett. 288:159-162(1991).

123. Chaperonins cpn10 signature
Chaperonins [1,2] are proteins involved in the folding of proteins or the assembly of oligomeric protein complexes. They seem to assist other polypeptides in maintaining or assuming conformations which permit their correct assembly into oligomeric structures. They are found in abundance in prokaryotes, chloroplasts and mitochondria. Chaperonins form oligomeric complexes and are composed of two different types of subunits: a 60 Kd protein, known as cpn60 (groEL in bacteria) and a 10 Kd protein, known ascpn 10 (groES in bacteria).The cpn10 protein binds to cpn60 in the presence of MgATP and suppresses the ATPase activity of the latter. Cpn10 is a protein of about 100 amino acid residues whose sequence is well conserved in bacteria, vertebrate mitochondriaand plants chloroplast [3,4]. Cpn10 assembles as an heptamer that forms a dome[5]. As a signature pattern for cpn10, a region located in the N-terminal section of the protein was selected.
Consensus pattern: [LIVMFY]-x-P-[ILT]-x-[DEN]-[KR]-[LIVMFA](3)-[KREQ]-x(8,9)-[SG]-x-[LIVMFY](3)-
Note: this pattern is found twice in the plant chloroplast protein which consist of the tandem repeat of a cpn10 domain

[1] Ellis R.J., van der Vies S.M. Annu. Rev. Biochem. 60:321-347(1991).
[2] Zeilsta-Ryalls J., Fayet O., Georgopoulos C. Annu. Rev. Microbiol. 45:301-325(1991).
[3] Hartman D.J., Hoogenraad N.J., Condron R., Hoj P.B. Proc. Natl. Acad. Sci. U.S.A. 89:3394-3398(1992).
[4] Bertsch U., Soll J., Seetharam R., Viitanen P.V. Proc. Natl. Acad. Sci. U.S.A. 89:8696-8700(1992).
[5] Hunt J.F., Weaver A.J., Landry S.J., Gierasch L., Deisenhofer J. Nature 379:37-45(1996).

124. Chaperonins cpn60 signature (cpn60_TCP1)
Chaperonins [1,2] are proteins involved in the folding of proteins or the assembly of oligomeric protein complexes. Their role seems to be to assist other polypeptides to maintain or assume conformations which permit their correct assembly into oligomeric structures. They are found in abundance in prokaryotes, chloroplasts and mitochondria. Chaperonins form oligomeric complexes and are composed of two different types of subunits: a 60 Kd protein, known as cpn60 (groEL in bacteria) and a 10 Kd protein, known as cpn10 (groES in bacteria).The cpn60 protein shows weak ATPase activity and is a highly conserved protein of about 550 to 580 amino acid residues which has been described by different names in different species: - Escherichia coli groEL protein, which is essential for the growth of the bacteria and the assembly of several bacteriophages. - Cyanobacterial groEL analogues. - Mycobacterium tuberculosis and leprae 65 Kd antigen, Coxiella burnetti heat shock protein B (gene htpB), Rickettsia tsutsugamushi major antigen 58, and Chlamydial 57 Kd hypersensitivity antigen (gene hypB). - Chloroplast RuBisCO subunit binding-protein alpha and beta chains, which bind ribulose bisphosphate carboxylase small and large subunits and are implicated in the assembly of the enzyme oligomer. - Mammalian mitochondrial matrix protein P1 (mitonin or P60). - Yeast HSP60 protein, a mitochondrial assembly factor. As a signature pattern for these proteins,

a rather well-conserved region of twelve residues, located in the last third of the cpn60sequence was chosen.
Consensus pattern: A-[AS]-x-[DEQ]-E-x(4)-G-G-[GA]-

[1] Ellis R.J., van der Vies S.M. Annu. Rev. Biochem. 60:321-347(1991).
[2] Zeilsta-Ryalls J., Fayet O., Georgopoulos C. Annu. Rev. Microbiol. 45:301-325(1991).

Chaperonins TCP-1 signatures (cpn60_TCP1)
The TCP-1 protein [1,2] (Tailless Complex Polypeptide 1) was first identified in mice where it is especially abundant in testis but present in all cell types. It has since been found and characterized in many other mammalian species, in Drosophila and in yeast. TCP-1 is a highly conserved protein of about 60 Kd (556 to 560 residues) which participates in a hetero-oligomeric900 Kd double-torus shaped particle [3] with 6 to 8 other different subunits. These subunits, the chaperonin containing TCP-1 (CCT) subunit beta, gamma,delta, epsilon, zeta and eta are evolutionary related to TCP-1 itself [4,5].The CCT is known to act as a molecular chaperone for tubulin, actin and probably some other proteins. The CCT subunits are highly related to archebacterial counterparts: - TF55 and TF56 [6], a molecular chaperone from Sulfolobus shibatae. TF55 has ATPase activity, is known to bind unfolded polypeptides and forms a oligomeric complex of two stacked nine-membered rings. - Thermosome [7], from Thermoplasma acidophilum. The thermosome is composed of two subunits (alpha and beta) and also seems to be a chaperone with ATPase activity. It forms an oligomeric complex of eight-membered rings. The TCP-1 family of proteins are weakly, but significantly [8], related to thecpn60/groEL chaperonin family (see <PDOC00268>).As signature patterns of this family of chaperonins, three conserved regions located in the N-terminal domain were chosen.
Consensus pattern: [RKEL]-[ST]-x-[LMFY]-G-P-x-[GSA]-x-x-K-[LIVMF](2)-
Consensus pattern: [LIVM]-[TS]-[NK]-D-[GA]-[AVNHK]-[TAV]-[LIVM](2)-x(2)-[LIVM]-x-[LIVM]-x-[SNH]-[PQH]-
Consensus pattern: Q-[DEK]-x-x-[LIVMGTA]-[GA]-D-G-T-

[1] Ellis J. Nature 358:191-192(1992).
[2] Nelson R.J., Craig E.A. Curr. Biol. 2:487-489(1992).
[3] Lewis V.A., Hynes G.M., Zheng D., Saibil H., Willison K.R. Nature 358:249-252(1992).
[4] Kubota H., Hynes G., Carne A., Ashworth A., Willison K.R. Curr. Biol. 4:89-99(1994)
[5] Kim S., Willison K.R., Horwich A.L. Trends Biochem. Sci. 20:543-548(1994).
[6] Trent J.D., Nimmesgern E., Wall J.S., Hartl F.U., Horwich A.L. Nature 354:490-493(1991).
[7] Waldmann T., Lupas A., Kellermann J., Peters J., Baumeister W. Biol. Chem. Hoppe-Seyler 376:119-126 (1995).
[8] Hemmingsen S.M. Nature 357:650-650(1992).

125. cyclin (Cyclins)
The cyclins include an internal duplication, which is related to that found in TFIIB and the RB protein.

[1]
Medline: 94203808 Evidence for a protein domain superfamily shared by the cyclins, TFIIB and RB/p 107. Gibson TJ, Thompson JD, Blocker A, Kouzarides T; Nucleic Acids Res 1994;22:946-952.
[2]
Medline: 96164440 The crystal structure of cyclin A Brown NR, Noble MEM, Endicott JA, Garman EF, Wakatsuki S, Mitchell E, Rasmussen B, Hunt T, Johnson LN; Structure. 1995;3:1235-1247.
Complex of cyclin and cyclin dependant kinase.
[3]
Medline: 96313126 Structural basis of cyclin-dependant kinase activation by phosphorylation. Russo AA, Jeffrey PD, Pavletich NP; Nat Struct Biol. 1996;3:696-700.
Cyclins regulate cyclin dependant kinases (CDKs).
The most divergent prosite members have been included. Swiss:P22674 the Uracil-DNA glycosylase 2 is the highest noise and may be related but has not been included.
Number of members: 189

Cyclins [1,2,3] are eukaryotic proteins which play an active role in controlling nuclear cell division cycles. Cyclins, together with the p34 (cdc2) or cdk2 kinases, form the Maturation Promoting Factor (MPF). There are two main groups of cyclins:

- G2/M cyclins, essential for the control of the cell cycle at the G2/M (mitosis) transition. G2/M cyclins accumulate

steadily during G2 and are abruptly destroyed as cells exit from mitosis (at the end of the M-phase).

- G1/S cyclins, essential for the control of the cell cycle at the G1/S (start) transition.

In most species, there are multiple forms of G1 and G2 cyclins. For example, in vertebrates, there are two G2 cyclins, A and B, and at least three G1 cyclins, C, D, and E.
A cyclin homolog has also been found in herpesvirus saimiri [4].
The best conserved region is in the central part of the cyclins' sequences, known as the 'cyclin-box'. From this, a 32 residue pattern has been derived.

- Consensus pattern: R-x(2)-[LIVMSA]-x(2)-[FYWS]-[LIVM]-x(8)-[LIVMFC]-x(4)-[LIVMFYA]-x(2)-[STAGC]-[LIVMFYQ]-x-[LIVMFYC]-[LIVMFY]-D-[RKH]-[LIVMFYW]

[1] Nurse P. Nature 344:503-508(1990).
[2] Norbury C., Nurse P. Curr. Biol. 1:23-24(1991).
[3] Lew D.J., Reed S.I. Trends Cell Biol. 2:77-81(1992).
[4] Nicholas J., Cameron K.R., Honess R.W. Nature 355:362-365(1992).

126. Cystatin domain
This is a very diverse family. Attempts to define separate subfamilies have failed. Typically, either the N-terminal or C-terminal end is very divergent. But splitting into two domains would make very short families. Cathelicidins are related to this family but have not been included. Number of members: 147
Inhibitors of cysteine proteases [1,2,3], which are found in the tissues and body fluids of animals, in the larva of the worm Onchocerca volvulus [4], as well as in plants, can be grouped into three distinct but related families:

- Type 1 cystatins (or stefins), molecules of about 100 amino acid residues with neither disulfide bonds nor carbohydrate groups.
- Type 2 cystatins, molecules of about 115 amino acid residues which contain one or two disulfide loops near their C-terminus.
- Kininogens, which are multifunctional plasma glycoproteins.

They are the precursor of the active peptide bradykinin and play a role in blood coagulation by helping to position optimally prekallikrein and factor XI next to factor XII. They are also inhibitors of cysteine proteases. Structurally, kininogens are made of three contiguous type-2 cystatin domains, followed by an additional domain (of variable length) which contains the sequence of bradykinin. The first of the three cystatin domains seems to have lost its inhibitory activity.
In all these inhibitors, there is a conserved region of five residues which has been proposed to be important for the binding to the cysteine proteases. The consensus pattern starts one residue before this conserved region.

- Consensus pattern: [GSTEQKRV]-Q-[LIVT]-[VAF]-[SAGQ]-G-x-[LIVMNK]-x(2)-[LIVMFY]-x-[LIVMFYA]-[DENQKRHSIV]

[1] BarrettA.J. Trends Biochem. Sci. 12:193-196(1987).
[2] Rawlings N.D., Barrett A.J. J. Mol. Evol. 30:60-71 (1990).
[3] Turk V., Bode W. FEBS Lett. 285:213-219(1991).
[4] Lustigman S., Brotman B., Huima T., Prince A.M. Mol. Biochem. Parasitol. 45:65-76(1991).

127. cytochrome_c (Cytochrome c)
The Pfam entry does not include all prosite members.
The cytochrome 556 and cytochrome c' families are not included.
Number of members: 259
In proteins belonging to cytochrome c family [1], the heme group is covalently attached by thioether bonds to two conserved cysteine residues. The consensus sequence for this site is Cys-X-X-Cys-His and the histidine residue is one of the two axial ligands of the heme iron. This arrangement is shared by all proteins known to belong to cytochrome c family, which presently includes cytochromes c, c', c1 to c6, c550 to c556, cc3/Hmc, cytochrome f and reaction center cytochrome c.

- Consensus pattern: C-{CPWHF}-{CPWR}-C-H-{CFYW}

[1] Mathews F.S. Prog. Biophys. Mol. Biol. 45:1-56(1985).

128. (DAGKa) Diacylglycerol kinase accessory domain (presumed)
Diacylglycerol (DAG) is a second messenger that acts as a protein kinase C activator. This domain is assumed to be an accessory domain: its function is unknown.

[1] Sakane F, Yamada K, Kanoh H, Yokoyama C, Tanabe T, Nature 1990;344:345-348.
[2] Sakane F, Imai S, Kai M, Wada I, Kanoh H, J Biol Chem 1996;271:8394-8401.
[3] Schaap D, de Widt J, van der Wal J, Vandekerckhove J, van, Damme J, Gussow D, Ploegh HL, van Blitterswijk WJ, van der, Bend RL, FEBS Lett 1990;275:151-158.
[4] Kanoh H, Yamada K, Sakane F, Trends Biochem Sci 1990;15:47-50.

129. (DAGKc) Diacylglycerol kinase catalytic domain (presumed)
Diacylglycerol (DAG) is a second messenger that acts as a protein kinase C activator. The catalytic domain is assumed from the finding of bacterial homologues.

[1] Sakane F, Yamada K, Kanoh H, Yokoyama C, Tanabe T, Nature 1990;344:345-348.
[2] Sakane F, Imai S, Kai M, Wada I, Kanoh H, J Biol Chem 1996;271:8394-8401.
[3] Schaap D, de Widt J, van der Wal J, Vandekerckhove J, van, Damme J, Gussow D, Ploegh HL, van Blitterswijk WJ, van der, Bend RL, FEBS Lett 1990;275:151-158.
[4] Kanoh H, Yamada K, Sakane F, Trends Biochem Sci 1990;15:47-50.

130. D-amino acid oxidases signature(DAO)
D-amino acid oxidase (EC 1.4.3.3) (DAMOX or DAO) is an FAD flavoenzyme that catalyzes the oxidation of neutral and basic D-amino acids into their corresponding keto acids. DAOs have been characterized and sequenced in fungi and vertebrates where they are known to be located in the peroxisomes. D-aspartate oxidase (EC 1.4.3.1) (DASOX) [1] is an enzyme, structurally related to DAO, which catalyzes the same reaction but is active only toward dicarboxylic D-amino acids. In DAO, a conserved histidine has been shown [2] to be important for the enzyme's catalytic activity. The conserved region around this residue has been developed as a signature pattern for these enzymes.
Consensus pattern: [LIVM](2)-H-[NHA]-Y-G-x-[GSA](2)-x-G-x(5)-G-x-A [H is a probable active site residue]o-

[1] Negri A., Ceciliani F., Tedeschi G., Simonic T., Ronchi S. J. Biol. Chem. 267:11865-11871(1992).
[2] Miyano M., Fukui K., Watanabe F., Takahashi S., Tada M., Kanashiro M., Miyake Y. J. Biochem. 109:171-177 (1991).

131. DEAD and DEAH box families ATP-dependent helicases signatures
A number of eukaryotic and prokaryotic proteins have been characterized [1,2,3] on the basis of their structural similarity. They all seem to be involved in ATP-dependent, nucleic-acid unwinding. Proteins currently known to belong to this family are: - Initiation factor eIF-4A. Found in eukaryotes, this protein is a subunit of a high molecular weight complex involved in 5'cap recognition and the binding of mRNA to ribosomes. It is an ATP-dependent RNA-helicase. - PRP5 and PRP28. These yeast proteins are involved in various ATP-requiring steps of the pre-mRNA splicing process. - P110, a mouse protein expressed specifically during spermatogenesis. - An3, a Xenopus putative RNA helicase, closely related to P110. - SPP81/DED1 and DBP1, two yeast proteins probably involved in pre-mRNA splicing and related to P110. - Caenorhabditis elegans helicase glh-1. - MSS116, a yeast protein required for mitochondrial splicing. - SPB4, a yeast protein involved in the maturation of 25S ribosomal RNA. - p68, a human nuclear antigen. p68 has ATPase and DNA-helicase activities in vitro. It is involved in cell growth and division. - Rm62 (p62), a Drosophila putative RNA helicase related to p68. - DBP2, a yeast protein related to p68. - DHH1, a yeast protein. - DRS1, a yeast protein involved in ribosome assembly. - MAK5, a yeast protein involved in maintenance of dsRNA killer plasmid. - ROK1, a yeast protein. - ste 13, a fission yeast protein. - Vasa, a Drosophila protein important for oocyte formation and specification of embryonic posterior structures. - Me31B, a Drosophila maternally expressed protein of unknown function. - dbpA, an Escherichia coli putative RNA helicase. - deaD, an Escherichia coli putative RNA helicase which can suppress a mutation in the rpsB gene for ribosomal protein S2. - rhlB, an Escherichia coli putative RNA helicase. - rhlE, an Escherichia coli putative RNA helicase. - srmB, an Escherichia coli protein that shows RNA-dependent ATPase activity. It probably interacts with 23 S ribosomal RNA. - Caenorhabditis elegans hypothetical proteins T26G10.1, ZK512.2 and ZK686.2. - Yeast hypothetical protein YHR065c. - Yeast hypothetical protein YHR169w. - Fission yeast hypothetical protein SpAC31A2.07c. - Bacillus subtilis hypothetical protein yxiN. All these proteins share a number of conserved sequence motifs. Some of them are specific to this

family while others are shared by other ATP-binding proteins or by proteins belonging to the helicases 'superfamily' [4,E1]. One of these motifs, called the 'D-E-A-D-box', represents a special version of the B motif of ATP-binding proteins. Some other proteins belong to a subfamily which have His instead of the second Asp and are thus said to be 'D-E-A-H-box' proteins [3,5,6,E1]. Proteins currently known to belong to this subfamily are: - PRP2, PRP16, PRP22 and PRP43. These yeast proteins are all involved in various ATP-requiring steps of the pre-mRNA splicing process. - Fission yeast prh1, which my be involved in pre-mRNA splicing. - Male-less (mle), a Drosophila protein required in males, for dosage compensation of X chromosome linked genes. - RAD3 from yeast. RAD3 is a DNA helicase involved in excision repair of DNA damaged by UV light, bulky adducts or cross-linking agents. Fission yeast rad15 (rhp3) and mammalian DNA excision repair protein XPD (ERCC-2) are the homologs of RAD3. - Yeast CHL1 (or CTF1), which is important for chromosome transmission and normal cell cycle progression in G(2)/M. - Yeast TPS1. - Yeast hypothetical protein YKL078w. - Caenorhabditis elegans hypothetical proteins C06E1.10 and K03H1.2. - Poxviruses' early transcription factor 70 Kd subunit which acts with RNA polymerase to initiate transcription from early gene promoters. - I8, a putative vaccinia virus helicase. - hrpA, an Escherichia coli putative RNA helicase. Signature patterns for both subfamilies were developed.
Consensus pattern: [LIVMF](2)-D-E-A-D-[RKEN]-x-[LIVMFYGSTN
Consensus pattern: [GSAH]-x-[LIVMF](3)-D-E-[ALIV]-H-[NECR]
Note: proteins belonging to this family also contain a copy of the ATP/GTP- binding motif 'A' (P-loop) (see the relevant entry <pDOC00017

[1] Schmid S.R., Linder P. Mol. Microbiol. 6:283-292(1992).
[2] Linder P., Lasko P., Ashburner M., Leroy P., Nielsen P.J., Nishi K., Schnier J., Slonimski P.P. Nature 337: 121-122(1989).
[3] Wassarman D.A., Steitz J.A. Nature 349:463-464(1991).
[4] Hodgman T.C. Nature 333:22-23(1988) and Nature 333:578-578(1988) (Errata).
[5] Harosh I., Deschavanne P. Nucleic Acids Res. 19:6331-6331(1991).
[6] Koonin E.V., Senkevich T.G. J. Gen. Virol. 73:989-993(1992).

132. (DHBP_synthase) 3,4-dihydroxy-2-butanone 4-phosphate synthase
3,4-Dihydroxy-2-butanone 4-phosphate is biosynthesized from ribulose 5-phosphate and serves as the biosynthetic precursor for the xylene ring of riboflavin. Sometimes found as a bifunctional enzyme with GTP_cyclohydro2.
Richter G, Krieger C, Volk R, Kis K, Ritz H, Gotze E, Bacher A, Methods Enzymol 1997;280:374-382.

133. (DHDPS) Dihydrodipicolinate synthetase signatures
Dihydrodipicolinate synthetase (EC 4.2.1.52) (DHDPS) [1] catalyzes, in higher plants chloroplast and in many bacteria (gene dapA), the first reaction specific to the biosynthesis of lysine and of diaminopimelate. DHDPS is responsible for the condensation of aspartate semialdehyde and pyruvate by aping-pong mechanism in which pyruvate first binds to the enzyme by forming a Schiff-base with a lysine residue. Three other proteins are structurally related to DHDPS and probably also act via a similar catalytic mechanism: - Escherichia coli N-acetylneuraminate lyase (EC 4.1.3.3) (gene nanA), which catalyzes the condensation of N-acetyl-D-mannosamine and pyruvate to form N-acetylneuraminate. - Rhizobium meliloti protein mosA [3], which is involved in the biosynthesis of the rhizopine 3-o-methyl-scyllo-inosamine. - Escherichia coli hypothetical protein yjhH. Two signature patterns for these enzymes were developed . The first one is centered on highly conserved region in the N-terminal part of these proteins. The second signature contains a lysine residue which has been shown, in Escherichia coli dapA [2], to be the one that forms a Schiff-base with the substrate.
Consensus pattern: [GSA]-[LIVM]-[LIVMFY]-x(2)-G-[ST]-[TG]-G-E-[GASNF]-x(6)-[EQ] -
Consensus pattern: Y-[DNS]-[LIVMFA]-P-x(2)-[ST]-x(3)-[LIVMG]-x(13,14)-[LIVM]- x-[SGA]-[LIVMF]-K-[DEQAF]-[STAC] [K is involved in Schiff-base formation]-

[1] Kaneko T., Hashimoto T., Kumpaisal R., Yamada Y. J. Biol. Chem. 265:17451-17455(1990).
[2] Laber B., Gomis-Rueth F.-X., Romao M.J., Huber R. Biochem. J. 288:691-695(1992).
[3] Murphy P.J., Trenz S.P., Grzemski W., de Bruijn F.J., Schell J. J. Bacteriol. 175:5193-5204 (1993).

134. (DHOdehase) Dihydroorotate dehydrogenase signatures
Dihydroorotate dehydrogenase (EC 1.3.3.1) (DHOdehase) catalyzes the fourth step in the de novo biosynthesis of pyrimidine, the conversion of dihydroorotate into orotate. DHOdehase is a ubiquitous FAD flavoprotein. In bacteria (gene pyrD), DHOdease is located on the inner side of the cytosolic membrane. In some yeasts, such as in Saccharomyces cerevisiae (gene URA1), it is a cytosolic protein while in other eukaryotes it is found in the mitochondria [1]. The sequence of DHOdease is rather well conserved and two signature patterns were developed specific to

this enzyme. The first corresponds to a region in the N-terminal section of the enzyme while the second is located in the C-terminal section and seems to be part of the FAD-binding domain.
Consensus pattern[GS]-x(4)-[GK]-[GSTA]-[LIVFSTA]-[GT]-x(3)-[NQR]-x-G-[NHY]-x(2)-P-[RT]
Consensus pattern[LIVM](2)-[GSA]-x-G-G-[IV]-x-[STGDN]-x(3)-[ACV]-x(6)-G-A

[1] Nagy M., Lacroute F., Thomas D. Proc. Natl. Acad. Sci. U.S.A. 89:8966-8970(1992).

135. (DMRL_synthase) 6,7-dimethyl-8-ribityllumazine synthase

136. (DNA_methylase) C-5 cytosine-specific DNA methylases signatures C-5 cytosine-specific DNA methylases (EC 2.1.1.73) (C5 Mtase) are enzymes that specifically methylate the C-5 carbon of cytosines in DNA [1,2,3]. Such enzymes are found in the proteins described below. - As a component of type II restriction-modification systems in prokaryotes and some bacteriophages. Such enzymes recognize a specific DNA sequence where they methylate a cytosine. In doing so, they protect DNA from cleavage by type II restriction enzymes that recognize the same sequence. The sequences of a large number of type II C-5 Mtases are known. - In vertebrates, there are a number of C-5 Mtases that methylate CpG dinucleotides. The sequence of the mammalian enzyme is known.C-5 Mtases share a number of short conserved regions. Two of them were selected. The first is centered around a conserved Pro-Cys dipeptide in which the cysteine has been shown [4] to be involved in the catalytic mechanism; it appears to form a covalent intermediate with the C6 position of cytosine. The second region is located at the C-terminal extremity in type-II enzymes
Consensus pattern: [DENKS]-x-[FLIV]-x(2)-[GSTC]-x-P-C-x(2)-[FYWLIM]-S [C is the active site residue]-
Consensus pattern: [RKQGTF]-x(2)-G-N-[STAG]-[LIVMF]-x(3)-[LIVMT]-x(3)-[LIVM]-x(3)-[LIVM]-

[1] Posfai J., Bhagwat A.S., Roberts R.J. Gene 74:261-263(1988).
[2] Kumar S., Cheng X., Klimasauskas S., Mi S., Posfai J., Roberts R.J., Wilson G.G. Nucleic Acids Res. 22: 1-10(1994).
[3] Lauster R., Trautner T.A., Noyer-Weidner M. J. Mol. Biol. 206:305-312(1989).
[4] Chen L., McMillan A.M., Chang W., Ezak-Nipkay K., Lane W.S., Verdine G.L. Biochemistry 30:11018-11025 (1991).

137. (DNAphotolyase) DNA photolyases class 2 signatures
Deoxyribodipyrimidine photolyase (EC 4.1.99.3) (DNA photolyase) [1,2] is a DNArepair enzyme. It binds to UV-damaged DNA containing pyrimidine dimers and, upon absorbing a near-UV photon (300 to 500 nm), breaks the cyclobutane ring joining the two pyrimidines of the dimer. DNA photolyase is an enzyme that requires two choromophore-cofactors for its activity: a reduced FADH2 and either 5,10-methenyltetrahydrofolate (5,10-MTFH) or an oxidized 8-hydroxy-5-deazaflavin (8-HDF) derivative (F420). The folate or deazaflavin chromophore appears to function as an antenna, while the FADH2 chromophore is thought to be responsible for electron transfer. On the basis of sequence similarities[3] DNA photolyases can be grouped into two classes. The second class contains enzymes from Myxococcus xanthus, methanogenic archaebacteria, insects, fish and marsupial mammals. It is not yet known what second cofactor is bound to class 2 enzymes. There are a number of conserved sequence regions in all known class 2 DNAphotolyases, especially in the C-terminal part. Two of these regions were selected as signature patterns.
Consensus pattern: F-x-E-E-x-[LIVM](2)-R-R-E-L-x(2)-N-F-
Consensus pattern: G-x-H-D-x(2)-W-x-E-R-x-[LIVM]-F-G-K-[LIVM]-R-[FY]-M-N-

[1] Sancar G.B., Sancar A. Trends Biochem. Sci. 12:259-261(1987).
[2] Jorns M.S. Biofactors 2:207-211(1990).
[3] Yasui A., Eker A.P.M., Yasuhira S., Yajima H., Kobayashi T., Takao M., Oikawa A. EMBO J. 13:6143-6151 (1994).

(DNAphotolyase2) DNA photolyases class 1 signatures
Deoxyribodipyrimidine photolyase (EC 4.1.99.3) (DNA photolyase) [1,2] is a DNA repair enzyme. It binds to UV-damaged DNA containing pyrimidine dimers and ,upon absorbing a near-UV photon (300 to 500 nm), breaks the cyclobutane ring joining the two pyrimidines of the dimer. DNA photolyase is an enzyme that requires two choromophore-cofactors for its activity: a reduced FADH2 and either 5,10-methenyltetrahydrofolate (5,10-MTFH) or an oxidized 8-hydroxy-5-deazaflavin (8-HDF) derivative (F420). The folate or deazaflavin chromophore appears to function as an antenna, while the FADH2 chromophore is thought to be responsible for electron transfer. On the basis of sequence similarities[3] DNA photolyases can be grouped into two classes. The first class contains enzymes from Gram-negative and Gram-positive bacteria, the halophilic archaebacteria Halobacterium halobium,

fungi and plants. Class 1 enzymes bind either 5,10-MTHF (E.coli, fungi, etc.) or 8-HDF (S.griseus, H.halobium).This family also includes Arabidopsis cryptochromes 1 (CRY1) and 2 (CRY2),which are blue light photoreceptors that mediate blue light-induced gene expression. There are a number of conserved sequence regions in all known class 1 DNA photolyases, especially in the C-terminal part. Two of these regions were selected as signature patterns

Consensus pattern: T-G-x-P-[LIVM](2)-D-A-x-M-[RA]-x-[LIVM]-

Consensus pattern: [DN]-R-x-R-[LIVM](2)-x-[STA](2)-F-[LIVMFA]-x-K-x-L-x(2,3)- W-[KRQ]-

[1] Sancar G.B., Sancar A. Trends Biochem. Sci. 12:259-261(1987).
[2] Jorns M.S. Biofactors 2:207-211(1990).
[3] Yasui A., Eker A.P.M., Yasuhira S., Yajima H., Kobayashi T., Takao M., Oikawa A. EMBO J. 13:6143-6151 (1994).
[4] Lin C., Ahmad M., Cashmore A.R. Plant J. 10:893-902(1996).

138. (DNA_pol I_A)

DNA polymerase family A signature

Replicative DNA polymerases (EC 2.7.7.7) are the key enzymes catalyzing the accurate replication of DNA. They require either a small RNA molecule or a protein as a primer for the de novo synthesis of a DNA chain. On the basis of sequence similarities a number of DNA polymerases have been grouped together [1,2,3] under the designation of DNA polymerase family A. The polymerases that belong to this family are listed below.

- Escherichia coli and various other bacterial polymerase I (gene polA).
- Thermus aquaticus Taq polymerase.
- Bacteriophage sp01 polymerase.
- Bacteriophage sp02 polymerase.
- Bacteriophage T5 polymerase.
- Bacteriophage T7 polymerase.
- Mycobacteriophage L5 polymerase.
- Yeast mitochondrial polymerase gamma (gene MIP1).

Five regions of similarity are found in all the above polymerases. One of these conserved regions, known as 'motif B' [1], is located in a domain which, in Escherichia coli polA, has been shown to bind deoxynucleotide triphosphate substrates; it contains a conserved tyrosine which has been shown, by photo- affinity labelling, to be in the active site; a conserved lysine, also part of this motif, can be chemically labelled, using pyridoxal phosphate. This conserved region was used as a signature for this family of DNA polymerases.

Consensus patternR-x(2)-[GSAV]-K-x(3)-[LIVMFY]-[AGQ]-x(2)-Y-x(2)-[GS]-x(3)-[LIVMA] Sequences known to belong to this class detected by the pattern ALL.

[1] Delarue M., Poch O., Todro N., Moras D., Argos P. Protein Eng. 3:461-467(1990).
[2] Ito J., Braithwaite D.K. Nucleic Acids Res. 19:4045-4057(1991).
[3] Braithwaite D.K., Ito J. Nucleic Acids Res. 21:787-802(1993).

139. DNA_pol_viral_C

DNA polymerase (viral) C-terminal domain

Number of members: 128

140. (DNA_topoisoII)

DNA topoisomerase II signature

DNA topoisomerase I (EC 5.99.1.2) [1,2,3,4,E1] is one of the two types of enzyme that catalyze the interconversion of topological DNA isomers. Type II topoisomerases are ATP-dependent and act by passing a DNA segment through a transient double-strand break. Topoisomerase II is found in phages, archaebacteria, prokaryotes, eukaryotes, and in African Swine Fever virus (ASF). In bacteriophage T4 topoisomerase II consists of three subunits (the product of genes 39, 52 and 60). In prokaryotes and in archaebacteria the enzyme, known as DNA gyrase, consists of two subunits (genes gyrA and gyrB [E2]). In some bacteria, a second type II topoisomerase has been identified; it is known as topoisomerase IV and is required for chromosome segregation, it also consists of two subunits (genes parC and parE). In eukaryotes, type II topoisomerase is a homodimer.

There are many regions of sequence homology between the different subtypes of topoisomerase II. The relation between the different subunits is shown in the following representation:

<----------------About-1400-residues---------------------->

[----------Protein 39-*-----][----Protein 52----] Phage T4

[----------gyrB-------*-----][--------gyrA-----------------] Prokaryote II

Archaebacteria

[----------parE-------*-----][--------parD-----------------] Prokaryote IV

[--------------------*-----------------------------------] Eukaryote and

ASF

'*': Position of the pattern.

As a signature pattern for this family of proteins, a region that contains a highly conserved pentapeptide was selected. The pattern is located in gyrB, in parE, and in protein 39 of phage T4 topoisomerase.
Consensus pattern[LIVMA]-x-E-G-[DN]-S-A-x-[STAG] Sequences known to belong to this class detected by the pattern ALL.


[1] Sternglanz R. Curr. Opin. Cell Biol. 1:533-535(1990).
[2] Bjornsti M.-A. Curr. Opin. Struct. Biol. 1:99-103(1991).
[3] Sharma A., Mondragon A. Curr. Opin. Struct. Biol. 5:39-47(1995).
[4] Roca J. Trends Biochem. Sci. 20:156-160(1995).


141. (DSPc) Tyrosine specific protein phosphatases signature and profiles
Tyrosine specific protein phosphatases (EC 3.1.3.48) (PTPase) [1 to 5] are enzymes that catalyze the removal of a phosphate group attached to a tyrosine residue. These enzymes are very important in the control of cell growth, proliferation, differentiation and transformation. Multiple forms of PTPase have been characterized and can be classified into two categories: soluble PTPases and transmembrane receptor proteins that contain PTPase domain (s). The currently known PTPases are listed below: Soluble PTPases. - PTPN1 (PTP-1B). - PTPN2 (T-cell PTPase; TC-PTP). - PTPN3 (H1) and PTPN4 (MEG), enzymes that contain an N-terminal band 4.1- like domain (see <PDOC00566>) and could act at junctions between the membrane and cytoskeleton. - PTPN5 (STEP). - PTPN6 (PTP-1C; HCP; SHP) and PTPN11 (PTP-2C; SH-PTP3; Syp), enzymes which contain two copies of the SH2 domain at its N-terminal extremity. The Drosophila protein corkscrew (gene csw) also belongs to this subgroup. - PTPN7 (LC-PTP; Hematopoietic protein-tyrosine phosphatase; HePTP). - PTPN8 (70Z-PEP). - PTPN9 (MEG2). - PTPN12 (PTP-G1; PTP-P19). - Yeast PTP1. - Yeast PTP2 which may be involved in the ubiquitin-mediated protein degradation pathway. - Fission yeast pyp1 and pyp2 which play a role in inhibiting the onset of mitosis. - Fission yeast pyp3 which contributes to the dephosphorylation of cdc2. - Yeast CDC 14 which may be involved in chromosome segregation. - Yersinia virulence plasmid PTPAses (gene yopH). - Autographa californica nuclear polyhedrosis virus 19 Kd PTPase.Dual specificity PTPases. - DUSP1 (PTPN10; MAP kinase phosphatase-1; MKP-1); which dephosphorylates MAP kinase on both Thr-183 and Tyr-185. - DUSP2 (PAC-1), a nuclear enzyme that dephosphorylates MAP kinases ERK1 and ERK2 on both Thr and Tyr residues. - DUSP3 (VHR). - DUSP4 (HVH2). - DUSP5 (HVH3). - DUSP6 (Pyst1; MKP-3). - DUSP7 (Pyst2; MKP-X). - Yeast MSG5, a PTPase that dephosphorylates MAP kinase FUS3. - Yeast YVH1. - Vaccinia virus H1 PTPase; a dual specificity phosphatase. Receptor PTPases. Structurally, all known receptor PTPases, are made up of a variable length extracellular domain, followed by a transmembrane region and a C-terminalcatalytic cytoplasmic domain. Some of the receptor PTPases contain fibronectintype III (FN-III) repeats, immunoglobulin-like domains, MAM domains orcarbonic anhydrase-like domains in their extracellular region. The cytoplasmic region generally contains two copies of the PTPAse domain. The first seems to have enzymatic activity, while the second is inactive but seems to affect substrate specificity of the first. In these domains, the catalytic cysteine is generally conserved but some other, presumably important, residues are not. In the following table, the domain structure of known receptor PTPases is shown: Extracellular Intracellular ----- Ig FN-3 CAH MAM PTPaseLeukocyte common antigen (LCA) (CD45) 0 2 0 0 2Leukocyte antigen related (LAR) 3 8 0 0 2 Drosophila DLAR 3900 2Drosophila DPTP 2 2 0 0 2PTP-alpha (LRP) 0 0 0 0 2PTP-beta 0 16 0 0 1PTP-gamma 0 1 1 0 2PTP-delta 0 >7 0 0 2 PTP-epsilon 0 0 0 0 2PTP-kappa 1 4 0 1 2PTP-mu 1 4 0 1 2PTP-zeta 0110 2PTPase domains consist of about 300 amino acids. There are two conserved cysteines, the second one has been shown to be

absolutely required for activity. Furthermore, a number of conserved residues in its immediate vicinity have also been shown to be important. A signature pattern for PTPase domains was derived centered on the active site cysteine. There are three profiles for PTPases, the first one spans the complete domain and is not specific to any subtype. The second profile is specific to dual-specificity PTPases and the third one to the PTP subfamily Consensus pattern: [LIVMF]-H-C-x(2)-G-x(3)-[STC]-[STAGP]-x-[LIVMFY] [C is the active site residue]-

[1] Fischer E.H., Charbonneau H., Tonks N.K. Science 253:401-406(1991).
[2] Charbonneau H., Tonks N.K. Annu. Rev. Cell Biol. 8:463-493(1992).
[3] Trowbridge I.S. J. Biol. Chem. 266:23517-23520(1991).
[4] Tonks N.K., Charbonneau H. Trends Biochem. Sci. 14:497-500(1989).
[5] Hunter T. Cell 58:1013-1016(1989).

142. (DUF10) Uncharacterized protein family UPF0076 signature
The following uncharacterized proteins have been shown [1] to share regions of similarities:

- Goat antigen UKl 14, a human homolog and the rat corresponding protein which is known as perchloric acid soluble protein (PSP1). PSP1 [2] may inhibit an initiation stage of cell-free protein synthesis. - Mouse heat-responsive protein HRSP12. - Yeast chromosome V hypothetical protein YER057c. - Yeast chromosome IX hypothetical protein YIL051c. - Caenorhabditis elegans hypothetical protein C23G10.2. - Escherichia coli hypothetical protein ycdK. - Escherichia coli hypothetical protein yhaR. - Escherichia coli hypothetical protein yjgF and HI0719, the corresponding Haemophilus influenzae protein. - Escherichia coli hypothetical protein yoaB. - Bacillus subtilis hypothetical protein yabJ. - Haemophilus influenzae hypothetical protein HI1627. - Helicobacter pylori hypothetical protein HP0944.
- Lactococcus lactis aldR. - Myxococcus xanthus dfrA. - Synechocystis strain PCC 6803 hypothetical protein slr0709. - Rhizobium strain NGR234 symbiotic plasmid hypothetical protein y4sK. - Pyrococcus horikoshii hypothetical protein PH0854.These are small proteins of around 15 Kd whose sequence is highly conserved.As a signature pattern, a well conserved region located in the C-terminal part of these proteins was selected.

Consensus pattern: [PA]-[ASTPV]-R-[SACVF]-x-[LIVMFY]-x(2)-[GSAKR]-x-[LMVA]-x(5,8)-[LIVM]-E-[MI]-

[1] Bairoch A. Unpublished observations (1995).
[2] Oka T., Tsuji H., Noda C., Sakai K., Hong Y.-M., Suzuki I., Munoz S., Natori Y. J. Biol. Chem. 270:30060-30067 (1995).

143. (DUF3)Domain of Unknown Function 3
Domain apparently occurring exclusively in eubacteria. Unknown function.

144. (DUF6) Integral membrane protein
This family includes many hypothetical membrane proteins of unknown function. Many of the proteins contain two copies of the aligned region.

145. (DUF7) Integral membrane protein
This family includes many hypothetical membrane proteins of unknown function. Swiss:P14502 has been implicated in resistance to ethidium bromide.

146. (DapB) Dihydrodipicolinate reductase signature
Dihydrodipicolinate reductase (EC 1.3.1.26) catalyzes the second step in the biosynthesis of diaminopimelic acid and lysine, the NAD or NADP-dependent reduction of 2,3-dihydrodipicolinate into 2,3,4,5-tetrahydrodipicolinate. This enzyme is present in bacteria (gene dapB) and higher plants. As a signature pattern the best conserved region in this enzyme was selected. It is located in the central section and is part of the substrate-binding region [1].
Consensus pattern: E-[IV]-x-E-x-H-x(3)-K-x-D-x-P-S-G-T-A-

[1] Scapin G., Blanchard J.S., Sacchettini J.C. Biochemistry 34:3502-3512(1995).

147. DedA family
This family combines the DedA related proteins and YIAN/YGIK family. Members of this family are not functionally characterised. These proteins contain multiple predicted transmembrane regions.

148. DegT/DnrJ/EryC1/StrS family

The members of this family exhibit some characteristics of the sensor protein of two-component signal transduction systems, however none of the members show any sequence similarity to these protein kinases. The members of this family do have the typical helix-turn-helix motif of DNA binding proteins.

[1] Stutzman-Engwall KJ, Otten SL, Hutchinson CR, J Bacteriol 1992;174:144-154.

149. (Desaturase) Fatty acid desaturases signatures

Fatty acid desaturases (EC 1.14.99.-) are enzymes that catalyze the insertion of a double bond at the delta position of fatty acids. There seems to be two distinct families of fatty acid desaturases which do not seem to be evolutionary related. Family 1 is composed of: -

Stearoyl-CoA desaturase (SCD) (EC 1.14.99.5) [1]. SCD is a key regulatory enzyme of unsaturated fatty acid biosynthesis. SCD introduces a cis double bond at the delta(9) position of fatty acyl-CoA's such as palmitoleoyl- and oleoyl-CoA. SCD is a membrane-bound enzyme that is thought to function as a part of a multienzyme complex in the endoplasmic reticulum of vertebrates and fungi. As a signature pattern for this family a conserved region in the C-terminal part of these enzymes was selected, this region is rich in histidine residues and in aromatic residues. Family 2 is composed of: - Plants stearoyl-acyl-carrier-protein desaturase (EC 1.14.99.6) [2], these enzymes catalyze the introduction of a double bond at the delta(9) position of steraoyl-ACP to produce oleoyl-ACP. This enzyme is responsible for the conversion of saturated fatty acids to unsaturated fatty acids in the synthesis of vegetable oils. - Cyanobacteria desA [3] an enzyme that can introduce a second cis double bond at the delta(12) position of fatty acid bound to membranes glycerolipids. DesA is involved in chilling tolerance; the phase transition temperature of lipids of cellular membranes being dependent on the degree of unsaturation of fatty acids of the membrane lipids. As a signature pattern for this family a conserved region in the C-terminal part of these enzymes was selected.

Consensus pattern: G-E-x-[FY]-H-N-[FY]-H-H-x-F-P-x-D-Y-

Consensus pattern: [ST]-[SA]-x(3)-[QR]-[LI]-x(5,6)-D-Y-x(2)-[LIVMFYW]-[LIVM]-[DE]-

[1] Kaestner K.H., Ntambi J.M., Kelly T.J. Jr., Lane M.D. J. Biol. Chem. 264:14755-14761(1989).
[2] Shanklin J., Somerville C.R. Proc. Natl. Acad. Sci. U.S.A. 88:2510-2514(1991).
[3] Wada H., Gombos Z., Murata N. Nature 347:200-203(1990).

150. Dihydroorotase signatures

Dihydroorotase (EC 3.5.2.3) (DHOase) catalyzes the third step in the de novo biosynthesis of pyrimidine, the conversion of ureidosuccinic acid (N-carbamoyl-L-aspartate) into dihydroorotate. Dihydroorotase binds a zinc ion which is required for its catalytic activity [1]. In bacteria, DHOase is a dimer of identical chains of about 400 amino-acid residues (gene pyrC). In higher eukaryotes, DHOase is part of a large multi-functional protein known as 'rudimentary' in Drosophila and CAD in mammals and which catalyzes the first three steps of pyrimidine biosynthesis [2]. The DHOase domain is located in the central part of this polyprotein. In yeasts, DHOase is encoded by a monofunctional protein (gene URA4). However, a defective DHOase domain [3] is found in a multifunctional protein (gene URA2) that catalyzes the first two steps of pyrimidine biosynthesis. The comparison of DHOase sequences from various sources shows [4] that there are two highly conserved regions. The first located in the N-terminal extremity contains two histidine residues suggested [3] to be involved in binding the zinc ion. The second is found in the C-terminal part. Signature patterns for both regions have been developed. Allantoinase (EC 3.5.2.5) is the enzyme that hydrolyzes allantoin intoallantoate. In yeast (gene DAL1) [5], it is the first enzyme in the allanto indegradation pathway; in amphibians [6] and fish it catalyzes the second step in the degradation of uric acid. The sequence of allantoinase is evolutionary related to that of DHOases.

Consensus pattern: D-[LIVMFYWSAP]-H-[LIVA]-H-[LIVF]-[RN]-x-[PGANF] [The two H's are probable zinc ligands]-

Consensus pattern: [GA]-[ST]-D-x-A-P-H-x(4)-K-

[1] Brown D.C., Collins K.D. J. Biol. Chem. 266:1597-1604(1991).
[2] Davidson J.N., Chen K.C., Jamison R.S., Musmanno L.A., Kern C.B. BioEssays 15:157-164(1993).
[3] Souciet J.-L., Nagy M., Le Gouar M., Lacroute F., Potier S. Gene 79:59-70(1989).
[4] Guyonvarch A., Nguyen-Juilleret M., Hubert J.-C., Lacroute F. Mol. Gen. Genet. 212:134-141(1988).
[5] Buckholz R.G., Cooper T.G. Yeast 7:913-923(1991).
[6] Hayashi S., Jain S., Chu R., Alvares K., Xu B., Erfurth F., Usuda N., Rao M.S., Reddy S.K., Noguchi T., Reddy J.K., Yeldandi A.Y. J. Biol. Chem. 269:12269-12276(1994).

151. dnaJ domains signatures and profile

The prokaryotic heat shock protein dnaJ interacts with the chaperone hsp70-like dnaK protein [1]. Structurally, the

dnaJ protein consists of an N- terminal conserved domain (called 'J' domain) of about 70 amino acids, a glycine-rich region ('G' domain') of about 30 residues, a central domain containing four repeats of a CXXCXGXG motif ('CRR' domain) and a C-terminal region of 120 to 170 residues. Such a structure is shown in the following schematic representation:
+--------+-+-----+-----+--------+------------------+ |N-terminal || Gly-R ||CXXCXGXG |C-terminal | +---------+-+-------+-----+----------+------------+
It has been shown [2] that the 'J' domain as well as the 'CRR' domain are also found in other prokaryotic and eukaryotic proteins which are listed below.

a) Proteins containing both a 'J' and a 'CRR' domain:

- Yeast protein MAS5/YDJ1 which seems to be involved in mitochondrial protein import.
- Yeast protein MDJ1, involved in mitochondrial biogenesis and protein folding.
- Yeast protein SCJ1, involved in protein sorting.
- Yeast protein XDJ1.
- Plants dnaJ homologs (from leek and cucumber).
- Human HDJ2, a dnaJ homolog of unknown function.
- Yeast hypothetical protein YNL077w.

b) Proteins containing a 'J' domain without a 'CRR' domain:

- Rhizobium fredii nolC, a protein involved in cultivar-specific nodulation of soybean.
- Escherichia coli cbpA [3], a protein that binds curved DNA.
- Yeast protein SEC63/NPLI, important for protein assembly into the endoplasmic reticulum and the nucleus.
- Yeast protein SIS 1, required for nuclear migration during mitosis.
- Yeast protein CAJ1.
- Yeast hypothetical protein YFR041 c.
- Yeast hypothetical protein YIR004w.
- Yeast hypothetical protein YJL162c.
- Plasmodium falciparum ring-infected erythrocyte surface antigen (RESA). RESA, whose function is not known, is associated with the membrane skeleton of newly invaded erythrocytes.
- Human HDJ1.
- Human HSJ1, a neuronal protein.
- Drosophila cysteine-string protein (csp).

A signature pattern for the 'J' domain was developed, based on conserved positions in the C-terminal half of this domain. A pattern for the 'CRR' domain, based on the first two copies of that motif was also developed. A profile for the 'J' domain was also developed.
Consensus pattern: [FY]-x(2)-[LIVMA]-x(3)-[FYWHNT]-[DENQSA]-x-L-x-[DN]-x(3)-[KR]-x(2)-[FYI]-
Consensus pattern: C-[DEGSTHKR]-x-C-x-G-x-[GK]-[AGSDM]-x(2)-[GSNKR]-x(4,6)-C-x(2,3)-C-x-G-x-G-

[1] Cyr D.M., Langer T., Douglas M.G. Trends Biochem. Sci. 19:176-181(1994).
[2] Bork P., Sander C., Valencia A., Bukau B. Trends Biochem. Sci. 17:129-129(1992).
[3] Ueguchi C., Kaneda M., Yamada H., Mizuno T. Proc. Natl. Acad. Sci. U.S.A. 91:1054-1058(1994).

152.

153. Dwarfin
This family known as the dwarfins also includes the drosophila protein MAD. The N-terminus of MAD can bind to DNA [2].

[1] Yingling JM, Das P, Savage C, Zhang M, Padgett RW, Wang XF, Proc Natl Acad Sci U S A 1996;93:8940-8944.
[2] Kim J, Johnson K, Chen HJ, Carroll S, Laughon A, Nature 1997;388:304-308.

154. Dynein light chain type 1 signature
Dynein is a multisubunit microtubule-dependent motor enzyme that acts as the force generating protein of eukaryotic cilia and flagella. The cytoplasmic isoform of dynein acts as a motor for the intracellular retrograde motility of vesicles and organelles along microtubules. Dynein is composed of a number of ATP-binding large subunits, intermediate

size subunits and small subunits. Among the small subunits, there is a family [1,2] of highly conserved proteins which consist of: - Chlamydomonas reinhardtii flagellar outer arm dynein 8 Kd and 11 Kd light chains. - Higher eukaryotes cytoplasmic dynein light chain 1. - Yeast cytoplasmic dynein light chain 1 (gene DYN2 or SLC1). - Caenorhabditis elegans hypothetical dynein light chains M18.2 and T26A5.9.These proteins are have from 89 to 120 amino acids. As a signature pattern, A highly conserved region was selected.
Consensus pattern: H-x-I-x-G-[KR]-x-F-[GA]-S-x-V-[ST]-[HY]-E -

[1] King S.M., Patel-King R.S. J. Biol. Chem. 270:11445-11452(1995).
[2] Dick T., Ray K., Salz H.K., Chia W. Mol. Cell. Biol. 16:1966-1977(1996).

155. dUTPase
dUTPase hydrolyzes dUTP to dUMP and pyrophosphate.

[1] Cedergren-Zeppezauer ES, Larsson G, Nyman PO, Dauter Z, Wilson KS, Nature 1992;355:740-743.
[2] Mol CD, Harris JM, McIntosh EM, Tainer JA, Structure 1996;4:1077-1092.

156. (dCMP cyt deam) Cytidine and deoxycytidylate deaminases zinc-binding region signature
Cytidine deaminase (EC 3.5.4.5) (cytidine aminohydrolase) catalyzes the hydrolysis of cytidine into uridine and ammonia while deoxycytidylatedeaminase (EC 3.5.4.12) (dCMP deaminase) hydrolyzes dCMP into dUMP. Both enzymes are known to bind zinc and to require it for their catalytic activity[1,2]. These two enzymes do not share any sequence similarity with the exception of a region that contains three conserved histidine and cysteine residues which are thought to be involved in the binding of the catalytic zincion. Such a region is also found in other proteins [3,4]: - Yeast cytosine deaminase (EC 3.5.4.1) (gene FCY1) which transforms cytosine into uracil. - Mammalian apolipoprotein B mRNA editing protein, responsible for the postranscriptional editing of a CAA codon into a UAA (stop) codon in the APOB mRNA. - Riboflavin biosynthesis protein ribG, which converts 2,5-diamino-6- (ribosylamino)-4(3H)-pyrimidinone 5'-phosphate into 5-amino-6-(ribosylamino)-2,4(1H,3H)-pyrimidinedione 5'-phosphate. - Bacillus cereus blasticidin-S deaminase (EC 3.5.4.23), which catalyzes the deamination of the cytosine moiety of the antibiotics blasticidin S, cytomycin and acetylblasticidin S. - Bacillus subtilis protein comEB. This protein is required for the binding and uptake of transforming DNA. - Bacillus subtilis hypothetical protein yaaJ. - Escherichia coli hypothetical protein yfhC. - Yeast hypothetical protein YJL035c. A signature pattern for this zinc-binding region was derived.
Consensus pattern: [CH]-[AGV]-E-x(2)-[LIVMFGAT]-[LIVM]-x(17,33)-P-C-x(2,8)-C-x(3)-[LIVM] [The C's and H are zinc ligands

[1] Yang C., Carlow D., Wolfenden R., Short S.A. Biochemistry 31:4168-4174(1992).
[2] Moore J.T., Silversmith R.E., Maley G.F., Maley F. J. Biol. Chem. 268:2288-2291(1993).
[3] Reizer J., Buskirk S., Bairoch A., Reizer A., Saier M.H. Jr. Protein Sci. 3:853-856(1994).
[4] Bhattacharya S., Navaratnam N., Morrison J.R., Scott J., Taylow W.R. Trends Biochem. Sci. 19:105-106 (1994).

157. Dehydrins signatures
A number of proteins are produced by plants that experience water-stress. Water-stress takes place when the water available to a plant falls below a critical level. The plant hormone abscisic acid (ABA) appears to modulate the response of plant to water-stress. Proteins that are expressed during water-stress are called dehydrins [1,2] or LEA group 2 proteins [3]. The proteins that belong to this family are listed below.

- Arabidopsis thaliana XERO 1, XERO 2 (LTI30), RAB18, ERD10 (LTI45) ERD14 and COR47.
- Barley dehydrins B8, B9, B17, and B18.
- Cotton LEA protein D-11.
- Craterostigma plantagineum dessication-related proteins A and B.
- Maize dehydrin M3 (RAB-17).
- Pea dehydrins DHN1, DHN2, and DHN3.
- Radish LEA protein.
- Rice proteins RAB 16B, 16C, 16D, RAB21, and RAB25.
- Tomato TAS 14.
- Wheat dehydrin RAB 15 and cold-shock protein cor410, cs66 and cs120.

Dehydrins share a number of structural features. One of the most notable features is the presence, in their central

region, of a continuous run of five to nine serines followed by a cluster of charged residues. Such a region has been found in all known dehydrins so far with the exception of pea dehydrins. A second conserved feature is the presence of two copies of alysine-rich octapeptide; the first copy is located just after the cluster of charged residues that follows the poly-serine region and the second copy is found at the C-terminal extremity. Signature patterns for both regions were derived.
Consensus pattern: S(5)-[DE]-x-[DE]-G-x(1,2)-G-x(0,1)-[KR](4
Consensus pattern: [KR]-[LIM]-K-[DE]-K-[LIM]-P-G-

[1] Close T.J., Kortt A.A., Chandler P.M. Plant Mol. Biol. 13:95-108(1989).
[2] Robertson M., Chandler P.M. Plant Mol. Biol. 19:1031-1044(1992).
[3] Dure L. III, Crouch M., Harada J., Ho T.-H. D., Mundy J., Quatrano R., Thomas T., Sung Z.R. Plant Mol. Biol. 12:475-486(1989).

158. (deoR) Bacterial regulatory proteins, deoR family signature
The many bacterial transcription regulation proteins which bind DNA through a helix-turn-helix' motif can be classified into subfamilies on the basis of sequence similarities. One of these subfamilies groups the following proteins[1,2]: - accR, the Agrobacterium tumefaciens plasmid pTiC58 repressor of opine catabolism and conjugal transfer. - agaR, the Escherichia coli aga operon putative repressor. - deoR, the Escherichia coli deoxyribose operon repressor. - fucR, the Escherichia coli L-fucose operon activator. - gatR, the Escherichia coli galactitol operon repressor. - glpR, the Escherichia coli glycerol-3-phosphate regulon repressor. - gutR (or sr1R), the Escherichia coli glucitol operon repressor. - iolR, from Bacillus subtilis. - lacR, the streptococci lactose phosphotransferase system repressor. - spoIIID, the Bacillus subtilis transcription regulator of the sigK gene. - yfjR, an Escherichia coli hypothetical protein. - ygbI, an Escherichia coli hypothetical protein. - yihW, an Escherichia coli hypothetical protein. - yjfQ, an Escherichia coli hypothetical protein. - yjhJ, an Escherichia coli hypothetical protein. The 'helix-turn-helix'DNA-binding motif of these proteins is located in the N-terminal part of the sequence. The pattern used to detect these proteins starts fourteen residues before the HTH motif and ends one residue after it.
Consensus pattern: R-x(3)-[LIVM]-x(3)-[LIVM]-x(16,17)-[STA]-x(2)-T-[LIVMA]- [RH]-[KRNA]-D-[LIVMF]-

[1] von Bodman S., Hayman G.T., Farrand S.K. Proc. Natl. Acad. Sci. U.S.A. 89:643-647(1992).
[2] Bairoch A. Unpublished observations (1993).

159. dsrm
Double-stranded RNA binding motif

[1] Burd CG, Dreyfuss G; Medline: 94310455, Conserved structures and diversity of functions of RNA-binding proteins. Science 1994;265:615-621.

Sequences gathered for seed by HMM_iterative_training Putative motif shared by proteins that bind to dsRNA. At least some DSRM proteins seem to bind to specific RNA targets. Exemplified by Staufen, which is involved in localization of at least five different mRNAs in the early Drosophila embryo. Also by interferon-induced protein kinase in humans, which is part of the cellular response to dsRNA.
Number of members: 116

160. Dynamin family signature
Dynamin [1,2] is a microtubule-associated force-producing protein of 100 Kd which is involved in the production of microtubule bundles and which is able to bind and hydrolyze GTP. Dynamin is structurally related to the following proteins: - Drosophila shibire protein (gene shi) [3]. Shibire is, very probably, the Drosophila cognate of mammalian dynamin. It seems to provide the motor for vesicular transport during endocytosis. - Yeast vacuolar sorting protein VPS1 (or SPO15) [4], a protein which could also be involved in microtubule-associated motility. - Yeast protein MGM1 [5], which is required for mitochondrial genome maintenance. - Yeast protein DNM1, which is involved in endocytosis. - Interferon induced Mx proteins [6,7]. Interferon alpha or beta induce the synthesis of a family of closely related proteins. Most of these proteins are known to confer resistance to influenza viruses and/or rhabdoviruses on transfected mammalian cell in culture. The three motifs found in all GTP-binding proteins are located in the N-terminal part of these proteins. The signature pattern that was developed for these proteins is based on a highly conserved region downstream of the ATP/GTP-binding motif 'A' (P-loop) (see <PDOC00017>).-
Consensus pattern: L-P-[RK]-G-[STN]-[GN]-[LIVM]-V-T-R-

[1] Vallee R.B., Shpetner H.S. Annu. Rev. Biochem. 59:909-932(1990).

[2] Obar R.A., Collins C.A., Hammarback J.A., Shpetner H.S., Vallee R.B. Nature 347:256-261 (1990).
[3] van der Bliek A., Meyerowitz E.M. Nature 351:411-414(1991).
[4] Rothman J.H., Raymond C.K., Gilbert T., O'Hara P.J., Stevens T.H. Cell 61:1063-1074(1990).
[5] Jones B.A., Fangman W.L. Genes Dev. 6:380-389(1992).
[6] Arnheiter H., Meier E. New Biol. 2:851-857(1990).
[7] Staeheli P., Pitossi F., Pavlovic J. Trends Cell Biol. 3:268-272(1993).

161. (dynamin_2) Dynamin central region
This region lies between the GTPase domain, see dynamin, and the pleckstrin homology (PH) domain.

162. E1-E2 ATPases phosphorylation site
E1-E2 ATPases (also known as P-type) are cation transport ATPases which form an aspartyl phosphate intermediate in the course of ATP hydrolysis. ATPases which belong to this family are listed below [1,2,3]. - Fungal and plant plasma membrane (H+) ATPases [reviewed in 4]. - Vertebrate (Na+, K+) ATPases (sodium pump) [reviewed in 5,6]. - Gastric (K+, H+) ATPases (proton pump). - Calcium (Ca++) ATPases (calcium pump) from the sarcoplasmic reticulum (SR), the endoplasmic reticulum (ER) and the plasma membrane. - Copper (Cu++) ATPases (copper pump) which are involved in two human genetic disorders: Menkes syndrome and Wilson disease [7]. - Bacterial potassium (K+) ATPases. - Bacterial cadmium efflux (Cd++) ATPases [reviewed in 8]. - Bacterial magnesium (Mg++) ATPases. - A probable cation ATPase from Leishmania. - fixI, a probable cation ATPase from Rhizobium meliloti, involved in nitrogen fixation. The region around the phosphorylated aspartate residue is perfectly conserved in all these ATPases and can be used as a signature pattern.
Consensus pattern: D-K-T-G-T-[LI]-[TI] [D is phosphorylated]

[1] Green N.M., McLennan D.H. Biochem. Soc. Trans. 17:819-822(1989).
[2] Green N.M. Biochem. Soc. Trans. 17:970-972(1989).
[3] Fagan M.J., Saier M.H. Jr. J. Mol. Evol. 38:57-99(1994).
[4] Serrano R. Biochim. Biophys. Acta 947:1-28(1988).
[5] Fambrough D.M. Trends Neurosci. 11:325-328(1988).
[6] Sweadner K.J. Biochim. Biophys. Acta 988:185-220(1989).
[7] Bull P.C., Cox D.W. Trends Genet. 10:246-251(1994).
[8] Silver S., Nucifora G., Chu L., Misra T.K. Trends Biochem. Sci. 14:76-80(1989).

163.E1_N
E1 Protein, N terminal domain
Number of members: 90

164. (El_dehydrog) Dehydrogenase E1 component
This family uses thiamine pyrophosphate as a cofactor. This family includes pyruvate dehydrogenase, 2-oxoglutarate dehydrogenase and 2-oxoisovalerate dehydrogenase.

165. (ECH) Enoyl-CoA hydratase/isomerase signature
Enoyl-CoA hydratase (EC 4.2.1.17) (ECH) [1] and 3-2trans-enoyl-CoA isomerase(EC 5.3.3.8) (ECI) [2] are two enzymes involved in fatty acid metabolism. ECH catalyzes the hydratation of 2-trans-enoyl-CoA into 3-hydroxyacyl-CoA and ECI shifts the 3- double bond of the intermediates of unsaturated fatty acid oxidation to the 2-trans position. Most eukaryotic cells have two fatty-acid beta-oxidation systems, one located in mitochondria and the other in peroxisomes. In mitochondria, ECH and ECI are separate yet structurally related monofunctional enzymes. Peroxisomes contain a trifunctional enzyme [3] consisting of an N-terminal domain that bears both ECH and ECI activity, and a C-terminal domain responsible for 3-hydroxyacyl-CoA dehydrogenase (HCDH) activity. In Escherichia coli (gene fadB) and Pseudomonas fragi (gene faoA), ECH and ECI are also part of a multifunctional enzyme which contains both a HCDH and a3-hydroxybutyryl-CoA epimerase domain [4].A number of other proteins have been found to be evolutionary related to the ECH/ECI enzymes or domains: - 3-hydroxbutyryl-coa dehydratase (EC 4.2.1.55) (crotonase), a bacterial enzyme involved in the butyrate/butanol-producing pathway. - Naphthoate synthase (EC 4.1.3.36) (DHNA synthetase) (gene menB) [5], a bacterial enzyme involved in the biosynthesis of menaquinone (vitamin K2). DHNA synthetase converts O-succinyl-benzoyl-CoA (OSB-CoA) to 1,4-dihydroxy- 2-naphthoic acid (DHNA). - 4-chlorobenzoate dehalogenase (EC 3.8.1.6) [6], a Pseudomonas enzyme which catalyzes the conversion of 4-chlorobenzoate-CoA to 4-hydroxybenzoate-CoA. - A Rhodobacter capsulatus protein of unknown function (ORF257) [7]. - Bacillus subtilis putative polyketide biosynthesis proteins pksH and pksI. - Escherichia coli carnitine racemase (gene caiD) [8]. - Escherichia coli hypothetical protein ygfG. - Yeast hypothetical protein YDR036c.As a signature pattern for these

enzymes, a conserved region richin glycine and hydrophobic residues was selected.
Consensus pattern: [LIVM]-[STA]-x-[LIVM]-[DENQRHSTA]-G-x(3)-[AG](3)-x(4)-[LIVMST]-x-[CSTA]-[DQHP]-[LIVMFY]-

[1] Minami-Ishii N., Taketani S., Osumi T., Hashimoto T. Eur. J. Biochem. 185:73-78(1989).
[2] Mueller-Newen G., Stoffel W. Biol. Chem. Hoppe-Seyler 372:613-624(1991).
[3] Palosaari P.M., Hiltunen J.K. J. Biol. Chem. 265:2446-2449(1990).
[4] Nakahigashi K., Inokuchi H. Nucleic Acids Res. 18:4937-4937(1990).
[5] Driscoll J.R., Taber H.W. J. Bacteriol. 174:5063-5071(1992).
[6] Babbitt P.C., Kenyon G.L., Matin B.M., Charest H., Sylvestre M., Scholten J.D., Chang K.-H., Liang P.-H., Dunaway-Mariano D. Biochemistry 31:5594-5604(1992).
[7] Beckman D.L., Kranz R.G. Gene 107:171-172(1991).
[8] Eichler K., Bourgis F., Buchet A., Kleber H.-P., Mandrand-Berthelot M.-A. Mol. Microbiol. 13:775-786(1994).

166. (EF1BD) Elongation factor 1 beta/beta'/delta chain signatures
Eukaryotic elongation factor 1 (EF-1) is responsible for the GTP-dependent binding of aminoacyl-tRNAs to the ribosomes [1]. EF-1 is composed of four subunits: the alpha chain which binds GTP and aminoacyl-tRNAs, the gamma chain that probably plays a role in anchoring the complex to other cellular components and the beta and delta (or beta') chains. The beta and delta chains are highly similar proteins that both stimulate the exchange of GDP bound to the alpha chain for GTP [2]. The beta and delta chains are hydrophilic proteins of around 23 to 31 Kd. Their C-terminal part seems important for the nucleotide exchange activity, while the N-terminal section is probably involved in the interaction with the gamma chain. Two signature patterns for this family of proteins were developed. The first corresponds to an acidic region in the central section; the second, to the C-terminal extremity of these proteins
Consensus pattern: [DE]-[DEG]-[DE](2)-[LIVMF]-D-L-F-G-
Consensus pattern: [IV]-Q-S-x-D-[LIVM]-x-A-[FWM]-[NQ]-K-[LIVM]-

[1] Riis B., Rattan I.S., Clark B.F.C., Merrick W.C. Trends Biochem. Sci. 15:420-424(1990).
[2] van Damme H.T.F., Amons R., Karssies R., Timmers C.J., Janssen G.M.C., Moeller W. Biochim. Biophys. Acta 1050:241-247(1990).

167. (EF1G_domain) Elongation factor 1 gamma, conserved domain

168. (EFG_C) Elongation factor G C-terminus
This family is always found associated with GTP EFTU. This family includes the carboxyl terminal regions of Elongation factor G, elongation factor 2 and some tetracycline resistance proteins.

169. (EFP) Elongation factor P signature
Elongation factor P (EF-P) [1] is a prokaryotic protein translation factor required for efficient peptide bond synthesis on 70S ribosomes from fMet-tRNAfMet. EF-P is a protein of 21 Kd. It is evolutionary related to yeiP, an hypothetical protein from Escherichia coli. As a signature pattern, a conserved region located in the C-terminal part of these proteins was selected.
Consensus pattern: K-x-[AV]-x(4)-G-x(2)-[LIV]-x-V-P-x(2)-[LIV]-x(2)-G-

[1] Aoki H., Adams S.-L., Turner M.A., Ganoza M.C. Biochimie 79:7-11 (1997).

170. (EF TS) Elongation factor Ts signatures
In prokaryotes elongation factor Ts (EF-Ts) is a component of the elongation cycle of protein biosynthesis. It associates with the EF-Tu.GDP complex and induces the exchange of GDP to GTP, it remains bound to the aminoacyl-tRNA.EF-Tu.GTP complex up to the GTP hydrolysis stage on the ribosome [1].EF-Ts is also a component of the chloroplast protein biosynthetic machinery and is encoded in the genome of some algal chloroplast [2]. It is also present in mitochondria [3]. As signature patterns for EF-Ts, two conserved regions located in the N-terminal part of the protein have been selected.
Consensus pattern: L-R-x(2)-T-[GSDNQ]-x-[GS]-[LIVMF]-x(0,1)-[DENKAC]-x-K-[KRNEQS]-A-L-
Consensus pattern: E-[LIVM]-[NV]-[SCV]-[QE]-T-D-F-V-[SA]-[KRN]-

[1] Bubunenko M.G., Kireeva M.L., GudkovA.T. Biochimie 74:419-425(1992)..
[2] Kostrzewa M., Zetsche K. Plant Mol. Biol. 23:67-76(1993).

[3] Xin H., Woriax V.L., Burkhart W.A., Spremulli L.L. J. Biol. Chem. 270:17243-17249(1995).

171.(EMP24_GP25L) emp24/gp25L/p24 family Members of this family are implicated in bringing cargo forward from the ER and binding to coat proteins by their cytoplasmic domains. Number of members: 30 Paccaud JP, Thomas DY, Bergeron JJ, Nilsson T, J Cell Biol 1998;140:751-765

172.ENV_polyprotein
ENV polyprotein (coat polyprotein)
Number of members: 224

173. (ERG4_ERG24) Ergosterol biosynthesis ERG4/ERG24 family signatures
Two fungal enzymes involved in ergosterol biosynthesis and which act by reducing double bonds in precursors of ergosterol have been shown to be evolutionary related [1]. These are C-14 sterol reductase (gene ERG24 in budding yeast and erg3 in Neurospora Crassa) and C-24(28) sterol reductase (gene ERG4 in budding yeast and stsl in fission yeast). Their sequences are also highly related to that of chicken lamin B receptor, which is thought to anchor the lamina to the inner nuclear membrane. These proteins are highly hydrophobic and seem to contain seven or eight transmembrane regions. As signature patterns, two conserved regions were selected. The first one is apparently located in a loop between the fourth and fifth transmembrane regions and the second is in the C-terminal section.
Consensus pattern: G-x(2)-[LIVM]-[YH]-D-x-[FYW]-x-G-x(2)-L-N-P-R -
Consensus pattern: [LIVM](2)-H-R-x(2)-R-D-x(3)-C-x(2)-K-Y-G-

[1] Lai M.H., Bard M., Pierson C.A., Alexander J.F., Goebl M., Carter G.T., Kirsch D.R. Gene 140:41-49(1994).

174. (ERM) Ezrin/radixin/moesin family
This family of proteins contain a band 4.1 domain (Band 41), at their amino terminus. This family represents the rest of these proteins.

[1] Yonemura S, Hirao M, Doi Y, Takahashi N, Kondo T, Tsukita S, J Cell Biol 1998;140:885-895.

175. ER lumen protein retaining receptor signatures
Proteins that reside in the lumen of the endoplasmic reticulum (ER) contain aC-terminal tetrapeptide (generally K-D-E-L or H-D-E-L) that serves as a signal for their retrieval (retrograde transport) from subsequent compartments of the secretory pathway. The signal is recognized by a receptor molecule that is believed to cycle between the cis side of the Golgi apparatus and the ER [1].This protein is known as the ER lumen protein retaining receptor or also as the 'KDEL receptor'. It has been characterized in a variety of species, including fungi (gene ERD2), plants, Plasmodium, Drosophila and mammals. In mammals two highly related forms of the receptor are known. Structurally, the receptor is a protein of about 220 residues that seems to contain seven transmembrane regions [2]. The N-terminal part (3 residues) is oriented toward the lumen while the C-terminal tail (about 12 residues) is cytoplasmic. There are three lumenal and three cytoplasmic loops. Two signature patterns for these receptors were developed. The first pattern corresponds to the C-terminal half of the first cytoplasmic loop as well as most of the second transmembrane domain. The second pattern is a perfectly conserved decapeptide that corresponds to the central part of the fifth transmembrane domain.
Consensus pattern: G-I-S-x-[KR]-x-Q-x-L-[FY]-x-[LIV](2)-F-x(2)-R-Y-
Consensus pattern: L-E-[SA]-V-A-I-[LM]-P-Q-L-

[1] Pelham H.R.B. Curr. Opin. Cell Biol. 3:585-591(1991).
[2] Townsley F.M., Wilson D.W., Pelham H.R.B. EMBO J. 12:2821-2829(1993).

176. (ETF_ beta) Electron transfer flavoprotein beta-subunit signature
The electron transfer flavoprotein (ETF) [1,2] serves as a specific electron acceptor for various mitochondrial dehydrogenases. ETF transfers electrons to the main respiratory chain via ETF-ubiquinone oxidoreductase. ETF is an heterodimer that consist of an alpha and a beta subunit and which bind one molecule of FAD per dimer. A similar system also exists in some bacteria. The beta subunit of ETF is a protein of about 28 Kd which is structurally related to the bacterial nitrogen fixation protein fixA which could play a role in a redox process and feed electrons to ferredoxin. Other related proteins are: - Escherichia coli hypothetical protein ydiQ. - Escherichia coli hypothetical protein ygcR.As a signature pattern for these proteins, a conserved region which is located in the central section was selected.
Consensus pattern: [IVA]-x-[KR]-x(2)-[DE]-[GD]-[GDE]-x(1,2)-[EQ]-x-[LIV]- x(4)-P-x-[LIVM](2)-[TAC]-

[1] Finocchiaro G., Ikeda Y., Ito M., Tanaka K. Prog. Clin. Biol. Res. 321:637-652(1990).
[2] Tsai M.H., Saier M.H. Jr. Res. Microbiol. 146:397-404(1995).

177. Endonuclease III signatures

Escherichia coli endonuclease III (EC 4.2.99.18) (gene nth) [1] is a DNA repair enzyme that acts both as a DNA N-glycosylase, removing oxidized pyrimidines from DNA, and as an apurinic/apyrimidinic (AP) endonuclease, introducing a single-strand nick at the site from which the damaged base was removed. Endonuclease III is an iron-sulfur protein that binds a single 4Fe-4Scluster. The 4Fe-4S cluster does not seem to be important for catalytic activity, but is probably involved in the proper positioning of the enzyme along the DNA strand [2].Endonuclease III is evolutionary related to the following proteins: - Fission yeast endonuclease III homolog (gene nth1) [3]. - Escherichia coli and related protein DNA repair protein mutY, which is an adenine glycosylase. MutY is a larger protein (350 amino acids) than endonuclease III (211 amino acids). - Micrococcus luteus ultraviolet N-glycosylase/AP lyase which initiates repair at cis-syn pyrimidine dimers. - ORF10 in plasmid pFV1 of the thermophilic archaebacteria Methanobacterium thermoformicicum [4]. Restriction methylase m.MthTI, which is encoded by this plasmid, generates 5-methylcytosine which is subject to deamination resulting in G-T mismatches. This protein could correct these mismatches. - Yeast hypothetical protein YAL015c. - Fission yeast hypothetical protein SpAC26A3.02. - Caenorhabditis elegans hypothetical protein R10E4.5. - Methanococcus jannaschii hypothetical protein MJ0613.The 4Fe-4S cluster is bound by four cysteines which are all located in a 17amino acid region at the C-terminal end of endonuclease III. A similar region is also present in the central section of mutY and in the C-terminus of ORF10and of the Micrococcus UV endonuclease. The 4Fe-4S cluster region does not exist in YAL015c. Two signature patterns for these proteins were developed: the first corresponds to the core of the iron-sulfur binding domain, the second corresponds to the best conserved region in the catalytic core of these enzymes.
Consensus pattern: C-x(3)-[KRS]-P-[KRAGL]-C-x(2)-C-x(5)-C [The four C's are 4Fe-4S ligands]-
Consensus pattern: [GST]-x-[LIVMF]-P-x(5)-[LIVMW]-x(2,3)-[LI]-[PAS]-G-V-[GA]-x(3)-[GAC]-x(3)-[LIVM]-x(2)-[SALV]-[LIVMFYW]-[GANK]-

[1] Kuo C.-F., McRee D., Fisher C.L., O'Handley S.F., Cunnigham R.P., Tainer J.A. Science 258:434-440(1992).
[2] Thomson A.J. Curr. Biol. 3:173-174(1993).
[3] Roldan-Arjona T., Anselmino C., Lindahl T. Nucleic Acids. Res. 3307-3312(1996).
[4] Noelling J., van Eeden F.J.M., Eggen R.I.L., de Vos W.M. Nucleic Acids Res. 20:6501-6507(1992).

178. (Epimerase) NAD dependent epimerase/dehydratase family

This family of proteins utilize NAD as a cofactor. The proteins in this family use nucleotide-sugar substrates for a variety of chemical reactions.

[1] Thoden JB, Hegeman AD, Wesenberg G, Chapeau MC, Frey PA, Holden HM, Biochemistry 1997;36: 6294-6304.

179. Exonuclease

This family includes a variety of exonuclease proteins, such as ribonuclease T and the epsilon subunit of DNA polymerase III.

[1] Koonin EV, Deutscher MP, Nucleic Acids Res 1993;21:2521-2522.

180. ENTH

ENTH domain

[1] Kay BK, Yamabhai M, Wendland B, Emr SD; Medline: 99156083, Identification of a novel domain shared by putative components of the endocytic and cytoskeletal machinery. Protein Sci 1999;8:435-438.

The ENTH (Epsin N-terminal homology) domain is found in proteins involved in endocytosis and cytoskeletal machinery. The function of the ENTH domain is unknown.
Number of members: 29

181. (eIF-1A) Eukaryotic initiation factor 1A signature

Eukaryotic translation initiation factor 1A (eIF-1A) [1] (formerly known aseiF-4C) is a protein that seems to be required for maximal rate of protein biosynthesis. It enhances ribosome dissociation into subunits and stabilizesthe binding of the initiator Met-tRNA to 40S ribosomal subunits.eIF-1A is a hydrophilic protein of about 15 to 17 Kd. Archaebacteria

also seem to possess a eIF-1A homolog. As a signature pattern, a conserved region in the central section of these proteins was selected.
Consensus pattern: [IM]-x-G-x-[GS]-[KRH]-x(4)-[CL]-x-D-G-x(2)-R-x(2)-[RH]-I- x-G

[1] Wei C.-L., Kainuma M., Hershey J.W.B. J. Biol. Chem. 270:22788-22794(1995).

182. (eIF-5A) Eukaryotic initiation factor 5A hypusine signature
Eukaryotic initiation factor 5A (eIF-5A) (formerly known as eIF-4D) [1,2] is a small protein whose precise role in the initiation of protein synthesis is not known. It appears to promote the formation of the first peptide bond. eIF-5Aseems to be the only eukaryotic protein to contain an hypusine residue. Hypusine is derived from lysine by the post-translational addition of a butylamino group (from spermidine) to the epsilon-amino group of lysine. The hypusine group is essential to the function of eIF-5A.A hypusine-containing protein has been found in archaebacteria such as Sulfolobus acidocaldarius or Methanococcus j annaschii; this protein is highlysimilar to eIF-5A and could play a similar role in protein biosynthesis. The signature developed for eIF-5A is centered around the hypusine residue.
Consensus pattern: [PT]-G-K-H-G-x-A-K [The first K is modified to hypusine]

[1] Park M.H., Wolff E.C., Folk J.E. Biofactors 4:95-104(1993).
[2] Schnier J., Schwelberger H.G., Smit-McBride Z., Kang H.A., Hershey J.W.B. Mol. Cell. Biol. 11:3105-3114 (1991).

183. (efhand) S-100/ICaBP type calcium binding protein signature
S-100 are small dimeric acidic calcium and zinc-binding proteins [1] abundant in the brain. They have two different types of calcium-binding sites: a low affinity one with a special structure and a 'normal' EF-hand type high affinity site. The vitamin-D dependent intestinal calcium-binding proteins (ICaBP or calbindin 9 Kd) also belong to this family of proteins, but it does not form dimers. In the past years the sequences of many new members of this family have been determined (for reviews see [2,3,4]); in most cases the function of these proteins is not yet known, although it is becoming clearthat they are involved in cell growth and differentiation, cell cycle regulation and metabolic control. These proteins are: -
Calcyclin (Prolactin receptor associated protein (PRA); clatropin; 2a9; 5B10; S100A6). - Calpactin I light chain (p10; p11; 42c; S100A10). - Calgranulin A (cystic fibrosis antigen (CFAg); MIF related protein 8 (MRP- 8); p8; S100A8). - Calgranulin B (MIF related protein 14 (MRP-14); p14; S100A9). - Calgranulin C. - Calgizzarin (S100C). - Placental calcium-binding protein (CAPL) (18a2; peL98; 42a; p9K; MTS1; metastatin; S100A4). - Protein S-100D (S100A5). - Protein S-100E (S100A3). - Protein S-100L (CAN19; S100A2). - Placental protein S-100P (S100E). - Psoriasin (S100A7). - Chemotactic cytokine CP-10 [5]. - Protein MRP-126 [6]. - Trichohyalin [7]. This is a large intermediate filament-associated protein that associates with keratin intermediate filaments (KIF); it contains a S-100 type domain in its N-terminal extremity. A number of these proteins are known to bind calcium while others are not (p10for example). Our EF-hand detecting pattern will fail to pick those proteins which have lost their calcium-binding properties. A pattern was developed which unambiguously picks up proteins belonging to this family. This pattern spans the region of the EF-hand high affinity site but makes no assumptions on the calcium-binding properties of this site.
Consensus pattern: [LIVMFYW](2)-x(2)-[LK]-D-x(3)-[DN]-x(3)-[DNSG]-[FY]-x- [ES]-[FYVC]-x(2)-[LIVMFS]-[LIVMF]

[1] Baudier J. (In) Calcium and Calcium Binding proteins, Gerday C., Bollis L., Giller R., Eds., pp102-113, Springer Verlag, Berlin, (1988).
[2] Moncrief N.D., Kretsinger R.H., Goodman M. J. Mol. Evol. 30:522-562(1990).
[3] Kligman D., Hilt D.C. Trends Biochem. Sci. 13:437-443(1988).
[4] Schaefer B.W., Wicki R., Engelkamp D., Mattei M.-G., Heizmann C.W. Genomics 25:638-643(1995).
[5] Lackmann M., Cornish C.J., Simpson R.J., Moritz R.L., Geczy C.L. J. Biol. Chem. 267:7499-7504(1992).
[6] Nakano T., Graf T. Oncogene 7:527-534(1992).
[7] Lee S.-C., Kim I.-G., Marekov L.N., O'Keefe E.J., Parry D.A.D., Steinert P.M., J. Biol. Chem. 268:12164-12176 (1993).

EF-hand calcium-binding domain
Many calcium-binding proteins belong to the same evolutionary family and share a type of calcium-binding domain known as the EF-hand [1 to 5]. This type of domain consists of a twelve residue loop flanked on both side by a twelve residue alpha-helical domain. In an EF-hand loop the calcium ion is coordinated in a pentagonal bipyramidal configuration. The six residues involved in the binding are in positions 1, 3, 5, 7, 9 and 12; these residues are denoted by X, Y, Z, -Y, -X and -Z. The invariant Glu or Asp at position 12 provides two oxygens for liganding Ca (bidentate ligand).

Listed below are the proteins which are known to contain EF-hand regions. For each type of protein the total number of EF-hand regions known or supposed to exist is indicated between parenthesis. This number does not include regions which clearly have lost their calcium-binding properties, or the atypical low-affinity site (which spans thirteen residues) found in the S-100/ ICaBP family of proteins [6].

- Aequorin and Renilla luciferin binding protein (LBP) (Ca=3).
- Alpha actinin (Ca=2). - Calbindin (Ca=4).
- Calcineurin B subunit (protein phosphatase 2B regulatory subunit) (Ca=4).
- Calcium-binding protein from Streptomyces erythraeus (Ca=3?).
- Calcium-binding protein from Schistosoma mansoni (Ca=2?).
- Calcium-binding proteins TCBP-23 and TCBP-25 from Tetrahymena thermophila (Ca=4?). - Calcium-dependent protein kinases (CDPK) from plants (Ca=4).
- Calcium vector protein from amphoxius (Ca=2).
- Calcyphosin (thyroid protein p24) (Ca=4?).
- Calmodulin (Ca=4, except in yeast where Ca=3).
- Calpain small and large chains (Ca=2). - Calretinin (Ca=6).
- Calcyclin (prolactin receptor associated protein) (Ca=2).
- Caltractin (centrin) (Ca=2 or 4).
- Cell Division Control protein 31 (gene CDC31) from yeast (Ca=2?).
- Diacylglycerol kinase (EC 2.7.1.107) (DGK) (Ca=2).
- FAD-dependent glycerol-3-phosphate dehydrogenase (EC 1.1.99.5) from mammals (Ca=1). - Fimbrin (plastin) (Ca=2).
- Flagellar calcium-binding protein (1f8) from Trypanosoma cruzi (Ca=1 or 2).
- Guanylate cyclase activating protein (GCAP) (Ca=3).
- Inositol phospholipid-specific phospholipase C isozymes gamma-1 and delta-1 (Ca=2) [10]. - Intestinal calcium-binding protein (ICaBPs) (Ca=2).
- MIF related proteins 8 (MRP-8 or CFAG) and 14 (MRP-14) (Ca=2).
- Myosin regulatory light chains (Ca=1). - Oncomodulin (Ca=2).
- Osteonectin (basement membrane protein BM-40) (SPARC) and proteins that contains an 'osteonectin' domain (QR1, matrix glycoprotein SC1) (see the entry <PDOC00535>) (Ca=1). - Parvalbumins alpha and beta (Ca=2).
- Placental calcium-binding protein (18a2) (nerve growth factor induced protein 42a) (p9k) (Ca=2).
- Recoverins (visinin, hippocalcin, neurocalcin, S-modulin) (Ca=2 to 3).
- Reticulocalbin (Ca=4). - S-100 protein, alpha and beta chains (Ca=2).
- Sarcoplasmic calcium-binding protein (SCPs) (Ca=2 to 3).
- Sea urchin proteins Spec 1 (Ca=4), Spec 2 (Ca=4?), Lps-1 (Ca=8).
- Serine/threonine protein phosphatase rdgc (EC 3.1.3.16) from Drosophila (Ca=2) - Sorcin V19 from hamster (Ca=2). - Spectrin alpha chain (Ca=2).
- Squidulin (optic lobe calcium-binding protein) from squid (Ca=4).
- Troponins C; from skeletal muscle (Ca=4), from cardiac muscle (Ca=3), from arthropods and molluscs (Ca=2).

There has been a number of attempts [7,8] to develop patterns that pick-up EF-hand regions, but these studies were made a few years ago when not so many different families of calcium-binding proteins were known. Therefore a new pattern was developed which takes into account all published sequences. This pattern includes the complete EF-hand loop as well as the first residue which follows the loop and which seem to always be hydrophobic.

- Consensus pattern: D-x-[DNS]-{ILVFYW}-[DENSTG]-[DNQGHRK]-{GP}-[LIVMC]-[DENQSTAGC]-x(2)-[DE]-[LIVMFYW]
- Note: positions 1 (X), 3 (Y) and 12 (-Z) are the most conserved.
- Note: the 6th residue in an EF-hand loop is, in most cases a Gly, but the number of exceptions to this 'rule' has gradually increased and therefore the pattern should include all the different residues which have been shown to exist in this position in functional Cabinding sites.
- Note: the pattern will, in some cases, miss one of the EF-hand regions in some proteins with multiple EF-hand domains.

[1] Kawasaki H., Kretsinger R.H. Protein Prof. 2:305-490(1995).
[2] Kretsinger R.H. Cold Spring Harbor Symp. Quant. Biol. 52:499-510(1987).
[3] Moncrief N.D., Kretsinger R.H., Goodman M. J. Mol. Evol. 30:522-562(1990).
[4] Nakayama S., Moncrief N.D., Kretsinger R.H. J. Mol. Evol. 34:416-448(1992).

[5] Heizmann C.W., Hunziker W. Trends Biochem. Sci. 16:98-103(1991).
[6] Kligman D., Hilt D.C. Trends Biochem. Sci. 13:437-443(1988).
[7] Strynadka N.C.J., James M.N.G. Annu. Rev. Biochem. 58:951-98(1989).
[8] Haiech J., Sallantin J. Biochimie 67:555-560(1985).
[9] Chauvaux S., Beguin P., Aubert J.-P., Bhat K.M., Gow L.A., Wood T.M., Bairoch A. Biochem. J. 265:261-265 (1990).
[10] Bairoch A., Cox J.A. FEBS Lett. 269:454-456(1990).

184. Enolase signature
Enolase (EC 4.2.1.11) is a glycolytic enzyme that catalyzes the dehydration of2-phospho-D-glycerate to phosphoenolpyruvate [1]. It is a dimeric enzyme that requires magnesium both for catalysis and stabilizing the dimer. Enolase is probably found in all organisms that metabolize sugars. In vertebrates, there are three different tissue-specific isozymes: alpha present in most tissues, beta in muscles and gamma found only in nervous tissues. Taucrystallin, one of the major lens proteins in some fish, reptiles and birds, has been shown [2] to be evolutionary related to enolase. As a signature pattern for enolase, the best conserved region was selected, it is located in the C-terminal third of the sequence.-
Consensus pattern: [LIV](3)-K-x-N-Q-I-G-[ST]-[LIV]-[ST]-[DE]-[STA]

[1] Lebioda L., Stec B., Brewer J.M. J. Biol. Chem. 264:3685-3693(1989).
[2] Wistow G., Piattigorsky J. Science 236:1554-1556(1987).

185. (F-actin_cap_A) F-actin capping protein alpha subunit signatures
The F-actin capping protein binds in a calcium-independent manner to the fast growing ends of actin filaments (barbed end) thereby blocking the exchange of subunits at these ends. Unlike gelsolin and severin this protein does not sever actin filaments. The F-actin capping protein is a heterodimer composed of two unrelated subunits: alpha and beta.The alpha subunit is a protein of about 268 to 286 amino acid residues whose sequence is well conserved in eukaryotic species [1]. As signature patterns two highly conserved regions in the C-terminal section of the alpha subunit were selected.
Consensus pattern: V-H-[FY](2)-E-D-G-N-V
Consensus pattern: F-K-[AE]-L-R-R-x-L-P-

[1] Cooper J.A., Caldwell J.E., Gattermeir D.J., Torres M.A., Amatruda J.F., Casella J.F. Cell Motil. Cytoskeleton 18:204-214(1991).

186. F-box domain

[1] Bai C, Sen P, Hofmann K, Ma L, Goebl M, Harper JW, Elledge SJ, Cell 1996;86:263-274.
[2] Skowyra D, Craig KL, Tyers M, Elledge SJ, Harper JW, Cell 1997;91:209-219.

187. F-protein
Negative factor, (F-Protein) or Nef.

[1] Arold S, Franken P, Strub M-P, Hoh F, Benichou S, Benarous R, Dumas C; Medline: 98035457, The crystal structure of HIV-1 Nef protein bound to the Fyn kinase SH3 domain suggests a role for this complex in altered T cell receptor signalling Structure 1997;5:1361-1372.

Nef protein accelerates virulent progression of AIDS by its interaction with cellular proteins involved in signal transduction and host cell activation. Nef has been shown to bind specifically to a subset of the Src kinase family.
Number of members: 1013

188. (FAD_binding_2)
Fumarate reductase / succinate dehydrogenase FAD-binding site
In bacteria two distinct, membrane-bound, enzyme complexes are responsible for the interconversion of fumarate and succinate (EC 1.3.99.1): fumarate reductase (Frd) is used in anaerobic growth, and succinate dehydrogenase (Sdh) is used in aerobic growth. Both complexes consist of two main components: a membrane-extrinsic component composed of a FAD-binding flavoprotein and an iron-sulfur protein; and an hydrophobic component composed of a membrane anchor protein and/or a cytochrome B. In eukaryotes mitochondrial succinate dehydrogenase (ubiquinone) (EC 1.3.5.1) is an enzyme composed of two subunits: a FAD flavoprotein and and iron-sulfur protein.

The flavoprotein subunit is a protein of about 60 to 70 Kd to which FAD is covalently bound to a histidine residue which is located in the N-terminal section of the protein [1]. The sequence around that histidine is well conserved in Frd and Sdh from various bacterial and eukaryotic species [2] and can be used as a signature pattern.
Consensus patternR-[ST]-H-[ST]-x(2)-A-x-G-G [H is the FAD binding site] Sequences known to belong to this class detected by the pattern ALL.

[1] Blaut M., Whittaker K., Valdovinos A., Ackrell B.A., Gunsalus R.P., Cecchini G. J. Biol. Chem. 264: 13599-13604(1989).
[2] Birch-Machin M.A., Farnsworth L., Ackrell B.A., Cochran B., Jackson S., Bindoff L.A., Aitken A., Diamond A.G., Turnbull D.M. J. Biol. Chem. 267:11553-11558(1992).

189. Fatty acid desaturases signatures (FA_desaturase)
Fatty acid desaturases (EC 1.14.99.-) are enzymes that catalyze the insertion of a double bond at the delta position of fatty acids. There seems to be two distinct families of fatty acid desaturases which do not seem to be evolutionary related. Family 1 is composed of: -
Stearoyl-CoA desaturase (SCD) (EC 1.14.99.5) [1]. SCD is a key regulatory enzyme of unsaturated fatty acid biosynthesis. SCD introduces a cis double bond at the delta(9) position of fatty acyl-CoA's such as palmitoleoyl- and oleoyl-CoA. SCD is a membrane-bound enzyme that is thought to function as a part of a multienzyme complex in the endoplasmic reticulum of vertebrates and fungi. As a signature pattern for this family a conserved region in the C-terminal part of these enzymes was selected, this region is rich in histidine residues and in aromatic residues.
Family 2 is composed of: - Plants stearoyl-acyl-carrier-protein desaturase (EC 1.14.99.6) [2], these enzymes catalyze the introduction of a double bond at the delta(9) position of steraoyl-ACP to produce oleoyl-ACP. This enzyme is responsible for the conversion of saturated fatty acids to unsaturated fatty acids in the synthesis of vegetable oils.
- Cyanobacteria desA [3] an enzyme that can introduce a second cis double bond at the delta(12) position of fatty acid bound to membranes glycerolipids. DesA is involved in chilling tolerance; the phase transition temperature of lipids of cellular membranes being dependent on the degree of unsaturation of fatty acids of the membrane lipids.
As a signature pattern for this family a conserved region in the C-terminal part of these enzymes was selected.
Consensus pattern: G-E-x-[FY]-H-N-[FY]-H-H-x-F-P-x-D-Y-
Consensus pattern: [ST]-[SA]-x(3)-[QR]-[LI]-x(5,6)-D-Y-x(2)-[LIVMFYW]-[LIVM]-[DE]-

[1] Kaestner K.H., Ntambi J.M., Kelly T.J. Jr., Lane M.D. J. Biol. Chem. 264:14755-14761(1989).
[2] Shanklin J., Somerville C.R. Proc. Natl. Acad. Sci. U.S.A. 88:2510-2514(1991).
[3] Wada H., Gombos Z., Murata N. Nature 347:200-203(1990).

190. Fructose-1-6-bisphosphatase active site (FBPase)
Fructose-1,6-bisphosphatase (EC 3.1.3.11) (FBPase) [1], a regulatory enzyme in gluconeogenesis, catalyzes the hydrolysis of fructose 1,6-bisphosphate to fructose 6-phosphate. It is involved in many different metabolic pathways and found in most organisms.Sedoheptulose-1,7-bisphosphatase (EC 3.1.3.37) (SBPase) [2] is an enzyme found plant chloroplast and in photosynthetic bacteria that catalyzes the hydrolysis of sedoheptulose 1,7-bisphosphate to sedoheptulose 7-phosphate, a step in the Calvin's reductive pentose phosphate cycle. It is functionally and structurally related to FBPase. In mammalian FBPase, a lysine residue has been shown to be involved in the catalytic mechanism [3]. The region around this residue is highly conserved and can be used as a signature pattern for FBPase and SBPase. It must be noted that, in some bacterial FBPase sequences, the active site lysine is replaced by an arginine
Consensus pattern: [AG]-[RK]-L-x(1,2)-[LIV]-[FY]-E-x(2)-P-[LIVM]-[GSA] [K/R is the active site residue]-

[1] Benkovic S.J., DeMaine M.M. Adv. Enzymol. 53:45-82(1982).
[2] Raines C.A., Lloyd J.C., Willingham N.M., Potts S., Dyer T.A. Eur. J. Biochem. 205:1053-1059(1992).
[3] Ke H., Thorpe C.M., Seaton B.A., Lipscomb W.N., Marcus F. J. Mol. Biol. 212:513-539(1989).

191. FGGY family of carbohydrate kinases signatures *
It has been shown [1] that four different type of carbohydrate kinases seem to be evolutionary related. These enzymes are: - L-fucolokinase (EC 2.7.1.51) (gene fucK). - Gluconokinase (EC 2.7.1.12) (gene gntK). - Glycerokinase (EC 2.7.1.30) (gene glpK). - Xylulokinase (EC 2.7.1.17) (gene xylB). - L-xylulose kinase (EC 2.7.1.53) (gene lyxK).These enzymes are proteins of from 480 to 520 amino acid residues. As consensus patterns for this family of kinases two conserved regionswere selected, one in the central section, the other in the C-terminal section.
Consensus pattern: [MFYGS]-x-[PST]-x(2)-K-[LIVMFYW]-x-W-[LIVMF]-x-[DENQTKR]- [ENQH]-
Consensus pattern: [GSA]-x-[LIVMFYW]-x-G-[LIVM]-x(7,8)-[HDENQ]-[LIVMF]-x(2)-[AS]-[STAIVM]-[LIVMFY]-[DEQ]-

[1] Reizer A., Deutscher J., Saier M.H. Jr., Reizer J. Mol. Microbiol. 5:1081-1089(1991).

192. FKBP-type peptidyl-prolyl cis-trans isomerase signatures/profile (FKBP)
FKBP [1,2,3] is the major high-affinity binding protein, in vertebrates, for the immunosuppressive drug FK506. It exhibits peptidyl-prolyl cis-trans isomerase activity (EC 5.2.1.8) (PPIase or rotamase). PPIase is an enzyme that accelerates protein folding by catalyzing the cis-trans isomerization of proline imidic peptide bonds in oligopeptides [4].At least three different forms of FKBP are known in mammalian species: - FKBP-12, which is cytosolic and inhibited by both FK506 and rapamycin. - FKBP-13, which is membrane associated and inhibited by both FK506 and rapamycin. - FKBP-25, which is preferentially inhibited by rapamycin. These forms of FKBP are evolutionary related and show extensive similarities[5,6,7] with the following proteins: - Fungal FKBP. - Mammalian hsp binding immunophilin (HBI) (also called p59). HBI is a protein which binds to hsp90 and contains two FKBP-like domains in its N- terminal section - the first of which seems to be functional. - The C-terminal part of the cell-surface protein mip from Legionella; a protein associated with macrophage infection by an unknown mechanism. - Escherichia coli slyD [8], a protein with a N-terminal FKBP domain followed by an histidine-rich metal-binding domain. - Escherichia coli fkpA. - Escherichia coli fklb (FKBP22). - Escherichia coli slpA. - Bacterial trigger factor (Tig). - Streptomyces hygroscopus and chrysomallus FK506-binding protein. - Chlamydia trachomatis 27 Kd membrane protein. - Neisseria meningitidis strain C114 PPiase. - Probable PPiases from Haemophilus influenzae (HI0754), Methanococcus jannaschii (MJ0278 and MJ0825), Pseudomonas fluorescens and Pseudomonase aeruginosa. Two signature patterns for these proteins were developed. One is based on a conserved region in the N-terminus of FKBP, the other is located in the central section. The profile for FKBP spans the complete domain.
Consensus pattern: [LIVMC]-x-[YF]-x-[GVL]-x(1,2)-[LFT]-x(2)-G-x(3)-[DE]-[STAEQK]-[STAN]-
Consensus pattern: [LIVMFY]-x(2)-[GA]-x(3,4)-[LIVMF]-x(2)-[LIVMFHK]-x(2)-G- x(4)-[LIVMF]-x(3)-[PSGAQ]-x(2)-[AG]-[FY]-G--

[1] Tropschug M., Wachter E., Mayer S., Schoenbrunner E.R., Schmid F.X. Nature 346:674-677(1990).
[2] Stein R.L. Curr. Biol. 1:234-236(1991).
[3] Siekierka J.J., Widerrecht G., Greulich H., Boulton D., Hung S.H.Y., Cryan J., Hodges P.J., Sigal N.H. J. Biol. Chem. 265:21011-21015(1990).
[4] Fischer G., Schmid F.X. Biochemistry 29:2205-2212(1990).
[5] Trandinh C.C., Pao G.M., Saier M.H. Jr. FASEB J. 6:3410-3420(1992).
[6] Galat A. Eur. J. Biochem. 216:689-707(1993).
[7] Hacker J., Fischer G. Mol. Microbiol. 10:445456(1993).
[8] Wuelfing C., Lomardero J., Plueckthun A. J. Biol. Chem. 269:2895-2901(1994).

193. MAPEG family (aka: FLAP/GST2/LTC4S family signature)
The following mammalian proteins are evolutionary related [1]:

- Leukotriene C4 synthase (EC 2.5.1.37) (gene LTC4S), an enzyme that catalyzes the production of LTC4 from LTA4.
- Microsomal glutathione S-transferase II (EC 2.5.1.18) (GST-II) (gene GST2), an enzyme that can also produces LTC4 fron LTA4.
- 5-lipoxygenase activating protein (gene FLAP), a protein that seems to be required for the activation of 5-lipoxygenase.

These are proteins of 150 to 160 residues that contain three transmembrane segments. As a signature pattern, a conserved region between the first and second transmembrane domains was selected.
Consensus patternc: G-x(3)-F-E-R-V-[FY]-x-A-[NQ]-x-N-C

[1] Jakobsson P.-J., Mancini J.A., Ford-Hutchinson A.W. J. Biol. Chem. 271:22203-22210(1996).

194. FMN-dependent alpha-hydroxy acid dehydrogenases active site (FMN_dh)
A number of oxidoreductases that act on alpha-hydroxy acids and which are FMN-containing flavoproteins have been shown [1,2,3] to be structurally related; these enzymes are: - Lactate dehydrogenase (EC 1.1.2.3), which consists of a dehydrogenase domain and a heme-binding domain called cytochrome b2 and which catalyzes the conversion of lactate into pyruvate. - Glycolate oxidase (EC 1.1.3.15) ((S)-2-hydroxy-acid oxidase), a peroxisomal enzyme that catalyzes the conversion of glycolate and oxygen to glyoxylate and hydrogen peroxide. - Long chain

alpha-hydroxy acid oxidase from rat (EC 1.1.3.15), a peroxisomal enzyme. - Lactate 2-monooxygenase (EC 1.13.12.4) (lactate oxidase) from Mycobacterium smegmatis, which catalyzes the conversion of lactate and oxygen to acetate, carbon dioxide and water. - (S)-mandelate dehydrogenase from Pseudomonas putida (gene mdlB), which catalyzes the reduction of (S)-mandelate to benzoylformate. The first step in the reaction mechanism of these enzymes is the abstraction of the proton from the alpha-carbon of the substrate producing a carbanion which can subsequently attach to the N5 atom of FMN. A conserved histidine has been shown [4] to be involved in the removal of the proton. The region around this active site residue is highly conserved and contains an arginine residue which is involved in substrate binding.
Consensus pattern: S-N-H-G-[AG]-R-Q [H is the active site residue] [R is a substrate-binding residue]-

[1] Giegel D.A., Williams C.H. Jr., Massey V. J. Biol. Chem. 265:6626-6632(1990).
[2] Tsou A.Y., Ransom S.C., Gerlt J.A., Buechter D.D., Babbitt P.C., Kenyon G.L. Biochemistry 29:9856-9862 (1990).
[3] Le K.H.D., Lederer F. J. Biol. Chem. 266:20877-20880(1991).
[4] Lindqvist Y., Branden C.-I. J. Biol. Chem. 264:3624-3628(1989).

195. Flavin-binding monooxygenase-like (FMO-like)
This family includes FMO proteins, cyclohexanone monooxygenase

196. (FPGS)
Folylpolyglutamate synthase signatures (aka Mur_ligase)
Folylpolyglutamate synthase (EC 6.3.2.17) (FPGS) [1] is the enzyme of folate metabolism that catalyzes ATP-dependent addition of glutamate moieties to tetrahydrofolate.
Its sequence is moderately conserved between prokaryotes (gene fo1C) and eukaryotes. We developed two signature patterns based on the conserved regions which are rich in glycine residues and could play a role in the catalytical activity and/or in substrate binding.
Consensus pattern [LIVMFY]-x-[LIVM]-[STAG]-G-T-[NK]-G-K-x-[ST]-x(7)- [LIVM](2)-x(3)-[GSK] Sequences known to belong to this class detected by the pattern ALL.
Consensus pattern[LIVMFY](2)-E-x-G-[LIVM]-[GA]-G-x(2)-D-x-[GST]-x-[LIVM](2) Sequences known to belong to this class detected by the pattern ALL.

[1] Shane B., Garrow T., Brenner A., Chen L., Choi Y.J., Hsu J.C., Stover P. Adv. Exp. Med. Biol. 338:629-634 (1993).

197. FYVE zinc finger
The FYVE zinc finger is named after four proteins that it has been found in: Fab 1, YOTB/ZK632.12, Vac1, and EEA1. The FYVE finger has been shown to bind two Zn++ ions [1]. The FYVE finger has eight potential zinc coordinating cysteine positions. Many members of this family also include two histidines in a motif R+HHC+XCG, where + represents a charged residue and X any residue. Members were included which do not conserve these histidine residues but are clearly related.

[1] Stenmark H, Aasland R, Toh BH, D'Arrigo A, J Biol Chem 1996;271:24048-24054.
[2] Gaullier JM, Simonsen A, D'Arrigo A, Bremnes B, Stenmark H, Aasland R, Nature 1998;394:432-433.

198. F_actin_cap_B
F-actin capping protein beta subunit signature
The F-actin capping protein binds in a calcium-independent manner to the fast growing ends of actin filaments (barbed end) thereby blocking the exchange of subunits at these ends. Unlike gelsolin and severin this protein does not sever actin filaments. The F-actin capping protein is a heterodimer composed of two unrelated subunits: alpha and beta.
The beta subunit is a protein of about 280 amino acid residues whose sequence is well conserved in eukaryotic species [1]. As a signature pattern a conserved hexapeptide in the N-terminal section of the beta subunit was selected.
Consensus pattern: C-D-Y-N-R-D Sequences known to belong to this class detected by the pattern ALL.

[1] Amatruda J.F., Cannon J.F., Tatchell K., Hug C., Cooper J.A. Nature 344:352-354(1990).

199. Isopenicillin N synthetase signatures (Fe_Asc_oxidored)
Isopenicillin N synthetase (IPNS) [1,2] is a key enzyme in the biosynthesis of penicillin and cephalosporin. In the

presence of oxygen, it removes iron and ascorbate, four hydrogen atoms from L-(alpha-aminoadipyl)-L-cysteinyl-d-valine to form the azetidinone and thiazolidine rings of isopenicillin. IPNS is an enzyme of about 330 amino-acid residues. Two cysteines are conserved in fungal and bacterial IPNS sequences; these may be involved in iron-binding and/or substrate-binding. Cephalosporium acremonium DAOCS/DACS [3] is a bifunctional enzyme involved in cephalosporin biosynthesis. The DAOCS domain, which is structurally related to IPNS, catalyzes the step from penicillin N to deacetoxycephalosporin C - used as a substrate by DACS to form deacetylcephalosporin C. Streptomycesclavuligerus possesses a monofunctional DAOCS enzyme (gene cefE) [4] also related to IPNS. Two signature patterns for these enzymes were derived, centered around the conserved cysteine residues.
Consensus pattern: [RK]-x-[STA]-x(2)-S-x-C-Y-[SL]-
Consensus pattern: [LIVM](2)-x-C-G-[STA]-x(2)-[STAG]-x(2)-T-x-[DNG]-

[1] Martin J.F. Trends Biotechnol. 5:306-308(1987).
[2] Chen G., Shiffman D., Mevarech M., Aharonowitz Y. Trends Biotechnol. 8:105-111(1990).
[3] Samson S.M., Dotzlaf J.E., Slisz M.L., Becker G.W., van Frank R.M., Veal L.E., Yeh W.K., Miller J.R., Queener S.W., Ingolia T.D. Bio/Technology 5:1207-1214(1987).
[4] Kovacevic S., Weigel B.J., Tobin M.B., Ingolia T.D., Miller J.R. J. Bacteriol. 171:754-760(1989).

200. Fibrillarin signature
Fibrillarin [1] is a component of a nucleolar small nuclear ribonucleoprotein(SnRNP) particle thought to participate in the first step of the processing of pre-rRNA. In mammals, fibrillarin is associated with the U3, U8 and U13small nuclear RNAs [2]. Fibrillarin is an extremely well conserved protein of about 320 amino acid residues. Structurally it consists of three different domains: - An N-terminal domain of about 80 amino acids which is very rich in glycine and contains a number of dimethylated arginine residues (DMA). - A central domain of about 90 residues which resembles that of RNA-binding proteins and contains an octameric sequence similar to the RNP-2 consensus found in such proteins. - A C-terminal alpha-helical domain. A protein evolutionary related to fibrillarin has been found [3] in archaebacteria such as Methanococcus vannielii or voltae. This protein (geneflpA) is involved in pre-rRNA processing. It lacks the Gly/Arg-rich N-terminal domain. As a signature pattern, a region was selected that starts with and encompases theRNP-2 like octapeptide sequence.
Consensus pattern: [GST]-[LIVMAP]-V-Y-A-[IV]-E-[FY]-[SA]-x-R-x(2)-R-[DE] -

[1] Aris J.P., Blobel G. Proc. Natl. Acad. Sci. U.S.A. 88:931-935(1991).
[2] Bandziulis R.J., Swanson M.S., Dreyfuss G. Genes Dev. 3:431-437(1989).
[3] Agha-Amiri K. J. Bacteriol. 176:2124-2127(1994).

201. Filamin/ABP280 repeat

[1] Fucini P, Renner C, Herberhold C, Noegel AA, Holak TA, Nat Struct Biol 1997;4:223-230.

202. Fucosyl transferase
This family of Fucosyltransferases are the enzymes transferring fucose from GDP-Fucose to GlcNAc in an alpha1,3 linkage [1].

[1] Breton C, Oriol R, Imberty A; Glycobiology 1998;8:87-94.

203. 2Fe-2S ferredoxins, iron-sulfur binding region signature (fer2A)
Ferredoxins [1] are a group of iron-sulfur proteins which mediate electron transfer in a wide variety of metabolic reactions. Ferredoxins can be divided into several subgroups depending upon the physiological nature of the iron sulfur cluster(s) and according to sequence similarities. One of these subgroups are the 2Fe-2S ferredoxins, which are proteins or domains of around one hundred amino acid residues that bind a single 2Fe-2S iron-sulfur cluster. The proteins that are known [2] to belong to this family are listed below. - Ferredoxin from photosynthetic organisms; namely plants and algae where it is located in the chloroplast or cyanelle; and cyanobacteria. - Ferredoxin from archaebacteria of the Halobacterium genus. - Ferredoxin IV (gene pftA) and V (gene fdxD) from Rhodobacter capsulatus. - Ferredoxin in the toluene degradation operon (gene xylT) and naphthalene degradation operon (gene nahT) of Pseudomonas putida. - Hypothetical Escherichia coli protein yfaE. - The N-terminal domain of the bifunctional ferredoxin/ferredoxin reductase electron transfer component of the benzoate 1,2-dioxygenase complex (gene benC) from Acinetobacter calcoaceticus, the toluene 4-monooxygenase complex (gene tmoF), the toluate 1,2-dioxygenase system (gene xylZ), and the xylene monooxygenase system (gene xylA) from Pseudomonas. - The N-terminal domain of phenol hydroxylase protein p5 (gene dmpP) from Pseudomonas Putida. - The N-terminal domain of

methane monooxygenase component C (gene mmoC) from Methylococcus capsulatus . - The C-terminal domain of the vanillate degradation pathway protein vanB in a Pseudomonas species. - The N-terminal domain of bacterial fumarate reductase iron-sulfur protein (gene frdB). - The N-terminal domain of CDP-6-deoxy-3,4-glucoseen reductase (gene ascD) from Yersinia pseudotuberculosis. - The central domain of eukaryotic succinate dehydrogenase (ubiquinone) iron- sulfur protein. - The N-terminal domain of eukaryotic xanthine dehydrogenase. - The N-terminal domain of eukaryotic aldehyde oxidase. In the 2Fe-2S ferredoxins, four cysteine residues bind the iron-sulfur cluster. Three of these cysteines are clustered together in the same region of the protein. Our signature pattern spans that iron-sulfur binding region.
Consensus pattern: C-{C}-{C}-[GA]-{C}-C-[GAST]-{CPDEKRHFYW}-C [The three C's are 2Fe-2S ligands]-

[1] Meyer J. Trends Ecol. Evol. 3:222-226(1988).
[2] Harayama S., Polissi A., Rekik M. FEBS Lett. 285:85-88(1991).

Adrenodoxin family, iron-sulfur binding region signature (fer2B)
Ferredoxins [1] are a group of iron-sulfur proteins which mediate electron transfer in a wide variety of metabolic reactions. Ferredoxins can be divided into several subgroups depending upon the physiological nature of the iron sulfur cluster(s) and according to sequence similarities. One family of ferredoxins groups together the following proteins that all bind a single 2Fe-2S iron-sulfur cluster: - Adrenodoxin (ADX) (adrenal ferredoxin), a vertebrate mitochondrial protein which transfers electrons from adrenodoxin reductase to cytochrome P450scc, which is involved in cholesterol side chain cleavage. - Putidaredoxin (PTX), a Pseudomonas putida protein which transfers electrons from putidaredoxin reductase to cytochrome P450-cam, which is involved in the oxidation of camphor. - Terpredoxin [2], a Pseudomonas protein which transfers electrons from terpredoxin reductase to cytochrome P450-terp, which is involved in the oxidation of alpha-terpineol. - Rhodocoxin [3], a Rhodococcus protein which transfers electrons from rhodocoxin reductase to cytochrome CYP116 (thcB), which is involved in the degradation of thiocarbamate herbicides. - Escherichia coli ferredoxin (gene fdx) [4] whose exact function is not yet known. - Rhodobacter capsulatus ferredoxin VI [5], which may transfer electrons to a yet uncharacterized oxygenase. - Caulobacter crescentus ferredoxin (gene fdxB) [6].In these proteins, four cysteine residues bind the iron-sulfur cluster. Three of these cysteines are clustered together in the same region of the protein. Our signature pattern spans that iron-sulfur binding region.
Consensus pattern: C-x(2)-[STAQ]-x-[STAMV]-C-[STA]-T-C-[HR] [The three C's are 2Fe-2S ligands]-

[1] Meyer J. Trends Ecol. Evol. 3:222-226(1988).
[2] Peterson J.A., Lu J.-Y., Geisselsoder J., Graham-Lorence S., Carmona C., Witney F., Lorence M.C. J. Biol. Chem. 267:14193-14203(1992).
[3] Nagy I., Schoofs G., Compernolle F., Proost P., Vanderleyden J., De Mot R. J. Bacteriol. 177:676-687(1995).
[4] Ta D.T., Vickery L.E. J. Biol. Chem. 267:11120-11125(1992).
[5] Naud I., Vincon M., Garin J., Gaillard J., Forest E., Jouanneau Y. Eur. J. Biochem. 222:933-939(1994).
[6] Amemiya K EMBL/Genbank: X51607.

204. 4Fe-4S ferredoxins, iron-sulfur binding region signature (fer4)
Ferredoxins [1] are a group of iron-sulfur proteins which mediate electron transfer in a wide variety of metabolic reactions. Ferredoxins can be divided into several subgroups depending upon the physiological nature of the iron-sulfur cluster(s). One of these subgroups are the 4Fe-4S ferredoxins, which are found in bacteria and which are thus often referred as 'bacterial-type' ferredoxins. The structure of these proteins [2] consists of the duplication of a domain of twenty six amino acid residues; each of these domains contains four cysteine residues that bind to a 4Fe-4S center. A number of proteins have been found [3] that include one or more 4Fe-4Sbinding domains similar to those of bacterial-type ferredoxins. These proteins are listed below (references are only provided for recently determined sequences). - The iron-sulfur proteins of the succinate dehydrogenase and the fumarate reductase complexes (EC 1.3.99.1). These enzyme complexes, which are components of the tricarboxylic acid cycle, each contain three subunits: a flavoprotein, an iron-sulfur protein, and a b-type cytochrome. The iron- sulfur proteins contain three different iron-sulfur centers: a 2Fe-2S, a 3Fe-3S and a 4Fe-4S. - Escherichia coli anaerobic glycerol-3-phosphate dehydrogenase (EC 1.1.99.5) This enzyme is composed of three subunits: A, B, and C. The C subunit seems to be an iron-sulfur protein with two ferredoxin-like domains in the N-terminal part of the protein. - Escherichia coli anaerobic dimethyl sulfoxide reductase. The B subunit of this enzyme (gene dmsB) is an iron-sulfur protein with four 4Fe-4S ferredoxin-like domains. - Escherichia coli formate hydrogenlyase. Two of the subunits of this oligomeric complex (genes hycB and hycF) seem to be iron-sulfur proteins that each contain two 4Fe-4S ferredoxin-like domains. - Methanobacterium formicicum formate dehydrogenase (EC 1.2.1.2). This enzyme is used by the archaebacteria to grow on formate. The beta chain of this dimeric enzyme probably binds two 4Fe-4S centers. - Escherichia coli

formate dehydrogenases N and O (EC 1.2.1.2). The beta chain of these two enzymes (genes fdnH and fdoH) are iron-sulfur proteins with four 4Fe-4S ferredoxin-like domains. - Desulfovibrio periplasmic [Fe] hydrogenase (EC 1.18.99.1). The large chain of this dimeric enzyme binds three 4Fe-4S centers, two of which are located in the ferredoxin-like N-terminal region of the protein. - Methanobacterium thermoautrophicum methyl viologen-reducing hydrogenase subunit mvhB, which contains six tandemly repeated ferredoxin-like domains and which probably binds twelve 4Fe-4S centers. - Salmonella typhimurium anaerobic sulfite reductase (EC 1.8.1.-) [4]. Two of the subunits of this enzyme (genes asrA and asrC) seem to both bind two 4Fe-4S centers. - A Ferredoxin-like protein (gene fixX) from the nitrogen-fixation genes locus of various Rhizobium species, and one from the Nifregion of Azotobacter species. - The 9 Kd polypeptide of chloroplast photosystem I [5] (gene psaC). This protein contains two low potential 4Fe-4S centers, referred as the A and B centers. - The chloroplast frxB protein which is predicted to carry two 4Fe-4S centers. - An ferredoxin from a primitive eukaryote, the enteric amoeba Entamobea histolytica. - Escherichia coli hypothetical protein yjjW, a protein with a N-terminal region belonging to the radical activating enzymes family (see <PDOC00834>) and two potential 4Fe-4S centers.The pattern of cysteine residues in the iron-sulfur region is sufficient todetect this class of 4Fe-4S binding proteins.
Consensus pattern: C-x(2)-C-x(2)-C-x(3)-C-[PEG] [The four C's are 4Fe-4S ligands]-

[1] Meyer J. Trends Ecol. Evol. 3:222-226(1988).
[2] Otaka E., Ooi T. J. Mol. Evol. 26:257-267(1987).
[3] Beinert H. FASEB J. 4:2483-2492(1990).
[4] Huang C.J., Barrett E.L. J. Bacteriol. 173:1544-1553(1991).
[5] Knaff D.B. Trends Biochem. Sci. 13:460-461(1988).

205. NifH/frxC family signatures (fer4_NifH)

Nitrogenase (EC 1.18.6.1) [1] is the enzyme system responsible for biological nitrogen fixation. Nitrogenase is an oligomeric complex which consists of two components:

component 1 which contains the active site for the reduction of nitrogen to ammonia and
component 2 (also called the iron protein).Component 2 is a homodimer of a protein (gene nifH) which binds a single 4Fe-4S iron sulfur cluster [2]. In the nitrogen fixation process nifH is first reduced by a protein such as ferredoxin; the reduced protein then transfers electrons to component 1 with the concomitant consumption of ATP.A number of proteins are known to be evolutionary related to nifH. These proteins are: - Chloroplast encoded frxC (or chlL) protein [3]. FrxC is encoded on the chloroplast genome of some plant species, its exact function is not known, but it could act as an electron carrier in the conversion of protochlorophyllide to chlorophyllide. - Rhodobacter capsulatus proteins bchL and bchX [4]. These proteins are also likely to play a role in chlorophyll synthesis. There are a number of conserved regions in the sequence of these proteins: in the N-terminal section there is an ATP-binding site motif 'A' (P-loop) and in the central section there are two conserved cysteines which have been shown, in nifH, to be the ligands of the 4Fe-4S cluster. Two signatures patterns that correspond to the regions around these cysteines were developed.

Consensus pattern: E-x-G-G-P-x(2)-[GA]-x-G-C-[AG]-G [C binds the iron-sulfur center]-
Consensus pattern: D-x-L-G-D-V-V-C-G-G-F-[AG]-x-P [C binds the iron-sulfur center]-

[1] Pau R.N. Trends Biochem. Sci. 14:183-186(1989).
[2] Georgiadis M.M., Komiya H., Chakrabarti P., Woo D., Kornuc J.J., Rees D.C. Science 257:1653-1659(1992).
[3] Fujita Y., Takahashi Y., Kohchi T., Ozeki H., Ohyama K., Matsubara H. Plant Mol. Biol. 13:551-561(1989).
[4] Burke D.H., Alberti M., Hearst J.E. J. Bacteriol. 175:2407-2413(1993).

206. Ferritin iron-binding regions signatures

Ferritin [1,2] is one of the major non-heme iron storage proteins. It consists of a mineral core of hydrated ferric oxide, and a multi-subunit protein shell which englobes the former and assures its solubility in an aqueous environment. In animals the protein is mainly cytoplasmic and there are generally two or more genes that encodes for closely related subunits (in mammals there are two subunits which are known as H(eavy) and L(ight)). In plants ferritin is found in the chloroplast [3].There are a number of well conserved region in the sequence of ferritins. Two of these regions to develop signature patterns were selected. The first pattern is located in the central part of the sequence of ferritin and it contains three conserved glutamate which are thought to be involved in the binding of iron. The second pattern is located in the C-terminal section, it corresponds to a region which forms a hydrophilic channel through which small molecules and ions can gain access to the central cavity of the molecule; this pattern also includes conserved acidic residues which are potential metal-binding sites.

Consensus pattern: E-x-[KR]-E-x(2)-E-[KR]-[LF]-[LIVMA]-x(2)-Q-N-x-R-x-G-R [The 3 E's are potential iron ligands]-
Consensus pattern: D-x(2)-[LIVMF]-[STAC]-[DH]-F-[LI]-[EN]-x(2)-[FY]-L-x(6)-[LIVM]-[KN] [The second D and the E are potential iron ligands]-

[1] Crichton R.R., Charloteaux-Wauters M. Eur. J. Biochem. 164:485-506(1987).
[2] Theil E.C. Annu. Rev. Biochem. 56:289-315(1987).
[3] Ragland M., Briat J.-F., Gagnon J., Laulhere J.-P., Massenet O., Theil E.C. J. Biol. Chem. 265:18339-18344 (1990).

207. Intermediate filaments signature (filament)
Intermediate filaments (IF) [1,2,3] are proteins which are primordial components of the cytoskeleton and the nuclear envelope. They generally form filamentous structures 8 to 14 nm wide. IF proteins are members of a very large multigene family of proteins which has been subdivided in five major subgroups: - Type I: Acidic cytokeratins. - Type II: Basic cytokeratins. - Type III: Vimentin, desmin, glial fibrillary acidic protein (GFAP), peripherin, and plasticin. - Type IV: Neurofilaments L, H and M, alpha-intemexin and nestin. - Type V: Nuclear lamins A, B1, B2 and C. All IF proteins are structurally similar in that they consist of: a central rod domain comprising some 300 to 350 residues which is arranged in coiled-coiled alpha-helices, with at least two short characteristic interruptions; a N-terminal non-helical domain (head) of variable length; and a C-terminal domain (tail) which is also non-helical, and which shows extreme length variation between different IF proteins. While IF proteins are evolutionary and structurally related, they have limited sequence homologies except in several regions of the rod domain. A conserved region at the C-terminal extremity of the rod domain was used as a sequence pattern for this class of proteins.
Consensus pattern: [IV]-x-[TACI]-Y-[RKH]-x-[LM]-L-[DE]-

[1] Quinlan R., Hutchison C., Lane B. Protein Prof. 2:801-952(1995).
[2] Steiner P.M., Roop D.R. Annu. Rev. Biochem. 57:593-625(1988).
[3] Stewart M. Curr. Opin. Cell Biol. 2:91-100(1990).

208. Flavodoxin signature
Flavodoxins [1,E1] are electron-transfer proteins that function in various electron transport systems. Flavodoxins bind one FMN molecule, which serves as a redox-active prosthetic group. Flavodoxins are functionally interchangeable with ferredoxins. They have been isolated from prokaryotes, cyanobacteria, and some eukaryotic algae. The signature pattern for these proteins is derived from a conserved region in their N-terminal section, this region is involved in the binding of the FMN phosphate group.
Consensus pattern: [LIV]-[LIVFY]-[FY]-x-[ST]-x(2)-[AGC]-x-T-x(3)-A-x(2)-[LIV]-

[1] Wakabayashi S., Kimura K., Matsubara H., Rogers L.J. Biochem. J. 263:981-984(1989).

209. Growth factor and cytokines receptors family signatures (fn3)
A number of receptors for lymphokines, hematopoeitic growth factors and growth hormone-related molecules have been found [1 to 5] to share a common binding domain. Receptors known to belong to this family are: - Cytokine receptor common beta chain. This chain is common to the IL-3, IL-5 and GM-CSF receptors. - Cytokine receptor common gamma chain. This chain is common to the IL-2, IL-4, IL-7 and IL-13 receptors. - Ciliary neurotrophic factor receptor (CNTFR). - Erythropoietin receptor (EPOR). - Granulocyte colony-stimulating factor receptor (G-CSFR). - Granulocyte-macrophage colony-stimulating factor receptor alpha chain (GM- CSFR). - Interleukin-2 receptor beta chain (IL2R-beta). - Interleukin-3 receptor alpha chain (IL3R). - Interleukin-4 receptor alpha chain (IL4R). - Interleukin-5 receptor alpha chain (IL5R). - Interleukin-6 receptor (IL6R). - Interleukin-7 receptor alpha chain (IL7R). - Interleukin-9 receptor (IL9R). - Growth hormone receptor (GRHR). - Prolactin receptor (PRLR). - Thrombopoeitin receptor (TPOR).The conserved region constitutes all or part of the extracellular ligand-binding region and is about 200 amino acid residues long. In the N-terminal of this domain there are two pairs of cysteines known, in the growth hormone receptor, to be involved in disulfide bonds. +----------------------------xxxxxxx----------------------+|C C C C Extracellular XXXXXXX Cytoplasmic | +-|-|-------|--|-------------------------xxxxxxx-----------+||||Transmembrane +-+ +--+ Two patterns to detect this family of receptors were used. The first one is derived from the first N-terminal disulfide loop, the second is a tryptophan-rich pattern located at the C-terminal extremity of the extracellular region.
Consensus pattern: C-[LVFYR]-x(7,8)-[STIVDN]-C-x-W [The two C's are linked by a disulfide bond]-
Consensus pattern: [STGL]-x-W-[SG]-x-W-S-

[1] Bazan J.F. Biochem. Biophys. Res. Commun. 164:788-795(1989).
[2] Bazan J.F. Proc. Natl. Acad. Sci. U.S.A. 87:6934-6938(1990).

[3] Cosman D., Lyman S.D., Idzerda R.L., Beckmann M.P., Park L.S., Goodwin R.G., March C.J. Trends Biochem. Sci. 15:265-270(1990).
[4] d'Andrea A.D., Fasman G.D., Lodish H.F. Cell 58:1023-1024(1989).
[5] d'Andrea A.D., Fasman G.D., Lodish H.F. Curr. Opin. Cell Biol. 2:648-651(1990).

210. Phosphoribosylglycinamide formyltransferase active site (formyl_transf) Phosphoribosylglycinamide formyltransferase (EC 2.1.2.2) (GART) [1] catalyzes the third step in de novo purine biosynthesis, the transfer of a formyl group to 5'-phosphoribosylglycinamide. In higher eukaryotes, GART is part of a multifunctional enzyme polypeptide that catalyzes three of the steps of purine biosynthesis. In bacteria, plants and yeast, GART is a monofunctional protein of about 200 amino-acid residues. In the Escherichia coli enzyme, an aspartic acid residue has been shown to be involved in the catalytic mechanism. The region around this active site residue is well conserved in GART from prokaryotic and eukaryotic sources and can be used as a signature pattern. Mammalian formyltetrahydrofolate dehydrogenase (EC 1.5.1.6) [2] is a cytosolicenzyme responsible for the NADP-dependent decarboxylative reduction of 10-formyltetrahydrofolate into tetrahydrofolate. It is a protein of about 900 amino acids consisting of three domains; the N-terminal domain (200 residues) is structurally related to GARTs.Escherichia coli methionyl-tRNA formyltransferase (EC 2.1.2.9) (gene fmt) [3]is the enzyme responsible for modifying the free amino group of the aminoacylmoiety of methionyl- A(fMet). The central part of fmt seems to be evolutionary related to GART's active site region.
Consensus pattern: G-x-[STM]-[IVT]-x-[FYWVQ]-[VMAT]-x-[DEVM]-x-[LIVMY]-D-x-G- x(2)-[LIVT]-x(6)-[LIVM] [D is the active site residue] -

[1] Inglese J., Smith J.M., Benkovic S.J. Biochemistry 29:6678-6687(1990).
[2] Cook R.J., Lloyd R.S., Wagner C. J. Biol. Chem. 266:4965-4973(1991).
[3] Guillon J.-M., Mechulam Y., Schmitter J.-M., Blanquet S., Fayat G. J. Bacteriol. 174:4294-4301(1992).

211. G10 protein signatures
A Xenopus protein known as G10 [1] has been found to be highly conserved in a wide range of eukaryotic species. The function of G10 is still unknown. G10 is a protein of about 17 to 18 Kd (143 to 157 residues) which is hydrophilic and whose C-terminal half is rich in cysteines and could be involved in metal-binding. As signature patterns, two of these cysteine-rich segments were selected.
Consensus pattern: L-C-C-x-[KR]-C-x(4)-[DE]-x-N-x(4)-C-x-C-R-V-P-
Consensus pattern: C-x-H-C-G-C-[KRH]-G-C-[SA]-

[1] McGrew L.L., Dworkin-Rastl E., Dworkin M.B., Richter J.D. Genes Dev. 3:803-815(1989).

212. G-protein alpha subunit
G proteins couple receptors of extracellular signals to intracellular signaling pathways. The G protein alpha subunit binds guanyl nucleotide and is a weak GTPase. Number of members: 195

[1] Coleman DE, Berghuis AM, Lee E, Linder ME, Gilman AG, Sprang SR, Science 1994;265:1405-1412.
[2] How G proteins work: a continuing story. Coleman DE, Sprang SR, Trends Biochem Sci 1996;21:41-44.

213. Glucose-6-phosphate dehydrogenase active site (G6PD)
Glucose-6-phosphate dehydrogenase (EC 1.1.1.49) (G6PD) [1] catalyzes the first step in the pentose pathway, the reduction of glucose-6-phosphate to gluconolactone 6-phosphate. A lysine residue has been identified as are active nucleophile associated with the activity of the enzyme. The sequence around this lysine is totally conserved from bacterial to mammalian G6PD's and can be used as a signature pattern
Consensus pattern: D-H-Y-L-G-K-[EQK] [K is the active site residue]-

[1] Jeffery J., Persson B., Wood I., Bergman T., Jeffery R., Joernvall H. Eur. J. Biochem. 212:41-49(1993).

214. GATA-type zinc finger domain
The GATA family of transcription factors are proteins that bind to DNA sites with the consensus sequence (A/T) GATA(A/G), found within the regulatory region of a number of genes. Proteins currently known to belong to this family are: - GATA-1 [1] (also known as Eryf1, GF-1 or NF-E1), which binds to the GATA region of globin genes and other genes expressed in erythroid cells. It is a transcriptional activator which probably serves as a general 'switch' factor for erythroid development. - GATA-2 [2], a transcriptional activator which regulates endothelin-1 gene expression in endothelial cells. - GATA-3 [3], a transcriptional activator which binds to the enhancer of the T-cell receptor alpha and delta genes. - GATA-4 [4], a transcriptional activator expressed in endodermally derived tissues

and heart. - Drosophila protein pannier (or DGATAa) (gene pnr) which acts as a repressor of the achaete-scute complex (as-c). - Bombyx mori BCFI [5], which regulates the expression of chorion genes. - Caenorhabditis elegans elt-1 and elt-2, transcriptional activators of genes containing the GATA region, including vitellogenin genes [6]. - Ustilago maydis urbs1 [7], a protein involved in the repression of the biosynthesis of siderophores. - Fission yeast protein GAF2.All these transcription factors contain a pair of highly similar 'zinc finger' type domains with the consensus sequence C-x2-C-x17-C-x2-C.Some other proteins contain a single zinc finger motif highly related to those of the GATA transcription factors. These proteins are: - Drosophila box A-binding factor (ABF) (also known as protein serpent (gene srp)) which may function as a transcriptional activator protein and may play a key role in the organogenesis of the fat body. - Emericella nidulans areA [8], a transcriptional activator which mediates nitrogen metabolite repression. - Neurospora crassa nit-2 [9], a transcriptional activator which turns on the expression of genes coding for enzymes required for the use of a variety of secondary nitrogen sources, during conditions of nitrogen limitation. - Neurospora crassa white collar proteins 1 and 2 (WC-1 and WC-2), which control expression of light-regulated genes. - Saccharomyces cerevisiae DAL81 (or UGA43), a negative nitrogen regulatory protein. - Saccharomyces cerevisiae GLN3, a positive nitrogen regulatory protein. - Saccharomyces cerevisiae GAT1. - Saccharomyces cerevisiae GZF3.

Consensus pattern: C-x-[DN]-C-x(4,5)-[ST]-x(2)-W-[HR]-[RK]-x(3)-[GN]-x(3,4)- C-N-[AS]-C [The four C's are zinc ligands]

[1] Trainor C.D., Evans T., Felsenfeld G., Boguski M.S. Nature 343:92-96(1990).
[2] Lee M.E., Temizer D.T., Clifford J.A., Quertermous T. J. Biol. Chem. 266:16188-16192(1991).
[3] Ho I.-C., Vorhees P., Marin N., Oakley B.K., Tsai S.-F., Orkin S.H., Leiden J.M. EMBO J. 10:1187-1192(1991).
[4] Spieth J., Shim Y.H., Lea K., Conrad R., Blumenthal T. Mol. Cell. Biol. 11:4651-4659(1991).
[5] Drevet J.R., Skeiky Y.A., Iatrou K. J. Biol. Chem. 269:10660-10667(1994).
[6] Hawkins M.G., McGhee J.D. J. Biol. Chem. 270:14666-14671(1995).
[7] Voisard C.P.O., Wang J., Xu P., Leong S.A., McEvoy J.L. Mol. Cell. Biol. 13:7091-7100(1993).
[8] Arst H.N. Jr., Kudla B., Martinez-Rossi N.M., Caddick M.X., Sibley S., Davies R.W. Trends Genet. 5:291-291 (1989).
[9] Fu Y.-H., Marzluf G.A. Mol. Cell. Biol. 10:1056-1065(1990).

215. Glutamine amidotransferases class-I active site (GATase)

A large group of biosynthetic enzymes are able to catalyze the removal of the ammonia group from glutamine and then to transfer this group to a substrate to form a new carbon-nitrogen group. This catalytic activity is known asglutamine amidotransferase (GATase) (EC 2.4.2.-) [1]. The GATase domain exists either as a separate polypeptidic subunit or as part of a larger polypeptide fused in different ways to a synthase domain. On the basis of sequence similarities two classes of GATase domains have been identified [2,3]: class-I(also known as trpG-type) and class-II (also known as purF-type). Class-I GATase domains have been found in the following enzymes: - The second component of anthranilate synthase (AS) (EC 4.1.3.27) [4]. AS catalyzes the biosynthesis of anthranilate from chorismate and glutamine. AS is generally a dimeric enzyme: the first component can synthesize anthranilate using ammonia rather than glutamine, whereas component II provides the GATase activity. In some bacteria and in fungi the GATase component of AS is part of a multifunctional protein that also catalyzes other steps of the biosynthesis of tryptophan. - The second component of 4-amino-4-deoxychorismate (ADC) synthase (EC 4.1.3. -), a dimeric prokaryotic enzyme that function in the pathway that catalyzes the biosynthesis of para-aminobenzoate (PABA) from chorismate and glutamine. The second component (gene pabA) provides the GATase activity [4]. - CTP synthase (EC 6.3.4.2). CTP synthase catalyzes the final reaction in the biosynthesis of pyrimidine, the ATP-dependent formation of CTP from UTP and glutamine. CTP synthase is a single chain enzyme that contains two distinct domains; the GATase domain is in the C-terminal section [2]. - GMP synthase (glutamine-hydrolyzing) (EC 6.3.5.2). GMP synthase catalyzes the ATP-dependent formation of GMP from xanthosine 5'-phosphate and glutamine. GMP synthase is a single chain enzyme that contains two distinct domains; the GATase domain is in the N-terminal section [5]. - Glutamine-dependent carbamoyl-phosphate synthase (EC 6.3.5.5) (GD-CPSase); an enzyme involved in both arginine and pyrimidine biosynthesis and which catalyzes the ATP-dependent formation of carbamoyl phosphate from glutamine and carbon dioxide. In bacteria GD-CPSase is composed of two subunits: the large chain (gene carB) provides the CPSase activity, while the small chain (gene carA) provides the GATase activity. In yeast the enzyme involved in arginine biosynthesis is also composed of two subunits: CPA1 (GATase), and CPA2 (CPSase). In most eukaryotes, the first three steps of pyrimidine biosynthesis are catalyzed by a large multifunctional enzyme (called URA2 in yeast, rudimentary in Drosophila, and CAD in mammals). The GATase domain is located at the N-terminal extremity of this polyprotein [6]. - Phosphoribosylformylglycinamidine synthase II (EC 6.3.5.3), an enzyme that catalyzes the fourth step in the de novo biosynthesis of purines. In some species of bacteria, FGAM synthase II is composed of two subunits: a small chain (gene purQ) which provides the GATase activity and a large

chain (gene purL) which provides the aminator activity. - The histidine amidotransferase hisH, an enzyme that catalyzes the fifth step in the biosynthesis of histidine in prokaryotes.In the second component of AS a cysteine has been shown [7] to be essentialfor the amidotransferase activity. The sequence around this residue is well conserved in all the above GATase domains and can be used as a signature pattern for class-I GATase.-
Consensus pattern: [PAS]-[LIVMFYT]-[LIVMFY]-G-[LIVMFY]-C-[LIVMFYN]-G-x-[QEH]- x-[LIVMFA] [C is the active site residue]-

[1] Buchanan J.M. Adv. Enzymol. 39:91-183(1973).
[2] Weng M., Zalkin H. J. Bacteriol. 169:3023-3028(1987).
[3] Nyunoya H., Lusty C.J. J. Biol. Chem. 259:9790-9798(1984).
[4] Crawford I.P. Annu. Rev. Microbiol. 43:567-600(1989).
[5] Zalkin H., Argos P., Narayana S.V.L., Tiedeman A.A., Smith J.M. J. Biol. Chem. 260:3350-3354(1985).
[6] Davidson J.N., Chen K.C., Jamison R.S., Musmanno L.A., Kern C.B. BioEssays 15:157-164(1993).
[7] Tso J.Y., Hermodson M.A., Zalkin H. J. Biol. Chem. 255:1451-1457(1980).

216. Glutamine amidotransferases class-II active site (GATase_2)
A large group of biosynthetic enzymes are able to catalyze the removal of the ammonia group from glutamine and then to transfer this group to a substrate to form a new carbon-nitrogen group. This catalytic activity is known as glutamine amidotransferase (GATase) (EC 2.4.2.-) [1]. The GATase domain exists either as a separate polypeptidic subunit or as part of a larger polypeptide fused in different ways to a synthase domain. On the basis of sequence similarities two classes of GATase domains have been identified [2,3]: class-I(also known as trpG-type) and class-II (also known as purF-type). Class-II GATase domains have been found in the following enzymes: - Amido phosphoribosyltransferase (glutamine phosphoribosylpyrophosphate amidotransferase) (EC 2.4.2.14). An enzyme which catalyzes the first step in purine biosynthesis, the transfer of the ammonia group of glutamine to PRPP to form 5-phosphoribosylamine (gene purF in bacteria, ADE4 in yeast). - Glucosamine--fructose-6-phosphate aminotransferase (EC 2.6.1.16). This enzyme catalyzes a key reaction in amino sugar synthesis, the formation of glucosamine 6-phosphate from fructose 6-phosphate and glutamine (gene glmS in Escherichia coli, nodM in Rhizobium, GFA1 in yeast) - Asparagine synthetase (glutamine-hydrolyzing) (EC 6.3.5.4). This enzyme is responsible for the synthesis of asparagine from aspartate and glutamine. A cysteine is present at the N-terminal extremity of the mature form of all these enzymes. The cysteine has been shown, in amido phosphoribosyltransferase [4] and in asparagine synthetase [5] to be important for the catalytic mechanism.
Consensus pattern: <x(0,11)-C-[GS]-[IV]-[LIVMFYW]-[AG] [C is the active site residue]-

[1] Buchanan J.M. Adv. Enzymol. 39:91-183(1973).
[2] Weng M., Zalkin H. J. Bacteriol. 169:3023-3028(1987).
[3] Nyunoya H., Lusty C.J. J. Biol. Chem. 259:9790-9798(1984).
[4] van Heeke G., Schuster M. J. Biol. Chem. 264:5503-5509(1989).
[5] Vollmer S.J., Switzer R.L., Hermodson M.A., Bower S.G., Zalkin H. J. Biol. Chem. 258:10582-10585(1983).

217. GDP dissociation inhibitor (GDI)

[1] Schalk I, Zeng K, Wu SK, Stura EA, Matteson J, Huang M, Tandon A, Wilson IA, Balch WE, Nature 1996; 381:42-48.

218. Oxidoreductase family (GFO_IDH_MocA)
This family of enzymes utilise NADP or NAD. This family: is called the GFO/IDH/MOCA family in swiss-prot.

[1] Kingston RL, Scopes RK, Baker EN, Structure 1996;4:1413-1428.

219. GHMP kinases putative ATP-binding domain
The following kinases contains, in their N-terminal section, a conserved Gly/Ser-rich region which is probably involved in the binding of ATP [1]. These kinases are listed below. - Galactokinase (EC 2.7.1.6). - Homoserine kinase (EC 2.7.1.39). - Mevalonate kinase (EC 2.7.1.36). - Phosphomevalonate kinase (EC 2.7.4.2). This group of kinases was called 'GHMP' (from the first letter of their substrate)
Consensus pattern: [LIVM]-[PK]-x-[GSTA]-x(0,1)-G-L-[GS]-S-S-[GSA]-[GSTAC]-

[1] Tsay Y.H., Robinson G.W. Mol. Cell. Biol. 11:620-631(1991).

220. Glucose inhibited division protein A family signatures (GIDA)
Bacterial glucose inhibited division protein A (gene gidA) is a protein of 70Kd whose function is not yet known and whose sequence is highly conserved. It is evolutionary related to yeast hypothetical protein YGL236C, Caenorhabditis elegans hypothetical protein F52H3.2 and a Bacillus subtilis protein called gid (and which is different from B.subtilis gidA). Two highly conserved regions were selected as signature patterns. Both regions are located in the central region of the protein.
Consensus pattern: [GS]-[PT]-x-Y-C-P-S-[LIVM]-E-x-K-[LIVM]-x-[KR]-
Consensus pattern: A-G-Q-x-[NT]-G-x(2)-G-Y-x-E-[SAG](3)-[QS]-G-[LIVM](2)-A-G-[LIVMT]-N-A-

221. (GLFV_dehydrog)
Glu / Leu / Phe / Val dehydrogenases active site

- Glutamate dehydrogenases (EC 1.4.1.2, EC 1.4.1.3, and EC 1.4.1.4) (GluDH) are enzymes that catalyze the NAD- or NADP-dependent reversible deamination of glutamate into alpha-ketoglutarate [1,2]. GluDH isozymes are generally involved with either ammonia assimilation or glutamate catabolism.
- Leucine dehydrogenase (EC 1.4.1.9) (LeuDH) is a NAD-dependent enzyme that catalyzes the reversible deamination of leucine and several other aliphatic amino acids to their keto analogues [3].
- Phenylalanine dehydrogenase (EC 1.4.1.20) (PheDH) is a NAD-dependent enzyme that catalyzes the reversible deamidation of L-phenylalanine into phenylpyruvate [4].
- Valine dehydrogenase (EC 1.4.1.8) (ValDH) is a NADP-dependent enzyme that catalyzes the reversible deamidation of L-valine into 3-methyl-2-oxobutanoate [5].

These dehydrogenases are structurally and functionally related. A conserved lysine residue located in a glycine-rich region has been implicated in the catalytic mechanism. The conservation of the region around this residue allows the derivation of a signature pattern for such type of enzymes.
Consensus pattern[LIV]-x(2)-G-G-[SAG]-K-x-[GV]-x(3)-[DNST]-[PL] [K is the active site residue] Sequences known to belong to this class detected by the pattern ALL.
Note all known sequences from this family have Pro in the last position of the pattern with the exception of yeast GluDH which as Leu.

[1] Britton K.L., Baker P.J., Rice D.W., Stillman T.J. Eur. J. Biochem. 209:851-859(1992).
[2] Benachenhou-Lahfa N., Forterre P., Labedan B. J. Mol. Evol. 36:335-346(1993).
[3] Nagata S., Tanizawa K., Esaki N., Sakamoto Y., Ohshima T., Tanaka H., Soda K. Biochemistry 27:9056-9062 (1988).
[4] Takada H., Yoshimura T., Ohshima T., Esaki N., Soda K. J. Biochem. 109:371-376(1991).
[5] Hutchinson C.R., Tang L. J. Bacteriol. 175:4176-4185(1993).

222. GMC oxidoreductases signatures
The following FAD flavoproteins oxidoreductases have been found [1,2] to be evolutionary related. These enzymes, which are called 'GMC oxidoreductases', are listed below. - Glucose oxidase (EC 1.1.3.4) (GOX) from Aspergillus niger. Reaction catalyzed: glucose + oxygen -> delta-gluconolactone + hydrogen peroxide. - Methanol oxidase (EC 1.1.3.13) (MOX) from fungi. Reaction catalyzed: methanol + oxygen -> acetaldehyde + hydrogen peroxide. - Choline dehydrogenase (EC 1.1.99.1) (CHD) from bacteria. Reaction catalyzed: choline + unknown acceptor -> betaine acetaldehyde + reduced acceptor. - Glucose dehydrogenase (GLD) (EC 1.1.99.10) from Drosophila. Reaction catalyzed: glucose + unknown acceptor -> delta-gluconolactone + reduced acceptor. - Cholesterol oxidase (CHOD) (EC 1.1.3.6) from Brevibacterium sterolicum and Streptomyces strain SA-COO. Reaction catalyzed: cholesterol + oxygen -> cholest-4-en-3-one + hydrogen peroxide. - AlkJ [3], an alcohol dehydrogenase from Pseudomonas oleovorans, which converts aliphatic medium-chain-length alcohols into aldehydes. This family also includes a lyase: - (R)-mandelonitrile lyase (EC 4.1.2.10) (hydroxynitrile lyase) from plants [4], an enzyme involved in cyanogenis, the release of hydrogen cyanide from injured tissues. These enzymes are proteins of size ranging from 556 (CHD) to 664 (MOX) amino acid residues which share a number of regions of sequence similarities. One of these regions, located in the N-terminal section, corresponds to the FAD ADP-binding domain. The function of the other conserved domains is not yet known; two of these domains were selected as signature patterns. The first one is located in the N-terminal section of these enzymes, about 50 residues after the ADP-binding domain, while the second one is located in the central section.
Consensus pattern: [GA]-[RKN]-x-[LIV]-G(2)-[GST](2)-x-[LIVM]-N-x(3)-[FYWA]- x(2)-[PAG]-x(5)-[DNESH]-
Consensus pattern: [GS]-[PSTA]-x(2)-[ST]-P-x-[LIVM](2)-x(2)-S-G-[LIVM]-G-

[1] Cavener D.R. J. Mol. Biol. 223:811-814(1992).
[2] Henikoff S., Henikoff J.G. Genomics 19:97-107(1994).
[3] van Beilen J.B., Eggink G., Enequist H., Bos R., Witholt B. Mol. Microbiol. 6:3121-3136(1992).
[4] Cheng I.P., Poulton J.E. Plant Cell Physiol. 34:1139-1143(1993).

223. (GMP_synt_C)
Glutamine amidotransferases class-I active site
A large group of biosynthetic enzymes are able to catalyze the removal of the ammonia group from glutamine and then to transfer this group to a substrate to form a new carbon-nitrogen group. This catalytic activity is known as glutamine amidotransferase (GATase) (EC 2.4.2.-) [1]. The GATase domain exists either as a separate polypeptidic subunit or as part of a larger polypeptide fused in different ways to a synthase domain. On the basis of sequence similarities two classes of GATase domains have been identified [2,3]: class-I (also known as trpG-type) and class-II (also known as purF-type). Class-I GATase domains have been found in the following enzymes:

- The second component of anthranilate synthase (AS) (EC 4.1.3.27) [4]. AS catalyzes the biosynthesis of anthranilate from chorismate and glutamine. AS is generally a dimeric enzyme: the first component can synthesize anthranilate using ammonia rather than glutamine, whereas component II provides the GATase activity. In some bacteria and in fungi the GATase component of AS is part of a multifunctional protein that also catalyzes other steps of the biosynthesis of tryptophan.
- The second component of 4-amino-4-deoxychorismate (ADC) synthase (EC 4.1.3. -), a dimeric prokaryotic enzyme that function in the pathway that catalyzes the biosynthesis of para-aminobenzoate (PABA) from chorismate and glutamine. The second component (gene pabA) provides the GATase activity [4].
- CTP synthase (EC 6.3.4.2). CTP synthase catalyzes the final reaction in the biosynthesis of pyrimidine, the ATP-dependent formation of CTP from UTP and glutamine. CTP synthase is a single chain enzyme that contains two distinct domains; the GATase domain is in the C-terminal section [2].
- GMP synthase (glutamine-hydrolyzing) (EC 6.3.5.2). GMP synthase catalyzes the ATP-dependent formation of GMP from xanthosine 5'-phosphate and glutamine. GMP synthase is a single chain enzyme that contains two distinct domains; the GATase domain is in the N-terminal section [5].
- Glutamine-dependent carbamoyl-phosphate synthase (EC 6.3.5.5) (GD-CPSase); an enzyme involved in both arginine and pyrimidine biosynthesis and which catalyzes the ATP-dependent formation of carbamoyl phosphate from glutamine and carbon dioxide. In bacteria GD-CPSase is composed of two subunits: the large chain (gene carB) provides the CPSase activity, while the small chain (gene carA) provides the GATase activity. In yeast the enzyme involved in arginine biosynthesis is also composed of two subunits: CPA1 (GATase), and CPA2 (CPSase). In most eukaryotes, the first three steps of pyrimidine biosynthesis are catalyzed by a large multi-functional enzyme (called URA2 in yeast, rudimentary in Drosophila, and CAD in mammals). The GATase domain is located at the N-terminal extremity of this polyprotein [6].
- Phosphoribosylformylglycinamidine synthase II (EC 6.3.5.3), an enzyme that catalyzes the fourth step in the de novo biosynthesis of purines. In some species of bacteria, FGAM synthase II is composed of two subunits: a small chain (gene purQ) which provides the GATase activity and a large chain (gene purL) which provides the aminator activity.
- The histidine amidotransferase hisH, an enzyme that catalyzes the fifth step in the biosynthesis of histidine in prokaryotes.

In the second component of AS a cysteine has been shown [7] to be essential for the amidotransferase activity. The sequence around this residue is well conserved in all the above GATase domains and can be used as a signature pattern for class-I GATase.
Consensus pattern[PAS]-[LIVMFYT]-[LIVMFY]-G-[LIVMFY]-C-[LIVMFYN]-G-x-[QEH]- x-[LIVMFA] [C is the active site residue] Sequences known to belong to this class detected by the pattern ALL, except for 6 sequences.
Note: in the first position of the pattern Pro is found in all cases except in the slime mold GD-CPSase where it is replaced by Ala.

[1] Buchanan J.M. Adv. Enzymol. 39:91-183(1973).
[2] Weng M., Zalkin H. J. Bacteriol. 169:3023-3028(1987).
[3] Nyunoya H., Lusty C.J. J. Biol. Chem. 259:9790-9798(1984).
[4] Crawford I.P. Annu. Rev. Microbiol. 43:567-600(1989).
[5] Zalkin H., Argos P., Narayana S.V.L., Tiedeman A.A., Smith J.M. J. Biol. Chem. 260:3350-3354(1985).
[6] Davidson J.N., Chen K.C., Jamison R.S., Musmanno L.A., Kern C.B. BioEssays 15:157-164(1993).
[7] Tso J.Y., Hermodson M.A., Zalkin H. J. Biol. Chem. 255:1451-1457(1980).

224. Glutathione peroxidases signatures (GSHPx)

Glutathione peroxidase (EC 1.11.1.9) (GSHPx) [1,2] is an enzyme that catalyzes the reduction of hydroxyperoxides by glutathione. Its main function is to protect against the damaging effect of endogenously formed hydroxyperoxides. In higher vertebrates at least four forms of GSHPx are known to exist: a ubiquitous cytosolic form (GSHPx-1), a gastrointestinal cytosolic for (GSHPx-GI) [3], a plasma secreted form (GSHPx-P) [4], and a epididymal secretory form (GSHPx-EP). In addition to these characterized forms, the sequence of a protein of unknown function [5] has been shown to be evolutionary related to those of GSHPx's. In filarial nematode parasites such as Brugia pahangi the major soluble cuticular protein, known as gp29, is a secreted GSHPx which could provide a mechanism of resistance to the immune reaction of the mammalian host by neutralizing the products of the oxidative burst of leukocytes [6].Escherichia coli protein btuE, a periplasmic protein involved in the transport of vitamin B 12, is also evolutionary related to GSHPx's; the significance of this relationship is not yet clear. Selenium, in the form of selenocysteine [7] is part of the catalytic site of GSHPx. The sequence around the selenocysteine residue is moderately well conserved in GSHPx's and the related proteins and can be used as a signature pattern. As a second signature for this family of proteins a highly conserved octapeptide located in the central section of these proteins was selected.

Consensus pattern: [GN]-[RKHNFYC]-x-[LIVMFC]-[LIVMF](2)-x-N-[VT]-x-[STC]-x-C-[GA]-x-T [C is the active site selenocysteine residue]

Consensus pattern: [LIV]-[AGD]-F-P-[CS]-[NG]-Q-

[1] Mannervik B. Meth. Enzymol. 113:490-495(1985).
[2] Mullenbach G.T., Tabrizi A., Irvine B.D., Bell G.I., Tainer J.A., Hallewell R.A. Protein Eng. 2:239-246(1988).
[3] Chu F.F., Doroshow J.H., Esworthy R.S. J. Biol. Chem. 268:2571-2576(1993).
[4] Takahashi K., Akasaka M., Yamamoto Y., Kobayashi C., Mizoguchi J., Koyama J. J. Biochem. 108:145-148 (1990).
[5] Dunn D.K., Howells D.D., Richardson J., Goldfarb P.S. Nucleic Acids Res. 17:6390-6390(1989).
[6] Cookson E., Blaxter M.L., Selkirk M.E. Proc. Natl. Acad. Sci. U.S.A. 89:5837-5841(1992).
[7] Stadtman T.C. Annu. Rev. Biochem. 59:111-127(1990).

225. (GST)

Glutathione S-transferases

Function: conjugation of reduced glutathione to a variety of targets. Also included in the alignment, but are not GSTs S-crystallins from squid. Similarity to GST was previously noted. Eukaryotic elongation factors 1-gamma. Not known to have GST activity; similarity not previously recognized. Supported by HMM and manual alignment inspection. HSP26 family of stress-related proteins. including auxin-regulated proteins in plants and stringent starvation proteins in E. coli. Not known to have GST activity. Similarity not previously recognized. Supported by HMM and manual alignment inspection. Alignment spans entire protein.

226. GTP1/OBG family signature

A widespread family of GTP-binding proteins has been recently characterized [1,2]. This family currently includes: - Mouse and Xenopus protein DRG. - Human protein DRG2. - Drosophila protein 128up. - Fission yeast protein gtp 1. - A Halobacterium cutirubrum hypothetical protein in a ribosomal protein gene cluster. - Bacillus subtilis protein obg. Obg has been experimentally shown to bind GTP. - Escherichia coli hypothetical protein yhbZ. - Haemophilus influenzae hypothetical protein HI0877. - Mycoplasma genitalium hypothetical protein MG384. - Yeast hypothetical protein YAL036c (FUN11). - Yeast hypothetical protein YGR173w. - Caenorhabditis elegans hypothetical protein C02F5.3.The function of the proteins that belong to this family is not yet known. They are polypeptides of about 40 to 48 Kd which contain the five small sequence elements characteristic of GTP-binding proteins [3]. As a signature pattern the region that correspond to the ATP/GTP B motif (also called G-3 inGTP-binding proteins) was selected.

Consensus pattern: D-[LIVM]-P-G-[LIVM](2)-[DEY]-[GN]-A-x(2)-G-x-G-

[1] Sazuka T., Tomooka Y., Ikawa Y., Noda M., Kumar S. Biochem. Biophys. Res. Commun. 189:363-370(1992).
[2] Hudson J.D., Young P.G. Gene 125:191-193(1993).
[3] Bourne H.R., Sanders D.A., McCormick F. Nature 349:117-127(1991).

227. (GTP_EFTU1)

ATP/GTP-binding site motif A (P-loop)

From sequence comparisons and crystallographic data analysis it has been shown [1,2,3,4,5,6] that an appreciable proportion of proteins that bind ATP or GTP share a number of more or less conserved sequence motifs. The best conserved of these motifs is a glycine-rich region, which typically forms a flexible loop between a beta-strand and

an alpha-helix. This loop interacts with one of the phosphate groups of the nucleotide. This sequence motif is generally referred to as the 'A' consensus sequence [1] or the 'P-loop' [5].There are numerous ATP- or GTP-binding proteins in which the P-loop is found. Listed below are a number of protein families for which the relevance of the presence of such motif has been noted: - ATP synthase alpha and beta subunits (see <PDOC00I 37>). - Myosin heavy chains. - Kinesin heavy chains and kinesin-like proteins (see <PDOC00343>). - Dynamins and dynamin-like proteins (see <PDOC00362>). - Guanylate kinase (see <PDOC00670>). - Thymidine kinase (see <PDOC00524>). - Thymidylate kinase (see <PDOC01034>). - Shikimate kinase (see <PDOC00868>). - Nitrogenase iron protein family (nifH/frxC) (see <PDOC00580>). - ATP-binding proteins involved in 'active transport' (ABC transporters) [7] (see <PDOC00185>). - DNA and RNA helicases [8,9,10]. - GTP-binding elongation factors (EF-Tu, EF-1alpha, EF-G, EF-2, etc.). - Ras family of GTP-binding proteins (Ras, Rho, Rab, Ral, Ypt1, SEC4, etc.). - Nuclear protein ran (see <PDOC00859>). - ADP-ribosylation factors family (see <PDOC00781>). - Bacterial dnaA protein (see <PDOC00771>). - Bacterial recA protein (see <PDOC00131>). - Bacterial recF protein (see <PDOC00539>). - Guanine nucleotide-binding proteins alpha subunits (Gi, Gs, Gt, G0, etc.). - DNA mismatch repair proteins mutS family (See <PDOC00388>). - Bacterial type II secretion system protein E (see <PDOC00567>).Not all ATP- or GTP-binding proteins are picked-up by this motif. A number of proteins escape detection because the structure of their ATP-binding site is completely different from that of the P-loop. Examples of such proteins are the E1-E2 ATPases or the glycolytic kinases. In other ATP-or GTP-binding proteins the flexible loop exists in a slightly different form; this is the case for tubulins or protein kinases. A special mention must be reserved for adenylate kinase, in which there is a single deviation from the P-loop pattern: in the last position Gly is found instead of Ser or Thr.

- Consensus pattern: [AG]-x(4)-G-K-[ST]-

[1] Walker J.E., Saraste M., Runswick M.J., Gay N.J. EMBO J. 1:945-951(1982).
[2] Moller W., Amons R. FEBS Lett. 186:1-7(1985).
[3] Fry D.C., Kuby S.A., Mildvan A.S. Proc. Natl. Acad. Sci. U.S.A. 83:907-911(1986).
[4] Dever T.E., Glynias M.J., Merrick W.C. Proc. Natl. Acad. Sci. U.S.A. 84:1814-1818(1987).
[5] Saraste M., Sibbald P.R., Wittinghofer A. Trends Biochem. Sci. 15:430-434(1990).
[6] Koonin E.V. J. Mol. Biol. 229:1165-1174(1993).
[7] Higgins C.F., Hyde S.C., Mimmack M.M., Gileadi U., Gill D.R., Gallagher M.P. J. Bioenerg. Biomembr. 22: 571-592(1990).
[8] Hodgman T.C. Nature 333:22-23(1988) and Nature 333:578-578(1988) (Errata).
[9] Linder P., Lasko P., Ashburner M., Leroy P., Nielsen P.J., Nishi K., Schnier J., Slonimski P.P. Nature 337: 121-122(1989).
[10] Gorbalenya A.E., Koonin E.V., Donchenko A.P., Blinov V.M. Nucleic Acids Res. 17:4713-4730(1989).

GTP-binding elongation factors signature (GTP_EFTU2)

Elongation factors [1,2] are proteins catalyzing the elongation of peptide chains in protein biosynthesis. In both prokaryotes and eukaryotes, there are three distinct types of elongation factors, as described in the following table:------ Eukaryotes Prokaryotes Function------ EF-1 alpha EF-Tu Binds GTP and an aminoacyl-tRNA; delivers the latter to the A site of ribosomes. EF-1beta EF-Ts Interacts with EF-1a/EF-Tu to displace GDP and thus allows the regeneration of GTP-EF-1a. EF-2 EF-G Binds GTP and peptidyl-tRNA and translocates the latter from the A site to the P site.---------------------------------------The GTP-binding elongation factor family also includes the following proteins: - Eukaryotic peptide chain release factor GTP-binding subunits [3]. These proteins interact with release factors that bind to ribosomes that have encountered a stop codon at their decoding site and help them to induce release of the nascent polypeptide. The yeast protein was known as SUP2 (and also as SUP35, SUF12 or GST1) and the human homolog as GSTl-Hs. - Prokaryotic peptide chain release factor 3 (RF-3) (gene prfC). RF-3 is a class-II RF, a GTP-binding protein that interacts with class I RFs (see <PDOC00607>) and enhance their activity [4]. - Prokaryotic GTP-binding protein lepA and its homolog in yeast (gene GUF1) and in Caenorhabditis elegans (ZK1236.1). - Yeast HBS1 [5]. - Rat statin S1 [6], a protein of unknown function which is highly similar to EF-1alpha. - Prokaryotic selenocysteine-specific elongation factor selB [7], which seems to replace EF-Tu for the insertion of selenocysteine directed by the UGA codon. - The tetracycline resistance proteins tetM/tetO [8,9] from various bacteria such as Campylobacter jejuni, Enterococcus faecalis, Streptococcus mutans and Ureaplasma urealyticum. Tetracycline binds to the prokaryotic ribosomal 30S subunit and inhibits binding of aminoacyl-tRNAs. These proteins abolish the inhibitory effect of tetracycline on protein synthesis. - Rhizobium nodulation protein nodQ [10]. - Escherichia coli hypothetical protein yihK [11].In EF-1-alpha, a specific region has been shown [12] to be involved in a conformational change mediated by the hydrolysis of GTP to GDP. This region is conserved in both EF-1 alpha/EF-Tu as well as EF-2/EF-G and thus seems typical for GTP-dependent proteins which bind non-initiator tRNAs to the ribosome. The pattern developed for this family of proteins include that conserved region.

Consensus pattern: D-[KRSTGANQFYW]-x(3)-E-[KRAQ]-x-[RKQD]-[GC]-[IVMK]-[ST]- [IV]-x(2)-[GSTACKRNQ]-

[1] Concise Encyclopedia Biochemistry, Second Edition, Walter de Gruyter, Berlin New-York (1988).
[2] Moldave K. Annu. Rev. Biochem. 54:1109-1149(1985).
[3] Stansfield I., Jones K.M., Kushnirov V.V., Dagkesamanskaya A.R., Poznyakovski A.I., Paushkin S.V., Nierras C.R., Cox B.S., Ter-Avanesyan M.D., Tuite M.F. EMBO J. 14:4365-4373(1995).
[4] Grentzmann G., Brechemier-Baey D., Heurgue-Hamard V., Buckingham R.H. J. Biol. Chem. 270: 10595-10600(1995).
[5] Nelson R.J., Ziegelhoffer T., Nicolet C., Werner-Washburne M., Craig E.A. Cell 71:97-105(1992).
[6] Ann D.K., Moutsatsos I.K., Nakamura T., Lin H.H., Mao P.-L., Lee M.-J., Chin S., Liem R.K.H., Wang E. J. Biol. Chem. 266:10429-10437(1991).
[7] Forchammer K., Leinfeldr W., Bock A. Nature 342:453-456(1989).
[8] Manavathu E.K., Hiratsuka K., Taylor D.E. Gene 62:17-26(1988).
[9] Leblanc D.J., Lee L.N., Titmas B.M., Smith C.J., Tenover F.C. J. Bacteriol. 170:3618-3626(1988).
[10] Cervantes E., Sharma S.B., Maillet F., Vasse J., Truchet G., Rosenberg C. Mol. Microbiol. 3:745-755(1989).
[11] Plunkett G. III, Burland V.D., Daniels D.L., Blattner F.R. Nucleic Acids Res. 21:3391-3398(1993).
[12] Moller W., Schipper A., Amons R. Biochimie 69:983-989(1987).

228. GTP cyclohydrolase II.
GTP cyclohydrolase II catalyses the first committed step in the biosynthesis of riboflavin.

[1] Richter G, Ritz H, Katzenmeier G, Volk R, Kohnle A, Lottspeich F, Allendorf D, Bacher A, J Bacteriol 1993; 175:4045-4051.

229. Galactose-1-phosphate uridyl transferase signatures (Ga1P_UDP_transf)
Galactose-1-phosphate uridyl transferase (EC 2.7.7.10) (galT) catalyzes the transfer of an uridyldiphosphate group on galactose (or glucose) 1-phosphate. During the reaction, the uridyl moiety links to a histidine residue. In the Escherichia coli enzyme, it has been shown [1] that two histidine residues separated by a single proline residue are essential for enzyme activity. On the basis of sequence similarities, two apparently unrelated families seem to exist. Class-I enzymes are found in eukaryotes as well as some bacteria such as Escherichia coli or Streptomyces lividans, while class-II enzymes have been found so far only in bacteria such as Bacillus subtilis or Lactobacillus helveticus [2]. Signature patterns for both families were developed. For class-I enzymes the signature is based on the active site residues. For class-II enzymes a region which also includes two conserved histidines was chosen.
Consensus pattern: F-E-N-[RK]-G-x(3)-G-x(4)-H-P-H-x-Q [The two H's are the active site residues]-
Consensus pattern: D-L-P-I-V-G-G-[ST]-[LIVM](2)-[SA]-H-[DEN]-H-[FY]-Q-G-G - Note: class-I enzymes are structurally related to the HIT family of proteins (see <PDOC00694

[1] Reichardt J.K.V., Berg P. Nucleic Acids Res. 16:9017-9026(1988).
[2] Mollet B., Pilloud N. J. Bacteriol. 173:4464-4473(1991).

230. Gamma-thionins family signature
The following small plant proteins are evolutionary related:

- Gamma-thionins from wheat endosperm (gamma-purothionins) and barley (gamma- hordothionins) which are toxic to animal cells and inhibit protein synthesis in cell free systems [1].
- A flower-specific thionin (FST) from tobacco [2].
- Antifungal proteins (AFP) from the seeds of Brassicaceae species such as radish, mustard, turnip and Arabidopsis thaliana [3].
- Inhibitors of insect alpha-amylases from sorghum [4].
- Probable protease inhibitor P322 from potato.
- A germination-related protein from cowpea [5].
- Anther-specific protein SF18 from sunflower [6]. SF18 is a protein that contains a gamma-thionin domain at its N-terminus and a proline-rich C- terminal domain.
- Soybean sulfur-rich protein SE60 [7].
- Vicia faba antibacterial peptides fabatin-1 and -2.

In their mature form, these proteins generally consist of about 45 to 50amino-acid residues. As shown in the following schematic representation, these peptides contain eight conserved cysteines involved in disulfide bonds.

+------------------------------------------+ |+-------------------+ ||||
xxCxxxxxxxxxxCxxxxxCxxxCxxxxxxxxxCxxxxxxCxCxxxC ******************|***||
|+---|----------------+ |+------------------+
'C': conserved cysteine involved in a disulfide bond.
'*': position of the pattern.
Consensus pattern: [KRG]-x-C-x(3)-[SV]-x(2)-[FYWH]-x-[GF]-x-C-x(5)-C-x(3)-C [The four C's are involved in disulfide bonds]-

[1] Bruix M., Jimenez M.A., Santoro J., Gonzalez C., Colilla F.J., Mendez E., Rico M. Biochemistry 32:715-724 (1993).
[2] Gu Q., Kawata E.E., Morse M.-J., Wu H.-M., Cheung A.Y. Mol. Gen. Genet. 234:89-96(1992).
[3] Terras F.R.G., Torrekens S., van Leuven F., Osborn R.W., Vanderleyden J., Cammue B.P.A., Broekaert W.F. FEBS Lett. 316:233-240(1993).
[4] Bloch C. Jr., Richardson M. FEBS Lett. 279:101-104(1991).
[5] Ishibashi N., Yamauchi D., Miniamikawa T. Plant Mol. Biol. 15:59-64(1990).
[7] Choi Y., Choi Y.D., Lee J.S. Plant Physiol. 101:699-700(1993).

231. Gelsolin. Gelsolin repeat. Number of members: 170

[1]Medline: 97433077. The crystal structure of plasma gelsolin: implications for actin severing, capping, and nucleation. Burtnick LD, Koepf EK, Grimes J, Jones EY, Stuart DI, McLaughlin PJ, Robinson RC; Cell 1997; 90:661-670.

232. Germin family signature
Germins [1] are a family of homopentameric cereal glycoproteins expressed during germination which may play a role in altering the properties of cell walls during germinative growth. It has been shown that wheat and barleygermins act as oxalate oxidases (EC 1.2.3.4), an enzyme that catalyzes the oxidative degradation of oxalate to carbonate and hydrogen peroxide. Germins are highly similar to: - Germin-like proteins from various plants such as rape, violet or white mustard. - Slime mold spherulins 1a and 1b which are proteins that accumulate specifically during spherulation, a process induced by various forms of environmental stress which leads to encystment and dormancy. As a signature pattern the best conserved region was selected: a decapeptide located in the central section of these proteins.
Consensus pattern: G-x(4)-H-x-H-P-x-A-x-E-[LIVM]-

[1] Lane B.G. FASEB J. 8:294-301(1994).

233. (GlutR)
Glutamyl-tRNA reductase signature
Delta-aminolevulinic acid (ALA) is the obligatory precursor for the synthesis of all tetrapyrroles including porphyrin derivatives such as chlorophyll and heme. ALA can be synthesized via two different pathways: the Shemin (or C4) pathway which involves the single step condensation of succinyl-CoA and glycine and which is catalyzed by ALA synthase (EC 2.3.1.37) and via the C5pathway from the five-carbon skeleton of glutamate. The C5 pathway operates in the chloroplast of plants and algae, in cyanobacteria, in some eubacteria and in archaebacteria.
The initial step in the C5 pathway is carried out by glutamyl-tRNA reductase (GluTR) [1] which catalyzes the NADP-dependent conversion of glutamate- tRNA(Glu) to glutamate-1-semialdehyde (GSA) with the concomitant release of tRNA(Glu) which can then be recharged with glutamate by glutamyl-tRNA synthetase.
GluTR is a protein of about 50 Kd (467 to 550 residues) which contains a few conserved region. The best conserved region is located in positions 99 to 122 in the sequence of known GluTR. This region seems important for the activity of the enzyme. We have developed a signature pattern from that conserved region.
Consensus patternH-[LIVM]-x(2)-[LIVM]-[GSTAC](3)-[LIVM]-[DEQ]-S-[LIVMA]-[LIVM](2)-[GF]-E-x-[EQR]-[IV]-[LIT]-[STAG]-Q-[LIVM]-[KR] Sequences known to belong to this class detected by the pattern ALL.

[1] Jahn D., Verkamp E., Soell D. Trends Biochem. Sci. 17:215-218(1992).

234. (Glycoprotease)
Glycoprotease family signature (aka Peptidase_M22)
Glycoprotease (GCP) (EC 3.4.24.57) [1], or o-syaloglycoprotein endopeptidase, is a metalloprotease secreted by Pasteurella haemolytica which specifically cleaves O-sialoglycoproteins such as glycophorin A. The sequence of

GCP is highly similar to the following uncharacterized proteins:

- Escherichia coli hypothetical protein ygjD (ORF-X).
- Bacillus subtilis hypothetical protein ydiE.
- Mycobacterium leprae hypothetical protein U229E.
- Mycobacterium tuberculosis hypothetical protein MtCY78.10.
- Synechocystis strain PCC 6803 hypothetical protein slr0807.
- Methanococcus jannaschii hypothetical protein MJ1130.
- Haloarcula marismortui hypothetical protein in HSH 3'region.
- Yeast hypothetical protein YKR038c.
- Yeast hypothetical protein QRI7.

One of the conserved regions contains two conserved histidines. It is possible that this region is involved in coordinating a metal ion such as zinc.
Consensus pattern[KR]-[GSAT]-x(4)-[FYWLH]-[DQNGK]-x-P-x-[LIVMFY]-x(3)-H-x(2)-[AG]-H-[LIVM] Sequences known to belong to this class detected by the pattern ALL.
Note: these proteins belong to family M22 in the classification of peptidases [2,E1].

[1] Abdullah K.M., Lo R.Y.C., Mellors A. J. Bacteriol. 173:5597-5603(1991).
[2] Rawlings N.D., Barrett A.J. Meth. Enzymol. 248:183-228(1995).

235. (Glucosamine_iso)
Glucosamine/galactosamine-6-phosphate isomerases signature
Glucosamine-6-phosphate isomerase (EC 5.3.1.10) (or Glc-6-P deaminase) is the enzyme responsible for the conversion of glucosamine 6-phosphate into fructose6 phosphate [1]. It is the last specific step in the pathway for N-acetylglucosamine (GlcNAC) utilization in bacteria such as Escherichia coli (gene nagB) or in fungi such as Candida albicans (gene NAG1).Glc-6-P isomerase is evolutionary related to: - A putative Escherichia coli galactosamine-6-phosphate isomerase (gene agaI) [2]. - Escherichia coli hypothetical protein yieK. - Bacillus subtilis hypothetical protein ybfT. As a signature pattern a conserved region located in the central part of these enzymes was selected. This region contains a conserved histidine which has been shown [1], in nagB, to be important for the pyranose ring-opening step of the catalytic mechanism
Consensus pattern: [LIVM]-x(3)-G-x-[LIT]-x-[LIV]-x-[LIVM]-x-G-[LIVM]-G-x- [DEN]-G-H-

[1] Oliva G., Fontes M.R.M., Garratt R.C., Altamirano M.M., Calcagno M.L., Horjales E. Structure 3:1323-1332 (1995).
[2] Reizer J., Ramseier T.M., Reizer A., Charbit A., Saier M.H. Jr. Microbiology 142:231-250(1996).

236. Pneumovirus attachment glycoprotein G (glycoprotein G)
This family includes attachment proteins from respiratory synctial virus. Glycoprotein G has not been shown to have any neuraminidase or hemagglutinin activity (Swiss-Prot). The amino terminus is thought to be cytoplasmic, and the carboxyl terminus extracellular. The extracellular region contains four completely conserved cysteine residues.

[1] Johnson PR, Spriggs MK, Olmsted RA, Collins PL, Proc Natl Acad Sci U S A 1987;84:5625-5629.

237. Glycosyl transferases group 1
Mutations in this domain of Swiss:P37287 lead to disease (Paroxysmal Nocturnal haemoglobinuria). Members of this family transfer activated sugars to a variety of substrates, including glycogen, Fructose-6-phosphate and lipopolysaccharides. Members of this family transfer UDP, ADP, GDP or CMP linked sugars. The eukaryotic glycogen synthases may be distant members of this family.

238. Glycosyl transferases (Glycos_transf_2)
Diverse family, transferring sugar from UDP-glucose, UDP-N-acetyl-galactosamine, GDP-mannose or CDP-abequose, to a range of substrates including cellulose, dolichol phosphate and teichoic acids.

239. (Glucos_transf_3)
Thymidine and pyrimidine-nucleoside phosphorylases signature
Thymidine phosphorylase (EC 2.4.2.4) catalyzes the reversible phosphorolysis of thymidine, deoxyuridine and their analogues to their respective bases and 2-deoxyribose 1-phosphate. This enzyme regulates the availability of

thymidine and is therefore essential to nucleic acid metabolism.
In Escherichia coli (gene deoA), the enzyme is a dimer of identical subunits of about 48 Kd [1]. In humans it was first identified as platelet-derived endothelial cell growth factor (PD-ECGF) [E1] before being recognized [2] as thymidine phosphorylase.
Bacterial pyrimidine-nucleoside phosphorylase (EC 2.4.2.2) (gene pdp) [3] is an enzyme evolutionary and structurally related to thymidine phosphorylase.
A a well conserved region of 19 residues located in the N-terminal part of these proteins signature pattern for these enzymes was selected.
Consensus patternS-[GS]-R-[GA]-[LIV]-x(2)-[TA]-[GA]-G-T-x-D-x-[LIV]-E Sequences known to belong to this class detected by the pattern ALL.

[1] Walter M.R., Cook W.J., Cole L.B., Short S.A., Koszalka G.W., Krenitsky T.A., Ealick S.E. J. Biol. Chem. 265:14016-14022(1990).
[2] Furukawa T., Yoshimura A., Sumizawa T., Haraguchi M., Akiyama S.-I., Fukui K., Yamada Y. Nature 356: 668-668(1992).
[3] Saxild H.H., Andersen L.N., Hammer K. J. Bacteriol. 178:424-434(1996).

240. Glycos_transf_4. Glycosyl transferase. Number of members: 44.

[1] Medline: 95252686. A family of UDP-GlcNAc/MurNAc: polyisoprenol-P GlcNAc/MurNAc-1-P transferases. Lehrman MA; Glycobiology 1994;4:768-771.

241. Glycosyl hydrolases family 15. 21 members.

242. Glycosyl hydrolases family 16 signature
It has been shown [1] that the following glycosyl hydrolases can be classified into a single family on the basis of sequence similarities: - Bacterial beta-1,3-1,4-glucanases, or lichenases, (EC 3.2.1.73) mainly from Bacillus but also from Clostridium thermocellum (gene licB), Fibrobacter succinogenes and Rhodothermus marinus (gene bglA). - Bacillus circulans beta-1,3-glucanase A1 (EC 3.2.1.39) (gene glcA). - Lamarinase (EC 3.2.1.6) from Clostridium thermocellum (gene lam1). - Streptomyces coelicolor agarase (EC 3.2.1.81) (gene dagA). - Alteromonas carrageenovora kappa-carrageenase (EC 3.2.1.83) (gene cgkA).Two closely clustered conserved glutamates have been shown [2] to be involved in the catalytic activity of Bacillus licheniformis lichenase. The region was used that contains these residues as a signature pattern.
Consensus pattern: E-[LIV]-D-[LIV]-x(0,1)-E-x(2)-[GQ]-[KRNF]-x-[PSTA] [The two E's are active site residues]-

[1] HenrissatB. Biochem. J. 280:309-316(1991).
[2] Juncosa M., Pons J., Dot T., Querol E., Planas A. J. Biol. Chem. 269:14530-14535(1994).

243. Glycosyl hydrolases family 17 signature
It has been shown [1,2] that the following glycosyl hydrolases can be classified into a single family on the basis of sequence similarities: - Glucan endo-1,3-beta-glucosidases (EC 3.2.1.39) (endo-(1->3)-beta- glucanase) from various plants. This enzyme may be involved in the defense of plants against pathogens through its ability to degrade fungal cell wall polysaccharides. - Glucan 1,3-beta-glucosidase (EC 3.2.1.58) (exo-(1->3)-beta-glucanase) from yeast (gene BGL2). This enzyme may play a role in cell expansion during growth, in cell-cell fusion during mating, and in spore release during sporulation. - Lichenases (EC 3.2.1.73) (endo-(1->3,1->4)-beta-glucanase) from various plants. The best conserved region in the sequence of these enzymes is located in their central section. This region contains a conserved tryptophan residue which could be involved in the interaction with the glucan substrates [2] and it also contains a conserved glutamate which has been shown [3] to act as the nucleophile in the catalytic mechanism. this region was used as a signature pattern.
Consensus pattern: [LIVM]-x-[LIVMFYWA](3)-[STAG]-E-[STA]-G-W-P-[STN]-x-[SAGQ] [E is an active site residue]-

[1] HenrissatB. Biochem. J. 280:309-316(1991).
[2] Ori N., Sessa G., Lotan T., Himmelhoch S., Fluhr R. EMBO J. 9:3429-3436(1990).
[3] Varghese J.N., Garrett T.P.J., Colman P.M., Chen L., Hoj P.J., Fincher G.B. Proc. Natl. Acad. Sci. U.S.A. 91:2785-2789(1994).

244. Glyoxalase I signatures
Glyoxalase I (EC 4.4.1.5) (lactoylglutathione lyase) catalyzes the first step of the glyoxal pathway, the transformation

of methylglyoxal and glutathioneinto S-lactoylglutathione which is then converted by glyoxalase II to lactic acid [1]. Glyoxalase I is an ubiquitous enzyme which binds one mole of zinc per subunit. The bacterial and yeast enzymes are monomeric while the mammalian one is homodimeric. The sequence of glyoxalase I is well conserved. In bacteria and mammals, the enzyme is a protein of about 130 to 180 residues while in fungi it is about twice longer. In these organisms the enzyme is built out of the tandem repeat of an homologous domain. Two signature patterns for this family were derived. The first one is located in the N-terminal region while the second one is located in the central section of the protein and contains a conserved histidine that could be implicated in the binding of the zinc atom.
Consensus pattern: [HQ]-[IVT]-x-[LIVFY]-x-[IV]-x(5)-[STA]-x(2)-F-[YM]-x(2,3)-[LMF]-G-[LMF]-
Consensus pattern: G-[NTKQ]-x(0,5)-[GA]-[LVFY]-[GH]-H-[IVF]-[CGA]-x-[STAGLE]-x(2)-[DNC]-

[1] Kim N.-S., Umezawa Y., Ohmura S., Kato S. J. Biol. Chem. 268:11217-11221(1993).

245. (Glypican)
Glypicans signature
Glypicans [1,2] are a family of heparan sulfate proteoglycans which are anchored to cell membranes by a glycosylphosphatidylinositol (GPI) linkage. Structurally, these proteins consist of three separate domains:

a) A signal sequence;
b) An extracellular domain of about 500 residues that contains 12 conserved cysteines probably involved in disulfide bonds and which also contains the sites of attachment of the heparan sulfate glycosaminoglycan side chains;
c) A C-terminal hydrophobic region which is post-translationally removed after formation of the GPI-anchor.

The proteins known to belong to this family are:

- Glypican 1 (GPC1).
- Glypican 2 (GPC2) or cerebroglycan.
- Glypican 3 (GPC3) or OCI-5. In man, defects in GPC3 are the cause of a X-linked genetic disease, Simpson-Galabi-Behmel syndrome (SGBS).
- K-glypican.
- Glypican 5 (GPC5).
- Drosophila protein dally.

The signature pattern that was developed for glypicans is located in the central section of the extracellular domain and contains five of the conserved cysteines.
Consensus patternC-x(2)-C-x-G-[LIVM]-x(4)-P-C-x(2)-[FY]-C-x(2)-[LIVM]-x(2)- G-C [The C's are probably involved in a disulfide bonds] Sequences known to belong to this class detected by the pattern ALL, except for dally.

[1] Weksberg R., Squire J.A., Templeton D.M. Nat. Genet. 12:225-227(1996).
[2] Watanabe K., Yamada H., Yamaguchi Y. J. Cell Biol. 130:1207-1218(1995).

246. Granins signatures
Granins (chromogranins or secretogranins) [1] are a family of acidic proteins present in the secretory granules of a wide variety of endocrine and neuro-endocrine cells. The exact function(s) of these proteins is not yet known but they seem to be the precursors of biologically active peptides and/or they may act as helper proteins in the packaging of peptide hormones and neuropeptides. Three members of this family of proteins show some sequence similarities: - Chromogranin A (CGA) [2]. CGA is a protein of about 420 residues; it is the precursor of the peptide pancreastatin which strongly inhibits glucose-induced insulin release from the pancreas. - Secretogranin 1 (chromogranin B). A sulfated protein of about 600 residues. - Secretogranin 2 (chromogranin C). A sulfated protein of about 650 residues. Apart from their subcellular location and the abundance of acidic residues(Asp and Glu), these proteins do not share many structural similarities. Only one short region, located in the C-terminal section, is conserved in all these proteins. Chromogranins A and B share a region of high similarity in their N-terminal section; this region includes two cysteine residues involved in a disulfide bond
Consensus pattern: [DE]-[SN]-L-[SAN]-x(2)-[DE]-x-E-L-
Consensus pattern: C-[LIVM](2)-E-[LIVM](2)-S-[DN]-[STA]-L-x-K-x-S-x(3)- [LIVM]-[STA]-x-E-C [The two C's are linked by a disulfide bond]-

[1] Huttner W.B., Gerdes H.-H., Rosa P. Trends Biochem. Sci. 16:27-30(1991).

[2] Simon J.-P., Aunis D. Biochem. J. 262:1-13(1989).

247. grpE protein signature

In prokaryotes the grpE protein [1] stimulates, jointly with dnaJ, the ATPase activity of the dnaK chaperone. It seems to accelerate the release of ADP from dnaK thus allowing dnaK to recycle more efficiently. GrpE is a protein of about 22 to 25 Kd. In yeast, an evolutionary related mitochondrial protein(gene GRPE) has been shown [2] to associate with the mitochondrial hsp70protein and to thus play a role in the import of proteins from the cytoplasm. As a signature pattern, the most conserved region of grpE was selected. It is located in the C-terminal section.

Consensus pattern: [FL]-[DN]-[PHEA]-x(2)-[HM]-x-A-[LIVMTN]-x(16,20)-G-[FY]-x(3)-[DEG]-x(2)-[LIVM]-[RI]-x-[SA]-x-V-x-[IV]-

[1] Georgopoulos C., Welch W. Annu. Rev. Cell Biol. 9:601-635(1993).
[2] Bolliger L., Deloche O., Glick B.S., Georgopoulos C., Jenoe P., Kronidou N., Horst M., Morishima N., Schatz G. EMBO J. 13:1998-2006(1994).

248. Guanylate kinase signature and profile

Guanylate kinase (EC 2.7.4.8) (GK) [1] catalyzes the ATP-dependent phosphorylation of GMP into GDP. It is essential for recycling GMP and indirectly, cGMP. In prokaryotes (such as Escherichia coli), lower eukaryotes (such as yeast) and in vertebrates, GK is a highly conserved monomeric protein of about 200 amino acids. GK has been shown [2,3,4] to be structurally similar to the following proteins: - Protein A57R (or SalG2R) from various strains of Vaccinia virus. This protein is highly similar to GK, but contains a frameshift mutation in the N-terminal section and could therefore be inactive in that virus. The following proteins are characterized by the presence in their sequence of one or more copies of the DHR domain, a SH3 domain (see <PDOC50002> as well as a C-terminal GK-like domain, these protein are collectively termed MAGUKs (membrane-associated guanylate kinase homologs) [5]: - Drosophila lethal(1)discs large-1 tumor suppressor protein (gene dlg1). This protein is associated with septate junctions in developing flies and defects in the dlg1 gene cause neoplastic overgrowth of the imaginal disks. - Mammalian tight junction protein Zo-1. - A family of mammalian synaptic proteins that seem to interact with the cytoplasmic tail of NMDA receptor subunits. This family currently consist of SAP90/PSD-95, CHAPSYN-110/PSD-93, SAP97/DLG1 and SAP102. - Vertebrate 55 Kd erythrocyte membrane protein (p55). p55 is a palmitoylated, membrane-associated protein of unknown function. - Caenorhabditis elegans protein lin-2, which may play a structural role in the induction of the vulva. - Rat protein CASK. - Human protein DLG2. - Human protein DLG3.There is an ATP-binding site (P-loop) in the N-terminal section of GK. This region is not conserved in the GK-like domain of the above proteins which are therefore unlikely to be kinases. However these proteins retain the residues known, in GK, to be involved in the binding of GMP. As a signature pattern a highly conserved region was selected that contains two arginine and a tyrosine which are involved in GMP-binding

Consensus pattern: T-[ST]-R-x(2)-[KR]-x(2)-[DE]-x(2)-G-x(2)-Y-x-[FY]-[LIVMK]-

[1] Stehle T., Schulz G.E. J. Mol. Biol. 224:1127-1141(1992).
[2] Bryant P.J., Woods D.F. Cell 68:621-622(1992).
[3] Goebl M.G. Trends Biochem. Sci. 17:99-99(1992).
[4] Zschocke P.D., Schiltz E., Schulz G.E. Eur. J. Biochem. 213:263-269(1993).
[5] Woods D.F., Bryant P.J. Mech. Dev. 44:85-89(1994).

249. (Glyco_hydro_35)

Glycosyl hydrolases family 35 putative active site

Beta-galactosidases (EC 3.2.1.23) from mammals, fungi, plants and the bacteria Xanthomonas manihotis are evolutionary related [1,2]. They belong to family 35 in the classification of glycosyl hydrolases [3,E1].

Mammalian beta-galactosidase is a lysosomal enzyme (gene GLB1) which cleaves the terminal galactose from gangliosides, glycoproteins, and glycosaminoglycans and whose deficiency is the cause of the genetic disease Gm (1) gangliosidosis (Morquio disease type B).

On of the best conserved regions in these enzymes contains a glutamic acid residue which, on the basis of similarities with other families of glycosyl hydrolases [4], probably acts as the proton donor in the catalytic mechanism. This region wss used as a signature pattern.

Consensus pattern: G-G-P-[LIVM](2)-x(2)-Q-x-E-N-E-[FY] [The second E is the putative active site residue] Sequences known to belong to this class detected by the pattern ALL.

[1] Taron C.H., Benner J.S., Hornstra L.J., Guthrie E.P. Glycobiology 5:603-610(1995).
[2] Carey A.T., Holt K., Picard S., Wilde R., Tucker G.A., Bird C.R., Schuch W., Seymour G.B. Plant Physiol.

108:1099-1107(1995).
[3] Henrissat B., Bairoch A. Biochem. J. 293:781-788(1993).
[4] Henrissat B., Callebaut I., Fabrega S., Lehn P., Mornon J.-P., Davies G. Proc. Natl. Acad. Sci. U.S.A. 92: 7090-7094(1995).

250. (Glyco_hydro_16)
Glycosyl hydrolases family 16 signature
It has been shown [1] that the following glycosyl hydrolases can be classified into a single family on the basis of sequence similarities:

- Bacterial beta-1,3-1,4-glucanases, or lichenases, (EC 3.2.1.73) mainly from Bacillus but also from Clostridium thermocellum (gene licB), Fibrobacter succinogenes and Rhodothermus marinus (gene bglA).
- Bacillus circulans beta-1,3-glucanase A1 (EC 3.2.1.39) (gene glcA).
- Lamarinase (EC 3.2.1.6) from Clostridium thermocellum (gene lam1).
- Streptomyces coelicolor agarase (EC 3.2.1.81) (gene dagA).
- Alteromonas carrageenovora kappa-carrageenase (EC 3.2.1.83) (gene cgkA).

Two closely clustered conserved glutamates have been shown [2] to be involved in the catalytic activity of Bacillus licheniformis lichenase. The region that contains these residues as a signature pattern was used.
Consensus pattern E-[LIV]-D-[LIV]-x(0,1)-E-x(2)-[GQ]-[KRNF]-x-[PSTA] [The two E's are active site residues]

[1] HenrissatB. Biochem. J. 280:309-316(1991).
[2] Juncosa M., Pons J., Dot T., Querol E., Planas A. J. Biol. Chem. 269:14530-14535(1994).

251. (Glyco_hydro_17)
Glycosyl hydrolases family 17 signature
(aka glycosyl_hydro4)
It has been shown [1,2] that the following glycosyl hydrolases can be classified into a single family on the basis of sequence similarities:

- Glucan endo-1,3-beta-glucosidases (EC 3.2.1.39) (endo-(1->3)-beta-glucanase) from various plants. This enzyme may be involved in the defense of plants against pathogens through its ability to degrade fungal cell wall polysaccharides.
- Glucan 1,3-beta-glucosidase (EC 3.2.1.58) (exo-(1->3)-beta-glucanase) from yeast (gene BGL2). This enzyme may play a role in cell expansion during growth, in cell-cell fusion during mating, and in spore release during sporulation.
- Lichenases (EC 3.2.1.73) (endo-(1->3,1->4)-beta-glucanase) from various plants.

The best conserved region in the sequence of these enzymes is located in their central section. This region contains a conserved tryptophan residue which could be involved in the interaction with the glucan substrates [2] and it also contains a conserved glutamate which has been shown [3] to act as the nucleophile in the catalytic mechanism. This region was used as a signature pattern.
Consensus pattern [LIVM]-x-[LIVMFYWA](3)-[STAG]-E-[STA]-G-W-P-[STN]-x-[SAGQ] [E is an active site residue]
Sequences known to belong to this class detected by the pattern ALL.

[1] HenrissatB. Biochem. J. 280:309-316(1991).
[2] Ori N., Sessa G., Lotan T., Himmelhoch S., Fluhr R. EMBO J. 9:3429-3436(1990).
[3] Varghese J.N., Garrett T.P.J., Colman P.M., Chen L., Hoj P.J., Fincher G.B. Proc. Natl. Acad. Sci. U.S.A. 91:2785-2789(1994).

252. (Glyco_hydro_3)
Glycosyl hydrolases family 3 active site
It has been shown [1,2] that the following glycosyl hydrolases can be, on the basis of sequence similarities, classified into a single family:

- Beta glucosidases (EC 3.2.1.21) from the fungi Aspergillus wentii (A-3), Hansenula anomala, Kluyveromyces fragilis, Saccharomycopsis fibuligera, (BGL1 and BGL2), Schizophyllum commune and Trichoderma reesei (BGL1).

- Beta glucosidases from the bacteria Agrobacterium tumefaciens (Cbg1), Butyrivibrio fibrisolvens (bglA), Clostridium thermocellum (bglB), Escherichia coli (bglX), Erwinia chrysanthemi (bgxA) and Ruminococcus albus.
- Alteromonas strain O-7 beta-hexosaminidase A (EC 3.2.1.52).
- Bacillus subtilis hypothetical protein yzbA.
- Escherichica coli hypothetical protein ycfO and HI0959, the corresponding Haemophilus influenzae protein.

One of the conserved regions in these enzymes is centered on a conserved aspartic acid residue which has been shown [3], in Aspergillus wentii beta- glucosidase A3, to be implicated in the catalytic mechanism. This region was used as a signature pattern.
Consensus pattern[LIVM](2)-[KR]-x-[EQK]-x(4)-G-[LIVMFT]-[LIVT]-[LIVMF]- [ST]-D-x(2)-[SGADNI] [D is the active site residue] Sequences known to belong to this class detected by the patternALL.

[1] HenrissatB. Biochem. J. 280:309-316(1991).
[2] Castle L.A., Smith K.D., Morris R.O. J. Bacteriol. 174:1478-1486(1992).
[3] Bause E., Legler G. Biochim. Biophys. Acta 626:459-465(1980).

253. (Glyco_hydro_28)
Polygalacturonase active site (aka PG)
Polygalacturonase (EC 3.2.1.15) (PG) (pectinase) [1,2] catalyzes the random hydrolysis of 1,4-alpha-D-galactosiduronic linkages in pectate and other galacturonans. In fruit, polygalacturonase plays an important role in cell wall metabolism during ripening. In plant bacterial pathogens such as Erwinia carotovora or Pseudomonas solanacearum and fungal pathogens such as Aspergillus niger, polygalacturonase is involved in maceration and soft-rotting of plant tissue.
Exo-poly-alpha-D-galacturonosidase (EC 3.2.1.82) (exoPG) [3] hydrolyzes peptic acid from the non-reducing end, releasing digalacturonate.
Prokaryotic, eukaryotic PG and exoPG share a few regions of sequence similarity. The best conserved of these regions was selected. It is centered on a conserved histidine most probably involved in the catalytic mechanism [4].
Consensus pattern[GSDENKRH]-x(2)-[VMFC]-x(2)-[GS]-H-G-[LIVMAG]-x(1,2)-[LIVM]-G-S [H is the putative active site residue] Sequences known to belong to this class detected by the patternALL.
Note: these proteins belong to family 28 in the classification of glycosyl hydrolases [5].

[1] Ruttowski E., Labitzke R., Khanh N.Q., Loeffler F., Gottschalk M., Jany K.-D. Biochim. Biophys. Acta 1087: 104-106(1990).
[2] Huang J., Schell M.A. J. Bacteriol. 172:3879-3887(1990).
[3] He S.Y., Collmer A. J. Bacteriol. 172:4988-4995(1990).
[4] Bussink H.J.D., Buxton F.P., Visser J. Curr. Genet. 19:467-474(1991).
[5] Henrissat B. Biochem. J. 280:309-316(1991).

254. (Glyco_hydro_32)
Glycosyl hydrolases family 32 active site
It has been shown [1,2] that the following glycosyl hydrolases can be classified into a single family on the basis of sequence similarities:

- Inulinase (EC 3.2.1.7) (or inulase) from the fungi Kluyveromyces marxianus.
- Beta-fructofuranosidase (EC 3.2.1.26), commonly known as invertase in fungi and plants and as sucrase in bacteria (gene sacA or scrB).
- Raffinose invertase (EC 3.2.1.26) (gene rafD) from Escherichia coli plasmid pRSD2.
- Levanase (EC 3.2.1.65) (gene sacC) from Bacillus subtilis.

One of the conserved regions in these enzymes is located in the N-terminal section and contains an aspartic acid residue which has been shown [3], in yeast invertase to be important for the catalytic mechanism. This region was used as a signature pattern.
Consensus pattern H-x(2)-P-x(4)-[LIVM]-N-D-P-N-G [D is the active site residue] Sequences known to belong to this class detected by the patternALL.

[1] HenrissatB. Biochem. J. 280:309-316(1991).
[2] Gunasekaran P., Karunakaran T., Cami B., Mukundan A.G., Preziosi L., Baratti J. J. Bacteriol. 172:6727-6735 (1990).

[3] Reddy V.A., Maley F. J. Biol. Chem. 265:10817-10120(1990).

255. (Glyco hydro_1)
Glycosyl hydrolases family 1 signatures
It has been shown [1 to 4] that the following glycosyl hydrolases can be, on the basis of sequence similarities, classified into a single family:

- Beta-glucosidases (EC 3.2.1.21) from various bacteria such as Agrobacterium strain ATCC 21400, Bacillus polymyxa, and Caldocellum saccharolyticum.
- Two plants (clover) beta-glucosidases (EC 3.2.1.21).
- Two different beta-galactosidases (EC 3.2.1.23) from the archaebacteria Sulfolobus solfataricus (genes bgaS and lacS).
- 6-phospho-beta-galactosidases (EC 3.2.1.85) from various bacteria such as Lactobacillus casei, Lactococcus lactis, and Staphylococcus aureus.
- 6-phospho-beta-glucosidases (EC 3.2.1.86) from Escherichia coli (genes bg1B and ascB) and from Erwinia chrysanthemi (gene arbB).
- Plants myrosinases (EC 3.2.3.1) (sinigrinase) (thioglucosidase).
- Mammalian lactase-phlorizin hydrolase (LPH) (EC 3.2.1.108 / EC 3.2.1.62). LPH, an integral membrane glycoprotein, is the enzyme that splits lactose in the small intestine. LPH is a large protein of about 1900 residues which contains four tandem repeats of a domain of about 450 residues which is evolutionary related to the above glycosyl hydrolases.

One of the conserved regions in these enzymes is centered on a conserved glutamic acid residue which has been shown [5], in the beta-glucosidase from Agrobacterium, to be directly involved in glycosidic bond cleavage by acting as a nucleophile. This region was used as a signature pattern. As a second signature pattern we selected a conserved region, found in the N-terminal extremity of these enzymes, this region also contains a glutamic acid residue.
Consensus pattern[LIVMFSTC]-[LIVFYS]-[LIV]-[LIVMST]-E-N-G-[LIVMFAR]-[CSAGN] [E is the active site residue]
Sequences known to belong to this class detected by the patternALL.
Note: this pattern will pick up the last two domains of LPH; the first two domains, which are removed from the LPH precursor by proteolytic processing, have lost the active site glutamate and may therefore be inactive [4].
Consensus patternF-x-[FYWM]-[GSTA]-x-[GSTA]-x-[GSTA](2)-[FYNH]-[NQ]-x-E-x-[GSTA] Sequences known to belong to this class detected by the pattern ALL.
Note: this pattern will pick up the last three domains of LPH.

[1] HenrissatB. Biochem. J. 280:309-316(1991).
[2] Henrissat B. Protein Seq. Data Anal. 4:61-62(1991).
[3] Gonzalez-Candelas L., Ramon D., Polaina J. Gene 95:31-38(1990).
[4] El Hassouni M., Henrissat B., Chippaux M., Barras F. J. Bacteriol. 174:765-777(1992).
[5] Withers S.G., Warren R.A.J., Street I.P., Rupitz K., Kempton J.B., Aebersold R. J. Am. Chem. Soc. 112: 5887-5889(1990).

256. Glyco_hydro_20
Glycosyl hydrolase family 20
Previous Pfam IDs: glycosyl_hydr11;
Number of members: 33

257. (Glyco_hydro_9)
Glycosyl hydrolases family 9 active sites signatures
(aka Glycosyl_hydr12)
The microbial degradation of cellulose and xylans requires several types of enzymes such as endoglucanases (EC 3.2.1.4), cellobiohydrolases (EC 3.2.1.91) (exoglucanases), or xylanases (EC 3.2.1.8) [1,2]. Fungi and bacteria produces a spectrum of cellulolytic enzymes (cellulases) and xylanases which, on the basis of sequence similarities, can be classified into families. One of these families is known as the cellulase family E [3] or as the glycosyl hydrolases family 9 [4,E1]. The enzymes which are currently known to belong to this family are listed below.

- Butyrivibrio fibrisolvens cellodextrinase 1 (ced1).
- Cellulomonas fimi endoglucanases B (cenB) and C (cenC).
- Clostridium cellulolyticum endoglucanase G (celCCG).

- Clostridium cellulovorans endoglucanase C (engC).
- Clostridium stercoararium endoglucanase Z (avicelase I) (celZ).
- Clostridium thermocellum endoglucanases D (celD), F (celF) and I (cell).
- Fibrobacter succinogenes endoglucanase A (endA).
- Pseudomonas fluorescens endoglucanase A (celA).
- Streptomyces reticuli endoglucanase 1 (cell).
- Thermomonospora fusca endoglucanase E-4 (celD).

- Dictyostelium discoideum spore germination specific endoglucanase 270-6. This slime mold enzyme may digest the spore cell wall during germination, to release the enclosed amoeba.
- Endoglucanases from plants such as Avocado or French bean. In plants this enzyme may be involved the fruit ripening process.

Two of the most conserved regions in these enzymes are centered on conserved residues which have been shown [5,6], in the endoglucanase D from Cellulomonas thermocellum, to be important for the catalytic activity. The first region contains an active site histidine and the second region contains two catalytically important residues: an aspartate and a glutamate. Both regions were used as signature patterns.
Consensus pattern [STV]-x-[LIVMFY]-[STV]-x(2)-G-x-[NKR]-x(4)-[PLIVM]-H-x-R [H is an active site residue] Sequences known to belong to this class detected by the pattern ALL, except for Cellulomonas fimi cenC and Streptomyces reticuli cell.
Consensus pattern [FYW]-x-D-x(4)-[FYW]-x(3)-E-x-[STA]-x(3)-N-[STA] [D and E are active site residues] Sequences known to belong to this class detected by the pattern ALL, except for Fibrobacter succinogenes endA whose sequence seems to be incorrect.

[1] Beguin P. Annu. Rev. Microbiol. 44:219-248(1990).
[2] Gilkes N.R., Henrissat B., Kilburn D.G., Miller R.C. Jr., Warren R.A.J. Microbiol. Rev. 55:303-315(1991).
[3] Henrissat B., Claeyssens M., Tomme P., Lemesle L., Mornon J.-P. Gene 81:83-95(1989).
[4] Henrissat B. Biochem. J. 280:309-316(1991).
[5] Tomme P., Chauvaux S., Beguin P., Millet J., Aubert J.-P., Claeyssens M. J. Biol. Chem. 266:10313-10318 (1991).
[6] Tomme P., van Beeumen J., Claeyssens M. Biochem. J. 285:319-324(1992).

258. Matrix protein (MA), p15 (GAG_ma)
The matrix protein, p15, is encoded by the gag gene. MA is involved in pathogenicity [1].

[1] Pozsgay JM, Beilharz MW, Wines BD, Hess AD, Pitha PM, J Virol 1993;67:5989-5999.

259. Gag polyprotein, inner coat protein p12 (GAG_P12)
The retroviral p12 is a virion structural protein. p12 is proline rich. The function carried out by p12 in assembly and replication is unknown. p12C is associated with pathogenicity of the virus

[1] Pozsgay JM, Beilharz MW, Wines BD, Hess AD, Pitha PM, J Virol 1993;67:5989-5999.

260. Glutamine synthetase signatures (GLN-SYNT)
Glutamine synthetase (EC 6.3.1.2) (GS) [1] plays an essential role in the metabolism of nitrogen by catalyzing the condensation of glutamate and ammonia to form glutamine. There seem to be three different classes of GS [2,3,4]: - Class I enzymes (GSI) are specific to prokaryotes, and are oligomers of 12 identical subunits. The activity of GSI-type enzyme is controlled by the adenylation of a tyrosine residue. The adenylated enzyme is inactive. - Class II enzymes (GSII) are found in eukaryotes and in bacteria belonging to the Rhizobiaceae, Frankiaceae, and Streptomycetaceae families (these bacteria have also a class-I GS). GSII are octamer of identical subunits. Plants have two or more isozymes of GSII, one of the isozymes is translocated into the chloroplast. - Class III enzymes (GSIII) has, currently, only been found in Bacteroides fragilis and in butyrivibrio fibrisolvens. It is a hexamer of identical chains. It is much larger (about 700 amino acids) than the GSI (450 to 470 amino acids) or GSII (350 to 420 amino acids) enzymes. While the three classes of GS's are clearly structurally related, the sequence similarities are not so extensive. As signature patterns three conserved regions were selected. The first pattern is based on a conserved tetrapeptide in the N-terminal section of the enzyme, the second one is based on a glycine-rich region which is thought to be involved in ATP-binding. The third pattern is specific to class I glutamine synthetases and includes the tyrosine residue which is reversibly adenylated.

Consensus pattern: [FYWL]-D-G-S-S-x(6,8)-[DENQSTAK]-[SA]-[DE]-x(2)-[LIVMFY]-
Consensus pattern: K-P-[LIVMFYA]-x(3,5)-[NPAT]-G-[GSTAN]-G-x-H-x(3)-S-
Consensus pattern: K-[LIVM]-x(5)-[LIVMA]-D-[RK]-[DN]-[LI]-Y [Y is the site of adenylation]-

[1] Eisenberg D., Almassy R.J., Janson C.A., Chapman M.S., Suh S.W., Cascio D., Smith W.W. Cold Spring Harbor Symp. Quant. Biol. 52:483-490(1987).
[2] Kumada Y., Benson D.R., Hillemann D., Hosted T.J., Rochefort D.A., Thompson C.J., Wohlleben W., Tateno Y. Proc. Natl. Acad. Sci. U.S.A. 90:3009-3013(1993).
[3] Shatters R.G., Kahn M.L. J. Mol. Evol. 29:422-428(1989).
[4] Brown J.R., Masuchi Y., Robb F.T., Doolittle W.F. J. Mol. Evol. 38:566-576(1994).

261. Globins profile (globin1)
Globins are heme-containing proteins involved in binding and/or transporting oxygen [1]. They belong to a very large and well studied family which is widely distributed in many organisms. The major groups of globins are: - Hemoglobins (Hb) from vertebrates. Hb is the protein responsible for transporting oxygen from the lungs to other tissues. It is a tetramer of two alpha and two beta chains. Most vertebrate species also express specific embryonic or fetal forms of hemoglobin where the alpha or the beta chains are replaced by a chain with higher oxygen affinity, as for the gamma, delta, epsilon and zeta chains in mammals, for example. - Myoglobins (Mg) from vertebrates. Mg is a monomeric protein responsible for oxygen storage in muscles. - Invertebrate globins [2]. A wide variety of globins are found in invertebrates. Molluscs generally have one or two muscle globins which are either monomeric or dimeric. Insects, such as the midge Chironomus thummi, have a large set of extracellular globins. Nematodes and annelids have a variety of intracellular and extracellular globins; some of them are multi- domain polypeptides (from two up to nine-domain globins) and some produce large, disulfide-bonded aggregates. - Leghemoglobins (Lg) from the root nodules of leguminous plants. Lg provides oxygen for bacteroids. - Flavohemoproteins from bacteria (Escherichia coli hmpA) and fungi [3]. These proteins consist of two distinct domains: an N-terminal globin domain and a C-terminal FAD-containing reductase domain. In bacteria such as Vitreoscilla, the enzyme-associated globin is a single domain protein. All these globins seem to have evolved from a common ancestor. The profile developed to detect members of the globin family is based on a structural alignment of selected globin sequences

[1] Concise Encyclopedia Biochemistry, Second Edition, Walter de Gruyter, Berlin New-York (1988).
[2] Goodman M., Pedwaydon J., Czelusniak J., Suzuki T., Gotoh T., Moens L., Shishikura F., Walz D., Vinogradov S. J. Mol. Evol. 27:236-249(1988).

Plant hemoglobins signature (globin2)
Leghemoglobins [1] are hemoproteins present in the root nodules of leguminousplants. Leghemoglobins are structurally and functionally related to hemoglobin and myoglobin. By providing oxygen to the bacteroids, they are essential for symbiotic nitrogen fixation. Structurally related hemoglobins from the nodules of non-leguminous plants [2,3], and from the roots of non-nodulating plants[4] have been recently sequenced. A signature pattern was developed that picks up the sequence of plants hemoglobins, exclusively.
Consensus pattern: [SN]-P-x-L-x(2)-H-A-x(3)-F-

[1] Powell R., Cannon F. BioEssays 9:117-121(1988).
[2] Kortt A.A., Trinick M.J., Appleby C.A. Eur. J. Biochem. 175:141-149(1988).
[3] Kortt A.A., Inglis A.S., Fleming A.I., Appleby C.A. FEBS Lett. 231:341-346(1988).
[4] Bogusz D., Appleby C.A., Landsmann J., Dennis E.S., Trinick M.J., Peacock W.J. Nature 331:178-180(1988).

262. Fructose-bisphosphate aldolase class-I active site (glycolytic_enz)
Fructose-bisphosphate aldolase [1,2] is a glycolytic enzyme that catalyzes the reversible aldol cleavage or condensation of fructose-1,6-bisphosphate into dihydroxyacetone-phosphate and glyceraldehyde 3-phosphate.There are two classes of fructose-bisphosphate aldolases with different catalytic mechanisms. Class-I aldolases [3], mainly found in higher eukaryotes, are homotetrameric enzymes which form a Schiff-base intermediate between the C-2 carbonyl group of the substrate (dihydroxyacetone phosphate)and the epsilon-amino group of a lysine residue. In vertebrates, three forms of this enzyme are found: aldolase A in muscle, aldolase B in liver and aldolase C in brain. The sequence around the lysine involved in the Schiff-base is highly conserved and can be used as a signature for this class of enzyme.
Consensus pattern: [LIVM]-x-[LIVMFYW]-E-G-x-[LS]-L-K-P-[SN] [K is involved in Schiff-base formation]-

[1] Perham R.N. Biochem. Soc. Trans. 18:185-187(1990).

[2] Marsh J.J., Lebherz H.G. Trends Biochem. Sci. 17:110-113(1992).
[3] Freemont P.S., Dunbar B., Fothergill-Gilmore L.A. Biochem. J. 249:779-788(1988).

263. Glycosyl hydrolases family 11 active sites signatures
The microbial degradation of cellulose and xylans requires several types of enzymes such as endoglucanases (EC 3.2.1.4), cellobiohydrolases (EC 3.2.1.91) (exoglucanases), or xylanases (EC 3.2.1.8) [1,2]. Fungi and bacteria produces a spectrum of cellulolytic enzymes (cellulases) and xylanases which, on the basis of sequence similarities, can be classified into families. One of these families is known as the cellulase family G [3] or as the glycosyl hydrolases family 11 [4,E1]. The enzymes which are currently known to belong to this family are listed below. - Aspergillus awamori xylanase C (xynC). - Bacillus circulans, pumilus, stearothermophilus and subtilis xylanase (xynA). - Clostridium acetobutylicum xylanase (xynB). - Clostridium stercorarium xylanase A (xynA). - Fibrobacter succinogenes xylanase C (xynC) which consist of two catalytic domains that both belong to family 10. - Neocallimastix patriciarum xylanase A (xynA). - Ruminococcus flavefaciens bifunctional xylanase XYLA (xynA). This protein consists of three domains: a N-terminal xylanase catalytic domain that belongs to family 11 of glycosyl hydrolases; a central domain composed of short repeats of Gln, Asn an Trp, and a C-terminal xylanase catalytic domain that belongs to family 10 of glycosyl hydrolases. - Schizophyllum commune xylanase A. - Streptomyces lividans xylanases B (xlnB) and C (xlnC). - Trichoderma reesei xylanases I and II. Two of the conserved regions in these enzymes are centered on glutamic acidresidues which have both been shown [5], in Bacillus pumilis xylanase, to be necessary for catalytic activity. Both regions were used as signature patterns.
Consensus pattern: [PSA]-[LQ]-x-E-Y-Y-[LIVM](2)-[DE]-x-[FYWHN] [E is an active site residue]-
Consensus pattern: [LIVMF]-x(2)-E-[AG]-[YWG]-[QRFGS]-[SG]-[STAN]-G-x-[SAF] [E is an active site residue]-

[1] Beguin P. Annu. Rev. Microbiol. 44:219-248(1990).
[2] Gilkes N.R., Henrissat B., Kilburn D.G., Miller R.C. Jr., Warren R.A.J. Microbiol. Rev. 55:303-315(1991).
[3] Henrissat B., Claeyssens M., Tomme P., Lemesle L., Mornon J.-P. Gene 81:83-95(1989).
[4] Henrissat B. Biochem. J. 280:309-316(1991).
[5] Ko E.P., Akatsuka H., Moriyama H., Shinmyo A., Hata Y., Katsube Y., Urabe I., Okada H. Biochem. J. 288: 117-121(1992).

264. Glycosyl hydrolase family 14
This family are beta amylases.

265. Glycosyl hydrolases family 1 signatures
It has been shown [1 to 4] that the following glycosyl hydrolases can be, on the basis of sequence similarities, classified into a single family: - Beta-glucosidases (EC 3.2.1.21) from various bacteria such as Agrobacterium strain ATCC 21400, Bacillus polymyxa, and Caldocellum saccharolyticum. - Two plants (clover) beta-glucosidases (EC 3.2.1.21). - Two different beta-galactosidases (EC 3.2.1.23) from the archaebacteria Sulfolobus solfataricus (genes bgaS and lacS). - 6-phospho-beta-galactosidases (EC 3.2.1.85) from various bacteria such as Lactobacillus casei, Lactococcus lactis, and Staphylococcus aureus. - 6-phospho-beta-glucosidases (EC 3.2.1.86) from Escherichia coli (genes bglB and ascB) and from Erwinia chrysanthemi (gene arbB). - Plants myrosinases (EC 3.2.3.1) (sinigrinase) (thioglucosidase). - Mammalian lactase-phlorizin hydrolase (LPH) (EC 3.2.1.108 / EC 3.2.1.62). LPH, an integral membrane glycoprotein, is the enzyme that splits lactose in the small intestine. LPH is a large protein of about 1900 residues which contains four tandem repeats of a domain of about 450 residues which is evolutionary related to the above glycosyl hydrolases. One of the conserved regions in these enzymes is centered on a conserved glutamic acid residue which has been shown [5], in the beta-glucosidase from Agrobacterium, to be directly involved in glycosidic bond cleavage by acting as a nucleophile. This region was used as a signature pattern. As a second signature pattern a conserved region was selected, found in the N-terminal extremity of these enzymes, this region also contains a glutamic acid residue.
Consensus pattern: [LIVMFSTC]-[LIVFYS]-[LIV]-[LIVMST]-E-N-G-[LIVMFAR]-[CSAGN] [E is the active site residue]
Note: this pattern will pick up the last two domains of LPH; the first two domains, which are removed from the LPH precursor by proteolytic processing, have lost the active site glutamate and may therefore be inactive [4].
Consensus pattern: F-x-[FYWM]-[GSTA]-x-[GSTA]-x-[GSTA](2)-[FYNH]-[NQ]-x-E-x-[GSTA]-

[1] HenrissatB. Biochem. J. 280:309-316(1991).
[2] Henrissat B. Protein Seq. Data Anal. 4:61-62(1991).
[3] Gonzalez-Candelas L., Ramon D., Polaina J. Gene 95:31-38(1990).
[4] E1 Hassouni M., Henrissat B., Chippaux M., Barras F. J. Bacteriol. 174:765-777(1992).
[5] Withers S.G., Warren R.A.J., Street I.P., Rupitz K., Kempton J.B., Aebersold R. J. Am. Chem. Soc. 112:

5887-5889(1990).

266. Glycosyl hydrolases family 2 signatures

It has been shown [1,2,E1] that the following glycosyl hydrolases can be, on the basis of sequence similarities, classified into a single family: - Beta-galactosidases (EC 3.2.1.23) from bacteria such as Escherichia coli (genes lacZ and ebgA), Clostridium acetobutylicum, Clostridium thermosulfurogenes, Klebsiella pneumoniae, Lactobacillus delbrueckii, or Streptococcus thermophilus and from the fungi Kluyveromyces lactis. - Beta-glucuronidase (EC 3.2.1.31) from Escherichia coli (gene uidA) and from mammals. One of the conserved regions in these enzymes is centered on a conserved glutamic acid residue which has been shown [3], in Escherichia coli lacZ, to be the general acid/base catalyst in the active site of the enzyme. This region was used as a signature pattern. As a second signature pattern a highly conserved region was selected located some sixty residues upstream from the active site glutamate.

Consensus pattern: N-x-[LIVMFYWD]-R-[STACN](2)-H-Y-P-x(4)-[LIVMFYWS](2)-x(3)- [DN]-x(2)-G-[LIVMFYW](4)-

Consensus pattern: [DENQLF]-[KRVW]-N-[HRY]-[STAPV]-[SAC]-[LIVMFS](3)-W-[GS]- x(2,3)-N-E [E is the active site residue]-

[1] HenrissatB. Biochem. J. 280:309-316(1991).
[2] Schroeder C.J., Robert C., Lenzen G., McKay L.L., Mercenier A. J. Gen. Microbiol. 137:369-380(1991).
[3] Gebler J.C., Aebersold R., Withers S.G. J. Biol. Chem. 267:11126-11130(1992).

267. Glycosyl hydrolases family 3 active site

It has been shown [1,2] that the following glycosyl hydrolases can be, on the basis of sequence similarities, classified into a single family:

- Beta glucosidases (EC 3.2.1.21) from the fungi Aspergillus wentii (A-3), Hansenula anomala, Kluyveromyces fragilis, Saccharomycopsis fibuligera, (BGL1 and BGL2), Schizophyllum commune and Trichoderma reesei (BGL1).
- Beta glucosidases from the bacteria Agrobacterium tumefaciens (Cbg1), Butyrivibrio fibrisolvens (bglA), Clostridium thermocellum (bglB), Escherichia coli (bglX), Erwinia chrysanthemi (bgxA) and Ruminococcus albus. - Alteromonas strain O-7 beta-hexosaminidase A (EC 3.2.1.52).
- Bacillus subtilis hypothetical protein yzbA.
- Escherichica coli hypothetical protein ycfO and HI0959, the corresponding Haemophilus influenzae protein.

One of the conserved regions in these enzymes is centered on a conserved aspartic acid residue which has been shown [3], in Aspergillus wentii beta-glucosidase A3, to be implicated in the catalytic mechanism. This region was used as a signature pattern.

Consensus pattern: [LIVM](2)-[KR]-x-[EQK]-x(4)-G-[LIVMFT]-[LIVT]-[LIVMF]-[ST]-D-x(2)-[SGADNI] [D is the active site residue]

[1] HenrissatB. Biochem. J. 280:309-316(1991).
[2] Castle L.A., Smith K.D., Morris R.O. J. Bacteriol. 174:1478-1486(1992).
[3] Bause E., Legler G. Biochim. Biophys. Acta 626:459-465(1980).

268. Glycosyl hydrolases family 8 signature

The microbial degradation of cellulose and xylans requires several types of enzymes such as endoglucanases (EC 3.2.1.4), cellobiohydrolases (EC 3.2.1.91)(exoglucanases), or xylanases (EC 3.2.1.8) [1,2]. Fungi and bacteria produces a spectrum of cellulolytic enzymes (cellulases) and xylanases which, on the basis of sequence similarities, can be classified into families. One of these families is known as the cellulase family D [3] or as the glycosyl hydrolases family 8 [4,E1]. The enzymes which are currently known to belong to this family are listed below. - Acetobacter xylinum endonuclease cmcAX. - Bacillus strain KSM-330 acidic endonuclease K (Endo-K). - Cellulomonas josui endoglucanase 2 (celB). - Cellulomonas uda endoglucanase. - Clostridium cellulolyticum endoglucanases C (celcCC). - Clostridium thermocellum endoglucanases A (celA). - Erwinia chrysanthemi minor endoglucanase y (celY). - Bacillus circulans beta-glucanase (EC 3.2.1.73). - Escherichia coli hypothetical protein yhjM. The most conserved region in these enzymes is a stretch of about 20 residues that contains two conserved aspartate. The first asparatate is thought [5] to act as the nucleophile in the catalytic mechanism. This region was used as a signature pattern.

Consensus pattern: A-[ST]-D-[AG]-D-x(2)-[IM]-A-x-[SA]-[LIVM]-[LIVMG]-x-A- x(3)-[FW] [The first D is an active site residue]-

[1] BeguinP. Annu. Rev. Microbiol. 44:219-248(1990).
[2] Gilkes N.R., Henrissat B., Kilburn D.G., Miller R.C. Jr., Warren R.A.J. Microbiol. Rev. 55:303-315(1991).
[3] Henrissat B., Claeyssens M., Tomme P., Lemesle L., Mornon J.-P. Gene 81:83-95(1989).
[4] Henrissat B. Biochem. J. 280:309-316(1991).
[5] Alzari P.M., Souchon H., Dominguez R. Structure 4:265-275(1996).

269. Glycosyl hydrolases family 9 active sites signatures

The microbial degradation of cellulose and xylans requires several types of enzymes such as endoglucanases (EC 3.2.1.4), cellobiohydrolases (EC 3.2.1.91) (exoglucanases), or xylanases (EC 3.2.1.8) [1,2]. Fungi and bacteria produce a spectrum of cellulolytic enzymes (cellulases) and xylanases which, on the basis of sequence similarities, can be classified into families. One of these families is known as the cellulase family E [3] or as the glycosyl hydrolases family 9 [4,E1]. The enzymes which are currently known to belong to this family are listed below. - Butyrivibrio fibrisolvens cellodextrinase 1 (ced1). - Cellulomonas fimi endoglucanases B (cenB) and C (cenC). - Clostridium cellulolyticum endoglucanase G (celCCG). - Clostridium cellulovorans endoglucanase C (engC). - Clostridium stercoararium endoglucanase Z (avicelase I) (celZ). - Clostridium thermocellum endoglucanases D (celD), F (celF) and I (celI). - Fibrobacter succinogenes endoglucanase A (endA). - Pseudomonas fluorescens endoglucanase A (celA). - Streptomyces reticuli endoglucanase 1 (cell). - Thermomonospora fusca endoglucanase E-4 (celD). - Dictyostelium discoideum spore germination specific endoglucanase 270-6. This slime mold enzyme may digest the spore cell wall during germination, to release the enclosed amoeba. - Endoglucanases from plants such as Avocado or French bean. In plants this enzyme may be involved the fruit ripening process. Two of the most conserved regions in these enzymes are centered on conserved residues which have been shown [5,6], in the endoglucanase D from Cellulomonas thermocellum, to be important for the catalytic activity. The first region contains an active site histidine and the second region contains two catalytically important residues: an aspartate and a glutamate. Both regions were used as signature patterns.

Consensus pattern: [STV]-x-[LIVMFY]-[STV]-x(2)-G-x-[NKR]-x(4)-[PLIVM]-H-x-R [H is an active site residue]-

Consensus pattern: [FYW]-x-D-x(4)-[FYW]-x(3)-E-x-[STA]-x(3)-N-[STA] [D and E are active site residues]-

[1] Beguin P. Annu. Rev. Microbiol. 44:219-248(1990).
[2] Gilkes N.R., Henrissat B., Kilburn D.G., Miller R.C. Jr., Warren R.A.J. Microbiol. Rev. 55:303-315(1991).
[3] Henrissat B., Claeyssens M., Tomme P., Lemesle L., Momon J.-P. Gene 81:83-95(1989).
[4] Henrissat B. Biochem. J. 280:309-316(1991).
[5] Tomme P., Chauvaux S., Beguin P., Millet J., Aubert J.-P., Claeyssens M. J. Biol. Chem. 266:10313-10318 (1991).
[6] Tomme P., van Beeumen J., Claeyssens M. Biochem. J. 285:319-324(1992).

270. Glyceraldehyde 3-phosphate dehydrogenase active site (gpdh)

Glyceraldehyde 3-phosphate dehydrogenase (EC 1.2.1.12) (GAPDH) [1] is a tetrameric NAD-binding enzyme common to both the glycolytic and gluconeogenic pathways. A cysteine in the middle of the molecule is involved in forming a covalent phosphoglycerol thioester intermediate. The sequence around this cysteine is totally conserved in eubacterial and eukaryotic GAPDHs and is also present, albeit in a variant form, in the otherwise highly divergent archaebacterial GAPDH [2].Escherichia coli D-erythrose 4-phosphate dehydrogenase (E4PDH) (gene epd orgapB) is an enzyme highly related to GAPDH [3].

Consensus pattern: [ASV]-S-C-[NT]-T-x(2)-[LIM] [C is the active site residue]-

[1] Harris J.I., Waters M. (In) The Enzymes (3rd edition) 13:1-50(1976).
[2] Fabry S., Lang J., Niermann T., Vingron M., Hensel R. Eur. J. Biochem. 179:405-413(1989).
[3] Zhao G., Pease A.J., Bharani N., Winkler M.E. J. Bacteriol. 177:2804-2812(1995).

271. Granulins signature

Granulins [1] are a family of cysteine-rich peptides of about 6 Kd which may have multiple biological activity. A precursor protein (known as acrogranin) potentially encodes seven different forms of granulin (grnA to grnG) which are probably released by post-translational proteolytic processing. A schematic representation of the structure of a granulin is shown below:

xxxCxxxxxCxxxxxCCxxxxxxxxCCxxxxxxCCxxxxxCCxxxxxCxxxxxxCx

**************'C': conserved cysteine probably involved in a disulfide bond.'*': position of the pattern. Granulins are evolutionary related to a PMP-D1, a peptide extracted from thepars intercerebralis of migratory locusts [2].

Consensus pattern: C-x-D-x(2)-H-C-C-P-x(4)-C [The four C's are probably involved in disulfide bonds]-

[1] Bhandari V., Palfree R.G., Bateman A. Proc. Natl. Acad. Sci. U.S.A. 89:1715-1719(1992).
[2] Nakakura N., Hietter H., van Dorsselaer A., Luu B. Eur. J. Biochem. 204:147-153(1992).

272. (HCV RdRp) Hepatitis C virus RNA dependent RNA polymerase
The RNA dependent RNA polymerase is also known as non-structural protein NS5B. NS5B is a 65 kDa protein that resembles other viral RNA polymerases. HCV replication is thought to occur in membrane bound replication complexes. These complexes transcribe the positive strand and the resulting minus strand is used as a template for the synthesis of genomic RNA. There are two viral proteins involved in the reaction, NS3 and NS5B.[1,2]

[1] Lohmann V, Korner F, Herian U, Bartenschlager R; J Virol 1997;71:8416-8428.
[2] Behrens SE, Tomei L, De Francesco R; EMBO J 1996;15:12-22.
[3] Ishido S, Fujita T, Hotta H; Biochem Biophys Res Commun 1998;244:35-40.

273. (HHH) Helix-hairpin-helix motif.

[1] Doherty AJ, Serpell LC, Ponting CP; Nucleic Acids Res 1996;24:2488-2497.

274. HIT family signature
Recently a family of small proteins of about 12 to 16 Kd has been described[1]. This family currently consists of: - Mammalian protein HINT (also known as Protein kinase C inhibitor 1 or PKCI- 1). HINT was incorrectly thought to be a specific inhibitor of PKC. It has been shown to bind zinc. - Fission yeast diadenosine 5',5'''-P1,P4-tetraphosphate asymmetrical hydrolase (Ap4Aase) (EC 3.6.1.17) [2] (gene aphl), which cleaves A-5'-PPPP- 5'A to yield AMP and ATP. - FHIT, a human protein whose gene is altered in different tumors and which acts [3] as a diadenosine 5',5'''-P1,P3-triphosphate hydrolase (Ap3Aase) (EC 3.6.1.29) cleaving A-5'-PPP-5'A to yield AMP and ADP. - Yeast proteins HNT1 and HNT2. - Maize zinc-binding protein ZBP14. - Escherichia coli hypothetical protein ycfF. - Haemophilus influenzae hypothetical protein HI0961. - Helicobacter pylori hypothetical protein HP0404. - Methanococcus jannaschii hypothetical protein MJ0866. - Mycobacterium leprae hypothetical protein U296A. - Synechocystis strain PCC 6803 hypothetical protein s1r1234. - Caenorhabditis elegans hypothetical protein F21C3.3. - A hypothetical 13.2 Kd protein in hisE 3'region in Azospirillum brasilense. - A hypothetical 13.1 Kd protein in p37 5'region in Mycoplasma hyorhinis. - A hypothetical 12.4 Kd protein in psbAII 5'region in Synechococcus strain PCC 7942.All these proteins contains a region with three clustered histidines. This region is responsible for the designation of this family: HIT, for 'HIstidineTriad [1]. This region was originally thought to be implied in the binding of a zinc ion but was later identified [4] as part of the alpha-phosphate binding site of a nucleotide-binding domain. As a signature pattern, the region of the histidine triad was selected.
Consensus pattern: [NQA]-x(4)-[GAV]-x-[QF]-x-[LIVM]-x-H-[LIVMFYT]-H-[LIVMFT]-H-[LIVMF](2)-[PSGA]-

[1] Seraphin B. DNA Seq. 3:177-179(1992).
[2] Huang Y., Garrison P.N., Barnes L.D. Biochem. J. 312:925-932(1995).
[3] Barnes L.D., Garrison P.N., Siprashvili Z., Guranowski A., Robinson A.K., Ingram S.W., Croce C.M., Ohta M., Huebner K. Biochemistry 35:11529-11535(1996).
[4] Brenner C., Garrison P., Gilmour J., Peisach D., Ringe D., Petsko G.A., Lowenstein J.M. Nat. Struct. Biol. 4:231-238(1997).

275. Myc-type, 'helix-loop-helix' dimerization domain signature (HLH)
A number of eukaryotic proteins, which probably are sequence specific DNA-binding proteins that act as transcription factors, share a conserved domain of 40 to 50 amino acid residues. It has been proposed [1] that this domain is formed of two amphipathic helices joined by a variable length linker region that could form a loop. This 'helix-loop-helix' (HLH) domain mediates protein dimerization and has been found in the proteins listed below [2,3,E1,E2]. Most of these proteins have an extra basic region of about 15 amino acid residues that is adjacent to the HLH domain and specifically binds to DNA. They are refered as basic helix-loop-helix proteins (bHLH), and are classified in two groups: class A (ubiquitous) and class B (tissue-specific). Members of the bHLH family bind variations on the core sequence 'CANNTG', also referred to as the E-box motif. The homo- or heterodimerization mediated by the HLH domain is independent of, but necessary for DNA binding, as two basic regions are required for DNA binding activity. The HLH proteins lacking the basic domain (Emc, Id) function as negative regulators since they form heterodimers, but fail to bind DNA. The hairy-related proteins (hairy, E(spl), deadpan) also repress transcription although they can bind DNA. The proteins of this subfamily act together with co-repressor proteins, like groucho, through their C-terminal motif WRPW. - The myc family of cellular oncogenes [4], which is currently known to contain four members: c-myc [E3], N-myc, L-myc, and B-myc. The myc genes are thought to play a role in cellular differentiation and

proliferation. - Proteins involved in myogenesis (the induction of muscle cells). In mammals MyoD1 (Myf-3), myogenin (Myf-4), Myf-5, and Myf-6 (Mrf4 or herculin), in birds CMD1 (QMF-1), in Xenopus MyoD and MF25, in Caenorhabditis elegans CeMyoD, and in Drosophila nautilus (nau). - Vertebrate proteins that bind specific DNA sequences ('E boxes') in various immunoglobulin chains enhancers: E2A or ITF-1 (E12/pan-2 and E47/pan-1), ITF-2 (tcf4), TFE3, and TFEB. - Vertebrate neurogenic differentiation factor 1 that acts as differentiation factor during neurogenesis. - Vertebrate MAX protein, a transcription regulator that forms a sequence- specific DNA-binding protein complex with myc or mad. - Vertebrate Max Interacting Protein 1 (MXI1 protein) which acts as a transcriptional repressor and may antagonize myc transcriptional activity by competing for max. - Proteins of the bHLH/PAS superfamily which are transcriptional activators. In mammals, AH receptor nuclear translocator (ARNT), single-minded homologs (SIM1 and SIM2), hypoxia-inducible factor 1 alpha (HIF1A), AH receptor (AHR), neuronal pas domain proteins (NPAS1 and NPAS2), endothelial pas domain protein 1 (EPAS1), mouse ARNT2, and human BMAL1. In drosophila, single-minded (SIM), AH receptor nuclear translocator (ARNT), trachealess protein (TRH), and similar protein (SIMA). - Mammalian transcription factors HES, which repress transcription by acting on two types of DNA sequences, the E box and the N box. - Mammalian MAD protein (max dimerizer) which acts as transcriptional repressor and may antagonize myc transcriptional activity by competing for max. - Mammalian Upstream Stimulatory Factor 1 and 2 (USF1 and USF2), which bind to a symmetrical DNA sequence that is found in a variety of viral and cellular promoters. - Human lyl-1 protein; which is involved, by chromosomal translocation, in T- cell leukemia. - Human transcription factor AP-4. - Mouse helix-loop-helix proteins MATH-1 and MATH-2 which activate E box- dependent transcription in collaboration with E47. - Mammalian stem cell protein (SCL) (also known as tall), a protein which may play an important role in hemopoietic differentiation. SCL is involved, by chromosomal translocation, in stem-cell leukemia. - Mammalian proteins Id1 to Id4 [5]. Id (inhibitor of DNA binding) proteins lack a basic DNA-binding domain but are able to form heterodimers with other HLH proteins, thereby inhibiting binding to DNA. - Drosophila extra-macrochaetae (emc) protein, which participates in sensory organ patterning by antagonizing the neurogenic activity of the achaete- scute complex. Emc is the homolog of mammalian Id proteins. - Human Sterol Regulatory Element Binding Protein 1 (SREBP-1), a transcriptional activator that binds to the sterol regulatory element 1 (SRE-1) found in the flanking region of the LDLR gene and in other genes. - Drosophila achaete-scute (AS-C) complex proteins T3 (l'sc), T4 (scute), T5 (achaete) and T8 (asense). The AS-C proteins are involved in the determination of the neuronal precursors in the peripheral nervous system and the central nervous system. - Mammalian homologs of achaete-scute proteins, the MASH-1 and MASH-2 proteins. - Drosophila atonal protein (ato) which is involved in neurogenesis. - Drosophila daughterless (da) protein, which is essential for neurogenesis and sex-determination. - Drosophila deadpan (dpn), a hairy-like protein involved in the functional differentiation of neurons. - Drosophila delilah (dei) protein, which is plays an important role in the differentiation of epidermal cells into muscle. - Drosophila hairy (h) protein, a transcriptional repressor which regulates the embryonic segmentation and adult bristle patterning. - Drosophila enhancer of split proteins E(spl), that are hairy-like proteins active during neurogenesis. also act as transcriptional repressors. - Drosophila twist (twi) protein, which is involved in the establishment of germ layers in embryos. - Maize anthocyanin regulatory proteins R-S and LC. - Yeast centromere-binding protein 1 (CPF1 or CBF1). This protein is involved in chromosomal segregation. It binds to a highly conserved DNA sequence, found in centromers and in several promoters. - Yeast INO2 and INO4 proteins. - Yeast phosphate system positive regulatory protein PHO4 which interacts with the upstream activating sequence of several acid phosphatase genes. - Yeast serine-rich protein TYE7 that is required for ty-mediated ADH2 expression. - Neurospora crassa nuc-1, a protein that activates the transcription of structural genes for phosphorus acquisition. - Fission yeast protein esc1 which is involved in the sexual differentiation process. The schematic representation of the helix-loop-helix domain is shown here: xxxxxxxxxxxxxxxxxxxxxxxx-------------------xxxxxxxxxxxxxxxxxxxxxxx Amphipathic helix 1 Loop Amphipathic helix 2. The signature pattern developed to detect this domain spans completely the second amphipathic helix.

Consensus pattern: [DENSTAP]-[KTR]-[LIVMAGSNT]-{FYWCPHKR}-[LIVMT]-[LIVM]- x(2)-[STA V]-[LIVMSTACKR]-x-[VMFYH]-[LIVMTA]-{P}-{P}-[LIVMRKHQ].-


[1] Murre C., McCaw P.S., Baltimore D. Cell 56:777-783(1989).

[2] Garrel J., Campuzano S. BioEssays 13:493-498(1991).

[3] Kato G.J., Dang C.V. FASEB J. 6:3065-3072(1992).

[4] Krause M., Fire A., Harrison S.W., Priess J., Weintraub H. Cell 63:907-919(1990).

[5] Riechmann V., van Cruechten I., Sablitzky F. Nucleic Acids Res. 22:749-755(1994).


276. HMG14 and HMG17 signature

High mobility group (HMG) proteins are a family of relatively low molecular weight nonhistone components in chromatin. HMG14 and HMG17 [1], two related proteins of about 100 amino acid residues, bind to the inner side of the nucleosomal DNA thus altering the interaction between the DNA and the histone octamer. These two proteins may

be involved in the process which maintains transcribable genes in a unique chromatin conformation. The trout nonhistone chromosomal protein H6 (histone T) also belongs to this family. As a signature pattern a conserved stretch of 10 residues located in the N-terminal section of HMG14 and HMG17 was selected.
Consensus pattern: R-R-S-A-R-L-S-A-[RK]-P-

[1] Bustin M., Reeves R. Prog. Nucleic Acid Res. Mol. Biol. 54:35-100(1996).

277. Hydroxymethylglutaryl-coenzyme A lyase active site (HMGL1)
3-hydroxy-3-methylglutaryl-coenzyme A lyase (HMG-CoA lyase or HL) (EC 4.1.3.4)catalyzes the transformation of HMG-CoA into acetyl-CoA and acetoacetate. In vertebrates it is a mitochondrial enyme which is involved in ketogenesis and in leucine catabolism [1]. In some bacteria, such as Pseudomonas mevalonii, it is involved in mevalonate catabolism (gene mvaB). A cysteine has been shown[2], in mvaB, to be required for the activity of the enzyme. The region around this residue is perfectly conserved and is used as a signature pattern.
Consensus pattern: S-V-A-G-L-G-G-C-P-Y [C is the active site residue]-

[1] Mitchell G.A., Robert M.-F., Hruz P.W., Wang S., Fontaine G., Behnke C.E., Mende-Mueller L.M., Schappert K., Lee C., Gibson K.M., Miziorko H.M. J. Biol. Chem. 268:4376-4381(1993).
[2] Hruz P.W., Narasimhan C., Miziorko H.M. Biochemistry 31:6842-6847(1992).

Alpha-isopropylmalate and homocitrate synthases signatures (HMGL2)
The following enzymes have been shown [1] to be functionally as well as evolutionary related: - Alpha-isopropylmalate synthase (EC 4.1.3.12) which catalyzes the first step in the biosynthesis of leucine, the condensation of acetyl-CoA and alpha- ketoisovalerate to form 2-isopropylmalate synthase. - Homocitrate synthase (EC 4.1.3.21) (gene nifV) which is involved in the biosynthesis of the iron-molybdenum cofactor of nitrogenase and catalyzes the condensation of acetyl-CoA and alpha-ketoglutarate into homocitrate. - Soybean late nodulin 56. - Methanococcus jannaschii hypothetical proteins MJ0503, MJ1195 and MJ1392. Two conserved regions were selected as signature patterns for these enzymes. The first region is located in the N-terminal section while the second region is located in the central section and contains two conserved histidine residues which could be implicated in the catalytic mechanism.
Consensus pattern: L-R-[DE]-G-x-Q-x(10)-K-
Consensus pattern: [LIVMFW]-x(2)-H-x-H-[DN]-D-x-G-x-[GAS]-x-[GASLI]-

[1] Wang S.-Z., Dean D.R., Chen J.-S., Johnson J.L. J. Bacteriol. 173:3041-3046(1991).

278. (HMG C0A synt) Hydroxymethylglutaryl-coenzyme A synthase active site Hydroxymethylglutaryl-coenzyme A synthase (EC 4.1.3.5) (HMG-CoA synthase) catalyzes the condensation of acetyl-CoA with acetoacetyl-CoA to produce HMG- CoA and CoA [1].In vertebrates there are two isozymes located in different subcellular compartments: a cytosolic form which is the starting point of the mevalonate pathway which leads to cholesterol and other sterolic and isoprenoid compounds and a mitochondrial form responsible for ketone body biosynthesis. HMG-CoA is also found in other eukaryotes such as insect, plants and fungi. A cysteine is known to act as the catalytic nucleophile in the first step of the reaction, the acetylation of the enzyme by acetyl-CoA. The conserved region was used around this active site residue as a signature pattern.
Consensus pattern: N-x-[DN]-[IV]-E-G-[IV]-D-x(2)-N-A-C-[FY]-x-G [C is the active site residue]-

[1] Rokosz L.L., Boulton D.A., Butkiewicz E.A., Sanyal G., Cueto M.A., Lachance P.A., Hermes J.D. Arch. Biochem. Biophys. 312:1-13(1994).

279. HMG (high mobility group) box

280. HSF-type DNA-binding domain signature
Heat shock factor (HSF) is a DNA-binding protein that specifically binds heat shock promoter elements (HSE). HSE is a palindromic element rich with repetitive purine and pyrimidine motifs: 5'-nGAAnnTTCnnGAAnnTTCn-3'. HSF is expressed at normal temperatures but is activated by heat shock or chemical stressors [1,2]. The sequences of HSF from various species show extensive similarity in a region of about 90 amino acids, which has been shown [3] to bind DNA. Some other proteins also contain a HSF domain, these are: - Yeast SFL1, a protein involved in cell surface assembly and regulation of the gene related to flocculation (asexual cell aggregation) [4]. - Yeast transcription factor SKN7 (or BRY1 or POS9), which binds to the promoter elements SCB and MCB essential for the control of G1 cyclins expression [5]. - Yeast MGA1. - Yeast hypothetical protein YJR147w. A pattern from the most conserved part of the HSF DNA-binding domain was derived, its central region.

Consensus pattern: L-x(3)-[FY]-K-H-x-N-x-[STAN]-S-F-[LIVM]-R-Q-L-[NH]-x-Y-x-[FYW]-[RKH]-K-[LIVM]-

[1] SorgerP.K. Cell 65:363-366(1991).
[2] Mager W.H., Moradas Ferreira P. Biochem. J. 290:1-13(1993).
[3] Vuister G.W., Kim S.-J., Orosz A., Marquardt J., Wu C., Bax A. Nat. Struct. Biol. 1:605-613(1994).
[4] Fujita A., Kikuchi Y., Kuhara S., Misumi Y., Matsumoto S., Kobayashi H. Gene 85:321-328(1989).
[5] Morgan B.A., Bouquin N., Merrill G.F., Johnston L.H. EMBO J. 14:5679-5689(1995).

281. Heat shock hsp20 proteins family profile

Prokaryotic and eukaryotic organisms respond to heat shock or other environmental stress by inducing the synthesis of proteins collectively known as heat-shock proteins (hsp) [1]. Amongst them is a family of proteins with an average molecular weight of 20 Kd, known as the hsp20 proteins [2 to 5]. These seem to act as chaperones that can protect other proteins against heat-induced denaturation and aggregation. Hsp20 proteins seem to form large heterooligomeric aggregates; their family is currently composed of the following members: - Vertebrate heat shock protein hsp27 (hsp25), induced by a variety of environmental stresses. - Drosophila heat shock proteins hsp22, hsp23, hsp26, hsp27, hsp67BA and BC. - Caenorhabditis elegans hsp16 multigene family. - Fungal HSP26 (budding yeast) and hsp30 (Neurospora crassa and Aspergillus Nidulans). - Plant small hsp's. Plants have four classes of hsp20: classes I and II which are cytoplasmic, class III which is chloroplastic and class IV which is found in the endomembrane. - Alpha-crystallin A and B chains. Alpha-crystallin is an abundant constituent of the eye lens of most vertebrate species. Its main function appears to be to maintain the correct refractive index of the lens. It is also found in other tissues where it seems to act as a chaperone [6]. - Schistosoma mansoni major egg antigen p40. Structurally, p40 is built of two tandem hsp20 domains. - A variety of prokaryotic proteins: ibpA and ibpB from Escherichia coli, hsp 18 from Clostridium acetobutylicum, spore protein SP21 (hspA) from Stigmatella aurantiaca, Mycobacterium leprae 18 Kd antigen and Mycobacterium tuberculosis 14 Kd antigen. - Methanococcus jannaschii hypothetical protein MJ0285.Structurally, this family is characterized by the presence of a conserved C-terminal domain of about 100 residues. The profile developed to detect members of the hsp20 family is based on an alignment of this domain. -Sequences known to belong to this class detected by the profile: ALL.

[1] Lindquist S., Craig E.A. Annu. Rev. Genet. 22:631-677(1988).
[2] de Jong W.W., Leunissen J.A.M., Voorter C.E.M. Mol. Biol. Evol. 10:103-126(1993).
[3] Caspers G.J., Leunissen J.A.M., de Jong W.W. J. Mol. Evol. 40:238-248(1995).
[4] Jaenicke R., Creighton T.E. Curr. Biol. 3:234-235(1993).
[5] Jakob U., Buchner J. Trends Biochem. Sci. 19:205-211(1994).
[6] Groenen P.J.T.A., Merck K.B., de Jong W.W., Bloemendal H. Eur. J. Biochem. 225:1-9(1994).

282. Heat shock hsp70 proteins family signatures

Prokaryotic and eukaryotic organisms respond to heat shock or other environmental stress by the induction of the synthesis of proteins collectively known as heat-shock proteins (hsp) [1]. Amongst them is a family of proteins with an average molecular weight of 70 Kd, known as the hsp70proteins [2,3,4]. In most species, there are many proteins that belong to the hsp70 family. Some of them are expressed under unstressed conditions. Hsp70proteins can be found in different cellular compartments (nuclear, cytosolic, mitochondrial, endoplasmic reticulum, etc.). Some of the hsp70 family proteinsare listed below: - In Escherichia coli and other bacteria, the main hsp70 protein is known as the dnaK protein. A second protein, hscA, has been recently discovered. dnaK is also found in the chloroplast genome of red algae. - In yeast, at least ten hsp70 proteins are known to exist: SSA1 to SSA4, SSB1, SSB2, SSC1, SSD1 (KAR2), SSE1 (MSI3) and SSE2. - In Drosophila, there are at least eight different hsp70 proteins: HSP70, HSP68, and HSC-1 to HSC-6. - In mammals, there are at least eight different proteins: HSPA1 to HSPA6, HSC70, and GRP78 (also known as the immunoglobulin heavy chain binding protein (BiP)). - In the sugar beet yellow virus (SBYV), a hsp70 homolog has been shown [5] to exist. - In archaebacteria, hsp70 proteins are also present [6].All proteins belonging to the hsp70 family bind ATP. A variety of functions has been postulated for hsp70 proteins. It now appears [7] that some hsp70proteins play an important role in the transport of proteins across membranes. They also seem to be involved in protein folding and in the assembly/disassembly of protein complexes [8]. Three signature patterns for the hsp70 family of proteins were derived; the first centered on a conserved pentapeptide found in the N-terminal section of these proteins; the two others on conserved regions located in the central part of the sequence.

Consensus pattern: [IV]-D-L-G-T-[ST]-x-[SC] -
Consensus pattern: [LIVMF]-[LIVMFY]-[DN]-[LIVMFS]-G-[GSH]-[GS]-[AST]-x(3)-[ST]-[LIVM]-[LIVMFC]-
Consensus pattern: [LIVMY]-x-[LIVMF]-x-G-G-x-[ST]-x-[LIVM]-P-x-[LIVM]-x-[DEQKRSTA]-

[1] Lindquist S., Craig E.A. Annu. Rev. Genet. 22:631-677(1988).
[2] Pelham H.R.B. Cell 46:959-961(1986).
[3] Pelham H.R.B. Nature 332:776-77(1988).
[4] Craig E.A. BioEssays 11:48-52(1989).
[5] Agranovsky A.A., Boyko V.P., Karasev A.V., Koonin E.V., Dolja V.V. J. Mol. Biol. 217:603-610(1991).
[6] Gupta R.S., Singh B. J. Bacteriol. 174:4594-4605(1992).
[7] Deshaies R.J., Koch B.D., Schekmam R. Trends Biochem. Sci. 13:384-388(1988).
[8] Craig E.A., Gross C.A. Trends Biochem. Sci. 16:135-140(1991).

283. Heat shock hsp90 proteins family signature
Prokaryotic and eukaryotic organisms respond to heat shock or other environmental stress by the induction of the synthesis of proteins collectively known as heat-shock proteins (hsp)

[1]. Amongst them is a family of proteins, with an average molecular weight of 90 Kd, known as the hsp90proteins. Proteins known to belong to this family are: - Escherichia coli and other bacteria heat shock protein c62.5 (gene htpG). - Vertebrate hsp 90-alpha (hsp 86) and hsp 90-beta (hsp 84). - Drosophila hsp 82 (hsp 83). - Trypanosoma cruzi hsp 85. - Plants Hsp82 or Hsp83. - Yeast and other fungi HSC82, and HSP82. - The endoplasmic reticulum protein 'endoplasmin' (also known as Erp99 in mouse, GRP94 in hamster, and hsp 108 in chicken).The exact function of hsp90 proteins is not yet known. In higher eukaryotes, hsp90 has been found associated with steroid hormone receptors, with tyrosine kinase oncogene products of several retroviruses, with eIF2alpha kinase, and with actin and tubulin. Hsp90 are probable chaperonins that possess ATPase activity [2,3].As a signature pattern for the hsp90 family of proteins, a highly conserved region found in the N-terminal part of these proteins was selected.

Consensus pattern: Y-x-[NQH]-K-[DE]-[IVA]-F-L-R-[ED] -

[1] Lindquist S., Craig E.A. Annu. Rev. Genet. 22:631-677(1988).
[2] Nadeau K., Das A., Walsh C.T. J. Biol. Chem. 268:1479-1487(1993).
[3] Jakob U., Buchner J. Trends Biochem. Sci. 19:205-211(1994).

284. Helix-turn-helix (HTH3)
This large family of DNA binding helix-turn helix proteins includes Cro Swiss:P03036 and CI Swiss:P03034.

285. Heme oxygenase signature
Heme oxygenase (EC 1.14.99.3) (HO) [1] is the microsomal enzyme that, in animals, carries out the oxidation of heme, it cleaves the heme ring at the alpha methene bridge to form biliverdin and carbon monoxide. Biliverdin is subsequently converted to bilirubin by biliverdin reductase. In mammals there are three isozymes of heme oxygenase: HO-1 to HO-3. The first two isozymes differ in their tissue expression and their inducibility: HO-1 is highly inducible by its substrate heme and by various non-heme substances, while HO-2 is non-inducible. It has been suggested [2] that HO-2 could be implicated in the production of carbon monoxide in the brain where it is said to act as a neurotransmitter.In the genome of the chloroplast of red algae as well as in cyanobacteria, there is a heme oxygenase (gene pbsA) that is the key enzyme in the synthesis of the chromophoric part of the photosynthetic antennae [3]. An heme oxygenase is also present in the bacteria Corynebacterium diphtheriae (gene hmuO), where it is involved in the acquisition of iron from the host heme [4].There is, in the central section of these enzymes, a well conserved region centered on a histidine residue which is proposed to play a key role in binding the substrate heme at the active center of the enzyme. This region was used as a signature pattern.
Consensus pattern: L-[IV]-A-H-[STACH]-Y-[STV]-[RT]-Y-[LIVM]-G [H binds the heme]

[1] Maines M.D. FASEB J. 2:2557-2568(1988).
[2] Barinaga M. Science 259:309-309(1993).
[3] Richaud C., Zabulon G. Proc. Natl. Acad. Sci. U.S.A. 94:11736-11741(1997).
[4] Schmitt M.P. J. Bacteriol. 179:838-845(1997).

286. Hepatitis core antigen.
The core antigen of hepatitis viruses possesses a carboxyl terminus rich in arginine. On this basis it was predicted that the core antigen would bind DNA [1]. There is some experimental evidence to support this [2].

[1] Pasek M, Goto T, Gilbert W, Zink B, Schaller H, Mckay P, Leadbetter G, Murray K; Nature 1979;282:575-579.

[2]
Gallina A, Bonelli F, Zentilin L, Rindi G, Muttini M, Milanesi G; J Virol 1989;63:4645-4652.

287. Histidine biosynthesis protein
Proteins involved in steps 4 and 6 of the histidine biosynthesis pathway are contained in this family. Histidine is formed by several complex and distinct biochemical reactions catalysed by eight enzymes. The enzymes in this Pfam entry are called His6 and His7 in eukaryotes and HisA and HisF in prokaryotes.

[1] Fani R, Tamburini E, Mori E, Lazcano A, Lio P, Barberio C, Casalone E, Cavalieri D, Perito B, Polsinelli M, Gene 1997;197:9-17.
[2] Fani R, Lio P, Chiarelli I, Bazzicalupo M, J Mol Evol 1994;38:489-495.

288. Histone deacetylase family
Histones can be reversibly acetylated on several lysine residues. Regulation of transcription is caused in part by this mechanism. Histone deacetylases catalyse the removal of the acetyl group. Histone deacetylases are related to other proteins [1].
Leipe DD, Landsman D, Nucleic Acids Res 1997;25:3693-3697.

289. Histidinol dehydrogenase signature
Histidinol dehydrogenase (EC 1.1.1.23) (HDH) catalyzes the terminal step in the biosynthesis of histidine in bacteria, fungi, and plants, the four-electron oxidation of L-histidinol to histidine.In bacteria HDH is a single chain polypeptide; in fungi it is the C-terminal domain of a multifunctional enzyme which catalyzes three different steps of histidine biosynthesis; and in plants it is expressed as nuclear encoded protein precursor which is exported to the chloroplast [1].As a signature pattern a highly conserved region located in the central part of HDH was selected. This region does not correspond to the part of the enzyme that, in most, but not all HDH sequences contains a cysteine residue which, in Salmonella typhimurium, has been said [2] to be important for the catalytic activity of the enzyme.
Consensus pattern: I-D-x(2)-A-G-P-[ST]-E-[LIVS]-[LIVMA](3)-[AC]-x(3)-A-x(4)-[LIVM]-[AV]-[SACL]-[DE]-[LIVMFC]-[LIVM]-[SA]-x(2)-E-H-

[1] Nagai A., Ward E., Beck J., Tada S., Chang J.-Y., Scheidegger A., Ryals J. Proc. Natl. Acad. Sci. U.S.A. 88:4133-4137(1991).
[2] Grubmeyer C.T., Gray W.R. Biochemistry 25:4778-4784(1986).

290. Homoserine dehydrogenase signature
Homoserine dehydrogenase (EC 1.1.1.3) (HDh) [1,2] catalyzes NAD-dependent reduction of aspartate beta-semialdehyde into homoserine. This reaction is the third step in a pathway leading from aspartate to homoserine. The latter participates in the biosynthesis of threonine and then isoleucine as well as in that of methionine. HDh is found either as a single chain protein as in some bacteria and yeast, or as a bifunctional enzyme consisting of an N-terminal aspartokinase domain and a C-terminal HDh domain as in bacteria such as Escherichia coli and in plants. As a signature pattern, the best conserved region of Hdh has been selected. This is a segment of 23 to 24 residues located in the central section and that contains two conserved aspartate residues.
Consensus pattern: A-x(3)-G-[LIVMFY]-[STAG]-x(2,3)-[DNS]-P-x(2)-D-[LIVM]-x-G- x-D-x(3)-K-

[1] Thomas D., Barbey R., Surdin-Kerjan Y. FEBS Lett. 323:289-293(1993).
[2] Cami B., Clepet C., Patte J.-C. Biochimie 75:487-495(1993).

291. haloacid dehalogenase-like hydrolase
This family is structurally different from the alpha/ beta hydrolase family (abhydrolase). This family includes L-2-haloacid dehalogenase, epoxide hydrolases and phosphatases. The structure of the family consists of two domains. One is an inserted four helix bundle, which is the least well conserved region of the alignment, between residues 16 and 96 of Swiss:P24069. The rest of the fold is composed of the core alpha/beta domain.

[1] Hisano T, Hata Y, Fujii T, Liu JQ, Kurihara T, Esaki N, Soda K, J Biol Chem 1996; 271:20322-20330.

292. DEAD and DEAH box families ATP-dependent helicases signatures (helicase_C)
A number of eukaryotic and prokaryotic proteins have been characterized [1,2,3] on the basis of their structural similarity. They all seem to be involved in ATP-dependent, nucleicacid unwinding. Proteins currently known to belong to this family are: - Initiation factor eIF-4A. Found in eukaryotes, this protein is a subunit of a high molecular weight

complex involved in 5'cap recognition and the binding of mRNA to ribosomes. It is an ATP-dependent RNA-helicase. - PRP5 and PRP28. These yeast proteins are involved in various ATP-requiring steps of the pre-mRNA splicing process. - P110, a mouse protein expressed specifically during spermatogenesis. - An3, a Xenopus putative RNA helicase, closely related to P110.- SPP81/DED1 and DBP1, two yeast proteins probably involved in pre-mRNA splicing and related to P110. - Caenorhabditis elegans helicase glh-1. - MSS116, a yeast protein required for mitochondrial splicing. - SPB4, a yeast protein involved in the maturation of 25S ribosomal RNA. - p68, a human nuclear antigen. p68 has ATPase and DNA-helicase activities in vitro. It is involved in cell growth and division. - Rm62 (p62), a Drosophila putative RNA helicase related to p68. - DBP2, a yeast protein related to p68. - DHH1, a yeast protein. - DRS1, a yeast protein involved in ribosome assembly. - MAK5, a yeast protein involved in maintenance of dsRNA killer plasmid. - ROK1, a yeast protein. - ste 13, a fission yeast protein. - Vasa, a Drosophila protein important for oocyte formation and specification of embryonic posterior structures. - Me31B, a Drosophila maternally expressed protein of unknown function. - dbpA, an Escherichia coli putative RNA helicase. - deaD, an Escherichia coli putative RNA helicase which can suppress a mutation in the rpsB gene for ribosomal protein S2. - rhlB, an Escherichia coli putative RNA helicase. - rhlE, an Escherichia coli putative RNA helicase. - srmB, an Escherichia coli protein that shows RNA-dependent ATPase activity. It probably interacts with 23 S ribosomal RNA. - Caenorhabditis elegans hypothetical proteins T26G10.1, ZK512.2 and ZK686.2. - Yeast hypothetical protein YHR065c. - Yeast hypothetical protein YHR169w. - Fission yeast hypothetical protein SpAC31A2.07c. - Bacillus subtilis hypothetical protein yxiN. All these proteins share a number of conserved sequence motifs. Some of them are specific to this family while others are shared by other ATP-binding proteins or by proteins belonging to the helicases 'superfamily' [4,E1]. One of these motifs, called the 'D-E-A-D-box', represents a special version of the B motif of ATP-binding proteins. Some other proteins belong to a subfamily which have His instead of the second Asp and are thus said to be 'D-E-A-H-box' proteins [3,5,6,E1]. Proteins currently known to belong to this subfamily are: - PRP2, PRP16, PRP22 and PRP43. These yeast proteins are all involved in various ATP-requiring steps of the pre-mRNA splicing process. - Fission yeast prh1, which my be involved in pre-mRNA splicing. - Male-less (mle), a Drosophila protein required in males, for dosage compensation of X chromosome linked genes. - RAD3 from yeast. RAD3 is a DNA helicase involved in excision repair of DNA damaged by UV light, bulky adducts or cross-linking agents. Fission yeast rad15 (rhp3) and mammalian DNA excision repair protein XPD (ERCC-2) are the homologs of RAD3. - Yeast CHL1 (or CTF1), which is important for chromosome transmission and normal cell cycle progression in G(2)/M. - Yeast TPS1. - Yeast hypothetical protein YKL078w. - Caenorhabditis elegans hypothetical proteins C06E1.10 and K03H1.2. - Poxviruses' early transcription factor 70 Kd subunit which acts with RNA polymerase to initiate transcription from early gene promoters. - I8, a putative vaccinia virus helicase. - hrpA, an Escherichia coli putative RNA helicase. Signature patterns were developed for both subfamilies.
Consensus pattern: [LIVMF](2)-D-E-A-D-[RKEN]-x-[LIVMFYGSTN]-
Consensus pattern: [GSAH]-x-[LIVMF](3)-D-E-[ALIV]-H-[NECR] -
Note: proteins belonging to this family also contain a copy of the ATP/GTP- binding motif 'A' (P-loop) (see the relevant entry <PDOC00017

[1] Schmid S.R., Linder P. Mol. Microbiol. 6:283-292(1992).
[2] Linder P., Lasko P., Ashburner M., Leroy P., Nielsen P.J., Nishi K., Schnier J., Slonimski P.P. Nature 337: 121-122(1989).
[3] Wassarman D.A., Steitz J.A. Nature 349:463-464(1991).
[4] Hodgman T.C. Nature 333:22-23(1988) and Nature 333:578-578(1988) (Errata).
[5] Harosh I., Deschavanne P. Nucleic Acids Res. 19:6331-6331(1991).
[6] Koonin E.V., Senkevich T.G. J. Gen. Virol. 73:989-993(1992).

293. Heme-binding domain in cytochrome b5 and oxidoreductases (heme_1)
Cytochrome b5 is a membrane-bound hemo protein which acts as an electron carrier for several membrane-bound oxygenases [1]. There are two homologous forms of b5, one found in microsomes and one found in the outer membrane of mitochondria. Two conserved histidine residues serve as axial ligands for the heme group. The structure of a number of oxidoreductases consists of the juxtaposition of a heme-binding domain homologous to that of b5 and either a flavodehydrogenase or a molybdopterin domain. These enzymes are:

- Lactate dehydrogenase (EC 1.1.2.3) [2], an enzyme that consists of a flavodehydrogenase domain and a heme-binding domain called cytochrome b2.
- Nitrate reductase (EC 1.6.6.1), a key enzyme involved in the first step of nitrate assimilation in plants, fungi and bacteria [3,4]. Consists of a molybdopterin domain (see <PDOC00484>), a heme-binding domain called cytochrome b557, as well as a cytochrome reductase domain.
- Sulfite oxidase (EC 1.8.3.1) [5], which catalyzes the terminal reaction in the oxidative degradation of sulfur-containing amino acids. Also consists of a molybdopterin domain and a heme-binding domain.

This family of proteins also includes:

- TU-36B, a Drosophila muscle protein of unknown function [6].
- Fission yeast hypothetical protein SpACIF12.10c.
- Yeast hypothetical protein YMR073c.
- Yeast hypothetical protein YMR272c.

A segment was used which includes the first of the two histidine heme ligands, as a signature pattern for the heme-binding domain of cytochrome b5 family.
Consensus pattern: [FY]-[LIVMK]-x(2)-H-P-[GA]-G [H is a heme axial ligand]-

[1] Ozols J. Biochim. Biophys. Acta 997:121-130(1989).
[2] Guiard B. EMBO J. 4:3265-3272(1985).
[3] Calza R., Huttner E., Vincentz M., Rouze P., Galangau F., Vaucheret H., Cherel I., Meyer C., Kronenberger J., Caboche M. Mol. Gen. Genet. 209:552-562(1987).
[4] Crawford N.M., Smith M., Bellissimo D., Davis R.W. Proc. Natl. Acad. Sci. U.S.A. 85:5006-5010(1988).
[5] Guiard B., Lederer F. Eur. J. Biochem. 100:441-453(1979).
[6] Levin R.J., Boychuk P.L., Croniger C.M., Kazzaz J.A., Rozek C.E. Nucleic Acids Res. 17:6349-6367(1989).

294. Hexapeptide-repeat containing-transferases signature
On the basis of sequence similarity, a number of transferases have been proposed [1,2,3,4] to belong to a single family. These proteins are: - Serine acetyltransferase (EC 2.3.1.30) (SAT) (gene cysE), an enzyme involved in cysteine biosynthesis. - Azotobacter chroococcum nitrogen fixation protein nifP. NifP is most probably a SAT involved in the optimization of nitrogenase activity. - Escherichia coli thiogalactoside acetyltransferase (EC 2.3.1.18) (gene lacA), an enzyme involved in the biosynthesis of lactose. - UDP-N-acetylglucosamine acyltransferase (EC 2.3.1.129) (gene 1pxA), an enzyme involved in the biosynthesis of lipid A, a phosphorylated glycolipid that anchors the lipopolysaccharide to the outer membrane of the cell. - UDP-3-0-[3-hydroxymyristoyl] glucosamine N-acyltransferase (EC 2.3.1.-) (gene lpxD or firA), which is also involved in the biosynthesis of lipid A. - Chloramphenicol acetyltransferase (CAT) (EC 2.3.1.28) from Agrobacterium tumefaciens, Bacillus sphaericus, Escherichia coli plasmid IncFII NR79, Pseudomonas aeruginosa, Staphylococcus aureus plasmid pIP630. These CAT are not evolutionary related to the main family of CAT (see <PDOC00093>). - Rhizobium nodulation protein nodL. NodL is an acetyltransferase involved in the O-acetylation of Nod factors. - Bacterial maltose O-acetyltransferase (EC 2.3.1.79). - Bacterial tetrahydrodipicolinate N-succinyltransferase (EC 2.3.1.117) (gene dapD) which catalyzes the fourth step in the biosynthesis of diaminopimelate and lysine from aspartate semialdehyde. - Bacterial N-acetylglucosamine-1-phosphate uridyltransferase (EC 2.7.7.23) (gene glmU or gcaD or tms), an enzyme involved in peptidoglycan and lipopolysaccharide biosynthesis. - Staphylococcus aureus protein capG which is involved in biosynthesis of type 1 capsular polysaccharide. - Yeast hypothetical protein YJL218w, which is highly similar to Escherichia coli lacA. - Fission yeast hypothetical protein SpAC18B11.09c. - Methanococcus jannaschii hypothetical protein MJ1064.These proteins have been shown [3,4] to contain a repeat structure composed of tandem repeats of a [LIV]-G-x(4) hexapeptide which, in the tertiary structure of lpxA [5], has been shown to form a left-handed parallel beta helix. Our signature pattern is based on a fourfold repeat of this hexapeptide.
Consensus pattern: [LIV]-[GAED]-x(2)-[STAV]-x-[LIV]-x(3)-[LIVAC]-x-[LIV]-[GAED]-x(2)-[STAVR]-x-[LIV]-[GAED]-x(2)-[STAV]-x-[LIV]- x(3)-[LIV]-

[1] Downie J.A. Mol. Microbiol. 3:1649-1651(1989).
[2] Parent R., Roy P.H. J. Bacteriol. 174:2891-2897(1992).
[3] Vaara M. FEMS Microbiol. Lett. 97:249-254(1992).
[4] Vuorio R., Haerkonen T., Tolvanen M., Vaara M. FEBS Lett. 337:289-292(1994).
[5] Raetz C.R.H., Roderick S.L. Science 270:997-1000(1995).

295. Hexokinases signature. Hexokinase (EC 2.7.1.1 ) [1,2] is an important glycolytic enzyme that catalyzes the phosphorylation of keto- and aldohexoses (e.g. glucose, mannose and fructose) using MgATP as the phosphoryl donor. In vertebrates there are four major isoenzymes, commonly referred as types I,II, III and IV. Type IV hexokinase, which is often incorrectly designated glucokinase [3], is only expressed in liver and pancreatic beta-cells and plays an important role in modulating insulin secretion; it is a protein of a molecular mass of about 50 Kd. Hexokinases of types I to III, which have low Km values for glucose, have a molecular mass of about 100 Kd. Structurally they

consist of a very small N-terminal hydrophobic membrane-binding domain followed by two highly similar domains of 450 residues. The first domain has lost its catalytic activity and has evolved into a regulatory domain. In yeast there are three different isozymes: hexokinase PI (gene HXK1), PII(gene HXKB), and glucokinase (gene GLK1). All three proteins have a molecular mass of about 50 Kd. All these enzymes contain one (or two in the case of types I to III isozymes)strongly conserved region which has been shown [4] to be involved in substrate binding. A pattern from that region has been derived
Consensus pattern: [LIVM]-G-F-[TN]-F-S-[FY]-P-x(5)-[LIVM]-[DNST]-x(3)-[LIVM]-x(2)-W-T-K-x-[LF]-

[1] Middleton R.J. Biochem. Soc. Trans. 18:180-183(1990).
[2] Griffin L.D., Gelb B.D., Wheeler D.A., Davison D., Adams V., McCabe E.R. Genomics 11:1014-1024(1991).
[3] Cornish-Bowden A., Luz Cardenas M. Trends Biochem. Sci. 16:281-282(1991).
[4] Schirch D.M., Wilson J.E. Arch. Biochem. Biophys. 254:385-396(1987).

296. Histone H2A signature (his1)
Histone H2A is one of the four histones, along with H2B, H3 and H4, which forms the eukaryotic nucleosome core. Using alignments of histone H2Asequences [1,2,E1] as a signature pattern, a conserved region in the N-terminal part of H2A. This region is conserved both in classical S-phase regulated H2A's and in variant histone H2A's which are synthesized throughout the cell cycle.
Consensus pattern: [AC]-G-L-x-F-P-V-

[1] Wells D.E., Brown D. Nucleic Acids Res. 19:2173-2188(1991).
[2] Thatcher T.H., Gorovsky M.A. Nucleic Acids Res. 22:174-179(1994).

Histone H4 signature (his2)
Histone H4 is one of the four histones, along with H2A, H2B and H3, which forms the eukaryotic nucleosome core. Along with H3, it plays a central role in nucleosome formation. The sequence of histone H4 has remained almost invariant in more then 2 billion years of evolution [1,E1]. The region used as a signature pattern is a pentapeptide found in positions 14 to 18 of all H4sequences. It contains a lysine residue which is often acetylated [2] and a histidine residue which is implicated in DNA-binding [3].
Consensus pattern: G-A-K-R-H-

[1] Thatcher T.H., Gorovsky M.A. Nucleic Acids Res. 22:174-179(1994).
[2] Doenecke D., Gallwitz D. Mol. Cell. Biochem. 44:113-128(1982).
[3] Ebralidse K.K., Grachev S.A., Mirzabekov A.D. Nature 331:365-367(1988).

Histone H3 signatures (his3)
Histone H3 is one of the four histones, along with H2A, H2B and H4, which forms the eukaryotic nucleosome core. It is a highly conserved protein of 135 amino acid residues [1,2,E1].The following proteins have been found to contain a C-terminal H3-like domain: - Mammalian centromeric protein CENP-A [3]. Could act as a core histone necessary for the assembly of centromeres. - Yeast chromatin-associated protein CSE4 [4]. - Caenorhabditis elegans chromosome III encodes two highly related proteins (F54C8.2 and F58A4.3) whose C-terminal section is evolutionary related to the last 100 residues of H3. The function of these proteins is not yet known. Two signature patterns were developed, The first one corresponds to a perfectly conserved heptapeptide in the N-terminal part of H3. The second one is derived from a conserved region in the central section of H3.
Consensus pattern: K-A-P-R-K-Q-L-
Consensus pattern: P-F-x-[RA]-L-[VA]-[KRQ]-[DEG]-[IV]-

[1] Wells D.E., Brown D. Nucleic Acids Res. 19:2173-2188(1991).
[2] Thatcher T.H., Gorovsky M.A. Nucleic Acids Res. 22:174-179(1994).
[3] Sullivan K.F., Hechenberger M., Masri K. J. Cell Biol. 127:581-592(1994).
[4] Stoler S., Keith K.C., Curnick K.E., Fitzgerald-Hayes M. Genes Dev. 9:573-586(1995).

Histone H2B signature (his4)
Histone H2B is one of the four histones, along with H2A, H3 and H4, which forms the eukaryotic nucleosome core. Using alignments of histone H2Bsequences [1,2,E1], a conserved region was selected in the C-terminal part ofH2B.
Consensus pattern: [KR]-E-[LIVM]-[EQ]-T-x(2)-[KR]-x-[LIVM](2)-x-[PAG]-[DE]-L- x-[KR]-H-A-[LIVM]-[STA]-E-G-

[1] Wells D.E., Brown D. Nucleic Acids Res. 19:2173-2188(1991).
[2] Thatcher T.H., Gorovsky M.A. Nucleic Acids Res. 22:174-179(1994).

297. Homeobox' domain signature and profile (home1)
The 'homeobox' is a protein domain of 60 amino acids [1 to 5.E1] first identified in a number of Drosophila homeotic and segmentation proteins. It has since been found to be extremely well conserved in many other animals, including vertebrates. This domain binds DNA through a helix-turn-helix type of structure. Some of the proteins which contain a homeobox domain play an important role in development. Most of these proteins are known to be sequence specific DNA-binding transcription factors. The homeobox domain has also been found to be very similar to a region of the yeast mating type proteins. These are sequence-specific DNA-binding proteins that act as master switches in yeast differentiation by controlling gene expression in a cell type-specific fashion. A schematic representation of the homeobox domain is shown below. The helix-turn-helix region is shown by the symbols 'H' (for helix), and 't' (for turn). xxxxxxxxxxxxxxxxxxxxxxxxxxxxxxHHHHHHHHtttHHHHHHHHHxxxxxxxxxx|||||| 1 10 20 30 40 50 60 The pattern to detect homeobox sequences that was developed is 24 residues long and spans positions 34 to 57 of the homeobox domain.
Consensus pattern: [LIVMFYG]-[ASLVR]-x(2)-[LIVMSTACN]-x-[LIVM]-x(4)-[LIV]-[RKNQESTAIY]-[LIVFSTNKH]-W-[FYVC]-x-[NDQTAH]-x(5)- [RKNAIMW] -

[1] Gehring W.J. (In) Guidebook to the homebox genes, Duboule D., Ed., pp1-10, Oxford University Press, Oxford, (1994).
[2] Buerglin T.R. (In) Guidebook to the homebox genes, Duboule D., Ed., pp25-72, Oxford University Press, Oxford, (1994).
[3] Gehring W.J. Trends Biochem. Sci. 17:277-280(1992).
[4] Gehring W.J., Hiromi Y. Annu. Rev. Genet. 20:147-173(1986).
[5] Schofield P.N. Trends Neurosci. 10:3-6(1987).

'Homeobox' antennapedia-type protein signature (home2)
The homeotic Hox proteins are sequence-specific transcription factors. They are part of a developmental regulatory system that provides cells with specific positional identities on the anterior-posterior (A-P) axis [1]. The box proteins contain a 'homeobox' domain. In Drosophila and other insects, there are eight different Hox genes that are encoded in two gene complexes, ANT-C and BX-C. In vertebrates there are 38 genes organized in four complexes. In six of the eight Drosophila Hox genes the homeobox domain is highly similar and a conserved hexapeptide is found five to sixteen amino acids upstream of the homeobox domain. The six Drosophila proteins that belong to this group are antennapedia (Antp), abdominal-A (abd-A), deformed (Dfd), proboscipedia (pb), sex combs reduced (scr) and ultrabithorax (ubx) and are collectively known as the 'antennapedia' subfamily. In vertebrates the corresponding Hox genes are known [2] as Hox-A2, A3, A4,A5, A6, A7, Hox-B1, B2, B3, B4, B5, B6, B7, B8, Hox-C4, C5, C6, C8, Hox-D1,D3, D4 and D8.Caenorhabditis elegans lin-39 and mab-5 are also members of the 'antennapedia' subfamily. As a signature pattern for this subfamily of homeobox proteins, the conserved hexapeptide was used.
Consensus pattern: [LIVMFE]-[FY]-P-W-M-[KRQTA]-

[1] McGinnis W., Krumlauf R. Cell 68:283-302(1992).
[2] Scott M.P. Cell 71:551-553(1992).

'Homeobox' engrailed-type protein signature (home3)
Most proteins which contain a 'homeobox' domain can be classified [1,2], on the basis of their sequence characteristics, in three subfamilies: engrailed, antennapedia and paired. Proteins currently known to belong to the engrailed subfamily are: - Drosophila segmentation polarity protein engrailed (en) which specifies the body segmentation pattern and is required for the development of the central nervous system. - Drosophila invected protein (inv). - Silk moth proteins engrailed and invected, which may be involved in the compartmentalization of the silk gland. - Honeybee E30 and E60. - Grasshopper (Schistocerca americana) G-En. - Mammalian and birds En-1 and En-2. - Zebrafish Eng-1, - 2 and -3. - Sea urchin (Tripneusteas gratilla) SU-HB-en. - Leech (Helobdella triserialis) Ht-En. - Caenorhabditis elegans ceh-16.Engrailed homeobox proteins are characterized by the presence of a conserved region of some 20 amino-acid residues located at the C-terminal of the 'homeobox' domain. As a signature pattern for this subfamily of proteins, a stretch of eight perfectly conserved residues in this region was used.
Consensus pattern: L-M-A-[EQ]-G-L-Y-N-

[1] Scott M.P., Tamkun J.W., Hartzell G.W. III Biochim. Biophys. Acta 989:25-48(1989).
[2] Gehring W.J. Science 236:1245-1252(1987).

298. Isocitrate lyase signature (ICL)
Isocitrate lyase (EC 4.1.3.1) [1,2] is an enzyme that catalyzes the conversion of isocitrate to succinate and glyoxylate. This is the first step in the glyoxylate bypass, an alternative to the tricarboxylic acid cycle in bacteria, fungi and plants. A cysteine, a histidine and a glutamate or aspartate have been found to be important for the enzyme's catalytic activity. Only one cysteine residue is conserved between the sequences of the fungal, plant and bacterial enzymes; it is located in the middle of a conserved hexapeptide that can be used as a signature pattern for this type of enzyme.
Consensus pattern: K-[KR]-C-G-H-[LMQ] [C is a putative active site residue]-

[1] Beeching J.R. Protein Seq. Data Anal. 2:463-466(1989).
[2] Atomi H., Ueda M., Hikida M., Hishida T., Teranishi Y., Tanaka A. J. Biochem. 107:262-266(1990).

299. Initiation factor 2 subunit
This family includes initiation factor 2B alpha, beta and delta subunits from eukaryotes, related proteins from archaebacteria and IF-2 from prokaryotes. Initiation factor 2 binds to Met-tRNA, GTP and the small ribosomal subunit.

[1] Kyrpides NC, Woese CR, Proc Natl Acad Sci U S A 1998;95:3726-3730.

300. Initiation factor 3 signature
Initiation factor 3 (IF-3) (gene infC) [1] is one of the three factors required for the initiation of protein biosynthesis in bacteria. IF-3 is thought to function as a fidelity factor during the assembly of the ternary initiation complex which consist of the 30S ribosomal subunit, the initiator tRNA and the messenger RNA. IF-3 binds to the 30S ribosomal subunit; it is a basic protein of 141 to 212 residues. The chloroplast initiation factor IF-3(chl) is a protein that enhances the poly(A,U,G)-dependent binding of the initiator tRNA to chloroplast ribosoma130s subunits. In its mature form it is a protein of about 400 residues whose central section is evolutionary related to the sequence of bacterial IF-3 [2].As a signature pattern a highly conserved region was selected located in the central section of bacterial IF-3 and of IF-3(ch1).
Consensus pattern: [KR]-[LIVM](2)-[DN]-[FY]-[GSN]-[KR]-[LIVMFYS]-x-[FY]-[DEQTH]-x(2)-[KRQ]-

[1] Liveris D., Schwartz J.J., Geertman R., Schwartz I. FEMS Microbiol. Lett. 112:211-216(1993).
[2] Lin Q., Ma L., Burkhart W., Spremulli L.L. J. Biol. Chem. 269:9436-9444(1994).

301. Imidazoleglycerol-phosphate dehydratase signatures (IGPD) Imidazoleglycerol-phosphate dehydratase (EC 4.2.1.19) is the enzyme that catalyzes the seventh step in the biosynthesis of histidine in bacteria, fungi and plants. In most organisms it is a monofunctional protein of about 22 to29 Kd. In some bacteria such as Escherichia coli it is the C-terminal domain of a bifunctional protein that include a histidinol-phosphatase domain [1]. Two signature patterns were developed that each include two consecutive histidine residues.
Consensus pattern: [LIVMY]-[DE]-x-H-H-x(2)-E-x(2)-[GCA]-[LIVM]-[STAC]-[LIVM]-
Consensus pattern: G-x-[DN]-x-H-H-x(2)-E-[STAGC]-x-[FY]-K -

[1] Carlomagno M.S., Chiariotti L., Alifano P., Nappo A.G., Bruni C.B. J. Mol. Biol. 203:585-606(1988).

302. Indole-3-glycerol phosphate synthase signature (IGPS)
Indole-3-glycerol phosphate synthase (EC 4.1.1.48) (IGPS) catalyzes the fourth step in the biosynthesis of tryptophan: the ring closure of 1-(2-carboxy-phenylamino)-1-deoxyribulose into indol-3-glycerol-phosphate.In some bacteria, IGPS is a single chain enzyme. In others - such as Escherichia coli - it is the N-terminal domain of a bifunctional enzyme that also catalyzes N-(5'-phosphoribosyl)anthranilate isomerase (PRAI) activity, the third step of tryptophan biosynthesis. In fungi, IGPS is the central domain of a trifunctional enzyme that also contains a PRAI C-terminal domain and a glutamine amidotransferase N-terminal domain. The N-terminal section of IGPS contains a highly conserved region which X-ray crystallography studies [1] have shown to be part of the active site cavity. This region was used as a signature pattern for IGPS.
Consensus pattern: [LIVMFY]-[LIVMC]-x-E-[LIVMFYC]-K-[KRSP]-[STAK]-S-P-[ST]-x(3)-[LIVMFYST]-

[1] Wilmanns M., Priestle J.P., Niermann T., Jansonius J.N. J. Mol. Biol. 223:477-507(1992).

303. (IL2) Interleukin 2. 31 members

304. (ILVD EDD) Dihydroxy-acid and 6-phosphogluconate dehydratases. Two dehydratases have been shown [1] to be evolutionary related: - Dihydroxy-acid dehydratase (EC 4.2.1.9) (gene ilvD or ILV3) which catalyzes the fourth step in the biosynthesis of isoleucine and valine, the dehydratation of 2,3-dihydroxy-isovaleic acid into alphaketoisovaleric acid. - 6-phosphogluconate dehydratase (EC 4.2.1.12) (gene edd) which catalyzes the first step in the Entner-Doudoroff pathway, the dehydratation of 6-phospho-D-gluconate into 6-phospho-2-dehydro-3-deoxy-D-gluconate. - Escherichia coli hypothetical protein yjhG. Both enzymes are proteins of about 600 amino acid residues. Two highly conserved regions have been developed as signature patterns. The first pattern is located in the N-terminal part and contains a cysteine that could be involved in the binding of a 2Fe-2S iron-sulfur cluster [2]. The second pattern is located in the C-terminal half.
Consensus pattern: C-D-K-x(2)-P-[GA]-x(3)-[GA] [The C could be a 2Fe-2S ligand]
Consensus pattern: [SA]-L-[LIVM]-T-D-[GA]-R-[LIVMF]-S-[GA]-[GAV]-[ST]-

[1] Egan S.E., Fliege R., Tong S., Shibata A., Wolf R.E. Jr., Conway T. J. Bacteriol. 174:4638-4646(1992).
[2] Velasco J.A., Cansado J., Pena M.C., Kawakami T., Laborda J., Notario V. Gene 137:179-185(1993).

305. IMP dehydrogenase / GMP reductase signature
IMP dehydrogenase (EC 1.1.1.205) (IMPDH) catalyzes the rate-limiting reaction of de novo GTP biosynthesis, the NAD-dependent reduction of IMP into XMP [1].Inhibition of IMP dehydrogenase activity results in the cessation of DNA synthesis. As IMP dehydrogenase is associated with cell proliferation, it is a possible target for cancer chemotherapy.
Mammalian and bacterial IMPDHs are tetramers of identical chains. There are two IMP dehydrogenase isozymes in humans [2].GMP reductase (EC 1.6.6.8) catalyzes the irreversible and NADPH-dependent reductive deamination of GMP into IMP [3]. It converts nucleobase, nucleoside and nucleotide derivatives of G to A nucleotides, and maintains intracellular balance of A and G nucleotides. IMP dehydrogenase and GMP reductase share many regions of sequence similarity. One of these regions is centered on a cysteine residue thought [3] to be involved in binding IMP. This region was used as a signature pattern.
Consensus pattern: [LIVM]-[RK]-[LIVM]-G-[LIVM]-G-x-G-S-[LIVM]-C-x-T [C is the putative IMP-binding residue]-

[1] Collart F.R., Huberman E. J. Biol. Chem. 263:15769-15772(1988).
[2] Natsumeda Y., Ohno S., Kawasaki H., Konno Y., Weber G., Suzuki K. J. Biol. Chem. 265:5292-5295(1990).
[3] Andrews S.C., Guest J.R. Biochem. J. 255:35-43(1988).

306. (IPPc) Inositol polyphosphate phosphatase family, catalytic domain

[1] York JD, Ponder JW, Chen ZW, Mathews FS, Majerus PW; Biochemistry 1994;33:13164-13171.
[2] Jefferson AB, Auethavekiat V, Pot DA, Williams LT, Majerus PW; J Biol Chem 1997;272:5983-5988.
[3] Zhang X, Jefferson AB, Auethavekiat V, Majerus PW; Proc Natl Acad Sci U S A 1995;92:4853-4856.
[4] York JD, Majerus PW. Proc Natl Acad Sci U S A 1990;87:9548-9552.
[5] Neuwald AF, York JD, Majerus PW; FEBS Lett 1991;294:16-18.

307. IQ calmodulin-binding motif

[1] Xie X, Harrison DH, Schlichting I, Sweet RM, Kalabokis VN, Szent-Gyorgyi AG, Cohen C; Nature 1994;368: 306-312.
[2] Rhoads AR, Friedberg F; FASEB J 1997; 11:331-340.

308. Inosine-uridine preferring nucleoside hydrolasefamily signature (IU nuc hydro)
Inosine-uridine preferring nucleoside hydrolase (EC 3.2.2.1) (IU-nucleosidehydrolase or IUNH) is an enzyme first identified in protozoan [1] that catalyzes the hydrolysis of all of the commonly occuring purine and pyrimidine nucleosides into ribose and the associated base, but has a preference for inosine and uridine as substrates. This enzyme is important for these parasitic organisms, which are deficient in de novo synthsis of purines, to salvage the host purine nucleosides. IUNH from Crithidia fasciculata has been sequenced and characterized, it is an homotetrameric enzyme of subunits of 34 Kd. An histidine has been shown to be important for the catalytic mechanism, it acts a proton donor to activate the hypoxanthine leaving group. IUNH is evolutionary related to a number of uncharacterized proteins from various biological sources, notably: - Escherichia coli hypothetical protein yaaF. - Escherichia coli hypothetical protein ybeK. - Escherichia coli hypothetical protein yeiK. - Fission yeast hypothetical protein

SpAC17G8.02. - Yeast hypothetical protein YDR400w. - An hypothetical protein from the archaebacteria Desulfurolobus ambivalens. As a signature pattern for these proteins, a highly conserved region was selected located in the N-terminal extremity. This region contains four conserved aspartates that have been shown [2] to be located in the active site cavity.
Consensus pattern: D-x-D-[PT]-[GA]-x-D-D-[TAV]-[VI]-A -

[1] Gopaul D.N., Meyer S.L., Degano M., Sacchettini J.C., Schramm V.L. Biochemistry 35:5963-5970(1996).
[2] Degano M., Gopaul D.N., Scapin G., Schramm V.L., Sacchettini J.C. Biochemistry 35:5971-5981 (1996).

309. (Insulinase)
Insulinase family, zinc-binding region signature
(aka Peptidase_M16)
A number of proteases dependent on divalent cations for their activity have been shown [1,2] to belong to one family, on the basis of sequence similarity. These enzymes are listed below.

- Insulinase (EC 3.4.24.56) (also known as insulysin or insulin-degrading enzyme or IDE), a cytoplasmic enzyme which seems to be involved in the cellular processing of insulin, glucagon and other small polypeptides.
- Escherichia coli protease III (EC 3.4.24.55) (pitrilysin) (gene ptr), a periplasmic enzyme that degrades small peptides.
- Mitochondrial processing peptidase (EC 3.4.24.64) (MPP). This enzyme removes the transit peptide from the precursor form of proteins imported from the cytoplasm across the mitochondrial inner membrane. It is composed of two nonidentical homologous subunits termed alpha and beta. The beta subunit seems to be catalytically active while the alpha subunit has probably lost its activity.
- Nardilysin (EC 3.4.24.61) (N-arginine dibasic convertase or NRD convertase) this mammalian enzyme cleaves peptide substrates on the N-terminus of Arg residues in dibasic stretches.
- Klebsiella pneumoniae protein pqqF. This protein is required for the biosynthesis of the coenzyme pyrroloquinoline-quinone (PQQ). It is thought to be protease that cleaves peptide bonds in a small peptide (gene pqqA) thus providing the glutamate and tyrosine residues necessary for the synthesis of PQQ.
- Yeast protein AXL1, which is involved in axial budding [3].
- Eimeria bovis sporozoite developmental protein.
- Escherichia coli hypothetical protein yddC and HI1368, the corresponding Haemophilus influenzae protein.
- Bacillus subtilis hypothetical protein ymxG.
- Caenorhabditis elegans hypothetical proteins C28F5.4 and F56D2.1.

It should be noted that in addition to the above enzymes, this family also includes the core proteins I and II of the mitochondrial bc1 complex (also called cytochrome c reductase or complex III), but the situation as to the activity or lack of activity of these subunits is quite complex:

- In mammals and yeast, core proteins I and II lack enzymatic activity.
- In Neurospora crassa and in potato core protein I is equivalent to the beta subunit of MPP.
- In Euglena gracilis, core protein I seems to be active, while subunit II is inactive.

These proteins do not share many regions of sequence similarity; the most noticeable is in the N-terminal section. This region includes a conserved histidine followed, two residues later by a glutamate and another histidine. In pitrilysin, it has been shown [4] that this H-x-x-E-H motif is involved in enzyme activity; the two histidines bind zinc and the glutamate is necessary for catalytic activity. Non active members of this family have lost from one to three of these active site residues. We developed a signature pattern that detect active members of this family as well as some inactive members.
Consensus pattern G-x(8,9)-G-x-[STA]-H-[LIVMFY]-[LIVMC]-[DERN]-[HRKL]-[LMFAT]-x-[LFSTH]-x-[GSTAN]-[GST] [The two H are zinc ligands] [E is the active site residue] Sequences known to belong to this class detected by the pattern ALL active members as well as all MPP alpha subunits and core II subunits. Does not detect inactive core I subunits.
Note: these proteins belong to family M16 in the classification of peptidases [5].

[1] Rawlings N.D., BarrettA.J. Biochem. J. 275:389-391(1991).

[2] Braun H.-P., Schmitz U.K. Trends Biochem. Sci. 20:171-175(1995).

[3] Becker A.B., Roth R.A. Proc. Natl. Acad. Sci. U.S.A. 89:3835-3839(1992).

[4] Fujita A., Oka C., Arikawa Y., Katagai T., Tonouchi A., Kuhara S., Misumi Y. Nature 372:567-570(1994)

[5] Rawlings N.D., Barrett A.J. Meth. Enzymol. 248:183-228(1995).

310. Involucrin repeat Eckert RL, Yaffe MB, Crish JF, Murthy S, Rorke EA, Welter JF, J Invest Dermatol 1993;100: 613-617.

311. Isochorismatase family. This family are hydrolase enzymes. Romao MJ, Turk D, Gomis-Ruth FX, Huber R, Schumacher G, Mollering H, Russmann L, J Mol Biol 1992;226:1111-1130

312. Inositol monophosphatase family signatures (inositol_P)
It has been shown [1] that several proteins share two sequence motifs. Two of these proteins are enzymes of the inositol phosphate second messenger signaling pathway: - Vertebrate and plants inositol monophosphatase (EC 3.1.3.25). - Vertebrate inositol polyphosphate 1-phosphatase (EC 3.1.3.57).The function of the other proteins is not yet clear: - Bacterial protein cysQ. CysQ could help to control the pool of PAPS (3'-phosphoadenoside 5'-phosphosulfate), or be useful in sulfite synthesis. - Escherichia coli protein suhB. Mutations in suhB results in the enhanced synthesis of heat shock sigma factor (htpR). - Neurospora crassa protein Qa-X. Probably involved in quinate metabolism. - Emericella nidulans protein qutG. Probably involved in quinate metabolism. - Yeast protein HAL2/MET22 [2] involved in salt tolerance as well as methionine biosynthesis. - Yeast hypothetical hypothetical protein YHR046c. - Caenorhabditis elegans hypothetical protein F13G3.5. - A Rhizobium leguminosarum hypothetical protein encoded upstream of the pss gene for exopolysaccharide synthesis. - Methanococcus jannaschii hypothetical protein MJ0109.It is suggested [1] that these proteins may act by enhancing the synthesis or degradation of phosphorylated messenger molecules. From the X-ray structure of human inositol monophosphatase [3], it seems that some of the conserved residues are involved in binding a metal ion and/or the phosphate group of the substrate.
Consensus pattern: [FWV]-x(0,1)-[LIVM]-D-P-[LIVM]-D-[SG]-[ST]-x(2)-[FY]-x-[HKRNSTY] [The first D and the T bind a metal ion]-
Consensus pattern: [WV]-D-x-[AC]-[GSA]-[GSAPV]-x-[LIVACP]-[LIV]-[LIVAC]-x(3)-[GH]-[GA]-

[1] Neuwald A.F., York J.D., Majerus P.W. FEBS Lett. 294:16-18(1991).
[2] Glaeser H.-U., Thomas D., Gaxiola R., Montrichard F., Surdin-Kerjan Y., Serrano R. EMBO J. 12:3105-3110 (1993).
[3] Bone R., Springer J.P., Atack J.R. Proc. Natl. Acad. Sci. U.S.A. 89:10031-10035(1992).

313. Ion transport protein
This family contains Sodium, Potassium, Calcium ion channel This family is 6 transmembrane helices in which the last two helices flank a loop which determines ion selectivity. In some sub-families (e.g. Na channels) the domain is repeated four times, whereas in others (e.g. K channels) the protein forms as a tetramer in the membrane. A bacterial structure of the protein is known for the last two helices but is not the Pfam family due to it lacking the first four helices

314. Isocitrate and isopropylmalate dehydrogenases signature (isodh)
Isocitrate dehydrogenase (IDH) [1,2] is an important enzyme of carbohydrate metabolism which catalyzes the oxidative decarboxylation of isocitrate into alpha-ketoglutarate. IDH is either dependent on NAD+ (EC 1.1.1.41) or on NADP+(EC 1.1.1.42). In eukaryotes there are at least three isozymes of IDH: two are located in the mitochondrial matrix (one NAD+-dependent, the other NADP+-dependent), while the third one (also NADP+-dependent) is cytoplasmic. In Escherichia coli the activity of a NADP+-dependent form of the enzyme is controlled by the phosphorylation of a serine residue; the phosphorylated form of IDH is completely inactivated. 3-isopropylmalate dehydrogenase (EC 1.1.1.85) (IMDH) [3,4] catalyzes the third step in the biosynthesis of leucine in bacteria and fungi, the oxidative decarboxylation of 3-isopropylmalate into 2-oxo-4-methylvalerate. Tartrate dehydrogenase (EC 1.1.1.93) [5] catalyzes the reduction of tartrate to oxaloglycolate. These enzymes are evolutionary related [1,3,4,5]. The best conserved region of these enzymes is a glycine-rich stretch of residues located in the C-terminal section. This region was used as a signature pattern.
Consensus pattern: [NS]-[LIMYT]-[FYDN]-G-[DNT]-[IMVY]-x-[STGDN]-[DN]-x(2)-[SGAP]-x(3,4)-G-[STG]-[LIVMPA]-G-[LIVMF]-

[1] Hurley J.H., Thorsness P.E., Ramalingam V., Helmers N.H., Koshland D.E. Jr., Stroud R.M. Proc. Natl.

Acad. Sci. U.S.A. 86:8635-8639(1989).
[2] Cupp J.R., McAlister-Henn L. J. Biol. Chem. 266:22199-22205(1991).
[3] Imada K., Sato M., Tanaka N., Katsube Y., Matsuura Y., Oshima T. J. Mol. Biol. 222:725-738(1991).
[4] Zhang T., Koshland D.E. Jr. Protein Sci. 4:84-92(1995).
[5] Tipton P.A., Beecher B.S. Arch. Biochem. Biophys. 313:15-21(1994).

315. Jacalin-like lectin domain.
Proteins containing this domain are lectins. It is found in
1 to 6 copies in these proteins. The domain is also found in the animal prostatic spermine-binding protein (Swiss: P15501).

[1] Sankaranarayanan R, Sekar K, Banerjee R, Sharma V, Surolia A, Vijayan M; Nat Struct Biol 1996;3:596-603.

316. KH domain
KH motifs probably bind RNA directly. Auto antibodies to Nova, a KH domain protein, cause paraneoplastic opsoclonus ataxia.

[1] Burd CG, Dreyfuss G, Science 1994;265:615-621.
[2] Musco G, Stier G, Joseph C, Castiglione Morelli MA, Nilges M, Gibson TJ, Pastore A, Cell 1996;85:237-245.

317. Kelch motif
The kelch motif was initially discovered in Kelch (Swiss:Q04652). In this protein there are six copies of the motif. It has been shown that Swiss:Q04652 is related to Galactose Oxidase [1] for which a structure has been solved [2]. The kelch motif forms a beta sheet. Several of these sheets associate to form a beta propeller structure as found in neur,

[1] Bork P, Doolittle RF, J Mol Biol 1994;236:1277-1282.
[2] Ito N, Phillips SE, Stevens C, Ogel ZB, McPherson MJ, Keen, JN, Yadav KD, Knowles PF, Nature 1991; 350:87-90.

318. Soybean trypsin inhibitor (Kunitz) protease inhibitors family signature
The soybean trypsin inhibitor (Kunitz) family [1] is one of the numerous families of proteinase inhibitors. It comprise plant proteins which have inhibitory activity against serine proteinases from the trypsin and subtilisin families, thiol proteinases and aspartic proteinases as well as some proteins that are probably involved in seed storage. This family is currently known to group the following proteins: - Trypsin inhibitors A, B, C, KTI1, and KTI2 from soybean. - Trypsin inhibitor DE3 from coral beans (Erythrina sp.). - Trypsin inhibitor DE5 from sandal bead tree. - Trypsin inhibitors 1A (WTI-1A), 1B (WTI-1B), and 2 (WTI-2) from goa bean. - Trypsin inhibitor from Acacia confusa. - Trypsin inhibitor from silk tree. - Chymotrypsin inhibitor 3 (WCI-3) from goa bean. - Cathepsin D inhibitors PDI and NDI from potato [2], which inhibit both cathepsin D (aspartic proteinase) and trypsin. - Alpha-amylase/subtilisin inhibitors from barley and wheat. - Albumin-1 (WBA-1) from goa bean seeds [3]. - Miraculin from Richadella dulcifica [4], a sweet taste protein. - Sporamin from sweet potato [5], the major tuberous root protein. - Thiol proteinase inhibitor PCPI 8.3 (P340) from potato tuber [6]. - Wound responsive protein gwin3 from poplar tree [7]. - 21 Kd seed protein from cocoa [8].All these proteins contain from 170 to 200 amino acid residues and one or twointrachain disulfide bonds. The best conserved region is found in their N-terminal section and is used as a signature pattern
Consensus pattern: [LIVM]-x-D-x-[EDNTY]-[DG]-[RKHDENQ]-x-[LIVM]-x(5)-Y-x-[LIVM] -

[1] Laskowski M., Kato I. Annu. Rev. Biochem. 49:593-626(1980).
[2] Ritonja A., Krizaj I., Mesko P., Kopitar M., Lucovnik P., Strukelj B., Pungercar J., Buttle D.J., Barrett A.J., Turk V. FEBS Lett. 267:13-15(1990).
[3] Kortt A.A., Strike P.M., de Jersey J. Eur. J. Biochem. 181:403-408(1989).
[4] Theerasilp S., Hitotsuya H., Nakajo S., Nakaja K., Nakamura Y., Kurihara Y. J. Biol. Chem. 264:6655-6659 (1989).
[5] Hattori T., Yoshida N., Nakamura K. Plant Mol. Biol. 13:563-572(1989).
[6] Krizaj I., Drobnic-Kosorok M., Brzin J., Jerala R., Turk V. FEBS Lett. 333:15-20(1993).
[7] Bradshaw H.D., Hollick J.B., Parsons T.J., Clarke H.R.G., Gordon M.P. Plant Mol. Biol. 14:51-59(1989).
[8] Tai H., McHenry L., Fritz P.J., Furtek D.B. Plant Mol. Biol. 16:913-915(1991).

319. Beta-ketoacyl synthases active site

Beta-ketoacyl-ACP synthase (KAS) [1] is the enzyme that catalyzes the condensation of malonyl-ACP with the growing fatty acid chain. It is found as a component of the following enzymatic systems: - Fatty acid synthetase (FAS), which catalyzes the formation of long-chain fatty acids from acetyl-CoA, malonyl-CoA and NADPH. Bacterial and plant chloroplast FAS are composed of eight separate subunits which correspond to different enzymatic activities; beta-ketoacyl synthase is one of these polypeptides. Fungal FAS consists of two multifunctional proteins, FAS1 and FAS2; the beta-ketoacyl synthase domain is located in the C-terminal section of FAS2. Vertebrate FAS consists of a single multifunctional chain; the beta-ketoacyl synthase domain is located in the N-terminal section [2]. - The multifunctional 6-methysalicylic acid synthase (MSAS) from Penicillium patulum [3]. This is a multifunctional enzyme involved in the biosynthesis of a polyketide antibiotic and which has a KAS domain in its N-terminal section. - Polyketide antibiotic synthase enzyme systems. Polyketides are secondary metabolites produced by microorganisms and plants from simple fatty acids. KAS is one of the components involved in the biosynthesis of the Streptomyces polyketide antibiotics granatacin [4], tetracenomycin C [5] and erythromycin. - Emericella nidulans multifunctional protein Wa. Wa is involved in the biosynthesis of conidial green pigment. Wa is protein of 216 Kd that contains a KAS domain. - Rhizobium nodulation protein nodE, which probably acts as a beta-ketoacyl synthase in the synthesis of the nodulation Nod factor fatty acyl chain. - Yeast mitochondrial protein CEM1. The condensation reaction is a two step process: the acyl component of an activated acyl primer is transferred to a cysteine residue of the enzyme and is then condensed with an activated malonyl donor with the concomitant release of carbon dioxide. The sequence around the active site cysteine is well conserved and can be used as a signature pattern.
Consensus pattern: G-x(4)-[LIVMFAP]-x(2)-[AGC]-C-[STA](2)-[STAG]-x(3)-[LIVMF] [C is the active site residue]

[1] Kauppinen S., Siggaard-Andersen M., von Wettstein-Knowles P. Carlsberg Res. Commun. 53:357-370(1988)
.
[2] Witkowski A., Rangan V.S., Randhawa Z.I., Amy C.M., Smith S. Eur. J. Biochem. 198:571-579(1991).
[3] Beck J., Ripka S., Siegner A., Schiltz E., Schweizer E. Eur. J. Biochem. 192:487-498(1990).
[4] Bibb M.J., Biro S., Motamedi H., Collins J.F., Hutchinson C.R. EMBO J. 8:2727-2736(1989).
[5] Sherman D.H., Malpartida F., Bibb M.J., Kieser H.M., Bibb M.J., Hopwood D.A. EMBO J. 8:2717-2725(1989).

320. Kinesin motor domain signature and profile
Kinesin [1,2,3] is a microtubule-associated force-producing protein that mayplay a role in organelle transport. Kinesin is an oligomeric complex composedof two heavy chains and two light chains. The kinesin motor activity isdirected toward the microtubule’s plus end.The heavy chain is composed of three structural domains: a large globular N-terminal domain which is responsible for the motor activity of kinesin (it isknown to hydrolyze ATP, to bind and move on microtubules), a central alpha-helical coiled coil domain that mediates the heavy chain dimerization; and asmall globular C-terminal domain which interacts with other proteins (such asthe kinesin light chains), vesicles and membranous organelles.A number of proteins have been recently found that contain a domain similarto that of the kinesin ’motor’ domain [1,4,E1]: - Drosophila claret segregational protein (ncd). Ncd is required for normal chromosomal segregation in meiosis, in females, and in early mitotic divisions of the embryo. The ncd motor activity is directed toward the microtubule’s minus end. - Drosophila kinesin-like protein (nod). Nod is required for the distributive chromosome segregation of nonexchange chromosomes during meiosis. - Human CENP-E [4]. CENP-E is a protein that associates with kinetochores during chromosome congression, relocates to the spindle midzone at anaphase, and is quantitatively discarded at the end of the cell division. CENP-E is probably an important motor molecule in chromosome movement and/ or spindle elongation. - Human mitotic kinesin-like protein-1 (MKLP-1), a motor protein whose activity is directed toward the microtubule’s plus end. - Yeast KAR3 protein, which is essential for yeast nuclear fusion during mating. KAR3 may mediate microtubule sliding during nuclear fusion and possibly mitosis. - Yeast CIN8 and KIP1 proteins which are required for the assembly of the mitotic spindle. Both proteins seem to interact with spindle microtubules to produce an outwardly directed force acting upon the poles. - Fission yeast cut7 protein, which is essential for spindle body duplication during mitotic division. - Emericella nidulans bimC, which plays an important role in nuclear division. - Emericella nidulans klpA. - Caenorhabditis elegans unc-104, which may be required for the transport of substances needed for neuronal cell differentiation. - Caenorhabditis elegans osm-3. - Xenopus Eg5, which may be involved in mitosis. - Arabidopsis thaliana KatA, KatB and katC. - Chlamydomonas reinhardtii FLA10/KHP1 and KLP1. Both proteins seem to play a role in the rotation or twisting of the microtubules of the flagella. - Caenorhabditis elegans hypothetical protein T09A5.2.The kinesin motor domain is located in the N-terminal part of most of theabove proteins, with the exception of KAR3, klpA, and ncd where it is locatedin the C-terminal section.The kinesin motor domain contains about 330 amino acids. An ATP-binding motifof type A is found near position 80 to 90, the C-terminal half of the domainis involved in microtubule-binding. The signature pattern for that domain isderived from a conserved decapeptide inside the microtubule-binding part.
Consensus pattern: [GSA]-[KRHPSTQVM]-[LIVMF]-x-[LIVMF]-[IVC]-D-L-[AH]-G-[SAN]-E

[1] Bloom G.S., Endow S.A. Protein Prof. 2:1109-1171 (1995).
[2] Vallee R.B., Shpetner H.S. Annu. Rev. Biochem. 59:909-932(1990).
[3] Brady S.T. Trends Cell Biol. 5:159-164(1995).
[4] Endow S.A. Trends Biochem. Sci. 16:221-225(1991).[E1]

321. Ribosomal protein L1 5 signature

Ribosomal protein L1 5 is one of the proteins from the large ribosomal subunit. In Escherichia coli, L1 5 is known to bind the 23 S rRNA. It belongs to a family of ribosomal proteins which, on the basis of sequence similarities [1], groups: - Eubacterial L15. - Plant chloroplast L15 (nuclear-encoded). - Archaebacterial L15. - Vertebrate L27a. - Tetrahymena thermophila L29. - Fungi L27a (L29, CRP-1, CYH2).L15 is a protein of 144 to 154 amino-acid residues. As a signature pattern, a conserved region was selected in the C-terminal section of these proteins.

Consensus pattern: K-[LIVM](2)-[GASL]-x-[GT]-x-[LIVMA]-x(2,5)-[LIVM]-x- [LIVMF]-x(3,4)-[LIVMFCA]-[ST]-x(2)-A-x(3)-[LIVM]-x(3)-G

[1] Otaka E., Hashimoto T., Mizuta K., Suzuki K. Protein Seq. Data Anal. 5:301-313(1993).

322. LBP / BPI / CETP family signature

The following mammalian lipid-binding serum glycoproteins belong to the same family [1,2,3]: - Lipopolysaccharide-binding protein (LBP). LBP binds to the lipid A moiety of bacterial lipopolysaccharides (LPS), a glycolipid present in the outer membrane of all Gram-negative bacteria. The LBP/LPS complex seems to interact with the CD14 receptor and may be responsible for the secretion of alpha-TNF. - Bactericidal permeability-increasing protein (BPI). Like LBP, BPI binds LPS and has a cytotoxic activity on Gram-negative bacteria. - Cholesteryl ester transfer protein (CETP). CETP is involved in the transfer of insoluble cholesteryl esters in reverse cholesterol transport. - Phospholipid transfer protein (PLTP). May play a key role in extracellular phospholipid transport and modulation of HDL particles. These proteins are structurally related and share many regions of sequencesimilarities. As a signature pattern one of these regions was selected, which is located in the N-terminal section of these proteins; a region which could be involved in the binding to the lipids [2].

Consensus pattern: [PA]-[GA]-[LIVMC]-x(2)-R-[IV]-[ST]-x(3)-L-x(5)-[EQ]-x(4)-[LIVM]-[EQK]-x(8)-P

[1] Schumann R.R., Leong S.R., Flaggs G.W., Gray P.W., Wright S.D., Mathison J.C., Tobias P.S., Ulevitch R.J. Science 249:1429-1431(1990).
[2] Gray P.W., Flaggs G., Leong S.R., Gumina R.J., Weiss J., Ooi C.E., Elsbach P. J. Biol. Chem. 264:9505-9509 (1989).
[3] Day J.R., Albers J.J., Lofton-Day C.E., Gilbert T.L., Ching A.F.T., Grant F.J., O'Hara P.J., Marcovina S.M., Adolphson J.L. J. Biol. Chem. 269:9388-9391(1994).

323. LIM domain signature and profile

Recently [1,2] a number of proteins have been found to contain a conserved cysteine-rich domain of about 60 amino-acid residues. These proteins are: - Caenorhabditis elegans mec-3; a protein required for the differentiation of the set of six touch receptor neurons in this nematode. - Caenorhabditis elegans lin-11; a protein required for the asymmetric division of vulval blast cells. - Vertebrate insulin gene enhancer binding protein isl-1. Isl-1 binds to one of the two cis-acting protein-binding domains of the insulin gene. - Vertebrate homeobox proteins lim-1, lim-2 (lim-5) and lim3. - Vertebrate lmx-1, which acts as a transcriptional activator by binding to the FLAT element; a beta-cell-specific transcriptional enhancer found in the insulin gene. - Mammalian LH-2, a transcriptional regulatory protein involved in the control of cell differentiation in developing lymphoid and neural cell types. - Drosophila protein apterous, required for the normal development of the wing and halter imaginal discs. - Vertebrate protein kinases LIMK-1 and LIMK-2. - Mammalian rhombotins. Rhombotin 1 (RBTN1 or TTG-1) and rhombotin-2 (RBTN2 or TTG-2) are proteins of about 160 amino acids whose genes are disrupted by chromosomal translocations in T-cell leukemia. - Mammalian and avian cysteine-rich protein (CRP), a 192 amino-acid protein of unknown function. Seems to interact with zyxin. - Mammalian cysteine-rich intestinal protein (CRIP), a small protein which seems to have a role in zinc absorption and may function as an intracellular zinc transport protein. - Vertebrate paxillin, a cytoskeletal focal adhesion protein. - Mouse testin. Mouse testin should not be confused with rat testin which is a thiol protease homolog. - Sunflower pollen specific protein SF3. - Chicken zyxin. Zyxin is a low-abundance adhesion plaque protein which has been shown to interact with CRP. - Yeast protein LRG1 which is involved in sporulation [4]. - Yeast rhotype GTPase activating protein RGA1/DBM1. - Caenorhabditis elegans homeobox protein ceh-14. - Caenorhabditis elegans homeobox protein unc-97. - Yeast hypothetical protein YKR090w. - Caenorhabditis elegans hypothetical proteins C28H8.6.These proteins generally have two tandem copies of a domain, called LIM (forLin-11 Isl-1 Mec-3) in their N-terminal section. Zyxin and paxillin areexceptions in that they contains respectively three and four LIM

domains attheir C-terminal extremity. In apterous, isl-1, LH-2, lin-11, lim-1 to lim-3,lmx-1 and ceh-14 and mec-3 there is a homeobox domain some 50 to 95 amino acids after theLIM domains.In the LIM domain, there are seven conserved cysteine residues and ahistidine. The arrangement followed by these conserved residues is C-x(2)-C-x (16,23)-H-x(2)-[CH]-x(2)-C-x(2)-C-x(16,21)-C-x(2,3)-[CHD]. The LIM domainbinds two zinc ions [5]. LIM does not bind DNA, rather it seems to act asinterface for protein-protein interaction. A pattern was developed that spans the first half of the LIM domain.
Consensus pattern: C-x(2)-C-x(15,21)-[FYWH]-H-x(2)-[CH]-x(2)-C-x(2)-C-x(3)- [LIVMF] [The 5 C's and the H bind zinc]

[1] Freyd G., Kim S.K., Horvitz H.R. Nature 344:876-879(1990).
[2] Baltz R., Evrard J.-L., Domon C., Steinmetz A. Plant Cell 4:1465-1466(1992).
[3] Sanchez-Garcia I., Rabbitts T.H. Trends Genet. 10:315-320(1994).
[4] Mueller A., Xu G., Wells R., Hollenberg C.P., Piepersberg W. Nucleic Acids Res. 22:3151-3154(1994).
[5] Michelsen J.W., Schmeichel K.L., Beckerle M.C., Winge D.R. Proc. Natl. Acad. Sci. U.S.A. 90:4404-4408 (1993).

324. (LRR) Leucine Rich Repeat
CAUTION: This Pfam may not find all Leucine Rich Repeats in a protein. Leucine Rich Repeats are short sequence motifs present in a number of proteins with diverse functions and cellular locations. These repeats are usually involved in protein-protein interactions. Each Leucine Rich Repeat is composed of a beta-alpha unit. These units form elongated non-globular structures. Leucine Rich Repeats are often flanked by cysteine rich domains. Number of members: 3017

[1] The leucine-rich repeat: a versatile binding motif. Kobe B, Deisenhofer J; Trends Biochem Sci 1994;19: 415-421.
[2] Crystal structure of porcine ribonuclease inhibitor, a protein with leucine-rich repeats. Kobe B, Deisenhofer J; Nature 1993;366:751-756.

325. Plant lipid transfer protein family signature (LTP)
Plant cells contain proteins, called lipid transfer proteins (LTP) [1,2,3], which are able to facilitate the transfer of phospholipids and other lipidsacross membranes. These proteins, whose subcellular location is not yet known, could play a major role in membrane biogenesis by conveying phospholipids such as waxes or cutin from their site of biosynthesis to membranes unable to form these lipids. Plant LTP's are proteins of about 9 Kd (90 amino acids) which contain eight conserved cysteine residues all involved in disulfide bridges, as shown in the following schematic representation.
+---------------+ |+------+ ||||| **************** xCxxxxCxxxxxxCCxxxxxxxxCxCxxxxxxxxxxxCxxxxxxCxx ||| | +--------|-------------+ |+-------------+
'C': conserved cysteine involved in a disulfide bond.
'*': position of the pattern.
Consensus pattern: [LIVM]-[PA]-x(2)-C-x-[LIVM]-x-[LIVM]-x-[LIVMFY]-x-[LIVM]-[ST]-x(3)-[DN]-C-x(2)-[LIVM] [The two C's are involved in disulfide bonds]

[1] Wirtz K.W.A. Annu. Rev. Biochem. 60:73-99(1991).
[2] Arondel V., Kader J.C. Experientia 46:579-585(1990).
[3] Ohlrogge J.B., Browse J., Somerville C.R. Biochim. Biophys. Acta 1082:1-26(1991).

326. (LAMP) Lysosome-associated membrane glycoproteins signatures
Lysosome-associated membrane glycoproteins (lamp) [1] are integral membrane proteins, specific to lysosomes, and whose exact biological function is not yet clear. Structurally, the lamp proteins consist of two internally homologous lysosome-luminal domains separated by a proline-rich hinge region; at the C-terminal extremity there is a transmembrane region followed by a very short cytoplasmic tail. In each of the duplicated domains, there are two conserved disulfide bonds. This structure is schematically represented in the figure below.
+-----+ +-----+ +-----+ +-----+ |||||||
xCxxxxxCxxxxxxxxxxxxCxxxxxCxxxxxxxxxxCxxxxxCxxxxxxxxxxxxxCxxxxxCxxxxxxxxx <--------------------------><Hinge><--------------------------><TM><C> In mammals, there are two closely related types of lamp: lamp-1 and lamp-2. In chicken lamp-1 is known as LEP100.The macrophage protein CD68 (or macrosialin) [2] is a heavily glycosylatedintegral membrane protein whose structure consists of a mucin-like domain followed by a proline-rich hinge; a single lamp-like domain; a transmembrane region and a short cytoplasmic tail. Two signature patterns for

this family of proteins were developed. The first oneis centered on the first conserved cysteine of the duplicated domains. The second corresponds to a region that includes the extremity of the second domain, the totality of the transmembrane region and the cytoplasmic tail.
Consensus pattern: [STA]-C-[LIVM]-[LIVMFYW]-A-x-[LIVMFYW]-x(3)-[LIVMFYW]-x(3)-Y [C is involved in a disulfide bond] -
Consensus pattern: C-x(2)-D-x(3,4)-[LIVM](2)-P-[LIVM]-x-[LIVM]-G-x(2)-[LIVM]- x-G-[LIVM](2)-x-[LIVM](4)-A-[FY]-x-[LIVM]-x(2)-[KR]-[RH]- x(1,2)-[STAG](2)-Y-[EQ] [C is involved in a disulfide bond]

[1] Fukuda M. J. Biol. Chem. 266:21327-21330(1991).
[2] Holness C.L., da Silva R.P., Fawcett J., Gordon S., Simmons D.L. J. Biol. Chem. 268:9661-9666(1993).

327. Lipolytic enzymes "G-D-S-L" family, serine active site
Recently [1], a family of lipolytic enzymes has been characterized. This family currently consist of the following proteins:

- Aeromonas hydrophila lipase/phosphatidylcholine-sterol acyltransferase.
- Xenorhabdus luminescens lipase 1.
- Vibrio mimicus arylesterase.
- Escherichia coli acyl-coA thioesterase I (gene tesA).
- Vibrio parahaemolyticus thermolabile hemolysin/atypical phospholipase.
- Rabbit phospholipase AdRab-B, an intestinal brush border protein with esterase and phospholipase A/lysophospholipase activity that could be involved in the uptake of dietary lipids. AdRab-B contains four repeats of about 320 amino acids.
- Arabidopsis thaliana and Brassic napus anther-specific proline-rich protein APG.
- A Pseudomonas putida hypothetical protein in trpE-trpG intergenic region. A serine has been identified a part of the active site in the Aeromonas, Vibrio mimicus and Escherichia coli enzymes. It is located in a conserved sequence motif that can be used as a signature pattern for these proteins.

- Consensus pattern: [LIVMFYAG](4)-G-D-S-[LIVM]-x(1,2)-[TAG]-G

[S is the active site residue]

328. (Lipoprotein 4) Prokaryotic membrane lipoprotein lipid attachment site
In prokaryotes, membrane lipoproteins are synthesized with a precursor signal peptide, which is cleaved by a specific lipoprotein signal peptidase (signalpeptidase II). The peptidase recognizes a conserved sequence and cuts upstreamof a cysteine residue to which a glyceride-fatty acid lipid is attached [1].Some of the proteins known to undergo such processing currently include (forrecent listings see [1,2,3]): - Major outer membrane lipoprotein (murein-lipoproteins) (gene lpp). - Escherichia coli lipoprotein-28 (gene nlpA). - Escherichia coli lipoprotein-34 (gene nlpB). - Escherichia coli lipoprotein nlpC. - Escherichia coli lipoprotein nlpD. - Escherichia coli osmotically inducible lipoprotein B (gene osmB). - Escherichia coli osmotically inducible lipoprotein E (gene osmE). - Escherichia coli peptidoglycan-associated lipoprotein (gene pal). - Escherichia coli rare lipoproteins A and B (genes rplA and rplB). - Escherichia coli copper homeostasis protein cutF (or nlpE). - Escherichia coli plasmids traT proteins. - Escherichia coli Col plasmids lysis proteins. - A number of Bacillus beta-lactamases. - Bacillus subtilis periplasmic oligopeptide-binding protein (gene oppA). - Borrelia burgdorferi outer surface proteins A and B (genes ospA and ospB). - Borrelia hermsii variable major protein 21 (gene vmp21) and 7 (gene vmp7). - Chlamydia trachomatis outer membrane protein 3 (gene omp3). - Fibrobacter succinogenes endoglucanase cel-3. - Haemophilus influenzae proteins Pal and Pcp. - Klebsiella pullulunase (gene pulA). - Klebsiella pullulunase secretion protein pulS. - Mycoplasma hyorhinis protein p37. - Mycoplasma hyorhinis variant surface antigens A, B, and C (genes vlpABC). - Neisseria outer membrane protein H.8. - Pseudomonas aeruginosa lipopeptide (gene lppL). - Pseudomonas solanacearum endoglucanase egl. - Rhodopseudomonas viridis reaction center cytochrome subunit (gene cytC). - Rickettsia 17 Kd antigen. - Shigella flexneri invasion plasmid proteins mxiJ and mxim. - Streptococcus pneumoniae oligopeptide transport protein A (gene amiA). - Treponema pallidium 34 Kd antigen. - Treponema pallidium membrane protein A (gene tmpA). - Vibrio harveyi chitobiase (gene chb). - Yersinia virulence plasmid protein yscJ. - Halocyanin from Natrobacterium pharaon is [4], a membrane associated copper- binding protein. This is the first archaebacterial protein known to be modified in such a fashion).From the precursor sequences of all these proteins, a consensus pattern and a set of rules to identify this type of post-translational modification was derived.
Consensus pattern: {DERK}(6)-[LIVMFWSTAG](2)-[LIVMFYSTAGCQ]-[AGS]-C [C is the lipid attachment site] Additional rules: 1) The cysteine must be between positions 15 and 35 of the sequence in consideration. 2) There must

be at least one Lys or one Arg in the first seven positions of the sequence.

[1] Hayashi S., WuH.C. J. Bioenerg. Biomembr. 22:451-471(1990).
[2] Klein P., Somorjai R.L., Lau P.C.K. Protein Eng. 2:15-20(1988).
[3] von Heijne G. Protein Eng. 2:531-534(1989).
[4] Mattar S., Scharf B., Kent S.B.H., Rodewald K., Oesterhelt D., Engelhard M. J. Biol. Chem. 269:14939-14945 (1994).

329. (Lopoprotein 5) Prokaryotic membrane lipoprotein lipid attachment site. In prokaryotes, membrane lipoproteins are synthesized with a precursor signal peptide, which is cleaved by a specific lipoprotein signal peptidase (signal peptidase II). The peptidase recognizes a conserved sequence and cuts upstream of a cysteine residue to which a glyceride-fatty acid lipid is attached [1].Some of the proteins known to undergo such processing currently include (for recent listings see [1,2,3]): - Major outer membrane lipoprotein (murein-lipoproteins) (gene lpp). - Escherichia coli lipoprotein-28 (gene nlpA). - Escherichia coli lipoprotein-34 (gene nlpB). - Escherichia coli lipoprotein nlpC. - Escherichia coli lipoprotein nlpD. - Escherichia coli osmotically inducible lipoprotein B (gene osmB). - Escherichia coli osmotically inducible lipoprotein E (gene osmE). - Escherichia coli peptidoglycan-associated lipoprotein (gene pal). - Escherichia coli rare lipoproteins A and B (genes rplA and rplB). - Escherichia coli copper homeostasis protein cutF (or nlpE). - Escherichia coli plasmids traT proteins. - Escherichia coli Col plasmids lysis proteins. - A number of Bacillus beta-lactamases. - Bacillus subtilis periplasmic oligopeptide-binding protein (gene oppA). - Borrelia burgdorferi outer surface proteins A and B (genes ospA and ospB). - Borrelia hermsii variable major protein 21 (gene vmp21) and 7 (gene vmp7). - Chlamydia trachomatis outer membrane protein 3 (gene omp3). - Fibrobacter succinogenes endoglucanase cel-3. - Haemophilus influenzae proteins Pal and Pcp. - Klebsiella pullulunase (gene pulA). - Klebsiella pullulunase secretion protein pulS. - Mycoplasma hyorhinis protein p37. - Mycoplasma hyorhinis variant surface antigens A, B, and C (genes vlp ABC). - Neisseria outer membrane protein H.8. - Pseudomonas aeruginosa lipopeptide (gene lppL). - Pseudomonas solanacearum endoglucanase egl. - Rhodopseudomonas viridis reaction center cytochrome subunit (gene cytC). - Rickettsia 17 Kd antigen. - Shigella flexneri invasion plasmid proteins mxij and mxiM. - Streptococcus pneumoniae oligopeptide transport protein A (gene amiA). - Treponema pallidium 34 Kd antigen. - Treponema pallidium membrane protein A (gene tmpA). - Vibrio harveyi chitobiase (gene chb). - Yersinia virulence plasmid protein yscJ. - Halocyanin from Natrobacterium pharaonis [4], a membrane associated copper- binding protein. This is the first archaebacterial protein known to be modified in such a fashion).From the precursor sequences of all these proteins, a consensus pattern and a set of rules to identify this type of post-translational modification have been developed.
Consensus pattern: {DERK}(6)-[LIVMFWSTAG](2)-[LIVMFYSTAGCQ]-[AGS]-C [C is the lipid attachment site] Additional rules: 1) The cysteine must be between positions 15 and 35 of the sequence in consideration. 2) There must be at least one Lys or one Arg in the first seven positions of the sequence.

[1] Hayashi S., Wu H.C. J. Bioenerg. Biomembr. 22:451-471(1990).
[2] Klein P., Somorjai R.L., Lau P.C.K. Protein Eng. 2:15-20(1988).
[3] von Heijne G. Protein Eng. 2:531-534(1989).
[4] Mattar S., Scharf B., Kent S.B.H., Rodewald K., Oesterhelt D., Engelhard M. J. Biol. Chem. 269:14939-14945 (1994).

330. (Lum binding) Riboflavin synthase alpha chain family Lum-binding site signature
The following proteins have been shown [1,2] to be structurally and evolutionary related: - Riboflavin synthase alpha chain (RS-alpha) (gene ribC in Escherichia coli, ribB in Bacillus subtilis and Photobacterium leiognathi, RIB5 in yeast). This enzyme synthesizes riboflavin from two moles of 6,7- dimethyl-8-(1'-D-ribityl)lumazine (Lum), a pteridine-derivative. - Photobacterium phosphoreum lumazine protein (LumP) (gene luxL). LumP is a protein that modulates the color of the bioluminescence emission of bacterial luciferase. In the presence of LumP, light emission is shifted to higher energy values (shorter wavelength). LumP binds non-covalently to 6,7-dimethyl-8-(1'-D-ribityl) lumazine. - Vibrio fischeri yellow fluorescent protein (YFP) (gene luxY). Like LumP, YFP modulates light emission but towards a longer wavelength. YFP binds non-covalently to FMN. These proteins seem to have evolved from the duplication of a domain of about100 residues. In its C-terminal section, this domain contains a conserved motif [KR]-V-N-[LI]-E which has been proposed to be the binding site for Lum.RS-alpha which binds two molecules of Lum has two perfect copies of this motif, while LumP which binds one molecule of Lum, has a Glu instead of Lys/Arg in the first position of the second copy of the motif. Similarily, YFP, which binds to one molecule of FMN, also seems to have a potentially dysfunctional binding site by substitution of Gly for Glu in the last positionof the first copy of the motif. Our signature pattern includes the Lum-binding motif.
Consensus pattern: [LIVMF]-x(5)-G-[STADNQ]-[KREQIYW]-V-N-[LIVM]-E

[1] O'Kane D.J., Woodward B., Lee J., Prasher D.C. Proc. Natl. Acad. Sci. U.S.A. 88:1100-1104(1991).
[2] O'Kane D.J., Prasher D.C. Mol. Microbiol. 6:443-449(1992).

331. Lysyl oxidase putative copper-binding region signature
Lysyl oxidase (LOX) [1] is an extracellular copper-dependent enzyme that catalyzes the oxidative deamination of peptidyl lysine residues in precursors of various collagens and elastins. The deaminated lysines are then able to form aldehyde cross-links.LOX binds a single copper atom which seems to reside within an octahedral coordination complex which includes at least three histidine ligands. Fourhistidine residues are clustered in a central region of the enzyme. This region is thought to be involved in cooper-binding and is called the 'copper-talon' [1]. This region was used as a signature pattern.
Consensus pattern: W-E-W-H-S-C-H-Q-H-Y-H

[1] Krebs C.J., Krawetz S.A. Biochim. Biophys. Acta 1202:7-12(1993).

332. Metallo-beta-lactamase superfamily (lactamase_B)

[1] Neuwald AF, Liu JS, Lipman DJ, Lawrence CE, Nucleic Acids Res 1997;25:1665-1677.
[2] Carfi A, Pares S, Duee E, Galleni M, Duez C, Frere JM, Dideberg O, EMBO J 1995;14:4914-4921.

333. L-lactate dehydrogenase active site (1dh1)
L-lactate dehydrogenase (EC 1.1.1.27) (LDH) [1] catalyzes the reversible NAD-dependent interconversion of pyruvate to L-lactate. In vertebrate muscles and in lactic acid bacteria it represents the final step in anaerobic glycolysis. This tetrameric enzyme is present in prokaryotic and eukaryotic organisms. Invertebrates there are three isozymes of LDH: the M form (LDH-A), found predominantly in muscle tissues; the H form (LDH-B), found in heart muscle and the X form (LDH-C), found only in the spermatozoa of mammals and birds. In birds and crocodilian eye lenses, LDH-B serves as a structural protein and is known as epsilon-crystallin [2].L-2-hydroxyisocaproate dehydrogenase (EC 1.1.1.-) (L-hicDH) [3] catalyzes the reversible and stereospecific interconversion between 2-ketocarboxylic acids and L-2-hydroxy-carboxylic acids. L-hicDH is evolutionary related to LDH's. As a signature for LDH's a region was selected that includes a conserved histidine which is essential to the catalytic mechanism.
Consensus pattern: [LIVMA]-G-[EQ]-H-G-[DN]-[ST] [H is the active site residue] -

[1] Abad-Zapatero C., Griffith J.P., Sussman J.L., Rossmann M.G. J. Mol. Biol. 198:445-467(1987).
[2] Hendriks W., Mulders J.W.M., Bibby M.A., Slingsby C., Bloemendal H., de Jong W.W. Proc. Natl. Acad. Sci. U.S.A. 85:7114-7118(1988).
[3] Lerch H.-P., Frank R., Collins J. Gene 83:263-270(1989).

Malate dehydrogenase active site signature (ldh2)
Malate dehydrogenase (EC 1.1.1.37) (MDH) [1,2] catalyzes the interconversion of malate to oxaloacetate utilizing the NAD/NADH cofactor system. The enzyme participates in the citric acid cycle and exists in all aerobic organisms. While prokaryotic organisms contains a single form of MDH, in eukaryotic cells there are two isozymes: one which is located in the mitochondrial matrix and the other in the cytoplasm. Fungi and plants also harbor a glyoxysomal form which functions in the glyoxylate pathway. In plants chloroplast there is an additional NADP-dependent form of MDH (EC 1.1.1.82) which is essential for both the universal C3 photosynthesis (Calvin) cycle and the more specializedC4 cycle. As a signature pattern for this enzyme a region was chosen that includes two residues involved in the catalytic mechanism [3]: an aspartic acid which is involved in a proton relay mechanism, and an arginine which binds the substrate.
Consensus pattern: [LIVM]-T-[TRKMN]-L-D-x(2)-R-[STA]-x(3)-[LIVMFY] [D and R are the active site residues]-

[1] McAlister-Henn L. Trends Biochem. Sci. 13:178-181(1988).
[2] Gietl C. Biochim. Biophys. Acta 1100:217-234(1992).
[3] Birktoft J.J., Rhodes G., Banaszak L.J. Biochemistry 28:6065-6081(1989).
[4] Cendrin F., Chroboczek J., Zaccai G., Eisenberg H., Mevarech M. Biochemistry 32:4308-4313(1993).

334. Legume lectins signatures
Leguminous plants synthesize sugar-binding proteins which are called legume lectins [1,2]. These lectins are generally found in the seeds. The exact function of legume lectins is not known but they may be involved in the attachment of nitrogen-fixing bacteria to legumes and in the protection against pathogens. Legume lectins bind calcium and manganese (or other transition metals). Legume lectins are synthesized as precursor proteins of about 230 to 260

amino acid residues. Some legume lectins are proteolytically processed to produce two chains: beta (which corresponds to the N-terminal) and alpha (C-terminal).The lectin concanavalin A (conA) from jack bean is exceptional in that the two chains are transposed and ligated (by formation of a new peptide bond). The N-terminus of mature conA thus corresponds to that of the alpha chain and the C-terminus to the beta chain. Two signature patterns specific to legume lectins have been developed: the first is located in the C-terminal section of the beta chain and contains a conserved aspartic acid residue important for the binding of calcium and manganese; the second one is located in the N-terminal of the alpha chain.
Consensus pattern: [LIV]-[STAG]-V-[DEQV]-[FLI]-D-[ST] [D binds manganese and calcium]-
Consensus pattern: [LIV]-x-[EDQ]-[FYWKR]-V-x-[LIVF]-G-[LF]-[ST]-

[1] Sharon N., Lis H. FASEB J. 4:3198-320(1990).
[2] Lis H., Sharon N. Annu. Rev. Biochem. 55:33-37(1986).

335. CoA-ligases (ligases- CoA)
This family includes the CoA ligases Succinyl-CoA synthetase alpha: and beta chains, malate CoA ligase and ATP-citrate lyase. Some members of the family utilise ATP others use GTP.

[1] Wolodko WT, Fraser ME, James MN, Bridger WA, J Biol Chem 1994;269:10883-10890.

336. linker histone H1 and H5 family
Linker histone H1 is an essential component of chromatin structure. H1 links nucleosomes into higher order structures Histone H1 is replaced by histone H5 in some cell types.

[1] Ramakrishnan V, Finch JT, Graziano V, Lee PL, Sweet RM, Nature 1993;362:219-223.

337. Lipocalin signature (lip1)
Proteins which transport small hydrophobic molecules such as steroids, bilins, retinoids, and lipids share limited regions of sequence homology and a common tertiary structure architecture [1 to 5]. This is an eight stranded antiparallel beta-barrel with a repeated + 1 topology enclosing a internal ligand binding site [1,3]. The name 'lipocalin'has been proposed [5] for this protein family. Proteins known to belong to this family are listed below (references are only provided for recently determined sequences). - Alpha-1-microglobulin (protein HC), which seems to bind porphyrin. - Alpha-1-acid glycoprotein (orosomucoid), which can bind a remarkable array of natural and synthetic compounds [6]. - Aphrodisin which, in hamsters, functions as an aphrodisiac pheromone. - Apolipoprotein D, which probably binds heme-related compounds. - Beta-lactoglobulin, a milk protein whose physiological function appears to bind retinol. - Complement component C8 gamma chain, which seems to bind retinol [7]. - Crustacyanin [8], a protein from lobster carapace, which binds astaxanthin, a carotenoid. - Epididymal-retinoic acid binding protein (E-RABP) [9] involved in sperm maturation. - Insectacyanin, a moth bilin-binding protein, and a related butterfly bilin-binding protein (BBP). - Late Lactation protein (LALP), a milk protein from tammar wallaby [10]. - Neutrophil gelatinase-associated lipocalin (NGAL) (p25) (SV-40 induced 24p3 protein) [11]. - Odorant-binding protein (OBP), which binds odorants. - Plasma retinol-binding proteins (PRBP). - Human pregnancy-associated endometrial alpha-2 globulin. - Probasin (PB), a rat prostatic protein. - Prostaglandin D synthase (EC 5.3.99.2) (GSH-independent PGD synthetase), a lipocalin with enzymatic activity [12]. - Purpurin, a retinal protein which binds retinol and heparin. - Quiescence specific protein p20K from chicken (embryo CH21 protein). - Rodent urinary proteins (alpha-2-microglobulin), which may bind pheromones. - VNSP 1 and 2, putative pheromone transport proteins from mouse vomeronasal organ [13]. - Von Ebner's gland protein (VEGP) [14] (also called tear lipocalin), a mammalian protein which may be involved in taste recognition. - A frog olfactory protein, which may transport odorants. - A protein found in the cerebrospinal fluid of the toad Bufo Marinus with a supposed function similar to transthyretin in transport across the blood brain barrier [15]. - Lizard's epididymal secretory protein IV (LESP IV), which could transport small hydrophobic molecules into the epididymal fluid during sperm maturation [16]. - Prokaryotic outer-membrane protein blc [17].The sequences of most members of the family, the core or kernal lipocalins, are characterized by three short conserved stretches of residues [3,18].Others, the outlier lipocalin group, share only one or two of these [3,18]. A signature pattern was built around the first, common to all outlier and kemallipocalins, which occurs near the start of the first beta-strand.
Consensus pattern: [DENG]-x-[DENQGSTARK]-x(0,2)-[DENQARK]-[LIVFY]-{CP}-G-{C}- W-[FYWLRH]-x-[LIVMTA]-
Note: it is suggested, on the basis of similarities of structure, function, and sequence, that this family forms an overall superfamily, called the calycins, with the avidin/streptavidin <PDOC00499> and the cytosolic fatty- acid binding proteins <PDOC00188> families [3,19]

[1] Cowan S.W., Newcomer M.E., Jones T.A. Proteins 8:44-61 (1990).
[2] Igaraishi M., Nagata A., Toh H., Urade H., Hayaishi N. Proc. Natl. Acad. Sci. U.S.A. 89:5376-5380(1992).
[3] Flower D.R., North A.C.T., Attwood T.K. Protein Sci. 2:753-761(1993).
[4] Godovac-Zimmermann J. Trends Biochem. Sci. 13:64-66(1988).
[5] Pervaiz S., Brew K. FASEB J. 1:209-214(1987).
[6] Kremer J.M.H., Wilting J., Janssen L.H.M. Pharmacol. Rev. 40:1-47(1989).
[7] Haefliger J.-A., Peitsch M.C., Jenne D., Tschopp J. Mol. Immunol. 28:123-131(1991).
[8] Keen J.N., Caceres I., Eliopoulos E.E., Zagalsky P.F., Findlay J.B.C. Eur. J. Biochem. 197:407-417(1991).
[9] Newcomer M.E. Structure 1:7-18(1993).
[10] Collet C., Joseph R. Biochim. Biophys. Acta 1167:219-222(1993).
[11] Kjeldsen L., Johnsen A.H., Sengelov H., Borregaard N. J. Biol. Chem. 268:10425-10432(1993).
[12] Peitsch M.C., Boguski M.S. Trends Biochem. Sci. 16:363-363(1991).
[13] Miyawaki A., Matsushita Y.R., Ryo Y., Mikoshiba T. EMBO J. 13:5835-5842(1994).
[14] Kock K., Ahlers C., Schmale H. Eur. J. Biochem. 221:905-916(1994).
[15] Achen M.G., Harms P.J., Thomas T., Richardson S.J., Wettenhall R.E.H., Schreiber G. J. Biol. Chem. 267: 23170-23174(1992).
[16] Morel L., Dufarre J.-P., Depeiges A. J. Biol. Chem. 268:10274-10281(1993).
[17] Bishop R.E., Penfold S.S., Frost L.S., Holtje J.V., Weiner J.H. J. Biol. Chem. 270:23097-23103(1995).
[18] Flower D.R., North A.C.T., Attwood T.K. Biochem. Biophys. Res. Commun. 180:69-74(1991).
[19] Flower D.R. FEBS Lett. 333:99-102(1993).

Cytosolic fatty-acid binding proteins signature (lip2)
A number of low molecular weight proteins which bind fatty acids and other organic anions are present in the cytosol [1,2]. Most of them are structurally related and have probably diverged from a common ancestor. This structure is a ten stranded antiparallel beta-barrel, albeit with a wide discontinuity between the fourth and fifth strands, with a repeated + 1 topology enclosing an internal ligand binding site [2,7]. Proteins known to belong to this family include: - Six, tissue-specific, types of fatty acid binding proteins (FABPs) found in liver, intestine, heart, epidermal, adipocyte, brain/retina. Heart FABP is also known as mammary-derived growth inhibitor (MDGI), a protein that reversibly inhibits proliferation of mammary carcinoma cells. Epidermal FABP is also known as psoriasis-associated FABP [3]. - Insect muscle fatty acid-binding proteins. - Testis lipid binding protein (TLBP). - Cellular retinol-binding proteins I and II (CRBP). - Cellular retinoic acid-binding protein (CRABP). - Gastrotropin, an ileal protein which stimulates gastric acid and pepsinogen secretion. It seems that gastrotropin binds to bile salts and bilirubins. - Fatty acid binding proteins MFB1 and MFB2 from the midgut of the insect Manduca sexta [4].In addition to the above cytosolic proteins, this family also includes: - Myelin P2 protein, which may be a lipid transport protein in Schwann cells. P2 is associated with the lipid bilayer of myelin. - Schistosoma mansoni protein Sm14 [5] which seems to be involved in the transport of fatty acids. - Ascaris suum p18 a secreted protein that may play a role in sequestering potentially toxic fatty acids and their peroxidation products or that may be involved in the maintenance of the impermeable lipid layer of the eggshell. - Hypothetical fatty acid-binding proteins F40F4.2, F40F4.3, F40F4.4 and ZK742.5 from Caenorhabditis elegans. As a signature pattern for these proteins a segment from the N-terminal extremity was use.
Consensus pattern: [GSAIVK]-x-[FYW]-x-[LIVMF]-x(4)-[NHG]-[FY]-[DE]-x-[LIVMFY]-[LIVM]-x(2)-[LIVMAKR]-
Note: it is suggested, on the basis of similarities of structure, function, and sequence, that this family forms an overall superfamily, called the calycins, with the lipocalin <PDOC00187> and avidin/streptavidin <PDOC00499> families [6,7].

[1] Bernier I., Jolles P. Biochimie 69:1127-1152(1987).
[2] Veerkamp J.H., Peeters R.A., Maatman R.G.H.J. Biochim. Biophys. Acta 1081:1-24(1991).
[3] Siegenthaler G., Hotz R., Chatellard-Gruaz D., Didierjean L., Hellman U., Saurat J.-H. Biochem. J. 302: 363-371(1994).
[4] Smith A.F., Tsuchida K., Hanneman E., Suzuki T.C., Wells M.A. J. Biol. Chem. 267:380-384(1992).
[5] Moser D., Tendler M., Griffiths G., Klinkert M.-Q. J. Biol. Chem. 266:8447-8454(1991).
[6] Flower D.R., North A.C.T, Attwood T.K. Protein Sci. 2:753-761(1993).
[7] Flower D.R. FEBS Lett. 333:99-102(1993).

338. Lipoxygenases iron-binding region signatures
Lipoxygenases (EC 1.13.11.-) are a class of iron-containing dioxygenases which catalyzes the hydroperoxidation of lipids, containing a cis,cis-1,4-pentadiene structure. They are common in plants where they may be involved in a number of diverse aspects of plant physiology including growth and development, pest resistance, and senescence or responses to wounding [1]. In mammals a number of lipoxygenases isozymes are involved in the metabolism of

prostaglandins and leukotrienes [2]. Sequence data is available for the following lipoxygenases: - Plant lipoxygenases (EC 1.13.11.12). Plants express a variety of cytosolic isozymes as well as what seems [3] to be a chloroplast isozyme. - Mammalian arachidonate 5-lipoxygenase (EC 1.13.11.34). - Mammalian arachidonate 12-lipoxygenase (EC 1.13.11.31). - Mammalian erythroid cell-specific 15-lipoxygenase (EC 1.13.11.33).The iron atom in lipoxygenases is bound by four ligands, three of which are histidine residues [4]. Six histidines are conserved in all lipoxygenase sequences, five of them are found clustered in a stretch of 40 amino acids. This region contains two of the three zinc-ligands; the other histidines have been shown [5] to be important for the activity of lipoxygenases. As signatures for this family of enzymes two patterns in the region of the histidine cluster were selected. The first pattern contains the first three conserved histidines and the second pattern includes the fourth and the fifth.
Consensus pattern: H-[EQ]-x(3)-H-x-[LM]-[NQRC]-[GST]-H-[LIVMSTAC](3)-E [The second and third H's bind iron]-
Consensus pattern: [LIVMA]-H-P-[LIVM]-x-[KRQ]-[LIVMF](2)-x-[AP]-H-

[1] Vick B.A., Zimmerman D.C. (In) Biochemistry of plants: A comprehensive treatise, Stumpf P.K., Ed., Vol. 9, pp.53-90, Academic Press, New-York, (1987).
[2] Needleman P., Turk J., Jakschik B.A., Morrison A.R., Lefkowith J.B. Annu. Rev. Biochem. 55:69-102(1986).
[3] Peng Y.L., Shirano Y., Ohta H., Hibino T., Tanaka K., Shibata D. J. Biol. Chem. 269:3755-3761 (1994).
[4] Boyington J.C., Gaffney B.J., Amzel L.M. Science 260:1482-1486(1993).
[5] Steczko J., Donoho G.P., Clemens J.C., Dixon J.E., Axelrod B. Biochemistry 31:4053-4057(1992).

339. Fumarate lyases signature (lyase_1)
A number of enzymes, belonging to the lyase class, for which fumarate is a substrate have been shown [1,2] to share a short conserved sequence around a methionine which is probably involved in the catalytic activity of this type of enzymes. These enzymes are: - Fumarase (EC 4.2.1.2) (fumarate hydratase), which catalyzes the reversible hydration of fumarate to L-malate. There seem to be 2 classes of fumarases: class I are thermolabile dimeric enzymes (as for example: Escherichia coli fumC); class II enzymes are thermostable and tetrameric and are found in prokaryotes (as for example: Escherichia coli fumA and fumB) as well as in eukaryotes. The sequence of the two classes of fumarases are not closely related. - Aspartate ammonia-lyase (EC 4.3.1.1) (aspartase), which catalyzes the reversible conversion of aspartate to fumarate and ammonia. This reaction is analogous to that catalyzed by fumarase, except that ammonia rather than water is involved in the transelimination reaction. - Arginosuccinase (EC 4.3.2.1) (argininosuccinate lyase), which catalyzes the formation of arginine and fumarate from argininosuccinate, the last step in the biosynthesis of arginine. - Adenylosuccinase (EC 4.3.2.2) (adenylosuccinate lyase) [3], which catalyzes the eight step in the de novo biosynthesis of purines, the formation of 5'-phosphoribosyl-5-amino-4-imidazolecarboxamide and fumarate from 1-(5-phosphoribosyl)-4-(N-succino-carboxamide). That enzyme can also catalyzes the formation of fumarate and AMP from adenylosuccinate. - Pseudomonas putida 3-carboxy-cis,cis-muconate cycloisomerase (EC 5.5.1.2) (3-carboxymuconate lactonizing enzyme) (gene pcaB) [4], an enzyme involved in aromatic acids catabolism
Consensus pattern: G-S-x(2)-M-x(2)-K-x-N-

[1] Woods S.A., Shwartzbach S.D., Guest J.R. Biochim. Biophys. Acta 954:14-26(1988).
[2] Woods S.A., Miles J.S., Guest J.R. FEMS Microbiol. Lett. 51:181-186(1988).
[3] Zalkin H., Dixon J.E. Prog. Nucleic Acid Res. Mol. Biol. 42:259-287(1992).
[4] Williams S.E., Woolridge E.M., Ransom S.C., Landro J.A., Babbitt P.C., Kozarich J.W. Biochemistry 31: 9768-9776(1992).

340. MCM family signature and profile
Proteins shown to be required for the initiation of eukaryotic DNA replication share a highly conserved domain of about 210 amino-acid residues [1,2,3]. The latter shows some similarities [4] with that of various other families of DNA-dependent ATPases. Eukaryotes seem to possess a family of six proteins that contain this domain. They were first identified in yeast where most of them have a direct role in the initiation of chromosomal DNA replication by interacting directly with autonomously replicating sequences (ARS). They were thus called 'minichromosome maintenance proteins' with gene symbols prefixed by MCM. These six proteins are: - MCM2, also known as cdc19 (in S.pombe) [E1]. - MCM3, also known as DNA polymerase alpha holoenzyme-associated protein P1, RLF beta subunit or ROA. - MCM4, also known as CDC54, cdc21 (in S.pombe) or dpa (in Drosophila). - MCM5, also known as CDC46 or nda4 (in S.pombe). - MCM6, also known as mis5 (in S.pombe). - MCM7, also known as CDC47 or Prolifera (in A.thaliana).This family is also present in archebacteria. In Methanococcus jannaschiithere are four members: MJ0363, MJ0961, MJ1489 and MJECL13.The presence of a putative ATP-binding domain implies that these proteins maybe involved in an ATP-consuming step in the initiation of DNA replication in eukaryotes. As a signature pattern, a perfectly conserved region was selected that represents a special version of the B motif found in ATP-binding

proteins.
Consensus pattern: G-[IVT]-[LVAC](2)-[IVT]-D-[DE]-[FL]-[DNST]

[1] Coxon A., Maundrell K., Kearsey S.E. Nucleic Acids Res. 20:5571-5577(1992).
[2] Hu B., Burkhart R., Schulte D., Musahl C., Knippers R. Nucleic Acids Res. 21:5289-5293(1993).
[3] Tye B.-K. Trends Cell Biol. 4:160-166(1994).
[4] Koonin E.V. Nucleic Acids Res. 21:2541-2547(1993).

341. Macrophage migration inhibitory factor family signature (MIF)
A protein called macrophage migration inhibitory factor (MIF) [1] seems to exert an important role in host inflammatory responses. It play a pivotal role in the host response to endotoxic shock and appears to serve as a pituitary "stress" hormone that regulates systemic inflammatory responses. MIF is a secreted protein of 115 residues which is not processed from a larger precursor. D-dopachrome tautomerase [2] is a mammalian cytoplasmic enzyme involved in melanin biosynthesis and that tautomerizes D-dopachrome with concomitant decarboxylation to give 5,6-dihydroxyindole (DHI). It is a protein of 117 residues highly related to MIF. It must be noted that MIF binds glutathione and has been said to be related to glutathione S-transferases. This assertion has been later disproved [3].As a signature pattern for these proteins, a conserved region was selected located in the central section.
Consensus pattern: [DE]-P-C-A-x(3)-[LIVM]-x-S-I-G-x-[LIVM]-G-

[1] Bucala R. Immunol. Lett. 43:23-26(1994).
[2] Odh G., Hindemith A., Rosengren A.-M., Rosengren E., Rorsman H. Biochem. Biophys. Res. Commun. 197: 619-624(1993).
[3] Pearson W.R. Protein Sci. 3:525-527(1994).

342. MIP family signature
Recently the sequence of a number of different proteins, that all seem to be transmembrane channel proteins, has been found to be highly related [1 to 4].These proteins are listed below. - Mammalian major intrinsic protein (MIP). MIP is the major component of lens fiber gap junctions. Gap junctions mediate direct exchange of ions and small molecule from one cell to another. - Mammalian aquaporins [5]. These proteins form water-specific channels that provide the plasma membranes of red cells and kidney proximal and collecting tubules with high permeability to water, thereby permitting water to move in the direction of an osmotic gradient. - Soybean nodulin-26, a major component of the peribacteroid membrane induced during nodulation in legume roots after Rhizobium infection. - Plants tonoplast intrinsic proteins (TIP). There are various isoforms of TIP: alpha (seed), gamma, Rt (root), and Wsi (water-stress induced). These proteins may allow the diffusion of water, amino acids and/or peptides from the tonoplast interior to the cytoplasm. - Bacterial glycerol facilitator protein (gene glpF), which facilitates the movement of glycerol across the cytoplasmic membrane. - Salmonella typhimurium propanediol diffusion facilitator (gene pduF). - Yeast FPS1, a glycerol uptake/efflux facilitator protein. - Drosophila neurogenic protein 'big brain' (bib). This protein may mediate intercellular communication; it may functions by allowing the transport of certain molecules(s) and thereby sending a signal for an exodermal cell to become an epidermoblast instead of a neuroblast. - Yeast hypothetical protein YFL054c. - A hypothetical protein from the pepX region of lactococcus lactis. The MIP family proteins seem to contain six transmembrane segments. Computer analysis shows that these protein probably arose by a tandem, intragenic duplication event from an ancestral protein that contained three transmembrane segments. As a signature pattern a well conserved region was selected which is located in a probable cytoplasmic loop between the second and third transmembrane regions.
Consensus pattern: [HNQA]-x-N-P-[STA]-[LIVMF]-[ST]-[LIVMF]-[GSTAFY]-

[1] Reizer J., Reizer A., Saier M.H. Jr. CRC Crit. Rev. Biochem. 28:235-257(1993).
[2] Baker M.E., Saier M.H. Jr. Cell 60:185-186(1990).
[3] Pao G.M., Wu L.-F., Johnson K.D., Hoefte H., Chrispeels M.J., Sweet G., Sandal N.N., Saier M.H. Jr. Mol. Microbiol. 5:33-37(1991).
[4] Wistow G.J., Pisano M.M., Chepelinsky A.B. Trends Biochem. Sci. 16:170-171(1991).
[5] Chrispeels M.J., Agre P. Trends Biochem. Sci. 19:421-425(1994).

343. Mandelate racemase / muconate lactonizing enzyme family signatures
Mandelate racemase (EC 5.1.2.2) (MR) and muconate lactonizing enzyme(EC 5.5.1.1) (MLE) are two bacterial enzymes involved in aromatic acid catabolism. They catalyze mechanistically distinct reactions yet they are related at the level of their primary, quaternary (homooctamer) and tertiary structures [1,2].A number of other proteins also seem to be evolutionary related to these two enzymes. These are: - The various plasmid-encoded chloromuconate

cycloisomerases (EC 5.5.1.7). - Escherichia coli protein rspA [3], rspA seems to be involved in the degradation of homoserine lactone (HSL) or of one of its metabolite. - Escherichia coli hypothetical protein ycjG. - Escherichia coli hypothetical protein yidU. - A hypothetical protein from Streptomyces ambofaciens [4]. Two signature patterns have been developed for these enzymes; both contain conserved acidic residues. The second pattern contains an aspartate and a glutamate which are ligands for either a magnesium ion (in MR) or a manganese ion (inMLE).
Consensus pattern: A-x-[SAGCN]-[SAG]-[LIVM]-[DEQ]-x-A-[LA]-x-[DE]-[LIA]-x-[GA]-[KRQ]-x(4)-[PSA]-[LIV]-x(2)-L-[LIVMF]-G-
Consensus pattern: [LIVF]-x(2)-D-x-[NH]-x(7)-[ACL]-x(6)-[LIVMF]-x(7)-[LIVM]- E-[DENQ]-P [D and E bind a divalent metal ion]-

[1] Neidhart D.J., Kenyon G.L., Gerlt J.A., Petsko G.A. Nature 347:692-694(1990).

[2] Petsko G.A., Kenyon G.L., Gerlt J.A., Ringe D., Kozarich J.W. Trends Biochem. Sci. 18:372-376(1993).

[3] Huisman G.W., Kolter R. Science 265:537-539(1994).

[4] Schneider D., Aigle B., Leblond P., Simonet J.M., Decaris B. J. Gen. Microbiol. 139:2559-2567(1993).

344.Merozoite Surface Antigen 2 (MSA-2) family Thomas AW, Carr DA, Carter JM, Lyon JA, Mol Biochem Parasitol 1990;43:211-220.

345. MSP (Major sperm protein) domain.
Major sperm proteins are involved in sperm motility. These proteins oligomerise to form filaments. Partial matches to this domain are also found in other non MSP proteins. These include Swiss:P40075 and Swiss:P34593.

[1] Bullock TL, Roberts TM, Stewart M, J Mol Biol 1996;263:284-296. [2] King KL, Stewart M, Roberts TM, Seavy M, J Cell Sci 1992;101:847-857.

346. (Matrix) Viral matrix protein. Found in Morbillivirus and paramyxovirus, pneumovirus. Number of members: 105

347. O-methyltransferase (methyltransf)
This family includes a range of O-methyltransferases. These enzymes utilise S-adenosyl methionine.

[1] Keller NP, Dischinger HC, Bhatnagar D, Cleveland TE, Ullah AH, Appl Environ Microbiol 1993;59:479-484.

348. Magnesium chelatase, subunit ChlI
Magnesium-chelatase is a three-component enzyme that catalyses the insertion of Mg2+ into protoporphyrin IX. This is the first unique step in the synthesis of (bacterio)chlorophyll. Due to this, it is thought that Mg-chelatase has an important role in channeling inter- mediates into the (bacterio)chlorophyll branch in response to conditions suitable for photosynthetic growth. ChlI and BchD have molecular weight between 38-42 kDa.

[1] Walker CJ, Willows RD, Biochem J 1997;327:321-333.
[2] Petersen BL, Jensen PE, Gibson LC, Stummann BM, Hunter CN, Henningsen KW, J Bacteriol 1998;180: 699-704.

349. Plasmid recombination enzyme (Mob_Pre)
With some plasmids, recombination can occur in a site specific manner that is independent of RecA. In such cases, the recombination event requires another protein called Pre. Pre is a plasmid recombination enzyme. This protein is: also known as Mob (conjugative mobilization).

[1] Priebe SD, Lacks SA, J Bacteriol 1989;171:4778-4784.

350. Monooxygenase
This family includes diverse enzymes that utilise FAD.

[1] Gatti DL, Palfey BA, Lah MS, Entsch B, Massey V, Ballou DP, Ludwig ML, Science 1994;266:110-114.

351. Mov34 family

Members of this family are found in proteasome regulatory subunits, eukaryotic initiation factor 3 (eIF3) subunits and regulators of transcription factors.

[1] Aravind L, Ponting CP, Protein Sci 1998;7:1250-1254.
[2] Hershey JW, Asano K, Naranda T, Vornlocher HP, Hanachi P, Merrick WC, Biochimie 1996;78:903-907.

352. Myc amino-terminal region (Myc_N_term)
The myc family belongs to the basic helix-loop-helix leucine zipper class of transcription factors, see HLH. Myc forms a heterodimer with Max, and this complex regulates cell growth through direct activation of genes involved in cell replication [2].

[1] Facchini LM, Penn LZ, FASEB J 1998;12:633-651.
[2] Grandori C, Eisenman RN, Trends Biochem Sci 1997;22:177-181.

353. (Metallothio_2) Metallothionein. Members of this family are metallothioneins. These proteins are cysteine rich proteins that bind to heavy metals. Members of this family appear to be closest to Class II metallothioneins, seed metalthio. Number of members: 55

[1] Medline: 98267202. Characterization of gene repertoires at mature stage of citrus fruits through random sequencing and analysis of redundant metallothionein-like genes expressed during fruit development. Moriguchi T, Kita M, Hisada S, Endo-Inagaki T, Omura M; Gene 1998;211:221-227.

354. MAGE family
The MAGE (melanoma antigen-encoding gene) family are expressed in a wide variety of tumors but not in normal cells, with the exception of the male germ cells, placenta, and, possibly, cells of the developing embryo. The cellular function of this family is unknown.

[1] McCurdy DK, Tai LQ, Nguyen J, Wang Z, Yang HM, Udar N, Naiem F, Concannon P, Gatti RA; Mol Genet Metab 1998;63:3-13.

355. Malic enzymes signature. Malic enzymes, or malate oxidoreductases, catalyze the oxidative decarboxylation of malate into pyruvate important for a wide range of metabolic pathways. There are three related forms of malic enzyme [1,2,3]: - NAD-dependent malic enzyme (EC 1.1.1.38), which uses preferentially NAD and has the ability to decarboxylate oxaloacetate (OAA). It is found in bacteria and insects. - NAD-dependent malic enzyme (EC 1.1.1.39), which uses preferentially NAD and is unable to decarboxylate OAA. It is found in the mitochondrial matrix of plants and is a heterodimer of highly related subunits. - NADP-dependent malic enzyme (EC 1.1.1.40), which has a preference for NADP and has the ability to decarboxylate OAA. This form has been found in fungi, animals and plants. In mammals, there are two isozymes: one, mitochondrial and the other, cytosolic. Plants also have two isozymes: chloroplastic and cytosolic. There are two other proteins which are closely structurally related to malicenzymes: - Escherichia coli protein sfcA, whose function is not yet known but which could be an NAD or NADP-dependent malic enzyme. - Yeast hypothetical protein YKL029c, a probable malic enzyme. There are three well conserved regions in the enzyme sequences. Two of them seem to be involved in binding NAD or NADP. The significance of the third one, located in the central part of the enzymes, is not yet known. This region has been developed as a signature pattern for these enzymes.
Consensus pattern: F-x-[D V]-D-x(2)-G-T-[GSA]-x-[IV]-x-[LIVMA]-[GAST](2)-[LIVMF] (2)-

[1] Artus N.N., Edwards G.E. FEBS Lett. 182:225-233(1985).
[2] Loeber G., Infante A.A., Maurer-Fogy L, Krystek E., Dworkin M.B. J. Biol. Chem. 266:3016-3021(1991).
[3] Long J.J., Wang J.-L., Berry J.O. J. Biol. Chem. 269:2827-2833(1994).

356. (matrixin)
Matrixins cysteine switch (aka peptidase_M10)
Mammalian extracellular matrix metalloproteinases (EC 3.4.24.-), also known as matrixins

[1] (see <PDOC00129>), are zinc-dependent enzymes. They are secreted by cells in an inactive form (zymogen) that differs from the mature enzyme by the presence of an N-terminal propeptide. A highly conserved octapeptide is found two residues downstream of the C-terminal end of the propeptide. This region has been shown to be involved in autoinhibition of matrixins [2,3]; a cysteine within the octapeptide chelates the active site zinc ion,

thus inhibiting the enzyme. This region has been called the 'cysteine switch' or 'autoinhibitor region'.

A cysteine switch has been found in the following zinc proteases:

- MMP-1 (EC 3.4.24.7) (interstitial collagenase).
- MMP-2 (EC 3.4.24.24) (72 Kd gelatinase).
- MMP-3 (EC 3.4.24.17) (stromelysin-1).
- MMP-7 (EC 3.4.24.23) (matrilysin).
- MMP-8 (EC 3.4.24.34) (neutrophil collagenase).
- MMP-9 (EC 3.4.24.35) (92 Kd gelatinase).
- MMP-10 (EC 3.4.24.22) (stromelysin-2).
- MMP-11 (EC 3.4.24.-) (stromelysin-3).
- MMP-12 (EC 3.4.24.65) (macrophage metalloelastase).
- MMP-13 (EC 3.4.24.-) (collagenase 3).
- MMP-14 (EC 3.4.24.-) (membrane-type matrix metalliproteinase 1).
- MMP-15 (EC 3.4.24.-) (membrane-type matrix metalliproteinase 2).
- MMP-16 (EC 3.4.24.-) (membrane-type matrix metalliproteinase 3).
- Sea urchin hatching enzyme (EC 3.4.24.12) (envelysin) [4].
- Chlamydomonas reinhardtii gamete lytic enzyme (GLE) [5].

Consensus patternP-R-C-[GN]-x-P-[DR]-[LIVSAPKQ] [C chelates the zinc ion] Sequences known to belong to this class detected by the pattern ALL, except for cat MMP-7 and mouse MMP-11.

[1] Woessner J. Jr. FASEB J. 5:2145-2154(1991).
[2] Sanchez-Lopez R., Nicholson R., Gesnel M.C., Matrisian L.M., Breathnach R. J. Biol. Chem. 263: 11892-11899(1988).
[3] Park A.J., Matrisian L.M., Kells A.F., Pearson R., Yuan Z., Navre M. J. Biol. Chem. 266:1584-1590(1991).
[4] Lepage T., Gache C. EMBO J. 9:3003-3012(1990).
[5] Kinoshita T., Fukuzawa H., Shimada T., Saito T., Matsuda Y. Proc. Natl. Acad. Sci. U.S.A. 89:4693-4697 (1992).

357. Vertebrate metallothioneins signature (metalthio)
Metallothioneins (MT) [1,2,3] are small proteins which bind heavy metals such as zinc, copper, cadmium, nickel, etc., through clusters of thiolate bonds. MT's occur throughout the animal kingdom and are also found in higher plants, fungi and some prokaryotes. On the basis of structural relationships MT's have been subdivided into three classes. Class I includes mammalian MT's as well as MT's from crustacean and molluscs, but with clearly related primary structure. Class II groups together MT's from various species such as sea urchins, fungi, insects and cyanobacteria which display none or only very distant correspondence to class I MT's. Class III MT's are atypical polypeptides containing gamma-glutamylcysteinyl units. Vertebrate class I MT's are proteins of 60 to 68 amino acid residues, 20 of these residues are cysteines that bind to 7 bivalent metal ions. As a signature pattern a region that spans 19 residues and which contains seven of the metal-binding cysteines was chosen, this region is located in the N-terminal section of class-I MT's.
Consensus pattern: C-x-C-[GSTAP]-x(2)-C-x-C-x(2)-C-x-C-x(2)-C-x-K-

[1] Hamer D.H. Annu. Rev. Biochem. 55:913-951(1986).
[2] Kagi J.H.R., Schaffer A. Biochemistry 27:8509-8515(1988).
[3] Binz P.-A. Thesis, 1996, University of Zurich.

358. Mitochondrial energy transfer proteins signature (mito_ carr)
Different types of substrate carrier proteins involved in energy transfer are found in the inner mitochondrial membrane [1 to 5]. These are: - The ADP,ATP carrier protein (AAC) (ADP/ATP translocase) which exports ATP into the cytosol and imports ADP into the mitochondrial matrix. The sequence of AAC has been obtained from various mammalian, plant and fungal species. - The 2-oxoglutarate/malate carrier protein (OGCP), which exports 2-oxoglutarate into the cytosol and imports malate or other dicarboxylic acids into the mitochondrial matrix. This protein plays an important role in several metabolic processes such as the malate/aspartate and the oxoglutarate/isocitrate shuttles. - The phosphate carrier protein, which transports phosphate groups from the cytosol into the mitochondrial matrix. - The brown fat uncoupling protein (UCP) which dissipates oxidative energy into heat by transporting protons from the cytosol into the mitochondrial matrix. - The tricarboxylate transport protein (or citrate transport protein) which is

involved in citrate-H+/malate exchange. It is important for the bioenergetics of hepatic cells as it provides a carbon source for fatty acid and sterol biosyntheses, and NAD for the glycolytic pathway. - The Grave's disease carrier protein (GDC), a protein of unknown function recognized by IgG in patients with active Grave's disease. - Yeast mitochondrial proteins MRS3 and MRS4. The exact function of these proteins is not known. They suppress a mitochondrial splice defect in the first intron of the COB gene and may act as carriers, exerting their suppressor activity by modulating solute concentrations in the mitochondrion. - Yeast mitochondrial FAD carrier protein (gene FLX1). - Yeast protein ACR1 [6], which seems essential for acetyl-CoA synthetase activity. - Yeast protein PET8. - Yeast protein PMT. - Yeast protein RIM2. - Yeast protein YHM1/SHM1. - Yeast protein YMC1. - Yeast protein YMC2. - Yeast hypothetical proteins YBR291c, YEL006w, YER053c, YFR045w, YHR002w, and YIL006w. - Caenorhabditis elegans hypothetical protein K11H3.3.Two other proteins have been found to belong to this family, yet are not localized in the mitochondrial inner membrane: - Maize amyloplast Brittle-1 protein. This protein, found in the endosperm of kernels, could play a role in amyloplast membrane transport. - Candida boidinii peroxisomal membrane protein PMP47 [7]. PMP47 is an integral membrane protein of the peroxisome and it may play a role as a transporter. These proteins all seem to be evolutionary related. Structurally, they consistof three tandem repeats of a domain of approximately one hundred residues. Each of these domains contains two transmembrane regions. As a signature pattern, one of the most conserved regions in the repeated domain was selected, located just after the first transmembrane region.
Consensus pattern: P-x-[DE]-x-[LIVAT]-[RK]-x-[LRH]-[LIVMFY]-[QGAIVM]-

[1] Klingenberg M. Trends Biochem. Sci. 15:108-112(1990).
[2] Walker J.E. Curr. Opin. Struct. Biol. 2:519-526(1992).
[3] Kuan J., Saier M.H. Jr. CRC Crit. Rev. Biochem. 28:209-233(1993).
[4] Kuan J., Saier M.H. Jr. Res. Microbiol. 144:671-672(1993).
[5] Nelson D.R., Lawson J.E., Klingenberg M., Douglas M.G. J. Mol. Biol. 230:1159-1170(1993).
[6] Palmieri F. FEBS Lett. 346:48-54(1994).
[7] Jank B., Habermann B., Schweyen R.J., Link T.A. Trends Biochem. Sci. 18:427-428(1993).

359. Prokaryotic molybdopterin oxidoreductases signatures (molybdopterin)

A number of different prokaryotic oxidoreductases that require and bind amolybdopterin cofactor have been shown [1,2,3] to share a number of regions of sequence similarity. These enzymes are: - Escherichia coli respiratory nitrate reductase (EC 1.7.99.4). This enzyme complex allows the bacteria to use nitrate as an electron acceptor during anaerobic growth. The enzyme is composed of three different chains: alpha, beta and gamma. The alpha chain (gene narG) is the molybdopterin-binding subunit. Escherichia coli encodes for a second, closely related, nitrate reductase complex which also contains a molybdopterin-binding alpha chain (gene narZ). - Escherichia coli anaerobic dimethyl sulfoxide reductase (DMSO reductase). DMSO reductase is the terminal reductase during anaerobic growth on various sulfoxide and N-oxide compounds. DMSO reductase is composed of three chains: A, B and C. The A chain (gene dmsA) binds molybdopterin. - Escherichia coli biotin sulfoxide reductases (genes bisC and bisZ). This enzyme reduces a spontaneous oxidation product of biotin, BDS, back to biotin. It may serve as a scavenger, allowing the cell to use biotin sulfoxide as a biotin source. - Methanobacterium formicicum formate dehydrogenase (EC 1.2.1.2). The alpha chain (gene fdhA) of this dimeric enzyme binds a molybdopterin cofactor. - Escherichia coli formate dehydrogenases -H (gene fdhF), -N (gene fdnG) and -O (gene fdoG). These enzymes are responsible for the oxidation of formate to carbon dioxide. In addition to molybdopterin, the alpha (catalytic) subunit also contains an active site, selenocysteine. - Wolinella succinogenes polysulfide reductase chain. This enzyme is a component of the phosphorylative electron transport system with polysulfide as the terminal acceptor. It is composed of three chains: A, B and C. The A chain (gene psrA) binds molybdopterin. - Salmonella typhimurium thiosulfate reductase (gene phsA). - Escherichia coli trimethylamine-N-oxide reductase (EC 1.6.6.9) (gene torA) [4]. - Nitrate reductase (EC 1.7.99.4) from Klebsiella pneumoniae (gene nasA), Alcaligenes eutrophus, Escherichia coli, Rhodobacter sphaeroides, Thiosphaera pantotropha (gene napA), and Synechococcus PCC 7942 (gene narB).These proteins range from 715 amino acids (fdhF) to 1246 amino acids (narZ) insize. Three signature patterns for these enzymes were derived. The first is based on a conserved region in the N-terminal section and contains two cysteine residues perhaps involved in binding the molybdopterin cofactor. It should be noted that this region is not present in bisC. The second pattern is derived from a conserved region located in the central part of these enzymes.
Consensus pattern: [STAN]-x-[CH]-x(2,3)-C-[STAG]-[GSTVMF]-x-C-x-[LIVMFYW]-x-[LIVMA]-x(3,4)-[DENQKHT]-
Consensus pattern: [STA]-x-[STAC](2)-x(2)-[STA]-D-[LIVMY](2)-L-P-x-[STAC](2)-x(2)-E-
Consensus pattern: A-x(3)-[GDT]-I-x-[DNQTK]-x-[DEA]-x-[LIVM]-x-[LIVMC]-x- [NS]-x(2)-[GS]-x(5)-A-x-[LIVM]-[ST]-

[1] Wootton J.C., Nicolson R.E., Cock J.M., Walters D.E., Burke J.F., Doyle W.A., Bray R.C. Biochim. Biophys.

Acta 1057:157-185(1991).
[2] Bilous P.T., Cole S.T., Anderson W.F., Weiner J.H. Mol. Microbiol. 2:785-795(1988).
[3] Trieber C.A., Rothery R.A., Weiner J.H. J. Biol. Chem. 269:7103-7109(1994).
[4] Mejean V., Lobbi-Nivol C., Lepelletier M., Giordano G., Chippaux M., Pascal M.-C. Mol. Microbiol. 11: 1169-1179(1994).

360. Bacterial mutT domain signature
The bacterial mutT protein is involved in the GO system [1] responsible for removing an oxidatively damaged form of guanine (8-hydroxyguanine or7,8-dihydro-8-oxoguanine) from DNA and the nucleotide pool. 8-oxo-dGTP is inserted opposite to dA and dC residues of template DNA with almost equal efficiency thus leading to A.T to G.C transversions. MutT specifically degrades 8-oxo-dGTP to the monophosphate with the concomitant release of pyrophosphate. MutT is a small protein of about 12 to 15 Kd. It has been shown [2,3] that a region of about 40 amino acid residues, which is found in the N-terminal part of mutT, can also be found in a variety of other prokaryotic, viral, and eukaryotic proteins. These proteins are:

- Streptomyces pneumoniae mutX.
- A mutT homolog from plasmid pSAM2 of Streptomyces ambofaciens.
- Bartonella bacilliformis invasion protein A (gene invA).
- Escherichia coli dATP pyrophosphohydrolase.
- Protein D250 from African swine fever viruses.
- Proteins D9 and D10 from a variety of poxviruses.
- Mammalian 7,8-dihydro-8-oxoguanine triphosphatase (EC 3.1.6.-) [4].
- Mammalian diadenosine 5',5'''-P1,P4-tetraphosphate asymmetrical hydrolase (Ap4Aase) (EC 3.6.1.17) [5], which cleaves A-5'-PPPP-5'A to yield AMP and ATP.
- A protein encoded on the antisense RNA of the basic fibroblast growth factor gene in higher vertebrates.
- Yeast protein YSA1.
- Escherichia coli hypothetical protein yfaO.
- Escherichia coli hypothetical protein ygdU and HI0901, the corresponding Haemophilus influenzae protein.
- Escherichia coli hypothetical protein yjaD and HI0432, the corresponding Haemophilus influenzae protein.
- Escherichia coli hypothetical protein yrfE.
- Bacillus subtilis hypothetical protein yqkG.
- Bacillus subtilis hypothetical protein yzgD.
- Yeast hypothetical protein YGL067w.

It is proposed [2] that the conserved domain could be involved in the active center of a family of pyrophosphate-releasing NTPases. As a signature pattern the core region of the domain was selected; it contains four conserved glutamate residues.
Consensus pattern: G-x(5)-E-x(4)-[STAGC]-[LIVMAC]-x-R-E-[LIVMFT]-x-E-E-

[1] Michaels M.L., Miller J.H. J. Bacteriol. 174:6321-6325(1992).
[2] Koonin E.V. Nucleic Acids Res. 21:4847-4847(1993).
[3] Mejean V., Salles C., Bullions M.J., Bessman M.J., Claverys J.-P. Mol. Microbiol. 11:323-330(1994).
[4] Sakumi K., Furuichi M., Tsuzuki T., Kakuma T., Kawabata S., Maki H., Sekiguchi M. J. Biol. Chem. 268: 23524-23530(1993).
[5] Thorne N.M.H., Hankin S., Wilkinson M.C., Nunez C., Barraclough R., McLennan A.G. Biochem. J. 311: 717-721(1995).

361. Myb DNA-binding domain repeat signatures
The retroviral oncogene v-myb , and its cellular counterpart c-myb, encodenuclear DNA-binding proteins that specifically recognize the sequence YAAC(G/T)G [1]. The myb family also includes the following proteins: - Drosophila D-myb [2]. - Vertebrate myb-like proteins A-myb and B-myb [3]. - Maize C1 protein, a trans-acting factor which controls the expression of genes involved in anthocyanin biosynthesis. - Maize P protein [4], a trans-acting factor which regulates the biosynthetic pathway of a flavonoid-derived pigment in certain floral tissues. - Arabidopsis thaliana protein GL1 [5], required for the initiation of differentiation of leaf hair cells (trichomes). - A number of myb/c1-related proteins in maize and barley, whose roles are not yet known [4]. - Yeast BAS 1 [7], a transcriptional activator for the HIS4 gene. - Yeast REB 1 [8], which recognizes sites within both the enhancer and the promoter of rRNA transcription, as well as upstream of many genes transcribed by RNA polymerase II. - Fission yeast cdc5, a possible transcription factor whose activity is required for cell cycle progression and growth during G2. - Fission yeast mybl,

which regulates telomere length and function. - Yeast hypothetical protein YMR213w.One of the most conserved regions in all of these proteins is a domain of 160amino acids. It consists of three tandem repeats of 51 to 53 amino acids. In myb, this repeat region has been shown [9] to be involved in DNA-binding. The major part of the first repeat is missing in retroviral v-myb sequences and in plant myb-related proteins. Yeast REB 1 differs from the other proteins in this family in having a single myb-like domain. As shown in the following schematic representation, two signature patterns for myb-like domains were developed; the first is located in the N-terminal section, the second spans the C-terminal extremity of the domain. xxxxxxxxxWxxxEDxxxxxxxxxxxxxxWxxIxxxxxxRxxxxxxxxWxxxx ********* ************************'*' : Position of the patterns.

Consensus pattern: W-[ST]-x(2)-E-[DE]-x(2)-[LIV]-

Consensus pattern: W-x(2)-[LI]-[SAG]-x(4,5)-R-x(8)-[YW]-x(3)-[LIVM]-

Note: this pattern detects the three copies of the domain in myb, d-myb, A-myb and B-myb; the second of the two complete copies of plant myb-related proteins, and the last two copies of yeast BAS1

[1] Biednkapp H., Borgmeyer U., Sippel A.E., Klempnauer K.-H. Nature 335:835-837(1988).
[2] Peters C.W.B., Sippel A.E., Vingron M., Klempnauer K.-H. EMBO J. 6:3085-3090(1987).
[3] Nomura N., Takahashi M., Matsui M., Ishii S., Date T., Sasamoto S., Ishizaki R. Nucleic Acids Res. 16: 11075-11090(1988).
[4] Grotewold E., Athma P., Peterson T. Proc. Natl. Acad. Sci. U.S.A. 88:4587-4591(1991).
[5] Oppenheimer D.G., Herman P.L., Sivakumaran S., Esch J., Marks M.D. Cell 67:483-493(1991).
[6] Marocco A., Wissenbach M., Becker D., Paz-Ares J., Saedler H., Salamini F., Rohde W. Mol. Gen. Genet. 216:183-187(1989).
[7] Tice-Baldwin K., Fink G.R., Arndt K.T. Science 246:931-935(1989).
[8] Ju Q., Morrow B.E., Warner J.R. Mol. Cell. Biol. 10:5226-5234(1990).
[9] Klempnauer K.-H., Sippel A.E. EMBO J. 6:2719-2725(1987).

362. NAD-dependent glycerol-3-phosphate dehydrogenase signature

NAD-dependent glycerol-3-phosphate dehydrogenase (EC 1.1.1.8) (GPD) catalyzes the reversible reduction of dihydroxyacetone phosphate to glycerol-3- phosphate. It is a eukaryotic cytosolic homodimeric protein of about 40 Kd. As a signature pattern a glycine-rich region that is probably [1] involved in NAD-binding was selected.

Consensus pattern: G-[AT]-[LIVM]-K-[DN]-[LIVM](2)-A-x-[GA]-x-G-[LIVMF]-x- [DE]-G-[LIVM]-x-[LIVMFYW]-G-x-N-

[1] Otto J., Argos P., Rossmann M.G. Eur. J. Biochem. 109:325-330(1980).

363. Nucleosome assembly protein (NAP)

It is thought that NAPs may be involved in regulating gene expression as a result of histone accessibility [1].

[1] Rodriguez P, Munroe D, Prawitt D, Chu LL, Bric E, Kim J, Reid LH, Davies C, Nakagama H, Loebbert R, Winterpacht A, Petruzzi MJ, Higgins MJ, Nowak N, Evans G, Shows T, Weissman BE, Zabel B, Housman DE, Pelletier J, Genomics 1997;44:253-265.
[2] Schnieders F, Dork T, Arnemann J, Vogel T, Werner M, Schmidtke J; Hum Mol Genet 1996;5:1801-1807.

364. NB-ARC domain van der Biezen EA, Jones JD, Curr Biol 1998;8:226-227.

365. Nucleoside diphosphate kinases active site

Nucleoside diphosphate kinases (EC 2.7.4.6) (NDK) [1] are enzymes required for the synthesis of nucleoside triphosphates (NTP) other than ATP. They provide NTPs for nucleic acid synthesis, CTP for lipid synthesis, UTP for polysaccharide synthesis and GTP for protein elongation, signal transduction and microtubule polymerization. In eukaryotes, there seems to be a small family of NDK isozymes each of which acts in a different subcellular compartment and/or has a distinct biological function. Eukaryotic NDK isozymes are hexamers of two highly related chains (Aand B) [2]. By random association (A6, A5B...AB5, B6), these two kinds of chain form isoenzymes differing in their isoelectric point. NDK are proteins of 17 Kd that act via a ping-pong mechanism in which a histidine residue is phosphorylated, by transfer of the terminal phosphate group from ATP. In the presence of magnesium, the phosphoenzyme can transfer its phosphate group to any NDP, to produce an NTP.NDK isozymes have been sequenced from prokaryotic and eukaryotic sources. It has also been shown [3] that the Drosophila awd (abnormal wing discs) protein, is a microtubule-associated NDK. Mammalian NDK is also known as metastasis inhibition factor nm23.The sequence of NDK has been highly conserved through evolution. There is a single histidine residue conserved in all known NDK isozymes, which is involved in the catalytic mechanism [2]. Our signature pattern contains this residue.

Consensus pattern: N-x(2)-H-[GA]-S-D-[SA]-[LIVMPKNE] [H is the putative active site residue]-

[1] Parks R., Agarwal R. (In) The Enzymes (3rd edition) 8:307-334(1973).
[2] Gilles A.-M., Presecan E., Vonica A., Lascu I. J. Biol. Chem. 266:8784-8789(1991).
[3] Biggs J., Hersperger E., Steeg P.S., Liotta L.A., Shearn A. Cell 63:933-940(1990).

366. Nitrite and sulfite reductases iron-sulfur/siroheme-binding site (NIR_SIR)
Nitrite reductases (NiR) [1] catalyze the reduction of nitrite into ammonium, the second step in the assimilation of nitrate. There are two types of NiR: the higher plant chloroplastic form of NiR (EC 1.7.7.1) is a monomeric protein that uses reduced ferredoxin as the electron donor; while fungal and bacterial NiR (EC 1.6.6.4) are homodimeric proteins that uses NAD(P)H as the electron donor. Both forms of NiR contain a siroheme-Fe and iron-sulfur centers. Sulfite reductase (NADPH) (EC 1.8.1.2) (SIR) [2] is the bacterial enzyme that catalyzes the reduction of sulfite to sulfide. SIR is an oligomeric enzyme with a subunit composition of alpha(8)-beta(4), the alpha component is a flavoprotein (SIR-FP), while the beta component is a siroheme, iron-sulfurprotein (SIR-HP).Sulfite reductase (ferredoxin) (EC 1.8.7.1) [3] is a cyanobacterial and plant monomeric enzyme that also catalyzes the reduction of sulfite to sulfide. Anaerobic sulfite reductase (EC 1.8.1.-) (ASR) [4], a bacterial enzyme that catalyzes the NADH-dependent reduction of sulfite to sulfide. ASR is an oligomeric enzyme composed of three different subunits. The C component (geneasrC) seems to be a siroheme, iron-sulfur protein. These enzymes share a region of sequence similarity in their C-terminal half; this region which spans about 80 amino acids includes four conserved cysteine residues. Two of the Cys are grouped together at the beginning of the domain, and the two others are grouped in the middle of the domain. The cysteines are involved in the binding of the iron-sulfur center; the last one also binds the siroheme group [2]. A signature pattern from the region around the second cluster of cysteines was derived.
Consensus pattern: [STV]-G-C-x(3)-C-x(6)-[DE]-[LIVMF]-[GAT]-[LIVMF] [The two C's are ison-sulfur ligands]-

[1] Campbell W.H., Kinghorn J.R. Trends Biochem. Sci. 15:315-319(1990).
[2] Crane B.R., Siegel L.M., Getzoff E.D. Science 270:59-67(1995).
[3] Gisselmann G., Klausmeier P., Schwenn J.D. Biochim. Biophys. Acta 1144:102-106(1993).
[4] Huang C.J., Barrett E.L. J. Bacteriol. 173:1544-1553(1991).

367. (NMT) Myristoyl-CoA:protein N-myristoyltransferase signatures. Myristoyl-CoA: protein N-myristoyltransferase (EC 2.1.97) (Nmt) [1] is the enzyme responsible for transferring a myristate group on the N-terminal glycine of a number of cellular eukaryotic and viral proteins. Nmt is a monomeric protein of about 50 to 60 Kd whose sequence appears to be well conserved. Two highly conserved regions have been developed as signature patterns. The first one is located in the central section, the second in the C-terminal part.
Consensus pattern: E-I-N-F-L-C-x-H-K-
Consensus pattern: K-F-G-x-G-D-G-

[1] Rudnick D.A., McWherter C.A., Gokel G.W., Gordon J.I. Adv. Enzymol. 67:375-430(1993).

368. ADP-glucose pyrophosphorylase signatures (NTP_transferase)
ADP-glucose pyrophosphorylase (glucose-1-phosphate adenylyltransferase) [1,2](EC 2.7.7.27) catalyzes a very important step in the biosynthesis of alpha 1,4-glucans (glycogen or starch) in bacteria and plants: synthesis of the activated glucosyl donor, ADP-glucose, from glucose-1-phosphate and ATP.ADP-glucose pyrophosphorylase is a tetrameric allosterically regulated enzyme. It is a homotetramer in bacteria while in plant chloroplasts and amyloplasts, it is a heterotetramer of two different, yet evolutionary related, subunits. There are a number of conserved regions in the sequence of bacterial and plant ADP-glucose pyrophosphorylase subunits. Three of these regions were selected as signature patterns. The first two are N-terminal and have been proposed to be part of the allosteric and/or substrate-binding sites in the Escherichia coli enzyme (gene glgC). The third pattern corresponds to a conserved region in the central part of the enzymes.
Consensus pattern: [AG]-G-G-x-G-[STK]-x-L-x(2)-L-[TA]-x(3)-A-x-P-A-[LV] -
Consensus pattern: W-[FY]-x-G-[ST]-A-[DNSH]-[AS]-[LIVMFYW]-
Consensus pattern: [APV]-[GS]-M-G-[LIVMN]-Y-[IVC]-[LIVMFY]-x(2)-[DENPHK] -

[1] Nakata P.A., Greene T.W., Anderson J.M., Smith-White B.J., Okita T.W., Preiss J. Plant Mol. Biol. 17: 1089-1093(1991).
[2] Preiss J., Ball K., Hutney J., Smith-White B.J., Li. L., Okitsa T.W. Pure Appl. Chem. 63:535-544(1991).

369. Sodium/hydrogen exchanger family

Na/H antiporters are key transporters in maintaining the pH of actively metabolizing cells. The molecular mechanisms of antiport are unclear. These antiporters contain 10-12 transmembrane regions (M) at the amino-terminus and a large cytoplasmic region at the carboxyl terminus. The transmembrane regions M3-M12 share identity with other members of the family. The M6 and M7 regions are highly conserved. Thus, this is thought to be the region that is involved in the transport of sodium and hydrogen ions. The cytoplasmic region has little similarity throughout the family.

[1] Dibrov P, Fliegel L; FEBS Lett 1998;424:1-5.
[2] Orlowski J, Grinstein S; J Biol Chem 1997;272:22373-22376.
[3] Numata M, Petrecca K, Lake N, Orlowski J; J Biol Chem 1998;273:6951-6959.

370. Sodium:sulfate symporter family signature (Na_sulph_symp)
Integral membrane proteins that mediate the intake of a wide variety of molecules with the concomitant uptake of sodium ions (sodium symporters) canbe grouped, on the basis of sequence and functional similarities into a number of distinct families. One of these families currently consists of the following proteins: - Mammalian sodium/sulfate cotransporter [1]. - Mammalian renal sodium/dicarboxylate cotransporter [2], which transports succinate and citrate. - Mammalian intestinal sodium/dicarboxylate cotransporter. - Chlamydomonas reinhardtii putative sulfur deprivation response regulator SAC1 [3]. - Caenorhabditis elegans hypothetical proteins B0285.6, F31F6.6, K08E5.2 and R107.1. - Escherichia coli hypothetical protein yfbS. - Haemophilus influenzae hypothetical protein HI0608. - Synechocystis strain PCC 6803 hypothetical protein s110640. - Methanococcus jannaschii hypothetical protein MJ0672.These transporters are proteins of from 430 to 620 amino acids which are highly hydrophobic and which probably contain about 12 transmembrane regions. As a signature pattern, a conserved region was selected which is located in or near the penultimate transmembrane region.
Consensus pattern: [STACP]-S-x(2)-F-x(2)-P-[LIVM]-[GSA]-x(3)-N-x-[LIVM]-V-

[1] Markovich D., Forgo J., Stange G., Biber J., Murer H. Proc. Natl. Acad. Sci. U.S.A. 90:8073-8077(1993).
[2] Pajor A.M. Am. J. Physiol. 270:642-648(1996).
[3] Davies J.P., Yildiz F.H., Grossman A. EMBO J. 15:2150-2159(1996).

371. NifU-like domain
This is an alignment of the carboxy-terminal domain. This is the only common region between the NifU protein from nitrogen-fixing bacteria and rhodobacterial species. The biochemical function ofNifU is unknown [1].
Ouzounis C, Bork P, Sander C, Trends Biochem Sci 1994;19:199-200.

372. Nitrilases / cyanide hydratase signatures
Nitrilases (EC 3.5.5.1) are enzymes that convert nitriles into their corresponding acids and ammonia. They are widespread in microbes as well as in plants where they convert indole-3-acetonitrile to the hormone indole-3-acetic acid. A conserved cysteine has been shown [1,2] to be essential for enzyme activity; it seems to be involved in a nucleophilic attack on the nitrile carbon atom. Cyanide hydratase (EC 4.2.1.66) converts HCN to formamide. In phytopathogenic fungi, it is used to avoid the toxic effect of cyanide released by wounded plants [3]. The sequence of cyanide hydrolase is evolutionary related to that of nitrilases. Yeast hypothetical proteins YIL164c and YIL165c also belong to this family. As signature patterns for these enzymes, two conserved regions were selected. The first is located in the N-terminal section while the second, which contains the active site cysteine, is located in the central section.
Consensus pattern: G-x(2)-[LIVMFY](2)-x-[IF]-x-E-x(2)-[LIVM]-x-G-Y-P-
Consensus pattern: G-[GAQ]-x(2)-C-[WA]-E-[NH]-x(2)-[PST]-[LIVMFYS]-x-[KR] [C is the active site residue]-

[1] Kobayashi M., Izui H., Nagasawa T., Yamada H. Proc. Natl. Acad. Sci. U.S.A. 90:247-251(1993).
[2] Kobayashi M., Komeda H., Yanaka N., Nagasawa T., Yamada H. J. Biol. Chem. 267:20746-20751(1992).
[3] Wang P., Vanetten H.D. Biochem. Biophys. Res. Commun. 187:1048-1054(1992).

373. NusB family
The NusB protein is involved in the regulation of rRNA biosynthesis by transcriptional antitermination.
Huenges M, Rolz C, Gschwind R, Peteranderl R, Berglechner F, Richter G, Bacher A, Kessler H,Gemmecker G, EMBO J 1998;17:4092-4100.

374. (Neur Chan) Neurotransmitter-gated ion-channels signature
Neurotransmitter-gated ion-channels [1,2,3,4] provide the molecular basis for rapid signal transmission at chemical

synapses. They are post-synapticoligomeric transmembrane complexes that transiently form a ionic channel upon the binding of a specific neurotransmitter. Presently, the sequence of subunits from five types of neurotransmitter-gated receptors are known: - The nicotinic acetylcholine receptor (AchR), an excitatory cation channel. In the motor endplates of vertebrates, it is composed of four different subunits (alpha, beta, gamma and delta or epsilon) with a molar stoichiometry of 2:1:1:1. In neurones, the AchR receptor is composed of two different types of subunits: alpha and non-alpha (also called beta). Nicotinic AchRs are also found in invertebrates. - The glycine receptor, an inhibitory chloride ion channel. The glycine receptor is a pentamer composed of two different subunits (alpha and beta). - The gamma-aminobutyric-acid (GABA) receptor, which is also an inhibitory chloride ion channel. The quaternary structure of the GABA receptor is complex; at least four classes of subunits are known to exist (alpha, beta, gamma, and delta) and there are many variants in each class (for example: six variants of the alpha class have already been sequenced). - The serotonin 5HT3 receptor. Serotonin is a biogenic hormone that functions as a neurotransmitter, a hormone and a mitogen. There are seven major groups of serotonin receptors; six of these groups (SHT1, 5HT2, and 5HT4 to 5HT7) transduce extracellular signal by activating G proteins, while 5HT3 is a ligand-gated cation-specific ion channel which, when activated causes fast, depolarizing responses in neurons. - The glutamate receptor, an excitatory cation channel. Glutamate is the main excitatory neurotransmitter in the brain. At least three different types of glutamate receptors have been described and are named according to their selective agonists (kainate, N-methyl-D-aspartate (NMDA) and quisqualate).All known sequences of subunits from neurotransmitter-gated ion-channels are structurally related. They are composed of a large extracellular glycosylated N-terminal ligand-binding domain, followed by three hydrophobic transmembrane regions which form the ionic channel, followed by an intracellular region of variable length. A fourth hydrophobic region is found at the C-terminal of the sequence. The sequence of subunits from the AchR, GABA, 5HT3, and Gly receptors are clearly evolutionary related and share many regions of sequence similarities. These sequence similarities are either absent or very weak in the Glu receptors. In the N-terminal extracellular domain of AchR/GABA/5HT3/Gly receptors, there are two conserved cysteine residues, which, in AchR, have been shown to form a disulfide bond essential to the tertiary structure of the receptor. A number of amino acids between the two disulfide-bonded cysteines are also conserved. Therefore this region was used as a signature pattern for this subclass of proteins.
Consensus pattern: C-x-[LIVMFQ]-x-[LIVMF]-x(2)-[FY]-P-x-D-x(3)-C [The two C's are linked by a disulfide bond]-

[1] Stroud R.M., McCarthy M.P., Shuster M. Biochemistry 29:11009-11023(1990).
[2] Betz H. Neuron 5:383-392(1990).
[3] Dingledine R., Myers S.J., Nicholas R.A. FASEB J. 4:2632-2645(1990).
[4] Barnard E.A. Trends Biochem. Sci. 17:368-374(1992).

375. Orotidine 5'-phosphate decarboxylase active site
Orotidine 5'-phosphate decarboxylase (EC 4.1.1.23) (OMPdecase) [1,2] catalyzes the last step in the de novo biosynthesis of pyrimidines, the decarboxylation of OMP into UMP. In higher eukaryotes OMPdecase is part, with orotatephosphoribosyltransferase, of a bifunctional enzyme, while the prokaryotic and fungal OMPdecases are monofunctional protein. Some parts of the sequence of OMPdecase are well conserved across species. The best conserved region is located in the N-terminal half of OMPdecases and is centered around a lysine residue which is essential for the catalytic function of the enzyme. This region has been developed as a signature pattern.
Consensus pattern: [LIVMFTA]-[LIVMF]-x-D-x-K-x(2)-D-I-[GP]-x-T-[LIVMTA] [K is the active site residue]-

[1] Jacquet M., Guilbaud R., Garreau H. Mol. Gen. Genet. 211:441-445(1988).
[2] Kimsey H.H., Kaiser D. J. Biol. Chem. 267:819-824(1992).

376. ATP synthase delta (OSCP) subunit signature
ATP synthase (proton-translocating ATPase) (EC 3.6.1.34) [1,2] is a component of the cytoplasmic membrane of eubacteria, the inner membrane of mitochondria, and the thylakoid membrane of chloroplasts. The ATPase complex is composed of an oligomeric transmembrane sector, called CF(0), which acts as a proton channel, and a catalytic core, termed coupling factor CF(1). One of the subunits of the ATPase complex, known as subunit delta in bacteria and chloroplasts or the Oligomycin Sensitivity Conferral Protein (OSCP) in mitochondria, seems to be part of the stalk that links CF(0) to CF(1). It either transmits conformational changes from CF(0) into CF(1) or is involved in proton conduction [3].
The different delta/OSCP subunits are proteins of approximately 200 amino-acid residues - once the transit peptide has been removed in the chloroplast and mitochondrial forms - which show only moderate sequence homology.
The signature pattern used to detect ATPase delta/OSCP subunits is based on a conserved region in the C-terminal section of these proteins.
Consensus pattern: [LIVM]-x-[LIVMFYT]-x(3)-[LIVMT]-[DENQK]-x(2)-[LIVM]-x-[GSA]-G-[LIVMFYGA]-x-[LIVM]-[KRHENQ]-x-[GSEN]

[1] Futai M., Noumi T., Maeda M. Annu. Rev. Biochem. 58:111-136(1989).
[2] Senior A.E. Physiol. Rev. 68:177-231(1988).
[3] Engelbrecht S., Junge W. Biochim. Biophys. Acta 1015:379-390(1990).

377. Aspartate and ornithine carbamoyltransferases signature
Aspartate carbamoyltransferase (EC 2.1.3.2) (ATCase) catalyzes the conversion of aspartate and carbamoyl phosphate to carbamoylaspartate, the second step in the de novo biosynthesis of pyrimidine nucleotides [1]. In prokaryotes ATCase consists of two subunits: a catalytic chain (gene pyrB) and a regulatory chain (gene pyrI), while in eukaryotes it is a domain in a multifunctional enzyme (called URA2 in yeast, rudimentary in Drosophila, and CAD in mammals [2]) that also catalyzes other steps of the biosynthesis of pyrimidines.
Ornithine carbamoyltransferase (EC 2.1.3.3) (OTCase) catalyzes the conversion of ornithine and carbamoyl phosphate to citrulline. In mammals this enzyme participates in the urea cycle [3] and is located in the mitochondrial matrix. In prokaryotes and eukaryotic microorganisms it is involved in the biosynthesis of arginine. In some bacterial species it is also involved in the degradation of arginine [4] (the arginine deaminase pathway). It has been shown [5] that these two enzymes are evolutionary related. The predicted secondary structure of both enzymes are similar and there are some regions of sequence similarities. One of these regions includes three residues which have been shown, by crystallographic studies [6], to be implicated in binding the phosphoryl group of carbamoyl phosphate. This region was selected as a signature for these enzymes.
Consensus pattern: F-x-[EK]-x-S-[GT]-R-T[S, R, and the 2nd T bind carbamoyl phosphate]
-Note: the residue in position 3 of the pattern allows to distinguish between an ATCase (Glu) and an OTCase (Lys).

[1] Lerner C.G., Switzer R.L. J. Biol. Chem. 261:11156-11165(1986).
[2] Davidson J.N., Chen K.C., Jamison R.S., Musmanno L.A., Kern C.B. BioEssays 15:157-164(1993).
[3] Takiguchi M., Matsubasa T., Amaya Y., Mori M. BioEssays 10:163-166(1989).
[4] Baur H., Stalon V., Falmagne P., Luethi E., Haas D. Eur. J. Biochem. 166:111-117(1987).
[5] Houghton J.E., Bencini D.A., O'Donovan G.A., Wild J.R. Proc. Natl. Acad. Sci. U.S.A. 81:4864-4868(1981).
[6] Ke H.-M., Honzatko R.B., Lipscomb W.N. Proc. Natl. Acad. Sci. U.S.A. 81:4037-4040(1984).

378. Oleosins signature Oleosins [1] are the proteinaceous components of plants' lipid storage bodies called oil bodies. Oil bodies are small droplets (0.2 to 1.5 mu-m in diameter) containing mostly triacylglycerol that are surrounded by a phospholipid/ oleosin annulus. Oleosins may have a structural role in stabilizing the lipid body during dessication of the seed, by preventing coalescence of the oil.
They may also provide recognition signals for specific lipase anchorage in lipolysis during seedling growth. Oleosins are found in the monolayer lipid/ water interface of oil bodies and probably interact with both the lipid and phospholipid moieties.
Oleosins are proteins of 16 Kd to 24 Kd and are composed of three domains: an N-terminal hydrophilic region of variable length (from 30 to 60 residues); a central hydrophobic domain of about 70 residues and a C-terminal amphipathic region of variable length (from 60 to 100 residues). The central hydrophobic domain is proposed to be made up of beta-strand structure and to interact with the lipids [2]. It is the only domain whose sequence is conserved and therefore a section from that domain was selected as a signature pattern.
Consensus pattern: [AG]-[ST]-x(2)-[AG]-x(2)-[LIVM]-[SAD]-T-P-[LIVMF](4)-F-S-P-[LIVM](3)-P-A

[1] Murphy D.J., Keen J.N., O'Sullivan J.N., Au D.M.Y., Edwards E.-W., Jackson P.J., Cummins I., Gibbons T., Shaw C.H., Ryan A.J. Biochim. Biophys. Acta 1088:86-94(1991).
[2] Tzen J.T.C., Lie G.C., Huang A.H.C. J. Biol. Chem. 267:15626-15634(1992).

379. (Orbi VP5) Orbivirus outer capsid protein VP5
This paper shows the location of the different capsid proteins and their relation to each other.

[1] Schoehn G, Moss SR, Nuttall PA, Hewat EA; Virology 1997;235:191-200.

380. Orn/DAP/Arg decarboxylases family 2 signatures Pyridoxal-dependent decarboxylases acting on ornithine, lysine, arginine and related substrates can be classified into two different families on the basis of sequence similarities [1,2,3]. The second family consists of:

- Eukaryotic ornithine decarboxylase (EC 4.1.1.17) (ODC). ODC catalyzes the transformation of ornithine into

putrescine.

- Prokaryotic diaminopimelic acid decarboxylase (EC 4.1.1.20) (DAPDC). DAPDC catalyzes the conversion of diaminopimelic acid into lysine; the last step in the biosynthesis of lysine.
- Pseudomonas syringae pv. tabaci protein tabA. tabA is probably involved in the biosynthesis of tabtoxin and is highly similar to DAPDC.
- Bacterial and plant biosynthetic arginine decarboxylase (EC 4.1.1.19) (ADC). ADC catalyzes the transformation of arginine into agmatine, the first step in the biosynthesis of putrescine from arginine.

The above proteins, while most probably evolutionary related, do not share extensive regions of sequence similarities. Two of the conserved regions were selected as signature patterns. The first pattern contains a conserved lysine residue which is known, in mouse ODC [4], to be the site of attachment of the pyridoxal-phosphate group. The second pattern contains a stretch of three consecutive glycine residues and has been proposed to be part of a substrate-binding region [5].
These enzymes are collectively known as group IV decarboxylases [3].
Consensus pattern: [FY]-[PA]-x-K-[SACV]-[NHCLFW]-x(4)-[LIVMF]-[LIVMTA]-x(2)-[LIVMA]-x(3)-[GTE] [K is the pyridoxal-P attachment site]
Consensus pattern: [GS]-x(2,6)-[LIVMSCP]-x(2)-[LIVMF]-[DNS]-[LIVMCA]-G-G-G-[LIVMFY]-[GSTPCEQ]

[1] Bairoch A. Unpublished observations (1993).
[2] Martin C., Cami B., Yeh P., Stragier P., Parsot C., Patte J.-C. Mol. Biol. Evol. 5:549-559(1988).
[3] Sandmeier E., Hale T.I., Christen P. Eur. J. Biochem. 221:997-1002(1994).
[4] Poulin R., Lu L., Ackermann B., Bey P., Pegg A.E. J. Biol. Chem. 267:150-158(1992).
[5] Moore R.C., Boyle S.M. J. Bacteriol. 172:4631-4640(1990).

381. Osteopontin signature Osteopontin is an acidic phosphorylated glycoprotein of about 40 Kd which is abundant in the mineral matrix of bones and which binds tightly to hydroxyapatite [1,2,3]. It is suggested that osteopontin might function as a cell attachment factor and could play a key role in the adhesion of osteoclasts to the mineral matrix of bone.
Osteopontin-K is a kidney protein which is highly similar to osteopontin and probably also involved in cell-adhesion. As a signature pattern a highly conserved region located at the N-terminal extremity of the mature protein was selected.
Consensus pattern: [KQ]-x-[TA]-x(2)-[GA]-S-S-E-E-K

[1] Butler W.T. Connect. Tissue Res. 23:123-36(1989).
[2] Gorski J.P. Calcif. Tissue Int. 50:391-396(1992).
[3] Denhardt D.T., Guo X. FASEB J. 7:1475-1482(1993).

382. Oxysterol-binding protein family signature
A number of eukaryotic proteins that seem to be involved with sterol synthesis and/or its regulation have been found [1] to be evolutionary related:

- Mammalian oxysterol-binding protein (OSBP). A protein of about 800 amino-acid residues that binds a variety of oxysterols: oxygenated derivatives of cholesterol. OSBP seems to play a complex role in the regulation of sterol metabolism.
- Yeast proteins HES1 and KES1; highly related proteins of 434 residues that seem to play a role in ergosterol synthesis.
- Yeast OSH1, a protein of 859 residues that also plays a role in ergosterol synthesis. - Yeast hypothetical protein YHR001w (437 residues).
- Yeast hypothetical protein YHR073w (996 residues).
- Yeast hypothetical protein YKR003w (448 residues).

All these proteins contain a moderately conserved domain of about 250 residues located in the C-terminal half of OBSP, OSH1 and YHR073w and in the central section of the other proteins. As a signature pattern, the best conserved part was selected of this domain, a region that contains a conserved pentapeptide.
Consensus pattern: E-[KQ]-x-S-H-[HR]-P-P-x-[STACF]-A

[1] Jiang B., Brown J.L., Sheraton J., Fortin N., Bussey H. Yeast 10:341-353(1994).

383. FMN oxidoreductase

384. Oxidoreductase FAD/NAD-binding domain
Number of members: 250

[1]
Medline: 92084635
The sequence of squash NADH:nitrate reductase and its relationship to the sequences of other flavoprotein oxidoreductases. A family of flavoprotein pyridine nucleotide cytochrome reductases.
Hyde GE, Crawford NM, Campbell W; J Biol Chem 1991;266:23542-23547.
[2]Medline: 95111952
Crystal structure of the FAD-containing fragment of corn nitrate reductase at 2.5 A resolution: relationship to other flavoprotein reductases.
Lu G, Campbell WH, Schneider G, Lindqvist Y; Structure 1994;2:809-821.

385. (oxidored molyb) Eukaryotic molybdopterin oxidoreductases signature
A number of different eukaryotic oxidoreductases that require and bind a molybdopterin cofactor have been shown [1] to share a few regions of sequence similarity. These enzymes are:

- Xanthine dehydrogenase (EC 1.1.1.204), which catalyzes the oxidation of xanthine to uric acid with the concomitant reduction of NAD. Structurally, this enzyme of about 1300 amino acids consists of at least three distinct domains: an N-terminal 2Fe-2S ferredoxin-like iron-sulfur binding domain (see <PDOC00175>), a central FAD/NAD-binding domain and a C-terminal Mo-pterin domain.
- Aldehyde oxidase (EC 1.2.3.1), which catalyzes the oxidation aldehydes into acids. Aldehyde oxidase is highly similar to xanthine dehydrogenase in its sequence and domain structure.
- Nitrate reductase (EC 1.6.6.1), which catalyzes the reduction of nitrate to nitrite. Structurally, this enzyme of about 900 amino acids consists of an N-terminal Mo-pterin domain, a central cytochrome b5-type heme-binding domain (see <PDOC00170>) and a C-terminal FAD/NAD-binding cytochrome reductase domain.
- Sulfite oxidase (EC 1.8.3.1), which catalyzes the oxidation of sulfite to sulfate. Structurally, this enzyme of about 460 amino acids consists of an N-terminal cytochrome b5-binding domain followed by a Mo-pterin domain.

There are a few conserved regions in the sequence of the molybdopterin-binding domain of these enzymes. The pattern used to detect these proteins is based on one of them. It contains a cysteine residue which could be involved in binding the molybdopterin cofactor.
Consensus pattern: [GA]-x(3)-[KRNQHT]-x(11,14)-[LIVMFYWS]-x(8)-[LIVMF]-x-C-x(2)-[DEN]-R-x(2)-[DE]

[1] Wootton J.C., Nicolson R.E., Cock J.M., Walters D.E., Burke J.F., Doyle W.A., Bray R.C. Biochim. Biophys. Acta 1057:157-185(1991).

386. (Oxidored q1) NADH-Ubiquinone/plastoquinone (complex I), various chains
This family is part of complex I which catalyses the transfer of two electrons from NADH to ubiquinone in a reaction that is associated with proton translocation across the membrane. Number of members: 1824

[1]
Medline: 93110040
The NADH:ubiquinone oxidoreductase (complex I) of respiratory chains. Walker JE; Q Rev Biophys 1992;25: 253-324.

387. (oxidored q3) NADH-ubiquinone/plastoquinone oxidoreductase chain 6. 179 members.

388. (oxidored q5) NADH-ubiquinone oxidoreductase chain 4, amino terminus

[1] Walker JE; Q Rev Biophys 1992;25:253-324.

389. (oxidored q6) Respiratory-chain NADH dehydrogenase 20 Kd subunit signature Respiratory-chain NADH dehydrogenase (EC 1.6.5.3) [1,2] (also known as complex I or NADH-ubiquinone oxidoreductase) is an oligomeric enzymatic complex located in the inner mitochondrial membrane which also seems to exist in the chloroplast and in cyanobacteria (as a NADH-plastoquinone oxidoreductase). Among the 25 to 30 polypeptide subunits of this

bioenergetic enzyme complex there is one with a molecular weight of 20 Kd (in mammals) [3], which is a component of the iron-sulfur (IP) fragment of the enzyme. It seems to bind a 4Fe-4S iron-sulfur cluster. The 20 Kd subunit has been found to be:

- Nuclear encoded, as a precursor form with a transit peptide in mammals, and in Neurospora crassa. - Mitochondrial encoded in Paramecium (gene psbG).
- Chloroplast encoded in various higher plants (gene ndhK or psbG). The 20 Kd subunit is highly similar to [4]:

- Synechocystis strain PCC 6803 proteins psbG1 and psbG2.
- Subunit B of Escherichia coli NADH-ubiquinone oxidoreductase (gene nuoB).
- Subunit NQ06 of Paracoccus denitrificans NADH-ubiquinone oxidoreductase.
- Subunit 7 of Escherichia coli formate hydrogenlyase (gene hycG).
- Subunit I of Escherichia coli hydrogenase-4 (gene hyfI).

As as signature pattern a highly conserved region was selected, located in the central section of this subunit and which contains a conserved cysteine that is probably involved in the binding of the 4Fe-4S center.
Consensus pattern: [GN]-x-D-[KRST]-[LIVMF](2)-P-[IV]-D-[LIVMFYW](2)-x-P-x-C-P-[PT] [The C is a putative 4Fe-4S ligand]

[1] Ragan C.I. Curr. Top. Bioenerg. 15:1-36(1987).
[2] Weiss H., Friedrich T., Hofhaus G., Preis D. Eur. J. Biochem. 197:563-576(1991).
[3] Arizmendi J.M., Runswick M.J., Skehel J.M., Walker J.E. FEBS Lett. 301:237-242(1992).
[4] Weidner U., Geier S., Ptock A., Friedrich T., Leif H., Weiss H. J. Mol. Biol. 233:109-122(1993).

390. p53 tumor antigen signature
The p53 tumor antigen [1 to 5, E1,E2] is a protein found in increased amounts in a wide variety of transformed cells. It is also detectable in many proliferating nontransformed cells, but it is undetectable or present at low levels in resting cells. It is frequently mutated or inactivated in many types of cancer. p53 seems to act as a tumor suppressor in some, but probably not all, tumor types. p53 is probably involved in cell cycle regulation, and may be a trans-activator that acts to negatively regulate cellular division by controlling a set of genes required for this process.
p53 is a phosphoprotein of about 390 amino acids which can be subdivided into four domains: a highly charged acidic region of about 75 to 80 residues, a hydrophobic proline-rich domain (position 80 to 150), a central region (from 150 to about 300), and a highly basic C-terminal region. The sequence of p53 is well conserved in vertebrate species; attempts to identify p53 in other eukaryotic philum has so far been unsuccessful.
As a signature pattern for p53 a perfectly conserved stretch of 13 residues located in the central region of the protein was selected. This region, known as domain IV in [3], is involved (along with an adjacent region) in the binding of the large T antigen of SV40. In man this region is the focus of a variety of point mutations in cancerous tumors.
Consensus pattern: M-C-N-S-S-C-M-G-G-M-N-R-R

[1] Levine A.J., Momand J., Finlay C.A. Nature 351:453-456(1991).
[2] Levine A.J., Momand J. Biochim. Biophys. Acta 1032:119-136(1990).
[3] Soussi T., Caron De Fromentel C., May P. Oncogene 5:945-952(1990).
[4] Lane D.P., Benchimol S. Genes Dev. 4:1-8(1990).
[5] Ulrich S.J., Anderson C.W., Mercer W.E., Appella E. J. Biol. Chem. 267:15259-15262(1992).

391. (P5CR) Delta 1-pyrroline-5-carboxylate reductase signature
Delta 1-pyrroline-5-carboxylate reductase (P5CR) (EC 1.5.1.2) [1,2] is the enzyme that catalyzes the terminal step in the biosynthesis of proline from glutamate, the NAD(P) dependent oxidation of 1-pyrroline-5-carboxylate into proline. The sequences of P5CR from eubacteria (gene proC), archaebacteria and eukaryotes show only a moderate level of overall similarity. As a signature pattern, the best conserved region located in the C-terminal section of P5CR was selected.
Consensus pattern: [PALF]-x(2,3)-[LIV]-x(3)-[LIVM]-[STAC]-[STV]-x-[GAN]-G-x-T-x(2)-[AG]-[LIV]-x(2)-[LMF]-[DENQK]

[1] Delauney A.J., Verma D.P. Mol. Gen. Genet. 221:299-305(1990).
[2] Savioz A., Jeenes D.J., Kocher H.P., Haas D. Gene 86:107-111(1990).

392. Poly-adenylate binding protein, unique domain.

393. (PAL) Phenylalanine and histidine ammonia-lyases active site

Phenylalanine ammonia-lyase (EC 4.3.1.5) (PAL) is a key enzyme of plant and fungi phenylpropanoid metabolism which is involved in the biosynthesis of a wide variety of secondary metabolites such as flavanoids, furanocoumarin phytoalexins and cell wall components. These compounds have many important roles in plants during normal growth and in responses to environmental stress.

PAL catalyzes the removal of an ammonia group from phenylalanine to form trans-cinnamate.

Histidine ammonia-lyase (EC 4.3.1.3) (histidase) catalyzes the first step in histidine degradation, the removal of an ammonia group from histidine to produce urocanic acid.

The two types of enzymes are functionally and structurally related [1]. They are the only enzymes which are known to have the modified amino acid dehydroalanine (DHA) in their active site. A serine residue has been shown [2,3,4] to be the precursor of this essential electrophilic moiety. The region around this active site residue is well conserved and can be used as a signature pattern.

Consensus pattern: G-[STG]-[LIVM]-[STG]-[AC]-S-G-[DH]-L-x-P-L-[SA]-x(2)-[SA] [S is the active site residue]

[1] Taylor R.G., Lambert M.A., Sexsmith E., Sadler S.J., Ray P.N., Mahuran D.J., McInnes R.R. J. Biol. Chem. 265:18192-18199(1990).

[2] Langer M., Reck G., Reed J., Retey J. Biochemistry 33:6462-6467(1994).

[3] Schuster B., Retey J. FEBS Lett. 349:252-254(1994).

[4] Taylor R.G., McInnes R.R. J. Biol. Chem. 269:27473-27477(1994).

394. PAS domain

-!- CAUTION. This family does not currently match all known examples of PAS domains.

PAS motifs appear in archaea, eubacteria and eukarya. Probably the most surprising identification of a PAS domain was that in EAG-like K+-channels[1,3].

Number of members: 308

[1]
Medline: 97446881
PAS domain S-boxes in archaea, bacteria and sensors for oxygen and redox.
Zhulin IB, Taylor BL, Dixon R; Trends Biochem Sci 1997;22:331-333.
[2]Medline: 95275818
1.4 A structure of photoactive yellow protein, a cytosolic photoreceptor: unusual fold, active site, and chromophore.
Borgstahl GE, Williams DR, Getzoff ED; Biochemistry 1995;34:6278-6287.
[3]Medline: 98044337
PAS: a multifunctional domain family comes to light.
Ponting CP, Aravind L; Curr Biol 1997;7:674-677.

395. (PBP) Phosphatidylethanolamine-binding protein family signature Mammalian phosphatidylethanolamine-binding protein (also knowns as basic cytosolic 21 Kd protein) is a 186 residue protein found in a variety of tissues [1]. It binds hydrophobic ligands, such as phosphatidylethanolamine, but also seems [2] to bind nucleotides such as GTP and FMN, it is suggested that it could act in membrane remodeling during growth and maturation. This protein belongs to a family that also includes:

- Drosophila antennal protein A5, a putative odorant-binding protein.
- Onchocerca volvulus antigen Ov-16 and the related proteins D1, D2 and D3.
- Plasmodium falciparum putative phosphatidylethanolamine-binding protein.
- Toxocara canis secreted antigen TES-26. This larval protein has been shown to bind phosphatidylethanolamine.
- Yeast protein DKA1 (also known as NSP1 or TFS1). The function of this protein is not very clear. - Yeast hypothetical protein YLR179C.
- Caenorhabditis elegans hypothetical protein F40A3.3.

As a signature pattern, the best conserved region was selected which is located in the end of the first third of the sequence of these proteins.

Consensus pattern: [FYL]-x-[LV]-[LIVF]-x-[TIV]-[DC]-P-D-x-P-[SN]-x(10)-H

[1] Seddiqi N., Bollengier F., Alliel P.M., Perin J.P., Bonnet F., Bucquoy S., Jolles P., Schoentgen F. J. Mol. Evol. 39:655-660(1994).

[2] Schoentgen F., Jolles P. FEBS Lett. 369:22-6(1995).

396. PCI domain
This domain has also been called the PINT motif (Proteasome, Int-6, Nip-1 and TRIP-15) [1].
Number of members: 49

[1]
Medline: 98308842
The PCI domain: a common theme in three multiprotein complexes.
Hofmann K, Bucher P; Trends Biochem Sci 1998;23:204-205.
[2]Medline: 98266368
Homologues of 26S proteasome subunits are regulators of transcription and translation.
Aravind L, Ponting CP; Protein Sci 1998;7:1250-1254.

397. (PCMT) Protein-L-isoaspartate (D-aspartate) O-methyltransferase signature. Protein-L-isoaspartate (D-aspartate) O-methyltransferase (EC 2.1.1.77) (PCMT)[1] (which is also known as L-isoaspartyl protein carboxyl methyltransferase)is an enzyme that catalyzes the transfer of a methyl group from S-adenosylmethionine to the free carboxyl groups of D-aspartyl or L-isoaspartyl residues in a variety of peptides and proteins. The enzyme does not act on normal L-aspartyl residues L-isoaspartyl and D-aspartyl are the products of the spontaneous de amidation and/or isomerization of normal L-aspartyl and L-asparaginyl residues in proteins. PCMT plays a role in the repair and/ordegradation of these damaged proteins; the enzymatic methyl esterification of the abnormal residues can lead to their conversion to normal L-aspartylresidues. PCMT is a well-conserved and widely distributed cytosolic protein of about 24Kd. As a signature pattern, a conserved region in the central part of this enzyme has been developed.
Consensus pattern: [GSA]-D-G-x(2)-G-[FYWV]-x(3)-[AS]-P-[FY]-[DN]-x-I -

[1] Kagan R.M., McFadden H.J., McFadden P.N., O'Connor C., Clarke S. Comp. Biochem. Physiol. 117b: 379-385(1997).

398. (PCNA) Proliferating cell nuclear antigen signatures
Proliferating cell nuclear antigen (PCNA) [1,2] is a protein involved in DNA replication by acting as a cofactor for DNA polymerase delta, the polymerase responsible for leading strand DNA replication.
A similar protein exists in yeast (gene POL30) [3] and is associated with polymerase III, the yeast analog of polymerase delta. In baculoviruses the ETL protein has been shown [4] to be highly related to PCNA and is probably associated with the viral encoded DNA polymerase. An homolog of PCNA is also found in archebacteria.
As signatures for this family of proteins, two conserved regions were selected located in the N-terminal section. The second one has been proposed to bind DNA.
Consensus pattern: [GA]-[LIVMF]-x-[LIVMA]-x-[SAV]-[LIVM]-D-x-[NSAE]-[HKR]-[VI]-x-[LY]-[VGA]-x-[LIVM]-x-[LIVM]-x(4)-F
-Consensus pattern: [RKA]-C-[DE]-[RH]-x(3)-[LIVMF]-x(3)-[LIVM]-x-[SGAN]-[LIVMF]-x-K-[LIVMF](2)

[1] Bravo R., Frank R., Blundell P.A., McDonald-Bravo H. Nature 326:515-517(1987).
[2] Suzuka I., Hata S., Matsuoka M., Kosugi S., Hashimoto J. Eur. J. Biochem. 195:571-575(1991).
[3] Bauer G.A., Burgess P.M.J. Nucleic Acids Res. 18:261-265(1990).
[4] O'Reilly D.R., Crawford A.M., Miller L.K. Nature 337:606-606(1989).

399. (PDT) Prephenate dehydratase signatures
Prephenate dehydratase (EC 4.2.1.51) (PDT) catalyzes the decarboxylation of prephenate into phenylpyruvate. In microorganisms PDT is involved in the terminal pathway of the biosynthesis of phenylalanine. In some bacteria such as Escherichia coli PDT is part of a bifunctional enzyme (P-protein) that also catalyzes the transformation of chorismate into prephenate (chorismate mutase) while in other bacteria it is a monofunctional enzyme. The sequence of monofunctional PDT align well with the C-terminal part of that of P-proteins [1].
As signature patterns for PDT two conserved regions were selected. The first region contains a conserved threonine which has been said to be essential for the activity of the enzyme in E. coli. The second region includes a conserved glutamate. Both regions are in the C-terminal part of PDT.
Consensus pattern: [FY]-x-[LIVM]-x(2)-[LIVM]-x(5)-[DN]-x(5)-T-R-F-[LIVMW]-x-[LIVM]

[1] Fischer R.S., Zhao G., Jensen R.A. J. Gen. Microbiol. 137:1293-1301(1991).

400. PDZ domain (Also known as DHR or GLGF).
PDZ domains are found in diverse signaling proteins.

[1] Ponting CP, Phillips C, Davies KE, Blake DJ Bioessays 1997;19:469-479.
[2] Doyle DA, Lee A, Lewis J, Kim E, Sheng M, MacKinnon R; Cell. 1996;85:1067-1076.
[3] Ponting CP; Protein Sci 1997;6:464-468.

401. (PPDK_N_term) PEP-utilizing enzymes signatures
A number of enzymes that catalyze the transfer of a phosphoryl group from phosphoenolpyruvate (PEP) via a phospho-histidine intermediate have been shown to be structurally related [1,2,3,4]. These enzymes are:

- Pyruvate,orthophosphate dikinase (EC 2.7.9.1) (PPDK). PPDK catalyzes the reversible phosphorylation of pyruvate and phosphate by ATP to PEP and diphosphate. In plants PPDK function in the direction of the formation of PEP, which is the primary acceptor of carbon dioxide in C4 and crassulacean acid metabolism plants. In some bacteria, such as Bacteroides symbiosus, PPDK functions in the direction of ATP synthesis.
- Phosphoenolpyruvate synthase (EC 2.7.9.2) (pyruvate,water dikinase). This enzyme catalyzes the reversible phosphorylation of pyruvate by ATP to form PEP, AMP and phosphate, an essential step in gluconeogenesis when pyruvate and lactate are used as a carbon source.
- Phosphoenolpyruvate-protein phosphotransferase (EC 2.7.3.9). This is the first enzyme of the phosphoenolpyruvate-dependent sugar phosphotransferase system (PTS), a major carbohydrate transport system in bacteria. The PTS catalyzes the phosphorylation of incoming sugar substrates concomitant with their translocation across the cell membrane. The general mechanism of the PTS is the following: a phosphoryl group from PEP is transferred to enzyme-1 (EI) of PTS which in turn transfers it to a phosphoryl carrier protein (HPr). Phospho-HPr then transfers the phosphoryl group to a sugar-specific permease.

All these enzymes share the same catalytic mechanism: they bind PEP and transfer the phosphoryl group from it to a histidine residue. The sequence around that residue is highly conserved and can be used as a signature pattern for these enzymes. As a second signature pattern a conserved region was selected in the C-terminal part of the PEP-utilizing enzymes. The biological significance of this region is not yet known.
Consensus pattern: G-[GA]-x-[TN]-x-H-[STA]-[STAV]-[LIVM](2)-[STAV]-[RG] [H is phosphorylated]
-Consensus pattern: [DEQSK]-x-[LIVMF]-S-[LIVMF]-G-[ST]-N-D-[LIVM]-x-Q-[LIVMFYGT]-[STALIV]-[LIVMF]-[GAS]-x(2)-R

[1] Reizer J., Hoischen C., Reizer A., Pham T.N., Saier M.H. Jr. Protein Sci. 2:506-521(1993).
[2] Reizer J., Reizer A., Merrick M.J., Plunkett G. III, Rose D.J., Saier M.H. Jr. Gene 181:103-108(1996).
[3] Pocalyko D.J., Carroll L.J., Martin B.M., Babbitt P.C., Dunaway-Mariano D. Biochemistry 29:10757-10765 (1990).
[4] Niersbach M., Kreuzaler F., Geerse R.H., Postma P., Hirsch H.J. Mol. Gen. Genet. 232:332-336(1992).

402. (PEPCK ATP) Phosphoenolpyruvate carboxykinase (ATP) signature Phosphoenolpyruvate carboxykinase (ATP) (EC 4.1.1.49) (PEPCK) [1] catalyzes the formation of phosphoenolpyruvate by decarboxylation of oxaloacetate while hydrolyzing ATP, a rate limiting step in gluconeogenesis (the biosynthesis of glucose).
The sequence of this enzyme has been obtained from Escherichia coli, yeast, and Trypanosoma brucei; these three sequences are evolutionary related and share many regions of similarity. As a signature pattern a highly conserved region was selected that contains four acidic residues and which is located in the central part of the enzyme. The beginning of the pattern is located about 10 residues to the C-terminus of an ATP-binding motif 'A' (P-loop) (see <PDOC00017>) and is also part of the ATP-binding domain [2].
Consensus pattern: L-I-G-D-D-E-H-x-W-x-[DE]-x-G-[IV]-x-N

- Note: phosphoenolpyruvate carboxykinase (GTP) (EC 4.1.1.32) an enzyme that catalyzes the same reaction, but using GTP instead of ATP, is not related to the above enzyme (see <PDOC00421>).

[1] Medina V., Pontarollo R., Glaeske D., Tabel H., Goldie H. J. Bacteriol. 172:7151-7156(1990).
[2] Matte A., Goldie H., Sweet R.M., Delbaere L.T.J. J. Mol. Biol. 256:126-143(1996).

403. (Pepcase) Phosphoenolpyruvate carboxylase active sites. Phosphoenolpyruvate carboxylase (EC 4.1.1.31) (PEPcase) catalyzes the irreversible beta-carboxylation of phosphoenolpyruvate by bicarbonate to yield oxaloacetate and phosphate. The enzyme is found in all plants and in a variety of microorganisms. A histidine [1] and a lysine

[2] have been implicated in the catalytic mechanism of this enzyme; the regions around these active site residues are highly conserved in PEPcase from various plants, bacteria and cyanobacteria and can be used as a signature patterns for this type of enzyme.
Consensus pattern: [VT]-x-T-A-H-P-T-[EQ]-x(2)-R-[KRH] [H is an active site residue]-
Consensus pattern: [IV]-M-[LIVM]-G-Y-S-D-S-x-K-D-[STAG]-G [K is an active site residue]-

[1] Terada K., Izui K. Eur. J. Biochem. 202:797-803(1991).
[2] Jiao J.-A., Podesta F.E., Chollet R., O'Leary M.H., Andreo C.S. Biochim. Biophys. Acta 1041:291-295(1990).

404. PET112 family signature
The following proteins from eukaryotes, prokaryotes and archaebacteria belong to the same family:

- Yeast mitochondrial protein PET112 [1], which plays an unknown role in the expression of mitochondrial genes, probably at the level of translation.
- Aspergillus nidulans mitochondrial protein nempA.
- Bacillus subtilis hypothetical protein yzdD.
- Moraxella catarrhalis hypothetical protein in bloR-1 3'region.
- Mycoplasma genitalium hypothetical protein MG100.
- Methanococcus jannaschii hypothetical proteins MJ0019 and MJ0160.

The size of these proteins range from 419 to 630 amino acids. As a signature pattern, a conserved region located in the N-terminal section was selected.
Consensus pattern: [DN]-x-[DN]-R-x(3)-P-L-[LIV]-E-[LIV]-x-[ST]-x-P

[1] Mulero J.J., Rosenthal J.K., Fox T.D. Curr. Genet. 25:299-304(1994).

405. (PFK) Phosphofructokinase signature
Phosphofructokinase (EC 2.7.1.11) (PFK) [1,2] is a key regulatory enzyme in the glycolytic pathway. It catalyzes the phosphorylation by ATP of fructose 6-phosphate to fructose 1,6-bisphosphate. In bacteria PFK is a tetramer of identical 36 Kd subunits. In mammals it is a tetramer of 80 Kd subunits. Each 80 Kd subunit consist of two homologous domains which are highly related to the bacterial 36 Kd subunits. In Human there are three, tissue-specific, types of PFK isozymes: PFKM (muscle), PFKL (liver), and PFKP (platelet). In yeast PFK is an octamer composed of four 100 Kd alpha chains (gene PFK1) and four 100 Kd beta chains (gene PFK2); like the mammalian 80 Kd subunits, the yeast 100 Kd subunits are composed of two homologous domains.
As a signature pattern for PFK a region that contains three basic residues involved in fructose-6-phosphate binding was selected.
Consensus pattern: [RK]-x(4)-G-H-x-Q-[QR]-G-G-x(5)-D-R [The R/K, the H and the Q/R are involved in fructose-6-P binding]

- Note: Escherichia coli has two phosphofructokinase isozymes which are encoded by genes pfkA (major) and pfkB (minor). The pfkB isozyme is not evolutionary related to other prokaryotic or eukaryotic PFK's (see <PDOC00504>).

[1] Poorman R.A., Randolph A., Kemp R.G., Heinrikson R.L. Nature 309:467-469(1984).
[2] Heinisch J., Ritzel R.G., von Borstel R.C., Aguilera A., Rodicio R., Zimmermann F.K. Gene 78:309-321(1989).

406. (PGAM) Phosphoglycerate mutase family phosphohistidine signature Phosphoglycerate mutase (EC 5.4.2.1) (PGAM) and bisphosphoglycerate mutase (EC 5.4.2.4) (BPGM) are structurally related enzymes which catalyze reactions involving the transfer of phospho groups between the three carbon atoms of phosphoglycerate [1,2]. Both enzymes can catalyze three different reactions, although in different proportions:

- The isomerization of 2-phosphoglycerate (2-PGA) to 3-phosphoglycerate (3-PGA) with 2,3-diphosphoglycerate (2,3-DPG) as the primer of the reaction.
- The synthesis of 2,3-DPG from 1,3-DPG with 3-PGA as a primer.
- The degradation of 2,3-DPG to 3-PGA (phosphatase EC 3.1.3.13 activity).

In mammals, PGAM is a dimeric protein. There are two isoforms of PGAM: the M (muscle) and B (brain) forms. In yeast, PGAM is a tetrameric protein. BPGM is a dimeric protein and is found mainly in erythrocytes where it plays

a major role in regulating hemoglobin oxygen affinity as a consequence of controlling 2,3-DPG concentration.
The catalytic mechanism of both PGAM and BPGM involves the formation of a phosphohistidine intermediate [3].
The bifunctional enzyme 6-phosphofructo-2-kinase / fructose-2,6-bisphosphatase (EC 2.7.1.105 and EC 3.1.3.46) (PF2K) [4] catalyzes both the synthesis and the degradation of fructose-2,6-bisphosphate. PF2K is an important enzyme in the regulation of hepatic carbohydrate metabolism. Like PGAM/BPGM, the fructose-2,6-bisphosphatase reaction involves a phosphohistidine intermediate and the phosphatase domain of PF2K is structurally related to PGAM/BPGM.
The bacterial enzyme alpha-ribazole-5'-phosphate phosphatase (gene cobC) which is involved in cobalamin biosynthesis also belongs to this family [5].
A signature pattern was built around the phosphohistidine residue.
Consensus pattern: [LIVM]-x-R-H-G-[EQ]-x(3)-N [H is the phosphohistidine residue]

- Note: some organisms harbor a form of PGAM independent of 2,3-DPG, this enzyme is not related to the family described above [6].

[1] Le Boulch P., Joulin V., Garel M.-C., Rosa J., Cohen-Solal M. Biochem. Biophys. Res. Commun. 156:874-881 (1988).
[2] White M.F., Fothergill-Gilmore L.A. FEBS Lett. 229:383-387(1988).
[3] Rose Z.B. Meth. Enzymol. 87:43-51(1982).
[4] Bazan J.F., Fletterick R.J., Pilkis S.J. Proc. Natl. Acad. Sci. U.S.A. 86:9642-9646(1989).
[5] O'Toole G.A., Trzebiatowski J.R., Escalante-Semerena J.C. J. Biol. Chem. 269:26503-26511(1994).
[6] Grana X., De Lecea L., El-Maghrabi M.R., Urena J.M., Caellas C., Carreras J., Puigdomenech P., Pilkis S.J., Climent F. J. Biol. Chem. 267:12797-12803(1992).

407. (PGI) Phosphoglucose isomerase signatures
Phosphoglucose isomerase (EC 5.3.1.9) (PGI) [1,2] is a dimeric enzyme that catalyzes the reversible isomerization of glucose-6-phosphate and fructose-6-phosphate. PGI is involved in different pathways: in most higher organisms it is involved in glycolysis; in mammals it is involved in gluconeogenesis; in plants in carbohydrate biosynthesis; in some bacteria it provides a gateway for fructose into the Entner-Doudouroff pathway. PGI has been shown [3] to be identical to neuroleukin, a neurotrophic factor which supports the survival of various types of neurons.
The sequence of PGI from many species ranging from bacteria to mammals is available and has been shown to be highly conserved. As signature patterns for this enzyme two conserved regions were selected, the first region is located in the central section of PGI, while the second one is located in its C-terminal section.
Consensus pattern: [DENS]-x-[LIVM]-G-G-R-[FY]-S-[LIVMT]-x-[STA]-[PSAC]-[LIVMA]-G
-Consensus pattern: [GS]-x-[LIVM]-[LIVMFYW]-x(4)-[FY]-[DN]-Q-x-G-V-E-x(2)-K

[1] Achari A., Marshall S.E., Muirhewad H., Palmieri R.H., Noltmann E.A. Philos. Trans. R. Soc. Lond., B, Biol. Sci. 293:145-157(1981).
[2] Smith M.W., Doolittle R.F. J. Mol. Evol. 34:544-545(1992).
[3] Faik P., Walker J.I.H., Redmill A.A.M., Morgan M.J. Nature 332:455-456(1988).

408. (PGK) Phosphoglycerate kinase signature
Phosphoglycerate kinase (EC 2.7.2.3) (PGK) [1] catalyzes the second step in the second phase of glycolysis, the reversible conversion of 1,3-diphosphoglycerate to 3-phosphoglycerate with generation of one molecule of ATP. PGK is found in all living organisms and its sequence has been highly conserved throughout evolution. It is a two-domain protein; each domain is composed of six repeats of an alpha/beta structural motif. As a signature pattern for PGK's, a conserved region in the N-terminal region was selected.
Consensus pattern: [KRHGTCVN]-[VT]-[LIVMF]-[LIVMC]-R-x-D-x-N-[SACV]-P

[1] Watson H.C., Littlechild J.A. Biochem. Soc. Trans. 18:187-190(1990).

409. (PGM PMM) Phosphoglucomutase and phosphomannomutase phosphoserine signature

- Phosphoglucomutase (EC 5.4.2.2) (PGM). PGM is an enzyme responsible for the conversion of D-glucose 1-phosphate into D-glucose 6-phosphate. PGM participates in both the breakdown and synthesis of glucose [1].
- Phosphomannomutase (EC 5.4.2.8) (PMM). PMM is an enzyme responsible for the conversion of D-mannose 1-phosphate into D-mannose 6-phosphate. PMM is required for different biosynthetic pathways in bacteria. For example, in enterobacteria such as Escherichia coli there are two different genes coding for this enzyme: rfbK

which is involved in the synthesis of the O antigen of lipopolysaccharide and cpsG which is required for the synthesis of the M antigen capsular polysaccharide [2]. In Pseudomonas aeruginosa PMM (gene algC) is involved in the biosynthesis of the alginate layer [3] and in Xanthomonas campestris (gene xanA) it is involved in the biosynthesis of xanthan [4]. In Rhizobium strain ngr234 (gene noeK) it is involved in the biosynthesis of the nod factor.

- Phosphoacetylglucosamine mutase (EC 5.4.2.3) which converts N-acetyl-D-glucosamine 1-phosphate into the 6-phosphate isomer.

The catalytic mechanism of both PGM and PMM involves the formation of a phosphoserine intermediate [1]. The sequence around the serine residue is well conserved and can be used as a signature pattern.
In addition to PGM and PMM there are at least three uncharacterized proteins that belong to this family [5,6]:

- Urease operon protein ureC from Helicobacter pylori.
- Escherichia coli protein mrsA.
- Paramecium tetraurelia parafusin, a phosphoglycoprotein involved in exocytosis.
- A Methanococcus vannielii hypothetical protein in the 3'region of the gene for ribosomal protein S10.
  Consensus pattern: [GSA]-[LIVM]-x-[LIVM]-[ST]-[PGA]-S-H-x-P-x(4)-[GNHE] [S is the phosphoserine residue]

  - Note: PMM from fungi do not belong to this family.

[1] Dai J.B., Liu Y., Ray W.J. Jr., Konno M. J. Biol. Chem. 267:6322-6337(1992).
[2] Stevenson G., Lee S.J., Romana L.K., Reeves P.R. Mol. Gen. Genet. 227:173-180(1991).
[3] Zielinski N.A., Chakrabarty A.M., Berry A. J. Biol. Chem. 266:9754-9763(1991).
[4] Koeplin R., Arnold W., Hoette B., Simon R., Wang G., Puehler A. J. Bacteriol. 174:191-199(1992).
[5] Bairoch A. Unpublished observations (1993).
[6] Subramanian S.V., Wyroba E., Andersen A.P., Satir B.H. Proc. Natl. Acad. Sci. U.S.A. 91:9832-9836 (1994).

410. PH domain profile
The 'pleckstrin homology' (PH) domain is a domain of about 100 residues that occurs in a wide range of proteins involved in intracellular signaling or as constituents of the cytoskeleton [1 to 7].
The function of this domain is not clear, several putative functions have been suggested: - binding to the beta/gamma subunit of heterotrimeric G proteins,

- binding to lipids, e.g. phosphatidylinositol-4,5-bisphosphate,
- binding to phosphorylated Ser/Thr residues,
- attachment to membranes by an unknown mechanism.

It is possible that different PH domains have totally different ligand requirements.
The 3D structure of several PH domains has been determined [8]. All known cases have a common structure consisting of two perpendicular anti-parallel beta sheets, followed by a C-terminal amphipathic helix. The loops connecting the beta-strands differ greatly in length, making the PH domain relatively difficult to detect. There are no totally invariant residues within the PH domain.
Proteins reported to contain one more PH domains belong to the following families:

- Pleckstrin, the protein where this domain was first detected, is the major substrate of protein kinase C in platelets. Pleckstrin is one of the rare proteins to contains two PH domains.
- Ser/Thr protein kinases such as the Act/Rac family, the beta-adrenergic receptor kinases, the mu isoform of PKC and the trypanosomal NrkA family.
- Tyrosine protein kinases belonging to the Btk/Itk/Tec subfamily.
- Insulin Receptor Substrate 1 (IRS-1).
- Regulators of small G-proteins like guanine nucleotide releasing factor GNRP (Ras-GRF) (which contains 2 PH domains), guanine nucleotide exchange proteins like vav, dbl, SoS and yeast CDC24, GTPase activating proteins like rasGAP and BEM2/IPL2, and the human break point cluster protein bcr.
- Cytoskeletal proteins such as dynamin (see <PDOC00362>), Caenorhabditis elegans kinesin-like protein unc-104 (see <PDOC00343>), spectrin beta-chain, syntrophin (2 PH domains) and yeast nuclear migration protein NUM1.
- Mammalian phosphatidylinositol-specific phospholipase C (PI-PLC) (see <PDOC50007>) isoforms gamma and

delta. Isoform gamma contains two PH domains, the second one is split into two parts separated by about 400 residues. - Oxysterol binding proteins OSBP, yeast OSH1 and YHR073w.

- Mouse protein citron, a putative rho/rac effector that binds to the GTP-bound forms of rho and rac,
- Several yeast proteins involved in cell cycle regulation and bud formation like BEM2, BEM3, BUD4 and the BEM1-binding proteins BOI2 (BEB1) and BOI1 (BOB1). - Caenorhabditis elegans protein MIG-10.
- Caenorhabditis elegans hypothetical proteins C04D8.1, K06H7.4 and ZK632.12.
- Yeast hypothetical proteins YBR129c and YHR155w.
The profile for the PH domain, which has been developed by Toby Gibson at the EMBL, covers the total length of domain. Several proteins contain large insertions in the PH domain and are thus difficult to detect with this profile. In some of these cases, the profile will align only to one half of the PH domain.
- Sequences known to belong to this class detected by the pattern: ALL. But it should be noted that while all sequences containing PH domains are detected, not all PH domains are. Some of the split domains lie below the cutoff threshold.

[1] Mayer B.J., Ren R., Clark K.L., Baltimore D. Cell 73:629-630(1993).
[2] Haslam R.J., Koide H.B., Hemmings B.A. Nature 363:309-310(1993).
[3] Musacchio A., Gibson T.J., Rice P., Thompson J., Saraste M. Trends Biochem. Sci. 18:343-348(1993).
[4] Gibson T.J., Hyvonen M., Musacchio A., Saraste M., Birney E. Trends Biochem. Sci. 19:349-353(1994).
[5] Pawson T. Nature 373:573-580(1995).
[6] Ingley E., Hemmings B.A. J. Cell. Biochem. 56:436-443(1994).
[7] Saraste M., Hyvonen M. Curr. Opin. Struct. Biol. 5:403-408(1995).
[8] Riddihough G. Nat. Struct. Biol. 1:755-757(1994).

411. PHD-finger

[1]
Medline: 95216093
The PHD finger: implications for chromatin-mediated transcriptional regulation.
Aasland R, Gibson TJ, Stewart AF; Trends Biochem Sci 1995;20:56-59.
Number of members: 181

412. (PI-PLC-X) Phosphatidylinositol-specific phospholipase C profiles Phosphatidylinositol-specific phospholipase C (EC 3.1.4.11), an eukaryotic intracellular enzyme, plays an important role in signal transduction processes [1]. It catalyzes the hydrolysis of 1-phosphatidyl-D-myo-inositol-3,4,5-triphosphate into the second messenger molecules diacylglycerol and inositol-1,4,5-triphosphate. This catalytic process is tightly regulated by reversible phosphorylation and binding of regulatory proteins [2 to 4].
In mammals, there are at least 6 different isoforms of PI-PLC, they differ in their domain structure, their regulation, and their tissue distribution. Lower eukaryotes also possess multiple isoforms of PI-PLC.
All eukaryotic PI-PLCs contain two regions of homology, sometimes referred to as 'X-box' and 'Y-box'. The order of these two regions is always the same (NH2-X-Y-COOH), but the spacing is variable. In most isoforms, the distance between these two regions is only 50-100 residues but in the gamma isoforms one PH domain, two SH2 domains, and one SH3 domain are inserted between the two PLC-specific domains. The two conserved regions have been shown to be important for the catalytic activity. At the C-terminal of the Y-box, there is a C2 domain (see <PDOC00380>) possibly involved in Ca-dependent membrane attachment.
Profile analysis shows that sequences with significant similarity to the X-box domain occur also in prokaryotic and trypanosome PI-specific phospholipases C. Apart from this region, the prokaryotic enzymes show no similarity to their eukaryotic counterparts.
Two profiles were developed, one covering the X-box, the other the Y-box.

[1] Meldrum E., Parker P.J., Carozzi A. Biochim. Biophys. Acta 1092:49-71(1991).
[2] Rhee S.G., Choi K.D. Adv. Second Messenger Phosphoprotein Res. 26:35-61(1992).
[3] Rhee S.G., Choi K.D. J. Biol. Chem. 267:12393-12396(1992).
[4] Sternweis P.C., Smrcka A.V. Trends Biochem. Sci. 17:502-506(1992).

413. (PI-PLC-Y) Phosphatidylinositol-specific phospholipase C profiles Phosphatidylinositol-specific phospholipase C (EC 3.1.4.11), an eukaryotic intracellular enzyme, plays an important role in signal transduction processes

[1]. It catalyzes the hydrolysis of 1-phosphatidyl-D-myo-inositol-3,4,5-triphosphate into the second messenger

molecules diacylglycerol and inositol-1,4,5-triphosphate. This catalytic process is tightly regulated by reversible phosphorylation and binding of regulatory proteins [2 to 4].
In mammals, there are at least 6 different isoforms of PI-PLC, they differ in their domain structure, their regulation, and their tissue distribution. Lower eukaryotes also possess multiple isoforms of PI-PLC.
All eukaryotic PI-PLCs contain two regions of homology, sometimes referred to as 'X-box' and 'Y-box'. The order of these two regions is always the same (NH2-X-Y-COOH), but the spacing is variable. In most isoforms, the distance between these two regions is only 50-100 residues but in the gamma isoforms one PH domain, two SH2 domains, and one SH3 domain are inserted between the two PLC-specific domains. The two conserved regions have been shown to be important for the catalytic activity. At the C-terminal of the Y-box, there is a C2 domain (see <PDOC00380>) possibly involved in Ca-dependent membrane attachment.
Profile analysis shows that sequences with significant similarity to the X-box domain occur also in prokaryotic and trypanosome PI-specific phospholipases C. Apart from this region, the prokaryotic enzymes show no similarity to their eukaryotic counterparts.
Two profiles were developed, one covering the X-box, the other the Y-box.

[1] Meldrum E., Parker P.J., Carozzi A. Biochim. Biophys. Acta 1092:49-71(1991).
[2] Rhee S.G., Choi K.D. Adv. Second Messenger Phosphoprotein Res. 26:35-61(1992).
[3] Rhee S.G., Choi K.D. J. Biol. Chem. 267:12393-12396(1992).
[4] Sternweis P.C., Smrcka A.V. Trends Biochem. Sci. 17:502-506(1992).

414. (PK) Pyruvate kinase active site signature
Pyruvate kinase (EC 2.7.1.40) (PK) [1] catalyzes the final step in glycolysis, the conversion of phosphoenolpyruvate to pyruvate with the concomitant phosphorylation of ADP to ATP. PK requires both magnesium and potassium ions for its activity. PK is found in all living organisms. In vertebrates there are four, tissues specific, isozymes: L (liver), R (red cells), M1 (muscle, heart, and brain), and M2 (early fetal tissues). In Escherichia coli there are two isozymes: PK-I (gene pykF) and PK-II (gene pykA). All PK isozymes seem to be tetramers of identical subunits of about 500 amino acid residues.
As a signature pattern for PK a conserved region was selected that includes a lysine residue which seems to be the acid/base catalyst responsible for the interconversion of pyruvate and enolpyruvate, and a glutamic acid residue implicated in the binding of the magnesium ion.
Consensus pattern: [LIVAC]-x-[LIVM](2)-[SAPCV]-K-[LIV]-E-[NKRST]-x-[DEQHS]-[GSTA]-[LIVM] [K is the active site residue] [E is a magnesium ligand]

[1] Muirhead H. Biochem. Soc. Trans. 18:193-196(1990).

415. (PLDc) Phospholipase D. Active site motif
Phosphatidylcholine-hydrolyzing phospholipase D (PLD) isoforms are activated by ADP-ribosylation factors (ARFs). PLD produces phosphatidic acid from phosphatidylcholine, which may be essential for the formation of certain types of transport vesicles or may be constitutive vesicular transport to signal transduction pathways.
PC-hydrolyzing PLD is a homologue of cardiolipin synthase, phosphatidylserine synthase, bacterial PLDs, and viral proteins.
Each of these appears to possess a domain duplication which is apparent by the presence of two motifs containing well-conserved histidine, lysine, and/or asparagine residues which may contribute to the active site. aspartic acid.
An E. coli endonuclease (nuc) and similar proteins appear to be PLD homologues but possess only one of these motifs.
The profile contained here represents only the putative active site regions, since an accurate multiple alignment of the repeat units has not been achieved.
Number of members: 139

[1]
Medline: 96303814
A novel family of phospholipase D homologues that includes phospholipid synthases and putative endonucleases: identification of duplicated repeats and potential active site residues.
Ponting CP, Kerr ID; Protein Sci 1996;5:914-922.
[2]Medline: 96334293
A duplicated catalytic motif in a new superfamily of phosphohydrolases and phospholipid synthases that includes poxvirus envelope proteins.
Koonin EV; Trends Biochem Sci 1996;21:242-243.

[3]Medline: 94327597
Cloning and expression of phosphatidylcholine-hydrolyzing phospholipase D from Ricinus communis L.
Wang X, Xu L, Zheng L; J Biol Chem 1994;269:20312-20317.
[4]Medline: 97386825
Regulation of eukaryotic phosphatidylinositol-specific phospholipase C and phospholipase D.
Singer WD, Brown HA, Sternweis PC; Annu Rev Biochem 1997;66:475-509.

416. (PMI type 1) Phosphomannose isomerase type I signatures

Phosphomannose isomerase (EC 5.3.1.8) (PMI) [1,2] is the enzyme that catalyzes the interconversion of mannose-6-phosphate and fructose-6-phosphate. In eukaryotes, it is involved in the synthesis of GDP-mannose which is a constituent of N- and O-linked glycans as well as GPI anchors. In prokaryotes, it is involved in a variety of pathways including capsular polysaccharide biosynthesis and D-mannose metabolism.

Three classes of PMI have been defined on the basis of sequence similarities [1]. The first class comprises all known eukaryotic PMI as well as the enzyme encoded by the manA gene in enterobacteria such as Escherichia coli. Class I PMI's are proteins of about 42 to 50 Kd which bind a zinc ion essential for their activity.

As signature patterns for class I PMI, two conserved regions were selected. The first one is located in the N-terminal section of these proteins, the second in the C-terminal half. Both patterns contain a residue involved [3] in the binding of the zinc ion.

Consensus pattern: Y-x-D-x-N-H-K-P-E [E is a zinc ligand]

-Consensus pattern: H-A-Y-[LIVM]-x-G-x(2)-[LIVM]-E-x-M-A-x-S-D-N-x-[LIVM]-R-A-G-x-T-P-K [H is a zinc ligand]

[1] Proudfoot A.E.I., Turcatti G., Wells T.N.C., Payton M.A., Smith D.J. Eur. J. Biochem. 219:415-423(1994).
[2] Coulin F., Magnenat E., Proudfoot A.E.I., Payton M.A., Scully P., Wells T.N.C. Biochemistry 32:14139-14144 (1993).
[3] Cleasby A., Wonacott A., Skarzynski T., Hubbard R.E., Davies G.J., Proudfoot A.E.I., Bernard A.R., Payton M.A., Wells T.N.C. Nat. Struct. Biol. 3:470-479(1996).

417. (PNP UDP 1) Purine and other phosphorylases family 1 signature

The following phosphorylases belongs to the same family:

- Purine nucleoside phosphorylase (EC 2.4.2.1) (PNP) from most bacteria (gene deoD). This enzyme catalyzes the cleavage of guanosine or inosine to respective bases and sugar-1-phosphate molecules [1].
- Uridine phosphorylase (EC 2.4.2.3) (UdRPase) from bacteria (gene udp) and mammals. Catalyzes the cleavage of uridine into uracil and ribose-1-phosphate. The products of the reaction are used either as carbon and energy sources or in the rescue of pyrimidine bases for nucleotide synthesis [2].
- 5'-methylthioadenosine phosphorylase (EC 2.4.2.28) (MTA phosphorylase) from Sulfolobus solfataricus [3].

As a signature pattern, a conserved region was selected in the central part of these enzymes.

Consensus pattern: [GST]-x-G-[LIVM]-G-x-[PA]-S-x-[GSTA]-I-x(3)-E-L

- Note: it shoudl be noted that mammalian and some bacterial PNP as well as eukaryotic MTA phosphorylase belong to a different family of phosphorylases (see <PDOC00954>).

[1] Takehara M., Ling F., Izawa S., Inoue Y., Kimura A. Biosci. Biotechnol. Biochem. 59:1987-1990(1995).
[2] Watanabe S.-I., Hino A., Wada K., Eliason J.F., Uchida T. J. Biol. Chem. 270:12191-12196(1995).
[3] Cacciapuoti G., Porcelli M., Bertoldo C., De Rosa M., Zappia V. J. Biol. Chem. 269:24762-24769(1994).

418. (PP2C) Protein phosphatase 2C signature

Protein phosphatase 2C (PP2C) is one of the four maj or classes of mammalian serine/threonine specific protein phosphatases (EC 3.1.3.16). PP2C [1] is a monomeric enzyme of about 42 Kd which shows broad substrate specificity and is dependent on divalent cations (mainly manganese and magnesium) for its activity. Its exact physiological role is still unclear. Three isozymes are currently known in mammals: PP2C-alpha, -beta and -gamma. In yeast, there are at least four PP2C homologs: phosphatase PTC1 [2] which has weak tyrosine phosphatase activity in addition to its activity on serines, phosphatases PTC2 and PTC3, and hypothetical protein YBR125c. Isozymes of PP2C are also known from Arabidopsis thaliana (ABI1, PPH1), Caenorhabditis elegans (FEM-2, F42G9.1, T23F11.1), Leishmania chagasi and Paramecium tetraurelia.

In Arabidopsis thaliana, the kinase associated protein phosphatase (KAPP) [3] is an enzyme that dephosphorylates the Ser/Thr receptor-like kinase RLK5 and which contains a C-terminal PP2C domain.

PP2C does not seem to be evolutionary related to the main family of serine/ threonine phosphatases: PP1, PP2A and PP2B . However, it is significantly similar to the catalytic subunit of pyruvate dehydrogenase phosphatase (EC 3.1.3.43) (PDPC) [4], which catalyzes dephosphorylation and concomitant reactivation of the alpha subunit of the E1 component of the pyruvate dehydrogenase complex. PDPC is a mitochondrial enzyme and, like PP2C, is magnesium-dependent.
As a signature pattern, the best conserved region was selected which is located in the N-terminal part and contains a perfectly conserved tripeptide. This region includes a conserved aspartate residue involved in divalent cation binding [5].
Consensus pattern: [LIVMFY]-[LIVMFYA]-[GSAC]-[LIVM]-[FYC]-D-G-H-[GAV]

- Note: PP2C belongs [6] to a superfamily which also includes bacterial proteins such as Bacillus spoIIE, rsbU and rsbW, Synechocystis PCC 6803 icfG as well as a domain in fungal adenylate cyclases.

[1] Wenk J., Trompeter H.-I., Pettrich K.-G., Cohen P.T.W., Campbell D.G., Mieskes G. FEBS Lett. 297:135-138 (1992).
[2] Maeda T., Tsai A.Y.M., Saito H. Mol. Cell. Biol. 13:5408-5417(1993).
[3] Stone J.M., Collinge M.A., Smith R.D., Horn M.A., Walker J.C. Science 266:793-795(1994).
[4] Lawson J.E., Niu X.-D., Browning K.S., Trong H.L., Yan J., Reed L.J. Biochemistry 32:8987-8993(1993).
[5] Das A.K., Helps N.R., Cohen P.T.W., Barford D. EMBO J. 24:6798-6809(1996).
[6] Bork P., Brown N.P., Hegyi H., Schultz J. Protein Sci. 5:1421-1425(1996).

419. (PPTA) Protein prenyltransferases alpha subunit repeat signature Protein prenyltransferases catalyze the transfer of an isoprenyl moiety to a cysteine four residues from the C-terminus of several proteins. They are heterodimeric enzymes consisting of alpha and beta subunits. The alpha subunit is thought to participate in a stable complex with the isoprenyl substrate; the beta subunit binds the peptide substrate. Distinct protein prenyltransferases might share a common alpha subunit. Both the alpha and beta subunit show repetitive sequence motifs [1]. These repeats have distinct structural and functional implications and are unrelated to each other. Known protein prenyltransferase alpha subunits are:

- Mammalian protein farnesyltransferase alpha subunit.
- Yeast protein RAM2, a protein farnesyltransferase alpha subunit.
- Yeast protein BET4, a protein geranylgeranyltransferase alpha subunit.

The conserved domain of the alpha subunit consists of about 34 amino acids and is repeated five times. It contains an invariant tryptophan possibly involved in heterodimerization with the conserved phenylalanines in the repeated domains of the beta subunits, via hydrophobic bonds. The signature pattern for this domain is centered on the invariant tryptophan.
Consensus pattern: [PSIAV]-x-[NDFV]-[NEQIY]-x-[LIVMAGP]-W-[NQSTHF]-[FYHQ]-[LIVMR]

[1] Boguski M.S., Murray A.W., Powers S. New Biol. 4:408-411(1992).

420. (PR55) Protein phosphatase 2A regulatory subunit PR55 signatures
Protein phosphatase 2A (PP2A) is a serine/threonine phosphatase involved in many aspects of cellular function including the regulation of metabolic enzymes and proteins involved in signal transduction. PP2A is a trimeric enzyme that consists of a core composed of a catalytic subunit associated with a 65 Kd regulatory subunit (PR65), also called subunit A; this complex then associates with a third variable subunit (subunit B), which confers distinct properties to the holoenzyme [1]. One of the forms of the variable subunit is a 55 Kd protein (PR55) which is highly conserved in mammals - where three isoforms are known to exist -, Drosophila and yeast (gene CDC55). This subunit could perform a substrate recognition function or be responsible for targeting the enzyme complex to the appropriate subcellular compartment.
As signature patterns, two perfectly conserved sequences of 15 residues were selected; one located in the N-terminal region, the other in the center of the protein.
Consensus pattern: E-F-D-Y-L-K-S-L-E-I-E-E-K-I-N
Consensus pattern: N-[AG]-H-[TA]-Y-H-I-N-S-I-S-[LIVM]-N-S-D

[1] Mayer-Jaekel R., Hemmings B.A. Trends Cell Biol. 4:287-291(1994).

421. N-(5’phosphoribosyl)anthranilate (PRA) isomerase

[1] Wilmanns M, Priestle JP, Niermann T, Jansonius JN; J Mol Biol 1992;223:477-507.

422. (PRK) Phosphoribulokinase signature

Phosphoribulokinase (EC 2.7.1.19) (PRK) [1,2] is one of the enzymes specific to the Calvin's reductive pentose phosphate cycle which is the major route by which carbon dioxide is assimilated and reduced by autotrophic organisms. PRK catalyzes the ATP-dependent phosphorylation of ribulose 5-phosphate into ribulose 1 ,5-bisphosphate which is the substrate for RubisCO.

PRK's of diverse origins show different properties with respect to the size of the protein, the subunit structure, or the enzymatic regulation. However an alignment of the sequences of PRK from plants, algae, photosynthetic and chemoautotrophic bacteria shows that there are a few regions of sequence similarity. As a signature pattern one of these regions was selected.

Consensus pattern: K-[LIVM]-x-R-D-x(3)-R-G-x-[ST]-x-E

[1] Kossmann J., Klintworth R., Bowien B. Gene 85:247-252(1989).
[2] Gibson J.L., Chen J.-H., Tower P.A., Tabita F.R. Biochemistry 29:8085-8093(1990).

423. (PRPP synt) Phosphoribosyl pyrophosphate synthetase signature

Phosphoribosyl pyrophosphate synthetase (EC 2.7.6.1) (PRPP synthetase) catalyzes the formation of PRPP from ATP and ribose 5-phosphate. PRPP is then used in various biosynthetic pathways, as for example in the formation of purines, pyrimidines, histidine and tryptophan. PRPP synthetase requires inorganic phosphate and magnesium ions for its stability and activity.

In mammals, three isozymes of PRPP synthetase are found; in yeast there are at least four isozymes.

As a signature pattern for this enzyme, a very conserved region was selected that has been suggested to be involved in binding divalent cations [1]. This region contains two conserved aspartic acid residues as well as a histidine, which are all potential ligands for a cation such as magnesium.

Consensus pattern: D-[LI]-H-[SA]-x-Q-[IMST]-[QM]-G-[FY]-F-x(2)-P-[LIVMFC]-D

[1] Bower S.G., Harlow K.W., Switzer R.L., Hoven-Jensen B. J. Biol. Chem. 264:10287-10291(1989).

424. (PRTP) Herpesvirus processing and transport protein

The members of this family are associate with capsid intermediates during packaging of the virus.

Number of members: 31

[1]
Medline: 98362148
Herpes simplex virus type 1 cleavage and packaging proteins
UL15 and UL28 are associated with B but not C capsids during packaging. Yu D, Weller SK; J Virol 1998;72: 7428-7439.

425. Photosystem I psaG / psaK (PSI PSAK) proteins signature

Photosystem I (PSI) [1] is an integral membrane protein complex that uses light energy to mediate electron transfer from plastocyanin to ferredoxin. It is found in the chloroplasts of plants and cyanobacteria. PSI is composed of at least 14 different subunits, two of which PSI-G (gene psaG) and PSI-K (gene psaK) are small hydrophobic proteins of about 7 to 9 Kd and evolutionary related [2]. Both seem to contain two transmembrane regions. Cyanobacteria seem to encode only for PSI-K.

As a signature pattern, the best-conserved region was selected which seems to correspond to the second transmembrane region.

- Consensus pattern: [GT]-F-x-[LIVM]-x-[DEA]-x(2)-[GA]-x-[GTA]-[SA]-x-G-H-x-[LIVM]-[GA]

[1] Golbeck J.H. Biochim. Biophys. Acta 895:167-204(1987).
[2] Kjaerulff S., Andersen B., Nielsen V.S., Moller B.L., Okkels J.S. J. Biol. Chem. 268:18912-18916(1993).

426. PTR2 family proton/oligopeptide symporters signatures

A family of eukaryotic and prokaryotic proteins that seem to be mainly involved in the intake of small peptides with the concomitant uptake of a proton has been recently characterized [1,2]. Proteins that belong to this family are: - Fungal peptide transporter PTR2.

- Mammalian intestine proton-dependent oligopeptide transporter PeptT1.
- Mammalian kidney proton-dependent oligopeptide transporter PeptT2.
- Drosophila opt1.
- Arabidopsis thaliana peptide transporters PTR2-A and PTR2-B (also known as the histidine transporting protein NTR1).
- Arabidopsis thaliana proton-dependent nitrate/chlorate transporter CHL1.
- Lactococcus proton-dependent di- and tri-peptide transporter dtpT.
- Caenorhabditis elegans hypothetical protein C06G8.2.
- Caenorhabditis elegans hypothetical protein F56F4.5.
- Caenorhabditis elegans hypothetical protein K04E7.2.
- Escherichia coli hypothetical protein ybgH.
- Escherichia coli hypothetical protein ydgR.
- Escherichia coli hypothetical protein yhiP.
- Escherichia coli hypothetical protein yjdL.
- Bacillus subtilis hypothetical protein yclF.

These integral membrane proteins are predicted to comprise twelve transmembrane regions. As signature patterns, two of the best conserved regions were selected. The first is a region that includes the end of the second transmembrane region, a cytoplasmic loop as well as the third transmembrane region. The second pattern corresponds to the core of the fifth transmembrane region.

- Consensus pattern: [GA]-[GAS]-[LIVMFYWA]-[LIVM]-[GAS]-D-x-[LIVMFYWT]-[LIVMFYW]-G-x(3)-[TAV]-[IV]-x(3)-[GSTAV]-x-[LIVMF]-x(3)-[GA]
- Consensus pattern: [FYT]-x(2)-[LMFY]-[FYV]-[LIVMFYWA]-x-[IVG]-N-[LIVMAG]-G-[GSA]-[LIMF]

[1] Paulsen I.T., Skurray R.A. Trends Biochem. Sci. 19:404-404(1994).
[2] Steiner H.-Y., Naider F., Becker J.M. Mol. Microbiol. 16:825-834(1995).

427. Pumilio-family RNA binding domains (aka PUM-HD, Pumilio homology domain)
Puf domains are necessary and sufficient for sequence specific RNA binding in fly Pumilio and worm FBF-1 and FBF-2. Both proteins function as translational repressors in early embryonic development by binding sequences in the 3' UTR of target mRNAs (e.g. the nanos response element (NRE) in fly Hunchback mRNA, or the point mutation element (PME) in worm fem-3 mRNA). Other proteins that contain Puf domains are also plausible RNA binding proteins. JSN1_YEAST, for instance, appears to also contain a single RRM domain by HMM analysis.
Puf domains usually occur as a tandem repeat of 8 domains.
The Pfam model does not necessarily recognize all 8 domains in all sequences; some sequences appear to have 5 or 6 domains on initial analysis, but further analysis suggests the presence of additional divergent domains.

[1] Zhang B, Gallegos M, Puoti A, Durkin E, Fields S, Kimble J, Wickens MP. Nature 1997;390:477-484.
[2] Zamore PD, Williamson JR, Lehmann R. RNA 1997;3:1421-1433.

428. PWWP domain. The PWWP domain is named after a conserved Pro-Trp-Trp-Pro motif. The function of the domain is currently unknown. Number of members: 19

[1] Medline: 98282232. WHSC1, a 90 kb SET domain-containing gene, expressed in early development and homologous to a Drosophila dysmorphy gene maps in the Wolf-Hirschhorn syndrome critical region and is fused to IgH in t(4;14) multiple myeloma. Stec I, Wright TJ, van Ommen GJB, de Boer PAJ, van Haeringen A, Moorman AFM, Altherr MR, den Dunnen JT; Hum Mol Genet 1998;7:1071-1082.

429. PX domain
Eukaryotic domain of unknown function present in phox proteins, PLD isoforms, a PI3K isoform.
Number of members: 71

[1]
Medline: 97084820
Novel domains in NADPH oxidase subunits, sorting nexins, and PtdIns 3-kinases: binding partners of SH3 domains?
Ponting CP; Protein Sci 1996;5:2353-2357.

430. ParA family ATPase

[1]
Medline: 91141297
A family of ATPases involved in active partitioning of diverse bacterial plasmids.
Motallebi-Veshareh M, Rouch DA, Thomas CM; Mol Microbiol 1990;4:1455-1463.
Number of members: 122

431. (Parvo coat) Parvovirus coat protein. 72 members.

432. Pectinesterase signatures
Pectinesterase (EC 3.1.1.11) (pectin methylesterase) catalyzes the hydrolysis of pectin into pectate and methanol. In plants, it plays an important role in cell wall metabolism during fruit ripening. In plant bacterial pathogens such as Erwinia carotovora and in fungal pathogens such as Aspergillus niger, pectinesterase is involved in maceration and soft-rotting of plant tissue. Prokaryotic and eukaryotic pectinesterases share a few regions of sequence similarity [1,2,3]. two of these regions were selected as signature patterns.
The first is based on a region in the N-terminal section of these enzymes; it contains a conserved tyrosine which may play a role in the catalytic mechanism [3]. The second pattern corresponds to the best conserved region, an octapeptide located in the central part of these enzymes.

- Consensus pattern: [GSTNP]-x(6)-[FYVHR]-[IVN]-[KEP]-x-G-[STIVKRQ]-Y-[DNQKRMV]-[EP]-x(3)-[LIMVA]
- Consensus pattern: [IV]-x-G-[STAD]-[LIVT]-D-[FYI]-[IV]-[FSN]-G

[1] Ray J., Knapp J., Grierson D., Bird C., Schuch W. Eur. J. Biochem. 174:119-124(1988).
[2] Plastow G.S. Mol. Microbiol. 2:247-254(1988).
[3] Markovic O., Joernvall H. Protein Sci. 1:1288-1292(1992).

433. Pentapeptide repeats (8 copies)
These repeats are found in many cyanobacterial proteins.
The repeats were first identified in hglK [1]. The function of these repeats is unknown.
The structure of this repeat has been predicted to be a beta-helix [2].
The repeat can be approximately described as A(D/N)LXX, where X can be any amino acid.Number of members: 75

[1] Medline: 96062225
The hglK gene is required for localization of heterocyst-specific glycolipids in the cyanobacterium Anabaena sp. strain PCC 7120.
Black K, Buikema WJ, Haselkorn R; J Bacteriol 1995;177:6440-6448.
[2] Medline: 98318059
Structure and distribution of pentapeptide repeats in bacteria.
Bateman A, Murzin A, Teichmann SA; Protein Sci 1998;7:1477-1480.
[3] Medline: 98316713
Characterisation of an Arabidopsis cDNA encoding a thylakoid lumen protein related to a novel 'pentapeptide repeat' family of proteins.
Kieselbach T, Mant A, Robinson C, Schroder WP; FEBS Lett 1998;428:241-244.

434. Polypeptide deformylase

[1] Medline: 97002011
A new subclass of the zinc metalloproteases superfamily revealed by the solution structure of peptide deformylase.
Meinnel T, Blanquet S, Dardel F; J Mol Biol 1996;262:375-386.
[2] Medline: 98332750
Solution structure of nickel-peptide deformylase.
Dardel F, Ragusa S, Lazennec C, Blanquet S, Meinnel T; J Mol Biol 1998;280:501-513.
Number of members: 21

435. Peptidyl-tRNA hydrolase signatures
Peptidyl-tRNA hydrolase (EC 3.1.1.29) (PTH) is a bacterial enzyme that cleaves peptidyl-tRNA or N-acyl-aminoacyl-tRNA to yield free peptides or N-acyl-amino acids and tRNA. The natural substrate for this enzyme may be peptidyl-tRNA which drop off the ribosome during protein synthesis [1,2]. Bacterial PTH has been found [2,3] to be evolutionary related to yeast hypothetical protein YHR189w.

PTH and YHR189w are proteins of about 200 amino acid residues. As signature patterns, two conserved regions were selected that each contain an histidine.

The first of these regions is located in the N-terminal section, the other in the central part.

- Consensus pattern: [FY]-x(2)-T-R-H-N-x-G-x(2)-[LIVMFA](2)-[DE]
- Consensus pattern: [GS]-x(3)-H-N-G-[LIVM]-[KR]-[DNS]-[LIVMT]

[1] Garcia-Villegas M.R., De La Vega F.M., Galindo J.M., Segura M., Buckingham R.H., Guameros G. EMBO J. 10:3549-3555(1991).
[2] De La Vega F.M., Galindo J.M., Old I.G., Guameros G. Gene 169:97-100(1996).
[3] Ouzounis C., Bork P., Casari G., Sander C. Protein Sci. 4:2424-2428(1995).

436. (Peptidase M17) Cytosol aminopeptidase signature

Cytosol aminopeptidase is a eukaryotic cytosolic zinc-dependent exopeptidase that catalyzes the removal of unsubstituted amino-acid residues from the N-terminus of proteins. This enzyme is often known as leucine aminopeptidase (EC 3.4.11.1) (LAP) but has been shown [1] to be identical with prolyl aminopeptidase (EC 3.4.11.5). Cytosol aminopeptidase is a hexamer of identical chains, each of which binds two zinc ions.

Cytosol aminopeptidase is highly similar to Escherichia coli pepA, a manganese dependent aminopeptidase. Residues involved in zinc ion-binding [2] in the mammalian enzyme are absolutely conserved in pepA where they presumably bind manganese.

A cytosol aminopeptidase from Rickettsia prowazekii [3] and one from Arabidopsis thaliana also belong to this family. As a signature pattern for these enzymes, a perfectly conserved octapeptide was selected which contains two residues involved in binding metal ions: an aspartate and a glutamate.

- Consensus pattern: N-T-D-A-E-G-R-L [The D and the E are zinc/manganese ligands]
- Note: these proteins belong to family M17 in the classification of peptidases [4,E1].

[1] Matsushima M., Takahashi T., Ichinose M., Miki K., Kurokawa K., Takahashi K. Biochem. Biophys. Res. Commun. 178:1459-1464(1991).
[2] Burley S.K., David P.R., Sweet R.M., Taylor A., Lipscomb W.N. J. Mol. Biol. 224:113-140(1992).
[3] Wood D.O., Solomon M.J., Speed R.R. J. Bacteriol. 175:159-165(1993).
[4] Rawlings N.D., Barrett A.J. Meth. Enzymol. 248:183-228(1995).

437. Assemblin (Peptidase family S21)

[1] Medline: 96399137
Three-dimensional structure of human cytomegalovirus protease.
Shieh HS, Kurumbail RG, Stevens AM, Stegeman RA, Sturman EJ, Pak JY, Wittwer AJ, Palmier MO, Wiegand RC, Holwerda BC, Stallings WC; Nature 1996;383:279-282.
Number of members: 29

438. Pollen proteins Ole e I family signature

The following plant pollen proteins, whose biological function is not yet known, are structurally related [1]:

- Olive tree pollen major allergen (Ole e I).
- Tomato anther-specific protein LAT52. - Maize pollen-specific protein ZmC13.

These proteins are most probably secreted and consist of about 145 residues.

As shown in the following schematic representation, there are six cysteines which are conserved in the sequence of these proteins. They seem to be involved in disulfide bonds.

xxxxxxCxCxxxxxxxxxCxxxxxxxxxxxxxxxxxCxxxxxCxxxxxxxxxxxxxxxxxxxxxCxxxxxxx
******'C': conserved cysteine involved in a disulfide bond.
'*': position of the pattern.

- Consensus pattern: [EQ]-G-x-V-Y-C-D-T-C-R [The two C's are probably involved in disulfide bonds]

[1] Villalba M., Batanero E., Lopez-Otin C., Sanchez L.M., Monsalve R.I., Gonzalez De La Pena M.A., Lahoz C., Rodriguez R. Eur. J. Biochem. 216:863-869(1993).

439. Pollen allergen
This family contains allergens lol PI, PII and PIII from Lolium perenne.
Number of members: 49

[1] Medline: 90105394
Complete primary structure of a Lolium perenne (perennial rye grass) pollen allergen, Lol p III: comparison with known Lol p I and II sequences.
Ansari AA, Shenbagamurthi P, Marsh DG; Biochemistry 1989;28:8665-8670.

440. Porphobilinogen deaminase cofactor-binding site
Porphobilinogen deaminase (EC 4.3.1.8), or hydroxymethylbilane synthase, is an enzyme involved in the biosynthesis of porphyrins and related macrocycles. It catalyzes the assembly of four porphobilinogen (PBG) units in a head to tail fashion to form hydroxymethylbilane.
The enzyme covalently binds a dipyrromethane cofactor to which the PBG subunits are added in a stepwise fashion. In the Escherichia coli enzyme (gene hemC), this cofactor has been shown [1] to be bound by the sulfur atom of a cysteine. The region around this cysteine is conserved in porphobilinogen deaminases from various prokaryotic and eukaryotic sources.

- Consensus pattern: E-R-x-[LIVMFA]-x(3)-[LIVMF]-x-G-[GSA]-C-x-[IVT]-P-[LIVMF]-[GSA] [C is the cofactor attachment site]

[1] Miller A.D., Hart G.J., Packman L.C., Battersby A.R. Biochem. J. 254:915-918(1988).

441. Presenilin
Mutations in presenilin-1 are a major cause of early onset Alzheimer's disease [2]. It has been found that presenilin-1 (Swiss:P49768) binds to beta-catenin in vivo [4]. This family also contains SPE proteins from C.elegans.
Number of members: 23

[1] Medline: 98045995
Presenilins and Alzheimer's disease.
Kim TW, Tanzi RE; Curr Opin Neurobiol 1997;7:683-688.
[2] Medline: 98045995
Presenilins and Alzheimer's disease.
Kim TW, Tanzi RE; Curr Opin Neurobiol 1997;7:683-688.
[3] Medline: 98099802
Interaction of presenilins with the filamin family of actin-binding proteins.
Zhang W, Han SW, McKeel DW, Goate A, Wu JY; J Neurosci 1998;18:914-922.
[4] Medline: 99004850
Destabilisation of beta-catenin by mutations in presenilin-1 potentiates neuronal apoptosis.
Zhang Z, Hartmann H, Do VM, Abramowski D, Sturchler-Pierrat C, Staufenbiel M, Sommer B, van de Wetering M, Clevers H, Saftig P, De Strooper B, He X, Yankner BA; Nature 1998;395:698-702.

442. (Pribosyltran) Purine/pyrimidine phosphoribosyl transferases signature Phosphoribosyltransferases (PRT) are enzymes that catalyze the synthesis of beta-n-5'-monophosphates from phosphoribosylpyrophosphate (PRPP) and an enzyme specific amine. A number of PRT's are involved in the biosynthesis of purine, pyrimidine, and pyridine nucleotides, or in the salvage of purines and pyrimidines. These enzymes are:

- Adenine phosphoribosyltransferase (EC 2.4.2.7) (APRT), which is involved in purine salvage.
- Hypoxanthine-guanine or hypoxanthine phosphoribosyltransferase (EC 2.4.2.8) (HGPRT or HPRT), which are involved in purine salvage.
- Orotate phosphoribosyltransferase (EC 2.4.2.10) (OPRT), which is involved in pyrimidine biosynthesis.
- Amido phosphoribosyltransferase (EC 2.4.2.14), which is involved in purine biosynthesis.
- Xanthine-guanine phosphoribosyltransferase (EC 2.4.2.22) (XGPRT), which is involved in purine salvage.

In the sequence of all these enzymes there is a small conserved region which may be involved in the enzymatic

activity and/or be part of the PRPP binding site [1].

- Consensus pattern: [LIVMFYWCTA]-[LIVM]-[LIVMA]-[LIVMFC]-[DE]-D-[LIVMS]-[LIVM]-[STAVD]-[STAR]-[GAC]-x-[STAR]
- Note: in position 11 of the pattern most of these enzymes have Gly.

[1] Hershey H.V., Taylor M.W. Gene 43:287-293(1986).

443. (Pro CA)
Prokaryotic-type carbonic anhydrases signatures
Carbonic anhydrases (EC 4.2.1.1) (CA) are zinc metalloenzymes which catalyze the reversible hydration of carbon dioxide. In Escherichia coli, CA (gene cynT) is involved in recycling carbon dioxide formed in the bicarbonate-dependent decomposition of cyanate by cyanase (gene cynS). By this action, it prevents the depletion of cellular bicarbonate [1]. In photosynthetic bacteria and plant chloroplast, CA is essential to inorganic carbon fixation [2]. Prokaryotic and plant chloroplast CA are structurally and evolutionary related and form a family distinct from the one which groups the many different forms of eukaryotic CA's (see <PDOC00146>). Hypothetical proteins yadF from Escherichia coli and HI1301 from Haemophilus influenzae also belong to this family. Two signature patterns were developed for this family of enzymes. Both patterns contain conserved residues that could be involved in binding zinc (cysteine and histidine).

- Consensus pattern: C-[SA]-D-S-R-[LIVM]-x-[AP]
- Consensus pattern: [EQ]-Y-A-[LIVM]-x(2)-[LIVM]-x(4)-[LIVMF](3)-x-G-H-x(2)-C-G

[1] Guilloton M.B., Korte J.J., Lamblin A.F., Fuchs J.A., Anderson P.M. J. Biol. Chem. 267:3731-3734(1992).
[2] Fukuzawa H., Suzuki E., Komukai Y., Miyachi S. Proc. Natl. Acad. Sci. U.S.A. 89:4437-4441 (1992).

444. (Prolyl_oligopep)
Prolyl oligopeptidase family serine active site
The prolyl oligopeptidase family [1,2,3] consist of a number of evolutionary related peptidases whose catalytic activity seems to be provided by a charge relay system similar to that of the trypsin family of serine proteases, but which evolved by independent convergent evolution. The known members of this family are listed below.

- Prolyl endopeptidase (EC 3.4.21.26) (PE) (also called post-proline cleaving enzyme). PE is an enzyme that cleaves peptide bonds on the C-terminal side of prolyl residues. The sequence of PE has been obtained from a mammalian species (pig) and from bacteria (Flavobacterium meningosepticum and Aeromonas hydrophila); there is a high degree of sequence conservation between these sequences.
- Escherichia coli protease II (EC 3.4.21.83) (oligopeptidase B) (gene prtB) which cleaves peptide bonds on the C-terminal side of lysyl and argininyl residues.
- Dipeptidyl peptidase IV (EC 3.4.14.5) (DPP IV). DPP IV is an enzyme that removes N-terminal dipeptides sequentially from polypeptides having unsubstituted N-termini provided that the penultimate residue is proline.
- Yeast vacuolar dipeptidyl aminopeptidase A (DPAP A) (gene: STE13) which is responsible for the proteolytic maturation of the alpha-factor precursor.
- Yeast vacuolar dipeptidyl aminopeptidase B (DPAP B) (gene: DAP2).
- Acylamino-acid-releasing enzyme (EC 3.4.19.1) (acyl-peptide hydrolase).

This enzyme catalyzes the hydrolysis of the amino-terminal peptide bond of an N-acetylated protein to generate a N-acetylated amino acid and a protein with a free amino-terminus.
A conserved serine residue has experimentally been shown (in E.coli proteaseII as well as in pig and bacterial PE) to be necessary for the catalytic mechanism. This serine, which is part of the catalytic triad (Ser, His, Asp), is generally located about 150 residues away from the C-terminal extremity of these enzymes (which are all proteins that contains about 700 to 800 amino acids).
Consensus pattern: D-x(3)-A-x(3)-[LIVMFYW]-x(14)-G-x-S-x-G-G-[LIVMFYW](2) [S is the active site residue] Sequences known to belong to this class detected by the pattern ALL, except for yeast DPAP A.
Note: these proteins belong to families S9A/S9B/S9C in the classification of peptidases [4].

[1] Rawlings N.D., Polgar L., Barrett A.J. Biochem. J. 279:907-911(1991).
[2] Barrett A.J., Rawlings N.D.
[3] Polgar L., Szabo E.

[4] Rawlings N.D., BarrettA.J. Meth. Enzymol. 244:19-61(1994).

445. (Pterin 4a)

Pterin 4 alpha carbinolamine dehydratase

Pterin 4 alpha carbinolamine dehydratase is aka DCoH (dimerisation cofactor of hepatocyte nuclear factor 1-alpha). Number of members: 11

[1] Cronk JD, Endrizzi JA, Alber T; Medline: 97052967 "High-resolution structures of the bifunctional enzyme and transcriptional coactivator DCoH and its complex with a product analogue." Protein Sci 1996;5:1963-1972.

446. (Pyridox oxidase)

Pyridoxamine 5'-phosphate oxidase signature

Pyridoxamine 5'-phosphate oxidase (EC 1.4.3.5) is a FMN flavoprotein involved in the de novo synthesis of pyridoxine (vitamin B6) and pyridoxal phosphate. It oxidizes pyridoxamine-5-P (PMP) and pyridoxine-5-P (PNP) to pyridoxal-5-P. The sequences of the enzyme from bacterial (genes pdxH or fprA) [1] and fungal (gene PDX3) [2] sources show that this protein has been highly conserved throughout evolution.

PdxH is evolutionary related [3] to one of the enzymes in the phenazine biosynthesis protein pathway, phzD (also known as phzG). As a signature pattern, a highly conserved region was selected located in the C-terminal part of these enzymes.

- Consensus pattern: [LIVF]-E-F-W-[QHG]-x(4)-R-[LIVM]-H-[DNE]-R

[1] Lam H.-M., Winkler M.E. J. Bacteriol. 174:6033-6045(1992).
[2] Loubbardi A., Karst F., Guilloton M., Marcireau C. J. Bacteriol. 177:1817-1823(1995).
[3] Pierson L.S. III, Gaffney T., Lam S., Gong F. FEMS Microbiol. Lett. 134:299-307(1995).

447. (Pyrophosphatase)

Inorganic pyrophosphatase signature

Inorganic pyrophosphatase (EC 3.6.1.1) (PPase) [1,2] is the enzyme responsible for the hydrolysis of pyrophosphate (PPi) which is formed principally as the product of the many biosynthetic reactions that utilize ATP. All known Ppases require the presence of divalent metal cations, with magnesium conferring the highest activity. Among other residues, a lysine has been postulated to be part or close to the active site. PPases have been sequenced from bacteria such as Escherichia coli (homohexamer), thermophilic bacteria PS-3 and Thermus thermophilus, from the archaebacteria Thermoplasma acidophilum, from fungi (homodimer), from a plant, and from bovine retina. In yeast, a mitochondrial isoform of PPase has been characterized which seems to be involved in energy production and whose activity is stimulated by uncouplers of ATP synthesis.

The sequences of PPases share some regions of similarities. As signature patterns a region was selected that contains three conserved aspartates that are involved in the binding of cations.

- Consensus pattern: D-[SGDN]-D-[PE]-[LIVMF]-D-[LIVMGAC]

[The three D's bind divalent metal cations]

[1] Lahti R., Kolakowski L.F. Jr., Heinonen J., Vihinen M., Pohjanoksa K., Cooperman B.S. Biochim. Biophys. Acta 1038:338-345(1990).
[2] Cooperman B.S., Baykov A.A., Lahti R. Trends Biochem. Sci. 17:262-266(1992).

448. (Peptidase S26)

Signal peptidases I signatures.

Signal peptidases (SPases) [1] (aka leader peptidases) remove the signal peptides from secretory proteins. In prokaryotes three types of SPasesare known: type I (gene lepB) which is responsible for the processing of the majority of exported pre-proteins; type II (gene Isp) which only process lipoproteins, and a third type involved in the processing of pili subunits. SPase I (EC 3.4.21.89) is an integral membrane protein that is anchored in the cytoplasmic membrane by one (in B. subtilis) or two (in E. coli) N-terminal transmembrane domains with the main part of the protein protuding in the periplasmic space. Two residues have been shown [2,3] to be essential for the catalytic activity of SPase I: a serine and an lysine. SPase I is evolutionary related to the yeast mitochondrial inner membrane protease subunit 1 and 2 (genes IMP1 and IMP2) which catalyze the removal of signal peptides required for the targeting of proteins from the mitochondrial matrix, across the inner membrane, into the inter-membrane space [4].In

eukaryotes the removal of signal peptides is effected by an oligomeric enzymatic complex composed of at least five subunits: the signal peptidase complex (SPC). The SPC is located in the endoplasmic reticulum membrane. Two components of mammalian SPC, the 18 Kd (SPC18) and the 21 Kd (SPC21) subunits as well as the yeast SEC11 subunit have been shown [5] to share regions of sequence similarity with prokaryotic SPases I and yeast IMP1/IMP2. Three signature patterns have been developed for these proteins. The first signature contains the putative active site serine, the second signature contains the putative active site lysine which is not conserved in the SPC subunits, and the third signature corresponds to a conserved region of unknown biological significance which is located in the C-terminal section of all these proteins.
Consensus pattern: [GS]-x-S-M-x-[PS]-[AT]-[LF] [S is an active site residue]-
Consensus pattern: K-R-[LIVMSTA](2)-G-x-[PG]-G-[DE]-x-[LIVM]-x-[LIVMFY] [K is an active site residue]-
Consensus pattern: [LIVMFYW](2)-x(2)-G-D-[NH]-x(3)-[SND]-x(2)-[SG]-

[1] Dalbey R.E., von Heijne G. Trends Biochem. Sci. 17:474-478(1992).
[2] Sung M., Dalbey R.E. J. Biol. Chem. 267:13154-13159(1992).
[3] Black M.T. J. Bacteriol. 175:4957-4961(1993).
[4] Nunnari J., Fox T.D., Walter P. Science 262:1997-2004(1993).
[5] van Dijl J.M., de Jong A., Vehmaanpera J., Venema G., Bron S. EMBO J. 11:2819-2828(1992).
[6] RawlingsN.D., BarrettA.J. Meth. Enzymol. 244:19-61(1994).[E1]

449. (Peptidase C1) Eukaryotic thiol (cysteine) proteases active sites. Eukaryotic thiol proteases (EC 3.4.22.-) [1] are a family of proteolytic enzymes which contain an active site cysteine. Catalysis proceeds through a thioester intermediate and is facilitated by a nearby histidine side chain; an asparagine completes the essential catalytic triad. The proteases which are currently known to belong to this family are listed below (references are only provided for recently determined sequences). - Vertebrate lysosomal cathepsins B (EC 3.4.22.1), H (EC 3.4.22.16), L (EC 3.4.22.15), and S (EC 3.4.22.27) [2]. - Vertebrate lysosomal dipeptidyl peptidase I (EC 3.4.14.1) (also known as cathepsin C) [2]. - Vertebrate calpains (EC 3.4.22.17). Calpains are intracellular calcium- activated thiol protease that contain both a N-terminal catalytic domain and a C-terminal calcium-binding domain. - Mammalian cathepsin K, which seems involved in osteoclastic bone resorption [3]. - Human cathepsin O [4]. - Bleomycin hydrolase. An enzyme that catalyzes the inactivation of the antitumor drug BLM (a glycopeptide). - Plant enzymes: barley aleurain (EC 3.4.22.16), EP-BI/B4; kidney bean EP-C1, rice bean SH-EP; kiwi fruit actinidin (EC 3.4.22.14); papaya latex papain (EC 3.4.22.2), chymopapain (EC 3.4.22.6), caricain (EC 3.4.22,30), and proteinase IV (EC 3.4.22.25); pea turgor-responsive protein 15A; pineapple stem bromelain (EC 3.4.22.32); rape COT44; rice oryzain alpha, beta, and gamma; tomato low-temperature induced, Arabidopsis thaliana A494, RD19A and RD21A. - House-dust mites allergens DerP1 and EurM1. - Cathepsin B-like proteinases from the worms Caenorhabditis elegans (genes gcp-1, cpr-3, cpr-4, cpr-5 and cpr-6), Schistosoma mansoni (antigen SM31) and Japonica (antigen SJ31), Haemonchus contortus (genes AC-1 and AC-2), and Ostertagia ostertagi (CP-1 and CP-3). - Slime mold cysteine proteinases CP1 and CP2. - Cruzipain from Trypanosoma cruzi and brucei. - Throphozoite cysteine proteinase (TCP) from various Plasmodium species. - Proteases from Leishmania mexicana, Theileria annulata and Theileria parva. - Baculoviruses cathepsin-like enzyme (v-cath). - Drosophila small optic lobes protein (gene sol), a neuronal protein that contains a calpain-like domain. - Yeast thiol protease BLH1/YCP1/LAP3. - Caenorhabditis elegans hypothetical protein C06G4.2, a calpain-like protein. Two bacterial peptidases are also part of this family: -
Aminopeptidase C from Lactococcus lactis (gene pepC) [5]. - Thiol protease tpr from Porphyromonas gingivalis. Three other proteins are structurally related to this family, but may have lost their proteolytic activity. - Soybean oil body protein P34. This protein has its active site cysteine replaced by a glycine. - Rat testin, a sertoli cell secretory protein highly similar to cathepsin L but with the active site cysteine is replaced by a serine. Rat testin should not be confused with mouse testin which is a LIM-domain protein (see <PDOC00382>). - Plasmodium falciparum serine-repeat protein (SERA), the major blood stage antigen. This protein of 111 Kd possesses a C-terminal thiol-protease-like domain [6], but the active site cysteine is replaced by a serine. The sequences around the three active site residues are well conserved and can be used as signature patterns.
Consensus pattern: Q-x(3)-[GE]-x-C-[YW]-x(2)-[STAGC]-[STAGCV] [C is the active site residue]- Note: the residue in position 4 of the pattern is almost always cysteine; the only exceptions are calpains (Leu), bleomycin hydrolase (Ser) and yeast YCP1 (Ser). -Note: the residue in position 5 of the pattern is always Gly except in papaya protease IV where it is Glu.
Consensus pattern: [LIVMGSTAN]-x-H-[GSACE]-[LIVM]-x-[LIVMAT](2)-G-x-[GSADNH] [H is the active site residue]-
Consensus pattern: [FYCH]-[WI]-[LIVT]-x-[KRQAG]-N-[ST]-W-x(3)-[FYW]-G-x(2)-G-[LFYW]-[LIVMFYG]-x-[LIVMF] [N is the active site residue] - Note: these proteins belong to family C1 (papain-type) and C2 (calpains) in the classification of peptidases [7,E1].-

[1] Dufour E. Biochimie 70:1335-1342(1988).
[2] Kirschke H., Barrett A.J., Rawlings N.D. Protein Prof. 2:1587-1643(1995).
[3] Shi G.-P., Chapman H.A., Bhairi S.M., Deleeuw C., Reddy V.Y., Weiss S.J. FEBS Lett. 357:129-134(1995).
[4] Velasco G., Ferrando A.A., Puente X.S., Sanchez L.M., Lopez-Otin C. J. Biol. Chem. 269:27136-27142(1994).
[5] Chapot-Chartier M.P., Nardi M., Chopin M.C., Chopin A., Gripon J.C. Appl. Environ. Microbiol. 59:330-333 (1993).
[6] Higgins D.G., McConnell D.J., Sharp P.M. Nature 340:604-604(1989).
[7] Rawlings N.D., Barrett A.J. Meth. Enzymol. 244:461-486(1994).

450. (peptidase M24) Aminopeptidase P and proline dipeptidase signature (1). Aminopeptidase P (EC 3.4.11.9) is the enzyme responsible for the release of any N-terminal amino acid adjacent to a proline residue. Proline dipeptidase (EC 3.4.13.9) (prolidase) splits dipeptides with a prolyl residue in the carboxyl terminal position. Bacterial aminopeptidase P II (gene pepP) [1], proline dipeptidase (gene pepQ)[2], and human proline dipeptidase (gene PEPD) [3] are evolutionary related. These proteins are manganese metalloenzymes. Yeast hypothetical proteins YER078c and YFR006w and Mycobacterium tuberculosis hypothetical protein MtCY49.29c also belong to this family. As a signature pattern for these enzymes a conserved region that contains three histidine residues has been developed
Consensus pattern: [HA]-[GSYR]-[LIVMT]-[SG]-H-x-[LIV]-G-[LIVM]-x-[IV]-H-[DE]-

[1] Yoshimoto T., Tone H., Honda T., Osatomi K., Kobayashi R., Tsuru D. J. Biochem. 105:412-416(1989).
[2] Nakahigashi K., Inokuchi H. Nucleic Acids Res. 18:6439-6439(1990).
[3] Endo F., Tanoue A., Nakai H., Hata A., Indo Y., Titani K., Matsuda I. J. Biol. Chem. 264:4476-4481(1989).
[4] Rawlings N.D., Barrett A.J. Meth. Enzymol. 248:183-228(1995).

Methionine aminopeptidase signatures. (2). Methionine aminopeptidase (EC 3.4.11.18) (MAP) is responsible for the removal of the amino-terminal (initiator) methionine from nascent eukaryotic cytosolic and cytoplasmic prokaryotic proteins if the penultimate amino acid is small and uncharged. All MAP studied to date are monomeric proteins that require cobalt ions for activity. Two subfamilies of MAP enzymes are known to exist [1,2]. While being evolutionary related, they only share a limited amount of sequence similarity mostly clustered around the residues shown, in the Escherichia coli MAP [3],to be involved in cobalt-binding. The first family consists of enzymes from prokaryotes as well as eukaryoticMAP-1, while the second group is made up of archebacterial MAP and eukaryoticMAP-2. The second subfamily also includes proteins which do not seem to be MAP, but that are clearly evolutionary related such as mouse proliferation-associated protein 1 and fission yeast curved DNA-binding protein. For each of these subfamilies, a specific signature pattern that includes residues known to be involved in colbalt-binding has been developed.
Consensus pattern: [MFY]-x-G-H-G-[LIVMC]-[GSH]-x(3)-H-x(4)-[LIVM]-x-[HN]-[YWV] [H is a cobalt ligand]-
Consensus pattern: [DA]-[LIVMY]-x-K-[LIVM]-D-x-G-x-[HQ]-[LIVM]-[DNS]-G-x(3)-[DN] [The second D and the last D/N are cobalt ligands]

[1] Arfin S.M., Kendall R.L., Hall L., Weaver L.H., Stewart A.E., Matthews B.W., Bradshaw R.A. Proc. Natl. Acad. Sci. U.S.A. 92:7714-7718(1995).
[2] Keeling P.J., Doolittle W.F. Trends Biochem. Sci. 21:285-286(1996).
[3] Roderick S.L., Mathews B.W. Biochemistry 32:3907-3912(1993).
[4] Rawlings N.D., Barrett A.J. Meth. Enzymol. 248:183-228(1995).

451. Cytochrome P450 cysteine heme-iron ligand signature
Cytochrome P450's [1,2,3,E1 are a group of enzymes involved in the oxidative metabolism of a high number of natural compounds (such as steroids, fatty acids, prostaglandins, leukotrienes, etc) as well as drugs, carcinogens and mutagens. Based on sequence similarities, P450's have been classified into about forty different families [4,5]. P450's are proteins of 400 to 530 amino acids; the only exception is Bacillus BM-3 (CYP102) which is a protein of 1048residues that contains a N-terminal P450 domain followed by a reductase domain. P450's are heme proteins. A conserved cysteine residue in the C-terminal part of P450's is involved in binding the heme iron in the fifth coordination site. From a region around this residue, a ten residue signature was developed specific to P450's.
Consensus pattern: [FW]-[SGNH]-x-[GD]-x-[RHPT]-x-C-[LIVMFAP]-[GAD] [C is the heme iron ligand]-

[1] Nebert D.W., Gonzalez F.J. Annu. Rev. Biochem. 56:945-993(1987).
[2] Coon M.J., Ding X., Pernecky S.J., Vaz A.D.N. FASEB J. 6:669-673(1992).
[3] Guengerich F.P. J. Biol. Chem. 266:10019-10022(1991).
[4] Nelson D.R., Kamataki T., Waxman D.J., Guengerich F.P., Estrabrook R.W., Feyereisen R., Gonzalez F.J.,

Coon M.J., Gunsalus I.C., Gotoh O., Okuda K., Nebert D.W. DNA Cell Biol. 12:1-51(1993).
[5] Degtyarenko K.N., Archakov A.I. FEBS Lett. 332:1-8(1993).

452. (Pec Lyase) Pectate lyase
This enzyme forms a right handed beta helix structure. Pectate lyase is an enzyme involved in the maceration and soft rotting of plant tissue.

[1] Yoder MD, Keen NT, Jurnak F, Science 1993;260:1503-1507.

453. (pep M24) Aminopeptidase P and proline dipeptidase signature (pep1)
Aminopeptidase P (EC 3.4.11.9) is the enzyme responsible for the release of any N-terminal amino acid adjacent to a proline residue. Proline dipeptidase(EC 3.4.13.9) (prolidase) splits dipeptides with a prolyl residue in the carboxyl terminal position. Bacterial aminopeptidase P II (gene pepP) [1], proline dipeptidase (gene pepQ)[2], and human proline dipeptidase (gene PEPD) [3] are evolutionary related. These proteins are manganese metalloenzymes. Yeast hypothetical proteins YER078c and YFR006w and Mycobacterium tuberculosis hypothetical protein MtCY49.29c also belong to this family. As a signature pattern for these enzymes a conserved region was selected that contains three histidine residues.
Consensus pattern: [HA]-[GSYR]-[LIVMT]-[SG]-H-x-[LIV]-G-[LIVM]-x-[IV]-H-[DE]-

[1] Yoshimoto T., Tone H., Honda T., Osatomi K., Kobayashi R., Tsuru D. J. Biochem. 105:412-416(1989).
[2] Nakahigashi K., Inokuchi H. Nucleic Acids Res. 18:6439-6439(1990).
[3] Endo F., Tanoue A., Nakai H., Hata A., Indo Y., Titani K., Matsuda I. J. Biol. Chem. 264:4476-4481(1989).
[4] Rawlings N.D., Barrett A.J. Meth. Enzymol. 248:183-228(1995).

Methionine aminopeptidase signatures (pep2)
Methionine aminopeptidase (EC 3.4.11.18) (MAP) is responsible for the removal of the amino-terminal (initiator) methionine from nascent eukaryotic cytosolic and cytoplasmic prokaryotic proteins if the penultimate amino acid is small and uncharged. All MAP studied to date are monomeric proteins that require cobalt ions for activity. Two subfamilies of MAP enzymes are known to exist [1,2]. While being evolutionary related, they only share a limited amount of sequence similarity mostly clustered around the residues shown, in the Escherichia coli MAP [3],to be involved in cobalt-binding. The first family consists of enzymes from prokaryotes as well as eukaryotic MAP-1, while the second group is made up of archebacterial MAP and eukaryotic MAP-2. The second subfamily also includes proteins which do not seem to be MAP, but that are clearly evolutionary related such as mouse proliferation-associated protein 1 and fission yeast curved DNA-binding protein. For each of these subfamilies, a specific signature pattern was developed that includes residues known to be involved in colbalt-binding.
Consensus pattern: [MFY]-x-G-H-G-[LIVMC]-[GSH]-x(3)-H-x(4)-[LIVM]-x-[HN]-[YWV] [H is a cobalt ligand]-
Consensus pattern: [DA]-[LIVMY]-x-K-[LIVM]-D-x-G-x-[HQ]-[LIVM]-[DNS]-G-x(3)-[DN] [The second D and the last D/N are cobalt ligands]

[1] Arfin S.M., Kendall R.L., Hall L., Weaver L.H., Stewart A.E., Matthews B.W., Bradshaw R.A. Proc. Natl. Acad. Sci. U.S.A. 92:7714-7718(1995).
[2] Keeling P.J., Doolittle W.F. Trends Biochem. Sci. 21:285-286(1996).
[3] Roderick S.L., Mathews B.W. Biochemistry 32:3907-3912(1993).
[4] Rawlings N.D., Barrett A.J. Meth. Enzymol. 248:183-228(1995).

454. Peroxidases signatures
Peroxidases (EC 1.11.1.-) [1] are heme-binding enzymes that carry out a variety of biosynthetic and degradative functions using hydrogen peroxide as the electron acceptor. Peroxidases are widely distributed throughout bacteria, fungi, plants, and vertebrates. In peroxidases the heme prosthetic group is protoporphyrin IX and the fifth ligand of the heme iron is a histidine (known as the proximal histidine). Another histidine residue (the distal histidine) serves as an acid-base catalyst in the reaction between hydrogen peroxide and the enzyme. The regions around these two active site residues are more or less conserved in a majority of peroxidases [2,3]. The enzymes in which one or both of these regions can be found are listed below. - Yeast cytochrome c peroxidase (EC 1.11.1.5).- Myeloperoxidase (EC 1.11.1.7) (MPO). MPO is found in granulocytes and monocytes and plays a major role in the oxygen-dependent microbicidal system of neutrophils. - Lactoperoxidase (EC 1.11.1.7) (LPO). LPO is a milk protein which acts as an antimicrobial agent. - Eosinophil peroxidase (EC 1.11.1.7) (EPO). An enzyme found in the cytoplasmic granules of eosinophils. - Thyroid peroxidase (EC 1.11.1.8) (TPO). TPO plays a central role in the biosynthesis of thyroid hormones. It catalyzes the iodination and coupling of the hormonogenic tyrosines in thyroglobulin to yield

the thyroid hormones T3 and T4. - Fungal ligninases. Ligninase catalyzes the first step in the degradation of lignin. It depolymerizes lignin by catalyzing the C(alpha)-C(beta) cleavage of the propyl side chains of lignin. - Plant peroxidases (EC 1.11.1.7). Plants expresses a large numbers of isozymes of peroxidases. Some of them play a role in cell-suberization by catalyzing the deposition of the aromatic residues of suberin on the cell wall, some are expressed as a defense response toward wounding, others are involved in the metabolism of auxin and the biosynthesis of lignin. - Prokaryotic catalase-peroxidases. Some bacterial species produce enzymes that exhibit both catalase and broad-spectrum peroxidase activities [4]. Examples of such enzymes are: catalase HP I from Escherichia coli (gene katG) and perA from Bacillus stearothermophilus.
Consensus pattern: [DET]-[LIVMTA]-x(2)-[LIVM]-[LIVMSTAG]-[SAG]-[LIVMSTAG]-H- [STA]-[LIVMFY] [H is the proximal heme-binding ligand] -
Consensus pattern: [SGATV]-x(3)-[LIVMA]-R-[LIVMA]-x-[FW]-H-x-[SAC] [H is an active site residue]-

[1] Dawson J.H. Science 240:433-439(1988).
[2] Kimura S., Ikeda-Saito M. Proteins 3:113-120(1988).
[3] Henrissat B., Saloheimo M., Lavaitte S., Knowles J.K.C. Proteins 8:251-257(1990).
[4] Welinder K.G. Biochim. Biophys. Acta 1080:215-220(1991).

455. pfkB family of carbohydrate kinases signatures
It has been shown [1,2,3] that the following carbohydrate and purine kinasesare evolutionary related and can be grouped into a single family, which isknown [1] as the 'pfkB family': - Fructokinase (EC 2.7.1.4) (gene scrK). - 6-phosphofructokinase isozyme 2 (EC 2.7.1.11) (phosphofructokinase-2) (gene pfkB). pfkB is a minor phosphofructokinase isozyme in Escherichia coli and is not evolutionary related to the major isozyme (gene pfkA). Plants 6-phosphofructokinase also belong to this family. - Ribokinase (EC 2.7.1.15) (gene rbsK). - Adenosine kinase (EC 2.7.1.20) (gene ADK). - 2-dehydro-3-deoxygluconokinase (EC 2.7.1.45) (gene: kdgK). - 1-phosphofructokinase (EC 2.7.1.56) (fructose 1-phosphate kinase) (gene fruK). - Inosine-guanosine kinase (EC 2.7.1.73) (gene gsk). - Tagatose-6-phosphate kinase (EC 2.7.1.144) (phosphotagatokinase) (gene lacC). - Escherichia coli hypothetical protein yeiC. - Escherichia coli hypothetical protein yeiI. - Escherichia coli hypothetical protein yhfQ. - Escherichia coli hypothetical protein yihV. - Bacillus subtilis hypothetical protein yxdC. - Yeast hypothetical protein YJR105w.All the above kinases are proteins of from 280 to 430 amino acid residues that share a few region of sequence similarity. Two of these regions were selected as signature patterns. The first pattern is based on a region rich in glycine which is located in the N-terminal section of these enzymes; while the second pattern is based on a conserved region in the C-terminal section.
Consensus pattern: [AG]-G-x(0,1)-[GAP]-x-N-x-[STA]-x(6)-[GS]-x(9)-G-
Consensus pattern: [DNSK]-[PSTV]-x-[SAG](2)-[GD]-D-x(3)-[SAGV]-[AG]-[LIVMFYA]-[LIVMSTAP]

[1] Wu L.-F., Reizer A., Reizer J., Cai B., Tomich J.M., Saier M.H. Jr. J. Bacteriol. 173:3117-3127(1991).
[2] Orchard L.M.D., Kornberg H.L. Proc. R. Soc. Lond., B, Biol. Sci. 242:87-90(1990).
[3] Blatch G.L., Scholle R.R., Woods D.R. Gene 95:17-23(1990).

456. Phospholipase A2 active sites signatures
Phospholipase A2 (EC 3.1.1.4) (PA2) [1,2] is an enzyme which releases fatty acids from the second carbon group of glycerol. PA2's are small and rigid proteins of 120 amino-acid residues that have four to seven disulfide bonds.PA2 binds a calcium ion which is required for activity. The side chains of two conserved residues, a histidine and an aspartic acid, participate in a 'catalytic network'. Many PA2's have been sequenced from snakes, lizards, bees and mammals. In the latter, there are at least four forms: pancreatic, membrane-associated as well as two less characterized forms. The venom of most snakes contains multiple forms of PA2. Some of them are presynaptic neurotoxins which inhibit neuromuscular transmission by blocking acetylcholine release from the nerve termini. Two different signature patterns were derived for PA2's. The first is centered on the active site histidine and contains three cysteines involved in disulfide bonds. The second is centered on the active site aspartic acid and also contains three cysteines involved in disulfide bonds.
Consensus pattern: C-C-x(2)-H-x(2)-C [H is the active site residue] This pattern will not detect some snake toxins homologous with PA2 but which have lost their catalytic activity as well as otoconin-22, a Xenopus protein from the aragonitic otoconia which is also unlikely to be enzymatically active.
Consensus pattern: [LIVMA]-C-{LIVMFYWPCST}-C-D-x(5)-C [D is the active site residue] The majority of functional and non-functional PA2's. Undetected sequences are bee PA2, gila monster PA2's, PA2 PL-X from habu and PA2 PA-5 from mulga.

[1] DavidsonF.F., Dennis E.A. J. Mol. Evol. 31:228-238(1990).

[2] Gomez F., Vandermeers A., Vandermeers-Piret M.-C., Herzog R., Rathe J., Stievenart M., Winand J., Christophe J. Eur. J. Biochem. 186:23-33(1989).

457. Phosphorylase pyridoxal-phosphate attachment site. Phosphorylases (EC 2.4.1.1 ) [1] are important allosteric enzymes in carbohydrate metabolism. They catalyze the formation of glucose 1-phosphatefrom polyglucose such as glycogen, starch or maltodextrin. Enzymes from different sources differ in their regulatory mechanisms and their natural substrates. However, all known phosphorylases share catalytic and structural properties. They are pyridoxal-phosphate dependent enzymes; the pyridoxal-P group is attached to a lysine residue around which the sequence is highly conserved and can be used as a signature pattern to detect this class of enzymes.
Consensus pattern: E-A-[SC]-G-x-[GS]-x-M-K-x(2)-[LM]-N [K is the pyridoxal-P attachment site]-

[1] Fukui T., Shimomura S., Nakano K. Mol. Cell. Biochem. 42:129-144(1982).

458. Protein kinases signatures and profile
Eukaryotic protein kinases [1 to 5] are enzymes that belong to a very extensive family of proteins which share a conserved catalytic core common toboth serine/threonine and tyrosine protein kinases. There are a number ofconserved regions in the catalytic domain of protein kinases. Two of these regions were selected to build signature patterns. The first region, which is located in the N-terminal extremity of the catalytic domain, is a glycine-rich stretch of residues in the vicinity of a lysine residue, which has been shown to be involved in ATP binding. The second region, which is located in the central part of the catalytic domain, contains a conserved aspartic acid residue which is important for the catalytic activity of the enzyme [6]; Two signature patterns were derived for that region: one specific for serine/threonine kinases and the other for tyrosine kinases. A profile was also developed which is based on the alignment in [1] and covers the entire catalytic domain.
Consensus pattern: [LIV]-G-{P}-G-{P}-[FYWMGSTNH]-[SGA]-{PW}-[LIVCAT]-{PD}-x- [GSTACLIVMFY]-x(5,18)-[LIVMFYWCSTAR]-[AIVP]-[LIVMFAGCKR]-K [K binds ATP]. The majority of known protein kinases belong to the class detected by this pattern, but it fails to find a number of them, especially viral kinases which are quite divergent in this region and are completely missed by this pattern.
Consensus pattern: [LIVMFYC]-x-[HY]-x-D-[LIVMFY]-K-x(2)-N-[LIVMFYCT](3) [D is an active site residue]. Most serine/ threonine specific protein kinases belong to this class detected by the pattern with 10 exceptions (half of them viral kinases) and also Epstein-Barr virus BGLF4 and Drosophila ninaC which have respectively Ser and Arg instead of the conserved Lys and which are therefore detected by the tyrosine kinase specific pattern described below.
Consensus pattern: [LIVMFYC]-x-[HY]-x-D-[LIVMFY]-[RSTAC]-x(2)-N-[LIVMFYC](3) [D is an active site residue] ALL tyrosine specific protein kinases with the exception of human ERBB3 and mouse blk belong to this class detected by the pattern. This pattern will also detect most bacterial aminoglycoside phosphotransferases [8,9] and herpesviruses gangciclovir kinases [10]; which are proteins structurally and evolutionary related to protein kinases. This profile also detects receptor guanylate cyclases and 2-5A-dependent ribonucleases. Sequence similarities between these two families and the eukaryotic protein kinase family have been noticed before. It also detects Arabidopsis thaliana kinase- like protein TMKL1 which seems to have lost its catalytic activity. If a protein analyzed includes the two protein kinase signatures, the probability of it being a protein kinase is close to 100%. Eukaryotic-type protein kinases have also been found in prokaryotes such as Myxococcus xanthus [11] and Yersinia pseudotuberculosis.

[1] Hanks S.K., Hunter T. FASEB J. 9:576-596(1995).
[2] Hunter T. Meth. Enzymol. 200:3-37(1991).
[3] Hanks S.K., Quinn A.M. Meth. Enzymol. 200:38-62(1991).
[4] Hanks S.K. Curr. Opin. Struct. Biol. 1:369-383(1991).
[5] Hanks S.K., Quinn A.M., Hunter T. Science 241:42-52(1988).
[6] Knighton D.R., Zheng J., Ten Eyck L.F., Ashford V.A., Xuong N.-H., Taylor S.S., Sowadski J.M. Science 253:407-414(1991).
[7] Bairoch A., Claverie J.-M. Nature 331:22(1988).
[8] Benner S. Nature 329:21-21(1987).
[9] Kirby R. J. Mol. Evol. 30:489-492(1992).
[10] Littler E., Stuart A.D., Chee M.S. Nature 358:160-162(1992).
[11] Munoz-Dorado J., Inouye S., Inouye M. Cell 67:995-1006(1991).

Receptor tyrosine kinase class II signature
A number of growth factors stimulate mitogenesis by interacting with a familyof cell surface receptors which possess an intrinsic, ligand-sensitive, protein tyrosine kinase activity [1]. These receptor tyrosine kinases (RTK)all share the same topology: an extracellular ligand-binding domain, a single transmembrane region and a cytoplasmic kinase

domain. However they can be classified into at least five groups. The prototype for class II RTK's is the insulin receptor, a heterotetramer of two alpha and two beta chains linked by disulfide bonds. The alpha and beta chains are cleavage products of a precursor molecule. The alpha chain contains the ligand binding site, the beta chain transverses the membrane and contains the tyrosine protein kinase domain. The receptors currently known to belong to class II are: - Insulin receptor from vertebrates. - Insulin growth factor I receptor from mammals. - Insulin receptor-related receptor (IRR), which is most probably a receptor for a peptide belonging to the insulin family. - Insects insulin-like receptors. - Molluscan insulin-related peptide(s) receptor (MIP-R). - Insulin-like peptide receptor from Branchiostoma lanceolatum. - The Drosophila developmental protein sevenless, a putative receptor for positional information required for the formation of the R7 photoreceptor cells. - The trk family of receptors (NTRK1, NTRK2 and NTRK3), which are high affinity receptors for nerve growth factor and related neurotrophic factors (BDNF and NT-3).And the following uncharacterized receptors: - ROS. - LTK (TYK1). - EDDR1 (cak, TRKE, RTK6). - NTRK3 (Tyro10, TKT). - A sponge putative receptor tyrosine kinase. While only the insulin and the insulin growth factor I receptors are known to exist in the tetrameric conformation specific to class II RTK's, all the above proteins share extensive homologies in their kinase domain, especially around the putative site of autophosphorylation. Hence, a signature pattern was developed for this class of RTK's, which includes the tyrosine residue, itself probably autophosphorylated.

Consensus pattern: [DN]-[LIV]-Y-x(3)-Y-Y-R [The second Y is the autophosphorylation site]

[1] Yarden Y., Ullrich A. Annu. Rev. Biochem. 57:443-478(1988).

Receptor tyrosine kinase class III signature

A number of growth factors stimulate mitogenesis by interacting with a family of cell surface receptors which possess an intrinsic, ligand-sensitive, protein tyrosine kinase activity [1]. These receptor tyrosine kinases (RTK)all share the same topology: an extracellular ligand-binding domain, a single transmembrane region and a cytoplasmic kinase domain. However they can be classified into at least five groups. The class III RTK's are characterized by the presence of five to seven immunoglobulin-like domains [2] in their extracellular section. Their kinase domain differs from that of other RTK's by the insertion of a stretch of 70 to 100 hydrophilic residues in the middle ofthis domain. The receptors currently known to belong to class III are: - Platelet-derived growth factor receptor (PDGF-R). PDGF-R exists as a homo- or heterodimer of two related chains: alpha and beta [3]. - Macrophage colony stimulating factor receptor (CSF-1-R) (also known as the fms oncogene). - Stem cell factor (mast cell growth factor) receptor (also known as the kit oncogene). - Vascular endothelial growth factor (VEGF) receptors Flt-1 and Flk-1/KDR [4].

- F1 cytokine receptor Flk-2/Flt-3 [5]. - The putative receptor Flt-4 [7]. a signature pattern Was developed for this class of RTK's which is based on a conserved region in the kinase domain.

Consensus pattern: G-x-H-x-N-[LIVM]-V-N-L-L-G-A-C-T-

[1] Yarden Y., Ullrich A. Annu. Rev. Biochem. 57:443-478(1988).
[2] Hunkapiller T., Hood L. Adv. Immunol. 44:1-63(1989).
[3] Lee K.-H., Bowen-Pope D.F., Reed R.R. Mol. Cell. Biol. 10:2237-2246(1990).
[4] Terman B.I., Dougher-Vermazen M., Carrion M.E., Dimitrov D., Armellino D.C., Gospodarowicz D., Boehlen P. Biochem. Biophys. Res. Commun. 187:1579-1586(1992).
[5] Lyman S.D., James L., Vanden Bos T., de Vries P., Brasel K., Gliniak B., Hollingsworth L.T., Picha K.S., McKenna H.J., Splett R.R. Cell 75:1157-1167(1993).
[6] Galland F., Karamysheva A., Pebusque M.J., Borg J.P., Rottapel R., Dubreuil P., Rosnet O., Birnbaum D. Oncogene 8:1233-1240(1993).

Receptor tyrosine kinase class V signatures

A number of growth factors stimulate mitogenesis by interacting with a familyof cell surface receptors which possess an intrinsic, ligand-sensitive, protein tyrosine kinase activity [1]. These receptor tyrosine kinases (RTK)all share the same topology: an extracellular ligand-binding domain, a single transmembrane region and a cytoplasmic kinase domain. However they can be classified into at least five groups on the basis of sequence similarities. The extracellular domain of class V RTK's consist of a region of about 300amino acids, amongst which 16 conserved cysteines probably involved in disulfide bonds; this region is followed by two copies of a fibronectin typeIII domain. The ligands for these receptors are proteins of about 200 to 300residues collectively known as Ephrins. The receptors currently known to belong to class V are [2,3,E1]: - EPHA1 (Eph-1; Esk). - EPHA2 (Eck; Mpk-5; Sek-2). - EPHA3 (Etk-1; Hek; Mek4; Tyro4; Rek4; Cek4). - EPHA4 (Sek; Hek8; Mpk-3; Cek8). - EPHA5 (Ehk-1; Hek7; Bsk; Cek7). - EPHA6 (Ehk-2). - EPHA7 (Ehk-3; Hek11; Mdk-1; Ebk). - EPHA8 (Eek). - EPHB1 (Eph-2; Elk; Net). - EPHB2 (Eph-3; Hek5; Drt; Erk; Nuk; Sek-3; Cek5; Qek5). - EPHB3 (Hek-2; Mdk-5). - EPHB4 (Htk; Mdk-2; Myk-1). - EPHB5 (Cek9).The EPHA subtype receptors bind to GPI-anchored ephrins while the EPHB subtype receptors bind to type-I membrane ephrins. Two

signature patterns were developed for this class of RTK's, which each include some of the conserved cysteine residues. Consensus pattern: F-x-[DN]-x-[GAW]-[GA]-C-[LIVM]-[SA]-[LIVM](2)-[SA]-[LV]-[KRHQ]-[LIVA]-x(3)-[KR]-C-[PSAW] [The two C's are probably involved in disulfide bonds]
Consensus pattern: C-x(2)-[DE]-G-[DEQ]-W-x(2,3)-[PAQ]-[LIVMT]-[GT]-x-C-x-C- x(2)-G-[HFY]-[EQ] [The three C's are probably involved in disulfide bonds]

[1] Yarden Y., Ullrich A. Annu. Rev. Biochem. 57:443-478(1988).
[2] Sajjadi F.G., Pasquale E.B., Subramani S. New Biol. 3:769-778(1991).
[3] Wicks I.P., Wilkinson D., Salvaris E., Boyd A.W. Proc. Natl. Acad. Sci. U.S.A. 89:1611-1615(1992).

459. Protein kinase C terminal domain

460. Plant thionins signature
Thionins are small, basic, plant proteins generally toxic to animal cells [1]. They seem to exert their toxic effect at the level of the cell membrane but their exact function is not known. They consist of a polypeptide chain of forty five to fifty amino acids with three to four internal disulfide bonds. They are found in seeds but also in the cell wall of leaves [2]. Thionins are processed from larger precursor proteins [3]. Crambin [4], a hydrophobic plant seed protein, also belongs to this family. The pattern to detect this family of proteins includes three of the six cysteine residues involved in disulfide bonds. +-----------+ |+---------------+ |||| xxCCxxxxxxxxxxCxxxxxxxxxCxxxCxxCxxxxxCxxxxxxxx *****|| +--+'C': conserved cysteine involved in a disulfide bond.'*': position of the pattern.
Consensus pattern: C-C-x(5)-R-x(2)-[FY]-x(2)-C [The three C's are involved in disulfide bonds] The proteins from the gamma-thionin family are not related to the above proteins and are described in a separate section.

[1] Vernon L.P., Evett G.E., Zeikus R.D., Gray W.R. Arch. Biochem. Biophys. 238:18-29(1985).
[2] Bohlmann H., Clausen S., Behnke S., Giese H., Hiller C., Reimann-Phillip U., Schrader G., Barkholt V., Apel K. EMBO J. 7:1559-1565(1988).
[3] Bohlmann H., Apel K. Mol. Gen. Genet. 207:446-454(1987).
[4] Teeter M.M., Mazer J.A., L'Italien J.J. Biochemistry 20:5437-5443(1981).

461. Polyprenyl synthetases signatures
A variety of isoprenoid compounds are synthesized by various organisms. For example in eukaryotes the isoprenoid biosynthetic pathway is responsible for the synthesis of a variety of end products including cholesterol, dolichol, ubiquinone or coenzyme Q. In bacteria this pathway leads to the synthesis of isopentenyl tRNA, isoprenoid quinones, and sugar carrier lipids. Among the enzymes that participate in that pathway, are a number of polyprenyl synthetase enzymes which catalyze a 1'4-condensation between 5 carbon isoprene units. Currently the sequence of some of these enzymes is known: - Eukaryotic farnesyl pyrophosphate synthetase (FPP synthetase) (EC 2.5.1.1 / EC 2.5.1.10) which catalyzes the sequential condensation of isopentenyl pyrophosphate (IPP) with dimethylallyl pyrophosphate (DMAPP), and then with the resultant geranyl pyrophosphate to form farnesyl pyrophosphate. FPP synthetase is a cytoplasmic dimeric enzyme. - Prokaryotic farnesyl pyrophosphate synthetase (gene ispA). - Prokaryotic octaprenyl diphosphate synthase (gene ispB). - Prokaryotic heptaprenyl diphosphate synthase (EC 2.5.1.30). - Eukaryotic geranylgeranyl pyrophosphate synthetase (GGPP synthetase) (EC 2.5.1.1 / EC 2.5.1.10 / EC 2.5.1.29) which catalyzes the sequential addition of the three molecules of IPP onto DMAPP to form geranylgeranyl pyrophosphate. In plants GGPP synthase is a chloroplast enzyme involved in the biosynthesis of terpenoids; in fungi, such as Neurospora crassa (gene al-3), this enzyme is involved in the biosynthesis of carotenoids. - Prokaryotic GGPP synthetase, which are involved in the biosynthesis of carotenoids (gene crtE). Such an enzyme is also encoded in the cyanelle genome of Cyanophora paradoxa. - Eukaryotic hexaprenyl pyrophosphate synthetase, which is involved in the biosynthesis of coenzyme Q and which catalyzes the formation of all trans- polyprenyl pyrophosphates generally ranging in length of between 6 and 10 isoprene units depending on the species. HP synthetase is a mitochondrial membrane-associated enzyme. It has been shown [1 to 5] that all the above enzymes share some regions of sequence similarity. Two of these regions are rich in aspartic-acid residues and could be involved in the catalytic mechanism and/or the binding of the substrates. signature patterns were developed for both regions. Possible additional members of this family of proteins are: - Bacillus subtilis spore germination protein C3 (gene gerC3). Both proteins are most probably also enzymes involved in isoprenoid metabolism [6].
Consensus pattern: [LIVM](2)-x-D-D-x(2,4)-D-x(4)-R-R-[GH]-
Consensus pattern: [LIVMFY]-G-x(2)-[FYL]-Q-[LIVM]-x-D-D-[LIVMFY]-x-[DNG]

[1] Ashby M.N., Edwards P.A. J. Biol. Chem. 265:13157-13164(1990).
[2] Fujisaki S., Hara H., Nishimura Y., Horiuchi K., Nishino T. J. Biochem. 108:995-1000(1990).

[3] Carattoli A., Romano N., Ballario P., Morelli G., Macino G. J. Biol. Chem. 266:5854-5859(1991).
[4] Kuntz M., Roemer S., Suire C., Hugueney P., Weil J.H., Schantz R., Camara B. Plant J. 2:25-34(1992).
[5] Math S.K., Hearst J.E., Poulter C.D. Proc. Natl. Acad. Sci. U.S.A. 89:6761-6764(1992).
[6] Bairoch A. Unpublished observations (1993).

462. Potato inhibitor I family signature

The potato inhibitor I family is one of the numerous families of serine proteinase inhibitors. Members of this protein family are found in plants; in the seeds of barley or beans [1,2,3], and in potato or tomato leaves where they accumulate in response to mechanical damage [4,5]. An inhibitor belonging to this family is also found in leech [6]. It is interesting to note that, currently, this is the only proteinase inhibitor family to be found both inplant and animal kingdoms. Structurally these inhibitors are small (60 to 90 residues) and in contrast with other families of protease inhibitors, they lack disulfide bonds. They have a single inhibitory site. The consensus pattern includes three out of the four residues conserved in all members of this family and is located in the N-terminal half.

Consensus pattern: [FYW]-P-[EQH]-[LIV](2)-G-x(2)-[STAGV]-x(2)-A- Barley subtilisinchymotrypsin inhibitor-2b has Glu instead of Gly. There is a trypsin inhibitor from the cucurbitaceae Momordica charantia [7], which is said to belong to the potato inhibitor I family but which shows only a very weak similarity with the other members of this family.

[1] Svendsen I., Hejgaard J., Chavan J.K. Carlsberg Res. Commun. 49:493-502(1984).
[2] Svendsen I., Boisen S., Hejgaard J. Carlsberg Res. Commun. 47:45-53(1982).
[3] Nozawa H., Yamagata H., Aizono Y., Yoshikawa M., Iwasaki T. J. Biochem. 106:1003-1008(1989).
[4] Cleveland T.E., Thornburg R.W., Ryan C.A. Plant Mol. Biol. 8:199-207(1987).
[5] Lee J.S., Brown W.E., Graham J.S., Pearce G., Fox E.A., Dreher T.W., Ahern K.G., Pearson G.D., Ryan C.A. Proc. Natl. Acad. Sci. U.S.A. 83:7277-7281(1986).
[6] Seemuller U., Eulitz M., Fritz H., Strobl A. Hoppe-Seyler's Z. Physiol. Chem. 361:1841-1846(1980).
[7] Zeng F.-Y., Qian R.-Q., Wang Y. FEBS Lett. 234:35-38(1988).

463. (pp binding) Phosphopantetheine attachment site

Phosphopantetheine (or pantetheine 4' phosphate) is the prosthetic group of acyl carrier proteins (ACP) in some multienzyme complexes where it serves as a 'swinging arm' for the attachment of activated fatty acid and amino-acid groups [1]. Phosphopantetheine is attached to a serine residue in these proteins [2]. ACP proteins or domains have been found in various enzyme systems which are listed below (references are only provided for recently determined sequences). - Fatty acid synthetase (FAS), which catalyzes the formation of long-chain fatty acids from acetyl-CoA, malonyl-CoA and NADPH. Bacterial and plant chloroplast FAS are composed of eight separate subunits which correspond to the different enzymatic activities; ACP is one of these polypeptides. Fungal FAS consists of two multifunctional proteins, FAS1 and FAS2; the ACP domain is located in the N-terminal section of FAS2. Vertebrate FAS consists of a single multifunctional enzyme; the ACP domain is located between the beta-ketoacyl reductase domain and the C-terminal thioesterase domain [3]. - Polyketide antibiotics synthase enzyme systems. Polyketides are secondary metabolites produced from simple fatty acids, by microorganisms and plants. ACP is one of the polypeptidic components involved in the biosynthesis of Streptomyces polyketide antibiotics actinorhodin, curamycin, granatacin, monensin, oxytetracycline and tetracenomycin C. - Bacillus subtilis putative polyketide synthases pksK, pksL and pksM which respectively contain three, five and one ACP domains. - The multifunctional 6-methysalicylic acid synthase (MSAS) from Penicillium patulum. This is a multifunctional enzyme involved in the biosynthesis of a polyketide antibiotic and which contains an ACP domain in the C-terminal extremity. - Multifunctional mycocerosic acid synthase (gene mas) from Mycobacterium bovis. - Gramicidin S synthetase I (gene grsA) from Bacillus brevis. This enzyme catalyzes the first step in the biosynthesis of the cyclic antibiotic gramicidin S. - Tyrocidine synthetase I (gene tycA) from Bacillus brevis. The reaction carried out by tycA is identical to that catalyzed by grsA - Gramicidin S synthetase II (gene grsB) from Bacillus brevis. This enzyme is a multifunctional protein that activates and polymerizes proline, valine, ornithine and leucine. GrsB contains four ACP domains. - Erythronolide synthase proteins 1, 2 and 3 from Saccharopolyspora erythraea which is involved in the biosynthesis of the polyketide antibiotic erythromicin. Each of these proteins contain two ACP domains. - Conidial green pigment synthase from Aspergillus nidulans. - ACV synthetase from various fungi. This enzyme catalyzes the first step in the biosynthesis of penicillin and cephalosporin. It contains three ACP domains. - Enterobactin synthetase component F (gene entF) from Escherichia coli. This enzyme is involved in the ATP-dependent activation of serine during enterobactin (enterochelin) biosynthesis. - Cyclic peptide antibiotic surfactin synthase subunits 1, 2 and 3 from Bacillus subtilis. Subunits 1 and 2 contains three related domains while subunit 3 only contains a single domain. - HC-toxin synthetase (gene HTS1) from Cochliobolus carbonum. This enzyme synthesizes HC-toxin, a cyclic tetrapeptide. HTS1 contains four ACP domains. - Fungal mitochondrial ACP [9], which is part of the respiratory chain NADH dehydrogenase (complex I). - Rhizobium nodulation protein nodF, which probably acts as an ACP in the synthesis of the nodulation Nod factor

fatty acyl chain.The sequence around the phosphopantetheine attachment site is conserved in all these proteins and can be used as a signature pattern. A profile was also developed that spans the complete ACP-like domain. Consensus pattern: [DEQGSTALMKRH]-[LIVMFYSTAC]-[GNQ]-[LIVMFYAG]-[DNEKHS]-S- [LIVMST]-{PCFY}-[STAGCPQLIVMF]-[LIVMATN]-[DENQGTAKRHLM]- [LIVMWSTA]-[LIVGSTACR]-x(2)-[LIVMFA] [S is the pantetheine attachment site]

[1] Concise Encyclopedia Biochemistry, Second Edition, Walter de Gruyter, Berlin New-York (1988).
[2] Pugh E.L., Wakil S.J. J. Biol. Chem. 240:4727-4733(1965).
[3] Witkowski A., Rangan V.S., Randhawa Z.1., Amy C.M., Smith S. Eur. J. Biochem. 198:571-579(1991).
[6] Scotti C., Piatti M., Cuzzoni A., Perani P., Tognoni A., Grandi G., Galizzi A., Albertini A.M. Gene 130:65-71 (1993).
[9] Sackmann U., Zensen R., Rohlen D., Jahnke U., Weiss H. Eur. J. Biochem. 200:463-469(1991).

464. (Prenyltrans) Terpene synthases signature

The following enzymes catalyze mechanistically related reactions which involvethe highly complex cyclic rearrangement of squalene or its 2,3 oxide: - Lanosterol synthase (EC 5.4.99.7) (oxidosqualene--lanosterol cyclase), which catalyzes the cyclization of (S)-2,3-epoxysqualene to lanosterol, the initial precursor of cholesterol, steroid hormones and vitamin D in vertebrates and of ergosterol in fungi (gene ERG7). - Cycloartenol synthase (EC 5.4.99.8) (2,3-epoxysqualene--cycloartenol cyclase), a plant enzyme that catalyzes the cyclization of (S)-2,3- epoxysqualene to cycloartenol. - Hopene synthase (EC 5.4.99.-) (squalene--hopene cyclase), a bacterial enzyme that catalyzes the cyclization of squalene into hopene, a key step in hopanoid (triterpenoid) metabolism.These enzymes are evolutionary related [1] proteins of about 70 to 85 Kd. As a signature pattern, a highly conserved region was selected which is rich in aromatic residues and which is located in the C-terminal section.
Consensus pattern: [DE]-G-S-W-x-G-x-W-[GA]-[LIVM]-x-[FY]-x-Y-[GA]

[1] Corey E.J., Matsuda S.P.T., Bartel B. Proc. Natl. Acad. Sci. U.S.A. 90:11628-11632(1993).

465. Prion protein signatures

Prion protein (PrP) [1,2,3] is a small glycoprotein found in high quantity in the brains of humans or animals infected with a number of degenerative neurological diseases such as Kuru, Creutzfeldt-Jacob disease (CJD), scrapie or bovine spongiform encephalopathy (BSE). PrP is encoded in the host genome and expressed both in normal and infected cells. It has a tendency to aggregate yielding polymers called rods. Structurally, PrP is a protein consisting of a signal peptide, followed by an N-terminal domain that contains tandem repeats of a short motif (PHGGGWGQin mammals, PHNPGY in chicken), itself followed by a highly conserved domain lly comes a C-terminal hydrophobic domain post-translationally removed when PrP is attachedto the extracellular side of the cell membrane by a GPI-anchor. The structureof PrP is shown in the following schematic representation: +---+--------+-******-------------****-----+-----+ |Sig| Tandem repeats C C S| |+---+--------+--------------------|--------|----|+-----+ +--------+| GPI'C': conserved cysteine involved in a disulfide bond.'*': position of the patterns. As signature pattern for PrP, a perfectly conserved alanine- and glycine-rich region of 16 residues was selected as well as a region centered on the second cysteine involved in the disulfide bond.
Consensus pattern: A-G-A-A-A-A-G-A-V-V-G-G-L-G-G-Y-
Consensus pattern: E-x-[ED]-x-K-[LIVM](2)-x-[KR]-[LIVM](2)-x-[QE]-M-C-x(2)- Q-Y [C is involved in a disulfide bond]

[1] Stahl N., Prusiner S.B. FASEB J. 5:2799-2807(1991).
[2] Brunori M., Chiara Silvestrini M., Pocchiari M. Trends Biochem. Sci. 13:309-313(1988).
[3] Prusiner S.B. Annu. Rev. Microbiol. 43:345-374(1989).

466. Cyclophilin-type peptidyl-prolyl cis-trans isomerase signature and profile (pro isomerase)

Cyclophilin [1] is the major high-affinity binding protein in vertebrates for the immunosuppressive drug cyclosporin A (CSA). It exhibits a peptidyl- prolyl cis-trans isomerase activity (EC 5.2.1.8) (PPIase or rotamase). PPIase is an enzyme that accelerates protein folding by catalyzing the cis-transisomerization of proline imidic peptide bonds in oligopeptides [2]. It is probable that CSA mediates some of its effects via an inhibitory action on PPIase. Cyclophilin is a cytosolic protein which belongs to a family [3,4,5]that also includes the following isozymes: - Cyclophilin B (or S-cyclophilin), a PPIase which is retained in an endoplasmic reticulum compartment. - Cyclophilin C, a cytoplasmic PPiase. - Mitochondrial matrix cyclophilin (cyp3). - A PPIase which seems specific for the folding of rhodopsin and is an integral membrane protein anchored by a C-terminal transmembrane region. This protein was first characterized in Drosophila (gene ninaA). - Bacterial periplasmic PPiase (gene ppiA). - Bacterial cytosolic PPiase (gene ppiB). - Natural-killer cell cyclophilin-related protein. This large protein (about 160 Kd) is a component of a putative tumor-recognition complex involved in the function of NK cells. It contains a cyclophilin-type PPiase domain. - Mammalian nucleoporin Nup358 [6], a nuclear pore complex protein of 358 Kd that contains a C-terminal cyclophilin-type PPiase domain. - Yeast hypothetical protein YJR032w. - Fission yeast hypothetical protein SpAC21E11.05c. - Caenorhabditis elegans hypothetical protein T27D1.1.The sequences of the different forms of cyclophilin-type PPIases are well conserved. As a signature pattern, a conserved region was selected in the central part of these enzymes.
Consensus pattern: [FY]-x(2)-[STCNLV]-x-F-H-[RH]-[LIVMN]-[LIVM]-x(2)-F- [LIVM]-x-Q-[AG]-G- FKBP's, a family of proteins that bind the immunosuppressive drug FK506, are also PPIases, but their sequence is not at all related to that of cyclophilin.

[1] Stamnes M.A., Rutherford S.L., Zuker C.S. Trends Cell Biol. 2:272-276(1992).
[2] Fischer G., Schmid F.X. Biochemistry 29:2205-2212(1990).
[3] Trandinh C.C., Pao G.M., Saier M.H. Jr. FASEB J. 6:3410-3420(1992).
[4] Galat A. Eur. J. Biochem. 216:689-707(1993).
[5] Hacker J., Fischer G. Mol. Microbiol. 10:445456(1993).
[6] Wu J., Matunis M.J., Kraemer D., Blobel G., Coutavas E. J. Biol. Chem. 270:14209-14213(1995).

467. Profilin signature
Profilin [1,2] is a small eukaryotic protein that binds to monomeric actin(G-actin) in a 1:1 ratio thus preventing the polymerization of actin into filaments (F-actin). It can also, in certain circumstance promotes actin polymerization. Profilin also binds to polyphosphoinositides such as PIP2.Overall sequence similarity among profilin from organisms which belong to different phyla (ranging from fungi to mammals) is low, but the N-terminal region is relatively well conserved. That region is thought to be involved inthe binding to actin. The signature pattern for profilin is based on conserved residues at the N-terminal extremity .A protein structurally similar to profilin is present in the genome of variola and vaccinia viruses (gene A42R).
Consensus pattern: <x(0,1)-[STA]-x(0,1)-W-[DENQH]-x-[YI]-x-[DEQ]

[1] Haarer B.K., Brown S.S. Cell Motil. Cytoskeleton 17:71-74(1990).
[2] Sohn R.H., Goldschmidt-Clermont P. BioEssays 16:465-472(1994).

468. Protamine P1 signature
Protamines are small, highly basic proteins, that substitute for histones in sperm chromatin during the haploid phase of spermatogenesis. They pack sperm DNA into a highly condensed, stable and inactive complex. There are two different types of mammalian protamine, called P1 and P2. P1 has been found in all species studied, while P2 is sometimes absent. There seems to be a single type of avian protamine whose sequence is closely related to that of mammalian PI [1].As a signature for this family of proteins, a conserved region was selected at the N-terminal extremity of the sequence.
Consensus pattern: [AV]-R-[NFY]-R-x(2,3)-[ST]-x-S-x-S-

[1] Oliva R., Goren R., Dixon G.H. J. Biol. Chem. 264:17627-17630(1989).

469. Sperm histone P2 (protamine P2)
This protein also known as protamine P2 can substitute for histones in the chromatin of sperm. The alignment contains both the sequence of the mature P2 protein and its propeptide.

470. Proteasome A-type subunits signature
The proteasome (or macropain) (EC 3.4.99.46) [1 to 5,E1] is an eukaryotic and archaebacterial multicatalytic proteinase complex that seems to be involved inan ATP/ubiquitin-dependent nonlysosomal proteolytic pathway. In eukaryotes the proteasome is composed of about 28 distinct subunits which form a highly ordered ring-shaped structure (20S ring) of about 700 Kd. Most proteasome subunits can be classified, on the basis on sequence similarities into two groups, A and B. Subunits that belong to the A-type group are proteins of from 210 to 290 amino acids that share a number of conserved sequence regions. Subunits that are known to belong to this family are listed below. - Vertebrate subunits C2 (nu), C3, C8, C9, iota and zeta. - Drosophila PROS-25, PROS-28.1, PROS-29 and PROS-35. - Yeast C1 (PRS1), C5 (PRS3), C7-alpha (Y8) (PRS2), Y7, Y13, PRE5, PRE6 and PUP2. - Arabidopsis thaliana subunits alpha and PSM30. - Thermoplasma acidophilum alpha-subunit. In this archaebacteria the proteasome is composed of only two different subunits.As a signature pattern for proteasome A-type subunits the best conserved region was selected, which is located in the N-terminal part of these proteins.
Consensus pattern: [FY]-x(4)-[STNV]-x-[FYW]-S-P-x-G-[RKH]-x(2)-Q-[LIVM]-[DE]- Y-[SAD]-x(2)-[SAG]-. These proteins belong to family T1 in the classification of peptidases [6,E2].

[1] Rivett A.J. Biochem. J. 291:1-10(1993).
[2] Rivett A.J. Arch. Biochem. Biophys. 268:1-8(1989).
[3] Goldberg A.L., Rock K.L Nature 357:375-379(1992).
[4] Wilk S. Enzyme Protein 47:187-188(1993).
[5] Hilt W., Wolf D.H. Trends Biochem. Sci. 21:96-102(1996).
[6] Rawlings N.D., Barrett A.J. Meth. Enzymol. 244:19-61(1994).

Proteasome B-type subunits signature
The proteasome (or macropain) (EC 3.4.99.46) [1 to 5,E1] is an eukaryotic and archaebacterial multicatalytic proteinase complex that seems to be involved in an ATP/ubiquitin-dependent nonlysosomal proteolytic pathway. In eukaryotes the proteasome is composed of about 28 distinct subunits which form a highly ordered ring-shaped structure (20S ring) of about 700 Kd. Most proteasome subunits can be classified, on the basis on sequence similarities into two groups, A and B. Subunits that belong to the B-type group are proteins of from 190 to 290 amino acids that share a number of conserved sequence regions. Subunits that are known to belong to this family are listed below. - Vertebrate subunits C5, beta, delta, epsilon, theta (C10-II), LMP2/RING12, C13 (LMP7/RING10), C7-I and MECL-1. - Yeast PRE1, PRE2 (PRG1), PRE3, PRE4, PRS3, PUP1 and PUP3. - Drosophila L(3)73AI. - Fission yeast pts1. - Thermoplasma acidophilum beta-subunit. In this archaebacteria the proteasome is composed of only two different subunits. As a signature pattern for proteasome B-type subunits the best conserved region was selected, which is located in the N-terminal part of these proteins.
Consensus pattern: [LIVMA]-[GSA]-[LIVMF]-x-[FYLVGAC]-x(2)-[GSACFY]-[LIVMSTAC](3)-[GAC]-[GSTACV]-[DES]-x(15)-[RK]-x(12,13)-G-x(2)-[GSTA]-D-.
These proteins belong to family T1 in the classification of peptidases [6,E2].

[1] Rivett A.J. Biochem. J. 291:1-10(1993).
[2] Rivett A.J. Arch. Biochem. Biophys. 268:1-8(1989).
[3] Goldberg A.L., Rock K.L Nature 357:375-379(1992).
[4] Wilk S. Enzyme Protein 47:187-188(1993).
[5] Hilt W., Wolf D.H. Trends Biochem. Sci. 21:96-102(1996).
[6] Rawlings N.D., Barrett A.J. Meth. Enzymol. 244:19-61(1994).

471. (pyr redox) Pyridine nucleotide-disulphide oxidoreductases class-I active site
The pyridine nucleotide-disulphide oxidoreductases are FAD flavoproteins which contains a pair of redox-active cysteines involved in the transfer of reducing equivalents from the FAD cofactor to the substrate. On the basis of sequence and structural similarities [1] these enzymes can be classified into two categories. The first category groups together the following enzymes [2 to 6]: - Glutathione reductase (EC 1.6.4.2) (GR). - Higher eukaryotes thioredoxin reductase (EC 1.6.4.5). - Trypanothione reductase (EC 1.6.4.8). - Lipoamide dehydrogenase (EC 1.8.1.4), the E3 component of alpha-ketoacid dehydrogenase complexes. - Mercuric reductase (EC 1.16.1.1).The sequence around the two cysteines involved in the redox-active disulfide bond is conserved and can be used as a signature pattern.
Consensus pattern: G-G-x-C-[LIVA]-x(2)-G-C-[LIVM]-P [The two C's form the active site disulfide bond]. In positions 6 and 7 of the pattern all known sequences have Asn-(Val/ Ile) with the exception of GR from plant chloroplasts and from cyanobacteria which have Ile-Arg [7].

[1] Kurlyan J., Krishna T.S.R., Wong L., Guenther B., Pahler A., Williams C.H. Jr., Model P. Nature 352:172-174 (1991).
[2] Rice D.W., Schulz G.E., Guest J.R. J. Mol. Biol. 174:483-496(1984).
[3] Brown N.L. Trends Biochem. Sci. 10:400-402(1985).
[4] Carothers D.J., Pons G., Patel M.S. Arch. Biochem. Biophys. 268:409-425(1989).
[5] Walsh C.T., Bradley M., Nadeau K. Trends Biochem. Sci. 16:305-309(1991).
[6] Gasdaska P.Y., Gasdaska J.R., Cochran S., Powis G. FEBS Lett. 373:5-9(1995).
[7] Creissen G., Edwards E.A., Enard C., Wellburn A., Mullineaux P. Plant J. 2:129-131(1991).

472. (pyridoxal deC) DDC / GAD / HDC / TyrDC pyridoxal-phosphate attachment site (pyridoxal deC)
Three different enzymes - all pyridoxal-dependent decarboxylases - seem to share regions of sequence similarity [1,2,3,4], especially in the vicinity of the lysine residue which serves as the attachment site for the pyridoxal-phosphate (PLP) group. These enzymes are: - Glutamate decarboxylase (EC 4.1.1.15) (GAD). Catalyzes the decarboxylation of glutamate into the neurotransmitter GABA (4-aminobutanoate). - Histidine decarboxylase (EC 4.1.1.22 (HDC). Catalyzes the decarboxylation of histidine to histamine. There are two completely unrelated types of HDC: those

that use PLP as a cofactor (found in Gram-negative bacteria and mammals), and those that contain a covalently bound pyruvoyl residue (found in Gram-positive bacteria). - Aromatic-L-amino-acid decarboxylase (EC 4.1.1.28) (DDC), also known as L-dopa decarboxylase or tryptophan decarboxylase. DDC catalyzes the decarboxylation of tryptophan to tryptamine. It also acts on 5-hydroxytryptophan and dihydroxyphenylalanine (L-dopa). - Tyrosine decarboxylase (EC 4.1.1.25) (TyrDC) which converts tyrosine into tyramine, a precursor of isoquinoline alkaloids and various amides.These enzymes are collectively known as group II decarboxylases [3,4].
Consensus pattern: S-[LIVMFYW]-x(5)-K-[LIVMFYWG](2)-x(3)-[LIVMFYW]-x-[CA]-x(2)-[LIVMFYWQ]-x(2)-[RK] [K is the pyridoxal-P attachment site]

[1] JacksonF.R. J. Mol. Evol. 31:325-329(1990).
[2] Joseph D.R., Sullivan P., Wang Y.-M., Kozak C., Fenstermacher D.A., Behrendsen M.E., Zahnow C.A. Proc. Natl. Acad. Sci. U.S.A. 87:733-737(1990).
[3] Sandmeier E., Hale T.I., Christen P. Eur. J. Biochem. 221:997-1002(1994).
[4] Ishii S., Mizugichi H., Nishino J., Hayashi H., Kagamiyama H. J. Biochem. 120:369-376(1996).

473. Regulator of chromosome condensation (RCC1) signatures (RCC1)
The regulator of chromosome condensation (RCC1) [1] is a eukaryotic protein which binds to chromatin and interacts with ran, a nuclear GTP-binding protein, to promote the loss of bound GDP and the uptake offresh GTP, thus acting as a guanine-nucleotide dissociation stimulator (GDS)[2]. The interaction of RCC1 with ran probably plays an important role in the regulation of gene expression. RCC1, known as PRP20 or SRM1 in yeast, pim1 in fission yeast and BJ1 in Drosophila, is a protein that contains seven tandem repeats of a domain of about 50 to 60 amino acids. As shown in the following schematic representation, the repeats make up the maj or part of the length of the protein. Outside the repeat region, there is just a small N-terminal domain of about 40 to 50 residues and, in the Drosophila protein only, a C-terminal domain of about 130 residues. +----+-------+-------+-------+-+-------+-------+-------+-------------+ |N-t.|Rpt. 1 |Rpt. 2 |Rpt. 3 |Rpt. 4 |Rpt. 5 |Rpt. 6 |Rpt. 7 | C-terminal |+----+-------+-------+-------+-------+-------+-------+-------+----------+ In Drosophila two signature patterns for RCC1 were developed. The first is found in the N-terminal part of the second repeat; this is the most conserved part of RCC1. The second is derived from conserved positions in the C-terminal part of each repeat and detects up to five copies of the repeated domain. The RCC1-type of repeat is also found in the X-linked retinitis pigmentosa GTPase regulator [3].
Consensus pattern: G-x-N-D-x(2)-[AV]-L-G-R-x-T-
Consensus pattern: [LIVMFA]-[STAGC](2)-G-x(2)-H-[STAGLI]-[LIVMFA]-x-[LIVM]-

[1] Dasso M. Trends Biochem. Sci. 18:96-101(1993).
[2] Boguski M.S., McCormick F. Nature 366:643-654(1993).
[3] Roepman R., Van Duijnhoven G., Rosenberg T., Pinckers A.J.L.G., Bleeker-Wagemakers L.M., Bergen A.A.B., Post J., Beck A., Reinhardt R., Ropers H.-H., Cremers F., Berger W. Hum. Mol. Genet. 5:1035-1041 (1996).

474. RNA 3'-terminal phosphate cyclase signature (RCT)
RNA 3'-terminal phosphate cyclase (EC 6.5.1.4) [1,2] catalyzes the conversion of 3'-phosphate to a 2',3'-cyclic phosphodiester at the end of RNA. The biological role of this enzyme is unknown but it is likely to function in some aspects of cellular RNA processing. The reaction catalyzed by the enzyme occurs in three steps: 1) adenylation of the enzyme by ATP; 2) the enzyme acts on RNA-3'terminal phosphate to produce RNA-3'terminal diphosphate adenylate; 3) Release of AMP and cyclisation by a non catalytic nucleophilic attack by the adjacent 2'hydroxyl on the phosphorus in the diester linkage. This enzyme, which has been characterized in human (where there seems to be at least three isozymes) and Escherichia coli (gene rtCA), seems to be taxonomically widespread. It is found in insects, plants, fungi (gene RTC1 inyeast) and in archeabacteria. RNA cyclase is a protein of from 36 to 42 Kd. The best conserved region, which is used as a signature pattern, is a glycine-rich stretch of residues located in the central part of the sequence and which is reminiscent of various ATP, GTPor AMP glycine-rich loops. In this context, the conserved Arg (His in the E.coli enzyme) could be the AMP-binding residue.
Consensus pattern: [RH]-G-x(2)-P-x-G(3)-x-[LIV]-

[1] Genschik P., Billy E., Swianiewicz M., Filipowicz W. EMBO J. 16:2955-2967(1997).
[2] Filipowicz W., Vincente O. Meth. Enzymol. 181:499-510(1990).

475. REV protein (anti-repression trans-activator protein)

476. Prokaryotic-type class I peptide chain release factors signature (RF-1) Peptide chain release factors (RFs) are

required for the termination of protein biosynthesis [1]. At present two classes of RFs can be distinguished. Class I RFs bind to ribosomes that have encountered a stop codon at their decoding site and induce release of the nascent polypeptide. Class II RFs are GTP-binding proteins that interact with class I RFs and enhance class I RF activity. In prokaryotes there are two class I RFs that act in a codon specific manner[2]: RF-1 (gene prfA) mediates UAA and UAG-dependent termination while RF-2(gene prfB) mediates UAA and UGA-dependent termination. RF-1 and RF-2 are structurally and evolutionary related proteins which have been shown [3] to make up a family that also contains the following proteins: - Fungal MRF1, a mitochondrial RF (m-RF) which recognizes the UAA and UAG codons. - Escherichia coli RF-H, a protein of unknown function. - Escherichia coli hypothetical protein yaeJ and a close Pseudomonas putida homolog. A highly conserved region located in the central part of the 40 to 45 Kd RF-1/2 and m-RF and in the N-terminal of the 15 to 16Kd RF-H and yaeJ is used as a signature pattern.
Consensus pattern: [AR]-[STA]-x-G-x-G-G-Q-[HNGCS]-V-N-x(3)-[ST]-A-[IV]
Note that prokaryotic-type class I RFs display no significant sequence similarity to prokaryotic-type class II which belong to the family of GTP-binding elongation factors nor to eukaryotic class I or class II RFs.

[1] Tate W.P. , Poole E.S., Mannering S.M. Prog. Nucleic Acids. Res. Mol. Biol. 52:293-335(1996).
[2] Craigen W.J., Lee C.C., Caskey C.T. Mol. Microbiol. 4:861-865(1990).
[3] Pel H.J., Rep M., Grivell L.A. Nucleic Acids Res. 20:4423-4428(1992).

477. RIO1/ZK632.3/MJ0444 family signature
The following uncharacterized proteins are evolutionary related [1]: - Yeast protein RIO1. - Caenorhabditis elegans hypothetical protein ZK632.3. - Methanococcus jannaschii hypothetical protein MJ0444. - Thermoplasma acidophilum hypothetical protein if rpoA2 3'region. The eukaryotic members of this family are proteins of about 55 to 60 Kd, while the archebacterial ones are half that size. The central part of these proteins is highly conserved. The best conserved region is used as a signature pattern.
Consensus pattern: [LIVM]-V-H-[GA]-D-L-S-E-[FY]-N-x-[LIVM]

[1] Bairoch A. Unpublished observations (1997).

478. (RIP) Shiga/ricin ribosomal inactivating toxins active site signature. A number of bacterial and plant toxins act by inhibiting protein synthesis in eukaryotic cells. The toxins of the Shiga and ricin family inactivate 60S ribosomal subunits by an N-glycosidic cleavage which releases a specific adenine base from the sugar-phosphate backbone of 28S rRNA [1,2,3]. The toxins which are known to function in this manner are: - Shiga toxin from Shigella dysenteriae [4]. This toxin is composed of one copy of an enzymatically active A subunit and five copies of a B subunit responsible for binding the toxin complex to specific receptors on the target cell surface. - Shiga-like toxins (SLT) are a group of Escherichia coli toxins very similar in their structure and properties to Shiga toxin. The sequence of two types of these toxins, SLT-1 [5] and SLT-2 [6], is known. - Ricin, a potent toxin from castor bean seeds. Ricin consists of two glycosylated chains linked by a disulfide bond. The A chain is enzymatically active. The B chain is a lectin with a binding preference for galactosides. Both chains are encoded by a single polypeptidic precursor. Ricin is classified as a type-II ribosome-inactivating protein (RIP); other members of this family are agglutinin, also from castor bean, and abrin from the seeds of the bean Abrus precatorius [7]. - Single chain ribosome-inactivating proteins (type-I RIP) from plants. Examples of such proteins are: barley protein synthesis inhibitors I and II, mongolian snake-gourd trichosanthin, sponge gourd luffin-A and -B, garden four-o'clock MAP, common pokeberry PAP-S and soapwort saporin-6 [7].All these toxins are structurally related. A conserved glutamic residue has been implicated [8] in the catalytic mechanism; it is located near a conserved arginine which also plays a role in catalysis [9]. The signature that has been developed for these proteins includes these catalytic residues.
Consensus pattern: [LIVMA]-x-[LIVMSTA](2)-x-E-[SAGV]-[STAL]-R-[FY]-[RKNQS]-x- [LIVM]-[EQS]-x(2)-[LIVMF] [E and R are active site residues]-

[1] Endo Y., Tsurugi K., Takeda Y., Ogasawara T., Igarashi K. Eur. J. Biochem. 171:45-50(1988).
[2] May M.J., Hartley M.R., Roberts L.M., Krieg P.A., Osborn R.W., Lord J.M. EMBO J. 8:301-308(1989).
[3] Funatsu G., Islam M.R., Minami Y., Sung-Sil K., Kimura M. Biochimie 73:1157-1161(1991).
[4] Strockbine N.A., Jackson M.P., Sung L.M., Holmes R.K., O'Brien A.D. J. Bacteriol. 170:1116-1122(1988).
[5] Calderwood S.B., Auclair F., Donohue-Rolfe A., Keusch G.T., Mekalanos J.J. Proc. Natl. Acad. Sci. U.S.A. 84:4364-4368(1987).
[6] Jackson M.P., Neill R.J., O'Brien A.D., Holmes R.K., Newland J.W. FEMS Microbiol. Lett. 44:109-114(1987).
[7] Barbieri L., Battelli M.G., Stirpe F. Biochim. Biophys. Acta 1154:237-282(1993).
[8] Hovde C.J., Calderwood S.B., Mekalanos J.J., Collier R.J. Proc. Natl. Acad. Sci. U.S.A. 85:2568-2572(1988).
[9] Monzingo A.F., Collins E.J., Ernst S.R., Irvin J.D., Robertus J.D. J. Mol. Biol. 233:705-715(1993).

479. Bacterial RNA polymerase, alpha chain (RNA pol A bac)
Members of this family include alpha subunit from eubacteria and alpha subunits from chloroplasts. The alpha subunit of RNA polymerase consists of two independently folded domains, referred to as amino-terminal and carboxyl terminal domains. The amino terminal domain is involved in the interaction with the other subunits of the RNA polymerase. The carboxyl-terminal domain interacts with the DNA and activators. The amino acid sequence of the alpha subunit is conserved in prokaryotic and chloroplast RNA polymerases. There are three regions of particularly strong conservation, two in the amino-terminal and one in the carboxyl-Comment: terminal [3].

[1] Zhang G, Darst SA; Science 1998;281:262-266.
[2] Jeon YH, Negishi T, Shirakawa M, Yamazaki T, Fujita N, Ishihama A, Kyogoku Y; Science 1995;270: 1495-1497.
[3] Ebright RH, Busby S; Curr Opin Genet Dev 1995;5:197-203.
[4] Murakami K, Kimura M, Owens JT, Meares CF, Ishihama A; Proc Natl Acad Sci USA 1997;94:1709-1714.

480. RNA polymerase beta subunit (RNA pol B)
RNA polymerases catalyse the DNA dependent polymerisation of RNA. Prokaryotes contain a single RNA polymerase compared to three in eukaryotes (not including mitochondrial and chloroplast polymerases). Each RNA polymerase complex contains two related members of this family, in each case they are the two largest subunits.

[1] Falkenburg D, Dworniczak B, Faust DM, Bautz EK; J Mol Biol 1987;195:929-937.

481. RNA polymerases H / 23 Kd subunits signature
In eukaryotes, there are three different forms of DNA-dependent RNA polymerases (EC 2.7.7.6 transcribing different sets of genes. Each class of RNA polymerase is an assemblage of ten to twelve different polypeptides. In archaebacteria, there is generally a single form of RNA polymerase which also consist of an oligomeric assemblage of 10 to 13 polypeptides. Archaebacterial subunit H (gene rpoH) [1,2] is a small protein of about 8.5 to 10 Kd, it is evolutionary related to the C-terminal part of a 23 Kd component shared by all three forms of eukaryotic RNA polymerases (gene RPB5 in yeast and POLR2E in mammals).As a signature pattern a conserved region was selected which is located at theN-terminal extremity of subunit H; this region contains two histidines that could play a role in the binding of a metal ion.
Consensus pattern: H-[NEI]-[LIVM]-V-P-x-H-x(2)-[LIVM]-x(2)-[DE]

[1] Klenk H.-P., Palm P., Lottspeich F., Zillig W. Proc. Natl. Acad. Sci. U.S.A. 89:407-410(1992).
[2] Thiru A., Hodach M., Eloranta J.J., Kostourou V., Weinzierl R.O., Matthews S.; J. Mol. Biol. 287:753-760 (1999).

482. RNA polymerases K / 14 to 18 Kd subunits signature
In eukaryotes, there are three different forms of DNA-dependent RNApolymerases (EC 2.7.7.6) transcribing different sets of genes. Each class of RNA polymerase is an assemblage of ten to twelve different polypeptides. In archaebacteria, there is generally a single form of RNA polymerase which also consist of an oligomeric assemblage of 10 to 13 polypeptides. A component of 14 to 18 Kd shared by all three forms of eukaryotic RNA polymerases and which has been sequenced in budding yeast (gene RPB6 orRPO26), in fission yeast (gene rpb6 or rpo15), in human and in African swine fever virus [1] is evolutionary related [2] to archaebacterial subunit K (gene rpoK). The archaebacterial protein is colinear with the C-terminal part of the eukaryotic subunit.
Consensus pattern: [ST]-x-[FY]-E-x-[AT]-R-x-[LIVM]-[GSA]-x-R-[SA]-x-Q

[1] Lu Z., Kutish G.F., Sussman M.D., Rock D.L. Nucleic Acids Res. 21:2940-2940(1993).
[2] McKune K., Woychik N.A. J. Bacteriol. 176:4754-4756(1994).

483. RNA polymerases L / 13 to 16 Kd subunits signature
In eukaryotes, there are three different forms of DNA-dependent RNApolymerases (EC 2.7.7.6 transcribing different sets of genes. Each class of RNA polymerase is an assemblage of ten to twelve different polypeptides. In archaebacteria, there is generally a single form of RNA polymerase which also consist of an oligomeric assemblage of 10 to 13 polypeptides. It has been shown that small subunits of about 13 to 16 Kd found in all three types of eukaryotic polymerases are highly conserved. Subunits known to belong to this family are: - Budding yeast RPC19 subunit from RNA polymerases I and III [1]. - Budding yeast RPB11 subunit from RNA polymerase II [2]. - Mammalian RPB11 (gene POLR2K) from RNA polymerase II. - Caenorhabditis elegans hypothetical protein F58A4.9. - Methanococcus jannaschii RNA polymerase subunit L (gene rpoL). - Sulfolobus acidocaldarius RNA polymerase subunit

L (gene rpoL) [3].As a signature pattern a conserved region was selected which is located at the N-terminal extremity of these polymerase subunits; this region contains two cysteines that could play a role in the binding of a metal ion. Consensus pattern: [DE](2)-H-[ST]-[LIVM]-[GAP]-N-x(11)-V-x-[FM]-x(2)-Y-x(3)- H-P

[1] Dequard-Chablat M., Riva M., Carles C., Sentenac A. J. Biol. Chem. 266:15300-15307(1991).
[2] Woychik N.A., McKune K., Lane W.S., Young R.A. Gene Expr. 3:77-82(1993).
[3] Langer D. EMBL/GenBank: X70805.

484. RNA polymerases N / 8 Kd subunits signature
In eukaryotes, there are three different forms of DNA-dependent RNA polymerases (EC 2.7.7.6) transcribing different sets of genes. Each class of RNA polymerase is an assemblage of ten to twelve different polypeptides. In archaebacteria, there is generally a single form of RNA polymerase which also consist of an oligomeric assemblage of 10 to 13 polypeptides. Archaebacterial subunit N (gene rpoN) [1] is a small protein of about 8 Kd, it is evolutionary related [2] to a 8.3 Kd component shared by all three forms of eukaryotic RNA polymerases (gene RPB 10 in yeast and POLR2J in mammals) as well as to African swine fever virus protein CP80R [3].As a signature pattern a conserved region was selected which is located at the N-terminal extremity of these polymerase subunits; this region contains two cysteines that could play a role in the binding of a metal ion.
Consensus pattern: [LIVMF](2)-P-[LIVM]-x-C-F-[ST]-C-G-

[1] Langer D., Hain J., Thuriaux P., Zillig W. Proc. Natl. Acad. Sci. U.S.A. 92:5768-5772(1995).
[2] McKune K., Woychik N.A. J. Bacteriol. 176:4754-4756(1994).
[3] Yanez R.J., Rodriguez J.M., Nogal M.L., Yuste L., Enriquez C., Rodriguez J.F., Vinuela E. Virology 208: 249-278(1995).

485. Ribonuclease HII

[1] Mian IS; Nucleic Acids Res 1997;25:3187-3189.

486. Ribonuclease PH signature
Prokaryotic ribonuclease PH (EC 2.7.7.56) (RNase PH) [1] is a phosphorolyticexoribonuclease that removes nucleotide residues following the -CCA terminus of tRNA and adds nucleotides to the ends of RNA molecules by using nucleoside diphosphates as substrates. RNase PH is a conserved protein of about 240 amino-acid residues. It is evolutionary related to Caenorhabditis elegans hypothetical protein B0564.1.As a signature pattern, the most highly conserved region was selected which is located in the central part of these proteins.
Consensus sequence: C-[DE]-[LIVM](2)-Q-[GTA]-D-G-[SG]-x(2)-[TA]-A

[1] Kelly K.O., Deutscher M.P. J. Biol. Chem. 267:17153-17158(1992).

487. RanBP1 domain

[1] Di Matteo G, Fuschi P, Zerfass K, Moretti S, Ricordy R, Cenciarelli C, Tripodi M, Jansen-Durr P, Lavia P; Cell Growth Differ 1995;6:1213-1224.

488. Rhodanese signatures
Rhodanese (thiosulfate sulfurtransferase) (EC 2.8.1.1) [1,2] is an enzyme which catalyzes the transfer of the sulfane atom of thiosulfate to cyanide, to form sulfite and thiocyanate. In vertebrates, rhodanese is a mitochondrial enzyme of about 300 amino-acid residues involved in forming iron-sulfur complexes and cyanide detoxification. A cysteine residue takes part in the catalytic mechanism. Some bacterial proteins closely related to rhodanese are also thought to express a sulfotransferase activity. These are: - Azotobacter vinelandii rhdA. - Escherichia coli sseA [3]. - Saccharopolyspora erythraea cysA [4]. - Synechococcus strain PCC 7942 rhdA [5]. RhdA is a periplasmic protein probably involved in the transport of sulfur compounds. Two patterns for the rhodanese family were developed. They are based on highly conserved regions, one which is located in the N-terminal region, the other at the C-terminal extremity of the enzyme.
Consensus pattern: [FY]-x(3)-H-[LIV]-P-G-A-x(2)-[LIVF]
Consensus pattern: [FY]-[DEAP]-G-[SA]-W-x-E-[FYW]

[1] Westley J. Meth. Enzymol. 77:285-291 (1981).
[2] Weiland K.L., Dooley T.P. Biochem. J. 275:227-231(1991).

[3] Rudd K.E. Unpublished observations (1993).
[4] Donadio S., Shafiee A., Hutchinson C.R. J. Bacteriol. 172:350-360(1990).
[5] Laudenbach D.E., Ehrhardt D., Green L., Grossman A.R. J. Bacteriol. 173:2751-2760(1991).

489. Ribonuclease III family signature
Prokaryotic ribonuclease III (EC 3.1.26.3) (gene rnc) [1] is an enzyme that digests double-stranded RNA. It is involved in the processing of ribosomal RNA precursors and of some mRNAs. RNase III is evolutionary related [2] to the following proteins: - Fission yeast pac1, a ribonuclease that probably inhibits mating and meiosis by degrading a specific mRNA required for sexual development. - Yeast ribonuclease III (gene RNT1), a dsRNA-specific nuclease that cleaves eukaryotic preribosomal RNA at various sites. - Caenorhabditis elegans hypothetical protein F26E4.13. - Paramecium bursaria chlorella virus 1 protein A464R. - Synechocystis strain PCC 6803 hypothetical protein slr0346.-Fission yeast hypothetical protein SpAC8A4.08c, a protein with a N-terminal helicase domain and a C-terminal RNase III domain. - Caenorhabditis elegans hypothetical protein K12H4.8, a protein with the same structure as SpAC8A4.08c.These proteins share regions of sequence similarity; one of which is a highly conserved stretch of 9 residues which has been developed as a signature pattern.
Consensus pattern: [DEQ]-[RQ]-[LM]-E-[FYW]-[LV]-G-D-[SAR]-

[1] Nashimoto H., Uchida H. Mol. Gen. Genet. 201:25-29(1985).
[2] Mian I.S. Nucleic Acids Res. 25:3187-3195(1997).

490. Rieske iron-sulfur protein signatures
Ubiquinol-cytochrome c reductase (EC 1.10.2.2) (also known as the bc1 complexor complex III) is one of the electron transport chains of mitochondria and of some aerobic prokaryotes; it catalyzes the oxidoreduction of ubiquinol and cytochrome c. In the chloroplast of plants and in cyanobacteria plastoquinone-plastocyanin reductase (EC 1.10.99.1) (also known as the b6f complex) is functionally similar and catalyzes the oxidoreduction of plastoquinol and cytochrome f. One of the components of these electron transfer systems is an iron-sulfur protein with a 2Fe-2S cluster, which is called the Rieske protein [1,2]. The Rieske protein contains approximately 190 amino acid residues. The iron-sulfur cluster is complexed to the protein through cysteine and histidine residues. Two perfectly conserved regions in Rieske proteins contains all the residuesthat bind the iron-sulfur cluster. Both regions contain two cysteines and a histidine. The first cysteine and the histidine are 2Fe-2S ligands while the remaining cysteines form a disulfide bond [3]. Two conserved regions were selected as signature patterns.
Consensus pattern: C-[TK]-H-L-G-C-[LIVST] [The first C and the H are 2Fe-2S ligands] [The second C is involved in a disulfide bond]
Consensus pattern: C-P-C-H-x-[GSA] [The first C and the H are 2Fe-2S ligands] [The second C is involved in a disulfide bond]

[1] Gatti F.L., Meinhardt S.W., Ohnishi T., Tzagoloff A. J. Mol. Biol. 205:421-435(1989).
[2] Kallas T., Spiller S., Malkin R. Proc. Natl. Acad. Sci. U.S.A. 85:5794-5798(1988).
[3] Iwata S., Saynovits M., Link T.A., Michel H. Structure 4:567-579(1996).

491. Ribosomal protein L1 signature
Ribosomal protein L1 is the largest protein from the large ribosomal subunit.In Escherichia coli, L1 is known to bind to the 23S rRNA. It belongs to a family of ribosomal proteins which, on the basis of sequence similarities [1, 2], groups: - Eubacterial L1. - Algal and plant chloroplast L1. - Cyanelle L1.- Archaebacterial L1. - Vertebrate L10A. - Yeast SSM1.As a signature pattern, the best conserved region was selected located in the central section of these proteins. It is located at the end of an alpha helix thought to be involved in RNA-binding.
Consensus pattern: [IM]-x(2)-[LIVA]-x(2,3)-[LIVM]-G-x(2)-[LMS]-[GSNH]-[PTKR]-[KRAV]-G-x-[LIMF]-P-[DENSTKQ]

[1] Nikonov S.V., Nevskaya N., Eliseikina I.A., Fomenkova N.P., Nikulin A., Ossina N., Garber M., Jonsson B.-H., Briand C., Al-Karadaghi S., Svensson L.A., Aevarsson A., Liljas A. EMBO J. 15:1350-1359(1996).
[2] Olvera J., Wool I.G. 2.3.CO;2-"Biochem. Biophys. Res. Commun. 220:954-957(1996).

492. Ribosomal protein L10 signature
Ribosomal protein L10 0 is one of the proteins from the large ribosomal subunit. L10 0 is a protein of 162 to 185 amino-acid residues which has only been found so far in eubacteria. A conserved region located in the N-terminal section of these proteins was used as a signature pattern.
Consensus pattern: [DEH]-x(2)-[GS]-[LIVMF]-[STN]-[VA]-x-[DEQK]-[LIVMA]-x(2)-[LIM]-R

493. Ribosomal protein L10e signature

A number of eukaryotic and archaebacterial ribosomal proteins can be grouped on the basis of sequence similarities. One of these families consists of: - Vertebrate L10 (QM) [1]. - Plant L10. - Caenorhabditis elegans L10 (F10B5.1). - Yeast L10 (QSR1). - Methanococcus jannaschii MJ0543.These proteins have 174 to 232 amino-acid residues. A conserved region located in the central section was selected as a signature pattern.

Consensus pattern: R-x-A-[FYW]-G-K-[PA]-x-G-x(2)-A-R-V

[1] Chan Y.-L., Diaz J.-J., Denoroy L., Madjar J.-J., Wool I.G. 2.3.CO;2-"Biochem. Biophys. Res. Commun. 255: 952-956(1996).

494. Ribosomal protein L11 signature

Ribosomal protein L11 is one of the proteins from the large ribosomal subunit. In Escherichia coli, L11 is known to bind directly to the 23 S rRNA. It belongs to a family of ribosomal proteins which, on the basis of sequence similarities [1,2], groups:

- Eubacterial L11.
- Plant chloroplast L1 1 (nuclear-encoded).
- Read algal chloroplast L11.
- Cyanelle L11.
- Archaebacterial L11.
- Mammalian L12.
- Plants L12.
- Yeast L12 (YL15).

L11 is a protein of 140 to 165 amino-acid residues. A conserved region located in the C-terminal section of these proteins was selected as a signature pattern. In Escherichia coli, the C-terminal half of L1 has been shown [3] to be in an extended and loosely folded conformation and is likely to be buried within the ribosomal structure.

Consensus pattern: [RKN]-x-[LIVM]-x-G-[ST]-x(2)-[SNQ]-[LIVM]-G-x(2)-[LIVM]-x(0,1)-[DENG]

[1] Pucciarelli G., Remacha M., Ballesta J.P.G.; Nucleic Acids Res. 18:4409-4416(1990).
[2] Otaka E., Hashimoto T., Mizuta K., Suzuki K.; Protein Seq. Data Anal. 5:301-313(1993).
[3] Choli T. Biochem. Int. 19:1323-1338(1989).

495. Ribosomal protein L7/L12 C-terminal domain

[1] Leijonmarck M, Liljas A; J Mol Biol 1987;195:555-579.

496. Ribosomal protein L13 signature

Ribosomal protein L13 is one of the proteins from the large ribosomal subunit. In Escherichia coli, L13 is known to be one of the early assembly proteins of the 50S ribosomal subunit. It belongs to a family of ribosomal proteins which, on the basis of sequence similarities [1], groups: - Eubacterial L13.

- Plant chloroplast L13 (nuclear-encoded). - Red algal chloroplast L13.
- Archaebacterial L13. - Mammalian L13a (Tum P198). - Yeast Rp22 and Rp23.

L1 1 is a protein of 140 to 250 amino-acid residues. As a signature pattern, a conserved region was selected located in the C-terminal section of these proteins.

Consensus pattern: [LIVM]-[KRV]-[GK]-M-[LIV]-[PS]-x(4,5)-[GS]-[NQEKRA]-x(5)-[LIVM]-x-[AIV]-[LFY]-x-[GDN]

[1] Chan Y.-L., Olvera J., Glueck A., Wool I.G. J. Biol. Chem. 269:5589-5594(1994).

497. Ribosomal protein L13e signature

A number of eukaryotic ribosomal proteins can be grouped on the basis of sequence similarities [1]. One of these families consists of:

- Vertebrate L13 (was previously known as Breast Basic Conserved protein 1 (BBC1)). - Drosophila L13. - Plant L13. - Yeast probable L13 (YM9375.11c). These proteins have 199 to 218 amino-acid residues. As a signature pattern, a stretch of about 16 residues in the first third of these proteins selected.

- Consensus pattern: [KR]-Y-x(2)-K-[LIVM]-R-[STA]-G-[KR]-G-F-[ST]-L-x-E

[1] Olvera J., Wool I.G. Biochem. Biophys. Res. Commun. 201:102-107(1994).

498. Ribosomal protein L14 signature
Ribosomal protein L14 is one of the proteins from the large ribosomal subunit. In eubacteria, L14 is known to bind directly to the 23S rRNA. It belongs to a family of ribosomal proteins which, on the basis of sequence similarities [1], groups: - Eubacterial L14. - Algal and plant chloroplast L14. - Cyanelle L14.

- Archaebacterial L14. - Yeast L17A. - Mammalian L23.
- Caenorhabditis elegans L23 (B0336.10). - Higher eukaryotes mitochondrial L14.
- Yeast mitochondrial Ym138 (gene MRPL38).

L14 is a protein of 119 to 137 amino-acid residues. As a signature pattern, a conserved region located in the C-terminal half of these proteins was selected.

- Consensus pattern: [GA]-[LIV](3)-x(9,10)-[DNS]-G-x(4)-[FY]-x(2)-[NT]-x(2)-V-[LIV]

[1] Otaka E., Hashimoto T., Mizuta K., Suzuki K. Protein Seq. Data Anal. 5:301-313(1993).

499. Ribosomal protein L15 signature
Ribosomal protein L15 is one of the proteins from the large ribosomal subunit. In Escherichia coli, L15 is known to bind the 23 S rRNA. It belongs to a family of ribosomal proteins which, on the basis of sequence similarities [1], groups: - Eubacterial L15. - Plant chloroplast L15 (nuclear-encoded).

- Archaebacterial L15. - Vertebrate L27a. - Tetrahymena thermophila L29.
- Fungi L27a (L29, CRP-1, CYH2).

L15 is a protein of 144 to 154 amino-acid residues. As a signature pattern, a conserved region was selected in the C-terminal section of these proteins.

- Consensus pattern: K-[LIVM](2)-[GASL]-x-[GT]-x-[LIVMA]-x(2,5)-[LIVM]-x-[LIVMF]-x(3,4)-[LIVMFCA]-[ST]-x(2)-A-x(3)-[LIVM]-x(3)-G

[1] Otaka E., Hashimoto T., Mizuta K., Suzuki K. Protein Seq. Data Anal. 5:301-313(1993).

500. Ribosomal protein L15e signature
A number of eukaryotic and archaebacterial ribosomal proteins can be grouped on the basis of sequence similarities [1]. One of these families consists of:

- Mammalian L15. - Insect L15. - Plant L15. - Yeast YL10 (L13) (Rp15r).
- Thermoplasma acidophilum L15.

These proteins have about 200 amino acid residues. As a signature pattern, a conserved region was selected located in the central section.

- Consensus pattern: [DE]-[KR]-A-R-x-L-G-[FY]-x-[SAP]-x(2)-G-[LIVMFY](4)-R-x-R-[IV]-x-R-G

[1] Zwickl P., Lupas A., Baumeister W. Biochem. Biophys. Res. Commun. 209:684-688(1995).

501. Ribosomal protein L17 signature
Ribosomal protein L17 is one of the proteins from the large ribosomal subunit. L17 belongs to a family of ribosomal proteins which, on the basis of sequence similarities, groups: - Eubacterial L17.

- Yeast mitochondrial YmL8 (gene MRPL8). Eubacterial L17 is a protein of 120 to 130 amino-acid residues. Yeast YmL8 is twice larger (238 residues), the sequence of its N-terminal half is colinear with that of eubacterial L17. As a signature pattern, a conserved region in the N-terminal section was selected.
- Consensus pattern: I-x-[ST]-[GT]-x(2)-[KR]-x-K-x(6)-[DE]-x-[LIMV]-[LIVMT]-T-x-[STAG]-[KR]

502. Ribosomal protein L18e signature
A number of eukaryotic and archaebacterial ribosomal proteins can be grouped on the basis of sequence similarities. One of these families consists of:

- Vertebrate L18 (known as L14 in Xenopus) [1]. - Plant L18.
- Yeast L18 (Rp28). - Halobacterium marismortui H129.
- Sulfolobus acidocaldarius H129e.

These proteins have 115 to 187 amino-acid residues., A stretch of about 13 residues in the first third of these proteins has been selected as a signature pattern.

- Consensus pattern: [KRE]-x-L-x(2)-[PS]-[KR]-x(2)-[RH]-[PSA]-x-[LIVM]-[NS]-[LIVM]-x-[RK]-[LIVM]

[1] Puder M., Barnard G.F., Staniunas R.J., Steele G.D. Jr., Chen L.B. Biochim. Biophys. Acta 1216:134-136 (1993).

503. Ribosomal L18p family
It has been shown that the amino terminal 93 amino acids of Swiss:P09895 are necessary and sufficient to bind 5S rRNA in vitro. The carboxyl-terminal half of the protein, comprising amino acids 151-296, serves to localize the protein to the nucleolus [1].
Number of members: 26

[1]
Medline: 96212235
Distinct domains in ribosomal protein L5 mediate 5 S rRNA binding and nucleolar localization.
Michael WM, Dreyfuss G; J Biol Chem 1996;271:11571-11574.

504. Ribosomal protein L19 signature
Ribosomal protein L19 is one of the proteins from the large ribosomal subunit. In Escherichia coli, L19 is known to be located at the 30S-50S ribosomal subunit interface and may play a role in the structure and function of the aminoacyl-tRNA binding site. It belongs to a family of ribosomal proteins which, on the basis of sequence similarities, groups: - Eubacterial L19.
- Red algal chloroplast L19. - Cyanelle L19.
L19 is a protein of 120 to 130 amino-acid residues.,
A conserved region in the C-terminal section has been selected as a signature pattern.

- Consensus pattern: [LIVM]-x-[KRGTI]-x-[GSAI]-[KRQDA]-[VG]-[RSN]-X(0,1)-[KR]-[SA]-[KY]-[KLI]-[LYS]-Y-[LIM]-R

505. Ribosomal protein L19e signature
A number of eukaryotic and archaebacterial ribosomal proteins can be grouped on the basis of sequence similarities. One of these families consists of:

- Mammalian ribosomal protein L19 [1]. - Drosophila ribosomal protein L19 [2].
- Slime mold (D. discoideum) vegetative specific protein V14 [3].
- Yeast ribosomal protein L19 (YL14). - Archebacterial ribosomal protein L19E.

These proteins have 148 to 203 amino-acid residues.
A stretch of about 20 residues in the N-terminal part of these proteins has been selected as a signature pattern.

- Consensus pattern: Q-[KR]-R-[LIVM]-x-[SA]-x(4)-[CV]-G-x(3)-[IV]-[WK]-[LIVF]-[DN]-P

[1] Chan Y.-L., Lin A., McNally J., Peleg D., Meyuhas O., Wool I.G. J. Biol. Chem. 262:1111-1115(1987).
[2] Hart K., Klein T., Wilcox M. Mech. Dev. 43:101-110(1993).
[3] Singleton C.K., Manning S.S., Ken R. Nucleic Acids Res. 17:9679-9692(1989).

506. Ribosomal protein L1e signature (Ribosomal_L4)
A number of eukaryotic and archaebacterial ribosomal proteins can be grouped on the basis of sequence similarities.

One of these families consists [1,2,3, 4] of: - Vertebrate L1 (L4). - Drosophila L1. - Plant L1. - Yeast L2 (Rp2).

- Fission yeast L2. - Halobacterium marismortui HmaL4 (HL6).
- Methanococcus jannaschii MJ0177.

These proteins have 246 (archaebacteria) to 427 (human) amino acids. A conserved region in the N-terminal part of these proteins has been selected as a signature pattern.

- Consensus pattern: N-x(3)-[KRM]-x(2)-A-[LIVT]-x-S-A-[LIV]-x-A-[ST]-[SGA]-x(7)-[RK]-[GS]-H

[1] Rafti F., Gargiulo G., Manzi A., Malva C., Graziani F. Nucleic Acids Res. 17:456-456(1989).
[2] Presutti C., Villa T., Bozzoni I. Nucleic Acids Res. 21:3900-3900(1993).
[3] Bagni C., Mariottini P., Annesi F., Amaldi F. Biochim. Biophys. Acta 1216:475-478(1993).
[3] Arndt E., Kroemer W., Hatakeyama T. J. Biol. Chem. 265:3034-3039(1990).

507. Ribosomal protein L2 signature
Ribosomal protein L2 is one of the proteins from the large ribosomal subunit. In Escherichia coli, L2 is known to bind to the 23S rRNA and to have peptidyltransferase activity. It belongs to a family of ribosomal proteins which, on the basis of sequence similarities [1,2], groups: - Eubacterial L2.

- Algal and plant chloroplast L2. - Cyanelle L2. - Archaebacterial L2.
- Plant L2. - Slime mold L2. - Marchantia polymorpha mitochondrial L2.
- Paramecium tetraurelia mitochondrial L2. - Fission yeast K5, K37 and KD4.
- Yeast YL6. - Vertebrate L8.

The best conserved region located in the C-terminal section of these proteins has been selected as a signature pattern.

- Consensus pattern: P-x(2)-R-G-[STAIV](2)-x-N-[APK]-x-[DE]

[1] Marty I., Meyer Y. Nucleic Acids Res. 20:1517-1522(1992).
[2] Otaka E., Hashimoto T., Mizuta K., Suzuki K. Protein Seq. Data Anal. 5:301-313(1993).

508. Ribosomal protein L20 signature
Ribosomal protein L20 is one of the proteins from the large ribosomal subunit.
In Escherichia coli, L20 is known to bind directly to the 23 S rRNA. It belongs to a family of ribosomal proteins which, on the basis of sequence similarities [1], groups: - Eubacterial L20. - Algal and plant chloroplast L20.

- Cyanelle L20.
  L20 is a protein of about 120 amino-acid residues. A conserved region located in the central section of these proteins has been selected as a signature pattern.
- Consensus pattern: K-x(3)-[KRC]-x-[LIVM]-W-[IV]-[STNALV]-R-[LIVM]-[NS]-x(3)-[RKHS]

[1] Otaka E., Hashimoto T., Mizuta K., Suzuki K. Protein Seq. Data Anal. 5:301-313(1993).

509. Ribosomal protein L21 signature
A number of eukaryotic and archaebacterial ribosomal proteins can be grouped on the basis of sequence similarities. One of these families consists of:

- Mammalian L21 [1]. - Entamoeba histolytica L21 [2].
- Caenorhabditis elegans L21 (C14B9.7). - Yeast L21E (URP1) [3].
- Halobacterium marismortui HL31 [4].

These proteins have 160 (eukaryotes) or 95 (archebacteria) amino-acid residues. A conserved region in the central part of these proteins has been selected as a signature pattern.

- Consensus pattern: G-[DE]-x-V-x(10)-[GV]-x(2)-[FYH]-x(2)-[FY]-x-G-x-T-G

[1] Devi K.R.G., Chan Y.-L., Wool I.G. Biochem. Biophys. Res. Commun. 162:364-370(1989).
[2] Petter R., Rozenblatt S., Nuchamowitz Y., Mirelman D. Mol. Biochem. Parasitol. 56:329-333(1992).
[3] Jank B., Waldherr M., Schweyen R.J. Curr. Genet. 23:15-18(1993).
[4] Hatakeyama T., Kimura M. Eur. J. Biochem. 172:703-711(1988).

510. Ribosomal protein L21 signature
Ribosomal protein L21 is one of the proteins from the large ribosomal subunit. In Escherichia coli, L21 is known to bind to the 23S rRNA in the presence of L20. It belongs to a family of ribosomal proteins which, on the basis of sequence similarities, groups: - Eubacterial L21.

- Marchantia polymorpha chloroplast L21. - Cyanelle L21.
- Spinach chloroplast L21 (nuclear-encoded).

Eubacterial L21 is a protein of about 100 amino-acid residues, the mature form of the spinach chloroplast L21 has 200 residues. A conserved region located in the C-terminal section of these proteins has been selected as a signature pattern.

- Consensus pattern: [IVT]-x(3)-[KR]-x(3)-[KRQ]-K-x(6)-G-[HF]-R-[RQ]-x(2)-[ST]

511. Ribosomal protein L22 signature
Ribosomal protein L22 is one of the proteins from the large ribosomal subunit. In Escherichia coli, L22 is known to bind 23 S rRNA. It belongs to a family of ribosomal proteins which, on the basis of sequence similarities [1,2,3], groups: - Eubacterial L22.

- Algal and plant chloroplast L22 (in legumes L22 is encoded in the nucleus instead of the chloroplast). - Cyanelle L22. - Archaebacterial L22.
- Mammalian L17. - Plant L17. - Yeast YL17.

A conserved region located in the C- terminal section of these proteins has been selected as a signature pattern.

- Consensus pattern: [RKQN]-x(4)-[RH]-[GAS]-x-G-[KRQS]-x(9)-[HDN]-[LIVM]-x-[LIVMS]-x-[LIVM]

[1] Gantt J.S., Baldauf S.L., Calie P.J., Weeden N.F., Palmer J.D. EMBO J. 10:3073-3078(1991).
[2] Madsen L.H., Kreiberg J.D., Gausing K. Curr. Genet. 19:417-422(1991).
[3] Otaka E., Hashimoto T., Mizuta K., Suzuki K. Protein Seq. Data Anal. 5:301-313(1993).

512. Ribosomal protein L23 signature
Ribosomal protein L23 is one of the proteins from the large ribosomal subunit. In Escherichia coli, L23 is known to bind a specific region on the 23S rRNA; in yeast, the corresponding protein binds to a homologous site on the 26S rRNA

[1]. It belongs to a family of ribosomal proteins which, on the basis of sequence similarities [2,3,4], groups: - Eubacterial L23.

- Algal and plant chloroplast L23. - Archaebacterial L23. - Mammalian L23A.
- Caenorhabditis elegans L23A (FSSD10.2). - Fungi L25.
- Yeast mitochondrial YmL41 (gene MRPL41 or MRP20).

A small conserved region in the C-terminal section of these proteins, which is probably involved in rRNA-binding has been selected as a signature pattern [2].

- Consensus pattern: [RK](2)-[AM]-[IVFYT]-[IV]-[RKT]-L-[STANEQK]-x(7)-[LIVMFT]

[1] E1 Baradi T.T.A.L., Raue H.A., van de Regt C.H.F., Verbree E.C., Planta R.J. EMBO J. 4:210-2107(1985).
[2] Raue H.A., Otaka E., Suzuki K. J. Mol. Evol. 28:418-426(1989).
[3] Fearon K., Mason T.L. J. Biol. Chem. 267:5162-5170(1992).
[4] Otaka E., Hashimoto T., Mizuta K. Protein Seq. Data Anal. 5:285-300(1993).

513. Ribosomal protein L24 signature

Ribosomal protein L24 is one of the proteins from the large ribosomal subunit. L24 belongs to a family of ribosomal proteins which, on the basis of sequence similarities, groups: - Eubacterial L24.

- Plant chloroplast L24 (nuclear-encoded). - Red algal L24. - Vertebrate L26.
- Yeast L26 (YL33). - Archaebacterial HmaL24 (HL15).
- A probable ribosomal protein from Sulfolobus acidocaldarius [1].

In their mature form, these proteins have 103 to 150 amino-acid residues.
A conserved stretch of 20 residues in their N-terminal section has been selected as a signature pattern.

- Consensus pattern: [GDEN]-D-x-V-x-[IV]-[LIVMA]-x-G-x(2)-[KRA]-[GNQ]-x(2,3)-[GA]-x-[IV]

[1] Ouzounis C., Kyrpides N., Sander C. Nucleic Acids Res. 23:565-570(1995).

514. Ribosomal protein L24e signature

A number of eukaryotic and archaebacterial ribosomal proteins can be grouped on the basis of sequence similarities. One of these families consists [1] of:

- Mammalian ribosomal protein L24.
- Yeast ribosomal protein L30A/B (Rp29) (YL21).
- Kluyveromyces lactis ribosomal protein L30.
- Arabidopsis thaliana ribosomal protein L24 homolog.
- Haloarcula marismortui ribosomal protein HL21/HL22.
- Methanococcus jannaschii MJ1201.

These proteins have 60 to 160 amino-acid residues. The most conserved region, which is located in the N-terminal region of these proteins has been selected as a signature pattern.

- Consensus pattern: [FY]-x-[GSH]-x(2)-[IV]-x-P-G-x-G-x(2)-[FYV]-x-[KRHE]-x-D

[1] Chan Y.-L., Olvera J., Wool I.G. Biochem. Biophys. Res. Commun. 202:1176-1180(1994).

515. Ribosomal protein L27 signature

Ribosomal protein L27 is one of the proteins from the large ribosomal subunit. L27 belongs to a family of ribosomal proteins which, on the basis of sequence similarities [1,2], groups: - Eubacterial L27.

- Plant chloroplast L27 (nuclear-encoded). - Algal chloroplast L27.
- Yeast mitochondrial YmL2 (gene MRPL2 or MRP7).

The schematic relationship between these groups of proteins is shown below. Eub. L27 NxxxxxxxxxAlgal L27 Nxxxxxxxxx
Plant L27 tttttNxxxxxxxxxxxxx
Yeast MRP7 tttNxxxxxxxxxxxxxxxxxxxxxxxxxxxxxxxxxxx
***'t': transit peptide.
N': N-terminal of mature protein.'*': position of the pattern.

- Consensus pattern: G-x-[LIVM](2)-x-R-Q-R-G-x(5)-G

[1] Elhag G.A., Bourque D.P. Biochemistry 31:6856-6864(1992).
[2] Otaka E., Hashimoto T., Mizuta K. Protein Seq. Data Anal. 5:285-300(1993).

516. Ribosomal L28 family

The ribosomal 28 family includes L28 proteins from bacteria and chloroplasts. The L24 protein from yeast Swiss: P36525 also contains a region of similarity to prokaryotic L28 proteins. L24 from yeast is also found in the large ribosomal subunit
Number of members: 24

517. Ribosomal protein L29 signature
Ribosomal protein L29 is one of the proteins from the large ribosomal subunit. L29 belongs to a family of ribosomal proteins which, on the basis of sequence similarities [1], groups: - Eubacterial L29. - Red algal L29.

- Archaebacterial L29. - Mammalian L35 - Caenorhabditis elegans L35 (ZK652.4).
- Yeast L35.

L29 is a protein of 63 to 138 amino-acid residues.
A conserved region located in the central section of L29 has been selected as a signature pattern.

- Consensus pattern: [KNQS]-[PSTL]-x(2)-[LIMFA]-[KRGSAN]-x-[LIVYSTA]-[KR]-[KRHQS]-[DESTANRL]-[LIV]-A-[KRCQVT]-[LIVMA]

[1] Otaka E., Hashimoto T., Mizuta K. Protein Seq. Data Anal. 5:285-300(1993).

518. Ribosomal protein L3 signature
Ribosomal protein L3 is one of the proteins from the large ribosomal subunit.
In Escherichia coli, L3 is known to bind to the 23S rRNA and may participate in the formation of the peptidyltransferase center of the ribosome. It belongs to a family of ribosomal proteins which, on the basis of sequence similarities [1,2,3,4], groups: - Eubacterial L3. - Red algal L3. - Cyanelle L3.

- Archaebacterial Halobacterium marismortui HmaL3 (HL1).
- Yeast L3 (also known as trichodermin resistance protein) (gene TCM1).
- Arabidopsis thaliana L3 (genes ARP1 and ARP2). - Mammalian L3 (L4).
- Mammalian mitochondrial L3. - Yeast mitochondrial YmL9 (gene MRPL9).

A conserved region located in the central section of these proteins has been selected as a signature pattern.

- Consensus pattern: [FL]-x(6)-[DN]-x(2)-[AGS]-x-[ST]-x-G-[KRH]-G-x(2)-G-x(3)-R

[1] Arndt E., Kroemer W., Hatakeyama T. J. Biol. Chem. 265:3034-3039(1990).
[2] Graack H.-R., Grohmann L., Kitakawa M., Schaefer K.L., Kruft V. Eur. J. Biochem. 206:373-380(1992).
[3] Herwig S., Kruft V., Wittmann-Liebold B. Eur. J. Biochem. 207:877-885(1992).
[4] Otaka E., Hashimoto T., Mizuta K., Suzuki K. Protein Seq. Data Anal. 5:301-313(1993).

519. Ribosomal protein L30 signature
Ribosomal protein L30 is one of the proteins from the large ribosomal subunit. L30 belongs to a family of ribosomal proteins which, on the basis of sequence similarities [1], groups: - Eubacterial L30. - Archaebacterial L30.

- Drosophila L7. - Slime mold L7. - Mammalian L7. - Fungi L7 (YL8).
- Yeast mitochondrial L33.

L30 from eubacteria are small proteins of about 60 residues, those from archaebacteria are proteins of about 150 residues. Eukaryotic L7 are proteins of about 250 to 270 residues. The schematic relationship between the three groups of proteins is shown below.Eub. L30 NxxxxxxxxxxC
Arc.L30 NxxxxxxxxxxxxxxxxxxxxxxxxxxxC
Euk.L7 NxxxxxxxxxxxxxxxxxxxxxxxxxxxxxxxxxxxxxxxxxxxxxxxxxxC
*******'*': position of the pattern.
The signature pattern for this family of ribosomal proteins spans the N-terminal half of the region common to all these proteins.

- Consensus pattern: [IVT]-[LIVM]-x(2)-[LF]-x-[LI]-x-[KRHQEG]-x(2)-[STNQH]-x-[IVT]-x(10)-[LMS]-[LIV]-x(2)-[LIVA]-x(2)-[LMFY]-[IVT]

[1] Mizuta K., Hashimoto T., Otaka E. Nucleic Acids Res. 20:1011-1016(1992).

520. Ribosomal protein L31 signature
Ribosomal protein L31 is one of the proteins from the large ribosomal subunit.

L31 is a protein of 66 to 97 amino-acid residues which has only been found so far in eubacteria and in some algal chloroplasts.
A conserved region located in the central section of these proteins has been selected as a signature pattern.

- Consensus pattern: H-P-F-[FY]-[TI]-x(9)-G-R-[AIV]-x-[KRQ]

521. Ribosomal protein L31 e signature
A number of eukaryotic and archaebacterial ribosomal proteins can be grouped on the basis of sequence similarities. One of these families consists of:

- Mammalian L31 [1]. - Chlamydomonas reinhardtii L31. - Yeast L34.
- Halobacterium marismortui HL30 [2].

These proteins have 87 to 128 amino-acid residues.
A conserved region, located in the central section has been selected as a signature pattern.

- Consensus pattern: V-[KR]-[LIVM]-x(3)-[LIVM]-N-x-[AKH]-x-W-x-[KR]-G

[1] Tanaka T., Kuwano Y., Kuzumaki T., Ishikawa K., Ogata K. Eur. J. Biochem. 162:45-48(1987).
[2] Bergmann U., Arndt E. Biochim. Biophys. Acta 1050:56-60(1990).

522. Ribosomal protein L33 signature
Ribosomal protein L33 is one of the proteins from the large ribosomal subunit.
In Escherichia coli, L33 has been shown to be on the surface of50S subunit. L33 belongs to a family of ribosomal proteins which, on the basis of sequence similarities [1,2,3], groups: - Eubacterial L33.

- Algal and plant chloroplast L33. - Cyanelle L33.

L33 is a small protein of 49 to 66 amino-acid residues. A conserved region located in the central section of L33 has been selected as a signature pattern.

- Consensus pattern: Y-x-[ST]-x-[KR]-[NS]-x(4)-[PATQ]-x(1,2)-[LIVM]-[EA]-x(2)-K-[FY]-[CSD]

[1] Kruft V., Kapp U., Wittmann-Liebold B. Biochimie 73:855-860(1991).
[2] Sharp P.M. Gene 139:129-130(1994).
[3] Otaka E., Hashimoto T., Mizuta K. Protein Seq. Data Anal. 5:285-300(1993).

523. Ribosomal protein L34 signature
Ribosomal protein L34 is one of the proteins from the large subunit of the prokaryotic ribosome. It is a small basic protein of 44 to 51 amino-acid residues [1]. L34 belongs to a family of ribosomal proteins which, on the basis of sequence similarities, groups: - Eubacterial L34.

- Red algal chloroplast L34. - Cyanelle L34.

A conserved region that corresponds to the N-terminal half of L34 has been selected as a signature pattern.

- Consensus pattern: K-[RG]-T-[FYWL]-[EQS]-x(5)-[KRHS]-x(4,5)-G-F-x(2)-R

[1] Old I.G., Margarita D., Saint Girons I. Nucleic Acids Res. 20:6097-6097(1992).

524. Ribosomal protein L34e signature
A number of eukaryotic and archaebacterial ribosomal proteins can be grouped on the basis of sequence similarities. One of these families consists of:

- Mammalian L34. - Mosquito L31 [1]. - Plant L34 [2].
- Yeast putative ribosomal protein YIL052c. - Methanococcus jannaschii MJ0655. These proteins have 89 to 129 amino-acid residues.

A conserved region located in the N-terminal section of these proteins has been selected as a signature pattern.

- Consensus pattern: Y-x-[ST]-x-S-[NY]-x(5)-[KR]-T-P-G

[1] Lan Q., Niu L.L., Fallon A.M. Biochim. Biophys. Acta 1218:460-462(1994).
[2] Gao J., Kim S.R., Chung Y.Y., Lee J.M., An G. Plant Mol. Biol. 25:761-770(1994).

525. Ribosomal protein L35Ae signature
A number of eukaryotic and archaebacterial ribosomal proteins can be grouped on the basis of sequence similarities. One of these families consists of:

- Vertebrate L35A. - Caenorhabditis elegans L35A (F10E7.7).
- Yeast L37A/L37B (Rp47). - Pyrococcus woesei L35A homolog [1].

These proteins have 87 to 110 amino-acid residues.
A highly conserved stretch of 22 residues in the C-terminal part of these proteins has been selected as a signature pattern.

- Consensus pattern: G-K-[LIVM]-x-R-x-H-G-x(2)-G-x-V-x-A-x-F-x(3)-[LI]-P

[1] Ouzounis C., Kyrpides N., Sander C. Nucleic Acids Res. 23:565-570(1995).

526. Ribosomal protein L36 signature
Ribosomal protein L36 is the smallest protein from the large subunit of the prokaryotic ribosome. It belongs to a family of ribosomal proteins which, on the basis of sequence similarities [1], groups: - Eubacterial L36. - Algal and plant chloroplast L36. - Cyanelle L36.L36 is a small basic and cysteine-rich protein of 37 amino-acid residues. As a signature pattern, a conserved region that corresponds to positions 11 to 36 in L36 and includes three conserved cysteine residues has been developed.
Consensus pattern: C-x(2)-C-x(2)-[LIVM]-x-R-x(3)-[LIVMN]-x-[LIVM]-x-C-x(3,4)-[KR]-H-x-Q-x-Q-

[1] Otaka E., Hashimoto T., Mizuta K. Protein Seq. Data Anal. 5:285-300(1993).

527. Ribosomal protein L36e signature
A number of eukaryotic ribosomal proteins can be grouped on the basis of sequence similarities. One of these families consists of: - Mammalian L36 [1].

- Drosophila L36 (M(1)1B). - Caenorhabditis elegans L36 (F37C12.4).
- Candida albicans L39. - Yeast YL39.

These proteins have 99 to 104 amino acids.
A conserved region in the central part of these proteins has been selected as a signature pattern.

- Consensus pattern: P-Y-E-[KR]-R-x-[LIVM]-[DE]-[LIVM](2)-[KR]

[1] Chan Y.-L., Paz V., Olvera J., Wool I.G. Biochem. Biophys. Res. Commun. 192:849-853(1993).

528. Ribosomal protein L39e signature
A number of eukaryotic and archaebacterial ribosomal proteins can be grouped on the basis of sequence similarities. One of these families consists of:

- Mammalian L39 [1]. - Plants L39. - Yeast L46 [2]. - Archebacterial L39e [3].

These proteins are very basic. About 50 residues long, they are the smallest proteins of eukaryotic-type ribosomes. A conserved region in the C-terminal section of these proteins has been selected as a signature pattern.

- Consensus pattern: [KRA]-T-x(3)-[LIVM]-[KRQF]-x-[NHS]-x(3)-R-[NHY]-W-R-R

[1] Lin A., McNally J., Wool I.G. J. Biol. Chem. 259:487-490(1984).

[2] Leer R.J., van Raamsdonk-Duin M.M.C., Kraakman P., Mager W.H., Planta R.J. Nucleic Acids Res. 13: 701-709(1985).
[3] Ramirez C., Louie K.A., Matheson A.T. FEBS Lett. 250:416-418(1989).

529. Ribosomal L40e family
Bovine L40 has been identified as a secondary RNA binding protein [1]. L40 is fused to a ubiquitin protein [2].
Number of members: 27

[1]
Medline: 88203200
RNA binding proteins of the large subunit of bovine mitochondrial ribosomes.
Piatyszek MA, Denslow ND, O'Brien TW; Nucleic Acids Res 1988;16:2565-2583.
[2]Medline: 96011832
The carboxyl extensions of two rat ubiquitin fusion proteins are ribosomal proteins S27a and L40.
Chan YL, Suzuki K, Wool IG; Biochem Biophys Res Commun 1995;215:682-690.

530. (Ribosomal L44) Ribosomal protein L44e signature
A number of eukaryotic and archaebacterial ribosomal proteins can be grouped on the basis of sequence similarities. One of these families consists of:

- Mammalian L44 [1]. - Trypanosoma brucei L44.
- Caenorhabditis elegans L44 (C09H10.2). - Fungal L44 (L41).
- Halobacterium marismortui LA [2].

These proteins have 92 to 105 amino-acid residues.
A conserved region located in the C-terminal part of these proteins has been selected as a signature pattern.

- Consensus pattern: K-x-[TV]-K-K-x(2)-L-[KR]-x(2)-C

[1] Gallagher M.J., Chan Y.-L., Lin A., Wool I.G. DNA 7:269-273(1988).
[2] Bergmann U., Wittmann-Liebold B. Biochim. Biophys. Acta 1173:195-200(1993

531. Ribosomal protein L5 signature
Ribosomal protein L5 is one of the proteins from the large ribosomal subunit.
In Escherichia coli, L5 is known to be involved in binding 5S RNA to the large ribosomal subunit. It belongs to a family of ribosomal proteins which, on the basis of sequence similarities [1,2,3,4], groups: - Eubacterial L5.

- Algal chloroplast L5. - Cyanelle L5. - Archaebacterial L5. - Mammalian L11.
- Tetrahymena thermophila L21. - Slime mold L5 (V18). - Yeast L16 (39A).
- Plants mitochondrial L5.

L5 is a protein of about 180 amino-acid residues.
A conserved region, located in the first third of these proteins has been selected as a signature pattern.

- Consensus pattern: [LIVM]-x(2)-[LIVM]-[STAVC]-[GE]-[QV]-x(2)-[LIVMA]-x-[STC]-x-[STAG]-[KRH]-x-[STA]

[1] Hatakeyama T., Hatakeyama T. Biochim. Biophys. Acta 1039:343-347(1990).
[2] Rosendahl G., Andreasen P.H., Kristiansen K. Gene 98:161-167(1991).
[3] Yang D., Gunther I., Matheson A.T., Auer J., Spicker G., Boeck A. Biochimie 73:679-682(1991).
[4] Otaka E., Hashimoto T., Mizuta K., Suzuki K. Protein Seq. Data Anal. 5:301-313(1993).

532. ribosomal L5P family C-terminus
This region is found associated with Ribosomal_L5.
Number of members: 60

533. Ribosomal protein L6 signatures
Ribosomal protein L6 is one of the proteins from the large ribosomal subunit. In Escherichia coli, L6 is known to bind directly to the 23 S rRNA and is located at the aminoacyl-tRNA binding site of the peptidyltransferase center.

It belongs to a family of ribosomal proteins which, on the basis of sequence similarities [1,2,3,4], groups: - Eubacterial L6.

- Algal chloroplast L6.
- Cyanelle L6.
- Archaebacterial L6.
- Marchantia polymorpha mitochondrial L6.
- Yeast mitochondrial YmL6 (gene MRPL6).
- Mammalian L9.
- Drosophila L9.
- Plants L9.
- Yeast L9 (YL11).

While all the above proteins are evolutionary related it is very difficult to derive a pattern that will find them all. Two patterns were therefore created, the first to detect eubacterial, cyanelle and mitochondrial L6, the second to detect archaebacterial L6 as well as eukaryotic L9.

- Consensus pattern: [PS]-[DENS]-x-Y-K-[GA]-K-G-[LIVM]
- Consensus pattern: Q-x(3)-[LIVM]-x(2)-[KR]-x(2)-R-x-F-x-D-G-[LIVM]-Y-[LIVM]-x(2)-[KR]

[1] Suzuki K., Olvera J., Wool I.G. Gene 93:297-300(1990).
[2] Schwank S., Harrer R., Schueller H.-J., Schweizer E. Curr. Genet. 24:136-140(1993).
[3] Golden B.L., Ramakrishnan V., White S.W. EMBO J. 12:4901-4908(1993).
[4] Otaka E., Hashimoto T., Mizuta K., Suzuki K. Protein Seq. Data Anal. 5:301-313(1993).

534. Ribosomal protein L6e signature
A number of eukaryotic and archaebacterial ribosomal proteins can be grouped on the basis of sequence similarities. One of these families consists of:

- Mammalian ribosomal protein L6 (L6 was previously known as TAX-responsive enhancer element binding protein 107).
- Caenorhabditis elegans ribosomal protein L6 (R151.3).
- Yeast ribosomal protein YL16A/YL16B.
- Mesembryanthemum crystallinum ribosomal protein YL16-like.

These proteins have 175 (yeast) to 287 (mammalian) amino acids. A highly conserved region in the central part of these proteins has been selected as a signature pattern.

- Consensus pattern: N-x(2)-P-L-R-R-x(4)-[FY]-V-I-A-T-S-x-K

535. Ribosomal protein L7Ae signature
A number of eukaryotic and archaebacterial ribosomal proteins can be grouped on the basis of sequence similarities. One of these families consists of:

- Vertebrate L7A (SURF3) [1]. - Plant L7A. - Yeast L7A (YL5) (Rp6).
- Yeast protein NHP2 [2]. - Yeast hypothetical protein YEL026w.
- Bacillus subtilis hypothetical protein ylxQ. - Halobacterium marismortui Hs6.
- Methanococcus jannaschii MJ1203.

These proteins have 100 to 265 amino-acid residues.
A conserved region located in the central section has been selected as a signature pattern.

- Consensus pattern: [CA]-x(4)-[IV]-P-[FY]-x(2)-[LIVM]-x-[GSQ]-[KRQ]-x(2)-L-G

[1] Colombo P., Yon J., Garson K., Fried M. Proc. Natl. Acad. Sci. U.S.A. 89:6358-6362(1992).
[2] Kolodrubetz D., Burgum A. Yeast 7:79-90(1991).

536. Ribosomal protein L9 signature

Ribosomal protein L9 is one of the proteins from the large ribosomal subunit.
In Escherichia coli, L9 is known to bind directly to the 23 S rRNA. It belongs to a family of ribosomal proteins which, on the basis of sequence similarities [1,2], groups: - Eubacterial L9. - Cyanobacterial L9.

- Plant chloroplast L9 (nuclear-encoded). - Red algal chloroplast L9.

A conserved region, located in the N-terminal section of these proteins has been selected as a signature pattern.

- Consensus pattern: G-x(2)-[GN]-x(4)-V-x(2)-G-[FY]-x(2)-N-[FY]-L-x(5)-[GA]-x(3)-[STN]

[1] Hoffman D.W., Davies C., Gerchman S.E., Kycia J.H., Porter S.J., White S.W., Ramakrishnan V. EMBO J. 13:205-212(1994).
[2] Otaka E., Hashimoto T., Mizuta K., Suzuki K. Protein Seq. Data Anal. 5:301-313(1993).

537. Ribosomal protein S10 signature
Ribosomal protein S10 is one of the proteins from the small ribosomal subunit. In Escherichia coli, S10 is known to be involved in binding tRNA to the ribosomes. It belongs to a family of ribosomal proteins which, on the basis of sequence similarities [1], groups: - Eubacterial S10.

- Algal chloroplast S10. - Cyanelle S10. - Archaebacterial S10.
- Marchantia polymorpha and Prototheca wickerhamii mitochondrial S10.
- Arabidopsis thaliana mitochondrial S10 (nuclear encoded). - Vertebrate S20.
- Plant S20. - Yeast URP2.

S10 is a protein of about 100 amino-acid residues.
A conserved region located in the center of these proteins has been selected as a signature pattern.

- Consensus pattern: [AV]-x(3)-[GDNSR]-[LIVMSTA]-x(3)-G-P-[LIVM]-x-[LIVM]-P-T

[1] Otaka E., Hashimoto T., Mizuta K. Protein Seq. Data Anal. 5:285-300(1993).

538. Ribosomal protein S11 signature
Ribosomal protein S11 [1] plays an essential role in selecting the correct tRNA in protein biosynthesis. It is located on the large lobe of the small ribosomal subunit. S11 belongs to a family of ribosomal proteins which, on the basis of sequence similarities, groups [2]: - Eubacterial S11.

- Algal and plant chloroplast S11. - Cyanelle S11. - Archaebacterial S11.
- Marchantia polymorpha and Prototheca wickerhamii mitochondrial S11.
- Acanthamoeba castellanii mitochondrial S11. - Neurospora crassa S14 (crp-2).
- Yeast S14 (RP59 or CRY1).
- Mammalian, Drosophila, Trypanosoma, and plant S14.
- Caenorhabditis elegans S14 (F37C12.9).

One of the best conserved regions in these proteins was selected as a signature pattern.

- Consensus pattern: [LIVMF]-x-[GSTAC]-[LIVMF]-x(2)-[GSTAL]-x(0,1)-[GSN]-[LIVMF]-x-[LIVM]-x(4)-[DEN]-x-T-P-x-[PA]-[STCH]-[DN]

[1] Kimura M., Kimura J., Hatakeyama T. FEBS Lett. 240:15-20(1988).
[2] Otaka E., Hashimoto T., Mizuta K. Protein Seq. Data Anal. 5:285-300(1993).

539. Ribosomal protein S12 signature
Ribosomal protein S12 is one of the proteins from the small ribosomal subunit. In Escherichia coli, S12 is known to be involved in the translation initiation step. It is a very basic protein of 120 to 150 amino-acid residues. S12 belongs to a family of ribosomal proteins which, on the basis of sequence similarities [1], groups: - Eubacterial S12. Archaebacterial S12.

- Algal and plant chloroplast S12. - Cyanelle S12.

- Protozoa and plant mitochondrial S12. - Yeast S28.
- Drosophila mitochondrial protein tko (Technical KnockOut). - Mammalian S23.

The best conserved regions in these proteins, located in the center of each sequence have been selected as a signature pattern.

- Consensus pattern: [RK]-x-P-N-S-[AR]-x-R

[1] Otaka E., Hashimoto T., Mizuta K. Protein Seq. Data Anal. 5:285-300(1993).

540. Ribosomal protein S12e signature
A number of eukaryotic ribosomal proteins can be grouped on the basis of sequence similarities. One of these families consists of: - Vertebrate S12 [1].

- Trypanosoma brucei S12 [2]. - Caenorhabditis elegans S12 (F54E7.2).
- Drosophila S12. - Yeast S12.

These proteins have 130 to 150 amino acids.
A conserved region in the N-terminal part of these proteins has been selected as a signature pattern.

- Consensus pattern: A-L-[KRQP]-x-V-L-x(2)-[SA]-x(3)-[DN]-G-L

[1] Lin A., Chan Y.-L., Jones R., Wool I.G. J. Biol. Chem. 262:14343-14351 (1987).
[2] Marchal C., Ismaili N., Pays E. Mol. Biochem. Parasitol. 57:331-334(1993).

541. Ribosomal protein S13 signature
Ribosomal protein S13 is one of the proteins from the small ribosomal subunit.
In Escherichia coli, S13 is known to be involved in binding fMet-tRNA and, hence, in the initiation of translation. It is a basic protein of 115 to 177 amino-acid residues and belongs to a family of ribosomal proteins which, on the basis of sequence similarities [1,2], groups: - Eubacterial S13.

- Plant chloroplast S13 (nuclear encoded). - Red algal chloroplast 513.
- Cyanelle S13. - Archaebacterial S13. - Plant mitochondrial S13.
- Mammalian and plant S18.

The best conserved regions in these proteins, located in their C-terminal part have been selected as a signature pattern.

- Consensus pattern: [KRQS]-G-x-R-H-x(2)-[GSNH]-x(2)-[LIVMC]-R-G-Q

[1] Chan Y.-L., Paz V., Wool I.G. Biochem. Biophys. Res. Commun. 178:1212-1218(1991).
[2] Otaka E., Hashimoto T., Mizuta K. Protein Seq. Data Anal. 5:285-300(1993).

542. Ribosomal protein S14p/S29e (Ribosomal protein S14 signature)
Ribosomal protein S14 is one of the proteins from the small ribosomal subunit. In Escherichia coli, S14 is known to be required for the assembly of 30S particles and may also be responsible for determining the conformation of 16S rRNA at the A site. It belongs to a family of ribosomal proteins which, on the basis of sequence similarities [1,2], groups:

- Eubacterial S14.
- Algal and plant chloroplast S14.
- Cyanelle S14.
- Archaebacterial Methanococcus vannielii S14.
- Plant mitochondrial S14.
- Yeast mitochondrial MRP2.
- Mammalian S29.
- Yeast YS29A/B.

S14 is a protein of 53 to 115 amino-acid residues. Our signature pattern is based on the few conserved positions

located in the center of these proteins.
Consensus pattern: [RP]-x(0,1)-C-x(I 1,12)-[LIVMF]-x-[LIVMF]-[SC]-[RG]-x(3)-[RN]

[1] Chan Y.-L., Suzuki K., Olvera J., Wool I.G. Nucleic Acids Res. 21:649-655(1993).
[2] Otaka E., Hashimoto T., Mizuta K. Protein Seq. Data Anal. 5:285-300(1993).

543. Ribosomal protein S15 signature
Ribosomal protein S15 is one of the proteins from the small ribosomal subunit.
In Escherichia coli, this protein binds to 16S ribosomal RNA and functions at early steps in ribosome assembly. It belongs to a family of ribosomal proteins which, on the basis of sequence similarities [1,2], groups: - Eubacterial S15.

- Archaebacterial Halobacterium marismortui HmaS15 (HS11).
- Plant chloroplast S15. - Yeast mitochondrial S28. - Mammalian S 13.
- Brugia pahangi and Wuchereria bancrofti S13 (S15). - Yeast S13 (YS15).

S 15 is a protein of 80 to 250 amino-acid residues.
A conserved region located in the C-terminal part of these proteins has been selected as a signature pattern.

- Consensus pattern: [LIVM]-x(2)-H-[LIVMFY]-x(5)-D-x(2)-[SAGN]-x(3)-[LF]-x(9)-[LIVM]-x(2)-[FY]

[1] Dang H., Ellis S.R. Nucleic Acids Res. 18:6895-6901(1990).
[2] Otaka E., Hashimoto T., Mizuta K. Protein Seq. Data Anal. 5:285-300(1993).

544. Ribosomal protein S16 signature
Ribosomal protein S16 is one of the proteins from the small ribosomal subunit. It belongs to a family of ribosomal proteins which, on the basis of sequence similarities [1], groups:

- Eubacterial S16.
- Algal and plant chloroplast S16.
- Cyanelle S 16.
- Neurospora crassa mitochondrial S24 (cyt-21).

S16 is a protein of about 100 amino-acid residues. A conserved region located in the N-terminal extremity of these proteins has been selected as a signature pattern.
Consensus pattern: [LIVMT]-x-[LIVM]-[KR]-L-[STAK]-R-x-G-[AKR]

[1] Otaka E., Hashimoto T., Mizuta K. Protein Seq. Data Anal. 5:285-300(1993).

545. Ribosomal protein S17 signature
Ribosomal protein S17 is one of the proteins from the small ribosomal subunit.
In Escherichia coli, S17 is known to bind specifically to the 5' end of 16S ribosomal RNA and is thought to be involved in the recognition of termination codons. It belongs to a family of ribosomal proteins which, on the basis of sequence similarities [1,2,3], groups: - Eubacterial S17.

- Plant chloroplast S17 (nuclear encoded). - Red algal chloroplast S17.
- Cyanelle S17. - Archaebacterial S17. - Mammalian and plant cytoplasmic S11.
- Yeast S18a and S18b (RP41; YS12).

The best conserved regions located in the C-terminal sections of these proteins have been selected as a signature pattern.

- Consensus pattern: G-D-x-[LIV]-x-[LIVA]-x-[QEK]-x-[RK]-P-[LIV]-S

[1] Gantt J.S., Thompson M.D. J. Biol. Chem. 265:2763-2767(1990).
[2] Herfurth E., Hirano H., Wittmann-Liebold B. Biol. Chem. Hoppe-Seyler 372:955-961(1991).
[3] Otaka E., Hashimoto T., Mizuta K. Protein Seq. Data Anal. 5:285-300(1993).

546. Ribosomal protein S17e signature

A number of eukaryotic and archaebacterial ribosomal proteins can be grouped on the basis of sequence similarities. One of these families consists of:

- Vertebrates S17 [1]. - Drosophila S17 [2]. -Neurospora crassa S17 (crp-3).
- Yeast S17a (RP51A) and S17b (RP51B) [3]. - Methanococcus jannaschii MJ0245.

These proteins have from 63 (in archebacteria) to 130 to 146 amino acids and are highly conserved. A region in the central part of these proteins has been selected as a signature.

- Consensus pattern: A-x-I-x-[ST]-K-x-L-R-N-[KR]-I-A-G-[FY]-x-T-H

[1] Chen I.-T., Roufa D.J. Gene 70:107-116(1988).
[2] Maki C., Rhoads D.D., Stewart M.J., van Slyke B., Denell R.E., Roufa D.J. Gene 79:289-298(1989).
[3] Abovich N., Rosbash M. Mol. Cell. Biol. 4:1871-1879(1984).

547. Ribosomal protein S18 signature

Ribosomal protein S18 is one of the proteins from the small ribosomal subunit. In Escherichia coli, S18 has been involved in aminoacyl-tRNA binding[1]. It appears to be situated at the tRNA A-site of the ribosome. It belongs to a family of ribosomal proteins which, on the basis of sequence similarities[2], groups: - Eubacterial S18. - Algal and plant chloroplast 518. - Cyanelle S18.As a signature pattern, a conserved region in the central section of the protein has been selected. This region contains two basic residues which may be involved in RNA-binding.- Consensus pattern: [IV]-[DY]-Y-x(2)-[LIVMT]-x(2)-[LIVM]-x(2)-[FYT]-[LIVM]- [ST]-[DERP]-x-[GY]-K-[LIVM]-x (3)-R-[LIVMAS]-

[1] McDougall J., Choli T., Kruft V., Kapp U., Wittmann-Liebold B. FEBS Lett. 245:253-260(1989).
[2] Otaka E., Hashimoto T., Mizuta K. Protein Seq. Data Anal. 5:285-300(1993).

548. Ribosomal protein S19 signature

Ribosomal protein S19 is one of the proteins from the small ribosomal subunit.

In Escherichia coli, S19 is known to form a complex with S 13 that binds strongly to 16S ribosomal RNA. S19 belongs to a family of ribosomal proteins which, on the basis of sequence similarities [1,2], groups: - Eubacterial S19.

- Algal and plant chloroplast S19.- Cyanelle S19.- Archaebacterial S19.
- Plant mitochondrial S19. - Eukaryotic S15 ('rig'protein).

S19 is a protein of 88 to 144 amino-acid residues. Our signature pattern is based on the few conserved positions located in the C-terminal section of these proteins.

- Consensus pattern: [STDNQ]-G-[KRQM]-x(6)-[LIVM]-x(4)-[LIVM]-[GSD]-x(2)-[LF]-[GAS]-[DE]-F-x(2)-[ST]

[1] Kitagawa M., Takasawa S., Kikuchi N., Itoh T., Teraoka H., Yamamoto H., Okamoto H. FEBS Lett. 283: 210-214(1991).
[2] Otaka E., Hashimoto T., Mizuta K. Protein Seq. Data Anal. 5:285-300(1993).

549.Ribosomal protein S19e signature

A number of eukaryotic and archaebacterial ribosomal proteins can be grouped on the basis of sequence similarities [1,2]. One of these families consists of: - Mammalian S19. - Drosophila S19.

- Ascaris lumbricoides S19g (ALEP-1) and S19s. - Yeast YS16 (RP55A and RP55B).
- Aspergillus S16. - Halobacterium marismortui HS12.

These proteins have 143 to 155 amino acids.

A well conserved stretch of 20 residues in the C-terminal part of these proteins has been selected as a signature pattern.

- Consensus pattern: P-x(6)-[SAN]-x(2)-[LIVMA]-x-R-x-[ALIV]-[LV]-Q-x-L-[EQ]

[1] Etter A., Aboutanos M., Tobler H., Mueller F. Proc. Natl. Acad. Sci. U.S.A. 88:1593-1596(1991).

[2] Suzuki K., Olvera J., Wool I.G. Biochimie 72:299-302(1990).

550. Ribosomal protein S2 signatures
Ribosomal protein S2 is one of the proteins from the small ribosomal subunit. S2 belongs to a family of ribosomal proteins which, on the basis of sequence similarities [1,2], groups: - Eubacterial S2. - Algal and plant chloroplast S2.

- Cyanelle S2. - Archaebacterial S2.
- Higher eukaryotes P40 (previously thought to be a laminin receptor).
- Yeast NAB1.- Plant mitochondrial S2. - Yeast mitochondrial MRP4.

S2 is a protein of 235 to 394 amino-acid residues.
Two conserved regions have been selected as signature patterns. One is located in the N-terminal section and the other in the central section.

- Consensus pattern: [LIVMFA]-x(2)-[LIVMFYC](2)-x-[STAC]-[GSTANQEKR]-[STALV]-[HY]-[LIVMF]-G
- Consensus pattern: P-x(2)-[LIVMF](2)-[LIVMS]-x-[GDN]-x(3)-[DENL]-x(3)-[LIVM]-x-E-x(4)-[GNQKRH]-[LIVM]-[AP]

[1] Davis S.C., Tzagoloff A., Ellis S.R. J. Biol. Chem. 267:5508-5514(1992).
[2] Tohgo A., Takasawa S., Munakata H., Yonekura H., Hayashi N., Okamoto H. FEBS Lett. 340:133-138(1994).

551. Ribosomal protein S21 signature
Ribosomal protein S21 is one of the proteins from the small ribosomal subunit. So far S21 has only been found in eubacteria. It is a protein of 55 to 70 amino-acid residues. A conserved region in the N-terminal section of the protein has been selected as a signature pattern.
Consensus pattern: [DE]-x-A-[LIY]-[KR]-R-F-K-[KR]-x(3)-[KR]

552. Ribosomal protein S21e signature
A number of eukaryotic ribosomal proteins can be grouped on the basis of sequence similarities. One of these families consists of: - Mammalian S21 [1].

- Caenorhabditis elegans S21 (F37C12.11). - Rice S21 [2].
- Yeast S21 (Ys25) [3]. - Fission yeast S28 [4].

These proteins have 82 to 87 amino acids.
A perfectly conserved nonapeptide in the N-terminal part of these proteins has been selected as a signature pattern.

- Consensus pattern: L-Y-V-P-R-K-C-S-[SA]

[1] Bhat K.S., Morrison S.G. Nucleic Acids Res. 21:2939-2939(1993).
[2] Nishi R., Hashimoto H., Uchimiya H., Kato A. Biochim. Biophys. Acta 1216:113-114(1993).
[3] Suzuki K., Otaka E. Nucleic Acids Res. 16:6223-6223(1988).
[4] Itoh T., Okata E., Matsui K.A. Biochemistry 24:7418-7423(1985).

553. Ribosomal protein S24e signature
A number of eukaryotic and archaebacterial ribosomal proteins can be grouped on the basis of sequence similarities. One of these families consists of:

- Vertebrate S24 [1]. - Yeast Rp50. - Mucor racemosus S24 [2].
- Halobacterium marismortui HS15 [3]. - Methanococcus jannaschii MJ0394.

These proteins have 101 to 148 amino acids.
A well conserved stretch in the central part of these proteins has been selected as a signature pattern.

- Consensus pattern: [FYA]-G-x(2)-[KR]-[STA]-x-G-[FY]-[GA]-x-[LIVM]-Y-[DN]-[SDN]

[1] Brown S.J., Jewell A., Maki C.G., Roufa D.J. Gene 91:293-296(1990).

[2] Sosa L., Fonzi W.A., Sypherd P.S. Nucleic Acids Res. 17:9319-9331(1989).
[3] Kimura J., Arndt E., Kimura M. FEBS Lett. 224:65-70(1987).

554. Ribosomal protein S26e signature
A number of eukaryotic ribosomal proteins can be grouped on the basis of sequence similarities. One of these families consists of: - Mammalian S26 [1].

- Octopus S26 [2]. - Drosophila S26 (DS31) [3]. - Plant cytoplasmic S26.
- Fungi S26 [4].

These proteins have 114 to 127 amino acids.
A conserved octapeptide in the central part of these proteins has been selected as a signature pattern.

- Consensus pattern: [YH]-C-V-S-C-A-I-H

[1] Kuwano Y., Nakanishi O., Nabeshima Y., Tanaka T., Ogata K. J. Biochem. 97:983-992(1985).
[2] Zinov'eva R.D., Tomarev S.I. Dokl. Akad. Nauk SSSR 304:464-469(1989).
[3] Itoh N., Ohta K., Ohta M., Kawasaki T., Yamashina I. Nucleic Acids Res. 17:2121-2121 (1989).
[4] Wu M., Tan H. Gene 150:401-402(1994).

555. Ribosomal protein S28e signature
A number of eukaryotic and archaebacterial ribosomal proteins can be grouped on the basis of sequence similarities. One of these families consists of:

- Mammalian S28 [1]. - Plant S28 [2]. - Fungi S33 [3].
- Methanococcus jannaschii MJ1202.

These proteins have from 64 to 78 amino acids.
A highly conserved nonapeptide from the C-terminal extremity of these proteins has been selected as a signature pattern.

- Consensus pattern: E-[ST]-E-R-E-A-R-x-L

[1] Chan Y.-L., Olvera J., Wool I.G. Biochem. Biophys. Res. Commun. 179:314-318(1991).
[2] Hwang I., Goodman H.M. Plant Physiol. 102:1357-1358(1993).
[3] Hoekstra R., Ferreira P.M., Bootsman T.C., Mager W.H., Planta R.J. Yeast 8:949-959(1992).

556. Ribosomal protein S3Ae signature
A number of eukaryotic and archaebacterial ribosomal proteins can be grouped on the basis of sequence similarities. One of these families consists of:

- Mammalian S3A (was originally known as v-fos transformation effector protein). - Caenorhabditis elegans S3A (F56F3.5).
- Plant cytoplasmic S3A (CYC07) [1]. - Yeast Rp10 (PLC1 and PLC2).
- Fission yeast Rp10 (SpAC13G6.02c). - Methanococcus jannaschii MJ0980. These proteins have from 220 to 250 amino acids.

A conserved stretch in their N-terminal section was selected as a signature pattern.

- Consensus pattern: [LIV]-x-[GH]-R-[IV]-x-E-x-[SC]-L-x-D-L

[1] Liu J.H., Reid D.M. Plant Physiol. 109:338-338(1995).

557. Ribosomal protein S3 signature
Ribosomal protein S3 is one of the proteins from the small ribosomal subunit.
In Escherichia coli, S3 is known to be involved in the binding of initiator Met-tRNA. It belongs to a family of ribosomal proteins which, on the basis of sequence similarities [1], groups: - Eubacterial S3.

- Algal and plant chloroplast S3. - Cyanelle S3. - Archaebacterial S3.
- Plant mitochondrial S3. - Vertebrate S3. - Insect S3.
- Caenorhabditis elegans S3 (C23G10.3). - Yeast S3 (Rp13).

S3 is a protein of 209 to 559 amino-acid residues.
A conserved region located in the C-terminal section has been selected as a signature pattern.

- Consensus pattern: [GSTA]-[KR]-x(6)-G-x-[LIVMT]-x(2)-[NQSCH]-x(1,3)-[LIVFCA]-x(3)-[LIV]-[DENQ]-x(7)-[LMT]-x(2)-G-x(2)-G

[1] Otaka E., Hashimoto T., Mizuta K. Protein Seq. Data Anal. 5:285-300(1993).

558. Ribosomal protein S4 signature
Ribosomal protein S4 is one of the proteins from the small ribosomal subunit.
In Escherichia coli, S4 is known to bind directly to 16S ribosomal RNA. Mutations in S4 have been shown to increase translational error frequencies.
It belongs to a family of ribosomal proteins which, on the basis of sequence similarities [1,2], groups: - Eubacterial S4. - Algal and plant chloroplast S4.

- Cyanelle S4. - Archaebacterial S4. - Mammalian S9. - Yeast YS11 (SUP45).
- Marchantia polymorpha mitochondrial S4. - Dictyostelium discoideum rp1024.
- Yeast protein NAM9 [3]. NAM9 has been characterized as a suppressor for ochre mutations in mitochondrial DNA. It could be a ribosomal protein that acts as a suppressor by decreasing translation accuracy.

S4 is a protein of 171 to 205 amino-acid residues (except for NAM9 which is much larger). The signature pattern for this protein is based on a conserved region located in the central section of these proteins.

- Consensus pattern: [LIVM]-[DE]-x-R-[LI]-x(3)-[LIVMC]-[VMFYHQ]-[KRT]-x(3)-[STAGCVF]-x-[ST]-x(3)-[SAI]-[KR]-x-[LIVMF](2)

[1] Mizuta K., Hashimoto T., Suzuki K.I., Otaka E. Nucleic Acids Res. 19:2603-2608(1991).
[2] Otaka E., Hashimoto T., Mizuta K. Protein Seq. Data Anal. 5:285-300(1993).
[3] Boguta M., Dmochowska A., Borsuk P., Wrobel K., Gargouri A., Lazowska J., Slonimski P., Szczesniak B., Kruszewska A. Mol. Cell. Biol. 12:402-412(1992).

559. Ribosomal protein S4e signature
A number of eukaryotic and archaebacterial ribosomal proteins can be grouped on the basis of sequence similarities. One of these families consists of:

- Mammalian S4 [1]. Two highly similar isoforms of this protein exist: one coded by a gene on chromosome Y, and the other on chromosome X.
- Plant cytoplasmic S4 [2] - Yeast S7 (YS6). - Archebacterial S4e.

These proteins have 233 to 264 amino acids.
A highly conserved stretch of 15 residues in their N-terminal section has been selected as a signature pattern. Four positions in this region are positively charged residues.

- Consensus pattern: H-x-K-R-[LIVMF]-[SANK]-x-P-x(2)-[WY]-x-[LIVM]-x-[KRP]

[1] Fisher E.M., Beer-Romero P., Brown L.G., Ridley A., McNeil J.A., Lawrence J.B., Willard H.F., Bieber F.R., Page D.C. Cell 63:1205-1218(1990).
[2] Braun H.P., Emmermann M., Mentzel H., Schmitz U.K. Biochim. Biophys. Acta 1218:435-438(1994).

560. Ribosomal protein S5 signature
Ribosomal protein S5 is one of the proteins from the small ribosomal subunit.
In Escherichia coli, S5 is known to be important in the assembly and function of the 30S ribosomal subunit. Mutations in S5 have been shown to increase translational error frequencies. It belongs to a family of ribosomal proteins which, on the basis of sequence similarities [1,2], groups: - Eubacterial S5.

- Cyanelle S5. - Red algal chloroplast S5. - Archaebacterial S5.
- Mammalian S2 (LLrep3). - Caenorhabditis elegans S2 (C49H3.11).
- Drosophila S2. - Plant S2. - Yeast S4 (SUP44). - Fungi mitochondrial S5.

S5 is a protein of 166 to 254 amino-acid residues. The signature pattern for this protein is based on a conserved region, rich in glycine residues, and located in the N-terminal section of these proteins.

- Consensus pattern: G-[KRQ]-x(3)-[FY]-x-[ACV]-x(2)-[LIVMA]-[LIVM]-[AG]-[DN]-x(2)-G-x-[LIVM]-G-x-[SAG]-x(5,6)-[DEQ]-[LIVMA]-x(2)-A-[LIVMF]

[1] All-Robyn J.A., Brown N., Otaka E., Liebman S.W. Mol. Cell. Biol. 10:6544-6553(1990).
[2] Otaka E., Hashimoto T., Mizuta K. Protein Seq. Data Anal. 5:285-300(1993).

561. Ribosomal protein S6 signature
Ribosomal protein S6 is one of the proteins from the small ribosomal subunit.
In Escherichia coli, S6 is known to bind together with S18 to 16S ribosomal RNA. It belongs to a family of ribosomal proteins which, on the basis of sequence similarities, groups: - Eubacterial S6. - Red algal chloroplast S6.

- Cyanelle S6.

S6 is a protein of 95 to 208 amino-acid residues. The signature pattern for this protein is based on a conserved region located in the N-terminal section of these proteins.

- Consensus pattern: G-x-[KRC]-[DENQRH]-L-[SA]-Y-x-I-[KRNSA]

562. Ribosomal protein S6e signature
A number of eukaryotic and archaebacterial ribosomal proteins can be grouped on the basis of sequence similarities. One of these families consists of:

- Mammalian S6 [1]. - Drosophila S6 [2]. - Plant S6 [3]. - Yeast S10 (YS4).
- Halobacterium marismortui HS13 [4]. - Methanococcus j annaschii MJ1260.

S6 is the major substrate of protein kinases in eukaryotic ribosomes [5]; it may have an important role in controlling cell growth and proliferation through the selective translation of particular classes of mRNA. These proteins have 135 to 249 amino acids.
A conserved stretch of 12 residues in the N-terminal part of these proteins has been selected as a signature pattern.

- Consensus pattern: [LIVM]-[STAMR]-G-G-x-D-x(2)-G-x-P-M

[1] Franco R., Rosenfeld M.G. J. Biol. Chem. 265:4321-4325(1990).
[2]
Watson K.L., Konrad K.D., Woods D.F., Bryant P.J. Proc. Natl. Acad. Sci. U.S.A. 89:11302-11306(1992).
[3] Hansen G., Estruch J.J., Spena A. Nucleic Acids Res. 20:5230-5230(1992).
[4] Kimura M., Arndt E., Hatakeyama T., Hatakeyama T., Kimura J. Can. J. Microbiol. 35:195-199(1989).
[5] Bandi H.R., Ferrari S., Krieg J., Meyer H.E., Thomas G. J. Biol. Chem. 268:4530-4533(1993).

563. Ribosomal protein S7 signature
Ribosomal protein S7 is one of the proteins from the small ribosomal subunit.
In Escherichia coli, S7 is known to bind directly to part of the 3'end of 16S ribosomal RNA. It belongs to a family of ribosomal proteins which, on the basis of sequence similarities [1,2,3], groups: - Eubacterial S7.

- Algal and plant chloroplast S7. - Cyanelle S7. - Archaebacterial S7.
- Plant mitochondrial S7. - Mammalian S5. - Plant S5.
- Caenorhabditis elegans S5 (T05E11.1).

The best conserved region located in the N-terminal section of these proteins has been selected as a signature pattern.

- Consensus pattern: [DENSK]-x-[LIVMDET]-x(3)-[LIVMFTA](2)-x(6)-G-K-[KR]-x(5)-[LIVMF]-[LIVMFC]-x(2)-[STAC]

[1] Klussmann S., Franke P., Bergmann U., Kostka S., Wittmann-Liebold B. Biol. Chem. Hoppe-Seyler 374: 305-312(1993).
[2] Otaka E., Hashimoto T., Mizuta K. Protein Seq. Data Anal. 5:285-300(1993).
[3] Ignatovich O., Cooper M., Kulesza H.M., Beggs J.D. Nucleic Acids Res. 23:4616-4619(1995).

564. Ribosomal protein S7e signature
A number of eukaryotic ribosomal proteins can be grouped on the basis of sequence similarities [1]. One of these families consists of:

- Mammalian S7.
- Xenopus S8.
- Insect S7.
- Yeast probable ribosomal protein S7 (N2212).
- Fission yeast probable ribosomal protein S7 (SpAC18G6.13c).

These proteins have about 200 amino acids. A highly conserved stretch of 14 residues which is located in the central section and which is rich in charged residues was selected as a signature pattern.
Consensus pattern: [KR]-L-x-R-E-L-E-K-K-F-[SAP]-x-[KR]-H

[1] Salazar C.E., Mills-Hamm D.M., Kumar V., Collins F.H. Nucleic Acids Res. 21:4147-4147(1993).

565. Ribosomal protein S8 signature
Ribosomal protein S8 is one of the proteins from the small ribosomal subunit.
In Escherichia coli, S8 is known to bind directly to 16S ribosomal RNA. It belongs to a family of ribosomal proteins which, on the basis of sequence similarities [1], groups: - Eubacterial S8. - Algal and plant chloroplast S8.

- Cyanelle S8. - Archaebacterial S8. - Marchantia polymorpha mitochondrial S8.
- Mammalian S15A. - Plant S15A. - Yeast S22 (S24).

The best conserved region located in the C-terminal section of these proteins has been selected as a signature pattern.

- Consensus pattern: [GE]-x(2)-[LIV](2)-[STY]-[ST]-x(2)-G-[LIVM](2)-x(4)-[AG]-[KRHAYI]

[1] Otaka E., Hashimoto T., Mizuta K. Protein Seq. Data Anal. 5:285-300(1993).

566. Ribosomal protein S8e signature
A number of eukaryotic and archaebacterial ribosomal proteins can be grouped on the basis of sequence similarities [1]. One of these families consists of:

- Mammalian S8. - Caenorhabditis elegans S8 (F42C5.8). - Leishmania major S8.
- Plant S8. - Yeast S8 (S14) (Rp19). - Archebacterial S8e.

These proteins have either about 220 amino acids (in eukaryotes) or about 125 amino acids (in archebacteria). A conserved stretch which is located in the N-terminal section and which is rich in positively charged residues has been selected as a signature pattern.

- Consensus pattern: [KR]-x(2)-[ST]-G-[GA]-x(5)-[HR]-[KG]-[KR]-x-K-x-E-[LM]-G

[1] Engemann S., Herfurth E., Briesemeister U., Wittmann-Liebold B. J. Protein Chem. 14:189-195(1995).

567. Ribosomal protein S9 signature
Ribosomal protein S9 is one of the proteins from the small ribosomal subunit.
It belongs to a family of ribosomal proteins which, on the basis of sequence similarities [1,2], groups: - Eubacterial S9. - Algal chloroplast S9.

- Cyanelle S9. - Archaebacterial S9. - Mammalian S16. - Plant S16.
- Yeast mitochondrial ribosomal S9.

A conserved region containing many charged residues and located in the central section of these proteins has been selected as a signature pattern.

- Consensus pattern: G-G-G-x(2)-[GSA]-Q-x(2)-[SA]-x(3)-[GSA]-x-[GSTAV]-[KR]-[GSAL]-[LIF]

[1] Chan Y.-L., Paz V., Olvera J., Wool I.G. FEBS Lett. 263:85-88(1990).
[2] Otaka E., Hashimoto T., Mizuta K. Protein Seq. Data Anal. 5:285-300(1993).

568. Ribulose-phosphate 3-epimerase family signatures
Ribulose-phosphate 3-epimerase (EC 5.1.3.1) (also known as pentose-5-phosphate 3-epimerase or PPE) is the enzyme that converts D-ribulose 5-phosphate into D-xylulose 5-phosphate in Calvin's reductive pentose phosphate cycle. In Alcaligenes eutrophus two copies of the gene coding for PPE are known [1], one is chromosomally encoded (cbbEC), the other one is on a plasmid (cbbeP). PPE has been found in a wide range of bacteria, archebacteria, fungi and plants. The sequence of PPE is highly related to:

- Escherichia coli D-allulose-6-phosphate 3-epimerase (gene alsE).
- Escherichia coli protein sgcE.
- Mycoplasma genitalium hypothetical protein MG112.

All these proteins have from 209 to 241 amino acid residues.
Two conserved regions which are located respectively in the N-terminal and in the central part of these proteins have been selected as signature patterns.

- Consensus pattern: [LIVMF]-H-[LIVMFY]-D-[LIVM]-x-D-x(1,2)-[FY]-[LIVM]-x-N-x-[STAV]
- Consensus pattern: [LIVMA]-x-[LIVM]-M-[ST]-[VS]-x-P-x(3)-G-Q-x-F-x(6)-[NK]-[LIVMC]

[1] Kusian B., Yoo J.G., Bednarski R., Bowien B. J. Bacteriol. 174:7337-7344(1992).

569. (Ricin B lectin) Similarity to lectin domain of ricin beta-chain, 3 copies.
This family consists of a triplicated domain involved in cell agglutination in ricin.

570. (Rotamase) PpiC-type peptidyl-prolyl cis-trans isomerase signature Peptidyl-prolyl cis-trans isomerase (EC 5.2.1.8) (PPIase or rotamase) is an enzyme that accelerates protein folding by catalyzing the cis-trans isomerization of proline imidic peptide bonds in oligopeptides [1]. Most characterized PPiases belong to two families, the cyclophilin-type (see <PDOC00154>) and the the FKBP-type (see <PDOC00426>). Recently a third family has been discovered [2,3]. So far, the only biochemically characterized member of this family is the Escherichia coli protein parvulin (gene ppiC), a small (92 residues) cytoplasmic enzyme that prefers amino acid residues with hydrophobic side chains like leucine and phenylalanine in the P1 position of the peptides substrates. PpiC is evolutionary related to a number of proteins that are also probably PPiases:

- Escherichia coli and Haemophilus influenzae ppiD. PpiD is a PPIase which contains a periplasmic ppiC-like domain anchored to the inner membrane and which seems to be involved in the folding of outer membrane proteins.
- Escherichia coli surA. SurA is a periplasmic protein that contains two ppiC-like domains.
- Nitrogen-assimilating bacteria protein nifM which is involved in the activation and stabilization of the iron-component (nifH) of nitrogenase.
- Bacillus subtilis protein prsA, a membrane-bound lipoprotein involved in protein export.
- Lactococcus and lactobacillus protease maturation protein prtM, a membrane-bound lipoprotein involved in the maturation of a secreted serine proteinase. - Yeast protein ESS1/PTF1 (processing/termination factor 1).
- Drosophila protein dodo (gene dod). - Mammalian protein PIN1,
- Campylobacter j ejuni cell binding factor 2 (CBF2), a secreted antigen.
- Bacillus subtilis hypothetical protein yacD.
- Helicobacter pylori hypothetical protein HP0175.
- A hypothetical slime mold protein.

A conserved region that contains a serine which could play a role in the catalytic mechanism of these enzymes has been selected as a signature pattern.

- Consensus pattern: F-[GSADEI]-x-[LVAQ]-A-x(3)-[ST]-x(3,4)-[STQ]-x(3,5)-[GER]-G-x-[LIVM]-[GS]

[1] Fischer G., Schmid F.X. Biochemistry 29:2205-2212(1990).
[2] Rudd K.E., Sofia H.J., Koonin E.V., Plunkett G. III, Lazar S., Rouviere P.E. Trends Biochem. Sci. 20:14-15 (1995).
[3] Rahfeld J.-U., Ruecknagel K.P., Schelbert B., Ludwig B., Hacker J., Mann K., Fischer G. FEBS Lett. 352: 180-184(1994).

571. (RmaAD) Ribosomal RNA adenine dimethylases signature
A number of enzymes responsible for the dimethylation of adenosines if ribosomal RNAs (EC 2.1.1.48) have been found [1,2] to be evolutionary related. These enzymes are:

- Bacterial 16S rRNA dimethylase (gene ksgA), which acts in the biogenesis of ribosomes by catalyzing the dimethylation of two adjacent adenosines in the loop of a conserved hairpin near the 3'-end of 16S rRNA. Inactivation of ksgA leads to resistance to the aminoglycoside antibiotic kasugamycin.
- Yeast 18S rRNA dimethylase (gene DIM1), which is functionally similar to ksgA and that dimethylates twin adenosines in the 3'-end of 18S rRNA.
- Bacterial 'erm'methylases. These enzymes confer resistance to macrolide-lincosamide-streptogramin B (MLS) antibiotics - such as erythromycin - by dimethylating the adenine residue at position 2058 of 23S rRNA thus resulting in a reduced affinity between ribosomes and the MLS antibiotics.
- Caenorhabditis elegans hypothetical protein EO2H1.1.

The best conserved regions in these enzymes is located in the N-terminal section and corresponds to a region that is probably involved in S-adenosyl methionine (SAM) binding.

- Consensus pattern: [LIVM]-[LIVMFY]-[DE]-x-G-[STAPV]-G-x-[GA]-x-[LIVMF]-[ST]-x(2)-[LIVM]-x(6)-[LIVMY]-x-[STAGV]-[LIVMFYHC]-E-x-D

[1] van Gemen B., van Knippenberg P.H. (In) Nucleic acid methylation, Clawson G.A., Willis D.B., Weissbach A., Jones P.A., Eds., pp. 19-36, Alan R. Liss Inc, New-York, (1990).
[2] Lafontaine D., Delcour J., Glasser A.L., Desgres J., Vandenhaute J. J. Mol. Biol. 241:492-497(1994).

572. (RuBisC0 small) Ribulose bisphosphate carboxylase, small chain. 206 members

573. ATP/GTP-binding site motif A (P-loop) (ras)
From sequence comparisons and crystallographic data analysis it has been shown [1,2,3,4,5,6] that an appreciable proportion of proteins that bind ATP or GTP share a number of more or less conserved sequence motifs. The best conserved of these motifs is a glycine-rich region, which typically forms a flexible loop between a beta-strand and an alpha-helix. This loop interacts with one of the phosphate groups of the nucleotide. This sequence motif is generally referred to as the 'A' consensus sequence [1] or the 'P-loop' [5]. There are numerous ATP- or GTP-binding proteins in which the P-loop is found. A number of protein families for which the relevance of the presence of such a motif has been noted are listed below: - ATP synthase alpha and beta subunits. - Myosin heavy chains. - Kinesin heavy chains and kinesin-like proteins. - Dynamins and dynamin-like proteins - Guanylate kinase - Thymidine kinase (- Thymidylate kinase. - Shikimate kinase. - Nitrogenase iron protein family (nifH/frxC) - ATP-binding proteins involved in 'active transport' (ABC transporters) [7] - DNA and RNA helicases [8,9,10]. - GTP-binding elongation factors (EF-Tu, EF-1alpha, EF-G, EF-2, etc.). - Ras family of GTP-binding proteins (Ras, Rho, Rab, Ral, Ypt1, SEC4, etc.). - Nuclear protein ran. - ADP-ribosylation factors family - Bacterial dnaA protein - Bacterial recA protein - Bacterial recF protein - Guanine nucleotide-binding proteins alpha subunits (Gi, Gs, Gt, G0, etc.). - DNA mismatch repair proteins mutS family - Bacterial type II secretion system protein E. Not all ATP- or GTP-binding proteins are picked-up by this motif. A number of proteins escape detection because the structure of their ATP-binding site is completely different from that of the P-loop. Examples of such proteins are the E1-E2 ATPases or the glycolytic kinases. In other ATP- or GTP-binding proteins the flexible loop exists in a slightly different form; this is the case for tubulins or protein kinases. A special mention must be reserved foradenylate kinase, in which there is a single deviation from the P-loop pattern: in the last position Gly is found instead of Ser or Thr.
Consensus pattern: [AG]-x(4)-G-K-[ST]

In addition to the proteins listed above, the 'A' motif is also found in a number of other proteins. Most of these proteins probably bind a nucleotide, but others are definitively not ATP- or GTP-binding (as for example chymotrypsin, or human ferritin light chain).

[1] Walker J.E., Saraste M., RunswickM.J., GayN.J. EMBO J. 1:945-951(1982).
[2] Moller W., Amons R. FEBS Lett. 186:1-7(1985).
[3] Fry D.C., Kuby S.A., Mildvan A.S. Proc. Natl. Acad. Sci. U.S.A. 83:907-911(1986).
[4] Dever T.E., Glynias M.J., Merrick W.C. Proc. Natl. Acad. Sci. U.S.A. 84:1814-1818(1987).
[5] Saraste M., Sibbald P.R., Wittinghofer A. Trends Biochem. Sci. 15:430-434(1990).
[6] Koonin E.V. J. Mol. Biol. 229:1165-1174(1993).
[7] Higgins C.F., Hyde S.C., Mimmack M.M., Gileadi U., Gill D.R., Gallagher M.P. J. Bioenerg. Biomembr. 22: 571-592(1990).
[8] Hodgman T.C. Nature 333:22-23(1988) and Nature 333:578-578(1988) (Errata).
[9] Linder P., Lasko P., Ashburner M., Leroy P., Nielsen P.J., Nishi K., Schnier J., Slonimski P.P. Nature 337: 121-122(1989).
[10] Gorbalenya A.E., Koonin E.V., Donchenko A.P., Blinov V.M. Nucleic Acids Res. 17:4713-4730(1989).

GTP-binding nuclear protein ran signature (ras)
Ran (or TC4) is a small abundant nuclear protein that binds and hydrolyzes GTP and which has been implicated in a large number of processes including nucleocytoplasmic transport, RNA synthesis, processing and export and cell cycle checkpoint control [1,2]. Ran is generally included in the RAS 'superfamily' of small GTP-binding proteins [3], but it is only slightly related to the other RAS proteins. It also differs from RAS proteins in that it lacks cysteine residues at its C- terminal and is therefore not subject to prenylation. Instead ran has an acidic C-terminus. It is, however similar to RAS family members in requiring a specific guanine nucleotide exchange factor (GEF) and a specific GTPase activating protein (GAP) as stimulators of overall GTPase activity. The region of the GTP-binding B motif which, in ran, is perfectly conserved has been selected as a signature pattern.
Consensus pattern: D-T-A-G-Q-E-K-[LF]-G-G-L-R-[DE]-G-Y-Y- Proteins belonging to this family also contain a copy of the ATP/GTP- binding motif 'A' (P-loop).

[1] Scheffzek K., Klebe C., Fritz-Wolf K., Kabsch W., Wittinghofer A. Nature 374:378-381(1995)
[2] Rush M.G., Drivas G., d'Eustachio P. BioEssays 18:103-112(1996).
[3] Valencia A., Chardin P., Wittinghofer A., Sander C. Biochemistry 30:4637-4648(1991).

574. recA signature
The bacterial recA protein [1,2,3,E1] is essential for homologous recombination and recombinational repair of DNA damage. RecA has many activities: it filaments, it binds to single- and double-stranded DNA, itbinds and hydrolyzes ATP, it is also a recombinase and, finally, it interacts with lexA causing its activation and leading to its autocatalytic cleavage. RecA is a protein of about 350 amino-acid residues. Its sequence is very well conserved [3,4,S,E1] among eubacterial species. It is also found in the chloroplast of plants [6]. The best conserved region, a nonapeptide located in the middle of the sequence which is part of the monomer-monomer interface in a recA filament has been selected as a signature pattern,.
Consensus pattern: A-L-[KR]-[IF]-[FY]-[STA]-[STAD]-[LIVMQ]-R-

[1] Smith K.C., Wang T.-C. V. BioEssays 10:12-16(1989).
[2] Lloyd A.T., Sharp P.M. J. Mol. Evol. 37:399-407(1993).
[3] Roca A.I., Cox M.M. Prog. Nucleic Acids Res. Mol. Biol. 56:129-223(1997).
[4] Karlin S., Weinstock G.M., Brendel V. J. Bacteriol. 177:6881-6893(1995).
[5] Eisen J.A. J. Mol. Evol. 41:1105-1123(1995).
[6] Cerutti H.D., Osman M., Grandoni P., Jagendorf A.T. Proc. Natl. Acad. Sci. U.S.A. 89:8068-8072(1992).[E1] http://www.tisr.ors/~jeisen/RecA/RecA.htm

575. Response regulator receiver domain
This domain receives the signal from the sensor partner inComment: bacterial two-component systems. It is usually found N-terminalComment: to a DNA binding effector domain.

[1] Pao GM, Saier MH; J Mol Evol 1995;40:136-154.

576. Ribonucleotide reductase large subunit signature

* Ribonucleotide reductase (EC 1.17.4.1) [1,2] catalyzes the reductive synthesis of deoxyribonucleotides from their corresponding ribonucleotides. It provides the precursors necessary for DNA synthesis. Ribonucleotide reductase is an oligomeric enzyme composed of a large subunit (700 to 1000 residues) and a small subunit (300 to 400 residues). There are regions of similarities in the sequence of the large chain from prokaryotes, eukaryotes and viruses. One of these regions has been selected as a signature pattern.
Consensus pattern: W-x(2)-[LF]-x(6,7)-G-[LIVM]-[FYRA]-[NH]-x(3)-[STAQLIVM]-[ASC]-x(2)-[PA]-

[1] Nillson O., Lundqvist T., Hahne S., Sjoberg B.-M. Biochem. Soc. Trans. 16:91-94(1988).
[2] Reichard P. Science 260:1773-1777(1993).

577. Ribonuclease T2 family histidine active sites
The fungal ribonucleases T2 from Aspergillus oryzae, M from Aspergillus saitoiand Rh from Rhizopeus niveus are structurally and functionally related 30 Kdglycoproteins [1] that cleave the 3'-5' internucleotide linkage of RNA via a nucleotide 2',3'-cyclic phosphate intermediates (EC 3.1.27.1 ).A number of other RNAses have been found to be evolutionary related to these fungal enzymes: - Self-incompatibility [2] in flowering plants is often controlled by a single gene (S-gene) that has several alleles. This gene prevents fertilization by self-pollen or by pollen bearing either of the two S- alleles expressed in the style. The self-incompatibility glycoprotein from several higher plants of the solanaceae family has been shown [2,3] to be a ribonuclease. - Phosphate-starvation induced RNAses LE and LX from tomato [4]. These two enzymes are probably involved in a phosphate-starvation rescue system. - Escherichia coli periplasmic RNAse I (EC 3.1.27.6) (gene rna) [5]. - Aeromonas hydrophila periplasmic RNAse. - Haemophilus influenzae hypothetical protein HI0526.Two histidines residues have been shown [6,7] to be involved in the catalytic mechanism of RNase T2 and Rh. These residues and the region around them arehighly conserved in all the sequence described above. Two signature patterns have been developed, one for each of the two active-site histidines. The second pattern also contains a cysteine which is known to be involved in a disulfide bond.
Consensus pattern: [FYWL]-x-[LIVM]-H-G-L-W-P [H is an active site residue]
Consensus pattern: [LIVMF]-x(2)-[HDGTY]-[EQ]-[FYW]-x-[KR]-H-G-x-C [H is an active site residue] [C is involved in a disulfide bond]

[1] Watanabe H., Naitoh A., Suyama Y., Inokuchi N., Shimada H., Koyama T., Ohgi K., Irie M. J. Biochem. 108: 303-310(1990).
[2] Haring V., Gray J.E., McClure B.A., Anderson M.A., Clarke A.E. Science 250:937-941 (1990).
[3] McClure B.A., Haring V., Ebert P.R., Anderson M.A., Simpson R.J., Sakiyama F., Clarke A.E. Nature 342: 95957(1989).
[4] Loeffler A., Glund K., Irie M. Eur. J. Biochem. 214:627-633(1993).
[5] Meador J. III, Kennell D. Gene 95:1-7(1990).
[6] Kawata Y., Sakiyama F., Hayashi F., Kyogoku Y. Eur. J. Biochem. 187:255-262(1990).
[7] Kurihara H., Mitsui Y., Ohgi K., Irie M., Mizuno H., Nakamura K.T. FEBS Lett. 306:189-192(1992).

578. Ribonucleotide reductase large subunit signature. Ribonucleotide reductase (EC 1.17.4.1) [1,2] catalyzes the reductive synthesis of deoxyribonucleotides from their corresponding ribonucleotides. It provides the precursors necessary for DNA synthesis. Ribonucleotide reductase is an oligomeric enzyme composed of a large subunit (700 to 1000 residues) and a small subunit (300 to 400 residues). There are regions of similarities in the sequence of the large chain from prokaryotes, eukaryotes and viruses. One of these regions has been developed as a signature pattern.
Consensus pattern: W-x(2)-[LF]-x(6,7)-G-[LIVM]-[FYRA]-[NH]-x(3)-[STAQLIVM]-[ASC]-x(2)-[PA]-

[1] Nillson O., Lundqvist T., Hahne S., Sjoberg B.-M. Biochem. Soc. Trans. 16:91-94(1988).
[2] Reichard P. Science 260:1773-1777(1993).

579. RNase H
RNase H digests the RNA strand of an RNA/DNA hybrid. Important enzyme in retroviral replication cycle, and often found as a domain associated with reverse transcriptases. Structure is a mixed alpha+beta fold with three a/b/a layers.

580. Eukaryotic putative RNA-binding region RNP-1 signature (rrm)
Many eukaryotic proteins that are known or supposed to bind single-strandedRNA contain one or more copies of a putative RNA-binding domain of about 90amino acids [1,2]. This region has been found in the following proteins: ** Heterogeneous nuclear ribonucleoproteins ** - hnRNP A1 (helix destabilizing protein) (twice). - hnRNP A2/B1 (twice). - hnRNP C (C1/C2) (once). - hnRNP E (UP2) (at least once). - hnRNP G (once). ** Small nuclear ribonucleoproteins

** - U1 snRNP 70 Kd (once). - U1 snRNP A (once). - U2 snRNP B" (once). ** Pre-RNA and mRNA associated proteins ** - Protein synthesis initiation factor 4B (eIF-4B) [3], a protein essential for the binding of mRNA to ribosomes (once). - Nucleolin (4 times). - Yeast single-stranded nucleic acid-binding protein (gene SSB1) (once). - Yeast protein NSR1 (twice). NSR1 is involved in pre-rRNA processing; it specifically binds nuclear localization sequences. - Poly (A) binding protein (PABP) (4 times). ** Others ** - Drosophila sex determination protein Sex-lethal (Sxl) (twice). - Drosophila sex determination protein Transformer-2 (Tra-2) (once). - Drosophila 'elav' protein (3 times), which is probably involved in the RNA metabolism of neurons. - Human paraneoplastic encephalomyelitis antigen HuD (3 times) [4], which is highly similar to elav and which may play a role in neuron-specific RNA processing. - Drosophila 'bicoid' protein (once) [5], a segment-polarity homeobox protein that may also bind to specific mRNAs. - La antigen (once), a protein which may play a role in the transcription of RNA polymerase III. - The 60 Kd Ro protein (once), a putative RNP complex protein. - A maize protein induced by abscisic acid in response to water stress, which seems to be a RNA-binding protein. - Three tobacco proteins, located in the chloroplast [6], which may be involved in splicing and/or processing of chloroplast RNAs (twice). - X16 [7], a mammalian protein which may be involved in RNA processing in relation with cellular proliferation and/or maturation. - Insulin-induced growth response protein Cl-4 from rat (twice). - Nucleolysins TIA-1 and TIAR (3 times) [8] which possesses nucleolytic activity against cytotoxic lymphocyte target cells. may be involved in apoptosis. - Yeast RNA15 protein, which plays a role in mRNA stability and/or poly-(A) tail length [9].Inside the putative RNA-binding domain there are two regions which are highly conserved. The first one is a hydrophobic segment of six residues (which is called the RNP-2 motif), the second one is an octapeptide motif (which is called RNP-1 or RNP-CS). The position of both motifs in the domain is shown in the following schematic representation:

xxxxxxx######xxxxxxxxxxxxxxxxxxxxxxxxxxxxxx########xxxxxxxxxxxxxxxxxxxxxxxxx

x RNP-2 RNP-1

The RNP-1 motif has been used as a signature pattern for this type of domain.

Consensus pattern: [RK]-G-{EDRKHPCG}-[AGSCI]-[FY]-[LIVA]-x-[FYLM] In most cases the residue in position 3 of the pattern is either Tyr or Phe.


[1] Bandziulis R.J., Swanson M.S., Dreyfuss G. Genes Dev. 3:431-437(1989).

[2] Dreyfuss G., Swanson M.S., Pinol-Roma S. Trends Biochem. Sci. 13:86-91(1988).

[3] Milburn S.C., Hershey J.W.B., Davies M.V., Kelleher K., Kaufman R.J. EMBO J. 9:2783-2790(1990).

[4] Szabo A., Dalmau J., Manley G., Rosenfeld M., Wong E., Henson J., Posner J.B., Furneaux H.M. Cell 67: 325-333(1991).

[5] Rebagliati M. Cell 58:231-232(1989).

[6] Li Y., Sugiura M. EMBO J. 9:3059-3066(1990).

[7] Ayane M., Preuss U., Koehler G., Nielsen P.J. Nucleic Acids Res. 19:1273-1278(1991).

[8] Kawakami A., Tian Q., Duan X., Streuli M., Schlossman S.F., Anderson P. Proc. Natl. Acad. Sci. U.S.A. 89: 8681-8685(1992).

[9] Minvielle-Sebastia L., Winsor B., Bonneaud N., Lacroute F. Mol. Cell. Biol. 11:3075-3087(1991).


581. Rubredoxin signature

Rubredoxins [1] are small electron-transfer prokaryotic proteins. They contain an iron atom which is ligated by four cysteine residues. Rubredoxins are, in some cases, functionally interchangeable with ferredoxins.

A conserved region that includes two of the cysteine residues that bind the iron atom has been selected as a pattern for these proteins.

Consensus pattern: [LIVM]-x(3)-W-x-C-P-x-C-[AGD] [The two C's bind the iron atom]

In Pseudomonas oleovorans rubredoxin 2 (gene alkG) [2], this pattern is found twice because alkG has two rubredoxin domains.

Rubrerythrin [3], a protein with inorganic pyrophosphatase activity from Desulfovibrio vulgaris possesses a C-terminal rubredoxin-like domain, but this domain is too divergent to be detected by the above pattern.


[1] Berg J.M., Holm R.H.(In) Iron-sulfur proteins, Spiro T.G., Ed., pp1-66, Wiley, New-York, (1982).

[2] Kok M., Oldenhuis R., der Linden M.P.G., Meulenberg C.H.C., Kingma J., Witholt B., J. Biol. Chem. 264: 5442-5451(1989).

[3] van Beeumen J.J., van Driessche G., Liu M.-Y., Le Gall J., J. Biol. Chem. 266:20645-20653(1991).


582. (rvp) Eukaryotic and viral aspartyl proteases active site

Aspartyl proteases, also known as acid proteases, (EC 3.4.23.-) are a widely distributed family of proteolytic enzymes [1,2,3] known to exist invertebrates, fungi, plants, retroviruses and some plant viruses. Aspartate proteases of eukaryotes are monomeric enzymes which consist of two domains. Each domain contains an active site centered

on a catalytic aspartyl residue. The two domains most probably evolved from the duplication of an ancestral gene encoding a primordial domain. Currently known eukaryotic aspartyl proteases are: - Vertebrate gastric pepsins A and C (also known as gastricsin). - Vertebrate chymosin (rennin), involved in digestion and used for making cheese. - Vertebrate lysosomal cathepsins D (EC 3.4.23.5) and E (EC 3.4.23.34). - Mammalian renin (EC 3.4.23.15) whose function is to generate angiotensin I from angiotensinogen in the plasma. - Fungal proteases such as aspergillopepsin A (EC 3.4.23.18), candidapepsin (EC 3.4.23.24), mucoropepsin (EC 3.4.23.23) (mucor rennin), endothiapepsin (EC 3.4.23.22), polyporopepsin (EC 3.4.23.29), and rhizopuspepsin (EC 3.4.23.21). - Yeast saccharopepsin (EC 3.4.23.25) (proteinase A) (gene PEP4). PEP4 is implicated in posttranslational regulation of vacuolar hydrolases. - Yeast barrier pepsin (EC 3.4.23.35) (gene BAR1); a protease that cleaves alpha-factor and thus acts as an antagonist of the mating pheromone. - Fission yeast sxa1 which is involved in degrading or processing the mating pheromones. Most retroviruses and some plant viruses, such as badnaviruses, encode for anaspartyl protease which is an homodimer of a chain of about 95 to 125 amino acids. In most retroviruses, the protease is encoded as a segment of a polyprotein which is cleaved during the maturation process of the virus. It is generally part of the pol polyprotein and, more rarely, of the gagpolyprotein. Conservation of the sequence around the two aspartates of eukaryotic aspartyl proteases and around the single active site of the viral proteases allows us to develop a single signature pattern for both groups of protease.
Consensus pattern: [LIVMFGAC]-[LIVMTADN]-[LIVFSA]-D-[ST]-G-[STAV]-[STAPDENQ]- x-[LIVMFSTNC]-x-[LIVMFGTA] [D is the active site residue] -

[1] Foltmann B. Essays Biochem. 17:52-84(1981).
[2] Davies D.R. Annu. Rev. Biophys. Chem. 19:189-215(1990).
[3] Rao J.K.M., Erickson J.W., Wlodawer A. Biochemistry 30:4663-4671(1991).
[4] Rawlings N.D., BarrettA.J. Meth. Enzymol. 248:105-120(1995).

583. (rvt) Reverse transcriptase (RNA-dependent DNA polymerase)
A reverse transcriptase gene is usually indicative of a mobile element such as a retrotransposon or retrovirus. Reverse transcriptases occur in a variety of mobile elements, including retrotransposons, retroviruses, group II introns, bacterial msDNAs, hepadnaviruses, and caulimoviruses. Number of members: 1233

[1] Medline: 91006031. Origin and evolution of retroelements based upon their reverse transcriptase sequences. Xiong Y, Eickbush TH; EMBO J 1990;9:3353-3362.

584. (S-AdoMet synt) S-adenosylmethionine synthetase signatures
S-adenosylmethionine synthetase (EC 2.5.1.6) is the enzyme that catalyzes the formation of S-adenosylmethionine (AdoMet) from methionine and ATP [1]. AdoMet is an important methyl donor for transmethylation and is also the propylamino donor in polyamine biosynthesis. In bacteria there is a single isoform of AdoMet synthetase (gene metK), there are two in budding yeast (genes SAM1 and SAM2) and in mammals while in plants there is generally a multigene family.The sequence of AdoMet synthetase is highly conserved throughout isozymes and species. Two signature patterns have been selected for this type of enzyme; the first is a hexapeptide which seems to be involved in ATP-binding; the second is an almost perfectly conserved glycine-rich nonapeptide.
Consensus pattern: G-A-G-D-Q-G-x(3)-G-[FYH]-Sequences known to belong to this class detected by the pattern:
Consensus pattern: G-[GA]-G-[ASC]-F-S-x-K-[DE]

[1] Horikawa S., Sasuga J., Shimizu K., Ozasa H., Tsukada K. J. Biol. Chem. 265:13683-13686(1990).

585. S1 RNA binding domain
The S 1 domain occurs in a wide range of RNAComment: associated proteins. It is structurally similarComment: to cold shock protein which binds nucleic acids.Comment:
The S1 domain has an OB-fold structure.

[1] Bycroft M, Hubbard TJ, Proctor M, Freund SM, Murzin AG; Cell 1997;88:235-242.

586. SAICAR synthetase signatures
Phosphoribosylaminoimidazole-succinocarboxamide synthase (EC 6.3.2.6) (SAICARsynthetase) catalyzes the seventh step in the de novo purine biosynthetic pathway; the ATP-dependent conversion of 5'-phosphoribosyl-5-aminoimidazole-4-carboxylic acid and aspartic acid to SAICAR [1]. In bacteria (gene purC),fungi (gene ADE1) and plants, SAICAR synthetase is a monofunctional protein;in higher vertebrates it is the N-terminal domain of a bifunctional enzyme that also catalyze phosphoribosylaminoimidazole carboxylase (AIRC) activity. Two conserved regions

in the central section of this enzyme have been selected as signature patterns for SAICAR synthetase.
Consensus pattern: [LIVMF](2)-P-[LIVM]-E-x-[LIVM]-[LIVMCA]-R-x(3)-[TA]-G-S-
Consensus pattern: [LIVM]-[LIVMA]-D-x-K-[LIVMFY]-E-F-G

[1] Zalkin H., Dixon J.E. Prog. Nucleic Acid Res. Mol. Biol. 42:259-287(1992).

587. (SCP) Extracellular proteins SCP/Tpx-1/Ag5/PR-1/Sc7 signatures
A variety of extracellular proteins from eukaryotes have been found to be evolutionary related: - Rodent sperm-coating glycoprotein (SCP), also known as acidic epididymal glycoprotein (AEG) . This protein is thought to be involved in sperm maturation [1]. It is a protein of about 220 residues and probably contains eight disulfide bonds. - Mammalian testis-specific protein Tpx-1 [2]. Tpx-1 is highly related to SCP's. - Mammalian glioma pathogenesis-related protein (GliPR). - Lizard helothermine, a toxin that blocks ryanodine receptors. - Venom allergen 5 (Ag5) from vespid wasps and venom allergen 3 (Ag3) from fire ants. These proteins are potent allergens and are the main cause of allergic reactions to stings from insects of the hymenoptera family [3]. Ag5/3 are proteins of about 200 residues and contain four disulfide bonds. - Plant pathogenesis proteins of the PR-1 family [4]. These proteins are synthesized during pathogen infection or other stress-related responses. They are proteins of about 130 to 140 residues and probably contain three disulfide bonds. - Proteins Sc7 and Sc14 from the basidomycete fungus Schizophyllum commune. These extracellular proteins are loosely associated with fruit body hyphal walls [5]. Sc7/14 are proteins of about 180 residues and probably contain two disulfide bonds. - Ancylostoma secreted protein from dog hookworm. - Yeast hypothetical proteins YJL078c, YJL079c and YKR013w.The exact function of these proteins is not yet known. Two conserved regions located in their C-terminal half have been selected as signature patterns. The second signature contains a cysteine which is known to be involved in a disulfide bond in Ag5.
Consensus pattern: [GDER]-H-[FYWH]-T-Q-[LIVM](2)-W-x(2)-[STN]
Consensus pattern: [LIVMFYH]-[LIVMFY]-x-C-[NQRHS]-Y-x-[PARH]-x-[GL]-N-[LIVMFYWDN] [C is involved in a disulfide bond]

[1] Mizuki N., Kasahara M. Mol. Cell. Endocrinol. 89:25-32(1992).
[2] Kasahara M., Gutknecht J., Brew K., Spurr N., Goodfellow P.N. Genomics 5:527-534(1989).
[3] Lu G., Villalba M., Coscia M.R., Hoffman D.R., King T.P. J. Immunol. 150:2823-2830(1993).
[4] Dixon D.C., Cutt J.R., Klessig D.F. EMBO J. 10:1317-1324(1991).
[5] Schuren F.H.J., Asgeirsdottir S.A., Kothe E.M., Scheer J.M.J., Wessels J.G.H. J. Gen. Microbiol. 139: 2083-2090(1993).

588. SET domain
SET domains appear to be protein-protein interactionComment: domains. It has been demonstrated that SET domainsComment: mediate interactions with a family of proteins thatComment: display similarity with dual-specificity phosphatasesComment: (dsPTPases) [2].

[1] Tripoulas N, LaJeunesse D, Gildea J, Shearn A; Genetics 1996;143:913-928.
[2] Cui X, De Vivo I, Slany R, Miyamoto A, Firestein R, Cleary, ML; Nat Genet 1998;18:331-337.

589. Src homology 3 (SH3) domain profile
The Src homology 3 (SH3) domain is a small protein domain of about 60 amino-acid residues first identified as a conserved sequence in the non-catalytic part of several cytoplasmic protein tyrosine kinases (e.g. Src, Abl, Lck) [1].Since then, it has been found in a great variety of other intracellular or membrane-associated proteins [2,3,4,5].The SH3 domain has a characteristic fold which consists of five or six beta-strands arranged as two tightly packed anti-parallel beta sheets. The linker regions may contain short helices [6].The function of the SH3 domain is not well understood. The current opinion is that they mediate assembly of specific protein complexes via binding to proline-rich peptides [7].In general SH3 domains are found as single copies in a given protein, but there is a significant number of protein with two SH3 domains and a few with 3 or 4 copies. So far, SH3 domains have been identified in the following proteins: - Many vertebrate, invertebrate and retroviral cytoplasmic (non-receptor) protein tyrosine kinases. In particular in the Src, Abl, Bkt, Csk and ZAP70 families of kinases. - Mammalian phosphatidylinositol-specific phospholipase C-gamma-1 and -2. - Mammalian phosphatidyl inositol 3-kinase regulatory p85 subunit. - Mammalian Ras GTPase-activating protein (GAP). - Adaptor proteins mediating binding of guanine nucleotide exchange factors to growth factor receptors: vertebrate GRB2, Caenorhabditis elegans sem-5 and Drosophila DRK. All of which have two SH3 domains. - Mammalian Vav oncoprotein, a guanine nucleotide exchange factor of the CDC24 family. - Some guanine-nucleotide releasing factors of the CDC25 family: yeast CDC25, yeast SCD25, fission yeast ste6. - MAGUK proteins. These proteins consist of at least three types of domains: one or more copies

of the DHR domain, a SH3 domain and a C-terminal guanylate kinase domain. Members of this family are: Drosophila lethal(1)discs large-1 tumor suppressor protein (gene Dlg1), mammalian tight junction protein ZO-1, vertebrate erythrocyte membrane protein p55, Caenorhabditis elegans protein lin-2, rat protein CASK and mammalian synaptic proteins SAP90/PSD-95, CHAPSYN-110/PSD-93, SAP97/DLG1 and SAP102. - Miscellanous proteins interacting with vertebrate receptor protein tyrosine kinases: mammalian cytoplasmic protein Nck (3 copies), oncoprotein Crk (2 copies). - Chicken Src substrate p80/85 protein (cortactin) and the similar human hemopoietic lineage cell specific protein Hs1. - Mammalian dihydrouridine-sensitive L-type calcium channel beta (regulatory) subunit including the related human myasthenic syndrome antigen B (MSYB). - Mammalian neutrophil cytosolic activators of NADPH oxidase: p47 (NCF-1), p67 (NCF-2), and a potential homolog from Caenorhabditis elegans (B0303.7). NCF-1 and -2 have two copies of the SH3 domain, while B0303.7 has four. - Some myosin heavy chains from amoebae, slime molds and yeast (gene MYO3). - Vertebrate and Drosophila spectrin and fodrin alpha-chain. - Human amphiphysin. - Yeast actin-binding protein ABP1. - Yeast actin-binding protein SLA1 (3 copies). - Yeast protein BEM1 and the fission yeast homolog scd2 (or ral3) (2 copies). - Yeast BEM1-binding proteins BOI2 (BEB1) and BOB1 (BOI1). - Yeast fusion protein FUS1. - Yeast protein RSV167. - Yeast protein SSU81. - Yeast hypothetical proteins YAR014c (1 copy), YFR024c (1 copy), YHL002w (1 copy), YHR016c (1 copy), YJL020C (1 copy), YHR114w (2 copies) and the fission yeast homolog SpAC12C2.05c. - Caenorhabditis elegans hypothetical proteins F42H10.3. The profile developed to detect SH3 domains is based on a structural alignment consisting of 5 gap-free blocks and 4 linker regions totaling 62 match positions.

[1] Mayer B.J., Hamaguchi M., Hanafusa H. Nature 332:272-275(1988).
[2] Musacchio A., Gibson T., Lehto V.P., Saraste M. FEBS Lett. 307:55-61(1992).
[3] Pawson T., Schlessinger J. Curr. Biol. 3:434-442(1993).
[4] Mayer B.J., Baltimore D. Trends Cell Biol. 3:8-13(1993).
[5] Pawson T. Nature 373:573-580(1995).
[6] Kuriyan J., Cowburn D. Curr. Opin. Struct. Biol. 3:828-837(1993).
[7] Morton C.J., Campbell I.D. Curr. Biol. 4:615-617(1994).

590. Serine hydroxymethyltransferase pyridoxal-phosphate attachment site (SHMT)
Serine hydroxymethyltransferase (EC 2.1.2.1) (SHMT) [1] catalyzes the transfer of the hydroxymethyl group of serine to tetrahydrofolate to form 5,10-methylenetetrahydrofolate and glycine. In vertebrates, it exists in acytoplasmic and a mitochondrial form whereas only one form is found in prokaryotes. Serine hydroxymethyltransferase is a pyridoxal-phosphate containing enzyme. The pyridoxal-P group is attached to a lysine residue around which the sequence is highly conserved in all forms of the enzyme.
Consensus pattern: [DEH]-[LIVMFY]-x-[STMV]-[GST]-[ST](2)-H-K-[ST]-[LF]-x-G-[PAC]-[RQ]-[GSA]-[GA] [K is the pyridoxal-P attachment site]

[1] Usha R., Savithri H.S., Rao N.A. Biochim. Biophys. Acta 1204:75-83(1994).

591. SIS domain
SIS (Sugar ISomerase) domains are found in many phosphosugar isomerases and phosphosugar binding proteins.

[1] Teplyakov A, Obmolova G, Badet-Denisot MA, Badet B, Polikarpov I; Structure 1998;6:1047-1055.

592. (SKI) Shikimate kinase signature
Shikimate kinase (EC 2.7.1.71) catalyzes the fifth step in the biosynthesis from chorismate of the aromatic amino acids (the shikimate pathway) inbacteria (gene aroK or aroL), plants and in fungi (where it is part of a multifunctional enzyme which catalyzes five consecutive steps in this pathway).Shikimate kinase is a small protein of about 200 residues. A conserved region that contains a run of three glycines has been selected as a signature pattern.
Consensus pattern: [KR]-x(2)-E-x(3)-[LIVMF]-x(8,12)-[LIVMF](2)-[SA]-x-G(3)- x-[LIVMF]. Proteins belonging to this family also contain a copy of the ATP/GTP- binding motif 'A' (P-loop).

593. SNAP-25 family
SNAP-25 (synaptosome-associated protein 25 kDa) proteins are components of SNARE complexes. Members of this family contain a cluster of cysteine residues that can be palmitoylated for membrane attachment [2].

[1]Brennwald P, Kearns B, Champion K, Keranen S, Bankaitis V, Novick P; Cell 1994;79:245-258.
[2] Risinger C, Blomqvist AG, Lundell I, Lambertsson A, Nassel D, Pieribone VA, Brodin L, Larhammar D; J Biol Chem 1993;268:24408-24414.

594. SNF2 and others N-terminal domain
This domain is found in proteins involved in a variety of processes including transcription regulation (e.g., SNF2, STH1, brahma, MOT1), DNA repair (e.g., ERCC6, RAD16, RAD5), DNA recombination (e.g., RAD54), and chromatin unwinding (e.g., ISWI) as well as a variety of other proteins with little functional information (e.g., lodestar, ETL1).

595. Staphylococcal nuclease homologues (Snase)
Present in all three domains of cellular life. Four copies in the transcriptional coactivator p 100. These, however, appear to lack the active site residues of Staphylococcal nuclease. Positions 14 (Asp-21), 34 (Arg-35), 39 (Asp-40), 42 (Glu-43) andComment: 110 (Arg-87) [SNase numbering in parentheses] are thought to be involved in substrate-binding and catalysis.

[1] Ponting CP; Protein Sci 1997;6:459-463.
[2] Callebaut I, Mornon JP; Biochem J 1997;321:125-132.

596. SPRY domainA
SPRY Domain is named from SPla and the RYanodine Receptor. Domain of unknown function. Distant homologues are domains in Comment: butyrophilin/marenostrin/pyrin homologues.

[1] Ponting C, Schultz J, Bork P; Trends Biochem Sci 1997;22:193-194.

597. (SQS PSY) Squalene and phytoene synthases signatures
Two different polyisoprene synthases have been shown [1,2,3] to share a number of regions of sequence similarities: - Squalene synthase (EC 2.5.1.21) (farnesyl-diphosphate farnesyltransferase) (SQS), which catalyzes the conversion of two molecules of farnesyl diphosphate (FPP) into squalene. It is the first committed step in the cholesterol biosynthetic pathway. The reaction carried out by SQS is catalyzed in two separate steps: the first is a head-to-head condensation of the two molecules of FPP to form presqualene diphosphate; this intermediate is then rearranged in a NADP-dependent reduction, to form squalene. SQS is found in eukaryotes. In yeast it is encoded by the ERG9 gene, in mammals by the FDFT1 gene. SQS seems to be membrane-bound. - Phytoene synthase (EC 2.5.1.-) (PSY), which catalyzes the conversion of two molecules of geranylgeranyl diphosphate (GGPP) into phytoene. It is the second step in the biosynthesis of carotenoids from isopentenyl diphosphate. The reaction carried out by PSY is catalyzed in two separate steps: the first is a head-to-head condensation of the two molecules of GGPP to form prephytoene diphosphate; this intermediate is then rearranged to form phytoene. PSY is found in all organisms that synthesize carotenoids: plants and photosynthetic bacteria as well as some non-photosynthetic bacteria and fungi. In bacteria PSY is encoded by the gene crtB. In plants PSY is localized in the chloroplast. As it can be seen from the description above, both SQS and PSY share a number of functional similarities which are also reflected at the level of their primary structure. In particular three well conserved regions are shared bySQS and PSY; they could be involved in substrate binding and/or the catalytic mechanism. Signature patterns have been developed for the second and third conserved regions; they are localized in the central part of these enzymes.
Consensus pattern: Y-[CSAM]-x(2)-[VSG]-A-[GSA]-[LIVAT]-[IV]-G-x(2)-[LMSC]- x(2)-[LIV]
Consensus pattern: [LIVM]-G-x(3)-Q-x(2,3)-N-[IF]-x-R-D-[LIVMFY]-x(2)-[DE]- x(4,7)-R-x-[FY]-x-P-

[1] Summers C., Karst F., Charles A.D. Gene 136:185-192(1993).
[2] Robinson G.W., Tsay Y.H., Kienzle B.K., Smith-Monroy C.A., Bishop R.W. Mol. Cell. Biol. 13:2706-2727 (1993).
[3] Roemer S., Hugueney P., Bouvier F., Camara B., Kuntz M. Biochem. Biophys. Res. Commun. 196:1414-1421 (1993).

598. SRP54-type proteins GTP-binding domain signature
The signal recognition particle (SRP) is an oligomeric complex that mediates targeting and insertion of the signal sequence of exported proteins into the membrane of the endoplasmic reticulum. SRP consists of a 7S RNA and six protein subunits. One of these subunits, the 54 Kd protein (SRP54), is a GTP-binding protein that interacts with the signal sequence when it emerges from the ribosome. The N-terminal 300 residues of SRP54 include the GTP-binding site (G-domain) and are evolutionary related to similar domains in other proteins which are listed below [1]. - Escherichia coli and Bacillus subtilis ffh protein (P48), a protein which seems to be the prokaryotic counterpart of SRP54. Ffh is associated with a 4.5S RNA in the prokaryotic SRP complex. - Signal recognition particle receptor alpha subunit (docking protein), an integral membrane GTP-binding protein which ensures, in conjunction with SRP, the correct targeting of nascent secretory proteins to the endoplasmic reticulum membrane. The G-domain is located at the C-terminal extremity of the protein. - Bacterial ftsY protein, a protein which is believed to play a similar role

to that of the docking protein in eukaryotes. The G-domain is located at the C-terminal extremity of the protein. - The pilA protein from Neisseria gonorrhoeae which seems to be the homolog of ftsY. - A protein from the archaebacteria Sulfolobus solfataricus. This protein is also believed to be a docking protein. The G-domain is also at the C- terminus. - Bacterial flagellar biosynthesis protein flhF. The best conserved regions in those domains are the sequence motifs that are part of the GTP-binding site, but as those regions are not specific to these proteins, they were not used as a signature pattern. Instead, a conserved region located at the C-terminal end of the domain was selected.
Consensus pattern: P-[LIVM]-x-[FYL]-[LIVMAT]-[GS]-x-[GS]-[EQ]-x(4)-[LIVMF]

[1] Althoff S., Selinger D., Wise J.A. Nucleic Acids Res. 22:1933-1947(1994).

599. (STphosphatase) Serine/threonine specific protein phosphatases signature Serine/threonine specific protein phosphatases (EC 3.1.3.16) (PP) [1,2,3] are enzymes that catalyze the removal of a phosphate group attached to a serine or evolutionary related. - Protein phosphatase-1 (PP1) is an enzyme of broad specificity. It is inhibited by two thermostable proteins, inhibitor-1 and -2. In mammals, there are two closely related isoforms of PP-1: PP-1 alpha and PP-1beta, produced by alternative splicing of the same gene. In Emericella nidulans, PP-1 (gene bimG) plays an important role in mitosis control by reversing the action of the nimA kinase. In yeast, PP-1 (gene SIT4) is involved in dephosphorylating the large subunit of RNA polymerase II. - Protein phosphatase-2A (PP2A) is also an enzyme of broad specificity. PP2A is a trimeric enzyme that consist of a core composed of a catalytic subunit associated with a 65 Kd regulatory subunit and a third variable subunit. In mammals, there are two closely related isoforms of the catalytic subunit of PP2A: PP2A-alpha and PP2A-beta, encoded by separate genes. - Protein phosphatase-2B (PP2B or calcineurin), a calcium-dependent enzyme whose activity is stimulated by calmodulin. It is composed of two subunits: the catalytic A-subunit and the calcium-binding B-subunit. The specificity of PP2B is restricted.In addition to the above-mentioned enzymes, some additional serine/threoninespecific protein phosphatases have been characterized and are listed below. - Mammalian phosphatase-X (PP-X), and Drosophila phosphatase-V (PP-V) which are closely related but yet distinct from PP2A. - Yeast phosphatase PPH3, which is similar to PP2A, but with different enzymatic properties. - Drosophila phosphatase-Y (PP-Y), and yeast phosphatases Z1 and Z2 (genes PPZ1 and PPZ2) which are closely related but yet distinct from PP 1. - Drosophila retinal degeneration protein C (gene rdgC), a calcium-binding phosphatase required to prevent light-induced retinal degeneration. - Phages Lambda and Phi-80 ORF-221 which have been shown to have phosphatase activity and are related to mammalian PP's. The best conserved regions in these proteins is a perfectly conserved pentapeptide that can be used as a signature pattern. Consensus pattern: [LIVM]-R-G-N-H-E-

[1] Cohen P. Annu. Rev. Biochem. 58:453-508(1989).
[2] Cohen P., Cohen P.T.W. J. Biol. Chem. 264:21435-21438(1989).
[3] Cohen P.T.W., Brewis N.D., Hughes V., Mann D.J. FEBS Lett. 268:355-359(1990).

600. Translation initiation factor SUI1 signature
In budding yeast (Saccharomyces cerevisiae), SUI1 is a translation initiation factor that functions in concert with eIF-2 and the initiator tRNA-Met in directing the ribosome to the proper start site of translation [1]. SUI1 is a protein of 108 residues. Close homologs of SUI1 have been found [2] in mammals, insects and plants. SUI1 is also evolutionary related to hypothetical proteins from Escherichia coli (yciH), Haemophilus influenzae (HI1225) and Methanococcus vannielii. A conserved region in the C-terminal section has been selected as a signature pattern.
Consensus pattern: [LIVM]-[EQ]-[LIVM]-Q-G-[DEN]-[KHQ]-[KRV]

[1] Yoon H., Donahue T.F. Mol. Cell. Biol. 12:248-260(1992).
[2] Fields C.A., Adams M.D. Biochem. Biophys. Res. Commun. 198:288-291(1994).

601. (S T dehydratase) Serine/threonine dehydratases pyridoxal-phosphate attachment site Serine and threonine dehydratases [1,2] are functionally and structurally related pyridoxal-phosphate dependent enzymes: - L-serine dehydratase (EC 4.2.1.13) and D-serine dehydratase (EC 4.2.1.14) catalyze the dehydratation of L-serine (respectively D-serine) into ammonia and pyruvate. - Threonine dehydratase (EC 4.2.1.16) (TDH) catalyzes the dehydratation of threonine into alpha-ketobutarate and ammonia. In Escherichia coli and other microorganisms, two classes of TDH are known to exist. One is involved in the biosynthesis of isoleucine, the other in hydroxamino acid catabolism.Threonine synthase (EC 4.2.99.2) is also a pyridoxal-phosphate enzyme, it catalyzes the transformation of homoserine-phosphate into threonine. It has been shown [3] that threonine synthase is distantly related to the serine/threonine dehydratases. In all these enzymes, the pyridoxal-phosphate group is attached to a lysine residue. The sequence around this residue is sufficiently conserved to allow the derivation of a pattern specific to serine/threonine

dehydratases and threonine synthases.
Consensus pattern: [DESH]-x(4,5)-[STVG]-x-[AS]-[FYI]-K-[DLIFSA]-[RVMF]-[GA]-[LIVMGA] [The K is the pyridoxal-P attachment site]

[1] Ogawa H., Gomi T., Konishi K., Date T., Naakashima H., Nose K., Matsuda Y., Peraino C., Pitot H.C., Fujioka M. J. Biol. Chem. 264:15818-15823(1989).
[2] Datta P., Goss T.J., Omnaas J.R., Patil R.V. Proc. Natl. Acad. Sci. U.S.A. 84:393-397(1987).
[3] Parsot C. EMBO J. 5:3013-3019(1986).
[4] Grabowski R., Hofmeister A.E.M., Buckel W. Trends Biochem. Sci. 18:297-300(1993).

Cysteine synthase/cystathionine beta-synthase P-phosphate attachment site
Cysteine synthase (CSase) is the pyridoxal-phosphate dependent enzyme responsible [1] for the formation of cysteine from O-acetyl-serine and hydrogen sulfide with the concomitant release of acetic acid. In bacteria suchas Escherichia coli, two forms of the enzyme are known (genes cysK and cysM).In plants there are also two forms, one located in the cytoplasm and the otherin chloroplasts. Cystathionine beta-synthase [2] catalyzes the first irreversiblestep in homocysteine transulfuration; the conjugation of homocysteine andserine forming cystathionine. Like Csase it is a pyridoxal-phosphate dependent enzyme. The two types of enzymes are evolutionary related. The pyridoxal-phosphategroup of CSases has been shown to be attached to a lysine residue which is located in the N-terminal section of these enzymes; the sequence around this residue is highly conserved and can be used as a signature pattern to detect this class of enzymes.
Consensus pattern: K-x-E-x(3)-[PA]-[STAGC]-x-S-[IVAP]-K-x-R-x-[STAG]-x(2)-[LIVM] [The 2nd K is the pyridoxal-P attachment site

[1] Saito K., Kurosawa M., Murakoshi I. FEBS Lett. 328:111-114(1993).
[2] Swaroop M., Bradley K., Ohura T., Tahara T., Roper M.D., Rosenberg L.E., Kraus J.P. J. Biol. Chem. 267: 11455-11461(1992).

602. S locus glycop
S-locus glycoprotein family. In Brassicaceae, self-incompatible plants have a self/non-self Comment: recognition system. This is sporophytically controlled by Comment: multiple alleles at a single locus (S). S-locus glycoproteins, Comment: as well as S-receptor kinases, are in linkage with the S-alleles [1].Number of members: 128

[1] Evolutionary aspects of the S-related genes of the Brassica self-incompatibility system: synonymous and nonsynonymous base substitutions. Hinata K, Watanabe M, Yamakawa S, Satta Y, Isogai A; Genetics 1995; 140:1099-1104.
[2] Polymorphism of the S-locus glycoprotein gene (SLG) and the S-locus related gene (SLR1) in Raphanus sativus L. and self-incompatible ornamental plants in the Brassicaceae. Sakamoto K, Kusaba M, Nishio T; Mol Gen Genet 1998;258:397-403.

603. (sdh cyt) Succinate dehydrogenase cytochrome b subunit signatures
Succinate dehydrogenase (SDH) is a membrane-bound complex of two main components: a membrane-extrinsic component composed of an FAD-binding flavoprotein and an iron-sulfur protein, and a hydrophobic component composed of a cytochrome B and a membrane anchor protein. The cytochrome b component is a mono heme transmembrane protein [1,2,3] belonging to a family that groups: - Cytochrome b-556 from bacterial SDH (gene sdhC). - Cytochrome b560 from the mammalian mitochondrial SDH complex. - Cytochrome b560 subunit encoded in the mitochondrial genome of some algae and in the plant Marchantia polymorpha. - Cytochrome b from yeast mitochondrial SDH complex (gene SDH3 or CYB3). - Protein cyt-1 from Caenorhabditis.These cytochromes are proteins of about 130 residues that comprise threetransmembrane regions. There are two conserved histidines which may beinvolved in binding the heme group. Two signature patterns have been developed that include these histidine residues.
Consensus pattern: R-P-[LIVMT]-x(3)-[LIVM]-x(6)-[LIVMWPK]-x(4)-S-x(2)-H-R-x-[ST] [H could be a heme ligand]
Consensus pattern: H-x(3)-[GA]-[LIVMT]-R-[HF]-[LIVMF]-x-[FYWM]-D-x-[GVA] [H could be a heme ligand]

[1] Yu L., Wei Y.-Y., Usui S., Yu C.-A. J. Biol. Chem. 267:24508-24515(1992).
[2] Abraham P.R., Mulder A., Van't Riet J., Raue H.A. Mol. Gen. Genet. 242:708-716(1994).
[3] Leblanc C., Boyen C., Richard O., Bonnard G., Grienenberger J.M., Kloareg B. J. Mol. Biol. 250:484-495 (1995).

604. Sec1 family

[1] The Sec1 family: a novel family of proteins involved in synaptic transmission and general secretion. Halachmi N, Lev Z; J Neurochem 1996;66:889-897.
Number of members: 40

605. Protein secE/sec61-gamma signature
In bacteria, the secE protein plays a role in protein export; it is one of the components - with secY and secA - of the preprotein translocase. In eukaryotes, the evolutionary related protein sec61-gamma playsa role in protein translocation through the endoplasmic reticulum; it is part of a trimeric complex that also consist of sec61-alpha and beta [1]. Both secE and sec61-gamma are small proteins of about 60 to 90 amino acids that contain a single transmembrane region at their C-terminal extremity (Escherichia colisecE is an exception, in that it possess an extra N-terminal segment of 60residues that contains two additional transmembrane domains).The sequence of secE/sec61-gamma is not extremely well conserved, however it is possible to derive a signature pattern centered on a conserved proline located 10 residues before the beginning of the transmembrane domain.
Consensus pattern: [LIVMFY]-x(2)-[DENQGA]-x(4)-[LIVMFTA]-x-[KRV]-x(2)-[KW]-P-x(3)-[SEQ]-x(7)-[LIVT]-[LIVGA]-[LIVFGAST]

[1] Hartmann E., Sommer T., Prehn S., Goerlich D., Jentsch S., Rapoport T.A. Nature 367:654-657(1994).

606. 11-S plant seed storage proteins signature
Plant seed storage proteins, whose principal function appears to be the major nitrogen source for the developing plant, can be classified, on the basis of their structure, into different families. 11-S are non-glycosylated proteins which form hexameric structures [1,2]. Each of the subunits in the hexamer is itself composed of an acidic and a basic chain derived from a single precursor and linked by a disulfide bond. This structure is shown in the following representation. +-----+ || xxxxxxxxxxxCxxxxxxxxxxxxxxxxxxxxxxNGxCxxxxxxxxxxxxxxxxxxxxxxxx *********
<------Acidic-subunit-------------><-----Basic-subunit------> <-----------------About-480-to-
500-residues----------------->'C': conserved cysteine involved in a disulfide bond.'*': position of the pattern. Proteins that belong to the 11-S family are: pea and broad bean legumins, rape cruciferin, rice glutelins, cotton beta-globulins, soybean glycinins, pumpkin 11-S globulin, oat globulin, sunflower helianthinin G3, etc. The region that includes the conserved cleavage site between the acidic and basic subunits (Asn-Gly) and a proximal cysteine residue which is involved in the interchain disulfide bond have been used as a signature pattern for this family of proteins.
Consensus pattern: N-G-x-[DE](2)-x-[LIVMF]-C-[ST]-x(11,12)-[PAG]-D [C is involved in a disulfide bond

[1] Hayashi M., Mori H., Nishimura M., Akazawa T., Hara-Nishimura I. Eur. J. Biochem. 172:627-632(1988).
[2] Shotwell M.A., Afonso C., Davies E., Chesnut R.S., Larkins B.A. Plant Physiol. 87:698-704(1988).

607. 7S seed storage protein
7S globulin is one of the main storage proteins of most angiosperms and gymnosperms. The 7S storage proteins are homotrimers.
Number of members: 67

[1] The three-dimensional structure of canavalin from jack bean (Canavalia ensiformis). Ko TP, Ng JD, McPherson A; Plant Physiol 1993;101:729-744.

608. Aspartate-semialdehyde dehydrogenase signature
Aspartate-semialdehyde dehydrogenase (ASD) catalyzes the second step in the common biosynthetic pathway leading from Asp to diaminopimelate and Lys, to Met, and to Thr; the NADP-dependent reductive dephosphorylation of L-aspartyl phosphate to L-aspartate-semialdehyde. In bacteria and fungi, ASDis a protein of about 40 Kd (340 to 370 residues) whose sequence is not extremely well conserved [1]. A conserved cysteine residue has been implicated as important for the catalytic activity [2].The region of conservation around the active site residue is too small to be used as signature pattern. Another more conserved region, located in the last third of the sequence, and which contains both a conserved cysteine as well as an histidine has been used instead.
Consensus pattern: [LIVM]-[SADN]-x(2)-C-x-R-[LIVM]-x(4)-[GSC]-H-[STA

[1] Baril C., Richaud C., Fourni E., Baranton G., Saint Girons I. J. Gen. Microbiol. 138:47-53(1992).
[2] Karsten W.E., Viola R.E. Biochim. Biophys. Acta 1121:234-238(1992).

N-acetyl-gamma-glutamyl-phosphate reductase active site

N-acetyl-gamma-glutamyl-phosphate reductase (EC 1.2.1.38) (AGPR) [1,2] is the enzyme that catalyzes the third step in the biosynthesis of arginine from glutamate, the NADP-dependent reduction of N-acetyl-5-glutamyl phosphate into N-acetylglutamate 5-semialdehyde.In bacteria it is a monofunctional protein of 35 to 38 Kd (gene argC) while in fungi it is part of a bifunctional mitochondrial enzyme (gene ARG5,6, arg11 orarg-6) which contains a N-terminal acetylglutamate kinase (EC 2.7.2.8) domain and a C-terminal AGPR domain. In the Escherichia coli enzyme, a cysteine has been shown to be implicated in the catalytic activity, the region around this residue is well conserved and can be used as a signature pattern.

Consensus pattern: [LIVM]-[GSA]-x-P-G-C-[FY]-[AVP]-T-[GA]-x(3)-[GTAC]-[LIVM]-x-P [C is the active site residue]

[1] Ludovice M., Martin J.F., Carrachas P., Liras P. J. Bacteriol. 174:4606-4613(1992).

[2] Gessert S.F., Kim J.H., Nargang F.E., Weiss R.L. J. Biol. Chem. 269:8189-8203(1994).

609. Sialyltransferase family,

Number of members: 18

610. SpoU rRNA Methylase family

This family of proteins probably use S-AdoMet. Number of members: 58

[1] SpoU protein of Escherichia coli belongs to a new family of putative rRNA methylases. Koonin EV, Rudd KE; Nucleic Acids Res 1993;21:5519-5519.

[2] The spoU gene of escherichia coli , the fourth gene of the spoT operon, is essential for tRNA (Gm18) 2' methyltransferase activity. Persson BC, Jager G, Gustafsson C; Nucleic Acids Res 1997;25:4093-4097.

611. Stathmin family signatures

Stathmin [1] (from the Greek 'stathmos'which means relay), is an ubiquitous intracellular protein, present in a variety of phosphorylated forms and which serves as a relay for diverse second messenger pathways. Its expression and phosphorylation are regulated throughout development and in response to extracellular signals regulating cell proliferation, differentiation and function. Stathmin is a highly conserved protein of 149 amino acid residues. Structurally, it consists of an N-terminal domain of about 45 residues followed by a 78 residue alpha-helical domain consisting of a heptad repeat coiled coil structure and a C-terminal domain of 25 residues. Protein SCG10 is a neuron-specific, membrane-associated protein that accumulates in the growth cones of developing neurons. It is highly similar in its sequence to stathmin, but differs in that it contains an additional N-terminal hydrophobic segment of 32 residues which is probably responsible for its interaction with membranes. Xenopus protein XB3 is also evolutionary related to stathmin and also contains an additional N-terminal hydrophobic domain [2]. A conserved decapeptide which ends with the first three residues of the coiled coil domain and a second pattern that corresponds to part of the central region of the coiled coil have been selected as signatures for proteins of the stathmin family.

Consensus pattern: P-[KRQ]-[KR](2)-[DE]-x-S-L-[EG]-E-

Consensus pattern: A-E-K-R-E-H-E-[KR]-E-

[1] Sobel A. Trends Biochem. Sci. 16:301-305(1991).

[2] Maucuer A., Moreau J., Mechali M., Sobel A. J. Biol. Chem. 268:16420-16429(1993).

612. SUA5/yciO/yrdC family signature. The following uncharacterized proteins have been shown [1] to share regions of similarities: - Yeast protein SUA5. - Escherichia coli hypothetical protein yciO and HI1198, the corresponding Haemophilus influenzae protein. - Escherichia coli hypothetical protein yrdC and HI0656, the corresponding Haemophilus influenzae protein. - Bacillus subtilis hypothetical protein ywlC. - Mycobacterium leprae hypothetical protein in rfe-hemK intergenic region. - Methanococcus jannaschii hypothetical protein MJ0062.These are proteins of from 20 to 46 Kd which contain a number of conserved regions in their N-terminal section. They can be picked up in the database by the following pattern.

Consensus pattern: [LIVMTA](3)-[LIVMFYC]-[PG]-T-[DE]-[STA]-x-[FY]-[GA]-[LIVM]-[GS]-

[1] Bairoch A., Rudd K.E., Robison K. Unpublished observations (1995).

613. Sucrose synthase

Sucrose synthases catalyse the synthesis of sucrose from UDP-glucose and fructose. This family includes the bulk of the sucrose synthase protein. However the carboxyl terminal region of the sucrose synthases belongs to the glycosyl transferase family Glycos transf 1.

614. Sulfotransferase proteins
Number of members: 59

615. Synaptophysin / synaptoporin signature
Synaptophysin and synaptoporin [1] are structurally related proteins, found in the membrane of synaptic vesicles, which may function as ionic or solute channels. These two glycoproteins seem to span the membrane four times. Both their N- and C-termini sequences seem to be cytoplasmically located. As a signature pattern for this family of proteins, a highly conserved region located in the beginning of the first intravesicular loop just after the first transmembrane domain has been selected. This region contains a cysteine residue that may be involved in a disulfide bond. Consensus pattern: L-S-V-[DE]-C-x-N-K-T [C may be involved in a disulfide bond

[1] Knaus P., Marqueze-Pouey B., Scherer H., Betz H. Neuron 5:453-462(1990).

616. Syndecans signature
Syndecans [1,2] (from the greek syndein; to bind together) are a family of transmembrane heparan sulfate proteoglycans which are implicated in the binding of extracellular matrix components and growth factors. Syndecans bind a variety of molecules via their heparan sulfate chains and can act as receptors or as co-receptors. Structurally, these proteins consist of four separate domains: a) A signal sequence; b) An extracellular domain (ectodomain) of variable length and whose sequence is not evolutionary conserved in the various forms of syndecans. The ectodomain contains the sites of attachment of the heparan sulfate glycosaminoglycan side chains; c) A transmembrane region; d) A highly conserved cytoplasmic domain of about 30 to 35 residues which could interact with cytoskeletal proteins. The proteins known to belong to this family are: - Syndecan 1. - Syndecan 2 or fibroglycan. - Syndecan 3 or neuroglycan or N-syndecan. - Syndecan 4 or amphiglycan or ryudocan. - Drosophila syndecan. - Caenorhabditis elegans probable syndecan (F57C7.3).The signature pattern that has been developed for syndecans starts with the last residue of the transmembrane region and includes the first 10 residues of the cytoplasmic domain. This region, which contains four basic residues, could act as a stop transfer site. Consensus pattern: [FY]-R-[IM]-[KR]-K(2)-D-E-G-S-Y

[1] Bernfield M., Kokenyesi R., Kato M., Hinkes M.T., Spring J., Gallo R.L., Lose E.J. Annu. Rev. Cell Biol. 8: 365-393(1992).
[2] David G. FASEB J. 7:1023-1030(1993).

617. Syntaxin / epimorphin family signature
The following proteins have been shown to be evolutionary related [1,2,3]: - Epimorphin (or syntaxin 2), a mammalian mesenchymal protein which plays an essential role in epithelial morphogenesis. - Syntaxin 1A (also known as antigen HPC-1) and syntaxin 1B which are synaptic proteins which may be involved in docking of synaptic vesicles at presynaptic active zones. - Syntaxin 3. - Syntaxin 4, which is potentially involved in docking of synaptic vesicles at presynaptic active zones. - Syntaxin 5, which mediates endoplasmic reticulum to golgi transport. - Syntaxin 6, which is involved in intracellular vesicle trafficking. - Syntaxin 7. - Yeast PEP12 (or VPS6) which is required for the transport of proteases to the vacuole. - Yeast SED5 which is required for the fusion of transport vesicles with the Golgi complex. - Yeast SSO1 and SSO2 which are required for vesicle fusion with the plasma membrane. - Yeast VAM3, which is required for vacuolar assembly. - Arabidopsis thaliana protein KNOLLE which may be involved in cytokinesis. - Caenorhabditis elegans hypothetical proteins F35C8.4, F48F7.2, F55A11.2 and T01B11.3.The above proteins share the following characteristics: a size ranging from30 Kd to 40 Kd; a C-terminal extremity which is highly hydrophobic and isprobably involved in anchoring the protein to the membrane; a central, well conserved region, which seems to be in a coiled-coil conformation. The pattern specific for this family is based on the most conserved region of the coiled coil domain.
Consensus pattern: [RQ]-x(3)-[LIVMA]-x(2)-[LIVM]-[ESH]-x(2)-[LIVMT]-x-[DEVM]-[LIVM]-x(2)-[LIVM]-[FS]-x(2)-[LIVM]-x(3)-[LIVT]-x(2)-Q- [GADEQ]-x(2)-[LIVM]-[DNQT]-x-[LIVMF]-[DESV]-x(2)-[LIVM]

[1] Bennett M.K., Garcia-Arraras J.E., Elferink L.A., Peterson K., Fleming A.M., Hazuka C.D., Scheller R.H. Cell 74:863-873(1993).
[2] Spring J., Kato M., Bernfield M. Trends Biochem. Sci. 18:124-125(1993).
[3] Pelham H.R.B. Cell 73:425-426(1993).

618. Sm protein
The U1, U2, U4/U6, and U5 small nuclear ribonucleoprotein particles (snRNPs) involved in pre-mRNA splicing contain seven Sm proteins (B/B D1, D2, D3, E, F and G) in common, which assemble around the Sm site present

in four of the major spliceosomal small nuclear RNAs. These proteins contain a common sequence motif in two segments, Sm1 and Sm2, separated by a short variable linker.

[1] Hermann H, Fabrizio P, Raker VA, Foulaki K, Hornig H, Brahms H, Luhrmann R EMBO J 1995;14:2076-2088.
[2] Kambach C, Walke S, Young R, Avis JM, de la Fortelle E, Raker VA, Luhrmann R, Li J, Nagai K; Cell 1999; 96:375-387.

619. Skp1 family

[1] Stebbins CE, Kaelin WG Jr, Pavletich NP; Science 1999;284:455-461.

620. Protein secY signatures
The eubacterial secY protein [1] plays an important role in protein export. It interacts with the signal sequences of secretory proteins as well as with two other components of the protein translocation system: secA and secE. SecY is an integral plasma membrane protein of 419 to 492 amino acid residues that apparently contains ten transmembrane segments. Such a structure probablyconfers to secY a 'translocator' function, providing a channel for periplasmic and outer-membrane precursor proteins.Homologs of secY are found in archaebacteria [2]. SecY is also encoded in the chloroplast genome of some algae [3] where it could be involved in a prokaryotic-like protein export system across the two membranes of the chloroplast endoplasmic reticulum (CER) which is present in chromophyte andcryptophyte algae. Two signature patterns have been developed for secY proteins. The first corresponds to the second transmembrane region, which is the most conserved section of these proteins. The second spans the C-terminal part of the fourth transmembrane region, a short intracellular loop, and the N-terminal part of the fifth transmembrane region. Consensus pattern: [GST]-[LIVMF](2)-x-[LIVM]-G-[LIVM]-x-P-[LIVMFY](2)-x-[AS]-[GSTQ]-[LIVMFAT](3)-Q-[LIVMFA](2)
Consensus pattern: [LIVMFYW](2)-x-[DE]-x-[LIVMF]-[STN]-x(2)-G-[LIVMF]-[GST]-[NST]-G-x-[GST]-[LIVMF](3)

[1] Ito K. Mol. Microbiol. 6:2423-2428(1992).
[2] Auer J., Spicker G., Boeck A. Biochimie 73:683-688(1991).
[3] Douglas S.E. FEBS Lett. 298:93-96(1992).

621. (Seed protein) Small hydrophilic plant seed proteins signature. The following small hydrophilic plant seed proteins are structurally related: - Arabidopsis thaliana proteins GEA1 and GEA6. - Cotton late embryogenesis abundant (LEA) protein D-19. - Carrot EMB-1 protein. - Barley LEA proteins B 19.1 A, B19.1B, B 19.3 and B 19.4. - Maize late embryogenesis abundant protein Emb564. - Radish late seed maturation protein p8B6. - Rice embryonic abundant protein Emp 1. - Sunflower 10 Kd late embryogenesis abundant protein (DS10). - Wheat Em proteins. These proteins contains from 83 to 153 amino acid residues and may play a role[1,2] in equipping the seed for survival, maintaining a minimal level of hydration in the dry organism and preventing the denaturation of cytoplasmic components. They may also play a role during imbibition by controlling water uptake. As a signature pattern, the best conserved region in the sequence of these proteins has been developed, it is a glycine-rich nonapeptide located in the N-terminal section.-
Consensus pattern: G-[EQ]-T-V-V-P-G-G-T-

[1] Dure L. III, Crouch M., Harada J., Ho T.-H. D., Mundy J., Quatrano R., Thomas T., Sung Z.R. Plant Mol. Biol. 12:475-486(1989).
[2] Gaubier P., Raynal M., Hull G., Huestis G.M., Grellet F., Arenas C., Pages M., Delseny M. Mol. Gen. Genet. 238:409-418(1993).

622. Serine carboxypeptidases, active sites
All known carboxypeptidases are either metallo carboxypeptidases or serinecarboxypeptidases. The catalytic activity of the serine carboxypeptidases, like that of the trypsin family serine proteases, is provided by a charge relay system involving an aspartic acid residue hydrogen-bonded to a histidine, which is itself hydrogen-bonded to a serine [1]. Proteins known to be serine carboxypeptidases are: - Barley and wheat serine carboxypeptidases I, II, and III [2]. - Yeast carboxypeptidase Y (YSCY) (gene PRC1), a vacuolar protease involved in degrading small peptides. - Yeast KEX1 protease, involved in killer toxin and alpha-factor precursor processing. - Fission yeast sxa2, a probable carboxypeptidase involved in degrading or processing mating pheromones [3]. - Penicillium j anthinellum carboxypeptidase S1 [4]. - Aspergullus niger carboxypeptidase pepF. - Aspergullus satoi carboxypeptidase cpdS. - Vertebrate protective protein / cathepsin A [5], a lysosomal protein which is not only a carboxypeptidase but also essential for the activity of both beta-galactosidase and neuraminidase. - Mosquito vitellogenic carboxypeptidase (VCP) [6].

- Naegleria fowleri virulence-related protein Nf314 [7]. - Yeast hypothetical protein YBR139w. - Caenorhabditis elegans hypothetical proteins C08H9.1, F13D12.6, F32A5.3, F41C3.5 and K10B2.2.This family also includes: - Sorghum (s)-hydroxymandelonitrile lyase (hydroxynitrile lyase) (HNL) [8], an enzyme involved in plant cyanogenesis. The sequences surrounding the active site serine and histidine residues are highly conserved in all these serine carboxypeptidases.
Consensus pattern: [LIVM]-x-[GTA]-E-S-Y-[AG]-[GS] [S is the active site residue]
Consensus pattern: [LIVF]-x(2)-[LIVSTA]-x-[IVPST]-x-[GSDNQL]-[SAGV]-[SG]-H-x-[IVAQ]-P-x(3)-[PSA] [H is the active site residue]

[1] Liao D.I., Remington S.J. J. Biol. Chem. 265:6528-6531(1990).
[2] Sorensen S.B., Svendsen I., Breddam K. Carlsberg Res. Commun. 54:193-202(1989).
[3] Imai Y., Yamamoto M. Mol. Cell. Biol. 12:1827-1834(1992).
[4] Svendsen I., Hofmann T., Endrizzi J., Remington J., Breddam K. FEBS Lett. 333:39-43(1993).
[5] Galjart N.J., Morreau H., Willemsen R., Gillemans N., Bonten E.J., d'Azzo A. J. Biol. Chem. 266:14754-14762 (1991).
[6] Cho W.L., Deitsch K.W., Raikhel A.S. Proc. Natl. Acad. Sci. U.S.A. 88:10821-10824(1991).
[7] Hu W.N., Kopachik W., Band R.N. Infect. Immun. 60:2418-2424(1992).
[8] Wajant H., Mundry K.W., Pfitzenmaier K. Plant Mol. Biol. 26:735-746(1994).
[9] Rawlings N.D., Barrett A.J. Meth. Enzymol. 244:19-61(1994).[E1]

623. Serpins signature. Serpins (SERine Proteinase INhibitors) [1,2,3,4] are a group of structurally related proteins. They are high molecular weight (400 to 500 amino acids),extracellular, irreversible serine protease inhibitors with a well defined structural-functional characteristic: a reactive region that acts as a 'bait' for an appropriate serine protease. This region is found in the C-terminal part of these proteins. Proteins which are known to belong to the serpin family are listed below (references are only provided for recently determined sequences): - Alpha-1 protease inhibitor (alpha-1-antitrypsin, contrapsin). - Alpha-1-antichymotrypsin, - Antithrombin III. - Alpha-2-antiplasmin. - Heparin cofactor II. - Complement C1 inhibitor. - Plasminogen activator inhibitors 1 (PAI-1) and 2 (PAI-2). - Glia derived nexin (GDN) (Protease nexin I). - Protein C inhibitor. - Rat hepatocytes SPI-1, SPI-2 and SPI-3 inhibitors. - Human squamous cell carcinoma antigen (SCCA) which may act in the modulation of the host immune response against tumor cells. - A lepidopteran protease inhibitor. - Leukocyte elastase inhibitor which, in contrast to other serpins, is an intracellular protein. - Neuroserpin [5], a neuronal inhibitor of plasminogen activators and plasmin. - Cowpox virus crmA [6], an inhibitor of the thiol protease interleukin-1B converting enzyme (ICE). CrmA is the only serpin known to inhibit a non-serine proteinase. - Some orthopoxviruses probable protease inhibitors, which may be involved in the regulation of the blood clotting cascade and/or of the complement cascade in the mammalian host. On the basis of strong sequence similarities, a number of proteins with no known inhibitory activity are said to belong to this family: - Birds ovalbumin and the related genes X and Y proteins. - Angiotensinogen; the precursor of the angiotensin active peptide. - Barley protein Z; the major endosperm albumin. - Corticosteroid binding globulin (CBG). - Thyroxine-binding globulin (TBG). - Sheep uterine milk protein (UTMP) and pig uteroferrin-associated protein (UFAP). - Hsp47, an endoplasmic reticulum heat-shock protein that binds strongly to collagen and could act as a chaperone in the collagen biosynthetic pathway [7]. - Maspin, which seems to function as a tumor supressor [5]. - Pigment epithelium-derived factor precursor (PEDF), a protein with a strong neutrophic activity [8]. - Ep45, an estrogen-regulated protein from Xenopus [9]. A signature pattern has been developed for this family of proteins, centered on a well conserved Pro-Phe sequence which is found ten to fifteen residues on the C-terminal side of the reactive bond
Consensus pattern: [LIVMFY]-x-[LIVMFYAC]-[DNQ]-[RKHQS]-[PST]-F-[LIVMFY]-[LIVMFYC]-x-[LIVMFAH]-

[1] Carrell R., Travis J. Trends Biochem. Sci. 10:20-24(1985).
[2] Carrell R., Pemberton P.A., Boswell D.R. Cold Spring Harbor Symp. Quant. Biol. 52:527-535(1987).
[3] Huber R., Carrell R.W. Biochemistry 28:8951-8966(1989).
[4] Remold-O'Doneel E. FEBS Lett. 315:105-108(1993).
[5] Osterwalder T., Contartese J., Stoeckli E.T., Kuhn T.B., Sonderegger P. EMBO J. 15:2944-2953(1996).
[6] Komiyama T., Ray C.A., Pickup D.J., Howard A.D., Thornberry N.A., Peterson E.P., Salvesen G. J. Biol. Chem. 269:19331-19337(1994).
[7] Clarke E., Sandwal B.D. Biochim. Biophys. Acta 1129:246-248(1992).
[8] Zou Z., Anisowicz A., Neveu M., Rafidi K., Sheng S., Sager R., Hendrix M.J., Seftor E., Thor A. Science 263: 526-529(1994).
[9] Steele F.R., Chader G.J., Johnson L.V., Tombran-Tink J. Proc. Natl. Acad. Sci. U.S.A. 90:1526-1530(1993).
[10] Holland L.J., Suksang C., Wall A.A., Roberts L.R., Moser D.R., Bhattacharya A. J. Biol. Chem. 267:

7053-7059(1992).

624. Sigma-54 interaction domain signatures and profile

Some bacterial regulatory proteins activate the expression of genes from promoters recognized by core RNA polymerase associated with the alternative sigma-54 factor. These have a conserved domain of about 230 residues involved in the ATP-dependent [1,2] interaction with sigma-54. This domain has been found in the proteins listed below: - acoR from Alcaligenes eutrophus, an activator of the acetoin catabolism operon acoXABC. - algB from Pseudomonas aeruginosa, an activator of alginate biosynthetic gene algD. - dctD from Rhizobium, an activator of dctA, the C4-dicarboxylate transport protein. - dhaR from Citrobacter freundii, a regulator of the dha operon for glycerol utilization. - fhlA from Escherichia coli, an activator of the formate dehydrogenase H and hydrogenase III structural genes. - flbD from Caulobacter crescentus, an activator of flagellar genes. - hoxA from Alcaligenes eutrophus, an activator of the hydrogenase operon. - hrpS from Pseudomonas syringae, an activator of hprD as well as other hrp loci involved in plant pathogenicity. - hupR1 from Rhodobacter capsulatus, an activator of the [NiFe] hydrogenase genes hupSL. - hydG from Escherichia coli and Salmonella typhimurium, an activator of the hydrogenase activity. - levR from Bacillus subtilis, which regulates the expression of the levanase operon (levDEFG and sacC). - nifA (as well as anfA and vnfA) from various bacteria, an activator of the nif nitrogen-fixing operon. - ntrC, from various bacteria, an activator of nitrogen assimilatory genes such as that for glutamine synthetase (glnA) or of the nif operon. - pgtA from Salmonella typhimurium, the activator of the inducible phospho- glycerate transport system. - pilR from Pseudomonas aeruginosa, an activator of pilin gene transcription. - rocR from Bacillus subtilis, an activator of genes for arginine utilization - tyrR from Escherichia coli, involved in the transcriptional regulation of aromatic amino-acid biosynthesis and transport. - wtsA, from Erwinia stewartii, an activator of plant pathogenicity gene wtsB. - xylR from Pseudomonas putida, the activator of the tol plasmid xylene catabolism operon xylCAB and of xy1S. - Escherichia coli hypothetical protein yfhA. - Escherichia coli hypothetical protein yhgB. About half of these proteins (algB, dcdT, flbD, hoxA, hupR1, hydG, ntrC, pgtA and pilR) belong to signal transduction two-component systems [3] and possess a domain that can be phosphorylated by a sensor-kinase protein in their N- terminal section. Almost all of these proteins possess a helix-turn-helix DNA-binding domain in their C-terminal section. The domain which interacts with the sigma-54 factor has an ATPase activity. This may be required to promote a conformational change necessary for theinteraction [4]. The domain contains an atypical ATP-binding motif A (P-loop) as well as a form of motif B. The two ATP-binding motifs are located in the N-terminal section of the domain; signature patterns have been developed for both motifs. Other regions of the domain are also conserved. One of them, located in the C-terminal section, has been selected as a third signature pattern.

Consensus pattern: [LIVMFY](3)-x-G-[DEQ]-[STE]-G-[STAV]-G-K-x(2)-[LIVMFY]

Consensus pattern: [GS]-x-[LIVMF]-x(2)-A-[DNEQASH]-[GNEK]-G-[STIM]-[LIVMFY](3)-[DE]-[EK]-[LIVM]

Consensus pattern: [FYW]-P-[GS]-N-[LIVM]-R-[EQ]-L-x-[NHAT]

[1] Morrett E., Segovia L. J. Bacteriol. 175:6067-6074(1993).
[2] Austin S., Kundrot C., Dixon R. Nucleic Acids Res. 19:2281-2287(1991).
[3] Albright L.M., Huala E., Ausubel F.M. Annu. Rev. Genet. 23:311-336(1989).
[4] Austin S., Dixon R. EMBO J. 11:2219-2228(1992).

625. Sigma-70 factors family signatures

Sigma factors [1] are bacterial transcription initiation factors that promote the attachment of the core RNA polymerase to specific initiation sites and arethen released. They alter the specificity of promoter recognition. Most bacteria express a multiplicity of sigma factors. Two of these factors, sigma-70 (gene rpoD), generally known as the major or primary sigma factor, and sigma-54 (gene rpoN or ntrA) direct the transcription of a wide variety of genes. The other sigma factors, known as alternative sigma factors, are required for the transcription of specific subsets of genes. With regard to sequence similarity, sigma factors can be grouped into two classes: the sigma-54 and sigma-70 families. The sigma-70 family includes, in addition to the primary sigma factor, a wide variety of sigma factors, some of which are listed below: - Bacillus sigma factors involved in the control of sporulation-specific genes: sigma-E (sigE or spoIIGB), sigma-F (sigF or spoIIAC), sigma-G (sigG or spoIIIG), sigma-H (sigH or spo0C) and sigma-K (sigK or spoIVCB/spoIIIC). - Escherichia coli and related bacteria sigma-32 (gene rpoH or htpR) involved in the expression of heat shock genes. - Escherichia coli and related bacteria sigma-27 (gene fliA) involved in the expression of the flagellin gene. - Escherichia coli sigma-S (gene rpoS or katF) which seems to be involved in the expression of genes required for protection against external stresses. - Myxococcus xanthus sigma-B (sigB) which is essential for the late-stage differentiation of that bacteria. Alignments of the sigma-70 family permit the identification of four regions of high conservation [2,3]. Each of these four regions can in turn be subdivided into a number of sub-regions. Signature patterns based on the two best-conserved sub-regions have been developed. The first pattern corresponds to sub-region 2.2;the exact function of this sub-region is not known although it could be involved in the binding of

the sigma factor to the core RNA polymerase. The second pattern corresponds to sub-region 4.2 which seems to harbor a DNA-binding 'helix-turn-helix' motif involved in binding the conserved -35region of promoters recognized by the major sigma factors. The second pattern starts one residue before the N-terminal extremity of the HTH region and ends six residues after its C-terminal extremity.
Consensus pattern: [DE]-[LIVMF](2)-[HEQS]-x-G-x-[LIVMFA]-G-L-[LIVMFYE]-x-[GSAM]-[LIVMAP]
Consensus pattern: [STN]-x(2)-[DEQ]-[LIVM]-[GAS]-x(4)-[LIVMF]-[PSTG]-x(3)-[LIVMA]-x-[NQR]-[LIVMA]-[EQH]-x(3)-[LIVMFW]-x(2)-[LIVM]

[1] Helmann J.D., Chamberlin M.J. Annu. Rev. Biochem. 57:839-872(1988).
[2] Gribskov M., Burgess R.R. Nucleic Acids Res. 14:6745-6763(1986).
[3] Lonetto M.A., Gribskov M., Gross C.A. J. Bacteriol. 174:3843-3849(1992).
[4] Lonetto M.A., Brown K.L., Rudd K.E., Buttner M.J. Proc. Natl. Acad. Sci. U.S.A. 91:7573-7577(1994).

626. Signal carboxyl-terminal domain. 430 members.

627. Signal peptidases I signatures
Signal peptidases (SPases) [1] (also known as leader peptidases) remove the signal peptides from secretory proteins. In prokaryotes three types of Spases are known: type I (gene lepB) which is responsible for the processing of the majority of exported pre-proteins; type II (gene lsp) which only process lipoproteins, and a third type involved in the processing of pili subunits. SPase I is an integral membrane protein that is anchored in the cytoplasmic membrane by one (in B. subtilis) or two (in E. coli) N-terminal transmembrane domains with the main part of the protein protuding in the periplasmic space. Two residues have been shown [2,3] to be essential for the catalytic activity of SPase I: a serine and an lysine.SPase I is evolutionary related to the yeast mitochondrial inner membrane protease subunit 1 and 2 (genes IMP1 and IMP2) which catalyze the removal of signal peptides required for the targeting of proteins from the mitochondrial matrix, across the inner membrane, into the inter-membrane space [4]. In eukaryotes the removal of signal peptides is effected by an oligomeric enzymatic complex composed of at least five subunits: the signal peptidase complex (SPC). The SPC is located in the endoplasmic reticulum membrane. Two components of mammalian SPC, the 18 Kd (SPC18) and the 21 Kd (SPC21) subunits as well as the yeast SEC11 subunit have been shown [5] to share regions of sequence similarity with prokaryotic SPases I and yeast IMP1/IMP2. Three signature patterns for these proteins have been developed. The first signature contains the putative active site serine, the second signature contains the putative active site lysine which is not conserved in the SPC subunits, and the third signature corresponds to a conserved region of unknown iological significance which is located in the C-terminal section of all these proteins.
Consensus pattern: [GS]-x-S-M-x-[PS]-[AT]-[LF] [S is an active site residue]
Consensus pattern: K-R-[LIVMSTA](2)-G-x-[PG]-G-[DE]-x-[LIVM]-x-[LIVMFY] [K is an active site residue]
Consensus pattern: [LIVMFYW](2)-x(2)-G-D-[NH]-x(3)-[SND]-x(2)-[SG]

[1] Dalbey R.E., von Heijne G. Trends Biochem. Sci. 17:474-478(1992).
[2] Sung M., Dalbey R.E. J. Biol. Chem. 267:13154-13159(1992).
[3] Black M.T. J. Bacteriol. 175:4957-4961(1993).
[4] Nunnari J., Fox T.D., Walter P. Science 262:1997-2004(1993).
[5] van Dijl J.M., de Jong A., Vehmaanpera J., Venema G., Bron S. EMBO J. 11:2819-2828(1992).
[6] Rawlings N.D., BarrettA.J. Meth. Enzymol. 244:19-61(1994).[E1]

628. (sodcu) Copper/Zinc superoxide dismutase signatures
Copper/Zinc superoxide dismutase (SODC) [1] is one of the three forms of an enzyme that catalyzes the dismutation of superoxide radicals. SODC binds one atom each of zinc and copper. Various forms of SODC are known: acytoplasmic form in eukaryotes, an additional chloroplast form in plants, an extracellular form in some eukaryotes, and a periplasmic form in prokaryotes. The metal binding sites are conserved in all the known SODC sequences [2]. Two signature patterns have been derived for this family of enzymes: the first one contains two histidine residues that bind the copper atom; the second one islocated in the C-terminal section of SODC and contains a cysteine which is involved in a disulfide bond.
Consensus pattern: [GA]-[IMFAT]-H-[LIVF]-H-x(2)-[GP]-[SDG]-x-[STAGDE] [The two H's are copper ligands]
Consensus pattern: G-[GN]-[SGA]-G-x-R-x-[SGA]-C-x(2)-[IV] [C is involved in a disulfide bond]

[1] Bannister J.V., Bannister W.H., Rotilio G. CRC Crit. Rev. Biochem. 22:111-154(1987).
[2] Smith M.W., Doolittle R.F. J. Mol. Evol. 34:175-184(1992).

629. (sodfe) Manganese and iron superoxide dismutases signature

Manganese superoxide dismutase (SODM) [1] is one of the three forms of an enzyme that catalyzes the dismutation of superoxide radicals. The four ligands of the manganese atom are conserved in all the known SODM sequences. These metal ligands are also conserved in the related iron form ofsuperoxide dismutases [2,3]. A short conserved region which includes two of the four ligands: an aspartate and a histidine has been selected as a signature. Consensus pattern: D-x-W-E-H-[STA]-[FY](2) [D and H are manganese/iron ligands]

[1] Bannister J.V., Bannister W.H., Rotilio G. CRC Crit. Rev. Biochem. 22:111-154(1987).
[2] Parker M.W., Blake C.C.F. FEBS Lett. 229:377-382(1988).
[3] Smith M.W., Doolittle R.F. J. Mol. Evol. 34:175-184(1992).

630. Spectrin repeat

Spectrin repeats are found in several proteins involved in cytoskeletal structure. These include spectrin, alpha-actinin and dystrophin.The sequence repeat used in this family is taken from the structural repeat in reference [2]. The spectrin repeat forms a three helix bundle. The second helix is interrupted by proline in some sequences. Number of members: 898

[1] Actin-binding proteins. 1: Spectrin super family. Hartwig JH; Protein Profile 1995;2:732-732.
[2] Crystal structure of the repetitive segments of spectrin. Yan Y, Winograd E, Viel A, Cronin T, Harrison SC, Branton D; Science 1993;262:2027-2030.

631. (subtilase) Streptomyces subtilisin-type inhibitors signature

Bacteria of the Streptomyces family produce a family of proteinase inhibitors [1] characterized by their strong activity toward subtilisin. They arecollectively known as SSI's: Streptomyces Subtilisin Inhibitors. Some SSI'salso inhibit trypsin or chymotrypsin. In their mature secreted form, SSI's areproteins of about 110 residues with two conserved disulfide bonds. +-------+ +--------------+||| | xxxxxxxxxxxxxxCxxxxxxxCxxxxxxxxxCx#xxxxxxxxxxxxCxxxxxx ************'C': conserved cysteine involved in a disulfide bond.'#': active site residue.'*': position of the pattern. Consensus pattern: C-x-P-x(2,3)-G-x-H-P-x(4)-A-C-[ATD]-x-L [The two C's are involved in a disulfide bond]

[1] Taguchi S., Kojima S., Terabe M., Miura K.-I., Momose H. Eur. J. Biochem. 220:911-918(1994).

632. Sugar transport proteins signatures

In mammalian cells the uptake of glucose is mediated by a family of closely related transport proteins which are called the glucose transporters [1,2,3].At least seven of these transporters are currently known to exist (in Human they are encoded by the GLUT1 to GLUT7 genes).These integral membrane proteins are predicted to comprise twelve membrane spanning domains. The glucose transporters show sequence similarities [4,5] with a number of other sugar or metabolite transport proteins listed below (references are only provided for recently determined sequences). - Escherichia coli arabinose-proton symport (araE). - Escherichia coli galactose-proton symport (galP). - Escherichia coli and Klebsiella pneumoniae citrate-proton symport (also known as citrate utilization determinant) (gene cit). - Escherichia coli alpha-ketoglutarate permease (gene kgtP). - Escherichia coli proline/betaine transporter (gene proP) [6]. - Escherichia coli xylose-proton symport (xylE). - Zymomonas mobilis glucose facilitated diffusion protein (gene glf). - Yeast high and low affinity glucose transport proteins (genes SNF3, HXT1 to HXT14). - Yeast galactose transporter (gene GAL2). - Yeast maltose permeases (genes MAL3T and MAL6T). - Yeast myo-inositol transporters (genes ITR1 and ITR2). - Yeast carboxylic acid transporter protein homolog JEN1. - Yeast inorganic phosphate transporter (gene PHO84). Kluyveromyces lactis lactose permease (gene LAC12). Neurospora crassa quinate transporter (gene Qa-y), and Emericella nidulans quinate permease (gene qutD). - Chlorella hexose carrier (gene HUP1). - Arabidopsis thaliana glucose transporter (gene STP1). - Spinach sucrose transporter. - Leishmania donovani transporters D1 and D2. - Leishmania enriettii probable transport protein (LTP). - Yeast hypothetical proteins YBR241c, YCR98c and YFL040w. - Caenorhabditis elegans hypothetical protein ZK637.1. - Escherichia coli hypothetical proteins yabE, ydjE and yhjE. - Haemophilus influenzae hypothetical proteins HI0281 and HI0418. - Bacillus subtilis hypothetical proteins yxbC and yxdF. It has been suggested [4] that these transport proteins have evolved from theduplication of an ancestral protein with six transmembrane regions, this hypothesis is based on the conservation of two G-R-[KR] motifs. The first one is located between the second and third transmembrane domains and the second one between transmembrane domains 8 and 9. Two patterns have been developed to detect this family of proteins. The first pattern is based on the G-R-[KR] motif; but because this motif is too short to be specific to this family of proteins, a pattern from a larger region centered on the second copy of this motif was derived. The second pattern is based on a number of conserved residues which are located at the end of the fourth transmembrane

segment and in the short loop region between the fourth and fifth segments.
Consensus pattern: [LIVMSTAG]-[LIVMFSAG]-x(2)-[LIVMSA]-[DE]-x-[LIVMFYWA]-G- R-[RK]-x(4,6)-[GSTA]
Consensus pattern: [LIVMF]-x-G-[LIVMFA]-x(2)-G-x(8)-[LIFY]-x(2)-[EQ]-x(6)- [RK]

[1] Silverman M. Annu. Rev. Biochem. 60:757-794(1991).
[2] Gould G.W., Bell G.I. Trends Biochem. Sci. 15:18-23(1990).
[3] Baldwin S.A. Biochim. Biophys. Acta 1154:17-49(1993).
[4] Maiden M.C.J., Davis E.O., Baldwin S.A., Moore D.C.M., Henderson P.J.F. Nature 325:641-643(1987).
[5] Henderson P.J.F. Curr. Opin. Struct. Biol. 1:590-601(1991).
[6] Culham D.E., Lasby B., Marangoni A.G., Milner J.L., Steer B.A., van Nues R.W., Wood J.M. J. Mol. Biol. 229:268-276(1993).

633. Synaptobrevin signature
Synaptobrevin [1] is an intrinsic membrane protein of small synaptic vesicles whose function is not yet known, but which is highly conserved in mammals, electric ray (where its is known as VAMP-1), Drosophila and yeast [2]. In yeast there are two closely related forms of synaptobrevin (genes SNC1 andSNC2) while in mammals there is at least 4 (genes SYB1, SYB2, SYB3 and SYBL1). Structurally synaptobrevin consist of aN-terminal cytoplasmic domain of from 90 to 110 residues, followed by a transmembrane region, and then by a short (from 2 to 22 residues) C-terminal intravesicular domain. As a signature pattern for synaptobrevin, a highly conserved stretch of residues located in the central part of the sequence was selected.
Consensus pattern: N-[LIVM]-[DENS]-[KL]-V-x-[DEQ]-R-x(2)-[KR]-[LIVM]-[STDE]- x-[LIVM]-x-[DE]-[KR]-[TA]-[DE]

[1] SuedhofT.C., Baumert M., Perin M.S., Jahn R. Neuron 2:1475-1481(1989).
[2] Gerst J.E., Rodgers L., Riggs M., Wigler M. Proc. Natl. Acad. Sci. U.S.A. 89:4338-4342(1992).

634. TBC domain. Identification of a TBC domain in GYP6_YEAST and GYP7_YEAST, which are GTPase activator proteins of yeast Ypt6 and Ypt7, imply that these domains are GTPase activator proteins of Rab-like small GTPases. Number of members: 55

[1] Medline: 96032578. Molecular cloning of a cDNA with a novel domain present in the tre-2 oncogene and the yeast cell cycle regulators BUB2 and cdc16. Richardson PM, Zon LI; Oncogene 1995;11:1139-1148.
[2]Medline: 97398935. A shared domain between a spindle assembly checkpoint protein and Ypt/Rab-specific GTPase-activators. Neuwald AF; Trends Biochem Sci 1997;22:243-244.

635. Transcription factor TFIID repeat signature (TBP)
Transcription factor TFIID (or TATA-binding protein, TBP) [1,2] is a general factor that plays a major role in the activation of eukaryotic genes transcribed by RNA polymerase II. TFIID binds specifically to the TATA box promoter element which lies close to the position of transcription initiation. There is a remarkable degree of sequence conservation of a C-terminal domain of about 180 residues in TFIID from various eukaryotic sources. This region isnecessary and sufficient for TATA box binding. The most significant structural feature of this domain is the presence of two conserved repeats of a 77 amino-acid region. The intramolecular symmetry generates a saddle-shaped structure that sits astride the DNA [3]. Drosophila TRF (TBP-related factor) [4] is a sequence-specific transcription factor that also binds to the TATA box and is highly similar to TFIID. Archaebacteria also possess a TBP homolog [5]. A signature pattern that spans the last 50 residues of the repeated region has been derived.-
Consensus pattern: Y-x-P-x(2)-[IF]-x(2)-[LIVM](2)-x-[KRH]-x(3)-P-[RKQ]-x(3)- L-[LIVM]-F-x-[STN]-G-[KR]-[LIVM]-x(3)-G-[TAGL]-[KR]-x(7)- [AGC]-x(7)-[LIVM

[1] Hoffmann A., Sinn E., Yamamoto T., Wang J., Roy A., Horikoshi M., Roeder R.G. Nature 346:387-390(1990).
[2] Gash A., Hoffmann A., Horikoshi M., Roeder R.G., Chua N.-H. Nature 346:390-394(1990).
[3] Nikolov D.B., Hu S.-H., Lin J., Gasch A., Hoffmann A., Horikoshi M., Chua N.-H., Roeder R.G., Burley S.K. Nature 360:40-46(1992).
[4] Crowley T.E., Hoey T., Liu J.-K., Jan Y.N., Jan L.Y., Tjian R. Nature 361:557-561(1993).
[5] Marsh T.L., Reich C.I., Whitelock R.B., Olsen G.J. Proc. Natl. Acad. Sci. U.S.A. 91:4180-4184(1994).

636. Translationally controlled tumor protein signatures (TCTP)
Mammalian translationally controlled tumor protein (TCTP) (or P23) is a protein which has been found to be preferentially synthesized in cells during the early growth phase of some types of tumor [1,2], but which is also expressed in normal cells. The physiological function of TCTP is still not known. It is a hydrophilic protein of 18 to 20 Kd. Close

homologs have been found in plants [3], earthworm [4], Caenorhabditis elegans (F52H2.11), Hydra, budding yeast (YKL056c) [5] and fission yeast (SpAC1F12.02c) Two of the best conserved regions have been selected as signature patterns for TCTP.
Consensus pattern: [IFA]-[GA]-[GAS]-N-[PAK]-S-[GA]-E-[GDE]-[PAGE]-[DEQGA]
Consensus pattern: [FLVH]-[FY]-[IVCT]-G-E-x-[MA]-x(2,5)-[DEN]-[GAST]-x-[LV]-[AV]-x(3)-[FYW]

[1] Boehm H., Beendorf R., Gaestel M., Gross B., Nuernberg P., Kraft R., Otto A., Bielka H. Biochem. Int. 19: 277-286(1989).
[2] Makrides S., Chitpatima S.T., Bandyopadhyay R., Brawerman G. Nucleic Acids Res. 16:2350-2350(1988).
[3] Pay A., Heberle-Bors E., Hirt H. Plant Mol. Biol. 19:501-503(1992).
[4] Stuerzenbaum S.R., Kille P., Morgan A.J. Biochim. Biophys. Acta 1398:294-304(1998).
[5] Rasmussen S.W. Yeast 10:563-568(1994).

637. TFIIS zinc ribbon domain signature
Transcription factor S-II (TFIIS) [1] is a eukaryotic protein necessary for efficient RNA polymerase II transcription elongation, past template-encoded pause sites. TFIIS shows DNA-binding activity only in the presence of RNA polymerase II. It is a protein of about 300 amino acids whose sequence is highly conserved in mammals, Drosophila, yeast (where it was first known as PPR2, a transcriptional regulator of URA4, and then as DST1, the DNA strand transfer protein alpha [2]) and in the archaebacteria Sulfolobus acidocaldarius [3].This family also includes the eukaryotic and archebacterial RNA polymerase subunits of the 15 Kd / M family (see <PDOC00790>) as well as the following viral proteins: -
Vaccinia virus RNA polymerase 30 Kd subunit (rpo30) [4]. - African swine fever virus protein I243L [5].The best conserved region of all these proteins contains four cysteines that bind a zinc ion and fold in a conformation termed a 'zinc ribbon' [6]. Besides these cysteines, there are a number of other conserved residues which can be used to help define a specific pattern for this type of domain.
Consensus pattern: C-x(2)-C-x(9)-[LIVMQSAR]-[QH]-[STQL]-[RA]-[SACR]-x-[DE]-[DET]-[PGSEA]-x(6)-C-x(2,5)-C-x(3)-[FW] [The four C's are zinc ligands]

[1] Hirashima S., Hirai H., Nakanishi Y., Natori S. J. Biol. Chem. 263:3858-3863(1988).
[2] Kipling D., Kearsey S.E. Nature 353:509-509(1991).
[3] Langer D., Zillig W. Nucleic Acids Res. 21:2251-2251(1993).
[4] Ahn B.-Y., Gershon P.D., Jones E.V., Moss B. Mol. Cell. Biol. 10:5433-5441(1990).
[5] Rodriguez J.M., Salas M.L., Vinuela E. Virology 186:40-52(1992).
[6] Qian X., Jeon C., Yoon H., Agarwal K., Weiss M.A. Nature 365:277-279(1993).

638. Tetrahydrofolate dehydrogenase/cyclohydrolase signatures (THF DHG CYH) Enzymes that participate in the transfer of one-carbon units are involved in various biosynthetic pathways. In many of these processes the transfers of one-carbon units are mediated by the coenzyme tetrahydrofolate (THF). Various reactions generate one-carbon derivatives of THF which can be interconverted between different oxidation states by formyltetrahydrofolate synthetase(EC 6.3.4.3), methylenetetrahydrofolate dehydrogenase (EC 1.5.1.5 or EC 1.5.1.15) and methenyltetrahydrofolate cyclohydrolase (EC 3.5.4.9).The dehydrogenase and cyclohydrolase activities are expressed by a variety of multifunctional enzymes: - Eukaryotic C-1-tetrahydrofolate synthase (C1-THF synthase), which catalyzes all three reactions described above. Two forms of C1-THF synthases are known [1], one is located in the mitochondrial matrix, while the second one is cytoplasmic. In both forms the dehydrogenase/cyclohydrolase domain is located in the N-terminal section of the 900 amino acids protein and consists of about 300 amino acid residues. The C1-THF synthases are NADP- dependent. - Eukaryotic mitochondrial bifunctional dehydrogenase/cyclohydrolase [2]. This is an homodimeric NAD-dependent enzyme of about 300 amino acid residues. - Bacterial folD [3]. FolD is an homodimeric bifunctional NADP-dependent enzyme of about 290 amino acid residues. The sequence of the dehydrogenase/cyclohydrolase domain is highly conserved in all forms of the enzyme. Two conserved regions have been selected as signature patterns. The first one is located in the N-terminal part of these enzymes and contains three acidic residues. The second pattern is a highly conserved sequence of 9 amino acids which is located in the C-terminal section.
Consensus pattern: [EQ]-x-[EQK]-[LIVM](2)-x(2)-[LIVM]-x(2)-[LIVMY]-N-x-[DN]-x(5)-[LIVMF](3)-Q-L-P-[LV]
Consensus pattern: P-G-G-V-G-P-[MF]-T-[IV]

[1] Shannon K.W., Rabinowitz J.C. J. Biol. Chem. 263:7717-7725(1988).
[2] Belanger C., Mackenzie R.E. J. Biol. Chem. 264:4837-4843(1989).
[3] d'Ari L., Rabinowitz J.C. J. Biol. Chem. 266:23953-23958(1991).

639. Triosephosphate isomerase active site (TIM)
Triosephosphate isomerase (EC 5.3.1.1) (TIM) [1] is the glycolytic enzyme that catalyzes the reversible interconversion of glyceraldehyde 3-phosphate and dihydroxyacetone phosphate. TIM plays an important role in several metabolic pathways and is essential for efficient energy production. It is a dimer of identical subunits, each of which is made up of about 250 amino-acid residues. A glutamic acid residue is involved in the catalytic mechanism [2]. The sequence around the active site residue is perfectly conserved in all known TIM's and can be used as a signature pattern for this type of enzyme.
Consensus pattern: [AV]-Y-E-P-[LIVM]-W-[SA]-I-G-T-[GK] [E is the active site residue]

[1] Lolis E., Alber T., Davenport R.C., Rose D., Hartman F.C., Petsko G.A. Biochemistry 29:6609-6618(1990).
[2] Knowles J.R. Nature 350:121-124(1991).

640. Thymidine kinase cellular-type signature (TK)
Thymidine kinase (TK) (EC 2.7.1.21) is an ubiquitous enzyme that catalyzes the ATP-dependent phosphorylation of thymidine. A comparison of TK sequences has shown [1,2,3] that there are two different families of TK. One family groups together TK from herpes viruses as well as cellular thymidylate kinases, while the second family currently consists of TK from the following sources: - Vertebrates. - Bacterial. - Bacteriophage T4. - Pox viruses. - African swine fever virus (ASF). - Fish lymphocystis disease virus (FLDV).A conserved region which is located in the C-terminal section of these enzymes has been selected as a signature pattern for this family of TKA.
Consensus pattern: [GA]-x(1,2)-[DE]-x-Y-x-[STAP]-x-C-[NKR]-x-[CH]-[LIVMFYWH]

[1] Boyle D.B., Coupar B.E.H., Gibbs A.J., Seigman L.J., Both G.W. Virology 156:355-365(1987).
[2]
Blasco R., Lopez-Otin C., Munoz M., Bockamp E.-O., Simon-Mateo C., Vinuela E. Virology 178:301-304(1990).
[3] Robertson G.R., Whalley J.M. Nucleic Acids Res. 16:11303-11317(1988).

641. Thymidine kinase from herpesvirus (TK herpes)

[1]
Medline: 96003730
Crystal structures of the thymidine kinase from herpes simplex virus type-1 in complex with deoxythymidine and ganciclovir.
Brown DG, Visse R, Sandhu G, Davies A, Rizkallah PJ, Melitz C, Summers WC, Sanderson MR; Nat Struct Biol 1995;2:876-881.
Number of members: 65

642. Nuclear transition protein 2 signatures (TP2)
In mammals, the second stage of spermatogenesis is characterized by the conversion of nucleosomal chromatin to the compact, non-nucleosomal and transcriptionally inactive form found in the sperm nucleus. This condensation is associated with a double-protein transition. The first transition corresponds to the replacement of histones by several spermatid-specific proteins, also called transition proteins, which are themselves replaced by protamines during the second transition. Nuclear transition protein 2 (TP2) is one of those spermatid-specific proteins. TP2 is a basic, zinc-binding protein [1] of 116 to 137 amino-acid residues. Structurally, TP2 consists of three distinct parts: a conserved serine-rich N-terminal domain of about 25 residues, a variable central domain of 20 to 50 residues which contains cysteine residues, and a conserved C-terminal domain of about 70 residues rich in lysines and arginines. Two signature patterns for TP2 have been developed: one located in the N-terminal domain, the other in the C-terminal.
Consensus pattern: H-x(3)-H-S-[NS]-S-x-P-Q-S
Consensus pattern: K-x-R-K-x(2)-E-G-K-x(2)-K-[KR]-K

[1] Baskaran R., Rao M.R.S. Biochem. Biophys. Res. Commun. 179:1491-1499(1991).

643. Thiamine pyrophosphate enzymes signature (TTP enzymes)
A number of enzymes require thiamine pyrophosphate (TPP) (vitamin B1) as a cofactor. It has been shown [1] that some of these enzymes are structurally related. These related TPP enzymes are: - Pyruvate oxidase (POX) (EC 1.2.3.3) Reaction catalyzed: pyruvate + orthophosphate + O(2) + H(2)O = acetyl phosphate + CO(2) + H(2)O(2). - Pyruvate decarboxylase (PDC) (EC 4.1.1.1) Reaction catalyzed: pyruvate = acetaldehyde + CO(2). - Indolepyruvate decarboxylase (EC 4.1.1.74) [2] Reaction catalyzed: indole-3-pyruvate = indole-3-acetaldehyde + CO(2). - Acetolactate synthase (ALS) (EC 4.1.3.18) Reaction catalyzed: 2 pyruvate = acetolactate + CO(2). - Benzoylformate decarboxylase (BFD) (EC 4.1.1.7) [3] Reaction catalyzed: benzoylformate = benzaldehyde + CO(2). A conserved region which is located in their C-terminal section has been selected as a signature pattern for these enzymes. Consensus pattern: [LIVMF]-[GSA]-x(5)-P-x(4)-[LIVMFYW]-x-[LIVMF]-x-G-D-[GSA]-[GSAC]

[1] Green J.B.A. FEBS Lett. 246:1-5(1989).
[2] Koga J., Adachi T., Hidaka H. Mol. Gen. Genet. 226:10-16(1991).
[3] Tsou A.Y., Ransom S.C., Gerlt J.A., Buechter D.D., Babbitt P.C., Kenyon G.L. Biochemistry 29:9856-9862 (1990).

644. TPR Domain

[1]
Medline: 95397415 Tetratrico peptide repeat interactions: to TPR or not to TPR? Lamb JR, Tugendreich S, Hieter P; Trends Biochem Sci 1995;20:257-259.
[2]Medline: 98151343 The structure of the tetratricopeptide repeats of protein phosphatase 5: implications for TPR-mediated protein-protein interactions. Das AK, Cohen PW, Barford D; EMBO J 1998;17:1192-1199.
Number of members: 621

645. Uroporphyrin-III C-methyltransferase signatures (TP methylase)
Uroporphyrin-III C-methyltransferase (EC 2.1.1.107) (SUMT) [1,2] catalyzes the transfer of two methyl groups from S-adenosyl-L-methionine to the C-2 and C-7atoms of uroporphyrinogen III to yield precorrin-2 via the intermediate formation of precorrin-1. SUMT is the first enzyme specific to the cobalamin pathway and precorrin-2 is a common intermediate in the biosynthesis of corrinoids such as vitamin B12, siroheme and coenzyme F430.The sequences of SUMT from a variety of eubacterial and archaebacterial species are currently available. In species such as Bacillus megaterium (gene cobA), Pseudomonas denitrificans (cobA) or Methanobacterium ivanovii (gene corA) SUMT is a protein of about 25 to 30 Kd. In Escherichia coli and related bacteria, the cysG protein, which is involved in the biosynthesis of siroheme, is a multifunctional protein composed of a N-terminal domain, probably involved in transforming precorrin-2 into siroheme, and a C-terminal domain which has SUMT activity. The sequence of SUMT is related to that of a number of P. denitrificans and Salmonella typhimurium enzymes involved in the biosynthesis of cobalamin which also seem to be SAM-dependent methyltransferases [3,4]. The similarity is especially strong with two of these enzymes: cobI/cbiL which encodes S-adenosyl-L-methionine--precorrin-2 methyltransferase and cobM/cbiF whose exact function is not known. Two signature patterns have been developed for these enzymes. The first corresponds to a well conserved region in the N-terminal extremity (called region 1 in [1,3]) and the second to a less conserved region located in the central part of these proteins (this pattern spans what are called regions 2 and 3 in [1,3]).
Consensus pattern: [LIVM]-[GS]-[STAL]-G-P-G-x(3)-[LIVMFY]-[LIVM]-T-[LIVM]-[KRHQG]-[AG]
Consensus pattern: V-x(2)-[LI]-x(2)-G-D-x(3)-[FYW]-[GS]-x(8)-[LIVF]-x(5,6)-[LIVMFYWPAC]-x-[LIVMY]-x-P-G

[1] Blanche F., Robin C., Couder M., Faucher D., Cauchois L., Cameron B., Crouzet J. J. Bacteriol. 173: 4637-4645(1991).
[2] Robin C., Blanche F., Cauchois L., Cameron B., Couder M., Crouzet J. J. Bacteriol. 173:4893-4896(1991).
[3] Crouzet J., Cameron B., Cauchois L., Rigault S., Rouyez M.-C., Blanche F., Thibaut D., Debussche L. J. Bacteriol. 172:5980-5990(1990).
[4] Roth J.R., Lawrence J.G., Rubenfield M., Kieffer-Higgins S., Church G.M. J. Bacteriol. 175:3303-3316(1993).
[5] Mattheakis L.C., Shen W.H., Collier R.J. Mol. Cell. Biol. 12:4026-4037(1992).

646. Tudor domain
Domain of unknown function present in several RNA-binding proteins. copies in the Drosophila Tudor protein. Slight ambiguities in the alignment.Number of members: 18

[1]Medline: 97200561 Tudor domains in proteins that interact with RNA. Ponting CP; Trends Biochem Sci 1997; 22:51-52.
[2]Medline: 97157029 The human EBNA-2 coactivator p100: multidomain organization and relationship to the staphylococcal nuclease fold and to the tudor protein involved in Drosophila melanogaster development. Callebaut I, Mornon JP; Biochem J 1997;321:125-132.

647. Terpene synthase family

It has been suggested that this gene family be designated tps (for terpene synthase) [1]. It has been split into six subgroups on the basis of phylogeny, called tpsa-tpsf. tpsa includes vetispiridiene synthase Swiss:Q39979, 5-epiaristolochene synthase, Swiss:Q40577 and (+)-delta-cadinene synthase Swiss:P93665.
tpsb includes (-)-limonene synthase, Swiss:Q40322.
tpsc includes kaurene synthase A, Swiss:004408.
tpsd includes taxadiene synthase, Swiss:Q41594, pinene synthase,
Swiss:024475 and myrcene synthase, Swiss:024474.
tpse includes kaurene synthase B.
tpsf includes linalool synthase.
Number of members: 51

[1]
Medline: 97413772 Monoterpene synthases from grand fir (Abies grandis). cDNA isolation, characterization, and functional expression of myrcene synthase, (-)-(4S)-limonene synthase, and (-)-(1 S,5S)-pinene synthase. Bohlmann J, Steele CL, Croteau R; J Biol Chem 1997;272:21784-21792.

648. ThiF family
This family contains a repeated domain in ubiquitin activating enzyme E1 and members of the bacterial ThiF/MoeB/HesA family.Number of members: 87

649. Thioester dehydrase
Members of this family are involved in fatty acid biosynthesis.
Number of members: 19

[1]
Medline: 96398612 Structure of a dehydratase-isomerase from the bacterial pathway for biosynthesis of unsaturated fatty acids: two catalytic activities in one active site. Leesong M, Henderson BS, Gillig JR, Schwab JM, Smith JL; Structure 1996;4:253-264.
Database Reference: SCOP; 1mka; fa; [SCOP-USA][CATH-PDBSUM]
Database reference: PFAMB; PB058036;

650. Tub family signatures
The mouse tubby mutation is the cause of maturity-onset obesity, insulin resistance and sensory deficits. This mutation maps to a gene, tub [1,2],which codes for a protein that belongs to a family which currently consists of the following members: - Mammalian tub, an hydrophilic protein of about 500 residues, which could be involved in the hypothalamic regulation of body weight. - Human protein TULP1 [3] which may be involved in retinis pigmentosa 14, a retinal degeneration disease. - Mouse protein p4-6 whose function is not known. - Caenorhabditis elegans hypothetical protein F10B5.4. - Several fragmentary sequences from plants, Drosophila and human ESTs. While the N-terminal part of these protein is not conserved in length nor in the sequence, the C-terminal 250 residues are highly conserved. Therefore, two regions were selected in the C-terminal part as signature patterns. The secondr egion is located at the C-terminal extremity and contains a penultimate cysteine residue that could be critical to the normal functioning of these proteins.
Consensus pattern: F-[KHQ]-G-R-V-[ST]-x-A-S-V-K-N-F-Q
Consensus pattern: A-F-[AG]-I-[SAC]-[LIVM]-[ST]-S-F-x-[GST]-K-x-A-C-E

[1] Kleyn P.W., Fan W., Kovats S.G., Lee J.L., Pulido J.C., Wu Y., Berkemeier L.R., Misumi D.J., Holmgren L., Charlat O., Woolf E.A., Tayber O., Brody T., Shu P., Hawkins F., Kennedy B., Baldini L., Ebeling C., Alperin G.D., Deeds J., Lakey N.D., Culpepper J., Chen H., Glueck
smann-Kuis M.A., Carlson G.A., Duyk G.M., Moore K.J. Cell 85:281-290(1996).
[2] Noben-Trauth K., Naggert J.K., North M.A., Nishina P.M. Nature 380:534-538(1996).
[3] North M.A., Naggert J.K., Yan Y., Noben-Trauth K., Nishina P.M. Proc. Natl. Acad. Sci. U.S.A. 94:3128-3133 (1997).

651. Eukaryotic DNA topoisomerase I active site
DNA topoisomerase I (EC 5.99.1.2) [1,2,3,4,E1] is one of the two types of enzyme that catalyze the interconversion of topological DNA isomers. Type Itopoisomerases act by catalyzing the transient breakage of DNA, one strand at a time, and the subsequent rejoining of the strands. When a eukaryotic type 1topoisomerase breaks a DNA backbone bond, it simultaneously forms a protein-DNA link where the hydroxyl group of a tyrosine residue is joined to a 3'-phosphate on DNA, at one end of the enzyme-severed DNA strand. In eukaryotes and pox virus topoisomerases I, there are a number of conserved residues in the region around the active site tyrosine.
Consensus pattern: [DEN]-x(6)-[GS]-[IT]-S-K-x(2)-Y-[LIVM]-x(3)-[LIVM] [Y is the active site tyrosine]

[1] Sternglanz R. Curr. Opin. Cell Biol. 1:533-535(1990).
[2] Sharma A., Mondragon A. Curr. Opin. Struct. Biol. 5:39-47(1995).
[3] Lynn R.M., Bjornsti M.-A., Caron P.R., Wang J.C. Proc. Natl. Acad. Sci. U.S.A. 86:3559-3563(1989).
[4] Roca J. Trends Biochem. Sci. 20:156-160(1995).[E1]

652. Transaldolase signatures
Transaldolase (EC 2.2.1.2) catalyzes the reversible transfer of a three-carbonketol unit from sedoheptulose 7-phosphate to glyceraldehyde 3-phosphate to form erythrose 4-phosphate and fructose 6-phosphate. This enzyme, together with transketolase, provides a link between the glycolytic and pentose-phosphate pathways. Transaldolase is an enzyme of about 34 Kd whose sequence has been well conserved throughout evolution. A lysine has been implicated [1]in the catalytic mechanism of the enzyme; it acts as a nucleophilic group that attacks the carbonyl group of fructose-6-phosphate.Transaldolase is evolutionary related [2] to a bacterial protein of about 20Kd (known as talC in Escherichia coli), whose exact function is not yet known. Two signature patterns have been developed for these proteins. The first, located in the N-terminal section, contains a perfectly conserved pentapeptide; these cond, includes the active site lysine.
Consensus pattern: [DG]-[IVSA]-T-[ST]-N-P-[STA]-[LIVMF](2)
Consensus pattern: [LIVM]-x-[LIVM]-K-[LIVM]-[PAS]-x-[ST]-x-[DENQPAS]-G-[LIVM]-x-[AGV]-x-[QEKRST]-x-[LIVM] [K is the active site residue]

[1] Miosga T., Schaaff-Gerstenschlaeger I., Franken E., Zimmermann F.K. Yeast 9:1241-1249(1993).
[2] Reizer J., Reizer A., Saier M.H. Jr. Microbiology 141:961-971(1995).

653. (Transpeptidase) Penicillin binding protein transpeptidase domain
The active site serine (residue 337 in Swiss:P14677) is conserved in all members of this family.

[1] Pares S, Mouz N, Petillot Y, Hakenbeck R, Dideberg O Nat Struct Biol 1996;3:284-289.

654. Trehalase signatures
Trehalase (EC 3.2.1.28) is the enzyme responsible for the degradation of the disaccharide alpha, alpha-trehalose yielding two glucose subunits [1]. It is an enzyme found in a wide variety of organisms and whose sequence has been highly conserved throughout evolution. Two of the most highly conserved regions have been selected as signature patterns. The first pattern is located in the central section, the second one is in the C-terminal region.
Consensus pattern: P-G-G-R-F-x-E-x-Y-x-W-D-x-Y
Consensus pattern: Q-W-D-x-P-x-[GA]-W-[PAS]-P

[1] Kopp M., Mueller H., Holzer H. J. Biol. Chem. 268:4766-4774(1993).
[2] Henrissat B., BairochA. Biochem. J. 293:781-788(1993).[E1]

655. Trehalose-6-phosphate synthase domain
OtsA (Trehalose-6-phosphate synthase) is homologous to regions in the subunits of yeast trehalose-6-phosphate synthase/phosphate complex, [1].

[1] Kaasen I, McDougall J, Strom AR; Gene 1994;145:9-15.

656. Tropomyosins signature
Tropomyosins [1,2] are family of closely related proteins present in muscle and non-muscle cells. In striated muscle, tropomyosin mediate the interactions between the troponin complex and actin so as to regulate muscle contraction. The role of tropomyosin in smooth muscle and non-muscle tissues is not clear. Tropomyosin is an alpha-helical protein that forms a coiled-coil dimer. Muscle isoforms of tropomyosin are characterized by having 284 amino acid residues and a highly conserved N-terminal region, whereas non-muscle forms are generally smaller and are heterogeneous in their N-terminal region. The signature pattern for tropomyosins is based on a very conserved region in the C-terminal section of tropomyosins and which is present in both muscle and non-muscle forms.
Consensus pattern: L-K-E-A-E-x-R-A-E

[1] Smilie L.B. Trends Biochem. Sci. 4:151-155(1979).
[2] McLeod A.R. BioEssays 6:208-212(1986).

657. Troponin

Troponin (Tn) contains three subunits, Ca2+ binding (TnC), inhibitory (TnI), and tropomyosin binding (TnT). this Pfam contains members of the TnT subunit.

Troponin is a complex of three proteins, Ca2+ binding (TnC), inhibitory (TnI), and tropomyosin binding (TnT).

The troponin complex regulates Ca++ induced muscle contraction.

This family includes troponin T and troponin 1. Troponin I binds to actin and troponin T binds to tropomyosin.

Number of members: 81 [1]

Medline: 87144593 Structure of co-crystals of tropomyosin and troponin. White SP, Cohen C, Phillips GN Jr; Nature 1987;325:826-828. [2]

Medline: 95155315 A direct regulatory role for troponin T and a dual role for troponin C in the Ca2+ regulation of muscle contraction. Potter JD, Sheng Z, Pan BS, Zhao J; J Biol Chem 1995;270:2557-2562.

[3]Medline: 95324796 The troponin complex and regulation of muscle contraction. Farah CS, Reinach FC; FASEB J 1995;9:755-767.

658. (Tryp mucin) Mucin-like glycoprotein

This family of trypanosomal proteins resemble vertebrate mucins. The protein consists of three regions. The N and C terminii are conserved between all members of the family, whereas the central region is not well conserved and contains a large number of threonine residues which can be glycosylated [1].

Indirect evidence suggested that these genes might encode the core protein of parasite mucins, glycoproteins that were proposed to be involved in the interaction with, and invasion of, mammalian host cells.

[1] Di Noia JM, Sanchez DO, Frasch AC; J Biol Chem 1995;270:24146-24149.
[2] Di Noia JM, D'Orso I, Aslund L, Sanchez DO, Frasch AC; J Biol Chem 1998;273:10843-10850.

659. Aminoacyl-transfer RNA synthetases class-I signature (tRNA synt 1) Aminoacyl-tRNA synthetases (EC 6.1.1.-) [1] are a group of enzymes which activate amino acids and transfer them to specific tRNA molecules as the first step in protein biosynthesis. In prokaryotic organisms there are at least twenty different types of aminoacyl-tRNA synthetases, one for each differentamino acid. In eukaryotes there are generally two aminoacyl-tRNA synthetases for each different amino acid: one cytosolic form and a mitochondrial form. While all these enzymes have a common function, they are widely diverse interms of subunit size and of quaternary structure. A few years ago it was found [2] that several aminoacyl-tRNA synthetases share a region of similarity in their N-terminal section, in particular the consensus tetrapeptide His-Ile-Gly-His ('HIGH') is very well conserved. The 'HIGH' region has been shown [3] to be part of the adenylate binding site. The 'HIGH' signature has been found in the aminoacyl-tRNA synthetases specific for arginine, cysteine, glutamic acid, glutamine, isoleucine, leucine, methionine, tyrosine, tryptophan, and valine. These aminoacyl-tRNA synthetases are referred to as class-I synthetases [4,5,6] and seem to share the same tertiarystructure based on a Rossmann fold. Consensus pattern: P-x(0,2)-[GSTAN]-[DENQGAPK]-x-[LIVMFP]-[HT]-[LIVMYAC]-G-[HNTG]-[LIVMFYSTAGPC]

[1] Schimmel P. Annu. Rev. Biochem. 56:125-158(1987).
[2] Webster T., Tsai H., Kula M., Mackie G.A., Schimmel P. Science 226:1315-1317(1984).
[3] Brick P., Bhat T.N., Blow D.M. J. Mol. Biol. 208:83-98(1988).
[4] Delarue M., Moras D. BioEssays 15:675-687(1993).
[5] Schimmel P. Trends Biochem. Sci. 16:1-3(1991).
[6] Nagel G.M., Doolittle R.F. Proc. Natl. Acad. Sci. U.S.A. 88:8121-8125(1991).

660. Aminoacyl-transfer RNA synthetases class-I signature (tRNA synt 1b)

Aminoacyl-tRNA synthetases (EC 6.1.1.-) [1] are a group of enzymes which activate amino acids and transfer them to specific tRNA molecules as the first step in protein biosynthesis. In prokaryotic organisms there are at least twenty different types of aminoacyl-tRNA synthetases, one for each different amino acid. In eukaryotes there are generally two aminoacyl-tRNA synthetases for each different amino acid: one cytosolic form and a mitochondrial form. While all these enzymes have a common function, they are widely diverse in terms of subunit size and of quaternary structure. A few years ago it was found [2] that several aminoacyl-tRNA synthetases share a region of similarity in their N-terminal section, in particular the consensus tetrapeptide His-Ile-Gly-His ('HIGH') is very well conserved. The HIGH' region has been shown [3] to be part of the adenylate binding site. The 'HIGH' signature has been found in the aminoacyl-tRNA synthetases specific forarginine, cysteine, glutamic acid, glutamine, isoleucine, leucine,

methionine, tyrosine, tryptophan, and valine. These aminoacyl-tRNA synthetases are referred to as class-I synthetases [4,5,6] and seem to share the same tertiary structure based on a Rossmann fold. Consensus pattern: P-x(0,2)-[GSTAN]-[DENQGAPK]-x-[LIVMFP]-[HT]-[LIVMYAC]-G-[HNTG]-[LIVMFYSTAGPC

[1] Schimmel P. Annu. Rev. Biochem. 56:125-158(1987).
[2] Webster T., Tsai H., Kula M., Mackie G.A., Schimmel P. Science 226:1315-1317(1984).
[3] Brick P., Bhat T.N., Blow D.M. J. Mol. Biol. 208:83-98(1988).
[4] Delarue M., Moras D. BioEssays 15:675-687(1993).
[5] Schimmel P. Trends Biochem. Sci. 16:1-3(1991).
[6] Nagel G.M., Doolittle R.F. Proc. Natl. Acad. Sci. U.S.A. 88:8121-8125(1991).

661. (tRNA-synt 1 C) tRNA synthetases class I (E and Q)
Other tRNA synthetase sub-families are too dissimilar to be included.
This family includes only glutamyl and glutaminyl tRNA synthetases.
In some organisms, a single glutamyl-tRNA synthetase aminoacylates both tRNA(Glu) and tRNA(Gln).

[1] Rath VL, Silvian LF, Beijer B, Sproat BS, Steitz TA; Structure 1998;6:439-449.

662. (tRNA-synt 1d) tRNA synthetases class I (R)
Other tRNA synthetase sub-families are too dissimilar to be included.
This family includes only arginyl tRNA synthetase.

663. Aminoacyl-transfer RNA synthetases class-II signatures (tRNA synt 2)
Aminoacyl-tRNA synthetases (EC 6.1.1.-) [1] are a group of enzymes which activate amino acids and transfer them to specific tRNA molecules as the first step in protein biosynthesis. In prokaryotic organisms there are at least twenty different types of aminoacyl-tRNA synthetases, one for each different amino acid. In eukaryotes there are generally two aminoacyl-tRNA synthetases for each different amino acid: one cytosolic form and a mitochondrial form. While all these enzymes have a common function, they are widely diverse interms of subunit size and of quaternary structure. The synthetases specific for alanine, asparagine, aspartic acid, glycine, histidine, lysine, phenylalanine, proline, serine, and threonine are referred to as class-II synthetases [2 to 6] and probably have a common folding pattern in their catalytic domain for the binding of ATP and amino acid which is different to the Rossmann fold observed for the class I synthetases [7].Class-II tRNA synthetases do not share a high degree of similarity, however at least three conserved regions are present [2,5,8]. Signature patterns have been derived from two of these regions.
Consensus pattern: [FYH]-R-x-[DE]-x(4,12)-[RH]-x(3)-F-x(3)-[DE
Consensus pattern: [GSTALVF]-{DENQHRKP}-[GSTA]-[LIVMF]-[DE]-R-[LIVMF]-x-[LIVMSTAG]-[LIVMFY]

[1] Schimmel P. Annu. Rev. Biochem. 56:125-158(1987).
[2] Delarue M., Moras D. BioEssays 15:675-687(1993).
[3] Schimmel P. Trends Biochem. Sci. 16:1-3(1991).
[4] Nagel G.M., Doolittle R.F. Proc. Natl. Acad. Sci. U.S.A. 88:8121-8125(1991).
[5] Cusack S., Haertlein M., Leberman R. Nucleic Acids Res. 19:3489-3498(1991).
[6] Cusack S. Biochimie 75:1077-1081(1993).
[7] Cusack S., Berthet-Colominas C., Haertlein M., Nassar N., Leberman R. Nature 347:249-255(1990).
[8] Leveque F., Plateau P., Dessen P., Blanquet S. Nucleic Acids Res. 18:305-312(1990).

664. Aminoacyl-transfer RNA synthetases class-I signature (tRNA synt 1e)
Aminoacyl-tRNA synthetases (EC 6.1.1.-) [1] are a group of enzymes which activate amino acids and transfer them to specific tRNA molecules as the first step in protein biosynthesis. In prokaryotic organisms there are at least twenty different types of aminoacyl-tRNA synthetases, one for each different amino acid. In eukaryotes there are generally two aminoacyl-tRNA synthetases for each different amino acid: one cytosolic form and a mitochondrial form. While all these enzymes have a common function, they are widely diverse in terms of subunit size and of quaternary structure. A few years ago it was found [2] that several aminoacyl-tRNA synthetases share a region of similarity in their N-terminal section, in particular the consensus tetrapeptide His-Ile-Gly-His ('HIGH') is very well conserved. The HIGH' region has been shown [3] to be part of the adenylate binding site. The 'HIGH' signature has been found in the aminoacyl-tRNA synthetases specific forarginine, cysteine, glutamic acid, glutamine, isoleucine, leucine, methionine, tyrosine, tryptophan, and valine. These aminoacyl-tRNA synthetases are referred to as class-I synthetases [4,5,6] and seem to share the same tertiary structure based on a Rossmann fold.
Consensus pattern: P-x(0,2)-[GSTAN]-[DENQGAPK]-x-[LIVMFP]-[HT]-[LIVMYAC]-G-[HNTG]-[LIVMFYSTAGPC

[1] Schimmel P. Annu. Rev. Biochem. 56:125-158(1987).
[2] Webster T., Tsai H., Kula M., Mackie G.A., Schimmel P. Science 226:1315-1317(1984).
[3] Brick P., Bhat T.N., Blow D.M. J. Mol. Biol. 208:83-98(1988).
[4] Delarue M., Moras D. BioEssays 15:675-687(1993).
[5] Schimmel P. Trends Biochem. Sci. 16:1-3(1991).
[6] Nagel G.M., Doolittle R.F. Proc. Natl. Acad. Sci. U.S.A. 88:8121-8125(1991).

665. Aminoacyl-transfer RNA synthetases class-II signatures (tRNA synt 2b)
Aminoacyl-tRNA synthetases (EC 6.1.1.-) [1] are a group of enzymes which activate amino acids and transfer them to specific tRNA molecules as the first step in protein biosynthesis. In prokaryotic organisms there are at least twenty different types of aminoacyl-tRNA synthetases, one for each different amino acid. In eukaryotes there are generally two aminoacyl-tRNA synthetases for each different amino acid: one cytosolic form and a mitochondrial form. While all these enzymes have a common function, they are widely diverse interms of subunit size and of quaternary structure. The synthetases specific for alanine, asparagine, aspartic acid, glycine, histidine, lysine, phenylalanine, proline, serine, and threonine are referred to as class-II synthetases [2 to 6] and probably have a common folding pattern in their catalytic domain for the binding of ATP and amino acid which is different to the Rossmann fold observed for the class I synthetases [7].Class-II tRNA synthetases do not share a high degree of similarity, however at least three conserved regions are present [2,5,8]. Signature patterns have been derived from two of these regions.
Consensus pattern: [FYH]-R-x-[DE]-x(4,12)-[RH]-x(3)-F-x(3)-[DE
Consensus pattern: [GSTALVF]- {DENQHRKP} -[GSTA]-[LIVMF]-[DE]-R-[LIVMF]-x-[LIVMSTAG]-[LIVMFY]

[1] Schimmel P. Annu. Rev. Biochem. 56:125-158(1987).
[2] Delarue M., Moras D. BioEssays 15:675-687(1993).
[3] Schimmel P. Trends Biochem. Sci. 16:1-3(1991).
[4] Nagel G.M., Doolittle R.F. Proc. Natl. Acad. Sci. U.S.A. 88:8121-8125(1991).
[5] Cusack S., Haertlein M., Leberman R. Nucleic Acids Res. 19:3489-3498(1991).
[6] Cusack S. Biochimie 75:1077-1081(1993).
[7] Cusack S., Berthet-Colominas C., Haertlein M., Nassar N., Leberman R. Nature 347:249-255(1990).
[8] Leveque F., Plateau P., Dessen P., Blanquet S. Nucleic Acids Res. 18:305-312(1990).

666. Thaumatin family signature
Thaumatin [1] is an intensively sweet-tasting protein (100 000 times sweeter than sucrose on a molar basis) from Thaumatococcus daniellii, an African brush. The protein is made of about 200 residues and contains 8 disulfide bonds. A number of proteins have been found to be related to thaumatins. These protein are listed below (references are only provided for recently determined sequences). - A maize alpha-amylase/trypsin inhibitor. - Two tobacco pathogenesis-related proteins: PR-R major and minor forms, which are induced after infection with viruses. - Salt-induced protein NP24 from tomato. - Osmotin, a salt-induced protein from tobacco. - Osmotin-like proteins OSML13, OSML15 and OSML81 from potato [2]. - P21, a leaf protein from soybean. - PWIR2, a leaf protein from wheat. - Zeamatin, a maize antifunal protein [3].The exact biological function of all these proteins is not yet known. A conserved region that includes three cysteine residues known (in thaumatin) to be involved in disulfide bonds has been selected as a signature pattern.
+--------------------------------------------------------------------------------+|+---------------+|*******
|||
xxCxxxxxxxxxxxxxxxxCxxCxxCxCxxxxxxxxxxxxxxCxxCxCxxxCxCxxCCxCxxxCxxxxx CxxxCx |||||||||||+--+ +-+ |+---+ +--++-+ |+--------------------------+'C': conserved cysteine involved in a disulfide bond. '*': position of the pattern.
Consensus pattern: G-x-[GF]-x-C-x-T-[GA]-D-C-x(1,2)-G-x(2,3)-C

[1] Edens L., Heslinga L., Klok R., Ledeboer A.M., Maat J., Toonen M.Y., Visser C., Verrips C.T. Gene 18:1-12 (1982).
[2] Zhu B., Chen T.H.H., Li P.H. Plant Physiol. 108:929-937(1995).
[3] Malehom D.E., Borgmeyer J.R., Smith C.E., Shah D.M.; Plant Physiol. 106:1471-1481(1994).

667. Thiolases signatures
Two different types of thiolase [1,2,3] are found both in eukaryotes and in prokaryotes: acetoacetyl-CoA thiolase (EC 2.3.1.9) and 3-ketoacyl-CoA thiolase(EC 2.3.1.16). 3-ketoacyl-CoA thiolase (also called thiolase I) has a broad chain-length specificity for its substrates and is involved in degradative pathways such as fatty acid beta-oxidation. Acetoacetyl-CoA thiolase (also called thiolase II) is specific for the thiolysis of acetoacetyl-CoA and involved in biosynthetic pathways such as poly beta-hydroxybutyrate synthesisor steroid biogenesis. In eukaryotes, there are

two forms of 3-ketoacyl-CoA thiolase: one located in the mitochondrion and the other in peroxisomes. There are two conserved cysteine residues important for thiolase activity. The first located in the N-terminal section of the enzymes is involved in the formation of an acyl-enzyme intermediate; the second located at the C-terminal extremity is the active site base involved in deprotonation in the condensation reaction. Mammalian nonspecific lipid-transfer protein (nsL-TP) (also known as sterol carrier protein 2) is a protein which seems to exist in two different forms: a 14 Kd protein (SCP-2) and a larger 58 Kd protein (SCP-x). The former is found in the cytoplasm or the mitochondria and is involved in lipid transport; the latter is found in peroxisomes.
The C-terminal part of SCP-x is identical to SCP-2 while the N-terminal portion is evolutionary related to thiolases [4]. Three signature patterns have been developed for this family of proteins, two of which are based on the regions around the biologically important cysteines. The third is based on a highly conserved region in the C-terminal part of these proteins.
Consensus pattern: [LIVM]-[NST]-x(2)-C-[SAGLI]-[ST]-[SAG]-[LIVMFYNS]-x-[STAG]-[LIVM]-x(6)-[LIVM] [C is involved in formation of acyl-enzyme intermediate]
Consensus pattern: N-x(2)-G-G-x-[LIVM]-[SA]-x-G-H-P-x-[GA]-x-[ST]-G
Consensus pattern: [AG]-[LIVMA]-[STAGCLIVM]-[STAG]-[LIVMA]-C-x-[AG]-x-[AG]-x- [AG]-x-[SAG] [C is the active site residue]

[1] Peoples O.P., Sinskey A.J. J. Biol. Chem. 264:15293-15297(1989).
[2] Yang S.-Y., Yang X.-Y.H., Healy-Louie G., Schulz H., Elzinga M. J. Biol. Chem. 265:10424-10429(1990).
[3] Igual J.C., Gonzalez-Bosch C., Dopazo J., Perez-Ortin J.E. J. Mol. Evol. 35:147-155(1992).
[4] Baker M.E., Billheimer J.T., Strauss J.F. III DNA Cell Biol. 10:695-698(1991).

668. Thioredoxin family active site
Thioredoxins [1 to 4] are small proteins of approximately one hundred amino-acid residues which participate in various redox reactions via the reversible oxidation of an active center disulfide bond. They exist in either a reduced form or an oxidized form where the two cysteine residues are linked in an intramolecular disulfide bond. Thioredoxin is present in prokaryotes and eukaryotes and the sequence around the redox-active disulfide bond is wellconserved. Bacteriophage T4 also encodes for a thioredoxin but its primary structure is not homologous to bacterial, plant and vertebrate thioredoxins. A number of eukaryotic proteins contain domains evolutionary related tothioredoxin, all of them seem to be protein disulphide isomerases (PDI). PDI(EC 5.3.4.1) [5,6,7] is an endoplasmic reticulum enzyme that catalyzes the rearrangement of disulfide bonds in various proteins. The various forms of PDI which are currently known are: - PDI major isozyme; a multifunctional protein that also function as the beta subunit of prolyl 4-hydroxylase (EC 1.14.11.2), as a component of oligosaccharyl transferase (EC 2.4.1.119), as thyroxine deiodinase (EC 3.8. 1.4), as glutathione-insulin transhydrogenase (EC 1.8.4.2) and as a thyroid hormone-binding protein ! - ERp60 (ER-60; 58 Kd microsomal protein). ERp60 was originally thought to be a phosphoinositide-specific phospholipase C isozyme and later to be a protease. - ERp72. - P5.All PDI contains two or three (ERp72) copies of the thioredoxin domain. Bacterial proteins that act as thiol:disulfide interchange proteins thatallows disulfide bond formation in some periplasmic proteins also contain a thioredoxin domain. These proteins are: -

Escherichia coli dsbA (or prfA) and its orthologs in Vibrio cholerae (tcpG) and
Haemophilus influenzae (por). - Escherichia coli dsbC (or xpRA) and its orthologs in Erwinia chrysanthemi and Haemophilus influenzae. - Escherichia coli dsbD (or dipZ) and its Haemophilus influenzae ortholog. - Escherichia coli dsbE (or ccmG) and orthologs in Haemophilus influenzae, Rhodobacter capsulatus (helX), Rhiziobiacae (cycY and tlpA). Consensus pattern: [LIVMF]-[LIVMSTA]-x-[LIVMFYC]-[FYWSTHE]-x(2)-[FYWGTN]-C- [GATPLVE]-[PHYWSTA]-C-x(6)-[LIVMFYWT] [The two C's form the redox-active bond]

[1] Holmgren A. Annu. Rev. Biochem. 54:237-271(1985).
[2] Gleason F.K., Holmgren A. FEMS Microbiol. Rev. 54:271-297(1988).
[3] Holmgren A. J. Biol. Chem. 264:13963-13966(1989).
[4] Eklund H., Gleason F.K., Holmgren A. Proteins 11:13-28(1991).
[5] Freedman R.B., Hawkins H.C., Murant S.J., Reid L. Biochem. Soc. Trans. 16:96-99(1988).
[6] Kivirikko K.I., Myllyla R., Pihlajaniemi T. FASEB J. 3:1609-1617(1989).
[7] Freedman R.B., Hirst T.R., Tuite M.F. Trends Biochem. Sci. 19:331-336(1994).

669. (Transcript fac2) Transcription factor TFIIB repeat signature
In eukaryotes the initiation of transcription of protein encoding genes by polymerase II is modulated by general and specific transcription factors. The general transcription factors operate through common promoters elements (such as the TATA box). At least seven different proteins associates to form the general transcription factors: TFIIA, -IIB,

-IID, - IIE, -IIF, -IIG, and -IIH[1].Transcription factor IIB (TFIIB) plays a central role in the transcription of class II genes, it associates with a complex of TFIID-IIA bound to DNA (DA complex) to form a ternary complex TFIID-IIA-IBB (DAB complex) which is then recognized by RNA polymerase II [2,3]. TFIIB is a protein of about 315 to 340amino acid residues which contains, in its C-terminal part an imperfect repeat of a domain of about 75 residues. This repeat could contribute an element of symmetry to the folded protein. The following proteins have been shown to be evolutionary related to TFIIB: - An archaebacterial TFIIB homolog. In Pyrococcus woesei a previously undetected open reading frame has been shown [4] to be highly related to TFIIB. - Fungal transcription factor IIIB 70 Kd subunit (gene PCF4/TDS4/BRF1) [5]. This protein is a general activator of RNA polymerase III transcription and plays a role analogous to that of TFIIB in pol III transcription. The central section of the repeated domain, which is the most conserved part of that domain has been selected as a signature pattern.
Consensus pattern: G-[KR]-x(3)-[STAGN]-x-[LIVMYA]-[GSTA](2)-[CSAV]-[LIVM]-[LIVMFY]-[LIVMA]-[GSA]-[STAC

[1] Weinmann R. Gene Expr. 2:81-91(1992).
[2] Hawley D. Trends Biochem. Sci. 16:317-318(1991).
[3] Ha I., Lane W.S., Reinberg D. Nature 352:689-695(1991).
[4] Ouzounis C., Sander C. Cell 71:189-190(1992).
[5] Khoo B., Brophy B., Jackson S.P. Genes Dev. 8:2879-2890(1994).

670. (transcritp fact) MADS-box domain signature and profile
A number of transcription factors contain a conserved domain of 56 amino-acid residues, sometimes known as the MADS-box domain [E1]. They are listed below: - Serum response factor (SRF) [1], a mammalian transcription factor that binds to the Serum Response Element (SRE). This is a short sequence of dyad symmetry located 300 bp to the 5' end of the transcription initiation site of genes such as c-fos. - Mammalian myocyte-specific enhancer factors 2A to 2D (MEF2A to MEF2D). These proteins are transcription factor which binds specifically to the MEF2 element present in the regulatory regions of many muscle-specific genes. - Drosophila myocyte-specific enhancer factor 2 (MEF2). - Yeast GRM/PRTF protein (gene MCM1) [2], a transcriptional regulator of mating-type-specific genes. - Yeast arginine metabolism regulation protein I (gene ARGR1 or ARG80). - Yeast transcription factor RLM1. - Yeast transcription factor SMP1. - Arabidopsis thaliana agamous protein (AG) [3], a probable transcription factor involved in regulating genes that determines stamen and carpel development in wild-type flowers. Mutations in the AG gene result in the replacement of the stamens by petals and the carpels by a new flower. - Arabidopsis thaliana homeotic proteins Apetala1 (AP1), Apetala3 (AP3) and Pistillata (PI) which act locally to specify the identity of the floral meristem and to determine sepal and petal development [4]. - Antirrhinum majus and tobacco homeotic protein deficiens (DEFA) and globosa (GLO) [5]. Both proteins are transcription factors involved in the genetic control of flower development. Mutations in DEFA or GLO cause the transformation of petals into sepals and of stamina into carpels. - Arabidopsis thaliana putative transcription factors AGL1 to AGL6 [6]. - Antirrhinum majus morphogenetic protein DEF H33 (squamosa).In SRF, the conserved domain has been shown [1] to be involved in DNA-binding and dimerization. A pattern that spans the complete length of the domain has been derived. The profile also spans the length of the MADS-box.
Consensus pattern: R-x-[RK]-x(5)-I-x-[DNGSK]-x(3)-[KR]-x(2)-T-[FY]-x-[RK](3)- x(2)-[LIVM]-x-K(2)-A-x-E-[LIVM]-[STA]-x-L-x(4)-[LIVM]-x- [LIVM](3)-x(6)-[LIVMF]-x(2)-[FY]

[1] Norman C., Runswick M., Pollock R., Treisman R. Cell 55:989-1003(1988).
[2] Passmore S., Maine G.T., Elble R., Christ C., Tye B.-K. J. Mol. Biol. 204:593-606(1988).
[3] Yanofsky M., Ma H., Bowman J., Drews G., Feldmann K.A., Meyerowitz E.M. Nature 346:35-39(1990).
[4] Goto K., Meyerowitz E.M. Genes Dev. 8:1548-1560(1994).
[5] Troebner W., Ramirez L., Motte P., Hue I., Huijser P., Loennig W.-E., Saedler H., Sommer H., Schwartz-Sommer Z. EMBO J. 11:4693-4704(1992).
[6] Ma H., Yanofsky M.F., Meyerowitz E.M. Genes Dev. 5:484-495(1991).[E1]

671. Transketolase signatures
Transketolase (EC 2.2.1.1) (TK) catalyzes the reversible transfer of a two-carbon ketol unit from xylulose 5-phosphate to an aldose receptor, such as ribose 5-phosphate, to form sedoheptulose 7-phosphate and glyceraldehyde 3-phosphate. This enzyme, together with transaldolase, provides a link between the glycolytic and pentose-phosphate pathways. TK requires thiamin pyrophosphate as a cofactor. In most sources where TK has been purified, it is a homodimer of approximately 70 Kd subunits. TK sequences from a variety of eukaryotic and prokaryotic sources [1,2] show that the enzyme has been evolutionarily conserved. In the peroxisomes of methylotrophic yeast Hansenula polymorpha, there is a highly related enzyme, dihydroxy-acetone synthase (DHAS) (EC 2.2.1.3) (also known as formaldehyde transketolase), which exhibits a very unusual specificity by including formaldehyde amongst its substrates. 1-deoxyxylulose-5-phosphate synthase (DXP synthase) [3] is an enzyme so far found in bacteria (gene dxs) and plants (gene CLA1) which catalyzes the thiamin pyrophosphoate-dependent acyloin condensation reaction between carbon atoms 2 and 3 of pyruvate and glyceraldehyde 3-phosphate to yield 1-deoxy-D- xylulose-5-phosphate (dxp), a precursor in the biosynthetic pathway to isoprenoids, thiamin (vitamin B1), and pyridoxol (vitamin B6). DXP synthase is evolutionary related to TK. Two regions of TK have been selected as signature patterns. The first, located in the N-terminal section, contains a histidine residue which appears to function inproton transfer during catalysis [4]. The second, located in the central section, contains conserved acidic residues that are part of the active cleft and may participate in substrate-binding [4].
Consensus pattern: R-x(3)-[LIVMTA]-[DENQSTHKF]-x(5,6)-[GSN]-G-H-[PLIVMF]-[GSTA]-x(2)-[LIMC]-[GS
Consensus pattern: G-[DEQGSA]-[DN]-G-[PAEQ]-[ST]-[HQ]-x-[PAGM]-[LIVMYAC]-[DEFYW]-x(2)-[STAP]-x(2)-[RGA]

[1] Abedinia M., Layfield R., Jones S.M., Nixon P.F., Mattick J.S. Biochem. Biophys. Res. Commun. 183: 1159-1166(1992).
[2] Fletcher T.S., Kwee I.L., Nakada T., Largman C., Martin B.M. Biochemistry 31:1892-1896(1992).
[3] Sprenger G.A., Schorken U., Wiegert T., Grolle S., De Graaf A.A., Taylor S.V., Begley T.P., Bringer-Meyer S., Sahm H. Proc. Natl. Acad. Sci. U.S.A. 94:12857-12862(1997).
[4] Lindqvist Y., Schneider G., Ermler U., Sundstroem M. EMBO J. 11:2373-2379(1992).

672. Transmembrane 4 family signature
Recently a number of eukaryotic cell surface antigens have been found to be evolutionary related [1,2,3]. The proteins known to belong to this family are listed below: - Mammalian antigen CD9 (MIC3); A protein involved in platelet activation and aggregation. - Mammalian leukocyte antigen CD37, expressed on B lymphocytes. - Mammalian leukocyte antigen CD53 (OX-44), which may be involved in growth regulation in hematopoietic cells. - Mammalian lysosomal membrane protein CD63 (melanoma-associated antigen ME491; antigen AD1). - Mammalian antigen CD81 (cell surface protein TAPA-1), which may play an important role in the regulation of lymphoma cell growth. - Mammalian antigen CD82 (protein R2; antigen C33; Kangai 1 (KAI1)), which associates with CD4 or CD8 and delivers costimulatory signals for the TCR/CD3 pathway. - Mammalian antigen CD151 (SFA-1; platelet-endothelial tetraspan antigen 3 (PETA-3)). - Mammalian cell surface glycoprotein A15 (TALLA-1; MXS1). - Mammalian novel antigen 2 (NAG-2). - Human tumor-associated antigen CO-029. - Schistosoma mansoni and j aponicum 23 Kd surface antigen (SM23 / SJ23).These proteins share the following characteristics: they all seem to be type III membrane proteins (type III proteins are integral membrane proteins that contain a N-terminal membrane-anchoring domain which is not cleaved during biosynthesis and which functions both as a translocation signal and as a membrane anchor); they also contain three additional transmembrane regions, at least seven conserved cysteines residues, and are of approximately the same size (218 to 284 residues). These proteins are collectively know as the 'transmembrane 4 super family' (TM4) because they span the plasma membrane four times. A schematic diagram of the domain structure of these proteins isshown below. +-+---+-------+----+-----+-----+--------------------+-----+----+|| TMa| Extra| TM2| Cyt| TM3 | Extracellular |TM4 |Cyt| +-+-----+-------+----C-----C-----+--------CC---------C-----C--+-----C----+ *********Cyt : cytoplasmic domain. TMa : transmembrane anchor.TM2 to TM4: transmembrane regions 2 to 4.'C' : conserved cysteine. '*': position of the pattern.
A conserved region that includes two cysteines and seems to be located in a short cytoplasmic loop between two transmembrane domains has been selected as a signature for these proteins.
Consensus pattern: G-x(3)-[LIVMF]-x(2)-[GSA]-[LIVMF](2)-G-C-x-[GA]-[STA]- x(2)-[EG]-x(2)-[CWN]-[LIVM](2)

[1] Levy S., Nguyen V.Q., Andria M.L., Takahashi S. J. Biol. Chem. 266:14597-14602(1991).
[2] Tomlinson M.G., Williams A.F., Wright M.D. Eur. J. Immunol. 23:136-40(1993).
[3] Barclay A.N., Birkeland M.L., Brown M.H., Beyers A.D., Davis S.J., Somoza C., Williams A.F. The leucocyte antigen factbooks. Academic Press, London / San Diego, (1993).

673. Tryptophan synthase alpha chain signature
Tryptophan synthase catalyzes the last step in the biosynthesis of tryptophan: the conversion of indoleglycerol phosphate and serine, totryptophan and glyceraldehyde 3-phosphate [1,2]. It has two functional domains: one for the aldol cleavage of indoleglycerol phosphate to indole and glyceraldehyde 3-phosphate and the other for the synthesis of tryptophan fromindole and serine. In bacteria and plants [3], each domain is found on a separate subunit (alpha and beta chains), while in fungi the two domains are fused together on a single multifunctional protein. A conserved region that contains three conserved acidic residues has been selected as a signature pattern for the alpha chain. The first and the third acidic residues are believed to serve as proton donors/acceptors in the enzyme's catalytic mechanism.

Consensus pattern: [LIVM]-E-[LIVM]-G-x(2)-[FYC]-[ST]-[DE]-[PA]-[LIVMY]- [AGLI]-[DE]-G

[1] Crawford I.P. Annu. Rev. Microbiol. 43:567-600(1989).
[2] Hyde C.C., Miles E.W. Bio/Technology 8:27-32(1990).
[3] Berlyn M.B., Last R.L., Fink G.R. Proc. Natl. Acad. Sci. U.S.A. 86:4604-4608(1989).

674. Tryptophan synthase beta chain pyridoxal-phosphate attachment site
Tryptophan synthase catalyzes the last step in the biosynthesis of tryptophan: the conversion of indoleglycerol phosphate and serine, totryptophan and glyceraldehyde 3-phosphate [1,2]. It has two functional domains: one for the aldol cleavage of indoleglycerol phosphate to indole and glyceraldehyde 3-phosphate and the other for the synthesis of tryptophan fromindole and serine. In bacteria and plants [3], each domain is found on a separate subunit (alpha and beta chains), while in fungi the two domains are fused together on a single multifunctional protein. The beta chain of the enzyme requires pyridoxal-phosphate as a cofactor. The pyridoxal-phosphate group is attached to a lysine residue. The region around this lysine residue also contains two histidine residues which are part of the pyridoxal-phosphate binding site. The signature pattern for the tryptophansynthase beta chain is derived from that conserved region.

- Consensus pattern: [LIVM]-x-H-x-G-[STA]-H-K-x-N [K is the pyridoxal-P attachment site]

[1] Crawford I.P. Annu. Rev. Microbiol. 43:567-600(1989).
[2] Hyde C.C., Miles E.W. Bio/Technology 8:27-32(1990).
[3] Berlyn M.B., Last R.L., Fink G.R. Proc. Natl. Acad. Sci. U.S.A. 86:4604-4608(1989).

675. Serine proteases, trypsin family, active sites
The catalytic activity of the serine proteases from the trypsin family is provided by a charge relay system involving an aspartic acid residue hydrogen-bonded to a histidine, which itself is hydrogen-bonded to a serine. The sequences in the vicinity of the active site serine and histidine residues are well conserved in this family of proteases [1]. A partial list of proteases known to belong to the trypsin family is shown below. - Acrosin. - Blood coagulation factors VII, IX, X, XI and XII, thrombin, plasminogen, and protein C. - Cathepsin G. - Chymotrypsins. - Complement components C1r, C1s, C2, and complement factors B, D and I. - Complement-activating component of RA-reactive factor. - Cytotoxic cell proteases (granzymes A to H). - Duodenase I. - Elastases 1, 2, 3A, 3B (protease E), leukocyte (medullasin). - Enterokinase (EC 3.4.21.9) (enteropeptidase). - Hepatocyte growth factor activator. - Hepsin. - Glandular (tissue) kallikreins (including EGF-binding protein types A, B, and C, NGF-gamma chain, gamma-renin, prostate specific antigen (PSA) and tonin). - Plasma kallikrein. - Mast cell proteases (MCP) 1 (chymase) to 8. - Myeloblastin (proteinase 3) (Wegener's autoantigen). - Plasminogen activators (urokinase-type, and tissue-type). - Trypsins I, II, III, and IV. - Tryptases. - Snake venom proteases such as ancrod, batroxobin, cerastobin, flavoxobin, and protein C activator. - Collagenase from common cattle grub and collagenolytic protease from Atlantic sand fiddler crab. - Apolipoprotein(a). - Blood fluke cercarial protease. - Drosophila trypsin like proteases: alpha, easter, snake-locus. - Drosophila protease stubble (gene sb). - Major mite fecal allergen Der p III. All the above proteins belong to family S1 in the classification of peptidases[2,E1] and originate from eukaryotic species. It should be noted thatbacterial proteases that belong to family S2A are similar enough in the regions of the active site residues that they can be picked up by the same patterns. These proteases are listed below. - Achromobacter lyticus protease I.- Lysobacter alpha-lytic protease. - Streptogrisin A and B (Streptomyces proteases A and B). - Streptomyces griseus glutamyl endopeptidase II. - Streptomyces fradiae proteases 1 and 2.
Consensus pattern: [LIVM]-[ST]-A-[STAG]-H-C [H is the active site residue]
Consensus pattern: [DNSTAGC]-[GSTAPIMVQH]-x(2)-G-[DE]-S-G-[GS]-[SAPHV]-[LIVMFYWH]-[LIVMFYSTANQH] [S is the active site residue]

[1] Brenner S. Nature 334:528-530(1988).
[2] Rawlings N.D., Barrett A.J. Meth. Enzymol. 244:19-61 (1994). [E1]

676. (tsp) Thrombospondin type 1 domain

[1] Bork P; FEBS lett 1993;327:125-130.

677. Tubulin subunits alpha, beta, and gamma signature
Tubulins [1,2], the major constituent of microtubules are dimeric proteins which consist of two closely related subunits (alpha and beta). Tubulin binds two molecules of GTP at two different sites (N and E). At the E (Exchangeable) site,

GTP is hydrolyzed during incorporation into the microtubule. Near the E site is an invariant region rich in glycines which is found in both chains and which is now [3] said to control the access of the nucleotide to its binding site. A signature pattern was developed from this region. With the exception of the simple eukaryotes, most species express a variety of closely related alpha and beta isotypes. In most species there is a third member of the tubulin family: gamma tubulin. Gamma tubulin is found at microtubule organizing centers (MTOC) such as the spindle poles or the centrosome, suggesting that it is involved in the minus-end nucleation of microtubule assembly [4].
Consensus pattern: [SAG]-G-G-T-G-[SA]-G

[1] Cleveland D.W., Sullivan K.F. Annu. Rev. Biochem. 54:331-365(1985).
[2] Joshi H.C., Cleveland D.W. Cell Motil. Cytoskeleton 16:159-163(1990).
[3] Hesse J., Thierauf M., Ponstingl H. J. Biol. Chem. 262:15472-15475(1987).
[4] Joshi H.C. BioEssays 15:637-643(1993).

Tubulin-beta mRNA autoregulation signal
The stability of beta-tubulin mRNAs are autoregulated by their own translation product [1]. Unpolymerized tubulin subunits bind directly (or activate a factor(s) which binds cotranslationally) to the nascent N-terminus of beta-tubulin. This binding is transduced through the adjacent ribosomes to activatean RNAse that degrades the polysome-bound mRNA. The recognition element has been shown to be the first four amino acids of beta-tubulin: Met-Arg-Glu-Ile. Mutations to this sequence abolish the autoregulation effect (except for the replacement of Glu by Asp); transposition of this sequence to an internal region of a polypeptide also suppresses the autoregulatory effect.
Consensus pattern: <M-R-[DE]-[IL]

[1] Cleveland D.W. Trends Biochem. Sci. 13:339-343(1988).

678. (tRNA-synt 2c) Aminoacyl-transfer RNA synthetases class-II signatures. Aminoacyl-tRNA synthetases (EC 6.1.1.-) [1] are a group of enzymes which activate amino acids and transfer them to specific tRNA molecules as the first step in protein biosynthesis. In prokaryotic organisms there are at least twenty different types of aminoacyl-tRNA synthetases, one for each different amino acid. In eukaryotes there are generally two aminoacyl-tRNA synthetases for each different amino acid: one cytosolic form and a mitochondrial form. While all these enzymes have a common function, they are widely diverse in terms of subunit size and of quaternary structure. The synthetases specific for alanine, asparagine, aspartic acid, glycine, histidine, lysine, phenylalanine, proline, serine, and threonine are referred to as class-II synthetases [2 to 6] and probably have a common folding pattern in their catalytic domain for the binding of ATP and amino acid which is different to the Rossmann fold observed for the class I synthetases [7].Class-II tRNA synthetases do not share a high degree of similarity, however at least three conserved regions are present [2,5,8]. Signature patterns have been derived from two of these regions.
Consensus pattern: [FYH]-R-x-[DE]-x(4,12)-[RH]-x(3)-F-x(3)-[DE]-
Consensus pattern: [GSTALVF]-{DENQHRKP}-[GSTA]-[LIVMF]-[DE]-R-[LIVMF]-x-[LIVMSTAG]-[LIVMFY]-

[1] Schimmel P. Annu. Rev. Biochem. 56:125-158(1987).
[2] Delarue M., Moras D. BioEssays 15:675-687(1993).
[3] Schimmel P. Trends Biochem. Sci. 16:1-3(1991).
[4] Nagel G.M., Doolittle R.F. Proc. Natl. Acad. Sci. U.S.A. 88:8121-8125(1991).
[5] Cusack S., Haertlein M., Leberman R. Nucleic Acids Res. 19:3489-3498(1991).
[6] Cusack S. Biochimie 75:1077-1081(1993).
[7] Cusack S., Berthet-Colominas C., Haertlein M., Nassar N., Leberman R. Nature 347:249-255(1990).
[8] Leveque F., Plateau P., Dessen P., Blanquet S. Nucleic Acids Res. 18:305-312(1990).

679. UBA-domain
The UBA-domain (ubiquitin associated domain) is a novel sequence motif found in several proteins having connections to ubiquitin and the ubiquitination pathway. The structure of the UBA domain consists of a compact three helix bundle [1]. Number of members: 84

[1] Structure of a human DNA repair protein UBA domain that interacts with HIV-1 Vpr. Dieckmann T, Withers-Ward ES, Jarosinski MA, Liu CF, Chen IS, Feigon J; Nat Struct Biol 1998;5:1042-1047.

680. UBX domain
Domain present in ubiquitin-regulatory proteins. Present in FAF1 and Shp1p.Number of members: 19

[1] The UBA domain: a sequence motif present in multiple enzyme classes of the ubiquitination pathway. Hofmann K, Bucher P; Trends Biochem Sci 1996;21:172-173.

681. (UCH) Ubiquitin carboxyl-terminal hydrolases family 1 cysteine active site
Ubiquitin carboxyl-terminal hydrolases (UCH) (deubiquitinating enzymes) [1,2] are thiol proteases that recognize and hydrolyze the peptide bond at the C-terminal glycine of ubiquitin. These enzymes are involved in the processing of poly-ubiquitin precursors as well as that of ubiquinated proteins. There are two distinct families of UCH. The first class consist of enzymes ofabout 25 Kd and is currently represented by: - Mammalian isozymes L1 and L3. - Yeast YUH1. - Drosophila Uch.One of the active site residues of class-I UCH [3] is a cysteine. A signature pattern has been derived from the region around that residue. Consensus pattern: Q-x(3)-N-[SA]-C-G-x(3)-[LIVM](2)-H-[SA]-[LIVM]-[SA] [C is the active site residue

[1] Jentsch S., Seufert W., Hauser H.-P. Biochim. Biophys. Acta 1089:127-139(1991).
[2] D'andrea A., Pellman D. Crit. Rev. Biochem. Mol. Biol. 33:337-352(1998).
[3] Johnston S.C., Larsen C.N., Cook W.J., Wilkinson K.D., Hill C.P. EMBO J. 16:3787-3796(1997).
[4] Rawlings N.D., Barrett A.J. Meth. Enzymol. 244:461-486(1994).

682. Ubiquitin carboxyl-terminal hydrolases family 2 signatures (UCH-1)
Ubiquitin carboxyl-terminal hydrolases (UCH) (deubiquitinating enzymes) [1,2] are thiol proteases that recognize and hydrolyze the peptide bond at the C-terminal glycine of ubiquitin. These enzymes are involved in the processing of poly-ubiquitin precursors as well as that of ubiquinated proteins. There are two distinct families of UCH. The second class consist of largeproteins (800 to 2000 residues) and is currently represented by: - Yeast UBP1, UBP2, UBP3, UBP4 (or DOA4/SSV7), UBP5, UBP7, UBP9, UBP10, UBP11, UBP12, UBP13, UBP14, UBP15 and UBP16. - Human tre-2. - Human isopeptidase T. - Human isopeptidase T-3. - Mammalian Ode-1. - Mammalian Unp. - Mouse Dub-1. - Drosophila fat facets protein (gene faf). - Mammalian faf homolog. - Drosophila D-Ubp-64E. - Caenorhabditis elegans hypothetical protein R10E11.3. - Caenorhabditis elegans hypothetical protein K02C4.3.These proteins only share two regions of similarity. The first region containsa conserved cysteine which is probably implicated in the catalytic mechanism. The second region contains two conserved histidines residues, one of which is also probably implicated in the catalytic mechanism. Signature patterns for both conserved regions have been developed.
Consensus pattern: G-[LIVMFY]-x(1,3)-[AGC]-[NASM]-x-C-[FYW]-[LIVMC]-[NST]-[SACV]-x-[LIVMS]-Q [C is the putative active site residue]
Consensus pattern: Y-x-L-x-[SAG]-[LIVMFT]-x(2)-H-x-G-x(4,5)-G-H-Y [The two H's are putative active site residues]

[1] Jentsch S., Seufert W., Hauser H.-P. Biochim. Biophys. Acta 1089:127-139(1991).
[2] D'andrea A., Pellman D. Crit. Rev. Biochem. Mol. Biol. 33:337-352(1998).
[3] Rawlings N.D., Barrett A.J. Meth. Enzymol. 244:461-486(1994).

683. Ubiquitin carboxyl-terminal hydrolases family 2 signatures (UCH-2)
Ubiquitin carboxyl-terminal hydrolases (UCH) (deubiquitinating enzymes) [1,2] are thiol proteases that recognize and hydrolyze the peptide bond at the C-terminal glycine of ubiquitin. These enzymes are involved in the processing of poly-ubiquitin precursors as well as that of ubiquinated proteins. There are two distinct families of UCH. The second class consist of largeproteins (800 to 2000 residues) and is currently represented by: - Yeast UBP1, UBP2, UBP3, UBP4 (or DOA4/SSV7), UBP5, UBP7, UBP9, UBP10, UBP11, UBP12, UBP13, UBP14, UBP15 and UBP16. - Human tre-2. - Human isopeptidase T. - Human isopeptidase T-3. - Mammalian Ode-1. - Mammalian Unp. - Mouse Dub-1. - Drosophila fat facets protein (gene faf). - Mammalian faf homolog. - Drosophila D-Ubp-64E. - Caenorhabditis elegans hypothetical protein R10E11.3. - Caenorhabditis elegans hypothetical protein K02C4.3.These proteins only share two regions of similarity. The first region containsa conserved cysteine which is probably implicated in the catalytic mechanism. The second region contains two conserved histidines residues, one of which is also probably implicated in the catalytic mechanism. Signature patterns for both conserved regions have been developed.
Consensus pattern: G-[LIVMFY]-x(1,3)-[AGC]-[NASM]-x-C-[FYW]-[LIVMC]-[NST]-[SACV]-x-[LIVMS]-Q [C is the putative active site residue]
Consensus pattern: Y-x-L-x-[SAG]-[LIVMFT]-x(2)-H-x-G-x(4,5)-G-H-Y [The two H's are putative active site residues]

[1] Jentsch S., Seufert W., Hauser H.-P. Biochim. Biophys. Acta 1089:127-139(1991).
[2] D'andrea A., Pellman D. Crit. Rev. Biochem. Mol. Biol. 33:337-352(1998).
[3] Rawlings N.D., Barrett A.J. Meth. Enzymol. 244:461-486(1994).

684. UDP-glycosyltransferases signature

UDP glycosyltransferases (UGT) are a superfamily of enzymes that catalyzes the addition of the glycosyl group from a UTP-sugar to a small hydrophobic molecule. This family currently consist of: - Mammalian UDP-glucoronosyl transferases (UDPGT) [1,2]. A large family of membrane-bound microsomal enzymes which catalyze the transfer of glucuronic acid to a wide variety of exogenous and endogenous lipophilic substrates. These enzymes are of major importance in the detoxification and subsequent elimination of xenobiotics such as drugs and carcinogens. - A large number of putative UDPGT from Caenorhabditis elegans. - Mammalian 2-hydroxyacylsphingosine 1-beta-galactosyltransferase [3] (also known as UDP-galactose-ceramide galactosyltransferase). This enzyme catalyzes the transfer of galactose to ceramide, a key enzymatic step in the biosynthesis of galactocerebrosides, which are abundant sphingolipids of the myelin membrane of the central nervous system and peripheral nervous system. - Plants flavonol O(3)-glucosyltransferase. An enzyme [4] that catalyzes the transfer of glucose from UDP-glucose to a flavanol. This reaction is essential and one of the last steps in anthocyanin pigment biosynthesis. - Baculoviruses ecdysteroid UDP-glucosyltransferase (EC 2.4.1.-) [5] (egt). This enzyme catalyzes the transfer of glucose from UDP-glucose to ectysteroids which are insect molting hormones. The expression of egt in the insect host interferes with the normal insect development by blocking the molting process. - Prokaryotic zeaxanthin glucosyl transferase (gene crtX), an enzyme involved in carotenoid biosynthesis and that catalyses the glycosylation reaction which converts zeaxanthin to zeaxanthin-beta- diglucoside. - Streptomyces macrolide glycosyltransferases [6]. These enzymes specifically inactivates macrolide anitibiotics via 2'-O-glycosylation using UDP-glucose.These enzymes share a conserved domain of about 50 amino acid residues locatedin their C-terminal section and from which a pattern has been extracted todetect them.
Consensus pattern: [FW]-x(2)-Q-x(2)-[LIVMYA]-[LIMV]-x(4,6)-[LVGAC]-[LVFYA]-[LIVMF]-[STAGCM]-[HNQ]-[STAGC]-G-x(2)-[STAG]-x(3)-[STAGL]- [LIVMFA]-x(4)-[PQR]-[LIVMT]-x(3)-[PA]-x(3)-[DES]-[QEHN]

[1] Dutton G.J. (In) Glucoronidation of drugs and other compounds, Dutton G.J., Ed., pp 1-78, CRC Press, Boca Raton, (1980).
[2] Burchell B., Nebert D.W., Nelson D.R., Bock K.W., Iyanagi T., Jansen P.L., Lancet D., Mulder G.J., Chowdhury J.R., Siest G., Tephly T.R., Mackenzie P.I. DNA Cell Biol. 10:487-494(1991).
[3] Schulte S., Stoffel W. Proc. Natl. Acad. Sci. U.S.A. 90:10265-10269(1993).
[4] Furtek D., Schiefelbein J.W., Johnston F., Nelson O.E. Jr. Plant Mol. Biol. 11:473-481(1988).
[5] O'Reilly D.R., Miller L.K. Science 245:1110-1112(1989).
[6] Hernandez C., Olano C., Mendez C., Salas J.A. Gene 134:139-140(1993).

685. UDP-glucose/GDP-mannose dehydrogenase family
The UDP-glucose/GDP-mannose dehydrogenaseses are a small group of enzymes which possesses the ability to catlyze the NAD-dependent 2-fold oxidation of an alcholol to an acid without the release of an aldehyde intermediate [2]. Number of members: 55

[1] Purification and characterization of guanosine diphospho-D-mannose dehydrogenase. A key enzyme in the biosynthesis of alginate by Pseudomonas aeruginosa. Roychoudhury S, May TB, Gill JF, Singh SK, Feingold DS, Chakrabarty AM; J Biol Chem 1989;264:9380-9385.
[2] Properties and kinetic analysis of UDP-glucose dehydrogenase from group A streptococci. Irreversible inhibition by UDP-chloroacetol. Campbell RE, Sala RF, van de Rijn I, Tanner ME; J Biol Chem 1997;272: 3416-3422.

686. Uracil-DNA glycosylase signature
Uracil-DNA glycosylase (EC 3.2.2.-) (UNG) [1] is a DNA repair enzyme that excises uracil residues from DNA by cleaving the N-glycosylic bond. Uracil in DNA can arise as a result of misincorportation of dUMP residues by DNA polymerase or deamination of cytosine. The sequence of uracil-DNA glycosylase is extremely well conserved [2] in bacteria and eukaryotes as well as in herpes viruses. More distantly related uracil-DNA glycosylases are also found in poxviruses [3].In eukaryotic cells, UNG activity is found in both the nucleus and the mitochondria. Human UNG1 protein is transported to both the mitochondria and the nucleus [4]. The N-terminal 77 amino acids of UNG1 seem to be required for mitochondrial localization [4], but the presence of a mitochondrial transitpeptide has not been directly demonstrated. As a signature for this type of enzyme, the most N-termina conserved region has been selected. This region contains an aspartic acid residue which has been proposed, based on X-ray structures [5,6] to act as a general base in the catalytic mechanism. Consensus pattern: [KR]-[LIV]-[LIVC]-[LIVM]-x-G-[QI]-D-P-Y [D is the active site residue]-

[1] Sancar A., Sancar G.B. Annu. Rev. Biochem. 57:29-67(1988).
[2] Olsen L.C., Aasland R., Wittwer C.U., Krokan H.E., Helland D.E. EMBO J. 8:3121-3125 (1989).

[3] Upton C., Stuart D.T., McFadden G. Proc. Natl. Acad. Sci. U.S.A. 90:4518-4522(1993).
[4] Slupphaug G., Markussen F.-H., Olsen L.C., Aasland R., Aarsaether N., Bakke O., Krokan H.E., Helland D.E. Nucleic Acids Res. 21:2579-2584(1993).
[5] Savva R., McAuley-Hecht K., Brown T., Pearl L. Nature 373:487-493(1995).
[6] Mol C.D., Arvai A.S., Slupphaug G., Kavli B., Alseth I., Krohan H.E., Tainer J.A. Cell 80:869-878(1995).
[7] Muller S.J., Caradonna S. Biochim. Biophys. Acta 1088:197-207(1991).
[8] Meyer-Siegler K., Mauro D.J., Seal G., Wurzer J., Deriel J.K., Sirover M.A. Proc. Natl. Acad. Sci. U.S.A. 88: 8460-8464(1991).
[9] Muller S.J., Caradonna S. J. Biol. Chem. 268:1310-1319(1993).
[10] Barnes D.E., Lindahl T., Sedgwick B. Curr. Opin. Cell Biol. 5:424-433(1993).

687. Uncharacterized protein family UPF0001 signature
The following uncharacterized proteins have been shown [1] to share regions ofsimilarities:

- Yeast chromosome II hypothetical protein YBL036c. - Caenorhabditis elegans hypothetical protein F09E5.8. - Bacillus subtilis hypothetical protein ylmE. - Escherichia coli hypothetical protein yggS and HI0090, the corresponding Haemophilus influenzae protein. - Helicobacter pylori hypothetical protein HP0395. - Mycobacterium tuberculosis hypothetical protein MtCY270.20. - Synechocystis strain PCC 6803 hypothetical protein slr0556. - A Pseudomonas aeruginosa hypothetical protein in pilT 5'region. - A Vibrio alginolyticus hypothetical protein in pilT 5'region. These are proteins of from 25 to 30 Kd which contain a number of conserved regions. The best conserved region which is located in the first third of these proteins has been selected as a signature pattern.

Consensus pattern: [FW]-H-[FM]-[IV]-G-x-[LIV]-Q-x-[NKR]-K-x(3)-[LIV]

[1] Bairoch A., Rudd K.E. Unpublished observations (1996).

688. Uncharacterized protein family UPF0003 signature
The following uncharacterized proteins have been shown [1] to share regions of similarities:

- Escherichia coli protein aefA. - Escherichia coli hypothetical protein yggB. - Escherichia coli hypothetical protein yjeP and HI0195.1, the corresponding Haemophilus influenzae protein. - Escherichia coli hypothetical protein ynaI. - Bacillus subtilis hypothetical protein yhdY. - Helicobacter pylori hypothetical protein HP0415. - Synechocystis strain PCC 6803 hypothetical protein s1r0639. - Archaeoglobus fulgidus hypothetical protein AF1546. - Methanococcus jannaschii hypothetical protein MJ0170.- Methanococcus jannaschii hypothetical protein MJ1143.The size of these proteins range from 30 to 120 Kd. They all contain a number of transmembrane regions. The best conserved region which is located in and just after the last potential transmembrane region has been selected as a signature pattern,.

Consensus pattern: G-[STIF]-V-x(2)-[LIVM]-x(6)-[LIVMF]-x(3)-[DQ]-x(3)-[LIV]- x-[LIV]-P-N-x(2)-[LIVMF]-[LIVFSTA]-x(5)-N

[1] Bairoch A. Unpublished observations (1997).

689. Uncharacterized protein family UPF0004 signature
The following uncharacterized proteins have been shown [1] to share regions of similarities:

- Escherichia coli hypothetical protein yliG. - Escherichia coli hypothetical protein yleA and HI0019, the corresponding Haemophilus influenzae protein. - Bacillus subtilis hypothetical protein yqeV. - Helicobacter pylori hypothetical protein HP0269. - Helicobacter pylori hypothetical protein HP0285. - Mycoplasma iowae hypothetical protein in 16S RNA 5'region. - Mycobacterium leprae hypothetical protein B2235_C2_195. - Pseudomonas aeruginosa hypothetical protein in hemL 3'region. - Synechocystis strain PCC 6803 hypothetical protein slr0082. - Synechocystis strain PCC 6803 hypothetical protein sll0996.
- Methanococcus jannaschii hypothetical protein MJ0865. - Methanococcus jannaschii hypothetical protein MJ0867. - Caenorhabditis elegans hypothetical protein F25B5.5.The size of these proteins range from 47 to 61 Kd. They contain six conserved cysteines, three of which are clustered in a region that can be used as a signature pattern.

Consensus pattern: [LIVM]-x-[LIVMT]-x(2)-G-C-x(3)-C-[STAN]-[FY]-C-x-[LIVM]- x(4)-G

[1] Bairoch A. Unpublished observations (1997).

690. Uncharacterized protein family UPF0005 signature
The following proteins seems to be evolutionary related [1]: - Mammalian protein TEGT (Testis Enhanced Gene Transcript). - Escherichia coli hypothetical protein yccA and HI0044, the corresponding Haemophilus influenzae protein. - A probable Pseudomonas aeruginosa ortholog of yccA. These are proteins of about 25 Kd which seem to contain seven transmembranedomains. A signature pattern that corresponds to a region that starts with the beginning of the third transmembrane domain and ends in the middle of the fourth one has been developed.
Consensus pattern: G-[LIVM](2)-[SA]-x(5,8)-G-x(2)-[LIVM]-G-P-x-L-x(4)-[SAG]- x(4,6)-[LIVM](2)-x(2)-A-x(3)-T-A-[LIVM](2)-F

[1] Walter L., Marynen P., Szpirer J., Levan G., Guenther E. Genomics 28:301-304(1995).

691. Uncharacterized protein family UPF0006 signatures
The following uncharacterized proteins have been shown [1] to share regions of similarities:

- Yeast chromosome II hypothetical protein YBL055c. - Escherichia coli hypothetical protein ycfH and HI0454, the corresponding Haemophilus influenzae protein. - Escherichia coli hypothetical protein yigW. - Escherichia coli hypothetical protein yjjV and HI0081, the corresponding Haemophilus influenzae protein. - Bacillus subtilis hypothetical protein yabD. - Haemophilus influenzae hypothetical protein HI1664. - Mycoplasma genitalium hypothetical protein MG009. These are proteins of from 24 to 47 Kd which contain a number of conserved regions. They can be picked up in the database by the following patterns.

Consensus pattern: [LIVMFY](2)-D-[STA]-H-x-H-[LIVMF]-[DN
Consensus pattern: P-[LIVM]-x-[LIVM]-H-x-R-x-[TA]-x-[DE
Consensus pattern: [LVSA]-[LIVA]-x(2)-[LIVM]-[PS]-x(3)-L-[LIVM]-[LIVMS]-E-T- D-x-P

[1] Bairoch A., Rudd K.E. Unpublished observations (1995).

692. Uncharacterized protein family UPF0007 signature
The following proteins seems to be evolutionary related [1]: - Escherichia coli hypothetical protein ygbP and HI0672, the corresponding Haemophilus influenzae protein. - Bacillus subtilis hypothetical protein yacM.- Mycobacterium tuberculosis hypothetical protein MtCY06G11.29c. - Synechocystis strain PCC 6803 hypothetical protein slr0951. - A Rhodobacter capsulatus hypothetical protein in nifR3 5'region. Except for the Rhodobacter protein which contains a C-terminal extension, all these proteins have from 225 to 236 amino acids. They are hydrophilic proteins that can be picked up in the database by the following pattern.
Consensus pattern: V-L-[IV]-H-D-[GA]-A-R

[1] Bairoch A. Unpublished observations (1997).

693. Uncharacterized protein family UPF0015 signature
The following uncharacterized proteins have been shown [1] to share regions of similarities:

- Yeast chromosome II hypothetical protein YBR002c. - Yeast chromosome XIII hypothetical protein YMR101 c. - Escherichia coli hypothetical protein yaeU and HI0920, the corresponding Haemophilus influenzae protein. - Helicobacter pylori hypothetical protein HP1221. - Mycobacterium leprae hypothetical protein B1937_F2_65. - A Corynebacterium glutamicum hypothetical protein in aroF 3'region. - A Streptomyces fradiae hypothetical protein in transposon Tn4556. - Synechocystis strain PCC 6803 hypothetical protein s110505. - Methanococcus jannaschii hypothetical protein MJ1372.These are proteins of about 26 to 40 Kd whose central region is well conserved. They can be picked up in the database by the following pattern.

Consensus pattern: [DE]-[LIVMF](3)-R-T-[SG]-G-x(2)-R-x-S-x-[FY]-[LIVM](2)-W-Q-

[1] Wolfe K.H., Lohan A.J.E. Yeast 10:S41-S46(1994).

694. Uncharacterized protein family UPF0016 signature
The following uncharacterized proteins have been shown [1] to share regions of similarities:

- Yeast hypothetical protein YBR187w. - Fission yeast hypothetical protein SpAC17G8.08c. - Mouse protein pFT27. - Synechocystis strain PCC 6803 hypothetical protein s110615. These are hydrophobic proteins of 200 to 320 amino acids that seem to contain six or seven transmembrane domains. A conserved region which seems, in the eukaryotic proteins of this family, to directly follow the second transmembrane domain has been selected as a signature pattern.

Consensus pattern: E-[LIVM]-G-D-K-T-F-[LIVMF](2)-A-

[1] Bairoch A. Unpublished observations (1996).

695. Uncharacterized protein family UPF0021 signature
The following uncharacterized proteins have been shown [1] to share regions of similarities:

- Yeast chromosome VII hypothetical protein YGL211w. - Dictyostelium discoideum protein veg136. - Methanococcus jannaschii hypothetical proteins MJ1157 and MJ1478.These are proteins of from 300 to 360 residues. They can be picked up in the database by the following pattern which is located in their N-terminalsection.

Consensus pattern: C-K-x(2)-F-x(4)-E-x(22,23)-S-G-G-K-D
[1] Bairoch A. Unpublished observations (1997).

696. Uncharacterized protein family UPF0023 signature
The following uncharacterized proteins have been shown [1] to share regions of similarities:

- Mouse protein 22A3. - Yeast chromosome XII hypothetical protein YLR022c. - Caenorhabditis elegans hypothetical protein W06E11.4. - Methanococcus jannaschii hypothetical protein MJ0592.These are hydrophilic proteins of about 30 Kd. They can be picked up in the database by the following pattern.

Consensus pattern: D-x-D-E-[LIV]-L-x(4)-V-F-x(3)-S-K-G-

[1] Bairoch A. Unpublished observations (1997).

697. Uncharacterized protein family UPF0024 signature. The following uncharacterized proteins have been shown [1] to share regions of similarities: - Escherichia coli hypothetical protein ygbO and HI0701, the corresponding Haemophilus influenzae protein. - Helicobacter pylori hypothetical protein HP0926. - Yeast chromosome XV hypothetical protein YOR243c. - Caenorhabditis elegans hypothetical protein B0024.11. - Methanococcus jannaschii hypothetical proteins MJ0588 and MJ1364.These are hydrophilic proteins of from 39 to 77 Kd. They can be picked up in the database by the following pattern.
Consensus pattern: G-x-K-D-[KR]-x-A-[LV]-T-x-Q-x-[LIVF]-[SGC]-

[1] Bairoch A. Unpublished observations (1997).

698. Uncharacterized protein family UPF0025 signature
The following uncharacterized proteins have been shown [1] to share regions of similarities:

- Escherichia coli hypothetical protein yfcE. - Bacillus subtilis hypothetical protein ysnB. - Mycoplasma genitalium and pneumoniae hypothetical protein MG207. - Methanococcus jannaschii hypothetical proteins MJ0623 and MJ0936. These are hydrophilic proteins of about 20 Kd. They can be picked up in the database by the following pattern.

Consensus pattern: D-V-[LIV]-x(2)-G-H-[ST]-H-x(12)-[LIVMF]-N-P-G

[1] Bairoch A. Unpublished observations (1997).

699. Uncharacterized protein family UPF0029 signature
The following uncharacterized proteins have been shown [1] to share regions of similarities:

- Yeast chromosome III hypothetical protein YCR59c. - Yeast chromosome IV hypothetical protein YDL177C. - Escherichia coli hypothetical protein yigZ and HI0722, the corresponding Haemophilus influenzae protein. -

Bacillus subtilis hypothetical protein yvyE. - A Thermus aquaticus hypothetical protein in pol 5'region. These proteins can be picked up in the database by the following pattern.

Consensus pattern: G-x(2)-[LIVM](2)-x(2)-[LIVM]-x(4)-[LIVM]-x(5)-[LIVM](2)-x- R-[FYW](2)-G-G-x(2)-[LIVM]-G

[1] Koonin E.V., Bork P., Sander C. EMBO J. 13:493-503(1994).

700. Uncharacterized protein family UPF0030 signature

The following uncharacterized proteins have been shown [1] to be highly similar: - Yeast chromosome VI hypothetical protein YFL060c. - Yeast chromosome XIII hypothetical protein YMR095c. - Yeast chromosome XIV hypothetical protein YNL334c. - Bacillus subtilis hypothetical protein yaaE. - Haemophilus influenzae hypothetical protein HI1648. - Methanococcus jannaschii hypothetical protein MJ1661.These are hydrophilic proteins of about 19 to 25 Kd. They can be picked up inthe database by the following pattern. Consensus pattern: [GA]-L-I-[LIV]-P-G-G-E-S-T-[STA]

[1] Bairoch A. Unpublished observations (1997).

701. Uncharacterized protein family UPF0032 signature

The following uncharacterized proteins have been shown [1] to share regions of similarities:

- Escherichia coli hypothetical protein yigU and HI0188, the corresponding Haemophilus influenzae protein. - Bacillus subtilis hypothetical protein ycbT. - Mycobacterium tuberculosis hypothetical protein MtCY49.33c and U2126A, the corresponding Mycobacterium leprae protein. - Synechocystis strain PCC 6803 hypothetical protein s110194. - Odontella sinensis and Porphyra purpurea chlroplast hypothetical protein ycf43.These proteins have from 245 to 317 amino acids and seem to contain at least six or seven transmembrane regions. A conserved region located in the central section of these proteins has been developed as a signature pattern,.

Consensus pattern: Y-x(2)-F-[LIVMA](2)-x-L-x(4)-G-x(2)-F-[EQ]-[LIVMF]-P- [LIVM] -

[1] Bairoch A., Rudd K.E. Unpublished observations (1996).

702. Uncharacterized protein family UPF0034 signature

The following uncharacterized proteins have been shown [1] to share regions of similarities:

- Escherichia coli hypothetical protein yhdG and HI0979, the corresponding Haemophilus influenzae protein. - Escherichia coli hypothetical protein yjbN and HI0634, the corresponding Haemophilus influenzae protein. - Escherichia coli hypothetical protein yohI and HI0270, the corresponding Haemophilus influenzae protein. - Bacillus subtilis hypothetical protein yacF. - Rhodobacter capsulatus protein nifR3 and related proteins in Azospirillum brasilense and Rhizobium leguminosarum. - Synechocystis strain PCC 6803 hypothetical protein slr0644. - Synechocystis strain PCC 6803 hypothetical protein sl10926.
- Caenorhabditis elegans hypothetical protein C45G9.2. - Yeast protein SMM1. - Yeast hypothetical protein YLR401c. - Yeast hypothetical protein YLR405w. - Yeast hypothetical protein YML080w. Although it has been proposed [2] that Rhodobacter capsulatus nifR3 is a transcriptional regulatory protein, it is believed that these proteins constitute a family of enzymes whose active site could include a conserved cysteine which has been used as the central part of a signature pattern.

Consensus pattern: [LIVM]-[DNG]-[LIVM]-N-x-G-C-P-x(3)-[LIVMASQ]-x(5)-G-[SAC]

[1] Bairoch A., Rudd K.E. Unpublished observations (1995). [2] Foster-Hartnett D., Cullen P.J., Gabbert K.K., Kranz R.G. Mol. Microbiol. 8:903-914(1993).

703. Uncharacterized protein family UPF0038 signature

The following uncharacterized proteins have been shown [1] to share regions of similarities:

- Escherichia coli hypothetical protein yacE and HI0890, the corresponding Haemophilus influenzae protein. - Mycobacterium tuberculosis hypothetical protein MtCY01B2.23 and 0410, the corresponding Mycobacterium leprae protein. - Synechocystis strain PCC 6803 hypothetical protein slr0553. - Other hypothetical proteins from Aeromonas hydrophila, Bacteroides nodosus, Neisseria gonorrhoeae, Pseudomonas putida, Thermus thermophilus and Xanthomonas campestris. - Human hypothetical protein pOV-2. - Yeast hypothetical protein YDR196C. - Caenorhabditis

elegans hypothetical protein T05G5.5.These proteins all contain, in their N-terminal extremity, an ATP/GTP-binding motif 'A' (P-loop) (see <PDOC00017>). The size of these proteins range from 200 to 290 residues (with the exception of the Mycobacterial sequences which are are 410 residues long). A conseved region some 50 residues away from the ATP-binding P-loop has been developed as a signature pattern.

Consensus pattern: G-x-[LI]-x-R-x(2)-L-x(4)-F-x(8)-[LIV]-x(5)-P-x-[LIV] -

[1] Rudd K.E., Bairoch A. Unpublished observations (1997).

704. Ubiquitin-conjugating enzymes active site
Ubiquitin-conjugating enzymes (UBC or E2 enzymes) [1,2,3] catalyze the covalent attachment of ubiquitin to target proteins. An activatedubiquitin moiety is transferred from an ubiquitin-activating enzyme (E1) to E2which later ligates ubiquitin directly to substrate proteins with or without the assistance of 'N-end' recognizing proteins (E3). In most species there are many forms of UBC (at least 9 in yeast) which are implicated in diverse cellular functions. A cysteine residue is required for ubiquitin-thiolester formation. There is a single conserved cysteine in UBC's and the region around that residue isconserved in the sequence of known UBC isozymes. That region has been used as a signature pattern.
Consensus pattern: [FYWLSP]-H-[PC]-[NH]-[LIV]-x(3,4)-G-x-[LIV]-C-[LIV]-x- [LIV] [C is the active site residue]

[1] Jentsch S., Seufert W., Sommer T., Reins H.-A. Trends Biochem. Sci. 15:195-198(1990).
[2] Jentsch S., Seufert W., Hauser H.-P. Biochim. Biophys. Acta 1089:127-139(1991).
[3] Hershko A. Trends Biochem. Sci. 16:265-268(1991).

705. Uroporphyrinogen decarboxylase signatures
Uroporphyrinogen decarboxylase (URO-D), the fifth enzyme of the heme biosynthetic pathway, catalyzes the sequential decarboxylation of the four acetyl side chains of uroporphyrinogen to yield coproporphyrinogen [1].URO-D deficiency is responsible for the Human genetic diseases familialporphyria cutanea tarda (fPCT) and hepatoerythropoietic porphyria (HEP).The sequence of URO-D has been well conserved throughout evolution. The best conserved region is located in the N-terminal section; it contains a perfectlyconserved hexapeptide. There are two arginine residues in this hexapeptide which could be involved in the binding, via salt bridges, to the carboxylgroups of the propionate side chains of the substrate. This region has been used as a signature pattern. A second signature pattern is based on a another well conserved region which is located in the central section of the protein.
Consensus pattern: P-x-W-x-M-R-Q-A-G-R
Consensus pattern: G-F-[STAGCV]-[STAGC]-x-P-[FYW]-T-[LV]-x(2)-Y-x(2)-[AE]- [GK]

[1] Garey J.R., Labbe-Bois R., Chelstowska A., Rytka J., Harrison L., Kushner J., Labbe P. Eur. J. Biochem. 205: 1011-1016(1992).

706. ubiE/COQ5 methyltransferase family signatures
The following methyltransferases have been shown [1] to share regions of similarities: -
Escherichia coli ubiE, which is involved in both ubiquinone and menaquinone biosynthesis and which catalyzes the S-adenosylmethionine dependent methylation of 2-polyprenyl-6-methoxy-1,4-benzoquinol into 2-polyprenyl-3- methyl-6-methoxy-1,4-benzoquinol and of demethylmenaquinol into menaquinol. - Yeast COQ5, a ubiquinone biosynthesis methlytransferase. - Bacillus subtilis spore germination protein C2 (gene: gercB or gerC2), a probable menaquinone biosynthesis methlytransferase. - Lactococcus lactis gerC2 homolog. - Caenorhabditis elegans hypothetical protein ZK652.9. - Leishmania donovani amastigote-specific protein A41.These are hydrophilic proteins of about 30 Kd (except for ZK652.9 which is 65Kd). They can be picked up in the database by the following patterns.
Consensus pattern: Y-D-x-M-N-x(2)-[LIVM]-S-x(3)-H-x(2)-W
Consensus pattern: R-V-[LIVM]-K-[PV]-G-G-x-[LIVMF]-x(2)-[LIVM]-E-x-S

[1] Lee P.T., Hsu A.Y., Ha H.T., Clarke C.F. J. Bacteriol. 179:1748-1754(1997).

707. Uricase signature
Uricase (urate oxidase) [1] is the peroxisomal enzyme responsible for the degradation of urate into allantoin. Some species, like primates and birds, have lost the gene for uricase and are therefore unable to degradeurate. Uricase is a protein of 300 to 400 amino acids. A highly conserved region located in the central part of the sequence has been used as a signature pattern.
Consensus pattern: [LV]-x-[LV]-[LIV]-K-[STV]-[ST]-x-[SN]-x-F-x(2)-[FY]-x(4)- [FY]-x(2)-L-x(5)-R

[1] Motojima K., Kanaya S., Goto S. J. Biol. Chem. 263:16677-16681(1988).

708. Universal stress protein family (Usp)
By a wide range of stress conditions members of the Usp family are predicted to be related to the MADS-box proteins transcript fact and bind to DNA [2]. Number of members: 39

[1] Expression and role of the universal stress protein, UspA, of Escherichia coli during growth arrest. Nystrom T, Neidhardt FC; Mol Microbiol 1994; 11:537-544.
[2] Sequence analysis of eukaryotic developmental proteins: ancient and novel domains. Mushegian AR, Koonin EV; Genetics 1996; 144:817-828.

709. Ubiquitin domain signature and profile
Ubiquitin [1,2,3] is a protein of seventy six amino acid residues, found in all eukaryotic cells and whose sequence is extremely well conserved from protozoan to vertebrates. It plays a key role in a variety of cellular processes, such as ATP-dependent selective degradation of cellular proteins,maintenance of chromatin structure, regulation of gene expression, stress response and ribosome biogenesis. In most species, there are many genes coding for ubiquitin. However they can be classified into two classes. The first class produces polyubiquitin molecules consisting of exact head to tail repeats of ubiquitin. The number of repeats is variable (up to twelve in a Xenopus gene). In the majority of polyubiquitin precursors, there is a final amino-acid after the last repeat. The second class of genes produces precursor proteins consisting of a single copy of ubiquitin fused to a C-terminal extension protein (CEP). There are two types of CEP proteins and both seem to be ribosomal proteins. Ubiquitin is a globular protein, the last four C-terminal residues (Leu-Arg- Gly-Gly) extending from the compact structure to form a 'tail'; important for its function. The latter is mediated by the covalent conjugation of ubiquitin to target proteins, by an isopeptide linkage between the C-terminal glycine and the epsilon amino group of lysine residues in the target proteins. There are a number of proteins which are evolutionary related to ubiquitin: - Ubiquitin-like proteins from baculoviruses as well as in some strains of bovine viral diarrhea viruses (BVDV). These proteins are highly similar to their eukaryotic counterparts. - Mammalian protein GDX [4]. GDX is composed of two domains, a N-terminal ubiquitin-like domain of 74 residues and a C-terminal domain of 83 residues with some similarity with the thyroglobulin hormonogenic site. - Mammalian protein FAU [5]. FAU is a fusion protein which consist of a N-terminal ubiquitin-like protein of 74 residues fused to ribosomal protein S30. - Mouse protein NEDD-8 [6], a ubiquitin-like protein of 81 residues. - Human protein BAT3, a large fusion protein of 1132 residues that contains a N-terminal ubiquitin-like domain. - Caenorhabditis elegans protein ubl-1 [7]. Ubl-1 is a fusion protein which consist of a N-terminal ubiquitin-like protein of 70 residues fused to ribosomal protein S27A. - Yeast DNA repair protein RAD23 [8]. RAD23 contains a N-terminal domain that seems to be distantly, yet significantly, related to ubiquitin. - Mammalian RAD23-related proteins RAD23A and RAD23B. - Mammalian BCL-2 binding athanogene-1 (BAG-1). BAG-1 is a protein of 274 residues that contains a central ubiquitin-like domain. - Human spliceosome associated protein 114 (SAP 114 or SF3A120). - Yeast protein DSK2, a protein involved in spindle pole body duplication and which contains a N-terminal ubiquitin-like domain. - Human protein CKAP1/TFCB, Schizosaccharomyces pombe protein alp11 and Caenorhabditis elegans hypothetical protein F53F4.3. These proteins contain a N-terminal ubiquitin domain and a C-terminal CAP-Gly domain. - Schizosaccharomyces pombe hypothetical protein SpAC26A3.16. This protein contains a N-terminal ubiquitin domain. - Yeast protein SMT3. - Human ubiquitin-like proteins SMT3A and SMT3B. - Human ubiquitin-like protein SMT3C (also known as PIC1; Ubl1, Sumo-1; Gmp-1 or Sentrin). This protein is involved in targeting ranGAP1 to the nuclear pore complex protein ranBP2. - SMT3-like proteins in plants and Caenorhabditis elegans. To identify ubiquitin and related proteins, a pattern has been developed based on conserved positions in the central section of the sequence. A profile was also developed that spans the complete length of the ubiquitin domain.
Consensus pattern: K-x(2)-[LIVM]-x-[DESAK]-x(3)-[LIVM]-[PA]-x(3)-Q-x-[LIVM]-[LIVMC]-[LIVMFY]-x-G-x(4)-[DE]

[1] Jentsch S., Seufert W., Hauser H.-P. Biochim. Biophys. Acta 1089:127-139(1991).
[2] Monia B.P., Ecker D.J., Croke S.T. Bio/Technology 8:209-215(1990).
[3] Finley D., Varshavsky A. Trends Biochem. Sci. 10:343-347(1985).
[4] Filippi M., Tribioli C., Toniolo D. Genomics 7:453-457(1990).
[5] Olvera J., Wool I.G. J. Biol. Chem. 268:17967-17974(1993).
[6] Kumar S., Yoshida Y., Noda M. Biochem. Biophys. Res. Commun. 195:393-399(1993).
[7] Jones D., Candido E.P. J. Biol. Chem. 268:19545-19551(1993).
[8] Melnick L., Sherman F. J. Mol. Biol. 233:372-388(1993).

710. VHS domain
Domain present in VPS-27, Hrs and STAM. Number of members: 27
711. Vinculin family signatures

Vinculin [1] is a eukaryotic protein that seems to be involved in the attachment of the actin-based microfilaments to the plasma membrane. Vinculinis located at the cytoplasmic side of focal contacts or adhesion plaques. In addition to actin, vinculin interacts with other structural proteins such as talin and alpha-actinins. Vinculin is a large protein of 116 Kd (about a 1000 residues). Structurally the protein consists of an acidic N-terminal domain of about 90 Kd separated from a basic C-terminal domain of about 25 Kd by a proline-rich region of about 50 residues. The central part of the N-terminal domain consists of a variable number (3 in vertebrates, 2 in Caenorhabditis elegans) of repeats of a 110 amino acids domain. Catenins [2] are proteins that associate with the cytoplasmic domain of a variety of cadherins. The association of catenins to cadherins produces a complex which is linked to the actin filament network, and which seems to be of primary importance for cadherins cell-adhesion properties. Three different types of catenins seem to exist: alpha, beta, and gamma. Alpha-catenins are proteins of about 100 Kd which are evolutionary related to vinculin. Interm of their structure the most significant differences are the absence, inalpha-catenin, of the repeated domain and of the proline-rich segment. Two signature patterns for this family of proteins have been devolped. The first pattern is located in the N-terminal section of both vinculin and alpha-catenins and is part, in vinculin, of a domain that seems to be involved with the interaction with talin. The second pattern is based on a conserved regionin the N-terminal part of the repeated domain of vinculin.
Consensus pattern: [KR]-x-[LIVMF]-x(3)-[LIVMA]-x(2)-[LIVM]-x(6)-R-Q-Q-E-L
Consensus pattern: [LIVM]-x-[QA]-A-x(2)-W-[IL]-x-[DN]-P

[1] Otto J.J. Cell Motil. Cytoskeleton 16:1-6(1990).
[2] Herrenknecht K., Ozawa M., Eckerskorn C., Lottspeich F., Lenter M., Kemler R. Proc. Natl. Acad. Sci. U.S.A. 88:9156-9160(1991).

712. (Vitellogenin N) Lipoprotein amino terminal region
This family contains regions from: Vitellogenin, Microsomal triglyceride transfer protein and apolipoprotein B-100. These proteins are all involved in lipid transport [1]. This family contains the LVln chain from lipovitellin, that contains two structural domains. Number of members: 33

[1] The structural basis of lipid interactions in lipovitellin, a soluble lipoprotein. Anderson TA, Levitt DG, Banaszak LJ Structure 1998;6:895-909.

713. (VMSA) Major surface antigen from hepadnavirus
714. ssDNA binding protein (Viral DNA bp)
This protein is found in herpesviruses and is needed for replication.
715. (Votage CLC) Voltage gated chloride channels
This family of ion channels contains 10 or 12 transmembrane helices. Each protein forms a single pore. It has been shown that some members of this family form homodimers. These proteins contain two CBS domains.

[1] Schmidt-Rose T, Jentsch TJ; J Biol Chem 1997;272:20515-20521.
[2] Zhang J, George AL Jr, Griggs RC, Fouad GT, Roberts J, Kwiecinski H, Connolly AM, Ptacek LJ; Neurology 1996;47:993-998.

716. von Willebrand factor type A domain (vwa)
More von Willebrand factor type A domains? Sequence similarities with malaria thrombospondin-related anonymous protein, dihydropyridine-sensitive calcium channel and inter-alpha-trypsin inhibitor.
Bork P, Rohde K; Biochem J 1991;279:908-911.

1. RUGGERI, Z.M. and WARE, J. von Willebrand factor. FASEB J. 7 308-316 (1993).

2. COLOMBATTI, A., BONALDO, P. and DOLIANA, R. Type A modules: interacting domains found in several non-fibrillar collagens and in other extracellular matrix proteins. MATRIX 13 297-306 (1993).

3. PERKINS, S.J., SMITH, K.F., WILLIAMS, S.C., HARIS, P.I., CHAPMAN, D. and SIM, R.B. The secondary structure of the von Willebrand factor type A domain in factor B of human complement by Fourier transform infrared spectroscopy. Its occurrence in collagen types VI, VII, XII and XIV, the integrins and other proteins by averaged structure predictions. J.MOL.BIOL. 238 104-119 (1994).

4. BORK, P. and ROHDE, K. More von Willebrand factor type A domains? Sequence similarities with malaria thrombospondin-related anonymous protein, dihydropyridine-sensitive calcium channel and inter-alpha-trypsin inhibitor. BIOCHEM.J. 279 908-910 (1991).

5. EDWARDS, Y.J.K. and PERKINS, S.J. The protein fold of the von Willebrand factor type A domain is predicted to be similar to the open twisted beta-sheet flanked by alpha-helices found in human ras-p21. FEBS LETT. 358 283-286 (1995).

6. LEE, J.O., RIEU, P., ARNAOUT, M.A. and LIDDINGTON, R. Crystal structure of the A domain from the alpha subunit of integrin CR3 (CD11b/CD18). CELL 80 631-638 (1995).

7. QU, A. and LEAHY, D.J. Crystal structure of the I-domain from the CD11a/CD18 (LFA-1, alpha L beta 2) integrin. PROC.NATL.ACAD.SCI.USA 92 10277-10281 (1995).

The von Willebrand factor is a large multimeric glycoprotein found in blood plasma. Mutant forms are involved in the aetiology of bleeding disorders [1]. In von Willebrand factor, the type A domain (vWF) is the prototype for a protein superfamily. The vWF domain is found in various plasma proteins: complement factors B, C2, CR3 and CR4; the integrins (I-domains); collagen types VI, VII, XII and XIV; and other extracellular proteins [2-4]. Proteins that incorporate vWF domains participate in numerous biological events (e.g., cell adhesion, migration, homing, pattern formation, and signal transduction), involving interaction with a large array of ligands [2].

Secondary structure prediction from 75 aligned vWF sequences has revealed a largely alternating sequence of alpha-helices and beta-strands [3]. Fold recognition algorithms were used to score sequence compatibility with a library of known structures: the vWF domain fold was predicted to be a doubly-wound, open, twisted beta-sheet flanked by alpha-helices [5]. 3D structures have been determined for the I-domains of integrins CD11b (with bound magnesium) [6] and CD11a (with bound manganese) [7]. The domain adopts a classic alpha/beta Rossmann fold and contains an unusual metal ion coordination site at its surface. It has been suggested that this site represents a general metal ion-dependent adhesion site (MIDAS) for binding protein ligands [6]. The residues constituting the MIDAS motif in the CD11b and CD 11 a I-domains are completely conserved, but the manner in which the metal ion is coordinated differs slightly [7].

VWFADOMAIN is a 3-element fingerprint that provides a signature for the vWF domain superfamily. The fingerprint was derived from an initial alignment of 14 sequences. Motif 1 includes the first beta-strand and 3 conserved residues involved in metal ion coordination in I-domains (Asp and 2 serines in positions 8, 10 and 12, respectively); motif 2 spans strands beta-2 and beta-2'; and motif 3 encodes beta-strand 3 and a conserved Asp (in position 7), which coordinates the metal ion [6,7]. Three iterations on OWL27.0 were required to reach convergence, at which point a true set comprising 56 sequences was identified. Numerous partial matches were also found.

717. (WD40) WD domain, G-beta repeat

The ancient regulatory-protein family of WD-repeat proteins.

Neer EJ, Schmidt CJ, Nambudripad R, Smith TF; Nature 1994;371:297-300.

Beta-transducin (G-beta) is one of the three subunits (alpha, beta, and gamma) of the guanine nucleotide-binding proteins (G proteins) which act as intermediaries in the transduction of signals generated by transmembrane receptors [1]. The alpha subunit binds to and hydrolyzes GTP; the functions of the beta and gamma subunits are less clear but they seem to be required for the replacement of GDP by GTP as well as for membrane anchoring and receptor recognition.

In higher eukaryotes G-beta exists as a small multigene family of highly conserved proteins of about 340 amino acid residues. Structurally G-beta consists of eight tandem repeats of about 40 residues, each containing a central Trp-Asp motif (this type of repeat is sometimes called a WD-40 repeat). Such a repetitive segment has been shown [E1,2,3,4,5] to exist in a number of other proteins listed below:

- Yeast STE4, a component of the pheromone response pathway. STE4 is a G-beta like protein that associates with GPA1 (G-alpha) and STE18 (G-gamma).
- Yeast MSI1, a negative regulator of RAS-mediated cAMP synthesis. MSI1 is most probably also a G-beta protein.

- Human and chicken protein 12.3. The function of this protein is not known, but on the basis of its similarity to G-beta proteins, it may also function in signal transduction.
- Chlamydomonas reinhardtii gblp. This protein is most probably the homolog of vertebrate protein 12.3.
- Human LIS1, a neuronal protein involved in type-1 lissencephaly [E2].
- Mammalian coatomer beta' subunit (beta'-COP), a component of a cytosolic protein complex that reversibly associates with Golgi membranes to form vesicles that mediate biosynthetic protein transport.

- Yeast CDC4, essential for initiation of DNA replication and separation of the spindle pole bodies to form the poles of the mitotic spindle.
- Yeast CDC20, a protein required for two microtubule-dependent processes: nuclear movements prior to anaphase

and chromosome separation.

- Yeast MAK11, essential for cell growth and for the replication of M1 double-stranded RNA.
- Yeast PRP4, a component of the U4/U6 small nuclear ribonucleoprotein with a probable role in mRNA splicing.
- Yeast PWP1, a protein of unknown function.
- Yeast SK18, a protein essential for controlling the propagation of double-stranded RNA.
- Yeast SOF1, a protein required for ribosomal RNA processing which associates with U3 small nucleolar RNA.
- Yeast TUP1 (also known as AER2 or SFL2 or CYC9), a protein which has been implicated in dTMP uptake, catabolite repression, mating sterility, and many other phenotypes.
- Yeast YCR57c, an ORF of unknown function from chromosome III.
- Yeast YCR72c, an ORF of unknown function from chromosome III.

- Slime mold coronin, an actin-binding protein.
- Slime mold AAC3, a developmentally regulated protein of unknown function.

- Drosophila protein Groucho (formerly known as E(spl); 'enhancer of split'), a protein involved in neurogenesis and that seems to interact with the Notch and Delta proteins.
- Drosophila TAF-II-80, a protein that is tightly associated with TFIID.

The number of repeats in the above proteins varies between 5 (PRP4, TUP1, and Groucho) and 8 (G-beta, STE4, MSI1, AAC3, CDC4, PWP1, etc.). In G-beta and G-beta like proteins, the repeats span the entire length of the sequence, while in other proteins, they make up the N-terminal, the central or the C-terminal section.
A signature pattern can be developed from the central core of the domain (positions 9 to 23).

- Consensus pattern: [LIVMSTAC]-[LIVMFYWSTAGC]-[LIMSTAG]-[LIVMSTAGC]-x(2)-[DN]-x(2)-[LIVMWSTAC]-x-[LIVMFSTAG]-W-[DEN]-[LIVMFSTAGCN]

[1] Gilman A. G. Annu. Rev. Biochem. 56:615-649(1987).
[2] Duronio R.J., Gordon J.I., Boguski M.S. Proteins 13:41-56(1992).
[3] van der Voorn L., Ploegh H.L. FEBS Lett. 307:131-134(1992).
[4] Neer E.J., Schmidt C.J., Nambudripad R., Smith T.F. Nature 371:297-300(1994).
[5] Smith T.F., Gaiatzes C.G., Saxena K., Neer E.J. Biochemistry In Press(1998).

718. WHEP-TRS domain containing proteins
A conserved domain of 46 amino acids has been shown [1] to exist in a number of higher eukaryote aminoacyl-transfer RNA synthetases. This domain is present one to six times in the following enzymes:

- Mammalian multifunctional aminoacyl-tRNA synthetase. The domain is present three times in a region that separates the N-terminal glutamyl-tRNA synthetase domain from the C-terminal prolyl-tRNA synthetase domain.
- Drosophila multifunctional aminoacyl-tRNA synthetase. The domain is present six times in the intercatalytic region.
- Mammalian tryptophanyl-tRNA synthetase. The domain is found at the N-terminal extremity.
- Mammalian, insect, nematode and plant glycyl-tRNA synthetase. The domain is found at the N-terminal extremity [2].
- Mammalian histidyl-tRNA synthetase. The domain is found at the N-terminal extremity.

This domain, which is called WHEP-TRS, could contain a central alpha-helical region and may play a role in the association oftRNA-synthetases into multienzyme complexes.
A signature pattern based on the first 29 positions of the WHEP-Domain has been developed.

- Consensus pattern: [QY]-G-[DNEA]-x-[LIV]-[KR]-x(2)-K-x(2)-[KRNG]-[AS]-x(4)-[LIV]-[DENK]-x(2)-[IV]-x(2)-L-x(3)-K

[1] Cerini C., Kerjan P., Astier M., Gratecos D., Mirande M., Semeriva M. EMBO J. 10:4267-4277(1991).
[2] Nada S., Chang P.K., Dignam J.D. J. Biol. Chem. 268:7660-7667(1993).

719. (Worm family 8) Putative membrane protein
Analysis of protein domain families in Caenorhabditis elegans.
Sonnhammer EL, Durbin R; Genomics 1997;46:200-216.
This family called family 8 in [1], may be a transmembrane protein
The specific function of this protein is unknown.
720. Xylose isomerase

Xylose isomerase (EC 5.3.1.5) [1] is an enzyme found in microorganisms which catalyzes the interconversion of D-xylose to D-xylulose. It can also isomerize D-ribose to D-ribulose and D-glucose to D-fructose. Xylose isomerase seems to require magnesium for its activity, while cobalt is necessary to stabilize the tetrameric structure of the enzyme. A number of residues are conserved in all known xylose isomerases.
Xylose isomerase also exists in plants [2] where it is homodimeric and is manganese-dependent.
Two signatures patterns for xylose isomerase have been developed. The first one is derived from a stretch of five conserved amino acids that includes a glutamic acid residue known to be one of the four residues involved in the binding of the magnesium ion [3]; this pattern also includes a lysine residue which is involved in the catalytic activity. The second pattern is derived from a conserved region in the N-terminal section of the enzyme that include an histidine residue which has been shown [4] to be involved in the catalytic mechanism of the enzyme.

- Consensus pattern: [LI]-E-P-K-P-x(2)-P
  [E is a magnesium ligand]
  [K is an active site residue]
- Consensus pattern: [FL]-H-D-x-D-[LIV]-x-[PD]-x-[GDE]
  [H is an active site residue]

[1] Dauter Z., Dauter M., Hemker J., Witzel H., Wilson K.S. FEBS Lett. 247:1-8(1989).
[2] Kristo P.A., Saarelainen R., Fagerstrom R., Aho S., Korhola M. Eur. J. Biochem. 237:240-246(1996).
[3] Henrick K., Collyer C.A., Blow D.M. J. Mol. Biol. 208:129-157(1989).
[4] Vangrysperre W., Ampe C., Kersters-Hilderson H., Tempst P. Biochem. J. 263:195-199(1989).

721. XPG protein signatures. Xeroderma pigmentosum (XP) [1] is a human autosomal recessive disease, characterized by a high incidence of sunlight-induced skin cancer. People's skin cells with this condition are hypersensitive to ultraviolet light, due to defects in the incision step of DNA excision repair. There are a minimum of seven genetic complementation groups involved in this pathway: XP-A to XP-G. The defect in XP-G can be corrected by a 133 Kd nuclear protein called XPG (or XPGC) [2].XPG belongs to a family of proteins [2,3,4,5,6] that are composed of twomain subsets: - Subset 1, to which belongs XPG, RAD2 from budding yeast and rad13 from fission yeast. RAD2 and XPG are single-stranded DNA endonucleases [7,8]. XPG makes the 3'incision in human DNA nucleotide excision repair [9]. - Subset 2, to which belongs mouse and human FEN-1, rad2 from fission yeast, and RAD27 from budding yeast. FEN-1 is a structure-specific endonuclease. In addition to the proteins listed in the above groups, this family also includes: - Fission yeast exo1, a 5'->3' double-stranded DNA exonuclease that could act in a pathway that corrects mismatched base pairs. - Yeast EXO1 (DHS1), a protein with probably the same function as exo1. - Yeast DIN7. Sequence alignment of this family of proteins reveals that similarities are largely confined to two regions. The first is located at the N-terminal extremity (N-region) and corresponds to the first 95 to 105 amino acids. The second region is internal (I-region) and found towards the C-terminus; it spans about 140 residues and contains a highly conserved core of 27 amino acids that includes a conserved pentapeptide (E-A-[DE]-A-[QS]). It is possible that the conserved acidic residues are involved in the catalytic mechanism of DNA excision repair in XPG. The amino acids linking the N- and I-regions are not conserved; indeed, they are largely absent from proteins belonging to the second subset. Two signature patterns have been developed for these proteins. The first corresponds to the central part of the N-region, the second to part of the I-region and includes the putative catalytic core pentapeptide
Consensus pattern: [VI]-[KRE]-P-x-[FYIL]-V-F-D-G-x(2)-[PIL]-x-[LVC]-K-
Consensus pattern: [GS]-[LIVM]-[PER]-[FYS]-[LIVM]-x-A-P-x-E-A-[DE]-[PAS]- [QS]-[CLM]-

[1] Tanaka K., Wood R.D. Trends Biochem. Sci. 19:83-86(1994).
[2] Scherly D., Nouspikel T., Corlet J., Ucla C., Bairoch A., Clarkson S.G. Nature 363:182-185(1993).
[3] Carr A.M., Sheldrick K.S., Murray J.M., Al-Harithy R., Watts F.Z., Lehmann A.R. Nucleic Acids Res. 21:1345-1349 (1993).
[4] Murray J.M., Tavassoli M., Al-Harithy R., Sheldrick K.S., Lehmann A.R., Carr A.M., Watts F.Z. Mol. Cell. Biol. 14:4878-4888(1994).
[5] Harrington J.J., Lieber M.R. Genes Dev. 8:1344-1355(1994).
[6] Szankasi P., Smith G.R. Science 267:1166-1169(1995).
[7] Habraken Y., Sung P., Prakash L., Prakash S. Nature 366:365-368(1993).
[8] O'Donovan A., Scherly D., Clarkson S.G., Wood R.D. J. Biol. Chem. 269:15965-15968(1994).
[9] O'Donovan A., Davies A.A., Moggs J.G., West S.C., Wood R.D. Nature 371:432-435(1994).

722. Xanthine/uracil permeases family
The following transport proteins which are involved in the uptake of xanthine or uracil are evolutionary related [1]:

- Uric uric acid-xanthine permease (gene uapA) from Aspergillus nidulans.
- Purine permease (gene uapC) from Aspergillus nidulans.
- Xanthine permease from Bacillus subtilis (gene pbuX).
- Uracil permease from Escherichia coli (gene uraA) [2] and Bacillus (gene pYrP).
- Hypothetical protein ycdG from Escherichia coli.
- Hypothetical protein ygfO from Escherichia coli.
- Hypothetical protein ygfU from Escherichia coli.
- Hypothetical protein yicE from Escherichia coli.
- Hypothetical protein yunJ from Bacillus subtilis.
- Hypothetical protein yunK from Bacillus subtilis.

They are proteins of from 430 to 595 residues that seem to contain 12 transmembrane domains.
The best conserved region which corresponds with what seems to be the tenth transmembrane domain has been selected as a signature pattern.

- Consensus pattern: [LIVM]-P-x-[PASIF]-V-[LIVM]-G-G-x(4)-[LIVM]-[FY]-[GSA]-x-[LIVM]-x(3)-G

[1] Diallinas G., Gorfinkiel L., Arst G., Cecchetto G., Scazzocchio C. J. Biol. Chem. 270:8610-8622(1995).
[2] Andersen P.S., Frees D., Fast R., Mygind B. J. Bacteriol. 177:2008-2013(1995).

723. Hypothetical yabO/yceC/sfhB family
The following proteins, which seems to belong to a family of pseudouridine synthases (EC 4.2.1.70) [1] have been shown to share regions of similarities:

- Escherichia coli and Haemophilus influenzae ribosomal large subunit pseudouridine synthase A (gene rluA). It is responsible for synthesis of pseudouridine from uracil-746 IN 23S rRNA.
- Escherichia coli and Haemophilus influenzae ribosomal large subunit pseudouridine synthase C (gene rluC). It is responsible for synthesis of pseudouridine from uracil at positions 955, 2504 and 2580 in 23S rRNA.
- Escherichia coli protein and homologs in other bacteria large subunit pseudouridine synthase D (gene rluD).
- Yeast DRAP deaminase (gene RIB2).
- Escherichia coli hypothetical protein yqcB and HI1435, the corresponding Haemophilus influenzae protein.
- Haemophilus influenzae hypothetical protein HI0042.
- Aquifex aeolicus hypothetical protein AQ_1758.
- Bacillus subtilis hypothetical protein yhcT.
- Bacillus subtilis hypothetical protein yjbO.
- Bacillus subtilis hypothetical protein ylyB.
- Helicobacter pylori hypothetical protein HP0347.
- Helicobacter pylori hypothetical protein HP0745.
- Helicobacter pylori hypothetical protein HP0956.
- Mycoplasma genitalium hypothetical protein MG209.
- Mycoplasma genitalium hypothetical protein MG370.
- Synechocystis strain PCC 6803 hypothetical protein s1r1592.
- Synechocystis strain PCC 6803 hypothetical protein s1r1629.
- Yeast hypothetical protein YDL036c.
- Yeast hypothetical protein YGR169c.
- Fission yeast hypothetical protein SpAC18B11.02c.
- Caenorhabditis elegans hypothetical protein K07E8.7.

These are proteins of from 21 to 50 Kd which contain a number of conserved regions in their central section. They can be picked up in the database by the following highly conserved pattern.
-Consensus pattern: [LIVCA]-[NHYT]-R-[LI]-D-x(2)-T-[STA]-G-[LIVAGC]-[LIVMF](2)-[LIVMFGC]-[SGTACV]

[1] Conrad J., Sun D., Englund N., Ofengand J. J. Biol. Chem. 273:18562-18566(1998).

In addition, the following bacterial proteins, which seems to belong to a family of pseudouridine synthases (EC 4.2.1.70) [1] also have been shown to share regions of similarities:

- Escherichia coli and Haemophilus influenzae 16S pseudouridylate 516 synthase (EC 4.2.1.70) (gene: rsuA). This

enzyme is responsible for the formation of pseudouridine from uracil-516 in 16S ribosomal RNA.

- Escherichia coli hypothetical protein yciL and HI1199, the corresponding Haemophilus influenzae protein.
- Escherichia coli hypothetical protein yjbC.
- Escherichia coli hypothetical protein ymfC and HI0694, the corresponding Haemophilus influenzae protein.
- Aquifex aeolicus hypothetical protein AQ_554.
- Aquifex aeolicus hypothetical protein AQ_1464.
- Bacillus subtilis hypothetical protein ypuL.
- Bacillus subtilis hypothetical protein ytzF.
- Borrelia burgdorferi hypothetical protein BB0129.
- Helicobacter pylori hypothetical protein HP1459.
- Synechocystis strain PCC 6803 hypothetical protein s1r0361.
- Synechocystis strain PCC 6803 hypothetical protein s1r0612.

These are proteins of from 25 to 40 Kd which contain a number of conserved regions in their central section. They can be picked up in the database by the following highly conserved pattern.

- Consensus pattern: G-R-L-D-x(2)-[STA]-x-G-[LIVFA]-[LIVMF](3)-[ST]-[DNST]

[1] Wrzesinski J., Bakin A., Nurse K., Lane B.G., Ofengand J. Biochemistry 34:8904-8913(1995).

724. Zinc finger present in dystrophin, CBP/p300
ZZ in dystrophin binds calmodulin
Putative zinc finger; binding not yet shown.
725. Zinc carboxypeptidase
There are a number of different types of zinc-dependent carboxypeptidases (EC 3.4.17.-) [1,2]. All these enzymes seem to be structurally and functionally related. The enzymes that belong to this family are listed below.

- Carboxypeptidase A1 (EC 3.4.17.1), a pancreatic digestive enzyme that can removes all C-terminal amino acids with the exception of Arg, Lys and Pro.
- Carboxypeptidase A2 (EC 3.4.17.15), a pancreatic digestive enzyme with a specificity similar to that of carboxypeptidase A1, but with a preference for bulkier C-terminal residues.
- Carboxypeptidase B (EC 3.4.17.2), also a pancreatic digestive enzyme, but that preferentially removes C-terminal Arg and Lys.
- Carboxypeptidase N (EC 3.4.17.3) (also known as arginine carboxypeptidase), a plasma enzyme which protects the body from potent vasoactive and inflammatory peptides containing C-terminal Arg or Lys (such as kinins or anaphylatoxins) which are released into the circulation.
- Carboxypeptidase H (EC 3.4.17.10) (also known as enkephalin convertase or carboxypeptidase E), an enzyme located in secretory granules of pancreatic islets, adrenal gland, pituitary and brain. This enzyme removes residual C-terminal Arg or Lys remaining after initial endoprotease cleavage during prohormone processing.
- Carboxypeptidase M (EC 3.4.17.12), a membrane bound Arg and Lys specific enzyme.
  It is ideally situated to act on peptide hormones at local tissue sites where it could control their activity before or after interaction with specific plasma membrane receptors.
- Mast cell carboxypeptidase (EC 3.4.17.1), an enzyme with a specificity to carboxypeptidase A, but found in the secretory granules of mast cells.
- Streptomyces griseus carboxypeptidase (Cpase SG) (EC 3.4.17.-) [3], which combines the specificities of mammalian carboxypeptidases A and B.
- Thermoactinomyces vulgaris carboxypeptidase T (EC 3.4.17.18) (CPT) [4], which also combines the specificities of carboxypeptidases A and B.
- AEBP1 [5], a transcriptional repressor active in preadipocytes. AEBP1 seems to regulate transcription by cleavage of other transcriptional proteins.
- Yeast hypothetical protein YHR132c.

All of these enzymes bind an atom of zinc. Three conserved residues are implicated in the binding of the zinc atom: two histidines and a glutamic acid Two signature patterns which contain these three zinc-ligands have been derived.

- Consensus pattern: [PK]-x-[LIVMFY]-x-[LIVMFY]-x(4)-H-[STAG]-x-E-x-[LIVM]-[STAG]-x(6)-[LIVMFYTA]
  [H and E are zinc ligands]
- Consensus pattern: H-[STAG]-x(3)-[LIVME]-x(2)-[LIVMFYW]-P-[FYW]

[H is a zinc ligand]

[1] Tan F., Chan S.J., Steiner D.F., Schilling J.W., Skidgel R.A. J. Biol. Chem. 264:13165-13170(1989).
[2] Reynolds D.S., Stevens R.L., Gurley D.S., Lane W.S., Austen K.F., Serafin W.E. J. Biol. Chem. 264:20094-20099 (1989).
[3] Narahashi Y. J. Biochem. 107:879-886(1990).
[4] Teplyakov A., Polyakov K., Obmolova G., Strokopytov B., Kuranova I., Osterman A.L., Grishin N.V., Smulevitch S.V., Zagnitko O.P., Galperina O.V., Matz M.V., Stepanov V.M. Eur. J. Biochem. 208:281-288(1992).
[5] He G.-P., Muise A., Li A.W., Ro H.-S. Nature 378:92-96(1995).
[6] Hourdou M.-L., Guinand M., Vacheron M.J., Michel G., Denoroy L., Duez C.M., Englebert S., Joris B., Weber G., Ghuysen J.-M. Biochem. J. 292:563-570(1993).
[7] Rawlings N.D., Barrett A.J. Meth. Enzymol. 248:183-228(1995).

726. Zinc finger, C2H2 type

The C2H2 zinc finger is the classical zinc finger domain.
The two conserved cysteines and histidines co-ordinate a zinc ion. The following pattern describes the zinc finger. #-X-C-X(1-5)-C-X3-#-X5-#-X2-H-X(3-6)-[H/C]
Where X can be any amino acid, and numbers in brackets indicate the number of residues. The positions marked # are those that are important for the stable fold of the zinc finger. The final position can be either his or cys.
The C2H2 zinc finger is composed of two short beta strands followed by an alpha helix. The amino terminal part of the helix binds the major groove in DNA binding zinc fingers.
'Zinc finger' domains [1-5] are nucleic acid-binding protein structures first identified in the Xenopus transcription factor TFIIIA. These domains have since been found in numerous nucleic acid-binding proteins. A zinc finger domain is composed of 25 to 30 amino-acid residues. There are two cysteine or histidine residues at both extremities of the domain, which are involved in the tetrahedral coordination of a zinc atom. It has been proposed that such a domain interacts with about five nucleotides. A schematic representation of a zinc finger domain is shown below:

```
  x x
  x   x
 x x
 x x
 x x
 x x
  C   H
 x  \ /  x
  x  Zn  x
 x /   \ x
  C   H
  x x x x x  x x x x x
```

Many classes of zinc fingers are characterized according to the number and positions of the histidine and cysteine residues involved in the zinc atom coordination. In the first class to be characterized, called C2H2, the first pair of zinc coordinating residues are cysteines, while the second pair are histidines. A number of experimental reports have demonstrated the zinc-dependent DNA or RNA binding property of some members of this class.
Some of the proteins known to include C2H2-type zinc fingers are listed below. The number of zinc finger regions found in each of these proteins are indicated between brackets; a '+' symbol indicates that only partial sequence data is available and that additional finger domains may be present.

- Saccharomyces cerevisiae: ACE2 (3), ADR1 (2), AZF1 (4), FZFI (5), MIG1 (2), MSN2 (2), MSN4 (2), RGM1 (2),

RIM1 (3), RME1 (3), SFP1 (2), SSL1 (1), STP1 (3), SWI5 (3), VAC1 (1) and ZMS1 (2).

- Emericella nidulans: brlA (2), creA (2).
- Drosophila: AEF-1 (4), Cf2 (7), ci-D (5), Disconnected (2), Escargot (5), Glass (5), Hunchback (6), Kruppel (5), Kruppel-H (4+), Odd-skipped (4), Odd-paired (4), Pep (3), Snail (5), Spalt-major (7), Serependity locus beta (6), delta (7), h-1 (8), Suppressor of hairy wing su(Hw) (12), Suppressor of variegation suvar(3)7 (5), Teashirt (3) and Tramtrack (2).
- Xenopus: transcription factor TFIIIA (9), p43 from RNP particle (9), Xfin (37 ! !), Xsna (5), gastrula XlcGF5.1 to XlcGF71.1 (from 4+ to 11+), Oocyte XlcOF2 to XlcOF22 (from 7 to 12).
- Mammalian: basonuclin (6), BCL-6/LAZ-3 (6), erythroid krueppel-like transcription factor (3), transcription factors Sp1 (3), Sp2 (3), Sp3 (3) and Sp(4) 3, transcriptional repressor YY1 (4), Wilms'tumor protein (4), EGR1/Krox24 (3), EGR2/Krox20 (3), EGR3/Pilot (3), EGR4/AT133 (4), Evi-1 (10), GLI1 (5), GLI2 (4+), GLI3 (3+), HIV-EP1/ZNF40 (4), HIV-EP2 (2), KR1 (9+), KR2 (9), KR3 (15+), KR4 (14+), KR5 (11+), HF.12 (6+), REX-1 (4), ZfX (13), ZfY (13), Zfp-35 (18), ZNF7 (15), ZNF8 (7), ZNF35 (10), ZNF42/MZF-1 (13), ZNF43 (22), ZNF46/Kup (2), ZNF76 (7), ZNF91 (36), ZNF133 (3).

In addition to the conserved zinc ligand residues it has been shown [6] that a number of other positions are also important for the structural integrity of the C2H2 zinc fingers. The best conserved position is found four residues after the second cysteine; it is generally an aromatic or aliphatic residue.

- Consensus pattern: C-x(2,4)-C-x(3)-[LIVMFYWC]-x(8)-H-x(3,5)-H
  [The two C's and two H's are zinc ligands]

[1] Klug A., Rhodes D. Trends Biochem. Sci. 12:464-469(1987).
[2] Evans R.M., Hollenberg S.M. Cell 52:1-3(1988).
[3] Payre F., Vincent A. FEBS Lett. 234:245-250(1988).
[4] Miller J., McLachlan A.D., Klug A. EMBO J. 4:1609-1614(1985).
[5] Berg J.M. Proc. Natl. Acad. Sci. U.S.A. 85:99-102(1988).
[6] Rosenfeld R., Margalit H. J. Biomol. Struct. Dyn. 11:557-570(1993).

727. Zinc finger, C3HC4 type (RING finger)
A number of eukaryotic and viral proteins contain a conserved cysteine-rich domain of 40 to 60 residues (called C3HC4 zinc-finger or 'RING' finger) [1] that binds two atoms of zinc, and is probably involved in mediating protein-protein interactions. The 3D structure of the zinc ligation system is unique to the RING domain and is refered to as the "cross-brace" motif. The spacing of the cysteines in such a domain is C-x(2)-C-x(9 to 39)-C-x(1 to 3)-H-x(2 to 3)-C-x(2)-C-x(4 to 48)-C-x(2)-C.
Proteins currently known to include the C3HC4 domain are listed below (references are only provided for recently determined sequences).

- Mammalian V(D)J recombination activating protein (gene RAG1). RAG1 activates the rearrangement of immunoglobulin and T-cell receptor genes.
- Mouse rpt-1. Rpt-1 is a trans-acting factor that regulates gene expression directed by the promoter region of the interleukin-2 receptor alpha chain or the LTR promoter region of HIV-1.
- Human rfp. Rfp is a developmentally regulated protein that may function in male germ cell development. Recombination of the N-terminal section of rfp with a protein tyrosine kinase produces the ret transforming protein.
- Human 52 Kd Ro/SS-A protein. A protein of unknown function from the Ro/SS-A ribonucleoprotein complex. Sera from patients with systemic lupus erythematosus or primary Sjogren's syndrome often contain antibodies that react with the Ro proteins.
- Human histocompatibility locus protein RING1.
- Human PML, a probable transcription factor. Chromosomal translocation of PML with retinoic receptor alpha creates a fusion protein which is the cause of acute promyelocytic leukemia (APL).
- Mammalian breast cancer type 1 susceptibility protein (BRCA1) [E1].
- Mammalian cbl proto-oncogene.
- Mammalian bmi-1 proto-oncogene.
- Vertebrate CDK-activating kinase (CAK) assembly factor MAT1, a protein that stabilizes the complex between the CDK7 kinase and cyclin H (MAT1 stands for Menage A Trois').
- Mammalian mel-18 protein. Mel-18 which is expressed in a variety of tumor cells is a transcriptional repressor that recognizes and bind a specific DNA sequence.
- Mammalian peroxisome assembly factor-1 (PAF-1) (PMP35), which is somewhat involved in the biogenesis of

peroxisomes. In humans, defects in PAF-1 are responsible for a form of Zellweger syndrome, an autosomal recessive disorder associated with peroxisomal deficiencies.

- Human MAT1 protein, which interacts with the CDK7-cyclin H complex.
- Human RING1 protein.
- Xenopus XNF7 protein, a probable transcription factor.
- Trypanosoma protein ESAG-8 (T-LR), which may be involved in the postranscriptional regulation of genes in VSG expression sites or may interact with adenylate cyclase to regulate its activity.
- Drosophila proteins Posterior Sex Combs (Psc) and Suppressor two of zeste (Su(z)2). The two proteins belong to the Polycomb group of genes needed to maintain the segment-specific repression of homeotic selector genes.
- Drosophila protein male-specific msl-2, a DNA-binding protein which is involved in X chromosome dosage compensation (the elevation of transcription of the male single X chromosome).
- Arabidopsis thaliana protein COP1 which is involved in the regulation of photomorphogenesis.
- Fungal DNA repair proteins RAD5, RAD16, RAD18 and rad8.
- Herpesviruses trans-acting transcriptional protein ICP0/IE110. This protein which has been characterized in many different herpesviruses is a transactivator and/or -repressor of the expression of many viral and cellular promoters.
- Baculoviruses protein CG30.
- Baculoviruses major immediate early protein (PE-38).
- Baculoviruses immediate-early regulatory protein IE-N/IE-2.
- Caenorhabditis elegans hypothetical proteins F54G8.4, R05D3.4 and T02C1.1.
- Yeast hypothetical proteins YER116c and YKR017c.

The central region of the domain was selected as a signature pattern for the C3HC4 finger.

- Consensus pattern: C-x-H-x-[LIVMFY]-C-x(2)-C-[LIVMYA]

[1] Borden K.L.B., Freemont P.S. Curr. Opin. Struct. Biol. 6:395-401(1996).

728. Zinc finger C-x8-C-x5-C-x3-H type (and similar).
729. Zinc finger, CCHC class
A family of CCHC zinc fingers, mostly from retroviral gag proteins (nucleocapsid). Prototype structure is from HIV. Also contains members involved in eukaryotic gene regulation, such as C. elegans GLH-1.
Structure is an 18-residue zinc finger; no examples of indels in the alignment.
730. Zn-finger in Ran binding protein and others.
731. AN1-like Zinc finger
Zinc finger at the C-terminus of An1 Swiss:Q91889, a ubiquitin-like protein in Xenopus laevis. The following pattern describes the zinc finger. C-X2-C-X(9-12)-C-X(1-2)-C-X4-C-X2-H-X5-H-X-C Where X can be any amino acid, and numbers in brackets indicate the number of residues.

[1] Linnen JM, Bailey CP, Weeks DL; Gene 1993;128:181-188.

732. 14-3-3 proteins
Structure of a 14-3-3 protein and implications for coordination of multiple signalling pathways.
Xiao B, Smerdon SJ, Jones DH, Dodson GG, Soneji Y, Aitken A, Gamblin SJ; Nature 1995;376:188-191.
Crystal structure of the zeta isoform of the 14-3-3 protein.
Liu D, Bienkowska J, Petosa C, Collier RJ, Fu H, Liddington R; Nature 1995;376:191-194.
Interaction of 14-3-3 with signaling proteins is mediated by the recognition of phosphoserine.
Muslin AJ, Tanner JW, Allen PM, Shaw AS; Cell 1996;84:889-897.
The 14-3-3 protein binds its target proteins with a common site located towards the C-terminus.
Ichimura T, Ito M, Itagaki C, Takahashi M, Horigome T, Omata S, Ohno S, Isobe T FEBS Lett 1997;413:273-276.
Molecular evolution of the 14-3-3 protein family.
Wang W, Shakes DC J Mol Evol 1996;43:384-398.
Function of 14-3-3 proteins.
Jin DY, Lyu MS, Kozak CA, Jeang KT Nature 1996;382:308-308.
The 14-3-3 proteins [1,2,3] are a family of closely related acidic homodimeric proteins of about 30 Kd which were first identified as being very abundant in mammalian brain tissues and located preferentially in neurons. The 14-3-3 proteins seem to have multiple biological activities and play a key role in signal transduction pathways and the cell cycle. They interacts with kinases such as PKC or Raf-1; they seem to also function as protein-kinase dependent activators of tyrosine and tryptophan hydroxylases and in plants they are associated with a complex that binds to the G-box promoter

elements.

The 14-3-3 family of proteins are ubiquitously found in all eukaryotic species studied and have been sequenced in fungi (yeast BMH1 and BMH2, fission yeast rad24 and rad25), plants, Drosophila, and vertebrates. The sequences of the 14-3-3 proteins are extremely well conserved. Two highly conserved regions have been selected as signature patterns: the first is a peptide of 11 residues located in the N-terminal section; the second, a 20 amino acid region located in the C-terminal section.

- Consensus pattern: R-N-L-[LIV]-S-[VG]-[GA]-Y-[KN]-N-[IVA]
- Consensus pattern: Y-K-[DE]-S-T-L-I-[IM]-Q-L-[LF]-[RHC]-D-N-[LF]-T-[LS]-W-[TAN]-[SAD]

[1] Aitken A. Trends Biochem. Sci. 20:95-97(1995).
[2] Morrison D. Science 266:56-57(1994).
[3] Xiao B., Smerdon S.J., Jones D.H., Dodson G.G., Soneji Y., Aitken A., Gamblin S.J. Nature 376:188-191(1995).

733. D-isomer specific 2-hydroxyacid dehydrogenases (2 Hacid DH)

This Pfam covers the Formate dehydrogenase, D-glycerate dehydrogenase and D-lactate dehydrogenase families in SCOP. A number of NAD-dependent 2-hydroxyacid dehydrogenases which seem to be specific for the D-isomer of their substrate have been shown [1,2,3,4] to be functionally and structurally related. These enzymes are listed below.

- D-lactate dehydrogenase (EC 1.1.1.28), a bacterial enzyme which catalyzes the reduction of D-lactate to pyruvate.
- D-glycerate dehydrogenase (EC 1.1.1.29) (NADH-dependent hydroxypyruvate reductase), a plant leaf peroxisomal enzyme that catalyzes the reduction of hydroxypyruvate to glycerate. This reaction is part of the glycolate pathway of photorespiration.
- D-glycerate dehydrogenase from the bacteria Hyphomicrobium methylovorum and Methylobacterium extorquens.
- 3-phosphoglycerate dehydrogenase (EC 1.1.1.95), a bacterial enzyme that catalyzes the oxidation of D-3-phosphoglycerate to 3-phosphohydroxypyruvate. This reaction is the first committed step in the 'phosphorylated' pathway of serine biosynthesis.
- Erythronate-4-phosphate dehydrogenase (EC 1.1.1.-) (gene pdxB), a bacterial enzyme involved in the biosynthesis of pyridoxine (vitamin B6).
- D-2-hydroxyisocaproate dehydrogenase (EC 1.1.1.-) (D-hicDH), a bacterial enzyme that catalyzes the reversible and stereospecific interconversion between 2-ketocarboxylic acids and D-2-hydroxy-carboxylic acids.
- Formate dehydrogenase (EC 1.2.1.2) (FDH) from the bacteria Pseudomonas sp. 101 and various fungi [5].
- Vancomycin resistance protein vanH from Enterococcus faecium; this protein is a D-specific alpha-keto acid dehydrogenase involved in the formation of a peptidoglycan which does not terminate by D-alanine thus preventing vancomycin binding.
- Escherichia coli hypothetical protein ycdW.
- Escherichia coli hypothetical protein yiaE.
- Haemophilus influenzae hypothetical protein HI1556.
- Yeast hypothetical protein YER081 w.
- Yeast hypothetical protein YIL074w.

All these enzymes have similar enzymatic activities and are structurally related. Three of the most conserved regions of these proteins have been selected to develop patterns. The first pattern is based on a glycine-rich region located in the central section of these enzymes; this region probably corresponds to the NAD-binding domain. The two other patterns contain a number of conserved charged residues, some of which may play a role in the catalytic mechanism.

- Consensus pattern: [LIVMA]-[AG]-[IVT]-[LIVMFY]-[AG]-x-G-[NHKRQGSAC]-[LIV]-G-x(13,14)-[LIVfMT]-x(2)-[FYwCTH]-[DNSTK]
- Consensus pattern: [LIVMFYWA]-[LIVFYWC]-x(2)-[SAC]-[DNQHR]-[IVFA]-[LIVF]-x-[LIVF]-[HNI]-x-P-x(4)-[STN]-x(2)-[LIVMF]-x-[GSDN]
- Consensus pattern: [LMFATC]-[KPQ]-x-[GSTDN]-x-[LIVMFYWR]-[LIVMFYW](2)-N-x-[STAGC]-R-[GP]-x-[LIVH]-[LIVMC]-[DNV]

[1] Grant G.A. Biochem. Biophys. Res. Commun. 165:1371-1374(1989).
[2] Kochhar S., Hunziker P., Leong-Morgenthaler P.M., Hottinger H. Biochem. Biophys. Res. Commun. 184:60-66 (1992).
[3] Ohta T., Taguchi H. J. Biol. Chem. 266:12588-12594(1991).
[4] Goldberg J.D., Yoshida T., Brick P. J. Mol. Biol. 236:1123-1140(1994).

[5] Popov V.O., Lamzin V.S. Biochem. J. 301:625-643(1994).

734. 2-oxo acid dehydrogenases acyltransferase (catalytic domain)
Refined crystal structure of the catalytic domain of dihysrolipoyl transacetylase (E2P) from azotobacter vineelandii at 2.6 angstroms resolution.
Mattevi A, Obmolova G, Kalk KH, Westphal AH, De Kok A, Hol WG; J Mol Biol 1993;230:1183-1199.
These proteins contain one to three copies of a lipoyl binding domain followed by the catalytic domain.
735. 3-beta hydroxysteriod dehydrogenase/isomerase family
Structure and tissue-specific expression of 3 beta-hydroxysteroid dehydrogenase/5-ene-4-ene isomerase genes in human and rat classical and peripheral
steroidogenic tissues.
Labrie F, Simard J, Luu-The V, Pelletier G, Belanger A, Lachance Y, Zhao HF, Labrie C, Breton N, de Launoit Y, et al J Steroid Biochem Mol Biol 1992;41:421-435.
The enzyme 3 beta-hydroxysteroid dehydrogenase/5-ene-4-ene isomerase (3 beta-HSD) catalyzes the oxidation and isomerization of 5-ene-3 beta-hydroxypregnene and 5-ene-hydroxyandrostene steroid precursors into the corresponding 4-ene-ketosteroids necessary for the formation of all classes of steroid hormones.
736. 3-hydroxyacyl-CoA dehydrogenase
This family also includes lambda crystallin.
Structure ofL-3-hydroxyacyl-coenzyme A dehydrogenase: preliminary chain tracing at 2.8-A resolution.
Birktoft JJ, Holden HM, Hamlin R, Xuong NH, Banaszak LJ; Proc Natl Acad Sci U S A 1987;84:8262-8266.
3-hydroxyacyl-CoA dehydrogenase (EC 1.1.1.35) (HCDH) [1] is an enzyme involved in fatty acid metabolism, it catalyzes the reduction of 3-hydroxyacyl-CoA to 3-oxoacyl-CoA. Most eukaryotic cells have 2 fatty-acid beta-oxidation systems, one located in mitochondria and the other in peroxisomes. In peroxisomes 3-hydroxyacyl-CoA dehydrogenase forms, with enoyl-CoA hydratase (ECH) and 3,2-trans-enoyl-CoA isomerase (ECI) a multifunctional enzyme where the N-terminal domain bears the hydratase/isomerase activities and the C-terminal domain the dehydrogenase activity. There are two mitochondrial enzymes: one which is monofunctional and the other which is, like its peroxisomal counterpart, multifunctional.
In Escherichia coli (gene fadB) and Pseudomonas fragi (gene faoA) HCDH is part of a multifunctional enzyme which also contains an ECH/ECI domain as well as a 3-hydroxybutyryl-CoA epimerase domain [2].
The other proteins structurally related to HCDH are:

- Bacterial 3-hydroxybutyryl-CoA dehydrogenase (EC 1.1.1.157) which reduces 3-hydroxybutanoyl-CoA to acetoacetyl-CoA [3].
- Eye lens protein lambda-crystallin [4], which is specific to lagomorphes (such as rabbit).

There are two major region of similarities in the sequences of proteins of the HCDH family, the first one located in the N-terminal, corresponds to the NAD-binding site, the second one is located in the center of the sequence. A signature pattern has been derived from this central region.

- Consensus pattern: [DNE]-x(2)-[GA]-F-[LIVMFY]-x-[NT]-R-x(3)-[PA]-[LIVMFY](2)-x(5)-[LIVMFYCT]-[LIVMFY]-x(2)-[GV]

[1] Birktoff J.J., Holden H.M., Hamlin R., Xuong N.-H., Banaszak L.J. Proc. Natl. Acad. Sci. U.S.A. 84:8262-8266 (1987).
[2] Nakahigashi K., Inokuchi H. Nucleic Acids Res. 18:4937-4937(1990).
[3] Mullany P., Clayton C.L., Pallen M.J., Slone R., Al-Saleh A., Tabaqchali S. FEMS Microbiol. Lett. 124:61-67(1994).
[4] Mulders J.W.M., Hendriks W., Blankesteijn W.M., Bloemendal H., de Jong W.W. J. Biol. Chem. 263:15462-15466 (1988).

737. 60s Acidic ribosomal protein
Proteins P1, P2, and P0, components of the eukaryotic ribosome stalk. New structural and functional aspects.
Remacha M, Jimenez-Diaz A, Santos C, Briones E, Zambrano R, Rodriguez Gabriel MA, Guarinos E, Ballesta JP; Biochem Cell Biol 1995;73:959-968.
This family includes archaebacterial L12, eukaryotic P0, P1 and P2.
738. 6-phosphogluconate dehydrogenases
6-phosphogluconate dehydrogenase (EC 1.1.1.44) (6PGD) catalyzes the third step in the hexose monophosphate shunt, the decarboxylating reduction of 6-phosphogluconate in to ribulose 5-phosphate.
Prokaryotic and eukaryotic 6PGD are proteins of about 470 amino acids whose sequence are highly conserved [1]. A

region which has been shown [2], from studies of the sheep 6PGD tertiary structure, to be involved in the binding of 6-phosphogluconate has been selected as a signature pattern.

- Consensus pattern: [LIVM]-x-D-x(2)-[GA]-[NQS]-K-G-T-G-x-W

[1] Reizer A., Deutscher J., Saier M.H. Jr., Reizer J. Mol. Microbiol. 5:1081-1089(1991).
[2] Adams M.J., Archibald I.G., Bugg C.E., Carne A., Gover S., Helliwell J.R., Pickersgill R.W., White S.W. EMBO J. 2:1009-1014(1983).

739. (7tm 1) G-protein coupled receptors [1 to 4,E1,E2] (also called R7G) are an extensive group of hormones, neurotransmitters, odorants and light receptors which transduce extracellular signals by interaction with guanine nucleotide-binding (G) proteins. The receptors that are currently known to belong to this family are listed below.

- 5-hydroxytryptamine (serotonin) 1A to 1F, 2A to 2C, 4, 5A, 5B, 6 and 7 [5].
- Acetylcholine, muscarinic-type, M1 to M5.
- Adenosine A1, A2A, A2B and A3 [6].
- Adrenergic alpha-1A to -1C; alpha-2A to -2D; beta-1 to -3 [7].
- Angiotensin II types I and II.
- Bombesin subtypes 3 and 4.
- Bradykinin B1 and B2.
- c3a and C5a anaphylatoxin.
- Cannabinoid CB1 and CB2.
- Chemokines C-C CC-CKR-1 to CC-CKR-8.
- Chemokines C-X-C CXC-CKR-1 to CXC-CKR-4.
- Cholecystokinin-A and cholecystokinin-B/gastrin.
- Dopamine D1 to D5 [8].
- Endothelin ET-a and ET-b [9].
- fMet-Leu-Phe (fMLP) (N-formyl peptide).
- Follicle stimulating hormone (FSH-R) [10].
- Galanin.
- Gastrin-releasing peptide (GRP-R).
- Gonadotropin-releasing hormone (GNRH-R).
- Histamine H1 and H2 (gastric receptor I).
- Lutropin-choriogonadotropic hormone (LSH-R) [10].
- Melanocortin MC1R to MC5R.
- Melatonin.
- Neuromedin B (NMB-R).
- Neuromedin K (NK-3R).
- Neuropeptide Y types 1 to 6.
- Neurotensin (NT-R).
- Octopamine (tyramine), from insects.
- Odorants [11].
- Opioids delta-, kappa- and mu-types [12].
- Oxytocin (OT-R).
- Platelet activating factor (PAF-R).
- Prostacyclin.
- Prostaglandin D2.
- Prostaglandin E2, EP1 to EP4 subtypes.
- Prostaglandin F2.
- Purinoreceptors (ATP) [13].
- Somatostatin types 1 to 5.
- Substance-K (NK-2R).
- Substance-P (NK-1R).
- Thrombin.
- Thromboxane A2.
- Thyrotropin (TSH-R) [10].
- Thyrotropin releasing factor (TRH-R).
- Vasopressin V1a, V1b and V2.

- Visual pigments (opsins and rhodopsin) [14].
- Proto-oncogene mas.
- A number of orphan receptors (whose ligand is not known) from mammals and birds.
- Caenorhabditis elegans putative receptors C06G4.5, C38C10.1, C43C3.2, T27D1.3 and ZC84.4.
- Three putative receptors encoded in the genome of cytomegalovirus: US27, US28, and UL33.
- ECRF3, a putative receptor encoded in the genome of herpesvirus saimiri.

The structure of all these receptors is thought to be identical. They have seven hydrophobic regions, each of which most probably spans the membrane. The N-terminus is located on the extracellular side of the membrane and is often glycosylated, while the C-terminus is cytoplasmic and generally phosphorylated. Three extracellular loops alternate with three intracellular loops to link the seven transmembrane regions. Most, but not all of these receptors, lack a signal peptide. The most conserved parts of these proteins are the transmembrane regions and the first two cytoplasmic loops. A conserved acidic-Arg-aromatic triplet is present in the N-terminal extremity of the second cytoplasmic loop [15] and could be implicated in the interaction with G proteins.
To detect this widespread family of proteins, a pattern that contains the conserved triplet and that also spans the major part of the third transmembrane helix has been developed.

- Consensus pattern: [GSTALIVMFYWC]-[GSTANCPDE]-{EDPKRH}-x(2)-[LIVMNQGA]-x(2)-[LIVMFT]-[GSTANC]-[LIVMFYWSTAC]-[DENH]-R-[FYWCSH]-x(2)-[LIVM]

[1] Strosberg A.D. Eur. J. Biochem. 196:1-10(1991).
[2] Kerlavage A.R. Curr. Opin. Struct. Biol. 1:394-401(1991).
[3] Probst W.C., Snyder L.A., Schuster D.I., Brosius J., Sealfon S.C. DNA Cell Biol. 11:1-20(1992).
[4] Savarese T.M., Fraser C.M. Biochem. J. 283:1-9(1992).
[5] Branchek T. Curr. Biol. 3:315-317(1993).
[6] Stiles G.L. J. Biol. Chem. 267:6451-6454(1992).
[7] Friell T., Kobilka B.K., Lefkowitz R.J., Caron M.G. Trends Neurosci. 11:321-324(1988).
[8] Stevens C.F. Curr. Biol. 1:20-22(1991).
[9] Sakurai T., Yanagisawa M., Masaki T. Trends Pharmacol. Sci. 13:103-107(1992).
[10] Salesse R., Remy J.J., Levin J.M., Jallal B., Garnier J. Biochimie 73:109-120(1991).
[11] Lancet D., Ben-Arie N. Curr. Biol. 3:668-674(1993).
[12] Uhl G.R., Childers S., Pasternak G. Trends Neurosci. 17:89-93(1994).
[13] Barnard E.A., Burnstock G., Webb T.E. Trends Pharmacol. Sci. 15:67-70(1994).
[14] Applebury M.L., Hargrave P.A. Vision Res. 26:1881-1895(1986).
[15] Attwood T.K., Eliopoulos E.E., Findlay J.B.C. Gene 98:153-159(1991).

(7tm 1) Visual pigments (opsins) retinal binding site
Visual pigments [1,2] are the light-absorbing molecules that mediate vision. They consist of an apoprotein, opsin, covalently linked to the chromophore cis-retinal. Vision is effected through the absorption of a photon by cis-retinal which is isomerized to trans-retinal. This isomerization leads to a change of conformation of the protein. Opsins are integral membrane proteins with seven transmembrane regions that belong to family 1 of G-protein coupled receptors. In vertebrates four different pigments are generally found. Rod cells, which mediate vision in dim light, contain the pigment rhodopsin. Cone cells, which function in bright light, are responsible for color vision and contain three or more color pigments (for example, in mammals: red, blue and green).
In Drosophila, the eye is composed of 800 facets or ommatidia. Each ommatidium contains eight photoreceptor cells (R1-R8): the R1 to R6 cells are outer cells, R7 and R8 inner cells. Each of the three types of cells (R1-R6, R7 and R8) expresses a specific opsin.
Proteins evolutionary related to opsins include squid retinochrome, also known as retinal photoisomerase, which converts various isomers of retinal into 11-cis retinal and mammalian retinal pigment epithelium (RPE) RGR [3], a protein that may also act in retinal isomerization.
The attachment site for retinal in the above proteins is a conserved lysine residue in the middle of the seventh transmembrane helix. The pattern that had been developed includes this residue.

- Consensus pattern: [LIVMWAC]-[PGC]-x(3)-[SAC]-K-[STALIMR]-[GSACPNV]-[STACP]-x(2)-[DENF]-[AP]-x(2)-[IY]
  [K is the retinal binding site]

[1] Applebury M.L., Hargrave P.A. Vision Res. 26:1881-1895(1986).

[2] Fryxell K.J., Meyerowitz E.M. J. Mol. Evol. 33:367-378(1991).
[3] Shen D., Jiang M., Hao W., Tao L., Salazar M., Fong H.K.W. Biochemistry 33:13117-13125(1994).

The following descriptions of protein family functions are not provided by the Pfam or Prosite databases.
740. BAH
BAH domain. Number of members: 65

[1] Medline: 97074677. Molecular cloning of polybromo, a nuclear protein containing multiple domains including five bromodomains, a truncated HMG-box, and two repeats of a novel domain. Nicolas RH, Goodwin GH; Gene 1996; 175:233-240.
[2] Medline: 99198739. The BAH (bromo-adjacent homology) domain: a link between DNA methylation, replication and transcriptional regulation. Callebaut I, Courvalin J-C, Mornon JP; FEBS letts 1999;446:189-193.

741. ELM2.
ELM2 domain. The ELM2 (Egl-27 and MTA1 homology 2) domain is a small domain of unknown function. Number of members: 10
742. Euk proin. EUKARYOTIC_PORIN The major protein of the outer mitochondrial membrane of eukaryotes is a porin that forms a voltage-dependent anion-selective channel (VDAC) that behaves as a general diffusion pore for small hydrophilic molecules [1 to 4]. The channel adopts an open conformation at low or zero membrane potential and a closed conformation at potentials above 30-40 mV.
This protein contains about 280 amino acids and its sequence is composed of between 12 to 16 beta-strands that span the mitochondrial outer membrane. Yeast contains two members of this family (genes POR1 and POR2); vertebrates have at least three members (genes VDAC1, VDAC2 and VDAC3) [5].
A conserved region located at the C-terminal part of these proteins was selected as a signature pattern.
Consensus pattern[YH]-x(2)-D-[SPCAD]-x-[STA]-x(3)-[TAG]-[KR]-[LIVMF]-[DNSTA]-[DNS]-x(4)-[GSTAN]-[LIVMA]-x-[LIVMY]

[1] Benz R. Biochim. Biophys. Acta 1197:167-196(1994).
[2] Manella C.A. Trends Biochem. Sci. 17:315-320(1992).
[3] Dihanich M. Experientia 46:146-153(1990).
[4] Forte M., Guy H.R., Mannella C.A. J. Bioenerg. Biomembr. 19:341-350(1987).
[5] Sampson M.J., Lovell R.S., Davison D.B., Craigen W.J. Genomics 36:192-196(1996).

743. Glyco hydor 19
Chitinases family 19 signatures
cross-reference(s) CHITINASE_19_1, CHITINASE_19_2
Chitinases (EC 3.2.1.14) [1] are enzymes that catalyze the hydrolysis of the beta-1,4-N-acetyl-D-glucosamine linkages in chitin polymers. From the view point of sequence similarity chitinases belong to either family 18 or 19 in the classification of glycosyl hydrolases [2,E1]. Chitinases of family 19 (also known as classes IA or I and IB or II) are enzymes from plants that function in the defense against fungal and insect pathogens by destroying their chitin-containing cell wall. Class IA/I and IB/II enzymes differ in the presence (IA/I) or absence (IB/11) of a N-terminal chitin-binding domain (see the relevant entry <PDOC00025>). The catalytic domain of these enzymes consist of about 220 to 230 amino acid residues.
Two highly conserved regions were selected as signature patterns, the first one is located in the N-terminal section and contains one of the six cysteines which are conserved in most, if not all, of these chitinases and which is probably involved in a disulfide bond.
Consensus patternC-x(4,5)-F-Y-[ST]-x(3)-[FY]-[LIVMF]-x-A-x(3)-[YF]-x(2)-F-[GSA]
Consensus pattern[LIVM]-[GSA]-F-x-[STAG](2)-[LIVMFY]-W-[FY]-W-[LIVM]

[1] Flach J., Pilet P.-E., Jolles P. Experientia 48:701-716(1992).
[2] Henrissat B. Biochem. J. 280:309-316(1991).

744. MBD
Methyl-CpG binding domain
The Methyl-CpG binding domain (MBD) binds to DNA that contains one or more symmetrically methylated CpGs [1]. DNA methylation in animals is associated with alterations in chromatin structure and silencing of gene expression. MBD has negligible non-specific affinity for DNA. In vitro foot-printing with MeCP2 showed the MBD can protect a 12 nucleotide region surrounding a methyl CpG pair [1]. MBDs are found in several Methyl-CpG binding proteins and also DNA

demethylase [2]. Number of members: 11

[1]Medline: 94232813. Dissection of the methyl-CpG binding domain from the chromosomal protein MeCP2. Nan X, Meehan RR, Bird A; Nucleic Acids Res 1993;21:4886-4892.
[2]Medline: 99158138. A mammalian protein with specific demethylase activity for mCpG DNA. Bhattacharya SK, Ramchandani S, Cervoni N, Szyf M; Nature 1999;397:579-583.

745. Peptidase C1
Eukaryotic thiol (cysteine) proteases active sites cross-reference(s) THIOL _PROTEASE_CYS; THIOL _PROTEASE_ HIS; THIOL_PROTEASE_ASN
Eukaryotic thiol proteases (EC 3.4.22.-) [1] are a family of proteolytic enzymes which contain an active site cysteine. Catalysis proceeds through a thioester intermediate and is facilitated by a nearby histidine side chain; an asparagine completes the essential catalytic triad. The proteases which are currently known to belong to this family are listed below (references are only provided for recently determined sequences).

- Vertebrate lysosomal cathepsins B (EC 3.4.22.1), H (EC 3.4.22.16), L (EC 3.4.22.15), and S (EC 3.4.22.27) [2].
- Vertebrate lysosomal dipeptidyl peptidase I (EC 3.4.14.1) (also known as cathepsin C) [2].
- Vertebrate calpains (EC 3.4.22.17). Calpains are intracellular calcium- activated thiol protease that contain both a N-terminal catalytic domain and a C-terminal calcium-binding domain.
- Mammalian cathepsin K, which seems involved in osteoclastic bone resorption [3].
- Human cathepsin O [4].
- Bleomycin hydrolase. An enzyme that catalyzes the inactivation of the antitumor drug BLM (a glycopeptide).
- Plant enzymes: barley aleurain (EC 3.4.22.16), EP-B1/B4; kidney bean EP-C1, rice bean SH-EP; kiwi fruit actinidin (EC 3.4.22.14); papaya latex papain (EC 3.4.22.2), chymopapain (EC 3.4.22.6), caricain (EC 3.4.22.30), and proteinase IV (EC 3.4.22.25); pea turgor-responsive protein 15A; pineapple stem bromelain (EC 3.4.22.32); rape COT44; rice oryzain alpha, beta, and gamma; tomato low-temperature induced, Arabidopsis thaliana A494, RD19A and RD21A.
- House-dust mites allergens DerP1 and EurM1.
- Cathepsin B-like proteinases from the worms Caenorhabditis elegans (genes gcp-1, cpr-3, cpr-4, cpr-5 and cpr-6), Schistosoma mansoni (antigen SM31) and Japonica (antigen SJ31), Haemonchus contortus (genes AC-1 and AC-2), and Ostertagia ostertagi (CP-1 and CP-3).
- Slime mold cysteine proteinases CP1 and CP2.
- Cruzipain from Trypanosoma cruzi and brucei.
- Throphozoite cysteine proteinase (TCP) from various Plasmodium species.
- Proteases from Leishmania mexicana, Theileria annulata and Theileria parva.
- Baculoviruses cathepsin-like enzyme (v-cath).
- Drosophila small optic lobes protein (gene sol), a neuronal protein that contains a calpain-like domain.
- Yeast thiol protease BLH1/YCP1/LAP3.
- Caenorhabditis elegans hypothetical protein C06G4.2, a calpain-like protein.

Two bacterial peptidases are also part of this family:

- Aminopeptidase C from Lactococcus lactis (gene pepC) [5].
- Thiol protease tpr from Porphyromonas gingivalis.

Three other proteins are structurally related to this family, but may have lost their proteolytic activity.

- Soybean oil body protein P34. This protein has its active site cysteine replaced by a glycine.
- Rat testin, a sertoli cell secretory protein highly similar to cathepsin L but with the active site cysteine is replaced by a serine. Rat testin should not be confused with mouse testin which is a LIM-domain protein (see <PDOC00382>).
- Plasmodium falciparum serine-repeat protein (SERA), the major blood stage antigen. This protein of 111 Kd possesses a C-terminal thiol-protease-like domain [6], but the active site cysteine is replaced by a serine.

The sequences around the three active site residues are well conserved and can be used as signature patterns.
Consensus patternQ-x(3)-[GE]-x-C-[YW]-x(2)-[STAGC]-[STAGCV] [C is the active site residue]
Note the residue in position 4 of the pattern is almost always cysteine; the only exceptions are calpains (Leu), bleomycin hydrolase (Ser) and yeast YCP1 (Ser). Note the residue in position 5 of the pattern is always Gly except in papaya protease IV where it is Glu. Consensus pattern[LIVMGSTAN]-x-H-[GSACE]-[LIVM]-x-[LIVMAT](2)-G-x-[GSADNH] [H is

the active site residue]
Consensus pattern[FYCH]-[WI]-[LIVT]-x-[KRQAG]-N-[ST]-W-x(3)-[FYW]-G-x(2)-G-[LFYW]-[LIVMFYG]-x-[LIVMF] [N is the active site residue]
Note these proteins belong to family C1 (papain-type) and C2 (calpains) in the classification ofpeptidases [7,E1].

[1] Dufour E. Biochimie 70:1335-1342(1988).
[2]Kirschke H., Barrett A.J., Rawlings N.D. Protein Prof. 2:1587-1643(1995).
[3]Shi G.-P., Chapman H.A., Bhairi S.M., Deleeuw C., Reddy V.Y., Weiss S.J. FEBS Lett. 357:129-134(1995).
[4]Velasco G., Ferrando A.A., Puente X.S., Sanchez L.M., Lopez-Otin C. J. Biol. Chem. 269:27136-27142(1994).
[5]Chapot-Chartier M.P., Nardi M., Chopin M.C., Chopin A., Gripon J.C. Appl. Environ. Microbiol. 59:330-333(1993).
[6]Higgins D.G., McConnell D.J., Sharp P.M. Nature 340:604-604(1989).
[7]Rawlings N.D., Barrett A.J. Meth. Enzymol. 244:461-486(1994).

746. Peptidase M22
Glycoprotease family signature cross-reference(s) GLYCOPROTEASE
Glycoprotease (GCP) (EC 3.4.24.57) [1], or o-syaloglycoprotein endopeptidase, is a metalloprotease secreted by Pasteurella haemolytica which specifically cleaves O-sialoglycoproteins such as glycophorin A. The sequence of GCP is highly similar to the following uncharacterized proteins:

- Escherichia coli hypothetical protein ygjD (ORF-X).
- Bacillus subtilis hypothetical protein ydiE.
- Mycobacterium leprae hypothetical protein U229E.
- Mycobacterium tuberculosis hypothetical protein MtCY78.10.
- Synechocystis strain PCC 6803 hypothetical protein slr0807.
- Methanococcus jannaschii hypothetical protein MJ1130.
- Haloarcula marismortui hypothetical protein in HSH 3 'region.
- Yeast hypothetical protein YKR038c.
- Yeast hypothetical protein QRI7.

One of the conserved regions contains two conserved histidines. It is possible that this region is involved in coordinating a metal ion such as zinc.
Consensus pattern[KR]-[GSAT]-x(4)-[FYWLH]-[DQNGK]-x-P-x-[LIVMFY]-x(3)-H-x(2)-[AG]-H-[LIVM]
Note these proteins belong to family M22 in the classification of peptidases [2,E1].

[1]Abdullah K.M., Lo R.Y.C., Mellors A. J. Bacteriol. 173:5597-5603(1991).
[2]Rawlings N.D., Barrett A.J. Meth. Enzymol. 248:183-228(1995).

747. SAM. SAM domain (Sterile alpha motif)
It has been suggested that SAM is an evolutionarily conserved protein binding domain that is involved in the regulation of numerous developmental processes in diverse eukaryotes. The SAM domain can potentially function as a protein interaction module through its ability to homo- and heterooligomerise with other SAM domains. Number of members: 81

[1]Medline: 96100659 SAM: A novel motif in yeast sterile alpha and Drosophila polyhomeotic proteins Ponting CP; Prot Sci 1995;4:1928-1930.
[2]Medline: 97160498 SAM as a protein interaction domain involved in developmental regulation. Shultz J, Ponting CP, Hofmann K, Bork P; Prot Sci 1997;6:249-253.
[3]Medline: 99101382 The crystal structure of an Eph receptor SAM domain reveals a mechanism for modular dimerization. Reference Author: Stapleton D, Balan I, Pawson T, Sicheri F; Nat Struct Biol 1999;6:44-49.

748. Tyrosinase signatures cross-reference(s) TYROSINASE_1; TYROSINASE_2
Tyrosinase (EC 1.14.18.1) [1] is a copper monooxygenases that catalyzes the hydroxylation of monophenols and the oxidation of o-diphenols to o-quinols.
This enzyme, found in prokaryotes as well as in eukaryotes, is involved in the formation of pigments such as melanins and other polyphenolic compounds.
Tyrosinase binds two copper ions (CuA and CuB). Each of the two copper ion has been shown [2] to be bound by three conserved histidines residues. The regions around these copper-binding ligands are well conserved and also shared by some hemocyanins, which are copper-containing oxygen carriers from the hemolymph of many molluscs and arthropods [3,4].

At least two proteins related to tyrosinase are known to exist in mammals:

- TRP-1 (TYRP1) [5], which is responsible for the conversion of 5,6-dihydroxyindole-2-carboxylic acid (DHICA) to indole-5,6-quinone-2-carboxylic acid.
- TRP-2 (TYRP2) [6], which is the melanogenic enzyme DOPAchrome tautomerase (EC 5.3.3.12) that catalyzes the conversion of DOPAchrome to DHICA. TRP-2 differs from tyrosinases and TRP-1 in that it binds two zinc ions instead of copper [7].

Other proteins that belong to this family are:

- Plants polyphenol oxidases (PPO) (EC 1.10.3.1) which catalyze the oxidation of mono- and o-diphenols to o-diquinones [8].
- Caenorhabditis elegans hypothetical protein C02C2.1.

Two signature patterns for tyrosinase and related proteins have been derived
The first one contains two of the histidines that bind CuA, and is located in the N-terminal section of tyrosinase. The second pattern contains a histidine that binds CuB, that pattern is located in the central section of the enzyme.
Consensus pattern H-x(4,5)-F-[LIVMFTP]-x-[FW]-H-R-x(2)-[LM]-x(3)-E
[The two H's are copper ligands]
Consensus patternD-P-x-F-[LIVMFYW]-x(2)-H-x(3)-D [H is a copper ligand]

[1]Lerch K. Prog. Clin. Biol. Res. 256:85-98(1988).
[2]Jackman M.P., Hajnal A., Lerch K. Biochem. J. 274:707-713(1991).
[3]Linzen B. Naturwissenschaften 76:206-211(1989).
[4]Lang W.H., van Holde K.E. Proc. Natl. Acad. Sci. U.S.A. 88:244-248(1991).
[5]Kobayashi T., Urabe K., Winder A., Jimenez-Cervantes C., Imokawa G., Brewington T., Solano F., Garcia-Borron J.C., Hearing V.J. EMBO J. 13:5818-5825(1994).
[6]Jackson I.J., Chambers D.M., Tsukamoto K., Copeland N.G., Gilbert D.J., Jenkins N.A., Hearing V. EMBO J. 11: 527-535(1992).
[7]Solano F., Martinez-Liarte J.H., Jimenez-Cervantes C., Garcia-Borron J.C., Lozano J.A. Biochem. Biophys. Res. Commun. 204:1243-1250(1994).
[8]Cary J.W., Lax A.R., Flurkey W.H. Plant Mol. Biol. 20:245-253(1992).

749. (Mur Ligase) Folylpolyglutamate synthase signatures
Folylpolyglutamate synthase (EC 6.3.2.17) (FPGS) [1] is the enzyme of folate metabolism that catalyzes ATP-dependent addition of glutamate moieties to tetrahydrofolate.
Its sequence is moderately conserved between prokaryotes (gene folC) and eukaryotes. We developed two signature patterns based on the conserved regions which are rich in glycine residues and could play a role in the catalytical activity and/or in substrate binding.
Description of pattern(s) and/or profile(s)
Consensus pattern[LIVMFY]-x-[LIVM]-[STAG]-G-T-[NK]-G-K-x-[ST]-x(7)- [LIVM](2)-x(3)-[GSK]
Consensus pattern[LIVMFY](2)-E-x-G-[LIVM]-[GA]-G-x(2)-D-x-[GST]-x-[LIVM](2)

[1]Shane B., Garrow T., Brenner A., Chen L., Choi Y.J., Hsu J.C., Stover P. Adv. Exp. Med. Biol. 338:629-634(1993).

750. (Peptidase M3) Neutral zinc metallopeptidases, zinc-binding region signature
The majority of zinc-dependent metallopeptidases (with the notable exception of the carboxypeptidases) share a common pattern of primary structure [1,2,3] in the part of their sequence involved in the binding of zinc, and can be grouped together as a superfamily,known as the metzincins, on the basis of this sequence similarity. They can be classified into a number of distinct families [4,E1] which are listed below along with the proteases which are currently known to belong to these families.

Family M1

- Bacterial aminopeptidase N (EC 3.4.11.2) (gene pepN).
- Mammalian aminopeptidase N (EC 3.4.11.2).
- Mammalian glutamyl aminopeptidase (EC 3.4.11.7) (aminopeptidase A). It may play a role in regulating growth and differentiation of early B-lineage cells.

- Yeast aminopeptidase yscII (gene APE2).
- Yeast alanine/arginine aminopeptidase (gene AAP1).
- Yeast hypothetical protein YIL137c.
- Leukotriene A-4 hydrolase (EC 3.3.2.6). This enzyme is responsible for the hydrolysis of an epoxide moiety of LTA-4 to form LTB-4; it has been shown that it binds zinc and is capable of peptidase activity.

Family M2

- Angiotensin-converting enzyme (EC 3.4.15.1) (dipeptidyl carboxypeptidase I) (ACE) the enzyme responsible for hydrolyzing angiotensin I to angiotensin II. There are two forms of ACE: a testis-specific isozyme and a somatic isozyme which has two active centers.

Family M3

- Thimet oligopeptidase (EC 3.4.24.15), a mammalian enzyme involved in the cytoplasmic degradation of small peptides.
- Neurolysin (EC 3.4.24.16) (also known as mitochondrial oligopeptidase M or microsomal endopeptidase).
- Mitochondrial intermediate peptidase precursor (EC 3.4.24.59) (MIP). It is involved the second stage of processing of some proteins imported in the mitochondrion.
- Yeast saccharolysin (EC 3.4.24.37) (proteinase yscD).
- Escherichia coli and related bacteria dipeptidyl carboxypeptidase (EC 3.4.15.5) (gene dcp).
- Escherichia coli and related bacteria oligopeptidase A (EC 3.4.24.70) (gene opdA or pr1C).
- Yeast hypothetical protein YKL134c.

Family M4

- Thermostable thermolysins (EC 3.4.24.27), and related thermolabile neutral proteases (bacillolysins) (EC 3.4.24.28) from various species of Bacillus.
- Pseudolysin (EC 3.4.24.26) from Pseudomonas aeruginosa (gene lasB).
- Extracellular elastase from Staphylococcus epidermidis.
- Extracellular protease prt1 from Erwinia carotovora.
- Extracellular minor protease smp from Serratia marcescens.
- Vibriolysin (EC 3.4.24.25) from various species of Vibrio.
- Protease prtA from Listeria monocytogenes.
- Extracellular proteinase proA from Legionella pneumophila.

Family M5

- Mycolysin (EC 3.4.24.31) from Streptomyces cacaoi.

Family M6

- Immune inhibitor A from Bacillus thuringiensis (gene ina). Ina degrades two classes of insect antibacterial proteins, attacins and cecropins.

Family M7

- Streptomyces extracellular small neutral proteases

Family M8

- Leishmanolysin (EC 3.4.24.36) (surface glycoprotein gp63), a cell surface protease from various species of Leishmania.

Family M9

- Microbial collagenase (EC 3.4.24.3) from Clostridium perfringens and Vibrio alginolyticus.

Family M10A

- Serralysin (EC 3.4.24.40), an extracellular metalloprotease from Serratia.
- Alkaline metalloproteinase from Pseudomonas aeruginosa (gene aprA).
- Secreted proteases A, B, C and G from Erwinia chrysanthemi.
- Yeast hypothetical protein YIL108w.

Family M10B

- Mammalian extracellular matrix metalloproteinases (known as matrixins) [5]: MMP-1 (EC 3.4.24.7) (interstitial collagenase), MMP-2 (EC 3.4.24.24) (72 Kd gelatinase), MMP-9 (EC 3.4.24.35) (92 Kd gelatinase), MMP-7 (EC 3.4.24.23) (matrylisin), MMP-8 (EC 3.4.24.34) (neutrophil collagenase), MMP-3 (EC 3.4.24.17) (stromelysin-1), MMP-10 (EC 3.4.24.22) (stromelysin-2), and MMP-11 (stromelysin-3), MMP-12 (EC 3.4.24.65) (macrophage metalloelastase).
- Sea urchin hatching enzyme (envelysin) (EC 3.4.24.12). A protease that allows the embryo to digest the protective envelope derived from the egg extracellular matrix.
- Soybean metalloendoproteinase 1.

Family M11

- Chlamydomonas reinhardtii gamete lytic enzyme (GLE).

Family M12A

- Astacin (EC 3.4.24.21), a crayfish endoprotease.
- Meprin A (EC 3.4.24.18), a mammalian kidney and intestinal brush border metalloendopeptidase.
- Bone morphogenic protein 1 (BMP-1), a protein which induces cartilage and bone formation and which expresses metalloendopeptidase activity. The Drosophila homolog of BMP-1 is the dorsal-ventral patterning protein tolloid.
- Blastula protease 10 (BP10) from Paracentrotus lividus and the related protein SpAN from Strongylocentrotus purpuratus.
- Caenorhabditis elegans protein toh-2.
- Caenorhabditis elegans hypothetical protein F42A10.8.
- Choriolysins L and H (EC 3.4.24.67) (also known as embryonic hatching proteins LCE and HCE) from the fish Oryzias lapides. These proteases participates in the breakdown of the egg envelope, which is derived from the egg extracellular matrix, at the time of hatching.

Family M12B

- Snake venom metalloproteinases [6]. This subfamily mostly groups proteases that act in hemorrhage. Examples are: adamalysin II (EC 3.4.24.46), atrolysin C/D (EC 3.4.24.42), atrolysin E (EC 3.4.24.44), fibrolase (EC 3.4.24.72), trimerelysin I (EC 3.4.25.52) and II (EC 3.4.25.53).
- Mouse cell surface antigen MS2.

Family M13

- Mammalian neprilysin (EC 3.4.24.11) (neutral endopeptidase) (NEP).
- Endothelin-converting enzyme 1 (EC 3.4.24.71) (ECE-1), which process the precursor of endothelin to release the active peptide.
- Kell blood group glycoprotein, a major antigenic protein of erythrocytes. The Kell protein is very probably a zinc endopeptidase.
- Peptidase O from Lactococcus lactis (gene pepO).

Family M27

- Clostridial neurotoxins, including tetanus toxin (TeTx) and the various botulinum toxins (BoNT). These toxins are zinc proteases that block neurotransmitter release by proteolytic cleavage of synaptic proteins such as synaptobrevins, syntaxin and SNAP-25 [7,8].

Family M30

- Staphylococcus hyicus neutral metalloprotease.

Family M32

- Thermostable carboxypeptidase 1 (EC 3.4.17.19) (carboxypeptidase Taq), an enzyme from Thermus aquaticus which is most active at high temperature.

Family M34

- Lethal factor (LF) from Bacillus anthracis, one of the three proteins composing the anthrax toxin.

Family M35

- Deuterolysin (EC 3.4.24.39) from Penicillium citrinum and related proteases from various species of Aspergillus.

Family M36

- Extracellular elastinolytic metalloproteinases from Aspergillus.

From the tertiary structure of thermolysin, the position of the residues acting as zinc ligands and those involved in the catalytic activity are known. Two of the zinc ligands are histidines which are very close together in the sequence; C-terminal to the first histidine is a glutamic acid residue which acts as a nucleophile and promotes the attack of a water molecule on the carbonyl carbon of the substrate. A signature pattern which includes the two histidine and the glutamic acid residues is sufficient to detect this superfamily of proteins.
Description of pattern(s) and/or profile(s)
Consensus pattem[GSTALIVN]-x(2)-H-E-[LIVMFYW]-{DEHRKP}-H-x-[LIVMFYWGSPQ] [The two H's are zinc ligands] [E is the active site residue]
Sequences known to belong to this class detected by the patternALL, except for members of families M5, M7 amd M11.
Other sequence(s) detected in SWISS-PROT55; including Neurospora crassa conidiation-specific protein 13 which could be a
zinc-protease.

[1]Jongeneel C.V., Bouvier J., Bairoch A. FEBS Lett. 242:211-214(1989).
[2]Murphy G.J.P., Murphy G., Reynolds J.J. FEBS Lett. 289:4-7(1991).
[3]Bode W., Grams F., Reinemer P., Gomis-Rueth F.-X., Baumann U., McKay D.B., Stoecker W. Zoology 99:237-246 (1996).
[4]Rawlings N.D., Barrett A.J. Meth. Enzymol. 248:183-228(1995).
[5]Woessner J. Jr. FASEB J. 5:2145-2154(1991).
[6]Hite L.A., Fox J.W., Bjarnason J.B.
[7]Montecucco C., Schiavo G. Trends Biochem. Sci. 18:324-327(1993).
[8]Niemann H., Blasi J., Jahn R. Trends Cell Biol. 4:179-185(1994).

751. PseudoU_synt_1
tRNA pseudouridine synthase is involved in the formation of pseudouridine at the anticodon stem and loop of transfer-RNAs Pseudouridine is an isomer of uridine (5-(beta-D-ribofuranosyl) uracil, and id the most abundant modified nucleoside found in all cellular RNAs. The TruA-like proteins also exhibit a conserved sequence with a strictly conserved aspartic acid, likely involved in catalysis. Number of members: 25

[1]Medline: 98254513. Transfer RNA-pseudouridine synthetase Pus1 of Saccaromyces cerevisiae contains one atom of zinc essential for its native conformation and tRNA recognition. Arluison V, Hountondji C, Robert B, Grosjean H; Biochemistry 1998;37:7268-7276.

752. EPSP synthase signatures
EPSP synthase (3-phosphoshikimate 1-carboxyvinyltransferase) (EC 2.5.1.19) catalyzes the sixth step in the biosynthesis from chorismate of the aromatic amino acids (the shikimate pathway) in bacteria (gene aroA), plants and fungi

(where it is part of a multifunctional enzyme which catalyzes five consecutive steps in this pathway) [1]. EPSP synthase has been extensively studied as it is the target of the potent herbicide glyphosate which inhibits the enzyme.
The sequence of EPSP from various biological sources shows that the structure of the enzyme has been well conserved throughout evolution. Two conserved regions were selected as signature patterns. The first pattern corresponds to a region that is part of the active site and which is also important for the resistance to glyphosate [2]. The second pattern is located in the C-terminal part of the protein and contains a conserved lysine which seems to be important for the activity of the enzyme.
Description of pattern(s) and/or profile(s)
Consensus pattern[LIVM]-x(2)-[GN]-N-[SA]-G-T-[STA]-x-R-x-[LIVMY]-x-[GSTA]
Consensus pattern[KR]-x-[KH]-E-[CST]-[DNE]-R-[LIVM]-x-[STA]-[LIVMC]-x(2)-[EN]-[LIVMF]-x-[KRA]-[LIVMF]-G

[1]Stallings W.C., Abdel-Megid S.S., Lim L.W., Shieh H.-S., Dayringer H.E., Leimgruber N.K., Stegeman R.A., Anderson K.S., Sikorski J.A., Padgette S.R., Kishore G.M. Proc. Natl. Acad. Sci. U.S.A. 88:5046-5050(1991).
[2]Padgette S.R., Re D.B., Gaser C.S., Eicholtz D.A., Frazier R.B., Hironaka C.M., Levine E.B., Shah D.M., Fraley R.T., Kishore G.M. J. Biol. Chem. 266:22364-22369(1991).

753. Glyco_hydro_18
Glycosyl hydrolases family 18. Number of members: 173

[1]Medline: 95219379. Crystal structure of a bacterial chitinase at 2.3 A resolution. Perrakis A, Tews I, Dauter Z, Oppenheim AB, Chet I, Wilson KS, Vorgias CE; Structure 1994;2:1169-1180.

754. Esterase
Putative esterase
This family contains Esterase D Swiss:P10768. However it is not clear if all members of the family have the same function. This family is possibly related to the COesterase family. Number of members: 36
755. (HMA) Heavy-metal-associated domain
A conserved domain of about 30 amino acid residues has been found [1] in a number of proteins that transport or detoxify heavy metals. This domain contains two conserved cysteines that could be involved in the binding of these metals. The domain has been termed Heavy-Metal-Associated (HMA). It has been found in:

- A variety of cation transport ATPases (E1-E2 ATPases) (see <PDOC00139>). The human copper ATP Ases ATP7A and ATP7B which are respectively involved in Menke's and Wilson's diseases. ATP7A and ATP7B both contain 6 tandem copies of the HMA domain. The copper ATPases CCC2 from budding yeast, copA from Enterococcus faecalis and synA from Synechococcus contain one copy of the HMA domain. The cadmium ATPases cadA from Bacillus firmus and from plasmid pl258 from Staphylococcus aureus also contain a single HMA domain, while a chromosomal Staphylococcus aureus cadA contains two copies. Other, less characterized ATPases that contain the HMA domain are: fixl from Rhizobium meliloti, pacS from Synechococcus strain PCC 7942), Mycobacterium leprae ctpA and ctpB and Escherichia coli hypothetical protein yhhO. In all these ATPases the HMA domain(s) are located in the N-terminal section.
- Mercuric reductase (EC 1.16.1.1) (gene merA) which is generally encoded by plasmids carried by mercury-resistant Gram-negative bacteria. Mercuric reductase is a class-1 pyridine nucleotide-disulphide oxidoreductase (see <PDOC00073>). There is generally one HMA domain (with the exception of a chromosomal merA from Bacillus strain RC607 which has two) in the N-terminal part of merA.
- Mercuric transport protein periplasmic component (gene merP), also encoded by plasmids carried by mercury-resistant Gram-negative bacteria. It seems to be a mercury scavenger that specifically binds to one Hg(2+) ion and which passes it to the mercuric reductase via the merT protein. The N-terminal half of merP is a HMA domain.
- Helicobacter pylori copper-binding protein copP.
- Yeast protein ATX1 [2], which could act in the transport and/or partitioning of copper.

The consensus pattern for HMA spans the complete domain.
Description of pattern(s) and/or profile(s)
Consensus pattern[LIVN]-x(2)-[LIVMFA]-x-C-x-[STAGCDNH]-C-x(3)-[LIVFG]-x(3)-[LIV]-x(9,11)-[IVA]-x-[LVFYS] [The two C's probably bind metals]

[1]Bull P.C., Cox D.W. Trends Genet. 10:246-252(1994).
[2]Lin S.-J., Culotta V.L. Proc. Natl. Acad. Sci. U.S.A. 92:3784-3788(1995).

756. (Peptidase M10) Matrixins cysteine switch
PROSITE cross-reference(s): CYSTEINE_SWITCH
Mammalian extracellular matrix metalloproteinases (EC 3.4.24.-), also known as matrixins [1] (see <PDOC00129>), are zinc-dependent enzymes. They are secreted by cells in an inactive form (zymogen) that differs from the mature enzyme by the presence of an N-terminal propeptide. A highly conserved octapeptide is found two residues downstream of the C-terminal end of the propeptide. This region has been shown to be involved in autoinhibition of matrixins [2,3]; a cysteine within the octapeptide chelates the active site zinc ion, thus inhibiting the enzyme. This region has been called the 'cysteine switch' or 'autoinhibitor region'.
A cysteine switch has been found in the following zinc proteases:

- MMP-1 (EC 3.4.24.7) (interstitial collagenase).
- MMP-2 (EC 3.4.24.24) (72 Kd gelatinase).
- MMP-3 (EC 3.4.24.17) (stromelysin-1).
- MMP-7 (EC 3.4.24.23) (matrilysin).
- MMP-8 (EC 3.4.24.34) (neutrophil collagenase).
- MMP-9 (EC 3.4.24.35) (92 Kd gelatinase).
- MMP-10 (EC 3.4.24.22) (stromelysin-2).
- MMP-11 (EC 3.4.24.-) (stromelysin-3).
- MMP-12 (EC 3.4.24.65) (macrophage metalloelastase).
- MMP-13 (EC 3.4.24.-) (collagenase 3).
- MMP-14 (EC 3.4.24.-) (membrane-type matrix metalliproteinase 1).
- MMP-15 (EC 3.4.24.-) (membrane-type matrix metalliproteinase 2).
- MMP-16 (EC 3.4.24.-) (membrane-type matrix metalliproteinase 3).
- Sea urchin hatching enzyme (EC 3.4.24.12) (envelysin) [4].
- Chlamydomonas reinhardtii gamete lytic enzyme (GLE) [5].

Description of pattern(s) and/or profile(s)
Consensus patternP-R-C-[GN]-x-P-[DR]-[LIVSAPKQ] [C chelates the zinc ion]

[1]Woessner J. Jr. FASEB J. 5:2145-2154(1991).
[2]Sanchez-Lopez R., Nicholson R., Gesnel M.C., Matrisian L.M., Breathnach R. J. Biol. Chem. 263:11892-11899 (1988).
[3]Park A.J., Matrisian L.M., Kells A.F., Pearson R., Yuan Z., Navre M. J. Biol. Chem. 266:1584-1590(1991).
[4]Lepage T., Gache C. EMBO J. 9:3003-3012(1990).
[5] Kinoshita T., Fukuzawa H., Shimada T., Saito T., Matsuda Y. Proc. Natl. Acad. Sci. U.S.A. 89:4693-4697(1992).

757. (Peptidase S8) Serine proteases, subtilase family, active sites
PROSITE cross-reference(s): PS00136; SUBTILASE_ASP, PS00137; SUBTILASE_HIS, PS00138; SUBTILASE_SER
Subtilases [1,2] are an extensive family of serine proteases whose catalytic activity is provided by a charge relay system similar to that of the trypsin family of serine proteases but which evolved by independent convergent evolution. The sequence around the residues involved in the catalytic triad (aspartic acid, serine and histidine) are completely different from that of the analogous residues in the trypsin serine proteases and can be used as signatures specific to that category of proteases.
The subtilase family currently includes the following proteases:

- Subtilisins (EC 3.4.21.62), these alkaline proteases from various Bacillus species have been the target of numerous studies in the past thirty years.
- Alkaline elastase YaB from Bacillus sp. (gene ale).
- Alkaline serine exoprotease A from Vibrio alginolyticus (gene proA).
- Aqualysin I from Thermus aquaticus (gene pstl).
- AspA from Aeromonas salmonicida.
- Bacillopeptidase F (esterase) from Bacillus subtilis (gene bpf).
- C5A peptidase from Streptococcus pyogenes (gene scpA).
- Cell envelope-located proteases PI, PII, and PIII from Lactococcus lactis.
- Extracellular serine protease from Serratia marcescens.
- Extracellular protease from Xanthomonas campestris.
- Intracellular serine protease (ISP) from various Bacillus.
- Minor extracellular serine protease epr from Bacillus subtilis (gene epr).

- Minor extracellular serine protease vpr from Bacillus subtilis (gene vpr).
- Nisin leader peptide processing protease nisP from Lactococcus lactis.
- Serotype-specific antigene 1 from Pasteurella haemolytica (gene ssa1).
- Thermitase (EC 3.4.21.66) from Thermoactinomyces vulgaris.
- Calcium-dependent protease from Anabaena variabilis (gene prcA).
- Halolysin from halophilic bacteria sp. 172p1 (gene hly).
- Alkaline extracellular protease (AEP) from Yarrowia lipolytica (gene xpr2).
- Alkaline proteinase from Cephalosporium acremonium (gene alp).
- Cerevisin (EC 3.4.21.48) (vacuolar protease B) from yeast (gene PRB1).
- Cuticle-degrading protease (pr1) from Metarhizium anisopliae.
- KEX-1 protease from Kluyveromyces lactis.
- Kexin (EC 3.4.21.61) from yeast (gene KEX-2).
- Oryzin (EC 3.4.21.63) (alkaline proteinase) from Aspergillus (gene alp).
- Proteinase K (EC 3.4.21.64) from Tritirachium album (gene proK).
- Proteinase R from Tritirachium album (gene proR).
- Proteinase T from Tritirachium album (gene proT).
- Subtilisin-like protease III from yeast (gene YSP3).
- Thermomycolin (EC 3.4.21.65) from Malbranchea sulfurea.
- Furin (EC 3.4.21.85), neuroendocrine convertases 1 to 3 (NEC-1 to -3) and PACE4 protease from mammals, other vertebrates, and invertebrates. These proteases are involved in the processing of hormone precursors at sites comprised of pairs of basic amino acid residues [3].
- Tripeptidyl-peptidase II (EC 3.4.14.10) (tripeptidyl aminopeptidase) from Human.
- Prestalk-specific proteins tagB and tagC from slime mold [4]. Both proteins consist of two domains: a N-terminal subtilase catalytic domain and a C-terminal ABC transporter domain (see <PDOC00185>).

Description of pattern(s) and/or profile(s)
Consensus pattern[STAIV]-x-[LIVMF]-[LIVM]-D-[DSTA]-G-[LIVMFC]-x(2,3)-[DNH]
[D is the active site residue]
Consensus patternH-G-[STM]-x-[VIC]-[STAGC]-[GS]-x-[LIVMA]-[STAGCLV]-[SAGM] [H is the active site residue]
Consensus patternG-T-S-x-[SA]-x-P-x(2)-[STAVC]-[AG] [S is the active site residue]
Note if a protein includes at least two of the three active site signatures, the probability of it being a serine protease from the subtilase family is 100%
Note these proteins belong to family S8 in the classification of peptidases [5,E1].

[1]Siezen R.J., de Vos W.M., Leunissen J.A.M., Dijkstra B.W. Protein Eng. 4:719-737(1991).
[2]Siezen R.J. (In) Proceeding subtilisin symposium, Hamburg, (1992).
[3]Barr P.J. Cell 66:1-3(1991).
[4]Shaulsky G., Kuspa A., Loomis W.F.; Genes Dev. 9:1111-1122(1995).
[5]Rawlings N.D., Barrett A.J. Meth. Enzymol. 244:19-61(1994).

758. (SSB) Single-strand binding protein family signatures
PROSITE cross-reference(s): PS00735; SSB_1,PS00736; SSB_2
The Escherichia coli single-strand binding protein [1] (gene ssb), also known as the helix-destabilizing protein, is a protein of 177 amino acids. It binds tightly, as a homotetramer, to single-stranded DNA (ss-DNA) and plays an important role in DNA replication, recombination and repair.
Closely related variants of SSB are encoded in the genome of a variety of large self-transmissible plasmids. SSB has also been characterized in bacteria such as Proteus mirabilis or Serratia marcescens.
Eukaryotic mitochondrial proteins that bind ss-DNA and are probably involved in mitochondrial DNA replication are structurally and evolutionary related to prokaryotic SSB. Proteins currently known to belong to this subfamily are listed below [2].

- Mammalian protein Mt-SSB (P16).
- Xenopus Mt-SSBs and Mt-SSBr.
- Drosophila MtSSB.
- Yeast protein RIM1.

Two signature patterns have been developed for these proteins. The first is a conserved region in the N-terminal section of the SSB's. The second is a centrally located region which, in Escherichia coli SSB, is known to be involved in the

binding of DNA.
Description of pattern(s) and/or profile(s)
Consensus pattern[LIVMF]-[NST]-[KRT]-[LIVM]-x-[LIVMF](2)-G-[NHRK]-[LIVM]-[GST]-x-[DET]
Consensus patternT-x-W-[HY]-[RNS]-[LIVM]-x-[LIVMF]-[FY]-[NGKR]

[1]Meyer R.R., Laine P.S. Microbiol. Rev. 54:342-380(1990).
[2]Stroumbakis N.D., Li Z., Tolias P.P. Gene 143:171-177(1994).

759. KDPG and KHG aldolases active site signatures
PROSITE cross-reference(s): PS00159; ALDOLASE_KDPG_KHG_1, PS00160; ALDOLASE_KDPG_KHG_2
4-hydroxy-2-oxoglutarate aldolase (EC 4.1.3.16) (KHG-aldolase) catalyzes the interconversion of 4-hydroxy-2-oxoglutarate into pyruvate and glyoxylate. Phospho-2-dehydro-3-deoxygluconate aldolase (EC 4.1.2.14) (KDPG-aldolase) catalyzes the interconversion of 6-phospho-2-dehydro-3-deoxy-D-gluconate into pyruvate and glyceraldehyde 3-phosphate.
These two enzymes are structurally and functionally related [1]. They are both homotrimeric proteins of approximately 220 amino-acid residues. They are class I aldolases whose catalytic mechanism involves the formation of a Schiff-base intermediate between the substrate and the epsilon-amino group of a lysine residue. In both enzymes, an arginine is required for catalytic activity.
Two signature patterns were developed for these enzymes. The first one contains the active site arginine and the second, the lysine involved in the Schiff-base formation.
Description of pattern(s) and/or profile(s)
Consensus patternG-[LIVM]-x(3)-E-[LIV]-T-[LF]-R [R is the active site residue]
Consensus patternG-x(3)-[LIVMF]-K-[LF]-F-P-[SA]-x(3)-G [K is involved in Schiff-base formation]

[1] Vlahos C J., Dekker E.E. J. Biol. Chem. 263:11683-11691(1988).

760. AP endonucleases family 1 signatures. PROSITE cross-reference(s): PS00726; AP_NUCLEASE_F1_1, PS00727; AP_NUCLEASE_F1_2, PS00728; AP_NUCLEASE_F1_3
DNA damaging agents such as the antitumor drugs bleomycin and neocarzinostatin or those that generate oxygen radicals produce a variety of lesions in DNA. Amongst these is base-loss which forms apurinic/apyrimidinic (AP) sites or strand breaks with atypical 3'termini. DNA repair at the AP sites is initiated by specific endonuclease cleavage of the phosphodiester backbone. Such endonucleases are also generally capable of removing blocking groups from the 3'terminus of DNA strand breaks.
AP endonucleases can be classified into two families on the basis of sequence similarity. Family 1 groups the enzymes listed below [1].

- Escherichia coli exonuclease III (EC 3.1.11.2) (gene xthA).
- Streptococcus pneumoniae and Bacillus subtilis exonuclease A (gene exoA).
- Mammalian AP endonuclease 1 (AP1) (EC 4.2.99.18).
- Drosophila recombination repair protein 1 (gene Rrp1).
- Arabidopsis thaliana apurinic endonuclease-redox protein (gene arp).

Except for Rrp1 and arp, these enzymes are proteins of about 300 amino-acid residues. Rrp1 and arp both contain additional and unrelated sequences in their N-terminal section (about 400 residues for Rrp1 and 270 for arp).
Three signature patterns were developed for this family of enzymes. The patterns are based on the most conserved regions. The first pattern contains a glutamate which has been shown [2], in the Escherichia coli enzyme to bind a divalent metal ion such as magnesium or manganese
Consensus pattem[APF]-D-[LIVMF](2)-x-[LIVM]-Q-E-x-K [E binds a divalent metal ion]
Consensus patternD-[ST]-[FY]-R-[KH]-x(7,8)-[FYW]-[ST]-[FYW](2)
Consensus patternN-x-G-x-R-[LIVM]-D-[LIVMFYH]-x-[LV]-x-S

[1] Barzilay G., Hickson I.S. BioEssays 17:713-719(1995).
[2] Mol C.D., Kuo C.-F., Thayer M.M., Cunningham R.P., Tainer J.A. Nature 374:381-386(1995).

761. (ER)Enhancer of rudimentary signature, PROSITE cross-reference(s): PS01290; ER
The Drosophila protein 'enhancer of rudimentary' (gene (e(r)) is a small protein of 104 residues whose function is not yet clear. From an evolutionary point of view, it is highly conserved [1] and has been found to exist in probably all multicellular eukaryotic organisms. It has been proposed that this protein plays a role in the cell cycle.

A conserved region in the central part of the protein was selected as as signaure pattern.
Consensus patternY-D-I-[SA]-x-L-[FY]-x-F-[IV]-D-x(3)-D-[LIV]-S

[1] Gelsthorpe M., Pulumati M., McCallum C., Dang-Vu K., Tsubota S.I. Gene 186:189-195(1997).

762. (ETF alpha) Electron transfer flavoprotein alpha-subunit signature, PROSITE cross-reference(s): PS00696; ETF_ ALPHA
The electron transfer flavoprotein (ETF) [1,2] serves as a specific electron acceptor for various mitochondrial dehydrogenases. ETF transfers electrons to the main respiratory chain via ETF-ubiquinone oxidoreductase. ETF is an heterodimer that consist of an alpha and a beta subunit and which bind one molecule of FAD per dimer. A similar system also exists in some bacteria.
The alpha subunit of ETF is a protein of about 32 Kd which is structurally related to the bacterial nitrogen fixation protein fixB which could play a role in a redox process and feed electrons to ferredoxin.
Other related proteins are:

- Escherichia coli hypothetical protein ydiR.
- Escherichia coli hypothetical protein ygcQ.

A highly conserved region which is located in the C-terminal section was selected as a signature pattern for these proteins.
Consensus pattern [LI]-Y-[LIVM]-[AT]-x-G-[IV]-[SD]-G-x-[IV]-Q-H-x(2)-G-x(6)-[IV]-x-A-[IV]-N

[1] Finocchiaro G., Ikeda Y., Ito M., Tanaka K. Prog. Clin. Biol. Res. 321:637-652(1990).
[2] Tsai M.H., Saier M.H. Jr. Res. Microbiol. 146:397-404(1995).

763. (lectin c) C-type lectin domain signature and profile
PROSITE cross-reference(s): PS00615; C_TYPE_LECTIN_1, PS50041; C_TYPE_LECTIN_2
A number of different families of proteins share a conserved domain which was first characterized in some animal lectins and which seem to function as a calcium-dependent carbohydrate-recognition domain [1,2,3]. This domain, which is known as the C-type lectin domain (CTL) or as the carbohydrate-recognition domain (CRD), consists of about 110 to 130 residues. There are four cysteines which are perfectly conserved and involved in two disulfide bonds. A schematic representation of the CTL domain is shown below.

```
                                +------+
                                |      |
xcxxxxcxxxxxxxCxxxxxxxxxxxxxxxxxxxxxxxxxxxxxxxxxxxxxxCxxxxWxCx
xxxCx
    |  |    |************|*
    +----+    +---------------------------------------------+
```

'C': conserved cysteine involved in a disulfide bond.
'c': optional cysteine involved in a disulfide bond.
'*': position of the pattern.
The categories of proteins, in which the CTL domain has been found, are listed below.
Type-II membrane proteins where the CTL domain is located at the C-terminal extremity of the proteins:

- Asialoglycoprotein receptors (ASGPR) (also known as hepatic lectins) [4]. The ASGPR's mediate the endocytosis of plasma glycoproteins to which the terminal sialic acid residue in their carbohydrate moieties has been removed.
- Low affinity immunoglobulin epsilon Fc receptor (lymphocyte IgE receptor), which plays an essential role in the

regulation of IgE production and in the differentiation of B cells.

- Kupffer cell receptor. A receptor with an affinity for galactose and fucose, that could be involved in endocytosis.
- A number of proteins expressed on the surface of natural killer T-cells: NKG2, NKR-P1, YE1/88 (Ly-49), CD69 and on B-cells: CD72, LyB-2. The CTL- domain in these proteins is distantly related to other CTL-domains; it is unclear whether they are likely to bind carbohydrates.

Proteins that consist of an N-terminal collagenous domain followed by a CTL- domain [5], these proteins are sometimes called 'collectins':

- Pulmonary surfactant-associated protein A (SP-A). SP-A is a calcium-dependent protein that binds to surfactant phospholipids and contributes to lower the surface tension at the air-liquid interface in the alveoli of the mammalian lung.
- Pulmonary surfactant-associated protein D (SP-D).
- Conglutinin, a calcium-dependent lectin-like protein which binds to a yeast cell wall extract and to immune complexes through the complement component (iC3b).
- Mannan-binding proteins (MBP) (also known as mannose-binding proteins). MBP's bind mannose and N-acetyl-D-glucosamine in a calcium-dependent manner.
- Bovine collectin-43 (CL-43).

Selectins (or LEC-CAM) [6,7]. Selectins are cell adhesion molecules implicated in the interaction of leukocytes with platelets or vascular endothelium. Structurally, selectins consist of a long extracellular domain, followed by a transmembrane region and a short cytoplasmic domain. The extracellular domain is itself composed of a CTL-domain, followed by an EGF-like domain and a variable number of SCR/Sushi repeats. Known selectins are:

- Lymph node homing receptor (also known as L-selectin, leukocyte adhesion molecule-1, (LAM-1), leu-8, gp90-mel, or LECAM-1)
- Endothelial leukocyte adhesion molecule 1 (ELAM-1, E-selectin or LECAM-2). The ligand recognized by ELAM-1 is sialyl-Lewis x.
- Granule membrane protein 140 (GMP-140, P-selectin, PADGEM, CD62, or LECAM-3). The ligand recognized by GMP-140 is Lewis x.

Large proteoglycans that contain a CTL-domain followed by one copy of a SCR/ Sushi repeat, in their C-terminal section:

- Aggrecan (cartilage-specific proteoglycan core protein). This proteoglycan is a major component of the extracellular matrix of cartilagenous tissues where it has a role in the resistance to compression.

    - Brevican.
    - Neurocan.
    - Versican (large fibroblast proteoglycan), a large chondroitin sulfate proteoglycan that may play a role in intercellular signalling.

In addition to the CTL and Sushi domains, these proteins also contain, in their N-terminal domain, an Ig-like V-type region, two or four link domains (see <PDOC00955>) and up to two EGF-like repeats.
Two type-I membrane proteins:

- Mannose receptor from macrophages. This protein mediates the endocytosis of glycoproteins by macrophages in several recognition and uptake processes. Its extracellular section consists of a fibronectin type II domain followed by eight tandem repeats of the CTL domain.
- 180 Kd secretory phospholipase A2 receptor (PLA2-R). A protein whose structure is highly similar to that of the mannose receptor.
- DEC-205 receptor. This protein is used by dendritic cells and thymic epithelial cells to capture and endocytose diverse carbohydrate-binding antigens and direct them to antigen-processing cellular compartiments. DEC-205 extracellular section consists of a fibronectin type II domain followed by ten tandem repeats of the CTL domain.
- Silk moth hemocytin, an humoral lectin which is involved in a self-defence mechanism. It is composed of 2 FA58C domains (see <PDOC00988>), a CTL domain, 2 VWFC domains (see <PDOC00928), and a CTCK (see <PDOC00912>).

Various other proteins that uniquely consist of a CTL domain:

- Invertebrate soluble galactose-binding lectins. A category to which belong a humoral lectin from a flesh fly; echinoidin, a lectin from the coelomic fluid of a sea urchin; BRA-2 and BRA-3, two lectins from the coelomic fluid of a barnacle, a lectin from the tunicate Polyandrocarpa misakiensis and a newt oviduct lectin. The physiological importance of these lectins is not yet known but they may play an important role in defense mechanisms.
- Pancreatic stone protein (PSP) (also known as pancreatic thread protein (PTP), or reg), a protein that might act as an inhibitor of spontaneous calcium carbonate precipitation.
- Pancreatitis associated protein (PAP), a protein that might be involved in the control of bacterial proliferation.
- Tetranectin, a plasma protein that binds to plasminogen and to isolated kringle 4.
- Eosinophil granule major basic protein (MBP), a cytotoxic protein.
- A galactose specific lectin from a rattlesnake.
- Two subunits of a coagulation factor IX/factor X-binding protein (IX/X-bp), a snake venom anticoagulant protein which binds with factors IX and X in the presence of calcium.
- Two subunits of a phospholipase A2 inhibitor from the plasma of a snake (PLI-A and PLI-B).
- A lipopolysaccharide-binding protein (LPS-BP) from the hemolymph of a cockroach [8].
- Sea raven antifreeze protein (AFP) [9].

As a signature pattern for this domain, the C-terminal region with its three conserved cysteines was selected.
Consensus patternC-[LIVMFYATG]-x(5,12)-[WL]-x-[DNSR]-x(2)-C-x(5,6)-[FYWLIVSTA]-[LIVMSTA]-C [The three C's are involved in disulfide bonds]
Note all CTL domains have five Trp residues before the second Cys, with the exception of tunicate lectin and cockroach LPS-BP which have Leu.
Note this documentation entry is linked to both a signature pattern and a profile. As the profile is much more sensitive than the pattern, you should use it if you have access to the necessary software tools to do so.

[1] Drickamer K. J. Biol. Chem. 263:9557-9560(1988).
[2] Drickamer K. Prog. Nucleic Acid Res. Mol. Biol. 45:207-232(1993).
[3] Drickamer K. Curr. Opin. Struct. Biol. 3:393-400(1993).
[4] Spiess M. Biochemistry 29:10009-10018(1990).
[5] Weis W.I., Kahn R., Fourme R., Drickamer K., Hendrickson W.A. Science 254:1608-1615(1991).
[6] Siegelman M. Curr. Biol. 1:125-128(1991).
[7] Lasky L.A. Science 238:964-969(1992).
[8] Jomori T., Natori S. J. Biol. Chem. 266:13318-13323(1991).
[9] Ng N.F.L., Hew C.-L. J. Biol. Chem. 267:16069-16075(1992).

764. (SRCR) Speract receptor repeated domain signature
PROSITE cross-reference(s): PS00420; SPERACT_RECEPTOR,
The receptor for the sea urchin egg peptide speract is a transmembrane glycoprotein of 500 amino acid residues [1]. Structurally it consists of a large extracellular domain of 450 residues, followed by a transmembrane region and a small cytoplasmic domain of 12 amino acids. The extracellular domain contains four repeats of a 115 amino acids domain. There are 17 positions that are perfectly conserved in the four repeats, among them are six cysteines, six glycines, and three glutamates.
Such a domain is also found, once, in the C-terminal section of mammalian macrophage scavenger receptor type I [2], a membrane glycoproteins implicated in the pathologic deposition of cholesterol in arterial walls during atherogenesis. The signature pattern that was derived spans part of the N-terminal section of the domain and contains 8 of the 17 conserved residues.
Consensus patternG-x(5)-G-x(2)-E-x(6)-W-G-x(2)-C-x(3)-[FYW]-x(8)-C-x(3)-G

[1] Dangott J.J., Jordan J.E., Bellet R.A., Garbers D.L. Proc. Natl. Acad. Sci. U.S.A. 86:2128-2132(1989).
[2] Freeman M., Ashkenas J., Rees D.J., Kingsley D.M., Copeland N.G., Jenkins N.A., Krieger M. Proc. Natl. Acad. Sci. U.S.A. 87:8810-8814(1990).

765. Bac_surface_Ag
Bacterial surface antigen
This entry includes the following surface antigens; D15 antigen from H.influenzae, OMA87 from P.multocida, OMP85 from N.meningitidis and N.gonorrhoeae. Number of members: 14

[1]Medline: 95255676. The sequencing of the 80-kDa D15 protective surface antigen of Haemophilus influenzae. Flack FS, Loosmore S, Chong P, Thomas WR; Gene 1995;156:97-99.
[2] Medline: 96333354. Cloning, sequencing, expression, and protective capacity of the oma87 gene encoding the Pasteurella multocida 87-kilodalton outer membrane antigen. Ruffolo CG, Adler B; Infect Immun 1996;64:3161-3167.

766. BRCA1 C Terminus (BRCT) domain
The BRCT domain is found predominantly in proteins involved in cell cycle checkpoint functions responsive to DNA damage. It has been suggested that the Retinoblastoma protein contains a divergent BRCT domain, this has not been included in this family. The BRCT domain of XRCC 1 forms a homodimer in the crystal structure Medline:99016060. This suggests that pairs of BRCT domains associate as homo- or heterodimers. Number of members: 131

[1] Medline: 96259550. BRCA1 protein products ...Functional motifs... Koonin EV, Altschul SF, Bork P; Nature Genet 1996;13:266-268.
[2] Medline: 97153217. From BRCA1 to RAP1: A widespread BRCT module closely associated with DNA repair Callebaut I, Mornon JP; Febs lett 1997;400:25-30.
[3] Medline: 97186552. A superfamily of conserved domains in DNA damage responsive cell cycle checkpoint proteins Bork P, Hofmann K, Bucher P, Neuwald AF, Altschul SF, Koonin EV; Faseb J 1997;11:68-76.
[4] Medline: 97402527. Gapped BLAST and PSI-BLAST: a new generation of protein database search programs. Altschul SF, Madden TL, Schaffer AA, Zhang J, Zhang Z, Miller W, Lipman DJ; Nucleic Acids Res 1997;25:3389-3402.
[5] Medline: 99016060. Structure of an XRCC1 BRCT domain: a new protein-protein interaction module. Zhang X, Morera S, Bates PA, Whitehead PC, Coffer AI, Hainbucher K, Nash RA, Sternberg MJ, Lindahl T, Freemont PS;

767. Kappa casein
Kappa-casein is a mammalian milk protein involved in a number of important physiological processes. In the gut, the ingested protein is split into an insoluble peptide (para kappa-casein) and a soluble hydrophilic glycopeptide (caseinomacropeptide). Caseinomacropeptide is responsible for increased efficiency of digestion, prevention of neonate hypersensitivity to ingested proteins, and inhibition of gastric pathogens. Number of members: 56

[1] Medline: 98072500. Nucleotide sequence evolution at the kappa-casein locus: evidence for positive selection within the family Bovidae. Ward TJ, Honeycutt RL, Derr JN; Genetics 1997;147:1863-1872.

768. Chitinases family 18 active site
PROSITE cross-reference(s) CHITINASE_18
Chitinases (EC 3.2.1.14) [1] are enzymes that catalyze the hydrolysis of the beta-1,4-N-acetyl-D-glucosamine linkages in chitin polymers. From the view point of sequence similarity chitinases belong to either family 18 or 19 in the classification of glycosyl hydrolases [2,E1]. Chitinases of family 18 (also known as classes III or V) groups a variety of proteins:

a) Chitinases from:

- Prokaryotes such as Alteromonas, Bacillus, Serratia, Streptomyces, etc.
- Plants such as Arabidopsis, cucumber, bean, tobacco, etc.
- Fungi such as Aphanocladium, Rhizopus, Saccharomyces, etc.
- Nematode (Brugia malayi).
- Insects (Manduca sexta).
- Baculoviruses (Autographa Californica Nuclear Polyhedrosis virus).

b) Other proteins:

- Hevamine, a rubber tree protein with chitinase and lysozyme activities.
- Kluyveromyces lactis killer toxin alpha subunit, which acts as a chitinase.
- Flavobacterium and Streptomyces endo-beta-N-acetylglucosaminidases (EC 3.2.1.96).
- Mammalian di-N-acetylchitobiase which is involved in the degradation of asparagine-linked glycoproteins.
- Human cartilage glycoprotein Gp-39.
- Jack bean concanavalin B (conB), a protein that has lost its catalytic activity.

Site directed mutagenesis experiments [3] and crystallographic data [4,5] have shown that a conserved glutamate is involved in the catalytic mechanism and probably acts as a proton donor. This glutamate is at the extremity of the best conserved region in these proteins.

Consensus pattern[LIVMFY]-[DN]-G-[LIVMF]-[DN]-[LIVMF]-[DN]-x-E [E is the active site residue]

[1] Flach J., Pilet P.-E., Jolles P. Experientia 48:701-716(1992).
[2] Henrissat B. Biochem. J. 280:309-316(1991).
[3] Watanabe T., Kohori K., Miyashita K., Fujii T., Sakai H., Uchida M., Tanaka H. J. Biol. Chem. 268:18567-18572 (1993).
[4] Perrakis A., Tews I., Dauter Z., Oppenheim A.B., Chet I., Wilson K.S., Vorgias C.E. Structure 2:1169-1180(1994).
[5] van Scheltinga A.C.T., Kalk K.H., Beintema J.J., Dijkstra B.W. Structure 2:1181-1189(1994).

769. gag_p17. gag gene protein p17 (matrix protein).
The matrix protein forms an icosahedral shell associated with the inner membrane of the mature immunodeficiency virus. Number of members: 1598

[1] Medline: 95055757. Three-dimensional structure of the human immunodeficiency virus type 1 matrix protein. Massiah MA, Starich MR, Paschall C, Summers MF, Christensen AM, Sundquist WI; J Mol Biol 1994;244:198-223.

770. GDA1/CD39 family of nucleoside phosphatases signature
PROSITE cross-reference(s); GDA1_CD39_NTPASE
A number of nucleoside diphosphate and triphosphate hydrolases as well as some yet uncharacterized proteins have been found to belong to the same family [1, 2]. This family currently consist of:

- Yeast guanosine-diphosphatase (EC 3.6.1.42) (GDPase) (gene GDA1). GDA1 is a golgi integral membrane enzyme that catalyzes the hydrolysis of GDP to GMP.
- Potato apyrase (EC 3.6.1.5) (adenosine diphosphatase) (ADPase). Apyrase acts on both ATP and ADP to produce AMP.
- Mammalian vascular ATP-diphosphohydrolase (EC 3.6.1.5) (also known as lymphoid cell activation antigen CD39).
- Toxoplasma gondii nucleoside-triphosphatases (EC 3.6.1.15) (NTPase). NTPase hydrolyses various nucleoside triphosphates to produce the corresponding nucleoside mono- and diphosphates. This enzyme is secreted into the invaded host cell into the parasitophorous vacuole, a specialized compartment where the parasite intracellulary resides.
- Pea nucleoside-triphosphatases (EC 3.6.1.15) (NTPase).

- Caenorhabditis elegans hypothetical protein C33H5.14.
- Caenorhabditis elegans hypothetical protein R07E4.4.
- Yeast chromosome V hypothetical protein YER005w.

The above uncharacterized proteins all seem to be membrane-bound.
All these proteins share a number of conserved domains. The best conserved of these domains have been selected. It is located in the central section of the proteins.
Consensus pattern[LIVM]-x-G-x(2)-E-G-x-[FY]-x-[FW]-[LIVA]-[TAG]-x-N-[HY]

[1] Handa M., Guidotti G. Biochem. Biophys. Res. Commun. 218:916-923(1996).
[2] Vasconcelos E.G., Ferreira S.T., de Carvalho T.M.U., de Souza W., Kettlun A.M., Mancilla M., Valenzuela M.A., Verjovski-Almeida S. J. Biol. Chem. 271:22139-22145(1996).

771. GTP cyclohydrolase I signatures
PROSITE cross-reference(s); GTP_CYCLOHYDROL_1_1, GTP_CYCLOHYDROL_1_2 GTP cyclohydrolase I (EC 3.5.4.16) catalyzes the biosynthesis of formic acid and dihydroneopterin triphosphate from GTP. This reaction is the first step in the biosynthesis of tetrahydrofolate in prokaryotes, of tetrahydrobiopterin in vertebrates, and of pteridine-containing pigments in insects.
GTP cyclohydrolase I is a protein of from 190 to 250 amino acid residues. The comparison of the sequence of the enzyme from bacterial and eukaryotic sources shows that the structure of this enzyme has been extremely well conserved throughout evolution [1].
Two conserved regions were selected as signature patterns. The first contains a perfectly conserved tetrapeptide which is part of the GTP-binding pocket [2], the second region also contains conserved residues involved in GTP-binding.
Consensus pattern[DEN]-[LIVM](2)-x(2)-[KRNQ]-[DEN]-[LIVM]-x(3)-[ST]-x-C-E- H-H
Consensus pattern[SA]-x-[RK]-x-Q-[LIVM]-Q-E-[RN]-[LI]-[TSN]

[1] Maier J., Witter K., Guetlich M., Ziegler I., Werner T., Ninnemann H. Biochem. Biophys. Res. Commun. 212: 705-711 (1995).
[2] Nar H., Huber R., Meining W., Schmid C., Weinkauf S., Bacher A. Structure 3:459-466(1995).

772. IlvC. Acetohydroxy acid isomeroreductase
Acetohydroxy acid isomeroreductase catalyses the conversion of acetohydroxy acids into dihydroxy valerates. This reaction is the second in the synthetic pathway of the essential branched side chain amino acids valine and isoleucine. Number of members: 29

[1] Medline: 97361822. The crystal structure of plant acetohydroxy acid isomeroreductase complexed with NADPH, two magnesium ions and a herbicidal transition state analog determined at 1.65 A resolution. Biou V, Dumas R, Cohen-Addad C, Douce R, Job D, Pebay-Peyroula E; EMBO J 1997;16:3405-3415.

773. Prokaryotic membrane lipoprotein lipid attachment site
PROSITE cross-reference(s); PROKAR _LIPOPROTEIN
In prokaryotes, membrane lipoproteins are synthesized with a precursor signal peptide, which is cleaved by a specific lipoprotein signal peptidase (signal peptidase II). The peptidase recognizes a conserved sequence and cuts upstream of a cysteine residue to which a glyceride-fatty acid lipid is attached [1]. Some of the proteins known to undergo such processing currently include (for recent listings see [1,2,3]):

- Major outer membrane lipoprotein (murein-lipoproteins) (gene 1pp).
- Escherichia coli lipoprotein-28 (gene nlpA).
- Escherichia coli lipoprotein-34 (gene nlpB).
- Escherichia coli lipoprotein nlpC.
- Escherichia coli lipoprotein nlpD.
- Escherichia coli osmotically inducible lipoprotein B (gene osmB).
- Escherichia coli osmotically inducible lipoprotein E (gene osmE).
- Escherichia coli peptidoglycan-associated lipoprotein (gene pal).
- Escherichia coli rare lipoproteins A and B (genes rplA and rplB).
- Escherichia coli copper homeostasis protein cutF (or nlpE).
- Escherichia coli plasmids traT proteins.
- Escherichia coli Col plasmids lysis proteins.
- A number of Bacillus beta-lactamases.
- Bacillus subtilis periplasmic oligopeptide-binding protein (gene oppA).
- Borrelia burgdorferi outer surface proteins A and B (genes ospA and ospB).
- Borrelia hermsii variable major protein 21 (gene vmp21) and 7 (gene vmp7).
- Chlamydia trachomatis outer membrane protein 3 (gene omp3).
- Fibrobacter succinogenes endoglucanase cel-3.
- Haemophilus influenzae proteins Pal and Pcp.
- Klebsiella pullulunase (gene pulA).
- Klebsiella pullulunase secretion protein pulS.
- Mycoplasma hyorhinis protein p37.
- Mycoplasma hyorhinis variant surface antigens A, B, and C (genes vlpABC).
- Neisseria outer membrane protein H.8.
- Pseudomonas aeruginosa lipopeptide (gene IppL).
- Pseudomonas solanacearum endoglucanase egl.
- Rhodopseudomonas viridis reaction center cytochrome subunit (gene cytC).
- Rickettsia 17 Kd antigen.
- Shigella flexneri invasion plasmid proteins mxiJ and mxiM.
- Streptococcus pneumoniae oligopeptide transport protein A (gene amiA).
- Treponema pallidium 34 Kd antigen.
- Treponema pallidium membrane protein A (gene tmpA).
- Vibrio harveyi chitobiase (gene chb).
- Yersinia virulence plasmid protein yscJ.

- Halocyanin from Natrobacterium pharaonis [4], a membrane associated copper- binding protein. This is the first archaebacterial protein known to be modified in such a fashion).

From the precursor sequences of all these proteins, we derived a consensus pattern and a set of rules to identify this type ofpost-translational modification.
Consensus pattem{DERK}(6)-[LIVMFWSTAG](2)-[LIVMFYSTAGCQ]-[AGS]-C [C is the lipid attachment site] Additional rules: 1) The cysteine must be between positions 15 and 35 of the sequence in consideration. 2) There must be at least one Lys or one Arg in the first seven positions of the sequence.

[1] Hayashi S., WuH.C. J. Bioenerg. Biomembr. 22:451-471(1990).
[2]Klein P., Somorjai R.L., Lau P.C.K. Protein Eng. 2:15-20(1988).
[3]von Heijne G. Protein Eng. 2:531-534(1989).
[4]Mattar S., Scharf B., Kent S.B.H., Rodewald K., Oesterhelt D., Engelhard M. J. Biol. Chem. 269:14939-14945 (1994).

774. Aminoacyl-transfer RNA synthetases class-II signatures
PROSITE cross-reference(s); AA_TRNA_LIGASE_II_1; AA_TRNA_LIGASE_II_2 Aminoacyl-tRNA synthetases (EC 6.1.1.-) [1] are a group of enzymes which activate amino acids and transfer them to specific tRNA molecules as the first step in protein biosynthesis. In prokaryotic organisms there are at least twenty different types of aminoacyl-tRNA synthetases, one for each different amino acid. In eukaryotes there are generally two aminoacyl-tRNA synthetases for each different amino acid: one cytosolic form and a mitochondrial form. While all these enzymes have a common function, they are widely diverse in terms of subunit size and of quaternary structure.
The synthetases specific for alanine, asparagine, aspartic acid, glycine, histidine, lysine, phenylalanine, proline, serine, and threonine are referred to as class-II synthetases [2 to 6] and probably have a common folding pattern in their catalytic domain for the binding of ATP and amino acid which is different to the Rossmann fold observed for the class I synthetases [7].
Class-II tRNA synthetases do not share a high degree of similarity, however at least three conserved regions are present [2,5,8]. Signature patterns from two of these regions have been derived.
Consensus pattern[FYH]-R-x-[DE]-x(4,12)-[RH]-x(3)-F-x(3)-[DE]
Consensus pattern[GSTALVF]-{DENQHRKP}-[GSTA]-[LIVMF]-[DE]-R-[LIVMF]-x-[LIVMSTAG]-[LIVMFY]

[1]Schimmel P. Annu. Rev. Biochem. 56:125-158(1987).
[2]Delarue M., Moras D. BioEssays 15:675-687(1993).
[3]Schimmel P. Trends Biochem. Sci. 16:1-3(1991).
[4]Nagel G.M., Doolittle R.F. Proc. Natl. Acad. Sci. U.S.A. 88:8121-8125(1991).
[5]Cusack S., Haertlein M., Leberman R. Nucleic Acids Res. 19:3489-3498(1991).
[6]Cusack S. Biochimie 75:1077-1081(1993).
[7]Cusack S., Berthet-Colominas C., Haertlein M., Nassar N., Leberman R. Nature 347:249-255(1990).
[8]Leveque F., Plateau P., Dessen P., Blanquet S. Nucleic Acids Res. 18:305-312(1990).

775. X. Trans-activation protein X
This protein is found in hepadnaviruses where it is indispensable for replication. Number of members: 91
776. Thymidylate synthase active site
Thymidylate synthase (EC 2.1.1.45) [1,2] catalyzes the reductive methylation of dUMP to dTMP with concomitant conversion of 5,10-methylenetetrahydrofolate to dihydrofolate. Thymidylate synthase plays an essential role in DNA synthesis and is an important target for certain chemotherapeutic drugs.
Thymidylate synthase is an enzyme of about 30 to 35 Kd in most species except in protozoan and plants where it exists as a bifunctional enzyme that includes a dihydrofolate reductase domain.
A cysteine residue is involved in the catalytic mechanism (it covalently binds the 5,6-dihydro-dUMP intermediate). The sequence around the active site of this enzyme is conserved from phages to vertebrates.
Consensus patternR-x(2)-[LIVM]-x(3)-[FW]-[QN]-x(8,9)-[LV]-x-P-C-[HAVM]-x(3)-[QMT]-[FYW]-x-[LV] [C is the active site residue]

[1] Benkovic S.J. Annu. Rev. Biochem. 49:227-251(1980).
[2] Ross P., O'Gara F., Condon S. Appl. Environ. Microbiol. 56:2156-2163(1990).

777. Glycosyl hydrolases family 31 signatures
It has been shown [1,2,3,E1] that the following glycosyl hydrolases can be, on the basis of sequence similarities, classified into a single family:

- Lysosomal alpha-glucosidase (EC 3.2.1.20) (acid maltase) is a vertebrate glycosidase active at low pH, which

hydrolyzes alpha(1->4) and alpha(1->6) linkages in glycogen, maltose, and isomaltose.

- Alpha-glucosidase (EC 3.2.1.20) from the yeast Candida tsukunbaensis.
- Alpha-glucosidase (EC 3.2.1.20) (gene malA) from the archebacteria Sulfolobus solfataricus.
- Intestinal sucrase-isomaltase (EC 3.2.1.48 / EC 3.2.1.10) is a vertebrate membrane-bound, multifunctional enzyme complex which hydrolyzes sucrose, maltose and isomaltose. The sucrase and isomaltase domains of the enzyme are homologous (41 % of amino acid identity) and have most probably evolved by duplication.
- Glucoamylase 1 (EC 3.2.1.3) (glucan 1,4-alpha-glucosidase) from various fungal species.
- Yeast hypothetical protein YBR229c.
- Fission yeast hypothetical protein SpAC30D11.01c.

An aspartic acid has been implicated [4] in the catalytic activity of sucrase, isomaltase, and lysosomal alpha-glucosidase. The region around this active residue is highly conserved and can be used as a signature pattern. A second region, which contains two conserved cysteines, has been used as an additional signature pattern.
Consensus pattern [GF]-[LIVMF]-W-x-D-M-[NSA]-E [D is the active site residue]
Consensus pattern G-[AV]-D-[LIVMTA]-C-G-[FY]-x(3)-[ST]-x(3)-L-C-x-R-W-x(2)-[LV]-[GSA]-[SA]-F-x-P-F-x-R-[DN]

[1] HenrissatB. Biochem. J. 280:309-316(1991).
[2] Kinsella B.T., Hogan S., Larkin A., Cantwell B.A. Eur. J. Biochem. 202:657-664(1991).
[3] Naim H.Y., Niermann T., Kleinhans U., Hollenberg C.P., Strasser A.W.M. FEBS Lett. 294:109-112(1991).
[4] Hermans M.M.P., Kroos M.A., van Beeumen J., Oostra B.A., Reuser A.J.J. J. Biol. Chem. 266:13507-13512 (1991).

778. Urease signatures
Urease (EC 3.5.1.5) is a nickel-binding enzyme that catalyzes the hydrolysis of urea to carbon dioxide and ammonia [1]. Historically, it was the first enzyme to be crystallized (in 1926). It is mainly found in plant seeds, microorganisms and invertebrates. In plants, urease is a hexamer of identical chains. In bacteria [2], it consists of either two or three different subunits (alpha, beta and gamma).
Urease binds two nickel ions per subunit; four histidine, an aspartate and a carbamated-lysine serve as ligands to these metals; an additional histidine is involved in the catalytic mechanism [3].
As signatures for this enzyme, a region was selected that contains two histidine that bind one of the nickel ions and the region of the active site histidine.
Consensus pattern T-[AY]-[GA]-[GAT]-[LIVM]-D-x-H-[LIVM]-H-x(3)-P [The two H's bind nickel]
Consensus pattern [LIVM](2)-[CT]-H-[HN]-L-x(3)-[LIVM]-x(2)-D-[LIVM]-x-F-A [H is the active site residue]

[1] Takishima K., Suga T., Mamiya G. Eur. J. Biochem. 175:151-165(1988).
[2] Mobley H.L.T., Husinger R.P. Microbiol. Rev. 53:85-108(1989).
[3] Jabri E., Carr M.B., Hausinger R.P., Karplus P.A. Science 268:998-1004(1995).

779. Tyrosine specific protein phosphatases signature and profiles
Tyrosine specific protein phosphatases (EC 3.1.3.48) (PTPase) [1 to 5] are enzymes that catalyze the removal of a phosphate group attached to a tyrosine residue. These enzymes are very important in the control of cell growth, proliferation, differentiation and transformation. Multiple forms of PTPase have been characterized and can be classified into two categories: soluble PTPases and transmembrane receptor proteins that contain PTPase domain(s). The currently known PTPases are listed below:
Soluble PTPases.

- PTPN1 (PTP-1B).
- PTPN2 (T-cell PTPase; TC-PTP).
- PTPN3 (H1) and PTPN4 (MEG), enzymes that contain an N-terminal band 4.1- like domain (see <PDOC00566>) and could act at junctions between the membrane and cytoskeleton.
- PTPN5 (STEP).
- PTPN6 (PTP-1C; HCP; SHP) and PTPN11 l (PTP-2C; SH-PTP3; Syp), enzymes which contain two copies of the SH2 domain at its N-terminal extremity. The Drosophila protein corkscrew (gene csw) also belongs to this subgroup.
- PTPN7 (LC-PTP; Hematopoietic protein-tyrosine phosphatase; HePTP).
- PTPN8 (70Z-PEP).
- PTPN9 (MEG2).
- PTPN12 (PTP-G1; PTP-P19).
- Yeast PTP1.

- Yeast PTP2 which may be involved in the ubiquitin-mediated protein degradation pathway.
- Fission yeast pyp 1 and pyp2 which play a role in inhibiting the onset of mitosis.
- Fission yeast pyp3 which contributes to the dephosphorylation of cdc2.
- Yeast CDC14 which may be involved in chromosome segregation.
- Yersinia virulence plasmid PTPAses (gene yopH).
- Autographa californica nuclear polyhedrosis virus 19 Kd PTPase.

Dual specificity PTPases.

- DUSP1 (PTPN10; MAP kinase phosphatase-1; MKP-1); which dephosphorylates MAP kinase on both Thr-183 and Tyr-185.
- DUSP2 (PAC-1), a nuclear enzyme that dephosphorylates MAP kinases ERK1 and ERK2 on both Thr and Tyr residues.
- DUSP3 (VHR).
- DUSP4 (HVH2).
- DUSP5 (HVH3).
- DUSP6 (Pyst1 MKP-3).
- DUSP7 (Pyst2; MKP-X).
- Yeast MSG5, a PTPase that dephosphorylates MAP kinase FUS3.
- Yeast YVH1.
- Vaccinia virus H1 PTPase; a dual specificity phosphatase.

Receptor PTPases.
Structurally, all known receptor PTPases, are made up of a variable length extracellular domain, followed by a transmembrane region and a C-terminal catalytic cytoplasmic domain. Some of the receptor PTPases contain fibronectin type III (FN-III) repeats, immunoglobulin-like domains, MAM domains or carbonic anhydrase-like domains in their extracellular region. The cytoplasmic region generally contains two copies of the PTPAse domain. The first seems to have enzymatic activity, while the second is inactive but seems to affect substrate specificity of the first. In these domains, the catalytic cysteine is generally conserved but some other, presumably important, residues are not.
In the following table, the domain structure of known receptor PTPases is shown:

| | Extracellular | | | | Intracellular |
|---|---|---|---|---|---|
| | Ig | FN-3 | CAH | MAM | PTPase |
| Leukocyte common antigen (LCA) (CD45) | 0 | 2 | 0 | 0 | 2 |
| Leukocyte antigen related (LAR) | 3 | 8 | 0 | 0 | 2 |
| Drosophila DLAR | 3 | 9 | 0 | 0 | 2 |
| Drosophila DPTP | 2 | 2 | 0 | 0 | 2 |
| PTP-alpha (LRP) | 0 | 0 | 0 | 0 | 2 |
| PTP-beta | 0 | 16 | 0 | 0 | 1 |
| PTP-gamma | 0 | 1 | 1 | 0 | 2 |
| PTP-delta | 0 | >7 | 0 | 0 | 2 |
| PTP-epsilon | 0 | 0 | 0 | 0 | 2 |
| PTP-kappa | 1 | 4 | 0 | 1 | 2 |
| PTP-mu | 1 | 4 | 0 | 1 | 2 |
| PTP-zeta | 0 | 1 | 1 | 0 | 2 |

PTPase domains consist of about 300 amino acids. There are two conserved cysteines, the second one has been shown to be absolutely required for activity. Furthermore, a number of conserved residues in its immediate vicinity have also been shown to be important.

A signature pattern was derived for PTPase domains centered on the active site cysteine.
There are three profiles for PTPases, the first one spans the complete domain and is not specific to any subtype. The second profile is specific to dual-specificity PTPases and the third one to the PTP subfamily.
Consensus pattern [LIVMF]-H-C-x(2)-G-x(3)-[STC]-[STAGP]-x-[LIVMFY] [C is the active site residue]
Note the M-phase inducer phosphatases (cdc25-type phosphatase) are tyrosine-protein phosphatases that are not structurally related to the above PTPases.
Notethis documentation entry is linked to both a signature pattern and to profiles. As profiles are much more sensitive than the pattern, you should use them if you have access to the necessary software tools to do so.

[1] Fischer E.H., Charbonneau H., Tonks N.K. Science 253:401-406(1991).
[2] Charbonneau H., Tonks N.K. Annu. Rev. Cell Biol. 8:463-493(1992).
[3] Trowbridge I.S. J. Biol. Chem. 266:23517-23520(1991).
[4] Tonks N.K., Charbonneau H. Trends Biochem. Sci. 14:497-500(1989).
[5] Hunter T. Cell 58:1013-1016(1989).

780. Connexins signatures
Gap junctions [1] are specialized regions of the plasma membrane which consist of closely packed pairs of transmembrane channels, the connexons, through which small molecules diffuse from a cell to a neighboring cell. Each connexon is composed of an hexamer of an integral membrane protein which is often referred to as connexin. In a given species there are a number of different, yet structurally related, tissue specific, forms of connexins. The types of connexins which are currently known are listed below.

- Connexin 56 (Cx56).
- Connexin 50 (Cx50) (lens fiber protein MP70).
- Connexin 46 (Cx46) (alpha-3).
- Connexin 45 (Cx45) (alpha-6).
- Connexin 43 (Cx43) (alpha-1).
- Connexin 40 (Cx40) (alpha-5).
- Connexin 38 (Cx38) (alpha-2).
- Connexin 37 (Cx37) (alpha-4).
- Connexin 33 (Cx33) (alpha-7).
- Connexin 32 (Cx32) (beta-1).
- Connexin 31.1 (Cx31.1) (beta-4).
- Connexin 31 (Cx31) (beta-3).
- Connexin 30.3 (Cx30.3) (beta-5).
- Connexin 26 (Cx26) (beta-2).

Structurally the connexins consist of a short cytoplasmic N-terminal domain, followed by four transmembrane segments that delimit two extracellular and one cytoplasmic loops; the C-terminal domain is cytoplasmic and its length is variable (from 20 residues in Cx26 to 260 residues in Cx56). The schematic representation of this structure is shown below.

```
NH2-***     ***      *************-COOH
   **   **  **    **
   **   **   **  **  Cytoplasmic
___**____**_____**____**_______________
   **   **   **  **  Membrane
   **   **   **  **
___**____**_____**____**_______________
   **   **   **  **  Extracellular
```

** ** ** **

** **

The sequences of the two extracellular loops are well conserved. In both loops there are three conserved cysteines which are involved in disulfide bonds. A signature patterns from each of these two loop regions has been built. Consensus patternC-[DN]-T-x-Q-P-G-C-x(2)-V-C-[FY]-D [The three C's are involved in disulfide bonds] Consensus patternC-x(3,4)-P-C-x(3)-[LIVM]-[DEN]-C-[FY]-[LIVM]-[SA]-[KR]-P [The three C's are involved in disulfide bonds]

[1] Goodenough D.A., Goliger J.A., Paul D.L. Annu. Rev. Biochem. 65:475-502(1996).

781. Gram-positive cocci surface proteins 'anchoring'hexapeptide

Surface proteins from Gram-positive cocci contains a conserved hexapeptide located a few residues downstream of a hydrophobic C-terminal membrane anchor region which is followed by a cluster of basic amino acids [1]. This structure is represented in the following schematic representation:

```
+-----------------------------------------+-+--------+-+
|   Variable length extracellular domain  |H| Anchor |B|
+-----------------------------------------+-+--------+-+
```

'H': conserved hexapeptide.

'B': cluster of basic residues. It has been proposed that this hexapeptide sequence is responsible for a post-translational modification necessary for the proper anchoring of the proteins which bear it, to the cell wall.

Proteins known to contain such hexapeptide are listed below:

- Aggregation substance from streptococcus faecalis (asa1).
- C5a peptidase from Streptococcus pyogenes (scpA).
- C protein alpha-antigen from Streptococcus agalactiae (bca).
- Cell surface antigen I/II (PAC) from Streptococcus mutans.
- Dextranase from Streptococcus downei (dex).
- Fibronectin-binding protein from Staphylococcus aureus (fnbA).
- Fimbrial subunits from Actinomyces naeslundii and viscosus.
- IgA binding protein from Streptococcus pyogenes (arp4).
- IgA binding protein (B antigen) from Streptococcus agalactiae (bag).
- IgG binding proteins from Streptococci and Staphylococcus aureus.
- Internalin A from Listeria monocytogenes (in1A).
- M proteins from streptococci.
- Muramidase-released protein from Streptococcus suis (mrp).
- Nisin leader peptide processing protease from Lactococcus lactis (nisP).
- Protein A from Staphylococcus aureus.
- Trypsin-resistant surface T protein from streptococci.
- Wall-associated protein from Streptococcus mutans (wapA).
- Wall-associated serine proteinases from Lactococcus lactis.

Consensus patternL-P-x-T-G-[STGAVDE]

[1] Schneewind O., Jones K.F., Fischetti V.A. J. Bacteriol. 172:3310-3317(1990).

782. Gamma-glutamyltranspeptidase signature

Gamma-glutamyltranspeptidase (EC 2.3.2.2) (GGT) [1] catalyzes the transfer of the gamma-glutamyl moiety of glutathione to an acceptor that may be an amino acid, a peptide or water (forming glutamate). GGT plays a key role in the gamma-glutamyl cycle, a pathway for the synthesis and degradation of glutathione. In prokaryotes and eukaryotes, it is an enzyme that consists of two polypeptide chains, a heavy and a light subunit, processed from a single chain precursor. The active site of GGT is known to be located in the light subunit.

The sequences of mammalian and bacterial GGT show a number of regions of high similarity [2]. Pseudomonas cephalosporin acylases (EC 3.5.1.-) that convert 7-beta-(4-carboxybutanamido)-cephalosporanic acid (GL-7ACA) into 7-aminocephalosporanic acid (7ACA) and glutaric acid are evolutionary related to GGT and also show some GGT activity [3]. Like GGT, these GL-7ACA acylases, are also composed of two subunits.
One of the conserved regions correspond to the N-terminal extremity of the mature light chains of these enzymes. This region has been used as a signature pattern.
Consensus patternT-[STA]-H-x-[ST]-[LIVMA]-x(4)-G-[SN]-x-V-[STA]-x-T-x-T-[LIVM]-[NE]-x(1,2)-[FY]-G

[1] Tate S.S., MeisterA. Meth. Enzymol. 113:400-419(1985).
[2] Suzuki H., Kumagai H., Echigo T., Tochikura T. J. Bacteriol. 171:5169-5172(1989).
[3] Ishiye M., Niwa M. Biochim. Biophys. Acta 1132:233-239(1992).

783. Ferrochelatase signature
Ferrochelatase (EC 4.99.1.1) (protoheme ferro-lyase) [1,2] catalyzes the last step in heme biosynthesis: the chelation of a ferrous ion to proto-porphyrin IX, to form protoheme.
In eukaryotes, ferrochelatase is a mitochondrial protein bound to the inner membrane, whose active site faces the mitochondrial matrix. The mature form of eukaryotic ferrochelatase is composed of about 360 amino acids. In bacteria, ferrochelatase (gene hemH) [3] is a protein of from 310 to 380 amino acids.
The human autosomal dominant disease protoporphyria is due to the reduced activity of ferrochelatase.
The signature pattern for this enzyme is based on a conserved region which contains a histidine residue which could be involved in binding iron.
Consensus pattern[LIVMF](2)-x-[ST]-x-H-[GS]-[LIVM]-P-x(4,5)-[DENQKR]-x-G-[DP]-x(1,2)-Y

[1] Labbe-Bois R. J. Biol. Chem. 265:7278-7283(1990).
[2] Brenner D.A., Frasier F. Proc. Natl. Acad. Sci. U.S.A. 88:849-853(1991).
[3] Miyamoto K., Nakahigashi K., Nishimura K., Inokuchi H. J. Mol. Biol. 219:393-398(1991).

784. Cellulose-binding domain, bacterial type
The microbial degradation of cellulose and xylans requires several types of enzyme such as endoglucanases (EC 3.2.1.4), cellobiohydrolases (EC 3.2.1.91) (exoglucanases), or xylanases (EC 3.2.1.8) [1].
Structurally, cellulases and xylanases generally consist of a catalytic domain joined to a cellulose-binding domain (CBD) by a short linker sequence rich in proline and/or hydroxy-amino acids.
The CBD of a number of bacterial cellulases has been shown to consist of about 105 amino acid residues [2]. Enzymes known to contain such a domain are:

- Endoglucanase (gene end1) from Butyrivibrio fibrisolvens.
- Endoglucanases A (gene cenA) and B (cenB) from Cellulomonas fimi.
- Exoglucanases A (gene cbhA) and B (cbhB) from Cellulomonas fimi.
- Endoglucanase E-2 (gene celB) from Thermomonospora fusca.
- Endoglucanase A (gene celA) from Microbispora bispora.
- Endoglucanases A (gene celA), B (celB) and C (celC) from Pseudomonas fluorescens.
- Endoglucanase A (gene celA) from Streptomyces lividans.
- Exocellobiohydrolase (gene cex) from Cellulomonas fimi.
- Xylanases A (gene xynA) and B (xynB) from Pseudomonas fluorescens.
- Arabinofuranosidase C (EC 3.2.1.55) (xylanase C) (gene xynC) from Pseudomonas fluorescens.
- Chitinase 63 (EC 3.2.1.14) from Streptomyces plicatus.
- Chitinase C from Streptomyces lividans.

The CBD domain is found either at the N-terminal or at the C-terminal extremity of these enzymes. As it is shown in the following schematic representation, there are two conserved cysteines in this CBD domain - one at each extremity of the domain - which have been shown [3] to be involved in a disulfide bond. There are also four conserved tryptophan residues which could be involved in the interaction of the CBD with polysaccharides.

```
+----------------------------------------------+
|                                  |
xCxxxxWxxxxxNxxxWxxxxxxxWxxxxxxxxWNxxxxxGxxxxxxxxxxCx
                    ********
```

'C': conserved cysteine involved in a disulfide bond. '*': position of the pattern.
Consensus patternW-N-[STAGR]-[STDN]-[LIVM]-x(2)-[GST]-x-[GST]-x(2)- [LIVMFT]-[GA]

[1] Gilkes N.R., Henrissat B., Kilburn D.G., Miller R.C. Jr., Warren R.A.J. Microbiol. Rev. 55:303-315(1991).
[2] Meinke A., Gilkes N.R., Kilburn D.G., Miller R.C. Jr., Warren R.A.J. Protein Seq. Data Anal. 4:349-353(1991).
[3] Gilkes N.R., Claeyssens M., Aebersold R., Henrissat B., Meinke A., Morrison H.D., Kilburn D.G., Warren R.A.J., Miller R.C. Jr. Eur. J. Biochem. 202:367-377(1991).

785. Amidases signature
It has been shown [1,2,3] that several enzymes from various prokaryotic and eukaryotic organisms which are involved in the hydrolysis of amides (amidases) are evolutionary related. These enzymes are listed below.

- Indoleacetamide hydrolase (EC 3.5.1.-), a bacterial plasmid-encoded enzyme that catalyzes the hydrolysis of indole-3-acetamide (IAM) into indole-3-acetate (IAA), the second step in the biosynthesis of auxins from tryptophan.
- Acetamidase from Emericella nidulans (gene amdS), an enzyme which allows acetamide to be used as a sole carbon or nitrogen source.
- Amidase (EC 3.5.1.4) from Rhodococcus sp. N-774 and Brevibacterium sp. R312 (gene amdA). This enzyme hydrolyzes propionamides efficiently, and also at a lower efficiency, acetamide, acrylamide and indoleacetamide.
- Amidase (EC 3.5.1.4) from Pseudomonas chlororaphis.
- 6-aminohexanoate-cyclic-dimer hydrolase (EC 3.5.2.12) (nylon oligomers degrading enzyme E1) (gene nylA), a bacterial plasmid encoded enzyme which catalyzes the first step in the degradation of 6-aminohexanoic acid cyclic dimer, a by-product of nylon manufacture [4].
- Glutamyl-tRNA(Gln) amidotransferase subunit A [5].
- Mammalian fatty acid amide hydrolase (gene FAAH) [6].
- A putative amidase from yeast (gene AMD2).
- Mycobacterium tuberculosis putative amidases amiA2, amiB2, amiC and amiD.

All these enzymes contain in their central section a highly conserved region rich in glycine, serine, and alanine residues. This region has been used as a signature pattern.
Consensus pattern: G-[GA]-S-[GS]-[GS]-G-x-[GSA]-[GSAVY]-x-[LIVM]-[GSA]-x(6)-[GSAT]-x-[GA]-x-[DE]-x-[GA]-x-S-[LIVM]-R-x-P-[GSAC]

[1] Mayaux J.-F., Cerbelaud E., Soubrier F., Faucher D., Petre D. J. Bacteriol. 172:6764-6773(1990).
[2] Hashimoto Y., Nishiyama M., Ikehata O., Horinouchi S., Beppu T. Biochim. Biophys. Acta 1088:225-233(1991).
[3] Chang T.-H., Abelson J. Nucleic Acids Res. 18:7180-7180(1990).
[4] Tsuchiya K., Fukuyama S., Kanzaki N., Kanagawa K., Negoro S., Okada H. J. Bacteriol. 171:3187-3191(1989).
[5] Curnow A.W., Hong K.W., Yuan R., Kim S.I., Martins O., Winkler W., Henkin T.M., Soll D. Proc. Natl. Acad. Sci. U.S.A. 94:11819-11826(1997).
[6] Cravatt B.F., Giang D.K., Mayfield S.P., Boger D.L., Lerner R.A., Gilula N.B. Nature 384:83-87(1996).

786. Glycosyl hydrolases family 10 active site
The microbial degradation of cellulose and xylans requires several types of enzymes such as endoglucanases (EC 3.2.1.4), cellobiohydrolases (EC 3.2.1.91) (exoglucanases), or xylanases (EC 3.2.1.8) [1,2]. Fungi and bacteria produces a spectrum of cellulolytic enzymes (cellulases) and xylanases which, on the basis of sequence similarities, can be classified into families. One of these families is known as the cellulase family F [3] or as the glycosyl hydrolases family 10 [4,E1]. The enzymes which are currently known to belong to this family are listed below.

- Aspergillus awamori xylanase A (xynA).
- Bacillus sp. strain 125 xylanase (xynA).

- Bacillus stearothermophilus xylanase.
- Butyrivibrio fibrisolvens xylanases A (xynA) and B (xynB).
- Caldocellum saccharolyticum bifunctional endoglucanase/exoglucanase (celB). This protein consists of two domains; it is the N-terminal domain, which has exoglucanase activity, which belongs to this family.
- Caldocellum saccharolyticum xylanase A (xynA).
- Caldocellum saccharolyticum ORF4. This hypothetical protein is encoded in the xynABC operon and is probably a xylanase.
- Cellulomonas fimi exoglucanase/xylanase (cex).
- Clostridium stercorarium thermostable celloxylanase.
- Clostridium thermocellum xylanases Y (xynY) and Z (xynZ).
- Cryptococcus albidus xylanase.
- Penicillium chrysogenum xylanase (gene xylP).
- Pseudomonas fluorescens xylanases A (xynA) and B (xynB).
- Ruminococcus flavefaciens bifunctional xylanase XYLA (xynA). This protein consists of three domains: a N-terminal xylanase catalytic domain that belongs to family 11 of glycosyl hydrolases; a central domain composed of short repeats of Gln, Asn an Trp, and a C-terminal xylanase catalytic domain that belongs to family 10 of glycosyl hydrolases.
- Streptomyces lividans xylanase A (xlnA).
- Thermoanaerobacter saccharolyticum endoxylanase A (xynA).
- Thermoascus aurantiacus xylanase.
- Thermophilic bacterium Rt8.B4 xylanase (xynA).

One of the conserved regions in these enzymes is centered on a conserved glutamic acid residue which has been shown [5], in the exoglucanase from Cellulomonas fimi, to be directly involved in glycosidic bond cleavage by acting as a nucleophile. This region has been used as a signature pattern.
Consensus pattern[GTA]-x(2)-[LIVN]-x-[IVMF]-[ST]-E-[LIY]-[DN]-[LIVMF] [E is the active site residue]

[1] Beguin P. Annu. Rev. Microbiol. 44:219-248(1990).
[2] Gilkes N.R., Henrissat B., Kilburn D.G., Miller R.C. Jr., Warren R.A.J. Microbiol. Rev. 55:303-315(1991).
[3] Henrissat B., Claeyssens M., Tomme P., Lemesle L., Mornon J.-P. Gene 81:83-95(1989).
[4] Henrissat B. Biochem. J. 280:309-316(1991).
[5] Tull D., Withers S.G., Gilkes N.R., Kilburn D.G., Warren R.A.J., Aebersold R. J. Biol. Chem. 266:15621-15625 (1991).

787. Fructose-bisphosphate aldolase class-II signatures
Fructose-bisphosphate aldolase (EC 4.1.2.13) [1,2] is a glycolytic enzyme that catalyzes the reversible aldol cleavage or condensation of fructose-1,6- bisphosphate into dihydroxyacetone-phosphate and glyceraldehyde 3-phosphate. There are two classes of fructose-bisphosphate aldolases with different catalytic mechanisms. Class-II aldolases [2], mainly found in prokaryotes and fungi, are homodimeric enzymes which require a divalent metal ion - generally zinc - for their activity.
This family also includes the following proteins:

- Escherichia coli galactitol operon protein gatY which catalyzes the transformation of tagatose 1,6-bisphosphate into glycerone phosphate and D- glyceraldehyde 3-phosphate.
- Escherichia coli N-acetyl galactosamine operon protein agaY which catalyzes the same reaction as that of gatY.

As signature patterns for this class of enzyme, two conserved regions were selected. The first pattern is located in the first half of the sequence and contains two histidine residues that have been shown [4] to be involved in binding a zinc ion. The second is located in the C-terminal section and contains clustered acidic residues and glycines.
Consensus pattern[FYVMT]-x(1,3)-[LIVMH]-[APN]-[LIVM]-x(1,2)-[LIVM]-H-x-D-H-[GACH] [The two H's are zinc ligands]
Consensus pattern[LIVM]-E-x-E-[LIVM]-G-x(2)-[GM]-[GSTA]-x-E

[1] Perham R.N. Biochem. Soc. Trans. 18:185-187(1990).
[2] Marsh J.J., Lebherz H.G. Trends Biochem. Sci. 17:110-113(1992).
[3] von der Osten C.H., Barbas C.F. III, Wong C.-H., Sinskey A.J. Mol. Microbiol. 3:1625-1637(1989).
[4] Berry A., Marshall K.E. FEBS Lett. 318:11-16(1993).

788. Prolyl oligopeptidase family serine active site

The prolyl oligopeptidase family [1,2,3] consist of a number of evolutionary related peptidases whose catalytic activity seems to be provided by a charge relay system similar to that of the trypsin family of serine proteases, but which evolved by independent convergent evolution. The known members of this family are listed below.

- Prolyl endopeptidase (EC 3.4.21.26) (PE) (also called post-proline cleaving enzyme). PE is an enzyme that cleaves peptide bonds on the C-terminal side of prolyl residues. The sequence of PE has been obtained from a mammalian species (pig) and from bacteria (Flavobacterium meningosepticum and Aeromonas hydrophila); there is a high degree of sequence conservation between these sequences.
- Escherichia coli protease II (EC 3.4.21.83) (oligopeptidase B) (gene prtB) which cleaves peptide bonds on the C-terminal side of lysyl and argininyl residues.
- Dipeptidyl peptidase IV (EC 3.4.14.5) (DPP IV). DPP IV is an enzyme that removes N-terminal dipeptides sequentially from polypeptides having unsubstituted N-termini provided that the penultimate residue is proline.
- Yeast vacuolar dipeptidyl aminopeptidase A (DPAP A) (gene: STE13) which is responsible for the proteolytic maturation of the alpha-factor precursor.
- Yeast vacuolar dipeptidyl aminopeptidase B (DPAP B) (gene: DAP2).
- Acylamino-acid-releasing enzyme (EC 3.4.19.1) (acyl-peptide hydrolase). This enzyme catalyzes the hydrolysis of the amino-terminal peptide bond of an N-acetylated protein to generate a N-acetylated amino acid and a protein with a free amino-terminus.

A conserved serine residue has experimentally been shown (in E.coli protease II as well as in pig and bacterial PE) to be necessary for the catalytic mechanism. This serine, which is part of the catalytic triad (Ser, His, Asp), is generally located about 150 residues away from the C-terminal extremity of these enzymes (which are all proteins that contains about 700 to 800 amino acids).
Consensus pattemD-x(3)-A-x(3)-[LIVMFYW]-x(14)-G-x-S-x-G-G-[LIVMFYW](2) [S is the active site residue]
Note these proteins belong to families S9A/S9B/S9C in the classification of peptidases [4,E1].

[1] Rawlings N.D., Polgar L., Barrett A.J. Biochem. J. 279:907-911(1991).
[2] Barrett A.J., Rawlings N.D. Biol. Chem. Hoppe-Seyler 373:353-360(1992).
[3] Polgar L., Szabo E. Biol. Chem. Hoppe-Seyler 373:361-366(1992).
[4] Rawlings N.D., Barrett A.J. Meth. Enzymol. 244:19-61(1994).

789. Formate--tetrahydrofolate ligase signatures
Formate--tetrahydrofolate ligase (EC 6.3.4.3) (formyltetrahydrofolate synthetase) (FTHFS) is one of the enzymes participating in the transfer of one-carbon units, an essential element of various biosynthetic pathways. In many of these processes the transfers of one-carbon units are mediated by the coenzyme tetrahydrofolate (THF). Various reactions generate one-carbon derivatives of THF which can be interconverted between different oxidation states by FTHFS, methylenetetrahydrofolate dehydrogenase (EC 1.5.1.5) and methenyltetrahydrofolate cyclohydrolase (EC 3.5.4.9).
In eukaryotes the FTHFS activity is expressed by a multifunctional enzyme, C-1-tetrahydrofolate synthase (C1-THF synthase), which also catalyzes the dehydrogenase and cyclohydrolase activities. Two forms of C1-THF synthases are known [1], one is located in the mitochondrial matrix, while the second one is cytoplasmic. In both forms the FTHFS domain consist of about 600 amino acid residues and is located in the C-terminal section of C1-THF synthase. In prokaryotes FTHFS activity is expressed by a monofunctional homotetrameric enzyme of about 560 amino acid residues [2].
The sequence of FTHFS is highly conserved in all forms of the enzyme. As signature patterns, two regions that are almost perfectly conserved were selected. The first one is a glycine-rich segment located in the N-terminal part of FTHFS and which could be part of an ATP-binding domain [2]. The second pattern is located in the central section of FTHFS.
Consensus patternG-[LIVM]-K-G-G-A-A-G-G-G-Y
Consensus pattern V-A-T-[IV]-R-A-L-K-x-[HN]-G-G

[1] Shannon K.W., Rabinowitz J.C. J. Biol. Chem. 263:7717-7725(1988).
[2] Lovell C.R., Przybyla A., Ljungdahl L.G. Biochemistry 29:5687-5694(1990).

790. Transthyretin signatures
Transthyretin (prealbumin) [1] is a thyroid hormone-binding protein that seems to transport thyroxine (T4) from the bloodstream to the brain. It is a protein of about 130 amino acids that assembles as a homotetramer and forms an internal channel that binds thyroxine. Transthyretin is mainly synthesized in the brain choroid plexus. In humans, variants of the protein are associated with distinct forms of amyloidosis.
The sequence of transthyretin is highly conserved in vertebrates. A number of uncharacterized proteins also belong to

this family:

- Escherichia coli hypothetical protein yedX.
- Bacillus subtilis hypothetical protein yunM.
- Caenorhabditis elegans hypothetical protein R09H10.3.
- Caenorhabditis elegans hypothetical protein ZK697.8.

Two regions were selected as signature patterns. The first located in the N-terminal extremity starts with a lysine known to be involved in binding T4. The second pattern is located in the C-terminal extremity.
Consensus pattern[KH]-[IV]-L-[DN]-x(3)-G-x-P-A-x(2)-[IV]-x-[IV] [The K binds thyroxine]
Consensus patternY-[TH]-[IV]-[AP]-x(2)-L-S-[PQ]-[FYW]-[GS]-[FY]-[QS]

[1] Schreiber G., Richardson S.J. Comp. Biochem. Physiol. 116B:137-160(1997).

791. Dihydropteroate synthase signatures
All organisms require reduced folate cofactors for the synthesis of a variety of metabolites. Most microorganisms must synthesize folate de novo because they lack the active transport system of higher vertebrate cells which allows these organisms to use dietary folates. Enzymes that are involved in the biosynthesis of folates are therefore the target of a variety of antimicrobial agents such as trimethoprim or sulfonamides.
Dihydropteroate synthase (EC 2.5.1.15) (DHPS) catalyzes the condensation of 6-hydroxymethyl-7,8-dihydropteridine pyrophosphate to para-aminobenzoic acid to form 7,8-dihydropteroate. This is the second step in the three steps pathway leading from 6-hydroxymethyl-7,8-dihydropterin to 7,8-dihydrofolate. DHPS is the target of sulfonamides which are substrates analog that compete with para-aminobenzoic acid.
Bacterial DHPS (gene sul or folP) [1] is a protein of about 275 to 315 amino acid residues which is either chromosomally encoded or found on various antibiotic resistance plasmids. In the lower eukaryote Pneumocystis carinii, DHPS is the C-terminal domain of a multifunctional folate synthesis enzyme (gene fas) [2].
Two signature patterns for DHPS were developed, the first signature is located in the N-terminal section of these enzymes, while the second signature is located in the central section.
Consensus pattern[LIVM]-x-[AG]-[LIVMF](2)-N-x-T-x-D-S-F-x-D-x-[SG]
Consensus pattern[GE]-[SA]-x-[LIVM](2)-D-[LIVM]-G-[GP]-x(2)-[STA]-x-P

[1] Slock J., Stahly D.P., Han C.-Y., Six E.W., Crawford I.P. J. Bacteriol. 172:7211-7226(1990).
[2] Volpes F., Dyer M., Scaife J.G., Darby G., Stammers D.K., Delves C.J. Gene 112:213-218(1992).

792. Phosphatidylinositol 3- and 4-kinases signatures
Phosphatidylinositol 3-kinase (PI3-kinase) (EC 2.7.1.137) [1] is an enzyme that phosphorylates phosphoinositides on the 3-hydroxyl group of the inositol ring. The exact function of the three products of PI3-kinase - PI-3-P, PI-3,4-P(2) and PI-3,4,5-P(3) - is not yet known, although it is proposed that they function as second messengers in cell signalling. Currently, three forms of PI3-kinase are known:

- The mammalian enzyme which is a heterodimer of a 110 Kd catalytic chain (p110) and an 85 Kd subunit (p85) which allows it to bind to activated tyrosine protein kinases. There are at least two different types of p100 subunits (alpha and beta).
- Yeast TOR1/DRR1 and TOR2/DRR2 [2], PI3-kinases required for cell cycle activation. Both are proteins of about 280 Kd.
- Yeast VPS34 [3], a PI3-kinase involved in vacuolar sorting and segregation. VPS34 is a protein of about 100 Kd.
- Arabidopsis thaliana and soybean VPS34 homologs.

Phosphatidylinositol 4-kinase (P14-kinase) (EC 2.7.1.67) [4] is an enzyme that acts on phosphatidylinositol (PI) in the first committed step in the production of the second messenger inositol-1,4,5,-trisphosphate. Currently the following forms of PI4-kinases are known:

- Human PI4-kinase alpha.
- Yeast PIK1, a nuclear protein of 120 Kd.
- Yeast STT4, a protein of 214 Kd.

The PI3- and PI4-kinases share a well conserved domain at their C-terminal section; this domain seems to be distantly related to the catalytic domain of protein kinases [2]. Two signature patterns were developed from the best conserved

parts of this domain.
Four additional proteins belong to this family:

- Mammalian FKBP-rapamycin associated protein (FRAP) [5], which acts as the target for the cell-cycle arrest and immunosuppressive effects of the FKBP12-rapamycin complex.
- Yeast protein ESR1 [6] which is required for cell growth, DNA repair and meiotic recombination.
- Yeast protein TEL1 which is involved in controlling telomere length.
- Yeast hypothetical protein YHR099w, a distantly related member of this family.
- Fission yeast hypothetical protein SpAC22E12.16C.

Consensus pattern[LIVMFAC]-K-x(1,3)-[DEA]-[DE]-[LIVMC]-R-Q-[DE]-x(4)-Q
Consensus pattern[GS]-x-[AV]-x(3)-[LIVM]-x(2)-[FYH]-[LIVM](2)-x-[LIVMF]-x-D-R-H-x(2)-N

[1] Hiles I.D., Otsu M., Volinia S., Fry M.J., Gout I., Dhand R., Panayotou G., Ruiz-Larrea F., Thompson A., Totty N.F., Hsuan J.J., Courtneidge S.A., Parker P.J., Waterfield M.D. Cell 70:419-429(1992).
[2] Kunz J., Henriquez R., Schneider U., Deuter-Reinhard M., Movva N., Hall M.N. Cell 73:585-596(1993).
[3] Schu P.V., Takegawa K., Fry M.J., Stack J.H., Waterfield M.D., Emr S.D. Science 260:88-91 (1993).
[4] Garcia-Bustos J.F., Marini F., Stevenson I., Frei C., Hall M.N. EMBO J. 13:2352-2361(1994).
[5] Brown E.J., Albers M.W., Shin T.B., Ichikawa K., Keith C.T., Lane W.S., Schreiber S.L. Nature 369:756-758(1994).
[6] Kato R., Ogawa H. Nucleic Acids Res. 22:3104-3112(1994).

793. FAD-dependent glycerol-3-phosphate dehydrogenase signatures
FAD-dependent glycerol-3-phosphate dehydrogenase (EC 1.1.99.5) (GPD) catalyzes the conversion of glycerol-3-phosphate into dihydroxyacetone phosphate. In bacteria [1] it is associated with the utilization of glycerol coupled to respiration. In Escherichia coli, two isozymes are known: one expressed under anaerobic conditions (gene glpA) and one in aerobic conditions (gene glpD). In eukaryotes, a mitochondrial form of GPD participates in the glycerol phosphate shuttle in conjunction with an NAD-dependent cytoplasmic GPD (EC 1.1.1.8) [2,3].
These enzymes are proteins of about 60 to 70 Kd which contain a probable FAD-binding domain in their N-terminal extremity. The mammalian enzyme differs from the bacterial or yeast proteins by having an EF-hand calcium-binding region (See <PDOC00018>) in its C-terminal extremity.
Two signature patterns were developed. One based on the first half of the FAD-binding domain and one which corresponds to a conserved region in the central part of these enzymes.
Consensus pattern[IV]-G-G-G-x(2)-G-[STACV]-G-x-A-x-D-x(3)-R-G
Consensus patternG-G-K-x(2)-[GSTE]-Y-R-x(2)-A

[1] Austin D., Larson T.J. J. Bacteriol. 173:101-107(1991).
[2] Roennow B., Kielland-Brandt M.C. Yeast 9:1121-1130(1993).
[3] Brown L.J., McDonald M.J., Lehn D.A., Moran S.M. J. Biol. Chem. 269:14363-14366(1994).

794. NOL1/NOP2/sun family signature
The following proteins seems to be evolutionary related:

- Mammalian proliferating-cell nucleolar antigen p120 (gene NOL1) which may play a role in the regulation of the cell cycle and the increased nucleolar activity that is associated with the cell proliferation.
- Yeast nucleolar protein NOP2 (or YNA1) which could be involved in nucleolar function during the onset of growth, and in the maintenance of nucleolar structure.
- Yeast hypothetical protein YBL024w.
- Bacterial protein sun (also known as fmu).
- Escherichia coli hypothetical protein yebU.
- Mycobacterium tuberculosis hypothetical protein MtCY21B4.24.
- Methanococcus jannaschii hypothetical protein MJ0026.

NOL1 is a protein of 855 residues, NOP2 consists of 618 residues, YBL024w of 684, sun is a protein of about 430 to 450 residues and MJ026 has 274 residues. They share a conserved central domain which contains some highly conserved regions. One of these regions was selected as a signature pattern.
Consensus pattern[FV]-D-[KRA]-[LIVMA]-L-x-D-[AV]-P-C-[ST]-[GA]
795. moaA / nifB / pqqE family signature
A number of proteins involved in the biosynthesis of metallo cofactors have been shown [1,2] to be evolutionary related.

These proteins are:

- Bacterial and archebacterial protein moaA, which is involved in the biosynthesis of the molybdenum cofactor (molybdopterin; MPT).
- Arabidopsis thaliana cnx2, a protein involved in molybdopterin biosynthesis and which is highlys similar to moaA.
- Bacillus subtilis narA, which seems to be the moaA ortholog in that bacteria.
- Bacterial protein nifB (or fixZ) which is involved in the biosynthesis of the nitrogenase iron-molybdenum cofactor.
- Bacterial protein pqqE which is involved in the biosynthesis of the cofactor pyrrolo-quinoline-quinone (PQQ).
- Pyrococcus furiosus cmo, a protein involved in the synthesis of a molybdopterin-based tungsten cofactor.
- Caenorhabditis elegans hypothetical protein F49E2.1.

All these proteins share, in their N-terminal region, a conserved domain that contains three cysteines. In moaA, these cysteines have been shown [1] to be important for the biological activity. They could be inolved in the binding of an iron-sulfur cluster.
Consensus pattern[LIV]-x(3)-C-[NP]-[LIVMF]-[QRS]-C-x-[FYM]-C [The three C's are putative Fe-S ligands

[1] Menendez C., Igloi G., Henninger H., Brandsch R. Arch. Microbiol. 164:142-151(1995).
[2] Hoff T., Schnorr K.M., Meyer C., Caboche M. J. Biol. Chem. 270:6100-6107(1995).

796. Forkhead-associated (FHA) domain profile
The forkhead-associated (FHA) domain [1,E1] is a putative nuclear signalling domain found in a variety of otherwise unrelated proteins. The FHA domain comprise approximately 55 to 75 amino acids and contains three highly conserved blocks separated by divergent spacer regions. Currently it has been found in the following proteins:

- Four transcription factors that also contain a forkhead (FH) domain: mouse myocyte nuclear factor 1 (MNF1), yeast transcription factor FHL1, which probably controls pre-mRNA processing, and yeast FKH1 and FKH2. In those protein the FHA domain is located N-terminal of the DNA-binding FH domain.
- Kinase-associated protein phosphatase (KAPP) from Arabidopsis thaliana, a protein which specifically interacts with the receptor-type Ser/Thr-kinase RLK5. In KAPP, the FHA domain maps to a region that interacts with the receptor-type protein kinase RLK5 only if the kinase is phosphorylated on serine residues [2].
- Two protein kinases from yeast that are involved in mediating the nuclear response to DNA damage: DUN1 and SPK1/SAD1 [3]. The latter is the only known protein containing two copies of the FHA domain.
- Protein kinase cds1 from fission yeast contains a FHA domain and might be the ortholog of SPK1.
- Protein kinase MEK1 from yeast, which is involved in meiotic recombination.
- Human nuclear antigen Ki67 which is expressed only in proliferating cells.
- Yeast hypothetical protein YHR115c, which contains a RING-finger C-terminal of the FHA domain.
- Yeast hypothetical proteins L8083.1 and 9346.10, which contain an extensive coiled-coil region C-terminal of the FHA domain.
- Caenorhabditis elegans hypothetical protein ZK632.2.
- Caenorhabditis elegans hypothetical protein C01G6.5.
- FraH from the prokaryote Anabaena, which contains a zinc-finger motif N-terminal of the FHA domain.
- An ORF from the bacterium Streptomyces, which is on the opposite strand of the protein kinase pks1, overlapping the ORF of the kinase.

[1] Hofmann K.O., Bucher P. Trends Biochem. Sci. 20:347-349(1995).
[2] Stone J.M., Collinge M.A., Smith R.D., Horn M.A., Walker J.C. Science 266:793-795(1994).
[3] Navas T.A., Zhou Z., Elledge S.J. Cell 80:29-39(1995).

797. Ald_Xan_dh_C
Aldehyde oxidase and xanthine dehydrogenase, C terminus

[1] Romao MJ, Archer M, Moura I, Moura JJ, LeGall J, Engh R, Schneider M, Hof P, Huber R; Medline: 96072968 "Crystal structure of the xanthine oxidase-related aldehyde oxido-reductase from D. gigas." Science 1995;270: 1170-1176.

Number of members: 54
798. Glyco_hydro_38
Glycosyl hydrolases family 38

Glycosyl hydrolases are key enzymes of carbohydrate metabolism.
Number of members: 20

[1] Henrissat B; Medline: 98313424; "Glycosidase families" Biochem Soc Trans 1998;26:153-156.

799. HECT
HECT-domain (ubiquitin-transferase).
The name HECT comes from Homologous to the E6-AP Carboxyl Terminus.
Number of members: 43

[1] Huibregtse JM, Scheffner M, Beaudenon S, Howley PM; Medline: 95223981; "A family of proteins structurally and functionally related to the E6-AP ubiquitin-protein ligase." Proc Natl Acad Sci U S A 1995;92:2563-2567.

800. HRDC
HRDC domain
The HRDC (Helicase and RNase D C-terminal) domain has a putative role in nucleic acid binding. Mutations in the HRDC domain cause human disease.
Number of members: 19

[1] Morozov V, Mushegian AR, Koonin EV, Bork P; Medline: 98060076; "A putative nucleic acid-binding domain in Bloom's and Werner's syndrome helicases" Trends Biochem Sci 1997;22:417-418.

801. Integrase
Integrase mediates integration of a DNA copy of the viral genome into the host chromosome. Integrase is composed of three domains. The amino-terminal domain is a zinc binding domain. The central domain is the catalytic domain [1]. The carboxyl terminal domain is a DNA binding domain [2].
Number of members: 581

[1] Dyda F, Hickman AB, Jenkins TM, Engelman A, Craigie R, Davies DR; Medline: 95099322. "Crystal structure of the catalytic domain of HIV-1 integrase: similarity to other polynucleotidyl transferases." Science 1994;266: 1981-1986.
[2] Lodi PJ, Ernst JA, Kuszewski J, Hickman AB, Engelman A, Craigie R, Clore GM, Gronenborn AM; Medline: 95359147; "Solution structure of the DNA binding domain of HIV-1 integrase." Biochemistry 1995;34:9826-9833

802. lig_chan
Ligand-gated ion channel
This family includes the four transmembrane regions of the ionotropic glutamate receptors and NMDA receptors.
Number of members: 128

[1] Tong G, Shepherd D, Jahr CE; Medline: 95184014; "Synaptic desensitization of NMDA receptors by calcineurin." Science 1995;267:1510-1512.

803. RhoGAP
RhoGAP domain
GTPase activator proteins towards Rho/Rac/Cdc42-like small GTPases.
Number of members: 97

[1] Musacchio A, Cantley LC, Harrison SC; Medline: 97121392; "Crystal structure of the breakpoint cluster region-homology domain from phosphoinositide 3-kinase p85 alpha subunit." Proc Natl Acad Sci U S A 1996;93: 14373-14378.
[2] Barrett T, Xiao B, Dodson EJ, Dodson G, Ludbrook SB, Nurmahomed K, Gamblin SJ, Musacchio A, Smerdon SJ, Eccleston JF; Medline: 97162209; "The structure of the GTPase-activating domain from p50rhoGAP." Nature 1997;385:458-461.
[3] Rittinger K, Walker PA, Eccleston JF, Nurmahomed K, Owen D, Laue E, Gamblin SJ, Smerdon SJ; Medline: 97404320; "Crystal structure of a small G protein in complex with the GTPase-activating protein rhoGAP." Nature 1997;388:693-697.
[4] Boguski MS, McCormick F; Medline: 94081948; "Proteins regulating Ras and its relatives." Nature 1993;366: 643-654.

804. vwd
von Willebrand factor type D domain

[1] Bork P; Medline: 93327926; "The modular architecture of a new family of growth regulators related to connective tissue growth factor." FEBS lett 1993;327:125-130.

Number of members: 92
805. zf-C4_Topoisom
Topoisomerase DNA binding C4 zinc finger

[1] Tse-Dinh YC, Beran-Steed RK; Medline: 89034032; "Escherichia coli DNA topoisomerase I is a zinc metalloprotein with three repetitive zinc-binding domains." J Biol Chem 1988;263:15857-15859.
[2] Ahumada A, Tse-Dinh YC; Medline: 99011409; "The Zn(II) binding motifs of E. coli DNA topoisomerase I is part of a high-affinity DNA binding domain." Biochem Biophys Res Commun 1998;251:509-514.

Number of members: 51
806. AIRC
AIR carboxylase
Members of this family catalyse the decarboxylation of 1-(5-phosphoribosyl)-5-amino-4-imidazole-carboxylate (AIR). This family catalyse the sixth step of de novo purine biosynthesis. Some members of this family contain two copies of this domain. Number of members: 35
807. Bromodomain signature and profile
PROSITE cross-reference(s): PS00633; BROMODOMAIN_1, PS50014; BROMODOMAIN_2
The bromodomain [1,2,3] is a conserved region of about 70 amino acids found in the following proteins:

- Higher eukaryotes transcription initiation factor TFIID 250 Kd subunit (TBP-associated factor p250) (gene CCG1). P250 associated with the TFIID TATA-box binding protein and seems essential for progression of the G1 phase of the cell cycle.
- Human RING3, a protein of unknown function encoded in the MHC class II locus.
- Mammalian CREB-binding protein (CBP), which mediates cAMP-gene regulation by binding specifically to phosphorylated CREB protein.
- Drosophila female sterile homeotic protein (gene fsh), required maternally for proper expression of other homeotic genes involved in pattern formation, such as Ubx.
- Drosophila brahma protein (gene brm), a protein required for the activation of multiple homeotic genes.
- Mammalian homologs of brahma. In human, three brahma-like proteins are known: SNF2a(hBRM), SNF2b, and BRG1.
- Human BS69, a protein that binds to adenovirus E1A and inhibits E1A transactivation
- Human peregrin (or Br140).
- Yeast BDF1 [3], a transcription factor involved in the expression of a broad class of genes including snRNAs.
- Yeast GCN5, a general transcriptional activator operating in concert with certain other DNA-binding transcriptional activators, such as GCN4, HAP2/3/4 or ADA2.
- Yeast NPS1/STH1, involved in G(2) phase control in mitosis.
- Yeast SNF2/SWI2, which is part of a complex with the SNF5, SNF6, SWI3 and ADR6/SWI1 proteins. This SWI-complex is involved in transcriptional activation.
- Yeast SPT7, a transcriptional activator of Ty elements and possibly other genes.
- Caenorhabditis elegans protein cbp-1.
- Yeast hypothetical protein YGR056w.
- Yeast hypothetical protein YKR008w.
- Yeast hypothetical protein L9638.1.

Some proteins contain a region which, while similar to some extent to a classical bromodomain, diverges from it by either lacking part of the domain or because of an insertion. These proteins are:

- Mammalian protein HRX (also known as All-1 or MLL), a protein involved in translocations leading to acute leukemias and which possibly acts as a transcriptional regulatory factor. HRX contains a region similar to the C- terminal half of the bromodomain.
- Caenorhabditis elegans hypothetical protein ZK783.4. The bromodomain of this protein has a 23 amino-acid insertion.
- Yeast protein YTA7. This protein contains a region with significant similarity to the C-terminal half of the bromodomain.

As it is a member of the AAA family (see <PDOC00572>) it is also in a functionally different context.

The above proteins generally contain a single bromodomain, but some of them contain two copies, this is the case of BDF1, CCG1, fsh, RING3, YKR008w and L9638.1.
The exact function of this domain is not yet known but it is thought to be involved in protein-protein interactions and it may be important for the assembly or activity of multicomponent complexes involved in transcriptional activation.
The consensus pattern that has been developed spans a major part of the bromodomain; a more sensitive detection is available through the use of a profile which spans the whole domain.
Consensus pattern[STANVF]-x(2)-F-x(4)-[DNS]-x(5,7)-[DENQTF]-Y-[HFY]-x(2)-[LIVMFY]-x(3)-[LIVM]-x(4)-[LIVM]-x(6,8)-Y-x(12,13)-[LIVM]-x(2)-N-[SACF]-x(2)-[FY] References

[1] Haynes S.R., Doolard C., Winston F., Beck S., Trowsdale J., Dawid I.B. Nucleic Acids Res. 20:2693-2603(1992).
[2] Tamkun J.W., Deuring R., Scott M.P., Kissinger M., Pattatucci A.M., Kaufman T.C., Kennison J.A. Cell 68:561-572 (1992).
[3] Tamkun J.W. Curr. Opin. Genet. Dev. 5:473-477(1995).

808. (CH) Actinin-type actin-binding domain signatures
PROSITE cross-reference(s): PS00019; ACTININ_1, PS00020; ACTININ_2
Alpha-actinin is a F-actin cross-linking protein which is thought to anchoractin to a variety of intracellular structures [1]. The actin-binding domain of alpha-actinin seems to reside in the first 250 residues of the protein. A similar actin-binding domain has been found in the N-terminal region of many different actin-binding proteins [2,3]:

- In the beta chain of spectrin (or fodrin).
- In dystrophin, the protein defective in Duchenne muscular dystrophy (DMD) and which may play a role in anchoring the cytoskeleton to the plasma membrane.
- In the slime mold gelation factor (or ABP-120).
- In actin-binding protein ABP-280 (or filamin), a protein that link actin filaments to membrane glycoproteins.
- In fimbrin (or plastin), an actin-bundling protein. Fimbrin differs from the above proteins in that it contains two tandem copies of the actin-binding domain and that these copies are located in the C-terminal part of the protein.

Two conserved regions were selected as signature patterns for this type of main. The first of this region is located at the beginning of the domain, hile the second one is located in the central section and has been shown to be essential for the binding of actin.
Consensus pattern[EQ]-x(2)-[ATV]-[FY]-x(2)-W-x-N
Consensus pattern [LIVM]-x-[SGN]-[LIVM]-[DAGHE]-[SAG]-x-[DNEAG]-[LIVM]-x-[DEAG]-x(4)-[LIVM]-x-[LM]-[SAG]-[LIVM]-[LIVMT]-W-x- [LIVM](2)

[1] Schleicher M., Andre E., Harmann A., Noegel A.A. Dev. Genet. 9:521-530(1988).
[2] Matsudaira P. Trends Biochem. Sci. 16:87-92(1991).
[3] Dubreuil R.R. BioEssays 13:219-226(1991).

809. (COX1) Heme-copper oxidase subunit I, copper B binding region signature PROSITE cross-reference(s): PS00077; COX1
Heme-copper respiratory oxidases [1] are oligomeric integral membrane protein complexes that catalyze the terminal step in the respiratory chain: they transfer electrons from cytochrome c or a quinol to oxygen. Some terminal oxidases generate a transmembrane proton gradient across the plasma membrane (prokaryotes) or the mitochondrial inner membrane (eukaryotes). The enzyme complex consists of 3-4 subunits (prokaryotes) up to 13 polypeptides (mammals) of which only the catalytic subunit (equivalent to mammalian subunit 1 (CO I)) is found in all heme-copper respiratory oxidases. The presence of a bimetallic center (formed by a high-spin heme and copper B) as well as a low-spin heme, both ligated to six conserved histidine residues near the outer side of four transmembrane spans within CO I is common to all family members [2-4].
In contrary to eukaryotes the respiratory chain of prokaryotes is branched to multiple terminal oxidases. The enzyme complexes vary in heme and copper composition, substrate type and substrate affinity. The different respiratory oxidases allow the cells to customize their respiratory systems according a variety of environmental growth conditions [1].
Recently also a component of an anaerobic respiratory chain has been found to contain the copper B binding signature of this family: nitric oxide reductase (NOR) exists in denitrifying species of Archae and Eubacteria.
Enzymes that belong to this family are:

- Mitochondrial-type cytochrome c oxidase (EC 1.9.3.1) which uses cytochrome c as electron donor. The electrons are transferred via copper A (Cu(A)) and heme a to the bimetallic center of CO I that is formed by a pentacoordinated heme a and copper B (Cu(B)). Subunit 1 contains 12 transmembrane regions. Cu(B) is said to be ligated to three of the conserved histidine residues within the transmembrane segments 6 and 7.
- Quinol oxidase from prokaryotes that transfers electrons from a quinol to the binuclear center of polypeptide I. This category of enzymes includes Escherichia coli cytochrome O terminal oxidase complex which is a component of the aerobic respiratory chain that predominates when cells are grown at high aeration.
- FixN, the catalytic subunit of a cytochrome c oxidase expressed in nitrogen-fixing bacteroids living in root nodules. The high affinity for oxygen allows oxidative phosphorylation under low oxygen concentrations. A similar enzyme has been found in other purple bacteria.
- Nitric oxide reductase (EC 1.7.99.7) from Pseudomonas stutzeri. NOR reduces nitrate to dinitrogen. It is a heterodimer of norC and the catalytic subunit norB. The latter contains the 6 invariant histidine residues and 12 transmembrane segments [5].

As a signature pattern the copper-binding region was used.
Consensus pattern[YWG]-[LIVFYWTA](2)-[VGS]-H-[LNP]-x-V-x(44,47)-H-H [The three H's are copper B ligands]
Notecytochrome bd complexes do not belong to this family.

[1] Garcia-Horsman J.A., Barquera B., Rumbley J., Ma J., Gennis R.B. J. Bacteriol. 176:5587-5600(1994).
[2] Castresana J., Luebben M., Saraste M., Higgins D.G. EMBO J. 13:2516-2525(1994).
[3] Capaldi R.A., Malatesta F., Darley-Usmar V.M. Biochim. Biophys. Acta 726:135-148(1983).
[4] Holm L., Saraste M., Wikstrom M. EMBO J. 6:2819-2823(1987).
[5] Saraste M., Castresana J. FEBS Lett. 341:1-4(1994).

810. (dehydrog_molyb) Eukaryotic molybdopterin oxidoreductases signature PROSITE cross-reference(s): PS00559; MOLYBDOPTERIN_EUK
A number of different eukaryotic oxidoreductases that require and bind a molybdopterin cofactor have been shown [1] to share a few regions of sequence similarity. These enzymes are:

- Xanthine dehydrogenase (EC 1.1.1.204), which catalyzes the oxidation of xanthine to uric acid with the concomitant reduction of NAD. Structurally, this enzyme of about 1300 amino acids consists of at least three distinct domains: an N-terminal 2Fe-2S ferredoxin-like iron-sulfur binding domain (see <PDOC00175>), a central FAD/NAD-binding domain and a C-terminal Mo-pterin domain.
- Aldehyde oxidase (EC 1.2.3.1), which catalyzes the oxidation aldehydes into acids. Aldehyde oxidase is highly similar to xanthine dehydrogenase in its sequence and domain structure.
- Nitrate reductase (EC 1.6.6.1), which catalyzes the reduction of nitrate to nitrite. Structurally, this enzyme of about 900 amino acids consists of an N-terminal Mo-pterin domain, a central cytochrome b5-type heme-binding domain (see <PDOC00170>) and a C-terminal FAD/NAD-binding cytochrome reductase domain.
- Sulfite oxidase (EC 1.8.3.1), which catalyzes the oxidation of sulfite to sulfate. Structurally, this enzyme of about 460 amino acids consists of an N-terminal cytochrome b5-binding domain followed by a Mo-pterin domain.

There are a few conserved regions in the sequence of the molybdopterin-binding domain of these enzymes. The pattern uses to detect these proteins is based on one of them. It contains a cysteine residue which could be involved in binding the molybdopterin cofactor.
Consensus pattern[GA]-x(3)-[KRNQHT]-x(11,14)-[LIVMFYWS]-x(8)-[LIVMF]-x-C-x(2)-[DEN]-R-x(2)-[DE]

[1] Wootton J.C., Nicolson R.E., Cock J.M., Walters D.E., Burke J.F., Doyle W.A., Bray R.C. Biochim. Biophys. Acta 1057:157-185(1991).

811. (DNA_ligase) ATP-dependent DNA ligase signatures
PROSITE cross-reference(s): PS00697; DNA_LIGASE_A1, PS00333; DNA_LIGASE_A2
DNA ligase (polydeoxyribonucleotide synthase) is the enzyme that joins two DNA fragments by catalyzing the formation of an internucleotide ester bond between phosphate and deoxyribose. It is active during DNA replication, DNA repair and DNA recombination. There are two forms of DNA ligase: one requires ATP (EC 6.5.1.1), the other NAD (EC 6.5.1.2). Eukaryotic, archaebacterial, virus and phage DNA ligases are ATP-dependent. During the first step of the joining reaction, the ligase interacts with ATP to form a covalent enzyme-adenylate intermediate. A conserved lysine residue is the site of adenylation [1,2].
Apart from the active site region, the only conserved region common to all ATP-dependent DNA ligases is found [3] in

the C-terminal section and contains a conserved glutamate as well as four positions with conserved basic residues. Signature patterns were developed for both conserved regions.
Consensus pattern[EDQH]-x-K-x-[DN]-G-x-R-[GACIVM] [K is the active site residue]
Consensus patternE-G-[LIVMA]-[LIVM](2)-[KR]-x(5,8)-[YW]-[QNEK]-x(2,6)-[KRH]-x(3,5)-K-[LIVMFY]-K
Sequences known to belong to this class detected by the patternALL, except for archebacterial DNA ligases.

[1] Tomkinson A.E., Totty N.F., Ginsburg M., Lindahl T. Proc. Natl. Acad. Sci. U.S.A. 88:400-404(1991).
[2] Lindahl T., Barnes D.E. Annu. Rev. Biochem. 61:251-281(1992).
[3] Kletzin A. Nucleic Acids Res. 20:5389-5396(1992).

812. (FAD_Gly3P_dh) FAD-dependent glycerol-3-phosphate dehydrogenase signatures PROSITE cross-reference(s): PS00977; FAD_G3PDH_1, PS00978; FAD_G3PDH_2
FAD-dependent glycerol-3-phosphate dehydrogenase (EC 1.1.99.5) (GPD) catalyzes the conversion of glycerol-3-phosphate into dihydroxyacetone phosphate. In bacteria [1] it is associated with the utilization of glycerol coupled to respiration. In Escherichia coli, two isozymes are known: one expressed under anaerobic conditions (gene glpA) and one in aerobic conditions (gene glpD). In eukaryotes, a mitochondrial form of GPD participates in the glycerol phosphate shuttle in conjunction with an NAD-dependent cytoplasmic GPD (EC 1.1.1.8) [2, 3].
These enzymes are proteins of about 60 to 70 Kd which contain a probable FAD-binding domain in their N-terminal extremity. The mammalian enzyme differs from the bacterial or yeast proteins by having an EF-hand calcium-binding region (See <PDOC00018>) in its C-terminal extremity.
Two signature patterns were developed. One based on the first half of the FAD-binding domain and one which corresponds to a conserved region in the central part of these enzymes.
Consensus pattern[IV]-G-G-G-x(2)-G-[STACV]-G-x-A-x-D-x(3)-R-G
Consensus patternG-G-K-x(2)-[GSTE]-Y-R-x(2)-A

[1] Austin D., Larson T.J. J. Bacteriol. 173:101-107(1991).
[2] Roennow B., Kielland-Brandt M.C. Yeast 9:1121-1130(1993).
[3] Brown L.J., McDonald M.J., Lehn D.A., Moran S.M. J. Biol. Chem. 269:14363-14366(1994).

813. (Fapy_DNA_glyco) Formamidopyrimidine-DNA glycosylase signature
PROSITE cross-reference(s): PS01242; FPG
Formamidopyrimidine-DNA glycosylase (EC 3.2.2.23) [1] (Fapy-DNA glycosylase) (gene fpg) is a bacterial enzyme involved in DNA repair and which excise oxidized purine bases to release 2,6-diamino-4-hydroxy-5N-methylformamidopyrimidine (Fapy) and 7,8-dihydro-8-oxoguanine (8-OxoG) residues. In addition to its glycosylase activity, FPG can also nick DNA at apurinic/apyrimidinic sites (AP sites). FPG is a monomeric protein of about 32 Kd which binds and require zinc for its activity.
The binding site for zinc seems to be located in the C-terminal part of the enzyme where fours conserved and essential [2] cysteines are located. A signature pattern was developed based on this region.
Consensus patternC-x(2,4)-C-x-[GTAQ]-x-[IV]-x(7)-R-[GSTAN]-[STA]-x-[FYI]-C- x(2)-C-Q
[The four C's are putative zinc ligands]

[1] Duwat P., de Oliveira R., Ehrlich S.D., Boiteux S. Microbiology 141:411-417(1995).
[2] O'Connor T.E., Graves R.J., Demurcia G., Castaing B., Laval J. J. Biol. Chem. 268:9063-9070(1993).

814. (G_glu_transpept) Gamma-glutamyltranspeptidase signature
PROSITE cross-reference(s): PS00462; G_GLU_TRANSPEPTIDASE
Gamma-glutamyltranspeptidase (EC 2.3.2.2) (GGT) [1] catalyzes the transfer of the gamma-glutamyl moiety of glutathione to an acceptor that may be an amino acid, a peptide or water (forming glutamate). GGT plays a key role in the gamma-glutamyl cycle, a pathway for the synthesis and degradation of glutathione. In prokaryotes and eukaryotes, it is an enzyme that consists of two polypeptide chains, a heavy and a light subunit, processed from a single chain precursor. The active site of GGT is known to be located in the light subunit.
The sequences of mammalian and bacterial GGT show a number of regions of high similarity [2]. Pseudomonas cephalosporin acylases (EC 3.5.1.-) that convert 7-beta-(4-carboxybutanamido)-cephalosporanic acid (GL-7ACA) into 7-aminocephalosporanic acid (7ACA) and glutaric acid are evolutionary related to GGT and also show some GGT activity [3]. Like GGT, these GL-7ACA acylases, are also composed of two subunits.
One of the conserved regions correspond to the N-terminal extremity of the mature light chains of these enzymes. This region was used as a signature pattern.
Consensus patternT-[STA]-H-x-[ST]-[LIVMA]-x(4)-G-[SN]-x-V-[STA]-x-T-x-T-[LIVM]-[NE]-x(1,2)-[FY]-G

[1] Tate S.S., Meister A. Meth. Enzymol. 113:400-419(1985).
[2] Suzuki H., Kumagai H., Echigo T., Tochikura T. J. Bacteriol. 171:5169-5172(1989).
[3] Ishiye M., Niwa M. Biochim. Biophys. Acta 1132:233-239(1992).

815. G-protein gamma subunit profile
PROSITE cross-reference(s): PS50058; G_PROTEIN_GAMMA
Guanine nucleotide-binding proteins (G proteins) [1] act as intermediaries in the transduction of signals generated by transmembrane receptors. G proteins consist of three subunits (alpha, beta, and gamma). The alpha subunit binds to and hydrolyzes GTP; the functions of the beta and gamma subunits are less clear but they seem to be required for the replacement of GDP by GTP as well as for membrane anchoring and receptor recognition.
The gamma subunits are small proteins (from 70 to 110 residues) that are bound to the membrane via a isoprenyl group (either a farnesyl or a geranylgeranyl) covalently linked to their C-terminus. In mammals there are at least 12 different isoforms of gamma subunits.
The Caenorhabditis elegans protein egl-10, which is a regulator of G-protein signalling, contains a G-protein gamma-like domain.
A profile was developed that spans the complete length of the gamma subunit.

[1] Pennington S.R. Protein Prof. 2:16-315(1995).

816. GNS1/SUR4 family signature
PROSITE cross-reference(s): PS01188; GNS1_SUR4
The following group of eukaryotic integral membrane proteins, whose exact function has not yet clearly been established, are evolutionary related [1]:

- Yeast GNS1 [2], a protein involved in synthesis of 1,3-beta-glucan.
- Yeast SUR4 (or APA1, SRE1) [3], a protein that could act in a glucose-signaling pathway that controls the expression of several genes that are transcriptionally regulated by glucose.
- Yeast hypothetical protein YJL196c.
- Caenorhabditis elegans hypothetical protein C40H1.4.
- Caenorhabditis elegans hypothetical protein D2024.3.

The proteins have from 290 to 435 amino acid residues. Structurally, they seem to be formed of three sections: a N-terminal region with two transmembrane domains, a central hydrophilic loop and a C-terminal region that contains from one to three transmembrane domains. A conserved region that contains three histidines was selected as a signature pattern. This region is located in the hydrophilic loop.
Consensus patternL-x-F-L-H-x-Y-H-H

[1] Bairoch A. Unpublished observations (1996).
[2] El-Sherbeini M., Clemas J.A. J. Bacteriol. 177:3227-3234(1995).
[3] Garcia-Arranz M., Maldonado A.M., Mazon M.J., Portillo F. J. Biol. Chem. 269:18076-18082(1994).

817. Immunoglobulins and major histocompatibility complex proteins signature
PROSITE cross-reference(s): PS00290; IG_MHC
The basic structure of immunoglobulin (Ig) [1] molecules is a tetramer of two light chains and two heavy chains linked by disulfide bonds. There are two types of light chains: kappa and lambda, each composed of a constant domain (CL) and a variable domain (VL). There are five types of heavy chains: alpha, delta, epsilon, gamma and mu, all consisting of a variable domain (VH) and three (in alpha, delta and gamma) or four (in epsilon and mu) constant domains (CH1 to CH4).
The major histocompatibility complex (MHC) molecules are made of two chains. In class I [2] the alpha chain is composed of three extracellular domains, a transmembrane region and a cytoplasmic tail. The beta chain (beta-2-microglobulin) is composed of a single extracellular domain. In class II [3], both the alpha and the beta chains are composed of two extracellular domains, a transmembrane region and a cytoplasmic tail.
It is known [4,5] that the Ig constant chain domains and a single extracellular domain in each type of MHC chains are related. These homologous domains are approximately one hundred amino acids long and include a conserved intradomain disulfide bond. A small pattern around the C-terminal cysteine is involved in this disulfide bond which can be used to detect these category of Ig related proteins.
Consensus pattern[FY]-x-C-x-[VA]-x-H-Sequences known to belong to this class detected by the pattern: Ig heavy chains type Alpha C region : All, in CH2 and CH3. Ig heavy chains type Delta C region : All, in CH3. Ig heavy chains type Epsilon

C region: All, in CH1, CH3 and CH4. Ig heavy chains type Gamma C region : All, in CH3 and also CH1 in some cases Ig heavy chains type Mu C region : All, in CH2, CH3 and CH4. Ig light chains type Kappa C region : In all CL except rabbit and Xenopus. Ig light chains type Lambda C region : In all CL except rabbit. MHC class I alpha chains : All, in alpha-3 domains, including in the cytomegalovirus MHC-1 homologous protein [6]. Beta-2-microglobulin : All. MHC class II alpha chains: All, in alpha-2 domains. MHC class II beta chains: All, in beta-2 domains.

[1] Gough N. Trends Biochem. Sci. 6:203-205(1981).
[2] Klein J., Figueroa F. Immunol. Today 7:41-44(1986).
[3] Figueroa F., Klein J.Immunol. Today 7:78-81(1986).
[4] Orr H.T., Lancet D., Robb R.J., Lopez de Castro J.A., Strominger J.L. Nature 282:266-270(1979).
[5] Cushley W., Owen M.J. Immunol. Today 4:88-92(1983).
[6] Beck S., Barrel B.G. Nature 331:269-272(1988).

818. (IGFBP) Insulin-like growth factor binding proteins signature PROSITE cross-reference(s): PS00222; IGF_BINDING The insulin-like growth factors (IGF-I and IGF-II) bind to specific binding proteins in extracellular fluids with high affinity [1,2,3]. These IGF-binding proteins (IGFBP) prolong the half-life of the IGFs and have been shown to either inhibit or stimulate the growth promoting effects of the IGFs on cells culture. They seem to alter the interaction of IGFs with their cell surface receptors. There are at least six different IGFBPs and they are structurally related.
The following growth-factor inducible proteins are structurally related to IGFBPs and could function as growth-factor binding proteins [4,5]:

- Mouse protein cyr61 and its probable chicken homolog, protein CEF-10.
- Human connective tissue growth factor (CTGF) and its mouse homolog, protein FISP-12.
- Vertebrate protein NOV.

As a signature pattern a conserved cysteine-rich region locatedin the N-terminal section of these proteins is used. Consensus patternG-C-[GS]-C-C-x(2)-C-A-x(6)-C Sequences known to belong to this class detected by the patternALL, except for IGFBP-6's.

[1] Rechler M.M. Vitam. Horm. 47:1-114(1993).
[2] Shimasaki S., Ling N. Prog. Growth Factor Res. 3:243-266(1991).
[3] Clemmons D.R. Trends Endocrinol. Metab. 1:412-417(1990).
[4] Bradham D.M., Igarashi A., Potter R.L., Grotendorst G.R. J. Cell Biol. 114:1285-1294(1991).
[5] Maloisel V., Martinerie C., Dambrine G., Plassiart G., Brisac M., Crochet J., Perbal B. Mol. Cell. Biol. 12:10-21 (1992).

819. LMWPc : Low molecular weight phosphotyrosine protein phosphatase
Number of members: 34

[1]Medline: 94329182, The crystal structure of a low-molecular-weight phosphotyrosine protein phosphatase. Su XD, Taddei N, Stefani M, Ramponi G, Nordlund P; Nature 1994;370:575-578.

820. (myosin_head) ATP/GTP-binding site motif A (P-loop)
PROSITE cross-reference(s): PS00017; ATP_GTP_A
From sequence comparisons and crystallographic data analysis it has been shown [1,2,3,4,5,6] that an appreciable proportion of proteins that bind ATP or GTP share a number of more or less conserved sequence motifs. The best conserved of these motifs is a glycine-rich region, which typically forms a flexible loop between a beta-strand and an alpha-helix. This loop interacts with one of the phosphate groups of the nucleotide. This sequence motif is generally referred to as the 'A' consensus sequence [1] or the 'P-loop' [5].
There are numerous ATP- or GTP-binding proteins in which the P-loop is found. A number of protein families for which the relevance of the presence of such motif has been noted is listed below:

- ATP synthase alpha and beta subunits (see <PDOC00137>).
- Myosin heavy chains.
- Kinesin heavy chains and kinesin-like proteins (see <PDOC00343>).
- Dynamins and dynamin-like proteins (see <PDOC00362>).
- Guanylate kinase (see <PDOC00670>).
- Thymidine kinase (see <PDOC00524>).

- Thymidylate kinase (see <PDOC01034>).
- Shikimate kinase (see <PDOC00868>).
- Nitrogenase iron protein family (nifH/frxC) (see <PDOC00580>).
- ATP-binding proteins involved in 'active transport' (ABC transporters) [7] (see <PDOC00185>).
- DNA and RNA helicases [8,9,10].
- GTP-binding elongation factors (EF-Tu, EF-1alpha, EF-G, EF-2, etc.).
- Ras family of GTP-binding proteins (Ras, Rho, Rab, Ral, Ypt1, SEC4, etc.).
- Nuclear protein ran (see <PDOC00859>).
- ADP-ribosylation factors family (see <PDOC00781>).
- Bacterial dnaA protein (see <PDOC00771>).
- Bacterial recA protein (see <PDOC00131 >).
- Bacterial recF protein (see <PDOC00539>).
- Guanine nucleotide-binding proteins alpha subunits (Gi, Gs, Gt, G0, etc.).
- DNA mismatch repair proteins mutS family (See <PDOC00388>).
- Bacterial type II secretion system protein E (see <PDOC00567>).

Not all ATP- or GTP-binding proteins are picked-up by this motif. A number of proteins escape detection because the structure of their ATP-binding site is completely different from that of the P-loop. Examples of such proteins are the E1-E2 ATPases or the glycolytic kinases. In other ATP- or GTP-binding proteins the flexible loop exists in a slightly different form; this is the case for tubulins or protein kinases. A special mention must be reserved for adenylate kinase, in which there is a single deviation from the P-loop pattern: in the last position Gly is found instead of Ser or Thr.
Consensus pattern[AG]-x(4)-G-K-[ST]

[1] Walker J.E., Saraste M., Runswick M.J., Gay N.J EMBO J. 1:945-951(1982).
[2] Moller W., Amons R. FEBS Lett. 186:1-7(1985).
[3] Fry D.C., Kuby S.A., Mildvan A.S. Proc. Natl. Acad. Sci. U.S.A. 83:907-911(1986).
[4] Dever T.E., Glynias M.J., Merrick W.C. Proc. Natl. Acad. Sci. U.S.A. 84:1814-1818(1987).
[5] Saraste M., Sibbald P.R., Wittinghofer A. Trends Biochem. Sci. 15:430-434(1990).
[6] Koonin E.V. J. Mol. Biol. 229:1165-1174(1993).
[7] Higgins C.F., Hyde S.C., Mimmack M.M., Gileadi U., Gill D.R., Gallagher M.P. J. Bioenerg. Biomembr. 22:571-592 (1990).
[8] Hodgman T.C. Nature 333:22-23(1988) and Nature 333:578-578(1988) (Errata).
[9] Linder P., Lasko P., Ashburner M., Leroy P., Nielsen P.J., Nishi K., Schnier J., Slonimski P.P. Nature 337:121-122 (1989).
[10] Gorbalenya A.E., Koonin E.V., Donchenko A.P., Blinov V.M. Nucleic Acids Res. 17:4713-4730(1989).

821. PE: PE family
This family named after a PE motif near to the amino terminus of the domain. The PE family of proteins all contain an amino-terminal region of about 110 amino acids. The carboxyl terminus of this family are variable and fall into several classes. The largest class of PE proteins is the highly repetitive PGRS class which have a high glycine content. The function of these proteins is uncertain but it has been suggested that they may be related to antigenic variation of Mycobacterium tuberculosis [1]. Number of members: 88

[1] Medline: 98295987. Deciphering the biology of Mycobacterium tuberculosis from the complete genome sequence. Cole ST, Brosch R, Parkhill J, Garnier T, Churcher C, Harris D, Gordon SV, Eiglmeier K, Gas S, Barry CE 3rd, Tekaia F, Badcock K, Basham D, Brown D, Chillingworth T, Connor R, Davies R, Devlin K, Feltwell T, Gentles S, Hamlin N, Holroyd S, Hornsby T, Jagels K, Barrell BG, et al; Nature 1998;393:537-544.

822. (RNB) Ribonuclease II family signature
PROSITE cross-reference(s): PS01175; RIBONUCLEASE_II
On the basis of sequence similarities, the following bacterial and eukaryotic proteins seem to form a family:

- Escherichia coli and related bacteria ribonuclease II (EC 3.1.13.1) (RNase II) (gene rnb) [1]. RNase II is an exonuclease involved in mRNA decay. It degrades mRNA by hydrolyzing single-stranded polyribonucleotides processively in the 3'to 5' direction.
- Bacterial protein vacB. In Shigella flexneri, vacB has been shown to be required for the expression of virulence genes at the posttranscriptional level.
- Yeast protein SSD1 (or SRK1) which is implicated in the control of the cell cycle G1 phase.

- Yeast protein DIS3 [2], which binds to ran (GSP1) and chances the the nucleotide-releasing activity of RCC1 on ran.
- Fission yeast protein dis3, which is implicated in mitotic control.
- Neurospora crassa cyt-4, a mitochondrial protein required for RNA 5' and 3' end processing and splicing.
- Yeast protein MSU1, which is involved in mitochondrial biogenesis.
- Synechocystis strain PCC 6803 protein zam [3], which control resistance to the carbonic anhydrase inhibitor acetazolamide.
- Caenorhabditis elegans hypothetical protein F48E8.6.

The size of these proteins range from 644 residues (rnb) to 1250 (SSD1). While their sequence is highly divergent they share a conserved domain in their C-terminal section [4]. It is possible that this domain plays a role in a putative exonuclease function that would be common to all these proteins. A signature pattern was developed based on the core of this conserved domain.
Consensus pattern[HI]-[FYE]-[GSTAM]-[LIVM]-x(4,5)-Y-[STAL]-x-[FWVAC]-[TV]-[SA]-P-[LIVMA]-[RQ]-[KR]-[FY]-x-D-x(3)-[HQ]

[1] Zilhao R., Camelo L., Arraiano C.M. Mol. Microbiol. 8:43-51 (1993).
[2] Noguchi E., Hayashi N., Azuma Y., Seki T., Nakamura M., Nakashima N., Yanagida M., He X., Mueller U., Sazer S., Nishimoto T. EMBO J. 15:5595-5605(1996).
[3] Beuf L., Bedu S., Cami B., Joset F. Plant Mol. Biol. 27:779-788(1995).
[4] Mian I.S. Nucleic Acids Res. 25:3187-3195(1997).

823. Src homology 2 (SH2) domain profile
PROSITE cross-reference(s): PS50001; SH2
The Src homology 2 (SH2) domain is a protein domain of about 100 amino-acid residues first identified as a conserved sequence region between the oncoproteins Src and Fps [1]. Similar sequences were later found in many other intracellular signal-transducing proteins [2]. SH2 domains function as regulatory modules of intracellular signalling cascades by interacting with high affinity to phosphotyrosine-containing target peptides in a sequence-specific and strictly phosphorylation-dependent manner [3,4,5,6].
The SH2 domain has a conserved 3D structure consisting of two alpha helices and six to seven beta-strands. The core of the domain is formed by a continuous beta-meander composed of two connected beta-sheets [7].
So far, SH2 domains have been identified in the following proteins:

- Many vertebrate, invertebrate and retroviral cytoplasmic (non-receptor) protein tyrosine kinases. In particular in the Src, Abl, Bkt, Csk and ZAP70 families of kinases.
- Mammalian phosphatidylinositol-specific phospholipase C gamma-1 and -2. Two copies of the SH2 domain are found in those proteins in between the catalytic X-'and 'Y-boxes' (see <PDOC50007>).
- Mammalian phosphatidyl inositol 3-kinase regulatory p85 subunit.
- Some vertebrate and invertebrate protein-tyrosine phosphatases.
- Mammalian Ras GTPase-activating protein (GAP).
- Adaptor proteins mediating binding of guanine nucleotide exchange factors to growth factor receptors: vertebrate GRB2, Caenorhabditis elegans sem-5 and Drosophila DRK.
- Mammalian Vav oncoprotein, a guanine-nucleotide exchange factor of the CDC24 family.
- Miscellanous proteins interacting with vertebrate receptor protein tyrosine kinases: oncoprotein Crk, mammalian cytoplasmic proteins Nck, She.
- STAT proteins (signal transducers and activators of transcription).
- Chicken tensin.
- Yeast transcriptional control protein SPT6.

The profile developed to detect SH2 domains is based on a structural alignment consisting of 8 gap-free blocks and 7 linker regions totaling 92 match positions.

[1] Sadowski I., Stone J.C., Pawson T. Mol. Cell. Biol. 6:4396-4408(1986).
[2] Russel R.B., Breed J., Barton G.J. FEBS Lett. 304:15-20(1992).
[3] Marangere L.E.M., Pawson T. J. Cell Sci. Suppl. 18:97-104(1994).
[4] Pawson T., Schlessinger J. Curr. Biol. 3:434-442(1993).
[5] Mayer B.J., Baltimore D. Trends Cell. Biol. 3:8-13(1993).
[6] Pawson T. Nature 373:573-580(1995).
[7] Kuriyan J., Cowburn D. Curr. Opin. Struct. Biol. 3:828-837(1993).

824. Sulfate transporters signature
PROSITE cross-reference(s): PS01130; SULFATE_TRANSP
A number of proteins involved in the transport of sulfate across a membrane as well as some yet uncharacterized proteins have been shown [1,2] to be evolutionary related. These proteins are:

- Neurospora crassa sulfate permease II (gene cys-14).
- Yeast sulfate permeases (genes SUL1 and SUL2).
- Rat sulfate anion transporter 1 (SAT-1).
- Mammalian DTDST, a probable sulfate transporter which, in Human, is involved in the genetic disease, diastrophic dysplasia (DTD).
- Sulfate transporters 1, 2 and 3 from the legume Stylosanthes hamata.
- Human pendrin (gene PDS), which is involved in a number of hearing loss genetic diseases.
- Human protein DRA (Down-Regulated in Adenoma).
- Soybean early nodulin 70.
- Escherichia coli hypothetical protein ychM.
- Caenorhabditis elegans hypothetical protein F41D9.5.

As expected by their transport function, these proteins are highly hydrophobic and seem to contain about 12 transmembrane domains. The best conserved region seems to be located in the second transmembrane region and is used as a signature pattern.
Consensus pattern[PAV]-x-Y-[GS]-L-Y-[STAG](2)-x(4)-[LIVFYA]-[LIVST]-[YI]-x(3)-[GA]-[GST]-S-[KR]

[1] Sandal N.N., Marcker K.A. Trends Biochem. Sci. 19:19-19(1994).
[2] Smith F.W., Hawkesford M.J., Prosser I.M., Clarkson D.T. Mol. Gen. Genet. 247:709-715(1995).

825. TYA: TYA transposon protein
Ty are yeast transposons. A 5.7kb transcript codes for p3 a fusion protein of TYA and TYB. The TYA protein is analogous to the gag protein of retroviruses. TYA a is cleaved to form 46kd protein which can form mature virion like particles [1].
Number of members: 59

[1] Medline: 97404699. Cryo-electron microscopy structure of yeast Ty retrotransposon virus-like particles. Palmer KJ, Tichelaar W, Myers N, Burns NR, Butcher SJ, Kingsman AJ, Fuller SD, Saibil HR; J Virol 1997;71:6863-6868.

826. Aldolase_II
Class II Aldolase and Adducin N-terminal domain.
-!- This family includes class II aldolases and adducins which have not been ascribed any enzymatic function. Number of members: 37
References:

[1] Medline: 93294819. The spatial structure of the class II L-fuculose-1-phosphate aldolase from Escherichia coli. Dreyer MK, Schulz GE; J Mol Biol 1993;231:549-553.
[2] Medline: 96256522. Catalytic mechanism of the metal-dependent fuculose aldolase from Escherichia coli as derived from the structure. Dreyer MK, Schulz GE; J Mol Biol 1996;259:458-466.

827. CBD_2
-!- Two tryptophan residues are involved in cellulose binding.
-!- Cellulose binding domain found in bacteria. Number of members: 51
References:

[1] Medline: 95284032. Solution structure of a cellulose-binding domain from Cellulomonas fimi by nuclear magnetic resonance spectroscopy. Xu GY, Ong E, Gilkes NR, Kilburn DG, Muhandiram DR, Harris-Brandts M, Carver JP, Kay LE, Harvey TS; Biochemistry 1995;34:6993-7009.

828. P
A unique feature of the eukaryotic subtilisin-like proprotein convertases is the presence of an additional highly conserved sequence of approximately 150 residues (P domain) located immediately downstream of the catalytic domain.
Number of members: 91
References:

[1] Medline: 94252314. A C-terminal domain conserved in precursor processing proteases is required for intramolecular N-terminal maturation of pro-Kex2 protease. Gluschankof P, Fuller RS; EMBO J 1994;13:2280-2288.
[2] Medline: 98225190. Regulatory roles of the P domain of the subtilisin-like prohormone convertases. Zhou A, Martin S, Lipkind G, LaMendola J, Steiner DF; J Biol Chem 1998;273:11107-11114.

829. Uncharacterized protein family UPF0020 signature
PROSITE cross-reference(s): PS01261; UPF0020
The following uncharacterized proteins have been shown [1] to share regions of similarities:

- Escherichia coli hypothetical protein ycbY and HI0116/15, the corresponding Haemophilus influenzae protein.
- Bacillus subtilis hypothetical protein ypsC.
- Synechocystis strain PCC 6803 hypothetical protein slr0064.
- Methanococcus jannaschii hypothetical proteins MJ0438 and MJ0710.

These are hydrophilic proteins of from 40 Kd to about 80 Kd. They can be picked up in the database by the following pattern.
Consensus patternD-P-[LIVMF]-C-G-[ST]-G-x(3)-[LI]-E
References:

[1] Bairoch A. Unpublished observations (1997).

830. Uncharacterized protein family UPF0031 signatures
PROSITE cross-reference(s): PS01049; UPF0031_1; PSO1050; UPF0031_2
The following uncharacterized proteins have been shown [1] to share regions of similarities:

- Yeast chromosome XI hypothetical protein YKL151 c.
- Caenorhabditis elegans hypothetical protein R107.2.
- Escherichia coli hypothetical protein yjeF.
- Bacillus subtilis hypothetical protein yxkO.
- Helicobacter pylori hypothetical protein HP1363.
- Mycobacterium tuberculosis hypothetical protein MtCY77.05c.
- Mycobacterium leprae hypothetical protein B229_C2_201.
- Synechocystis strain PCC 6803 hypothetical protein s111433.
- Methanococcus jannaschii hypothetical protein MJ1586.

These are proteins of about 30 to 40 Kd whose central region is well conserved. They can be picked up in the database by the following patterns.
Consensus pattern[SAV]-[IVW]-[LVA]-[LIV]-G-[PNS]-G-L-[GP]-x-[DENQT]
Consensus pattern[GA]-G-x-G-D-[TV]-[LT]-[STA]-G-x-[LIVM]
831. (ACOX)
Acyl-CoA oxidase
This is a family of Acyl-CoA oxidases EC:1.3.3.6. Acyl-coA oxidase converts acyl-CoA into trans-2-enoyl-CoA [1].
Number of members: 39

[1] Hayashi H, De Bellis L, Yamaguchi K, Kato A, Hayashi M, Nishimura M; Medline: 98192624. "Molecular characterization of a glyoxysomal long chain acyl-CoA oxidase that is synthesized as a precursor of higher molecular mass in pumpkin." J Biol Chem 1998;273:8301-8307.

832. (AICARFT_IMPCHas)
AICARFT/IMPCHase bienzyme
This is a family of bifunctional enzymes catalysing the last steps in de novo purine biosynthesis. The bifunctional enzyme is found in both prokaryotes and eukaryotes. The second last step is catalysed by 5-aminoimidazole-4-carboxamide ribonucleotide formyltransferase EC:2.1.2.3 (AICARFT), this enzyme catalyses the formylation of AICAR with 10-formyl-tetrahydrofolate to yield FAICAR and tetrahydrofolate [1]. The last step is catalysed by IMP (Inosine monophosphate) cyclohydrolase EC:3.5.4.10 (IMPCHase), cyclizing FAICAR (5-formylaminoimidazole-4-carboxamide ribonucleotide) to IMP [1].
Number of members: 22

[1] Akira T, Komatsu M, Nango R, Tomooka A, Konaka K, Yamauchi M, Kitamura Y, Nomura S, Tsukamoto I;

Medline: 97473523 "Molecular cloning and expression of a rat cDNA encoding 5-aminoimidazole-4-carboxamide ribonucleotide formyltransferase/IMP cyclohydrolase" [published erratum appears in Gene 1998 Feb 27;208(2):337] Gene 1997;197:289-293.

[2] Rayl EA, Moroson BA, Beardsley GP; Medline: 96147205 "The human purH gene product, 5-aminoimidazole-4-carboxamide ribonucleotide formyltransferase/IMP cyclohydrolase. Cloning, sequencing, expression, purification, kinetic analysis, and domain mapping." J Biol Chem 1996;271:2225-2233.

833. (AOX)

Alternative oxidase

The alternative oxidase is used as a second terminal oxidase in the mitochondria, electrons are transfered directly from reduced ubiquinol to oxygen forming water [2]. This is not coupled to ATP synthesis and is not inhibited by cyanide, this pathway is a single step process [1]. In rice the transcript levels of the alternative oxidase are increased by low temperature [1].

Number of members: 27

[1] Ito Y, Saisho D, Nakazono M, Tsutsumi N, Hirai A; Medline: 98086211 "Transcript levels of tandem-arranged alternative oxidase genes in rice are increased by low temperature." Gene 1997;203:121-129.

[2] Li Q, Ritzel RG, McLean LL, McIntosh L, Ko T, Bertrand H, Nargang FE; Medline: 96366413 "Cloning and analysis of the alternative oxidase gene of Neurospora crassa." Genetics 1996;142:129-140.

834. (APH)

Protein kinases signatures and profile

Cross-reference(s): PS00107; PROTEIN_KINASE_ATP, PS00108; PROTEIN_KINASE_ST, PS00109; PROTEIN_KINASE_TYR, PS50011; PROTEIN_KINASE_DOM

Eukaryotic protein kinases [1 to 5] are enzymes that belong to a very extensive family of proteins which share a conserved catalytic core common to both serine/threonine and tyrosine protein kinases. There are a number of conserved regions in the catalytic domain of protein kinases. Two of these regions have been selected to build signature patterns. The first region, which is located in the N-terminal extremity of the catalytic domain, is a glycine-rich stretch of residues in the vicinity of a lysine residue, which has been shown to be involved in ATP binding. The second region, which is located in the central part of the catalytic domain, contains a conserved aspartic acid residue which is important for the catalytic activity of the enzyme [6]; two signature patterns were derived for that region: one specific for serine/ threonine kinases and the other for tyrosine kinases. A profile was developed which is based on the alignment in [1] and covers the entire catalytic domain.

Consensus pattern: [LIV]-G-{P}-G-{P}-[FYWMGSTNH]-[SGA]-{PW}-[LIVCAT]-{PD}-x- [GSTACLIVMFY]-x(5,18)-[LIVMFYWCSTAR]-[AIVP]-[LIVMFAGCKR]-K [K binds ATP]

Sequences known to belong to this class detected by the pattern the majority of known protein kinases but it fails to find a number of them, especially viral kinases which are quite divergent in this region and are completely missed by this pattern.

Consensus pattern: [LIVMFYC]-x-[HY]-x-D-[LIVMFY]-K-x(2)-N-[LIVMFYCT](3) [D is an active site residue]

Sequences known to belong to this class detected by the pattern. Most serine/ threonine specific protein kinases with 10 exceptions (half of them viral kinases) and also Epstein-Barr virus BGLF4 and Drosophila ninaC which have respectively Ser and Arg instead of the conserved Lys and which are therefore detected by the tyrosine kinase specific pattern described below.

Consensus pattern: [LIVMFYC]-x-[HY]-x-D-[LIVMFY]-[RSTAC]-x(2)-N-[LIVMFYC](3) [D is an active site residue] tyrosine specific protein kinases with the exception of human ERBB3 and mouse blk. This pattern will also detect most bacterial aminoglycoside phosphotransferases [8,9] and herpesviruses ganciclovir kinases [10]; which are proteins structurally and evolutionary related to protein kinases. Sequences known to belong to this class detected by the profile ALL, except for three viral kinases. This profile also detects receptor guanylate cyclases (see <PDOC00430>) and 2-5A-dependent ribonucleases. Sequence similarities between these two families and the eukaryotic protein kinase family have been noticed before. It also detects Arabidopsis thaliana kinase- like protein TMKL1 which seems to have lost its catalytic activity.

Note if a protein analyzed includes the two protein kinase signatures, the probability of it being a protein kinase is close to 100%. Note eukaryotic-type protein kinases have also been found in prokaryotes such as Myxococcus xanthus [11] and Yersinia pseudotuberculosis. Note the patterns shown above has been updated since their publication in [7]. Note this documentation entry is linked to both signature patterns and a profile. As the profile is much more sensitive than the patterns, you should use it if you have access to the necessary software tools to do so.

References

[1] Hanks S.K., Hunter T., FASEB J. 9:576-596(1995).
[2] Hunter T., Meth. Enzymol. 200:3-37(1991).
[3] Hanks S.K., Quinn A.M., Meth. Enzymol. 200:38-62(1991).
[4] Hanks S.K., Curr. Opin. Struct. Biol. 1:369-383(1991).
[5] Hanks S.K., Quinn A.M., Hunter T., Science 241:42-52(1988).
[6] Knighton D.R., Zheng J., Ten Eyck L.F., Ashford V.A., Xuong N.-H., Taylor, S.S., Sowadski J.M., Science 253: 407-414(1991).
[7] Bairoch A., Claverie J.-M., Nature 331:22(1988).
[8] Benner S., Nature 329:21-21(1987).
[9] Kirby R., J. Mol. Evol. 30:489-492(1992).
[10] Littler E., Stuart A.D., Chee M.S., Nature 358:160-162(1992).
[11] Munoz-Dorado J., Inouye S., Inouye M., Cell 67:995-1006(1991).

835. (Asp_Glu_race)
Aspartate and glutamate racemases signatures
Cross-reference(s) PS00923; ASP_GLU_RACEMASE_1 PS00924; ASP_GLU_RACEMASE_2
Aspartate racemase (EC 5.1.1.13) and glutamate racemase (EC 5.1.1.3) are two evolutionary related bacterial enzymes that do not seem to require a cofactor for their activity [1]. Glutamate racemase, which interconverts L-glutamate into D-glutamate, is required for the biosynthesis of peptidoglycan and some peptide-based antibiotics such as gramicidin S. In addition to characterized aspartate and glutamate racemases, this family also includes a hypothetical protein from Erwinia carotovora and one from Escherichia coli (ygeA). Two conserved cysteines are present in the sequence of these enzymes. They are expected to play a role in catalytic activity by acting as bases in proton abstraction from the substrate. Signature patterns were developed for both cysteines.
Consensus pattern: [IVA]-[LIVM]-x-C-x(0,1)-N-[ST]-[MSA]-[STH]-[LIVFYSTANK]
Consensus pattern: [LIVM](2)-x-[AG]-C-T-[DEH]-[LIVMFY]-[PNGRS]-x-[LIVM]

[1] Gallo K.A., Knowles J.R., Biochemistry 32:3981-3990(1993).

836. (ATP-sulfurylase)
ATP-sulfurylase
This family consists of ATP-sulfurylase or sulfate adenylyltransferase EC:2.7.7.4 some of which are part of a bifunctional polypeptide chain associated with adenosyl phosphosulphate (APS) kinase APS_kinase. Both enzymes are required for PAPS (phosphoadenosine-phosphosulfate) synthesis from inorganic sulphate [2]. ATP sulfurylase catalyses the synthesis of adenosine-phosphosulfate APS from ATP and inorganic sulphate [1].
Number of members: 37

[1] Kurima K, Warman ML, Krishnan S, Domowicz M, Krueger RC Jr, Deyrup A, Schwartz NB; Medline: 98337975 "A member of a family of sulfate-activating enzymes causes murine brachymorphism" [published erratum appears in Proc Natl Acad Sci U S A 1998 Sep 29;95(20):12071] Proc Natl Acad Sci U S A 1998;95:8681-8685.

[2] Rosenthal E, Leustek T; Medline: 96096529 "A multifunctional Urechis caupo protein, PAPS synthetase, has both ATP sulfurylase and APS kinase activities." Gene 1995;165:243-248.

837. (ATP-synt_F)
ATP synthase (F/14-kDa) subunit
This family includes 14-kDa subunit from vATPases [1], which is in the peripheral catalytic part of the complex [2]. The family also includes archaebacterial ATP synthase subunit F [3].
Number of members: 23

[1] Guo Y, Kaiser K, Wieczorek H, Dow JA; Medline: 96269411 "The Drosophila melanogaster gene vha14 encoding a 14-kDa F-subunit of the vacuolar ATPase." Gene 1996;172:239-243.
[2] Peng SB, Crider BP, Tsai SJ, Xie XS, Stone DK; Medline: 96216416 "Identification of a 14-kDa subunit associated with the catalytic sector of clathrin-coated vesicle H+-ATPase." J Biol Chem 1996;271:3324-3327.
[3] Wilms R, Freiberg C, Wegerle E, Meier I, Mayer F, Muller V; Medline: 96324968 "Subunit structure and organization of the genes of the A1A0 ATPase from the Archaeon Methanosarcina mazei Gol." J Biol Chem 1996;271: 18843-18852.

838. (CBD_4)

Starch binding domain
Number of members: 48
839. (CbiX)
The function of CbiX is uncertain, however it is found in cobalamin biosynthesis operons and so may have a related function. Some CbiX proteins contain a striking histidine-rich region at their C-terminus, which suggests that it might be involved in metal chelation [1].
Number of members: 6

[1] Raux E, Lanois A, Warren MJ, Rambach A, Thermes C; Medline: 98416126 "Cobalamin (vitamin B12) biosynthesis: identification and characterization of a Bacillus megaterium cobI operon." Biochem J 1998;335:159-166.

840. (Complex1_51K)
Respiratory-chain NADH dehydrogenase 51 Kd subunit signatures Cross-reference(s) PS00644; COMPLEX1_51K_1 PS00645; COMPLEX1_51K_2
Respiratory-chain NADH dehydrogenase (EC 1.6.5.3) [1,2] (also known as complex I or NADH-ubiquinone oxidoreductase) is an oligomeric enzymatic complex located in the inner mitochondrial membrane which also seems to exist in the chloroplast and in cyanobacteria (as a NADH-plastoquinone oxidoreductase). Among the 25 to 30 polypeptide subunits of this bioenergetic enzyme complex there is one with a molecular weight of 51 Kd (in mammals), which is the second largest subunit of complex I and is a component of the iron-sulfur (IP) fragment of the enzyme. It seems to bind to NAD, FMN, and a 2Fe-2S cluster.
The 51 Kd subunit is highly similar to [3,4]:

- Subunit alpha of Alcaligenes eutrophus NAD-reducing hydrogenase (gene hoxF) which also binds to NAD, FMN, and a 2Fe-2S cluster.
- Subunit NQO1 of Paracoccus denitrificans NADH-ubiquinone oxidoreductase.
- Subunit F of Escherichia coli NADH-ubiquinone oxidoreductase (gene nuoF).

The 51 Kd subunit and the bacterial hydrogenase alpha subunit contains three regions of sequence similarities. The first one most probably corresponds to the NAD-binding site, the second to the FMN-binding site, and the third one, which contains three cysteines, to the iron-sulfur binding region. Signature patterns have been developed for the FMN-binding and for the 2Fe-2S binding regions.
Consensus pattern: G-[AM]-G-[AR]-Y-[LIVM]-C-G-[DE](2)-[STA](2)-[LIM](2)-[EN]- S
Consensus pattern: E-S-C-G-x-C-x-P-C-R-x-G [The three C's are putative 2Fe-2S ligands]

[1] Ragan C.I., Curr. Top. Bioenerg. 15:1-36(1987).
[2] Weiss H., Friedrich T., Hofhaus G., Preis D., Eur. J. Biochem. 197:563-576(1991).
[3] FeamleyI.M., Walker J.E. Biochim. Biophys. Acta 1140:105-134(1992).
[4] Weidner U., Geier S., Ptock A., Friedrich T., Leif H., Weiss H., J. Mol. Biol. 233:109-122(1993).

841. (DAP_epimerase)
Diaminopimelate epimerase signature
Cross-reference(s) PS01326; DAP_EPIMERASE
Diaminopimelate epimerase (EC 5.1.1.7) catalyzes the isomeriazation of L,L- to D,L-mesodiaminopimelate in the biosynthetic pathway leading from aspartate to lysine. This enzyme is a protein of about 30 Kd. Two conserved cysteines seem [1] to function as the acid and base in the catalytic mechanism. As a signature pattern, the region surrounding the first of these two active site cysteines were selected.
Consensus pattern: N-x-D-G-S-x(4)-C-G-N-[GA]-x-R [C is an active site residue] Sequences known to belong to this class detected by the pattern ALL, except for an Anabaena dapF which has a Ser instead of the active site Cys.

[1] Cirilli M., Zheng R., Scapin G., Blanchard J.S., Biochemistry 37:16452-16458(1998).

842. (DNA_gyraseB_ C)
DNA topoisomerase II signature
Cross-reference(s) PS00177; TOPOISOMERASE_II
DNA topoisomerase I (EC 5.99.1.2) [1,2,3,4,E 1] is one of the two types of enzyme that catalyze the interconversion of topological DNA isomers. Type II topoisomerases are ATP-dependent and act by passing a DNA segment through a transient double-strand break. Topoisomerase II is found in phages, archaebacteria, prokaryotes, eukaryotes, and in African Swine Fever virus (ASF). In bacteriophage T4 topoisomerase II consists of three subunits (the product of genes

39, 52 and 60). In prokaryotes and in archaebacteria the enzyme, known as DNA gyrase, consists of two subunits (genes gyrA and gyrB [E2]). In some bacteria, a second type II topoisomerase has been identified; it is known as topoisomerase IV and is required for chromosome segregation, it also consists of two subunits (genes parC and parE). In eukaryotes, type II topoisomerase is a homodimer.
There are many regions of sequence homology between the different subtypes of topoisomerase II. The relation between the different subunits is shown in the following representation:

```
<----------------About-1400-residues----------------------->

[----------Protein 39-*-----][----Protein 52----]          Phage T4
[----------gyrB-------*-----][--------gyrA-----------------]  Prokaryote II
                                                     Archaebacteria
[----------parE-------*-----][--------parD-----------------]  Prokaryote IV
[---------------------*------------------------------------]  Eukaryote and
                                                     ASF
```

'*': Position of the pattern.
As a signature pattern for this family of proteins, a region that contains a highly conserved pentapeptide was selected. The pattern is located in gyrB, in parE, and in protein 39 of phage T4 topoisomerase.
Consensus pattern: [LIVMA]-x-E-G-[DN]-S-A-x-[STAG]

[1] Sternglanz R., Curr. Opin. Cell Biol. 1:533-535(1990).
[2] Bjornsti M.-A., Curr. Opin. Struct. Biol. 1:99-103(1991).
[3] Sharma A., Mondragon A., Curr. Opin. Struct. Biol. 5:39-47(1995).
[4] Roca J., Trends Biochem. Sci. 20:156-160(1995).

843. (DUF16)
Protein of unknown function
The function of this protein is unknown. It appears to only occur in Mycoplasma pneumoniae.
Number of members: 26

[1] Himmelreich R, Hilbert H, Plagens H, Pirkl E, Li BC, Herrmann R; Medline: 97105885 "Complete sequence analysis of the genome of the bacterium Mycoplasma pneumoniae." Nucleic Acids Res 1996;24:4420-4449.

844. (DUF21)
Domain of unknown function
This transmembrane region has no known function. Many of the sequences in this family are annotated as hemolysins, however this is due to a similarity to Swiss:Q54318 that does not contain this domain. This domain is found in the N-terminus of the proteins adjacent to two intracellular CBS domains CBS.
Number of members: 42
845. (DUF56)
Integral membrane protein
The members of this family are putative integral membrane proteins. The function of the family is unknown, however the family includes Sec59 from yeast. Sec59 is a dolichol kinase EC:2.7.1.108, but it is not clear if the enzymatic activity resides in this region or its N terminal region.
Number of members: 13
846. (DUF94)
Domain of unknown function
The function of this domain is unknown. It is found in both eukaryotes and archaebacteria. The alignment contains a completely conserved aspartate residue that may be functionally important. The eukaryotic domains contains three conserved cysteines and a histidine that might be metal binding, however these are absent in the archaebacterial proteins.
Number of members: 9

847. (FF)
FF domain
This domain may be involved in protein-protein interaction [1].
Number of members: 42

[1] Bedford MT, Leder P; Medline: 99322199 "The FF domain: a novel motif that often accompanies WW domains." Trends Biochem Sci 1999;24:264-265.

848. (FLO_LFY)
Floricaula / Leafy protein
This family consists of various plant development proteins which are homologues of floricaula (FLO) and Leafy (LFY) proteins which are floral meristem identity proteins. Mutations in the sequences of these proteins affect flower and leaf development.
Number of members: 16

[1] Hofer J, Turner L, Hellens R, Ambrose M, Matthews P, Michael A, Ellis N; Medline: 97411151 "UNIFOLIATA regulates leaf and flower morphogenesis in pea." Curr Biol 1997;7:581-587.
[2] Weigel D, Alvarez J, Smyth DR, Yanofsky MF, Meyerowitz EM; Medline: 92274452 "LEAFY controls floral meristem identity in Arabidopsis." Cell 1992;69:843-859.

849. (G-patch)
G-patch domain
This domain is found in a number of RNA binding proteins, and is also found in proteins that contain RNA binding domains. This suggests that this domain may have an RNA binding function. This domain has seven highly conserved glycines.
Number of members: 47

[1] Aravind L, Koonin EV; Medline: 10470032 "G-patch: a new conserved domain in eukaryotic RNA-processing proteins and type D retroviral polyproteins." Trends Biochem Sci 1999;24:342-344.

850. (Gram-ve_porins)
General diffusion Gram-negative porins signature
Cross-reference(s) PS00576; GRAM_NEG_PORIN
The outer membrane of Gram-negative bacteria acts as a molecular filter for hydrophilic compounds. Proteins, known as porins [1], are responsible for the 'molecular sieve' properties of the outer membrane. Porins form large water- filled channels which allows the diffusion of hydrophilic molecules into the periplasmic space. Some porins form general diffusion channels that allows any solutes up to a certain size (that size is known as the exclusion limit) to cross the membrane, while other porins are specific for a solute and contain a binding site for that solute inside the pores (these are known as selective porins). As porins are the major outer membrane proteins, they also serve as receptor sites for the binding of phages and bacteriocins. General diffusion porins generally assemble as trimer in the membrane and the transmembrane core of these proteins is composed exclusively of beta strands [2]. It has been shown [3] that a number of general porins are evolutionary related, these porins are:

- Enterobacteria phoE.
- Enterobacteria ompC.
- Enterobacteria ompF.
- Enterobacteria nmpC.
- Bacteriophage PA-2 LC.
- Neisseria PI.A.
- Neisseria PI.B.

As a signature pattern a conserved region was selected, located in the C-terminal part of these proteins, which spans two putative transmembrane beta strands.
Consensus pattern: [LIVMFY]-x(2)-G-x(2)-Y-x-F-x-K-x(2)-[SN]-[STAV]-[LIVMFYW]- V

[1] Benz R., Bauer K., Eur. J. Biochem. 176:1-19(1988).
[2] Jap B.K., Walian P.J., Q. Rev. Biophys. 23:367-403(1990).
[3] Jeanteur D., Lakey J.H., Pattus F., Mol. Microbiol. 5:2153-2164(1991).

851. (HlyD)
HlyD family secretion proteins signature
Cross-reference(s) PS00543; HLYD_FAMILY
Gram-negative bacteria produce a number of proteins which are secreted into the growth medium by a mechanism that does not require a cleaved N-terminal signal sequence. These proteins, while having different functions, require the help of two or more proteins for their secretion across the cell envelope. Amongst which a protein belonging to the ABC transporters family (see the relevant entry <pDOC00185>) and a protein belonging to a family which is currently composed [1 to 5] of the following members:

| Gene | Species | Protein which is exported |
|---|---|---|
| ----- | --------------------- | ------------------------------------------------------ |
| hlyD | Escherichia coli | Hemolysin |
| appD | A.pleuropneumoniae | Hemolysin |
| lcnD | Lactococcus lactis | Lactococcin A |
| 1ktD | A.actinomycetemcomitans Pasteurella haemolytica | Leukotoxin |
| rtxD | A.pleuropneumoniae | Toxin-III |
| cyaD | Bordetella pertussis | Calmodulin-sensitive adenylate cyclase-hemolysin (cyclolysin) |
| cvaA | Escherichia coli | Colicin V |
| prtE | Erwinia chrysanthemi | Extracellular proteases B and C |
| aprE | Pseudomonas aeruginosa | Alkaline protease |
| emrA | Escherichia coli | Drugs and toxins |
| yjcR | Escherichia coli | Unknown |

These proteins are evolutionary related and consist of from 390 to 480 amino acid residues. They seem to be anchored in the inner membrane by a N-terminal transmembrane region. Their exact role in the secretion process is not yet known. The C-terminal section of these proteins is the best conserved region; a signature pattern from that region was derived.
Consensus pattern: [LIVM]-x(2)-G-[LM]-x(3)-[STGAV]-x-[LIVMT]-x-[LIVMT]-[GE]-x-[KR]-x-[LIVMFYW](2)-x-[LIVMFYW](3)
Sequences known to belong to this class detected by the pattern ALL, except for emrA and yjcR. References:


[1] Gilson L., Mahanty H.K., Kolter R., EMBO J. 9:3875-3884(1990).
[2] Letoffe S., Delepelaire P., Wandersman C., EMBO J. 9:1375-1382(1990).
[3] Stoddard G.W., Petzel J.P., van Belkum M.J., Kok J., McKay L.L., Appl. Environ. Microbiol. 58:1952-1961(1992).
[4] Duong F., Lazdunski A., Cami B., Murgier M., Gene 121:47-54(1992).
[5] Lewis K., Trends Biochem. Sci. 19:119-123(1994).


852. (IBR)
In Between Ring fingers
The IBR (In Between Ring fingers) domain is found to occur between pairs of ring fingers (zf-C3HC4). The function of this domain is unknown. This domain has also been called the C6HC domain and DRIL (for double RING finger linked) domain [2].
Number of members: 25


[1] Morett E, Bork P; Medline: 10366851 "A novel transactivation domain in parkin."Trends Biochem Sci 1999;24: 229-231.
[2] van der Reijden BA, Erpelinck-Verschueren CA, Lowenberg B, Jansen JH; Medline: 99349709 "TRIADs: a new class of proteins with a novel cysteine-rich signature." Protein Sci 1999;8:1557-1561.


853. (IPPT)
IPP transferase


[1] Durand JM, Bjork GR, Kuwae A, Yoshikawa M, Sasakawa C; Medline: 97440126 "The modified nucleoside 2-methylthio-N6-isopentenyladenosine in tRNA of Shigella flexneri is required for expression of virulence genes." J Bacteriol 1997;179:5777-5782.
[2] Boguta M, Hunter LA, Shen WC, Gillman EC, Martin NC, Hopper AK; Medline: 94187700 "Subcellular locations

of MOD5 proteins: mapping of sequences sufficient for targeting to mitochondria and demonstration that mitochondrial and nuclear isoforms commingle in the cytosol." Mol Cell Biol 1994;14:2298-2306.
[3] Gillman EC, Slusher LB, Martin NC, Hopper AK; Medline: 91203856 "MOD5 translation initiation sites determine N6-isopentenyladenosine modification of mitochondrial and cytoplasmic tRNA." Mol Cell Biol 1991;11:2382-2390.

854. (KE2)
KE2 family protein
The function of members of this family is unknown, although they have been suggested to contain a DNA binding leucine zipper motif [2].
Number of members: 9

[1] Ha H, Abe K, Artzt K; Medline: 92084131 "Primary structure of the embryo-expressed gene KE2 from the mouse H-2K region." Gene 1991;107:345-346.
[2] Shang HS, Wong SM, Tan HM, Wu M; Medline: 95129859 "YKE2, a yeast nuclear gene encoding a protein showing homology to mouse KE2 and containing a putative leucine-zipper motif." Gene 1994;151:197-201.

855. (Lipoprotein_6)
Prokaryotic membrane lipoprotein lipid attachment site
Cross-reference(s) PS00013; PROKAR_LIPOPROTEIN
In prokaryotes, membrane lipoproteins are synthesized with a precursor signal peptide, which is cleaved by a specific lipoprotein signal peptidase (signal peptidase II). The peptidase recognizes a conserved sequence and cuts upstream of a cysteine residue to which a glyceride-fatty acid lipid is attached [1]. Some of the proteins known to undergo such processing currently include (for recent listings see [1,2,3]):

- Major outer membrane lipoprotein (murein-lipoproteins) (gene 1pp).
- Escherichia coli lipoprotein-28 (gene nlpA).
- Escherichia coli lipoprotein-34 (gene nlpB).
- Escherichia coli lipoprotein nlpC.
- Escherichia coli lipoprotein nlpD.
- Escherichia coli osmotically inducible lipoprotein B (gene osmB).
- Escherichia coli osmotically inducible lipoprotein E (gene osmE).
- Escherichia coli peptidoglycan-associated lipoprotein (gene pal).
- Escherichia coli rare lipoproteins A and B (genes rplA and rplB).
- Escherichia coli copper homeostasis protein cutF (or nlpE).
- Escherichia coli plasmids traT proteins.
- Escherichia coli Col plasmids lysis proteins.
- A number of Bacillus beta-lactamases.
- Bacillus subtilis periplasmic oligopeptide-binding protein (gene oppA).
- Borrelia burgdorferi outer surface proteins A and B (genes ospA and ospB).
- Borrelia hermsii variable major protein 21 (gene vmp21) and 7 (gene vmp7).
- Chlamydia trachomatis outer membrane protein 3 (gene omp3).
- Fibrobacter succinogenes endoglucanase cel-3.
- Haemophilus influenzae proteins Pal and Pcp.
- Klebsiella pullulunase (gene pulA).
- Klebsiella pullulunase secretion protein pulS.
- Mycoplasma hyorhinis protein p37.
- Mycoplasma hyorhinis variant surface antigens A, B, and C (genes vlpABC).
- Neisseria outer membrane protein H.8.
- Pseudomonas aeruginosa lipopeptide (gene lppL).
- Pseudomonas solanacearum endoglucanase egl.
- Rhodopseudomonas viridis reaction center cytochrome subunit (gene cytC).
- Rickettsia 17 Kd antigen.
- Shigella flexneri invasion plasmid proteins mxiJ and mxim.
- Streptococcus pneumoniae oligopeptide transport protein A (gene amiA).
- Treponema pallidium 34 Kd antigen.
- Treponema pallidium membrane protein A (gene tmpA).
- Vibrio harveyi chitobiase (gene chb).
- Yersinia virulence plasmid protein yscJ.

- Halocyanin from Natrobacterium pharaonis [4], a membrane associated copper-binding protein. This is the first archaebacterial protein known to be modified in such a fashion).

From the precursor sequences of all these proteins, a consensus pattern and a set of rules to identify this type of post-translational modification were derived.
Consensus pattern: {DERK}(6)-[LIVMFWSTAG](2)-[LIVMFYSTAGCQ]-[AGS]-C [C is the lipid attachment site] Additional rules: 1)
The cysteine must be between positions 15 and 35 of the sequence in consideration. 2) There must be at least one Lys or one Arg in the first seven positions of the sequence. Sequences known to belong to this class detected by the pattern ALL. Other sequence(s) detected in SWISS-PROT some 100 prokaryotic proteins. Some of them are not membrane lipoproteins, but at least half of them could be.
References

[1] Hayashi S., Wu H.C., J. Bioenerg. Biomembr. 22:451-471(1990).
[2] Klein P., Somorjai R.L., Lau P.C.K., Protein Eng. 2:15-20(1988).
[3] von Heijne G., Protein Eng. 2:531-534(1989).
[4] Mattar S., Scharf B., Kent S.B.H., Rodewald K., Oesterhelt D., Engelhard M. J. Biol. Chem. 269:14939-14945 (1994).

856. (Lipoprotein_7)
Adhesin lipoprotein
This family consists of the p50 and variable adherence-associated antigen (Vaa) adhesins from Mycoplasma hominis. M. hominis is a mycoplasma associated with human urogenital diseases, pneumonia, and septic arthritis [1]. An adhesin is a cell surface molecule that mediates adhesion to other cells or to the surrounding surface or substrate. The Vaa antigen is a 50-kDa surface lipoprotein that has four tandem repetitive DNA sequences encoding a periodic peptide structure, and is highly immunogenic in the human host [1]. p50 is also a 50-kDa lipoprotein, having three repeats A,B and C, that may be a tetramer of 191-kDa in its native environment [2].
Number of members: 18

[1] Zhang Q, Wise KS; Medline: 96294788 "Molecular basis of size and antigenic variation of a Mycoplasma hominis adhesin encoded by divergent vaa genes. " Infect Immun 1996;64:2737-2744.
[2] Henrich B, Kitzerow A, Feldmann RC, Schaal H, Hadding U; Medline: 97047675 "Repetitive elements of the Mycoplasma hominis adhesin p50 can be differentiated by monoclonal antibodies." Infect Immun 1996;64: 4027-4034.

857. (MaoC_like)
MaoC like domain
The MaoC protein is found to share similarity with a wide variety of enzymes; estradiol 17 beta-dehydrogenase 4, peroxisomal hydratase-dehydrogenase-epimerase, fatty acid synthase beta subunit. All these enzymes contain other domains. This domain is also present in the NodN nodulation protein N. No specific function has been assigned to this region of any of these proteins. The maoC gene is part of a operon with maoA which is involved in the synthesis of monoamine oxidase [1].
Number of members: 46

[1] Sugino H, Sasaki M, Azakami H, Yamashita M, Murooka Y Medline: 96235221 "A monoamine-regulated Klebsiella aerogenes operon containing the monoamine oxidase structural gene (maoA) and the maoC gene." J Bacteriol 1992;174:2485-2492.

858. (MSP)
Manganese-stabilizing protein / photosystem II polypeptide
This family consists of the 33 KDa photosystem II polypeptide from the oxygen evolving complex (OEC) of plants and cyanobacteria. The protein is also known as the manganese-stabilizing protein as it is associated with the manganese complex of the OEC and may provide the ligands for the complex [1].
Number of members: 17

[1] Philbrick JB, Zilinskas BA; Medline: 88334494 "Cloning, nucleotide sequence and mutational analysis of the gene encoding the Photosystem II manganese-stabilizing polypeptide of Synechocystis 6803." Mol Gen Genet 1988; 212:418-425.

859. (NAC)

[1] Makarova KS, Aravind L, Galperin MY, Grishin NV, Tatusov RL, Wolf YI, Koonin EV; Medline: 99342100 "Comparative genomics of the Archaea (Euryarchaeota): evolution of conserved protein families, the stable core, and the variable shell." Genome Res 1999;9:608-628.

Number of members: 27

860. (Nop)

Putative snoRNA binding domain

This family consists of various Pre RNA processing ribonucleoproteins. The function of the aligned region is unknown however it may be a common RNA or snoRNA or Nop1p binding domain. Nop5p (Nop58p) Swiss:Q12499 from yeast is the protein component of a ribonucleoprotein protein required for pre-18s rRNA processing and is suggested to function with Nop1p in a snoRNA complex [1]. Nop56p Swiss:000567 and Nop5p interact with Nop1p and are required for ribosome biogenesis [2]. Prp31p Swiss:p49704 is required for pre-mRNA splicing in S. cerevisiae [3].

Number of members: 23

[1] Wu P, Brockenbrough JS, Metcalfe AC, Chen S, Aris JP; Medline: 98298165 "Nop5p is a small nucleolar ribonucleoprotein component required for pre- 18 S rRNA processing in yeast." J Biol Chem 1998;273:16453-16463.

[2] Gautier T, Berges T, Tollervey D, Hurt E;Medline: 8038777 "Nucleolar KKE/D repeat proteins Nop56p and Nop58p interact with Nop1p and are required for ribosome biogenesis." Mol Cell Biol 1997;17:7088-7098.

[3] Weidenhammer EM, Singh M, Ruiz-Noriega M, Woolford JL Jr; Medline: 96184869 "The PRP31 gene encodes a novel protein required for pre-mRNA splicing in Saccharomyces cerevisiae." Nucleic Acids Res 1996;24: 1164-1170.

861. (Nramp)

Natural resistance-associated macrophage protein

The natural resistance-associated macrophage protein (NRAMP) family consists of Nramp1, Nramp2, and yeast proteins Smf1 and Smf2. The NRAMP family is a novel family of functional related proteins defined by a conserved hydrophobic core of ten transmembrane domains [5]. This family of membrane proteins are divalent cation transporters. Nramp1 is an integral membrane protein expressed exclusively in cells of the immune system and is recruited to the membrane of a phagosome upon phagocytosis [1]. By controlling divalent cation concentrations Nramp1 may regulate the interphagosomal replication of bacteria [1]. Mutations in Nramp 1 may genetically predispose an individual to susceptibility to diseases including leprosy and tuberculosis conversely this might however provide protection form rheumatoid arthritis [1]. Nramp2 is a multiple divalent cation transporter for Fe2+, Mn2+ and Zn2+ amongst others it is expressed at high levels in the intestine; and is major transferrin-independent iron uptake system in mammals [1]. The yeast proteins Smf1 and Smf2 may also transport divalent cations [3].

Number of members: 36

[1] Govoni G, Gros P; Medline: 98383996 "Macrophage NRAMP1 and its role in resistance to microbial infections." Inflamm Res 1998;47:277-284.

[2] Agranoff DD, Krishna S Medline: 98294035 "Metal ion homeostasis and intracellular parasitism." Mol Microbiol 1998;28:403-412.

[3] Pinner E, Gruenheid S, Raymond M, Gros P; Medline: 98030569 "Functional complementation of the yeast divalent cation transporter family SMF by NRAMP2, a member of the mammalian natural resistance- associated macrophage protein family." J Biol Chem 1997;272:28933-28938.

[4] Cellier M, Belouchi A, Gros P; Medline: 96402487 "Resistance to intracellular infections: comparative genomic analysis ofNramp." Trends Genet 1996;12:201-204.

[5] Cellier M, Prive G, Belouchi A, Kwan T, Rodrigues V, Chia W, Gros P; Medline: 96036029 "Nramp defines a family of membrane proteins." Proc Natl Acad Sci U S A 1995;92:10089-10093.

862. (NTP_transf_2)

Nucleotidyltransferase domain

Members of this family belong to a large family ofnucleotidyltransferases [1].

Number of members: 83

[1] Holm L, Sander C; Medline: 96005605 "DNA polymerase beta belongs to an ancient nucleotidyltransferase superfamily." Trends Biochem Sci 1995;20:345-347.

863. (Paramyxo_P)
Paramyxovirus P phosphoprotein
This family consists of paramyxovirus P phosphoprotein from sendai virus and human and bovine parainfluenza viruses. The P protein is an essential part of the viral RNA polymerase complex formed form the P and L proteins [1]. The exact role of the P protein in this complex in unknown but it is involved in multiple protein-protein interactions and binding the polymerase complex to the nucleocapsid or ribonucleoprotein template [1]. It also appears to be important for the proper folding of the L protein [1]. The paramyxoviruses have a negative sense ssRNA genome [1].
Number of members: 15

[1] Bowman MC, Smallwood S, Moyer SA; Medline: 99329169 "Dissection of Individual Functions of the Sendai Virus Phosphoprotein in Transcription." J Virol 1999;73:6474-6483.
[2] Matsuoka Y, Curran J, Pelet T, Kolakofsky D, Ray R, Compans RW; Medline: 91237868 "The P gene of human parainfluenza virus type 1 encodes P and C proteins but not a cysteine-rich V protein." J Virol 1991;65:3406-3410.

864. (Patatin)
This family consists of various patatin glycoproteins from plants. The patatin protein accounts for up to 40% of the total soluble protein in potato tubers [2]. Patatin is a storage protein but it also has the enzymatic activity of lipid acyl hydrolase, catalysing the cleavage of fatty acids from membrane lipids [2].
Number of members: 21

[1] Banfalvi Z, Kostyal Z, Barta E; Medline: 95107249 "Solanum brevidens possesses a non-sucrose-inducible patatin gene." Mol Gen Genet 1994;245:517-522.
[2] Mignery GA, Pikaard CS, Park WD; Medline: 88226014 "Molecular characterization of the patatin multigene family of potato." Gene 1988;62:27-44.

865. (Pentapeptide_2)
Pentapeptide repeats (8 copies)
These repeats are found in many mycobacterial proteins. These repeats are most common in the PPE family of proteins, where they are found in the MPTR subfamily of PPE proteins. The function of these repeats is unknown. The repeat can be approximately described as XNXGX, where X can be any amino acid. These repeats are similar to Pentapeptide [1], however it is not clear if these two families are structurally related.
Number of members: 362

[1] Bateman A, Murzin A, Teichmann SA; Medline: 98318059 "Structure and distribution of pentapeptide repeats in bacteria." Protein Sci 1998;7:1477-1480.
[2] Cole ST, Brosch R, Parkhill J, Garnier T, Churcher C, Harris D, Gordon SV, Eiglmeier K, Gas S, Barry CE 3rd, Tekaia F, Badcock K, Basham D, Brown D, Chillingworth T, Connor R, Davies R, Devlin K, Feltwell T, Gentles S, Hamlin N, Holroyd S, Hornsby T, Jagels K, Barrell BG; Medline: 98295987 "Deciphering the biology of Mycobacterium tuberculosis from the complete genome sequence." Nature 1998;393:537-544.

866. (Peptidase_C13)
Peptidase C13 family
This family of peptidases is known as the hemoglobinase family because it contains a globin degrading enzyme from blood parasites Swiss:P42665. However relatives are found in plants and other organisms that have other functions. Members of this family are asparaginyl peptidases [1].
Number of members: 26

[1] Chen JM, Dando PM, Rawlings ND, Brown MA, Young NE, Stevens RA, Hewitt E, Watts C, Barrett AJ; Medline: 97218252 "Cloning, isolation, and characterization of mammalian legumain, an asparaginyl endopeptidase." J Biol Chem 1997;272:8090-8098.

867. (Pro_dh)
Proline dehydrogenase
Number of members: 25

[1] Ling M, Allen SW, Wood JM; Medline: 95055736 "Sequence analysis identifies the proline dehydrogenase and delta 1- pyrroline-5-carboxylate dehydrogenase domains of the multifunctional Escherichia coli PutA protein." J Mol Biol 1994;243:950-956.

868. (PsbP)

This family consists of the 23 kDa subunit of oxygen evolving system of photosystem II or PsbP from various plants (where it is encoded by the nuclear genome) and Cyanobacteria. The 23 KDa PsbP protein is required for PSII to be fully operational in vivo, it increases the affinity of the water oxidation site for Cl- and provides the conditions required for high affinity binding of Ca2+ [2].

Number of members: 25

[1] Rova EM, Mc Ewen B, Fredriksson PO, Styring S; Medline: 97067138 "Photoactivation and photoinhibition are competing in a mutant of Chlamydomonas reinhardtii lacking the 23-kDa extrinsic subunit of photosystem II." J Biol Chem 1996;271:28918-28924.

[2] Kochhar A, Khurana JP, Tyagi AK; Medline: 97191538 "Nucleotide sequence of the psbP gene encoding precursor of 23-kDa polypeptide of oxygen-evolving complex in Arabidopsis thaliana and its expression in the wild-type and a constitutively photomorphogenic mutant." DNA Res 1996;3:277-285.

869. (PUA)

The PUA domain named after PseudoUridine synthase and Archaeosine transglycosylase, was detected in archaeal and eukaryotic pseudouridine synthases, archaeal archaeosine synthases, a family of predicted ATPases that may be involved in RNA modification, a family of predicted archaeal and bacterial rRNA methylases. Additionally, the PUA domain was detected in a family of eukaryotic proteins that also contain a domain homologous to the translation initiation factor eIF1/SUI1; these proteins may comprise a novel type of translation factors. Unexpectedly, the PUA domain was detected also in bacterial and yeast glutamate kinases; this is compatible with the demonstrated role of these enzymes in the regulation of the expression of other genes [1]. It is predicted that the PUA domain is an RNA binding domain.

Number of members: 48

[1] Aravind L, Koonin EV; Medline: 99193178 "Novel predicted RNA-binding domains associated with the translation machinery." J Mol Evol 1999;48:291-302.

870. (RF1)

eRF1-like proteins

Members of this family are peptide chain release factors. The eukaryotic Release Factor 1 proteins (eRFl s) are involved in termination of translation. The eRF1 protein is functional for all stop codons and appears to abolish read-through of these codons. This family also includes other proteins for which the precise molecular function is unknown. Many of them are from Archaebacteria. These proteins may also be involved in translation termination but this awaits experimental verification.

Number of members: 25

[1] Frolova L, Le Goff X, Rasmussen HH, Cheperegin S, Drugeon G, Kress M, Arman I, Haenni AL, Celis JE, Philippe M, et al; Medline: 95082951 "A highly conserved eukaryotic protein family possessing properties of polypeptide chain release factor" [see comments] Nature 1994;372:701-703.

[2] Drugeon G, Jean-Jean O, Frolova L, Le Goff X, Philippe M, Kisselev L, Haenni AL; Medline: 97315314 "Eukaryotic release factor 1 (eRF1) abolishes readthrough and competes with suppressor tRNAs at all three termination codons in messenger RNA." Nucleic Acids Res 1997;25:2254-2258.

871. (Ribosomal_L14e)

Ribosomal protein L14

This family includes the eukaryotic ribosomal protein L14.

Number of members: 15

872. (Ribosomal_S27)

Ribosomal protein S27a

This family of ribosomal proteins consists mainly of the 40S ribosomal protein S27a which is synthesized as a C-terminal extension of ubiquitin (CEP). The S27a domain compromises the C-terminal half of the protein. The synthesis of ribosomal proteins as extensions of ubiquitin promotes their incorporation into nascent ribosomes by a transient metabolic stabilization and is required for efficient ribosome biogenesis [3]. The ribosomal extension protein S27a contains a basic region that is proposed to form a zinc finger; its fusion gene is proposed as a mechanism to maintain a fixed ratio between ubiquitin necessary for degrading proteins and ribosomes a source of proteins [2].

Number of members: 36

873. (Spermine_synth)

Spermine/spermidine synthase

Spermine and spermidine are polyamines. This family includes spermidine synthase that catalyses the fifth (last) step in the biosynthesis of spermidine from arginine, and spermine synthase.
Number of members: 39

[1] Mezquita J, Pau M, Mezquita C; Medline: 97449308 "Characterization and expression of two chicken cDNAs encoding ubiquitin fused to ribosomal proteins of 52 and 80 amino acids." Gene 1997;195:313-319.
[2] Redman KL, Rechsteiner M; Medline: 89181932 "Identification of the long ubiquitin extension as ribosomal protein S27a." Nature 1989;338:438-440.
[3] Finley D, Bartel B, Varshavsky A; Medline: 89181925 "The tails of ubiquitin precursors are ribosomal proteins whose fusion to ubiquitin facilitates ribosome biogenesis." Nature 1989;338:394-401.

874. (Surp)
Surp module

[1] Denhez F, Lafyatis R; Medline: 94266805 "Conservation of regulated alternative splicing and identification of functional domains in vertebrate homologs to the Drosophila splicing regulator, suppressor-of-white-apricot." J Biol Chem 1994;269:16170-16179.

This domain is also known as the SWAP domain. SWAP stands for Suppressor-of-White-APricot. It has been suggested that these domains may be RNA binding [1].
Number of members: 32
875. (TFIIE)
TFIIE alpha subunit
The general transcription factor TFIIE has an essential role in eukaryotic transcription initiation together with RNA polymerase II and other general factors. Human TFIIE consists of two subunits TFIIE-alpha Swiss:P29083 and TFIIE-beta Swiss:P29084 and joins the preinitiation complex after RNA polymerase II and TFIIF [1]. This family consists of the conserved amino terminal region of eukaryotic TFIIE-alpha [2] and proteins from archaebacteria that are presumed to be TFIIE-alpha subunits also Swiss:029501 [3].
Number of members: 12

[1] Ohkuma Y, Sumimoto H, Hoffmann A, Shimasaki S, Horikoshi M, Roeder RG; Medline: 92065982 "Structural motifs and potential sigma homologies in the large subunit of human general transcription factor TFIIE." Nature 1991;354:398-401.
[2] Ohkuma Y, Hashimoto S, Roeder RG, Horikoshi M; Medline: 93087200 Identification of two large subdomains in TFIIE-alpha on the basis of homology between Xenopus and human sequences. Nucleic Acids Res 1992;20: 5838-5838.
[3] Klenk HP, Clayton RA, Tomb JF, White O, Nelson KE, Ketchum KA, Dodson RJ, Gwinn M, Hickey EK, Peterson JD, Richardson DL, Kerlavage AR, Graham DE, Kyrpides NC, Fleischmann RD, Quackenbush J, Lee NH, Sutton GG, Gill S, Kirkness EF, Dougherty BA, McKenney K, Adams MD, Loftus B, Venter JC, et al; Medline: 98049343 "The complete genome sequence of the hyperthermophilic, sulphate- reducing archaeon Archaeoglobus fulgidus." Nature 1997;390:364-370.

876. (Transglut_core)
Cross-reference(s) PS00547; TRANSGLUTAMINASES
Transglutaminases (EC 2.3.2.13) (TGase) [1,2] are calcium-dependent enzymes that catalyze the cross-linking of proteins by promoting the formation of isopeptide bonds between the gamma-carboxyl group of a glutamine in one polypeptide chain and the epsilon-amino group of a lysine in a second polypeptide chain. TGases also catalyze the conjugation of polyamines to proteins. The best known transglutaminase is blood coagulation factor XIII, a plasma tetrameric protein composed of two catalytic A subunits and two non-catalytic B subunits. Factor XIII is responsible for cross-linking fibrin chains, thus stabilizing the fibrin clot. Other forms of transglutaminases are widely distributed in various organs, tissues and body fluids. Sequence data is available for the following forms of TGase:

- Transglutaminase K (Tgase K), a membrane-bound enzyme found in mammalian epidermis and important for the formation of the cornified cell envelope (gene TGM1).
- Tissue transglutaminase (TGase C), a monomeric ubiquitous enzyme located in the cytoplasm (gene TGM2).
- Transglutaminase 3, responsible for the later stages of cell envelope formation in the epidermis and the hair follicle (gene TGM3).
- Transglutaminase 4 (gene TGM4).

A conserved cysteine is known to be involved in the catalytic mechanism of TGases. The erythrocyte membrane band 4.2 protein, which probably plays an important role in regulating the shape of erythrocytes and their mechanical properties, is evolutionary related to TGases. However the active site cysteine is substituted by an alanine and the 4.2 protein does not show TGase activity.
Consensus pattern:[GT]-Q-[CA]-W-V-x-[SA]-[GA]-[IVT]-x(2)-T-x-[LMSC]-R-[CSA]-[LV]-G [The first C is the active site residue] Sequences known to belong to this class detected by the patternALL. Other sequence(s) detected in SWISS-PROTNONE.

[1] Ichinose A., Bottenus R.E., Davie E.W. J. Biol. Chem. 265:13411-13414(1990).
[2] Greenberg C.S., Birckbichler P.J., Rice R.H. FASEB J. 5:3071-3077(1991).

877. (TruB_N)
TruB family pseudouridylate synthase (N terminal domain)
Members of this family are involved in modifying bases in RNA molecules. They carry out the conversion of uracil bases to pseudouridine. This family includes TruB, a pseudouridylate synthase that specifically converts uracil 55 to pseudouridine in most tRNAs. This family also includes Cbf5p that modifies rRNA [2].
Number of members: 33

[1] Nurse K, Wrzesinski J, Bakin A, Lane BG, Ofengand J; Medline: 96079944 "Purification, cloning, and properties of the tRNA psi 55 synthase from Escherichia coli." RNA 1995;1:102-112.
[2] Lafontaine DLJ, Bousquet-Antonelli C, Henry Y, Caizergues-Ferrer M, Tollervey D; Medline: 98139521 "The box H + ACA snoRNAs carry Cbf5p, the putative rRNA pseudouridine synthase." Genes Dev 1998;12:527-537.

878.) (UDPGP)
UTP--glucose-1-phosphate uridylyltransferase
This family consists of UTP--glucose-1-phosphate uridylyltransferases, EC:2.7.7.9. Also known as UDP-glucose pyrophosphorylase (UDPGP) and Glucose-1-phosphate uridylyltransferase. UTP--glucose-1-phosphate uridylyltransferase catalyses the interconversion of MgUTP + glucose-1-phosphate and UDP-glucose + MgPPi [1]. UDP-glucose is an important intermediate in mammalian carbohydrate interconversion involved in various metabolic roles depending on tissue type [1]. In Dictyostelium (slime mold) mutants in this enzyme abort the development cycle [2]. Also within the family is UDP-N-acetylglucosamine Swiss:Q16222 or AGX1 [3] and two hypothetical proteins from Borrelia burgdorferi the lyme disease spirochaete Swiss:051893 and Swiss:051036.
Number of members: 18

[1] Duggleby RG, Chao YC, Huang JG, Peng HL, Chang HY; Medline: 96202932 "Sequence differences between human muscle and liver cDNAs for UDPglucose pyrophosphorylase and kinetic properties of the recombinant enzymes expressed in Escherichia coli." Eur J Biochem 1996;235:173-179.

[2] Ragheb JA, Dottin RP; Medline: 87231075 "Structure and sequence of a UDP glucose pyrophosphorylase gene of Dictyostelium discoideum." Nucleic Acids Res 1987;15:3891-3906.

[3] Mio T, Yabe T, Arisawa M, Yamada-Okabe H; Medline: 98269105 "The eukaryotic UDP-N-acetylglucosamine pyrophosphorylases. Gene cloning, protein expression, and catalytic mechanism. J Biol Chem 1998;273: 14392-14397.

879. (UPF004)
Uncharacterized protein family UPF0044 signature
Cross-reference(s) PS01301; UPF0044
The following uncharacterized proteins have been shown [1] to be highlysimilar:

- Bacillus subtilis hypothetical protein yqeI.
- Escherichia coli hypothetical protein yhbY and HI1333, the corresponding Haemophilus influenzae protein.
- Methanococcus jannaschii hypothetical protein MJ0652.

These are small proteins of 10 to 15 Kd. They can be picked up in the database by the following pattern. This pattern is located in the N-terminal part of these proteins.
Consensus pattern: L-[ST]-x(3)-K-x(3)-[KR]-[SGA]-x-[GA]-H-x-L-x-P-[LIV]-x(2)- [LIV]-[GA]-x(2)-G Sequences known to belong to this class detected by the patternALL. Other sequence(s) detected in SWISS-PROTNONE.

880. (zf-A20)
A20-like zinc finger
A20- (an inhibitor of cell death)-like zinc fingers. The zinc finger mediates self-association in A20. These fingers also mediate IL-1-induced NF-kappa B activation.
Number of members: 22

[1] Heyninck K, Beyaert R; Medline: 99126071 "The cytokine-inducible zinc finger protein A20 inhibits IL-1-induced NF- kappaB activation at the level of TRAF6. FEBS Lett 1999;442:147-150.
[2] De Valck D, Heyninck K, Van Criekinge W, Contreras R,Beyaert R, Fiers W; Medline: 96390831 "A20, an inhibitor of cell death, self-associates by its zinc finger domain." FEBS Lett 1996;384:61-64.
[3] Song HY, Rothe M, Goeddel DV; Medline: 96270609 "The tumor necrosis factor-inducible zinc finger protein A20 interacts with TRAF1/TRAF2 and inhibits NF-kappaB activation. Proc Natl Acad Sci U S A 1996;93:6721-6725.
[4] Opipari AW Jr, Boguski MS, Dixit VM; Medline: 90368626 "The A20 cDNA induced by tumor necrosis factor alpha encodes a novel type of zinc finger protein." J Biol Chem 1990;265:14705-14708.

881. (zf PARP)
Poly(ADP-ribose) polymerase zinc finger domain
Cross-reference(s) PS00347; PARP_ZN_FINGER_1 PS50064; PARP_ZN_FINGER_2
Poly(ADP-ribose) polymerase (EC 2.4.2.30) (PARP) [1,2] is a eukaryotic enzyme that catalyzes the covalent attachment of ADP-ribose units from NAD(+) to various nuclear acceptor proteins. This post-translational modification of nuclear proteins is dependent on DNA. It appears to be involved in the regulation of various important cellular processes such as differentiation, proliferation and tumor transformation as well as in the regulation of the molecular events involved in the recovery of the cell from DNA damage. Structurally, PARP, about 1000 amino-acids residues long, consists of three distinct domains: an N-terminal zinc-dependent DNA-binding domain, a central automodification domain and a C-terminal NAD-binding domain. The DNA-binding region contains a pair of zinc finger domains which have been shown to bind DNA in a zinc-dependent manner. The zinc finger domains of PARP seem to bind specifically to single-stranded DNA. DNA ligase III [3] contains, in its N-terminal section, a single copy of a zinc finger highly similar to those of PARP.
Consensus pattern: C-[KR]-x-C-x(3)-I-x-K-x(3)-[RG]-x(16,18)-W-[FYH]-H-x(2)-C [The three C's and the H are zinc ligands] Sequences known to belong to this class detected by the patternALL. Other sequence(s) detected in SWISS-PROTNONE. Sequences known to belong to this class detected by the profile ALL. Other sequence(s) detected in SWISS-PROTNONE.
Note: This documentation entry is linked to both signature patterns and a profile. As the profile is much more sensitive than the patterns, you should use it if you have access to the necessary software tools to do so.

[1] Althaus F.R., Richter C.R. Mol. Biol. Biochem. Biophys. 37:1-126(1987).
[2] de Murcia G., Menissier de Murcia J. Trends Biochem. Sci. 19:172-176(1994).
[3] Wei Y.-F., Robins P., Carter K., Caldecott K., Pappin D.J.C., Yu G.-L., Wang R.-P., Shell B.K., Nash R.A., Schar P., Barnes D.E., Haseltine W.A., Lindahl T. Mol. Cell. Biol. 15:3206-3216(1995).

A. Asparaginase 2
Asparaginase II (L-asparagine aminohydrolase II) is an extracellular protein that may be associated with the cell wall and whose expression is affected by the availability of nitrogen. Asparaginase II catalyzes the reaction of L-Asparagine + $H_2O$ = L-Aspartate + $NH_3$. As many leukemias have high requirements for aspartic acid, asparaginase II proteins are useful as reagents for screening compounds for activity as leukemia chemotherapy products. Asparaginase II protein can also be over- or under-expressed to alter amino acid content in plant tissues or to modify nitrogen fixation and/or nitrogen metabolism in plants.
Ref: Bon et al. (1997) Appl Biochem Biotechnol 63-65: 203-12

B. Chloroa b-bind
Chlorophyll a-b binding proteins are located in the thylakoid membranes of the chloroplast and bind chlorophyll a and chlorophyll b, thereby triggering a chemical reaction (photosynthesis). These proteins are useful in controlling the rate, efficiency and/or output of photosynthesis. Overexpression of chlorophyll a-b binding proteins is expected to increase the rate of photosynthesis.
Ref: Leutwiler et al. (1986) Nucleic Acids Res 14: 4051-64
Brandt et al. (1992) Plant Mol Biol 19: 699-703

C. DMRL synthase
DMRL Synthase (6,7-Dimethyl-8-Ribityllumazine Synthase) catalyzes the last step in riboflavin (Vitamin $B_2$) syn-

thesis, condensing 5-amino-6-(1'-D)-ribityl-amino-2,4(1H, 3H)-Pyrimidinedione with L-3,4-Dihydroxy-2-Butanone 4-Phosphate producing 6,7-Dimethyl-8-(1-D-Ribityl)Luminazine . The enzyme forms a homopentamer. Engineering of these proteins or those with homologous sequences/structures may allow control of the amounts of vitamin $B_2$ available in plants and/or accumulation of pigment, as well as altering reactions requiring hydrogen ion carriers/transmitters.
Ref: Garcia-Ramirez et al. (1995) J Biol Chem 270: 23801-7

D. E1 N
These proteins are ATP-dependent DNA helicases that are required for initiation of viral DNA replication. They form a complex with the viral E2 protein. The E1-E2 complex binds to the replication origin that contains binding sites for both proteins. The majority of sequences known for this group of proteins are from various papillomaviruses, a type of double stranded DNA virus. In plants, the prototype double stranded DNA virus is Cauliflower Mosaic virus (CaMV). Manipulation of these proteins, especially to produce variant proteins that form non-productive complexes, enables production of plants that are resistant to infection by double stranded DNA viruses.
Ref: Yang et al. (1993) PNAS USA 90: 5086-90
Ustav and Stenlund (1991) EMBO J 10: 449-57
Callaway et al. (1996) Mol Plant Microbe Interact 9: 810-8

E. EF1 G
Elongation Factor-1 is composed of four subunits: alpha, beta, delta and gamma. Gamma subunits are presumed to play a role in anchoring the complex to other cellular components. Studies of EF-1 genes in plants suggests that different forms of the EF-1 subunits may be expressed in particular organs or in response to stress. Manipulation of the activity of these proteins, either by altered expression level or by structural mutation, may result in the accumulation of a particular protein in a chosen organ or allow production of particular proteins during stress conditions.
Ref: Kinzy et al. (1994) NAR 22: 2703-7
Dunn et al. (1993) Plant Mol Biol 23: 221-5
Aguilar et al. (1991) Plant Mol Biol 17: 351-60

F. ENV polyprotein
This family comprises the envelope or coat proteins known from a number of different retroviruses. In mammalian species, retroviruses are responsible for diseases such as leukemia and HIV. In plants, retroviruses are known in both monocot (e.g. Zeon-1) and dicot (e.g. Arabidopsis and tobacco) species and have been shown to induce mutant alleles at new loci. Engineering of plant ENV proteins may allow mobilization or targeting of endogenous or introduced retroviruses, in essence generating a new method for mutant production, gene tagging and the like.
Ref: Mamoun et al (1990) J Virol 64: 4180-8
Grandbastien et al. (1989) Nature 337: 376-80
Wright and Voytas (1998) Genetics 149: 703-15

G. Glycosyl hvdr9
Proteins having this domain (previously known as the glycosyl hydrolase family 5 domain) catalyze the endohydrolysis of 1,4-β-D-glucosidic linkages in cellulose. Numerous plant proteins with this domain exist and are expressed in an organ specific manner. They are involved in the fruit ripening process, in cell elongation and plant reproduction. Modulation of the activity of these proteins, either by over- or under-expression or by mutation of the polypeptide, could be used to affect post-harvest physiology (e.g. rate of ripening) or for engineering reproductive sterility.
Ref: Giorda et al. (1990) Biochemistry 29: 7264-9
Tucker et al. (1988) Plant Physiol 88: 1257-62
Shani et al. (1997) 43: 837-42
Milligan and Gasser (1995) Plant Mol Biol 28: 691-711

H. Glycosyl hydr14
The β-amylases (family 14 of glycosyl hydrolases) catalyze the hydrolysis of 1,4-α-glucosidic linkages in polysaccharides and remove successive maltose units from the non-reducing ends of the chains. Mutants of β-amylase in Arabidopsis exhibited altered degradation of starch throughout the diurnal cycle. In addition, the mutant phenotypes indicated that these enzymes not only affect carbohydrate metabolism/catabolism, but also influence the amount of pigment stored within particular cells. Manipulation of the β-amylase genes enables control of plant pigmentation (for example, fibre pigment in cotton) as well as carbohydrate synthesis and degradation.
Ref: Zeeman et al. (1998) Plant J 15: 357-65
Hirano and Nakamura (1997) Plant Physiol 114: 5675-82

Kitamoto et al. (1988) J Bacteriol 170: 5848-54

I. Glycosyl hydr15

Glycosyl hydrolases from family 15 (such as 1,4-Alpha-D-Glucan glucohydrolase,) catalyze the hydrolysis of terminal 1,4-linked alpha-D-glucose residues successively from the non-reducing ends of the chains resulting in the release of β-D-Glucose. In plants these proteins have been tied to the mobilization of the xyloglucan stored in the cotyledonary cell walls. Proteins such as these could be varied to affect the rate of plant growth (for example during germination), storage and/or use of glucose and other sugars by plant tissues and alteration of the properties, such as elasticity, of plant cell walls.

Ref: Crombie et al. (1998) Plant J 15: 27-38
Hata et al. (1991) Agric Biol Chem 55: 941-9

J. Glycosyl hydr20

Members of the family 20 glycosyl hydrolases catalyze the hydrolysis of terminal non-reducing N-acetly-D-hexosamine residues in N-acetyl-β-D-hexosaminides. N-acetyl-β - glucosaminidase belongs to this family and exists in several different forms (consisting of various combinations of alpha and beta chains) depending on the organism. Family 20 glycosyl hydrolases have been implicated in lysosomal storage diseases (such as Sandhoff disease) and glycogen storage disease in humans. These types of proteins are also responsible for the hydrolysis of chitin. In plants, these proteins could be useful in controlling carbohydrate catabolism, thereby influencing the amount of sugars available for storage and/or use in other metabolic pathways. In addition, it is possible that such proteins could be used to engineer an endogenous insect protection mechanism, e.g. by secretion of a chitin-hydrolyzing composition by the plant.

Ref: Graham et al (1988) J Biol Chem 263: 16823-9
O'Dowd et al. (1988) Biochemistry 27: 5216-26

K. HMG box

The HMG box is a novel type of DNA-binding domain found in a diverse group of proteins. Numerous plant proteins contain this domain, such as the HMG1/2-like proteins. The expression of some of these HMG proteins appears to be regulated by circadian rhythms and in a light dependent manner, occurring at higher levels in roots, for example and lower levels in light-grown tissues such as cotyledons. Generally, HMG proteins are thought to influence transcription regulation. In plants, HMGs are believed to have a role in maintaining patterns of circadian-regulated expression for other genes, suggesting that these proteins could be exploited to control growth and development.

Ref: Laudet et al. (1993) Nucleic Acids Res 21: 2493-501
Zheng et al. (1993) Plant Mol Biol 23: 813-23
Grasser et al. (1993) Plant Mol Biol 23: 619-25

L. IL2

Interleukin-2 (IL-2)is produced in mammals by T cells in response to antigenic or mitogenic stimulation and is crucial for proper regulation and functioning of the immune response. IL-2 is capable of stimulating B cells, monocytes, lymphokine-activated killer cells, natural killer cells and glioma cells. Plant extracts have also been shown to stimulate the immune system (for example, mistletoe therapy for human cancer). It is known that IL-2 is involved in feedback inhibition pathways that impact the inflammatory response as well as the growth inhibition of tumor reactive T cells. Plant proteins containing IL-2-like sequences are useful as immunity-based therapeutics, acting in a manner similar to IL-2 in mammals.

Ref: Heike et al. (1997) Scand J Immunol 45: 221-6
Ariel et al. (1998) J Immunol 161: 2465-72
Schink (1997) Anticancer Drugs 8 Suppl 1: S47-51

M. Oxidored FMN

NADPH dehydrogenases catalyze the reaction NADPH + acceptor = NADP(+) + reduced acceptor. One member of this family is yeast "old yellow enzyme" (OYE) and is thought to be involved in oxylipin metabolism. A second yeast family member is a protein that binds estrogen binding protein (EBP) in addition to exhibiting oxidoreductase activity. An Arabidopsis homolog to OYE has been described and estrogen binding proteins in plants have been reported. Plant proteins from this class have the potential to be used to modify lipid metabolism/catabolism. These proteins may also have use as therapeutics for breast and prostate cancer, and other abnormal growth in steroid-sensitive tissues.

Ref: Baker et al. (1998) Proc Soc Exp Biol Med 217: 317-21
Schaller and Weiler (1997) J Biol Chem 272: 28066-72

Mandani et al. (1994) PNAS USA 91: 922-6

N. Oxidored q2

The NADH-plastoquinone oxidoreductases catalyze the reaction NADH + plastoquinone = NAD(+) + plastoquinol. In plants these reactions occur in the chloroplast and are believed to participate in a chloroplast respiratory system. Here, the NDH complex is postulated to act as a valve to remove excess reduction equivalents in the chloroplasts. Manipulation of these proteins may improve the rate or efficiency of photosynthesis.

Ref: Burrows et al. (1998) EMBO J 17: 868-76
Kofer et al (1998) Mol Gen Genet 258: 166-73
Maier et al. (1995) J Mol Biol 251: 614-28

O. PABP

Polyadenylate binding proteins bind the poly (A) tail of mRNA. Plants, as exemplified by Arabidopsis, contain numerous PABP genes that are expressed in an organ-specific manner. For example, PABP2 is functional in roots and shoots, while PABP5 is expressed predominantly in immature flowers. The PABP proteins are implicated in numerous aspects of posttranscriptional regulation including mRNA turnover and translational initiation. Control of activity of PABP proteins provides the ability to control the expression of various genes in particular organs during development.

Ref: Hilson et al (1993) Plant Physiol 103: 525-33
Belostotsky and Meagher (1993) PNAS USA 90: 6686-90

P. Parvo coat

Parvoviruses are linear single-stranded DNA viruses that are encapsulated by three capsid proteins. Plants are susceptible to infection by single stranded DNA viruses such as Maize streak virus (MSV) and various Gemini viruses. The coat proteins in these plant viruses are critical to the virus life cycle within the plant. For example, the coat protein of MSV is thought to be involved in intra- and inter-cellular movement within the plant. Engineering of proteins having similarity to parvoviral coat proteins, especially to produce proteins that interfere with maturation of the virus particle, enables the production of plants having better resistance to natural plant single-stranded DNA viruses.

Ref: Liu et al. (1997) J Gen Virol 78: 1265-70
Rohde et al. (1990) Virology 176: 648-51

Q. Pkinase C

Plant serine/threonine protein kinases possessing this domain are expressed in all tissues and are known to undergo serine-specific autophosphorylation and specifically phosphorylate two ribosomal proteins, P14 and P16. During development, these proteins predominate during high metabolic activity in growing buds, root tips, leaf margins and germinating seeds. They are thought to be involved in the control of plant growth and development. In addition, two genes encoding proteins from this family have been described that help plant cells adapt during cold or high salt stresses. Consequently, engineering Pkinase C proteins provides a way to control general growth/development of the plant as well as a means to provide endogenous protection against environmental stresses.

Ref: Zhang et al. (1994) J Biol Chem 269: 17586-92
Mizoguchi et al. (1995) FEBS Lett 358: 199-204

R. REV

The REV proteins act post-transcriptionally to relieve negative repression of GAG and ENV production in retroviruses such as Human Immounodeficiency Virus type I (HIV-1). Plants contain retrovirus-like viruses such as pararetroviruses and retrotransposons (i.e. transposons having long terminal repeats). Plant retrotransposons in particular have been used to create mutations at various loci, thereby permitting gene isolation, gene tagging and the like. Manipulation of plant REV proteins enables control of transposition frequencies of corresponding transposable elements and provides a new tool for genetic engineering of plants.

Ref: Sodroski et al. (1986) Nature 321: 412-7
Franchini et al. (1989) PNAS USA 86: 2433-7
Marquet et al. (1995) 77: 113-24
Grandbastien et al. (1989) Nature 337: 376-80
Wright and Voytas (1998) Genetics 149: 703-15

S. RuBisCo small

Ribulose 1,5-bisphosphate carboxylase/oxygenase (RuBisCo) catalyzes the initial step in the C3 photosynthetic

carbon reduction cycle, adding carbon dioxide to D-ribulose 1,5-bisphosphate to form two molecules of 3-phospho-D-glycerate. RuBisCo is comprised of two subunits, one large which is synthesized in the chloroplast, and one small which is synthesized in the cytoplasm and then transported in to the chloroplast. The expression of the small subunit of RuBisCo is light regulated. Manipulation of these proteins could increase the efficiency of photosynthesis or allow alterations in developmental timing.
Ref: Giuliano et al. (1988) PNAS USA 85: 7089-93
Dedonder et al. (1993) Plant Physiol 101: 801-8

T. Sialyltransf
Members of the CMP-N-acetylneuraminate-β-galactosamide-α-2,3-sialyltransferase family catalyze the following reaction:
CMP-N-acetylneuraminate + β-D-galactosyl-1,3-N-acetyl-α-D-galactosaminyl-R = CMP + α-N-acetylneraminyl-2,3-β-D-galactosyl-1,3-N -acetyl-alpha-D-galactosaminyl-R. These proteins are though to be responsible for the synthesis of the sequence neurac-α-2,3-gal-β-1,3-galnac- found on sugar chains)-linked to threonine or serine and also as a terminal sequence on certain gangliosides in mammalian cells. In plants, glycosyltransferases in the Golgi apparatus synthesize cell wall polysaccharides and elaborate the complex glycans of glycoproteins. Engineering of plant sialyltransferases allows targeting of proteins to particular cellular locations or enables the making of changes in cell wall structure.
Ref: Wee et al. (1998) Plant Cell 10: 1759-68
Lee et al. (1994) J Biol Chem 269: 10028-33
Kitagawa and Paulson (1994) J Biol Chem 269: 1394-401

U. Signal
Many plant proteins in this family contain sequences similar to those found in both components of the prokaryotic family of signal transducers known as the two-component systems. This suggests that activation may require a transfer of a phosphate group between the transmitter domain and the receiver domain. One family member in Arabidopsis appears to be involved in ethylene (a plant hormone) signal transduction. Other proteins in this family appear to be involved in the regulation of gene transcription under conditions of environmental stress. Signal proteins can be exploited to affect plant growth and development and/or control plant responses to stress conditions such as cold, nutrient availability, etc.
Ref: Chang et al. (1993) Science 262: 539-44
Nagaya et al. (1993) Gene 131: 119-124
Gottfert et al. (1990) PNAS USA 87: 2680-4

V. vMSA
vMSA proteins are major surface antigens presenting on the envelope of various retroviruses. Surface antigens of retroviruses are often involved in tropism of the virus. Plants contain retrovirus-like viruses such as pararetroviruses and retrotransposons (i.e. transposons having long terminal repeats). Plant retrotransposons in particular have been used to create mutants at various loci, thereby permitting gene isolation, gene tagging and the like. Manipulation of plant vMSA proteins enables control of tropism of plant retroviruses that might be used for genetic engineering tools, thus enabling targeting of the virus to particular species and/or tissues of plants.
Ref: Okamoto et al. (1988) J Gen Virol 69: 2575-83
Grandbastien et al. (1989) Nature 337: 376-80
Wright and Voytas (1998) Genetics 149: 703-15

W. zf-CCCH
This family of proteins is defined by having two CX(8)CX(5)CX(3)H-type zinc finger domains. These proteins cover a broad range of functions. For example, the COP1 protein acts as a repressor of photomorphogenesis in darkness; light stimuli abolish this suppressive action. In addition, COP1 protein can function as a negative transcriptional regulator capable of direct interaction with components of the G-protein signaling pathway. As a second example, a zf-CCCH protein identified in Arabidopsis appears to be involved in the resistance to DNA damage induced by UV light and chemical DNA-damaging agents. Overexpression of this class of proteins permits production of plants that are better suited to adverse environments. Manipulation of expression of zf-CCCH proteins functioning as transcriptional regulators, such as COP1, enables manipulation of some signal transduction pathways.
Ref: Pang et al. (1993) Nucleic Acids Res 21: 1647-53
Deng et al. (1992) Cell 71: 791-801

X. zf-RanBP

Proteins falling within this category contain many X-X-F-G and X-F-X-F-G repeats, and may contain RANBP1-like or PPIase domains. Plant proteins having domains similar to these include PAS1 and GMSTI. PAS1 has been shown to have dramatic developmental affects that appear to be correlated with both cell division and cell wall elongation. GMSTI has high identity to the yeast STI stress-inducible gene and has been shown to be heat inducible. Proteins such as these may be useful for controlling growth and form of development.
Ref: Vittorioso et al. (1998) Mol Cell Biol 18: 3034-43
Hernandez Torres et al. (1995) 27: 1221-6

Y. Peptidase M48.
Proteins belonging to this peptidase family are metalloproteases that bind zinc as a cofactor and are located in the membranes of the endoplasmic reticulum. They function in $NH_2$-terminal proteolytic processing, as shown for the yeast STE24 gene product. This gene is required for the correct processing of $\alpha$-factor, a yeast pheromone. Family M48 peptidases also appear to be required for some prenylation reactions, mediating COOH-terminal CAAX processing. Prenylation reactions are believed to be involved in the regulation of protein-protein and protein-membrane interactions. As an example, RAS GTPase activity is regulated in part by localization to the inner side of the plasma membrane upon prenylation. In plants, proteins from this family could be involved in pollen-stigma interactions such as those mediating self-pollenation vs. outcrossing, or could be members of several secondary metabolism pathways.
Ref: Fujimura-Kamada et al. (1997) J Cell Biol. 136: 271-85. Tam et al. (1998) J Cell Biol. 142: 635-49.

Z. DNA Pol Viral N
The DNA pol Viral N domain is located at the N-terminal region of DNA polymerase isolated from several retroid viruses such as the Cauliflower Mosaic Virus. The domain motif has also been found in numerous other species from humans to cyanobacteria. In these organisms, this motif seems to be associated with two types of sequences; retrotransposons and mitochondrial genes. In the mitochondrial sequences this domain is potentially involved in the self-splicing conducted by group II introns. Various manipulations of this gene in plants allows control of the numerous retrotransposons endogenous to plant genomes or allows engineering of mitochondrial function, especially to increase efficiency of energy utilization by cells.
REF: Chapdelaine and Bonen (1991) Cell 65: 465-72
Ferat and Miche (1993) Nature 364: 358-61
Wilson et al. (1994) 368: 32-8
Cambareri et al. (1994) 242: 658-65
Gaardner et al. (1981) NAR 9: 2871-2888
Cummings et al. (1990) Curr Genet 17: 375-402
Hattori et al. (1986) Nature 321: 625-8

Aa. Calpain inhib
This domain is found in calpastatin, an inhibitor protein specific for calpain. Calpain is a non-lysosomal calcium-dependent intracellular protease that appears to be involved in the dynamic changes of the cytoskeleton, especially actin-related structures, during early *Drosophila* embryogenesis [1]. Calpastatins co-exist in cells with calpains and the subcellular distribution of calpastatin is thought to be important to calpain regulation [2]. In plants calpains and calpastatins could be involved in embryogenesis and non-embryogenic organ reiteration. Mutations occurring in calpain inhibitor repeat domains would produce developmental abnormalities such as abnormal leaf, root or flower development.
Refs

1 Emori Y and Saigo K (1994) J Biol Chem 269: 25137-42.
2 Mellgren RL, Lane RD, Mericle MT (1989) Biochim Biophys Acta 999: 71-77.

Ab. chorismate bind
Chorismate binding domains are present in plant anthranilate synthase (AS) genes. AS genes catalyze the first step in the biosynthesis of tryptophan by converting chorismate and L-glutamine to anthranilate, pyruvate and L-glutamate. Some of these genes are involved in feedback inhibition by tryptophan [1] while some are feedback insensitive [2]. In Arabidopsis, two AS genes have overlapping, but different distributions. One of these AS genes is induced by wounding and bacterial pathogen infiltration [1]. Mutations in the chorismate binding domain would affect the production of tryptophan and could influence the plant's defense system. AS gene products can be used for *in vitro* synthesis of tryptophan and tryptophan derivatives.
Refs

1 Niyogi KK, Fink GR (1992) Plant Cell 4: 721-33.
2 Song HS, Brotherton JE, Gonzales RA, Wilholm JM (1998) Plant Physiol 117:533-43.

Ac. late protein L2

Papillomaviruses are encapsulated double stranded DNA viruses. Plants are susceptible to infection by double stranded DNA viruses such as Cauliflower Mosaic virus (CaMV). The coat proteins in these plant viruses are critical to the virus life cycle within the plant. For example, the coat protein of CaMV is thought to be involved in intra- and inter-cellular movement within the plant [1]. Engineering of proteins having similarity to papillomavirus coat proteins may enable the production of plants having better resistance to natural plant double stranded DNA viruses.

Refs

1 Thompson SR, Melcher U (1993) J Gen Virol 74: 1141-8.

Ad. Peptidase M41

Proteins belonging to this peptidase family are metalloproteases that bind zinc as a cofactor and are integral membrane proteins. They seem to be involved in the degradation of carboxy-terminal-tagged cytoplasmic proteins. In plants, these proteins are located in the thylakoid membranes of the chloroplasts, their expression is light regulated and they are thought to be involved in degradation of soluble stromal proteins and turn-over of thylkoid proteins [1]. Manipulation of expression and structure of these proteins would have effects on the efficiency of photosynthesis and the development of chloroplasts.

Refs

1 Lindahl M, Tabak s, Cseke L, Pichersky E, Andersson B, Adam Z (1996) J Biol Chem 271: 29329-34.

Ae. UPF0051

There is some evidence that, in plants, proteins in this family are involved in ATP synthesis in chloroplasts [1, 2]. Mutations in these proteins or altering their expression would affect the efficiency of photosynthesis and energy production.

Refs

1 Kostrzewa M, Zetsche K (1992) J Mol Biol 227: 961-70.
2 Kostrzewa M, Zetsche K (1993) Plant Mol Biol 23: 67-76

Af. E7

Papillomaviruses are encapsulated double stranded DNA viruses. The Papillomavirus early protein 7 (E7) is known as a potent immortalizing and transforming agent. Transformation by E7 is thought to be mediated by the physical association of E7 with cellular proteins regulating entry into the cell cycle [1]. The result is entry into the cell cycle and suppression of terminal differentiation in mammalian cells. Thus, engineering of proteins having similarity to papillomavirus E7 protein enables the production of plants having altered cellular proliferation characteristics and possibly altered morphology. For example, overexpression of E7-like proteins would be expected to result in proliferation of cells of the tissue in which the E7 protein is expressed, perhaps with suppression of differentiation events. Thus, for example, overexpression of E7-like proteins in meristem cells can result in taller plants and suppression of leafing and/or flowering.

Refs

1 Zwerschke W, Jansen-Durr P Adv Cancer Res 2000;78:1-29

Ag. Peptidase U7

This protein is known to be an integral membrane protein in the cyanobacterium Synechocystis where it functions to digest cleaved signal peptides [1]. This activity is necessary to maintain proper secretion of mature proteins across the membrane. In higher plants this protein may be present in the plastid or chloroplast membranes where it would function by enabling protein movement into and out of the chloroplasts. Mutations in this protein would be expected to affect the development of plastids, including chloroplasts, or alter the energy transfer system within the chloroplasts, thereby affecting growth and development.

Refs

1 Kaneko T, Sato S, Kotani H, Tanaka A, Asamizu E, Nakamura Y, Miyajima N, Hirosawa M, Sugiura M, Sasamoto S, Kimura T, Hosouchi T, Matsuno A, Muraki A, Nakazaki N, Naruo K, Okumura S, Shimpo S,

Takeuchi C, Wada T, Watanabe A, Yamada M, Yasuda M, Tabata S (1996) DNA Res 3:109-36.

AA. Activities of Polypeptides Comprising Signal Peptides

Polypeptides comprising signal peptides are a family of proteins that are typically targeted to (1) a particular organelle or intracellular compartment, (2) interact with a particular molecule or (3) for secretion outside of a host cell. Example of polypeptides comprising signal peptides include, without limitation, secreted proteins, soluble proteins, receptors, proteins retained in the ER, etc.

These proteins comprising signal peptides are useful to modulate ligand-receptor interactions, cell-to-cell communication, signal transduction, intracellular communication, and activities and/or chemical cascades that take part in an organism outside or within of any particular cell.

One class of such proteins are soluble proteins which are transported out of the cell. These proteins can act as ligands that bind to receptor to trigger signal transduction or to permit communication between cells.

Another class is receptor proteins which also comprise a retention domain that lodges the receptor protein in the membrane when the cell transports the receptor to the surface of the cell. Like the soluble ligands, receptors can also modulate signal transduction and communication between cells.

In addition the signal peptide itself can serve as a ligand for some receptors. An example is the interaction of the ER targeting signal peptide with the signal recognition particle (SRP). Here, the SRP binds to the signal peptide, halting translation, and the resulting SRP complex then binds to docking proteins located on the surface of the ER, prompting transfer of the protein into the ER.

A description of signal peptide residue composition is described below in Subsection IV.C.1.

## III. Methods of Modulating Polypeptide Production

It is contemplated that polynucleotides of the invention can be incorporated into a host cell or in-vitro system to modulate polypeptide production. For instance, the SDFs prepared as described herein can be used to prepare expression cassettes useful in a number of techniques for suppressing or enhancing expression.

An example are polynucleotides comprising sequences to be transcribed, such as coding sequences, of the present invention can be inserted into nucleic acid constructs to modulate polypeptide production. Typically, such sequences to be transcribed are heterologous to at least one element of the nucleic acid construct to generate a chimeric gene or construct.

Another example of useful polynucleotides are nucleic acid molecules comprising regulatory sequences of the present invention. Chimeric genes or constructs can be generated when the regulatory sequences of the invention linked to heterologous sequences in a vector construct. Within the scope of invention are such chimeric gene and/or constructs.

Also within the scope of the invention are nucleic acid molecules, whereof at least a part or fragment of these DNA molecules are presented in REF AND SEQ TABLES 1 AND 2 of the present application, and wherein the coding sequence is under the control of its own promoter and/or its own regulatory elements. Such molecules are useful for transforming the genome of a host cell or an organism regenerated from said host cell for modulating polypeptide production.

Additionally, a vector capable of producing the oligonucleotide can be inserted into the host cell to deliver the oligonucleotide.

More detailed description of components to be included in vector constructs are described both above and below.

Whether the chimeric vectors or native nucleic acids are utilized, such polynucleotides can be incorporated into a host cell to modulate polypeptide production. Native genes and/or nucleic acid molecules can be effective when exogenous to the host cell.

Methods of modulating polypeptide expression includes, without limitation:

Suppression methods, such as

- Antisense
- Ribozymes
- Co-suppression
- Insertion of Sequences into the Gene to be Modulated
- Regulatory Sequence Modulation.

as well as Methods for Enhancing Production, such as

- Insertion of Exogenous Sequences; and

Regulatory Sequence Modulation.

III.A. Suppression

Expression cassettes of the invention can be used to suppress expression of endogenous genes which comprise the SDF sequence. Inhibiting expression can be useful, for instance, to tailor the ripening characteristics of a fruit (Oeller et al., Science 254:437 (1991)) or to influence seed size_(WO98/07842) or to provoke cell ablation (Mariani et al., Nature 357: 384-387 (1992).

As described above, a number of methods can be used to inhibit gene expression in plants, such as antisense, ribozyme, introduction of exogenous genes into a host cell, insertion of a polynucleotide sequence into the coding sequence and/or the promoter of the endogenous gene of interest, and the like.

IILA.1. Antisense

An expression cassette as described above can be transformed into host cell or plant to produce an antisense strand of RNA. For plant cells, antisense RNA inhibits gene expression by preventing the accumulation of mRNA which encodes the enzyme of interest, *see,* e.g., Sheehy et al., Proc. Nat. Acad. Sci. USA, 85:8805 (1988), and Hiatt et al., U.S. Patent No. 4,801,340.

III.A.2. Ribozymes

Similarly, ribozyme constructs can be transformed into a plant to cleave mRNA and down-regulate translation.

III.A.3. Co-Suppression

Another method of suppression is by introducing an exogenous copy of the gene to be suppressed. Introduction of expression cassettes in which a nucleic acid is configured in the sense orientation with respect to the promoter has been shown to prevent the accumulation of mRNA. A detailed description of this method is described above.

III.A.4. Insertion of Sequences into the Gene to be Modulated

Yet another means of suppressing gene expression is to insert a polynucleotide into the gene of interest to disrupt transcription or translation of the gene.

Homologous recombination could be used to target a polynucleotide insert to a gene using the Cre-Lox system (A.C. Vergunst et al., Nucleic Acids Res. 26:2729 (1998), A.C. Vergunst et al., Plant Mol. Biol. 38:393 (1998), H. Albert et al., Plant J. 7:649 (1995)).

In addition, random insertion of polynucleotides into a host cell genome can also be used to disrupt the gene of interest. Azpiroz-Leehan et al., Trends in Genetics 13:152 (1997). In this method, screening for clones from a library containing random insertions is preferred for identifying those that have polynucleotides inserted into the gene of interest. Such screening can be performed using probes and/or primers described above based on sequences from REF AND SEQ TABLES 1 AND 2, fragments thereof, and substantially similar sequence thereto. The screening can also be performed by selecting clones or any transgenic plants having a desired phenotype.

III.A.5. Regulatory SequenceModulation

The SDFs described in REF and SEQ TABLES 1 and 2, and fragments thereof are examples of nucleotides of the invention that contain regulatory sequences that can be used to suppress or inactivate transcription and/or translation from a gene of interest as discussed in I.C.5.

III.A.6. Genes Comprising Dominant-Negative Mutations

When suppression of production of the endogenous, native protein is desired it is often helpful to express a gene comprising a dominant negative mutation. Production of protein variants produced from genes comprising dominant negative mutations is a useful tool for research Genes comprising dominant negative mutations can produce a variant polypeptide which is capable of competing with the native polypeptide, but which does not produce the native result. Consequently, over expression of genes comprising these mutations can titrate out an undesired activity of the native protein. For example, The product from a gene comprising a dominant negative mutation of a receptor can be used to constitutively activate or suppress a signal transduction cascade, allowing examination of the phenotype and thus the

trait(s) controlled by that receptor and pathway. Alternatively, the protein arising from the gene comprising a dominant-negative mutation can be an inactive enzyme still capable of binding to the same substrate as the native protein and therefore competes with such native protein.

Products from genes comprising dominant-negative mutations can also act upon the native protein itself to prevent activity. For example, the native protein may be active only as a homo-multimer or as one subunit of a hetero-multimer. Incorporation of an inactive subunit into the multimer with native subunit(s) can inhibit activity.

Thus, gene function can be modulated in host cells of interest by insertion into these cells vector constructs comprising a gene comprising a dominant-negative mutation.

III.B. Enhanced Expression

Enhanced expression of a gene of interest in a host cell can be accomplished by either (1) insertion of an exogenous gene; or (2) promoter modulation.

IILB.1. Insertion of an Exogenous Gene

Insertion of an expression construct encoding an exogenous gene can boost the number of gene copies expressed in a host cell.

Such expression constructs can comprise genes that either encode the native protein that is of interest or that encode a variant that exhibits enhanced activity as compared to the native protein. Such genes encoding proteins of interest can be constructed from the sequences from REF AND SEQ TABLES 1 AND 2, fragments thereof, and substantially similar sequence thereto.

Such an exogenous gene can include either a constitutive promoter permitting expression in any cell in a host organism or a promoter that directs transcription only in particular cells or times during a host cell life cycle or in response to environmental stimuli.

III.B.2. Regulatory Sequence Modulation

The SDFs of REF and SEQ TABLES 1 AND 2, and fragments thereof, contain regulatory sequences that can be used to enhance expression of a gene of interest. For example, some of these sequences contain useful enhancer elements. In some cases, duplication of enhancer elements or insertion of exogenous enhancer elements will increase expression of a desired gene from a particular promoter. As other examples, all 11 promoters require binding of a regulatory protein to be activated, while some promoters may need a protein that signals a promoter binding protein to expose a polymerase binding site. In either case, over-production of such proteins can be used to enhance expression of a gene of interest by increasing the activation time of the promoter.

Such regulatory proteins are encoded by some of the sequences in REF AND SEQ TABLES 1 AND 2, fragments thereof, and substantially similar sequences thereto.

Coding sequences for these proteins can be constructed as described above.

**IV. Gene Constructs and Vector Construction**

To use isolated SDFs of the present invention or a combination of them or parts and/or mutants and/or fusions of said SDFs in the above techniques, recombinant DNA vectors which comprise said SDFs and are suitable for transformation of cells, such as plant cells, are usually prepared. The SDF construct can be made using standard recombinant DNA techniques (Sambrook et al. 1989) and can be introduced to the species of interest by *Agrobacterium*-mediated transformation or by other means of transformation (e.g., particle gun bombardment) as referenced below.

The vector backbone can be any of those typical in the art such as plasmids, viruses, artificial chromosomes, BACs, YACs and PACs and vectors of the sort described by

(a) **BAC:** Shizuya et al., Proc. Natl. Acad. Sci. USA 89: 8794-8797 (1992); Hamilton et al., Proc. Natl. Acad. Sci. USA 93: 9975-9979 (1996);
(b) **YAC:** Burke et al., Science 236:806-812 (1987);.
(c) **PAC:** Sternberg N. et al., Proc Natl Acad Sci U S A. Jan;87(1):103-7 (1990);
(d) **Bacteria-Yeast Shuttle Vectors:** Bradshaw et al., Nucl Acids Res 23: 4850-4856 (1995);
(e) **Lambda Phage Vectors:** Replacement Vector, e.g., Frischauf et al., J. Mol Biol 170: 827-842 (1983); or Insertion vector, e.g., Huynh et al., In: Glover NM (ed) DNA Cloning: A practical Approach, Vol.1 Oxford: IRL Press (1985);
(f) **T-DNA gene fusion vectors :**Walden et al., Mol Cell Biol 1: 175-194 (1990); and
(g) **Plasmid vectors:** Sambrook et al., infra.

Typically, a vector will comprise the exogenous gene, which in its turn comprises an SDF of the present invention to be introduced into the genome of a host cell, and which gene may be an antisense construct, a ribozyme construct chimeraplast, or a coding sequence with any desired transcriptional and/or translational regulatory sequences, such as promoters, UTRs, and 3' end termination sequences. Vectors of the invention can also include origins of replication, scaffold attachment regions (SARs), markers, homologous sequences, introns, etc.

A DNA sequence coding for the desired polypeptide, for example a cDNA sequence encoding a full length protein, will preferably be combined with transcriptional and translational initiation regulatory sequences which will direct the transcription of the sequence from the gene in the intended tissues of the transformed plant.

For example, for over-expression, a plant promoter fragment may be employed that will direct transcription of the gene in all tissues of a regenerated plant. Alternatively, the plant promoter may direct transcription of an SDF of the invention in a specific tissue (tissue-specific promoters) or may be otherwise under more precise environmental control (inducible promoters).

If proper polypeptide productionis desired, a polyadenylation region at the 3'-end of the coding region is typically included. The polyadenylation region can be derived from the natural gene, from a variety of other plant genes, or from T-DNA.

The vector comprising the sequences from genes or SDF or the invention may comprise a marker gene that confers a selectable phenotype on plant cells. The vector can include promoter and coding sequence, for instance. For example, the marker may encode biocide resistance, particularly antibiotic resistance, such as resistance to kanamycin, G418, bleomycin, hygromycin, or herbicide resistance, such as resistance to chlorosulfuron or phosphinotricin.

IV.A. Coding Sequences

Generally, the sequence in the transformation vector and to be introduced into the genome of the host cell does not need to be absolutely identical to an SDF of the present invention. Also, it is not necessary for it to be full length, relative to either the primary transcription product or fully processed mRNA. Furthermore, the introduced sequence need not have the same intron or exon pattern as a native gene. Also, heterologous non-coding segments can be incorporated into the coding sequence without changing the desired amino acid sequence of the polypeptide to be produced.

IV.B. Promoters

As explained above, introducing an exogenous SDF from the same species or an orthologous SDF from another species can modulate the expression of a native gene corresponding to that SDF of interest. Such an SDF construct can be under the control of either a constitutive promoter or a highly regulated inducible promoter (e.g., a copper inducible promoter). The promoter of interest can initially be either endogenous or heterologous to the species in question. When re-introduced into the genome of said species, such promoter becomes exogenous to said species. Over-expression of an SDF transgene can lead to co-suppression of the homologous endogeneous sequence thereby creating some alterations in the phenotypes of the transformed species as demonstrated by similar analysis of the chalcone synthase gene (Napoli et al., Plant Cell 2 :279 (1990) and van der Krol et al., Plant Cell 2:291 (1990)). If an SDF is found to encode a protein with desirable characteristics, its over-production can be controlled so that its accumulation can be manipulated in an organ- or tissue-specific manner utilizing a promoter having such specificity.

Likewise, if the promoter of an SDF (or an SDF that includes a promoter) is found to be tissue-specific or developmentally regulated, such a promoter can be utilized to drive or facilitate the transcription of a specific gene of interest (e.g., seed storage protein or root-specific protein). Thus, the level of accumulation of a particular protein can be manipulated or its spatial localization in an organ- or tissue- specific manner can be altered.

IV. C Signal Peptides

SDFs of the present invention containing signal peptides are indicated in the REF and SEQ TABLES. In some cases it may be desirable for the protein encoded by an introduced exogenous or orthologous SDF to be targeted (1) to a particular organelle intracellular compartment, (2) to interact with a particular molecule such as a membrane molecule or (3) for secretion outside of the cell harboring the introduced SDF. This will be accomplished using a signal peptide.

Signal peptides direct protein targeting, are involved in ligand-receptor interactions and act in cell to cell communication. Many proteins, especially soluble proteins, contain a signal peptide that targets the protein to one of several different intracellular compartments. In plants, these compartments include, but are not limited to, the endoplasmic reticulum (ER), mitochondria, plastids (such as chloroplasts), the vacuole, the Golgi apparatus, protein storage vessicles (PSV) and, in general, membranes. Some signal peptide sequences are conserved, such as the Asn-Pro-Ile-Arg amino acid motif found in the N-terminal propeptide signal that targets proteins to the vacuole (Marty (1999) The Plant Cell 11: 587-599). Other signal peptides do not have a consensus sequence *per se,* but are largely composed of hydrophobic

amino acids, such as those signal peptides targeting proteins to the ER (Vitale and Denecke (1999) The Plant Cell 11: 615-628). Still others do not appear to contain either a consensus sequence or an identified common secondary sequence, for instance the chloroplast stromal targeting signal peptides (Keegstra and Cline (1999) The Plant Cell 11: 557-570). Furthermore, some targeting peptides are bipartite, directing proteins first to an organelle and then to a membrane within the organelle (e.g. within the thylakoid lumen of the chloroplast; see Keegstra and Cline (1999) The Plant Cell 11: 557-570). In addition to the diversity in sequence and secondary structure, placement of the signal peptide is also varied. Proteins destined for the vacuole, for example, have targeting signal peptides found at the N-terminus, at the C-terminus and at a surface location in mature, folded proteins. Signal peptides also serve as ligands for some receptors.

These characteristics of signal proteins can be used to more tightly control the phenotypic expression of introduced SDFs. In particular, associating the appropriate signal sequence with a specific SDF can allow sequestering of the protein in specific organelles (plastids, as an example), secretion outside of the cell, targeting interaction with particular receptors, etc. Hence, the inclusion of signal proteins in constructs involving the SDFs of the invention increases the range of manipulation of SDF phenotypic expression. The nucleotide sequence of the signal peptide can be isolated from characterized genes using common molecular biological techniques or can be synthesized in vitro.

In addition, the native signal peptide sequences, both amino acid and nucleotide, described in the REF and SEQ tables can be used to modulate polypeptide transport. Further variants of the native signal peptides described in the REF and SEQ tables are contemplated. Insertions, deletions, or substitutions can be made. Such variants will retain at least one of the functions of the native signal peptide as well as exhibiting some degree of sequence identity to the native sequence.

Also, fragments of the signal peptides of the invention are useful and can be fused with other sinal peptides of interest to modulate transport of a polypeptide.

### V. Transformation Techniques

A wide range of techniques for inserting exogenous polynucleotides are known for a number of host cells, including, without limitation, bacterial, yeast, mammalian, insect and plant cells.

Techniques for transforming a wide variety of higher plant species are well known and described in the technical and scientific literature. *See, e.g.* Weising et al., Ann. Rev. Genet. 22:421 (1988); and Christou, Euphytica, v. 85, n.1-3: 13-27, (1995).

DNA constructs of the invention may be introduced into the genome of the desired plant host by a variety of conventional techniques. For example, the DNA construct may be introduced directly into the genomic DNA of the plant cell using techniques such as electroporation and microinjection of plant cell protoplasts, or the DNA constructs can be introduced directly to plant tissue using ballistic methods, such as DNA particle bombardment. Alternatively, the DNA constructs may be combined with suitable T-DNA flanking regions and introduced into a conventional *Agrobacterium tumefaciens* host vector. The virulence functions of the *Agrobacterium tumefaciens* host will direct the insertion of the construct and adjacent marker into the plant cell DNA when the cell is infected by the bacteria (McCormac et al., Mol. Biotechnol. 8:199 (1997); Hamilton, Gene 200:107 (1997)); Salomon et al. EMBO J. 3:141 (1984); Herrera-Estrella et al. EMBO J. 2:987 (1983).

Microinjection techniques are known in the art and well described in the scientific and patent literature. The introduction of DNA constructs using polyethylene glycol precipitation is described in Paszkowski et al. EMBO J. 3:2717 (1984). Electroporation techniques are described in Fromm et al. Proc. Natl Acad. Sci. USA 82:5824 (1985). Ballistic transformation techniques are described in Klein et al. Nature 327:773 (1987). *Agrobacterium tumefaciens*-mediated transformation techniques, including disarming and use of binary or cointegrate vectors, are well described in the scientific literature. See, for example Hamilton, CM., Gene 200:107 (1997); Müller et al. Mol. Gen. Genet. 207: 171 (1987); Komari et al. Plant J. 10:165 (1996); Venkateswarlu et al. Biotechnology 9:1103 (1991) and Gleave, AP., Plant Mol. Biol. 20: 1203 (1992); Graves and Goldman, Plant Mol. Biol. 7:34 (1986) and Gould et al., Plant Physiology 95:426 (1991).

Transformed plant cells which are derived by any of the above transformation techniques can be cultured to regenerate a whole plant that possesses the transformed genotype and thus the desired phenotype such as seedlessness. Such regeneration techniques rely on manipulation of certain phytohormones in a tissue culture growth medium, typically relying on a biocide and/or herbicide marker which has been introduced together with the desired nucleotide sequences. Plant regeneration from cultured protoplasts is described in Evans et al., Protoplasts Isolation and Culture in "Handbook of Plant Cell Culture," pp. 124-176, MacMillan Publishing Company, New York, 1983; and Binding, Regeneration of Plants, Plant Protoplasts, pp. 21-73, CRC Press, Boca Raton, 1988. Regeneration can also be obtained from plant callus, explants, organs, or parts thereof. Such regeneration techniques are described generally in Klee et al. Ann. Rev. of Plant Phys. 38:467 (1987). Regeneration of monocots (rice) is described by Hosoyama et al. (Biosci. Biotechnol. Biochem. 58:1500 (1994)) and by Ghosh et al. (J. Biotechnol. 32:1 (1994)). The nucleic acids of the invention can be used to confer desired traits on essentially any plant.

Thus, the invention has use over a broad range of plants, including species from the genera *Anacardium,*

*Arachis, Asparagus, Atropa, Avena, Brassica, Citrus, Citrullus, Capsicum, Carthamus, Cocos, Coffea, Cucumis, Cucurbita, Daucus, Elaeis, Fragaria, Glycine, Gossypium, Helianthus, Heterocallis, Hordeum, Hyoscyamus, Lactuca, Linum, Lolium,Lupinus, Lycopersicon, Malus, Manihot, Majorana, Medicago, Nicotiana, Olea, Oryza, Panieum, Pannesetum, Persea, Phaseolus, Pistachia, Pisum, Pyrus, Prunus, Raphanus, Ricinus, Secale, Senecio, Sinapis, Solanum, Sorghum, Theobromus, Trigonella, Triticum, Vicia, Vitis, Vigna, and, Zea.*

One of skill will recognize that after the expression cassette is stably incorporated in transgenic plants and confirmed to be operable, it can be introduced into other plants by sexual crossing. Any of a number of standard breeding techniques can be used, depending upon the species to be crossed.

The particular sequences of SDFs identified are provided in the attached REF AND SEQ TABLES 1 AND 2. One of ordinary skill in the art, having this data, can obtain cloned DNA fragments, synthetic DNA fragments or polypeptides constituting desired sequences by recombinant methodology known in the art or described herein.

## EXAMPLES

The invention is illustrated by way of the following examples. The invention is not limited by these examples as the scope of the invention is defined solely by the claims following.

## EXAMPLE 1: cDNA PREPARATION

A number of the nucleotide sequences disclosed in REF AND SEQ TABLES 1 AND 2 herein as representative of the SDFs of the invention can be obtained by sequencing genomic DNA (gDNA) and/or cDNA from corn plants grown from HYBRID SEED # 35A19, purchased from Pioneer Hi-Bred International, Inc., Supply Management, P.O. Box 256, Johnston, Iowa 50131-0256.

A number of the nucleotide sequences disclosed in REF AND SEQ TABLES 1 AND 2 herein as representative of the SDFs of the invention can also be obtained by sequencing genomic DNA from *Arabidopsis thaliana,* Wassilewskija ecotype or by sequencing cDNA obtained from mRNA from such plants as described below. This is a true breeding strain. Seeds of the plant are available from the Arabidopsis Biological Resource Center at the Ohio State University, under the accession number CS2360. Seeds of this plant were deposited under the terms and conditions of the Budapest Treaty at the American Type Culture Collection, Manassas, VA on August 31, 1999, and were assigned ATCC No. PTA-595.

Other methods for cloning full-length cDNA are described, for example, by Seki et al., Plant Journal 15:707-720 (1998) "High-efficiency cloning of Arabidopsis full-length cDNA by biotinylated Cap trapper"; Maruyama et al., Gene 138:171 (1994) "Oligo-capping a simple method to replace the cap structure of eukaryotic mRNAs with oligoribonucleotides"; and WO 96/34981.

Tissues were, or each organ was, individually pulverized and frozen in liquid nitrogen. Next, the samples were homogenized in the presence of detergents and then centrifuged. The debris and nuclei were removed from the sample and more detergents were added to the sample. The sample was centrifuged and the debris was removed. Then the sample was applied to a 2M sucrose cushion to isolate polysomes. The RNA was isolated by treatment with detergents and proteinase K followed by ethanol precipitation and centrifugation. The polysomal RNA from the different tissues was pooled according to the following mass ratios: 15/15/1 for male inflorescences, female inflorescences and root, respectively. The pooled material was then used for cDNA synthesis by the methods described below.

Starting material for cDNA synthesis for the exemplary corn cDNA clones with sequences presented in REF AND SEQ TABLES 1 AND 2 was poly(A)-containing polysomal mRNAs from inflorescences and root tissues of corn plants grown from HYBRID SEED # 35A19. Male inflorescences and female (pre-and post-fertilization) inflorescences were isolated at various stages of development. Selection for poly(A) containing polysomal RNA was done using oligo d(T) cellulose columns, as described by Cox and Goldberg, "Plant Molecular Biology: A Practical Approach", pp. 1-35, Shaw ed., c. 1988 by IRL, Oxford. The quality and the integrity of the polyA+ RNAs were evaluated.

Starting material for cDNA synthesis for the exemplary *Arabidopsis* cDNA clones with sequences presented in REF AND SEQ TABLES 1 AND 2 was polysomal RNA isolated from the top-most inflorescence tissues of *Arabidopsis thaliana* Wassilewskija (Ws.) and from roots of *Arabidopsis thaliana* Landsberg erecta (L. er.), also obtained from the Arabidopsis Biological Resource Center. Nine parts inflorescence to every part root was used, as measured by wet mass. Tissue was pulverized and exposed to liquid nitrogen. Next, the sample was homogenized in the presence of detergents and then centrifuged. The debris and nuclei were removed from the sample and more detergents were added to the sample. The sample was centrifuged and the debris was removed and the sample was applied to a 2M sucrose cushion to isolate polysomal RNA. Cox et al., "Plant Molecular Biology: A Practical Approach", pp. 1-35, Shaw ed., c. 1988 by IRL, Oxford.

The polysomal RNA was used for cDNA synthesis by the methods described below. Polysomal mRNA was then isolated as described above for corn cDNA. The quality of the RNA was assessed electrophoretically.

Following preparation of the mRNAs from various tissues as described above, selection of mRNA with intact 5' ends and specific attachment of an oligonucleotide tag to the 5' end of such mRNA was performed using either a chemical or enzymatic approach. Both techniques take advantage of the presence of the "cap" structure, which characterizes the 5' end of most intact mRNAs and which comprises a guanosine generally methylated once, at the 7 position.

The chemical modification approach involves the optional elimination of the 2', 3'-cis diol of the 3' terminal ribose, the oxidation of the 2', 3'-cis diol of the ribose linked to the cap of the 5' ends of the mRNAs into a dialdehyde, and the coupling of the such obtained dialdehyde to a derivatized oligonucleotide tag. Further detail regarding the chemical approaches for obtaining mRNAs having intact 5' ends are disclosed in International Application No. WO96/34981 published November 7, 1996.

The enzymatic approach for ligating the oligonucleotide tag to the intact 5' ends of mRNAs involves the removal of the phosphate groups present on the 5' ends of uncapped incomplete mRNAs, the subsequent decapping of mRNAs having intact 5' ends and the ligation of the phosphate present at the 5' end of the decapped mRNA to an oligonucleotide tag. Further detail regarding the enzymatic approaches for obtaining mRNAs having intact 5' ends are disclosed in Dumas Milne Edwards J.B. (Doctoral Thesis of Paris VI University, Le clonage des ADNc complets: difficultés et perspectives nouvelles. Apports pour l'étude de la regulation de l'expression de la tryptophane hydroxylase de rat, 20 Dec. 1993), EP0 625572 and Kato et al., Gene 150:243-250 (1994).

In both the chemical and the enzymatic approach, the oligonucleotide tag has a restriction enzyme site (e.g. an EcoRI site) therein to facilitate later cloning procedures. Following attachment of the oligonucleotide tag to the mRNA, the integrity of the mRNA is examined by performing a Northern blot using a probe complementary to the oligonucleotide tag.

For the mRNAs joined to oligonucleotide tags using either the chemical or the enzymatic method, first strand cDNA synthesis is performed using an oligo-dT primer with reverse transcriptase. This oligo-dT primer can contain an internal tag of at least 4 nucleotides, which can be different from one mRNA preparation to another. Methylated dCTP is used for cDNA first strand synthesis to protect the internal EcoRI sites from digestion during subsequent steps. The first strand cDNA is precipitated using isopropanol after removal of RNA by alkaline hydrolysis to eliminate residual primers.

Second strand cDNA synthesis is conducted using a DNA polymerase, such as Klenow fragment and a primer corresponding to the 5' end of the ligated oligonucleotide. The primer is typically 20-25 bases in length. Methylated dCTP is used for second strand synthesis in order to protect internal EcoRI sites in the cDNA from digestion during the cloning process.

Following second strand synthesis, the full-length cDNAs are cloned into a phagemid vector, such as pBlueScript™ (Stratagene). The ends of the full-length cDNAs are blunted with T4 DNA polymerase (Biolabs) and the cDNA is digested with EcoRI. Since methylated dCTP is used during cDNA synthesis, the EcoRI site present in the tag is the only hemimethylated site; hence the only site susceptible to EcoRI digestion. In some instances, to facilitate subcloning, an Hind III adapter is added to the 3' end of full-length cDNAs.

The full-length cDNAs are then size fractionated using either exclusion chromatography (AcA, Biosepra) or electrophoretic separation which yields 3 to 6 different fractions. The full-length cDNAs are then directionally cloned either into pBlueScript™ using either the EcoRI and SmaI restriction sites or, when the Hind III adapter is present in the full-length cDNAs, the EcoRI and Hind III restriction sites. The ligation mixture is transformed, preferably by electroporation, into bacteria, which are then propagated under appropriate antibiotic selection.

Clones containing the oligonucleotide tag attached to full-length cDNAs are selected as follows.

The plasmid cDNA libraries made as described above are purified (e.g. by a column available from Qiagen). A positive selection of the tagged clones is performed as follows. Briefly, in this selection procedure, the plasmid DNA is converted to single stranded DNA using phage F1 gene II endonuclease in combination with an exonuclease (Chang et al., Gene 127:95 (1993)) such as exonuclease III or T7 gene 6 exonuclease. The resulting single stranded DNA is then purified using paramagnetic beads as described by Fry et al., Biotechniques 13: 124 (1992). Here the single stranded DNA is hybridized with a biotinylated oligonucleotide having a sequence corresponding to the 3' end of the oligonucleotide tag. Preferably, the primer has a length of 20-25 bases. Clones including a sequence complementary to the biotinylated oligonucleotide are selected by incubation with streptavidin coated magnetic beads followed by magnetic capture. After capture of the positive clones, the plasmid DNA is released from the magnetic beads and converted into double stranded DNA using a DNA polymerase such as ThermoSequenase™ (obtained from Amersham Pharmacia Biotech). Alternatively, protocols such as the Gene Trapper™ kit (Gibco BRL) can be used. The double stranded DNA is then transformed, preferably by electroporation, into bacteria. The percentage of positive clones having the 5' tag oligonucleotide is typically estimated to be between 90 and 98% from dot blot analysis.

Following transformation, the libraries are ordered in microtiter plates and sequenced. The *Arabidopsis* library was deposited at the American Type Culture Collection on January 7, 2000 as *"E-coli* liba 010600" under the accession number **PTA-1161.**

## EXAMPLE 2: SOUTHERN HYBRIDIZATIONS

The SDFs of the invention can be used in Southern hybridizations as described above. The following describes extraction of DNA from nuclei of plant cells, digestion of the nuclear DNA and separation by length, transfer of the separated fragments to membranes, preparation of probes for hybridization, hybridization and detection of the hybridized probe.

The procedures described herein can be used to isolate related polynucleotides or for diagnostic purposes. Moderate stringency hybridization conditions, as defined above, are described in the present example. These conditions result in detection of hybridization between sequences having at least 70% sequence identity. As described above, the hybridization and wash conditions can be changed to reflect the desired percentage of sequence identity between probe and target sequences that can be detected.

In the following procedure, a probe for hybridization is produced from two PCR reactions using two primers from genomic sequence of *Arabidopsis thaliana.* As described above, the particular template for generating the probe can be any desired template.

The first PCR product is assessed to validate the size of the primer to assure it is of the expected size. Then the product of the first PCR is used as a template, with the same pair of primers used in the first PCR, in a second PCR that produces a labeled product used as the probe.

Fragments detected by hybridization, or other bands of interest, can be isolated from gels used to separate genomic DNA fragments by known methods for further purification and/or characterization.

### Buffers for nuclear DNA extraction

1. 10X HB

| | **1000 ml** | |
|---|---|---|
| 40 mM spermidine | 10.2 g | Spermine (Sigma S-2876) and spermidine (Sigma S-2501) |
| 10 mM spermine | 3.5 g | Stabilize chromatin and the nuclear membrane |
| 0.1 M EDTA (disodium) | 37.2 g | EDTA inhibits nuclease |
| 0.1 M Tris | 12.1 g | Buffer |
| 0.8 M KC1 | 59.6 g | Adjusts ionic strength for stability of nuclei |

Adjust pH to 9.5 with 10 N NaOH. It appears that there is a nuclease present in leaves. Use of pH 9.5 appears to inactivate this nuclease.

2. 2 M sucrose (684 g per 1000 ml)

Heat about half the final volume of water to about 50°C. Add the sucrose slowly then bring the mixture to close to final volume; stir constantly until it has dissolved. Bring the solution to volume.

3. Sarkosyl solution (lyses nuclear membranes)

| | 1000 ml |
|---|---|
| N-lauroyl sarcosine (Sarkosyl) | 20.0 g |
| 0.1 M Tris | 12.1 g |
| 0.04 M EDTA (Disodium) | 14.9 g |

Adjust the pH to 9.5 after all the components are dissolved and bring up to the proper volume.

4. 20% Triton X-100

80 ml Triton X-100

320 ml 1xHB (w/o β-ME and PMSF)

Prepare in advance; Triton takes some time to dissolve

A. Procedure

1. Prepare 1X "H" buffer (keep ice-cold during use)

| | | 1000 ml |
|---|---|---|
| 10X HB | | 100 ml |
| 2 M sucrose | | 250 ml a non-ionic osmoticum |
| Water | | 634 ml |

**Added just before use:**

| | |
|---|---|
| 100 mM PMSF* | 10 ml a protease inhibitor; protects nuclear membrane proteins |
| β-mercaptoethanol | 1 ml inactivates nuclease by reducing disulfide bonds |

*100 mM PMSF (phenyl methyl sulfonyl fluoride, Sigma P-7626) (add 0.0875 g to 5 ml 100% ethanol)

2. Homogenize the tissue in a blender (use 300-400 ml of 1xHB per blender). Be sure that you use 5-10 ml of HB buffer per gram of tissue. Blenders generate heat so be sure to keep the homogenate cold. It is necessary to put the blenders in ice periodically.
3. Add the 20% Triton X-100 (25 ml per liter of homogenate) and gently stir on ice for 20 min. This lyses plastid, but not nuclear, membranes.
4. Filter the tissue suspension through several nylon filters into an ice-cold beaker. The first filtration is through a 250-micron membrane; the second is through an 85-micron membrane; the third is through a 50-micron membrane; and the fourth is through a 20-micron membrane. Use a large funnel to hold the filters. Filtration can be sped up by gently squeezing the liquid through the filters.
5. Centrifuge the filtrate at 1200 x g for 20 min. at 4°C to pellet the nuclei.
6. Discard the dark green supernatant. The pellet will have several layers to it. One is starch; it is white and gritty. The nuclei are gray and soft. In the early steps, there may be a dark green and somewhat viscous layer of chloroplasts.
Wash the pellets in about 25 ml cold H buffer (with Triton X-100) and resuspend by swirling gently and pipetting. After the pellets are resuspended.
Pellet the nuclei again at 1200 - 1300 x g. Discard the supernatant.
Repeat the wash 3-4 times until the supernatant has changed from a dark green to a pale green. This usually happens after 3 or 4 resuspensions. At this point, the pellet is typically grayish white and very slippery. The Triton X-100 in these repeated steps helps to destroy the chloroplasts and mitochondria that contaminate the prep.
Resuspend the nuclei for a final time in a total of 15 ml of H buffer and transfer the suspension to a sterile 125 ml Erlenmeyer flask.
7. Add 15 ml, dropwise, cold 2% Sarkosyl, 0.1 M Tris, 0.04 M EDTA solution (pH 9.5) while swirling gently. This lyses the nuclei. The solution will become very viscous.
8. Add 30 grams of CsCl and gently swirl at room temperature until the CsCl is in solution. The mixture will be gray, white and viscous.
9. Centrifuge the solution at 11,400 x g at 4°C for at least 30 min. The longer this spin is, the firmer the protein pellicle.
10. The result is typically a clear green supernatant over a white pellet, and (perhaps) under a protein pellicle. Carefully remove the solution under the protein pellicle and above the pellet. Determine the density of the solution by weighing 1 ml of solution and add CsC1 if necessary to bring to 1.57 g/ml. The solution contains dissolved solids (sucrose etc) and the refractive index alone will not be an accurate guide to CsC1 concentration.
11. Add 20 μl of 10 mg/ml EtBr per ml of solution.
12. Centrifuge at 184,000 x g for 16 to 20 hours in a fixed-angle rotor.
13. Remove the dark red supernatant that is at the top of the tube with a plastic transfer pipette and discard. Carefully remove the DNA band with another transfer pipette. The DNA band is usually visible in room light; otherwise, use a long wave UV light to locate the band.
14. Extract the ethidium bromide with isopropanol saturated with water and salt. Once the solution is clear, extract at least two more times to ensure that all of the EtBr is gone. Be very gentle, as it is very easy to shear the DNA at this step. This extraction may take a while because the DNA solution tends to be very viscous. If the solution is too viscous, dilute it with TE.
15. Dialyze the DNA for at least two days against several changes (at least three times) of TE (10 mM Tris, 1mM EDTA, pH 8) to remove the cesium chloride.
16. Remove the dialyzed DNA from the tubing. If the dialyzed DNA solution contains a lot of debris, centrifuge the DNA solution at least at 2500 x g for 10 min. and carefully transfer the clear supernatant to a new tube. Read the A260 concentration of the DNA.

17. Assess the quality of the DNA by agarose gel electrophoresis (1% agarose gel) of the DNA. Load 50 ng and 100 ng (based on the OD reading) and compare it with known and good quality DNA. Undigested lambda DNA and a lambda-HindIII-digested DNA are good molecular weight makers.

**Protocol for Digestion of Genomic DNA**

Protocol:

1. The relative amounts of DNA for different crop plants that provide approximately a balanced number of genome equivalent is given in Table 3. Note that due to the size of the wheat genome, wheat DNA will be underrepresented. Lambda DNA provides a useful control for complete digestion.
2. Precipitate the DNA by adding 3 volumes of 100% ethanol. Incubate at -20° C for at least two hours. Yeast DNA can be purchased and made up at the necessary concentration, therefore no precipitation is necessary for yeast DNA.
3. Centrifuge the solution at 11,400 x g for 20 min. Decant the ethanol carefully (be careful not to disturb the pellet). Be sure that the residual ethanol is completely removed either by vacuum desiccation or by carefully wiping the sides of the tubes with a clean tissue.
4. Resuspend the pellet in an appropriate volume of water. Be sure the pellet is fully resuspended before proceeding to the next step. This may take about 30 min.
5. Add the appropriate volume of 10X reaction buffer provided by the manufacturer of the restriction enzyme to the resuspended DNA followed by the appropriate volume of enzymes. Be sure to mix it properly by slowly swirling the tubes.
6. Set-up the lambda digestion-control for each DNA that you are digesting.
7. Incubate both the experimental and lambda digests overnight at 37°C. Spin down condensation in a microfuge before proceeding.
8. After digestion, add 2 μl of loading dye (typically 0.25% bromophenol blue, 0.25% xylene cyanol in 15% Ficoll or 30% glycerol) to the lambda-control digests and load in 1% TPE-agarose gel (TPE is 90 mM Tris-phosphate, 2 mM EDTA, pH 8). If the lambda DNA in the lambda control digests are completely digested, proceed with the precipitation of the genomic DNA in the digests.
9. Precipitate the digested DNA by adding 3 volumes of 100% ethanol and incubating in -20°C for at least 2 hours (preferably overnight).
EXCEPTION: *Arabidopsis* and yeast DNA are digested in an appropriate volume; they don’t have to be precipitated.
10. Resuspend the DNA in an appropriate volume of TE (e.g., 22 μl x 50 blots = 1100 μl) and an appropriate volume of 10X loading dye (e.g., 2.4 μl x 50 blots = 120 μl). Be careful in pipetting the loading dye - it is viscous. Be sure you are pipetting the correct volume.

Table 3

| Some guide points in digesting genomic DNA. | | | | |
|---|---|---|---|---|
| **Species** | **Genome Size** | **Size Relative to Arabidopsis** | **Genome Equivalent to 2 μg Arabidopsis DNA** | **Amount of DNA per blot** |
| Arabidopsis | 120 Mb | 1X | 1X | 2 μg |
| Brassica | 1,100 Mb | 9.2X | 0.54X | 10 μg |
| Corn | 2,800 Mb | 23.3X | 0.43X | 20 μg |
| Cotton | 2,300 Mb | 19.2X | 0.52X | 20 μg |
| Oat | 11,300 Mb | 94X | 0.11X | 20μg |
| Rice | 400 Mb | 3.3X | 0.75X | 5 μg |
| Soybean | 1,100 Mb | 9.2X | 0.54X | 10 μg |
| Sugarbeet | 758 Mb | 6.3X | 0.8X | 10 μg |
| Sweetclover | 1,100 Mb | 9.2X | 0.54X | 10 μg |
| Wheat | 16,000 Mb | 133X | 0.08X | 20 μg |
| Yeast | 15 Mb | 0.12X | 1X | 0.25 μg |

**Protocol for Southern Blot Analysis**

The digested DNA samples are electrophoresed in 1% agarose gels in 1x TPE buffer. Low voltage; overnight separations are preferred. The gels are stained with EtBr and photographed.

1. For blotting the gels, first incubate the gel in 0.25 N HCl (with gentle shaking) for about 15 min.

2. Then briefly rinse with water. The DNA is denatured by 2 incubations. Incubate (with shaking) in 0.5 M NaOH in 1.5 M NaCl for 15 min.

3. The gel is then briefly rinsed in water and neutralized by incubating twice (with shaking) in 1.5 M Tris pH 7.5 in 1.5 M NaCl for 15 min.

4. A nylon membrane is prepared by soaking it in water for at least 5 min, then in 6X SSC for at least 15 min. before use. (20x SSC is 175.3 g NaCl, 88.2 g sodium citrate per liter, adjusted to pH 7.0.)

5. The nylon membrane is placed on top of the gel and all bubbles in between are removed. The DNA is blotted from the gel to the membrane using an absorbent medium, such as paper toweling and 6x SCC buffer. After the transfer, the membrane may be lightly brushed with a gloved hand to remove any agarose sticking to the surface. 760

6. The DNA is then fixed to the membrane by UV crosslinking and baking at 80°C. The membrane is stored at 4°C until use.

B. Protocol for PCR Amplification of Genomic Fragments in Arabidopsis

Amplification procedures:

1. Mix the following in a 0.20 ml PCR tube or 96-well PCR plate:

| **Volume** | **Stock** | **Final Amount or Conc.** |
|---|---|---|
| 0.5 μl | ~ 10 ng/μl genomic DNA[1] | 5 ng |
| 2.5 μl | **10X PCR buffer** | 20 mM Tris, 50 mM KCl |
| 0.75 μl | 50 mM $MgCl_2$ | 1.5 mM |

(continued)

| Volume | Stock | Final Amount or Conc. |
|---|---|---|
| 1 μl | 10 pmol/μl Primer 1 (Forward) | 10 pmol |
| 1 μl | 10 pmol/μl Primer 2 (Reverse) | 10 pmol |
| 0.5 μl | 5 mM dNTPs | 0.1 mM |
| 0.1 μl | 5 units/μl Platinum Taq™ (Life Technologies, Gaithersburg, MD) DNA Polymerase | 1 units |
| (to 25 μl) | **Water** | |
| [1]Arabidopsis DNA is used in the present experiment, but the procedure is a general one. | | |

2. The template DNA is amplified using a Perkin Elmer 9700 PCR machine:
1) 94°C for 10 min. followed by

| 2)<br>5 cycles: | 3)<br>5 cycles: | 4)<br>25 cycles: |
|---|---|---|
| 94 °C - 30 sec<br>62 °C - 30 sec<br>72 °C - 3 min | 94 °C - 30 sec<br>58 °C - 30 sec<br>72 °C - 3 min | 94 °C - 30 sec<br>53 °C - 30 sec<br>72 °C - 3 min |

5) 72°C for 7 min. Then the reactions are stopped by chilling to 4°C.
The procedure can be adapted to a multi-well format if necessary.

**Quantification and Dilution of PCR Products:**

1. The product of the PCR is analyzed by electrophoresis in a 1% agarose gel. A linearized plasmid DNA can be used as a quantification standard (usually at 50, 100, 200, and 400 ng). These will be used as references to approximate the amount of PCR products. HindIII-digested Lambda DNA is useful as a molecular weight marker. The gel can be run fairly quickly; e.g., at 100 volts. The standard gel is examined to determine that the size of the PCR products is consistent with the expected size and if there are significant extra bands or smeary products in the PCR reactions.

2. The amounts of PCR products can be estimated on the basis of the plasmid standard.

3. For the small number of reactions that produce extraneous bands, a small amount of DNA from bands with the correct size can be isolated by dipping a sterile 10-μl tip into the band while viewing though a UV Transilluminator. The small amount of agarose gel (with the DNA fragment) is used in the labeling reaction.

**C. Protocol for PCR-DIG-Labeling of DNA**

Solutions:

Reagents in PCR reactions (diluted PCR products, 10X PCR Buffer, 50 mM $MgCl_2$, 5 U/μl Platinum Taq Polymerase, and the primers)

10X dNTP + DIG-11-dUTP **[1:5]:** (2 mM dATP, 2 mM dCTP, 2 mM dGTP, 1.65 mM dTTP, 0.35 mM DIG-11-dUTP)

10X dNTP + DIG-11-dUTP **[1:10]:** (2 mM dATP, 2 mM dCTP, 2 mM dGTP, 1.81 mM dTTP, 0.19 mM DIG-11-dUTP)

10X dNTP + DIG-11-dUTP **[1:15]:** (2 mM dATP, 2 mM dCTP, 2 mM dGTP, 1.875 mM dTTP, 0.125 mM DIG-11-dUTP)

TE buffer (10 mM Tris, 1 mM EDTA, pH 8)

Maleate buffer: In 700 ml of deionized distilled water, dissolve 11.61 g maleic acid and 8.77 g NaCl. Add NaOH to adjust the pH to 7.5. Bring the volume to 1 L. Stir for 15 min. and sterilize.

10% blocking solution: In 80 ml deionized distilled water, dissolve 1.16g maleic acid. Next, add NaOH to adjust the pH to 7.5. Add 10 g of the blocking reagent powder (Boehringer Mannheim, Indianapolis, IN, Cat. no. 1096176). Heat to 60° C while stirring to dissolve the powder. Adjust the volume to 100 ml with water. Stir and sterilize.

1% blocking solution: Dilute the 10% stock to 1% using the maleate buffer.

Buffer 3 (100 mM Tris, 100 mM NaCl, 50 mM $MgCl_2$, pH9.5). Prepared from autoclaved solutions of 1M Tris pH 9.5, 5 M NaCl, and 1 M $MgC1_2$ in autoclaved distilled water.

Procedure:

1. PCR reactions are performed in 25 µl volumes containing:

| | |
|---|---|
| PCR buffer | 1 X |
| $MgCl_2$ | 1.5 mM |
| 10X dNTP + DIG-11-dUTP | 1X (please see the note below) |
| Platinum Taq™ Polymerase | 1 unit |
| 10 pg probe DNA | |
| 10 pmol primer 1 | |
| **Note:** | Use for: |
| 10X dNTP + DIG-11-dUTP (1:5) | < 1 kb |
| 10X dNTP + DIG-11-dUTP (1:10) | 1 kb to 1.8 kb |
| 10X dNTP + DIG-11-dUTP (1:15) | > 1.8 kb |

2. The PCR reaction uses the following amplification cycles:
1) 94°C for 10 min.

| 2)<br>5 cycles: | 3)<br>5 cycles: | 4)<br>25 cycles: |
|---|---|---|
| 95°C - 30 sec<br>61°C - 1 min<br>73°C - 5 min | 95°C - 30 sec<br>59°C - 1 min<br>75°C - 5 min | 95°C - 30 sec<br>51°C - 1 min<br>73°C - 5 min |

5) 72°C for 8 min. The reactions are terminated by chilling to 4°C (hold).
3. The products are analyzed by electrophoresis- in a 1% agarose gel, comparing to an aliquot of the unlabelled probe starting material.
4. The amount of DIG-labeled probe is determined as follows:
Make serial dilutions of the diluted control DNA in dilution buffer (TE: 10 mM Tris and 1 mM EDTA, pH 8) as shown in the following table:

| DIG-labeled control DNA starting conc. | Stepwise Dilution | Final Conc. (Dilution Name) |
|---|---|---|
| 5 ng/µl | 1 µl in 49 µl TE | 100 pg/µl (A) |
| 100 pg/µl (A) | 25 µl in 25 µl TE | 50 pg/µl (B) |
| 50 pg/µl (B) | 25 µl in 25 µl TE | 25 pg/µl (C) |
| 25 pg/µl (C) | 20 µl in 30 µl TE | 10 pg/µl (D) |

a. Serial deletions of a DIG-labeled standard DNA ranging from 100 pg to 10 pg are spotted onto a positively charged nylon membrane, marking the membrane lightly with a pencil to identify each dilution.

b. Serial dilutions (e.g., 1:50, 1:2500, 1:10,000) of the newly labeled DNA probe are spotted.

c. The membrane is fixed by UV crosslinking.

d. The membrane is wetted with a small amount of maleate buffer and then incubated in 1% blocking solution for 15 min at room temp.

e. The labeled DNA is then detected using alkaline phosphatase conjugated anti-DIG antibody (Boehringer Mannheim, Indianapolis, IN, cat. no. 1093274) and an NBT substrate according to the manufacture's instruction.

f. Spot intensities of the control and experimental dilutions are then compared to estimate the concentration of the PCR-DIG-labeled probe.

**D. Prehybridization and Hybridization of Southern Blots**

Solutions:

| | |
|---|---|
| 100% | Formamide purchased from Gibco |
| 20X | SSC (1X = 0.15 M NaCl, 0.015 M $Na_3$citrate) |
| per L: | 175gNaCl |
| | 87.5 g $Na_3$citrate-$2H_20$ |
| 20% | Sarkosyl (N-lauroyl-sarcosine) |
| 20% | SDS (sodium dodecyl sulphate) |
| 10% | Blocking Reagent: In 80 ml deionized distilled water, dissolve 1.16 g maleic acid. Next, add NaOH to adjust the pH to 7.5. Add 10 g of the blocking reagent powder. Heat to 60°C while stirring to dissolve the powder. Adjust the volume to 100 ml with water. Stir and sterilize. |

Prehybridization Mix:

| Final Concentration | Components | Volume (per 100 ml) | Stock |
|---|---|---|---|
| 50% | Formamide | 50 ml | 100% |
| 5X | SSC | 25 ml | 20X |
| 0.1 % | Sarkosyl | 0.5 ml | 20% |
| 0.02% | SDS | 0.1 ml | 20% |
| 2% | Blocking Reagent | 20 ml | 10% |
| | Water | 4.4 ml | |

General Procedures:

1. Place the blot in a heat-sealable plastic bag and add an appropriate volume of prehybridization solution (30 ml/ $100cm^2$) at room temperature. Seal the bag with a heat sealer, avoiding bubbles as much as possible. Lay down the bags in a large plastic tray (one tray can accommodate at least 4-5 bags). Ensure that the bags are lying flat in the tray so that the prehybridization solution is evenly distributed throughout the bag. Incubate the blot for at least 2 hours with gentle agitation using a waver shaker.

2. Denature DIG-labeled DNA probe by incubating for 10 min. at 98°C using the PCR machine and immediately cool it to 4°C.

3. Add probe to prehybridization solution (25 ng/ml; 30 ml = 750 ng total probe) and mix well but avoid foaming. Bubbles may lead to background.

4. Pour off the prehybridization solution from the hybridization bags and add new prehybridization and probe solution mixture to the bags containing the membrane.

5. Incubate with gentle agitation for at least 16 hours.

6. Proceed to medium stringency post-hybridization wash:

Three times for 20 min. each with gentle agitation using 1X SSC, 1% SDS at 60°C.

All wash solutions must be prewarmed to 60° C. Use about 100 ml of wash solution per membrane.

To avoid background keep the membranes fully submerged to avoid drying in spots; agitate sufficiently to avoid having membranes stick to one another.

7. After the wash, proceed to immunological detection and CSPD development.

**E. Procedure for Immunological Detection with CSPD**

Solutions:

| | |
|---|---|
| Buffer 1: | Maleic acid buffer (0.1 M maleic acid, 0.15 M NaCl; adjusted to pH 7.5 with NaoH) |
| Washing buffer: | Maleic acid buffer with 0.3% (v/v) Tween 20. |
| Blocking stock solution | 10% blocking reagent in buffer 1. Dissolve (10X concentration): blocking reagent powder (Boehringer Mannheim, Indianapolis, IN, cat. no. 1096176) by constantly stirring on a 65°C heating block or heat in a microwave, autoclave and store at 4° C. |
| Buffer 2 (1X blocking solution): | Dilute the stock solution 1:10 in Buffer 1. |
| Detection buffer: | 0.1 M Tris, 0.1 M NaCl, pH 9.5 |

Procedure:

1. After the post-hybridization wash the blots are briefly rinsed (1-5 min.) in the maleate washing buffer with gentle shaking.

2. Then the membranes are incubated for 30 min. in Buffer 2 with gentle shaking.

3. Anti-DIG-AP conjugate (Boehringer Mannheim, Indianapolis, IN, cat. no. 1093274) at 75 mU/ml (1:10,000) in Buffer 2 is used for detection. 75 ml of solution can be used for 3 blots.

4. The membrane is incubated for 30 min. in the antibody solution with gentle shaking.

5. The membrane are washed twice in washing buffer with gentle shaking. About 250 mls is used per wash for 3 blots.

6. The blots are equilibrated for 2-5 min in 60 ml detection buffer.

7. Dilute CSPD (1:200) in detection buffer. (This can be prepared ahead of time and stored in the dark at 4 C).
The following steps must be done individually. Bags (one for detection and one for exposure) are generally cut and

ready before doing the following steps.

8. The blot is carefully removed from the detection buffer and excess liquid removed without drying the membrane. The blot is immediately placed in a bag and 1.5 ml of CSPD solution is added. The CSPD solution can be spread over the membrane. Bubbles present at the edge and on the surface of the blot are typically removed by gentle rubbing. The membrane is incubated for 5 min. in CSPD solution.

9. Excess liquid is removed and the membrane is blotted briefly (DNA side up) on Whatman 3MM paper. Do not let the membrane dry completely.

10. Seal the damp membrane in a hybridization bag and incubate for 10 min at 37°C to enhance the luminescent reaction.

11. Expose for 2 hours at room temperature to X-ray film. Multiple exposures can be taken. Luminescence continues for at least 24 hours and signal intensity increases during the first hours.

**Example 3: Transformation of Carrot Cells**

Transformation of plant cells can be accomplished by a number of methods, as described above. Similarly, a number of plant genera can be regenerated from tissue culture following transformation. Transformation and regeneration of carrot cells as described herein is illustrative.

Single cell suspension cultures of carrot *(Daucus carota)* cells are established from hypocotyls of cultivar Early Nantes in $B_5$ growth medium (O.L. Gamborg et al., Plant Physiol. 45 : 3 72 (1970)) plus 2,4-D and 15 mM $CaCl_2$ ($B_5$ -44 medium) by methods known in the art. The suspension cultures are subcultured by adding 10 ml of the suspension culture to 40 ml of $B_5$-44 medium in 250 ml flasks every 7 days and are maintained in a shaker at 150 rpm at 27 °C in the dark.

The suspension culture cells are transformed with exogenous DNA as described by Z. Chen et al. Plant Mol. Bio. 36:163 (1998). Briefly, 4-days post-subculture cells are incubated with cell wall digestion solution containing 0.4 M sorbitol, 2% driselase, 5mM MES (2-[N-Morpholino] ethanesulfonic acid) pH 5.0 for 5 hours. The digested cells are pelleted gently at 60 xg for 5 min. and washed twice in W5 solution containing 154 mM NaCl, 5 mM KCl, 125 mM $CaCl_2$ and 5mM glucose, pH 6.0. The protoplasts are suspended in MC solution containing 5 mM MES, 20 mM $CaCl_2$, 0.5 M mannitol, pH 5.7 and the protoplast density is adjusted to about 4 x $10^6$ protoplasts per ml.

15-60 μg of plasmid DNA is mixed with 0.9 ml of protoplasts. The resulting suspension is mixed with 40% polyethylene glycol (MW 8000, PEG 8000), by gentle inversion a few times at room temperature for 5 to 25 min. Protoplast culture medium known in the art is added into the PEG-DNA-protoplast mixture. Protoplasts are incubated in the culture medium for 24 hour to 5 days and cell extracts can be used for assay of transient expression of the introduced gene. Alternatively, transformed cells can be used to produce transgenic callus, which in turn can be used to produce transgenic plants, by methods known in the art. See, for example, Nomura and Komamine, Plt. Phys. 79:988-991 (1985), *Identification and Isolation of Single Cells that Produce Somatic Embryos in Carrot Suspension Cultures.*

The invention being thus described, it will be apparent to one of ordinary skill in the art that various modifications of the materials and methods for practicing the invention can be made. Such modifications are to be considered within the scope of the invention as defined by the following claims.

Each of the references from the patent and periodical literature cited herein is hereby expressly incorporated in its entirety by such citation.

**Claims**

**1.** An isolated nucleic acid molecule comprising a nucleic acid having a nucleotide sequence which encodes an amino acid sequence exhibiting at least 40% sequence identity to an amino acid sequence encoded by

(a) a nucleotide sequence described in SEQ ID No.17540 or a fragment thereof; or
(b) a complement of a nucleotide sequence shown in SEQ ID No.17540 or a fragment thereof.

**2.** An isolated nucleic acid molecule comprising a nucleic acid having a nucleotide sequence which exhibits at least 65% sequence identity to

(a) a nucleotide sequence shown in SEQ ID No.17540 or a fragment thereof; or
(b) a complement of a nucleotide sequence shown in SEQ ID No.17540 or a fragment thereof.

**3.** An isolated nucleic acid molecule comprising a nucleic acid having a nucleotide sequence which exhibits at least 65% sequence identity to a gene comprising

(a) a nucleotide sequence shown in SEQ ID No.17540 or a fragment thereof; or
(b) a complement of a nucleotide sequence shown in SEQ ID No.17540 or a fragment thereof.

**4.** An isolated nucleic acid molecule according to claim 1 wherein the nucleotide sequence encodes an amino acid sequence exhibiting at least 95% sequence identity to an amino acid sequence encoded by

(a) a nucleotide sequence described in SEQ ID No.17540 or a fragment thereof; or
(b) a complement of a nucleotide sequence shown in SEQ ID No. 17540 or a fragment thereof.

**5.** An isolated nucleic acid molecule which is the reverse of the isolated nucleotide sequence according to any one of claims 1-3, such that the reverse nucleotide sequence has a sequence order which is the reverse of the sequence order of said isolated nucleotide sequence according to any one of claims 1-3.

**6.** An isolated nucleic acid molecule comprising a nucleic acid capable of hybridizing to a nucleic acid having a sequence selected from the group consisting of :

(a) a nucleotide sequence which is shown in SEQ ID No.17540; and
(b) a nucleotide sequence which is complementary to a nucleotide sequence shown in SEQ ID No.17540;

under conditions that permit formation of a nucleic acid duplex at a temperature from about 40°C and 48°C below the melting temperature of the nucleic acid duplex.

**7.** The nucleic acid molecule according to any one of claims 1 to 3 or 5 to 6, wherein said nucleic acid comprises an open reading frame.

**8.** The isolated nucleic acid molecule of any one of claims 1 to 3 or 5 to 6, wherein said nucleic acid is capable of functioning as a promoter, a 3' end termination sequence, an untranslated region (UTR), or as a regulatory sequence.

**9.** The isolated nucleic acid molecule of claim 8, wherein said nucleic acid is a promoter and comprises a sequence selected from the group consisting of a TATA box sequence, a CAAT box sequence, a motif of GCAATCG or any transcriptoin-factor binding sequence, and any combination thereof.

**10.** The isolated nucleic acid molecule of claim 8, wherein the nucleic acid sequence is a regulatory sequence which is capable of promoting seed-specific expression, embryo-specific expression, ovule-specific expression, tapetum-specific expression or root-specific expression of a sequence or any combination thereof.

**11.** A vector construct comprising a nucleic acid molecule according to any one of claims 1-3 or 5-10, wherein said nucleic acid molecule is heterologous to any element in said vector construct.

**12.** A vector construct according to claim 11 comprising:

(a) a first nucleic acid having a regulatory sequence capable of causing transcription and/or translation; and
(b) a second nucleic acid having the sequence of said isolated nucleic acid molecule according to any one of claims 1 to 3 or 5;

wherein said first and second nucleic acids are operably linked and
wherein said second nucleic acid is heterologous to any element in said vector construct.

**13.** The vector construct according to claim 12, wherein said first nucleic acid is native to said second nucleic acid.

**14.** The vector construct according to claim 12, wherein said first nucleic acid is heterologous to said second nucleic acid.

**15.** A vector construct according to claim 11 comprising:

(c) a first nucleic acid having having the sequence of said isolated nucleic acid molecule according to claim 8; and

(d) a second nucleic acid;

wherein said first and second nucleic acids are operably linked and
wherein said first nucleic acid is heterologous to any element in said vector construct.

**16.** The vector construct according to claim 15, wherein said first nucleic acid is native to said second nucleic acid.

**17.** The vector construct according to claim 15, wherein said first nucleic acid is heterologous to said second nucleic acid.

**18.** A host cell comprising an isolated nucleic acid molecule according to any one of claims 1-3 or 5, wherein said nucleic acid molecule is flanked by exogenous sequence.

**19.** A host cell comprising a vector construct of any one of claims 11-17.

**20.** An isolated polypeptide comprising an amino acid sequence

(a) exhibiting at least 40% sequence identity of an amino acid sequence encoded by a sequence shown in SEQ ID No.17540 or a fragment thereof; and
(b) capable of exhibiting at least one of the biological activities of the polypeptide encoded by said nucleotide seqence shown in SEQ ID No.17540 or a fragment thereof.

**21.** The isolated polypeptide of claim 20, wherein said amino acid sequence exhibits at least 75% sequence identity to an amino acid sequence encoded by a sequence shown in SEQ ID No.17540 or a fragment thereof.

**22.** The isolated polypeptide of claim 20, wherein said amino acid sequence exhibits at least 85% sequence identity to an amino acid sequence encoded by a sequence shown in SEQ ID No.17540 or a fragment thereof.

**23.** The isolated polypeptide of claim 20, wherein said amino acid sequence exhibits at least 90% sequence identity to an amino acid sequence encoded by a sequence shown in SEQ ID No.17540 or a fragment thereof.

**24.** The isolated polypeptide of claim 20, wherein said amino acid sequence is SEQ ID No. 17541 or SEQ ID No. 17542 or SEQ ID No. 17543.

**25.** An antibody capable of binding the isolated polypeptide of any one of claims 20-24.

**26.** A method of introducing an isolated nucleic acid into a host cell comprising:

(a) providing an isolated nucleic acid molecule according to any one of claims 1-3 or 5; and
(b) contacting said isolated nucleic with said host cell under conditions that permit insertion of said nucleic acid into said host cell.

**27.** A method of transforming a host cell which comprises contacting a host cell with a vector construct according to any one of claims 11-17.

**28.** A method of modulating transcription and/or translation of a nucleic acid in a host cell comprising:

(a) providing the host cell of claim 26 or 27; and
(b) culturing said host cell under conditions that permit transcription or translation.

**29.** A method for detecting a nucleic acid in a sample which comprises:

(a) providing an isolated nucleic acid molecule according to any one of claims 1-3 or 5-6;
(b) contacting said isolated nucleic acid molecule with a sample under conditions which permit a comparison of the sequence of said isolated nucleic acid molecule with the sequence of DNA in said sample; and
(c) analyzing the result of said comparison.

**30.** The method according to claim 29, wherein said isolated nucleic acid molecule and said sample are contacted under conditions which permit the formation of a duplex between complementary nucleic acid sequences.

**31.** A plant or cell of a plant which comprises a nucleic acid molecule according to any one of claims 1-3 or 5 which is exogenous to said plant or plant cell.

**32.** A plant or cell of a plant which comprises a nucleic acid molecule according to any one of claims 1-3 or 5, wherein said nucleic acid molecule is heterologous to said plant or said cell of a plant.

**33.** A plant or cell of a plant which has been transformed with a nucleic acid molecule according to any one of claims 1-3 or 5.

**34.** A plant of cell of a plant which comprises a vector construct according to any one of claims 11-17.

**35.** A plant of cell of a plant which has been transformed with a vector construct according to any one of claims 11-17.

**36.** A plant which has been regenerated from a plant cell according to any one of claims 31-35.

**37.** An isolated nucleic acid molecule comprising a nucleic acid having a nucleotide sequence which encodes an amino acid sequence exhibiting at least 40% sequence identity to an amino acid sequence encoded by

(a) a nucleotide sequence described in REF and/or SEQ Table 1 or 2 or a fragment thereof; or
(b) a complement of a nucleotide sequence shown in REF and/or SEQ Table 1 or 2 or a fragment thereof.

**38.** An isolated nucleic acid molecule comprising a nucleic acid having a nucleotide sequence which exhibits at least 65% sequence identity to

(a) a nucleotide sequence shown in REF and/or SEQ Table 1 or 2 or a fragment thereof; or
(b) a complement of a nucleotide sequence shown in REF and/or SEQ Table 1 or 2 or a fragment thereof.

**39.** An isolated nucleic acid molecule comprising a nucleic acid having a nucleotide sequence which exhibits at least 65% sequence identity to a gene comprising

(a) a nucleotide sequence shown in REF and/or SEQ Table 1 or 2 or a fragment thereof; or
(b) a complement of a nucleotide sequence shown in REF and/or SEQ Table 1 or 2 or a fragment thereof.

**40.** An isolated nucleic acid molecule which is the reverse of the isolated nucleotide sequence according to any one of claims 37-39, such that the reverse nucleotide sequence has a sequence order which is the reverse of the sequence order of said isolated nucleotide sequence according to any one of claims 37-39.

**41.** An isolated nucleic acid molecule comprising a nucleic acid capable of hybridizing to a nucleic acid having a sequence selected from the group consisting of :

(a) a nucleotide sequence which is shown in REF and/or SEQ Table 1 or 2; and
(b) a nucleotide sequence which is complementary to a nucleotide sequence shown in REF and/or SEQ Table 1 or 2;

under conditions that permit formation of a nucleic acid duplex at a temperature from about 40°C and 48°C below the melting temperature of the nucleic acid duplex.

**42.** The nucleic acid molecule according to any one of claims 37-41, wherein said nucleic acid comprises an open reading frame.

**43.** The isolated nucleic acid molecule of any one of claims 37-41, wherein said nucleic acid is capable of functioning as a promoter, a 3' end termination sequence, an untranslated region (UTR), or as a regulatory sequence.

**44.** The isolated nucleic acid molecule of claim 43, wherein said nucleic acid is a promoter and comprises a sequence selected from the group consisting of a TATA box sequence, a CAAT box sequence, a motif of GCAATCG or any transcriptoin-factor binding sequence, and any combination thereof.

**45.** The isolated nucleic acid molecule of claim 43, wherein the nucleic acid sequence is a regulatory sequence which

is capable of promoting seed-specific expression, embryo-specific expression, ovule-specific expression, tapetum-specific expression or root-specific expression of a sequence or any combination thereof.

**46.** A vector construct comprising a nucleic acid molecule according to any one of claims 37-45, wherein said nucleic acid molecule is heterologous to any element in said vector construct.

**47.** A vector construct according to claim 46 comprising:

(a) a first nucleic acid having a regulatory sequence capable of causing transcription and/or translation; and
(b) a second nucleic acid having the sequence of said isolated nucleic acid molecule according to any one of claims 37-40;

wherein said first and second nucleic acids are operably linked and
wherein said second nucleic acid is heterologous to any element in said vector construct.

**48.** The vector construct according to claim 47, wherein said first nucleic acid is native to said second nucleic acid.

**49.** The vector construct according to claim 47, wherein said first nucleic acid is heterologous to said second nucleic acid.

**50.** A vector construct according to claim 46 comprising:

(c) a first nucleic acid having the sequence of said isolated nucleic acid molecule according to claim 43; and
(d) a second nucleic acid;

wherein said first and second nucleic acids are operably linked and
wherein said first nucleic acid is heterologous to any element in said vector construct.

**51.** The vector construct according to claim 50, wherein said first nucleic acid is native to said second nucleic acid.

**52.** The vector construct according to claim 50, wherein said first nucleic acid is heterologous to said second nucleic acid.

**53.** A host cell comprising an isolated nucleic acid molecule according to any one of claims 37-40, wherein said nucleic acid molecule is flanked by exogenous sequence.

**54.** A host cell comprising a vector construct of any one of claims 46-52.

**55.** An isolated polypeptide comprising an amino acid sequence

(a) exhibiting at least 40% sequence identity of an amino acid sequence encoded by a sequence shown in REF and/or SEQ Table 1 or 2 or a fragment thereof; and
(b) capable of exhibiting at least one of the biological activities of the polypeptide encoded by said nucleotide seqence shown in REF and/or SEQ Table 1 or 2 or a fragment thereof.

**56.** The isolated polypeptide of claim 55, wherein said amino acid sequence exhibits at least 75% sequence identity to an amino acid sequence encoded by a sequence shown in SEQ Table 1 or 2 or a fragment thereof.

**57.** The isolated polypeptide of claim 55, wherein said amino acid sequence exhibits at least 85% sequence identity to an amino acid sequence encoded by a sequence shown in SEQ Table 1 or 2 or a fragment thereof.

**58.** The isolated polypeptide of claim 55, wherein said amino acid sequence exhibits at least 90% sequence identity to an amino acid sequence encoded by a sequence shown in SEQ Table 1 or 2 or a fragment thereof.

**59.** An antibody capable of binding the isolated polypeptide of any one of claims 55-58.

**60.** A method of introducing an isolated nucleic acid into a host cell comprising:

(a) providing an isolated nucleic acid molecule according to any one of claims 37-40; and
(b) contacting said isolated nucleic with said host cell under conditions that permit insertion of said nucleic acid

into said host cell.

**61.** A method of transforming a host cell which comprises contacting a host cell with a vector construct according to any one of claims 46-52.

**62.** A method of modulating transcription and/or translation of a nucleic acid in a host cell comprising:

(a) providing the host cell of claim 60 or 61; and
(b) culturing said host cell under conditions that permit transcription or translation.

**63.** A method for detecting a nucleic acid in a sample which comprises:

(a) providing an isolated nucleic acid molecule according to any one of claims 37-41;
(b) contacting said isolated nucleic acid molecule with a sample under conditions which permit a comparison of the sequence of said isolated nucleic acid molecule with the sequence of DNA in said sample; and
(c) analyzing the result of said comparison.

**64.** The method according to claim 63, wherein said isolated nucleic acid molecule and said sample are contacted under conditions which permit the formation of a duplex between complementary nucleic acid sequences.

**65.** A plant or cell of a plant which comprises a nucleic acid molecule according to any one of claims 37-40 which is exogenous to said plant or plant cell.

**66.** A plant or cell of a plant which comprises a nucleic acid molecule according to any one of claims 37-40, wherein said nucleic acid molecule is heterologous to said plant or said cell of a plant.

**67.** A plant or cell of a plant which has been transformed with a nucleic acid molecule according to any one of claims 37-40.

**68.** A plant of cell of a plant which comprises a vector construct according to any one of claims 46-52.

**69.** A plant of cell of a plant which has been transformed with a vector construct according to any one of claims 46-52.

**70.** A plant which has been regenerated from a plant cell according to any one of claims 65-69.

SCHEMATIC 1

SCHEMATIC OF A GENE

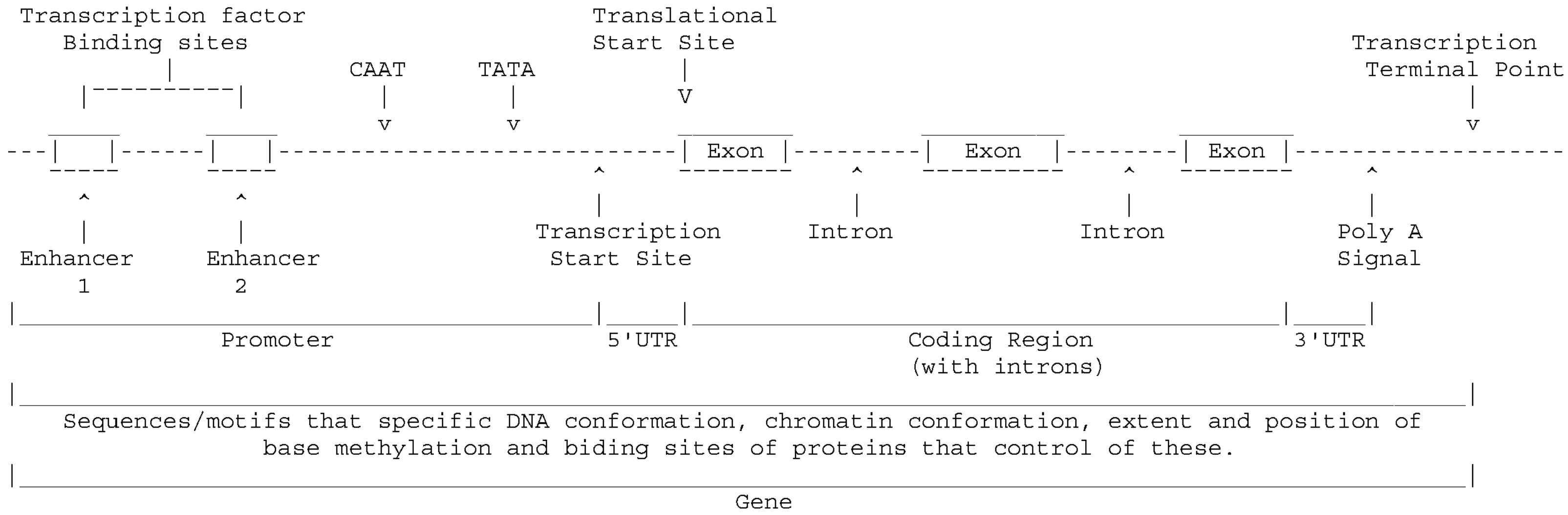

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9535505 A
- US 5445943 A
- US 5410270 A
- US 6004753 A
- US 5945306 A
- US 5945287 A
- US 5945308 A
- US 5919686 A
- US 5919661 A
- US 5919627 A
- US 5874248 A
- US 5871973 A
- US 5871971 A
- US 5871930 A
- WO 9946380 A
- WO 9933981 A
- WO 9933870 A
- WO 9931252 A
- WO 9915658 A
- WO 9906572 A
- WO 9858052 A
- WO 9958672 A
- WO 9810858 A
- US 5766847 A
- US 5859330 A
- US 5723766 A
- US 6010907 A
- US 6004804 A
- WO 9958723 A
- WO 9907865 A
- US 5034323 A
- US 5231020 A
- US 5283184 A
- US 700152 A
- US 08879827 B
- US 08912272 B
- US 09026039 B
- WO 9807842 A
- US 4801340 A, Hiatt
- WO 9634981 A
- EP 0625572 A


### Non-patent literature cited in the description


- **BLATTNER et al.** *Science,* 1997, vol. 277, 1453
- **GOFFEAU et al.** *Science,* 1996, vol. 274, 546
- C. elegans Sequencing Consortium. *Science,* 1998, vol. 282, 2012
- **SALOMON et al.** *EMBO J.,* 1984, vol. 3, 141
- **HERRERA-ESTRELLA et al.** *EMBO J.,* 1983, vol. 2, 987
- **ESCUDERO et al.** *Plant J.,* 1996, vol. 10, 355
- **ISHIDA et al.** *Nature Biotechnology,* 1996, vol. 14, 745
- **MAY et al.** *Bio/Technology,* 1995, vol. 13, 486
- **ARMALEO et al.** *Current Genetics,* 1990, vol. 17, 97
- **WANG ; GOODMAN.** *PlantJ.,* 1995, vol. 8, 37
- **SMITH ; WATERMAN.** *Add. APL. Math.,* 1981, vol. 2, 482
- **NEEDLEMAN ; WUNSCH.** *J. Mol. Biol.,* 1970, vol. 48, 443
- **PEARSON ; LIPMAN.** *Proc. Natl. Acad Sci. (USA),* 1988, vol. 85, 2444
- **SPIKER ; THOMPSON.** *Plant Physiol.,* 1996, vol. 110, 15-21
- **KOLTONOW et al.** *Plant Cell,* 1990, vol. 2, 1201
- **XU et al.** *Plant Mol. Biol.,* 1995, vol. 27, 237
- **YAMAMOTO et al.** *Plant Cell,* 1991, vol. 3, 37
- Hybridization with Nucleic Acid Probes. **P. TIJESSEN.** Laboratory Techniques in Biochemistry and Molecular Biology. Elsevier, 1993
- **BONNER et al.** *J. Mol. Biol.,* 1973, vol. 81, 123
- **MATTEUCCI et al.** *J. Am. Chem. Soc.,* 1981, vol. 103, 3185
- **URDEA et al.** *Proc. Natl. Acad.,* 1981, vol. 80, 7461
- **SAMBROOK et al.** Molecular Cloning, a Laboratory Manual. Cold Spring Harbor Laboratory Press, 1989
- **G.H. KELLER ; M.M. MANAK.** DNA Probes. Stockton Press, 1993, 1-25
- **CARELS et al.** *J Mol. Evol.,* 1998, vol. 46, 45
- **FENNOY et al.** *Nucl. Acids Res.,* 1993, vol. 21 (23), 5294
- Arabidopsis Protocols. **ALONSO-BLANCO et al.** Methods in Molecular Biology. Humana Press, 1998, vol. 82, 137-146

- **BURR.** Mapping Genes with Recombinant Inbreds. *The Maize Handbook,* 1994, 249-254
- **BURR et al.** *Genetics,* 1998, vol. 118, 519
- **GARDINER, J. et al.** *Genetics,* 1993, vol. 134, 917
- **MORGANTE et al.** *The Plant Journal,* 1993, vol. 3, 165
- **PANAUD et al.** *Genome,* 1995, vol. 38, 1170
- **SENIOR et al.** *Crop Science,* 1996, vol. 36, 1676
- **TARAMINO et al.** *Genome,* 1996, vol. 39, 277
- **AHN et al.** *Molecular and General Genetics,* 1993, vol. 241, 483-90
- **U.H. REFSETH et al.** *Electrophoresis,* 1997, vol. 18, 1519
- **VICENTE ; TANKSLEY.** *Genetics,* 1993, vol. 134, 585
- **TANKSLEY ; MCCOUCH.** *Science,* 1997, vol. 277, 1063
- **MARRA et al.** *Genomic Research,* 1997, vol. 7, 1072-1084
- **TANKSLEY et al.** *Science,* 1997, vol. 277, 1063
- **SAMBROOK et al.** Molecular Cloning: A Laboratory Manual. Cold Spring Harbor Laboratory Press, 1989
- **AUSUBEL et al.** Current Protocols in Molecular Biology. Greene Publishing, 1992
- **OELLER et al.** *Science,* 1991, vol. 254, 437-439
- **HAMILTON et al.** *Nature,* 1990, vol. 346, 284-287
- **S.D. MICHAELS et al.** *Plant Cell,* 1999, vol. 11, 949
- **S.J. LILJEGREN.** *Plant Cell,* 1999, vol. 11, 1007
- **D. DE MARTINIS et al.** *Plant Cell,* 1999, vol. 11, 1061
- **HASELOFF et al.** *Nature,* 1988, vol. 334, 585
- **NAPOLI et al.** *The Plant Cell,* 1990, vol. 2, 279
- **VAUCHERET, H et al.** *The Plant Journal,* 1998, vol. 16, 651-659
- **J.J. SCHWARZ et al.** *Mol. Cell. Biol.,* 1992, vol. 12, 266
- **A. MARTINEZ et al.** *Mol. Gen. Genet.,* 1999, vol. 261, 546
- **L. MCALISTER-HENN et al.** *Methods,* 1999, vol. 19, 330
- **J.C. HU et al.** *Methods,* 2000, vol. 20, 80
- **M. GOLOVKIN et al.** *J. Biol. Chem.,* 1999, vol. 274, 36428
- **K. ICHIMURA et al.** *Biochem. Biophys. Res. Comm.,* 1998, vol. 253, 532
- **B. LUO et al.** *J. Mol. Biol.,* 1997, vol. 266, 479
- **KACZOR et al.** *Plant Physiol.,* 1994, vol. 1-4, 1411-7
- **SMITH.** *Biochem. J.,* 1988, vol. 249, 423-8
- **SCHNEIDER.** *Z. naturforsch. [C,* 1976, vol. 31, 55-63
- **S. PIERROU et al.** *Anal. Biochem.,* 1995, vol. 229, 99
- **S. CHUSACULTANACHAI et al.** *J Biol. Chem.,* 1999, vol. 274, 23591
- **Q. LIN et al.** *J Biol. Chem.,* 1997, vol. 272, 27274
- **FROEHLICH K.-U. ; FRIES H.W. ; RUEDIGER M. ; ERDMANN R. ; BOTSTEIN D. ; MECKE D.** *J. Cell Biol.,* 1991, vol. 114, 443-453
- **ERDMANN R. ; WIEBEL F.F. ; FLESSAU A. ; RYTKA J. ; BEYER A. ; FROEHLICH K.-U. ; KUNAU W.-H.** *Cell,* 1991, vol. 64, 499-510
- **PETERS J.-M. ; WALSH M.J. ; FRANKE W.W.** *EMBO J.,* 1990, vol. 9, 1757-1767
- **KUNAU W.-H. ; BEYER A. ; GOETTE K. ; MARZIOCH M. ; SAIDOWSKY J. ; SKALETZ-ROROWSKI A. ; WIEBEL F.F.** *Biochimie,* 1993, vol. 75, 209-224
- **CONFALONIERI F. ; DUGUET M.** *BioEssays,* 1995, vol. 17, 639-650
- **HILT W. ; WOLFD.H.** *Trends Biochem. Sci.,* 1996, vol. 21, 96-102
- **HIGGINS C.F. ; HYDE S.C. ; MIMMACK M.M. ; GILEADI U. ; GILL D.R. ; GALLAGHER M.P.** *J. Bioenerg. Biomembr.,* 1990, vol. 22, 571-592
- **HIGGINS C.F. ; GALLAGHER M.P. ; MIMMACK M.M. ; PEARCE S.R.** *BioEssays,* 1988, vol. 8, 111-116
- **HIGGINS C.F. ; HILES I.D. ; SALMOND G.P.C. ; GILL D.R. ; DOWNIE J.A. ; EVANS I.J. ; HOLLAND I.B. ; GRAY L. ; BUCKELS S.D. ; BELL A.W.** *Nature,* 1986, vol. 323, 448-450
- **DOOLITTLE R.F. ; JOHNSON M.S. ; HUSAIN I. ; VAN HOUTEN B. ; THOMAS D.C. ; SANCAR A.** *Nature,* 1986, vol. 323, 451-453
- **BLIGHT M.A. ; HOLLAND I.B.** *Mol. Microbiol.,* 1990, vol. 4, 873-880
- **STODDARD G.W. ; PETZEL J.P. ; VAN BELKUM M.J. ; KOK J. ; MCKAY L.L.** *Appl. Environ. Microbiol.,* 1992, vol. 58, 1952-1961
- **ROSTECK P.R. JR. ; REYNOLDS P.A. ; HERSHBERGER C.L.** *Gene,* 1991, vol. 102, 27-32
- **GOTTESMAN M.M. ; PASTAN I.** *J. Biol. Chem.,* 1988, vol. 263, 12163-12166
- **VALLE D. ; GAERTNER J.** *Nature,* 1993, vol. 361, 682-683
- **AGUILAR-BRYAN L. ; NICHOLS C.G. ; WECHSLER S.W. ; CLEMENT J.P. IV ; BOYD A.E. III ; GONZALEZ G. ; HERRERA-SOSA H. ; NGUY K. ; BRYAN J. ; NELSON D.A.** *Science,* 1995, vol. 268, 423-426
- **ROSE T.M. ; SCHULTZ E.R. ; TODARO G.J.** *Proc. Natl. Acad. Sci. U.S.A.,* 1992, vol. 89, 11287-11291

- **COSTA E. ; GUIDOTTI A.** *Life Sci.,* 1991, vol. 49, 325-344
- **TOH H.** *Protein Seq. Data Anal.,* 1991, vol. 4, 111-117
- **SMITH D.J. ; EARL A.J. ; TURNER G.** *EMBO J.,* 1990, vol. 9, 2743-2750
- **SCHROEDER J.** *Nucleic Acids Res.,* 1989, vol. 17, 460-460
- **MALLONEE D.H. ; ADAMS J.L. ; HYLEMON P.B.** *J. Bacteriol.,* 1992, vol. 174, 2065-2071
- **TURGAY K. ; KRAUSE M. ; MARAHIEL M.A.** *Mol. Microbiol.,* 1992, vol. 6, 529-546
- **DRISCOLL J.R. ; TABER H.W.** *J. Bacteriol.,* 1992, vol. 174, 5063-5071
- **BABBITT P.C. ; KENYON G.L. ; MATIN B.M. ; CHAREST H. ; SYLVESTRE M. ; SCHOLTEN J.D. ; CHANG K.-H. ; LIANG P.-H. ; DUNAWAY-MARIANO D.** *Biochemistry,* 1992, vol. 31, 5594-5604
- **FARRELL D.H. ; MIKESELL P. ; ACTIS L.A. ; CROSA J.H.** *Gene,* 1990, vol. 86, 45-51
- **OHME-TAKAGI M ; SHINSHI H.** *Plant Cell,* 1995, vol. 7, 173-182
- **WEIGEL D.** *Plant Cell,* 1995, vol. 7, 388-389
- **MUSHEGIAN AR ; KOONIN EV.** *Genetics,* 1996, vol. 144, 817-828
- **HERRSCHER RF ; KAPLAN MH ; LELSZ DL ; DAS C ; SCHEUERMANN R ; TUCKER PW.** *Genes Dev,* 1995, vol. 9, 3067-3082
- **YUAN YC ; WHITSON RH ; LIU Q ; ITAKURA K ; CHEN Y.** *Nat Struct Biol,* 1998, vol. 5, 959-964
- **FUTAI M. ; NOUMI T. ; MAEDA M.** *Annu. Rev. Biochem.,* 1989, vol. 58, 111-136
- **SENIOR A.E.** *Physiol. Rev.,* 1988, vol. 68, 177-231
- **MIKI J. ; MAEDA M. ; MUKOHATA Y. ; FUTAI M.** *FEBS Lett.,* 1988, vol. 232, 221-226
- **LEWIS M.L. ; CHANG J.A. ; SIMONI R.D.** *J. Biol. Chem.,* 1990, vol. 265, 10541-10550
- **CAIN B.D. ; SIMONI R.D.** *J. Biol. Chem.,* 1989, vol. 264, 3292-3300
- **IVASCHENKO A.T. ; KARPENYUK T.A. ; PONOMARENKO S.V.** *Biokhimiia,* 1991, vol. 56, 406-419
- **RECIPON H. ; PERASSO R. ; ADOUTTE A. ; QUETIER F.** *J. Mol. Evol.,* 1992, vol. 34, 292-303
- **NELSON N. J.** *Bioenerg. Biomembr.,* 1989, vol. 21, 553-571
- **GOGARTEN J.P. ; KIBAK H. ; DITTRICH P. ; TAIZ L. ; BOWMAN E.J. ; BOWMAN B.J. ; MANOLSON M.F. ; POOLE R.J. ; DATE T. ; OSHIMA T.** *Proc. Natl. Acad. Sci. U.S.A.,* 1989, vol. 86, 6661-665
- **DREYFUS G. ; WILLIAMS A.W. ; KAWAGISHI I. ; MACNAB R.M.** *J. Bacteriol.,* 1993, vol. 175, 3131-3138
- **ABRAHAMS JP ; LESLIE AG ; LUTTER R ; WALKER JE.** Structure at 2.8 A resolution of F1-ATPase from bovine heart mitochondria. *Nature,* 1994, vol. 370, 621-628
- **CHANG Z. ; NYGAARD P. ; CHINAULT A.C. ; KELLEMS R.E.** *Biochemistry,* 1991, vol. 30, 2273-2280
- **NEUWALD AF ; LANDSMAN D.** *Trends Biochem Sci,* 1997, vol. 22, 154-155
- **GRUER M.J. ; ARTYMIUK P.J. ; GUEST J.R.** *Trends Biochem. Sci,* 1997, vol. 22, 3-6
- **TANAKA K. ; IKEDA, MATSUBARA Y. ; HYMAN D.B.** *Enzyme,* 1987, vol. 38, 91-107
- **MATSUBARA Y. ; INDO Y. ; NAITO E. ; OZASA H. ; GLASSBERG R. ; VOCKLEY J. ; IKEDA Y. ; KRAUS J. ; TANAKA K.** *J. Biol. Chem.,* 1989, vol. 264, 16321-16331
- **AOYAMA T. ; UENO L ; KAMIJO T. ; HASHIMOTO T.** *J. Biol. Chem.,* 1994, vol. 269, 19088-19094
- **EICHLER K. ; BOURGIS F. ; BUCHET A. ; KLEBER H.-P. ; MANDRAND-BERTHELOT M.-A.** *Mol. Microbiol.,* 1994, vol. 13, 775-786
- **SERRE L ; VERBREE EC ; DAUTER Z ; STUITJE AR ; DEREWENDA ZS.** The Escherichia coli malonyl-CoA:acyl carrier protein transacylase at 1.5-A resolution. Crystal structure of a fatty acid synthase component. *J Biol Chem,* 1995, vol. 270, 12961-12964
- **STEFANI M. ; RAMPONI G.** *Life Chem. Rep.,* 1995, vol. 12, 271-301
- **STEFANI M. ; TADDEI N. ; RAMPONI G.** *Cell. Mol. Life Sci.,* 1997, vol. 53, 141-151
- **PEARSE B.M. ; ROBINSON M.S.** *Annu. Rev. Cell Biol.,* 1990, vol. 6, 151-171
- **LEE J. ; JONGEWARD G.D. ; STERNBERG P.W.** *Genes Dev.,* 1994, vol. 8, 60-73
- **NAKAYAMA Y. ; GOEBL M. ; O'BRINE G.B. ; LEMMON S. ; PINGCHANG C.E. ; KIRCHHAUSEN T.** *Eur. J. Biochem.,* 1991, vol. 202, 569-574
- **WIESMUELLER L. ; WITTBRODT J. ; NOEGEL A.A. ; SCHLEICHER M.** *J. Biol. Chem.,* 1991, vol. 266, 2480-2485
- **SILVA M.M. ; POLAND B.W. ; HOFFMAN C.R. ; FROMM H.J. ; HONZATKO R.B.** *J. Mol. Biol.,* 1995, vol. 254, 431-446
- **BOUYOUB A. ; BARBIER G. ; FORTERRE P. ; LABEDAN B.** *J. Mol. Biol.,* 1996, vol. 261, 144-154
- **SGANGA M.W. ; AKSAMIT R.R. ; CANTONI G.L. ; BAUER C.E.** *Proc. Natl. Acad. Sci. U.S.A.,* 1992, vol. 89, 6328-6332
- **CHAE HZ ; ROBISON K ; POOLE LB ; CHURCH G ; STORZ G ; RHEE SG.** *Proc Natl Acad Sci U S A,* 1994, vol. 91, 7017-7021
- **POOLMAN B. ; ROYER T.J. ; MAINZER S.E. ; SCHMIDT B.F.** *J. Bacteriol.,* 1990, vol. 172, 4037-4047

- **LINDAHL T. ; SEDGWICK B.** *Annu. Rev. Biochem.,* 1988, vol. 57, 133-157
- **BERDAL K.G. ; BJORAS M. ; BJELLAND S. ; SEEBERG E.C.** *EMBO J.,* 1990, vol. 9, 4563-4568
- **CHEN J. ; DERFLER B. ; SAMSON L.** *EMBO J.,* 1990, vol. 9, 4569-4575
- **NINNEMANN O. ; JANNIAUX J.-C. ; FROMMER W.B.** *EMBO J.,* 1994, vol. 13, 3464-3471
- **SIEWE R.M. ; WEIL B. ; BURKOVSKI A. ; EIKMANNS B.J. ; EIKMANNS M. ; KRAEMER R.** *J. Biol. Chem.,* 1996, vol. 271, 5398-5403
- **SAIER M.H. JR.** *Adv. Microbiol. Physiol.,* 1998, vol. 40, 81-136
- **LI X.-Y. ; MANTOVANI R. ; HOOFT VAN HUIJSDUIJNEN R. ; ANDRE I. ; BENOIST C. ; MATHIS D.** *Nucleic Acids Res.,* 1992, vol. 20, 1087-1091
- **OLESEN J.T. ; FIKES J.D. ; GUARENTE L.** *Mol. Cell. Biol.,* 1991, vol. 11, 611-619
- **VAN VLIET F. ; CRABEEL M. ; BOYEN A. ; TRICOT C. ; STALON V. ; FALMAGNE P. ; NAKAMURA Y. ; BAUMBERG S. ; GLANSDORFF N.** *Gene,* 1990, vol. 95, 99-104
- **MORRIS C.J. ; REEVE J.N.** *J. Bacteriol.,* 1988, vol. 170, 3125-3130
- **HUBER AH ; NELSON WJ ; WEIS WI.** *Cell,* 1997, vol. 90, 871-882
- **GUMBINER BM.** *Curr Opin Cell Biol,* 1995, vol. 7, 634-640
- **CAVALLO R ; RUBENSTEIN D ; PEIFER M.** *Curr Opin Genet Dev,* 1997, vol. 7, 459-466
- **SU LK ; VOGELSTEIN B ; KINZLER KW.** *Science,* 1993, vol. 262, 1734-1737
- **MASIARZ FR ; MUNEMITSU S ; POLAKIS P.** *Science,* 1993, vol. 262, 1731-1734
- **PEIFER M ; WIESCHAUS E.** *Cell,* 1990, vol. 63, 1167-1176
- **SCHIMMEL P.** *Annu. Rev. Biochem.,* 1987, vol. 56, 125-158
- **DELARUE M. ; MORAS D.** *BioEssays,* 1993, vol. 15, 675-687
- **SCHIMMEL P.** *Trends Biochem.,* 1991, vol. 16, 1-3
- **NAGEL G.M. ; DOOLITTLE R.F.** *Proc. Natl. Acad. Sci. U.S.A.,* 1991, vol. 88, 8121-8125
- **CUSACK S. ; HAERTLEIN M. ; LEBERMAN R.** *Nucleic Acids Res.,* 1991, vol. 19, 3489-3498
- **CUSACK S.** *Biochimie,* 1993, vol. 75, 1077-1081
- **CUSACK S. ; BERTHET-COLOMINAS C. ; HAERTLEIN M. ; NASSAR N. ; LEBERMAN R.** *Nature,* 1990, vol. 347, 249-255
- **LEVEQUE F. ; PLATEAU P. ; DESSEN P. ; BLANQUET S.** *Nucleic Acids Res.,* 1990, vol. 18, 305-312
- **HAMMONDS-ODIE LP ; JACKSON TR ; PROFIT AA ; BLADER IJ ; TURCK CW ; PRESTWICH GD ; THEIBERT AB.** *J Biol Chem,* 1996, vol. 271, 18859-18868
- **TANAKA K ; IMAJOH-OHMI S ; SAWADA T ; SHIRAI R ; HASHIMOTO Y ; IWASAKI S ; KAIBUCHI K ; KANAHO Y ; SHIRAI T ; TERADA Y.** *Eur J Biochem,* 1997, vol. 245, 512-519
- **VITALE N ; MOSS J ; VAUGHAN M.** Molecular characterization of the GTPase-activating domain of ADP-ribosylation factor domain protein 1 (ARD1). *J Biol Chem,* 1998, vol. 273, 2553-2560
- **WILSON C ; WARDELL MR ; WEISGRABER KH ; MAHLEY RW ; AGARD DA.** *Science,* 1991, vol. 252, 1817-1822
- **WEBER E. ; CHEVALIER M.R. ; JUND R.** *J. Mol. Evol.,* 1988, vol. 27, 341-350
- **VANDENBOL M. ; JAUNIAUX J.-C. ; GRENSON M.** *Gene,* 1989, vol. 83, 153-159
- **REIZER J. ; FINLEY K. ; KAKUDA D. ; MCLEOD C.L. ; REIZER A. ; SAIER M.H. JR.** *Protein Sci.,* 1993, vol. 2, 20-30
- **LI W. ; BRANDRISS M.C.** *J. Bacteriol.,* 1992, vol. 174, 4148-4156
- **HU C.-A.A. ; DELAUNEY A.J. ; VERMA D.P.S.** *Proc. Natl. Acad. Sci. U.S.A.,* 1992, vol. 89, 9354-9358
- **RAFALSKI J.A. ; FALCO S.C.** *J. Biol. Chem.,* 1988, vol. 263, 2146-2151
- **PEARSON B.M. ; HERNANDO Y. ; PAYNE J. ; WOLFS.S. ; KALOGEROPOULOS A. ; SCHWEIZER M.** *Yeast,* 1996, vol. 12, 1021-1031
- **OLLIS DL ; CHEAH E ; CYGLER M ; DIJKSTRA B ; FROLOW F ; FRANKEN SM ; HAREL M ; REMINGTON SJ ; SILMAN I ; SCHRAG J.** *Protein Eng,* 1992, vol. 5, 197-211
- **VAN ETTEN R.L. ; DAVIDSON R. ; STEVIS P.E. ; MACARTHUR H. ; MOORE D.L.** *J. Biol. Chem.,* 1991, vol. 266, 2313-2319
- **OSTANIN K. ; HARMS E.H. ; STEVIS P.E. ; KUCIEL R. ; ZHOU M.-M. ; VAN ETTEN R.L.** *J. Biol. Chem.,* 1992, vol. 267, 22830-22836
- **SCHNEIDER G. ; LINDQVIST Y. ; VIHKO P.** *EMBO J.,* 1993, vol. 12, 2609-2615
- **GRUER M.J. ; ARTYMIUK P.J. ; GUEST J.R.** *Trends Biochem. Sci.,* 1997, vol. 22, 3-6
- **SHETERLINE P. ; CLAYTON J. ; SPARROW J.C.** Actins. Academic Press Ltd, 1996
- **POLLARD T.D. ; COOPER J.A.** *Annu. Rev. Biochem.,* 1986, vol. 55, 987-1036
- **POLLARD T.D.** *Curr. Opin. Cell Biol.,* 1990, vol. 1, 33-40
- **RUBENSTEIN P.A.** *BioEssays,* 1990, vol. 12, 309-315

- **MEAGHER R.B. ; MCLEAN B.G.** *Cell Motil. Cytoskeleton,* 1990, vol. 16, 164-166
- **SCHULZ G.E.** *Cold Spring Harbor Symp. Quant. Biol.,* 1987, vol. 52, 429-439
- **LILJELUND P. ; SANNI A. ; FRIESEN J.D. ; LACROUTE F.** *Biochem. Biophys. Res. Commun.,* 1989, vol. 165, 464-473
- **WIESMUELLER L. ; NOEGEL A.A. ; BARZU O. ; GERISCH G. ; SCHLEICHER M.** *J. Biol. Chem.,* 1990, vol. 265, 6339-6345
- **KATH T.H. ; SCHMID R. ; SCHAEFER G.** *Arch. Biochem. Biophys.,* 1993, vol. 307, 405-410
- **JOERNVALL H. ; PERSSON B. ; KROOK M. ; ATRIAN S. ; GONZALEZ-DUARTE R. ; JEFFERY J. ; GHOSH D.** *Biochemistry,* 1995, vol. 34, 6003-6013
- **VILLARROYA A. ; JUAN E. ; EGESTAD B. ; JOERNVALL H.** *Eur. J. Biochem.,* 1989, vol. 180, 191-197
- **PERSSON B. ; KROOK M. ; JOERNVALL H.** *Eur. J. Biochem.,* 1991, vol. 200, 537-543
- **NEIDLE E.L. ; HARTNETT C. ; ORNSTON N.L. ; BAIROCH A. ; REKIK M. ; HARAYAMA S.** *Eur. J. Biochem.,* 1992, vol. 204, 113-120
- **BRANDEN C.-I. ; JOERNVALL H. ; EKLUND H. ; FURUGREN B.** The Enzymes. 1975, vol. 11, 104-190
- **JOERNVALL H. ; PERSSON B. ; JEFFERY J.** *Eur. J. Biochem.,* 1987, vol. 167, 195-201
- **SUN H.-W. ; PLAPP B.V.** *J. Mol. Evol.,* 1992, vol. 34, 522-535
- **PERSSON B. ; HALLBORN J. ; WALFRIDSSON M. ; HAHN-HAEGERDAL B. ; KERAENEN S. ; PENTTILAE M. ; JOERNVALL H.** *FEBS Lett.,* 1993, vol. 324, 9-14
- **KNIGHT M.E. ; HALPIN C. ; SCHUCH W.** *Plant Mol. Biol.,* 1992, vol. 19, 793-801
- **KOGA H. ; ARAMAKI H. ; YAMAGUCHI E. ; TAKEUCHI K. ; HORIUCHI T. ; GUNSALUS I.C.** *J. Bacteriol.,* 1986, vol. 166, 1089-1095
- **JOERNVALL H. ; PERSSON B. ; DU BOIS G. ; LAVERS G.C. ; CHEN J.H. ; GONZALEZ P. ; RAO P.V. ; ZIGLER J.S. JR.** *FEBS Lett.,* 1993, vol. 322, 240-244
- **HEMPEL J. ; HARPER K. ; LINDAHL R.** *Biochemistry,* 1989, vol. 28, 1160-1167
- **WERETILNYK E.A. ; HANSON A.D.** *Proc. Natl. Acad. Sci. U.S.A.,* 1990, vol. 87, 2745-2749
- **NIEGEMANN E. ; SCHULZ A. ; BARTSCH K.** *Arch. Microbiol.,* 1993, vol. 160, 454-460
- **HIDALGO E. ; CHEN Y.-M. ; LIN E.C.C. ; AGUILAR J.** *J. Bacteriol.,* 1991, vol. 173, 6118-6123
- **NORDLUND I. ; SHINGLER V.** *Biochim. Biophys. Acta,* 1990, vol. 1049, 227-230
- **STEELE M.I. ; LORENZ D. ; HATTER K. ; PARK A. ; SOKATCH J.R.** *J. Biol. Chem.,* 1992, vol. 267, 13585-13592
- **KRZYWICKI K.A. ; BRANDRISS M.C.** *Mol. Cell. Biol.,* 1984, vol. 4, 2837-2842
- **GUERRERO F.D. ; JONES J.T. ; MULLET J.E.** *Plant Mol. Biol.,* 1990, vol. 15, 11-26
- **COOK R.J. ; LLOYD R.S. ; WAGNER C.** *J. Biol. Chem.,* 1991, vol. 266, 4965-4973
- **BOHREN K.M. ; BULLOCK B. ; WERMUTH B. ; GABBAY K.H.** *J. Biol. Chem.,* 1989, vol. 264, 9547-9551
- **BRUCE N.C. ; WILLEY D.L. ; COULSON A.F.W. ; JEFFERY J.** *Biochem. J.,* 1994, vol. 299, 805-811
- **LARSON SB ; GREENWOOD A ; CASCIO D ; DAY J ; MCPHERSON A.** *J Mol Biol,* 1994, vol. 235, 1560-1584
- **BAIROCH A.** *Unpublished observations,* 1992
- **SUNG M.H. ; TANIZAWA K. ; TANAKA H. ; KURAMITSU S. ; KAGAMIYAMA H. ; HIROTSU K. ; OKAMOTO A. ; HIGUCHI T. ; SODA K.** *J. Biol. Chem.,* 1991, vol. 266, 2567-2572
- **BAIROCH A.** *Unpublished observations,* 1991
- **BAIROCH A.** *Unpublished observations,* 1992
- **YONAHA K. ; NISHIE M. ; AIBARA S.** *J. Biol. Chem.,* 1992, vol. 267, 12506-12510
- **HOLAK TA.** *FEBS Lett,* 1997, vol. 401, 127-132
- **LUX SE ; JOHN KM ; BENNETT V.** *Nature,* 1990, vol. 345, 736-739
- **GREEN J.M. ; MERKEL W.K. ; NICHOLS B.P.** *J. Bacteriol.,* 1992, vol. 174, 5317-5323
- **OUZOUNIS C. ; SANDER C.** *FEBS Lett.,* 1993, vol. 322, 159-164
- **VAN DER ZEL A ; LAM H.-M. ; WINKLER M.E.** *Nucleic Acids Res.,* 1989, vol. 17, 8379-8379
- **RAYNAL P. ; POLLARD H.B.** *Biochim. Biophys. Acta,* 1994, vol. 1197, 63-93
- **BARTON G.J. ; NEWMAN R.H. ; FREEMONT P.S. ; CRUMPTON M.J.** *Eur. J. Biochem.,* 1991, vol. 198, 749-760
- **BURGOYNE R.D. ; GEISOW M.J.** *Cell Calcium,* 1989, vol. 10, 1-10
- **HAIGLER H.T. ; FITCH J.M. ; JONES J.M. ; SCHLAEPFER D.D.** *Trends Biochem. Sci.,* 1989, vol. 14, 48-50
- **KLEE C.B.** *Biochemistry,* 1988, vol. 27, 6645-6653
- **SMITH P.D. ; MOSS S.E.** *Trends Genet.,* 1994, vol. 10, 241-246
- **HUBER R. ; ROEMISCH J. ; PAQUES E.-P.** *EMBO J.,* 1990, vol. 9, 3867-3874
- **FIEDLER K. ; SIMONS K.** *Trends Biochem. Sci.,* 1995, vol. 20, 177-178
- **BOMAN A.L. ; KAHN R.A.** *Trends Biochem. Sci.,* 1995, vol. 20, 147-150

- **MOSS J. ; VAUGHAN M.** *Cell. Signal.,* 1993, vol. 4
- **MOSS J. ; VAUGHAN M.** *Prog. Nucleic Acid Res. Mol. Biol.,* 1993, vol. 45, 47-65
- **AMOR J.C. ; HARRISON D.H. ; KAHN R.A. ; RINGE D.** *Nature,* 1994, vol. 372, 704-708
- **VALENCIA A. ; CHARDIN P. ; WITTINGHOFER A. ; SANDER C.** *Biochemistry,* 1991, vol. 30, 4637-4648
- **WALKER J.E. ; SARASTE M. ; RUNSWICK M.J. ; GAY N.J.** *EMBO J.,* 1982, vol. 1, 945-951
- **MOLLER W. ; AMONS R.** *FEBS Lett.,* 1985, vol. 186, 1-7
- **FRY D.C. ; KUBY S.A. ; MILDVAN A.S.** *Proc. Natl. Acad. Sci. U.S.A.,* 1986, vol. 83, 907-911
- **DEVER T.E. ; GLYNIAS M.J. ; MERRICK W.C.** *Proc. Natl. Acad. Sci. U.S.A.,* 1987, vol. 84, 1814-1818
- **SARASTE M. ; SIBBALD P.R. ; WITTINGHOFER A.** *Trends Biochem. Sci.,* 1990, vol. 15, 430-434
- **KOONIN E.V.** *J. Mol. Biol.,* 1993, vol. 229, 1165-1174
- **HODGMAN T.C.** *Nature,* 1988, vol. 333, 22-23
- *Nature,* 1988, vol. 333, 578-578
- **LINDER P. ; LASKO P. ; ASHBURNER M. ; LEROY P. ; NIELSEN P.J. ; NISHI K. ; SCHNIER J. ; SLONIMSKI P.P.** *Nature,* 1989, vol. 337, 121-122
- **GORBALENYA A.E. ; KOONIN E.V. ; DONCHENKO A.P. ; BLINOV V.M.** *Nucleic Acids Res.,* 1989, vol. 17, 4713-4730
- **OUZOUNIS C. ; KYRPIDES N.C.** *J. Mol. Evol.,* 1994, vol. 39, 101-104
- **JENKINSON C.P. ; GRODY W.W. ; CEDERBAUM S.D.** *Comp. Biochem. Physiol.,* vol. 114B, 107-132
- **KANYO Z.F. ; SCOLNICK L.R. ; ASH D.E. ; CHRISTIANSON D.W.** *Nature,* 1996, vol. 383, 554-557
- **FOLTMANN B.** *Essays Biochem.,* 1981, vol. 17, 52-84
- **DAVIES D.R.** *Annu. Rev. Biophys. Chem.,* 1990, vol. 19, 189-215
- **RAO J.K.M. ; ERICKSON J.W. ; WLODAWER A.** *Biochemistry,* 1991, vol. 30, 4663-4671
- **RAWLINGS N.D. ; BARRETT A.J.** *Meth. Enzymol.,* 1995, vol. 248, 105-120
- **WILSON KP ; SHEWCHUK LM ; BRENNAN RG ; OTSUKA AJ ; MATTHEWS BW.** *Proc Natl Acad Sci USA,* 1992, vol. 89, 9257-9261
- **ZOLLMAN S ; GODT D ; PRIVE GG ; COUDERC JL ; LASKI FA.** *Proc Natl Acad Sci U S A,* 1994, vol. 91, 10717-10721
- **BARDWELL VJ ; TREISMAN R.** *Genes Dev,* 1994, vol. 8, 1664-1677
- **AHMAD KF ; ENGEL CK ; PRIVE GG.** *Proc Natl Acad Sci U S A,* 1998, vol. 95, 12123-12128
- **DEWEINDT C ; ALBAGLI O ; BERNARDIN F ; DHORDAIN P ; QUIEF S ; LANTOINE D ; KERCKAERT JP ; LEPRINCE D.** *Cell Growth Differ,* 1995, vol. 6, 1495-1503
- **HUYNH KD ; BARDWELL VJ.** *Oncogene,* 1998, vol. 17, 2473-2484
- **WONG CW ; PRIVALSKY ML.** *J Biol Chem,* 1998, vol. 273, 27695-27702
- **SALMOND G.P.C. ; REEVES P.J.** *Trends Biochem. Sci.,* 1993, vol. 18, 7-12
- **REEVES P.J. ; WHITCOMBE D. ; WHARAM S. ; GIBSON M. ; ALLISON G. ; BUNCE N. ; BARALLON R. ; DOUGLAS P. ; MULHOLLAND V. ; STEVENS S.** *Mol. Microbiol.,* 1993, vol. 8, 443-456
- **MARTIN P.R. ; HOBBS M. ; FREE P.D. ; JESKE Y. ; MATTICK J.S.** *Mol. Microbiol.,* 1993, vol. 9, 857-868
- **HOBBS M. ; MATTICK J.S.** *Mol. Microbiol.,* 1993, vol. 10, 233-243
- **GENIN S. ; BOUCHER C.A.** *Mol. Gen. Genet.,* 1994, vol. 243, 112-118
- **WALTER DE GRUYTER.** Concise Encyclopedia Biochemistry. 1988
- **TAKAGI T.** *Curr. Opin. Struct. Biol.,* 1993, vol. 3, 413-418
- **COUTURE M. ; CHAMBERLAND H. ; ST-PIERRE B. ; LAFONTAINE J. ; GUERTIN M.** *Mol. Gen. Genet.,* 1994, vol. 243, 185-197
- **GALLAGHER P.G. ; FORGET B.G.** *J. Biol. Chem.,* 1995, vol. 270, 26358-26363
- **HUANG M. ; GU G. ; FERGUSON E.L. ; CHALFIE M.** *Nature,* 1995, vol. 378, 292-295
- **SVENSSON B. ; SVENDSEN I. ; HOEJRUP P. ; ROEPSTORFF P. ; LUDVIGSEN S. ; POULSEN F.M.** *Biochemistry,* 1992, vol. 31, 8767-8770
- **POTTER S. ; UKNES S. ; LAWTON K. ; WINTER A.M. ; CHANDLER D. ; DIMAIO J. ; NOVITZKY R. ; WARD E. ; RYALS J.** *Mol. Plant Microbe Interact.,* 1993, vol. 6, 680-685
- **LASKOWSKI M. ; KATO I.** *Annu. Rev. Biochem.,* 1980, vol. 49, 593-626
- **TASHIRO M. ; HASHINO K. ; SHIOZAKI M. ; IBUKI F. ; MAKI Z.** *J. Biochem.,* 1987, vol. 102, 297-306
- **BREITENEDER H. ; PETTENBURGER K. ; BITO A. ; VALENTA R. ; KRAFT D. ; RUMPOLD H. ; SCHEINER O. ; BREITENBACH M.** *EMBO J.,* 1989, vol. 8, 1935-1938
- **CROWELL D. ; JOHN M.E. ; RUSSELL D. ; AMASINO R.M.** *Plant Mol. Biol.,* 1992, vol. 18, 459-466
- **WARNER S.A.J. ; SCOTT R. ; DRAPER J.** *Plant Mol. Biol.,* 1992, vol. 19, 555-561

- **HURST H.C.** *Protein Prof.,* 1995, vol. 2, 105-168
- **ELLENBERGER T.** *Curr. Opin. Struct. Biol.,* 1994, vol. 4, 12-21
- **KNOWLES J.R.** *Annu. Rev. Biochem.,* 1989, vol. 58, 195-221
- **SAMOLS D. ; THRONTON C.G. ; MURTIF V.L. ; KUMAR G.K. ; HAASE F.C. ; WOOD H.G.** *J. Biol. Chem.,* 1988, vol. 263, 6461-6464
- **GOSS N.H. ; WOOD H.G.** *Meth. Enzymol.,* 1984, vol. 107, 261-278
- **SHENOY B.C. ; XIE Y. ; PARK V.L. ; KUMAR G.K. ; BEEGEN H. ; WOOD H.G. ; SAMOLS D.** *J. Biol. Chem.,* 1992, vol. 267, 18407-18412
- **YEAMAN S.J.** *Biochem. J.,* 1989, vol. 257, 625-632
- **YEAMAN S.J.** *Trends Biochem. Sci.,* 1986, vol. 11, 293-296
- **RUSSEL G.C. ; GUEST J.R.** *Biochim. Biophys. Acta,* 1991, vol. 1076, 225-232
- **FUJIWARA K. ; OKAMURA-IKEDA K. ; MOTOKAWA Y.** *J. Biol. Chem.,* 1986, vol. 261, 8836-8841
- **BEHAL R.H. ; BROWNING K.S. ; HALL T.B. ; REED L.J.** *Proc. Natl. Acad. Sci. U.S.A.,* 1989, vol. 86, 8732-8736
- **PRIEFERT H. ; HEIN S. ; KRUEGER N. ; ZEH K. ; SCHMIDT B. ; STEINBUECHEL A.** *J. Bacteriol.,* 1991, vol. 173, 4056-4071
- **PONTING CP ; PARKER PJ.** Medline: 96367095 Extending the C2 domain family: C2s in PKCs delta, epsilon, eta and theta, phospholipases, GAPs and perforin. *Protein Sci,* 1996, vol. 5, 162-166
- **AZZI A. ; BOSCOBOINIK D. ; HENSEY C.** *Eur. J. Biochem.,* 1992, vol. 208, 547-557
- **STABEL S.** *Semin. Cancer Biol.,* 1994, vol. 5, 277-284
- **BROSE N. ; HOFMANN K.O. ; HATA Y. ; SUEDHOF T.C.** *J. Biol. Chem.,* 1995, vol. 270, 25273-25280
- **SOSSIN W.S. ; SCHWARTZ J.H.** *Trends Biochem. Sci.,* 1993, vol. 18, 207-208
- **DAVLETOV B.A. ; SUEDHOF T.C.** *J. Biol. Chem.,* 1993, vol. 268, 26386-26390
- **FUKUDA M. ; ARUGA J. ; NIINOBE M. ; AIMOTO S. ; MIKOSHIBA K.** *J. Biol. Chem.,* 1994, vol. 269, 29206-29211
- **SUTTON R.B. ; DAVLETOV B.A. ; BERGHUIS A.M. ; SUEDHOF T.C. ; SPRANG S.R.** *Cell,* 1995, vol. 80, 929-938
- **KAWAMUKAI M. ; GERST J. ; FIELD J. ; RIGGS M. ; RODGERS L. ; WIGLER M. ; YOUNG D.** *Mol. Biol. Cell,* 1992, vol. 3, 167-180
- **YU G. ; SWISTON J. ; YOUNG D.** *J. Cell Sci.,* 1994, vol. 107, 1671-1678
- **RIEHEMANN K ; SORG C.** Medline: 93242656. Sequence homologies between four cytoskeleton-associated proteins. *Trends Biochem Sci,* 1993, vol. 18, 82-83
- **RIEHEMANN K. ; SORG C.** *Trends Biochem. Sci.,* 1993, vol. 18, 82-83
- **GILKES N.R. ; HENRISSAT B. ; KILBURN D.G. ; MILLER R.C. JR. ; WARREN R.A.J.** *Microbiol. Rev.,* 1991, vol. 55, 303-315
- **BATEMAN A.** Medline: 97172695 The structure of a domain common to archaebacteria and the homocystinuria disease protein. *Trends Biochem Sci,* 1997, vol. 22, 12-13
- **SINTCHAK MD ; FLEMING MA ; FUTER O ; RAYBUCK SA ; CHAMBERS SP ; CARON PR ; MURCKO MA ; WILSON KP.** Medline: 96279836 Structure and mechanism of inosine monophosphate dehydrogenase in complex with the immunosuppressant mycophenolic-acid. *Cell,* 1996, vol. 85, 921-930
- **PONTING CP.** Medline: 97259972 CBS domains in C1C chloride channels implicated in myotonia and nephrolithiasis (kidney stones. *J Mol Med,* 1997, vol. 75, 160-163
- **MOREIN S ; TROUARD TP ; HAUKSSON JB ; RILFORS L ; ARVIDSON G ; LINDBLOM G.** Medline: 97075020 Two-dimensional 1H-NMR of transmembrane peptides from Escherichia coli phosphatidylglycerophosphate synthase in micelles. *Eur J Biochem,* 1996, vol. 241, 489-497
- **NIKAWA J.-I. ; KODAKI T. ; YAMASHITA S.** *J. Biol. Chem.,* 1987, vol. 262, 4876-4881
- **JELMSTAD R.H. ; BELL R.M.** *J. Biol. Chem.,* 1991, vol. 266, 5094-5134
- **LI J ; VRIELINK A ; BRICK P ; BLOW DM.** Medline: 94032271. Crystal structure of cholesterol oxidase complexed with a steroid substrate: implications for flavin adenine dinucleotide dependent alcohol oxidases. *Biochemistry,* 1993, vol. 32, 11507-11515
- **CAVENER D.R.** *J. Mol. Biol.,* 1992, vol. 223, 811-814
- **HENIKOFF S. ; HENIKOFF J.G.** *Genomics,* 1994, vol. 19, 97-107
- **VAN BEILEN J.B. ; EGGINK G. ; ENEQUIST H. ; BOS R. ; WITHOLT B.** *Mol. Microbiol.,* 1992, vol. 6, 3121-3136
- **CHENG I.P. ; POULTON J.E.** *Plant Cell Physiol.,* 1993, vol. 34, 1139-1143
- **PARGE H.E. ; ARVAI A.S. ; MURTARI D.J. ; REED S.I. ; TAINER J.A.** *Science,* 1993, vol. 262, 387-395
- **ALLENDE J.E. ; ALLENDE C.C.** *FASEB J.,* 1995, vol. 9, 313-323
- **REED J.C. ; BIDWAI A.P. ; GLOVER C.V.C.** *J. Biol. Chem.,* 1994, vol. 269, 18192-18200

- **WANG J ; HARTLING JA ; FLANAGAN JM.** Medline: 98050920. The structure of ClpP at 2.3 angstroms resolution suggests a model for ATP-dependent proteolysis. *Cell,* 1997, vol. 91, 447-456
- **MAURIZI M.R. ; CLARK W.P. ; KIM S.-H. ; GOTTESMAN S.** *J. Biol. Chem.,* 1990, vol. 265, 12546-12552
- **GOTTESMAN S. ; MAURIZI M.R.** *Microbiol. Rev.,* 1992, vol. 56, 592-621
- **RAWLINGS N.D. ; BARRETT A.J.** *Meth. Enzymol.,* 1994, vol. 244, 19-61
- **YAU KW.** Medline: 94224763. Cyclic nucleotide-gated channels: an expanding new family of ion channels. *Proc Natl Acad Sci USA,* 1994, vol. 91, 3481-3483
- **WEBER LT. ; SHABB J.B. ; CORBIN J.D.** *Biochemistry,* 1989, vol. 28, 6122-6127
- **KAUPP U.B.** *Trends Neurosci.,* 1991, vol. 14, 150-157
- **SHABB J.B. ; CORBIN J.D.** *J. Biol. Chem.,* 1992, vol. 267, 5723-5726
- **MOGI T ; SAIKI K ; ANRAKU Y.** Medline: 95191390 Biosynthesis and functional role of haem O and haem A. *Mol Microbiol,* 1994, vol. 14, 391-398
- **NOBREGA M.P. ; NOBREGA F.G. ; TZAGOLOFF A.** *J. Biol. Chem.,* 1990, vol. 265, 14220-14226
- **CAO J. ; HOSLER J. ; SHAPLEIGH J. ; REVZIN A. ; FERGUSON-MILLER S.** *J. Biol. Chem.,* 1992, vol. 267, 24273-24278
- **CHEPURI V. ; GENNIS R.B.** *J. Biol. Chem.,* 1990, vol. 265, 12978-12986
- **TSUKIHARA T ; AOYAMA H ; YAMASHITA E ; TOMIZAKI T ; YAMAGUCHI H ; SHINZAWA-ITOH K ; NAKASHIMA R ; YAONO R ; YOSHIKAWA S.** Medline: 96216288 The whole structure of the 13-subunit oxidized cytochrome c oxidase at 2.8 A. *Science,* 1996, vol. 272, 1136-1144
- **CAPALDI R.A. ; MALATESTA F. ; DARLEY-USMAR V.M.** *Biochim. Biophys. Acta,* 1983, vol. 726, 135-148
- **RIZZUTO R. ; SANDONA D. ; BRINI M. ; CAPALDI R.A. ; BISSON R.** *Biochim. Biophys. Acta,* 1991, vol. 1129, 100-104
- **MYERS M. ; RICHMOND R.C. ; OAKESHOTT J.G.** *Mol. Biol. Evol.,* 1988, vol. 5, 113-119
- **KREJCI E. ; DUVAL N. ; CHATONNET A. ; VINCENS P. ; MASSOULIE J.** *Proc. Natl. Acad. Sci. U.S.A.,* 1991, vol. 88, 6647-6651
- **CYGLER M. ; SCHRAG J.D. ; SUSSMAN J.L. ; HAREL M. ; SILMAN I. ; GENTRY M.K. ; DOCTOR B.P.** *Protein Sci.,* 1993, vol. 2, 366-382
- **LOCKRIDGE O.** *BioEssays,* 1988, vol. 9, 125-128
- **WANG C.-S. ; HARTSUCK J.A.** *Biochim. Biophys. Acta,* 1993, vol. 1166, 1-19
- **WALDROP GL ; RAYMENT I ; HOLDEN HM.** Medline: 94347758 Three-dimensional structure of the biotin carboxylase subunit. of acetyl-CoA carboxylase. *Biochemistry,* 1994, vol. 33, 10249-10256
- **SIMMER JP ; KELLY RE ; RINKER AG JR ; SCULLY JL ; EVANS DR.** Medline: 90285162 Mammalian carbamyl phosphate synthetase (CPS). DNA sequence and evolution of the CPS domain of the Syrian hamster multifunctional protein CAD. *Biol Chem,* 1990, vol. 265, 10395-10402
- **SIMMER J.P. ; KELLY R.E. ; RINKER A.G. JR. ; SCULLY J.L. ; EVANS D.R.** *J. Biol. Chem.,* 1990, vol. 265, 10395-10402
- **DAVIDSON J.N. ; CHEN K.C. ; JAMISON R.S. ; MUSMANNO L.A. ; KERN C.B.** *BioEssays,* 1993, vol. 15, 157-164
- **SIMMER JP ; KELLY RE ; SCULLY JL ; EVANS DR.** *Biol Chem,* 1990, vol. 265, 10395-10402
- **SHA B ; PHILLIPS SE ; BANKAITIS VA ; LUO M.** Medline: 98121119 Crystal structure of the Saccharomyces cerevisiae phosphatidylinositol-transfer protein. *Nature,* 1998, vol. 391, 506-510
- **SCHINDELIN H ; JIANG W ; INOUYE M ; HEINEMANN U.** Medline: 94255482 Crystal structure of CspA, the major cold shock protein of Escherichia coli. *Proc Natl Acad Sci U S A,* 1994, vol. 91, 5119-5123
- **DONIGER J. ; LANDSMAN D. ; GONDA M.A. ; WISTOW G.** *New Biol.,* 1992, vol. 4, 389-395
- **WISTOW G.** *Nature,* 1990, vol. 344, 823-824
- **JONES P.G. ; INOUYE M.** *Mol. Microbiol.,* 1994, vol. 11, 811-818
- **LANDSMAN D.** *Nucleic Acids Res.,* 1992, vol. 20, 2861-2864
- **MERMOD N. ; O'NEILL E.A. ; KELLY T.J. ; TJIAN R.** *Cell,* 1989, vol. 58, 741-753
- **RUPP R.A.W. ; KRUSE U. ; MULTHAUP G. ; GOEBEL U. ; BEYREUTHER K. ; SIPPEL A.E.** *Nucleic Acids Res.,* 1990, vol. 18, 2607-2616
- **NAGATA K. ; GUGGENHEIMER R.A. ; ENOMOTO T. ; LICHY J.H. ; HURWITZ J.** *Proc. Natl. Acad. Sci. U.S.A.,* 1982, vol. 79, 6438-6442
- **SANTORO C. ; MERMOD N. ; ANDREWS P.C. ; TJIAN R.** *Nature,* 1988, vol. 334, 2118-2224
- **GIL G. ; SMITH J.R. ; GOLDSTEIN J.L. ; SLAUGHTER C.A. ; ORTH K. ; BROWN M.S. ; OSBORNE T.F.** *Proc. Natl. Acad. Sci. U.S.A,* 1988, vol. 85, 8963-8967
- **ALEVIZOPOULOS A. ; DUSSERRE Y. ; TSAI-PFLUGFELDER M. ; VON DER WEID T. ; WAHLI W. ; MERMOD N.** *Genes Dev.,* 1995, vol. 9, 3051-3066
- **TREVES S. ; VILSEN B. ; CHIOZZI P. ; ANDERSEN J.P. ; ZORZATO F.** *Biochem. J.,* 1992, vol. 283, 767-772

- **THORNTON CG ; KUMAR GK ; HAASE FC ; PHILLIPS NF ; WOO SB ; PARK VM ; MAGNER WJ ; SHENOY BC ; WOOD HG ; SAMOLS D.** *J Bacteriol,* 1993, vol. 175, 5301-5308
- **TOH H ; KONDO H ; TANABE T.** Medline: 93358891 Molecular evolution of biotin-dependent carboxylases. *Eur J Biochem,* 1993, vol. 215, 687-696
- **SCHROEDER J. ; SCHROEDER G.** *Z. Naturforsch,* 1990, vol. 45C, 1-8
- **LANZ T. ; TROPF S. ; MARNER F.-J. ; SCHROEDER J. ; SCHROEDER G.** *J. Biol. Chem.,* 1991, vol. 266, 9971-9976
- **SCHALLER A. ; SCHMID J. ; LEIBINGER U. ; AMRHEIN N.** *J. Biol. Chem.,* 1991, vol. 266, 21434-21438
- **JONES D.G.L. ; REUSSER U. ; BRAUS G.H.** *Mol. Microbiol.,* 1991, vol. 5, 2143-2152
- **KIRCHHAUSEN T. ; DAVIS A.C. ; FRUCHT S. ; O'BRINE GRECO B. ; PAYNE G.S. ; TUBB B.** *J. Biol. Chem.,* 1991, vol. 266, 11153-11157
- **NAKAI M. ; TAKADA T. ; ENDO T.** *Biochim. Biophys. Acta,* 1993, vol. 1174, 282-284
- **PHAN H.L. ; FINLAY J.A. ; CHU D.S. ; TAN P.K. ; KIRCHHAUSEN T. ; PAYNE G.S.** *EMBO J.,* 1994, vol. 13, 1706-1717
- **KUGE O. ; HARA-KUGE S. ; ORCI L. ; RAVAZZOLA M. ; AMHERDT M. ; TANIGAWA G. ; WIELAND F.T. ; ROTHMAN J.E.** *J. Cell Biol.,* 1993, vol. 123, 1727-1734
- **KEEN J.H.** *Annu. Rev. Biochem.,* 1990, vol. 59, 415-438
- **BRODSKY F.M.** *Science,* 1988, vol. 242, 1396-1402
- **BRODSKY F.M. ; HILL B.L. ; ACTON S.L. ; NAETHKE I. ; WONG D.H. ; PONNAMBALAM S. ; PARHAM P.** *Trends Biochem. Sci.,* 1991, vol. 16, 208-213
- **NATHKE IS ; HEUSER J ; LUPAS A ; STOCK J ; TURCK CW ; BRODSKY FM.** Medline: 92191269 Folding and trimerization of clathrin subunits at the triskelion hub. *Cell,* 1992, vol. 68, 899-910
- **KIRCHHAUSEN T ; HARRISON SC ; CHOW EP ; MATTALIANO RJ ; RAMACHANDRAN KL ; SMART J ; BROSIUS J.** Medline: 88097376 Clathrin heavy chain: molecular cloning and complete primary structure. *Proc Natl Acad Sci U S A,* 1987, vol. 84, 8805-8809
- **MAYNE R ; BREWTON RG.** Medline: 94059583 New members of the collagen superfamily. *Curr Opin Cell Biol,* 1993, vol. 5, 883-890
- **XU K. ; ELLIOTT T.** *J. Bacteriol.,* 1993, vol. 175, 4990-4999
- **KOHNO H. ; FURUKAWA T. ; YOSHINAGA T. ; TOKUNAGA R. ; TAKETANI S.** *J. Biol. Chem.,* 1993, vol. 268, 21359-21363
- **CAMADRO J.M. ; CHAMBON H. ; JOLLES J. ; LABBE P.** *Eur. J. Biochem.,* 1986, vol. 156, 579-587
- **XU K. ; ELLIOTT T.** *J. Bacteriol.,* 1994, vol. 176, 3196-3203
- **MOLENKAMP R ; SPAAN WJ.** Medline: 98087828 Identification of a specific interaction between the coronavirus mouse hepatitis virus A59 nucleocapsid protein and packaging signal. *Virology,* 1997, vol. 239, 78-86
- **MESSERSCHMIDT A ; HUBER R.** Medline: 90126844 The blue oxidases, ascorbate oxidase, laccase and ceruloplasmin. Modelling and structural relationships. *Eur J Biochem,* 1990, vol. 187, 341-352
- **KIPREOS E.T. ; LANDER L.E. ; WING J.P. ; HE W.W. ; HEDGECOCK E.M.** *Cell,* 1996, vol. 85, 829-839
- **BURNATOWSKA-HLEDIN M.A. ; SPIELMAN W.S. ; SMITH W.L. ; SHI P. ; MEYER J.M. ; DEWITT D.L.** *Am. J. Physiol.,* 1995, vol. 268, f1198-F1210
- **MATHIAS N. ; JOHNSON S.L. ; WINEY M. ; ADAMS A.E. ; GOETSCH L. ; PRINGLE J.R. ; BYERS B. ; GOEBL M.G.** *Mol. Cell. Biol.,* 1996, vol. 16, 6634-6643
- **KNOWLES P.F. ; DOOLEY D.M.** In) Metal ions in biological systems. Marcel Dekker, 1993, vol. 30, 361-403
- **PARSONS M.R. ; CONVERY M.A. ; WILMOT C.M. ; YADAV K.D.S. ; BLAKELEY V. ; CORNER A.S. ; PHILLIPS S.E.V. ; MCPHERSON M.J. ; KNOWLES P.F.** *Structure,* 1995, vol. 3, 1171-1184
- **DUFOUR E.** *Biochimie,* 1988, vol. 70, 1335-1342
- **KIRSCHKE H. ; BARRETT A.J. ; RAWLINGS N.D.** *Protein Prof.,* 1995, vol. 2, 1587-1643
- **SHI G.-P. ; CHAPMAN H.A. ; BHAIRI S.M. ; DELEEUW C. ; REDDY V.Y. ; WEISS S.J.** *FEBS Lett.,* 1995, vol. 357, 129-134
- **VELASCO G. ; FERRANDO A.A. ; PUENTE X.S. ; SANCHEZ L.M. ; LOPEZ-OTIN C.** *J. Biol. Chem.,* 1994, vol. 269, 27136-27142
- **CHAPOT-CHARTIER M.P. ; NARDI M. ; CHOPIN M.C. ; CHOPIN A. ; GRIPON J.C.** *Appl. Environ. Microbiol.,* 1993, vol. 59, 330-333
- **HIGGINS D.G. ; MCCONNELL D.J. ; SHARP P.M.** *Nature,* 1989, vol. 340, 604-604
- **RAWLINGS N.D. ; BARRETT A.J.** *Meth. Enzymol.,* 1994, vol. 244, 461-486
- **CLAUSEN T ; HUBER R ; LABER B ; POHLENZ HD ; MESSERSCHMIDT A.** Medline: 96428687 Crystal structure of the pyridoxal-5'-phosphate dependent cystathionine beta-lyase from Escherichia coli at 1.83 A. *J Mol Biol,* 1996, vol. 262, 202-224

- **CLAUSEN T ; HUBER R ; PRADE L ; WAHL MC ; MESSERSCHMIDT A.** Medline: 99059720 Crystal structure of Escherichia coli cystathionine gamma-synthase at 1.5 A resolution. *EMBO J,* 1998, vol. 17, 6827-6838
- **ONO B.I. ; TANAKA K. ; NAITO K. ; HEIKE C. ; SHINODA S. ; YAMAMOTO S. ; OHMORI S. ; OSHIMA T. ; TOH-E A.** *J. Bacteriol.,* 1992, vol. 174, 3339-3347
- **BARTON A.B. ; KABACK D.B. ; CLARK M.W. ; KENG T. ; OUELLETTE B.F.F. ; STORMS R.K. ; ZENG B. ; ZHONG W.W. ; FORTIN N. ; DELANEY S.** *Yeast,* 1993, vol. 9, 363-369
- **LU G ; CAMPBELL WH ; SCHNEIDER G ; LINDQVIST Y.** Medline: 95111952 Crystal structure of the FAD-containing fragment of corn nitrate reductase at 2.5 A resolution: relationship to other flavoprotein reductases. *Structure,* 1994, vol. 2, 809-821
- **HYDE GE ; CRAWFORD NM ; CAMPBELL WH.** Medline: 92084635 The sequence of squash NADH:nitrate reductase and its relationship to the sequences of other flavoprotein oxidoreductases. A family of flavoprotein pyridine nucleotide cytochrome reductases. *J Biol Chem,* 1991, vol. 266, 23542-23547
- **SPARROW C.P. ; RAETZ C.R.H.** *J. Biol. Chem.,* 1985, vol. 260, 12084-12091
- **SHEN H. ; HEACOCK P.N. ; CLANCEY C.J. ; DOWHAN W.** *J. Biol. Chem.,* 1996, vol. 271, 789-795
- **SAITO S. ; GOTO K. ; TONOSAKI A. ; KONDO H.** *J. Biol. Chem.,* 1997, vol. 272, 9503-9509
- **CLEMENT JM ; KENT C.** Medline: 10208837 CTP:Phosphocholine Cytidylyltransferase: Insights into Regulatory Mechanisms and Novel Functions. *Biochem Biophys Res Commun,* 1999, vol. 257, 643-650
- **WEBER I.T. ; SHABB J.B. ; CORBIN J.D.** *Biochemistry,* 1989, vol. 28, 6122-6127
- **TAKEICHI M.** *Annu. Rev. Biochem.,* 1990, vol. 59, 237-252
- **TAKEICHI M.** *Trends Genet.,* 1987, vol. 3, 213-217
- **MAHONEY P.A. ; WEBER U. ; ONOFRECHUK P. ; BIESSMANN H. ; BRYANT P.J. ; GOODMAN C.S.** *Cell,* 1991, vol. 67, 853-868
- **MICHALAK M. ; MILNER R.E. ; BURNS K. ; OPAS M.** *Biochem. J.,* 1992, vol. 285, 681-692
- **BERGERON J.J.M. ; BRENNER M.B. ; THOMAS D.Y. ; WILLIAMS D.B.** *Trends Biochem. Sci.,* 1994, vol. 19, 124-128
- **WATANABE D. ; YAMADA K. ; NISHINA Y. ; TAJIMA Y. ; KOSHIMIZU U. ; NAGATA A. ; NISHIMUNE Y.** *J. Biol. Chem.,* 1994, vol. 269, 7744-7749
- **DEUTSCH H.F.** *Int. J. Biochem.,* 1987, vol. 19, 101-113
- **FERNLEY R.T.** *Trends Biochem. Sci.,* 1988, vol. 13, 356-359
- **TASHIAN R.E.** *BioEssays,* 1989, vol. 10, 186-192
- **EDWARDS Y.** *Biochem. Soc. Trans.,* 1990, vol. 18, 171-175
- **SKAGGS L.A. ; BERGENHEM N.C.H. ; VENTA P.J. ; TASHIAN R.E.** *Gene,* 1993, vol. 126, 291-292
- **FUJIWARA S. ; FUKUZAWA H. ; TACHIKI A. ; MIYACHI S.** *Proc. Natl. Acad. Sci. U.S.A.,* 1990, vol. 87, 9779-9783
- **HUANG S. ; XUE Y. ; SAUER-ERIKSSON E. ; CHIRICA L. ; LINDSKOG S. ; JONSSON B.H.** *J. Mol. Biol.,* 1998, vol. 283, 301-310
- **HOLT C. ; SAWYER L.** *Protein Eng.,* 1988, vol. 2, 251-259
- **MURTHY M.R.N. ; REID T.J. III ; SICIGNANO A. ; TANAKA N. ; ROSSMANN M.G.** *J. Mol. Biol.,* 1981, vol. 152, 465-499
- **MELIK-ADAMYAN W.R. ; BARYNIN V.V. ; VAGIN A.A. ; BORISOV V.V. ; VAINSHTEIN B.K. ; FITA I. ; MURTHY M.R.N. ; ROSSMANN M.G.** *J. Mol. Biol.,* 1986, vol. 188, 63-72
- **VON OSSOWKI I. ; HAUSNER G. ; LOEWEN P.C.** *J. Mol. Evol.,* 1993, vol. 37, 71-76
- **WRIGHT H.T. ; SANDRASEGARAM G. ; WRIGHT C.S.** *J. Mol. Evol.,* 1991, vol. 33, 283-294
- **LERNER D.R. ; RAIKHEL N.V.** *J. Biol. Chem.,* 1992, vol. 267, 11085-11091
- **BUTLER A.R. ; O'DONNEL R.W. ; MARTIN V.J. ; GOODAY G.W. ; STARK M.J.R.** *Eur. J. Biochem.,* 1991, vol. 199, 483-488
- **FLACH J. ; PILET P.-E. ; JOLLES P.** *Experientia,* 1992, vol. 48, 701-716
- **HENRISSAT B.** *Biochem. J.,* 1991, vol. 280, 309-316
- **PARO R.** *Trends Genet.,* 1990, vol. 6, 416-421
- **SINGH P.B. ; MILLER J.R. ; PEARCE J. ; KOTHARY R. ; BURTON R.D. ; PARO R. ; JAMES T.C. ; GAUNT S.J.** *Nucleic Acids Res.,* 1991, vol. 19, 789-794
- **AASLAND R. ; STEWART A.F.** *Nucleic Acids Res.,* 1995, vol. 23, 3168-3173
- **KOONIN E.V. ; ZHOU S. ; LUCCHESIS J.C.** *Nucleic Acids Res.,* 1995, vol. 23, 4229-4233
- **KARPUSAS M. ; BRANCHAUD B. ; REMINGTON S.J.** *Biochemistry,* 1990, vol. 29, 2213-2219
- **GOTTESMAN S. ; SQUIRES C. ; PICHERSKY E. ; CARRINGTON M. ; HOBBS M. ; MATTICK J.S. ; DALRYMPLE B. ; KURAMITSU H. ; SHIROZA T. ; FOSTER T.** *Proc. Natl. Acad. Sci. U.S.A.,* 1990, vol. 87, 3513-3517
- **PARSELL D.A. ; SANCHEZ Y. ; STITZEL J.D. ; LINDQUIST S.** *Nature,* 1991, vol. 353, 270-273

- **LEONHARDT S.A. ; FEARON K. ; DANESE P.N. ; MASON T.L.** *Mol. Cell. Biol.,* 1993, vol. 13, 6304-6313
- **FEDOROV AA ; LAPPALAINEN P ; FEDOROV EV ; DRUBIN DG ; ALMO SC.** Medline: 97290449 Structure determination of yeast cofilin. *Nat Struct Biol,* 1997, vol. 4, 366-369
- **LEONARD SA ; GITTIS AG ; PETRELLA EC ; POLLARD TD ; LATTMAN EE.** Medline: 97290450 Crystal structure of the actin-binding protein actophorin from Acanthamoeba. *Nat Struct Biol,* 1997, vol. 4, 369-373
- **JIANG CJ ; WEEDS AG ; KHAN S ; HUSSEY PJ.** Medline: 97420794 F-actin and G-actin binding are uncoupled by mutation of conserved tyrosine residues in maize actin depolymerizing factor. *Proc Natl Acad Sci U S A,* 1997, vol. 94, 9973-9978
- **LAPPALAINEN P ; DRUBIN DG.** Medline: 97357155 Cofilin promotes rapid actin filament turnover in vivo. *Nature,* 1997, vol. 388, 78-82
- **HAWKINS M. ; POPE B. ; MACIVER S.K. ; WEEDS A.G.** *Biochemistry,* 1993, vol. 32, 9985-9993
- **IIDA K. ; MORIYAMA K. ; MATSUMOTO S. ; KAWASAKI H. ; NISHIDA E. ; YAHARA I.** *Gene,* 1993, vol. 124, 115-120
- **QUIRK S. ; MACIVER S.K. ; AMPE C. ; DOBERSTEIN S.K. ; KAISER D.A. ; VAN DAMME J. ; VANDEKERCKHOVE J. ; POLLARD T.D.** *Biochemistry,* 1993, vol. 32, 8525-8533
- **MCKIM K.S. ; MATHESON C. ; MARRA M.A. ; WAKARCHUK M.F. ; BAILLIE D.L.** *Mol. Gen. Genet.,* 1994, vol. 242, 346-357
- **MORIYAMA K. ; YONEZAWA N. ; SAKAI H. ; YAHARA I. ; NISHIDA E.** *J. Biol. Chem.,* 1992, vol. 267, 7240-7244
- **RAGAN C.I.** *Curr. Top. Bioenerg.,* 1987, vol. 15, 1-36
- **WEISS H. ; FRIEDRICH T. ; HOFHAUS G. ; PREIS D.** *Eur. J. Biochem.,* 1991, vol. 197, 563-576
- **FEAMLEY I.M. ; WALKER J.E.** *Biochim. Biophys. Acta,* 1992, vol. 1140, 105-134
- **WEIDNER U. ; GEIER S. ; PTOCK A. ; FRIEDRICH T. ; LEIF H. ; WEISS H.** *J. Mol. Biol.,* 1993, vol. 233, 109-122
- **GARRET T.P.J. ; CLINGELEFFER D.J. ; GUSS J.M. ; ROGERS S.J. ; FREEMAN H.C.** *J. Biol. Chem.,* 1984, vol. 259, 2822-2825
- **RYDEN L.G. ; HUNT L.T.** *J. Mol. Evol.,* 1993, vol. 36, 41-66
- **MCMANUS J.D. ; BRUNE D.C. ; HAN J. ; SANDERS-LOEHR J. ; MEYER T.E. ; CUSANOVICH M.A. ; TOLLIN G. ; BLANKENSHIP R.E.** *J. Biol. Chem.,* 1992, vol. 267, 6531-6540
- **MANN K. ; SCHAEFER W. ; THOENES U. ; MESSERSCHMIDT A. ; MEHRABIAN Z. ; NALBANDYAN R.** *FEBS Lett.,* 1992, vol. 314, 220-223
- **MATTAR S. ; SCHARF B. ; KENT S.B.H. ; RODEWALD K. ; OESTERHELT D. ; ENGELHARD M.** *J. Biol. Chem.,* 1994, vol. 269, 14939-14945
- **YANO T. ; FUKUMORI Y. ; YAMANAKA T.** *FEBS Lett.,* 1991, vol. 288, 159-162
- **ELLIS R.J. ; VAN DER VIES S.M.** *Annu. Rev. Biochem.,* 1991, vol. 60, 321-347
- **ZEILSTA-RYALLS J. ; FAYET O. ; GEORGOPOULOS C.** *Annu. Rev. Microbiol.,* 1991, vol. 45, 301-325
- **HARTMAN D.J. ; HOOGENRAAD N.J. ; CONDRON R. ; HOJ P.B.** *Proc. Natl. Acad. Sci. U.S.A.,* 1992, vol. 89, 3394-3398
- **BERTSCH U. ; SOLL J. ; SEETHARAM R. ; VIITANEN P.V.** *Proc. Natl. Acad. Sci. U.S.A.,* 1992, vol. 89, 8696-8700
- **HUNT J.F. ; WEAVER A.J. ; LANDRY S.J. ; GIERASCH L. ; DEISENHOFER J.** *Nature,* 1996, vol. 379, 37-45
- **ELLIS J.** *Nature,* 1992, vol. 358, 191-192
- **NELSON R.J. ; CRAIG E.A.** *Curr. Biol.,* 1992, vol. 2, 487-489
- **LEWIS V.A. ; HYNES G.M. ; ZHENG D. ; SAIBIL H. ; WILLISON K.R.** *Nature,* 1992, vol. 358, 249-252
- **KUBOTA H. ; HYNES G. ; CARNE A. ; ASHWORTH A. ; WILLISON K.R.** *Curr. Biol.,* 1994, vol. 4, 89-99
- **KIM S. ; WILLISON K.R. ; HORWICH A.L.** *Trends Biochem. Sci.,* 1994, vol. 20, 543-548
- **TRENT J.D. ; NIMMESGERN E. ; WALL J.S. ; HARTL F.U. ; HORWICH A.L.** *Nature,* 1991, vol. 354, 490-493
- **WALDMANN T. ; LUPAS A. ; KELLERMANN J. ; PETERS J. ; BAUMEISTER W.** *Biol. Chem. Hoppe-Seyler,* 1995, vol. 376, 119-126
- **HEMMINGSEN S.M.** *Nature,* 1992, vol. 357, 650-650
- **GIBSON TJ ; THOMPSON JD ; BLOCKER A ; KOUZARIDES T.** Medline: 94203808 Evidence for a protein domain superfamily shared by the cyclins, TFIIB and RB/p 107. *Nucleic Acids Res,* 1994, vol. 22, 946-952
- **BROWN NR ; NOBLE MEM ; ENDICOTT JA ; GARMAN EF ; WAKATSUKI S ; MITCHELL E ; RASMUSSEN B ; HUNT T ; JOHNSON LN.** Medline: 96164440 The crystal structure of cyclin A. *Structure,* 1995, vol. 3, 1235-1247
- **RUSSO AA ; JEFFREY PD ; PAVLETICH NP.** Medline: 96313126 Structural basis of cyclin-dependant kinase activation by phosphorylation. *Nat Struct Biol.,* 1996, vol. 3, 696-700
- **NURSE P.** *Nature,* 1990, vol. 344, 503-508
- **NORBURY C. ; NURSE P.** *Curr. Biol.,* 1991, vol. 1, 23-24
- **LEW D.J. ; REED S.I.** *Trends Cell Biol.,* 1992, vol. 2, 77-81

- **NICHOLAS J. ; CAMERON K.R. ; HONESS R.W.** *Nature,* 1992, vol. 355, 362-365
- **BARRETTA.J.** *Trends Biochem. Sci.,* 1987, vol. 12, 193-196
- **RAWLINGS N.D. ; BARRETT A.J.** *J. Mol. Evol.,* 1990, vol. 30, 60-71
- **TURK V. ; BODE W.** *FEBS Lett.,* 1991, vol. 285, 213-219
- **LUSTIGMAN S. ; BROTMAN B. ; HUIMA T. ; PRINCE A.M.** *Mol. Biochem. Parasitol.,* 1991, vol. 45, 65-76
- **MATHEWS F.S.** *Prog. Biophys. Mol. Biol.,* 1985, vol. 45, 1-56
- **SAKANE F ; YAMADA K ; KANOH H ; YOKOYAMA C ; TANABE T.** *Nature,* 1990, vol. 344, 345-348
- **SAKANE F ; IMAI S ; KAI M ; WADA I ; KANOH H.** *J Biol Chem,* 1996, vol. 271, 8394-8401
- **SCHAAP D ; DE WIDT J ; VAN DER WAL J ; VANDEKERCKHOVE J ; VAN, DAMME J ; GUSSOW D ; PLOEGH HL ; VAN BLITTERSWIJK WJ ; VAN DER, BEND RL.** *FEBS Lett,* 1990, vol. 275, 151-158
- **KANOH H ; YAMADA K ; SAKANE F.** *Trends Biochem Sci,* 1990, vol. 15, 47-50
- **NEGRI A. ; CECILIANI F. ; TEDESCHI G. ; SIMONIC T. ; RONCHI S.** *J. Biol. Chem.,* 1992, vol. 267, 11865-11871
- **MIYANO M. ; FUKUI K. ; WATANABE F. ; TAKAHASHI S. ; TADA M. ; KANASHIRO M. ; MIYAKE Y.** *J. Biochem.,* 1991, vol. 109, 171-177
- **SCHMID S.R. ; LINDER P.** *Mol. Microbiol.,* 1992, vol. 6, 283-292
- **WASSARMAN D.A. ; STEITZ J.A.** *Nature,* 1991, vol. 349, 463-464
- **HAROSH I. ; DESCHAVANNE P.** *Nucleic Acids Res.,* 1991, vol. 19, 6331-6331
- **KOONIN E.V. ; SENKEVICH T.G.** *J. Gen. Virol.,* 1992, vol. 73, 989-993
- **RICHTER G ; KRIEGER C ; VOLK R ; KIS K ; RITZ H ; GOTZE E ; BACHER A.** *Methods Enzymol,* 1997, vol. 280, 374-382
- **KANEKO T. ; HASHIMOTO T. ; KUMPAISAL R. ; YAMADA Y.** *J. Biol. Chem.,* 1990, vol. 265, 17451-17455
- **LABER B. ; GOMIS-RUETH F.-X. ; ROMAO M.J. ; HUBER R.** *Biochem. J.,* 1992, vol. 288, 691-695
- **MURPHY P.J. ; TRENZ S.P. ; GRZEMSKI W. ; DE BRUIJN F.J. ; SCHELL J.** *J. Bacteriol,* 1993, vol. 175, 5193-5204
- **NAGY M. ; LACROUTE F. ; THOMAS D.** *Proc. Natl. Acad. Sci. U.S.A.,* 1992, vol. 89, 8966-8970
- **POSFAI J. ; BHAGWAT A.S. ; ROBERTS R.J.** *Gene,* 1988, vol. 74, 261-263
- **KUMAR S. ; CHENG X. ; KLIMASAUSKAS S. ; MI S. ; POSFAI J. ; ROBERTS R.J. ; WILSON G.G.** *Nucleic Acids Res.,* 1994, vol. 22, 1-10
- **LAUSTER R. ; TRAUTNER T.A. ; NOYER-WEIDNER M.** *J. Mol. Biol.,* 1989, vol. 206, 305-312
- **CHEN L. ; MCMILLAN A.M. ; CHANG W. ; EZAK-NIPKAY K. ; LANE W.S. ; VERDINE G.L.** *Biochemistry,* 1991, vol. 30, 11018-11025
- **SANCAR G.B. ; SANCAR A.** *Trends Biochem. Sci.,* 1987, vol. 12, 259-261
- **JORNS M.S.** *Biofactors,* 1990, vol. 2, 207-211
- **YASUI A. ; EKER A.P.M. ; YASUHIRA S. ; YAJIMA H. ; KOBAYASHI T. ; TAKAO M. ; OIKAWA A.** *EMBO J.,* 1994, vol. 13, 6143-6151
- **YASUI A. ; EKER A.P.M. ; YASUHIRA S. ; YAJIMA H. ; KOBAYASHI T. ; TAKAO M. ; OIKAWA A.** *EMBO J.,* 1994, vol. 13, 6143-6151
- **LIN C. ; AHMAD M. ; CASHMORE A.R.** *Plant J.,* 1996, vol. 10, 893-902
- **DELARUE M. ; POCH O. ; TODRO N. ; MORAS D. ; ARGOS P.** *Protein Eng.,* 1990, vol. 3, 461-467
- **ITO J. ; BRAITHWAITE D.K.** *Nucleic Acids Res.,* 1991, vol. 19, 4045-4057
- **BRAITHWAITE D.K. ; ITO J.** *Nucleic Acids Res.,* 1993, vol. 21, 787-802
- **STERNGLANZ R.** *Curr. Opin. Cell Biol.,* 1990, vol. 1, 533-535
- **BJORNSTI M.-A.** *Curr. Opin. Struct. Biol.,* 1991, vol. 1, 99-103
- **SHARMA A. ; MONDRAGON A.** *Curr. Opin. Struct. Biol.,* 1995, vol. 5, 39-47
- **ROCA J.** *Trends Biochem. Sci.,* 1995, vol. 20, 156-160
- **FISCHER E.H. ; CHARBONNEAU H. ; TONKS N.K.** *Science,* 1991, vol. 253, 401-406
- **CHARBONNEAU H. ; TONKS N.K.** *Annu. Rev. Cell Biol.,* 1992, vol. 8, 463-493
- **TROWBRIDGE I.S.** *J. Biol. Chem.,* 1991, vol. 266, 23517-23520
- **TONKS N.K. ; CHARBONNEAU H.** *Trends Biochem. Sci.,* 1989, vol. 14, 497-500
- **HUNTER T.** *Cell,* 1989, vol. 58, 1013-1016
- **BAIROCH A.** *Unpublished observations,* 1995
- **OKA T. ; TSUJI H. ; NODA C. ; SAKAI K. ; HONG Y.-M. ; SUZUKI I. ; MUNOZ S. ; NATORI Y.** *J. Biol. Chem.,* 1995, vol. 270, 30060-30067
- **SCAPIN G. ; BLANCHARD J.S. ; SACCHETTINI J.C.** *Biochemistry,* 1995, vol. 34, 3502-3512
- **STUTZMAN-ENGWALL KJ ; OTTEN SL ; HUTCHINSON CR.** *J Bacteriol,* 1992, vol. 174, 144-154
- **KAESTNER K.H. ; NTAMBI J.M. ; KELLY T.J. JR. ; LANE M.D.** *J. Biol. Chem.,* 1989, vol. 264, 14755-14761

- **SHANKLIN J. ; SOMERVILLE C.R.** *Proc. Natl. Acad. Sci. U.S.A.,* 1991, vol. 88, 2510-2514
- **WADA H. ; GOMBOS Z. ; MURATA N.** *Nature,* 1990, vol. 347, 200-203
- **BROWN D.C. ; COLLINS K.D.** *J. Biol. Chem.,* 1991, vol. 266, 1597-1604
- **SOUCIET J.-L. ; NAGY M. ; LE GOUAR M. ; LACROUTE F. ; POTIER S.** *Gene,* 1989, vol. 79, 59-70
- **GUYONVARCH A. ; NGUYEN-JUILLERET M. ; HUBERT J.-C. ; LACROUTE F.** *Mol. Gen. Genet.,* 1988, vol. 212, 134-141
- **BUCKHOLZ R.G. ; COOPER T.G.** *Yeast,* 1991, vol. 7, 913-923
- **HAYASHI S. ; JAIN S. ; CHU R. ; ALVARES K. ; XU B. ; ERFURTH F. ; USUDA N. ; RAO M.S. ; REDDY S.K. ; NOGUCHI T.** *J. Biol. Chem.,* 1994, vol. 269, 12269-12276
- **CYR D.M. ; LANGER T. ; DOUGLAS M.G.** *Trends Biochem. Sci.,* 1994, vol. 19, 176-181
- **BORK P. ; SANDER C. ; VALENCIA A. ; BUKAU B.** *Trends Biochem. Sci.,* 1992, vol. 17, 129-129
- **UEGUCHI C. ; KANEDA M. ; YAMADA H. ; MIZUNO T.** *Proc. Natl. Acad. Sci. U.S.A.,* 1994, vol. 91, 1054-1058
- **YINGLING JM ; DAS P ; SAVAGE C ; ZHANG M ; PADGETT RW ; WANG XF.** *Proc Natl Acad Sci U S A,* 1996, vol. 93, 8940-8944
- **KIM J ; JOHNSON K ; CHEN HJ ; CARROLL S ; LAUGHON A.** *Nature,* 1997, vol. 388, 304-308
- **KING S.M. ; PATEL-KING R.S.** *J. Biol. Chem.,* 1995, vol. 270, 11445-11452
- **DICK T. ; RAY K. ; SALZ H.K ; CHIA W.** *Mol. Cell. Biol.,* 1996, vol. 16, 1966-1977
- **CEDERGREN-ZEPPEZAUER ES ; LARSSON G ; NYMAN PO ; DAUTER Z ; WILSON KS.** *Nature,* 1992, vol. 355, 740-743
- **MOL CD ; HARRIS JM ; MCINTOSH EM ; TAINER JA.** *Structure,* 1996, vol. 4, 1077-1092
- **YANG C. ; CARLOW D. ; WOLFENDEN R. ; SHORT S.A.** *Biochemistry,* 1992, vol. 31, 4168-4174
- **MOORE J.T. ; SILVERSMITH R.E. ; MALEY G.F. ; MALEY F.** *J. Biol. Chem.,* 1993, vol. 268, 2288-2291
- **REIZER J. ; BUSKIRK S. ; BAIROCH A. ; REIZER A. ; SAIER M.H. JR.** *Protein Sci.,* 1994, vol. 3, 853-856
- **BHATTACHARYA S. ; NAVARATNAM N. ; MORRISON J.R. ; SCOTT J. ; TAYLOW W.R.** *Trends Biochem. Sci.,* 1994, vol. 19, 105-106
- **CLOSE T.J. ; KORTT A.A. ; CHANDLER P.M.** *Plant Mol. Biol.,* 1989, vol. 13, 95-108
- **ROBERTSON M. ; CHANDLER P.M.** *Plant Mol. Biol.,* 1992, vol. 19, 1031-1044
- **DURE L. III ; CROUCH M. ; HARADA J. ; HO T.-H. D. ; MUNDY J. ; QUATRANO R. ; THOMAS T. ; SUNG Z.R.** *Plant Mol. Biol.,* 1989, vol. 12, 475-486
- **VON BODMAN S. ; HAYMAN G.T. ; FARRAND S.K.** *Proc. Natl. Acad. Sci. U.S.A.,* 1992, vol. 89, 643-647
- **BURD CG ; DREYFUSS G.** Medline: 94310455, Conserved structures and diversity of functions of RNA-binding proteins. *Science,* 1994, vol. 265, 615-621
- **VALLEE R.B ; SHPETNER H.S.** *Annu. Rev. Biochem.,* 1990, vol. 59, 909-932
- **OBAR R.A ; COLLINS C.A ; HAMMARBACK J.A ; SHPETNER H.S ; VALLEE R.B.** *Nature,* 1990, vol. 347, 256-261
- **VAN DER BLIEK A ; MEYEROWITZ E.M.** *Nature,* 1991, vol. 351, 411-414
- **ROTHMAN J.H ; RAYMOND C.K ; GILBERT T ; O'HARA P.J ; STEVENS T.H.** *Cell,* 1990, vol. 61, 1063-1074
- **JONES B.A ; FANGMAN W.L.** *Genes Dev.,* 1992, vol. 6, 380-389
- **ARNHEITER H ; MEIER E.** *New Biol.,* 1990, vol. 2, 851-857
- **STAEHELI P ; PITOSSI F ; PAVLOVIC J.** *Trends Cell Biol.,* 1993, vol. 3, 268-272
- **GREEN N.M ; MCLENNAN D.H.** *Biochem. Soc. Trans.,* 1989, vol. 17, 819-822
- **GREEN N.M.** *Biochem. Soc. Trans.,* 1989, vol. 17, 970-972
- **FAGAN M.J ; SAIER M.H. JR.** *J. Mol. Evol.,* 1994, vol. 38, 57-99
- **SERRANO R.** *Biochim. Biophys. Acta,* 1988, vol. 947, 1-28
- **FAMBROUGH D.M.** *Trends Neurosci.,* 1988, vol. 11, 325-328
- **SWEADNER K.J.** *Biochim. Biophys. Acta,* 1989, vol. 988, 185-220
- **BULL P.C ; COX D.W.** *Trends Genet.,* 1994, vol. 10, 246-251
- **SILVER S. ; NUCIFORA G. ; CHU L. ; MISRA T.K.** *Trends Biochem. Sci.,* 1989, vol. 14, 76-80
- **MINAMI-ISHII N. ; TAKETANI S. ; OSUMI T. ; HASHIMOTO T.** *Eur. J. Biochem.,* 1989, vol. 185, 73-78
- **MUELLER-NEWEN G. ; STOFFEL W.** *Biol. Chem. Hoppe-Seyler,* 1991, vol. 372, 613-624
- **PALOSAARI P.M ; HILTUNEN J.K.** *J. Biol. Chem.,* 1990, vol. 265, 2446-2449
- **NAKAHIGASHI K. ; INOKUCHI H.** *Nucleic Acids Res.,* 1990, vol. 18, 4937-4937
- **DRISCOLL J.R. ; TABER H.W.** *J. Bacteriol,* 1992, vol. 174, 5063-5071
- **BECKMAN D.L. ; KRANZ R.G.** *Gene,* 1991, vol. 107, 171-172

- **RIIS B. ; RATTAN I.S. ; CLARK B.F.C. ; MERRICK W.C.** *Trends Biochem. Sci.,* 1990, vol. 15, 420-424
- **VAN DAMME H.T.F. ; AMONS R. ; KARSSIES R. ; TIMMERS C.J. ; JANSSEN G.M.C. ; MOELLER W.** *Biochim. Biophys. Acta,* 1990, vol. 1050, 241-247
- **AOKI H. ; ADAMS S.-L ; TURNER M.A. ; GANOZA M.C.** *Biochimie,* 1997, vol. 79, 7-11
- **BUBUNENKO M.G. ; KIREEVA M.L ; GUDKOVA.T.** *Biochimie,* 1992, vol. 74, 419-425
- **KOSTRZEWA M. ; ZETSCHE K.** *Plant Mol. Biol.,* 1993, vol. 23, 67-76
- **XIN H. ; WORIAX V.L ; BURKHART W.A. ; SPREMULLI L.L.** *J. Biol. Chem.,* 1995, vol. 270, 17243-17249
- **PACCAUD JP ; THOMAS DY ; BERGERON JJ ; NILSSON T.** EMP24_GP25L) emp24/gp25L/p24 family Members of this family are implicated in bringing cargo forward from the ER and binding to coat proteins by their cytoplasmic domains. Number of members: 30. *J Cell Biol,* 1998, vol. 140, 751-765
- **LAI M.H ; BARD M ; PIERSON C.A ; ALEXANDER J.F ; GOEBL M. ; CARTER G.T ; KIRSCH D.R.** *Gene,* 1994, vol. 140, 41-49
- **YONEMURA S ; HIRAO M ; DOI Y ; TAKAHASHI N ; KONDO T ; TSUKITA S.** *J Cell Biol,* 1998, vol. 140, 885-895
- **PELHAM H.R.B.** *Curr. Opin. Cell Biol.,* 1991, vol. 3, 585-591
- **TOWNSLEY F.M. ; WILSON D.W. ; PELHAM H.R.B.** *EMBO J.,* 1993, vol. 12, 2821-2829
- **FINOCCHIARO G. ; IKEDA Y. ; ITO M. ; TANAKA K.** *Prog. Clin. Biol. Res.,* 1990, vol. 321, 637-652
- **TSAI M.H. ; SAIER M.H. JR.** *Res. Microbiol.,* 1995, vol. 146, 397-404
- **KUO C.-F ; MCREE D ; FISHER C.L ; O'HANDLEY S.F ; CUNNIGHAM R.P ; TAINER J.A.** *Science,* 1992, vol. 258, 434-440
- **THOMSON A.J.** *Curr. Biol.,* 1993, vol. 3, 173-174
- **ROLDAN-ARJONA T ; ANSELMINO C ; LINDAHL T.** *Nucleic Acids. Res.,* 1996, 3307-3312
- **NOELLING J. ; VAN EEDEN F.J.M. ; EGGEN R.I.L ; DE VOS W.M.** *Nucleic Acids Res.,* 1992, vol. 20, 6501-6507
- **THODEN JB ; HEGEMAN AD ; WESENBERG G ; CHAPEAU MC ; FREY PA ; HOLDEN HM.** *Biochemistry,* 1997, vol. 36, 6294-6304
- **KOONIN EV ; DEUTSCHER MP.** *Nucleic Acids Res,* 1993, vol. 21, 2521-2522
- **KAY BK ; YAMABHAI M ; WENDLAND B ; EMR SD.** Medline: 99156083, Identification of a novel domain shared by putative components of the endocytic and cytoskeletal machinery. *Protein Sci,* 1999, vol. 8, 435-438
- **WEI C.-L. ; KAINUMA M. ; HERSHEY J.W.B.** *J. Biol. Chem.,* 1995, vol. 270, 22788-22794
- **PARK M.H ; WOLFF E.C ; FOLK J.E.** *Biofactors,* 1993, vol. 4, 95-104
- **SCHNIER J. ; SCHWELBERGER H.G ; SMIT-MCBRIDE Z ; KANG H.A ; HERSHEY J.W.B.** *Mol. Cell. Biol.,* 1991, vol. 11, 3105-3114
- **BAUDIER J.** Calcium and Calcium Binding proteins. Springer Verlag, 1988, 102-113
- **MONCRIEF N.D ; KRETSINGER R.H. ; GOODMAN M.** *J. Mol. Evol.,* 1990, vol. 30, 522-562
- **KLIGMAN D. ; HILT D.C.** *Trends Biochem. Sci.,* 1988, vol. 13, 437-443
- **SCHAEFER B.W ; WICKI R. ; ENGELKAMP D. ; MATTEI M.-G ; HEIZMANN C.W.** *Genomics,* 1995, vol. 25, 638-643
- **LACKMANN M. ; CORNISH C.J ; SIMPSON R.J ; MORITZ R.L ; GECZY C.L.** *J. Biol. Chem.,* 1992, vol. 267, 7499-7504
- **NAKANO T ; GRAF T.** *Oncogene,* 1992, vol. 7, 527-534
- **LEE S.-C. ; KIM I.-G ; MAREKOV L.N ; O'KEEFE E.J ; PARRY D.A.D ; STEINERT P.M.** *J. Biol. Chem.,* 1993, vol. 268, 12164-12176
- **KAWASAKI H ; KRETSINGER R.H.** *Protein Prof.,* 1995, vol. 2, 305-490
- **KRETSINGER R.H.** *Cold Spring Harbor Symp. Quant. Biol.,* 1987, vol. 52, 499-510
- **MONCRIEF N.D ; KRETSINGER R.H ; GOODMAN M.** *J. Mol. Evol.,* 1990, vol. 30, 522-562
- **NAKAYAMA S ; MONCRIEF N.D ; KRETSINGER R.H.** *J. Mol. Evol.,* 1992, vol. 34, 416-448
- **HEIZMANN C.W ; HUNZIKER W.** *Trends Biochem. Sci.,* 1991, vol. 16, 98-103
- **KLIGMAN D. ; HILT D.C.** *Trends Biochem. Sci.,* 1988, vol. 13, 437-443
- **STRYNADKA N.C.J ; JAMES M.N.G.** *Annu. Rev. Biochem.,* 1989, vol. 58, 951-98
- **HAIECH J. ; SALLANTIN J.** *Biochimie,* 1985, vol. 67, 555-560
- **CHAUVAUX S. ; BEGUIN P. ; AUBERT J.-P ; BHAT K.M ; GOW L.A ; WOOD T.M ; BAIROCH A.** *Biochem. J.,* 1990, vol. 265, 261-265
- **BAIROCH A. ; COX J.A.** *FEBS Lett.,* 1990, vol. 269, 454-456
- **LEBIODA L. ; STEC B. ; BREWER J.M.** *J. Biol. Chem.,* 1989, vol. 264, 3685-3693
- **WISTOW G. ; PIATTIGORSKY J.** *Science,* 1987, vol. 236, 1554-1556
- **COOPER J.A ; CALDWELL J.E ; GATTERMEIR D.J ; TORRES M.A ; AMATRUDA J.F ; CASELLA J.F.** *Cell Motil. Cytoskeleton,* 1991, vol. 18, 204-214
- **BAI C ; SEN P ; HOFMANN K ; MA L ; GOEBL M ; HARPER JW ; ELLEDGE SJ.** *Cell,* 1996, vol. 86, 263-274

- **SKOWYRA D ; CRAIG KL ; TYERS M ; ELLEDGE SJ ; HARPER JW.** *Cell,* 1997, vol. 91, 209-219
- **AROLD S ; FRANKEN P ; STRUB M-P ; HOH F ; BENICHOU S ; BENAROUS R ; DUMAS C.** Medline: 98035457, The crystal structure of HIV-1 Nef protein bound to the Fyn kinase SH3 domain suggests a role for this complex in altered T cell receptor signalling. *Structure,* 1997, vol. 5, 1361-1372
- **BLAUT M. ; WHITTAKER K ; VALDOVINOS A ; ACKRELL B.A ; GUNSALUS R.P ; CECCHINI G.** *J. Biol. Chem.,* 1989, vol. 264, 13599-13604
- **BIRCH-MACHIN M.A ; FARNSWORTH L ; ACKRELL B.A ; COCHRAN B ; JACKSON S ; BINDOFF L.A ; AITKEN A ; DIAMOND A.G ; TURNBULL D.M.** *J. Biol. Chem.,* 1992, vol. 267, 11553-11558
- **KAESTNER K.H ; NTAMBI J.M ; KELLY T.J. JR ; LANE M.D.** *J. Biol. Chem.,* 1989, vol. 264, 14755-14761
- **SHANKLIN J ; SOMERVILLE C.R.** *Proc. Natl. Acad. Sci. U.S.A.,* 1991, vol. 88, 2510-2514
- **WADA H ; GOMBOS Z ; MURATA N.** *Nature,* 1990, vol. 347, 200-203
- **BENKOVIC S.J ; DEMAINE M.M.** *Adv. Enzymol.,* 1982, vol. 53, 45-82
- **RAINES C.A ; LLOYD J.C ; WILLINGHAM N.M ; POTTS S ; DYER T.A.** *Eur. J. Biochem.,* 1992, vol. 205, 1053-1059
- **KE H. ; THORPE C.M ; SEATON B.A ; LIPSCOMB W.N ; MARCUS F.** *J. Mol. Biol.,* 1989, vol. 212, 513-539
- **REIZER A ; DEUTSCHER J ; SAIER M.H. JR ; REIZER J.** *Mol. Microbiol.,* 1991, vol. 5, 1081-1089
- **TROPSCHUG M. ; WACHTER E. ; MAYER S. ; SCHOENBRUNNER E.R. ; SCHMID F.X.** *Nature,* 1990, vol. 346, 674-677
- **STEIN R.L.** *Curr. Biol.,* 1991, vol. 1, 234-236
- **SIEKIERKA J.J. ; WIDERRECHT G. ; GREULICH H. ; BOULTON D. ; HUNG S.H.Y. ; CRYAN J. ; HODGES P.J. ; SIGAL N.H.** *J. Biol. Chem.,* 1990, vol. 265, 21011-21015
- **FISCHER G. ; SCHMID F.X.** *Biochemistry,* 1990, vol. 29, 2205-2212
- **TRANDINH C.C. ; PAO G.M. ; SAIER M.H. JR.** *FASEB J.,* 1992, vol. 6, 3410-3420
- **GALAT A.** *Eur. J. Biochem.,* 1993, vol. 216, 689-707
- **HACKER J. ; FISCHER G.** *Mol. Microbiol.,* 1993, vol. 10, 445456
- **WUELFING C. ; LOMARDERO J. ; PLUECKTHUN A.** *J. Biol. Chem.,* 1994, vol. 269, 2895-2901
- **JAKOBSSON P.-J. ; MANCINI J.A. ; FORD-HUTCHINSON A.W.** *J. Biol. Chem.,* 1996, vol. 271, 22203-22210
- **GIEGEL D.A. ; WILLIAMS C.H. JR. ; MASSEY V.** *J. Biol. Chem.,* 1990, vol. 265, 6626-6632
- **TSOU A.Y ; RANSOM S.C ; GERLT J.A ; BUECHTER D.D ; BABBITT P.C ; KENYON G.L.** *Biochemistry,* 1990, vol. 29, 9856-9862
- **LE K.H.D ; LEDERER F.** *J. Biol. Chem.,* 1991, vol. 266, 20877-20880
- **LINDQVIST Y. ; BRANDEN C.-I.** *J. Biol. Chem.,* 1989, vol. 264, 3624-3628
- **SHANE B. ; GARROW T ; BRENNER A ; CHEN L. ; CHOI Y.J ; HSU J.C ; STOVER P.** *Adv. Exp. Med. Biol.,* 1993, vol. 338, 629-634
- **STENMARK H ; AASLAND R ; TOH BH ; D'ARRIGO A.** *J Biol Chem,* 1996, vol. 271, 24048-24054
- **GAULLIER JM ; SIMONSEN A ; D'ARRIGO A ; BREMNES B ; STENMARK H ; AASLAND R.** *Nature,* 1998, vol. 394, 432-433
- **AMATRUDA J.F. ; CANNON J.F. ; TATCHELL K. ; HUG C. ; COOPER J.A.** *Nature,* 1990, vol. 344, 352-354
- **MARTIN J.F.** *Trends Biotechnol.,* 1987, vol. 5, 306-308
- **CHEN G. ; SHIFFMAN D. ; MEVARECH M. ; AHARONOWITZ Y.** *Trends Biotechnol,* 1990, vol. 8, 105-111
- **SAMSON S.M ; DOTZLAF J.E ; SLISZ M.L ; BECKER G.W ; VAN FRANK R.M ; VEAL L.E ; YEH W.K ; MILLER J.R ; QUEENER S.W ; INGOLIA T.D.** *Bio/Technology,* 1987, vol. 5, 1207-1214
- **KOVACEVIC S. ; WEIGEL B.J ; TOBIN M.B ; INGOLIA T.D ; MILLER J.R.** *J. Bacteriol,* 1989, vol. 171, 754-760
- **ARIS J.P. ; BLOBEL G.** *Proc. Natl. Acad. Sci. U.S.A.,* 1991, vol. 88, 931-935
- **BANDZIULIS R.J ; SWANSON M.S ; DREYFUSS G.** *Genes Dev.,* 1989, vol. 3, 431-437
- **AGHA-AMIRI K.** *J. Bacteriol.,* 1994, vol. 176, 2124-2127
- **FUCINI P ; RENNER C ; HERBERHOLD C ; NOEGEL AA ; HOLAK TA.** *Nat Struct Biol,* 1997, vol. 4, 223-230
- **BRETON C ; ORIOL R ; IMBERTY A.** *Glycobiology,* 1998, vol. 8, 87-94
- **MEYER J.** *Trends Ecol. Evol.,* 1988, vol. 3, 222-226
- **HARAYAMA S. ; POLISSI A. ; REKIK M.** *FEBS Lett.,* 1991, vol. 285, 85-88
- **PETERSON J.A ; LU J.-Y ; GEISSELSODER J ; GRAHAM-LORENCE S ; CARMONA C ; WITNEY F ; LORENCE M.C.** *J. Biol. Chem.,* 1992, vol. 267, 14193-14203
- **NAGY I ; SCHOOFS G ; COMPERNOLLE F ; PROOST P ; VANDERLEYDEN J ; DE MOT R.** *J. Bacteriol.,* 1995, vol. 177, 676-687
- **TA D.T ; VICKERY L.E.** *J. Biol. Chem.,* 1992, vol. 267, 11120-11125
- **NAUD I ; VINCON M ; GARIN J ; GAILLARD J. ; FOREST E ; JOUANNEAU Y.** *Eur. J. Biochem.,* 1994, vol. 222, 933-939

- **MEYER J.** *Trends Ecol. Evol.,* 1988, vol. 3, 222-226
- **OTAKA E ; OOI T.** *J. Mol. Evol.,* 1987, vol. 26, 257-267
- **BEINERT H.** *FASEB J.,* 1990, vol. 4, 2483-2492
- **HUANG C.J ; BARRETT E.L.** *J. Bacteriol.,* 1991, vol. 173, 1544-1553
- **KNAFF D.B.** *Trends Biochem. Sci.,* 1988, vol. 13, 460-461
- **PAU R.N.** *Trends Biochem. Sci.,* 1989, vol. 14, 183-186
- **GEORGIADIS M.M ; KOMIYA H ; CHAKRABARTI P ; WOO D ; KORNUC J.J ; REES D.C.** *Science,* 1992, vol. 257, 1653-1659
- **FUJITA Y ; TAKAHASHI Y ; KOHCHI T ; OZEKI H ; OHYAMA K ; MATSUBARA H.** *Plant Mol. Biol.,* 1989, vol. 13, 551-561
- **BURKE D.H ; ALBERTI M ; HEARST J.E.** *J. Bacteriol,* 1993, vol. 175, 2407-2413
- **CRICHTON R.R ; CHARLOTEAUX-WAUTERS M.** *Eur. J. Biochem.,* 1987, vol. 164, 485-506
- **THEIL E.C.** *Annu. Rev. Biochem.,* 1987, vol. 56, 289-315
- **RAGLAND M ; BRIAT J.-F ; GAGNON J ; LAULHERE J.-P ; MASSENET O ; THEIL E.C.** *J. Biol. Chem.,* 1990, vol. 265, 18339-18344
- **QUINLAN R ; HUTCHISON C ; LANE B.** *Protein Prof.,* 1995, vol. 2, 801-952
- **STEINER P.M ; ROOP D.R.** *Annu. Rev. Biochem.,* 1988, vol. 57, 593-625
- **STEWART M.** *Curr. Opin. Cell Biol.,* 1990, vol. 2, 91-100
- **WAKABAYASHI S. ; KIMURA K. ; MATSUBARA H. ; ROGERS L.J.** *Biochem. J.,* 1989, vol. 263, 981-984
- **BAZAN J.F.** *Biochem. Biophys. Res. Commun.,* 1989, vol. 164, 788-795
- **BAZAN J.F.** *Proc. Natl. Acad. Sci. U.S.A.,* 1990, vol. 87, 6934-6938
- **COSMAN D ; LYMAN S.D ; IDZERDA R.L ; BECKMANN M.P ; PARK L.S ; GOODWIN R.G ; MARCH C.J.** *Trends Biochem.,* 1990, vol. 15, 265-270
- **D'ANDREA A.D ; FASMAN G.D ; LODISH H.F.** *Cell,* 1989, vol. 58, 1023-1024
- **D'ANDREA A.D ; FASMAN G.D ; LODISH H.F.** *Curr. Opin. Cell Biol.,* 1990, vol. 2, 648-651
- **INGLESE J ; SMITH J.M ; BENKOVIC S.J.** *Biochemistry,* 1990, vol. 29, 6678-6687
- **COOK R.J ; LLOYD R.S ; WAGNER C.** *J. Biol. Chem.,* 1991, vol. 266, 4965-4973
- **GUILLON J.-M ; MECHULAM Y ; SCHMITTER J.-M ; BLANQUET S ; FAYAT G.** *J. Bacteriol.,* 1992, vol. 174, 4294-4301
- **MCGREW L.L ; DWORKIN-RASTL E ; DWORKIN M.B ; RICHTER J.D.** *Genes Dev.,* 1989, vol. 3, 803-815
- **COLEMAN DE ; BERGHUIS AM ; LEE E ; LINDER ME ; GILMAN AG ; SPRANG SR.** *Science,* 1994, vol. 265, 1405-1412
- **COLEMAN DE ; SPRANG SR.** How G proteins work: a continuing story. *Trends Biochem Sci,* 1996, vol. 21, 41-44
- **JEFFERY J ; PERSSON B ; WOOD I ; BERGMAN T ; JEFFERY R ; JOERNVALL H.** *Eur. J. Biochem.,* 1993, vol. 212, 41-49
- **TRAINOR C.D ; EVANS T ; FELSENFELD G ; BOGUSKI M.S.** *Nature,* 1990, vol. 343, 92-96
- **LEE M.E ; TEMIZER D.T ; CLIFFORD J.A ; QUERTERMOUS T.** *J. Biol. Chem.,* 1991, vol. 266, 16188-16192
- **HO I.-C ; VORHEES P ; MARIN N ; OAKLEY B.K ; TSAI S.-F ; ORKIN S.H ; LEIDEN J.M.** *EMBO J.,* 1991, vol. 10, 1187-1192
- **SPIETH J ; SHIM Y.H ; LEA K ; CONRAD R ; BLUMENTHAL T.** *Mol. Cell. Biol.,* 1991, vol. 11, 4651-4659
- **DREVET J.R ; SKEIKY Y.A ; IATROU K.** *J. Biol. Chem.,* 1994, vol. 269, 10660-10667
- **HAWKINS M.G ; MCGHEE J.D.** *J. Biol. Chem.,* 1995, vol. 270, 14666-14671
- **VOISARD C.P.O ; WANG J ; XU P ; LEONG S.A ; MCEVOY J.L.** *Mol. Cell. Biol.,* 1993, vol. 13, 7091-7100
- **ARST H.N. JR ; KUDLA B ; MARTINEZ-ROSSI N.M ; CADDICK M.X ; SIBLEY S ; DAVIES R.W.** *Trends Genet.,* 1989, vol. 5, 291-291
- **FU Y.-H ; MARZLUF G.A.** *Mol. Cell. Biol.,* 1990, vol. 10, 1056-1065
- **BUCHANAN J.M.** *Adv. Enzymol.,* 1973, vol. 39, 91-183
- **WENG M ; ZALKIN H.** *J. Bacteriol,* 1987, vol. 169, 3023-3028
- **NYUNOYA H. ; LUSTY C.J.** *J. Biol. Chem.,* 1984, vol. 259, 9790-9798
- **CRAWFORD I.P.** *Annu. Rev. Microbiol.,* 1989, vol. 43, 567-600
- **ZALKIN H ; ARGOS P ; NARAYANA S.V.L ; TIEDEMAN A.A ; SMITH J.M.** *J. Biol. Chem.,* 1985, vol. 260, 3350-3354
- **DAVIDSON J.N ; CHEN K.C ; JAMISON R.S ; MUSMANNO L.A ; KERN C.B.** *BioEssays,* 1993, vol. 15, 157-164
- **TSO J.Y ; HERMODSON M.A ; ZALKIN H.** *J. Biol. Chem.,* 1980, vol. 255, 1451-1457
- **WENG M ; ZALKIN H.** *J. Bacteriol.,* 1987, vol. 169, 3023-3028
- **NYUNOYA H ; LUSTY C.J.** *J. Biol. Chem.,* 1984, vol. 259, 9790-9798
- **VAN HEEKE G ; SCHUSTER M.** *J. Biol. Chem.,* 1989, vol. 264, 5503-5509
- **VOLLMER S.J ; SWITZER R.L ; HERMODSON M.A ; BOWER S.G ; ZALKIN H.** *J. Biol. Chem.,* 1983, vol. 258, 10582-10585

- **SCHALK I ; ZENG K ; WU SK ; STURA EA ; MATTESON J ; HUANG M ; TANDON A ; WILSON IA ; BALCH WE.** *Nature,* 1996, vol. 381, 42-48
- **KINGSTON RL ; SCOPES RK ; BAKER EN.** *Structure,* 1996, vol. 4, 1413-1428
- **TSAY Y.H. ; ROBINSON G.W.** *Mol. Cell. Biol.,* 1991, vol. 11, 620-631
- **BRITTON K.L ; BAKER P.J ; RICE D.W ; STILLMAN T.J.** *Eur. J. Biochem.,* 1992, vol. 209, 851-859
- **BENACHENHOU-LAHFA N ; FORTERRE P ; LABEDAN B.** *J. Mol. Evol.,* 1993, vol. 36, 335-346
- **NAGATA S ; TANIZAWA K ; ESAKI N ; SAKAMOTO Y ; OHSHIMA T ; TANAKA H ; SODA K.** *Biochemistry,* 1988, vol. 27, 9056-9062
- **TAKADA H ; YOSHIMURA T ; OHSHIMA T ; ESAKI N ; SODA K.** *J. Biochem.,* 1991, vol. 109, 371-376
- **HUTCHINSON C.R ; TANG L.** *J. Bacteriol,* 1993, vol. 175, 4176-4185
- **CAVENER D.R.** *J. Mol. Biol.,* 1992, vol. 223, 811-814
- **HENIKOFF S ; HENIKOFF J.G.** *Genomics,* 1994, vol. 19, 97-107
- **VAN BEILEN J.B ; EGGINK G ; ENEQUIST H ; BOS R ; WITHOLT B.** *Mol. Microbiol,* 1992, vol. 6, 3121-3136
- **CHENG I.P ; POULTON J.E.** *Plant Cell Physiol.,* 1993, vol. 34, 1139-1143
- **WENG M ; ZALKIN H.** *J. Bacteriol.,* 1987, vol. 169, 3023-3028
- **NYUNOYA H ; LUSTY C.J.** *J. Biol. Chem.,* 1984, vol. 259, 9790-9798
- **DAVIDSON J.N ; CHEN K.C ; JAMISON R.S ; MUSMANNO L.A ; KERN C.B.** *BioEssays,* 1993, vol. 15, 157-164
- **MANNERVIK B.** *Meth. Enzymol.,* 1985, vol. 113, 490-495
- **MULLENBACH G.T ; TABRIZI A ; IRVINE B.D ; BELL G.I ; TAINER J.A ; HALLEWELL R.A.** *Protein Eng.,* 1988, vol. 2, 239-246
- **CHU F.F ; DOROSHOW J.H ; ESWORTHY R.S.** *J. Biol. Chem.,* 1993, vol. 268, 2571-2576
- **TAKAHASHI K ; AKASAKA M. ; YAMAMOTO Y ; KOBAYASHI C ; MIZOGUCHI J ; KOYAMA J.** *J. Biochem.,* 1990, vol. 108, 145-148
- **DUNN D.K ; HOWELLS D.D ; RICHARDSON J ; GOLDFARB P.S.** *Nucleic Acids Res.,* 1989, vol. 17, 6390-6390
- **COOKSON E. ; BLAXTER M.L ; SELKIRK M.E.** *Proc. Natl. Acad. Sci. U.S.A.,* 1992, vol. 89, 5837-5841
- **STADTMAN T.C.** *Annu. Rev. Biochem.,* 1990, vol. 59, 111-127
- **SAZUKA T ; TOMOOKA Y ; IKAWA Y ; NODA M ; KUMAR S.** *Biochem. Biophys. Res. Commun.,* 1992, vol. 189, 363-370
- **HUDSON J.D ; YOUNG P.G.** *Gene,* 1993, vol. 125, 191-193
- **BOURNE H.R ; SANDERS D.A ; MCCORMICK F.** *Nature,* 1991, vol. 349, 117-127
- **WALKER J.E ; SARASTE M ; RUNSWICK M.J ; GAY N.J.** *EMBO J.,* 1982, vol. 1, 945-951
- **MOLLER W ; AMONS R.** *FEBS Lett.,* 1985, vol. 186, 1-7
- **FRY D.C ; KUBY S.A ; MILDVAN A.S.** *Proc. Natl. Acad. Sci. U.S.A.,* 1986, vol. 83, 907-911
- **DEVER T.E ; GLYNIAS M.J ; MERRICK W.C.** *Proc. Natl. Acad. Sci. U.S.A.,* 1987, vol. 84, 1814-1818
- **SARASTE M ; SIBBALD P.R ; WITTINGHOFER A.** *Trends Biochem. Sci.,* 1990, vol. 15, 430-434
- **KOONIN E.V.** *J. Mol. Biol.,* 1993, vol. 229, 1165-1174
- **HIGGINS C.F ; HYDE S.C ; MIMMACK M.M ; GILEADI U ; GILL D.R ; GALLAGHER M.P.** *J. Bioenerg. Biomembr.,* 1990, vol. 22, 571-592
- **LINDER P. ; LASKO P ; ASHBURNER M ; LEROY P ; NIELSEN P.J ; NISHI K ; SCHNIER J ; SLONIMSKI P.P.** *Nature,* 1989, vol. 337, 121-122
- **GORBALENYA A.E ; KOONIN E.V ; DONCHENKO A.P ; BLINOV V.M.** *Nucleic Acids Res.,* 1989, vol. 17, 4713-4730
- Concise Encyclopedia Biochemistry. Walter de Gruyter, 1988
- **MOLDAVE K.** *Annu. Rev. Biochem.,* 1985, vol. 54, 1109-1149
- **STANSFIELD I ; JONES K.M ; KUSHNIROV V.V ; DAGKESAMANSKAYA A.R ; POZNYAKOVSKI A.I ; PAUSHKIN S.V ; NIERRAS C.R ; COX B.S ; TER-AVANESYAN M.D ; TUITE M.F.** *EMBO J.,* 1995, vol. 14, 4365-4373
- **GRENTZMANN G ; BRECHEMIER-BAEY D ; HEURGUE-HAMARD V ; BUCKINGHAM R.H.** *J. Biol. Chem.,* 1995, vol. 270, 10595-10600
- **NELSON R.J ; ZIEGELHOFFER T ; NICOLET C ; WERNER-WASHBURNE M ; CRAIG E.A.** *Cell,* 1992, vol. 71, 97-105
- **ANN D.K ; MOUTSATSOS I.K ; NAKAMURA T ; LIN H.H ; MAO P.-L ; LEE M.-J ; CHIN S ; LIEM R.K.H ; WANG E.** *J. Biol. Chem.,* 1991, vol. 266, 10429-10437
- **FORCHAMMER K. ; LEINFELDR W ; BOCK A.** *Nature,* 1989, vol. 342, 453-456
- **MANAVATHU E.K ; HIRATSUKA K. ; TAYLOR D.E.** *Gene,* 1988, vol. 62, 17-26
- **LEBLANC D.J ; LEE L.N ; TITMAS B.M ; SMITH C.J ; TENOVER F.C.** *J. Bacteriol,* 1988, vol. 170, 3618-3626
- **CERVANTES E ; SHARMA S.B ; MAILLET F ; VASSE J ; TRUCHET G. ; ROSENBERG C.** *Mol. Microbiol.,* 1989, vol. 3, 745-755
- **PLUNKETT G. III ; BURLAND V.D ; DANIELS D.L ; BLATTNER F.R.** *Nucleic Acids Res.,* 1993, vol. 21, 3391-3398

- **MOLLER W ; SCHIPPER A ; AMONS R.** *Biochimie,* 1987, vol. 69, 983-989
- **RICHTER G ; RITZ H ; KATZENMEIER G ; VOLK R ; KOHNLE A ; LOTTSPEICH F ; ALLENDORF D ; BACHER A.** *J Bacteriol,* 1993, vol. 175, 4045-4051
- **REICHARDT J.K.V ; BERG P.** *Nucleic Acids Res.,* 1988, vol. 16, 9017-9026
- **MOLLET B. ; PILLOUD N.** *J. Bacteriol,* 1991, vol. 173, 4464-4473
- **BRUIX M ; JIMENEZ M.A ; SANTORO J. ; GONZALEZ C. ; COLILLA F.J ; MENDEZ E ; RICO M.** *Biochemistry,* 1993, vol. 32, 715-724
- **GU Q ; KAWATA E.E ; MORSE M.-J ; WU H.-M ; CHEUNG A.Y.** *Mol. Gen. Genet.,* 1992, vol. 234, 89-96
- **TERRAS F.R.G ; TORREKENS S ; VAN LEUVEN F ; OSBORN R.W ; VANDERLEYDEN J ; CAMMUE B.P.A ; BROEKAERT W.F.** *FEBS Lett,* 1993, vol. 316, 233-240
- **BLOCH C. JR ; RICHARDSON M.** *FEBS Lett.,* 1991, vol. 279, 101-104
- **ISHIBASHI N ; YAMAUCHI D ; MINIAMIKAWA T.** *Plant Mol. Biol.,* 1990, vol. 15, 59-64
- **CHOI Y ; CHOI Y.D ; LEE J.S.** *Plant Physiol.,* 1993, vol. 101, 699-700
- **BURTNICK LD ; KOEPF EK ; GRIMES J ; JONES EY ; STUART DI ; MCLAUGHLIN PJ ; ROBINSON RC.** Medline: 97433077. The crystal structure of plasma gelsolin: implications for actin severing, capping, and nucleation. *Cell,* 1997, vol. 90, 661-670
- **LANE B.G.** *FASEB J,* 1994, vol. 8, 294-301
- **JAHN D ; VERKAMP E ; SOELL D.** *Trends Biochem. Sci.,* 1992, vol. 17, 215-218
- **ABDULLAH K.M ; LO R.Y.C ; MELLORS A.** *J. Bacteriol.,* 1991, vol. 173, 5597-5603
- **RAWLINGS N.D ; BARRETT A.J.** *Meth. Enzymol.,* 1995, vol. 248, 183-228
- **OLIVA G ; FONTES M.R.M ; GARRATT R.C ; ALTAMIRANO M.M ; CALCAGNO M.L ; HORJALES E.** *Structure,* 1995, vol. 3, 1323-1332
- **REIZER J ; RAMSEIER T.M ; REIZER A ; CHARBIT A ; SAIER M.H. JR.** *Microbiology,* 1996, vol. 142, 231-250
- **JOHNSON PR ; SPRIGGS MK ; OLMSTED RA ; COLLINS PL.** *Proc Natl Acad Sci U S A,* 1987, vol. 84, 5625-5629
- **WALTER M.R ; COOK W.J ; COLE L.B ; SHORT S.A ; KOSZALKA G.W ; KRENITSKY T.A ; EALICK S.E.** *J. Biol. Chem.,* 1990, vol. 265, 14016-14022
- **FURUKAWA T ; YOSHIMURA A ; SUMIZAWA T ; HARAGUCHI M ; AKIYAMA S.-I ; FUKUI K ; YAMADA Y.** *Nature,* 1992, vol. 356, 668-668
- **SAXILD H.H ; ANDERSEN L.N ; HAMMER K.** *J. Bacteriol,* 1996, vol. 178, 424-434
- **LEHRMAN MA.** Medline: 95252686. A family of UDP-GlcNAc/MurNAc: polyisoprenol-P GlcNAc/MurNAc-1-P transferases. *Glycobiology,* 1994, vol. 4, 768-771
- **HENRISSATB.** *Biochem. J.,* 1991, vol. 280, 309-316
- **JUNCOSA M ; PONS J ; DOT T ; QUEROL E ; PLANAS A.** *J. Biol. Chem.,* 1994, vol. 269, 14530-14535
- **HENRISSATB.** *Biochem. J.,* 1991, vol. 280, 309-316
- **ORI N ; SESSA G ; LOTAN T ; HIMMELHOCH S ; FLUHR R.** *EMBO J.,* 1990, vol. 9, 3429-3436
- **VARGHESE J.N ; GARRETT T.P.J ; COLMAN P.M ; CHEN L ; HOJ P.J ; FINCHER G.B.** *Proc. Natl. Acad. Sci. U.S.A.,* 1994, vol. 91, 2785-2789
- **KIM N.-S ; UMEZAWA Y ; OHMURA S ; KATO S.** *J. Biol. Chem.,* 1993, vol. 268, 11217-11221
- **WEKSBERG R ; SQUIRE J.A ; TEMPLETON D.M.** *Nat. Genet.,* 1996, vol. 12, 225-227
- **WATANABE K ; YAMADA H ; YAMAGUCHI Y.** *J. Cell Biol.,* 1995, vol. 130, 1207-1218
- **HUTTNER W.B ; GERDES H.-H ; ROSA P.** *Trends Biochem. Sci.,* 1991, vol. 16, 27-30
- **SIMON J.-P ; AUNIS D.** *Biochem. J.,* 1989, vol. 262, 1-13
- **GEORGOPOULOS C ; WELCH W.** *Annu. Rev. Cell Biol.,* 1993, vol. 9, 601-635
- **BOLLIGER L ; DELOCHE O ; GLICK B.S ; GEORGOPOULOS C ; JENOE P ; KRONIDOU N ; HORST M ; MORISHIMA N ; SCHATZ G.** *EMBO J.,* 1994, vol. 13, 1998-2006
- **STEHLE T ; SCHULZ G.E.** *J. Mol. Biol.,* 1992, vol. 224, 1127-1141
- **BRYANT P.J ; WOODS D.F.** *Cell,* 1992, vol. 68, 621-622
- **GOEBL M.G.** *Trends Biochem. Sci.,* 1992, vol. 17, 99-99
- **ZSCHOCKE P.D ; SCHILTZ E ; SCHULZ G.E.** *Eur. J. Biochem.,* 1993, vol. 213, 263-269
- **WOODS D.F ; BRYANT P.J.** *Mech. Dev.,* 1994, vol. 44, 85-89
- **TARON C.H ; BENNER J.S ; HORNSTRA L.J ; GUTHRIE E.P.** *Glycobiology,* 1995, vol. 5, 603-610
- **CAREY A.T ; HOLT K ; PICARD S ; WILDE R ; TUCKER G.A ; BIRD C.R ; SCHUCH W ; SEYMOUR G.B.** *Plant Physiol.,* 1995, vol. 108, 1099-1107
- **HENRISSAT B ; BAIROCH A.** *Biochem. J.,* 1993, vol. 293, 781-788
- **HENRISSAT B ; CALLEBAUT I ; FABREGA S ; LEHN P ; MORNON J.-P ; DAVIES G.** *Proc. Natl. Acad. Sci. U.S.A.,* 1995, vol. 92, 7090-7094

- **CASTLE L.A ; SMITH K.D ; MORRIS R.O.** *J. Bacteriol,* 1992, vol. 174, 1478-1486
- **BAUSE E ; LEGLER G.** *Biochim. Biophys. Acta,* 1980, vol. 626, 459-465
- **RUTTOWSKI E ; LABITZKE R ; KHANH N.Q ; LOEFFLER F ; GOTTSCHALK M ; JANY K.-D.** *Biochim. Biophys. Acta,* 1990, vol. 1087, 104-106
- **HUANG J. ; SCHELL M.A.** *J. Bacteriol.,* 1990, vol. 172, 3879-3887
- **HE S.Y ; COLLMER A.** *J. Bacteriol,* 1990, vol. 172, 4988-4995
- **BUSSINK H.J.D ; BUXTON F.P ; VISSER J.** *Curr. Genet.,* 1991, vol. 19, 467-474
- **HENRISSAT B.** *Biochem. J.,* 1991, vol. 280, 309-316
- **GUNASEKARAN P ; KARUNAKARAN T ; CAMI B ; MUKUNDAN A.G ; PREZIOSI L ; BARATTI J.** *J. Bacteriol.,* 1990, vol. 172, 6727-6735
- **REDDY V.A ; MALEY F.** *J. Biol. Chem.,* 1990, vol. 265, 10817-10120
- **HENRISSAT B.** *Protein Seq. Data Anal.,* 1991, vol. 4, 61-62
- **GONZALEZ-CANDELAS L ; RAMON D ; POLAINA J.** *Gene,* 1990, vol. 95, 31-38
- **EL HASSOUNI M ; HENRISSAT B ; CHIPPAUX M ; BARRAS F.** *J. Bacteriol.,* 1992, vol. 174, 765-777
- **WITHERS S.G ; WARREN R.A.J ; STREET I.P ; RUPITZ K ; KEMPTON J.B ; AEBERSOLD R.** *J. Am. Chem. Soc.,* 1990, vol. 112, 5887-5889
- **BEGUIN P.** *Annu. Rev. Microbiol.,* 1990, vol. 44, 219-248
- **GILKES N.R ; HENRISSAT B ; KILBURN D.G ; MILLER R.C. JR ; WARREN R.A.** *J. Microbiol. Rev.,* 1991, vol. 55, 303-315
- **HENRISSAT B ; CLAEYSSENS M. ; TOMME P ; LEMESLE L ; MORNON J.-P.** *Gene,* 1989, vol. 81, 83-95
- **TOMME P ; CHAUVAUX S ; BEGUIN P ; MILLET J ; AUBERT J.-P ; CLAEYSSENS M.** *J. Biol. Chem.,* 1991, vol. 266, 10313-10318
- **TOMME P ; VAN BEEUMEN J ; CLAEYSSENS M.** *Biochem. J.,* 1992, vol. 285, 319-324
- **POZSGAY JM ; BEILHARZ MW ; WINES BD ; HESS AD ; PITHA PM.** *J Virol,* 1993, vol. 67, 5989-5999
- **EISENBERG D ; ALMASSY R.J ; JANSON C.A ; CHAPMAN M.S ; SUH S.W ; CASCIO D ; SMITH W.W.** *Cold Spring Harbor Symp. Quant. Biol.,* 1987, vol. 52, 483-490
- **KUMADA Y ; BENSON D.R ; HILLEMANN D ; HOSTED T.J ; ROCHEFORT D.A ; THOMPSON C.J ; WOHLLEBEN W ; TATENO Y.** *Proc. Natl. Acad. Sci. U.S.A.,* 1993, vol. 90, 3009-3013
- **SHATTERS R.G ; KAHN M.L.** *J. Mol. Evol.,* 1989, vol. 29, 422-428
- **BROWN J.R ; MASUCHI Y ; ROBB F.T ; DOOLITTLE W.F.** *J. Mol. Evol.,* 1994, vol. 38, 566-576
- **GOODMAN M ; PEDWAYDON J ; CZELUSNIAK J. ; SUZUKI T ; GOTOH T ; MOENS L ; SHISHIKURA F ; WALZ D ; VINOGRADOV S.** *J. Mol. Evol.,* 1988, vol. 27, 236-249
- **POWELL R ; CANNON F.** *BioEssays,* 1988, vol. 9, 117-121
- **KORTT A.A ; TRINICK M.J ; APPLEBY C.A.** *Eur. J. Biochem.,* 1988, vol. 175, 141-149
- **KORTT A.A ; INGLIS A.S ; FLEMING A.I ; APPLEBY C.A.** *FEBS Lett.,* 1988, vol. 231, 341-346
- **BOGUSZ D ; APPLEBY C.A ; LANDSMANN J ; DENNIS E.S ; TRINICK M.J ; PEACOCK W.J.** *Nature,* 1988, vol. 331, 178-180
- **PERHAM R.N.** *Biochem. Soc. Trans.,* 1990, vol. 18, 185-187
- **MARSH J.J ; LEBHERZ H.G.** *Trends Biochem. Sci.,* 1992, vol. 17, 110-113
- **FREEMONT P.S ; DUNBAR B ; FOTHERGILL-GILMORE L.A.** *Biochem. J.,* 1988, vol. 249, 779-788
- **GILKES N.R ; HENRISSAT B ; KILBURN D.G ; MILLER R.C. JR ; WARREN R.A.** *J. Microbiol. Rev.,* 1991, vol. 55, 303-315
- **HENRISSAT B. ; CLAEYSSENS M ; TOMME P ; LEMESLE L ; MORNON J.-P.** *Gene,* 1989, vol. 81, 83-95
- **KO E.P ; AKATSUKA H ; MORIYAMA H ; SHINMYO A ; HATA Y ; KATSUBE Y ; URABE I ; OKADA H.** *Biochem. J.,* 1992, vol. 288, 117-121
- **GONZALEZ-CANDELAS L ; RAMON D ; POLAINA J.** *Gene,* 1990, vol. 95, 31-38
- **E1 HASSOUNI M ; HENRISSAT B ; CHIPPAUX M ; BARRAS F.** *J. Bacteriol.,* 1992, vol. 174, 765-777
- **SCHROEDER C.J ; ROBERT C ; LENZEN G ; MCKAY L.L ; MERCENIER A.** *J. Gen. Microbiol,* 1991, vol. 137, 369-380
- **GEBLER J.C ; AEBERSOLD R ; WITHERS S.G.** *J. Biol. Chem.,* 1992, vol. 267, 11126-11130
- **BEGUINP.** *Annu. Rev. Microbiol.,* 1990, vol. 44, 219-248
- **HENRISSAT B ; CLAEYSSENS M ; TOMME P ; LEMESLE L ; MORNON J.-P.** *Gene,* 1989, vol. 81, 83-95
- **ALZARI P.M ; SOUCHON H ; DOMINGUEZ R.** *Structure,* 1996, vol. 4, 265-275
- **GILKES N.R ; HENRISSAT B. ; KILBURN D.G ; MILLER R.C. JR ; WARREN R.A.** *J. Microbiol. Rev.,* 1991, vol. 55, 303-315
- **HENRISSAT B ; CLAEYSSENS M ; TOMME P ; LEMESLE L ; MOMON J.-P.** *Gene,* 1989, vol. 81, 83-95
- **HARRIS J.I ; WATERS M.** The Enzymes. 1976, vol. 13, 1-50

- **FABRY S ; LANG J ; NIERMANN T ; VINGRON M ; HENSEL R.** *Eur. J. Biochem.,* 1989, vol. 179, 405-413
- **ZHAO G. ; PEASE A.J ; BHARANI N ; WINKLER M.E.** *J. Bacteriol,* 1995, vol. 177, 2804-2812
- **BHANDARI V ; PALFREE R.G ; BATEMAN A.** *Proc. Natl. Acad. Sci. U.S.A.,* 1992, vol. 89, 1715-1719
- **NAKAKURA N ; HIETTER H ; VAN DORSSELAER A ; LUU B.** *Eur. J. Biochem.,* 1992, vol. 204, 147-153
- **LOHMANN V ; KORNER F ; HERIAN U ; BARTENSCHLAGER R.** *J Virol,* 1997, vol. 71, 8416-8428
- **BEHRENS SE ; TOMEI L ; DE FRANCESCO R.** *EMBO J,* 1996, vol. 15, 12-22
- **ISHIDO S ; FUJITA T ; HOTTA H.** *Biochem Biophys Res Commun,* 1998, vol. 244, 35-40
- **DOHERTY AJ ; SERPELL LC ; PONTING CP.** *Nucleic Acids Res,* 1996, vol. 24, 2488-2497
- **SERAPHIN B.** *DNA Seq.,* 1992, vol. 3, 177-179
- **HUANG Y ; GARRISON P.N ; BARNES L.D.** *Biochem. J.,* 1995, vol. 312, 925-932
- **BARNES L.D ; GARRISON P.N ; SIPRASHVILI Z ; GURANOWSKI A ; ROBINSON A.K ; INGRAM S.W ; CROCE C.M ; OHTA M ; HUEBNER K.** *Biochemistry,* 1996, vol. 35, 11529-11535
- **BRENNER C ; GARRISON P ; GILMOUR J ; PEISACH D ; RINGE D ; PETSKO G.A ; LOWENSTEIN J.M.** *Nat. Struct. Biol.,* 1997, vol. 4, 231-238
- **MURRE C ; MCCAW P.S ; BALTIMORE D.** *Cell,* 1989, vol. 56, 777-783
- **GARREL J ; CAMPUZANO S.** *BioEssays,* 1991, vol. 13, 493-498
- **KATO G.J ; DANG C.V.** *FASEB J.,* 1992, vol. 6, 3065-3072
- **KRAUSE M ; FIRE A. ; HARRISON S.W ; PRIESS J ; WEINTRAUB H.** *Cell,* 1990, vol. 63, 907-919
- **RIECHMANN V ; VAN CRUECHTEN I ; SABLITZKY F.** *Nucleic Acids Res.,* 1994, vol. 22, 749-755
- **BUSTIN M ; REEVES R.** *Prog. Nucleic Acid Res. Mol. Biol,* 1996, vol. 54, 35-100
- **MITCHELL G.A ; ROBERT M.-F ; HRUZ P.W ; WANG S ; FONTAINE G ; BEHNKE C.E ; MENDE-MUELLER L.M ; SCHAPPERT K. ; LEE C ; GIBSON K.M.** *J. Biol. Chem.,* 1993, vol. 268, 4376-4381
- **HRUZ P.W ; NARASIMHAN C ; MIZIORKO H.M.** *Biochemistry,* 1992, vol. 31, 6842-6847
- **WANG S.-Z ; DEAN D.R ; CHEN J.-S ; JOHNSON J.L.** *J. Bacteriol,* 1991, vol. 173, 3041-3046
- **ROKOSZ L.L ; BOULTON D.A ; BUTKIEWICZ E.A ; SANYAL G ; CUETO M.A ; LACHANCE P.A ; HERMES J.D.** *Arch. Biochem. Biophys.,* 1994, vol. 312, 1-13
- **SORGERP.K.** *Cell,* 1991, vol. 65, 363-366
- **MAGER W.H ; MORADAS FERREIRA P.** *Biochem. J.,* 1993, vol. 290, 1-13
- **VUISTER G.W ; KIM S.-J ; OROSZ A. ; MARQUARDT J ; WU C ; BAX A.** *Nat. Struct. Biol.,* 1994, vol. 1, 605-613
- **FUJITA A ; KIKUCHI Y ; KUHARA S ; MISUMI Y ; MATSUMOTO S ; KOBAYASHI H.** *Gene,* 1989, vol. 85, 321-328
- **MORGAN B.A ; BOUQUIN N ; MERRILL G.F ; JOHNSTON L.H.** *EMBO J.,* 1995, vol. 14, 5679-5689
- **LINDQUIST S ; CRAIG E.A.** *Annu. Rev. Genet,* 1988, vol. 22, 631-677
- **DE JONG W.W ; LEUNISSEN J.A.M ; VOORTER C.E.M.** *Mol. Biol. Evol.,* 1993, vol. 10, 103-126
- **CASPERS G.J ; LEUNISSEN J.A.M ; DE JONG W.W.** *J. Mol. Evol,* 1995, vol. 40, 238-248
- **JAENICKE R ; CREIGHTON T.E.** *Curr. Biol,* 1993, vol. 3, 234-235
- **JAKOB U ; BUCHNER J.** *Trends Biochem. Sci.,* 1994, vol. 19, 205-211
- **GROENEN P.J.T.A ; MERCK K.B ; DE JONG W.W ; BLOEMENDAL H.** *Eur. J. Biochem.,* 1994, vol. 225, 1-9
- **LINDQUIST S ; CRAIG E.A.** *Annu. Rev. Genet.,* 1988, vol. 22, 631-677
- **PELHAM H.R.B.** *Cell,* 1986, vol. 46, 959-961
- **PELHAM H.R.B.** *Nature,* 1988, vol. 332, 776-77
- **CRAIG E.A.** *BioEssays,* 1989, vol. 11, 48-52
- **AGRANOVSKY A.A ; BOYKO V.P ; KARASEV A.V ; KOONIN E.V ; DOLJA V.V.** *J. Mol. Biol.,* 1991, vol. 217, 603-610
- **GUPTA R.S ; SINGH B.** *J. Bacteriol.,* 1992, vol. 174, 4594-4605
- **DESHAIES R.J ; KOCH B.D ; SCHEKMAM R.** *Trends Biochem. Sci.,* 1988, vol. 13, 384-388
- **CRAIG E.A ; GROSS C.A.** *Trends Biochem. Sci.,* 1991, vol. 16, 135-140
- **NADEAU K ; DAS A ; WALSH C.T.** *J. Biol. Chem.,* 1993, vol. 268, 1479-1487
- **MAINES M.D.** *FASEB J.,* 1988, vol. 2, 2557-2568
- **BARINAGA M.** *Science,* 1993, vol. 259, 309-309
- **RICHAUD C ; ZABULON G.** *Proc. Natl. Acad. Sci. U.S.A.,* 1997, vol. 94, 11736-11741
- **SCHMITT M.P.** *J. Bacteriol,* 1997, vol. 179, 838-845
- **PASEK M ; GOTO T ; GILBERT W ; ZINK B ; SCHALLER H ; MCKAY P ; LEADBETTER G ; MURRAY K.** *Nature,* 1979, vol. 282, 575-579

- **GALLINA A ; BONELLI F ; ZENTILIN L ; RINDI G ; MUTTINI M ; MILANESI G.** *J Virol,* 1989, vol. 63, 4645-4652
- **FANI R ; TAMBURINI E ; MORI E ; LAZCANO A ; LIO P ; BARBERIO C ; CASALONE E ; CAVALIERI D ; PERITO B ; POLSINELLI M.** *Gene,* 1997, vol. 197, 9-17
- **FANI R ; LIO P ; CHIARELLI I ; BAZZICALUPO M.** *J Mol Evol,* 1994, vol. 38, 489-495
- **LEIPE DD ; LANDSMAN D.** *Nucleic Acids Res,* 1997, vol. 25, 3693-3697
- **NAGAI A ; WARD E ; BECK J ; TADA S. ; CHANG J.-Y ; SCHEIDEGGER A ; RYALS J.** *Proc. Natl. Acad. Sci. U.S.A.,* 1991, vol. 88, 4133-4137
- **GRUBMEYER C.T ; GRAY W.R.** *Biochemistry,* 1986, vol. 25, 4778-4784
- **THOMAS D ; BARBEY R. ; SURDIN-KERJAN Y.** *FEBS Lett,* 1993, vol. 323, 289-293
- **CAMI B ; CLEPET C ; PATTE J.-C.** *Biochimie,* 1993, vol. 75, 487-495
- **HISANO T ; HATA Y ; FUJII T ; LIU JQ ; KURIHARA T ; ESAKI N ; SODA K.** *J Biol Chem,* 1996, vol. 271, 20322-20330
- **SCHMID S.R ; LINDER P.** *Mol. Microbiol.,* 1992, vol. 6, 283-292
- **LINDER P ; LASKO P ; ASHBURNER M ; LEROY P ; NIELSEN P.J ; NISHI K ; SCHNIER J ; SLONIMSKI P.P.** *Nature,* 1989, vol. 337, 121-122
- **WASSARMAN D.A ; STEITZ J.A.** *Nature,* 1991, vol. 349, 463-464
- **HODGMAN T.C.** *Nature,* 1988, vol. 333, 22-23
- **HAROSH I ; DESCHAVANNE P.** *Nucleic Acids Res.,* 1991, vol. 19, 6331-6331
- **KOONIN E.V ; SENKEVICH T.G.** *J. Gen. Virol,* 1992, vol. 73, 989-993
- **OZOLS J.** *Biochim. Biophys. Acta,* 1989, vol. 997, 121-130
- **GUIARD B.** *EMBO J.,* 1985, vol. 4, 3265-3272
- **CALZA R ; HUTTNER E ; VINCENTZ M ; ROUZE P ; GALANGAU F ; VAUCHERET H ; CHEREL I ; MEYER C ; KRONENBERGER J. ; CABOCHE M.** *Mol. Gen. Genet,* 1987, vol. 209, 552-562
- **CRAWFORD N.M ; SMITH M ; BELLISSIMO D ; DAVIS R.W.** *Proc. Natl. Acad. Sci. U.S.A.,* 1988, vol. 85, 5006-5010
- **GUIARD B ; LEDERER F.** *Eur. J. Biochem.,* 1979, vol. 100, 441-453
- **LEVIN R.J ; BOYCHUK P.L ; CRONIGER C.M ; KAZZAZ J.A ; ROZEK C.E.** *Nucleic Acids Res.,* 1989, vol. 17, 6349-6367
- **DOWNIE J.A.** *Mol. Microbiol.,* 1989, vol. 3, 1649-1651
- **PARENT R ; ROY P.H.** *J. Bacteriol.,* 1992, vol. 174, 2891-2897
- **VAARA M.** *FEMS Microbiol. Lett.,* 1992, vol. 97, 249-254
- **VUORIO R ; HAERKONEN T ; TOLVANEN M ; VAARA M.** *FEBS Lett.,* 1994, vol. 337, 289-292
- **RAETZ C.R.H ; RODERICK S.L.** *Science,* 1995, vol. 270, 997-1000
- **MIDDLETON R.J.** *Biochem. Soc. Trans.,* 1990, vol. 18, 180-183
- **GRIFFIN L.D ; GELB B.D ; WHEELER D.A ; DAVISON D. ; ADAMS V ; MCCABE E.R.** *Genomics,* 1991, vol. 11, 1014-1024
- **CORNISH-BOWDEN A ; LUZ CARDENAS M.** *Trends Biochem. Sci,* 1991, vol. 16, 281-282
- **SCHIRCH D.M ; WILSON J.E.** *Arch. Biochem. Biophys,* 1987, vol. 254, 385-396
- **WELLS D.E ; BROWN D.** *Nucleic Acids Res.,* 1991, vol. 19, 2173-2188
- **THATCHER T.H ; GOROVSKY M.A.** *Nucleic Acids Res.,* 1994, vol. 22, 174-179
- **DOENECKE D ; GALLWITZ D.** *Mol. Cell. Biochem.,* 1982, vol. 44, 113-128
- **EBRALIDSE K.K ; GRACHEV S.A ; MIRZABEKOV A.D.** *Nature,* 1988, vol. 331, 365-367
- **SULLIVAN K.F ; HECHENBERGER M ; MASRI K.** *J. Cell Biol.,* 1994, vol. 127, 581-592
- **STOLER S ; KEITH K.C ; CURNICK K.E ; FITZGERALD-HAYES M.** *Genes Dev,* 1995, vol. 9, 573-586
- **THATCHER T.H. ; GOROVSKY M.A.** *Nucleic Acids Res.,* 1994, vol. 22, 174-179
- **GEHRING W.J.** Guidebook to the homebox genes. Oxford University Press, 1994, 1-10
- **BUERGLIN T.R.** Guidebook to the homebox genes. Oxford University Press, 1994, 25-72
- **GEHRING W.J.** *Trends Biochem. Sci.,* 1992, vol. 17, 277-280
- **GEHRING W.J ; HIROMI Y.** *Annu. Rev. Genet.,* 1986, vol. 20, 147-173
- **SCHOFIELD P.N.** *Trends Neurosci,* 1987, vol. 10, 3-6
- **MCGINNIS W ; KRUMLAUF R.** *Cell,* 1992, vol. 68, 283-302
- **SCOTT M.P.** *Cell,* 1992, vol. 71, 551-553
- **SCOTT M.P ; TAMKUN J.W ; HARTZELL G.W. III.** *Biochim. Biophys. Acta,* 1989, vol. 989, 25-48
- **GEHRING W.J.** *Science,* 1987, vol. 236, 1245-1252
- **BEECHING J.R.** *Protein Seq. Data Anal.,* 1989, vol. 2, 463-466
- **ATOMI H ; UEDA M ; HIKIDA M ; HISHIDA T ; TERANISHI Y ; TANAKA A.** *J. Biochem.,* 1990, vol. 107, 262-266
- **KYRPIDES NC ; WOESE CR.** *Proc Natl Acad Sci U S A,* 1998, vol. 95, 3726-3730
- **LIVERIS D ; SCHWARTZ J.J ; GEERTMAN R ; SCHWARTZ I.** *FEMS Microbiol. Lett,* 1993, vol. 112, 211-216
- **LIN Q ; MA L ; BURKHART W ; SPREMULLI L.L.** *J. Biol. Chem.,* 1994, vol. 269, 9436-9444

- **CARLOMAGNO M.S ; CHIARIOTTI L ; ALIFANO P ; NAPPO A.G ; BRUNI C.B.** *J. Mol. Biol.,* 1988, vol. 203, 585-606
- **WILMANNS M ; PRIESTLE J.P ; NIERMANN T ; JANSONIUS J.N.** *J. Mol. Biol.,* 1992, vol. 223, 477-507
- **EGAN S.E ; FLIEGE R ; TONG S ; SHIBATA A ; WOLF R.E. JR ; CONWAY T.** *J. Bacteriol,* 1992, vol. 174, 4638-4646
- **VELASCO J.A ; CANSADO J ; PENA M.C ; KAWAKAMI T ; LABORDA J ; NOTARIO V.** *Gene,* 1993, vol. 137, 179-185
- **COLLART F.R ; HUBERMAN E.** *J. Biol. Chem.,* 1988, vol. 263, 15769-15772
- **NATSUMEDA Y ; OHNO S ; KAWASAKI H ; KONNO Y ; WEBER G ; SUZUKI K.** *J. Biol. Chem.,* 1990, vol. 265, 5292-5295
- **ANDREWS S.C ; GUEST J.R.** *Biochem. J.,* 1988, vol. 255, 35-43
- **YORK JD ; PONDER JW ; CHEN ZW ; MATHEWS FS ; MAJERUS PW.** *Biochemistry,* 1994, vol. 33, 13164-13171
- **JEFFERSON AB ; AUETHAVEKIAT V ; POT DA ; WILLIAMS LT ; MAJERUS PW.** *J Biol Chem,* 1997, vol. 272, 5983-5988
- **ZHANG X ; JEFFERSON AB ; AUETHAVEKIAT V ; MAJERUS PW.** *Proc Natl Acad Sci U S A,* 1995, vol. 92, 4853-4856
- **YORK JD ; MAJERUS PW.** *Proc Natl Acad Sci U S A,* 1990, vol. 87, 9548-9552
- **NEUWALD AF ; YORK JD ; MAJERUS PW.** *FEBS Lett,* 1991, vol. 294, 16-18
- **XIE X ; HARRISON DH ; SCHLICHTING I ; SWEET RM ; KALABOKIS VN ; SZENT-GYORGYI AG ; COHEN C.** *Nature,* 1994, vol. 368, 306-312
- **RHOADS AR ; FRIEDBERG F.** *FASEB J,* 1997, vol. 11, 331-340
- **GOPAUL D.N ; MEYER S.L ; DEGANO M ; SACCHETTINI J.C ; SCHRAMM V.L.** *Biochemistry,* 1996, vol. 35, 5963-5970
- **DEGANO M ; GOPAUL D.N ; SCAPIN G ; SCHRAMM V.L ; SACCHETTINI J.C.** *Biochemistry,* 1996, vol. 35, 5971-5981
- **RAWLINGS N.D ; BARRETTA.J.** *Biochem. J.,* 1991, vol. 275, 389-391
- **BRAUN H.-P ; SCHMITZ U.K.** *Trends Biochem. Sci.,* 1995, vol. 20, 171-175
- **BECKER A.B ; ROTH R.A.** *Proc. Natl. Acad. Sci. U.S.A.,* 1992, vol. 89, 3835-3839
- **FUJITA A ; OKA C ; ARIKAWA Y ; KATAGAI T ; TONOUCHI A ; KUHARA S ; MISUMI Y.** *Nature,* 1994, vol. 372, 567-570
- **RAWLINGS N.D ; BARRETT A.** *J. Meth. Enzymol,* 1995, vol. 248, 183-228
- **ECKERT RL ; YAFFE MB ; CRISH JF ; MURTHY S ; RORKE EA ; WELTER JF.** Involucrin repeat. *J Invest Dermatol,* 1993, vol. 100, 613-617
- **ROMAO MJ ; TURK D ; GOMIS-RUTH FX ; HUBER R ; SCHUMACHER G ; MOLLERING H ; RUSSMANN L.** Isochorismatase family. This family are hydrolase enzymes. *J Mol Biol,* 1992, vol. 226, 1111-1130
- **NEUWALD A.F ; YORK J.D ; MAJERUS P.W.** *FEBS Lett.,* 1991, vol. 294, 16-18
- **GLAESER H.-U ; THOMAS D ; GAXIOLA R ; MONTRICHARD F ; SURDIN-KERJAN Y ; SERRANO R.** *EMBO J.,* 1993, vol. 12, 3105-3110
- **BONE R ; SPRINGER J.P ; ATACK J.R.** *Proc. Natl. Acad. Sci. U.S.A.,* 1992, vol. 89, 10031-10035
- **HURLEY J.H ; THORSNESS P.E ; RAMALINGAM V ; HELMERS N.H ; KOSHLAND D.E. JR ; STROUD R.M.** *Proc. Natl. Acad. Sci. U.S.A.,* 1989, vol. 86, 8635-8639
- **CUPP J.R ; MCALISTER-HENN L.** *J. Biol. Chem.,* 1991, vol. 266, 22199-22205
- **IMADA K ; SATO M ; TANAKA N ; KATSUBE Y ; MATSUURA Y ; OSHIMA T.** *J. Mol. Biol.,* 1991, vol. 222, 725-738
- **ZHANG T ; KOSHLAND D.E. JR.** *Protein Sci,* 1995, vol. 4, 84-92
- **TIPTON P.A ; BEECHER B.S.** *Arch. Biochem. Biophys,* 1994, vol. 313, 15-21
- **SANKARANARAYANAN R ; SEKAR K ; BANERJEE R ; SHARMA V ; SUROLIA A ; VIJAYAN M.** *Nat Struct Biol,* 1996, vol. 3, 596-603
- **BURD CG ; DREYFUSS G.** *Science,* 1994, vol. 265, 615-621
- **MUSCO G ; STIER G ; JOSEPH C ; CASTIGLIONE MORELLI MA ; NILGES M ; GIBSON TJ ; PASTORE A.** *Cell,* 1996, vol. 85, 237-245
- **BORK P ; DOOLITTLE RF.** *J Mol Biol,* 1994, vol. 236, 1277-1282
- **ITO N ; PHILLIPS SE ; STEVENS C ; OGEL ZB ; MCPHERSON MJ ; KEEN, JN ; YADAV KD ; KNOWLES PF.** *Nature,* 1991, vol. 350, 87-90
- **LASKOWSKI M ; KATO I.** *Annu. Rev. Biochem.,* 1980, vol. 49, 593-626
- **RITONJA A ; KRIZAJ I ; MESKO P ; KOPITAR M ; LUCOVNIK P ; STRUKELJ B ; PUNGERCAR J ; BUTTLE D.J ; BARRETT A.J ; TURK V.** *FEBS Lett,* 1990, vol. 267, 13-15
- **KORTT A.A ; STRIKE P.M ; DE JERSEY J.** *Eur. J. Biochem.,* 1989, vol. 181, 403-408
- **THEERASILP S ; HITOTSUYA H ; NAKAJO S ; NAKAJA K ; NAKAMURA Y ; KURIHARA Y.** *J. Biol. Chem.,* 1989, vol. 264, 6655-6659
- **HATTORI T ; YOSHIDA N ; NAKAMURA K.** *Plant Mol. Biol.,* 1989, vol. 13, 563-572
- **KRIZAJ I ; DROBNIC-KOSOROK M ; BRZIN J ; JERALA R ; TURK V.** *FEBS Lett,* 1993, vol. 333, 15-20
- **BRADSHAW H.D ; HOLLICK J.B. ; PARSONS T.J ; CLARKE H.R.G ; GORDON M.P.** *Plant Mol. Biol,* 1989, vol. 14, 51-59

- **TAI H ; MCHENRY L ; FRITZ P.J ; FURTEK D.B.** *Plant Mol. Biol,* 1991, vol. 16, 913-915
- **KAUPPINEN S ; SIGGAARD-ANDERSEN M. ; VON WETTSTEIN-KNOWLES P.** *Carlsberg Res. Commun,* 1988, vol. 53, 357-370
- **WITKOWSKI A ; RANGAN V.S ; RANDHAWA Z.I ; AMY C.M ; SMITH S.** *Eur. J. Biochem.,* 1991, vol. 198, 571-579
- **BECK J ; RIPKA S ; SIEGNER A ; SCHILTZ E ; SCHWEIZER E.** *Eur. J. Biochem.,* 1990, vol. 192, 487-498
- **BIBB M.J ; BIRO S ; MOTAMEDI H ; COLLINS J.F ; HUTCHINSON C.R.** *EMBO J.,* 1989, vol. 8, 2727-2736
- **SHERMAN D.H ; MALPARTIDA F ; BIBB M.J ; KIESER H.M ; BIBB M.J ; HOPWOOD D.A.** *EMBO J.,* 1989, vol. 8, 2717-2725
- **BLOOM G.S ; ENDOW S.A.** *Protein Prof.,* 1995, vol. 2, 1109-1171
- **BRADY S.T.** *Trends Cell Biol,* 1995, vol. 5, 159-164
- **ENDOW S.A.** *Trends Biochem. Sci,* 1991, vol. 16, 221-225
- **OTAKA E ; HASHIMOTO T ; MIZUTA K ; SUZUKI K.** *Protein Seq. Data Anal,* 1993, vol. 5, 301-313
- **SCHUMANN R.R ; LEONG S.R ; FLAGGS G.W ; GRAY P.W ; WRIGHT S.D ; MATHISON J.C ; TOBIAS P.S ; ULEVITCH R.J.** *Science,* 1990, vol. 249, 1429-1431
- **GRAY P.W ; FLAGGS G ; LEONG S.R ; GUMINA R.J ; WEISS J ; OOI C.E ; ELSBACH P.** *J. Biol. Chem.,* 1989, vol. 264, 9505-9509
- **DAY J.R ; ALBERS J.J ; LOFTON-DAY C.E ; GILBERT T.L ; CHING A.F.T ; GRANT F.J ; O'HARA P.J ; MARCOVINA S.M ; ADOLPHSON J.L.** *J. Biol. Chem.,* 1994, vol. 269, 9388-9391
- **FREYD G ; KIM S.K ; HORVITZ H.R.** *Nature,* 1990, vol. 344, 876-879
- **BALTZ R ; EVRARD J.-L ; DOMON C ; STEINMETZ A.** *Plant Cell,* 1992, vol. 4, 1465-1466
- **SANCHEZ-GARCIA I ; RABBITTS T.H.** *Trends Genet.,* 1994, vol. 10, 315-320
- **MUELLER A ; XU G ; WELLS R ; HOLLENBERG C.P ; PIEPERSBERG W.** *Nucleic Acids Res.,* 1994, vol. 22, 3151-3154
- **MICHELSEN J.W ; SCHMEICHEL K.L ; BECKERLE M.C ; WINGE D.R.** *Proc. Natl. Acad. Sci. U.S.A.,* 1993, vol. 90, 4404-4408
- **KOBE B ; DEISENHOFER J.** The leucine-rich repeat: a versatile binding motif. *Trends Biochem Sci,* 1994, vol. 19, 415-421
- **KOBE B ; DEISENHOFER J.** Crystal structure of porcine ribonuclease inhibitor, a protein with leucine-rich repeats. *Nature,* 1993, vol. 366, 751-756
- **WIRTZ K.W.A.** *Annu. Rev. Biochem.,* 1991, vol. 60, 73-99
- **ARONDEL V ; KADER J.C.** *Experientia,* 1990, vol. 46, 579-585
- **OHLROGGE J.B ; BROWSE J ; SOMERVILLE C.R.** *Biochim. Biophys. Acta,* 1991, vol. 1082, 1-26
- **FUKUDA M.** *J. Biol. Chem.,* 1991, vol. 266, 21327-21330
- **HOLNESS C.L ; DA SILVA R.P ; FAWCETT J ; GORDON S ; SIMMONS D.L.** *J. Biol. Chem.,* 1993, vol. 268, 9661-9666
- **HAYASHI S ; WUH.C.** *J. Bioenerg. Biomembr.,* 1990, vol. 22, 451-471
- **KLEIN P ; SOMORJAI R.L ; LAU P.C.K.** *Protein Eng.,* 1988, vol. 2, 15-20
- **VON HEIJNE G.** *Protein Eng.,* 1989, vol. 2, 531-534
- **MATTAR S ; SCHARF B ; KENT S.B.H ; RODEWALD K ; OESTERHELT D ; ENGELHARD M.** *J. Biol. Chem.,* 1994, vol. 269, 14939-14945
- **HAYASHI S ; WU H.C.** *J. Bioenerg. Biomembr.,* 1990, vol. 22, 451-471
- **O'KANE D.J ; WOODWARD B ; LEE J ; PRASHER D.C.** *Proc. Natl. Acad. Sci. U.S.A.,* 1991, vol. 88, 1100-1104
- **O'KANE D.J ; PRASHER D.C.** *Mol. Microbiol.,* 1992, vol. 6, 443-449
- **KREBS C.J ; KRAWETZ S.A.** *Biochim. Biophys. Acta,* 1993, vol. 1202, 7-12
- **NEUWALD AF ; LIU JS ; LIPMAN DJ ; LAWRENCE CE.** *Nucleic Acids Res,* 1997, vol. 25, 1665-1677
- **CARFI A ; PARES S ; DUEE E ; GALLENI M ; DUEZ C ; FRERE JM ; DIDEBERG O.** *EMBO J,* 1995, vol. 14, 4914-4921
- **ABAD-ZAPATERO C ; GRIFFITH J.P ; SUSSMAN J.L ; ROSSMANN M.G.** *J. Mol. Biol.,* 1987, vol. 198, 445-467
- **HENDRIKS W ; MULDERS J.W.M ; BIBBY M.A ; SLINGSBY C ; BLOEMENDAL H ; DE JONG W.W.** *Proc. Natl. Acad. Sci. U.S.A.,* 1988, vol. 85, 7114-7118
- **LERCH H.-P ; FRANK R ; COLLINS J.** *Gene,* 1989, vol. 83, 263-270
- **MCALISTER-HENN L.** *Trends Biochem. Sci.,* 1988, vol. 13, 178-181
- **GIETL C.** *Biochim. Biophys. Acta,* 1992, vol. 1100, 217-234
- **BIRKTOFT J.J ; RHODES G ; BANASZAK L.J.** *Biochemistry,* 1989, vol. 28, 6065-6081
- **CENDRIN F ; CHROBOCZEK J ; ZACCAI G ; EISENBERG H ; MEVARECH M.** *Biochemistry,* 1993, vol. 32, 4308-4313
- **SHARON N ; LIS H.** *FASEB J.,* 1990, vol. 4, 3198-320
- **LIS H ; SHARON N.** *Annu. Rev. Biochem.,* 1986, vol. 55, 33-37

- **WOLODKO WT ; FRASER ME ; JAMES MN ; BRIDGER WA.** *J Biol Chem,* 1994, vol. 269, 10883-10890
- **RAMAKRISHNAN V ; FINCH JT ; GRAZIANO V ; LEE PL ; SWEET RM.** *Nature,* 1993, vol. 362, 219-223
- **COWAN S.W ; NEWCOMER M.E ; JONES T.A.** *Proteins,* 1990, vol. 8, 44-61
- **IGARAISHI M ; NAGATA A ; TOH H ; URADE H ; HAYAISHI N.** *Proc. Natl. Acad. Sci. U.S.A.,* 1992, vol. 89, 5376-5380
- **FLOWER D.R ; NORTH A.C.T. ; ATTWOOD T.K.** *Protein Sci,* 1993, vol. 2, 753-761
- **GODOVAC-ZIMMERMANN J.** *Trends Biochem. Sci.,* 1988, vol. 13, 64-66
- **PERVAIZ S ; BREW K.** *FASEB J.,* 1987, vol. 1, 209-214
- **KREMER J.M.H ; WILTING J ; JANSSEN L.H.M.** *Pharmacol. Rev.,* 1989, vol. 40, 1-47
- **HAEFLIGER J.-A ; PEITSCH M.C ; JENNE D ; TSCHOPP J.** *Mol. Immunol,* 1991, vol. 28, 123-131
- **KEEN J.N ; CACERES I ; ELIOPOULOS E.E ; ZAGALSKY P.F ; FINDLAY J.B.C.** *Eur. J. Biochem.,* 1991, vol. 197, 407-417
- **NEWCOMER M.E.** *Structure,* 1993, vol. 1, 7-18
- **COLLET C ; JOSEPH R.** *Biochim. Biophys. Acta,* 1993, vol. 1167, 219-222
- **KJELDSEN L ; JOHNSEN A.H ; SENGELOV H ; BORREGAARD N.** *J. Biol. Chem.,* 1993, vol. 268, 10425-10432
- **PEITSCH M.C ; BOGUSKI M.S.** *Trends Biochem. Sci.,* 1991, vol. 16, 363-363
- **MIYAWAKI A ; MATSUSHITA Y.R ; RYO Y ; MIKOSHIBA T.** *EMBO J.,* 1994, vol. 13, 5835-5842
- **KOCK K ; AHLERS C ; SCHMALE H.** *Eur. J. Biochem.,* 1994, vol. 221, 905-916
- **ACHEN M.G ; HARMS P.J ; THOMAS T ; RICHARDSON S.J ; WETTENHALL R.E.H ; SCHREIBER G.** *J. Biol. Chem.,* 1992, vol. 267, 23170-23174
- **MOREL L ; DUFARRE J.-P ; DEPEIGES A.** *J. Biol. Chem.,* 1993, vol. 268, 10274-10281
- **BISHOP R.E ; PENFOLD S.S ; FROST L.S ; HOLTJE J.V ; WEINER J.H.** *J. Biol. Chem.,* 1995, vol. 270, 23097-23103
- **FLOWER D.R ; NORTH A.C.T ; ATTWOOD T.K.** *Biochem. Biophys. Res. Commun.,* 1991, vol. 180, 69-74
- **FLOWER D.R.** *FEBS Lett,* 1993, vol. 333, 99-102
- **BERNIER I ; JOLLES P.** *Biochimie,* 1987, vol. 69, 1127-1152
- **VEERKAMP J.H ; PEETERS R.A ; MAATMAN R.G.H.** *J. Biochim. Biophys. Acta,* 1991, vol. 1081, 1-24
- **SIEGENTHALER G ; HOTZ R ; CHATELLARD-GRUAZ D ; DIDIERJEAN L ; HELLMAN U ; SAURAT J.-H.** *Biochem. J.,* 1994, vol. 302, 363-371
- **SMITH A.F ; TSUCHIDA K ; HANNEMAN E ; SUZUKI T.C ; WELLS M.A.** *J. Biol. Chem.,* 1992, vol. 267, 380-384
- **MOSER D ; TENDLER M ; GRIFFITHS G ; KLINKERT M.-Q.** *J. Biol. Chem.,* 1991, vol. 266, 8447-8454
- **FLOWER D.R ; NORTH A.C.T ; ATTWOOD T.K.** *Protein Sci.,* 1993, vol. 2, 753-761
- **FLOWER D.R.** *FEBS Lett.,* 1993, vol. 333, 99-102
- **VICK B.A ; ZIMMERMAN D.C.** Biochemistry of plants: A comprehensive treatise. Academic Press, 1987, vol. 9, 53-90
- **NEEDLEMAN P ; TURK J ; JAKSCHIK B.A ; MORRISON A.R ; LEFKOWITH J.B.** *Annu. Rev. Biochem.,* 1986, vol. 55, 69-102
- **PENG Y.L ; SHIRANO Y ; OHTA H ; HIBINO T ; TANAKA K ; SHIBATA D.** *J. Biol. Chem.,* 1994, vol. 269, 3755-3761
- **BOYINGTON J.C ; GAFFNEY B.J ; AMZEL L.M.** *Science,* 1993, vol. 260, 1482-1486
- **STECZKO J ; DONOHO G.P ; CLEMENS J.C ; DIXON J.E ; AXELROD B.** *Biochemistry,* 1992, vol. 31, 4053-4057
- **WOODS S.A ; SHWARTZBACH S.D ; GUEST J.R.** *Biochim. Biophys. Acta,* 1988, vol. 954, 14-26
- **WOODS S.A ; MILES J.S ; GUEST J.R.** *FEMS Microbiol. Lett.,* 1988, vol. 51, 181-186
- **ZALKIN H. ; DIXON J.E.** *Prog. Nucleic Acid Res. Mol. Biol.,* 1992, vol. 42, 259-287
- **WILLIAMS S.E ; WOOLRIDGE E.M ; RANSOM S.C ; LANDRO J.A ; BABBITT P.C ; KOZARICH J.W.** *Biochemistry,* 1992, vol. 31, 9768-9776
- **COXON A ; MAUNDRELL K ; KEARSEY S.E.** *Nucleic Acids Res.,* 1992, vol. 20, 5571-5577
- **HU B ; BURKHART R ; SCHULTE D ; MUSAHL C ; KNIPPERS R.** *Nucleic Acids Res.,* 1993, vol. 21, 5289-5293
- **TYE B.-K.** *Trends Cell Biol.,* 1994, vol. 4, 160-166
- **KOONIN E.V.** *Nucleic Acids Res.,* 1993, vol. 21, 2541-2547
- **BUCALA R.** *Immunol. Lett.,* 1994, vol. 43, 23-26
- **ODH G ; HINDEMITH A ; ROSENGREN A.-M ; ROSENGREN E ; RORSMAN H.** *Biochem. Biophys. Res. Commun.,* 1993, vol. 197, 619-624
- **PEARSON W.R.** *Protein Sci.,* 1994, vol. 3, 525-527
- **REIZER J ; REIZER A ; SAIER M.H. JR.** *CRC Crit. Rev. Biochem.,* 1993, vol. 28, 235-257
- **BAKER M.E ; SAIER M.H. JR.** *Cell,* 1990, vol. 60, 185-186

- **PAO G.M ; WU L.-F ; JOHNSON K.D ; HOEFTE H ; CHRISPEELS M.J ; SWEET G ; SANDAL N.N ; SAIER M.H. JR.** *Mol. Microbiol.,* 1991, vol. 5, 33-37
- **WISTOW G.J ; PISANO M.M ; CHEPELINSKY A.B.** *Trends Biochem. Sci.,* 1991, vol. 16, 170-171
- **CHRISPEELS M.J ; AGRE P.** *Trends Biochem. Sci.,* 1994, vol. 19, 421-425
- **NEIDHART D.J ; KENYON G.L ; GERLT J.A ; PETSKO G.A.** *Nature,* 1990, vol. 347, 692-694
- **PETSKO G.A ; KENYON G.L ; GERLT J.A ; RINGE D ; KOZARICH J.W.** *Trends Biochem. Sci.,* 1993, vol. 18, 372-376
- **HUISMAN G.W ; KOLTER R.** *Science,* 1994, vol. 265, 537-539
- **SCHNEIDER D ; AIGLE B ; LEBLOND P ; SIMONET J.M ; DECARIS B.** *J. Gen. Microbiol,* 1993, vol. 139, 2559-2567
- **THOMAS AW ; CARR DA ; CARTER JM ; LYON JA.** Merozoite Surface Antigen 2 (MSA-2) family. *Mol Biochem Parasitol,* 1990, vol. 43, 211-220
- **BULLOCK TL ; ROBERTS TM ; STEWART M.** *J Mol Biol,* 1996, vol. 263, 284-296
- **KING KL ; STEWART M ; ROBERTS TM ; SEAVY M.** *J Cell Sci,* 1992, vol. 101, 847-857
- **KELLER NP ; DISCHINGER HC ; BHATNAGAR D ; CLEVELAND TE ; ULLAH AH.** *Appl Environ Microbiol,* 1993, vol. 59, 479-484
- **WALKER CJ ; WILLOWS RD.** *Biochem J,* 1997, vol. 327, 321-333
- **PETERSEN BL ; JENSEN PE ; GIBSON LC ; STUMMANN BM ; HUNTER CN ; HENNINGSEN KW.** *J Bacteriol,* 1998, vol. 180, 699-704
- **PRIEBE SD ; LACKS SA.** *J Bacteriol,* 1989, vol. 171, 4778-4784
- **GATTI DL ; PALFEY BA ; LAH MS ; ENTSCH B ; MASSEY V ; BALLOU DP ; LUDWIG ML.** *Science,* 1994, vol. 266, 110-114
- **ARAVIND L ; PONTING CP.** *Protein Sci,* 1998, vol. 7, 1250-1254
- **HERSHEY JW ; ASANO K ; NARANDA T ; VORNLOCHER HP ; HANACHI P ; MERRICK WC.** *Biochimie,* 1996, vol. 78, 903-907
- **FACCHINI LM ; PENN LZ.** *FASEB J,* 1998, vol. 12, 633-651
- **GRANDORI C ; EISENMAN RN.** *Trends Biochem Sci,* 1997, vol. 22, 177-181
- **MORIGUCHI T ; KITA M ; HISADA S ; ENDO-INAGAKI T ; OMURA M.** Medline: 98267202. Characterization of gene repertoires at mature stage of citrus fruits through random sequencing and analysis of redundant metallothionein-like genes expressed during fruit development. *Gene,* 1998, vol. 211, 221-227
- **MCCURDY DK ; TAI LQ ; NGUYEN J ; WANG Z ; YANG HM ; UDAR N ; NAIEM F ; CONCANNON P ; GATTI RA.** *Mol Genet Metab,* 1998, vol. 63, 3-13
- **ARTUS N.N ; EDWARDS G.E.** *FEBS Lett.,* 1985, vol. 182, 225-233
- **LOEBER G ; INFANTE A.A ; MAURER-FOGY L ; KRYSTEK E ; DWORKIN M.B.** *J. Biol. Chem.,* 1991, vol. 266, 3016-3021
- **LONG J.J. ; WANG J.-L ; BERRY J.O.** *J. Biol. Chem.,* 1994, vol. 269, 2827-2833
- **WOESSNER J. JR.** *FASEB J.,* 1991, vol. 5, 2145-2154
- **SANCHEZ-LOPEZ R ; NICHOLSON R ; GESNEL M.C ; MATRISIAN L.M ; BREATHNACH R.** *J. Biol. Chem.,* 1988, vol. 263, 11892-11899
- **PARK A.J ; MATRISIAN L.M ; KELLS A.F ; PEARSON R ; YUAN Z ; NAVRE M.** *J. Biol. Chem.,* 1991, vol. 266, 1584-1590
- **LEPAGE T ; GACHE C.** *EMBO J.,* 1990, vol. 9, 3003-3012
- **KINOSHITA T ; FUKUZAWA H ; SHIMADA T ; SAITO T ; MATSUDA Y.** *Proc. Natl. Acad. Sci. U.S.A.,* 1992, vol. 89, 4693-4697
- **HAMER D.H.** *Annu. Rev. Biochem.,* 1986, vol. 55, 913-951
- **KAGI J.H.R ; SCHAFFER A.** *Biochemistry,* 1988, vol. 27, 8509-8515
- **BINZ P.-A.** Thesis. University of Zurich, 1996
- **KLINGENBERG M.** *Trends Biochem. Sci.,* 1990, vol. 15, 108-112
- **WALKER J.E.** *Curr. Opin. Struct. Biol.,* 1992, vol. 2, 519-526
- **KUAN J ; SAIER M.H. JR.** *CRC Crit. Rev. Biochem.,* 1993, vol. 28, 209-233
- **KUAN J. ; SAIER M.H. JR.** *Res. Microbiol.,* 1993, vol. 144, 671-672
- **NELSON D.R ; LAWSON J.E ; KLINGENBERG M ; DOUGLAS M.G.** *J. Mol. Biol.,* 1993, vol. 230, 1159-1170
- **PALMIERI F.** *FEBS Lett.,* 1994, vol. 346, 48-54
- **JANK B ; HABERMANN B ; SCHWEYEN R.J ; LINK T.A.** *Trends Biochem. Sci.,* 1993, vol. 18, 427-428
- **WOOTTON J.C ; NICOLSON R.E ; COCK J.M ; WALTERS D.E ; BURKE J.F ; DOYLE W.A ; BRAY R.C.** *Biochim. Biophys. Acta,* 1991, vol. 1057, 157-185
- **BILOUS P.T ; COLE S.T ; ANDERSON W.F ; WEINER J.H.** *Mol. Microbiol.,* 1988, vol. 2, 785-795
- **TRIEBER C.A ; ROTHERY R.A ; WEINER J.H.** *J. Biol. Chem.,* 1994, vol. 269, 7103-7109
- **MEJEAN V ; LOBBI-NIVOL C ; LEPELLETIER M ; GIORDANO G ; CHIPPAUX M ; PASCAL M.-C.** *Mol. Microbiol.,* 1994, vol. 11, 1169-1179
- **MICHAELS M.L ; MILLER J.H.** *J. Bacteriol,* 1992, vol. 174, 6321-6325
- **KOONIN E.V.** *Nucleic Acids Res.,* 1993, vol. 21, 4847-4847

- **MEJEAN V ; SALLES C ; BULLIONS M.J ; BESSMAN M.J ; CLAVERYS J.-P.** *Mol. Microbiol.,* 1994, vol. 11, 323-330
- **SAKUMI K ; FURUICHI M ; TSUZUKI T ; KAKUMA T ; KAWABATA S ; MAKI H ; SEKIGUCHI M.** *J. Biol. Chem.,* 1993, vol. 268, 23524-23530
- **THORNE N.M.H ; HANKIN S ; WILKINSON M.C ; NUNEZ C ; BARRACLOUGH R ; MCLENNAN A.G.** *Biochem. J.,* 1995, vol. 311, 717-721
- **BIEDNKAPP H. ; BORGMEYER U. ; SIPPEL A.E. ; KLEMPNAUER K.-H.** *Nature,* 1988, vol. 335, 835-837
- **PETERS C.W.B. ; SIPPEL A.E. ; VINGRON M. ; KLEMPNAUER K.-H.** *EMBO J.,* 1987, vol. 6, 3085-3090
- **NOMURA N. ; TAKAHASHI M. ; MATSUI M. ; ISHII S. ; DATE T. ; SASAMOTO S. ; ISHIZAKI R.** *Nucleic Acids Res.,* 1988, vol. 16, 11075-11090
- **GROTEWOLD E. ; ATHMA P. ; PETERSON T.** *Proc. Natl. Acad. Sci. U.S.A.,* 1991, vol. 88, 4587-4591
- **OPPENHEIMER D.G. ; HERMAN P.L. ; SIVAKUMARAN S. ; ESCH J. ; MARKS M.D.** *Cell,* 1991, vol. 67, 483-493
- **MAROCCO A. ; WISSENBACH M. ; BECKER D. ; PAZ-ARES J. ; SAEDLER H. ; SALAMINI F. ; ROHDE W.** *Mol. Gen. Genet.,* 1989, vol. 216, 183-187
- **TICE-BALDWIN K. ; FINK G.R. ; ARNDT K.T.** *Science,* 1989, vol. 246, 931-935
- **JU Q. ; MORROW B.E. ; WARNER J.R.** *Mol. Cell. Biol.,* 1990, vol. 10, 5226-5234
- **KLEMPNAUER K.-H. ; SIPPEL A.E.** *EMBO J.,* 1987, vol. 6, 2719-2725
- **OTTO J. ; ARGOS P. ; ROSSMANN M.G.** *Eur. J. Biochem.,* 1980, vol. 109, 325-330
- **RODRIGUEZ P ; MUNROE D ; PRAWITT D ; CHU LL ; BRIC E ; KIM J ; REID LH ; DAVIES C ; NAKAGAMA H ; LOEBBERT R.** *Genomics,* 1997, vol. 44, 253-265
- **SCHNIEDERS F ; DORK T ; ARNEMANN J ; VOGEL T ; WERNER M ; SCHMIDTKE J.** *Hum Mol Genet,* 1996, vol. 5, 1801-1807
- **VAN DER BIEZEN EA ; JONES JD.** NB-ARC domain. *Curr Biol,* 1998, vol. 8, 226-227
- **PARKS R. ; AGARWAL R.** *The Enzymes,* 1973, vol. 8, 307-334
- **GILLES A.-M. ; PRESECAN E. ; VONICA A. ; LASCU I.** *J. Biol. Chem.,* 1991, vol. 266, 8784-8789
- **BIGGS J. ; HERSPERGER E. ; STEEG P.S. ; LIOTTA L.A. ; SHEARN A.** *Cell,* 1990, vol. 63, 933-940
- **CAMPBELL W.H. ; KINGHORN J.R.** *Trends Biochem. Sci.,* 1990, vol. 15, 315-319
- **CRANE B.R. ; SIEGEL L.M. ; GETZOFF E.D.** *Science,* 1995, vol. 270, 59-67
- **GISSELMANN G. ; KLAUSMEIER P. ; SCHWENN J.D.** *Biochim. Biophys. Acta,* 1993, vol. 1144, 102-106
- **HUANG C.J. ; BARRETT E.L.** *J. Bacteriol.,* 1991, vol. 173, 1544-1553
- **RUDNICK D.A. ; MCWHERTER C.A. ; GOKEL G.W. ; GORDON J.I.** *Adv. Enzymol.,* 1993, vol. 67, 375-430
- **NAKATA P.A. ; GREENE T.W. ; ANDERSON J.M. ; SMITH-WHITE B.J. ; OKITA T.W. ; PREISS J.** *Plant Mol. Biol.,* 1991, vol. 17, 1089-1093
- **PREISS J. ; BALL K. ; HUTNEY J. ; SMITH-WHITE B.J. ; LI. L. ; OKITSA T.W.** *Pure Appl. Chem.,* 1991, vol. 63, 535-544
- **DIBROV P ; FLIEGEL L.** *FEBS Lett,* 1998, vol. 424, 1-5
- **ORLOWSKI J ; GRINSTEIN S.** *J Biol Chem,* 1997, vol. 272, 22373-22376
- **NUMATA M ; PETRECCA K ; LAKE N ; ORLOWSKI J.** *J Biol Chem,* 1998, vol. 273, 6951-6959
- **MARKOVICH D. ; FORGO J. ; STANGE G. ; BIBER J. ; MURER H.** *Proc. Natl. Acad. Sci. U.S.A.,* 1993, vol. 90, 8073-8077
- **PAJOR A.M.** *Am. J. Physiol.,* 1996, vol. 270, 642-648
- **DAVIES J.P. ; YILDIZ F.H. ; GROSSMAN A.** *EMBO J.,* 1996, vol. 15, 2150-2159
- **OUZOUNIS C ; BORK P ; SANDER C.** *Trends Biochem Sci,* 1994, vol. 19, 199-200
- **KOBAYASHI M. ; IZUI H. ; NAGASAWA T. ; YAMADA H.** *Proc. Natl. Acad. Sci. U.S.A.,* 1993, vol. 90, 247-251
- **KOBAYASHI M. ; KOMEDA H. ; YANAKA N. ; NAGASAWA T. ; YAMADA H.** *J. Biol. Chem.,* 1992, vol. 267, 20746-20751
- **WANG P. ; VANETTEN H.D.** *Biochem. Biophys. Res. Commun.,* 1992, vol. 187, 1048-1054
- **HUENGES M ; ROLZ C ; GSCHWIND R ; PETERANDERL R ; BERGLECHNER F ; RICHTER G ; BACHER A ; KESSLER H ; GEMMECKER G.** *EMBO J,* 1998, vol. 17, 4092-4100
- **STROUD R.M. ; MCCARTHY M.P. ; SHUSTER M.** *Biochemistry,* 1990, vol. 29, 11009-11023
- **BETZ H.** *Neuron,* 1990, vol. 5, 383-392
- **DINGLEDINE R. ; MYERS S.J. ; NICHOLAS R.A.** *FASEB J.,* 1990, vol. 4, 2632-2645
- **BARNARD E.A.** *Trends Biochem. Sci.,* 1992, vol. 17, 368-374
- **JACQUET M. ; GUILBAUD R. ; GARREAU H.** *Mol. Gen. Genet.,* 1988, vol. 211, 441-445
- **KIMSEY H.H. ; KAISER D.** *J. Biol. Chem.,* 1992, vol. 267, 819-824
- **ENGELBRECHT S. ; JUNGE W.** *Biochim. Biophys. Acta,* 1990, vol. 1015, 379-390
- **LERNER C.G. ; SWITZER R.L.** *J. Biol. Chem.,* 1986, vol. 261, 11156-11165

- **TAKIGUCHI M. ; MATSUBASA T. ; AMAYA Y. ; MORI M.** *BioEssays,* 1989, vol. 10, 163-166
- **BAUR H. ; STALON V. ; FALMAGNE P. ; LUETHI E. ; HAAS D.** *Eur. J. Biochem.,* 1987, vol. 166, 111-117
- **HOUGHTON J.E. ; BENCINI D.A. ; O'DONOVAN G.A. ; WILD J.R.** *Proc. Natl. Acad. Sci. U.S.A.,* 1981, vol. 81, 4864-4868
- **KE H.-M. ; HONZATKO R.B. ; LIPSCOMB W.N.** *Proc. Natl. Acad. Sci. U.S.A.,* 1984, vol. 81, 4037-4040
- **MURPHY D.J. ; KEEN J.N. ; O'SULLIVAN J.N. ; AU D.M.Y. ; EDWARDS E.-W. ; JACKSON P.J. ; CUMMINS I. ; GIBBONS T. ; SHAW C.H. ; RYAN A.J.** *Biochim. Biophys. Acta,* 1991, vol. 1088, 86-94
- **TZEN J.T.C. ; LIE G.C. ; HUANG A.H.C.** *J. Biol. Chem.,* 1992, vol. 267, 15626-15634
- **SCHOEHN G ; MOSS SR ; NUTTALL PA ; HEWAT EA.** *Virology,* 1997, vol. 235, 191-200
- **BAIROCH A.** *Unpublished observations,* 1993
- **MARTIN C. ; CAMI B. ; YEH P. ; STRAGIER P. ; PARSOT C. ; PATTE J.-C.** *Mol. Biol. Evol.,* 1988, vol. 5, 549-559
- **SANDMEIER E. ; HALE T.I. ; CHRISTEN P.** *Eur. J. Biochem.,* 1994, vol. 221, 997-1002
- **POULIN R. ; LU L. ; ACKERMANN B. ; BEY P. ; PEGG A.E.** *J. Biol. Chem.,* 1992, vol. 267, 150-158
- **MOORE R.C. ; BOYLE S.M.** *J. Bacteriol.,* 1990, vol. 172, 4631-4640
- **BUTLER W.T.** *Connect. Tissue Res.,* 1989, vol. 23, 123-36
- **GORSKI J.P.** *Calcif. Tissue Int.,* 1992, vol. 50, 391-396
- **DENHARDT D.T. ; GUO X.** *FASEB J.,* 1993, vol. 7, 1475-1482
- **JIANG B. ; BROWN J.L. ; SHERATON J. ; FORTIN N. ; BUSSEY H.** *Yeast,* 1994, vol. 10, 341-353
- **HYDE GE ; CRAWFORD NM ; CAMPBELL W.** *J Biol Chem,* 1991, vol. 266, 23542-23547
- **LU G ; CAMPBELL WH ; SCHNEIDER G ; LINDQVIST Y.** *Structure,* 1994, vol. 2, 809-821
- **WOOTTON J.C. ; NICOLSON R.E. ; COCK J.M. ; WALTERS D.E. ; BURKE J.F. ; DOYLE W.A. ; BRAY R.C.** *Biochim. Biophys. Acta,* 1991, vol. 1057, 157-185
- **WALKER JE.** *Q Rev Biophys,* 1992, vol. 25, 253-324
- **ARIZMENDI J.M. ; RUNSWICK M.J. ; SKEHEL J.M. ; WALKER J.E.** *FEBS Lett.,* 1992, vol. 301, 237-242
- **LEVINE A.J. ; MOMAND J. ; FINLAY C.A.** *Nature,* 1991, vol. 351, 453-456
- **LEVINE A.J. ; MOMAND J.** *Biochim. Biophys. Acta,* 1990, vol. 1032, 119-136
- **SOUSSI T. ; CARON DE FROMENTEL C. ; MAY P.** *Oncogene,* 1990, vol. 5, 945-952
- **LANE D.P. ; BENCHIMOL S.** *Genes Dev.,* 1990, vol. 4, 1-8
- **ULRICH S.J. ; ANDERSON C.W. ; MERCER W.E. ; APPELLA E.** *J. Biol. Chem.,* 1992, vol. 267, 15259-15262
- **DELAUNEY A.J. ; VERMA D.P.** *Mol. Gen. Genet.,* 1990, vol. 221, 299-305
- **SAVIOZ A. ; JEENES D.J. ; KOCHER H.P. ; HAAS D.** *Gene,* 1990, vol. 86, 107-111
- **TAYLOR R.G. ; LAMBERT M.A. ; SEXSMITH E. ; SADLER S.J. ; RAY P.N. ; MAHURAN D.J. ; MCINNES R.R.** *J. Biol. Chem.,* 1990, vol. 265, 18192-18199
- **LANGER M. ; RECK G. ; REED J. ; RETEY J.** *Biochemistry,* 1994, vol. 33, 6462-6467
- **SCHUSTER B. ; RETEY J.** *FEBS Lett.,* 1994, vol. 349, 252-254
- **TAYLOR R.G. ; MCINNES R.R.** *J. Biol. Chem.,* 1994, vol. 269, 27473-27477
- **ZHULIN IB ; TAYLOR BL ; DIXON R.** *Trends Biochem Sci,* 1997, vol. 22, 331-333
- **BORGSTAHL GE ; WILLIAMS DR ; GETZOFF ED.** *Biochemistry,* 1995, vol. 34, 6278-6287
- **PONTING CP ; ARAVIND L.** *Curr Biol,* 1997, vol. 7, 674-677
- **SEDDIQI N. ; BOLLENGIER F. ; ALLIEL P.M. ; PERIN J.P. ; BONNET F. ; BUCQUOY S. ; JOLLES P. ; SCHOENTGEN F.** *J. Mol. Evol.,* 1994, vol. 39, 655-660
- **SCHOENTGEN F. ; JOLLES P.** *FEBS Lett.,* 1995, vol. 369, 22-6
- **HOFMANN K ; BUCHER P.** *Trends Biochem Sci,* 1998, vol. 23, 204-205
- **KAGAN R.M. ; MCFADDEN H.J. ; MCFADDEN P.N. ; O'CONNOR C. ; CLARKE S.** *Comp. Biochem. Physiol.,* 1997, vol. 117b, 379-385
- **BRAVO R. ; FRANK R. ; BLUNDELL P.A. ; MCDONALD-BRAVO H.** *Nature,* 1987, vol. 326, 515-517
- **SUZUKA I. ; HATA S. ; MATSUOKA M. ; KOSUGI S. ; HASHIMOTO J.** *Eur. J. Biochem.,* 1991, vol. 195, 571-575
- **BAUER G.A. ; BURGESS P.M.J.** *Nucleic Acids Res.,* 1990, vol. 18, 261-265
- **O'REILLY D.R. ; CRAWFORD A.M. ; MILLER L.K.** *Nature,* 1989, vol. 337, 606-606
- **FISCHER R.S. ; ZHAO G. ; JENSEN R.A.** *J. Gen. Microbiol.,* 1991, vol. 137, 1293-1301
- **PONTING CP ; PHILLIPS C ; DAVIES KE ; BLAKE DJ.** *Bioessays,* 1997, vol. 19, 469-479
- **DOYLE DA ; LEE A ; LEWIS J ; KIM E ; SHENG M ; MACKINNON R.** *Cell,* 1996, vol. 85, 1067-1076
- **PONTING CP.** *Protein Sci,* 1997, vol. 6, 464-468

- **REIZER J. ; HOISCHEN C. ; REIZER A. ; PHAM T.N. ; SAIER M.H. JR.** *Protein Sci.,* 1993, vol. 2, 506-521
- **REIZER J. ; REIZER A. ; MERRICK M.J. ; PLUNKETT G. III ; ROSE D.J. ; SAIER M.H. JR.** *Gene,* 1996, vol. 181, 103-108
- **POCALYKO D.J. ; CARROLL L.J. ; MARTIN B.M. ; BABBITT P.C. ; DUNAWAY-MARIANO D.** *Biochemistry,* 1990, vol. 29, 10757-10765
- **NIERSBACH M. ; KREUZALER F. ; GEERSE R.H. ; POSTMA P. ; HIRSCH H.J.** *Mol. Gen. Genet.,* 1992, vol. 232, 332-336
- **MEDINA V. ; PONTAROLLO R. ; GLAESKE D. ; TABEL H. ; GOLDIE H.** *J. Bacteriol.,* 1990, vol. 172, 7151-7156
- **MATTE A. ; GOLDIE H. ; SWEET R.M. ; DELBAERE L.T.J.** *J. Mol. Biol.,* 1996, vol. 256, 126-143
- **TERADA K. ; IZUI K.** *Eur. J. Biochem.,* 1991, vol. 202, 797-803
- **JIAO J.-A. ; PODESTA F.E. ; CHOLLET R. ; O'LEARY M.H. ; ANDREO C.S.** *Biochim. Biophys. Acta,* 1990, vol. 1041, 291-295
- **MULERO J.J. ; ROSENTHAL J.K. ; FOX T.D.** *Curr. Genet.,* 1994, vol. 25, 299-304
- **POORMAN R.A. ; RANDOLPH A. ; KEMP R.G. ; HEINRIKSON R.L.** *Nature,* 1984, vol. 309, 467-469
- **HEINISCH J. ; RITZEL R.G. ; VON BORSTEL R.C. ; AGUILERA A. ; RODICIO R. ; ZIMMERMANN F.K.** *Gene,* 1989, vol. 78, 309-321
- **LE BOULCH P. ; JOULIN V. ; GAREL M.-C. ; ROSA J. ; COHEN-SOLAL M.** *Biochem. Biophys. Res. Commun.,* 1988, vol. 156, 874-881
- **WHITE M.F. ; FOTHERGILL-GILMORE L.A.** *FEBS Lett.,* 1988, vol. 229, 383-387
- **ROSE Z.B.** *Meth. Enzymol,* 1982, vol. 87, 43-51
- **BAZAN J.F. ; FLETTERICK R.J. ; PILKIS S.J.** *Proc. Natl. Acad. Sci. U.S.A.,* 1989, vol. 86, 9642-9646
- **O'TOOLE G.A. ; TRZEBIATOWSKI J.R. ; ESCALANTE-SEMERENA J.C.** *J. Biol. Chem.,* 1994, vol. 269, 26503-26511
- **GRANA X. ; DE LECEA L. ; EL-MAGHRABI M.R. ; URENA J.M. ; CAELLAS C. ; CARRERAS J. ; PUIGDOMENECH P. ; PILKIS S.J. ; CLIMENT F.** *J. Biol. Chem.,* 1992, vol. 267, 12797-12803
- **ACHARI A. ; MARSHALL S.E. ; MUIRHEWAD H. ; PALMIERI R.H. ; NOLTMANN E.A.** *Philos. Trans. R. Soc. Lond., B, Biol. Sci.,* 1981, vol. 293, 145-157
- **SMITH M.W. ; DOOLITTLE R.F.** *J. Mol. Evol.,* 1992, vol. 34, 544-545
- **FAIK P. ; WALKER J.I.H. ; REDMILL A.A.M. ; MORGAN M.J.** *Nature,* 1988, vol. 332, 455-456
- **WATSON H.C. ; LITTLECHILD J.A.** *Biochem. Soc. Trans.,* 1990, vol. 18, 187-190
- **DAI J.B. ; LIU Y. ; RAY W.J. JR. ; KONNO M.** *J. Biol. Chem.,* 1992, vol. 267, 6322-6337
- **STEVENSON G. ; LEE S.J. ; ROMANA L.K. ; REEVES P.R.** *Mol. Gen. Genet.,* 1991, vol. 227, 173-180
- **ZIELINSKI N.A. ; CHAKRABARTY A.M. ; BERRY A.** *J. Biol. Chem.,* 1991, vol. 266, 9754-9763
- **KOEPLIN R. ; ARNOLD W. ; HOETTE B. ; SIMON R. ; WANG G. ; PUEHLER A.** *J. Bacteriol.,* 1992, vol. 174, 191-199
- **BAIROCH A.** *Unpublished observations,* 1993
- **SUBRAMANIAN S.V. ; WYROBA E. ; ANDERSEN A.P. ; SATIR B.H.** *Proc. Natl. Acad. Sci. U.S.A.,* 1994, vol. 91, 9832-9836
- **MAYER B.J. ; REN R. ; CLARK K.L. ; BALTIMORE D.** *Cell,* 1993, vol. 73, 629-630
- **HASLAM R.J. ; KOIDE H.B. ; HEMMINGS B.A.** *Nature,* 1993, vol. 363, 309-310
- **MUSACCHIO A. ; GIBSON T.J. ; RICE P. ; THOMPSON J. ; SARASTE M.** *Trends Biochem. Sci.,* 1993, vol. 18, 343-348
- **GIBSON T.J. ; HYVONEN M. ; MUSACCHIO A. ; SARASTE M. ; BIRNEY E.** *Trends Biochem. Sci.,* 1994, vol. 19, 349-353
- **PAWSON T.** *Nature,* 1995, vol. 373, 573-580
- **INGLEY E. ; HEMMINGS B.A.** *J. Cell. Biochem.,* 1994, vol. 56, 436-443
- **SARASTE M. ; HYVONEN M.** *Curr. Opin. Struct. Biol.,* 1995, vol. 5, 403-408
- **RIDDIHOUGH G.** *Nat. Struct. Biol.,* 1994, vol. 1, 755-757
- **AASLAND R ; GIBSON TJ ; STEWART AF.** *Trends Biochem Sci,* 1995, vol. 20, 56-59
- **MELDRUM E. ; PARKER P.J. ; CAROZZI A.** *Biochim. Biophys. Acta,* 1991, vol. 1092, 49-71
- **RHEE S.G. ; CHOI K.D.** *Adv. Second Messenger Phosphoprotein Res.,* 1992, vol. 26, 35-61
- **RHEE S.G. ; CHOI K.D.** *J. Biol. Chem.,* 1992, vol. 267, 12393-12396
- **STERNWEIS P.C. ; SMRCKA A.V.** *Trends Biochem. Sci.,* 1992, vol. 17, 502-506
- **STERNWEIS P.C. ; SMRCKA A.V.** *Trends Biochem. Sci.,* 1992, vol. 17, 502-506
- **MUIRHEAD H.** *Biochem. Soc. Trans.,* 1990, vol. 18, 193-196
- **PONTING CP ; KERR ID.** *Protein Sci,* 1996, vol. 5, 914-922
- **KOONIN EV.** *Trends Biochem Sci,* 1996, vol. 21, 242-243
- **WANG X ; XU L ; ZHENG L.** *J Biol Chem,* 1994, vol. 269, 20312-20317

- **SINGER WD ; BROWN HA ; STERNWEIS PC.** *Annu Rev Biochem,* 1997, vol. 66, 475-509
- **PROUDFOOT A.E.I. ; TURCATTI G. ; WELLS T.N.C. ; PAYTON M.A. ; SMITH D.J.** *Eur. J. Biochem.,* 1994, vol. 219, 415-423
- **COULIN F. ; MAGNENAT E. ; PROUDFOOT A.E.I. ; PAYTON M.A. ; SCULLY P. ; WELLS T.N.C.** *Biochemistry,* 1993, vol. 32, 14139-14144
- **CLEASBY A. ; WONACOTT A. ; SKARZYNSKI T. ; HUBBARD R.E. ; DAVIES G.J. ; PROUDFOOT A.E.I. ; BERNARD A.R. ; PAYTON M.A. ; WELLS T.N.C.** *Nat. Struct. Biol.,* 1996, vol. 3, 470-479
- **TAKEHARA M. ; LING F. ; IZAWA S. ; INOUE Y. ; KIMURA A.** *Biosci. Biotechnol. Biochem.,* 1995, vol. 59, 1987-1990
- **WATANABE S.-I. ; HINO A. ; WADA K. ; ELIASON J.F. ; UCHIDA T.** *J. Biol. Chem.,* 1995, vol. 270, 12191-12196
- **CACCIAPUOTI G. ; PORCELLI M. ; BERTOLDO C. ; DE ROSA M. ; ZAPPIA V.** *J. Biol. Chem.,* 1994, vol. 269, 24762-24769
- **WENK J. ; TROMPETER H.-I. ; PETTRICH K.-G. ; COHEN P.T.W. ; CAMPBELL D.G. ; MIESKES G.** *FEBS Lett.,* 1992, vol. 297, 135-138
- **MAEDA T. ; TSAI A.Y.M. ; SAITO H.** *Mol. Cell. Biol.,* 1993, vol. 13, 5408-5417
- **STONE J.M. ; COLLINGE M.A. ; SMITH R.D. ; HORN M.A. ; WALKER J.C.** *Science,* 1994, vol. 266, 793-795
- **LAWSON J.E. ; NIU X.-D. ; BROWNING K.S. ; TRONG H.L. ; YAN J. ; REED L.J.** *Biochemistry,* 1993, vol. 32, 8987-8993
- **DAS A.K. ; HELPS N.R. ; COHEN P.T.W. ; BARFORD D.** *EMBO J.,* 1996, vol. 24, 6798-6809
- **BORK P. ; BROWN N.P. ; HEGYI H. ; SCHULTZ J.** *Protein Sci.,* 1996, vol. 5, 1421-1425
- **BOGUSKI M.S. ; MURRAY A.W. ; POWERS S.** *New Biol.,* 1992, vol. 4, 408-411
- **MAYER-JAEKEL R. ; HEMMINGS B.A.** *Trends Cell Biol.,* 1994, vol. 4, 287-291
- **WILMANNS M ; PRIESTLE JP ; NIERMANN T ; JANSONIUS JN.** *J Mol Biol,* 1992, vol. 223, 477-507
- **KOSSMANN J. ; KLINTWORTH R. ; BOWIEN B.** *Gene,* 1989, vol. 85, 247-252
- **GIBSON J.L. ; CHEN J.-H. ; TOWER P.A. ; TABITA F.R.** *Biochemistry,* 1990, vol. 29, 8085-8093
- **BOWER S.G. ; HARLOW K.W. ; SWITZER R.L. ; HOVEN-JENSEN B.** *J. Biol. Chem.,* 1989, vol. 264, 10287-10291
- **YU D ; WELLER SK.** *J Virol,* 1998, vol. 72, 7428-7439
- **GOLBECK J.H.** *Biochim. Biophys. Acta,* 1987, vol. 895, 167-204
- **KJAERULFF S. ; ANDERSEN B. ; NIELSEN V.S. ; MOLLER B.L. ; OKKELS J.S.** *J. Biol. Chem.,* 1993, vol. 268, 18912-18916
- **PAULSEN I.T. ; SKURRAY R.A.** *Trends Biochem. Sci.,* 1994, vol. 19, 404-404
- **STEINER H.-Y. ; NAIDER F. ; BECKER J.M.** *Mol. Microbiol.,* 1995, vol. 16, 825-834
- **ZHANG B ; GALLEGOS M ; PUOTI A ; DURKIN E ; FIELDS S ; KIMBLE J ; WICKENS MP.** *Nature,* 1997, vol. 390, 477-484
- **ZAMORE PD ; WILLIAMSON JR ; LEHMANN R.** *RNA,* 1997, vol. 3, 1421-1433
- **STEC I ; WRIGHT TJ ; VAN OMMEN GJB ; DE BOER PAJ ; VAN HAERINGEN A ; MOORMAN AFM ; ALTHERR MR ; DEN DUNNEN JT.** *Hum Mol Genet,* 1998, vol. 7, 1071-1082
- **PONTING CP.** *Protein Sci,* 1996, vol. 5, 2353-2357
- **MOTALLEBI-VESHAREH M ; ROUCH DA ; THOMAS CM.** *Mol Microbiol,* 1990, vol. 4, 1455-1463
- **RAY J. ; KNAPP J. ; GRIERSON D. ; BIRD C. ; SCHUCH W.** *Eur. J. Biochem.,* 1988, vol. 174, 119-124
- **PLASTOW G.S.** *Mol. Microbiol.,* 1988, vol. 2, 247-254
- **MARKOVIC O. ; JOERNVALL H.** *Protein Sci.,* 1992, vol. 1, 1288-1292
- **BLACK K ; BUIKEMA WJ ; HASELKORN R.** *J Bacteriol,* 1995, vol. 177, 6440-6448
- **BATEMAN A ; MURZIN A ; TEICHMANN SA.** *Protein Sci,* 1998, vol. 7, 1477-1480
- **KIESELBACH T ; MANT A ; ROBINSON C ; SCHRODER WP.** *FEBS Lett,* 1998, vol. 428, 241-244
- **MEINNEL T ; BLANQUET S ; DARDEL F.** *J Mol Biol,* 1996, vol. 262, 375-386
- **DARDEL F ; RAGUSA S ; LAZENNEC C ; BLANQUET S ; MEINNEL T.** *J Mol Biol,* 1998, vol. 280, 501-513
- **GARCIA-VILLEGAS M.R. ; DE LA VEGA F.M. ; GALINDO J.M. ; SEGURA M. ; BUCKINGHAM R.H. ; GUAMEROS G.** *EMBO J.,* 1991, vol. 10, 3549-3555
- **DE LA VEGA F.M. ; GALINDO J.M. ; OLD I.G. ; GUAMEROS G.** *Gene,* 1996, vol. 169, 97-100
- **OUZOUNIS C. ; BORK P. ; CASARI G. ; SANDER C.** *Protein Sci.,* 1995, vol. 4, 2424-2428
- **MATSUSHIMA M. ; TAKAHASHI T. ; ICHINOSE M. ; MIKI K. ; KUROKAWA K. ; TAKAHASHI K.** *Biochem. Biophys. Res. Commun.,* 1991, vol. 178, 1459-1464
- **BURLEY S.K. ; DAVID P.R. ; SWEET R.M. ; TAYLOR A. ; LIPSCOMB W.N.** *J. Mol. Biol.,* 1992, vol. 224, 113-140
- **WOOD D.O. ; SOLOMON M.J. ; SPEED R.R.** *J. Bacteriol.,* 1993, vol. 175, 159-165
- **RAWLINGS N.D. ; BARRETT A.J.** *Meth. Enzymol.,* 1995, vol. 248, 183-228

- **SHIEH HS ; KURUMBAIL RG ; STEVENS AM ; STEGEMAN RA ; STURMAN EJ ; PAK JY ; WITTWER AJ ; PALMIER MO ; WIEGAND RC ; HOLWERDA BC.** *Nature,* 1996, vol. 383, 279-282
- **VILLALBA M. ; BATANERO E. ; LOPEZ-OTIN C. ; SANCHEZ L.M. ; MONSALVE R.I. ; GONZALEZ DE LA PENA M.A. ; LAHOZ C. ; RODRIGUEZ R.** *Eur. J. Biochem.,* 1993, vol. 216, 863-869
- **ANSARI AA ; SHENBAGAMURTHI P ; MARSH DG.** *Biochemistry,* 1989, vol. 28, 8665-8670
- **MILLER A.D. ; HART G.J. ; PACKMAN L.C. ; BATTERSBY A.R.** *Biochem. J.,* 1988, vol. 254, 915-918
- **KIM TW ; TANZI RE.** *Curr Opin Neurobiol,* 1997, vol. 7, 683-688
- **ZHANG W ; HAN SW ; MCKEEL DW ; GOATE A ; WU JY.** *J Neurosci,* 1998, vol. 18, 914-922
- **ZHANG Z ; HARTMANN H ; DO VM ; ABRAMOWSKI D ; STURCHLER-PIERRAT C ; STAUFENBIEL M ; SOMMER B ; VAN DE WETERING M ; CLEVERS H ; SAFTIG P.** *Nature,* 1998, vol. 395, 698-702
- **HERSHEY H.V. ; TAYLOR M.W.** *Gene,* 1986, vol. 43, 287-293
- **GUILLOTON M.B. ; KORTE J.J. ; LAMBLIN A.F. ; FUCHS J.A. ; ANDERSON P.M.** *J. Biol. Chem.,* 1992, vol. 267, 3731-3734
- **FUKUZAWA H. ; SUZUKI E. ; KOMUKAI Y. ; MIYACHI S.** *Proc. Natl. Acad. Sci. U.S.A.,* 1992, vol. 89, 4437-4441
- **RAWLINGS N.D. ; POLGAR L. ; BARRETT A.J.** *Biochem. J.,* 1991, vol. 279, 907-911
- **RAWLINGS N.D. ; BARRETTA.J.** *Meth. Enzymol.,* 1994, vol. 244, 19-61
- **CRONK JD ; ENDRIZZI JA ; ALBER T.** High-resolution structures of the bifunctional enzyme and transcriptional coactivator DCoH and its complex with a product analogue. *Protein Sci,* 1996, vol. 5, 1963-1972
- **LAM H.-M. ; WINKLER M.E.** *J. Bacteriol.,* 1992, vol. 174, 6033-6045
- **LOUBBARDI A. ; KARST F. ; GUILLOTON M. ; MARCIREAU C.** *J. Bacteriol.,* 1995, vol. 177, 1817-1823
- **PIERSON L.S. III ; GAFFNEY T. ; LAM S. ; GONG F.** *FEMS Microbiol. Lett.,* 1995, vol. 134, 299-307
- **LAHTI R. ; KOLAKOWSKI L.F. JR. ; HEINONEN J. ; VIHINEN M. ; POHJANOKSA K. ; COOPERMAN B.S.** *Biochim. Biophys. Acta,* 1990, vol. 1038, 338-345
- **COOPERMAN B.S. ; BAYKOV A.A. ; LAHTI R.** *Trends Biochem. Sci.,* 1992, vol. 17, 262-266
- **DALBEY R.E. ; VON HEIJNE G.** *Trends Biochem. Sci.,* 1992, vol. 17, 474-478
- **SUNG M. ; DALBEY R.E.** *J. Biol. Chem.,* 1992, vol. 267, 13154-13159
- **BLACK M.T.** *J. Bacteriol.,* 1993, vol. 175, 4957-4961
- **NUNNARI J. ; FOX T.D. ; WALTER P.** *Science,* 1993, vol. 262, 1997-2004
- **VAN DIJL J.M. ; DE JONG A. ; VEHMAANPERA J. ; VENEMA G. ; BRON S.** *EMBO J.,* 1992, vol. 11, 2819-2828
- **RAWLINGSN.D. ; BARRETTA.J.** *Meth. Enzymol.,* 1994, vol. 244, 19-61
- **YOSHIMOTO T. ; TONE H. ; HONDA T. ; OSATOMI K. ; KOBAYASHI R. ; TSURU D. J.** *Biochem.,* 1989, vol. 105, 412-416
- **NAKAHIGASHI K. ; INOKUCHI H.** *Nucleic Acids Res.,* 1990, vol. 18, 6439-6439
- **ENDO F. ; TANOUE A. ; NAKAI H. ; HATA A. ; INDO Y. ; TITANI K. ; MATSUDA I.** *J. Biol. Chem.,* 1989, vol. 264, 4476-4481
- **ARFIN S.M. ; KENDALL R.L. ; HALL L. ; WEAVER L.H. ; STEWART A.E. ; MATTHEWS B.W. ; BRADSHAW R.A.** *Proc. Natl. Acad. Sci. U.S.A.,* 1995, vol. 92, 7714-7718
- **DOOLITTLE W.F.** *Trends Biochem. Sci.,* 1996, vol. 21, 285-286
- **RODERICK S.L. ; MATHEWS B.W.** *Biochemistry,* 1993, vol. 32, 3907-3912
- **NEBERT D.W. ; GONZALEZ F.J.** *Annu. Rev. Biochem.,* 1987, vol. 56, 945-993
- **COON M.J. ; DING X. ; PERNECKY S.J. ; VAZ A.D.N.** *FASEB J.,* 1992, vol. 6, 669-673
- **GUENGERICH F.P.** *J. Biol. Chem.,* 1991, vol. 266, 10019-10022
- **NELSON D.R. ; KAMATAKI T. ; WAXMAN D.J. ; GUENGERICH F.P. ; ESTRABROOK R.W. ; FEYEREISEN R. ; GONZALEZ F.J. ; COON M.J. ; GUNSALUS I.C. ; GOTOH O.** *DNA Cell Biol.,* 1993, vol. 12, 1-51
- **DEGTYARENKO K.N. ; ARCHAKOV A.I.** *FEBS Lett.,* 1993, vol. 332, 1-8
- **YODER MD ; KEEN NT ; JURNAK F.** *Science,* 1993, vol. 260, 1503-1507
- **KEELING P.J. ; DOOLITTLE W.F.** *Trends Biochem. Sci.,* 1996, vol. 21, 285-286
- **DAWSON J.H.** *Science,* 1988, vol. 240, 433-439
- **KIMURA S. ; IKEDA-SAITO M.** *Proteins,* 1988, vol. 3, 113-120
- **HENRISSAT B. ; SALOHEIMO M. ; LAVAITTE S. ; KNOWLES J.K.C.** *Proteins,* 1990, vol. 8, 251-257
- **WELINDER K.G.** *Biochim. Biophys. Acta,* 1991, vol. 1080, 215-220
- **WU L.-F. ; REIZER A. ; REIZER J. ; CAI B. ; TOMICH J.M. ; SAIER M.H. JR.** *J. Bacteriol.,* 1991, vol. 173, 3117-3127
- **ORCHARD L.M.D. ; KORNBERG H.L.** *Proc. R. Soc. Lond., B, Biol. Sci.,* 1990, vol. 242, 87-90
- **BLATCH G.L. ; SCHOLLE R.R. ; WOODS D.R.** *Gene,* 1990, vol. 95, 17-23

- **DAVIDSONF.F. ; DENNIS E.A.** *J. Mol. Evol.,* 1990, vol. 31, 228-238
- **GOMEZ F. ; VANDERMEERS A. ; VANDERMEERS-PIRET M.-C. ; HERZOG R. ; RATHE J. ; STIEVENART M. ; WINAND J. ; CHRISTOPHE J.** *Eur. J. Biochem.,* 1989, vol. 186, 23-33
- **FUKUI T. ; SHIMOMURA S. ; NAKANO K.** *Mol. Cell. Biochem.,* 1982, vol. 42, 129-144
- **HANKS S.K. ; HUNTER T.** *FASEB J.,* 1995, vol. 9, 576-596
- **HUNTER T.** *Meth. Enzymol.,* 1991, vol. 200, 3-37
- **HANKS S.K. ; QUINN A.M.** *Meth. Enzymol.,* 1991, vol. 200, 38-62
- **HANKS S.K.** *Curr. Opin. Struct. Biol,* 1991, vol. 1, 369-383
- **HANKS S.K. ; QUINN A.M. ; HUNTER T.** *Science,* 1988, vol. 241, 42-52
- **KNIGHTON D.R. ; ZHENG J. ; TEN EYCK L.F. ; ASHFORD V.A. ; XUONG N.-H. ; TAYLOR S.S. ; SOWADSKI J.M.** *Science,* 1991, vol. 253, 407-414
- **BAIROCH A. ; CLAVERIE J.-M.** *Nature,* 1988, vol. 331, 22
- **BENNER S.** *Nature,* 1987, vol. 329, 21-21
- **KIRBY R. J.** *Mol. Evol.,* 1992, vol. 30, 489-492
- **LITTLER E. ; STUART A.D. ; CHEE M.S.** *Nature,* 1992, vol. 358, 160-162
- **MUNOZ-DORADO J. ; INOUYE S. ; INOUYE M.** *Cell,* 1991, vol. 67, 995-1006
- **YARDEN Y. ; ULLRICH A.** *Annu. Rev. Biochem.,* 1988, vol. 57, 443-478
- **HUNKAPILLER T. ; HOOD L.** *Adv. Immunol.,* 1989, vol. 44, 1-63
- **LEE K.-H. ; BOWEN-POPE D.F. ; REED R.R.** *Mol. Cell. Biol.,* 1990, vol. 10, 2237-2246
- **TERMAN B.I. ; DOUGHER-VERMAZEN M. ; CARRION M.E. ; DIMITROV D. ; ARMELLINO D.C. ; GOSPODAROWICZ D. ; BOEHLEN P.** *Biochem. Biophys. Res. Commun.,* 1992, vol. 187, 1579-1586
- **LYMAN S.D. ; JAMES L. ; VANDEN BOS T. ; DE VRIES P. ; BRASEL K. ; GLINIAK B. ; HOLLINGSWORTH L.T. ; PICHA K.S. ; MCKENNA H.J. ; SPLETT R.R.** *Cell,* 1993, vol. 75, 1157-1167
- **GALLAND F. ; KARAMYSHEVA A. ; PEBUSQUE M.J. ; BORG J.P. ; ROTTAPEL R. ; DUBREUIL P. ; ROSNET O. ; BIRNBAUM D.** *Oncogene,* 1993, vol. 8, 1233-1240
- **SAJJADI F.G. ; PASQUALE E.B. ; SUBRAMANI S.** *New Biol.,* 1991, vol. 3, 769-778
- **WICKS I.P. ; WILKINSON D. ; SALVARIS E. ; BOYD A.W.** *Proc. Natl. Acad. Sci. U.S.A.,* 1992, vol. 89, 1611-1615
- **VERNON L.P. ; EVETT G.E. ; ZEIKUS R.D. ; GRAY W.R.** *Arch. Biochem. Biophys.,* 1985, vol. 238, 18-29
- **BOHLMANN H. ; CLAUSEN S. ; BEHNKE S. ; GIESE H. ; HILLER C. ; REIMANN-PHILLIP U. ; SCHRADER G. ; BARKHOLT V. ; APEL K.** *EMBO J.,* 1988, vol. 7, 1559-1565
- **BOHLMANN H. ; APEL K.** *Mol. Gen. Genet.,* 1987, vol. 207, 446-454
- **TEETER M.M. ; MAZER J.A. ; L'ITALIEN J.J.** *Biochemistry,* 1981, vol. 20, 5437-5443
- **ASHBY M.N. ; EDWARDS P.A.** *J. Biol. Chem.,* 1990, vol. 265, 13157-13164
- **FUJISAKI S. ; HARA H. ; NISHIMURA Y. ; HORIUCHI K. ; NISHINO T. J.** *Biochem.,* 1990, vol. 108, 995-1000
- **CARATTOLI A. ; ROMANO N. ; BALLARIO P. ; MORELLI G. ; MACINO G.** *J. Biol. Chem.,* 1991, vol. 266, 5854-5859
- **KUNTZ M. ; ROEMER S. ; SUIRE C. ; HUGUENEY P. ; WEIL J.H. ; SCHANTZ R. ; CAMARA B.** *Plant J.,* 1992, vol. 2, 25-34
- **MATH S.K. ; HEARST J.E. ; POULTER C.D.** *Proc. Natl. Acad. Sci. U.S.A.,* 1992, vol. 89, 6761-6764
- **SVENDSEN I. ; HEJGAARD J. ; CHAVAN J.K.** *Carlsberg Res. Commun.,* 1984, vol. 49, 493-502
- **SVENDSEN I. ; BOISEN S. ; HEJGAARD J.** *Carlsberg Res. Commun.,* 1982, vol. 47, 45-53
- **NOZAWA H. ; YAMAGATA H. ; AIZONO Y. ; YOSHIKAWA M. ; IWASAKI T. J.** *Biochem.,* 1989, vol. 106, 1003-1008
- **CLEVELAND T.E. ; THORNBURG R.W. ; RYAN C.A.** *Plant Mol. Biol.,* 1987, vol. 8, 199-207
- **LEE J.S. ; BROWN W.E. ; GRAHAM J.S. ; PEARCE G. ; FOX E.A. ; DREHER T.W. ; AHERN K.G. ; PEARSON G.D. ; RYAN C.A.** *Proc. Natl. Acad. Sci. U.S.A.,* 1986, vol. 83, 7277-7281
- **SEEMULLER U. ; EULITZ M. ; FRITZ H. ; STROBL A. ; HOPPE-SEYLER'S Z.** *Physiol. Chem.,* 1980, vol. 361, 1841-1846
- **ZENG F.-Y. ; QIAN R.-Q. ; WANG Y.** *FEBS Lett.,* 1988, vol. 234, 35-38
- **PUGH E.L. ; WAKIL S.J.** *J. Biol. Chem.,* 1965, vol. 240, 4727-4733
- **WITKOWSKI A. ; RANGAN V.S. ; RANDHAWA Z.1. ; AMY C.M. ; SMITH S.** *Eur. J. Biochem.,* 1991, vol. 198, 571-579
- **SCOTTI C. ; PIATTI M. ; CUZZONI A. ; PERANI P. ; TOGNONI A. ; GRANDI G. ; GALIZZI A. ; ALBERTINI A.M.** *Gene,* 1993, vol. 130, 65-71
- **SACKMANN U. ; ZENSEN R. ; ROHLEN D. ; JAHNKE U. ; WEISS H.** *Eur. J. Biochem.,* 1991, vol. 200, 463-469
- **COREY E.J. ; MATSUDA S.P.T. ; BARTEL B.** *Proc. Natl. Acad. Sci. U.S.A.,* 1993, vol. 90, 11628-11632
- **STAHL N. ; PRUSINER S.B.** *FASEB J.,* 1991, vol. 5, 2799-2807

- **BRUNORI M. ; CHIARA SILVESTRINI M. ; POCCHIARI M.** *Trends Biochem. Sci.,* 1988, vol. 13, 309-313
- **PRUSINER S.B.** *Annu. Rev. Microbiol.,* 1989, vol. 43, 345-374
- **STAMNES M.A. ; RUTHERFORD S.L. ; ZUKER C.S.** *Trends Cell Biol.,* 1992, vol. 2, 272-276
- **TRANDINH C.C. ; PAO G.M. ; SAIER M.H. JR.** *FASEB J.,* 1992, vol. 6, 3410-3420
- **WU J. ; MATUNIS M.J. ; KRAEMER D. ; BLOBEL G. ; COUTAVAS E.** *J. Biol. Chem.,* 1995, vol. 270, 14209-14213
- **HAARER B.K. ; BROWN S.S.** *Cell Motil. Cytoskeleton,* 1990, vol. 17, 71-74
- **SOHN R.H. ; GOLDSCHMIDT-CLERMONT P.** *BioEssays,* 1994, vol. 16, 465-472
- **OLIVA R. ; GOREN R. ; DIXON G.H.** *J. Biol. Chem.,* 1989, vol. 264, 17627-17630
- **RIVETT A.J.** *Biochem. J.,* 1993, vol. 291, 1-10
- **RIVETT A.J.** *Arch. Biochem. Biophys.,* 1989, vol. 268, 1-8
- **GOLDBERG A.L. ; ROCK K.L.** *Nature,* 1992, vol. 357, 375-379
- **WILK S.** *Enzyme Protein,* 1993, vol. 47, 187-188
- **HILT W. ; WOLF D.H.** *Trends Biochem. Sci.,* 1996, vol. 21, 96-102
- **KURLYAN J. ; KRISHNA T.S.R. ; WONG L. ; GUENTHER B. ; PAHLER A. ; WILLIAMS C.H. JR. ; MODEL P.** *Nature,* 1991, vol. 352, 172-174
- **RICE D.W. ; SCHULZ G.E. ; GUEST J.R.** *J. Mol. Biol.,* 1984, vol. 174, 483-496
- **BROWN N.L.** *Trends Biochem. Sci.,* 1985, vol. 10, 400-402
- **CAROTHERS D.J. ; PONS G. ; PATEL M.S.** *Arch. Biochem. Biophys,* 1989, vol. 268, 409-425
- **WALSH C.T. ; BRADLEY M. ; NADEAU K.** *Trends Biochem. Sci.,* 1991, vol. 16, 305-309
- **GASDASKA P.Y. ; GASDASKA J.R. ; COCHRAN S. ; POWIS G.** *FEBS Lett.,* 1995, vol. 373, 5-9
- **CREISSEN G. ; EDWARDS E.A. ; ENARD C. ; WELLBURN A. ; MULLINEAUX P.** *Plant J.,* 1991, vol. 2, 129-131
- **JACKSONF.R.** *J. Mol. Evol.,* 1990, vol. 31, 325-329
- **JOSEPH D.R. ; SULLIVAN P. ; WANG Y.-M. ; KOZAK C. ; FENSTERMACHER D.A. ; BEHRENDSEN M.E. ; ZAHNOW C.A.** *Proc. Natl. Acad. Sci. U.S.A.,* 1990, vol. 87, 733-737
- **ISHII S. ; MIZUGICHI H. ; NISHINO J. ; HAYASHI H. ; KAGAMIYAMA H. J.** *Biochem.,* 1996, vol. 120, 369-376
- **DASSO M.** *Trends Biochem. Sci.,* 1993, vol. 18, 96-101
- **BOGUSKI M.S. ; MCCORMICK F.** *Nature,* 1993, vol. 366, 643-654
- **ROEPMAN R. ; VAN DUIJNHOVEN G. ; ROSENBERG T. ; PINCKERS A.J.L.G. ; BLEEKER-WAGEMAKERS L.M. ; BERGEN A.A.B. ; POST J. ; BECK A. ; REINHARDT R. ; ROPERS H.-H.** *Hum. Mol. Genet.,* 1996, vol. 5, 1035-1041
- **GENSCHIK P. ; BILLY E. ; SWIANIEWICZ M. ; FILIPOWICZ W.** *EMBO J.,* 1997, vol. 16, 2955-2967
- **FILIPOWICZ W. ; VINCENTE O.** *Meth. Enzymol.,* 1990, vol. 181, 499-510
- **TATE W.P. ; POOLE E.S. ; MANNERING S.M.** *Prog. Nucleic Acids. Res. Mol. Biol.,* 1996, vol. 52, 293-335
- **CRAIGEN W.J. ; LEE C.C. ; CASKEY C.T.** *Mol. Microbiol.,* 1990, vol. 4, 861-865
- **PEL H.J. ; REP M. ; GRIVELL L.A.** *Nucleic Acids Res.,* 1992, vol. 20, 4423-4428
- **ENDO Y. ; TSURUGI K. ; TAKEDA Y. ; OGASAWARA T. ; IGARASHI K.** *Eur. J. Biochem.,* 1988, vol. 171, 45-50
- **MAY M.J. ; HARTLEY M.R. ; ROBERTS L.M. ; KRIEG P.A. ; OSBORN R.W. ; LORD J.M.** *EMBO J.,* 1989, vol. 8, 301-308
- **FUNATSU G. ; ISLAM M.R. ; MINAMI Y. ; SUNG-SIL K. ; KIMURA M.** *Biochimie,* 1991, vol. 73, 1157-1161
- **STROCKBINE N.A. ; JACKSON M.P. ; SUNG L.M. ; HOLMES R.K. ; O'BRIEN A.D.** *J. Bacteriol.,* 1988, vol. 170, 1116-1122
- **CALDERWOOD S.B. ; AUCLAIR F. ; DONOHUE-ROLFE A. ; KEUSCH G.T. ; MEKALANOS J.J.** *Proc. Natl. Acad. Sci. U.S.A.,* 1987, vol. 84, 4364-4368
- **JACKSON M.P. ; NEILL R.J. ; O'BRIEN A.D. ; HOLMES R.K. ; NEWLAND J.W.** *FEMS Microbiol. Lett.,* 1987, vol. 44, 109-114
- **BARBIERI L. ; BATTELLI M.G. ; STIRPE F.** *Biochim. Biophys. Acta,* 1993, vol. 1154, 237-282
- **HOVDE C.J. ; CALDERWOOD S.B. ; MEKALANOS J.J. ; COLLIER R.J.** *Proc. Natl. Acad. Sci. U.S.A.,* 1988, vol. 85, 2568-2572
- **MONZINGO A.F. ; COLLINS E.J. ; ERNST S.R. ; IRVIN J.D. ; ROBERTUS J.D.** *J. Mol. Biol.,* 1993, vol. 233, 705-715
- **ZHANG G ; DARST SA.** *Science,* 1998, vol. 281, 262-266
- **JEON YH ; NEGISHI T ; SHIRAKAWA M ; YAMAZAKI T ; FUJITA N ; ISHIHAMA A ; KYOGOKU Y.** *Science,* 1995, vol. 270, 1495-1497
- **EBRIGHT RH ; BUSBY S.** *Curr Opin Genet Dev,* 1995, vol. 5, 197-203
- **MURAKAMI K ; KIMURA M ; OWENS JT ; MEARES CF ; ISHIHAMA A.** *Proc Natl Acad Sci USA,* 1997, vol. 94, 1709-1714
- **FALKENBURG D ; DWORNICZAK B ; FAUST DM ; BAUTZ EK.** *J Mol Biol,* 1987, vol. 195, 929-937

- **KLENK H.-P. ; PALM P. ; LOTTSPEICH F. ; ZILLIG W.** *Proc. Natl. Acad. Sci. U.S.A.,* 1992, vol. 89, 407-410
- **THIRU A. ; HODACH M. ; ELORANTA J.J. ; KOSTOUROU V. ; WEINZIERL R.O. ; MATTHEWS S.** *J. Mol. Biol.,* 1999, vol. 287, 753-760
- **LU Z. ; KUTISH G.F. ; SUSSMAN M.D. ; ROCK D.L.** *Nucleic Acids Res.,* 1993, vol. 21, 2940-2940
- **MCKUNE K. ; WOYCHIK N.A.** *J. Bacteriol.,* 1994, vol. 176, 4754-4756
- **DEQUARD-CHABLAT M. ; RIVA M. ; CARLES C. ; SENTENAC A.** *J. Biol. Chem.,* 1991, vol. 266, 15300-15307
- **WOYCHIK N.A. ; MCKUNE K. ; LANE W.S. ; YOUNG R.A.** *Gene Expr.,* 1993, vol. 3, 77-82
- **LANGER D. ; HAIN J. ; THURIAUX P. ; ZILLIG W.** *Proc. Natl. Acad. Sci. U.S.A.,* 1995, vol. 92, 5768-5772
- **YANEZ R.J. ; RODRIGUEZ J.M. ; NOGAL M.L. ; YUSTE L. ; ENRIQUEZ C. ; RODRIGUEZ J.F. ; VINUELA E.** *Virology,* 1995, vol. 208, 249-278
- **MIAN IS.** *Nucleic Acids Res,* 1997, vol. 25, 3187-3189
- **KELLY K.O. ; DEUTSCHER M.P.** *J. Biol. Chem.,* 1992, vol. 267, 17153-17158
- **DI MATTEO G ; FUSCHI P ; ZERFASS K ; MORETTI S ; RICORDY R ; CENCIARELLI C ; TRIPODI M ; JANSEN-DURR P ; LAVIA P.** *Cell Growth Differ,* 1995, vol. 6, 1213-1224
- **WESTLEY J.** *Meth. Enzymol.,* 1981, vol. 77, 285-291
- **WEILAND K.L. ; DOOLEY T.P.** *Biochem. J.,* 1991, vol. 275, 227-231
- **RUDD K.E.** *Unpublished observations,* 1993
- **DONADIO S. ; SHAFIEE A. ; HUTCHINSON C.R.** *J. Bacteriol,* 1990, vol. 172, 350-360
- **LAUDENBACH D.E. ; EHRHARDT D. ; GREEN L. ; GROSSMAN A.R.** *J. Bacteriol.,* 1991, vol. 173, 2751-2760
- **NASHIMOTO H. ; UCHIDA H.** *Mol. Gen. Genet.,* 1985, vol. 201, 25-29
- **MIAN I.S.** *Nucleic Acids Res.,* 1997, vol. 25, 3187-3195
- **GATTI F.L. ; MEINHARDT S.W. ; OHNISHI T. ; TZAGOLOFF A.** *J. Mol. Biol.,* 1989, vol. 205, 421-435
- **KALLAS T. ; SPILLER S. ; MALKIN R.** *Proc. Natl. Acad. Sci. U.S.A.,* 1988, vol. 85, 5794-5798
- **IWATA S. ; SAYNOVITS M. ; LINK T.A. ; MICHEL H.** *Structure,* 1996, vol. 4, 567-579
- **NIKONOV S.V. ; NEVSKAYA N. ; ELISEIKINA I.A. ; FOMENKOVA N.P. ; NIKULIN A. ; OSSINA N. ; GARBER M. ; JONSSON B.-H. ; BRIAND C. ; AL-KARADAGHI S.** *EMBO J.,* 1996, vol. 15, 1350-1359
- **OLVERA J. ; WOOL I.G.** *2.3.CO;2-''Biochem. Biophys. Res. Commun.,* 1996, vol. 220, 954-957
- **CHAN Y.-L. ; DIAZ J.-J. ; DENOROY L. ; MADJAR J.-J. ; WOOL I.G.** *2.3.CO;2-''Biochem. Biophys. Res. Commun.,* 1996, vol. 255, 952-956
- **PUCCIARELLI G. ; REMACHA M. ; BALLESTA J.P.G.** *Nucleic Acids Res.,* 1990, vol. 18, 4409-4416
- **OTAKA E. ; HASHIMOTO T. ; MIZUTA K. ; SUZUKI K.** *Protein Seq. Data Anal.,* 1993, vol. 5, 301-313
- **CHOLI T.** *Biochem. Int.,* 1989, vol. 19, 1323-1338
- **LEIJONMARCK M ; LILJAS A.** *J Mol Biol,* 1987, vol. 195, 555-579
- **CHAN Y.-L. ; OLVERA J. ; GLUECK A. ; WOOL I.G.** *J. Biol. Chem.,* 1994, vol. 269, 5589-5594
- **OLVERA J. ; WOOL I.G.** *Biochem. Biophys. Res. Commun.,* 1994, vol. 201, 102-107
- **ZWICKL P. ; LUPAS A. ; BAUMEISTER W.** *Biochem. Biophys. Res. Commun.,* 1995, vol. 209, 684-688
- **PUDER M. ; BARNARD G.F. ; STANIUNAS R.J. ; STEELE G.D. JR. ; CHEN L.B.** *Biochim. Biophys. Acta,* 1993, vol. 1216, 134-136
- **MICHAEL WM ; DREYFUSS G.** *J Biol Chem,* 1996, vol. 271, 11571-11574
- **CHAN Y.-L. ; LIN A. ; MCNALLY J. ; PELEG D. ; MEYUHAS O. ; WOOL I.G.** *J. Biol. Chem.,* 1987, vol. 262, 1111-1115
- **HART K. ; KLEIN T. ; WILCOX M.** *Mech. Dev.,* 1993, vol. 43, 101-110
- **SINGLETON C.K. ; MANNING S.S. ; KEN R.** *Nucleic Acids Res.,* 1989, vol. 17, 9679-9692
- **RAFTI F. ; GARGIULO G. ; MANZI A. ; MALVA C. ; GRAZIANI F.** *Nucleic Acids Res.,* 1989, vol. 17, 456-456
- **PRESUTTI C. ; VILLA T. ; BOZZONI I.** *Nucleic Acids Res.,* 1993, vol. 21, 3900-3900
- **BAGNI C. ; MARIOTTINI P. ; ANNESI F. ; AMALDI F.** *Biochim. Biophys. Acta,* 1993, vol. 1216, 475-478
- **ARNDT E. ; KROEMER W. ; HATAKEYAMA T.** *J. Biol. Chem.,* 1990, vol. 265, 3034-3039
- **MARTY I. ; MEYER Y.** *Nucleic Acids Res.,* 1992, vol. 20, 1517-1522
- **DEVI K.R.G. ; CHAN Y.-L. ; WOOL I.G.** *Biochem. Biophys. Res. Commun.,* 1989, vol. 162, 364-370
- **PETTER R. ; ROZENBLATT S. ; NUCHAMOWITZ Y. ; MIRELMAN D.** *Mol. Biochem. Parasitol.,* 1992, vol. 56, 329-333
- **JANK B. ; WALDHERR M. ; SCHWEYEN R.J.** *Curr. Genet.,* 1993, vol. 23, 15-18
- **HATAKEYAMA T. ; KIMURA M.** *Eur. J. Biochem.,* 1988, vol. 172, 703-711
- **GANTT J.S. ; BALDAUF S.L. ; CALIE P.J. ; WEEDEN N.F. ; PALMER J.D.** *EMBO J.,* 1991, vol. 10, 3073-3078

- **MADSEN L.H. ; KREIBERG J.D. ; GAUSING K.** *Curr. Genet.,* 1991, vol. 19, 417-422
- **BARADI T.T.A.L. ; RAUE H.A. ; VAN DE REGT C.H.F. ; VERBREE E.C. ; PLANTA R.J.** *EMBO J.,* 1985, vol. 4, 210-2107
- **RAUE H.A. ; OTAKA E. ; SUZUKI K.** *J. Mol. Evol.,* 1989, vol. 28, 418-426
- **FEARON K. ; MASON T.L.** *J. Biol. Chem.,* 1992, vol. 267, 5162-5170
- **OTAKA E. ; HASHIMOTO T. ; MIZUTA K.** *Protein Seq. Data Anal.,* 1993, vol. 5, 285-300
- **OUZOUNIS C. ; KYRPIDES N. ; SANDER C.** *Nucleic Acids Res.,* 1995, vol. 23, 565-570
- **CHAN Y.-L. ; OLVERA J. ; WOOL I.G.** *Biochem. Biophys. Res. Commun.,* 1994, vol. 202, 1176-1180
- **ELHAG G.A. ; BOURQUE D.P.** *Biochemistry,* 1992, vol. 31, 6856-6864
- **GRAACK H.-R. ; GROHMANN L. ; KITAKAWA M. ; SCHAEFER K.L. ; KRUFT V.** *Eur. J. Biochem.,* 1992, vol. 206, 373-380
- **HERWIG S. ; KRUFT V. ; WITTMANN-LIEBOLD B.** *Eur. J. Biochem.,* 1992, vol. 207, 877-885
- **MIZUTA K. ; HASHIMOTO T. ; OTAKA E.** *Nucleic Acids Res.,* 1992, vol. 20, 1011-1016
- **TANAKA T. ; KUWANO Y. ; KUZUMAKI T. ; ISHIKAWA K. ; OGATA K.** *Eur. J. Biochem.,* 1987, vol. 162, 45-48
- **BERGMANN U. ; ARNDT E.** *Biochim. Biophys. Acta,* 1990, vol. 1050, 56-60
- **KRUFT V. ; KAPP U. ; WITTMANN-LIEBOLD B.** *Biochimie,* 1991, vol. 73, 855-860
- **SHARP P.M.** *Gene,* 1994, vol. 139, 129-130
- **OLD I.G. ; MARGARITA D. ; SAINT GIRONS I.** *Nucleic Acids Res.,* 1992, vol. 20, 6097-6097
- **LAN Q. ; NIU L.L. ; FALLON A.M.** *Biochim. Biophys. Acta,* 1994, vol. 1218, 460-462
- **GAO J. ; KIM S.R. ; CHUNG Y.Y. ; LEE J.M. ; AN G.** *Plant Mol. Biol.,* 1994, vol. 25, 761-770
- **CHAN Y.-L. ; PAZ V. ; OLVERA J. ; WOOL I.G.** *Biochem. Biophys. Res. Commun.,* 1993, vol. 192, 849-853
- **LIN A. ; MCNALLY J. ; WOOL I.G.** *J. Biol. Chem.,* 1984, vol. 259, 487-490
- **LEER R.J. ; VAN RAAMSDONK-DUIN M.M.C. ; KRAAKMAN P. ; MAGER W.H.** *Planta R.J. Nucleic Acids Res.,* 1985, vol. 13, 701-709
- **RAMIREZ C. ; LOUIE K.A. ; MATHESON A.T.** *FEBS Lett.,* 1989, vol. 250, 416-418
- **PIATYSZEK MA ; DENSLOW ND ; O'BRIEN TW.** *Nucleic Acids Res,* 1988, vol. 16, 2565-2583
- **CHAN YL ; SUZUKI K ; WOOL IG.** *Biochem Biophys Res Commun,* 1995, vol. 215, 682-690
- **GALLAGHER M.J. ; CHAN Y.-L. ; LIN A. ; WOOL I.G.** *DNA,* 1988, vol. 7, 269-273
- **BERGMANN U. ; WITTMANN-LIEBOLD B.** *Biochim. Biophys. Acta,* 1993, vol. 1173, 195-200
- **HATAKEYAMA T. ; HATAKEYAMA T.** *Biochim. Biophys. Acta,* 1990, vol. 1039, 343-347
- **ROSENDAHL G. ; ANDREASEN P.H. ; KRISTIANSEN K.** *Gene,* 1991, vol. 98, 161-167
- **YANG D. ; GUNTHER I. ; MATHESON A.T. ; AUER J. ; SPICKER G. ; BOECK A.** *Biochimie,* 1991, vol. 73, 679-682
- **SUZUKI K. ; OLVERA J. ; WOOL I.G.** *Gene,* 1990, vol. 93, 297-300
- **SCHWANK S. ; HARRER R. ; SCHUELLER H.-J. ; SCHWEIZER E.** *Curr. Genet.,* 1993, vol. 24, 136-140
- **GOLDEN B.L. ; RAMAKRISHNAN V. ; WHITE S.W.** *EMBO J.,* 1993, vol. 12, 4901-4908
- **COLOMBO P. ; YON J. ; GARSON K. ; FRIED M.** *Proc. Natl. Acad. Sci. U.S.A.,* 1992, vol. 89, 6358-6362
- **KOLODRUBETZ D. ; BURGUM A.** *Yeast,* 1991, vol. 7, 79-90
- **HOFFMAN D.W. ; DAVIES C. ; GERCHMAN S.E. ; KYCIA J.H. ; PORTER S.J. ; WHITE S.W. ; RAMAKRISHNAN V.** *EMBO J.,* 1994, vol. 13, 205-212
- **KIMURA M. ; KIMURA J. ; HATAKEYAMA T.** *FEBS Lett.,* 1988, vol. 240, 15-20
- **LIN A. ; CHAN Y.-L. ; JONES R. ; WOOL I.G.** *J. Biol. Chem.,* 1987, vol. 262, 14343-14351
- **MARCHAL C. ; ISMAILI N. ; PAYS E.** *Mol. Biochem. Parasitol.,* 1993, vol. 57, 331-334
- **CHAN Y.-L. ; PAZ V. ; WOOL I.G.** *Biochem. Biophys. Res. Commun.,* 1991, vol. 178, 1212-1218
- **CHAN Y.-L. ; SUZUKI K. ; OLVERA J. ; WOOL I.G.** *Nucleic Acids Res.,* 1993, vol. 21, 649-655
- **DANG H. ; ELLIS S.R.** *Nucleic Acids Res.,* 1990, vol. 18, 6895-6901
- **GANTT J.S. ; THOMPSON M.D.** *J. Biol. Chem.,* 1990, vol. 265, 2763-2767
- **HERFURTH E. ; HIRANO H. ; WITTMANN-LIEBOLD B.** *Biol. Chem. Hoppe-Seyler,* 1991, vol. 372, 955-961
- **CHEN I.-T. ; ROUFA D.J.** *Gene,* 1988, vol. 70, 107-116
- **MAKI C. ; RHOADS D.D. ; STEWART M.J. ; VAN SLYKE B. ; DENELL R.E. ; ROUFA D.J.** *Gene,* 1989, vol. 79, 289-298
- **ABOVICH N. ; ROSBASH M.** *Mol. Cell. Biol.,* 1984, vol. 4, 1871-1879
- **MCDOUGALL J. ; CHOLI T. ; KRUFT V. ; KAPP U. ; WITTMANN-LIEBOLD B.** *FEBS Lett.,* 1989, vol. 245, 253-260

- **KITAGAWA M. ; TAKASAWA S. ; KIKUCHI N. ; ITOH T. ; TERAOKA H. ; YAMAMOTO H. ; OKAMOTO H.** *FEBS Lett.,* 1991, vol. 283, 210-214
- **ETTER A. ; ABOUTANOS M. ; TOBLER H. ; MUELLER F.** *Proc. Natl. Acad. Sci. U.S.A.,* 1991, vol. 88, 1593-1596
- **SUZUKI K. ; OLVERA J. ; WOOL I.G.** *Biochimie,* 1990, vol. 72, 299-302
- **DAVIS S.C. ; TZAGOLOFF A. ; ELLIS S.R.** *J. Biol. Chem.,* 1992, vol. 267, 5508-5514
- **TOHGO A. ; TAKASAWA S. ; MUNAKATA H. ; YONEKURA H. ; HAYASHI N. ; OKAMOTO H.** *FEBS Lett.,* 1994, vol. 340, 133-138
- **BHAT K.S. ; MORRISON S.G.** *Nucleic Acids Res.,* 1993, vol. 21, 2939-2939
- **NISHI R. ; HASHIMOTO H. ; UCHIMIYA H. ; KATO A.** *Biochim. Biophys. Acta,* 1993, vol. 1216, 113-114
- **SUZUKI K. ; OTAKA E.** *Nucleic Acids Res.,* 1988, vol. 16, 6223-6223
- **ITOH T. ; OKATA E. ; MATSUI K.A.** *Biochemistry,* 1985, vol. 24, 7418-7423
- **BROWN S.J. ; JEWELL A. ; MAKI C.G. ; ROUFA D.J.** *Gene,* 1990, vol. 91, 293-296
- **SOSA L. ; FONZI W.A. ; SYPHERD P.S.** *Nucleic Acids Res.,* 1989, vol. 17, 9319-9331
- **KIMURA J. ; ARNDT E. ; KIMURA M.** *FEBS Lett.,* 1987, vol. 224, 65-70
- **KUWANO Y. ; NAKANISHI O. ; NABESHIMA Y. ; TANAKA T. ; OGATA K.** *J. Biochem.,* 1985, vol. 97, 983-992
- **ZINOV'EVA R.D. ; TOMAREV S.I.** *Dokl. Akad. Nauk SSSR,* 1989, vol. 304, 464-469
- **ITOH N. ; OHTA K. ; OHTA M. ; KAWASAKI T. ; YAMASHINA I.** *Nucleic Acids Res.,* 1989, vol. 17, 2121-2121
- **WU M. ; TAN H.** *Gene,* 1994, vol. 150, 401-402
- **CHAN Y.-L. ; OLVERA J. ; WOOL I.G.** *Biochem. Biophys. Res. Commun.,* 1991, vol. 179, 314-318
- **HWANG I. ; GOODMAN H.M.** *Plant Physiol.,* 1993, vol. 102, 1357-1358
- **HOEKSTRA R. ; FERREIRA P.M. ; BOOTSMAN T.C. ; MAGER W.H. ; PLANTA R.J.** *Yeast,* 1992, vol. 8, 949-959
- **LIU J.H. ; REID D.M.** *Plant Physiol.,* 1995, vol. 109, 338-338
- **MIZUTA K. ; HASHIMOTO T. ; SUZUKI K.I. ; OTAKA E.** *Nucleic Acids Res.,* 1991, vol. 19, 2603-2608
- **BOGUTA M. ; DMOCHOWSKA A. ; BORSUK P. ; WROBEL K. ; GARGOURI A. ; LAZOWSKA J. ; SLONIMSKI P. ; SZCZESNIAK B. ; KRUSZEWSKA A.** *Mol. Cell. Biol.,* 1992, vol. 12, 402-412
- **FISHER E.M. ; BEER-ROMERO P. ; BROWN L.G. ; RIDLEY A. ; MCNEIL J.A. ; LAWRENCE J.B. ; WILLARD H.F. ; BIEBER F.R. ; PAGE D.C.** *Cell,* 1990, vol. 63, 1205-1218
- **BRAUN H.P. ; EMMERMANN M. ; MENTZEL H. ; SCHMITZ U.K.** *Biochim. Biophys. Acta,* 1994, vol. 1218, 435-438
- **ALL-ROBYN J.A. ; BROWN N. ; OTAKA E. ; LIEBMAN S.W.** *Mol. Cell. Biol.,* 1990, vol. 10, 6544-6553
- **FRANCO R. ; ROSENFELD M.G.** *J. Biol. Chem.,* 1990, vol. 265, 4321-4325
- **WATSON K.L. ; KONRAD K.D. ; WOODS D.F. ; BRYANT P.J.** *Proc. Natl. Acad. Sci. U.S.A.,* 1992, vol. 89, 11302-11306
- **HANSEN G. ; ESTRUCH J.J. ; SPENA A.** *Nucleic Acids Res.,* 1992, vol. 20, 5230-5230
- **KIMURA M. ; ARNDT E. ; HATAKEYAMA T. ; HATAKEYAMA T. ; KIMURA J.** *Can. J. Microbiol.,* 1989, vol. 35, 195-199
- **BANDI H.R. ; FERRARI S ; KRIEG J. ; MEYER H.E. ; THOMAS G.** *J. Biol. Chem.,* 1993, vol. 268, 4530-4533
- **KLUSSMANN S. ; FRANKE P. ; BERGMANN U. ; KOSTKA S. ; WITTMANN-LIEBOLD B.** *Biol. Chem. Hoppe-Seyler,* 1993, vol. 374, 305-312
- **IGNATOVICH O. ; COOPER M. ; KULESZA H.M. ; BEGGS J.D.** *Nucleic Acids Res.,* 1995, vol. 23, 4616-4619
- **SALAZAR C.E. ; MILLS-HAMM D.M. ; KUMAR V. ; COLLINS F.H.** *Nucleic Acids Res.,* 1993, vol. 21, 4147-4147
- **ENGEMANN S. ; HERFURTH E. ; BRIESEMEISTER U. ; WITTMANN-LIEBOLD B.** *J. Protein Chem.,* 1995, vol. 14, 189-195
- **CHAN Y.-L. ; PAZ V. ; OLVERA J. ; WOOL I.G.** *FEBS Lett.,* 1990, vol. 263, 85-88
- **KUSIAN B. ; YOO J.G. ; BEDNARSKI R. ; BOWIEN B.** *J. Bacteriol.,* 1992, vol. 174, 7337-7344
- **RUDD K.E. ; SOFIA H.J. ; KOONIN E.V. ; PLUNKETT G. III ; LAZAR S. ; ROUVIERE P.E.** *Trends Biochem. Sci.,* 1995, vol. 20, 14-15
- **RAHFELD J.-U. ; RUECKNAGEL K.P. ; SCHELBERT B. ; LUDWIG B. ; HACKER J. ; MANN K. ; FISCHER G.** *FEBS Lett.,* 1994, vol. 352, 180-184
- **VAN GEMEN B. ; VAN KNIPPENBERG P.H.** Nucleic acid methylation. Alan R. Liss Inc, 1990, 19-36
- **LAFONTAINE D. ; DELCOUR J. ; GLASSER A.L. ; DESGRES J. ; VANDENHAUTE J.** *J. Mol. Biol.,* 1994, vol. 241, 492-497
- **WALKER J.E. ; SARASTE M. ; RUNSWICKM.J. ; GAYN.J.** *EMBO J.,* 1982, vol. 1, 945-951
- **SCHEFFZEK K. ; KLEBE C. ; FRITZ-WOLF K. ; KABSCH W. ; WITTINGHOFER A.** *Nature,* 1995, vol. 374, 378-381

- **RUSH M.G. ; DRIVAS G. ; D'EUSTACHIO P.** *BioEssays,* 1996, vol. 18, 103-112
- **SMITH K.C. ; WANG T.-C. V.** *BioEssays,* 1989, vol. 10, 12-16
- **LLOYD A.T. ; SHARP P.M.** *J. Mol. Evol.,* 1993, vol. 37, 399-407
- **ROCA A.I. ; COX M.M.** *Prog. Nucleic Acids Res. Mol. Biol.,* 1997, vol. 56, 129-223
- **KARLIN S. ; WEINSTOCK G.M. ; BRENDEL V.** *J. Bacteriol,* 1995, vol. 177, 6881-6893
- **EISEN J.A.** *J. Mol. Evol.,* 1995, vol. 41, 1105-1123
- **CERUTTI H.D. ; OSMAN M. ; GRANDONI P. ; JAGENDORF A.T.** *Proc. Natl. Acad. Sci. U.S.A.,* 1992, vol. 89, 8068-8072
- **PAO GM ; SAIER MH.** *J Mol Evol,* 1995, vol. 40, 136-154
- **NILLSON O. ; LUNDQVIST T. ; HAHNE S. ; SJOBERG B.-M.** *Biochem. Soc. Trans.,* 1988, vol. 16, 91-94
- **REICHARD P.** *Science,* 1993, vol. 260, 1773-1777
- **WATANABE H. ; NAITOH A. ; SUYAMA Y. ; INOKUCHI N. ; SHIMADA H. ; KOYAMA T. ; OHGI K. ; IRIE M.** *J. Biochem.,* 1990, vol. 108, 303-310
- **HARING V. ; GRAY J.E. ; MCCLURE B.A. ; ANDERSON M.A. ; CLARKE A.E.** *Science,* 1990, vol. 250, 937-941
- **MCCLURE B.A. ; HARING V. ; EBERT P.R. ; ANDERSON M.A. ; SIMPSON R.J. ; SAKIYAMA F. ; CLARKE A.E.** *Nature,* 1989, vol. 342, 95957
- **LOEFFLER A. ; GLUND K. ; IRIE M.** *Eur. J. Biochem.,* 1993, vol. 214, 627-633
- **MEADOR J. III ; KENNELL D.** *Gene,* 1990, vol. 95, 1-7
- **KAWATA Y. ; SAKIYAMA F. ; HAYASHI F. ; KYOGOKU Y.** *Eur. J. Biochem.,* 1990, vol. 187, 255-262
- **KURIHARA H. ; MITSUI Y. ; OHGI K. ; IRIE M. ; MIZUNO H. ; NAKAMURA K.T.** *FEBS Lett.,* 1992, vol. 306, 189-192
- **BANDZIULIS R.J. ; SWANSON M.S. ; DREYFUSS G.** *Genes Dev.,* 1989, vol. 3, 431-437
- **DREYFUSS G. ; SWANSON M.S. ; PINOL-ROMA S.** *Trends Biochem. Sci.,* 1988, vol. 13, 86-91
- **MILBURN S.C. ; HERSHEY J.W.B. ; DAVIES M.V. ; KELLEHER K. ; KAUFMAN R.J.** *EMBO J.,* 1990, vol. 9, 2783-2790
- **SZABO A. ; DALMAU J. ; MANLEY G. ; ROSENFELD M. ; WONG E. ; HENSON J. ; POSNER J.B. ; FURNEAUX H.M.** *Cell,* 1991, vol. 67, 325-333
- **REBAGLIATI M.** *Cell,* 1989, vol. 58, 231-232
- **LI Y. ; SUGIURA M.** *EMBO J.,* 1990, vol. 9, 3059-3066
- **AYANE M. ; PREUSS U. ; KOEHLER G. ; NIELSEN P.J.** *Nucleic Acids Res.,* 1991, vol. 19, 1273-1278
- **KAWAKAMI A. ; TIAN Q. ; DUAN X. ; STREULI M. ; SCHLOSSMAN S.F. ; ANDERSON P.** *Proc. Natl. Acad. Sci. U.S.A.,* 1992, vol. 89, 8681-8685
- **MINVIELLE-SEBASTIA L. ; WINSOR B. ; BONNEAUD N. ; LACROUTE F.** *Mol. Cell. Biol.,* 1991, vol. 11, 3075-3087
- **BERG J.M. ; HOLM R.H.** Iron-sulfur proteins. Wiley, 1982, 1-66
- **KOK M. ; OLDENHUIS R. ; DER LINDEN M.P.G. ; MEULENBERG C.H.C. ; KINGMA J. ; WITHOLT B.** *J. Biol. Chem.,* 1989, vol. 264, 5442-5451
- **VAN BEEUMEN J.J. ; VAN DRIESSCHE G. ; LIU M.-Y. ; LE GALL J.** *J. Biol. Chem.,* 1991, vol. 266, 20645-20653
- **RAWLINGS N.D. ; BARRETTA.J.** *Meth. Enzymol.,* 1995, vol. 248, 105-120
- **XIONG Y ; EICKBUSH TH.** *EMBO J,* 1990, vol. 9, 3353-3362
- **HORIKAWA S. ; SASUGA J. ; SHIMIZU K. ; OZASA H. ; TSUKADA K.** *J. Biol. Chem.,* 1990, vol. 265, 13683-13686
- **BYCROFT M ; HUBBARD TJ ; PROCTOR M ; FREUND SM ; MURZIN AG.** *Cell,* 1997, vol. 88, 235-242
- **ZALKIN H. ; DIXON J.E.** *Prog. Nucleic Acid Res. Mol. Biol.,* 1992, vol. 42, 259-287
- **MIZUKI N. ; KASAHARA M.** *Mol. Cell. Endocrinol.,* 1992, vol. 89, 25-32
- **KASAHARA M. ; GUTKNECHT J. ; BREW K. ; SPURR N. ; GOODFELLOW P.N.** *Genomics,* 1989, vol. 5, 527-534
- **LU G. ; VILLALBA M. ; COSCIA M.R. ; HOFFMAN D.R. ; KING T.P.** *J. Immunol.,* 1993, vol. 150, 2823-2830
- **DIXON D.C. ; CUTT J.R. ; KLESSIG D.F.** *EMBO J.,* 1991, vol. 10, 1317-1324
- **SCHUREN F.H.J. ; ASGEIRSDOTTIR S.A. ; KOTHE E.M. ; SCHEER J.M.J. ; WESSELS J.G.H.** *J. Gen. Microbiol.,* 1993, vol. 139, 2083-2090
- **TRIPOULAS N ; LAJEUNESSE D ; GILDEA J ; SHEARN A.** *Genetics,* 1996, vol. 143, 913-928
- **CUI X ; DE VIVO I ; SLANY R ; MIYAMOTO A ; FIRESTEIN R ; CLEARY, ML.** *Nat Genet,* 1998, vol. 18, 331-337
- **MAYER B.J. ; HAMAGUCHI M. ; HANAFUSA H.** *Nature,* 1988, vol. 332, 272-275
- **MUSACCHIO A. ; GIBSON T. ; LEHTO V.P. ; SARASTE M.** *FEBS Lett.,* 1992, vol. 307, 55-61
- **PAWSON T. ; SCHLESSINGER J.** *Curr. Biol.,* 1993, vol. 3, 434-442
- **MAYER B.J. ; BALTIMORE D.** *Trends Cell Biol.,* 1993, vol. 3, 8-13
- **KURIYAN J. ; COWBURN D.** *Curr. Opin. Struct. Biol.,* 1993, vol. 3, 828-837
- **MORTON C.J. ; CAMPBELL I.D.** *Curr. Biol.,* 1994, vol. 4, 615-617

- **USHA R. ; SAVITHRI H.S. ; RAO N.A.** *Biochim. Biophys. Acta,* 1994, vol. 1204, 75-83
- **TEPLYAKOV A ; OBMOLOVA G ; BADET-DENISOT MA ; BADET B ; POLIKARPOV I.** *Structure,* 1998, vol. 6, 1047-1055
- **BRENNWALD P ; KEARNS B ; CHAMPION K ; KERANEN S ; BANKAITIS V ; NOVICK P.** *Cell,* 1994, vol. 79, 245-258
- **RISINGER C ; BLOMQVIST AG ; LUNDELL I ; LAMBERTSSON A ; NASSEL D ; PIERIBONE VA ; BRODIN L ; LARHAMMAR D.** *J Biol Chem,* 1993, vol. 268, 24408-24414
- **PONTING CP.** *Protein Sci,* 1997, vol. 6, 459-463
- **CALLEBAUT I ; MORNON JP.** *Biochem J,* 1997, vol. 321, 125-132
- **PONTING C ; SCHULTZ J ; BORK P.** *Trends Biochem Sci,* 1997, vol. 22, 193-194
- **SUMMERS C. ; KARST F. ; CHARLES A.D.** *Gene,* 1993, vol. 136, 185-192
- **ROBINSON G.W. ; TSAY Y.H. ; KIENZLE B.K. ; SMITH-MONROY C.A. ; BISHOP R.W.** *Mol. Cell. Biol.,* 1993, vol. 13, 2706-2727
- **ROEMER S. ; HUGUENEY P. ; BOUVIER F. ; CAMARA B. ; KUNTZ M.** *Biochem. Biophys. Res. Commun.,* 1993, vol. 196, 1414-1421
- **ALTHOFF S. ; SELINGER D. ; WISE J.A.** *Nucleic Acids Res.,* 1994, vol. 22, 1933-1947
- **COHEN P.** *Annu. Rev. Biochem.,* 1989, vol. 58, 453-508
- **COHEN P. ; COHEN P.T.W.** *J. Biol. Chem.,* 1989, vol. 264, 21435-21438
- **COHEN P.T.W. ; BREWIS N.D. ; HUGHES V. ; MANN D.J.** *FEBS Lett.,* 1990, vol. 268, 355-359
- **YOON H. ; DONAHUE T.F.** *Mol. Cell. Biol.,* 1992, vol. 12, 248-260
- **FIELDS C.A. ; ADAMS M.D.** *Biochem. Biophys. Res. Commun.,* 1994, vol. 198, 288-291
- **OGAWA H. ; GOMI T. ; KONISHI K. ; DATE T. ; NAAKASHIMA H. ; NOSE K. ; MATSUDA Y. ; PERAINO C. ; PITOT H.C. ; FUJIOKA M.** *J. Biol. Chem.,* 1989, vol. 264, 15818-15823
- **DATTA P. ; GOSS T.J. ; OMNAAS J.R. ; PATIL R.V.** *Proc. Natl. Acad. Sci. U.S.A.,* 1987, vol. 84, 393-397
- **PARSOT C.** *EMBO J.,* 1986, vol. 5, 3013-3019
- **GRABOWSKI R. ; HOFMEISTER A.E.M. ; BUCKEL W.** *Trends Biochem. Sci.,* 1993, vol. 18, 297-300
- **SAITO K. ; KUROSAWA M. ; MURAKOSHI I.** *FEBS Lett.,* 1993, vol. 328, 111-114
- **SWAROOP M. ; BRADLEY K. ; OHURA T. ; TAHARA T. ; ROPER M.D. ; ROSENBERG L.E. ; KRAUS J.P.** *J. Biol. Chem.,* 1992, vol. 267, 11455-11461
- **HINATA K ; WATANABE M ; YAMAKAWA S ; SATTA Y ; ISOGAI A.** Evolutionary aspects of the S-related genes of the Brassica self-incompatibility system: synonymous and nonsynonymous base substitutions. *Genetics,* 1995, vol. 140, 1099-1104
- **SAKAMOTO K ; KUSABA M ; NISHIO T.** Polymorphism of the S-locus glycoprotein gene (SLG) and the S-locus related gene (SLR1) in Raphanus sativus L. and self-incompatible ornamental plants in the Brassicaceae. *Mol Gen Genet,* 1998, vol. 258, 397-403
- **YU L. ; WEI Y.-Y. ; USUI S. ; YU C.-A.** *J. Biol. Chem.,* 1992, vol. 267, 24508-24515
- **ABRAHAM P.R. ; MULDER A. ; VAN'T RIET J. ; RAUE H.A.** *Mol. Gen. Genet.,* 1994, vol. 242, 708-716
- **LEBLANC C. ; BOYEN C. ; RICHARD O. ; BONNARD G. ; GRIENENBERGER J.M. ; KLOAREG B.** *J. Mol. Biol.,* 1995, vol. 250, 484-495
- **HALACHMI N ; LEV Z.** The Sec1 family: a novel family of proteins involved in synaptic transmission and general secretion. *J Neurochem,* 1996, vol. 66, 889-897
- **HARTMANN E. ; SOMMER T. ; PREHN S. ; GOERLICH D. ; JENTSCH S. ; RAPOPORT T.A.** *Nature,* 1994, vol. 367, 654-657
- **HAYASHI M. ; MORI H. ; NISHIMURA M. ; AKAZAWA T. ; HARA-NISHIMURA I.** *Eur. J. Biochem.,* 1988, vol. 172, 627-632
- **SHOTWELL M.A. ; AFONSO C. ; DAVIES E. ; CHESNUT R.S. ; LARKINS B.A.** *Plant Physiol.,* 1988, vol. 87, 698-704
- **KO TP ; NG JD ; MCPHERSON A.** The three-dimensional structure of canavalin from jack bean (Canavalia ensiformis). *Plant Physiol,* 1993, vol. 101, 729-744
- **BARIL C. ; RICHAUD C. ; FOURNI E. ; BARANTON G. ; SAINT GIRONS I.** *J. Gen. Microbiol.,* 1992, vol. 138, 47-53
- **KARSTEN W.E. ; VIOLA R.E.** *Biochim. Biophys. Acta,* 1992, vol. 1121, 234-238
- **LUDOVICE M. ; MARTIN J.F. ; CARRACHAS P. ; LIRAS P.** *J. Bacteriol.,* 1992, vol. 174, 4606-4613
- **GESSERT S.F. ; KIM J.H. ; NARGANG F.E. ; WEISS R.L.** *J. Biol. Chem.,* 1994, vol. 269, 8189-8203
- **KOONIN EV ; RUDD KE.** SpoU protein of Escherichia coli belongs to a new family of putative rRNA methylases. *Nucleic Acids Res,* 1993, vol. 21, 5519-5519
- **PERSSON BC ; JAGER G ; GUSTAFSSON C.** The spoU gene of escherichia coli , the fourth gene of the spoT operon, is essential for tRNA (Gm18) 2' methyltransferase activity. *Nucleic Acids Res,* 1997, vol. 25, 4093-4097

- **SOBEL A.** *Trends Biochem. Sci.,* 1991, vol. 16, 301-305
- **MAUCUER A. ; MOREAU J. ; MECHALI M. ; SOBEL A.** *J. Biol. Chem.,* 1993, vol. 268, 16420-16429
- **KNAUS P. ; MARQUEZE-POUEY B. ; SCHERER H. ; BETZ H.** *Neuron,* 1990, vol. 5, 453-462
- **BERNFIELD M. ; KOKENYESI R. ; KATO M. ; HINKES M.T. ; SPRING J. ; GALLO R.L. ; LOSE E.J.** *Annu. Rev. Cell Biol.,* 1992, vol. 8, 365-393
- **DAVID G.** *FASEB J.,* 1993, vol. 7, 1023-1030
- **BENNETT M.K. ; GARCIA-ARRARAS J.E. ; ELFERINK L.A. ; PETERSON K. ; FLEMING A.M. ; HAZUKA C.D. ; SCHELLER R.H.** *Cell,* 1993, vol. 74, 863-873
- **SPRING J. ; KATO M. ; BERNFIELD M.** *Trends Biochem. Sci.,* 1993, vol. 18, 124-125
- **PELHAM H.R.B.** *Cell,* 1993, vol. 73, 425-426
- **HERMANN H ; FABRIZIO P ; RAKER VA ; FOULAKI K ; HORNIG H ; BRAHMS H ; LUHRMANN R.** *EMBO J,* 1995, vol. 14, 2076-2088
- **KAMBACH C ; WALKE S ; YOUNG R ; AVIS JM ; DE LA FORTELLE E ; RAKER VA ; LUHRMANN R ; LI J ; NAGAI K.** *Cell,* 1999, vol. 96, 375-387
- **STEBBINS CE ; KAELIN WG JR ; PAVLETICH NP.** *Science,* 1999, vol. 284, 455-461
- **ITO K.** *Mol. Microbiol.,* 1992, vol. 6, 2423-2428
- **AUER J. ; SPICKER G. ; BOECK A.** *Biochimie,* 1991, vol. 73, 683-688
- **DOUGLAS S.E.** *FEBS Lett.,* 1992, vol. 298, 93-96
- **GAUBIER P. ; RAYNAL M. ; HULL G. ; HUESTIS G.M. ; GRELLET F. ; ARENAS C. ; PAGES M. ; DELSENY M.** *Mol. Gen. Genet.,* 1993, vol. 238, 409-418
- **LIAO D.I. ; REMINGTON S.J.** *J. Biol. Chem.,* 1990, vol. 265, 6528-6531
- **SORENSEN S.B. ; SVENDSEN I. ; BREDDAM K.** *Carlsberg Res. Commun.,* 1989, vol. 54, 193-202
- **IMAI Y. ; YAMAMOTO M.** *Mol. Cell. Biol.,* 1992, vol. 12, 1827-1834
- **SVENDSEN I. ; HOFMANN T. ; ENDRIZZI J. ; REMINGTON J. ; BREDDAM K.** *FEBS Lett.,* 1993, vol. 333, 39-43
- **GALJART N.J. ; MORREAU H. ; WILLEMSEN R. ; GILLEMANS N. ; BONTEN E.J. ; D'AZZO A.** *J. Biol. Chem.,* 1991, vol. 266, 14754-14762
- **CHO W.L. ; DEITSCH K.W. ; RAIKHEL A.S.** *Proc. Natl. Acad. Sci. U.S.A.,* 1991, vol. 88, 10821-10824
- **HU W.N. ; KOPACHIK W. ; BAND R.N.** *Infect. Immun.,* 1992, vol. 60, 2418-2424
- **WAJANT H. ; MUNDRY K.W. ; PFITZENMAIER K.** *Plant Mol. Biol.,* 1994, vol. 26, 735-746
- **CARRELL R. ; TRAVIS J.** *Trends Biochem. Sci,* 1985, vol. 10, 20-24
- **CARRELL R. ; PEMBERTON P.A. ; BOSWELL D.R.** *Cold Spring Harbor Symp. Quant. Biol.,* 1987, vol. 52, 527-535
- **HUBER R. ; CARRELL R.W.** *Biochemistry,* 1989, vol. 28, 8951-8966
- **REMOLD-O'DONEEL E.** *FEBS Lett.,* 1993, vol. 315, 105-108
- **OSTERWALDER T. ; CONTARTESE J. ; STOECKLI E.T. ; KUHN T.B. ; SONDEREGGER P.** *EMBO J.,* 1996, vol. 15, 2944-2953
- **KOMIYAMA T. ; RAY C.A. ; PICKUP D.J. ; HOWARD A.D. ; THORNBERRY N.A. ; PETERSON E.P. ; SALVESEN G.** *J. Biol. Chem.,* 1994, vol. 269, 19331-19337
- **CLARKE E. ; SANDWAL B.D.** *Biochim. Biophys. Acta,* 1992, vol. 1129, 246-248
- **ZOU Z. ; ANISOWICZ A. ; NEVEU M. ; RAFIDI K. ; SHENG S. ; SAGER R. ; HENDRIX M.J. ; SEFTOR E. ; THOR A.** *Science,* 1994, vol. 263, 526-529
- **STEELE F.R. ; CHADER G.J. ; JOHNSON L.V. ; TOMBRAN-TINK J.** *Proc. Natl. Acad. Sci. U.S.A.,* 1993, vol. 90, 1526-1530
- **HOLLAND L.J. ; SUKSANG C. ; WALL A.A. ; ROBERTS L.R. ; MOSER D.R. ; BHATTACHARYA A.** *J. Biol. Chem.,* 1992, vol. 267, 7053-7059
- **MORRETT E. ; SEGOVIA L.** *J. Bacteriol.,* 1993, vol. 175, 6067-6074
- **AUSTIN S. ; KUNDROT C. ; DIXON R.** *Nucleic Acids Res.,* 1991, vol. 19, 2281-2287
- **ALBRIGHT L.M. ; HUALA E. ; AUSUBEL F.M.** *Annu. Rev. Genet.,* 1989, vol. 23, 311-336
- **AUSTIN S. ; DIXON R.** *EMBO J.,* 1992, vol. 11, 2219-2228
- **HELMANN J.D. ; CHAMBERLIN M.J.** *Annu. Rev. Biochem.,* 1988, vol. 57, 839-872
- **GRIBSKOV M. ; BURGESS R.R.** *Nucleic Acids Res.,* 1986, vol. 14, 6745-6763
- **LONETTO M.A. ; GRIBSKOV M. ; GROSS C.A.** *J. Bacteriol.,* 1992, vol. 174, 3843-3849
- **LONETTO M.A. ; BROWN K.L. ; RUDD K.E. ; BUTTNER M.J.** *Proc. Natl. Acad. Sci. U.S.A.,* 1994, vol. 91, 7573-7577
- **BANNISTER J.V. ; BANNISTER W.H. ; ROTILIO G.** *CRC Crit. Rev. Biochem.,* 1987, vol. 22, 111-154
- **SMITH M.W. ; DOOLITTLE R.F.** *J. Mol. Evol.,* 1992, vol. 34, 175-184
- **PARKER M.W. ; BLAKE C.C.F.** *FEBS Lett.,* 1988, vol. 229, 377-382
- **HARTWIG JH.** Actin-binding proteins. 1: Spectrin super family. *Protein Profile,* 1995, vol. 2, 732-732

- **YAN Y ; WINOGRAD E ; VIEL A ; CRONIN T ; HARRISON SC ; BRANTON D.** Crystal structure of the repetitive segments of spectrin. *Science,* 1993, vol. 262, 2027-2030
- **TAGUCHI S. ; KOJIMA S. ; TERABE M. ; MIURA K.-I. ; MOMOSE H.** *Eur. J. Biochem.,* 1994, vol. 220, 911-918
- **SILVERMAN M.** *Annu. Rev. Biochem.,* 1991, vol. 60, 757-794
- **GOULD G.W. ; BELL G.I.** *Trends Biochem. Sci.,* 1990, vol. 15, 18-23
- **BALDWIN S.A.** *Biochim. Biophys. Acta,* 1993, vol. 1154, 17-49
- **MAIDEN M.C.J. ; DAVIS E.O. ; BALDWIN S.A. ; MOORE D.C.M. ; HENDERSON P.J.F.** *Nature,* 1987, vol. 325, 641-643
- **HENDERSON P.J.F.** *Curr. Opin. Struct. Biol.,* 1991, vol. 1, 590-601
- **CULHAM D.E. ; LASBY B. ; MARANGONI A.G. ; MILNER J.L. ; STEER B.A. ; VAN NUES R.W. ; WOOD J.M.** *J. Mol. Biol.,* 1993, vol. 229, 268-276
- **SUEDHOFT.C. ; BAUMERT M. ; PERIN M.S. ; JAHN R.** *Neuron,* 1989, vol. 2, 1475-1481
- **GERST J.E. ; RODGERS L. ; RIGGS M. ; WIGLER M.** *Proc. Natl. Acad. Sci. U.S.A.,* 1992, vol. 89, 4338-4342
- **RICHARDSON PM ; ZON LI.** Medline: 96032578. Molecular cloning of a cDNA with a novel domain present in the tre-2 oncogene and the yeast cell cycle regulators BUB2 and cdc16. *Oncogene,* 1995, vol. 11, 1139-1148
- **NEUWALD AF.** Medline: 97398935. A shared domain between a spindle assembly checkpoint protein and Ypt/Rab-specific GTPase-activators. *Trends Biochem Sci,* 1997, vol. 22, 243-244
- **HOFFMANN A. ; SINN E. ; YAMAMOTO T. ; WANG J. ; ROY A. ; HORIKOSHI M. ; ROEDER R.G.** *Nature,* 1990, vol. 346, 387-390
- **GASH A. ; HOFFMANN A. ; HORIKOSHI M. ; ROEDER R.G. ; CHUA N.-H.** *Nature,* 1990, vol. 346, 390-394
- **NIKOLOV D.B. ; HU S.-H. ; LIN J. ; GASCH A. ; HOFFMANN A. ; HORIKOSHI M. ; CHUA N.-H. ; ROEDER R.G. ; BURLEY S.K.** *Nature,* 1992, vol. 360, 40-46
- **CROWLEY T.E. ; HOEY T. ; LIU J.-K. ; JAN Y.N. ; JAN L.Y. ; TJIAN R.** *Nature,* 1993, vol. 361, 557-561
- **MARSH T.L. ; REICH C.I. ; WHITELOCK R.B. ; OLSEN G.J.** *Proc. Natl. Acad. Sci. U.S.A.,* 1994, vol. 91, 4180-4184
- **BOEHM H. ; BEENDORF R. ; GAESTEL M. ; GROSS B. ; NUERNBERG P. ; KRAFT R. ; OTTO A. ; BIELKA H.** *Biochem. Int.,* 1989, vol. 19, 277-286
- **MAKRIDES S. ; CHITPATIMA S.T. ; BANDYOPADHYAY R. ; BRAWERMAN G.** *Nucleic Acids Res.,* 1988, vol. 16, 2350-2350
- **PAY A. ; HEBERLE-BORS E. ; HIRT H.** *Plant Mol. Biol.,* 1992, vol. 19, 501-503
- **STUERZENBAUM S.R. ; KILLE P. ; MORGAN A.J.** *Biochim. Biophys. Acta,* 1998, vol. 1398, 294-304
- **RASMUSSEN S.W.** *Yeast,* 1994, vol. 10, 563-568
- **HIRASHIMA S. ; HIRAI H. ; NAKANISHI Y. ; NATORI S.** *J. Biol. Chem.,* 1988, vol. 263, 3858-3863
- **KIPLING D. ; KEARSEY S.E.** *Nature,* 1991, vol. 353, 509-509
- **LANGER D. ; ZILLIG W.** *Nucleic Acids Res.,* 1993, vol. 21, 2251-2251
- **AHN B.-Y. ; GERSHON P.D. ; JONES E.V. ; MOSS B.** *Mol. Cell. Biol.,* 1990, vol. 10, 5433-5441
- **RODRIGUEZ J.M. ; SALAS M.L. ; VINUELA E.** *Virology,* 1992, vol. 186, 40-52
- **QIAN X. ; JEON C. ; YOON H. ; AGARWAL K. ; WEISS M.A.** *Nature,* 1993, vol. 365, 277-279
- **SHANNON K.W. ; RABINOWITZ J.C.** *J. Biol. Chem.,* 1988, vol. 263, 7717-7725
- **BELANGER C. ; MACKENZIE R.E.** *J. Biol. Chem.,* 1989, vol. 264, 4837-4843
- **D'ARI L. ; RABINOWITZ J.C.** *J. Biol. Chem.,* 1991, vol. 266, 23953-23958
- **LOLIS E. ; ALBER T. ; DAVENPORT R.C. ; ROSE D. ; HARTMAN F.C. ; PETSKO G.A.** *Biochemistry,* 1990, vol. 29, 6609-6618
- **KNOWLES J.R.** *Nature,* 1991, vol. 350, 121-124
- **BOYLE D.B. ; COUPAR B.E.H. ; GIBBS A.J. ; SEIGMAN L.J. ; BOTH G.W.** *Virology,* 1987, vol. 156, 355-365
- **BLASCO R. ; LOPEZ-OTIN C. ; MUNOZ M. ; BOCKAMP E.-O. ; SIMON-MATEO C. ; VINUELA E.** *Virology,* 1990, vol. 178, 301-304
- **ROBERTSON G.R. ; WHALLEY J.M.** *Nucleic Acids Res.,* 1988, vol. 16, 11303-11317
- **BROWN DG ; VISSE R ; SANDHU G ; DAVIES A ; RIZKALLAH PJ ; MELITZ C ; SUMMERS WC ; SANDERSON MR.** *Nat Struct Biol,* 1995, vol. 2, 876-881
- **BASKARAN R. ; RAO M.R.S.** *Biochem. Biophys. Res. Commun.,* 1991, vol. 179, 1491-1499
- **GREEN J.B.A.** *FEBS Lett.,* 1989, vol. 246, 1-5
- **KOGA J. ; ADACHI T. ; HIDAKA H.** *Mol. Gen. Genet.,* 1991, vol. 226, 10-16
- **TSOU A.Y. ; RANSOM S.C. ; GERLT J.A. ; BUECHTER D.D. ; BABBITT P.C. ; KENYON G.L.** *Biochemistry,* 1990, vol. 29, 9856-9862
- **LAMB JR ; TUGENDREICH S ; HIETER P.** Medline: 95397415 Tetratrico peptide repeat interactions: to TPR or not to TPR?. *Trends Biochem Sci,* 1995, vol. 20, 257-259

- **DAS AK ; COHEN PW ; BARFORD D.** Medline: 98151343 The structure of the tetratricopeptide repeats of protein phosphatase 5: implications for TPR-mediated protein-protein interactions. *EMBO J,* 1998, vol. 17, 1192-1199
- **BLANCHE F. ; ROBIN C. ; COUDER M. ; FAUCHER D. ; CAUCHOIS L. ; CAMERON B. ; CROUZET J.** *J. Bacteriol.,* 1991, vol. 173, 4637-4645
- **ROBIN C. ; BLANCHE F. ; CAUCHOIS L. ; CAMERON B. ; COUDER M. ; CROUZET J.** *J. Bacteriol.,* 1991, vol. 173, 4893-4896
- **CROUZET J. ; CAMERON B. ; CAUCHOIS L. ; RIGAULT S. ; ROUYEZ M.-C. ; BLANCHE F. ; THIBAUT D. ; DEBUSSCHE L.** *J. Bacteriol.,* 1990, vol. 172, 5980-5990
- **ROTH J.R. ; LAWRENCE J.G. ; RUBENFIELD M. ; KIEFFER-HIGGINS S. ; CHURCH G.M.** *J. Bacteriol.,* 1993, vol. 175, 3303-3316
- **MATTHEAKIS L.C. ; SHEN W.H. ; COLLIER R.J.** *Mol. Cell. Biol.,* 1992, vol. 12, 4026-4037
- **PONTING CP.** Medline: 97200561 Tudor domains in proteins that interact with RNA. *Trends Biochem Sci,* 1997, vol. 22, 51-52
- **CALLEBAUT I ; MORNON JP.** Medline: 97157029 The human EBNA-2 coactivator p100: multidomain organization and relationship to the staphylococcal nuclease fold and to the tudor protein involved in Drosophila melanogaster development. *Biochem J,* 1997, vol. 321, 125-132
- **BOHLMANN J ; STEELE CL ; CROTEAU R.** Medline: 97413772 Monoterpene synthases from grand fir (Abies grandis). cDNA isolation, characterization, and functional expression of myrcene synthase, (-)-(4S)-limonene synthase, and (-)-(1 S,5S)-pinene synthase. *J Biol Chem,* 1997, vol. 272, 21784-21792
- **LEESONG M ; HENDERSON BS ; GILLIG JR ; SCHWAB JM ; SMITH JL.** Medline: 96398612 Structure of a dehydratase-isomerase from the bacterial pathway for biosynthesis of unsaturated fatty acids: two catalytic activities in one active site. *Structure,* 1996, vol. 4, 253-264
- **KLEYN P.W. ; FAN W. ; KOVATS S.G. ; LEE J.L. ; PULIDO J.C. ; WU Y. ; BERKEMEIER L.R. ; MISUMI D.J. ; HOLMGREN L. ; CHARLAT O.** *Cell,* 1996, vol. 85, 281-290
- **NOBEN-TRAUTH K. ; NAGGERT J.K. ; NORTH M.A. ; NISHINA P.M.** *Nature,* 1996, vol. 380, 534-538
- **NORTH M.A. ; NAGGERT J.K. ; YAN Y. ; NOBEN-TRAUTH K. ; NISHINA P.M.** *Proc. Natl. Acad. Sci. U.S.A.,* 1997, vol. 94, 3128-3133
- **LYNN R.M. ; BJORNSTI M.-A. ; CARON P.R. ; WANG J.C.** *Proc. Natl. Acad. Sci. U.S.A.,* 1989, vol. 86, 3559-3563
- **MIOSGA T. ; SCHAAFF-GERSTENSCHLAEGER I. ; FRANKEN E. ; ZIMMERMANN F.K.** *Yeast,* 1993, vol. 9, 1241-1249
- **REIZER J. ; REIZER A. ; SAIER M.H. JR.** *Microbiology,* 1995, vol. 141, 961-971
- **PARES S ; MOUZ N ; PETILLOT Y ; HAKENBECK R ; DIDEBERG O.** *Nat Struct Biol,* 1996, vol. 3, 284-289
- **KOPP M. ; MUELLER H. ; HOLZER H.** *J. Biol. Chem.,* 1993, vol. 268, 4766-4774
- **HENRISSAT B. ; BAIROCHA.** *Biochem. J.,* 1993, vol. 293, 781-788
- **KAASEN I ; MCDOUGALL J ; STROM AR.** *Gene,* 1994, vol. 145, 9-15
- **SMILIE L.B.** *Trends Biochem. Sci.,* 1979, vol. 4, 151-155
- **MCLEOD A.R.** *BioEssays,* 1986, vol. 6, 208-212
- **WHITE SP ; COHEN C ; PHILLIPS GN JR.** Medline: 87144593 Structure of co-crystals of tropomyosin and troponin. *Nature,* 1987, vol. 325, 826-828
- **POTTER JD ; SHENG Z ; PAN BS ; ZHAO J.** Medline: 95155315 A direct regulatory role for troponin T and a dual role for troponin C in the Ca2+ regulation of muscle contraction. *J Biol Chem,* 1995, vol. 270, 2557-2562
- **FARAH CS ; REINACH FC.** Medline: 95324796 The troponin complex and regulation of muscle contraction. *FASEB J,* 1995, vol. 9, 755-767
- **DI NOIA JM ; SANCHEZ DO ; FRASCH AC.** *J Biol Chem,* 1995, vol. 270, 24146-24149
- **DI NOIA JM ; D'ORSO I ; ASLUND L ; SANCHEZ DO ; FRASCH AC.** *J Biol Chem,* 1998, vol. 273, 10843-10850
- **WEBSTER T. ; TSAI H. ; KULA M. ; MACKIE G.A. ; SCHIMMEL P.** *Science,* 1984, vol. 226, 1315-1317
- **BRICK P. ; BHAT T.N. ; BLOW D.M.** *J. Mol. Biol.,* 1988, vol. 208, 83-98
- **SCHIMMEL P.** *Trends Biochem. Sci.,* 1991, vol. 16, 1-3
- **RATH VL ; SILVIAN LF ; BEIJER B ; SPROAT BS ; STEITZ TA.** *Structure,* 1998, vol. 6, 439-449
- **EDENS L. ; HESLINGA L. ; KLOK R. ; LEDEBOER A.M. ; MAAT J. ; TOONEN M.Y. ; VISSER C. ; VERRIPS C.T.** *Gene,* 1982, vol. 18, 1-12
- **ZHU B. ; CHEN T.H.H. ; LI P.H.** *Plant Physiol.,* 1995, vol. 108, 929-937
- **MALEHOM D.E. ; BORGMEYER J.R. ; SMITH C.E. ; SHAH D.M.** *Plant Physiol.,* 1994, vol. 106, 1471-1481
- **PEOPLES O.P. ; SINSKEY A.J.** *J. Biol. Chem.,* 1989, vol. 264, 15293-15297
- **YANG S.-Y. ; YANG X.-Y.H. ; HEALY-LOUIE G. ; SCHULZ H. ; ELZINGA M.** *J. Biol. Chem.,* 1990, vol. 265, 10424-10429
- **IGUAL J.C. ; GONZALEZ-BOSCH C. ; DOPAZO J. ; PEREZ-ORTIN J.E.** *J. Mol. Evol.,* 1992, vol. 35, 147-155
- **BAKER M.E. ; BILLHEIMER J.T. ; STRAUSS J.F. III.** *DNA Cell Biol.,* 1991, vol. 10, 695-698

- **HOLMGREN A.** *Annu. Rev. Biochem.,* 1985, vol. 54, 237-271
- **GLEASON F.K. ; HOLMGREN A.** *FEMS Microbiol. Rev.,* 1988, vol. 54, 271-297
- **HOLMGREN A.** *J. Biol. Chem.,* 1989, vol. 264, 13963-13966
- **EKLUND H. ; GLEASON F.K. ; HOLMGREN A.** *Proteins,* 1991, vol. 11, 13-28
- **FREEDMAN R.B. ; HAWKINS H.C. ; MURANT S.J. ; REID L.** *Biochem. Soc. Trans.,* 1988, vol. 16, 96-99
- **KIVIRIKKO K.I. ; MYLLYLA R. ; PIHLAJANIEMI T.** *FASEB J.,* 1989, vol. 3, 1609-1617
- **FREEDMAN R.B. ; HIRST T.R. ; TUITE M.F.** *Trends Biochem. Sci.,* 1994, vol. 19, 331-336
- **WEINMANN R.** *Gene Expr.,* 1992, vol. 2, 81-91
- **HAWLEY D.** *Trends Biochem. Sci.,* 1991, vol. 16, 317-318
- **HA I. ; LANE W.S. ; REINBERG D.** *Nature,* 1991, vol. 352, 689-695
- **OUZOUNIS C. ; SANDER C.** *Cell,* 1992, vol. 71, 189-190
- **KHOO B. ; BROPHY B. ; JACKSON S.P.** *Genes Dev.,* 1994, vol. 8, 2879-2890
- **NORMAN C. ; RUNSWICK M. ; POLLOCK R. ; TREISMAN R.** *Cell,* 1988, vol. 55, 989-1003
- **PASSMORE S. ; MAINE G.T. ; ELBLE R. ; CHRIST C. ; TYE B.-K.** *J. Mol. Biol.,* 1988, vol. 204, 593-606
- **YANOFSKY M. ; MA H. ; BOWMAN J. ; DREWS G. ; FELDMANN K.A. ; MEYEROWITZ E.M.** *Nature,* 1990, vol. 346, 35-39
- **GOTO K. ; MEYEROWITZ E.M.** *Genes Dev.,* 1994, vol. 8, 1548-1560
- **TROEBNER W. ; RAMIREZ L. ; MOTTE P. ; HUE I. ; HUIJSER P. ; LOENNIG W.-E. ; SAEDLER H. ; SOMMER H. ; SCHWARTZ-SOMMER Z.** *EMBO J.,* 1992, vol. 11, 4693-4704
- **MA H. ; YANOFSKY M.F. ; MEYEROWITZ E.M.** *Genes Dev.,* 1991, vol. 5, 484-495
- **ABEDINIA M. ; LAYFIELD R. ; JONES S.M. ; NIXON P.F. ; MATTICK J.S.** *Biochem. Biophys. Res. Commun.,* 1992, vol. 183, 1159-1166
- **FLETCHER T.S. ; KWEE I.L. ; NAKADA T. ; LARGMAN C. ; MARTIN B.M.** *Biochemistry,* 1992, vol. 31, 1892-1896
- **SPRENGER G.A. ; SCHORKEN U. ; WIEGERT T. ; GROLLE S. ; DE GRAAF A.A. ; TAYLOR S.V. ; BEGLEY T.P. ; BRINGER-MEYER S. ; SAHM H.** *Proc. Natl. Acad. Sci. U.S.A.,* 1997, vol. 94, 12857-12862
- **LINDQVIST Y. ; SCHNEIDER G. ; ERMLER U. ; SUNDSTROEM M.** *EMBO J.,* 1992, vol. 11, 2373-2379
- **LEVY S. ; NGUYEN V.Q. ; ANDRIA M.L. ; TAKAHASHI S.** *J. Biol. Chem.,* 1991, vol. 266, 14597-14602
- **TOMLINSON M.G. ; WILLIAMS A.F. ; WRIGHT M.D.** *Eur. J. Immunol.,* 1993, vol. 23, 136-40
- **BARCLAY A.N. ; BIRKELAND M.L. ; BROWN M.H. ; BEYERS A.D. ; DAVIS S.J. ; SOMOZA C. ; WILLIAMS A.F.** The leucocyte antigen factbooks. Academic Press, 1993
- **CRAWFORD I.P.** *Annu. Rev. Microbiol.,* 1989, vol. 43, 567-600
- **HYDE C.C. ; MILES E.W.** *Bio/Technology,* 1990, vol. 8, 27-32
- **BERLYN M.B. ; LAST R.L. ; FINK G.R.** *Proc. Natl. Acad. Sci. U.S.A.,* 1989, vol. 86, 4604-4608
- **BRENNER S.** *Nature,* 1988, vol. 334, 528-530
- **BORK P.** *FEBS lett,* 1993, vol. 327, 125-130
- **CLEVELAND D.W. ; SULLIVAN K.F.** *Annu. Rev. Biochem.,* 1985, vol. 54, 331-365
- **JOSHI H.C. ; CLEVELAND D.W.** *Cell Motil. Cytoskeleton,* 1990, vol. 16, 159-163
- **HESSE J. ; THIERAUF M. ; PONSTINGL H.** *J. Biol. Chem.,* 1987, vol. 262, 15472-15475
- **JOSHI H.C.** *BioEssays,* 1993, vol. 15, 637-643
- **CLEVELAND D.W.** *Trends Biochem. Sci.,* 1988, vol. 13, 339-343
- **DIECKMANN T ; WITHERS-WARD ES ; JAROSINSKI MA ; LIU CF ; CHEN IS ; FEIGON J.** Structure of a human DNA repair protein UBA domain that interacts with HIV-1 Vpr. *Nat Struct Biol,* 1998, vol. 5, 1042-1047
- **HOFMANN K ; BUCHER P.** The UBA domain: a sequence motif present in multiple enzyme classes of the ubiquitination pathway. *Trends Biochem Sci,* 1996, vol. 21, 172-173
- **JENTSCH S. ; SEUFERT W. ; HAUSER H.-P.** *Biochim. Biophys. Acta,* 1991, vol. 1089, 127-139
- **D'ANDREA A. ; PELLMAN D.** *Crit. Rev. Biochem. Mol. Biol.,* 1998, vol. 33, 337-352
- **JOHNSTON S.C. ; LARSEN C.N. ; COOK W.J. ; WILKINSON K.D. ; HILL C.P.** *EMBO J.,* 1997, vol. 16, 3787-3796
- **DUTTON G.J.** Glucoronidation of drugs and other compounds. CRC Press, 1980, 1-78
- **BURCHELL B. ; NEBERT D.W. ; NELSON D.R. ; BOCK K.W. ; IYANAGI T. ; JANSEN P.L. ; LANCET D. ; MULDER G.J. ; CHOWDHURY J.R. ; SIEST G.** *DNA Cell Biol.,* 1991, vol. 10, 487-494
- **SCHULTE S. ; STOFFEL W.** *Proc. Natl. Acad. Sci. U.S.A.,* 1993, vol. 90, 10265-10269
- **FURTEK D. ; SCHIEFELBEIN J.W. ; JOHNSTON F. ; NELSON O.E. JR.** *Plant Mol. Biol.,* 1988, vol. 11, 473-481
- **O'REILLY D.R. ; MILLER L.K.** *Science,* 1989, vol. 245, 1110-1112
- **HERNANDEZ C. ; OLANO C. ; MENDEZ C. ; SALAS J.A.** *Gene,* 1993, vol. 134, 139-140

- **ROYCHOUDHURY S ; MAY TB ; GILL JF ; SINGH SK ; FEINGOLD DS ; CHAKRABARTY AM.** Purification and characterization of guanosine diphospho-D-mannose dehydrogenase. A key enzyme in the biosynthesis of alginate by Pseudomonas aeruginosa. *J Biol Chem,* 1989, vol. 264, 9380-9385
- **CAMPBELL RE ; SALA RF ; VAN DE RIJN I ; TANNER ME.** Properties and kinetic analysis of UDP-glucose dehydrogenase from group A streptococci. Irreversible inhibition by UDP-chloroacetol. *J Biol Chem,* 1997, vol. 272, 3416-3422
- **SANCAR A. ; SANCAR G.B.** *Annu. Rev. Biochem.,* 1988, vol. 57, 29-67
- **OLSEN L.C. ; AASLAND R. ; WITTWER C.U. ; KROKAN H.E. ; HELLAND D.E.** *EMBO J.,* 1989, vol. 8, 3121-3125
- **UPTON C. ; STUART D.T. ; MCFADDEN G.** *Proc. Natl. Acad. Sci. U.S.A.,* 1993, vol. 90, 4518-4522
- **SLUPPHAUG G. ; MARKUSSEN F.-H. ; OLSEN L.C. ; AASLAND R. ; AARSAETHER N. ; BAKKE O. ; KROKAN H.E. ; HELLAND D.E.** *Nucleic Acids Res.,* 1993, vol. 21, 2579-2584
- **SAVVA R. ; MCAULEY-HECHT K. ; BROWN T. ; PEARL L.** *Nature,* 1995, vol. 373, 487-493
- **MOL C.D. ; ARVAI A.S. ; SLUPPHAUG G. ; KAVLI B. ; ALSETH I. ; KROHAN H.E. ; TAINER J.A.** *Cell,* 1995, vol. 80, 869-878
- **MULLER S.J. ; CARADONNA S.** *Biochim. Biophys. Acta,* 1991, vol. 1088, 197-207
- **MEYER-SIEGLER K. ; MAURO D.J. ; SEAL G. ; WURZER J. ; DERIEL J.K. ; SIROVER M.A.** *Proc. Natl. Acad. Sci. U.S.A.,* 1991, vol. 88, 8460-8464
- **MULLER S.J. ; CARADONNA S.** *J. Biol. Chem.,* 1993, vol. 268, 1310-1319
- **BARNES D.E. ; LINDAHL T. ; SEDGWICK B.** *Curr. Opin. Cell Biol.,* 1993, vol. 5, 424-433
- **WALTER L. ; MARYNEN P. ; SZPIRER J. ; LEVAN G. ; GUENTHER E.** *Genomics,* 1995, vol. 28, 301-304
- **KOONIN E.V. ; BORK P. ; SANDER C.** *EMBO J.,* 1994, vol. 13, 493-503
- **FOSTER-HARTNETT D. ; CULLEN P.J. ; GABBERT K.K. ; KRANZ R.G.** *Mol. Microbiol.,* 1993, vol. 8, 903-914
- **JENTSCH S. ; SEUFERT W. ; SOMMER T. ; REINS H.-A.** *Trends Biochem. Sci.,* 1990, vol. 15, 195-198
- **HERSHKO A.** *Trends Biochem. Sci.,* 1991, vol. 16, 265-268
- **GAREY J.R. ; LABBE-BOIS R. ; CHELSTOWSKA A. ; RYTKA J. ; HARRISON L. ; KUSHNER J. ; LABBE P.** *Eur. J. Biochem.,* 1992, vol. 205, 1011-1016
- **LEE P.T. ; HSU A.Y. ; HA H.T. ; CLARKE C.F.** *J. Bacteriol.,* 1997, vol. 179, 1748-1754
- **MOTOJIMA K. ; KANAYA S. ; GOTO S.** *J. Biol. Chem.,* 1988, vol. 263, 16677-16681
- **NYSTROM T ; NEIDHARDT FC.** Expression and role of the universal stress protein, UspA, of Escherichia coli during growth arrest. *Mol Microbiol,* 1994, vol. 11, 537-544
- **MUSHEGIAN AR ; KOONIN EV.** Sequence analysis of eukaryotic developmental proteins: ancient and novel domains. *Genetics,* 1996, vol. 144, 817-828
- **MONIA B.P. ; ECKER D.J. ; CROKE S.T.** *Bio/Technology,* 1990, vol. 8, 209-215
- **FINLEY D. ; VARSHAVSKY A.** *Trends Biochem. Sci.,* 1985, vol. 10, 343-347
- **FILIPPI M. ; TRIBIOLI C. ; TONIOLO D.** *Genomics,* 1990, vol. 7, 453-457
- **OLVERA J. ; WOOL I.G.** *J. Biol. Chem.,* 1993, vol. 268, 17967-17974
- **KUMAR S. ; YOSHIDA Y. ; NODA M.** *Biochem. Biophys. Res. Commun.,* 1993, vol. 195, 393-399
- **JONES D. ; CANDIDO E.P.** *J. Biol. Chem.,* 1993, vol. 268, 19545-19551
- **MELNICK L. ; SHERMAN F.** *J. Mol. Biol.,* 1993, vol. 233, 372-388
- **OTTO J.J.** *Cell Motil. Cytoskeleton,* 1990, vol. 16, 1-6
- **HERRENKNECHT K. ; OZAWA M. ; ECKERSKORN C. ; LOTTSPEICH F. ; LENTER M. ; KEMLER R.** *Proc. Natl. Acad. Sci. U.S.A.,* 1991, vol. 88, 9156-9160
- **ANDERSON TA ; LEVITT DG ; BANASZAK LJ.** The structural basis of lipid interactions in lipovitellin, a soluble lipoprotein. *Structure,* 1998, vol. 6, 895-909
- **SCHMIDT-ROSE T ; JENTSCH TJ.** *J Biol Chem,* 1997, vol. 272, 20515-20521
- **ZHANG J ; GEORGE AL JR ; GRIGGS RC ; FOUAD GT ; ROBERTS J ; KWIECINSKI H ; CONNOLLY AM ; PTACEK LJ.** *Neurology,* 1996, vol. 47, 993-998
- **BORK P ; ROHDE K.** *Biochem J,* 1991, vol. 279, 908-911
- **RUGGERI, Z.M. ; WARE, J.** von Willebrand factor. *FASEB J.,* 1993, vol. 7, 308-316
- **COLOMBATTI, A. ; BONALDO, P. ; DOLIANA, R.** Type A modules: interacting domains found in several non-fibrillar collagens and in other extracellular matrix proteins. *MATRIX,* 1993, vol. 13, 297-306
- **PERKINS, S.J. ; SMITH, K.F. ; WILLIAMS, S.C. ; HARIS, P.I. ; CHAPMAN, D. ; SIM, R.B.** The secondary structure of the von Willebrand factor type A domain in factor B of human complement by Fourier transform infrared spectroscopy. Its occurrence in collagen types VI, VII, XII and XIV, the integrins and other proteins by averaged structure predictions. *J.MOL.BIOL.,* 1994, vol. 238, 104-119

- **BORK, P. ; ROHDE, K.** More von Willebrand factor type A domains? Sequence similarities with malaria thrombospondin-related anonymous protein, dihydropyridine-sensitive calcium channel and inter-alpha-trypsin inhibitor. *BIOCHEM.J.,* 1991, vol. 279, 908-910
- **EDWARDS, Y.J.K. ; PERKINS, S.J.** The protein fold of the von Willebrand factor type A domain is predicted to be similar to the open twisted beta-sheet flanked by alpha-helices found in human ras-p21. *FEBS LETT.,* 1995, vol. 358, 283-286
- **LEE, J.O. ; RIEU, P. ; ARNAOUT, M.A. ; LIDDINGTON, R.** Crystal structure of the A domain from the alpha subunit of integrin CR3 (CD11b/CD18). *CELL,* 1995, vol. 80, 631-638
- **QU, A. ; LEAHY, D.J.** Crystal structure of the I-domain from the CD11a/CD18 (LFA-1, alpha L beta 2) integrin. *PROC.NATL.ACAD.SCI.USA,* 1995, vol. 92, 10277-10281
- **NEER EJ ; SCHMIDT CJ ; NAMBUDRIPAD R ; SMITH TF.** *Nature,* 1994, vol. 371, 297-300
- **GILMAN A. G.** *Annu. Rev. Biochem.,* 1987, vol. 56, 615-649
- **DURONIO R.J. ; GORDON J.I. ; BOGUSKI M.S.** *Proteins,* 1992, vol. 13, 41-56
- **VAN DER VOORN L. ; PLOEGH H.L.** *FEBS Lett.,* 1992, vol. 307, 131-134
- **NEER E.J. ; SCHMIDT C.J. ; NAMBUDRIPAD R. ; SMITH T.F.** *Nature,* 1994, vol. 371, 297-300
- **SMITH T.F. ; GAIATZES C.G. ; SAXENA K. ; NEER E.J.** *Biochemistry,* 1998
- **CERINI C. ; KERJAN P. ; ASTIER M. ; GRATECOS D. ; MIRANDE M. ; SEMERIVA M.** *EMBO J.,* 1991, vol. 10, 4267-4277
- **NADA S. ; CHANG P.K. ; DIGNAM J.D.** *J. Biol. Chem.,* 1993, vol. 268, 7660-7667
- **SONNHAMMER EL ; DURBIN R.** *Genomics,* 1997, vol. 46, 200-216
- **DAUTER Z. ; DAUTER M. ; HEMKER J. ; WITZEL H. ; WILSON K.S.** *FEBS Lett.,* 1989, vol. 247, 1-8
- **KRISTO P.A. ; SAARELAINEN R. ; FAGERSTROM R. ; AHO S. ; KORHOLA M.** *Eur. J. Biochem.,* 1996, vol. 237, 240-246
- **HENRICK K. ; COLLYER C.A. ; BLOW D.M.** *J. Mol. Biol.,* 1989, vol. 208, 129-157
- **VANGRYSPERRE W. ; AMPE C. ; KERSTERS-HILDERSON H. ; TEMPST P.** *Biochem. J.,* 1989, vol. 263, 195-199
- **TANAKA K. ; WOOD R.D.** *Trends Biochem. Sci.,* 1994, vol. 19, 83-86
- **SCHERLY D. ; NOUSPIKEL T. ; CORLET J. ; UCLA C. ; BAIROCH A. ; CLARKSON S.G.** *Nature,* 1993, vol. 363, 182-185
- **CARR A.M. ; SHELDRICK K.S. ; MURRAY J.M. ; AL-HARITHY R. ; WATTS F.Z. ; LEHMANN A.R.** *Nucleic Acids Res.,* 1993, vol. 21, 1345-1349
- **MURRAY J.M. ; TAVASSOLI M. ; AL-HARITHY R. ; SHELDRICK K.S. ; LEHMANN A.R. ; CARR A.M. ; WATTS F.Z.** *Mol. Cell. Biol.,* 1994, vol. 14, 4878-4888
- **HARRINGTON J.J. ; LIEBER M.R.** *Genes Dev.,* 1994, vol. 8, 1344-1355
- **SZANKASI P. ; SMITH G.R.** *Science,* 1995, vol. 267, 1166-1169
- **HABRAKEN Y. ; SUNG P. ; PRAKASH L. ; PRAKASH S.** *Nature,* 1993, vol. 366, 365-368
- **O'DONOVAN A. ; SCHERLY D. ; CLARKSON S.G. ; WOOD R.D.** *J. Biol. Chem.,* 1994, vol. 269, 15965-15968
- **O'DONOVAN A. ; DAVIES A.A. ; MOGGS J.G. ; WEST S.C. ; WOOD R.D.** *Nature,* 1994, vol. 371, 432-435
- **DIALLINAS G. ; GORFINKIEL L. ; ARST G. ; CECCHETTO G. ; SCAZZOCCHIO C.** *J. Biol. Chem.,* 1995, vol. 270, 8610-8622
- **ANDERSEN P.S. ; FREES D. ; FAST R. ; MYGIND B.** *J. Bacteriol.,* 1995, vol. 177, 2008-2013
- **CONRAD J. ; SUN D. ; ENGLUND N. ; OFENGAND J.** *J. Biol. Chem.,* 1998, vol. 273, 18562-18566
- **WRZESINSKI J. ; BAKIN A. ; NURSE K. ; LANE B.G. ; OFENGAND J.** *Biochemistry,* 1995, vol. 34, 8904-8913
- **TAN F. ; CHAN S.J. ; STEINER D.F. ; SCHILLING J.W. ; SKIDGEL R.A.** *J. Biol. Chem.,* 1989, vol. 264, 13165-13170
- **REYNOLDS D.S. ; STEVENS R.L. ; GURLEY D.S. ; LANE W.S. ; AUSTEN K.F. ; SERAFIN W.E.** *J. Biol. Chem.,* 1989, vol. 264, 20094-20099
- **NARAHASHI Y.** *J. Biochem.,* 1990, vol. 107, 879-886
- **TEPLYAKOV A. ; POLYAKOV K. ; OBMOLOVA G. ; STROKOPYTOV B. ; KURANOVA I. ; OSTERMAN A.L. ; GRISHIN N.V. ; SMULEVITCH S.V. ; ZAGNITKO O.P. ; GALPERINA O.V.** *Eur. J. Biochem.,* 1992, vol. 208, 281-288
- **HE G.-P. ; MUISE A. ; LI A.W. ; RO H.-S.** *Nature,* 1995, vol. 378, 92-96
- **HOURDOU M.-L. ; GUINAND M. ; VACHERON M.J. ; MICHEL G. ; DENOROY L. ; DUEZ C.M. ; ENGLEBERT S. ; JORIS B. ; WEBER G. ; GHUYSEN J.-M.** *Biochem. J.,* 1993, vol. 292, 563-570
- **KLUG A. ; RHODES D.** *Trends Biochem. Sci.,* 1987, 464-469
- **EVANS R.M. ; HOLLENBERG S.M.** *Cell,* 1988, vol. 52, 1-3
- **PAYRE F. ; VINCENT A.** *FEBS Lett.,* 1988, vol. 234, 245-250
- **MILLER J. ; MCLACHLAN A.D. ; KLUG A.** *EMBO J.,* 1985, vol. 4, 1609-1614
- **BERG J.M.** *Proc. Natl. Acad. Sci. U.S.A.,* 1988, vol. 85, 99-102
- **ROSENFELD R. ; MARGALIT H.** *J. Biomol. Struct. Dyn.,* 1993, vol. 11, 557-570

- **BORDEN K.L.B. ; FREEMONT P.S.** *Curr. Opin. Struct. Biol.,* 1996, vol. 6, 395-401
- **LINNEN JM ; BAILEY CP ; WEEKS DL.** *Gene,* 1993, vol. 128, 181-188
- **XIAO B ; SMERDON SJ ; JONES DH ; DODSON GG ; SONEJI Y ; AITKEN A ; GAMBLIN SJ.** *Nature,* 1995, vol. 376, 188-191
- **LIU D ; BIENKOWSKA J ; PETOSA C ; COLLIER RJ ; FU H ; LIDDINGTON R.** *Nature,* 1995, vol. 376, 191-194
- **MUSLIN AJ ; TANNER JW ; ALLEN PM ; SHAW AS.** *Cell,* 1996, vol. 84, 889-897
- **ICHIMURA T ; ITO M ; ITAGAKI C ; TAKAHASHI M ; HORIGOME T ; OMATA S ; OHNO S ; ISOBE T.** *FEBS Lett,* 1997, vol. 413, 273-276
- **WANG W ; SHAKES DC.** *J Mol Evol,* 1996, vol. 43, 384-398
- **JIN DY ; LYU MS ; KOZAK CA ; JEANG KT.** *Nature,* 1996, vol. 382, 308-308
- **AITKEN A.** *Trends Biochem. Sci.,* 1995, vol. 20, 95-97
- **MORRISON D.** *Science,* 1994, vol. 266, 56-57
- **XIAO B. ; SMERDON S.J. ; JONES D.H. ; DODSON G.G. ; SONEJI Y. ; AITKEN A. ; GAMBLIN S.J.** *Nature,* 1995, vol. 376, 188-191
- **GRANT G.A.** *Biochem. Biophys. Res. Commun.,* 1989, vol. 165, 1371-1374
- **KOCHHAR S. ; HUNZIKER P. ; LEONG-MORGENTHALER P.M. ; HOTTINGER H.** *Biochem. Biophys. Res. Commun.,* 1992, vol. 184, 60-66
- **OHTA T. ; TAGUCHI H.** *J. Biol. Chem.,* 1991, vol. 266, 12588-12594
- **GOLDBERG J.D. ; YOSHIDA T. ; BRICK P.** *J. Mol. Biol.,* 1994, vol. 236, 1123-1140
- **POPOV V.O. ; LAMZIN V.S.** *Biochem. J.,* 1994, vol. 301, 625-643
- **MATTEVI A ; OBMOLOVA G ; KALK KH ; WESTPHAL AH ; DE KOK A ; HOL WG.** *J Mol Biol,* 1993, vol. 230, 1183-1199
- **LABRIE F ; SIMARD J ; LUU-THE V ; PELLETIER G ; BELANGER A ; LACHANCE Y ; ZHAO HF ; LABRIE C ; BRETON N ; DE LAUNOIT Y et al.** *J Steroid Biochem Mol Biol,* 1992, vol. 41, 421-435
- **BIRKTOFT JJ ; HOLDEN HM ; HAMLIN R ; XUONG NH ; BANASZAK LJ.** *Proc Natl Acad Sci U S A,* 1987, vol. 84, 8262-8266
- **BIRKTOFF J.J. ; HOLDEN H.M. ; HAMLIN R. ; XUONG N.-H. ; BANASZAK L.J.** *Proc. Natl. Acad. Sci. U.S.A.,* 1987, vol. 84, 8262-8266
- **MULLANY P. ; CLAYTON C.L. ; PALLEN M.J. ; SLONE R. ; AL-SALEH A. ; TABAQCHALI S.** *FEMS Microbiol. Lett.,* 1994, vol. 124, 61-67
- **MULDERS J.W.M. ; HENDRIKS W. ; BLANKESTEIJN W.M. ; BLOEMENDAL H. ; DE JONG W.W.** *J. Biol. Chem.,* 1988, vol. 263, 15462-15466
- **REMACHA M ; JIMENEZ-DIAZ A ; SANTOS C ; BRIONES E ; ZAMBRANO R ; RODRIGUEZ GABRIEL MA ; GUARINOS E ; BALLESTA JP.** *Biochem Cell Biol,* 1995, vol. 73, 959-968
- **REIZER A. ; DEUTSCHER J. ; SAIER M.H. JR. ; REIZER J.** *Mol. Microbiol.,* 1991, vol. 5, 1081-1089
- **ADAMS M.J. ; ARCHIBALD I.G. ; BUGG C.E. ; CARNE A. ; GOVER S. ; HELLIWELL J.R. ; PICKERSGILL R.W. ; WHITE S.W.** *EMBO J.,* 1983, vol. 2, 1009-1014
- **STROSBERG A.D.** *Eur. J. Biochem.,* 1991, vol. 196, 1-10
- **KERLAVAGE A.R.** *Curr. Opin. Struct. Biol.,* 1991, vol. 1, 394-401
- **PROBST W.C. ; SNYDER L.A. ; SCHUSTER D.I. ; BROSIUS J. ; SEALFON S.C.** *DNA Cell Biol.,* 1992, vol. 11, 1-20
- **SAVARESE T.M. ; FRASER C.M.** *Biochem. J.,* 1992, vol. 283, 1-9
- **BRANCHEK T.** *Curr. Biol.,* 1993, vol. 3, 315-317
- **STILES G.L.** *J. Biol. Chem.,* 1992, vol. 267, 6451-6454
- **FRIELL T. ; KOBILKA B.K. ; LEFKOWITZ R.J. ; CARON M.G.** *Trends Neurosci.,* 1988, vol. 11, 321-324
- **STEVENS C.F.** *Curr. Biol.,* 1991, vol. 1, 20-22
- **SAKURAI T. ; YANAGISAWA M. ; MASAKI T.** *Trends Pharmacol. Sci.,* 1992, vol. 13, 103-107
- **SALESSE R. ; REMY J.J. ; LEVIN J.M. ; JALLAL B. ; GARNIER J.** *Biochimie,* 1991, vol. 73, 109-120
- **LANCET D. ; BEN-ARIE N.** *Curr. Biol.,* 1993, vol. 3, 668-674
- **UHL G.R. ; CHILDERS S. ; PASTERNAK G.** *Trends Neurosci.,* 1994, vol. 17, 89-93
- **BARNARD E.A. ; BURNSTOCK G. ; WEBB T.E.** *Trends Pharmacol. Sci.,* 1994, vol. 15, 67-70
- **APPLEBURY M.L. ; HARGRAVE P.A.** *Vision Res.,* 1986, vol. 26, 1881-1895
- **ATTWOOD T.K. ; ELIOPOULOS E.E. ; FINDLAY J.B.C.** *Gene,* 1991, vol. 98, 153-159
- **FRYXELL K.J. ; MEYEROWITZ E.M.** *J. Mol. Evol.,* 1991, vol. 33, 367-378
- **SHEN D. ; JIANG M. ; HAO W. ; TAO L. ; SALAZAR M. ; FONG H.K.W.** *Biochemistry,* 1994, vol. 33, 13117-13125
- **NICOLAS RH ; GOODWIN GH.** Medline: 97074677. Molecular cloning of polybromo, a nuclear protein containing multiple domains including five bromodomains, a truncated HMG-box, and two repeats of a novel domain. *Gene,* 1996, vol. 175, 233-240

- **CALLEBAUT I ; COURVALIN J-C ; MORNON JP.** Medline: 99198739. The BAH (bromo-adjacent homology) domain: a link between DNA methylation, replication and transcriptional regulation. *FEBS letts,* 1999, vol. 446, 189-193
- **BENZ R.** *Biochim. Biophys. Acta,* 1994, vol. 1197, 167-196
- **MANELLA C.A.** *Trends Biochem. Sci.,* 1992, vol. 17, 315-320
- **DIHANICH M.** *Experientia,* 1990, vol. 46, 146-153
- **FORTE M. ; GUY H.R. ; MANNELLA C.A.** *J. Bioenerg. Biomembr.,* 1987, vol. 19, 341-350
- **SAMPSON M.J. ; LOVELL R.S. ; DAVISON D.B. ; CRAIGEN W.J.** *Genomics,* 1996, vol. 36, 192-196
- **NAN X ; MEEHAN RR ; BIRD A.** Medline: 94232813. Dissection of the methyl-CpG binding domain from the chromosomal protein MeCP2. *Nucleic Acids Res,* 1993, vol. 21, 4886-4892
- **BHATTACHARYA SK ; RAMCHANDANI S ; CERVONI N ; SZYF M.** Medline: 99158138. A mammalian protein with specific demethylase activity for mCpG DNA. *Nature,* 1999, vol. 397, 579-583
- **ABDULLAH K.M. ; LO R.Y.C. ; MELLORS A.** *J. Bacteriol.,* 1991, vol. 173, 5597-5603
- **PONTING CP.** Medline: 96100659 SAM: A novel motif in yeast sterile alpha and Drosophila polyhomeotic proteins. *Prot Sci,* 1995, vol. 4, 1928-1930
- **SHULTZ J ; PONTING CP ; HOFMANN K ; BORK P.** Medline: 97160498 SAM as a protein interaction domain involved in developmental regulation. *Prot Sci,* 1997, vol. 6, 249-253
- **STAPLETON D ; BALAN I ; PAWSON T ; SICHERI F.** Medline: 99101382 The crystal structure of an Eph receptor SAM domain reveals a mechanism for modular dimerization. *Nat Struct Biol,* 1999, vol. 6, 44-49
- **LERCH K.** *Prog. Clin. Biol. Res.,* 1988, vol. 256, 85-98
- **JACKMAN M.P. ; HAJNAL A. ; LERCH K.** *Biochem. J.,* 1991, vol. 274, 707-713
- **LINZEN B.** *Naturwissenschaften,* 1989, vol. 76, 206-211
- **LANG W.H. ; VAN HOLDE K.E.** *Proc. Natl. Acad. Sci. U.S.A.,* 1991, vol. 88, 244-248
- **KOBAYASHI T. ; URABE K. ; WINDER A. ; JIMENEZ-CERVANTES C. ; IMOKAWA G. ; BREWINGTON T. ; SOLANO F. ; GARCIA-BORRON J.C. ; HEARING V.J.** *EMBO J.,* 1994, vol. 13, 5818-5825
- **JACKSON I.J. ; CHAMBERS D.M. ; TSUKAMOTO K. ; COPELAND N.G. ; GILBERT D.J. ; JENKINS N.A. ; HEARING V.** *EMBO J.,* 1992, vol. 11, 527-535
- **SOLANO F. ; MARTINEZ-LIARTE J.H. ; JIMENEZ-CERVANTES C. ; GARCIA-BORRON J.C. ; LOZANO J.A.** *Biochem. Biophys. Res. Commun.,* 1994, vol. 204, 1243-1250
- **CARY J.W. ; LAX A.R. ; FLURKEY W.H.** *Plant Mol. Biol.,* 1992, vol. 20, 245-253
- **SHANE B. ; GARROW T. ; BRENNER A. ; CHEN L. ; CHOI Y.J. ; HSU J.C. ; STOVER P.** *Adv. Exp. Med. Biol.,* 1993, vol. 338, 629-634
- **JONGENEEL C.V. ; BOUVIER J. ; BAIROCH A.** *FEBS Lett.,* 1989, vol. 242, 211-214
- **MURPHY G.J.P. ; MURPHY G. ; REYNOLDS J.J.** *FEBS Lett.,* 1991, vol. 289, 4-7
- **BODE W. ; GRAMS F. ; REINEMER P. ; GOMIS-RUETH F.-X. ; BAUMANN U. ; MCKAY D.B. ; STOECKER W.** *Zoology,* 1996, vol. 99, 237-246
- **MONTECUCCO C. ; SCHIAVO G.** *Trends Biochem. Sci.,* 1993, vol. 18, 324-327
- **NIEMANN H. ; BLASI J. ; JAHN R.** *Trends Cell Biol.,* 1994, vol. 4, 179-185
- **ARLUISON V ; HOUNTONDJI C ; ROBERT B ; GROSJEAN H.** Medline: 98254513. Transfer RNA-pseudouridine synthetase Pus1 of Saccaromyces cerevisiae contains one atom of zinc essential for its native conformation and tRNA recognition. *Biochemistry,* 1998, vol. 37, 7268-7276
- **STALLINGS W.C. ; ABDEL-MEGID S.S. ; LIM L.W. ; SHIEH H.-S. ; DAYRINGER H.E. ; LEIMGRUBER N.K. ; STEGEMAN R.A. ; ANDERSON K.S. ; SIKORSKI J.A. ; PADGETTE S.R.** *Proc. Natl. Acad. Sci. U.S.A.,* 1991, vol. 88, 5046-5050
- **PADGETTE S.R. ; RE D.B. ; GASER C.S. ; EICHOLTZ D.A. ; FRAZIER R.B. ; HIRONAKA C.M. ; LEVINE E.B. ; SHAH D.M. ; FRALEY R.T. ; KISHORE G.M.** *J. Biol. Chem.,* 1991, vol. 266, 22364-22369
- **PERRAKIS A ; TEWS I ; DAUTER Z ; OPPENHEIM AB ; CHET I ; WILSON KS ; VORGIAS CE.** Medline: 95219379. Crystal structure of a bacterial chitinase at 2.3 A resolution. *Structure,* 1994, vol. 2, 1169-1180
- **BULL P.C. ; COX D.W.** *Trends Genet.,* 1994, vol. 10, 246-252
- **LIN S.-J. ; CULOTTA V.L.** *Proc. Natl. Acad. Sci. U.S.A.,* 1995, vol. 92, 3784-3788
- **SANCHEZ-LOPEZ R. ; NICHOLSON R. ; GESNEL M.C. ; MATRISIAN L.M. ; BREATHNACH R.** *J. Biol. Chem.,* 1988, vol. 263, 11892-11899
- **PARK A.J. ; MATRISIAN L.M. ; KELLS A.F. ; PEARSON R. ; YUAN Z. ; NAVRE M.** *J. Biol. Chem.,* 1991, vol. 266, 1584-1590
- **LEPAGE T. ; GACHE C.** *EMBO J.,* 1990, vol. 9, 3003-3012
- **KINOSHITA T. ; FUKUZAWA H. ; SHIMADA T. ; SAITO T. ; MATSUDA Y.** *Proc. Natl. Acad. Sci. U.S.A.,* 1992, vol. 89, 4693-4697

- **SIEZEN R.J. ; DE VOS W.M. ; LEUNISSEN J.A.M. ; DIJKSTRA B.W.** *Protein Eng.,* 1991, vol. 4, 719-737
- **SIEZEN R.J.** *Proceeding subtilisin symposium,* 1992
- **BARR P.J.** *Cell,* 1991, vol. 66, 1-3
- **SHAULSKY G. ; KUSPA A. ; LOOMIS W.F.** *Genes Dev.,* 1995, vol. 9, 1111-1122
- **MEYER R.R. ; LAINE P.S.** *Microbiol. Rev.,* 1990, vol. 54, 342-380
- **STROUMBAKIS N.D. ; LI Z. ; TOLIAS P.P.** *Gene,* 1994, vol. 143, 171-177
- **VLAHOS C J. ; DEKKER E.E.** *J. Biol. Chem.,* 1988, vol. 263, 11683-11691
- **BARZILAY G. ; HICKSON I.S.** *BioEssays,* 1995, vol. 17, 713-719
- **MOL C.D. ; KUO C.-F. ; THAYER M.M. ; CUNNINGHAM R.P. ; TAINER J.A.** *Nature,* 1995, vol. 374, 381-386
- **GELSTHORPE M. ; PULUMATI M. ; MCCALLUM C. ; DANG-VU K. ; TSUBOTA S.I.** *Gene,* 1997, vol. 186, 189-195
- **DRICKAMER K.** *J. Biol. Chem.,* 1988, vol. 263, 9557-9560
- **DRICKAMER K.** *Prog. Nucleic Acid Res. Mol. Biol.,* 1993, vol. 45, 207-232
- **DRICKAMER K.** *Curr. Opin. Struct. Biol.,* 1993, vol. 3, 393-400
- **SPIESS M.** *Biochemistry,* 1990, vol. 29, 10009-10018
- **WEIS W.I. ; KAHN R. ; FOURME R. ; DRICKAMER K. ; HENDRICKSON W.A.** *Science,* 1991, vol. 254, 1608-1615
- **SIEGELMAN M.** *Curr. Biol.,* 1991, vol. 1, 125-128
- **LASKY L.A.** *Science,* 1992, vol. 238, 964-969
- **JOMORI T. ; NATORI S.** *J. Biol. Chem.,* 1991, vol. 266, 13318-13323
- **NG N.F.L. ; HEW C.-L.** *J. Biol. Chem.,* 1992, vol. 267, 16069-16075
- **DANGOTT J.J. ; JORDAN J.E. ; BELLET R.A. ; GARBERS D.L.** *Proc. Natl. Acad. Sci. U.S.A.,* 1989, vol. 86, 2128-2132
- **FREEMAN M. ; ASHKENAS J. ; REES D.J. ; KINGSLEY D.M. ; COPELAND N.G. ; JENKINS N.A. ; KRIEGER M.** *Proc. Natl. Acad. Sci. U.S.A.,* 1990, vol. 87, 8810-8814
- **FLACK FS ; LOOSMORE S ; CHONG P ; THOMAS WR.** Medline: 95255676. The sequencing of the 80-kDa D15 protective surface antigen of Haemophilus influenzae. *Gene,* 1995, vol. 156, 97-99
- **RUFFOLO CG ; ADLER B.** Medline: 96333354. Cloning, sequencing, expression, and protective capacity of the oma87 gene encoding the Pasteurella multocida 87-kilodalton outer membrane antigen. *Infect Immun,* 1996, vol. 64, 3161-3167
- **KOONIN EV ; ALTSCHUL SF ; BORK P.** Medline: 96259550. BRCA1 protein products ...Functional motifs... *Nature Genet,* 1996, vol. 13, 266-268
- **CALLEBAUT I ; MORNON JP.** Medline: 97153217. From BRCA1 to RAP1: A widespread BRCT module closely associated with DNA repair. *Febs lett,* 1997, vol. 400, 25-30
- **BORK P ; HOFMANN K ; BUCHER P ; NEUWALD AF ; ALTSCHUL SF ; KOONIN EV.** Medline: 97186552. A superfamily of conserved domains in DNA damage responsive cell cycle checkpoint proteins. *Faseb J,* 1997, vol. 11, 68-76
- **ALTSCHUL SF ; MADDEN TL ; SCHAFFER AA ; ZHANG J ; ZHANG Z ; MILLER W ; LIPMAN DJ.** Medline: 97402527. Gapped BLAST and PSI-BLAST: a new generation of protein database search programs. *Nucleic Acids Res,* 1997, vol. 25, 3389-3402
- **ZHANG X ; MORERA S ; BATES PA ; WHITEHEAD PC ; COFFER AI ; HAINBUCHER K ; NASH RA ; STERNBERG MJ ; LINDAHL T ; FREEMONT PS.** *Medline: 99016060. Structure of an XRCC1 BRCT domain: a new protein-protein interaction module*
- **WARD TJ ; HONEYCUTT RL ; DERR JN.** Medline: 98072500. Nucleotide sequence evolution at the kappa-casein locus: evidence for positive selection within the family Bovidae. *Genetics,* 1997, vol. 147, 1863-1872
- **WATANABE T. ; KOHORI K. ; MIYASHITA K. ; FUJII T. ; SAKAI H. ; UCHIDA M. ; TANAKA H.** *J. Biol. Chem.,* 1993, vol. 268, 18567-18572
- **PERRAKIS A. ; TEWS I. ; DAUTER Z. ; OPPENHEIM A.B. ; CHET I. ; WILSON K.S. ; VORGIAS C.E.** *Structure,* 1994, vol. 2, 1169-1180
- **VAN SCHELTINGA A.C.T. ; KALK K.H. ; BEINTEMA J.J. ; DIJKSTRA B.W.** *Structure,* 1994, vol. 2, 1181-1189
- **MASSIAH MA ; STARICH MR ; PASCHALL C ; SUMMERS MF ; CHRISTENSEN AM ; SUNDQUIST WI.** Medline: 95055757. Three-dimensional structure of the human immunodeficiency virus type 1 matrix protein. *J Mol Biol,* 1994, vol. 244, 198-223
- **HANDA M. ; GUIDOTTI G.** *Biochem. Biophys. Res. Commun.,* 1996, vol. 218, 916-923
- **VASCONCELOS E.G. ; FERREIRA S.T. ; DE CARVALHO T.M.U. ; DE SOUZA W. ; KETTLUN A.M. ; MANCILLA M. ; VALENZUELA M.A. ; VERJOVSKI-ALMEIDA S.** *J. Biol. Chem.,* 1996, vol. 271, 22139-22145
- **MAIER J. ; WITTER K. ; GUETLICH M. ; ZIEGLER I. ; WERNER T. ; NINNEMANN H.** *Biochem. Biophys. Res. Commun.,* 1995, vol. 212, 705-711
- **NAR H. ; HUBER R. ; MEINING W. ; SCHMID C. ; WEINKAUF S. ; BACHER A.** *Structure,* 1995, vol. 3, 459-466

- **BIOU V ; DUMAS R ; COHEN-ADDAD C ; DOUCE R ; JOB D ; PEBAY-PEYROULA E.** Medline: 97361822. The crystal structure of plant acetohydroxy acid isomeroreductase complexed with NADPH, two magnesium ions and a herbicidal transition state analog determined at 1.65 A resolution. *EMBO J,* 1997, vol. 16, 3405-3415
- **HAYASHI S. ; WUH.C.** *J. Bioenerg. Biomembr.,* 1990, vol. 22, 451-471
- **KLEIN P. ; SOMORJAI R.L. ; LAU P.C.K.** *Protein Eng.,* 1988, vol. 2, 15-20
- **VON HEIJNE G.** *Protein Eng.,* 1989, vol. 2, 531-534
- **BENKOVIC S.J.** *Annu. Rev. Biochem.,* 1980, vol. 49, 227-251
- **ROSS P. ; O'GARA F. ; CONDON S.** *Appl. Environ. Microbiol.,* 1990, vol. 56, 2156-2163
- **KINSELLA B.T. ; HOGAN S. ; LARKIN A. ; CANTWELL B.A.** *Eur. J. Biochem.,* 1991, vol. 202, 657-664
- **NAIM H.Y. ; NIERMANN T. ; KLEINHANS U. ; HOLLENBERG C.P. ; STRASSER A.W.M.** *FEBS Lett.,* 1991, vol. 294, 109-112
- **HERMANS M.M.P. ; KROOS M.A. ; VAN BEEUMEN J. ; OOSTRA B.A. ; REUSER A.J.J.** *J. Biol. Chem.,* 1991, vol. 266, 13507-13512
- **TAKISHIMA K. ; SUGA T. ; MAMIYA G.** *Eur. J. Biochem.,* 1988, vol. 175, 151-165
- **MOBLEY H.L.T. ; HUSINGER R.P.** *Microbiol. Rev.,* 1989, vol. 53, 85-108
- **JABRI E. ; CARR M.B. ; HAUSINGER R.P. ; KARPLUS P.A.** *Science,* 1995, vol. 268, 998-1004
- **GOODENOUGH D.A. ; GOLIGER J.A. ; PAUL D.L.** *Annu. Rev. Biochem.,* 1996, vol. 65, 475-502
- **SCHNEEWIND O. ; JONES K.F. ; FISCHETTI V.A.** *J. Bacteriol.,* 1990, vol. 172, 3310-3317
- **TATE S.S. ; MEISTERA.** *Meth. Enzymol.,* 1985, vol. 113, 400-419
- **SUZUKI H. ; KUMAGAI H. ; ECHIGO T. ; TOCHIKURA T.** *J. Bacteriol.,* 1989, vol. 171, 5169-5172
- **ISHIYE M. ; NIWA M.** *Biochim. Biophys. Acta,* 1992, vol. 1132, 233-239
- **LABBE-BOIS R.** *J. Biol. Chem.,* 1990, vol. 265, 7278-7283
- **BRENNER D.A. ; FRASIER F.** *Proc. Natl. Acad. Sci. U.S.A.,* 1991, vol. 88, 849-853
- **MIYAMOTO K. ; NAKAHIGASHI K. ; NISHIMURA K. ; INOKUCHI H.** *J. Mol. Biol.,* 1991, vol. 219, 393-398
- **MEINKE A. ; GILKES N.R. ; KILBURN D.G. ; MILLER R.C. JR. ; WARREN R.A.J.** *Protein Seq. Data Anal.,* 1991, vol. 4, 349-353
- **GILKES N.R. ; CLAEYSSENS M. ; AEBERSOLD R. ; HENRISSAT B. ; MEINKE A. ; MORRISON H.D. ; KILBURN D.G. ; WARREN R.A.J. ; MILLER R.C. JR.** *Eur. J. Biochem.,* 1991, vol. 202, 367-377
- **MAYAUX J.-F. ; CERBELAUD E. ; SOUBRIER F. ; FAUCHER D. ; PETRE D.** *J. Bacteriol.,* 1990, vol. 172, 6764-6773
- **HASHIMOTO Y. ; NISHIYAMA M. ; IKEHATA O. ; HORINOUCHI S. ; BEPPU T.** *Biochim. Biophys. Acta,* 1991, vol. 1088, 225-233
- **CHANG T.-H. ; ABELSON J.** *Nucleic Acids Res.,* 1990, vol. 18, 7180-7180
- **TSUCHIYA K. ; FUKUYAMA S. ; KANZAKI N. ; KANAGAWA K. ; NEGORO S. ; OKADA H.** *J. Bacteriol.,* 1989, vol. 171, 3187-3191
- **CURNOW A.W. ; HONG K.W. ; YUAN R. ; KIM S.I. ; MARTINS O. ; WINKLER W. ; HENKIN T.M. ; SOLL D.** *Proc. Natl. Acad. Sci. U.S.A.,* 1997, vol. 94, 11819-11826
- **CRAVATT B.F. ; GIANG D.K. ; MAYFIELD S.P. ; BOGER D.L. ; LERNER R.A. ; GILULA N.B.** *Nature,* 1996, vol. 384, 83-87
- **BEGUIN P.** *Annu. Rev. Microbiol.,* 1990, vol. 44, 219-248
- **HENRISSAT B. ; CLAEYSSENS M. ; TOMME P. ; LEMESLE L. ; MORNON J.-P.** *Gene,* 1989, vol. 81, 83-95
- **TULL D. ; WITHERS S.G. ; GILKES N.R. ; KILBURN D.G. ; WARREN R.A.J. ; AEBERSOLD R.** *J. Biol. Chem.,* 1991, vol. 266, 15621-15625
- **PERHAM R.N.** *Biochem. Soc. Trans.,* 1990, vol. 18, 185-187
- **MARSH J.J. ; LEBHERZ H.G.** *Trends Biochem. Sci.,* 1992, vol. 17, 110-113
- **VON DER OSTEN C.H. ; BARBAS C.F. III ; WONG C.-H. ; SINSKEY A.J.** *Mol. Microbiol.,* 1989, vol. 3, 1625-1637
- **BERRY A. ; MARSHALL K.E.** *FEBS Lett.,* 1993, vol. 318, 11-16
- **BARRETT A.J. ; RAWLINGS N.D.** *Biol. Chem. Hoppe-Seyler,* 1992, vol. 373, 353-360
- **POLGAR L. ; SZABO E.** *Biol. Chem. Hoppe-Seyler,* 1992, vol. 373, 361-366
- **LOVELL C.R. ; PRZYBYLA A. ; LJUNGDAHL L.G.** *Biochemistry,* 1990, vol. 29, 5687-5694
- **SLOCK J. ; STAHLY D.P. ; HAN C.-Y. ; SIX E.W. ; CRAWFORD I.P.** *J. Bacteriol.,* 1990, vol. 172, 7211-7226
- **VOLPES F. ; DYER M. ; SCAIFE J.G. ; DARBY G. ; STAMMERS D.K. ; DELVES C.J.** *Gene,* 1992, vol. 112, 213-218
- **HILES I.D. ; OTSU M. ; VOLINIA S. ; FRY M.J. ; GOUT I. ; DHAND R. ; PANAYOTOU G. ; RUIZ-LARREA F. ; THOMPSON A. ; TOTTY N.F.** *Cell,* 1992, vol. 70, 419-429
- **KUNZ J. ; HENRIQUEZ R. ; SCHNEIDER U. ; DEUTER-REINHARD M. ; MOVVA N. ; HALL M.N.** *Cell,* 1993, vol. 73, 585-596
- **SCHU P.V. ; TAKEGAWA K. ; FRY M.J. ; STACK J.H. ; WATERFIELD M.D. ; EMR S.D.** *Science,* 1993, vol. 260, 88-91

- **GARCIA-BUSTOS J.F. ; MARINI F. ; STEVENSON I. ; FREI C. ; HALL M.N.** *EMBO J.,* 1994, vol. 13, 2352-2361
- **BROWN E.J. ; ALBERS M.W. ; SHIN T.B. ; ICHIKAWA K. ; KEITH C.T. ; LANE W.S. ; SCHREIBER S.L.** *Nature,* 1994, vol. 369, 756-758
- **KATO R. ; OGAWA H.** *Nucleic Acids Res.,* 1994, vol. 22, 3104-3112
- **AUSTIN D. ; LARSON T.J.** *J. Bacteriol.,* 1991, vol. 173, 101-107
- **ROENNOW B. ; KIELLAND-BRANDT M.C.** *Yeast,* 1993, vol. 9, 1121-1130
- **BROWN L.J. ; MCDONALD M.J. ; LEHN D.A. ; MORAN S.M.** *J. Biol. Chem.,* 1994, vol. 269, 14363-14366
- **MENENDEZ C. ; IGLOI G. ; HENNINGER H. ; BRANDSCH R.** *Arch. Microbiol.,* 1995, vol. 164, 142-151
- **HOFF T. ; SCHNORR K.M. ; MEYER C. ; CABOCHE M.** *J. Biol. Chem.,* 1995, vol. 270, 6100-6107
- **HOFMANN K.O. ; BUCHER P.** *Trends Biochem. Sci.,* 1995, vol. 20, 347-349
- **NAVAS T.A. ; ZHOU Z. ; ELLEDGE S.J.** *Cell,* 1995, vol. 80, 29-39
- **ROMAO MJ ; ARCHER M ; MOURA I ; MOURA JJ ; LEGALL J ; ENGH R ; SCHNEIDER M ; HOF P ; HUBER R.** Medline: 96072968 "Crystal structure of the xanthine oxidase-related aldehyde oxido-reductase from D. gigas. *Science,* 1995, vol. 270, 1170-1176
- **HENRISSAT B.** Medline: 98313424; "Glycosidase families. *Biochem Soc Trans,* 1998, vol. 26, 153-156
- **HUIBREGTSE JM ; SCHEFFNER M ; BEAUDENON S ; HOWLEY PM.** Medline: 95223981; "A family of proteins structurally and functionally related to the E6-AP ubiquitin-protein ligase. *Proc Natl Acad Sci U S A,* 1995, vol. 92, 2563-2567
- **MOROZOV V ; MUSHEGIAN AR ; KOONIN EV ; BORK P.** Medline: 98060076; "A putative nucleic acid-binding domain in Bloom's and Werner's syndrome helicases. *Trends Biochem Sci,* 1997, vol. 22, 417-418
- **DYDA F ; HICKMAN AB ; JENKINS TM ; ENGELMAN A ; CRAIGIE R ; DAVIES DR.** Medline: 95099322. "Crystal structure of the catalytic domain of HIV-1 integrase: similarity to other polynucleotidyl transferases. *Science,* 1994, vol. 266, 1981-1986
- **LODI PJ ; ERNST JA ; KUSZEWSKI J ; HICKMAN AB ; ENGELMAN A ; CRAIGIE R ; CLORE GM ; GRONENBORN AM.** Medline: 95359147; "Solution structure of the DNA binding domain of HIV-1 integrase. *Biochemistry,* 1995, vol. 34, 9826-9833
- **TONG G ; SHEPHERD D ; JAHR CE.** Medline: 95184014; "Synaptic desensitization of NMDA receptors by calcineurin. *Science,* 1995, vol. 267, 1510-1512
- **MUSACCHIO A ; CANTLEY LC ; HARRISON SC.** Medline: 97121392; "Crystal structure of the breakpoint cluster region-homology domain from phosphoinositide 3-kinase p85 alpha subunit. *Proc Natl Acad Sci U S A,* 1996, vol. 93, 14373-14378
- **BARRETT T ; XIAO B ; DODSON EJ ; DODSON G ; LUDBROOK SB ; NURMAHOMED K ; GAMBLIN SJ ; MUSACCHIO A ; SMERDON SJ ; ECCLESTON JF.** Medline: 97162209; "The structure of the GTPase-activating domain from p50rhoGAP. *Nature,* 1997, vol. 385, 458-461
- **RITTINGER K ; WALKER PA ; ECCLESTON JF ; NURMAHOMED K ; OWEN D ; LAUE E ; GAMBLIN SJ ; SMERDON SJ.** Medline: 97404320; "Crystal structure of a small G protein in complex with the GTPase-activating protein rhoGAP. *Nature,* 1997, vol. 388, 693-697
- **BOGUSKI MS ; MCCORMICK F.** Medline: 94081948; "Proteins regulating Ras and its relatives. *Nature,* 1993, vol. 366, 643-654
- **BORK P.** Medline: 93327926; "The modular architecture of a new family of growth regulators related to connective tissue growth factor. *FEBS lett,* 1993, vol. 327, 125-130
- **TSE-DINH YC ; BERAN-STEED RK.** Medline: 89034032; "Escherichia coli DNA topoisomerase I is a zinc metalloprotein with three repetitive zinc-binding domains. *J Biol Chem,* 1988, vol. 263, 15857-15859
- **AHUMADA A ; TSE-DINH YC.** Medline: 99011409; "The Zn(II) binding motifs of E. coli DNA topoisomerase I is part of a high-affinity DNA binding domain. *Biochem Biophys Res Commun,* 1998, vol. 251, 509-514
- **HAYNES S.R. ; DOOLARD C. ; WINSTON F. ; BECK S. ; TROWSDALE J. ; DAWID I.B.** *Nucleic Acids Res.,* 1992, vol. 20, 2693-2603
- **TAMKUN J.W. ; DEURING R. ; SCOTT M.P. ; KISSINGER M. ; PATTATUCCI A.M. ; KAUFMAN T.C. ; KENNISON J.A.** *Cell,* 1992, vol. 68, 561-572
- **TAMKUN J.W.** *Curr. Opin. Genet. Dev.,* 1995, vol. 5, 473-477
- **SCHLEICHER M. ; ANDRE E. ; HARMANN A. ; NOEGEL A.A.** *Dev. Genet.,* 1988, vol. 9, 521-530
- **MATSUDAIRA P.** *Trends Biochem. Sci.,* 1991, vol. 16, 87-92
- **DUBREUIL R.R.** *BioEssays,* 1991, vol. 13, 219-226
- **GARCIA-HORSMAN J.A. ; BARQUERA B. ; RUMBLEY J. ; MA J. ; GENNIS R.B.** *J. Bacteriol.,* 1994, vol. 176, 5587-5600

- **CASTRESANA J. ; LUEBBEN M. ; SARASTE M. ; HIGGINS D.G.** *EMBO J.,* 1994, vol. 13, 2516-2525
- **HOLM L. ; SARASTE M. ; WIKSTROM M.** *EMBO J.,* 1987, vol. 6, 2819-2823
- **SARASTE M. ; CASTRESANA J.** *FEBS Lett.,* 1994, vol. 341, 1-4
- **TOMKINSON A.E. ; TOTTY N.F. ; GINSBURG M. ; LINDAHL T.** *Proc. Natl. Acad. Sci. U.S.A.,* 1991, vol. 88, 400-404
- **LINDAHL T. ; BARNES D.E.** *Annu. Rev. Biochem.,* 1992, vol. 61, 251-281
- **KLETZIN A.** *Nucleic Acids Res.,* 1992, vol. 20, 5389-5396
- **DUWAT P. ; DE OLIVEIRA R. ; EHRLICH S.D. ; BOITEUX S.** *Microbiology,* 1995, vol. 141, 411-417
- **O'CONNOR T.E. ; GRAVES R.J. ; DEMURCIA G. ; CASTAING B. ; LAVAL J.** *J. Biol. Chem.,* 1993, vol. 268, 9063-9070
- **TATE S.S. ; MEISTER A.** *Meth. Enzymol.,* 1985, vol. 113, 400-419
- **PENNINGTON S.R.** *Protein Prof.,* 1995, vol. 2, 16-315
- **BAIROCH A.** *Unpublished observations,* 1996
- **EL-SHERBEINI M. ; CLEMAS J.A.** *J. Bacteriol.,* 1995, vol. 177, 3227-3234
- **GARCIA-ARRANZ M. ; MALDONADO A.M. ; MAZON M.J. ; PORTILLO F.** *J. Biol. Chem.,* 1994, vol. 269, 18076-18082
- **GOUGH N.** *Trends Biochem. Sci.,* 1981, vol. 6, 203-205
- **KLEIN J. ; FIGUEROA F.** *Immunol. Today,* 1986, vol. 7, 41-44
- **FIGUEROA F. ; KLEIN J.** *Immunol. Today,* 1986, vol. 7, 78-81
- **ORR H.T. ; LANCET D. ; ROBB R.J. ; LOPEZ DE CASTRO J.A. ; STROMINGER J.L.** *Nature,* 1979, vol. 282, 266-270
- **CUSHLEY W. ; OWEN M.** *J. Immunol. Today,* 1983, vol. 4, 88-92
- **BECK S. ; BARREL B.G.** *Nature,* 1988, vol. 331, 269-272
- **RECHLER M.M.** *Vitam. Horm.,* 1993, vol. 47, 1-114
- **SHIMASAKI S. ; LING N.** *Prog. Growth Factor Res.,* 1991, vol. 3, 243-266
- **CLEMMONS D.R.** *Trends Endocrinol. Metab.,* 1990, vol. 1, 412-417
- **BRADHAM D.M. ; IGARASHI A. ; POTTER R.L. ; GROTENDORST G.R.** *J. Cell Biol.,* 1991, vol. 114, 1285-1294
- **MALOISEL V. ; MARTINERIE C. ; DAMBRINE G. ; PLASSIART G. ; BRISAC M. ; CROCHET J. ; PERBAL B.** *Mol. Cell. Biol.,* 1992, vol. 12, 10-21
- **SU XD ; TADDEI N ; STEFANI M ; RAMPONI G ; NORDLUND P.** Medline: 94329182, The crystal structure of a low-molecular-weight phosphotyrosine protein phosphatase. *Nature,* 1994, vol. 370, 575-578
- **WALKER J.E. ; SARASTE M. ; RUNSWICK M.J. ; GAY N.J.** *EMBO J.,* 1982, vol. 1, 945-951
- **COLE ST ; BROSCH R ; PARKHILL J ; GARNIER T ; CHURCHER C ; HARRIS D ; GORDON SV ; EIGLMEIER K ; GAS S ; BARRY CE 3RD.** Medline: 98295987. Deciphering the biology of Mycobacterium tuberculosis from the complete genome sequence. *Nature,* 1998, vol. 393, 537-544
- **ZILHAO R. ; CAMELO L. ; ARRAIANO C.M.** *Mol. Microbiol.,* 1993, vol. 8, 43-51
- **NOGUCHI E. ; HAYASHI N. ; AZUMA Y. ; SEKI T. ; NAKAMURA M. ; NAKASHIMA N. ; YANAGIDA M. ; HE X. ; MUELLER U. ; SAZER S.** *EMBO J.,* 1996, vol. 15, 5595-5605
- **BEUF L. ; BEDU S. ; CAMI B. ; JOSET F.** *Plant Mol. Biol.,* 1995, vol. 27, 779-788
- **SADOWSKI I. ; STONE J.C. ; PAWSON T.** *Mol. Cell. Biol.,* 1986, vol. 6, 4396-4408
- **RUSSEL R.B. ; BREED J. ; BARTON G.J.** *FEBS Lett.,* 1992, vol. 304, 15-20
- **MARANGERE L.E.M. ; PAWSON T.** *J. Cell Sci.,* 1994, vol. 18, 97-104
- **MAYER B.J. ; BALTIMORE D.** *Trends Cell. Biol.,* 1993, vol. 3, 8-13
- **SANDAL N.N. ; MARCKER K.A.** *Trends Biochem. Sci.,* 1994, vol. 19, 19-19
- **SMITH F.W. ; HAWKESFORD M.J. ; PROSSER I.M. ; CLARKSON D.T.** *Mol. Gen. Genet.,* 1995, vol. 247, 709-715
- **PALMER KJ ; TICHELAAR W ; MYERS N ; BURNS NR ; BUTCHER SJ ; KINGSMAN AJ ; FULLER SD ; SAIBIL HR.** Medline: 97404699. Cryo-electron microscopy structure of yeast Ty retrotransposon virus-like particles. *J Virol,* 1997, vol. 71, 6863-6868
- **DREYER MK ; SCHULZ GE.** Medline: 93294819. The spatial structure of the class II L-fuculose-1-phosphate aldolase from Escherichia coli. *J Mol Biol,* 1993, vol. 231, 549-553
- **DREYER MK ; SCHULZ GE.** Medline: 96256522. Catalytic mechanism of the metal-dependent fuculose aldolase from Escherichia coli as derived from the structure. *J Mol Biol,* 1996, vol. 259, 458-466
- **XU GY ; ONG E ; GILKES NR ; KILBURN DG ; MUHANDIRAM DR ; HARRIS-BRANDTS M ; CARVER JP ; KAY LE ; HARVEY TS.** Medline: 95284032. Solution structure of a cellulose-binding domain from Cellulomonas fimi by nuclear magnetic resonance spectroscopy. *Biochemistry,* 1995, vol. 34, 6993-7009

- **GLUSCHANKOF P ; FULLER RS.** Medline: 94252314. A C-terminal domain conserved in precursor processing proteases is required for intramolecular N-terminal maturation of pro-Kex2 protease. *EMBO J,* 1994, vol. 13, 2280-2288
- **ZHOU A ; MARTIN S ; LIPKIND G ; LAMENDOLA J ; STEINER DF.** Medline: 98225190. Regulatory roles of the P domain of the subtilisin-like prohormone convertases. *J Biol Chem,* 1998, vol. 273, 11107-11114
- **HAYASHI H ; DE BELLIS L ; YAMAGUCHI K ; KATO A ; HAYASHI M ; NISHIMURA M.** Medline: 98192624. "Molecular characterization of a glyoxysomal long chain acyl-CoA oxidase that is synthesized as a precursor of higher molecular mass in pumpkin. *J Biol Chem,* 1998, vol. 273, 8301-8307
- **AKIRA T ; KOMATSU M ; NANGO R ; TOMOOKA A ; KONAKA K ; YAMAUCHI M ; KITAMURA Y ; NOMURA S ; TSUKAMOTO I.** Medline: 97473523 "Molecular cloning and expression of a rat cDNA encoding 5-aminoimidazole-4-carboxamide ribonucleotide formyltransferase/IMP cyclohydrolase. *Gene,* 27 February 1998, vol. 208 (2), 337
- *Gene,* 1997, vol. 197, 289-293
- **RAYL EA ; MOROSON BA ; BEARDSLEY GP.** Medline: 96147205 "The human purH gene product, 5-aminoimidazole-4-carboxamide ribonucleotide formyltransferase/IMP cyclohydrolase. Cloning, sequencing, expression, purification, kinetic analysis, and domain mapping. *J Biol Chem,* 1996, vol. 271, 2225-2233
- **ITO Y ; SAISHO D ; NAKAZONO M ; TSUTSUMI N ; HIRAI A.** Medline: 98086211 "Transcript levels of tandem-arranged alternative oxidase genes in rice are increased by low temperature. *Gene,* 1997, vol. 203, 121-129
- **LI Q ; RITZEL RG ; MCLEAN LL ; MCINTOSH L ; KO T ; BERTRAND H ; NARGANG FE.** Medline: 96366413 "Cloning and analysis of the alternative oxidase gene of Neurospora crassa. *Genetics,* 1996, vol. 142, 129-140
- **HANKS S.K.** *Curr. Opin. Struct. Biol.,* 1991, vol. 1, 369-383
- **KNIGHTON D.R. ; ZHENG J. ; TEN EYCK L.F. ; ASHFORD V.A. ; XUONG N.-H. ; TAYLOR, S.S. ; SOWADSKI J.M.** *Science,* 1991, vol. 253, 407-414
- **KIRBY R.** *J. Mol. Evol.,* 1992, vol. 30, 489-492
- **GALLO K.A. ; KNOWLES J.R.** *Biochemistry,* 1993, vol. 32, 3981-3990
- **KURIMA K ; WARMAN ML ; KRISHNAN S ; DOMOWICZ M ; KRUEGER RC JR ; DEYRUP A ; SCHWARTZ NB.** Medline: 98337975 "A member of a family of sulfate-activating enzymes causes murine brachymorphism. *Proc Natl Acad Sci U S A,* 29 September 1998, vol. 95 (20), 12071
- *Proc Natl Acad Sci U S A,* 1998, vol. 95, 8681-8685
- **ROSENTHAL E ; LEUSTEK T.** Medline: 96096529 "A multifunctional Urechis caupo protein, PAPS synthetase, has both ATP sulfurylase and APS kinase activities. *Gene,* 1995, vol. 165, 243-248
- **GUO Y ; KAISER K ; WIECZOREK H ; DOW JA.** Medline: 96269411 "The Drosophila melanogaster gene vha14 encoding a 14-kDa F-subunit of the vacuolar ATPase. *Gene,* 1996, vol. 172, 239-243
- **PENG SB ; CRIDER BP ; TSAI SJ ; XIE XS ; STONE DK.** Medline: 96216416 "Identification of a 14-kDa subunit associated with the catalytic sector of clathrin-coated vesicle H+-ATPase. *J Biol Chem,* 1996, vol. 271, 3324-3327
- **WILMS R ; FREIBERG C ; WEGERLE E ; MEIER I ; MAYER F ; MULLER V.** Medline: 96324968 "Subunit structure and organization of the genes of the A1A0 ATPase from the Archaeon Methanosarcina mazei Gol. *J Biol Chem,* 1996, vol. 271, 18843-18852
- **RAUX E ; LANOIS A ; WARREN MJ ; RAMBACH A ; THERMES C.** Medline: 98416126 "Cobalamin (vitamin B12) biosynthesis: identification and characterization of a Bacillus megaterium cobI operon. *Biochem J,* 1998, vol. 335, 159-166
- **FEAMLEYI.M. ; WALKER J.E.** *Biochim. Biophys. Acta,* 1992, vol. 1140, 105-134
- **CIRILLI M. ; ZHENG R. ; SCAPIN G. ; BLANCHARD J.S.** *Biochemistry,* 1998, vol. 37, 16452-16458
- **HIMMELREICH R ; HILBERT H ; PLAGENS H ; PIRKL E ; LI BC ; HERRMANN R.** Medline: 97105885 "Complete sequence analysis of the genome of the bacterium Mycoplasma pneumoniae. *Nucleic Acids Res,* 1996, vol. 24, 4420-4449
- **BEDFORD MT ; LEDER P.** Medline: 99322199 "The FF domain: a novel motif that often accompanies WW domains. *Trends Biochem Sci,* 1999, vol. 24, 264-265
- **HOFER J ; TURNER L ; HELLENS R ; AMBROSE M ; MATTHEWS P ; MICHAEL A ; ELLIS N.** Medline: 97411151 "UNIFOLIATA regulates leaf and flower morphogenesis in pea. *Curr Biol,* 1997, vol. 7, 581-587
- **WEIGEL D ; ALVAREZ J ; SMYTH DR ; YANOFSKY MF ; MEYEROWITZ EM.** Medline: 92274452 "LEAFY controls floral meristem identity in Arabidopsis. *Cell,* 1992, vol. 69, 843-859
- **ARAVIND L ; KOONIN EV.** Medline: 10470032 "G-patch: a new conserved domain in eukaryotic RNA-processing proteins and type D retroviral polyproteins. *Trends Biochem Sci,* 1999, vol. 24, 342-344
- **BENZ R. ; BAUER K.** *Eur. J. Biochem.,* 1988, vol. 176, 1-19
- **JAP B.K. ; WALIAN P.J.** *Q. Rev. Biophys.,* 1990, vol. 23, 367-403
- **JEANTEUR D. ; LAKEY J.H. ; PATTUS F.** *Mol. Microbiol.,* 1991, vol. 5, 2153-2164

- **GILSON L. ; MAHANTY H.K. ; KOLTER R.** *EMBO J.,* 1990, vol. 9, 3875-3884
- **LETOFFE S. ; DELEPELAIRE P. ; WANDERSMAN C.** *EMBO J.,* 1990, vol. 9, 1375-1382
- **DUONG F. ; LAZDUNSKI A. ; CAMI B. ; MURGIER M.** *Gene,* 1992, vol. 121, 47-54
- **LEWIS K.** *Trends Biochem. Sci.,* 1994, vol. 19, 119-123
- **MORETT E ; BORK P.** Medline: 10366851 ''A novel transactivation domain in parkin. *Trends Biochem Sci,* 1999, vol. 24, 229-231
- **VAN DER REIJDEN BA ; ERPELINCK-VERSCHUEREN CA ; LOWENBERG B ; JANSEN JH.** Medline: 99349709 ''TRIADs: a new class of proteins with a novel cysteine-rich signature. *Protein Sci,* 1999, vol. 8, 1557-1561
- **DURAND JM ; BJORK GR ; KUWAE A ; YOSHIKAWA M ; SASAKAWA C.** Medline: 97440126 ''The modified nucleoside 2-methylthio-N6-isopentenyladenosine in tRNA of Shigella flexneri is required for expression of virulence genes. *J Bacteriol,* 1997, vol. 179, 5777-5782
- **BOGUTA M ; HUNTER LA ; SHEN WC ; GILLMAN EC ; MARTIN NC ; HOPPER AK.** Medline: 94187700 ''Subcellular locations of MOD5 proteins: mapping of sequences sufficient for targeting to mitochondria and demonstration that mitochondrial and nuclear isoforms commingle in the cytosol. *Mol Cell Biol,* 1994, vol. 14, 2298-2306
- **GILLMAN EC ; SLUSHER LB ; MARTIN NC ; HOPPER AK.** Medline: 91203856 ''MOD5 translation initiation sites determine N6-isopentenyladenosine modification of mitochondrial and cytoplasmic tRNA. *Mol Cell Biol,* 1991, vol. 11, 2382-2390
- **HA H ; ABE K ; ARTZT K.** Medline: 92084131 ''Primary structure of the embryo-expressed gene KE2 from the mouse H-2K region. *Gene,* 1991, vol. 107, 345-346
- **SHANG HS ; WONG SM ; TAN HM ; WU M.** Medline: 95129859 ''YKE2, a yeast nuclear gene encoding a protein showing homology to mouse KE2 and containing a putative leucine-zipper motif. *Gene,* 1994, vol. 151, 197-201
- **HAYASHI S. ; WU H.C.** *J. Bioenerg. Biomembr.,* 1990, vol. 22, 451-471
- **ZHANG Q ; WISE KS.** Medline: 96294788 ''Molecular basis of size and antigenic variation of a Mycoplasma hominis adhesin encoded by divergent vaa genes. *Infect Immun,* 1996, vol. 64, 2737-2744
- **HENRICH B ; KITZEROW A ; FELDMANN RC ; SCHAAL H ; HADDING U.** Medline: 97047675 ''Repetitive elements of the Mycoplasma hominis adhesin p50 can be differentiated by monoclonal antibodies. *Infect Immun,* 1996, vol. 64, 4027-4034
- **SUGINO H ; SASAKI M ; AZAKAMI H ; YAMASHITA M ; MUROOKA Y.** Medline: 96235221 ''A monoamine-regulated Klebsiella aerogenes operon containing the monoamine oxidase structural gene (maoA) and the maoC gene. *J Bacteriol,* 1992, vol. 174, 2485-2492
- **PHILBRICK JB ; ZILINSKAS BA.** Medline: 88334494 ''Cloning, nucleotide sequence and mutational analysis of the gene encoding the Photosystem II manganese-stabilizing polypeptide of Synechocystis 6803. *Mol Gen Genet,* 1988, vol. 212, 418-425
- **MAKAROVA KS ; ARAVIND L ; GALPERIN MY ; GRISHIN NV ; TATUSOV RL ; WOLF YI ; KOONIN EV.** Medline: 99342100 ''Comparative genomics of the Archaea (Euryarchaeota): evolution of conserved protein families, the stable core, and the variable shell. *Genome Res,* 1999, vol. 9, 608-628
- **WU P ; BROCKENBROUGH JS ; METCALFE AC ; CHEN S ; ARIS JP.** Medline: 98298165 ''Nop5p is a small nucleolar ribonucleoprotein component required for pre- 18 S rRNA processing in yeast. *J Biol Chem,* 1998, vol. 273, 16453-16463
- **GAUTIER T ; BERGES T ; TOLLERVEY D ; HURT E.** Medline: 8038777 ''Nucleolar KKE/D repeat proteins Nop56p and Nop58p interact with Nop1p and are required for ribosome biogenesis. *Mol Cell Biol,* 1997, vol. 17, 7088-7098
- **WEIDENHAMMER EM ; SINGH M ; RUIZ-NORIEGA M ; WOOLFORD JL JR.** Medline: 96184869 ''The PRP31 gene encodes a novel protein required for pre-mRNA splicing in Saccharomyces cerevisiae. *Nucleic Acids Res,* 1996, vol. 24, 1164-1170
- **GOVONI G ; GROS P.** Medline: 98383996 ''Macrophage NRAMP1 and its role in resistance to microbial infections. *Inflamm Res,* 1998, vol. 47, 277-284
- **AGRANOFF DD ; KRISHNA S.** Medline: 98294035 ''Metal ion homeostasis and intracellular parasitism. *Mol Microbiol,* 1998, vol. 28, 403-412
- **PINNER E ; GRUENHEID S ; RAYMOND M ; GROS P.** Medline: 98030569 ''Functional complementation of the yeast divalent cation transporter family SMF by NRAMP2, a member of the mammalian natural resistance- associated macrophage protein family. *J Biol Chem,* 1997, vol. 272, 28933-28938
- **CELLIER M ; BELOUCHI A ; GROS P.** Medline: 96402487 ''Resistance to intracellular infections: comparative genomic analysis ofNramp. *Trends Genet,* 1996, vol. 12, 201-204
- **CELLIER M ; PRIVE G ; BELOUCHI A ; KWAN T ; RODRIGUES V ; CHIA W ; GROS P.** Medline: 96036029 ''Nramp defines a family of membrane proteins. *Proc Natl Acad Sci U S A,* 1995, vol. 92, 10089-10093

- **HOLM L ; SANDER C.** Medline: 96005605 "DNA polymerase beta belongs to an ancient nucleotidyltransferase superfamily. *Trends Biochem Sci,* 1995, vol. 20, 345-347
- **BOWMAN MC ; SMALLWOOD S ; MOYER SA.** Medline: 99329169 "Dissection of Individual Functions of the Sendai Virus Phosphoprotein in Transcription. *J Virol,* 1999, vol. 73, 6474-6483
- **MATSUOKA Y ; CURRAN J ; PELET T ; KOLAKOFSKY D ; RAY R ; COMPANS RW.** Medline: 91237868 "The P gene of human parainfluenza virus type 1 encodes P and C proteins but not a cysteine-rich V protein. *J Virol,* 1991, vol. 65, 3406-3410
- **BANFALVI Z ; KOSTYAL Z ; BARTA E.** Medline: 95107249 "Solanum brevidens possesses a non-sucrose-inducible patatin gene. *Mol Gen Genet,* 1994, vol. 245, 517-522
- **MIGNERY GA ; PIKAARD CS ; PARK WD.** Medline: 88226014 "Molecular characterization of the patatin multigene family of potato. *Gene,* 1988, vol. 62, 27-44
- **BATEMAN A ; MURZIN A ; TEICHMANN SA.** Medline: 98318059 "Structure and distribution of pentapeptide repeats in bacteria. *Protein Sci,* 1998, vol. 7, 1477-1480
- **COLE ST ; BROSCH R ; PARKHILL J ; GARNIER T ; CHURCHER C ; HARRIS D ; GORDON SV ; EIGLMEIER K ; GAS S ; BARRY CE 3RD.** Medline: 98295987 "Deciphering the biology of Mycobacterium tuberculosis from the complete genome sequence. *Nature,* 1998, vol. 393, 537-544
- **CHEN JM ; DANDO PM ; RAWLINGS ND ; BROWN MA ; YOUNG NE ; STEVENS RA ; HEWITT E ; WATTS C ; BARRETT AJ.** Medline: 97218252 "Cloning, isolation, and characterization of mammalian legumain, an asparaginyl endopeptidase. *J Biol Chem,* 1997, vol. 272, 8090-8098
- **LING M ; ALLEN SW ; WOOD JM.** Medline: 95055736 "Sequence analysis identifies the proline dehydrogenase and delta 1- pyrroline-5-carboxylate dehydrogenase domains of the multifunctional Escherichia coli PutA protein. *J Mol Biol,* 1994, vol. 243, 950-956
- **ROVA EM ; MC EWEN B ; FREDRIKSSON PO ; STYRING S.** Medline: 97067138 "Photoactivation and photoinhibition are competing in a mutant of Chlamydomonas reinhardtii lacking the 23-kDa extrinsic subunit of photosystem II. *J Biol Chem,* 1996, vol. 271, 28918-28924
- **KOCHHAR A ; KHURANA JP ; TYAGI AK.** Medline: 97191538 "Nucleotide sequence of the psbP gene encoding precursor of 23-kDa polypeptide of oxygen-evolving complex in Arabidopsis thaliana and its expression in the wild-type and a constitutively photomorphogenic mutant. *DNA Res,* 1996, vol. 3, 277-285
- **ARAVIND L ; KOONIN EV.** Medline: 99193178 "Novel predicted RNA-binding domains associated with the translation machinery. *J Mol Evol,* 1999, vol. 48, 291-302
- **FROLOVA L ; LE GOFF X ; RASMUSSEN HH ; CHEPEREGIN S ; DRUGEON G ; KRESS M ; ARMAN I ; HAENNI AL ; CELIS JE ; PHILIPPE M et al.** Medline: 95082951 "A highly conserved eukaryotic protein family possessing properties of polypeptide chain release factor" [see comments. *Nature,* 1994, vol. 372, 701-703
- **DRUGEON G ; JEAN-JEAN O ; FROLOVA L ; LE GOFF X ; PHILIPPE M ; KISSELEV L ; HAENNI AL.** Medline: 97315314 "Eukaryotic release factor 1 (eRF1) abolishes readthrough and competes with suppressor tRNAs at all three termination codons in messenger RNA. *Nucleic Acids Res,* 1997, vol. 25, 2254-2258
- **MEZQUITA J ; PAU M ; MEZQUITA C.** Medline: 97449308 "Characterization and expression of two chicken cDNAs encoding ubiquitin fused to ribosomal proteins of 52 and 80 amino acids. *Gene,* 1997, vol. 195, 313-319
- **REDMAN KL ; RECHSTEINER M.** Medline: 89181932 "Identification of the long ubiquitin extension as ribosomal protein S27a. *Nature,* 1989, vol. 338, 438-440
- **FINLEY D ; BARTEL B ; VARSHAVSKY A.** Medline: 89181925 "The tails of ubiquitin precursors are ribosomal proteins whose fusion to ubiquitin facilitates ribosome biogenesis. *Nature,* 1989, vol. 338, 394-401
- **DENHEZ F ; LAFYATIS R.** Medline: 94266805 "Conservation of regulated alternative splicing and identification of functional domains in vertebrate homologs to the Drosophila splicing regulator, suppressor-of-white-apricot. *J Biol Chem,* 1994, vol. 269, 16170-16179
- **OHKUMA Y ; SUMIMOTO H ; HOFFMANN A ; SHIMASAKI S ; HORIKOSHI M ; ROEDER RG.** Medline: 92065982 "Structural motifs and potential sigma homologies in the large subunit of human general transcription factor TFIIE. *Nature,* 1991, vol. 354, 398-401
- **OHKUMA Y ; HASHIMOTO S ; ROEDER RG ; HORIKOSHI M.** Medline: 93087200 Identification of two large subdomains in TFIIE-alpha on the basis of homology between Xenopus and human sequences. *Nucleic Acids Res,* 1992, vol. 20, 5838-5838
- **KLENK HP ; CLAYTON RA ; TOMB JF ; WHITE O ; NELSON KE ; KETCHUM KA ; DODSON RJ ; GWINN M ; HICKEY EK ; PETERSON JD.** Medline: 98049343 "The complete genome sequence of the hyperthermophilic, sulphate- reducing archaeon Archaeoglobus fulgidus. *Nature,* 1997, vol. 390, 364-370
- **ICHINOSE A. ; BOTTENUS R.E. ; DAVIE E.W.** *J. Biol. Chem.,* 1990, vol. 265, 13411-13414

- **GREENBERG C.S. ; BIRCKBICHLER P.J. ; RICE R.H.** *FASEB J.,* 1991, vol. 5, 3071-3077
- **NURSE K ; WRZESINSKI J ; BAKIN A ; LANE BG ; OFENGAND J.** Medline: 96079944 "Purification, cloning, and properties of the tRNA psi 55 synthase from Escherichia coli. *RNA,* 1995, vol. 1, 102-112
- **LAFONTAINE DLJ ; BOUSQUET-ANTONELLI C ; HENRY Y ; CAIZERGUES-FERRER M ; TOLLERVEY D.** Medline: 98139521 "The box H + ACA snoRNAs carry Cbf5p, the putative rRNA pseudouridine synthase. *Genes Dev,* 1998, vol. 12, 527-537
- **DUGGLEBY RG ; CHAO YC ; HUANG JG ; PENG HL ; CHANG HY.** Medline: 96202932 "Sequence differences between human muscle and liver cDNAs for UDPglucose pyrophosphorylase and kinetic properties of the recombinant enzymes expressed in Escherichia coli. *Eur J Biochem,* 1996, vol. 235, 173-179
- **RAGHEB JA ; DOTTIN RP.** Medline: 87231075 "Structure and sequence of a UDP glucose pyrophosphorylase gene of Dictyostelium discoideum. *Nucleic Acids Res,* 1987, vol. 15, 3891-3906
- **MIO T ; YABE T ; ARISAWA M ; YAMADA-OKABE H.** Medline: 98269105 "The eukaryotic UDP-N-acetylglucosamine pyrophosphorylases. Gene cloning, protein expression, and catalytic mechanism. *J Biol Chem,* 1998, vol. 273, 14392-14397
- **HEYNINCK K ; BEYAERT R.** Medline: 99126071 "The cytokine-inducible zinc finger protein A20 inhibits IL-1-induced NF- kappaB activation at the level of TRAF6. *FEBS Lett,* 1999, vol. 442, 147-150
- **DE VALCK D ; HEYNINCK K ; VAN CRIEKINGE W ; CONTRERAS R ; BEYAERT R ; FIERS W.** Medline: 96390831 "A20, an inhibitor of cell death, self-associates by its zinc finger domain. *FEBS Lett,* 1996, vol. 384, 61-64
- **SONG HY ; ROTHE M ; GOEDDEL DV.** Medline: 96270609 "The tumor necrosis factor-inducible zinc finger protein A20 interacts with TRAF1/TRAF2 and inhibits NF-kappaB activation. *Proc Natl Acad Sci U S A,* 1996, vol. 93, 6721-6725
- **OPIPARI AW JR ; BOGUSKI MS ; DIXIT VM.** Medline: 90368626 "The A20 cDNA induced by tumor necrosis factor alpha encodes a novel type of zinc finger protein. *J Biol Chem,* 1990, vol. 265, 14705-14708
- **ALTHAUS F.R. ; RICHTER C.R.** *Mol. Biol. Biochem. Biophys.,* 1987, vol. 37, 1-126
- **DE MURCIA G. ; MENISSIER DE MURCIA J.** *Trends Biochem. Sci.,* 1994, vol. 19, 172-176
- **WEI Y.-F. ; ROBINS P. ; CARTER K. ; CALDECOTT K. ; PAPPIN D.J.C. ; YU G.-L. ; WANG R.-P. ; SHELL B.K. ; NASH R.A. ; SCHAR P.** *Mol. Cell. Biol.,* 1995, vol. 15, 3206-3216
- **BON et al.** *Appl Biochem Biotechnol,* 1997, vol. 63-65, 203-12
- **LEUTWILER et al.** *Nucleic Acids Res,* 1986, vol. 14, 4051-64
- **BRANDT et al.** *Plant Mol Biol,* 1992, vol. 19, 699-703
- **GARCIA-RAMIREZ et al.** *J Biol Chem,* 1995, vol. 270, 23801-7
- **YANG et al.** *PNAS USA,* 1993, vol. 90, 5086-90
- **USTAV ; STENLUND.** *EMBO J,* 1991, vol. 10, 449-57
- **CALLAWAY et al.** *Mol Plant Microbe Interact,* 1996, vol. 9, 810-8
- **KINZY et al.** *NAR,* 1994, vol. 22, 2703-7
- **DUNN et al.** *Plant Mol Biol,* 1993, vol. 23, 221-5
- **AGUILAR et al.** *Plant Mol Biol,* 1991, vol. 17, 351-60
- **MAMOUN et al.** *J Virol,* 1990, vol. 64, 4180-8
- **GRANDBASTIEN et al.** *Nature,* 1989, vol. 337, 376-80
- **WRIGHT ; VOYTAS.** *Genetics,* 1998, vol. 149, 703-15
- **GIORDA et al.** *Biochemistry,* 1990, vol. 29, 7264-9
- **TUCKER et al.** *Plant Physiol,* 1988, vol. 88, 1257-62
- **SHANI et al.** *PLANT PHYSIOL,* 1997, vol. 43, 837-42
- **MILLIGAN ; GASSER.** *Plant Mol Biol,* 1995, vol. 28, 691-711
- **ZEEMAN et al.** *Plant J,* 1998, vol. 15, 357-65
- **HIRANO ; NAKAMURA.** *Plant Physiol,* 1997, vol. 114, 5675-82
- **KITAMOTO et al.** *J Bacteriol,* 1988, vol. 170, 5848-54
- **CROMBIE et al.** *Plant J,* 1998, vol. 15, 27-38
- **HATA et al.** *Agric Biol Chem,* 1991, vol. 55, 941-9
- **GRAHAM et al.** *J Biol Chem,* 1988, vol. 263, 16823-9
- **O'DOWD et al.** *Biochemistry,* 1988, vol. 27, 5216-26
- **LAUDET et al.** *Nucleic Acids Res,* 1993, vol. 21, 2493-501
- **ZHENG et al.** *Plant Mol Biol,* 1993, vol. 23, 813-23
- **GRASSER et al.** *Plant Mol Biol,* 1993, vol. 23, 619-25
- **HEIKE et al.** *Scand J Immunol,* 1997, vol. 45, 221-6
- **ARIEL et al.** *J Immunol,* 1998, vol. 161, 2465-72
- **SCHINK.** *Anticancer Drugs,* 1997, vol. 8 (1), 47-51
- **BAKER et al.** *Proc Soc Exp Biol Med,* 1998, vol. 217, 317-21

- **SCHALLER ; WEILER.** *J Biol Chem,* 1997, vol. 272, 28066-72
- **MANDANI et al.** *PNAS USA,* 1994, vol. 91, 922-6
- **BURROWS et al.** *EMBO J,* 1998, vol. 17, 868-76
- **KOFER et al.** *Mol Gen Genet,* 1998, vol. 258, 166-73
- **MAIER et al.** *J Mol Biol,* 1995, vol. 251, 614-28
- **HILSON et al.** *Plant Physiol,* 1993, vol. 103, 525-33
- **BELOSTOTSKY ; MEAGHER.** *PNAS USA,* 1993, vol. 90, 6686-90
- **LIU et al.** *J Gen Virol,* 1997, vol. 78, 1265-70
- **ROHDE et al.** *Virology,* 1990, vol. 176, 648-51
- **ZHANG et al.** *J Biol Chem,* 1994, vol. 269, 17586-92
- **MIZOGUCHI et al.** *FEBS Lett,* 1995, vol. 358, 199-204
- **SODROSKI et al.** *Nature,* 1986, vol. 321, 412-7
- **FRANCHINI et al.** *PNAS USA,* 1989, vol. 86, 2433-7
- **MARQUET et al.** *PNAS USA,* 1995, vol. 77, 113-24
- **GIULIANO et al.** *PNAS USA,* 1988, vol. 85, 7089-93
- **DEDONDER et al.** *Plant Physiol,* 1993, vol. 101, 801-8
- **WEE et al.** *Plant Cell,* 1998, vol. 10, 1759-68
- **LEE et al.** *J Biol Chem,* 1994, vol. 269, 10028-33
- **KITAGAWA ; PAULSON.** *J Biol Chem,* 1994, vol. 269, 1394-401
- **CHANG et al.** *Science,* 1993, vol. 262, 539-44
- **NAGAYA et al.** *Gene,* 1993, vol. 131, 119-124
- **GOTTFERT et al.** *PNAS USA,* 1990, vol. 87, 2680-4
- **OKAMOTO et al.** *J Gen Virol,* 1988, vol. 69, 2575-83
- **PANG et al.** *Nucleic Acids Res,* 1993, vol. 21, 1647-53
- **DENG et al.** *Cell,* 1992, vol. 71, 791-801
- **VITTORIOSO et al.** *Mol Cell Biol,* 1998, vol. 18, 3034-43
- **HERNANDEZ TORRES et al.** *MOL CELL BIOL,* 1995, vol. 27, 1221-6
- **FUJIMURA-KAMADA et al.** *J Cell Biol.,* 1997, vol. 136, 271-85
- **TAM et al.** *J Cell Biol.,* 1998, vol. 142, 635-49
- **CHAPDELAINE ; BONEN.** *Cell,* 1991, vol. 65, 465-72
- **FERAT ; MICHE.** *Nature,* 1993, vol. 364, 358-61
- **WILSON et al.** *NATURE,* 1994, vol. 368, 32-8
- **CAMBARERI et al.** *NATURE,* 1994, vol. 242, 658-65
- **GAARDNER et al.** *NAR,* 1981, vol. 9, 2871-2888
- **CUMMINGS et al.** *Curr Genet,* 1990, vol. 17, 375-402
- **HATTORI et al.** *Nature,* 1986, vol. 321, 625-8
- **EMORI Y ; SAIGO K.** *J Biol Chem,* 1994, vol. 269, 25137-42
- **MELLGREN RL ; LANE RD ; MERICLE MT.** *Biochim Biophys Acta,* 1989, vol. 999, 71-77
- **NIYOGI KK ; FINK GR.** *Plant Cell,* 1992, vol. 4, 721-33
- **SONG HS ; BROTHERTON JE ; GONZALES RA ; WILHOLM JM.** *Plant Physiol,* 1998, vol. 117, 533-43
- **THOMPSON SR ; MELCHER U.** *J Gen Virol,* 1993, vol. 74, 1141-8
- **LINDAHL M ; TABAK S ; CSEKE L ; PICHERSKY E ; ANDERSSON B ; ADAM Z.** *J Biol Chem,* 1996, vol. 271, 29329-34
- **KOSTRZEWA M ; ZETSCHE K.** *J Mol Biol,* 1992, vol. 227, 961-70
- **KOSTRZEWA M ; ZETSCHE K.** *Plant Mol Biol,* 1993, vol. 23, 67-76
- **ZWERSCHKE W ; JANSEN-DURR P.** *Adv Cancer Res,* 2000, vol. 78, 1-29
- **KANEKO T ; SATO S ; KOTANI H ; TANAKA A ; ASAMIZU E ; NAKAMURA Y ; MIYAJIMA N ; HIROSAWA M ; SUGIURA M ; SASAMOTO S.** *DNA Res,* 1996, vol. 3, 109-36
- **OELLER et al.** *Science,* 1991, vol. 254, 437
- **MARIANI et al.** *Nature,* 1992, vol. 357, 384-387
- **SHEEHY et al.** *Proc. Nat. Acad. Sci. USA,* 1988, vol. 85, 8805
- **A.C. VERGUNST et al.** *Nucleic Acids Res.,* 1998, vol. 26, 2729
- **A.C. VERGUNST et al.** *Plant Mol. Biol.,* 1998, vol. 38, 393
- **H. ALBERT et al.** *Plant J.,* 1995, vol. 7, 649
- **AZPIROZ-LEEHAN et al.** *Trends in Genetics,* 1997, vol. 13, 152
- **SHIZUYA et al.** *Proc. Natl. Acad. Sci. USA,* 1992, vol. 89, 8794-8797
- **HAMILTON et al.** *Proc. Natl. Acad. Sci. USA,* 1996, vol. 93, 9975-9979
- **BURKE et al.** *Science,* 1987, vol. 236, 806-812
- **STERNBERG N. et al.** *Proc Natl Acad Sci U S A.,* January 1990, vol. 87 (1), 103-7
- **BRADSHAW et al.** *Nucl Acids Res,* 1995, vol. 23, 4850-4856
- **FRISCHAUF et al.** *J. Mol Biol,* 1983, vol. 170, 827-842
- **HUYNH et al.** DNA Cloning: A practical Approach. IRL Press, 1985, vol. 1
- **WALDEN et al.** *Mol Cell Biol,* 1990, vol. 1, 175-194
- **NAPOLI et al.** *Plant Cell,* 1990, vol. 2, 279

- **VAN DER KROL et al.** *Plant Cell,* 1990, vol. 2, 291
- **MARTY.** *The Plant Cell,* 1999, vol. 11, 587-599
- **VITALE ; DENECKE.** *The Plant Cell,* 1999, vol. 11, 615-628
- **KEEGSTRA ; CLINE.** *The Plant Cell,* 1999, vol. 11, 557-570
- **WEISING et al.** *Ann. Rev. Genet.,* 1988, vol. 22, 421
- **CHRISTOU.** *Euphytica,* 1995, vol. 85 (1-3), 13-27
- **MCCORMAC et al.** *Mol. Biotechnol.,* 1997, vol. 8, 199
- **HAMILTON.** *Gene,* 1997, vol. 200, 107
- **PASZKOWSKI et al.** *EMBO J.,* 1984, vol. 3, 2717
- **FROMM et al.** *Proc. Natl Acad. Sci. USA,* 1985, vol. 82, 5824
- **KLEIN et al.** *Nature,* 1987, vol. 327, 773
- **HAMILTON, CM.** *Gene,* 1997, vol. 200, 107
- **MÜLLER et al.** *Mol. Gen. Genet.,* 1987, vol. 207, 171
- **KOMARI et al.** *Plant J.,* 1996, vol. 10, 165
- **VENKATESWARLU et al.** *Biotechnology,* 1991, vol. 9, 1103
- **GLEAVE, AP.** *Plant Mol. Biol.,* 1992, vol. 20, 1203
- **GRAVES ; GOLDMAN.** *Plant Mol. Biol.,* 1986, vol. 7, 34
- **GOULD et al.** *Plant Physiology,* 1991, vol. 95, 426
- Handbook of Plant Cell Culture. **EVANS et al.** Protoplasts Isolation and Culture. MacMillan Publishing Company, 1983, 124-176
- **BINDING.** Regeneration of Plants, Plant Protoplasts. CRC Press, 1988, 21-73
- **KLEE et al.** *Ann. Rev. of Plant Phys.,* 1987, vol. 38, 467
- **HOSOYAMA et al.** *Biosci. Biotechnol. Biochem.,* 1994, vol. 58, 1500
- **GHOSH et al.** *J. Biotechnol.,* 1994, vol. 32, 1
- **SEKI et al.** High-efficiency cloning of Arabidopsis full-length cDNA by biotinylated Cap trapper. *Plant Journal,* 1998, vol. 15, 707-720
- **MARUYAMA et al.** Oligo-capping a simple method to replace the cap structure of eukaryotic mRNAs with oligoribonucleotides. *Gene,* 1994, vol. 138, 171
- **COX ; GOLDBERG.** Plant Molecular Biology: A Practical Approach. IRL, 1988, 1-35
- **COX et al.** Plant Molecular Biology: A Practical Approach. IRL, 1988, 1-35
- **KATO et al.** *Gene,* 1994, vol. 150, 243-250
- **CHANG et al.** *Gene,* 1993, vol. 127, 95
- **FRY et al.** *Biotechniques,* 1992, vol. 13, 124
- **O.L. GAMBORG et al.** *Plant Physiol.,* 1970, vol. 45, 3 72
- **Z. CHEN et al.** *Plant Mol. Bio.,* 1998, vol. 36, 163
- **NOMURA ; KOMAMINE.** *Plt. Phys.,* 1985, vol. 79, 988-991